(19) Europäisches Patentamt
European Patent Office
Office européen des brevets

(11) EP 2 476 761 A2

(12) EUROPEAN PATENT APPLICATION

(43) Date of publication:
**18.07.2012 Bulletin 2012/29**

(51) Int Cl.:
***C12Q 1/68** (2006.01)* ***G01N 33/569** (2006.01)*

(21) Application number: **11195429.3**

(22) Date of filing: **07.07.2006**

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.07.2005 US 696776 P**

(62) Document number(s) of the earlier application(s) in accordance with Art. 76 EPC:
**06760840.6 / 1 910 565**

(71) Applicant: **Athlomics Pty Ltd**
**Toowong, QLD 4066 (AU)**

(72) Inventors:
- **Brandon, Richard Bruce**
  **Boonah**
  **Queensland 4310 (AU)**
- **Thomas, Mervyn Rees**
  **Chapel Hill**
  **Queensland 4169 (AU)**

(74) Representative: **Dempster, Robert Charles**
**D Young & Co LLP**
**120 Holborn**
**London EC1N 2DY (GB)**

Remarks:
•The complete document including Reference Tables and the Sequence Listing can be downloaded from the EPO website
•This application was filed on 22-12-2011 as a divisional application to the application mentioned under INID code 62.
•Claims filed after the date of filing of the application/after the date of receipt of the divisional application (Rule 68(4) EPC).

# (54) Polynucleotide marker genes and their expression, for diagnosis of endotoxemia

(57) The invention discloses isolated endotoxemia marker polynucleotide selected from any one of 163 different polynucleotide sequences, or variants thereof. Endotoxemia related conditions are diagnosed in a test subject by aberrant expression of at least one of the endotoxemia markers or variants thereof. Of practical use, is the early diagnosis of disease, determining those animals at risk of developing endotoxemia, monitoring of an animals immune response to the disease and the enablement of better treatments. Of particular interest is the diagnosis of laminitis in hoofed animals, including horses.

EP 2 476 761 A2

## Description

### FIELD OF THE INVENTION

This invention relates generally to a method and apparatus for the diagnosis, detection of host response, monitoring, treatment and management of endotoxemia and endotoxemia-induced conditions in mammals. The invention also relates to the use of this method in monitoring, treatment and management of conditions that can lead to endotoxemia. The invention has practical use in early diagnosis, diagnosis of mild or sub-clinical endotoxemia, in the detection of specific cell immune responses as part of active or progressive disease, in monitoring clinically affected animals, and in enabling better treatment and management decisions to be made in clinically and sub-clinically affected animals. Additionally, the invention has practical use in monitoring patients in critical care or intensive care units for endotoxemia and in predicting clinical outcome.

### BACKGROUND OF THE INVENTION

Endotoxemia (also called septic shock and septic syndrome) is generally considered to result from an inability of the host defense mechanisms to cope with foreign organisms, including gram positive and negative bacteria, viruses, fungi and parasites. The majority of endotoxemia cases are caused by gram negative bacteria (Glauser et al., 1991, Lancet 338 (Sept) 732-736), in particular a product of gram negative bacteria called endotoxin or lipopolysaccharide (LPS), which is a component of the bacterial outer cell wall.

Because the main initiator of endotoxemia is endotoxin, many tests have been developed to measure these molecules in fluids, including the Limulus Amoebocyte Lysate (LAL) test (Cohen J., 2000, Intensive Care Med. 26: S51-S56), rabbit pyrogen test, and lethality in mice and chicken embryos (Hurley JC. Clin Microbiol Reviews 8(2): 268-292). However, measurement of endotoxin in fluids, especially blood, is a poor predictor of clinical outcome in endotoxemia for a number of reasons:

● The levels of endotoxin required to trigger the biological cascade of events leading to endotoxemia varies widely from patient to patient.

● The bioavailability of endotoxin varies from patient to patient depending upon the body's ability to detoxify or neutralize it.

● Some patients develop sensitivity to endotoxin, or are tolerant to endotoxin.

● Various biological fluids (and even fluid containers) contain agents that bind endotoxin that are capable of enhancing or limiting the biological effect of endotoxin, or can interfere with the measurement of endotoxin.

● Some endotoxins are more potent than others.

● The specificity and sensitivity of the LAL assay is at its limit when used for assaying endotoxin in blood or serum.

For these reasons, efforts have been made to develop assays for the determination of the biological effects of endotoxin - as a means of determining clinical outcome - including the measurement of molecules such as Tumor Necrosis Factor (TNF), C3a, C5a, Factor XII (Hageman Factor), interleukin-1 (IL-1), $\gamma$-interferon and various other cytokines, and the measurement of the level of activation of leukocytes. Such measurements have contributed to a "sepsis score" concept developed by Casey et al. (1993, Ann Intern Med. 119: 771-778).

However, none of these tests have been sufficiently sensitive, specific or practical enough to be used in routine clinical practice. In addition, the efficacy of available treatments also limits the practical use of such prognostic and monitoring tests.

Despite this, there are a number of features of endotoxemia that make early detection, monitoring, determination of clinical outcome, prognosis determination, early intervention and informed management of affected animals clinically and economically important, viz:

● Many and varied conditions can lead to endotoxemia.

● Endotoxemia can lead to many other conditions.

● Endotoxemia is often a peracute condition causing death if not correctly managed.

● It is estimated that 20-30% of human patients in intensive care units in the USA are affected and that more than 100,000 humans die each year in the USA alone (Young L & Glauser M (Eds) Gram negative septicemia and septic shock. WB Saunders Philadelphia (1991); Parrillo JE. 1990, Ann Intern Med. 113: 227-242).

● The extent of the condition in less developed countries is likely to be far higher due to poor hygiene and medical infrastructure.

● In up to 50% of cases an etiological agent is not determined.

● The condition is most common in hospitals in patients with other underlying diseases.

● The extent of subclinical disease and its effects on human health, animal husbandry, athletic performance, and ethical management are not known.

Apart from the direct detection of endotoxin, there have been many efforts to use secondary indicators of sepsis to diagnose and monitor this condition, including measuring heart rate, temperature, respiratory rate, cardiac output, systemic vascular resistance, plasma IL-6 levels, macrophage inflammatory protein-2, chemokine KC, protein C and C-reactive protein (Panacek et al., 2004, Crit Care Med. 32: 2173-2182; Ulloa and Tracey, 2005, Trends Mol Med. 1:56-63).

Currently no panel of biomarkers is used to define sepsis in humans (Buras et al., 2006, Nature Reviews Drug Discovery, 4: 854-865). However, a cytokine profile has been suggested (Ulloa et al., 2005, Trends Mol Biol. 11: 56-63) Reasons for this are the complexity of the disease, difficulty in defining the stage of disease and the apparent existence of two distinct but not mutually exclusive phases of inflammatory and anti-inflammatory responses (Bone RC., 1996, Crit Care Med. 24: 1125-1128).

Given the current limitations of diagnostic, monitoring and prognostic procedures for endotoxemia, especially in sub-clinical or early-stages, there is a need for more effective modalities for early detection, diagnosis, monitoring, prognosis and management of the various phases of sepsis including, acute, peracute, early stage, advanced, and sub-clinical endotoxemia.

An example of a complication arising from endotoxemia is laminitis that causes profound lameness in hoofed animals. It occurs in perissodactyl and artiodactyl animals, including horses, cattle, goats, sheep and other hoofed animals (ungulates). It is believed the condition results from the action of endotoxin on tissues and the lamellae of the inner hoof wall. Failure of the lamellae results in separation of the inner hoof capsule from the pedal bone and the subsequent (weight-bearing) driving of the pedal bone through the hoof capsule, and crushing of the corium, sole and coronet (Sloet van Oldruitenborgh-Oosterbaan MM., 1999, Vet Quarterly 21(4) 121-127).

There are a number of features of laminitis that make early detection, monitoring, early intervention and informed management of affected animals clinically and economically important, viz:

● The exact cause of laminitis is not known.

● The extent of subclinical disease (often called the developmental stage; Hood DM., 1999, Vet Clin Nth Amer Eq Pract 15(2): 287-294) and its effects on animal husbandry, athletic performance, and ethical management are not known.

● The first 72 hours of the disease (developmental and acute stages) is the most critical period for monitoring. Animals that have not suffered major mechanical or structural failures at this stage are likely to recover.

● The pathogenesis of the disease is poorly understood.

● Laminitis is the largest killer of horses worldwide, usually as a result of euthanasia due to progressive disease that causes serious disability and pain.

● Present diagnostics are only partially effective once the disease is established, by which time preventative management or ameliorating therapies have little effect.

● There are few practical interventions available.

Thus, there is a need for more effective modalities for early diagnosis, diagnosis of mild or sub-clinical laminitis, in the detection of specific immune responses as part of active or progressive disease, and in monitoring animals clinically affected by laminitis. Such modalities would enable better treatment and management decisions to be made in clinically and sub-clinically affected animals prior to irreversible tissue damage.

Existing technology for diagnosing endotoxemia or for monitoring conditions that lead to endotoxemia or for evaluating sequelae of endotoxemia, is limited in that the detection of bacterial endotoxin in body fluids does not correlate well with clinical signs, and the sensitivity and specificity of these technologies is insufficient to be clinically useful. In addition, because the conditions are often peracute, advanced and irreversible tissue damage may have occurred (and possibly death) by the time endotoxin is able to be detected.

In addition, existing technologies for diagnosis or evaluation of laminitis are limited and are almost entirely reliant upon clinical evaluation and the detection of lameness. In many instances the lameness can be very subtle or sub-clinical. In addition, many of these clinical changes can only be observed in advanced stages of disease, at which time irreversible tissue damage has occurred, and where humane euthanasia is the only recourse.

## SUMMARY OF THE INVENTION

The present invention represents a significant advance over current technologies for the management of affected animals. In certain advantageous embodiments, it relies upon measuring the level of certain markers in cells, especially circulating leukocytes, of the host rather than detecting endotoxin. As such, these methods are suitable for widespread screening of symptomatic and asymptomatic animals. In certain embodiments where circulating leukocytes are the subject of analysis, it is proposed that detection of a host response to endotoxemia and its sequelae (also referred to herein as "endotoxemia-related conditions") will be feasible at very early stages of its progression before extensive tissue damage has occurred.

Thus, the present invention addresses the problem of diagnosing endotoxemia-related conditions by detecting a host response that may be measured in host cells. Advantageous embodiments involve monitoring the expression of certain genes in peripheral leukocytes of the immune system, which may be reflected in changing patterns of RNA levels or protein production that correlate with the presence of active disease or response to disease.

Accordingly, in one aspect, the present invention provides methods for diagnosing the presence of an endotoxemia-related condition in a test subject, especially in an equine test subject. These methods generally comprise detecting in the test subject aberrant expression of at least one gene (also referred to herein as an "endotoxemia marker gene") selected from the group consisting of: (a) a gene having a polynucleotide expression product comprising a nucleotide sequence that shares at least 50% (and at least 51% to at least 99% and all integer percentages in between) sequence identity with the sequence set forth in any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof; (b) a gene having a polynucleotide expression product comprising a nucleotide sequence that encodes a polypeptide comprising the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (c) a gene having a polynucleotide expression product comprising a nucleotide sequence that encodes a polypeptide that shares at least 50% (and at least 51% to at least 99% and all integer percentages in between) sequence similarity with at least a portion of the sequence set forth in SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 31, 133, 135,138, 140, 142, 144, 146, 148, 150, 52, 54, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247; 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 15 contiguous amino acid residues of that sequence; and (d) a gene having a polynucleotide expression product comprising a nucleotide sequence that hybridizes to the sequence of (a), (b), (c) or a complement thereof, under at least low, medium, or high stringency conditions. In accordance with the present invention, these endotoxemia marker genes are aberrantly expressed in animals with an endotoxemia-related condition such as but not limited to an endotoxemia-induced condition, illustrative examples of which include shock, depression, abdominal discomfort, reduced pain threshold, laminitis and idiopathic conditions.

As used herein, polynucleotide expression products of endotoxemia marker genes are referred to herein as "endotoxemia marker polynucleotides." Polypeptide expression products of the endotoxemia marker genes are referred to herein as "endotoxemia marker polypeptides."

Thus, in some embodiments, the methods comprise detecting aberrant expression of an endotoxemia marker polynucleotide selected from the group consisting of (a) a polynucleotide comprising a nucleotide sequence that shares at least 50% (and at least 51% to at least 99% and all integer percentages in between) sequence identity with the sequence set forth in any one of SEQ ID NO: 1, 3, 4, 5, 6, 7, 9,10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof; (b) a polynucleotide comprising a nucleotide sequence that encodes a polypeptide comprising the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In other embodiments, the methods comprise detecting aberrant expression of an endotoxemia marker polypeptide selected from the group consisting of: (i) a polypeptide comprising an amino acid sequence that shares at least 50% (and at least 51% to at least 99% and all integer percentages in between) sequence similarity with the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (ii) a polypeptide comprising a portion of the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 5 contiguous amino acid residues of that sequence; (iii) a polypeptide comprising an amino acid sequence that shares at least 30% similarity with at least 15 contiguous amino acid residues of the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; and (iv) a polypeptide comprising a portion of the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 5 contiguous amino acid residues of that sequence and is immuno-interactive with an antigen-binding molecule that is immuno-interactive with a sequence of (i), (ii) or (iii).

Typically, such aberrant expression is detected by: (1) measuring in a biological sample obtained from the test subject the level or functional activity of an expression product of at least one endotoxemia marker gene and (2) comparing the measured level or functional activity of each expression product to the level or functional activity of a corresponding expression product in a reference sample obtained from one or more normal subjects or from one or more subjects lacking disease, wherein a difference in the level or functional activity of the expression product in the biological sample as compared to the level or functional activity of the corresponding expression product in the reference sample is indicative of the presence of an endotoxemia-related condition in the test subject. In some embodiments, the methods further comprise diagnosing the presence, stage or degree of an endotoxemia-related condition in the test subject when the measured level or functional activity of the or each expression product is different than the measured level or functional activity of the or each corresponding expression product. In these embodiments, the difference typically represents an at least about 10%, 20%, 30%, 40%, 50%, 60%, 70%, 80% or 90%, or even an at least about 100%, 200%, 300%, 400%, 500%, 600%, 700%, 800%, 900% or 1000% increase, or an at least about 10%, 20%, 30% 40%, 50%, 60%, 70%, 80%, 90%, 92%, 94%, 96%, 97%, 98% or 99%, or even an at least about 99.5%, 99.9%, 99.95%, 99.99%, 99.995% or 99.999% decrease in the level or functional activity of an individual expression product as compared to the level or functional activity of an individual corresponding expression product., which is hereafter referred to as "aberrant expression." In illustrative examples of this type, the presence of an endotoxemia-related condition is determined by detecting a decrease in the level or functional activity of at least one endotoxemia marker polynucleotide selected from (a) a polynucleotide comprising a nucleotide sequence that shares at least 50% (and at least 51% to at least 99% and all integer percentages in between) sequence identity with the sequence set forth in any one of SEQ ID NO: 5, 6, 7, 11, 13, 15, 16, 17, 18, 19, 21, 25, 26, 35, 37, 38, 41, 42, 43, 44, 50, 52, 54, 58, 60, 63, 64, 69, 70, 71, 73, 77, 79, 80, 81, 82, 83, 86, 92, 93, 96, 98, 100, 101, 102, 103, 104, 106, 115, 117, 128, 134, 137, 141, 143, 153, 155, 158, 162, 164, 166, 174, 182, 186, 188, 190, 195, 197, 199, 201, 205, 206, 207, 209, 210, 211, 214, 215, 216, 222, 223, 240, 244, 246, 248, 259, 260, 269, 272, 280, 282, 284, 286, 290, 298, 300, 302, 305, 306, 307, 309, 312, 314, 315, 316, 318, 320, 321, 323, or a complement thereof; (b) a polynucleotide comprising a nucleotide sequence that encodes a polypeptide comprising the amino acid sequence set forth in any one of SEQ ID NO: 8, 12, 14, 20, 22, 36, 51, 53, 55, 59, 65, 72, 74, 78, 87, 97, 99, 105, 116, 118, 129, 135, 138, 142, 144, 54, 56, 159, 163, 165, 167, 75, 183, 187, 189, 191, 196, 198, 200, 202, 208, 217, 241, 247, 249, 261, 273, 281, 283, 285, 287, 291, 299, 301, 303, 308, 310, 313, 317, 319, 322, 324; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 8, 12, 14, 20, 22, 36, 51, 53, 55, 59, 65, 72, 74, 78, 87, 97, 99, 105, 116, 118, 129, 135, 138, 142, 144, 154, 156, 159, 163, 165, 167, 175, 183, 187, 189, 191, 196, 198, 200, 202, 208, 217, 241, 247, 249, 261, 273, 281, 283, 285, 287, 291, 299, 301, 303, 308, 310, 313, 317, 319, 322, 324, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In other illustrative examples, the presence of an endotoxemia-related condition is determined by detecting an **increase** in the level or functional activity of at least one endotoxemia marker polynucleotide selected from (a) a polynucleotide comprising a nucleotide sequence that shares at least 50% (and at least 51% to at least 99% and all integer percentages in between) sequence identity with the sequence set forth in any one of SEQ ID NO: 1, 3, 4, 9, 10, 23, 27, 29, 31, 33, 39, 45, 47, 49, 56, 61, 66, 67, 68, 75, 76, 84, 88, 90, 94, 107, 109, 110, 111, 113, 114, 119, 121, 122, 123, 124, 125, 126, 130, 132, 136, 139, 145, 147, 149, 151, 157, 160, 168, 169, 170, 172, 173, 176, 178, 180, 184, 192, 193, 194, 203, 212, 218, 220, 224, 225, 227, 229, 231, 233, 235, 236, 237, 239, 242, 245, 250, 252, 254, 255, 257, 262, 264, 266, 268, 270, 271, 274, 276, 278, 279, 288, 292, 294, 296, 304, 311, 325, or a complement thereof; (b) a polynucleotide comprising a nucleotide sequence that encodes a polypeptide comprising the amino acid sequence set forth in any one of SEQ ID NO: 2, 24, 28, 30, 32, 34, 40, 46, 48, 57, 62, 85, 89, 91, 95, 108, 112, 120, 127, 131, 133, 140, 146, 148, 150, 152, 161, 171, 177, 179, 181, 185, 204, 213, 219, 221, 226, 228, 230, 232, 234, 238, 243, 251, 253, 256, 258, 263, 265, 267, 275, 277, 289, 293, 295, 297, 326; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 8, 12, 14, 20, 22, 36, 51, 53, 55, 59, 65, 72, 74, 78, 87, 97, 99, 105, 116, 118, 129, 135, 138, 142, 144, 154, 156, 159, 163, 165, 167, 175, 183, 187, 189, 191, 196, 198, 200, 202, 208, 217, 241, 247, 249, 261, 273, 281, 283, 285, 287, 291, 299, 301, 303, 308, 310, 313, 317, 319, 322, 324wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In some embodiments, the method further comprises diagnosing the absence of an endotoxemia-related condition when the measured level or functional activity of the or each expression product is the same as or similar to the measured level or functional activity of the or each corresponding expression product. In these embodiments, the measured level or functional activity of an individual expression product varies from the measured level or functional activity of an individual corresponding expression product by no more than about 20%, 18%, 16%, 14%, 12%, 10%, 9%, 8%, 7%, 6%, 5%, 4%, 3%, 2%, 1% or 0.1%, which is hereafter referred to as "normal expression.".

In some embodiments, the methods comprise measuring the level or functional activity of individual expression products of at least about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 endotoxemia marker polynucleotides. For example, the methods may comprise measuring the level or functional activity of an endotoxemia marker polynucleotide either alone or in combination with as much as 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 other endotoxemia marker polynucleotide(s). In another example, the methods may comprise measuring the level or functional activity of an endotoxemia marker polypeptide either alone or in combination with as much as 49, 48, 47, 46, 45, 44, 43, 42, 41, 40, 39, 38, 37, 36,35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 other endotoxemia marker polypeptides(s). In illustrative examples of this type, the methods comprise measuring the level or functional activity of individual expression products of at least 1, 2, 3, 4, 5 or 6 endotoxemia marker genes that have a very high correlation ($p<0.001$)with the presence or risk of an endotoxemia-related condition (hereafter referred to as **"level one correlation endotoxemia marker genes"),** representative examples of which include, but are not limited to, (a) a polynucleotide comprising a nucleotide sequence that shares at least 50% (and at least 51% to at least 99% and all integer percentages in between) sequence identity with the sequence set forth in any one of SEQ ID NO: 11, 23, 29, 35, 43, 44, 68, 81, 82, 84, 104, 105, 107, 119, 130, 136, 147, 155, 174, 192, 193, 245, 254, 255, 262, 264, 270, 271, 279, 296, 325, or a complement thereof; (b) a polynucleotide comprising a nucleotide sequence that encodes a polypeptide comprising the amino acid sequence set forth in any one of SEQ ID NO: 12, 24, 30, 36, 85, 108, 120, 131, 148, 156, 175, 256, 263, 265, 297, 326; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 12, 24, 30, 36, 85, 108, 120, 131, 148, 156, 175, 256, 263, 265, 297, 326, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In other illustrative examples, the methods comprise measuring the level or functional activity of individual expression products of at least 1, 2, 3, 4, 5, 6, 7 or 8 endotoxemia marker genes that have a high correlation ($p<0.005$) with the presence or risk of an endotoxemia-related condition (hereafter referred to as "**level two correlation endotoxemia marker genes**"), representative examples of which include, but are not limited to, (a) a polynucleotide comprising a nucleotide sequence that shares at least 50% (and at least 51% to at least 99% and all integer percentages in between) sequence identity with the sequence set forth in any one of SEQ ID NO: 1, 7, 9, 10, 17, 18, 21, 25, 26, 33, 54, 61, 64, 79, 80, 90, 94, 115, 117, 121, 122, 125, 126, 143, 160, 162, 164, 172, 173, 178, 184, 186, 194, 199, 205, 206, 225, 229, 242, 244, 252, 257, 259, 274, 276, 282, 284, 288, 294, 306, 316, 318, or a complement thereof; (b) a polynucleotide comprising a nucleotide sequence that encodes a polypeptide comprising the amino acid sequence set forth in any one of SEQ ID NO: 2, 8, 22, 34, 55, 62, 65, 91, 95, 116, 118, 127, 144, 161, 163, 165, 179, 185, 187, 200, 226, 230, 243, 253, 258, 275, 277, 283, 285, 289, 295, 317, 319; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 2, 8, 22, 34, 55, 62, 65, 91, 95, 116, 118, 127, 144, 161, 163, 165, 179, 185, 187, 200, 226, 230, 243, 253, 258, 275, 277, 283, 285, 289, 295, 317, 319, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In still other illustrative examples, the methods comprise measuring the level or functional activity of individual expression products of at least 1, 2, 3, 4, 5, 6, 7, 8, 9 or 10 endotoxemia marker genes that have a medium correlation ($p<0.05$)with the presence or risk of an endotoxemia-related condition (hereafter referred to as "**level three correlation endotoxemia marker genes**"), representative examples of which include, but are not limited to, (a) a polynucleotide comprising a nucleotide sequence that shares at least 50% (and at least 51% to at least 99% and all integer percentages in between) sequence identity with the sequence set forth in any one of SEQ ID NO: 3, 4, 5, 6, 13, 15, 16, 27, 31, 37, 38, 39, 41, 42, 45, 47, 49, 52, 56, 58, 63, 66, 67, 69, 70, 71, 77, 83, 86, 88, 96, 98, 100, 101, 106, 109, 110, 111, 113, 114, 128, 132, 134, 137, 139, 141, 145, 149, 151, 153, 157, 158, 166, 168, 169, 176, 180, 188, 190, 197, 203, 207, 209, 210, 211, 214, 215, 218, 220, 222, 223, 224, 231, 233, 236, 237, 239, 240, 241, 246, 250, 260, 266, 268, 269, 272, 278, 280, 286, 290, 292, 300, 304, 309, 312, 314, 315, 321, 323 , or a complement thereof; (b) a polynucleotide comprising a nucleotide sequence that encodes a polypeptide comprising the amino acid sequence set forth in any one of SEQ ID NO: 14, 28, 32, 40, 46, 48, 53, 57, 59, 72, 78, 87, 89, 97, 99, 112, 129, 133, 135, 138, 140, 142, 146, 150, 152, 154, 159, 167, 177, 181, 189, 191, 198, 204, 208, 219, 221, 232, 234, 238, 247, 251, 261, 267, 273, 281, 287, 291, 293, 301, 310, 313, 322, 324; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 14, 28, 32, 40, 46, 48, 53, 57, 59, 72, 78, 87, 89, 97, 99, 112, 129, 133, 135, 138, 140, 142, 146, 150, 152, 154, 159, 167, 177, 181, 189, 191, 198, 204, 208, 219, 221, 232, 234, 238, 247, 251, 261, 267, 273, 281, 287, 291, 293, 301, 310, 313, 322, 324, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In still other illustrative examples, the methods comprise measuring the level or functional activity of individual expression products of at least 1, 2, 3, 4, 5, 6, 7, 8, 9 or 10 endotoxemia marker genes that have a moderate correlation (significant at 72 hours post-induction only and $p<0.05$)with the presence or risk of an endotoxemia-related condition (hereafter referred to as **"level four correlation endotoxemia marker genes"),** representative examples of which include, but are not limited to, (a) a polynucleotide comprising a nucleotide sequence that shares at least 50% (and at least 51 % to at least 99% and all integer percentages in between) sequence identity with the sequence set forth in any one of SEQ ID NO: 19, 50, 60, 73, 75, 92, 93, 102, 103, 123, 124, 170, 182, 195, 201, 212, 216, 227, 235, 248, 298, 302, 305, 307, 311, 320, or a complement thereof; (b) a polynucleotide comprising a nucleotide sequence that encodes a polypeptide comprising the amino acid sequence set forth in any one of SEQ ID NO: 20, 51, 74, 76, 171, 183, 196, 202, 213, 217, 228, 249, 299, 303, 308; (c) a polynucleotide comprising a nucleotide sequence that encodes a polypeptide that shares at least 50% (and at least 51% to at least 99% and all integer percentages in between) sequence similarity with at least a portion of the sequence set forth in SEQ ID NO: 20, 51, 74, 76, 171, 183, 196, 202, 213, 217, 228, 249, 299, 303, 308, wherein the portion comprises at least 15 contiguous amino acid residues of that sequence; and (d) a polynucleotide comprising a nucleotide sequence that hybridizes to the sequence of (a), (b), (c) or a complement thereof, under at least low, medium, or high stringency conditions.

In some embodiments, the methods comprise measuring the level or functional activity of an expression product of at least 1 level one correlation endotoxemia marker gene. In other embodiments, the methods comprise measuring the level or functional activity of an expression product of at least 2 level one correlation endotoxemia marker genes. In still other embodiments, the methods comprise measuring the level or functional activity of an expression product of at least 1 level one correlation endotoxemia marker gene and the level or functional activity of an expression product of at least 1 level two endotoxemia marker gene. In still other embodiments, the methods comprise measuring the level or functional activity of an expression product of at least 2 level one correlation endotoxemia marker genes and the level or functional activity of an expression product of at least 1 level two correlation endotoxemia marker gene. In still other embodiments, the methods comprise measuring the level or functional activity of an expression product of at least 1 level one correlation endotoxemia marker gene and the level or functional activity of an expression product of at least 2 level two correlation endotoxemia marker genes.

In some embodiments, the methods comprise measuring the level or functional activity of an expression product of at least 1 level one correlation endotoxemia marker gene and the level or functional activity of an expression product of at least 1 level three correlation endotoxemia marker gene. In other embodiments, the methods comprise measuring the level or functional activity of an expression product of at least 2 level one correlation endotoxemia marker genes and the level or functional activity of an expression product of at least 1 level three correlation endotoxemia marker gene. In still other embodiments, the methods comprise measuring the level or functional activity of an expression product of at least 1 level one correlation endotoxemia marker gene and the level or functional activity of an expression product of at least 2 level three correlation endotoxemia marker genes. In still other embodiments, the methods comprise measuring the level or functional activity of an expression product of at least 1 level one correlation endotoxemia marker gene and the level or functional activity of an expression product of at least 3 level three correlation endotoxemia marker genes.

In some embodiments, the methods comprise measuring the level or functional activity of an expression product of at least 1 level one correlation endotoxemia marker gene and the level or functional activity of an expression product of at least 1 level four correlation endotoxemia marker gene. In other embodiments, the methods comprise measuring the level or functional activity of an expression product of at least 2 level one correlation endotoxemia marker genes and the level or functional activity of an expression product of at least 1 level four correlation endotoxemia marker gene. In still other embodiments, the methods comprise measuring the level or functional activity of an expression product of at least 1 level one correlation endotoxemia marker gene and the level or functional activity of an expression product of at least 2 level four correlation endotoxemia marker gene. In still other embodiments, the methods comprise measuring the level or functional activity of an expression product of at least 1 level one correlation endotoxemia marker gene and the level or functional activity of an expression product of at least 3 level four correlation endotoxemia marker genes. In still other embodiments, the methods comprise measuring the level or functional activity of an expression product of at least 1 level one correlation endotoxemia marker gene and the level or functional activity of an expression product of at least 4 level four correlation endotoxemia marker genes.

In some embodiments, the methods comprise measuring the level or functional activity of an expression product of at least 1 level two correlation endotoxemia marker gene. In other embodiments, the methods comprise measuring the level or functional activity of an expression product of at least 2 level two correlation endotoxemia marker genes. In still other embodiments, the methods comprise measuring the level or functional activity of an expression product of at least 1 level two correlation endotoxemia marker gene and the level or functional activity of an expression product of at least 1 level three correlation endotoxemia marker gene. In other embodiments, the methods comprise measuring the level or functional activity of an expression product of at least 2 level two correlation endotoxemia marker genes and the level or functional activity of an expression product of at least 1 level three correlation endotoxemia marker gene. In still other embodiments, the methods comprise measuring the level or functional activity of an expression product of at least 1 level two correlation endotoxemia marker gene and the level or functional activity of an expression product of at least 2 level three correlation endotoxemia marker genes. In still other embodiments, the methods comprise measuring the level or functional activity of an expression product of at least 1 level two correlation endotoxemia marker gene and the level or functional activity of an expression product of at least 3 level three correlation endotoxemia marker genes. In still other embodiments, the methods comprise measuring the level or functional activity of an expression product of at least 1 level two correlation endotoxemia marker gene and the level or functional activity of an expression product of at least 4 level three correlation endotoxemia marker genes.

In some embodiments, the methods comprise measuring the level or functional activity of an expression product of at least 1 level two correlation endotoxemia marker gene and the level or functional activity of an expression product of at least 1 level four correlation endotoxemia marker gene. In other embodiments, the methods comprise measuring the level or functional activity of an expression product of at least 2 level two correlation endotoxemia marker genes and the level or functional activity of an expression product of at least 1 level four correlation endotoxemia marker gene. In still other embodiments, the methods comprise measuring the level or functional activity of an expression product of at least 1 level two correlation endotoxemia marker gene and the level or functional activity of an expression product of at least 2 level four correlation endotoxemia marker genes. In still other embodiments, the methods comprise measuring the level or functional activity of an expression product of at least 1 level two correlation endotoxemia marker gene and the level or functional activity of an expression product of at least 3 level four correlation endotoxemia marker genes. In still other embodiments, the methods comprise measuring the level or functional activity of an expression product of at least 1 level two correlation endotoxemia marker gene and the level or functional activity of an expression product of at least 4 level four correlation endotoxemia marker genes. In still other embodiments, the methods comprise measuring the level or functional activity of an expression product of at least 1 level two correlation endotoxemia marker gene and the level or functional activity of an expression product of at least 5 level four correlation endotoxemia marker genes.

In some embodiments, the methods comprise measuring the level or functional activity of an expression product of at least 1 level two correlation endotoxemia marker gene. In other embodiments, the methods comprise measuring the level or functional activity of an expression product of at least 2 level two correlation endotoxemia marker gene. In still other embodiments, the methods comprise measuring the level or functional activity of an expression product of at least 1 level two correlation endotoxemia marker gene and the level or functional activity of an expression product of at least 1 level five correlation endotoxemia marker gene. In other embodiments, the methods comprise measuring the level or functional activity of an expression product of at least 2 level two correlation endotoxemia marker genes and the level or functional activity of an expression product of at least 1 level five correlation endotoxemia marker gene. In still other embodiments, the methods comprise measuring the level or functional activity of an expression product of at least 1 level two correlation endotoxemia marker gene and the level or functional activity of an expression product of at least 2 level five correlation endotoxemia marker genes. In still other embodiments, the methods comprise measuring the level or functional activity of an expression product of at least 1 level two correlation endotoxemia marker gene and the level or functional activity of an expression product of at least 3 level five correlation endotoxemia marker genes. In still other embodiments, the methods comprise measuring the level or functional activity of an expression product of at least 1 level two correlation endotoxemia marker gene and the level or functional activity of an expression product of at least 4 level five correlation endotoxemia marker genes. In still other embodiments, the methods comprise measuring the level or functional activity of an expression product of at least 1 level two correlation endotoxemia marker gene and the level or functional activity of an expression product of at least 5 level five correlation endotoxemia marker genes.

In some embodiments, the methods comprise measuring the level or functional activity of an expression product of at least 1 level three correlation endotoxemia marker gene. In other embodiments, the methods comprise measuring the level or functional activity of an expression product of at least 2 level three correlation endotoxemia marker genes. In still other embodiments, the methods comprise measuring the level or functional activity of an expression product of at least 1 level three correlation endotoxemia marker gene and the level or functional activity of an expression product of at least 1 level four correlation endotoxemia marker gene. In other embodiments, the methods comprise measuring the level or functional activity of an expression product of at least 2 level three correlation endotoxemia marker genes and the level or functional activity of an expression product of at least 1 level four correlation endotoxemia marker gene. In still other embodiments, the methods comprise measuring the level or functional activity of an expression product of at least 1 level three correlation endotoxemia marker gene and the level or functional activity of an expression product of at least 2 level four correlation endotoxemia marker genes. In still other embodiments, the methods comprise measuring the level or functional activity of an expression product of at least 1 level three correlation endotoxemia marker gene and the level or functional activity of an expression product of at least 3 level four correlation endotoxemia marker genes. In still other embodiments, the methods comprise measuring the level or functional activity of an expression product of at least 1 level three correlation endotoxemia marker gene and the level or functional activity of an expression product of at least 4 level four correlation endotoxemia marker genes. In still other embodiments, the methods comprise measuring the level or functional activity of an expression product of at least 1 level three correlation endotoxemia marker gene and the level or functional activity of an expression product of at least 5 level four correlation endotoxemia marker genes.

In some embodiments, the methods comprise measuring the level or functional activity of an expression product of at least 1 level four correlation endotoxemia marker gene. In other embodiments, the methods comprise measuring the level or functional activity of an expression product of at least 2 level four correlation endotoxemia marker genes. In other embodiments, the methods comprise measuring the level or functional activity of an expression product of at least 3 level four correlation endotoxemia marker genes. In still other embodiments, the methods comprise measuring the level or functional activity of an expression product of at least 3 level four correlation endotoxemia marker genes. In still other embodiments, the methods comprise measuring the level or functional activity of an expression product of at least 4 level four correlation endotoxemia marker genes. In still other embodiments, the methods comprise measuring the level or functional activity of an expression product of at least 5 level four correlation endotoxemia marker genes. In still other embodiments, the methods comprise measuring the level or functional activity of an expression product of at least 6 level four correlation endotoxemia marker genes.

Advantageously, the biological sample comprises blood, especially peripheral blood, which suitably includes leukocytes. Suitably, the expression product is selected from a RNA molecule or a polypeptide. In some embodiments, the expression product is the same as the corresponding expression product. In other embodiments, the expression product is a variant (e.g., an allelic variant) of the corresponding expression product.

In certain embodiments, the expression product or corresponding expression product is a target RNA (e.g., mRNA) or a DNA copy of the target RNA whose level is measured using at least one nucleic acid probe that hybridists under at least low, medium, or high stringency conditions to the target RNA or to the DNA copy, wherein the nucleic acid probe comprises at least 15 contiguous nucleotides of an endotoxemia marker polynucleotide. In these embodiments, the measured level or abundance of the target RNA or its DNA copy is normalized to the level or abundance of a reference RNA or a DNA copy of the reference RNA that is present in the same sample. Suitably, the nucleic acid probe is immobilized on a solid or semi-solid support. In illustrative examples of this type, the nucleic acid probe forms part of a spatial array of nucleic acid probes. In some embodiments, the level of nucleic acid probe that is bound to the target RNA or to the DNA copy is measured by hybridization (e.g., using a nucleic acid array). In other embodiments, the level of nucleic acid probe that is bound to the target RNA or to the DNA copy is measured by nucleic acid amplification (e.g., using a polymerase chain reaction (PCR)). In still other embodiments, the level of nucleic acid probe that is bound to the target RNA or to the DNA copy is measured by nuclease protection assay.

In other embodiments, the expression product or corresponding expression product is a target polypeptide whose level is measured using at least one antigen-binding molecule that is immuno-interactive with the target polypeptide. In these embodiments, the measured level of the target polypeptide is normalized to the level of a reference polypeptide that is present in the same sample. Suitably, the antigen-binding molecule is immobilized on a solid or semi-solid support. In illustrative examples of this type, the antigen-binding molecule forms part of a spatial array of antigen-binding molecule. In some embodiments, the level of antigen-binding molecule that is bound to the target polypeptide is measured by immunoassay (e.g., using an ELISA).

In still other embodiments, the expression product or corresponding expression product is a target polypeptide whose level is measured using at least one substrate for the target polypeptide with which it reacts to produce a reaction product. In these embodiments, the measured functional activity of the target polypeptide is normalized to the functional activity of a reference polypeptide that is present in the same sample.

In some embodiments, a system is used to perform the diagnostic methods as broadly described above, which suitably comprises at least one end station coupled to a base station. The base station is suitably caused (a) to receive subject data from the end station via a communications network, wherein the subject data represents parameter values corresponding to the measured or normalized level or functional activity of at least one expression product in the biological sample, and (b) to compare the subject data with predetermined data representing the measured or normalized level or functional activity of at least one corresponding expression product in the reference sample to thereby determine any difference in the level or functional activity of the expression product in the biological sample as compared to the level or functional activity of the corresponding expression product in the reference sample. Desirably, the base station is further caused to provide a diagnosis for the presence, absence or degree of endotoxemia-related conditions. In these embodiments, the base station may be further caused to transfer an indication of the diagnosis to the end station via the communications network.

In another aspect, the invention contemplates use of the methods broadly described above in the monitoring, treatment and management of conditions that can lead to endotoxemia, illustrative examples of which include retained placenta, meningitis, endometriosis, shock, toxic shock (i.e., a sequelae to tampon use), gastroenteritis, appendicitis, ulcerative colitis, Crohn's disease, inflammatory bowel disease, acid gut syndrome, liver failure and cirrhosis, failure of colostrum transfer in neonates, ischemia (in any organ), bacteraemia, infections within body cavities such as the peritoneal, pericardial, thecal, and pleural cavities, bums, severe wounds, excessive exercise or stress, hemodialysis, conditions involving intolerable pain (e.g., pancreatitis, kidney stones), surgical operations, and non-healing lesions. In these embodiments, the diagnostic methods of the invention are typically used at a frequency that is effective to monitor the early development of an endotoxemia-related condition to thereby enable early therapeutic intervention and treatment of that condition. In illustrative examples, the diagnostic methods are used at least at 1, 2, 3, 4,5, 6, 7, 8, 9, 10, 11, 12, 13, 14, 15, 16, 17, 18, 19, 20, 21, 22, 23 or 24 hour intervals.

In yet another aspect, the present invention provides methods for treating, preventing or inhibiting the development of an endotoxemia-related condition in a subject. These methods generally comprise detecting aberrant expression of at least one endotoxemia marker gene in the subject, and administering to the subject an effective amount of an agent that treats or ameliorates the symptoms or reverses or inhibits the development of the endotoxemia-related condition in the subject. Representative examples of such treatments or agents include but are not limited to, antibiotics, steroids, intravenous fluids, vasoactives, palliative support for damaged or distressed organs (e.g. oxygen for respiratory distress, fluids for hypovolemia) and close monitoring of vital organs.

In still another aspect, the present invention provides isolated polynucleotides, referred to herein as "endotoxemia marker polynucleotides," which are generally selected from: (a) a polynucleotide comprising a nucleotide sequence that shares at least 50% (and at least 51% to at least 99% and all integer percentages in between) sequence identity with the sequence set forth in any one of SEQ ID NO: 3, 4, 9, 15, 16, 17, 18, 25, 26, 37, 38, 41, 42, 43, 44, 49, 63, 66, 67, 68, 69, 70, 75, 76, 79, 80, 81, 82, 83, 92, 93, 100, 101, 102, 103, 106, 109, 110, 113, 114, 121, 122, 123, 124, 125, 136, 157, 168, 169, 172, 173, 192, 193, 194, 205, 206, 209, 210, 211, 214, 215, 222, 223, 224, 235, 236, 239, 244, 245, 254, 259, 268, 269, 270, 271, 278, 279, 304, 305, 306, 311, 314, 315 or 320, or a complement thereof; (b) a polynucleotide comprising a portion of the sequence set forth in any one of SEQ ID NO: 3, 4, 9, 15, 16, 17, 18, 25, 26, 37, 38, 41, 42, 43, 44, 49, 63, 66, 67, 68, 69, 70, 75, 76, 79, 80, 81, 82, 83, 92, 93, 100, 101, 102, 103, 106, 109, 110, 113, 114, 121, 122, 123, 124, 125, 136, 157, 168, 169, 172, 173, 192, 193, 194, 205, 206, 209, 210, 211, 214, 215, 222, 223, 224, 235, 236, 239, 244, 245, 254, 259, 268, 269, 270, 271, 278, 279, 304, 305, 306, 311, 314, 315 or 320, or a complement thereof, wherein the portion comprises at least 15 contiguous nucleotides of that sequence or complement; (c) a polynucleotide that hybridizes to the sequence of (a) or (b) or a complement thereof, under at least low, medium or high stringency conditions; and (d) a polynucleotide comprising a portion of any one of SEQ ID NO: 3, 4, 9, 15, 16, 17, 18, 25, 26, 37, 38, 41, 42, 43, 44, 49, 63, 66, 67, 68, 69, 70, 75, 76, 79, 80, 81, 82, 83, 92, 93, 100, 101, 102, 103, 106, 109, 110, 113, 114, 121, 122, 123, 124, 125, 136, 157, 168, 169, 172, 173, 192, 193, 194, 205, 206, 209, 210, 211, 214, 215, 222, 223, 224, 235, 236, 239, 244, 245, 254, 259, 268, 269, 270, 271, 278, 279, 304, 305, 306, 311, 314, 315 or 320, or a complement thereof, wherein the portion comprises at least 15 contiguous nucleotides of that sequence or complement and hybridizes to a sequence of (a), (b) or (c), or a complement thereof, under at least low, medium or high stringency conditions.

In yet another aspect, the present invention provides a nucleic acid construct comprising a polynucleotide as broadly described above in operable connection with a regulatory element, which is operable in a host cell. In certain embodiments, the construct is in the form of a vector, especially an expression vector.

In still another aspect, the present invention provides isolated host cells containing a nucleic acid construct or vector as broadly described above. In certain advantageous embodiments, the host cells are selected from bacterial cells, yeast cells and insect cells.

In still another aspect, the present invention provides probes for interrogating nucleic acid for the presence of a polynucleotide as broadly described above. These probes generally comprise a nucleotide sequence that hybridizes under at least low stringency conditions to a polynucleotide as broadly described above. In some embodiments, the probes consist essentially of a nucleic acid sequence which corresponds or is complementary to at least a portion of a nucleotide sequence encoding the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion is at least 15 nucleotides in length. In other embodiments, the probes comprise a nucleotide sequence which is capable of hybridizing to at least a portion of a nucleotide sequence encoding the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326 under at least low, medium or high stringency conditions, wherein the portion is at least 15 nucleotides in length. In still other embodiment, the probes comprise a nucleotide sequence that is capable of hybridizing to at least a portion of any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325 under at least low, medium or high stringency conditions, wherein the portion is at least 15 nucleotides in length. Representative probes for detecting the endotoxemia marker polynucleotides according to the resent invention are set forth in SEQ ID NO: 326-2315 (see Table 2).

In a related aspect, the invention provides a solid or semi-solid support comprising at least one nucleic acid probe as broadly described above immobilized thereon. In some embodiments, the solid or semi-solid support comprises a spatial array of nucleic acid probes immobilized thereon.

In a further aspect, the present invention provides isolated polypeptides, referred to herein as "endotoxemia marker polypeptides," which are generally selected from: (i) a polypeptide comprising an amino acid sequence that shares at least 50% (and at least 51% to at least 99% and all integer percentages in between) sequence similarity with a polypeptide expression product of an endotoxemia marker gene as broadly described above, for example, especially an endotoxemia marker gene that comprises a nucleotide sequence that shares at least 50% (and at least 51 % to at least 99% and all integer percentages in between) sequence identity with the sequence set forth in any one of SEQ ID NO: 3, 4, 9, 15, 16, 17, 18, 25, 26, 37, 38, 41, 42, 43, 44, 49, 63, 66, 67, 68, 69, 70, 75, 76, 79, 80, 81, 82, 83, 92, 93, 100, 101, 102, 103, 106, 109, 110, 113, 114, 121, 122, 123, 124, 125, 136, 157, 168, 169, 172, 173, 192, 193, 194, 205, 206, 209, 210, 211, 214, 215, 222, 223, 224, 235, 236, 239, 244, 245, 254, 259, 268, 269, 270, 271, 278, 279, 304, 305, 306, 311, 314, 315 or 320; (ii) a portion of the polypeptide according to (i) wherein the portion comprises at least 5 contiguous amino acid residues of that polypeptide; (iii) a polypeptide comprising an amino acid sequence that shares at least 30% similarity (and at least 31% to at least 99% and all integer percentages in between) with at least 15 contiguous amino acid residues of the polypeptide according to (i); and (iv) a polypeptide comprising an amino acid sequence that is immuno-interactive with an antigen-binding molecule that is immuno-interactive with a sequence of (i), (ii) or (iii).

Still a further aspect of the present invention provides an antigen-binding molecule that is immuno-interactive with an endotoxemia marker polypeptide as broadly described above.

In a related aspect, the invention provides a solid or semi-solid support comprising at least one antigen-binding molecule as broadly described above immobilized thereon. In some embodiments, the solid or semi-solid support comprises a spatial array of antigen-binding molecules immobilized thereon.

Still another aspect of the invention provides the use of one or more endotoxemia marker polynucleotides as broadly described above, or the use of one or more probes as broadly described above, or the use of one or more endotoxemia marker polypeptides as broadly described above, or the use of one or more antigen-binding molecules as broadly described above, in the manufacture of a kit for diagnosing the presence of an endotoxemia-related condition in a subject.

In still other aspects, the invention is directed to the use of the diagnostic methods as broadly described above, or one or more endotoxemia marker polynucleotides as broadly described above, or the use of one or more probes as broadly described above, or the use of one or more endotoxemia marker polypeptides as broadly described above, or the use of one or more antigen-binding molecules as broadly described above, for diagnosing an endotoxemia-related condition animals (vertebrates), mammals, non-human mammals, animals, such as horses involved in load bearing or athletic activities (e.g., races) and pets (e.g., dogs and cats).

The aspects of the invention are directed to animals (vertebrates), mammals, non-human mammals, animals, such as horses involved in load bearing or athletic activities (e.g., races) and pets (e.g., dogs and cats).

## BRIEF DESCRIPTION OF THE DRAWINGS

Figure 1 is a graphical representation of a receiver operating curve (ROC) for comparison of gene expression 24 hours post-induction. The ROC curve generated from these data demonstrated that 24 hours post-induction, was well separated from 0 hours. The sensitivity and selectivity using two principal components are 1.00 and 1.00 respectively.

Figure 2 is a graphical representation of ROC for comparison of gene expression 72 hours post-induction. The ROC curve generated from these data demonstrated that 72 hours post-induction, was well separated from 0 hours. The sensitivity and selectivity using two principal components are 0.667 and 1.00 respectively. Using four principal components this can be improved to 0.883 and 1 respectively (not shown).

## DETAILED DESCRIPTION OF THE INVENTION

### *1. Definition*

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e., to at least one) of the grammatical object of the article. By way of example, "an element" means one element or more than one element.

The term "aberrant expression," as used herein to describe the expression of an endotoxemia marker gene, refers to the overexpression or underexpression of an endotoxemia marker gene relative to the level of expression of the endotoxemia marker gene or variant thereof in cells obtained from a healthy subject or from a subject lacking endotoxemia, and/or to a higher or lower level of an endotoxemia marker gene product (e.g., transcript or polypeptide) in a tissue sample or body fluid obtained from a healthy subject or from a subject lacking endotoxemia. In particular, an endotoxemia marker gene is aberrantly expressed if the level of expression of the endotoxemia marker gene is higher by at least about 10%, 20%, 30%, 40%, 50%, 60%, 70%, 80% or 90%, or even an at least about 100%, 200%, 300%, 400%, 500%, 600%, 700%, 800%, 900% or 1000%, or lower by at least about 10%, 20%, 30% 40%, 50%, 60%, 70%, 80%, 90%, 92%, 94%, 96%, 97%, 98% or 99%, or even an at least about 99.5%, 99.9%, 99.95%, 99.99%, 99.995% or 99.999% than the level of expression of the endotoxemia marker gene by cells obtained from a healthy subject or from a subject without endotoxemia, and/or relative to the level of expression of the endotoxemia marker gene in a tissue sample or body fluid obtained from a healthy subject or from a subject without endotoxemia.

By "about" is meant a quantity, level, value, number, frequency, percentage, dimension, size, amount, weight or length that varies by as much as 30, 25, 20, 25, 10, 9, 8, 7, 6, 5, 4, 3, 2 or 1 % to a reference quantity, level, value, number, frequency, percentage, dimension, size, amount, weight or length.

The term "amplicon" refers to a target sequence for amplification, and/or the amplification products of a target sequence for amplification. In certain other embodiments an "amplicon" may include the sequence of probes or primers used in amplification.

By "antigen-binding molecule" is meant a molecule that has binding affinity for a target antigen. It will be understood that this term extends to immunoglobulins, immunoglobulin fragments and non-immunoglobulin derived protein frameworks that exhibit antigen-binding activity.

As used herein, the term "binds specifically," "specifically immuno-interactive" and the like when referring to an antigen-binding molecule refers to a binding reaction which is determinative of the presence of an antigen in the presence of a heterogeneous population of proteins and other biologics. Thus, under designated immunoassay conditions, the specified antigen-binding molecules bind to a particular antigen and do not bind in a significant amount to other proteins or antigens present in the sample. Specific binding to an antigen under such conditions may require an antigen-binding molecule that is selected for its specificity for a particular antigen. For example, antigen-binding molecules can be raised to a selected protein antigen, which bind to that antigen but not to other proteins present in a sample. A variety of immunoassay formats may be used to select antigen-binding molecules specifically immuno-interactive with a particular protein. For example, solid-phase ELISA immunoassays are routinely used to select monoclonal antibodies specifically immuno-interactive with a protein. See Harlow and Lane (1988) Antibodies, A Laboratory Manual, Cold Spring Harbor Publications, New York, for a description of immunoassay formats and conditions that can be used to determine specific immunoreactivity.

By "biologically active portion" is meant a portion of a full-length parent peptide or polypeptide which portion retains an activity of the parent molecule. As used herein, the term "biologically active portion" includes deletion mutants and peptides, for example of at least about 8, 9, 10, 11, 12, 13, 14, 15, 16, 17, 18, 19, 20, 21, 22, 23, 24, 25, 30, 40, 50, 60, 70, 80, 90, 100, 120, 150, 300, 400, 500, 600, 700, 800, 900, 1000 contiguous amino acids, which comprise an activity of a parent molecule. Portions of this type may be obtained through the application of standard recombinant nucleic acid techniques or synthesized using conventional liquid or solid phase synthesis techniques. For example, reference may be made to solution synthesis or solid phase synthesis as described, for example, in Chapter 9 entitled "Peptide Synthesis" by Atherton and Shephard which is included in a publication entitled "Synthetic Vaccines" edited by Nicholson and published by Blackwell Scientific Publications. Alternatively, peptides can be produced by digestion of a peptide or polypeptide of the invention with proteinases such as endoLys-C, endoArg-C, endoGlu-C and staphylococcus V8-protease. The digested fragments can be purified by, for example, high performance liquid chromatographic (HPLC) techniques. Recombinant nucleic acid techniques can also be used to produce such portions.

The term "biological sample" as used herein refers to a sample that may be extracted, untreated, treated, diluted or concentrated from an animal. The biological sample may include a biological fluid such as whole blood, serum, plasma, saliva, urine, sweat, ascitic fluid, peritoneal fluid, synovial fluid, amniotic fluid, cerebrospinal fluid, tissue biopsy, and the like. In certain embodiments, the biological sample is blood, especially peripheral blood.

As used herein, the term "cis-acting sequence," "cis-acting element" or "cis-regulatory region" or "regulatory region" or similar term shall be taken to mean any sequence of nucleotides, which when positioned appropriately relative to an expressible genetic sequence, is capable of regulating, at least in part, the expression of the genetic sequence. Those skilled in the art will be aware that a cis-regulatory region may be capable of activating, silencing, enhancing, repressing or otherwise altering the level of expression and/or cell-type-specificity and/or developmental specificity of a gene sequence at the transcriptional or post-transcriptional level. In certain embodiments of the present invention, the cis-acting sequence is an activator sequence that enhances or stimulates the expression of an expressible genetic sequence.

Throughout this specification, unless the context requires otherwise, the words "comprise," "comprises" and "comprising" will be understood to imply the inclusion of a stated step or element or group of steps or elements but not the exclusion of any other step or element or group of steps or elements.

By "corresponds to" or "corresponding to" is meant a polynucleotide (a) having a nucleotide sequence that is substantially identical or complementary to all or a portion of a reference polynucleotide sequence or (b) encoding an amino acid sequence identical to an amino acid sequence in a peptide or protein. This phrase also includes within its scope a peptide or polypeptide having an amino acid sequence that is substantially identical to a sequence of amino acids in a reference peptide or protein.

By "effective amount", in the context of treating or preventing a condition is meant the administration of that amount of active to an individual in need of such treatment or prophylaxis, either in a single dose or as part of a series, that is effective for the prevention of incurring a symptom, holding in check such symptoms, and/or treating existing symptoms, of that condition. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

The terms "expression" or "gene expression" refer to either production of RNA message or translation of RNA message into proteins or polypeptides. Detection of either types of gene expression in use of any of the methods described herein are part of the invention.

By "expression vector" is meant any autonomous genetic element capable of directing the transcription of a polynucleotide contained within the vector and suitably the synthesis of a peptide or polypeptide encoded by the polynucleotide. Such expression vectors are known to practitioners in the art.

As used herein, the term "functional activity" generally refers to the ability of a molecule (e.g., a transcript or polypeptide) to perform its designated function including a biological, enzymatic, or therapeutic function. In certain embodiments, the functional activity of a molecule corresponds to its specific activity as determined by any suitable assay known in the art.

The term "gene" as used herein refers to any and all discrete coding regions of the cell's genome, as well as associated non-coding and regulatory regions. The gene is also intended to mean the open reading frame encoding specific polypeptides, introns, and adjacent 5' and 3' non-coding nucleotide sequences involved in the regulation of expression. In this regard, the gene may further comprise control signals such as promoters, enhancers, termination and/or polyadenylation signals that are naturally associated with a given gene, or heterologous control signals. The DNA sequences may be cDNA or genomic DNA or a fragment thereof. The gene may be introduced into an appropriate vector for extrachromosomal maintenance or for integration into the host.

By "high density polynucleotide arrays" and the like is meant those arrays that contain at least 400 different features per $cm^2$.

The phrase "high discrimination hybridization conditions" refers to hybridization conditions in which single base mismatch may be determined.

By "housekeeping gene" is meant a gene that is expressed in virtually all cells since it is fundamental to the any cell's functions (e.g., essential proteins and RNA molecules).

"Hybridization" is used herein to denote the pairing of complementary nucleotide sequences to produce a DNA-DNA hybrid or a DNA-RNA hybrid. Complementary base sequences are those sequences that are related by the base-pairing rules. In DNA, A pairs with T and C pairs with G. In RNA, U pairs with A and C pairs with G. In this regard, the terms "match" and "mismatch" as used herein refer to the hybridization potential of paired nucleotides in complementary nucleic acid strands. Matched nucleotides hybridize efficiently, such as the classical A-T and G-C base pair mentioned above. Mismatches are other combinations of nucleotides that do not hybridize efficiently.

The phrase "hybridizing specifically to" and the like refer to the binding, duplexing, or hybridizing of a molecule only to a particular nucleotide sequence under stringent conditions when that sequence is present in a complex mixture (e.g., total cellular) DNA or RNA.

Reference herein to "immuno-interactive" includes reference to any interaction, reaction, or other form of association between molecules and in particular where one of the molecules is, or mimics, a component of the immune system.

By "isolated" is meant material that is substantially or essentially free from components that normally accompany it in its native state. For example, an "isolated polynucleotide", as used herein, refers to a polynucleotide, which has been purified from the sequences which flank it in a naturally-occurring state, e.g., a DNA fragment which has been removed from the sequences that are normally adjacent to the fragment. Alternatively, an "isolated peptide" or an "isolated polypeptide" and the like, as used herein, refer to in vitro isolation and/or purification of a peptide or polypeptide molecule from its natural cellular environment, and from association with other components of the cell, i.e., it is not associated with in vivo substances.

By "marker gene" is meant a gene that imparts a distinct phenotype to cells expressing the marker gene and thus allows such transformed cells to be distinguished from cells that do not have the marker. A selectable marker gene confers a trait for which one can 'select' based on resistance to a selective agent (e.g., a herbicide, antibiotic, radiation, heat, or other treatment damaging to untransformed cells). A screenable marker gene (or reporter gene) confers a trait that one can identify through observation or testing, i.e., by 'screening' (e.g. β-glucuronidase, luciferase, or other enzyme activity not present in untransformed cells).

As used herein, a "naturally-occurring" nucleic acid molecule refers to a RNA or DNA molecule having a nucleotide sequence that occurs in nature. For example a naturally-occurring nucleic acid molecule can encode a protein that occurs in nature.

By "obtained from" is meant that a sample such as, for example, a nucleic acid extract or polypeptide extract is isolated from, or derived from, a particular source. For instance, the extract may be isolated directly from a biological fluid or tissue of the subject.

The term "oligonucleotide" as used herein refers to a polymer composed of a multiplicity of nucleotide residues (deoxyribonucleotides or ribonucleotides, or related structural variants or synthetic analogues thereof, including nucleotides with modified or substituted sugar groups and the like) linked via phosphodiester bonds (or related structural variants or synthetic analogues thereof). Thus, while the term "oligonucleotide" typically refers to a nucleotide polymer in which the nucleotide residues and linkages between them are naturally-occurring, it will be understood that the term also includes within its scope various analogues including, but not restricted to, peptide nucleic acids (PNAs), phosphorothioate, phosphorodithioate, phophoroselenoate, phosphorodiselenoate, phosphoroanilothioate, phosphoraniladate, phosphoroamidate, methyl phosphonates, 2-0-methyl ribonucleic acids, and the like. The exact size of the molecule can vary depending on the particular application. Oligonucleotides are a polynucleotide subset with 200 bases or fewer in length. Preferably, oligonucleotides are 10 to 60 bases in length and most preferably 12, 13, 14, 15, 16, 17, 18, 19, or 20 to 40 bases in length. Oligonucleotides are usually single stranded, e.g., for probes; although oligonucleotides may be double stranded, e.g., for use in the construction of a variant nucleic acid sequence. Oligonucleotides of the invention can be either sense or antisense oligonucleotides.

The term "oligonucleotide array" refers to a substrate having oligonucleotide probes with different known sequences deposited at discrete known locations associated with its surface. For example, the substrate can be in the form of a two dimensional substrate as described in U.S. Patent No. 5,424,186. Such substrate may be used to synthesize two-dimensional spatially addressed oligonucleotide (matrix) arrays. Alternatively, the substrate may be characterized in that it forms a tubular array in which a two dimensional planar sheet is rolled into a three-dimensional tubular configuration. The substrate may also be in the form of a microsphere or bead connected to the surface of an optic fiber as, for example, disclosed by Chee *et al.* in WO 00/39587. Oligonucleotide arrays have at least two different features and a density of at least 400 features per $cm^2$. In certain embodiments, the arrays can have a density of about 500, at least one thousand, at least 10 thousand, at least 100 thousand, at least one million or at least 10 million features per $cm^2$. For example, the substrate may be silicon or glass and can have the thickness of a glass microscope slide or a glass cover slip, or may be composed of other synthetic polymers. Substrates that are transparent to light are useful when the method of performing an assay on the substrate involves optical detection. The term also refers to a probe array and the substrate to which it is attached that form part of a wafer.

The term "operably connected" or "operably linked" as used herein means placing a structural gene under the regulatory control of a promoter, which then controls the transcription and optionally translation of the gene. In the construction of heterologous promoter/structural gene combinations, it is generally preferred to position the genetic sequence or promoter at a distance from the gene transcription start site that is approximately the same as the distance between that genetic sequence or promoter and the gene it controls in its natural setting; i.e. the gene from which the genetic sequence or promoter is derived. As is known in the art, some variation in this distance can be accommodated without loss of function. Similarly, the preferred positioning of a regulatory sequence element with respect to a heterologous gene to be placed under its control is defined by the positioning of the element in its natural setting; i.e., the genes from which it is derived.

The term "pathogen" is used herein in its broadest sense to refer to an organism or an infectious agent whose infection of cells of viable animal tissue elicits a disease response.

The term "polynucleotide" or "nucleic acid" as used herein designates mRNA, RNA, cRNA, cDNA or DNA. The term typically refers to polymeric form of nucleotides of at least 10 bases in length, either ribonucleotides or deoxynucleotides or a modified form of either type of nucleotide. The term includes single and double stranded forms of DNA.

The terms "polynucleotide variant" and "variant" refer to polynucleotides displaying substantial sequence identity with a reference polynucleotide sequence or polynucleotides that hybridize with a reference sequence under stringent conditions that are defined hereinafter. These terms also encompass polynucleotides in which one or more nucleotides have been added or deleted, or replaced with different nucleotides. In this regard, it is well understood in the art that certain alterations inclusive of mutations, additions, deletions and substitutions can be made to a reference polynucleotide whereby the altered polynucleotide retains a biological function or activity of the reference polynucleotide. The terms "polynucleotide variant" and "variant" also include naturally-occurring allelic variants.

"Polypeptide", "peptide" and "protein" are used interchangeably herein to refer to a polymer of amino acid residues and to variants and synthetic analogues of the same. Thus, these terms apply to amino acid polymers in which one or more amino acid residues is a synthetic non-naturally-occurring amino acid, such as a chemical analogue of a corresponding naturally-occurring amino acid, as well as to naturally-occurring amino acid polymers.

The term "polypeptide variant" refers to polypeptides which are distinguished from a reference polypeptide by the addition, deletion or substitution of at least one amino acid residue. In certain embodiments, one or more amino acid residues of a reference polypeptide are replaced by different amino acids. It is well understood in the art that some amino acids may be changed to others with broadly similar properties without changing the nature of the activity of the polypeptide (conservative substitutions) as described hereinafter.

By "primer" is meant an oligonucleotide which, when paired with a strand of DNA, is capable of initiating the synthesis of a primer extension product in the presence of a suitable polymerizing agent. The primer is preferably single-stranded for maximum efficiency in amplification but can alternatively be double-stranded. A primer must be sufficiently long to prime the synthesis of extension products in the presence of the polymerization agent. The length of the primer depends on many factors, including application, temperature to be employed, template reaction conditions, other reagents, and source of primers. For example, depending on the complexity of the target sequence, the primer may be at least about 5, 6, 7, 8, 9, 10, 11, 12, 13, 14, 15, 16, 17, 18, 19, 20, 21, 22, 23, 24, 25, 26, 27, 28, 29, 30, 35, 40, 50, 75, 100, 150, 200, 300, 400, 500, to one base shorter in length than the template sequence at the 3' end of the primer to allow extension of a nucleic acid chain, though the 5' end of the primer may extend in length beyond the 3' end of the template sequence. In certain embodiments, primers can be large polynucleotides, such as from about 35 nucleotides to several kilobases or more. Primers can be selected to be "substantially complementary" to the sequence on the template to which it is designed to hybridize and serve as a site for the initiation of synthesis. By "substantially complementary", it is meant that the primer is sufficiently complementary to hybridize with a target polynucleotide. Desirably, the primer contains no mismatches with the template to which it is designed to hybridize but this is not essential. For example, non-complementary nucleotide residues can be attached to the 5' end of the primer, with the remainder of the primer sequence being complementary to the template. Alternatively, non-complementary nucleotide residues or a stretch of non-complementary nucleotide residues can be interspersed into a primer, provided that the primer sequence has sufficient complementarity with the sequence of the template to hybridize therewith and thereby form a template for synthesis of the extension product of the primer.

"Probe" refers to a molecule that binds to a specific sequence or subsequence or other moiety of another molecule. Unless otherwise indicated, the term "probe" typically refers to a polynucleotide probe that binds to another polynucleotide, often called the "target polynucleotide", through complementary base pairing. Probes can bind target polynucleotides lacking complete sequence complementarity with the probe, depending on the stringency of the hybridization conditions. Probes can be labeled directly or indirectly and include primers within their scope.

The term "recombinant polynucleotide" as used herein refers to a polynucleotide formed in vitro by the manipulation of nucleic acid into a form not normally found in nature. For example, the recombinant polynucleotide may be in the form of an expression vector. Generally, such expression vectors include transcriptional and translational regulatory nucleic acid operably linked to the nucleotide sequence.

By "recombinant polypeptide" is meant a polypeptide made using recombinant techniques, i.e., through the expression of a recombinant or synthetic polynucleotide.

By "regulatory element" or "regulatory sequence" is meant nucleic acid sequences (e.g., DNA) necessary for expression of an operably linked coding sequence in a particular host cell. The regulatory sequences that are suitable for prokaryotic cells for example, include a promoter, and optionally a cis-acting sequence such as an operator sequence and a ribosome binding site. Control sequences that are suitable for eukaryotic cells include promoters, polyadenylation signals, transcriptional enhancers, translational enhancers, leader or trailing sequences that modulate mRNA stability, as well as targeting sequences that target a product encoded by a transcribed polynucleotide to an intracellular compartment within a cell or to the extracellular environment.

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, California, USA) using standard defaults as used in the reference manual accompanying the software.

"Similarity" refers to the percentage number of amino acids that are identical or constitute conservative substitutions as defined in Table A *infra.* Similarity may be determined using sequence comparison programs such as GAP (Deveraux et al. 1984, Nucleic Acids Research 12, 387-395). In this way, sequences of a similar or substantially different length to those cited herein might be compared by insertion of gaps into the alignment, such gaps being determined, for example, by the comparison algorithm used by GAP.

Terms used to describe sequence relationships between two or more polynucleotides or polypeptides include "reference sequence," "comparison window," "sequence identity," "percentage of sequence identity" and "substantial identity". A "reference sequence" is at least 12 but frequently 15 to 18 and often at least 25 monomer units, inclusive of nucleotides and amino acid residues, in length. Because two polynucleotides may each comprise (1) a sequence (i.e., only a portion of the complete polynucleotide sequence) that is similar between the two polynucleotides, and (2) a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window" refers to a conceptual segment of at least 6 contiguous positions, usually about 50 to about 100, more usually about 100 to about 150 in which a sequence is compared to a reference sequence of the same number of contiguous positions after the two sequences are optimally aligned. The comparison window may comprise additions or deletions (i.e., gaps) of about 20% or less as compared to the reference sequence (which does not comprise additions or deletions) for optimal alignment of the two sequences.

Optimal alignment of sequences for aligning a comparison window may be conducted by computerized implementations of algorithms (GAP, BESTFIT, FASTA, and TFASTA in the Wisconsin Genetics Software Package Release 7.0, Genetics Computer Group, 575 Science Drive Madison, WI, USA) or by inspection and the best alignment (i.e., resulting in the highest percentage homology over the comparison window) generated by any of the various methods selected. Reference also may be made to the BLAST family of programs as for example disclosed by Altschul et al., 1997, Nucl. Acids Res. 25:3389. A detailed discussion of sequence analysis can be found in Unit 19.3 of Ausubel et al., "Current Protocols in Molecular Biology", John Wiley & Sons Inc, 1994-1998, Chapter 15.

The terms "subject" or "individual" or "patient", used interchangeably herein, refer to any subject, particularly a vertebrate subject, and even more particularly a mammalian subject, for whom therapy or prophylaxis is desired. Suitable vertebrate animals that fall within the scope of the invention include, but are not restricted to, primates, avians, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes). A preferred subject is an equine animal in need of treatment or prophylaxis of endotoxemia. However, it will be understood that the aforementioned terms do not imply that symptoms are present.

The phrase "substantially similar affinities" refers herein to target sequences having similar strengths of detectable hybridization to their complementary or substantially complementary oligonucleotide probes under a chosen set of stringent conditions.

The term "template" as used herein refers to a nucleic acid that is used in the creation of a complementary nucleic acid strand to the "template" strand. The template may be either RNA and/or DNA, and the complementary strand may also be RNA and/or DNA. In certain embodiments, the complementary strand may comprise all or part of the complementary sequence to the "template," and/or may include mutations so that it is not an exact, complementary strand to the "template". Strands that are not exactly complementary to the template strand may hybridize specifically to the template strand in detection assays described here, as well as other assays known in the art, and such complementary strands that can be used in detection assays are part of the invention.

The term "transformation" means alteration of the genotype of an organism, for example a bacterium, yeast, mammal, avian, reptile, fish or plant, by the introduction of a foreign or endogenous nucleic acid.

The term "treat" is meant to include both therapeutic and prophylactic treatment.

By "vector" is meant a polynucleotide molecule, suitably a DNA molecule derived, for example, from a plasmid, bacteriophage, yeast, virus, mammal, avian, reptile or fish into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, i.e., a vector that exists as an extrachromosomal entity, the replication of which is independent of chromosomal replication, e.g., a linear or closed circular plasmid, an extrachromosomal element, a minichromosome, or an artificial chromosome. The vector can contain any means for assuring self-replication. Alternatively, the vector can be one which, when introduced into the host cell, is integrated into the genome and replicated together with the chromosome(s) into which it has been integrated. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. The vector can also include a selection marker such as an antibiotic resistance gene that can be used for selection of suitable transformants. Examples of such resistance genes are known to those of skill in the art.

The terms "wild-type" and "normal" are used interchangeably to refer to the phenotype that is characteristic of most of the members of the species occurring naturally and contrast for example with the phenotype of a mutant.

## *2. Abbreviations*

The following abbreviations are used throughout the application:

nt = nucleotide
nts = nucleotides
aa = amino acid(s)
kb = kilobase(s) or kilobase pair(s)
kDa = kilodalton(s)
d = day
h = hour
s = seconds

### *3. Markers of endotoxemia and uses therefor*

The present invention concerns the early detection, diagnosis, or prognosis of endotoxemia or its sequelae (also referred to herein as "endotoxemia-related conditions"). Markers of endotoxemia, in the form of RNA molecules of specified sequences, or polypeptides expressed from these RNA molecules in cells, especially in blood cells, and more especially in peripheral blood cells, of subjects with or susceptible to endotoxemia, are disclosed. These markers are indicators of endotoxemia-related conditions and, when differentially expressed as compared to their expression in normal subjects or in subjects lacking endotoxemia-related conditions, are diagnostic for the presence of those conditions in tested subjects. Such markers provide considerable advantages over the prior art in this field. In certain advantageous embodiments where leukocytes (e.g., peripheral blood cells) are used for the analysis, it is possible to diagnose active endotoxemia-related conditions before serum antibodies to endotoxin, or endotoxaemia-causing agent are detected.

It will be apparent that the nucleic acid sequences disclosed herein will find utility in a variety of applications in detection, diagnosis, prognosis and treatment of endotoxemia-related conditions. Examples of such applications within the scope of the present disclosure comprise amplification of endotoxemia markers using specific primers, detection of endotoxemia markers by hybridization with oligonucleotide probes, incorporation of isolated nucleic acids into vectors, expression of vector-incorporated nucleic acids as RNA and protein, and development of immunological reagents corresponding to marker encoded products.

The identified endotoxemia markers may in turn be used to design specific oligonucleotide probes and primers. Such probes and primers may be of any length that would specifically hybridize to the identified marker gene sequences and may be at least about 10, 11, 12, 13, 14, 15, 16, 17, 18, 19, 20, 21, 22, 23, 24, 25, 26, 27, 28, 29, 30, 35, 40, 50, 75, 100, 150, 200, 300, 400, 500 nucleotides in length and in the case of probes, up to the full length of the sequences of the marker genes identified herein. Probes may also include additional sequence at their 5' and/or 3' ends so that they extent beyond the target sequence with which they hybridize.

When used in combination with nucleic acid amplification procedures, these probes and primers enable the rapid analysis of biological samples (e.g., peripheral blood samples) for detecting marker genes or for detecting or quantifying marker gene transcripts. Such procedures include any method or technique known in the art or described herein for duplicating or increasing the number of copies or amount of a target nucleic acid or its complement.

The identified markers may also be used to identify and isolate full-length gene sequences, including regulatory elements for gene expression, from genomic DNA libraries, which are suitably but not exclusively of equine origin. The cDNA sequences identified in the present disclosure may be used as hybridization probes to screen genomic DNA libraries by conventional techniques. Once partial genomic clones have been identified, full-length genes may be isolated by "chromosomal walking" (also called "overlap hybridization") using, for example, the method disclosed by Chinault & Carbon (1979, Gene 5: 111-126). Once a partial genomic clone has been isolated using a cDNA hybridization probe, non-repetitive segments at or near the ends of the partial genomic clone may be used as hybridization probes in further genomic library screening, ultimately allowing isolation of entire gene sequences for the endotoxemia markers of interest. It will be recognized that full-length genes may be obtained using the full-length or partial cDNA sequences or short expressed sequence tags (ESTs) described in this disclosure using standard techniques as disclosed for example by Sambrook, et al. (MOLECULAR CLONING. A LABORATORY MANUAL (Cold Spring Harbor Press, 1989) and Ausubel et al., (CURRENT PROTOCOLS IN MOLECULAR BIOLOGY, John Wiley & Sons, Inc. 1994). In addition, the disclosed sequences may be used to identify and isolate full-length cDNA sequences using standard techniques as disclosed, for example, in the above-referenced texts. Sequences identified and isolated by such means may be useful in the detection of the endotoxemia marker genes using the detection methods described herein, and are part of the invention.

One of ordinary skill in the art could select segments from the identified marker genes for use in the different detection, diagnostic, or prognostic methods, vector constructs, antigen-binding molecule production, kit, and/or any of the embodiments described herein as part of the present invention. Marker gene sequences that are desirable for use in the invention are those set fort in SEQ ID NO: 1, 3, 5, 7, 8, 10, 12, 14, 16, 17, 18, 20, 22, 24, 26, 27, 28, 30, 32, 34, 35, 36, 37, 38, 39, 40, 41, 42, 43, 44, 45, 46, 47, 48, 49, 50, 52, 54, 55, 56 or 57 (see Table 1).

### *4. Nucleic acid molecules of the invention*

As described in the Examples and in Tables 1, the present disclosure provides 179 markers of endotoxemia, identified by GeneChip® analysis of blood obtained from normal horses and from horses with clinical evidence of an endotoxemia-related condition. Of the 179 markers identified, 121 comprise coding regions sequences (see the markers relating to SEQ ID NO: 1, 5, 6, 7, 11, 13, 19, 21, 23, 27, 29, 31, 33, 35, 39, 45, 47, 50, 52, 54, 56, 58, 60, 61, 64, 71, 73, 77, 78, 84, 86, 88, 90, 94, 96, 98, 102, 103, 104, 107, 111, 115, 117, 119, 126, 128, 130, 132, 134, 137, 139, 141, 143, 145, 147, 149, 151, 153, 155, 158, 160, 162, 164, 166, 170, 174, 176, 178, 180, 182, 184, 186, 188, 190, 195, 197, 199, 201, 203, 207, 212, 216, 218, 220, 225, 227, 229, 231, 233, 237, 240, 242, 246, 248, 250, 252, 255, 257, 260, 262, 264, 266, 272, 274, 276, 280, 282, 284, 286, 288, 290, 292, 294, 296, 298, 300, 302, 307, 309, 312, 316, 318, 321, 323 or 325) and 58 comprise 5' and/or 3' untranslated sequences only (see the markers relating to SEQ ID NO: 3, 4, 9, 15, 16, 17, 18, 25, 26, 37, 38, 41, 42, 43, 44, 49, 63, 66, 67, 68, 69, 70, 75, 76, 79, 80, 81, 82, 83, 92, 93, 100, 101, 102, 103, 106, 109, 110, 113, 114, 121, 122, 123, 124, 125, 136, 157, 168, 169, 172, 173, 192, 193, 194, 205, 206, 209, 210, 211, 214, 215, 222, 223, 224, 235, 236, 239, 244, 245, 254, 259, 268, 269, 270, 271, 278, 279, 304, 305, 306, 311, 314, 315 or 320). These sequences, which are presented in Table 1, are diagnostic for the presence, stage or degree of an endotoxemia-related condition (also referred to herein as "endotoxemia marker polynucleotides"). Sequence analysis has revealed that the endotoxaemia marker genes can be classified into subgroups. For example, several endotoxaemia marker genes encode membrane associated polypeptides involved in the immune response (e.g., SEQ ID NO. 59, 219, 230, 253, 285, and 295), whereas others encode cytoplasm associated polypeptides (e.g., SEQ ID NO: 217, 241, 247, 265, 267, 287, and 291), while still others encode extracellular polypeptides (e.g., SEQ ID NO: 30, 85, 108, 127, 140, and 226), whereas still others encode nuclear polypeptides (e.g., 8, 12, 20, 28, 40, 55, 281, 283 and 313) and still others encode cytoskeleton molecules (e.g., SEQ ID NO: 105 and 213).

In accordance with the present invention, the sequences of isolated nucleic acids disclosed herein find utility *inter alia* as hybridization probes or amplification primers. These nucleic acids may be used, for example, in diagnostic evaluation of biological samples or employed to clone full-length cDNAs or genomic clones corresponding thereto. In certain embodiments, these probes and primers represent oligonucleotides, which are of sufficient length to provide specific hybridization to a RNA or DNA sample extracted from the biological sample. The sequences typically will be about 10-20 nucleotides, but may be longer. Longer sequences, e.g., of about 30, 40, 50, 100, 500 and even up to full-length, are desirable for certain embodiments.

Nucleic acid molecules having contiguous stretches of about 10, 15, 17, 20, 30, 40, 50, 60, 75 or 100 or 500 nucleotides of a sequence set forth in any one of SEQ ID NO: 1, 3, 5, 7, 8, 10, 12, 14, 16, 17, 18, 20, 22, 24, 26, 27, 28, 30, 32, 34, 35, 36, 37, 38, 39, 40, 41, 42, 43, 44, 45, 46, 47, 48, 49, 50, 52, 54, 55, 56 or 57 are contemplated. Molecules that are complementary to the above mentioned sequences and that bind to these sequences under high stringency conditions are also contemplated. These probes are useful in a variety of hybridization embodiments, such as Southern and northern blotting. In some cases, it is contemplated that probes may be used that hybridize to multiple target sequences without compromising their ability to effectively diagnose an endotoxemia-related condition. In general, it is contemplated that the hybridization probes described herein are useful both as reagents in solution hybridization, as in PCR, for detection of expression of corresponding genes, as well as in embodiments employing a solid phase.

Various probes and primers may be designed around the disclosed nucleotide sequences. For example, in certain embodiments, the sequences used to design probes and primers may include repetitive stretches of adenine nucleotides (poly-A tails) normally attached at the ends of the RNA for the identified marker genes. In other embodiments, probes and primers may be specifically designed to not include these or other segments from the identified marker genes, as one of ordinary skilled in the art may deem certain segments more suitable for use in the detection methods disclosed. In any event, the choice of primer or probe sequences for a selected application is within the realm of the ordinary skilled practitioner. Illustrative probe sequences for detection of endotoxemia marker genes are presented in Tables 2.

Primers may be provided in double-stranded or single-stranded form, although the single-stranded form is desirable. Probes, while perhaps capable of priming, are designed to bind to a target DNA or RNA and need not be used in an amplification process. In certain embodiments, the probes or primers are labeled with radioactive species $^{32}P$, $^{14}C$, $^{35}S$, $^{3}H$, or other label), with a fluorophore (e.g., rhodamine, fluorescein) or with a chemillumiscent label (e.g., luciferase).

The present invention provides substantially full-length cDNA sequences as well as EST and partial cDNA sequences that are useful as markers of endotoxemia-related condition. It will be understood, however, that the present disclosure is not limited to these disclosed sequences and is intended particularly to encompass at least isolated nucleic acids that are hybridizable to nucleic acids comprising the disclosed sequences or that are variants of these nucleic acids. For example, a nucleic acid of partial sequence may be used to identify a structurally-related gene or the full-length genomic or cDNA clone from which it is derived. Methods for generating cDNA and genomic libraries which may be used as a target for the above-described probes are known in the art (see, for example, Sambrook *et al.,* 1989, *supra* and Ausubel *et al.*, 1994, *supra*). All such nucleic acids as well as the specific nucleic acid molecules disclosed herein are collectively referred to as "endotoxemia marker polynucleotides." Additionally, the present invention includes within its scope isolated or purified expression products of endotoxemia marker polynucleotides (i.e., RNA transcripts and polypeptides).

Accordingly, the present invention encompasses isolated or substantially purified nucleic acid or protein compositions. An "isolated" or "purified" nucleic acid molecule or protein, or biologically active portion thereof, is substantially or essentially free from components that normally accompany or interact with the nucleic acid molecule or protein as found in its naturally occurring environment. Thus, an isolated or purified polynucleotide or polypeptide is substantially free of other cellular material, or culture medium when produced by recombinant techniques, or substantially free of chemical precursors or other chemicals when chemically synthesized. Suitably, an "isolated" polynucleotide is free of sequences (especially protein encoding sequences) that naturally flank the polynucleotide (i.e., sequences located at the 5' and 3' ends of the polynucleotide) in the genomic DNA of the organism from which the polynucleotide was derived. For example, in various embodiments, an isolated endotoxaemia marker polynucleotide can contain less than about 5 kb, 4 kb, 3 kb, 2 kb, 1 kb, 0.5 kb, or 0.1 kb of nucleotide sequences that naturally flank the polynucleotide in genomic DNA of the cell from which the polynucleotide was derived. A polypeptide that is substantially free of cellular material includes preparations of protein having less than about 30%, 20%, 10%, 5%, (by dry weight) of contaminating protein. When the protein of the invention or biologically active portion thereof is recombinantly produced, culture medium suitably represents less than about 30%, 20%, 10%, or 5% (by dry weight) of chemical precursors or non-protein-of-interest chemicals.

The present invention also encompasses portions of the full-length or substantially full-length nucleotide sequences of the endotoxemia marker genes or their transcripts or DNA copies of these transcripts. Portions of an endotoxemia marker nucleotide sequence may encode polypeptide portions or segments that retain the biological activity of the native polypeptide. Alternatively, portions of an endotoxemia marker nucleotide sequence that are useful as hybridization probes generally do not encode amino acid sequences retaining such biological activity. Thus, portions of an endotoxemia marker nucleotide sequence may range from at least about 15, 16, 17, 18, 19, 20, 21, 22, 23, 24, 25, 26, 27, 28, 29, 30, 40, 50, 60, 80, 90, 100 nucleotides, or almost up to the full-length nucleotide sequence encoding the endotoxemia marker polypeptides of the invention.

A portion of an endotoxemia marker nucleotide sequence that encodes a biologically active portion of an endotoxemia marker polypeptide of the invention may encode at least about 5, 6, 7, 8, 9, 10, 11, 12, 13, 14, 15, 16, 17, 18, 19, 20, 21, 22, 23, 24, 25, 30, 40, 50, 60, 70, 80, 90, 100, 120, 150, 300, 400, 500, 600, 700, 800, 900 or 1000, or even at least about 2000, 3000, 4000 or 5000 contiguous amino acid residues, or almost up to the total number of amino acids present in a full-length endotoxemia marker polypeptide. Portions of an endotoxemia marker nucleotide sequence that are useful as hybridization probes or PCR primers generally need not encode a biologically active portion of an endotoxemia marker polypeptide.

Thus, a portion of an endotoxemia marker nucleotide sequence may encode a biologically active portion of an endotoxemia marker polypeptide, or it may be a fragment that can be used as a hybridization probe or PCR primer using standard methods known in the art. A biologically active portion of an endotoxemia marker polypeptide can be prepared by isolating a portion of one of the endotoxemia marker nucleotide sequences of the invention, expressing the encoded portion of the endotoxemia marker polypeptide (e.g., by recombinant expression *in vitro*), and assessing the activity of the encoded portion of the endotoxemia marker polypeptide. Nucleic acid molecules that are portions of an endotoxemia marker nucleotide sequence comprise at least about 15, 16, 17, 18, 19, 20, 25, 30, 50, 75, 100, 150, 200, 250, 300, 350, 400, 450, 500, 550, 600, or 650 nucleotides, or almost up to the number of nucleotides present in a full-length endotoxemia marker nucleotide sequence.

The invention also contemplates variants of the endotoxemia marker nucleotide sequences. Nucleic acid variants can be naturally-occurring, such as allelic variants (same locus), homologues (different locus), and orthologues (different organism) or can be non naturally-occurring. Naturally occurring variants such as these can be identified with the use of well-known molecular biology techniques, as, for example, with polymerase chain reaction (PCR) and hybridization techniques as known in the art. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product). For nucleotide sequences, conservative variants include those sequences that, because of the degeneracy of the genetic code, encode the amino acid sequence of one of the endotoxemia marker polypeptides of the invention. Variant nucleotide sequences also include synthetically derived nucleotide sequences, such as those generated, for example, by using site-directed mutagenesis but which still encode an endotoxemia marker polypeptide of the invention. Generally, variants of a particular nucleotide sequence of the invention will have at least about 30%, 40% 50%, 55%, 60%, 65%, 70%, generally at least about 75%, 80%, 85%, desirably about 90% to 95% or more, and more suitably about 98% or more sequence identity to that particular nucleotide sequence as determined by sequence alignment programs described elsewhere herein using default parameters.

The endotoxemia marker nucleotide sequences of the invention can be used to isolate corresponding sequences and alleles from other organisms, particularly other mammals, especially other equine species. Methods are readily available in the art for the hybridization of nucleic acid sequences. Coding sequences from other organisms may be isolated according to well known techniques based on their sequence identity with the coding sequences set forth herein. In these techniques all or part of the known coding sequence is used as a probe which selectively hybridizes to other endotoxemia marker coding sequences present in a population of cloned genomic DNA fragments or cDNA fragments (i.e., genomic or cDNA libraries) from a chosen organism. Accordingly, the present invention also contemplates polynucleotides that hybridize to the endotoxemia marker gene nucleotide sequences, or to their complements, under stringency conditions described below. As used herein, the term "hybridizes under low stringency, medium stringency, high stringency, or very high stringency conditions" describes conditions for hybridization and washing. Guidance for performing hybridization reactions can be found in Ausubel *et al.*, (1998, *supra*), Sections 6.3.1-6.3.6. Aqueous and non-aqueous methods are described in that reference and either can be used. Reference herein to low stringency conditions include and encompass from at least about 1% v/v to at least about 15% v/v formamide and from at least about 1 M to at least about 2 M salt for hybridization at 42° C, and at least about 1 M to at least about 2 M salt for washing at 42° C. Low stringency conditions also may include 1% Bovine Serum Albumin (BSA), 1 mM EDTA, 0.5 M $NaHPO_4$ (pH 7.2), 7% SDS for hybridization at 65° C, and (i) 2 x SSC, 0.1 % SDS; or (ii) 0.5% BSA, 1 mM EDTA, 40 mM $NaHPO_4$ (pH 7.2), 5% SDS for washing at room temperature. One embodiment of low stringency conditions includes hybridization in 6 x sodium chloride/sodium citrate (SSC) at about 45° C, followed by two washes in 0.2 x SSC, 0.1% SDS at least at 50° C (the temperature of the washes can be increased to 55° C for low stringency conditions). Medium stringency conditions include and encompass from at least about 16% v/v to at least about 30% v/v formamide and from at least about 0.5 M to at least about 0.9 M salt for hybridization at 42° C, and at least about 0.1 M to at least about 0.2 M salt for washing at 55° C. Medium stringency conditions also may include 1% Bovine Serum Albumin (BSA), 1 mM EDTA, 0.5 M $NaHPO_4$ (pH 7.2), 7% SDS for hybridization at 65° C, and (i) 2 x SSC, 0.1% SDS; or (ii) 0.5% BSA, 1 mM EDTA, 40 mM $NaHPO_4$ (pH 7.2), 5% SDS for washing at 60-65° C. One embodiment of medium stringency conditions includes hybridizing in 6 x SSC at about 45° C, followed by one or more washes in 0.2 x SSC, 0.1% SDS at 60° C. High stringency conditions include and encompass from at least about 31% v/v to at least about 50% v/v formamide and from about 0.01 M to about 0.15 M salt for hybridization at 42° C, and about 0.01 M to about 0.02 M salt for washing at 55° C. High stringency conditions also may include 1% BSA, 1 mM EDTA, 0.5 M $NaHPO_4$ (pH 7.2), 7% SDS for hybridization at 65° C, and (i) 0.2 x SSC, 0.1% SDS; or (ii) 0.5% BSA, 1 mM EDTA, 40 mM $NaHPO_4$ (pH 7.2), 1% SDS for washing at a temperature in excess of 65° C. One embodiment of high stringency conditions includes hybridizing in 6 x SSC at about 45° C, followed by one or more washes in 0.2 x SSC, 0.1% SDS at 65° C.

In certain embodiments, an antigen-binding molecule of the invention is encoded by a polynucleotide that hybridizes to a disclosed nucleotide sequence under very high stringency conditions. One embodiment of very high stringency conditions includes hybridizing 0.5 M sodium phosphate, 7% SDS at 65° C, followed by one or more washes at 0.2 x SSC, 1% SDS at 65° C.

Other stringency conditions are well known in the art and a skilled addressee will recognize that various factors can be manipulated to optimize the specificity of the hybridization. Optimization of the stringency of the final washes can serve to ensure a high degree of hybridization. For detailed examples, see Ausubel *et al.*, *supra* at pages 2.10.1 to 2.10.16 and Sambrook *et al.* (1989, *supra)* at sections 1.101 to 1.104.

While stringent washes are typically carried out at temperatures from about 42° C to 68° C, one skilled in the art will appreciate that other temperatures may be suitable for stringent conditions. Maximum hybridization rate typically occurs at about 20° C to 25° C below the $T_m$ for formation of a DNA-DNA hybrid. It is well known in the art that the $T_m$ is the melting temperature, or temperature at which two complementary polynucleotide sequences dissociate. Methods for estimating $T_m$ are well known in the art (see Ausubel *et al.*, *supra* at page 2.10.8). In general, the $T_m$ of a perfectly matched duplex of DNA may be predicted as an approximation by the formula:

$T_m$= 81.5 + 16.6 ($log_{10}$ M) + 0.41 (%G+C) - 0.63 (% formamide) - (600/length)

wherein: M is the concentration of $Na^+$, preferably in the range of 0.01 molar to 0.4 molar; %G+C is the sum of guanosine and cytosine bases as a percentage of the total number of bases, within the range between 30% and 75% G+C; % formamide is the percent formamide concentration by volume; length is the number of base pairs in the DNA duplex. The $T_m$ of a duplex DNA decreases by approximately 1° C with every increase of 1% in the number of randomly mismatched base pairs. Washing is generally carries out at $T_m$ -15° C for high stringency, or $T_m$ - 30° C for moderate stringency.

In one example of a hybridization procedure, a membrane (e.g., a nitrocellulose membrane or a nylon membrane) containing immobilized DNA is hybridized overnight at 42° C in a hybridization buffer (50% deionised formamide, 5 x SSC, 5 x Denhardt's solution (0.1% ficoll, 0.1 % polyvinylpyrollidone and 0.1% bovine serum albumin), 0.1% SDS and 200 mg/mL denatured salmon sperm DNA) containing labeled probe. The membrane is then subjected to two sequential medium stringency washes (i.e., 2 x SSC, 0.1% SDS for 15 min at 45° C, followed by 2 x SSC, 0.1% SDS for 15 min at 50° C), followed by two sequential higher stringency washes (i.e., 0.2 x SSC, 0.1% SDS for 12 min at 55° C followed by 0.2 x SSC and 0.1%SDS solution for 12 min at 65-68° C.

### *5. Polypeptides of the invention*

The present invention also contemplates full-length polypeptides encoded by the endotoxemia marker genes of the invention as well as the biologically active portions of those polypeptides, which are referred to collectively herein as "endotoxemia marker polypeptides." Biologically active portions of full-length endotoxemia marker polypeptides include portions with immuno-interactive activity of at least about 6, 8, 10, 12, 14, 16, 18, 20, 25, 30, 40, 50, 60 amino acid residues in length. For example, immuno-interactive fragments contemplated by the present invention are at least 6 and desirably at least 8 amino acid residues in length, which can elicit an immune response in an animal for the production of antigen-binding molecules that are immuno-interactive with an endotoxemia marker polypeptide of the invention. Such antigen-binding molecules can be used to screen other mammals, especially equine mammals, for structurally and/or functionally related endotoxemia marker polypeptides. Typically, portions of a full-length endotoxemia marker polypeptide may participate in an interaction, for example, an intramolecular or an inter-molecular interaction. An inter-molecular interaction can be a specific binding interaction or an enzymatic interaction (e.g., the interaction can be transient and a covalent bond is formed or broken). Biologically active portions of a full-length endotoxemia marker polypeptide include peptides comprising amino acid sequences sufficiently similar to or derived from the amino acid sequences of a (putative) full-length endotoxemia marker polypeptide, for example, the amino acid sequences shown in SEQ ID NO: 2, 4, 6, 9, 11, 13, 15, 19, 21, 23, 25, 29, 31, 33, 51, 53 or 58, which include less amino acids than a full-length endotoxemia marker polypeptide, and exhibit at least one activity of that polypeptide. Typically, biologically active portions comprise a domain or motif with at least one activity of a full-length endotoxemia marker polypeptide. A biologically active portion of a full-length endotoxemia marker polypeptide can be a polypeptide which is, for example, 5, 6, 7, 8, 9, 10, 11, 12, 13, 14, 15, 16, 17, 18, 19, 20, 21, 22, 23, 24, 25, 30, 40, 50, 60, 70, 80, 90, 100, 120, 150, 300, 400, 500, 600, 700, 800, 900 or 1000, or even at least about 2000 or 3000, or more amino acid residues in length. Suitably, the portion is a "biologically-active portion" having no less than about 1%, 10%, 25% 50% of the activity of the full-length polypeptide from which it is derived.

The present invention also contemplates variant endotoxemia marker polypeptides. "Variant" polypeptides include proteins derived from the native protein by deletion (so-called truncation) or addition of one or more amino acids to the N-terminal and/or C-terminal end of the native protein; deletion or addition of one or more amino acids at one or more sites in the native protein; or substitution of one or more amino acids at one or more sites in the native protein. Variant proteins encompassed by the present invention are biologically active, that is, they continue to possess the desired biological activity of the native protein. Such variants may result from, for example, genetic polymorphism or from human manipulation. Biologically active variants of a native endotoxemia marker polyeptide of the invention will have at least 40%, 50%, 60%, 70%, generally at least 75%, 80%, 85%, preferably about 90% to 95% or more, and more preferably about 98% or more sequence similarity with the amino acid sequence for the native protein as determined by sequence alignment programs described elsewhere herein using default parameters. A biologically active variant of a protein of the invention may differ from that protein generally by as much 1000, 500, 400, 300, 200, 100, 50 or 20 amino acid residues or suitably by as few as 1-15 amino acid residues, as few as 1-10, such as 6-10, as few as 5, as few as 4, 3, 2, or even 1 amino acid residue.

An endotoxemia marker polypeptide of the invention may be altered in various ways including amino acid substitutions, deletions, truncations, and insertions. Methods for such manipulations are generally known in the art. For example, amino acid sequence variants of an endotoxemia marker protein can be prepared by mutations in the DNA. Methods for mutagenesis and nucleotide sequence alterations are well known in the art. See, for example, Kunkel (1985, Proc. Natl. Acad. Sci. USA 82:488-492), Kunkel et al. (1987, Methods in Enzymol. 154:367-382), U.S. Pat. No. 4,873,192, Watson, J. D. et al. ("Molecular Biology of the Gene", Fourth Edition, Benjamin/Cummings, Menlo Park, Calif., 1987) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.). Methods for screening gene products of combinatorial libraries made by point mutations or truncation, and for screening cDNA libraries for gene products having a selected property are known in the art. Such methods are adaptable for rapid screening of the gene libraries generated by combinatorial mutagenesis of endotoxemia marker polypeptides. Recursive ensemble mutagenesis (REM), a technique which enhances the frequency of functional mutants in the libraries, can be used in combination with the screening assays to identify endotoxemia marker polypeptide variants (Arkin and Yourvan (1992) Proc. Natl. Acad. Sci. USA 89:7811-7815; Delgrave et al. (1993) Protein Engineering 6:327-331). Conservative substitutions, such as exchanging one amino acid with another having similar properties, may be desirable as discussed in more detail below.

Variant endotoxemia marker polypeptides may contain conservative amino acid substitutions at various locations along their sequence, as compared to the parent endotoxemia marker amino acid sequence. A "conservative amino acid substitution" is one in which the amino acid residue is replaced with an amino acid residue having a similar side chain. Families of amino acid residues having similar side chains have been defined in the art, which can be generally sub-classified as follows:

Acidic: The residue has a negative charge due to loss of H ion at physiological pH and the residue is attracted by aqueous solution so as to seek the surface positions in the conformation of a peptide in which it is contained when the peptide is in aqueous medium at physiological pH. Amino acids having an acidic side chain include glutamic acid and aspartic acid.

Basic: The residue has a positive charge due to association with H ion at physiological pH or within one or two pH units thereof (e.g., histidine) and the residue is attracted by aqueous solution so as to seek the surface positions in the conformation of a peptide in which it is contained when the peptide is in aqueous medium at physiological pH. Amino acids having a basic side chain include arginine, lysine and histidine.

Charged: The residues are charged at physiological pH and, therefore, include amino acids having acidic or basic side chains (i.e., glutamic acid, aspartic acid, arginine, lysine and histidine).

Hydrophobic: The residues are not charged at physiological pH and the residue is repelled by aqueous solution so as to seek the inner positions in the conformation of a peptide in which it is contained when the peptide is in aqueous medium. Amino acids having a hydrophobic side chain include tyrosine, valine, isoleucine, leucine, methionine, phenylalanine and tryptophan.

Neutral/polar: The residues are not charged at physiological pH, but the residue is not sufficiently repelled by aqueous solutions so that it would seek inner positions in the conformation of a peptide in which it is contained when the peptide is in aqueous medium. Amino acids having a neutral/polar side chain include asparagine, glutamine, cysteine, histidine, serine and threonine.

This description also characterizes certain amino acids as "small" since their side chains are not sufficiently large, even if polar groups are lacking, to confer hydrophobicity. With the exception of proline, "small" amino acids are those with four carbons or less when at least one polar group is on the side chain and three carbons or less when not. Amino acids having a small side chain include glycine, serine, alanine and threonine. The gene-encoded secondary amino acid proline is a special case due to its known effects on the secondary conformation of peptide chains. The structure of proline differs from all the other naturally-occurring amino acids in that its side chain is bonded to the nitrogen of the $\alpha$-amino group, as well as the $\alpha$-carbon. Several amino acid similarity matrices (e.g., PAM120 matrix and PAM250 matrix as disclosed for example by Dayhoff et al. (1978) A model of evolutionary change in proteins. Matrices for determining distance relationships In M. O. Dayhoff, (ed.), Atlas of protein sequence and structure, Vol. 5, pp. 345-358, National Biomedical Research Foundation, Washington DC; and by Gonnet et al., 1992, Science 256(5062): 144301445), however, include proline in the same group as glycine, serine, alanine and threonine. Accordingly, for the purposes of the present invention, proline is classified as a "small" amino acid.

The degree of attraction or repulsion required for classification as polar or nonpolar is arbitrary and, therefore, amino acids specifically contemplated by the invention have been classified as one or the other. Most amino acids not specifically named can be classified on the basis of known behavior.

Amino acid residues can be further sub-classified as cyclic or noncyclic, and aromatic or nonaromatic, self-explanatory classifications with respect to the side-chain substituent groups of the residues, and as small or large. The residue is considered small if it contains a total of four carbon atoms or less, inclusive of the carboxyl carbon, provided an additional polar substituent is present; three or less if not. Small residues are, of course, always nonaromatic. Dependent on their structural properties, amino acid residues may fall in two or more classes. For the naturally-occurring protein amino acids, sub-classification according to the this scheme is presented in the Table 3.

Conservative amino acid substitution also includes groupings based on side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. For example, it is reasonable to expect that replacement of a leucine with an isoleucine or valine, an aspartate with a glutamate, a threonine with a serine, or a similar replacement of an amino acid with a structurally related amino acid will not have a major effect on the properties of the resulting variant polypeptide. Whether an amino acid change results in a functional endotoxemia marker polypeptide can readily be determined by assaying its activity. Conservative substitutions are shown in Table 4 below under the heading of exemplary substitutions. More preferred substitutions are shown under the heading of preferred substitutions. Amino acid substitutions falling within the scope of the invention, are, in general, accomplished by selecting substitutions that do not differ significantly in their effect on maintaining (a) the structure of the peptide backbone in the area of the substitution, (b) the charge or hydrophobicity of the molecule at the target site, or (c) the bulk of the side chain. After the substitutions are introduced, the variants are screened for biological activity.

Alternatively, similar amino acids for making conservative substitutions can be grouped into three categories based on the identity of the side chains. The first group includes glutamic acid, aspartic acid, arginine, lysine, histidine, which all have charged side chains; the second group includes glycine, serine, threonine, cysteine, tyrosine, glutamine, asparagine; and the third group includes leucine, isoleucine, valine, alanine, proline, phenylalanine, tryptophan, methionine, as described in Zubay, G., Biochemistry, third edition, Wm.C. Brown Publishers (1993).

Thus, a predicted non-essential amino acid residue in an endotoxemia marker polypeptide is typically replaced with another amino acid residue from the same side chain family. Alternatively, mutations can be introduced randomly along all or part of an endotoxemia marker gene coding sequence, such as by saturation mutagenesis, and the resultant mutants can be screened for an activity of the parent polypeptide to identify mutants which retain that activity. Following mutagenesis of the coding sequences, the encoded peptide can be expressed recombinantly and the activity of the peptide can be determined.

Accordingly, the present invention also contemplates variants of the naturally-occurring endotoxemia marker polypeptide sequences or their biologically-active fragments, wherein the variants are distinguished from the naturally-occurring sequence by the addition, deletion, or substitution of one or more amino acid residues. In general, variants will display at least about 30, 40, 50, 55, 60, 65, 70, 75, 80, 85, 90, 91, 92, 93, 94, 95, 96, 97, 98, 99 % similarity to a parent endotoxemia marker polypeptide sequence as, for example, set forth in any one of SEQ ID NO: 2, 4, 6, 9, 11, 13, 15, 19, 21, 23, 25, 29, 31, 33, 51, 53 or 58. Desirably, variants will have at least 30, 40, 50, 55, 60, 65, 70, 75, 80, 85, 90, 91, 92, 93, 94, 95, 96, 97, 98, 99 % sequence identity to a parent endotoxemia marker polypeptide sequence as, for example, set forth in any one of SEQ ID NO: 2, 4, 6, 9, 11, 13, 15, 19, 21, 23, 25, 29, 31, 33, 51, 53 or 58. Moreover, sequences differing from the native or parent sequences by the addition, deletion, or substitution of 1, 2, 3, 4, 5, 6, 7, 8, 9, 10, 11, 12, 13, 14, 15, 16, 17, 18, 19, 20, 30, 40, 50, 60 ,70, 80 ,90, 100, 150, 200, 300, 500 or more amino acids but which retain the properties of the parent endotoxemia marker polypeptide are contemplated. endotoxemia marker polypeptides also include polypeptides that are encoded by polynucleotides that hybridize under stringency conditions as defined herein, especially high stringency conditions, to the endotoxemia marker polynucleotide sequences of the invention, or the non-coding strand thereof, as described above.

In one embodiment, variant polypeptides differ from an endotoxemia marker sequence by at least one but by less than 50, 40, 30, 20, 15, 10, 8, 6, 5, 4, 3 or 2 amino acid residue(s). In another, variant polypeptides differ from the corresponding sequence in any one of SEQ ID NO: 2, 4, 6, 9, 11, 13, 15, 19, 21, 23, 25, 29, 31, 33, 51, 53 or 58 by at least 1% but less than 20%, 15%, 10% or 5% of the residues. (If this comparison requires alignment the sequences should be aligned for maximum similarity. "Looped" out sequences from deletions or insertions, or mismatches, are considered differences.) The differences are, suitably, differences or changes at a non-essential residue or a conservative substitution.

A "non-essential" amino acid residue is a residue that can be altered from the wild-type sequence of an embodiment polypeptide without abolishing or substantially altering one or more of its activities. Suitably, the alteration does not substantially alter one of these activities, for example, the activity is at least 20%, 40%, 60%, 70% or 80% of wild-type. An "essential" amino acid residue is a residue that, when altered from the wild-type sequence of an endotoxemia marker polypeptide of the invention, results in abolition of an activity of the parent molecule such that less than 20% of the wild-type activity is present.

In other embodiments, a variant polypeptide includes an amino acid sequence having at least about 50%, 55%, 60%, 65%, 70%, 75%, 80%, 85%, 90%, 91%, 92%, 93%, 94% 95%, 96%, 97%, 98% or more similarity to a corresponding sequence of an endotoxemia marker polypeptide as, for example, set forth in any one of SEQ ID NO: 2, 4, 6, 9, 11, 13, 15, 19, 21, 23, 25, 29, 31, 33, 51, 53 or 58, and has the activity of that endotoxemia marker polypeptide.

Endotoxemia marker polypeptides of the invention may be prepared by any suitable procedure known to those of skill in the art. For example, the polypeptides may be prepared by a procedure including the steps of: (a) preparing a chimeric construct comprising a nucleotide sequence that encodes at least a portion of an endotoxemia marker polynucleotide and that is operably linked to a regulatory element; (b) introducing the chimeric construct into a host cell; (c) culturing the host cell to express the endotoxemia marker polypeptide; and (d) isolating the endotoxemia marker polypeptide from the host cell. In illustrative examples, the nucleotide sequence encodes at least a portion of the sequence set forth in any one of SEQ ID NO: 2, 4, 6, 9, 11, 13, 15, 19, 21, 23, 25, 29, 31, 33, 51, 53 or 58 or a variant thereof.

The chimeric construct is typically in the form of an expression vector, which is suitably selected from self-replicating extra-chromosomal vectors (e.g., plasmids) and vectors that integrate into a host genome.

The regulatory element will generally be appropriate for the host cell employed for expression of the endotoxemia marker polynucleotide. Numerous types of expression vectors and regulatory elements are known in the art for a variety of host cells. Illustrative elements of this type include, but are not restricted to, promoter sequences (e.g., constitutive or inducible promoters which may be naturally occurring or combine elements of more than one promoter), leader or signal sequences, ribosomal binding sites, transcriptional start and stop sequences, translational start and termination sequences, and enhancer or activator sequences.

In some embodiments, the expression vector comprises a selectable marker gene to permit the selection of transformed host cells. Selectable marker genes are well known in the art and will vary with the host cell employed.

The expression vector may also include a fusion partner (typically provided by the expression vector) so that the endotoxemia marker polypeptide is produced as a fusion polypeptide with the fusion partner. The main advantage of fusion partners is that they assist identification and/or purification of the fusion polypeptide. In order to produce the fusion polypeptide, it is necessary to ligate the endotoxemia marker polynucleotide into an expression vector so that the translational reading frames of the fusion partner and the endotoxemia marker polynucleotide coincide. Well known examples of fusion partners include, but are not limited to, glutathione-S-transferase (GST), Fc potion of human IgG, maltose binding protein (MBP) and hexahistidine ($HIS_6$), which are particularly useful for isolation of the fusion polypeptide by affinity chromatography. In some embodiments, fusion polypeptides are purified by affinity chromatography using matrices to which the fusion partners bind such as but not limited to glutathione-, amylose-, and nickel- or cobalt-conjugated resins. Many such matrices are available in "kit" form, such as the QIAexpress™ system (Qiagen) useful

with ($HIS_6$) fusion partners and the Pharmacia GST purification system. Other fusion partners known in the art are light-emitting proteins such as green fluorescent protein (GFP) and luciferase, which serve as fluorescent "tags" that permit the identification and/or isolation of fusion polypeptides by fluorescence microscopy or by flow cytometry. Flow cytometric methods such as fluorescence activated cell sorting (FACS) are particularly useful in this latter application.

Desirably, the fusion partners also possess protease cleavage sites, such as for Factor $X_a$ or Thrombin, which permit the relevant protease to partially digest the fusion polypeptide and thereby liberate the endotoxemia marker polypeptide from the fusion construct. The liberated polypeptide can then be isolated from the fusion partner by subsequent chromatographic separation.

Fusion partners also include within their scope "epitope tags," which are usually short peptide sequences for which a specific antibody is available. Well known examples of epitope tags for which specific monoclonal antibodies are readily available include c-Myc, influenza virus, hemagglutinin and FLAG tags.

The chimeric constructs of the invention are introduced into a host by any suitable means including "transduction" and "transfection", which are art recognized as meaning the introduction of a nucleic acid, for example, an expression vector, into a recipient cell by nucleic acid-mediated gene transfer. "Transformation," however, refers to a process in which a host's genotype is changed as a result of the cellular uptake of exogenous DNA or RNA, and, for example, the transformed cell comprises the expression system of the invention. There are many methods for introducing chimeric constructs into cells. Typically, the method employed will depend on the choice of host cell. Technology for introduction of chimeric constructs into host cells is well known to those of skill in the art. Four general classes of methods for delivering nucleic acid molecules into cells have been described: (1) chemical methods such as calcium phosphate precipitation, polyethylene glycol (PEG)-mediate precipitation and lipofection; (2) physical methods such as microinjection, electroporation, acceleration methods and vacuum infiltration; (3) vector based methods such as bacterial and viral vector-mediated transformation; and (4) receptor-mediated. Transformation techniques that fall within these and other classes are well known to workers in the art, and new techniques are continually becoming known. The particular choice of a transformation technology will be determined by its efficiency to transform certain host species as well as the experience and preference of the person practicing the invention with a particular methodology of choice. It will be apparent to the skilled person that the particular choice of a transformation system to introduce a chimeric construct into cells is not essential to or a limitation of the invention, provided it achieves an acceptable level of nucleic acid transfer.

Recombinant endotoxemia marker polypeptides may be produced by culturing a host cell transformed with a chimeric construct. The conditions appropriate for expression of the endotoxemia marker polynucleotide will vary with the choice of expression vector and the host cell and are easily ascertained by one skilled in the art through routine experimentation. Suitable host cells for expression may be prokaryotic or eukaryotic. An illustrative host cell for expression of a polypeptide of the invention is a bacterium. The bacterium used may be *Escherichia coli*. Alternatively, the host cell may be a yeast cell or an insect cell such as, for example, *SF9* cells that may be utilized with a baculovirus expression system.

Recombinant endotoxemia marker polypeptides can be conveniently prepared using standard protocols as described for example in Sambrook, *et al.,* (1989, *supra),* in particular Sections 16 and 17; Ausubel *et al.,* (1994, *supra),* in particular Chapters 10 and 16; and Coligan et al., CURRENT PROTOCOLS IN PROTEIN SCIENCE (John Wiley & Sons, Inc. 1995-1997), in particular Chapters 1, 5 and 6. Alternatively, the endotoxemia marker polypeptides may be synthesized by chemical synthesis, e.g., using solution synthesis or solid phase synthesis as described, for example, in Chapter 9 of Atherton and Shephard (*supra*) and in Roberge et al (1995, Science 269: 202).

### *6. Antigen-binding molecules*

The invention also provides antigen-binding molecules that are specifically immuno-interactive with an endotoxemia marker polypeptide of the invention. In one embodiment, the antigen-binding molecule comprise whole polyclonal antibodies. Such antibodies may be prepared, for example, by injecting an endotoxemia marker polypeptide of the invention into a production species, which may include mice or rabbits, to obtain polyclonal antisera. Methods of producing polyclonal antibodies are well known to those skilled in the art. Exemplary protocols which may be used are described for example in Coligan et al., CURRENT PROTOCOLS IN IMMUNOLOGY, (John Wiley & Sons, Inc, 1991), and Ausubel *et al.,* (1994-1998, *supra),* in particular Section III of Chapter 11.

In lieu of polyclonal antisera obtained in a production species, monoclonal antibodies may be produced using the standard method as described, for example, by Köhler and Milstein (1975, Nature 256, 495-497), or by more recent modifications thereof as described, for example, in Coligan *et al.,* (1991, *supra)* by immortalizing spleen or other antibody producing cells derived from a production species which has been inoculated with one or more of the endotoxemia marker polypeptides of the invention.

The invention also contemplates as antigen-binding molecules Fv, Fab, Fab' and $F(ab')_2$ immunoglobulin fragments. Alternatively, the antigen-binding molecule may comprise a synthetic stabilized Fv fragment. Exemplary fragments of this type include single chain Fv fragments (sFv, frequently termed scFv) in which a peptide linker is used to bridge the N terminus or C terminus of a $V_H$ domain with the C terminus or N-terminus, respectively, of a $V_L$ domain. ScFv lack all constant parts of whole antibodies and are not able to activate complement. ScFvs may be prepared, for example, in accordance with methods outlined in Kreber *et al* (Kreber et al. 1997, J. Immunol. Methods; 201(1): 35-55). Alternatively, they may be prepared by methods described in U.S. Patent No 5,091,513, European Patent No 239,400 or the articles by Winter and Milstein (1991, Nature 349:293) and Plückthun et al (1996, In Antibody engineering: A practical approach. 203-252). In another embodiment, the synthetic stabilized Fv fragment comprises a disulfide stabilized Fv (dsFv) in which cysteine residues are introduced into the $V_H$ and $V_L$ domains such that in the fully folded Fv molecule the two residues will form a disulfide bond between them. Suitable methods of producing dsFv are described for example in (Glockscuther et al. Biochem. 29: 1363-1367; Reiter et al. 1994, J. Biol. Chem. 269: 18327-18331; Reiter et al. 1994, Biochem. 33: 5451-5459; Reiter et al. 1994. Cancer Res. 54: 2714-2718; Webber et al. 1995, Mol. Immunol. 32: 249-258).

Phage display and combinatorial methods for generating anti-endotoxemia marker polypeptide antigen-binding molecules are known in the art (as described in, e.g., Ladner *et al.* U.S. Patent No. 5,223,409; Kang et al. International Publication No. WO 92/18619; Dower et al. International Publication No. WO 91/17271; Winter et al. International Publication WO 92/20791; Markland et al. International Publication No. WO 92/15679; Breitling et al. International Publication WO 93/01288; McCafferty et al. International Publication No. WO 92/01047; Garrard et al. International Publication No. WO 92/09690; Ladner et al. International Publication No. WO 90/02809; Fuchs et al. (1991) Bio/Technology 9: 1370-1372; Hay et al. (1992) Hum Antibod Hybridomas 3:81-85; Huse et al. (1989) Science 246:1275-1281; Griffths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrad et al. (1991) Bio/Technology 9:1373-1377; Hoogenboom et al. (1991) Nuc Acid Res 19:4133-4137; and Barbas et al. (1991) PNAS 88:7978-7982). The antigen-binding molecules can be used to screen expression libraries for variant endotoxemia marker polypeptides. They can also be used to detect and/or isolate the endotoxemia marker polypeptides of the invention. Thus, the invention also contemplates the use of antigen-binding molecules to isolate endotoxemia marker polypeptides using, for example, any suitable immunoaffinity based method including, but not limited to, immunochromatography and immunoprecipitation. A suitable method utilises solid phase adsorption in which anti-endotoxemia marker polypeptide antigen-binding molecules are attached to a suitable resin, the resin is contacted with a sample suspected of containing an endotoxemia marker polypeptide, and the endotoxemia marker polypeptide, if any, is subsequently eluted from the resin. Illustrative resins include: Sepharose® (Pharmacia), Poros® resins (Roche Molecular Biochemicals, Indianapolis), Actigel Superflow™ resins (Sterogene Bioseparations Inc., Carlsbad Calif.), and Dynabeads™ (Dynal Inc., Lake Success, N.Y.).

The antigen-binding molecule can be coupled to a compound, e.g., a label such as a radioactive nucleus, or imaging agent, e.g. a radioactive, enzymatic, or other, e.g., imaging agent, e.g., a NMR contrast agent. Labels which produce detectable radioactive emissions or fluorescence are preferred. An anti- endotoxemia marker polypeptide antigen-binding molecule (e.g., monoclonal antibody) can be used to detect endotoxemia marker polypeptides (e.g., in a cellular lysate or cell supernatant) in order to evaluate the abundance and pattern of expression of the protein. In certain advantageous application in accordance with the present invention, such antigen-binding molecules can be used to monitor endotoxemia marker polypeptides levels in biological samples (including whole cells and fluids) for diagnosing the presence, absence, degree, or stage of development of endotoxemia. Detection can be facilitated by coupling (i.e., physically linking) the antibody to a detectable substance (i.e., antibody labeling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include $^{125}I$, $^{131}I$, $^{35}S$ or $^{3}H$. The label may be selected from a group including a chromogen, a catalyst, an enzyme, a fluorophore, a chemiluminescent molecule, a lanthanide ion such as Europium ($Eu^{34}$), a radioisotope and a direct visual label. In the case of a direct visual label, use may be made of a colloidal metallic or non-metallic particle, a dye particle, an enzyme or a substrate, an organic polymer, a latex particle, a liposome, or other vesicle containing a signal producing substance and the like.

A large number of enzymes useful as labels is disclosed in United States Patent Specifications U.S. 4,366,241, U.S. 4,843,000, and U.S. 4,849,338. Enzyme labels useful in the present invention include alkaline phosphatase, horseradish peroxidase, luciferase, β-galactosidase, glucose oxidase, lysozyme, malate dehydrogenase and the like. The enzyme label may be used alone or in combination with a second enzyme in solution.

### *7. Methods of detecting aberrant endotoxemia marker gene expression or the presence of endotoxemia marker polynucleotides*

The present invention is predicated in part on the discovery that: horses with clinical evidence of endotoxemia-related conditions have aberrant expression of certain genes (referred to herein as "endotoxemia marker genes") whose transcripts include, but are not limited to, SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260,262,264,266,268,269,270,271,272,274,276,278,279,280,282,284, 286, 288, 290, 292, 294, 296, 298, 300, 302, 304, 305, 306, 307, 309, 311, 312, 314, 315, 316, 318, 320 321, 323 or 325, as compared to normal horses or to horses lacking endotoxemia-related conditions.

Accordingly, in certain embodiments, the invention features a method for diagnosing the presence, absence, degree or stage of an endotoxemia-related condition in a subject, which is typically of equine origin, by detecting aberrant expression of an endotoxemia marker gene in a biological sample obtained from the subject. Accordingly, in order to make such diagnoses, it will be desirable to qualitatively or quantitatively determine the levels of endotoxemia marker gene transcripts or the level or functional activity of endotoxemia marker polypeptides. In some embodiments, the presence, degree, or stage of development of an endotoxemia-related condition is diagnosed when an endotoxemia marker gene product is expressed at a detectably lower level in the biological sample as compared to the level at which that gene is expressed in a reference sample obtained from normal subjects or from subjects lacking that condition. In other embodiments, the presence, degree, or stage of development of an endotoxemia-related condition is diagnosed when an endotoxemia marker gene product is expressed at a detectably higher level in the biological sample as compared to the level at which that gene is expressed in a reference sample obtained from normal subjects or from subjects lacking that condition. Generally, such diagnoses are made when the level or functional activity of an endotoxemia marker gene product in the biological sample varies from the level or functional activity of a corresponding endotoxemia marker gene product in the reference sample by at least 10%, 20%, 30%, 40%, 50%, 60%, 70%, 80%, 90%, 92%, 94%, 96%, 97%, 98% or 99%, or even by at least about 99.5%, 99.9%, 99.95%, 99.99%, 99.995% or 99.999%, or even by at least about 100%, 200%, 300%, 400%, 500%, 600%, 700%, 800%, 900% or 1000%. The corresponding gene product is generally selected from the same gene product that is present in the biological sample, a gene product expressed from a variant gene (e.g., an homologous or orthologous gene) including an allelic variant, or a splice variant or protein product thereof. In some embodiments, the method comprises measuring the level or functional activity of individual expression products of at least about 2, 3, 4, 5, 6, 7, 8, 9, 10, 11, 12, 13, 14, 15, 16, 17, 18, 19, 20, 21, 22, 23, 24, 25, 26, 27, 28 29 or 30 endotoxemia marker genes.

Generally, the biological sample contains blood, especially peripheral blood, or a fraction or extract thereof. Typically, the biological sample comprises blood cells such as mature, immature and developing leukocytes, including lymphocytes, polymorphonuclear leukocytes, neutrophils, monocytes, reticulocytes, basophils, coelomocytes, hemocytes, eosinophils, megakaryocytes, macrophages, dendritic cells natural killer cells, or fraction of such cells (e.g., a nucleic acid or protein fraction). In specific embodiments, the biological sample comprises leukocytes including peripheral blood mononuclear cells (PBMC).

## 7.1 Nucleic acid-based diagnostics

Nucleic acid used in polynucleotide-based assays can be isolated from cells contained in the biological sample, according to standard methodologies (Sambrook, *et al., 1989, supra;* and Ausubel *et al.,* 1994, *supra).* The nucleic acid is typically fractionated (e.g., poly $A^+$ RNA) or whole cell RNA. Where RNA is used as the subject of detection, it may be desired to convert the RNA to a complementary DNA. In some embodiments, the nucleic acid is amplified by a template-dependent nucleic acid amplification technique. A number of template dependent processes are available to amplify the endotoxemia marker sequences present in a given template sample. An exemplary nucleic acid amplification technique is the polymerase chain reaction (referred to as PCR) which is described in detail in U.S. Pat. Nos. 4,683,195, 4,683,202 and 4,800,159, *Ausubel et al.* (*supra*), and in Innis et al., ("PCR Protocols", Academic Press, Inc., San Diego Calif., 1990). Briefly, in PCR, two primer sequences are prepared that are complementary to regions on opposite complementary strands of the marker sequence. An excess of deoxynucleoside triphosphates are added to a reaction mixture along with a DNA polymerase, e.g., *Taq* polymerase. If a cognate endotoxemia marker sequence is present in a sample, the primers will bind to the marker and the polymerase will cause the primers to be extended along the marker sequence by adding on nucleotides. By raising and lowering the temperature of the reaction mixture, the extended primers will dissociate from the marker to form reaction products, excess primers will bind to the marker and to the reaction products and the process is repeated. A reverse transcriptase PCR amplification procedure may be performed in order to quantify the amount of mRNA amplified. Methods of reverse transcribing RNA into cDNA are well known and described in Sambrook *et al.,* 1989, *supra.* Alternative methods for reverse transcription utilize thermostable, RNA-dependent DNA polymerases. These methods are described in WO 90/07641. Polymerase chain reaction methodologies are well known in the art.

In certain advantageous embodiments, the template-dependent amplification involves the quantification of transcripts in real-time. For example, RNA or DNA may be quantified using the Real-Time PCR technique (Higuchi, 1992, et al., Biotechnology 10: 413-417). By determining the concentration of the amplified products of the target DNA in PCR reactions that have completed the same number of cycles and are in their linear ranges, it is possible to determine the relative concentrations of the specific target sequence in the original DNA mixture. If the DNA mixtures are cDNAs synthesized from RNAs isolated from different tissues or cells, the relative abundance of the specific mRNA from which the target sequence was derived can be determined for the respective tissues or cells. This direct proportionality between the concentration of the PCR products and the relative mRNA abundance is only true in the linear range of the PCR reaction. The final concentration of the target DNA in the plateau portion of the curve is determined by the availability of reagents in the reaction mix and is independent of the original concentration of target DNA.

Another method for amplification is the ligase chain reaction ("LCR"), disclosed in EPO No. 320 308. In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. U.S. Pat. No. 4,883,750 describes a method similar to LCR for binding probe pairs to a target sequence.

Qβ Replicase, described in PCT Application No. PCT/US87/00880, may also be used. In this method, a replicative sequence of RNA that has a region complementary to that of a target is added to a sample in the presence of an RNA polymerase. The polymerase will copy the replicative sequence that can then be detected.

An isothermal amplification method, in which restriction endonucleases and ligases are used to achieve the amplification of target molecules that contain nucleotide 5'α-thio-triphosphates in one strand of a restriction site may also be useful in the amplification of nucleic acids in the present invention, Walker et al., (1992, Proc. Natl. Acad. Sci. U.S.A 89: 392-396).

Strand Displacement Amplification (SDA) is another method of carrying out isothermal amplification of nucleic acids which involves multiple rounds of strand displacement and synthesis, i.e., nick translation. A similar method, called Repair Chain Reaction (RCR), involves annealing several probes throughout a region targeted for amplification, followed by a repair reaction in which only two of the four bases are present. The other two bases can be added as biotinylated derivatives for easy detection. A similar approach is used in SDA. Target specific sequences can also be detected using a cyclic probe reaction (CPR). In CPR, a probe having 3' and 5' sequences of non-specific DNA and a middle sequence of specific RNA is hybridized to DNA that is present in a sample. Upon hybridization, the reaction is treated with RNase H, and the products of the probe identified as distinctive products that are released after digestion. The original template is annealed to another cycling probe and the reaction is repeated.

Still another amplification method described in GB Application No. 2 202 328, and in PCT Application No. PCT/US89/01025, may be used. In the former application, "modified" primers are used in a PCR-like, template- and enzyme-dependent synthesis. The primers may be modified by labeling with a capture moiety (e.g., biotin) and/or a detector moiety (e.g., enzyme). In the latter application, an excess of labeled probes are added to a sample. In the presence of the target sequence, the probe binds and is cleaved catalytically. After cleavage, the target sequence is released intact to be bound by excess probe. Cleavage of the labelled probe signals the presence of the target sequence.

Other nucleic acid amplification procedures include transcription-based amplification systems (TAS), including nucleic acid sequence based amplification (NASBA) and 3SR (Kwoh et al., 1989, Proc. Natl. Acad. Sci. U.S.A., 86: 1173; Gingeras et al., PCT Application WO 88/10315). In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer which has target specific sequences. Following polymerization, DNA/RNA hybrids are digested with RNase H while double stranded DNA molecules are heat denatured again. In either case the single stranded DNA is made fully double stranded by addition of second target specific primer, followed by polymerisation. The double-stranded DNA molecules are then multiply transcribed by an RNA polymerase such as T7 or SP6. In an isothermal cyclic reaction, the RNAs are reverse transcribed into single stranded DNA, which is then converted to double stranded DNA, and then transcribed once again with an RNA polymerase such as T7 or SP6. The resulting products, whether truncated or complete, indicate target specific sequences.

Vincent and Kong disclose a method termed helicase-dependent isothermal DNA amplification (HDA) (Vincent and Kong, EMBO Reports, 5(8):795-800, 2004). This method uses DNA helicase to separate DNA strands and hence does not require thermal cycling. The entire reaction can be carried out at one temperature and this method should have broad application to point-of-care DNA diagnostics.

Davey et al., EPO No. 329 822 disclose a nucleic acid amplification process involving cyclically synthesizing single-stranded RNA ("ssRNA"), ssDNA, and double-stranded DNA (dsDNA), which may be used in accordance with the present invention. The ssRNA is a template for a first primer oligonucleotide, which is elongated by reverse transcriptase (RNA-dependent DNA polymerase). The RNA is then removed from the resulting DNA:RNA duplex by the action of ribonuclease H (RNase H, an RNase specific for RNA in duplex with either DNA or RNA). The resultant ssDNA is a template for a second primer, which also includes the sequences of an RNA polymerase promoter (exemplified by T7 RNA polymerase) 5' to its homology to the template. This primer is then extended by DNA polymerase (exemplified by the large "Klenow" fragment of E. coli DNA polymerase I), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Miller *et al.* in PCT Application WO 89/06700 disclose a nucleic acid sequence amplification scheme based on the hybridization of a promoter/primer sequence to a target single-stranded DNA ("ssDNA") followed by transcription of many RNA copies of the sequence. This scheme is not cyclic, *i.e.*, new templates are not produced from the resultant RNA transcripts. Other amplification methods include "RACE" and "one-sided PCR" (Frohman, M. A., In: "PCR Protocols: A Guide to Methods and Applications", Academic Press, N.Y., 1990; Ohara et al., 1989, Proc. Natl Acad. Sci. U.S.A., 86: 5673-567).

Methods based on ligation of two (or more) oligonucleotides in the presence of nucleic acid having the sequence of the resulting "di-oligonucleotide", thereby amplifying the di-oligonucleotide, may also be used for amplifying target nucleic acid sequences. Wu et al., (1989, Genomics 4: 560).

Depending on the format, the endotoxemia marker nucleic acid of interest is identified in the sample directly using a template-dependent amplification as described, for example, above, or with a second, known nucleic acid following amplification. Next, the identified product is detected. In certain applications, the detection may be performed by visual means (e.g., ethidium bromide staining of a gel). Alternatively, the detection may involve indirect identification of the product *via* chemiluminescence, radioactive scintigraphy of radiolabel or fluorescent label or even via a system using electrical or thermal impulse signals (Affymax Technology; Bellus, 1994, J Macromol. Sci. Pure, Appl. Chem., A31 (1): 1355-1376).

In some embodiments, amplification products or "amplicons" are visualized in order to confirm amplification of the endotoxemia marker sequences. One typical visualization method involves staining of a gel with ethidium bromide and visualization under UV light. Alternatively, if the amplification products are integrally labeled with radio- or fluorometrically-labelled nucleotides, the amplification products can then be exposed to x-ray film or visualized under the appropriate stimulating spectra, following separation. In some embodiments, visualization is achieved indirectly. Following separation of amplification products, a labeled nucleic acid probe is brought into contact with the amplified endotoxemia marker sequence. The probe is suitably conjugated to a chromophore but may be radiolabeled. Alternatively, the probe is conjugated to a binding partner, such as an antigen-binding molecule, or biotin, and the other member of the binding pair carries a detectable moiety or reporter molecule. The techniques involved are well known to those of skill in the art and can be found in many standard texts on molecular protocols (e.g., see Sambrook *et al.,* 1989, *supra* and *Ausubel et al.* 1994, *supra).* For example, chromophore or radiolabel probes or primers identify the target during or following amplification.

In certain embodiments, target nucleic acids are quantified using blotting techniques, which are well known to those of skill in the art. Southern blotting involves the use of DNA as a target, whereas Northern blotting involves the use of RNA as a target. Each provide different types of information, although cDNA blotting is analogous, in many aspects, to blotting or RNA species. Briefly, a probe is used to target a DNA or RNA species that has been immobilized on a suitable matrix, often a filter of nitrocellulose. The different species should be spatially separated to facilitate analysis. This often is accomplished by gel electrophoresis of nucleic acid species followed by "blotting" on to the filter. Subsequently, the blotted target is incubated with a probe (usually labeled) under conditions that promote denaturation and rehybridisation. Because the probe is designed to base pair with the target, the probe will bind a portion of the target sequence under renaturing conditions. Unbound probe is then removed, and detection is accomplished as described above.

Following detection/quantification, one may compare the results seen in a given subject with a control reaction or a statistically significant reference group of normal subjects or of subjects lacking an endotoxemia-related condition. In this way, it is possible to correlate the amount of a endotoxemia marker nucleic acid detected with the progression or severity of the disease.

Also contemplated are genotyping methods and allelic discrimination methods and technologies such as those described by Kristensen et al. (Biotechniques 30(2): 318-322), including the use of single nucleotide polymorphism analysis, high performance liquid chromatography, TaqMan®, liquid chromatography, and mass spectrometry.

Also contemplated are biochip-based technologies such as those described by Hacia et al. (1996, Nature Genetics 14: 441-447) and Shoemaker et al. (1996, Nature Genetics 14: 450-456). Briefly, these techniques involve quantitative methods for analysing large numbers of genes rapidly and accurately. By tagging genes with oligonucleotides or using fixed probe arrays, one can employ biochip technology to segregate target molecules as high density arrays and screen these molecules on the basis of hybridization. See also Pease et al. (1994, Proc. Natl. Acad. Sci. U.S.A. 91: 5022-5026); Fodor et al. (1991, Science 251: 767-773). Briefly, nucleic acid probes to endotoxemia marker polynucleotides are made and attached to biochips to be used in screening and diagnostic methods, as outlined herein. The nucleic acid probes attached to the biochip are designed to be substantially complementary to specific expressed endotoxemia marker nucleic acids, i.e., the target sequence (either the target sequence of the sample or to other probe sequences, for example in sandwich assays), such that hybridization of the target sequence and the probes of the present invention occurs. This complementarity need not be perfect; there may be any number of base pair mismatches which will interfere with hybridization between the target sequence and the nucleic acid probes of the present invention. However, if the number of mismatches is so great that no hybridization can occur under even the least stringent of hybridization conditions, the sequence is not a complementary target sequence. In certain embodiments, more than one probe per sequence is used, with either overlapping probes or probes to different sections of the target being used. That is, two, three, four or more probes, with three being desirable, are used to build in a redundancy for a particular target. The probes can be overlapping (i.e. have some sequence in common), or separate.

As will be appreciated by those of ordinary skill in the art, nucleic acids can be attached to or immobilized on a solid support in a wide variety of ways. By "immobilized" and grammatical equivalents herein is meant the association or binding between the nucleic acid probe and the solid support is sufficient to be stable under the conditions of binding, washing, analysis, and removal as outlined below. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as, streptavidin to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.

In general, the probes are attached to the biochip in a wide variety of ways, as will be appreciated by those in the art. As described herein, the nucleic acids can either be synthesized first, with subsequent attachment to the biochip, or can be directly synthesized on the biochip.

The biochip comprises a suitable solid or semi-solid substrate or solid support. By "substrate" or "solid support" is meant any material that can be modified to contain discrete individual sites appropriate for the attachment or association of the nucleic acid probes and is amenable to at least one detection method. As will be appreciated by practitioners in the art, the number of possible substrates are very large, and include, but are not limited to, glass and modified or functionalised glass, plastics (including acrylics, polystyrene and copolymers of styrene and other materials, polypropylene, polyethylene, polybutylene, polyurethanes, Teflon™, etc.), polysaccharides, nylon or nitrocellulose, resins, silica or silica-based materials including silicon and modified silicon, carbon, metals, inorganic glasses, plastics, etc. In general, the substrates allow optical detection and do not appreciably fluorescese.

Generally the substrate is planar, although as will be appreciated by those of skill in the art, other configurations of substrates may be used as well. For example, the probes may be placed on the inside surface of a tube, for flow-through sample analysis to minimize sample volume. Similarly, the substrate may be flexible, such as a flexible foam, including closed cell foams made of particular plastics.

In certain embodiments, oligonucleotides probes are synthesized on the substrate, as is known in the art. For example, photoactivation techniques utilizing photopolymerisation compounds and techniques can be used. In an illustrative example, the nucleic acids are synthesized *in situ,* using well known photolithographic techniques, such as those described in WO 95/25116; WO 95/35505; U.S. Pat. Nos. 5,700,637 and 5,445,934; and references cited within; these methods of attachment form the basis of the Affymetrix GeneChip™ technology.

In an illustrative biochip analysis, oligonucleotide probes on the biochip are exposed to or contacted with a nucleic acid sample suspected of containing one or more endotoxemia polynucleotides under conditions favoring specific hybridization. Sample extracts of DNA or RNA, either single or double-stranded, may be prepared from fluid suspensions of biological materials, or by grinding biological materials, or following a cell lysis step which includes, but is not limited to, lysis effected by treatment with SDS (or other detergents), osmotic shock, guanidinium isothiocyanate and lysozyme. Suitable DNA, which may be used in the method of the invention, includes cDNA. Such DNA may be prepared by any one of a number of commonly used protocols as for example described in Ausubel, *et al.,* 1994, *supra,* and Sambrook, *et al.*, *et al.,* 1989, *supra.*

Suitable RNA, which may be used in the method of the invention, includes messenger RNA, complementary RNA transcribed from DNA (cRNA) or genomic or subgenomic RNA. Such RNA may be prepared using standard protocols as for example described in the relevant sections of Ausubel, *et al.* 1994, *supra* and Sambrook, *et al.* 1989, *supra*).

cDNA may be fragmented, for example, by sonication or by treatment with restriction endonucleases. Suitably, cDNA is fragmented such that resultant DNA fragments are of a length greater than the length of the immobilized oligonucleotide probe(s) but small enough to allow rapid access thereto under suitable hybridization conditions. Alternatively, fragments of cDNA may be selected and amplified using a suitable nucleotide amplification technique, as described for example above, involving appropriate random or specific primers.

Usually the target endotoxemia marker polynucleotides are detectably labeled so that their hybridization to individual probes can be determined. The target polynucleotides are typically detectably labeled with a reporter molecule illustrative examples of which include chromogens, catalysts, enzymes, fluorochromes, chemiluminescent molecules, bioluminescent molecules, lanthanide ions (e.g., $Eu^{34}$), a radioisotope and a direct visual label. In the case of a direct visual label, use may be made of a colloidal metallic or non-metallic particle, a dye particle, an enzyme or a substrate, an organic polymer, a latex particle, a liposome, or other vesicle containing a signal producing substance and the like. Illustrative labels of this type include large colloids, for example, metal colloids such as those from gold, selenium, silver, tin and titanium oxide. In some embodiments in which an enzyme is used as a direct visual label, biotinylated bases are incorporated into a target polynucleotide. Hybridization is detected by incubation with streptavidin-reporter molecules.

Suitable fluorochromes include, but are not limited to, fluorescein isothiocyanate (FITC), tetramethylrhodamine isothiocyanate (TRITC), R-Phycoerythrin (RPE), and Texas Red. Other exemplary fluorochromes include those discussed by *Dower et al.* (International Publication WO 93/06121). Reference also may be made to the fluorochromes described in U.S. Patents 5,573,909 (Singer et al), 5,326,692 (Brinkley et al). Alternatively, reference may be made to the fluorochromes described in U.S. Patent Nos. 5,227,487, 5,274,113, 5,405,975, 5,433,896, 5,442,045, 5,451,663, 5,453,517, 5,459,276, 5,516,864, 5,648,270 and 5,723,218. Commercially available fluorescent labels include, for example, fluorescein phosphoramidites such as Fluoreprime™ (Pharmacia), Fluoredite™ (Millipore) and FAM (Applied Biosystems International)

Radioactive reporter molecules include, for example, $^{32}P$, which can be detected by an X-ray or phosphoimager techniques.

The hybrid-forming step can be performed under suitable conditions for hybridizing oligonucleotide probes to test nucleic acid including DNA or RNA. In this regard, reference may be made, for example, to NUCLEIC ACID HYBRIDIZATION, A PRACTICAL APPROACH (Homes and Higgins, eds.) (IRL press, Washington D.C., 1985). In general, whether hybridization takes place is influenced by the length of the oligonucleotide probe and the polynucleotide sequence under test, the pH, the temperature, the concentration of mono- and divalent cations, the proportion of G and C nucleotides in the hybrid-forming region, the viscosity of the medium and the possible presence of denaturants. Such variables also influence the time required for hybridization. The preferred conditions will therefore depend upon the particular application. Such empirical conditions, however, can be routinely determined without undue experimentation.

In certain advantageous embodiments, high discrimination hybridization conditions are used. For example, reference may be made to Wallace et al. (1979, Nucl. Acids Res. 6: 3543) who describe conditions that differentiate the hybridization of 11 to 17 base long oligonucleotide probes that match perfectly and are completely homologous to a target sequence as compared to similar oligonucleotide probes that contain a single internal base pair mismatch. Reference also may be made to Wood et al. (1985, Proc. Natl. Acid. Sci. USA 82: 1585) who describe conditions for hybridization of 11 to 20 base long oligonucleotides using 3M tetramethyl ammonium chloride wherein the melting point of the hybrid depends only on the length of the oligonucleotide probe, regardless of its GC content. In addition, Drmanac *et al.* (*supra*) describe hybridization conditions that allow stringent hybridization of 6-10 nucleotide long oligomers, and similar conditions may be obtained most readily by using nucleotide analogues such as 'locked nucleic acids (Christensen et al., 2001 Biochem J354: 481-4).

Generally, a hybridization reaction can be performed in the presence of a hybridization buffer that optionally includes a hybridization-optimizing agent, such as an isostabilising agent, a denaturing agent and/or a renaturation accelerant. Examples of isostabilising agents include, but are not restricted to, betaines and lower tetraalkyl ammonium salts. Denaturing agents are compositions that lower the melting temperature of double stranded nucleic acid molecules by interfering with hydrogen bonding between bases in a double stranded nucleic acid or the hydration of nucleic acid molecules. Denaturing agents include, but are not restricted to, formamide, formaldehyde, dimethylsulfoxide, tetraethyl acetate, urea, guanidium isothiocyanate, glycerol and chaotropic salts. Hybridisation accelerants include heterogeneous nuclear ribonucleoprotein (hnRP) A1 and cationic detergents such as cetyltrimethylammonium bromide (CTAB) and dodecyl trimethylammonium bromide (DTAB), polylysine, spermine, spermidine, single stranded binding protein (SSB), phage T4 gene 32 protein and a mixture of ammonium acetate and ethanol. Hybridization buffers may include target polynucleotides at a concentration between about 0.005 nM and about 50 nM, preferably between about 0.5 nM and 5 nM, more preferably between about 1 nM and 2 nM.

A hybridization mixture containing the target endotoxemia marker polynucleotides is placed in contact with the array of probes and incubated at a temperature and for a time appropriate to permit hybridization between the target sequences in the target polynucleotides and any complementary probes. Contact can take place in any suitable container, for example, a dish or a cell designed to hold the solid support on which the probes are bound. Generally, incubation will be at temperatures normally used for hybridization of nucleic acids, for example, between about 20° C and about 75° C, example, about 25° C, about 30° C, about 35° C, about 40° C, about 45° C, about 50° C, about 55° C, about 60° C, or about 65° C. For probes longer than 14 nucleotides, 20° C to 50° C is desirable. For shorter probes, lower temperatures are preferred. A sample of target polynucleotides is incubated with the probes for a time sufficient to allow the desired level of hybridization between the target sequences in the target polynucleotides and any complementary probes. For example, the hybridization may be carried out at about 45° C +/-10° C in formamide for 1-2 days.

After the hybrid-forming step, the probes are washed to remove any unbound nucleic acid with a hybridization buffer, which can typically comprise a hybridization optimizing agent in the same range of concentrations as for the hybridization step. This washing step leaves only bound target polynucleotides. The probes are then examined to identify which probes have hybridized to a target polynucleotide.

The hybridization reactions are then detected to determine which of the probes has hybridized to a corresponding target sequence. Depending on the nature of the reporter molecule associated with a target polynucleotide, a signal may be instrumentally detected by irradiating a fluorescent label with light and detecting fluorescence in a fluorimeter; by providing for an enzyme system to produce a dye which could be detected using a spectrophotometer; or detection of a dye particle or a colored colloidal metallic or non metallic particle using a reflectometer; in the case of using a radioactive label or chemiluminescent molecule employing a radiation counter or autoradiography. Accordingly, a detection means may be adapted to detect or scan light associated with the label which light may include fluorescent, luminescent, focussed beam or laser light. In such a case, a charge couple device (CCD) or a photocell can be used to scan for emission of light from a probe:target polynucleotide hybrid from each location in the micro-array and record the data directly in a digital computer. In some cases, electronic detection of the signal may not be necessary. For example, with enzymatically generated color spots associated with nucleic acid array format, visual examination of the array will allow interpretation of the pattern on the array. In the case of a nucleic acid array, the detection means is suitably interfaced with pattern recognition software to convert the pattern of signals from the array into a plain language genetic profile. In certain embodiments, oligonucleotide probes specific for different endotoxemia marker gene products are in the form of a nucleic acid array and detection of a signal generated from a reporter molecule on the array is performed using a 'chip reader'. A detection system that can be used by a 'chip reader' is described for example by Pirrung et al (U.S. Patent No. 5,143,854). The chip reader will typically also incorporate some signal processing to determine whether the signal at a particular array position or feature is a true positive or maybe a spurious signal. Exemplary chip readers are described for example by Fodor et al (U.S. Patent No., 5,925,525). Alternatively, when the array is made using a mixture of individually addressable kinds of labeled microbeads, the reaction may be detected using flow cytometry.

7.2 Protein-based diagnostic

Consistent with the present invention, the presence of an aberrant concentration of an endotoxemia marker protein is indicative of the presence, degree, or stage of development of an endotoxemia-related condition. Endotoxemia marker protein levels in biological samples can be assayed using any suitable method known in the art. For example, when an endotoxemia marker protein is an enzyme, the protein can be quantified based upon its catalytic activity or based upon the number of molecules of the protein contained in a sample. Antibody-based techniques may be employed, such as, for example, immunohistological and immunohistochemical methods for measuring the level of a protein of interest in a tissue sample. For example, specific recognition is provided by a primary antibody (polyclonal or monoclonal) and a secondary detection system is used to detect presence (or binding) of the primary antibody. Detectable labels can be conjugated to the secondary antibody, such as a fluorescent label, a radiolabel, or an enzyme (e.g., alkaline phosphatase, horseradish peroxidase) which produces a quantifiable, e.g., coloured, product. In another suitable method, the primary antibody itself can be detectably labeled. As a result, immunohistological labeling of a tissue section is provided. In some embodiments, a protein extract is produced from a biological sample (e.g., tissue, cells) for analysis. Such an extract (e.g., a detergent extract) can be subjected to western-blot or dot/slot assay of the level of the protein of interest, using routine immunoblotting methods (Jalkanen et al., 1985, J. Cell. Biol. 101: 976-985; Jalkanen et al., 1987, J. Cell. Biol. 105: 3087-3096).

Other useful antibody-based methods include immunoassays, such as the enzyme-linked immunosorbent assay (ELISA) and the radioimmunoassay (RIA). For example, a protein-specific monoclonal antibody, can be used both as an immunoadsorbent and as an enzyme-labeled probe to detect and quantify an endotoxemia marker protein of interest. The amount of such protein present in a sample can be calculated by reference to the amount present in a standard preparation using a linear regression computer algorithm (see Lacobilli et al., 1988, Breast Cancer Research and Treatment 11: 19-30). In other embodiments, two different monoclonal antibodies to the protein of interest can be employed, one as the immunoadsorbent and the other as an enzyme-labeled probe.

Additionally, recent developments in the field of protein capture arrays permit the simultaneous detection and/or quantification of a large number of proteins. For example, low-density protein arrays on filter membranes, such as the universal protein array system (Ge, 2000 Nucleic Acids Res. 28(2):e3) allow imaging of arrayed antigens using standard ELISA techniques and a scanning charge-coupled device (CCD) detector. Immunosensor arrays have also been developed that enable the simultaneous detection of clinical analytes. It is now possible using protein arrays, to profile protein expression in bodily fluids, such as in sera of healthy or diseased subjects, as well as in subjects pre- and post-drug treatment.

Protein capture arrays typically comprise a plurality of protein-capture agents each of which defines a spatially distinct feature of the array. The protein-capture agent can be any molecule or complex of molecules which has the ability to bind a protein and immobilize it to the site of the protein-capture agent on the array. The protein-capture agent may be a protein whose natural function in a cell is to specifically bind another protein, such as an antibody or a receptor. Alternatively, the protein-capture agent may instead be a partially or wholly synthetic or recombinant protein which specifically binds a protein. Alternatively, the protein-capture agent may be a protein which has been selected *in vitro* from a mutagenized, randomized, or completely random and synthetic library by its binding affinity to a specific protein or peptide target. The selection method used may optionally have been a display method such as ribosome display or phage display, as known in the art. Alternatively, the protein-capture agent obtained *via in vitro* selection may be a DNA or RNA aptamer which specifically binds a protein target (see, e.g., Potyrailo et al., 1998 Anal. Chem. 70:3419-3425; Cohen et al., 1998, Proc. Natl. Acad. Sci. USA 95:14272-14277; Fukuda, et al., 1997 Nucleic Acids Symp. Ser. 37: 237-238; available from SomaLogic). For example, aptamers are selected from libraries of oligonucleotides by the Selex™ process and their interaction with protein can be enhanced by covalent attachment, through incorporation of brominated deoxyuridine and UV-activated crosslinking (photoaptamers). Aptamers have the advantages of ease of production by automated oligonucleotide synthesis and the stability and robustness of DNA; universal fluorescent protein stains can be used to detect binding. Alternatively, the *in vitro* selected protein-capture agent may be a polypeptide (e.g., an antigen) (see, e.g., Roberts and Szostak, 1997 Proc. Natl. Acad. Sci. USA, 94:12297-12302).

An alternative to an array of capture molecules is one made through 'molecular imprinting' technology, in which peptides (e.g., from the C-terminal regions of proteins) are used as templates to generate structurally complementary, sequence-specific cavities in a polymerisable matrix; the cavities can then specifically capture (denatured) proteins which have the appropriate primary amino acid sequence (e.g., available from ProteinPrint™ and Aspira Biosystems).

Exemplary protein capture arrays include arrays comprising spatially addressed antigen-binding molecules, commonly referred to as antibody arrays, which can facilitate extensive parallel analysis of numerous proteins defining a proteome or subproteome. Antibody arrays have been shown to have the required properties of specificity and acceptable background, and some are available commercially (e.g., BD Biosciences, Clontech, BioRad and Sigma). Various methods for the preparation of antibody arrays have been reported (see, e.g., Lopez et al., 2003 J. Chromatogr. B 787: 19-27; Cahill, 2000 Trends in Biotechnology 7:47-51; U.S. Pat. App. Pub. 2002/0055186; U.S. Pat. App. Pub. 2003/0003599; PCT publication WO 03/062444; PCT publication WO 03/077851; PCT publication WO 02/59601; PCT publication WO 02/39120; PCT publication WO 01/79849; PCT publication WO 99/39210). The antigen-binding molecules of such arrays may recognise at least a subset of proteins expressed by a cell or population of cells, illustrative examples of which include growth factor receptors, hormone receptors, neurotransmitter receptors, catecholamine receptors, amino acid derivative receptors, cytokine receptors, extracellular matrix receptors, antibodies, lectins, cytokines, serpins, proteases, kinases, phosphatases, ras-like GTPases, hydrolases, steroid hormone receptors, transcription factors, heat-shock transcription factors, DNA-binding proteins, zinc-finger proteins, leucine-zipper proteins, homeodomain proteins, intracellular signal transduction modulators and effectors, apoptosis-related factors, DNA synthesis factors, DNA repair factors, DNA recombination factors, cell-surface antigens, hepatitis C virus (HCV) proteases and HIV proteases.

Antigen-binding molecules for antibody arrays are made either by conventional immunization (e.g., polyclonal sera and hybridomas), or as recombinant fragments, usually expressed in *E. coli,* after selection from phage display or ribosome display libraries (e.g., available from Cambridge Antibody Technology, BioInvent, Affitech and Biosite). Alternatively, 'combibodies' comprising non-covalent associations ofVH and VL domains, can be produced in a matrix format created from combinations of diabody-producing bacterial clones (e.g., available from Domantis). Exemplary antigen-binding molecules for use as protein-capture agents include monoclonal antibodies, polyclonal antibodies, Fv, Fab, Fab' and $F(ab')_2$ immunoglobulin fragments, synthetic stabilized Fv fragments, e.g., single chain Fv fragments (scFv), disulfide stabilized Fv fragments (dsFv), single variable region domains (dAbs) minibodies, combibodies and multivalent antibodies such as diabodies and multi-scFv, single domains from camelids or engineered human equivalents.

Individual spatially distinct protein-capture agents are typically attached to a support surface, which is generally planar or contoured. Common physical supports include glass slides, silicon, microwells, nitrocellulose or PVDF membranes, and magnetic and other microbeads.

While microdrops of protein delivered onto planar surfaces are widely used, related alternative architectures include CD centrifugation devices based on developments in microfluidics (e.g., available from Gyros) and specialized chip designs, such as engineered microchannels in a plate (e.g., The Living Chip™, available from Biotrove) and tiny 3D posts on a silicon surface (e.g., available from Zyomyx).

Particles in suspension can also be used as the basis of arrays, providing they are coded for identification; systems include color coding for microbeads (e.g., available from Luminex, Bio-Rad and Nanomics Biosystems) and semiconductor nanocrystals (e.g., QDots™, available from Quantum Dots), and barcoding for beads (UltraPlex™, available from Smartbeads) and multimetal microrods (Nanobarcodes™ particles, available from Surromed). Beads can also be assembled into planar arrays on semiconductor chips (e.g., available from LEAPS technology and BioArray Solutions). Where particles are used, individual protein-capture agents are typically attached to an individual particle to provide the spatial definition or separation of the array. The particles may then be assayed separately, but in parallel, in a compartmentalized way, for example in the wells of a microtiter plate or in separate test tubes.

In operation, a protein sample, which is optionally fragmented to form peptide fragments (see, e.g., U.S. Pat. App. Pub. 2002/0055186), is delivered to a protein-capture array under conditions suitable for protein or peptide binding, and the array is washed to remove unbound or non-specifically bound components of the sample from the array. Next, the presence or amount of protein or peptide bound to each feature of the array is detected using a suitable detection system. The amount of protein bound to a feature of the array may be determined relative to the amount of a second protein bound to a second feature of the array. In certain embodiments, the amount of the second protein in the sample is already known or known to be invariant.

For analyzing differential expression of proteins between two cells or cell populations, a protein sample of a first cell or population of cells is delivered to the array under conditions suitable for protein binding. In an analogous manner, a protein sample of a second cell or population of cells to a second array, is delivered to a second array which is identical to the first array. Both arrays are then washed to remove unbound or non-specifically bound components of the sample from the arrays. In a final step, the amounts of protein remaining bound to the features of the first array are compared to the amounts of protein remaining bound to the corresponding features of the second array. To determine the differential protein expression pattern of the two cells or populations of cells, the amount of protein bound to individual features of the first array is subtracted from the amount of protein bound to the corresponding features of the second array.

In an illustrative example, fluorescence labeling can be used for detecting protein bound to the array. The same instrumentation as used for reading DNA microarrays is applicable to protein-capture arrays. For differential display, capture arrays (e.g. antibody arrays) can be probed with fluorescently labeled proteins from two different cell states, in which cell lysates are labeled with different fluorophores (e.g., Cy-3 and Cy-5) and mixed, such that the color acts as a readout for changes in target abundance. Fluorescent readout sensitivity can be amplified 10-100 fold by tyramide signal amplification (TSA) (e.g., available from PerkinElmer Lifesciences). Planar waveguide technology (e.g., available from Zeptosens) enables ultrasensitive fluorescence detection, with the additional advantage of no washing procedures. High sensitivity can also be achieved with suspension beads and particles, using phycoerythrin as label (e.g., available from Luminex) or the properties of semiconductor nanocrystals (e.g., available from Quantum Dot). Fluorescence resonance energy transfer has been adapted to detect binding of unlabelled ligands, which may be useful on arrays (e.g., available from Affibody). Several alternative readouts have been developed, including adaptations of surface plasmon resonance (e.g., available from HTS Biosystems and Intrinsic Bioprobes), rolling circle DNA amplification (e.g., available from Molecular Staging), mass spectrometry (e.g., available from Sense Proteomic, Ciphergen, Intrinsic and Bioprobes), resonance light scattering (e.g., available from Genicon Sciences) and atomic force microscopy (e.g., available from BioForce Laboratories). A microfluidics system for automated sample incubation with arrays on glass slides and washing has been co-developed by NextGen and Perkin Elmer Life Sciences.

In certain embodiments, the techniques used for detection of endotoxemia marker expression products will include internal or external standards to permit quantitative or semi-quantitative determination of those products, to thereby enable a valid comparison of the level or functional activity of these expression products in a biological sample with the corresponding expression products in a reference sample or samples. Such standards can be determined by the skilled practitioner using standard protocols. In specific examples, absolute values for the level or functional activity of individual expression products are determined.

In specific embodiments, the diagnostic method is implemented using a system as disclosed, for example, in International Publication No. WO 02/090579 and in copending PCT Application No. PCT/AU03/01517 filed November 14, 2003, comprising at least one end station coupled to a base station. The base station is typically coupled to one or more databases comprising predetermined data from a number of individuals representing the level or functional activity of endotoxemia marker expression products, together with indications of the actual status of the individuals (e.g., presence, absence, degree, or stage of development of an endotoxemia-related condition) when the predetermined data was collected. In operation, the base station is adapted to receive from the end station, typically *via* a communications network, subject data representing a measured or normalized level or functional activity of at least one expression product in a biological sample obtained from a test subject and to compare the subject data to the predetermined data stored in the database(s). Comparing the subject and predetermined data allows the base station to determine the status of the subject in accordance with the results of the comparison. Thus, the base station attempts to identify individuals having similar parameter values to the test subject and once the status has been determined on the basis of that identification, the base station provides an indication of the diagnosis to the end station.

7.3 Kits

All the essential materials and reagents required for detecting and quantifying endotoxemia maker gene expression products may be assembled together in a kit. The kits may also optionally include appropriate reagents for detection of labels, positive and negative controls, washing solutions, blotting membranes, microtiter plates dilution buffers and the like. For example, a nucleic acid-based detection kit may include (i) an endotoxemia marker polynucleotide (which may be used as a positive control), (ii) a primer or probe that specifically hybridizes to an endotoxemia marker polynucleotide. Also included may be enzymes suitable for amplifying nucleic acids including various polymerases (Reverse Transcriptase, Taq, Sequenase™ DNA ligase etc. depending on the nucleic acid amplification technique employed), deoxynucleotides and buffers to provide the necessary reaction mixture for amplification. Such kits also generally will comprise, in suitable means, distinct containers for each individual reagent and enzyme as well as for each primer or probe. Alternatively, a protein-based detection kit may include (i) an endotoxemia marker polypeptide (which may be used as a positive control), (ii) an antigen-binding molecule that is immuno-interactive with an endotoxemia marker polynucleotide. The kit can also feature various devices and reagents for performing one of the assays described herein; and/or printed instructions for using the kit to quantify the expression of an endotoxemia marker gene.

***8. Methods of treatment or prophylaxis***

The present invention also extends to the management of endotoxaemia-related conditions, or prevention of further progression of endotoxaemia-related conditions, or assessment of the efficacy of therapies in subjects following positive diagnosis for the presence, or stage of endotoxaemia-related conditions in the subjects. Generally, the management of endotoxaemia-related conditions is highly intensive and can include identification and amelioration of the underlying cause and aggressive use of therapeutic compounds such as, vasoactive compounds, antibiotics, steroids, antibodies to endotoxin, and anti tumour necrosis factor agents. In addition, palliative therapies[1] aimed at restoring and protecting organ function can be used such as intravenous fluids and oxygen.

[1] Cohen J & Glauser MP. Lancet 338: 736-739 (1991).

Typically, the therapeutic agents will be administered in pharmaceutical (or veterinary) compositions together with a pharmaceutically acceptable carrier and in an effective amount to achieve their intended purpose. The dose of active compounds administered to a subject should be sufficient to achieve a beneficial response in the subject over time such as a reduction in, or relief from, the symptoms of endotoxaemia. The quantity of the pharmaceutically active compounds(s) to be administered may depend on the subject to be treated inclusive of the age, sex, weight and general health condition thereof. In this regard, precise amounts of the active compound(s) for administration will depend on the judgement of the practitioner. In determining the effective amount of the active compound(s) to be administered in the treatment or prevention of endotoxaemia, the medical practitioner or veterinarian may evaluate severity of any symptom associated with the presence of endotoxaemia including tachycardia, fever, chills, vomiting, diarrhoea, skin rash, headaches, confusion, muscle aches, seizures. In any event, those of skill in the art may readily determine suitable dosages of the therapeutic agents and suitable treatment regimens without undue experimentation.

The therapeutic agents may by administered in concert with adjunctive (palliative) therapies to increase oxygen supply to major organs, increase blood flow to major organs and/or to reduce the inflammatory response. Illustrative examples of such adjunctive therapies include non steroidal-anti inflammatory drugs (NSAIDs), intravenous saline and oxygen.

In order that the invention may be readily understood and put into practical effect, particular preferred embodiments will now be described by way of the following nonlimiting examples.

**EXAMPLES**

EXAMPLE 1

**IDENTIFICATION OF SPECIFIC DIAGNOSTIC GENES FOR ENDOTOXAEMIA-RELATED**

**CONDITIONS**

***Experimental Disease Trial Design***

A clinical trial was performed on three blocks of fours horses each. The first block consisted of four horses that were dosed orally with 12.5 mg/kg of oligofructose[2] as part of the trial procedure described by Pollitt[3] which is specifically designed to induce endotoxaemia and subsequent acute laminitis. The second block consisted of four horses that underwent the same trial procedure but were dosed with normal saline (0.9%) solution (controls). The same four horses in the second block then underwent the trial procedure (following a period of recovery) for a second time but were dosed with oligofructose (block three). All horses were stalled under the same conditions for the duration of the procedure (120 hours).

[2] Raftilose®, Orafti Active Food Ingredients, Aanndorenstraat, B-3300 Tienen, Belgium.

[3] van Eps A & Pollitt CC. Equine Vet J. 36(3):255-60 (2004).

Endotoxaemia-related conditions in horses (including laminitis) can be induced experimentally and one of the more reliable methods of induction is by carbohydrate overload through oral dosing with oligofructose.

Blood samples were collected at four time points - Hour 0 prior to dosing and at hours 24, 48, and 72 hours after dosing. The sample at Hour 0 acted as a control for each horse.

The following tests and observations were undertaken at all of the above time points:

(i) physical examination, rectal temperature, digital pulse, hoof temperature, heart and respiratory rate, faecal pH, hoof shifting; and

(ii) haematology and biochemistry.

Blood samples from each of the animals on Hours 0, 24, 48 and 72 of the trial were analysed using GeneChips™ (method of use is described below in detail in "Generation of Gene Expression Data") containing thousands of genes expressed in white blood cells of horses. Analysis of these data (see "Identification of Diagnostic Marker Genes" below) reveals a number of specific genes that differ in expression between animals before and after experimental induction of endotoxaemia and laminitis from Hour 24 following dosing. It is possible to design an assay that measures the RNA level in the sample from the expression of at least one and desirably at least two endotoxaemia marker genes, representative transcript sequences of which are set forth in SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325.

## ***Materials and Methods***

### Blood Collection

Blood is collected from a horse (in a non-agitated state) for the purpose of extraction of high quality RNA or protein. Suitable blood collection tubes for the collection, preservation, transport and isolation of RNA include PAXgene™ tubes (PreAnalytix Inc., Valencia, CA, USA). Alternatively, blood can be collected into tubes containing solutions designed for the preservation of nucleic acids (available from Roche, Ambion, Invitrogen and ABI). For the determination of protein levels, 50 mL of blood is prevented from clotting by collection into a tube containing 4 mL of 4% sodium citrate. White blood cells and plasma are isolated and stored frozen for later analysis and detection of specific proteins. PAXgene tubes can be kept at room temperature prior to RNA extraction. Clinical signs are recorded in a standard format.

### Total RNA Extraction

A kit available from Qiagen Inc (Valencia, CA, USA) has the reagents and instructions for the isolation of total RNA from 2.5 mL blood collected in the PAXgene Blood RNA Tube. Isolation begins with a centrifugation step to pellet nucleic acids in the PAXgene blood RNA tube. The pellet is washed and resuspended and incubated in optimized buffers together with Proteinase K to bring about protein digestion. An additional centrifugation is carried out to remove residual cell debris and the supernatant is transferred to a fresh microcentrifuge tube. Ethanol is added to adjust binding conditions, and the lysate is applied to the PAXgene RNA spin column. During brief centrifugation, RNA is selectively bound to the silica-gel membrane as contaminants pass through. Remaining contaminants are removed in three efficient wash steps and RNA is then eluted in Buffer BR5.

Determination of RNA quantity and quality is necessary prior to proceeding and can be achieved using an Agilent Bioanalyzer and Absorbance 260/280 ratio using a spectrophotometer.

Generation of Gene Expression Data

*Choice of Method*

Measurement of specific RNA levels in a tissue sample can be achieved using a variety of technologies. Two common and readily available technologies that are well known in the art are:

● GeneChip® analysis using Affymetrix technology.

● Real-Time Polymerase Chain Reaction (TaqMan™ from Applied Biosystems for example).

GeneChips® quantitate RNA by detection of labeled cRNA hybridized to short oligonucleotides built on a silicon substrate. Details on the technology and methodology can be found at www.affymetrix.com.

Real-Time Polymerase Chain Reaction (RT-PCR) quantitates RNA using two PCR primers, a labeled probe and a thermostable DNA polymerase. As PCR product is generated a dye is released into solution and detected. Internal controls such as 18S RNA probes are often used to determine starting levels of total RNA in the sample. Each gene and the internal control are run separately. Details on the technology and methods can be found at www.appliedbiosytems.com or www.qiagen.com or www.biorad..com. Applied Biosystems offer a service whereby the customer provides DNA sequence information and payment and is supplied in return all of the reagents required to perform RT-PCR analysis on individual genes.

GeneChip® analysis has the advantage of being able to analyze thousands of genes at a time. However it is expensive and takes over 3 days to perform a single assay. RT-PCR generally only analyses one gene at a time, but is inexpensive and can be completed within a single day.

RT-PCR is the method of choice for gene expression analysis if the number of specific genes to be analyzed is less than 20. GeneChip® or other gene expression analysis technologies (such as Illumina Bead Arrays) are the method of choice when many genes need to be analyzed simultaneously.

The methodology for GeneChip® data generation and analysis and Real Time PCR is presented below in brief.

GeneChip® Data Generation

*cDNA & cRNA Generation*

The following method for cDNA and cRNA generation from total RNA has been adapted from the protocol provided and recommended by Affymetrix (www.affymetrix.com).

The steps are:

● A total of 3 μg of total RNA is used as a template to generate double stranded cDNA.

● cRNA is generated and labeled using biotinylated Uracil (dUTP).

● biotin-labeled cRNA is cleaned and the quantity determined using a spectrophotometer and MOPS gel analysis.

● labeled cRNA is fragmented to ~ 300bp in size.

● RNA quantity is determined on an Agilent "Lab-on-a-Chip" system (Agilent Technologies).

*Hybridization, Washing & Staining*

The steps are:

● A hybridization cocktail is prepared containing 0.05 μg/μL of labeled and fragmented cRNA, spike-in positive hybridization controls, and the Affymetrix oligonucleotides B2, bioB, bioC, bioD and cre.

● The final volume (80 μL) of the hybridization cocktail is added to the GeneChip® cartridge.

● The cartridge is placed in a hybridization oven at constant rotation for 16 hours.

● The fluid is removed from the GeneChip® and stored.

● The GeneChip® is placed in the fluidics station.

● The experimental conditions for each GeneChip® are recorded as an .EXP file.

● All washing and staining procedures are carried out by the Affymetrix fluidics station with an attendant providing the appropriate solutions.

● The GeneChip® is washed, stained with steptavidin-phycoerythin dye and then washed again using low salt solutions.

● After the wash protocols are completed, the dye on the probe array is 'excited' by laser and the image captured by a CCD camera using an Affymetrix Scanner (manufactured by Agilent).

*Scanning & Data File Generation*

The scanner and MAS 5 software generates an image file from a single GeneChip® called a .DAT file (see figure overleaf).
The .DAT file is then pre-processed prior to any statistical analysis.
Data pre-processing steps (prior to any statistical analysis) include:
● DAT File Quality Control (QC).
● CEL File Generation.
● Scaling and Normalization.

*.DAT File Quality Control*

The .DAT file is an image. The image is inspected manually for artifacts (e.g. high/low intensity spots, scratches, high regional or overall background). (The B2 oligonucleotide hybridization performance is easily identified by an alternating pattern of intensities creating a border and array name.) The MAS 5 software used the B2 oligonucleotide border to align a grid over the image so that each square of oligonucleotides was centered and identified.
The other spiked hybridization controls (bioB, bioC, bioD and cre) are used to evaluate sample hybridization efficiency by reading "present" gene detection calls with increasing signal values, reflecting their relative concentrations. (If the .DAT file is of suitable quality it is converted to an intensity data file (.CEL file) by Affymetrix MAS 5 software).

*.CEL File Generation*

The .CEL files generated by the MAS 5 software from .DAT files contain calculated raw intensities for the probe sets. Gene expression data is obtained by subtracting a calculated background from each cell value. To eliminate negative intensity values, a noise correction fraction based from a local noise value from the standard deviation of the lowest 2% of the background is applied.
All .CEL files generated from the GeneChips® are subjected to specific quality metrics parameters.
Some metrics are routinely recommended by Affymetrix and can be determined from Affymetrix internal controls provided as part of the GeneChip®. Other metrics are based on experience and the processing of many GeneChips®.

Analysis of GeneChip® Data

Two illustrative approaches to normalising data may be used:
● Affymetrix MAS 5 Algorithm.
● Robust Multi-chip Analysis (RMA) algorithm of Irizarry (Irizarray et al., 2002, Biostatistics (in print)).
Those of skill in the art will recognise that many other approaches might be adopted, without materially affecting the invention.

*Affymetrix MAS 5 Algorithm*

.CEL files are used by Affymetrix MAS 5 software to normalize or scale the data. Scaled data from one chip are compared to similarly scaled data from other chips.
Affymetrix MAS 5 normalization is achieved by applying the default "Global Scaling" option of the MAS 5 algorithm to the .CEL files. This procedure subtracts a robust estimate of the center of the distribution of probe values, and divides by a robust estimate of the probe variability. This produces a set of chips with common location and scale at the probe level.
Gene expression indices are generated by a robust averaging procedure on all the probe pairs for a given gene. The results are constrained to be non-negative.
Given that scaling takes place at the level of the probe, rather than at the level of the gene, it is possible that even after normalization there may be chip-to-chip differences in overall gene expression level. Following standard MAS5 normalization, values for each gene were de-trended with respect to median chip intensity. That is, values for each gene were regressed on the median chip intensity, and residuals were calculated. These residuals were taken as the de-trended estimates of expression for each gene
Median chip intensity was calculated using the Affymetrix MAS5 algorithm, but with a scale factor fixed at one.

*RMA Algorithm*

This algorithm quantifies the expression of a set of chips, rather than of a single chip. It estimates background intensities using a robust statistical model applied to perfect match probe data. It does not make use of mis-match probe data. Following implicit background correction, chips are processed using Quantile Quantile normalization (Rizarray et al., 2002, Biostatistics (in print)).

DNA Extraction

A kit available from Qiagen Inc (Valencia, CA, USA) has the reagents and instructions for the isolation of total DNA from 8.5 mL blood collected in the PAXgene Blood DNA Tube. Isolation begins with the addition of additional lysis solution followed by a centrifugation step. The pellet is washed and resuspended and incubated in optimized buffers together with Proteinase K to bring about protein digestion. DNA is precipitated using alcohol and an additional centrifugation is carried out to pellet the nucleic acid. Remaining contaminants are removed in a wash step and the DNA is then resuspended in Buffer BG4.

Determination of DNA quantity and quality is necessary prior to proceeding and can be achieved using a spectrophotometer or agarose gel electrophoresis.

Genotyping Analysis

Many methods are available to genotype DNA. A review of allelic discrimination methods can be found in Kristensen et al. (Biotechniques 30(2): 318-322 (2001). An illustrative method for genotyping using allele-specific PCR is described here.

Primer Design

Upstream and downstream PCR primers specific for particular alleles can be designed using freely available computer programs, such as Primer3 (http://frodo.wi.mit.edu/primer3/primer3_code.html). Alternatively the DNA sequences of the various alleles can be aligned using a program such as ClustalW (http://www.ebi.ac.uklclustalw/) and specific primers designed to areas where DNA sequence differences exist but retaining enough specificity to ensure amplification of the correct amplicon. Preferably a PCR amplicon is designed to have a restriction enzyme site in one allele but not the other. Primers are generally 18-25 base pairs in length with similar melting temperatures.

PCR Amplification

The composition of PCR reactions has been described elsewhere (Clinical Applications of PCR, Dennis Lo (Editor), Blackwell Publishing, 1998). Briefly, a reaction contains primers, DNA, buffers and a thermostable polymerase enzyme. The reaction is cycled (up to 50 times) through temperature steps of denaturation, hybridization and DNA extension on a thermocycler such as the MJ Research Thermocycler model PTC-96V.

DNA Analysis

PCR products can be analyzed using a variety of methods including size differentiation using mass spectrometry, capillary gel electrophoresis and agarose gel electrophoresis. If the PCR amplicons have been designed to contain differential restriction enzyme sites, the DNA in the PCR reaction is purified using DNA-binding columns or precipitation and re-suspended in water, and then restricted using the appropriate restriction enzyme. The restricted DNA can then be run on an agarose gel where DNA is separated by size using electric current. Various alleles of a gene will have different sizes depending on whether they contain restriction sites. Thus, homozygotes and heterozygotes can be determined.

Real-Time PCR Data Generation

Background information for conducting Real-time PCR may be obtained, for example, at http://dorakmt.tripod.com/genetics/realtime.html and in a review by Bustin SA (2000, J Mol Endocrinol 25:169-193).

*TaqMan™ Primer and Probe Design Guidelines:*

1. The Primer Express™ (ABI) software designs primers with a melting temperature (Tm) of 58-60° C, and probes with a Tm value of 10° C higher. The Tm of both primers should be equal.

2. Primers should be 15-30 bases in length.

3. The G+C content should ideally be 30-80%. If a higher G+C content is unavoidable, the use of high annealing and melting temperatures, cosolvents such as glycerol, DMSO, or 7-deaza-dGTP may be necessary.

4. The run of an identical nucleotide should be avoided. This is especially true for G, where runs of four or more Gs is not allowed.

5. The total number of Gs and Cs in the last five nucleotides at the 3' end of the primer should not exceed two (the newer version of the software has an option to do this automatically). This helps to introduce relative instability to the 3' end of primers to reduce non-specific priming. The primer conditions are the same for SYBR Green assays.

6. Maximum amplicon size should not exceed 400 bp (ideally 50-150 bases). Smaller amplicons give more consistent results because PCR is more efficient and more tolerant of reaction conditions (the short length requirement has nothing to do with the efficiency of 5' nuclease activity).

7. The probes should not have runs of identical nucleotides (especially four or more consecutive Gs), G+C content should be 30-80%, there should be more Cs than Gs, and not a G at the 5' end. The higher number of Cs produces a higher ΔRn. The choice of probe should be made first.

8. To avoid false-positive results due to amplification of contaminating genomic DNA in the cDNA preparation, it is preferable to have primers spanning exon-exon junctions. This way, genomic DNA will not be amplified (the PDAR kit for human GAPDH amplification has such primers),

9. If a TaqMan™ probe is designed for allelic discrimination, the mismatching nucleotide (the polymorphic site) should be in the middle of the probe rather than at the ends,

10. Use primers that contain dA nucleotides near the 3' ends so that any primer-dimer generated is efficiently degraded by AmpErase™ UNG (mentioned in p.9 of the manual for EZ RT-PCR kit; P/N 402877). If primers cannot be selected with dA nucleotides near the ends, the use of primers with 3' terminal dU-nucleotides should be considered.

(See also the general principles of PCR Primer Design by InVitroGen.)

*General Method:*

1. Reverse transcription of total RNA to cDNA should be done with random hexamers (not with oligo-dT). If oligo-dT has to be used long mRNA transcripts or amplicons greater than two kilobases upstream should be avoided, and 18S RNA cannot be used as normalizer,

2. Multiplex PCR will only work properly if the control primers are limiting (ABI control reagents do not have their primers limited),

3. The range of target cDNA used is 10 ng to 1 μg. If DNA is used (mainly for allelic discrimination studies), the optimum amount is 100 ng to 1 μg,

4. It is ideal to treat each RNA preparation with RNAse free DNAse to avoid genomic DNA contamination. Even the best RNA extraction methods yield some genomic DNA. Of course, it is ideal to have primers not amplifying genomic DNA at all but sometimes this may not be possible,

5. For optimal results, the reagents (before the preparation of the PCR mix) and the PCR mixture itself (before loading) should be vortexed and mixed well. Otherwise there may be shifting Rn value during the early (0 - 5) cycles of PCR. It is also important to add probe to the buffer component and allow it to equilibrate at room temperature prior to reagent mix formulation.

*TaqMan™ Primers and Probes:*

The TaqMan™ probes ordered from ABI at midi-scale arrive already resuspended at 100 μM. If a 1/20 dilution is made, this gives a 5 μM solution. This stock solution should be aliquoted, frozen and kept in the dark. Using 1 μL of this in a 50 μL reaction gives the recommended 100 nM final concentration.

The primers arrive lyophilized with the amount given on the tube in pmols (such as 150.000 pmol which is equal to 150 nmol). If X nmol of primer is resuspended in X μL of $H_2O$, the resulting solution is 1 mM. It is best to freeze this stock solution in aliquots. When the 1 mM stock solution is diluted 1/100, the resulting working solution will be 10 μM. To get the recommended 50 - 900 nM final primer concentration in 50 μL reaction volume, 0.25 - 4.50 μL should be used per reaction (2.5 μL for 500 nM final concentration).

The PDAR primers and probes are supplied as a mix in one tube. They have to be used 2.5 μL in a 50 μL reaction volume.

*Setting up One-step TaqMan™ Reaction:*

One-step real-time PCR uses RNA (as opposed to cDNA) as a template. This is the preferred method if the RNA solution has a low concentration but only if singleplex reactions are run. The disadvantage is that RNA carryover prevention enzyme AmpErase cannot be used in one-step reaction format. In this method, both reverse transcriptase and real-time PCR take place in the same tube. The downstream PCR primer also acts as the primer for reverse transcriptase (random hexamers or oligo-dT cannot be used for reverse transcription in one-step RT-PCR). One-step reaction requires higher dNTP concentration (greater than or equal to 300 mM vs 200 mM) as it combines two reactions needing dNTPs in one. A typical reaction mix for one-step PCR by Gold RT-PCR kit is as follows:

| Reagents | Volume |
|---|---|
| $H_2O$ + RNA : | 20.5 μL [24 μL ifPDAR is used] |
| 10X TaqMan buffer : | 5.0 μL |
| $MgCl_2$ (25 mM) : | 11.0 μL |
| dATP (10mM) : | 1.5 μL [for final concentration of 300 μM] |
| dCTP (10mM) : | 1.5 μL [for final concentration of 300 μM] |
| dGTP (10mM): | 1.5 μL [for final concentration of 300 μM] |
| dUTP (20mM): | 1.5 μL [for final concentration of 600 μM] |
| Primer F (10 μM)* : | 2.5 μL [for final concentration of 500 nM] |
| Primer R (10 μM)* : | 2.5 μL [for final concentration of 500 nM] |
| TaqMan Probe * : | 1.0 μL [for final concentration of 100 nM] |
| AmpliTaq Gold: | 0.25 μL [can be increased for higher efficiency] |
| Reverse Transcriptase : | 0.25 μL |
| RNAse inhibitor: | 1.00 μL |
| * If a PDAR is used, 2.5 μL of primer + probe mix used. | |

Ideally 10 pg - 100 ng RNA should be used in this reaction. Note that decreasing the amount of template from 100 ng to 50 ng will increase the $C_T$ value by 1. To decrease a $C_T$ value by 3, the initial amount of template should be increased 8-fold. ABI claims that 2 picograms of RNA can be detected by this system and the maximum amount of RNA that can be used is 1 microgram. For routine analysis, 10 pg - 100 ng RNA and 100 pg - 1 μg genomic DNA can be used.

*Cycling Parameters for One-step PCR:*

Reverse transcription (by MuLV) 48° C for 30 min.
AmpliTaq activation 95° C for 10 min.
PCR: denaturation 95° C for 15 sec and annealing/extension 60° C for 1 min (repeated 40 times) (On ABI 7700, minimum holding time is 15 seconds.)
The recently introduced EZ one-step™ RT-PCR kit allows the use of UNG as the incubation time for reverse transcription is 60° C thanks to the use of a thermostable reverse transcriptase. This temperature also a better option to avoid primer dimers and non-specific bindings at 48° C.

*Operating the ABI 7700:*

Make sure the following before starting a run:
1. Cycle parameters are correct for the run.
2. Choice of spectral compensation is correct (*off* for singleplex, *on* for multiplex reactions).
3. Choice of "Number of PCR Stages" is correct in the Analysis Options box (Analysis/Options). This may have to be manually assigned after a run if the data is absent in the amplification plot but visible in the plate view, and the X-axis of the amplification is displaying a range of 0-1 cycles.
4. No Template Control is labeled as such (for accurate ΔRn calculations).
5. The choice of dye component should be made correctly before data analysis.
6. You must save the run before it starts by giving it a name (not leaving as untitled). Also at the end of the run, first save the data before starting to analyze.
7. The ABI software requires extreme caution. Do not attempt to stop a run after clicking on the Run button. You will have problems and if you need to switch off and on the machine, you have to wait for at least an hour to restart the run.
When analyzing the data, remember that the default setting for baseline is 3 - 15. If any $C_T$ value is <15, the baseline should be changed accordingly (the baseline stop value should be 1-2 smaller than the smallest $C_T$ value). For a useful discussion of this matter, see the ABI Tutorial on Setting Baselines and Thresholds. (Interestingly, this issue is best discussed in the manual for TaqMan™ Human Endogenous Control Plate.)

If the results do not make sense, check the raw spectra for a possible CDC camera saturation during the run. Saturation of CDC camera may be prevented by using optical caps rather than optical adhesive cover. It is also more likely to happen when SYBR Green I is used, when multiplexing and when a high concentration of probe is used.

Interpretation of Results:

At the end of each reaction, the recorded fluorescence intensity is used for the following calculations:

$Rn^+$ is the Rn value of a reaction containing all components, $Rn^-$ is the Rn value of an unreacted sample (baseline value or the value detected in NTC). ΔRn is the difference between $Rn^+$ and $Rn^-$. It is an indicator of the magnitude of the signal generated by the PCR.

There are three illustrative methods to quantitate the amount of template:

1. Absolute standard method: In this method, a known amount of standard such as in vitro translated RNA (cRNA) is used.

2. Relative standard: Known amounts of the target nucleic acid are included in the assay design in each run,

3. Comparative $C_T$ method: This method uses no known amount of standard but compares the relative amount of the target sequence to any of the reference values chosen and the result is given as relative to the reference value (such as the expression level of resting lymphocytes or a standard cell line).

*The Comparative CT Method (ΔΔCT) for Relative Quantitation of Gene Expression:*

This method enables relative quantitation of template and increases sample throughput by eliminating the need for standard curves when looking at expression levels relative to an active reference control (normalizer). For this method to be successful, the dynamic range of both the target and reference should be similar. A sensitive method to control this is to look at how $\Delta C_T$ (the difference between the two CT values of two PCRs for the same initial template amount) varies with template dilution. If the efficiencies of the two amplicons are approximately equal, the plot of log input amount versus $\Delta C_T$ will have a nearly horizontal line (a slope of <0.10). This means that both PCRs perform equally efficiently across the range of initial template amounts. If the plot shows unequal efficiency, the standard curve method should be used for quantitation of gene expression. The dynamic range should be determined for both (1) minimum and maximum concentrations of the targets for which the results are accurate and (2) minimum and maximum ratios of two gene quantities for which the results are accurate. In conventional competitive RT-PCR, the dynamic range is limited to a target-to-competitor ratio of about 10:1 1 to 1:10 (the best accuracy is obtained for 1:1 ratio). The real-time PCR is able to achieve a much wider dynamic range.

Running the target and endogenous control amplifications in separate tubes and using the standard curve method requires the least amount of optimization and validation. The advantage of using the comparative $C_T$ method is that the need for a standard curve is eliminated (more wells are available for samples). It also eliminates the adverse effect of any dilution errors made in creating the standard curve samples.

As long as the target and normalizer have similar dynamic ranges, the comparative $C_T$ method ($\Delta\Delta C_T$ method) is the most practical method. It is expected that the normalizer will have a higher expression level than the target (thus, a smaller $C_T$ value). The calculations for the quantitation start with getting the difference ($\Delta C_T$) between the $C_T$ values of the target and the normalizer:

$$\Delta C_T = C_T\,(\text{target}) - C_T\,(\text{normalizer})$$

This value is calculated for each sample to be quantitated (unless, the target is expressed at a higher level than the normalizer, this should be a positive value. It is no harm if it is negative). One of these samples should be chosen as the reference (baseline) for each comparison to be made. The comparative $\Delta\Delta C_T$ calculation involves finding the difference between each sample's $\Delta C_T$ and the baseline's $\Delta C_T$. If the baseline value is representing the minimum level of expression, the $\Delta\Delta C_T$ values are expected to be negative (because the $\Delta C_T$ for the baseline sample will be the largest as it will have the greatest $C_T$ value). If the expression is increased in some samples and decreased in others, the $\Delta\Delta C_T$ values will be a mixture of negative and positive ones. The last step in quantitation is to transform these values to absolute values. The formula for this is:

$$\text{comparative expression level} = 2^{-\Delta\Delta CT}$$

For expressions increased compared to the baseline level this will be something like $2^3$ = 8 times increase, and for decreased expression it will be something like $2^{-3}$ = 1/8 of the reference level. Microsoft Excel can be used to do these calculations by simply entering the $C_T$ values (there is an online ABI tutorial at http://www.appliedbiosystems.com/support/tutorials/7700amp/ on the use of spread sheet programs to produce amplification plots; the Taqman™ Human Endogenous Control Plate protocol also contains detailed instructions on using MS Excel for real-time PCR data analysis).

The other (absolute) quantification methods are outlined in the ABI User Bulletins (http://docs.appliedbiosystems.com/search.taf?_UserReference=A8658327189850A13A0C598 E). The Bulletins #2 and #5 are most useful for the general understanding of real-time PCR and quantification.

Recommendations on Procedures:

1. Use positive-displacement pipettes to avoid inaccuracies in pipetting,

2. The sensitivity of real-time PCR allows detection of the target in 2 pg of total RNA. The number of copies of total RNA used in the reaction should ideally be enough to give a signal by 25-30 cycles (preferably less than 100 ng). The amount used should be decreased or increased to achieve this.

3. The optimal concentrations of the reagents are as follows:

i. Magnesium chloride concentration should be between 4 and 7 mM. It is optimized as 5.5 mM for the primers/probes designed using the Primer Express software.

ii. Concentrations of dNTPs should be balanced with the exception of dUTP (if used). Substitution of dUTP for dTTP for control of PCR product carryover requires twice dUTP that of other dNTPs. While the optimal range for dNTPs is 500 μM to 1 mM (for one-step RT-PCR), for a typical TaqMan reaction (PCR only), 200 μM of each dNTP (400 μM of dUTP) is used.

iii. Typically 0.25 μL (1.25 U) AmpliTaq DNA Polymerase (5.0 U/μL) is added into each 50 μL reaction. This is the minimum requirement. If necessary, optimization can be done by increasing this amount by 0.25 U increments.

iv. The optimal probe concentration is 50-200 nM, and the primer concentration is 100-900 nM. Ideally, each primer pair should be optimized at three different temperatures (58, 60 and 62° C for TaqMan primers) and at each combination of three concentrations (50, 300, 900 nM). This means setting up three different sets (for three temperatures) with nine reactions in each (50/50 mM, 50/300 mM, 50/900, 300/50, 300/300, 300/900, 900/50, 900/300, 900/900 mM) using a fixed amount of target template. If necessary, a second round of optimization may improve the results. Optimal performance is achieved by selecting the primer concentrations that provide the lowest $C_T$ and highest $\Delta$Rn. Similarly, the probe concentration should be optimized for 25-225 nM.

4. If AmpliTaq Gold DNA Polymerase is being used, there has to be a 9-12 min pre-PCR heat step at 92 - 95° C to activate it. If AmpliTaq Gold DNA Polymerase is used, there is no need to set up the reaction on ice. A typical TaqMan reaction consists of 2 min at 50° C for UNG (see below) incubation, 10 min at 95° C for Polymerase activation, and 40 cycles of 15 sec at 95° C (denaturation) and 1 min at 60° C (annealing and extension). A typical reverse transcription cycle (for cDNA synthesis), which should precede the TaqMan reaction if the starting material is total RNA, consists of 10 min at 25° C (primer incubation), 30 min at 48° C (reverse transcription with conventional reverse transcriptase) and 5 min at 95° C (reverse transcriptase inactivation).

5. AmpErase uracil-N-glycosylase (UNG) is added in the reaction to prevent the reamplification of carry-over PCR products by removing any uracil incorporated into amplicons. This is why dUTP is used rather than dTTP in PCR reaction. UNG does not function above 55 °C and does not cut single-stranded DNA with terminal dU nucleotides. UNG-containing master mix should not be used with one-step RT-PCR unless r*Tth* DNA polymerase is being used for reverse transcription and PCR (TaqMan EZ RT-PCR kit).

6. It is necessary to include at least three No Amplification Controls (NAC) as well as three No Template Controls (NTC) in each reaction plate (to achieve a 99.7% confidence level in the definition of +/- thresholds for the target amplification, six replicates of NTCs must be run). NAC former contains sample and no enzyme. It is necessary to rule out the presence of fluorescence contaminants in the sample or in the heat block of the thermal cycler (these would cause false positives). If the absolute fluorescence of the NAC is greater than that of the NTC after PCR, fluorescent contaminants may be present in the sample or in the heating block of the thermal cycler.

7. The dynamic range of a primer/probe system and its normalizer should be examined if the $\Delta\Delta C_T$ method is going to be used for relative quantitation. This is done by running (in triplicate) reactions of five RNA concentrations (for example, 0, 80 pg/μL, 400 pg/μL, 2 ng/μL and 50 ng/μL). The resulting plot of log of the initial amount vs $C_T$ values (standard curve) should be a (near) straight line for both the target and normalizer real-time RT-PCRs for the same range of total RNA concentrations.

8. The passive reference is a dye (ROX) included in the reaction (present in the TaqMan universal PCR master mix). It does not participate in the 5' nuclease reaction. It provides an internal reference for background fluorescence emission. This is used to normalize the reporter-dye signal. This normalization is for non-PCR-related fluorescence fluctuations occurring well-to-well (concentration or volume differences) or over time and different from the normalization for the amount of cDNA or efficiency of the PCR. Normalization is achieved by dividing the emission intensity of reporter dye by the emission intensity of the passive reference. This gives the ratio defined as Rn.

9. If multiplexing is done, the more abundant of the targets will use up all the ingredients of the reaction before the other target gets a chance to amplify. To avoid this, the primer concentrations for the more abundant target should be limited.

10. TaqMan Universal PCR master mix should be stored at 2 to 8° C (not at -20° C).

11. The GAPDH probe supplied with the TaqMan Gold RT-PCR kit is labeled with a JOE reporter dye, the same probe provided within the Pre-Developed TaqMan™ Assay Reagents (PDAR) kit is labeled with VIC. Primers for these human GAPDH assays are designed not to amplify genomic DNA.

12. The carryover prevention enzyme, AmpErase UNG, cannot be used with one-step RT-PCR which requires incubation at 48° C but may be used with the EZ RT-PCR kit.

13. One-step RT-PCR can only be used for singleplex reactions, and the only choice for reverse transcription is the downstream primer (not random hexamers or oligo-dT).

14. It is ideal to run duplicates to control pipetting errors but this inevitably increases the cost.

15. If multiplexing, the spectral compensation option (in Advanced Options) should be checked before the run.

16. Normalization for the fluorescent fluctuation by using a passive reference (ROX) in the reaction and for the amount of cDNA/PCR efficiency by using an endogenous control (such as GAPDH, active reference) are different processes.

17. ABI 7700 can be used not only for quantitative RT-PCR but also endpoint PCR. The latter includes presence/absence assays or allelic discrimination assays (such as SNP typing).

18. Shifting Rn values during the early cycles (cycle 0-5) of PCR means initial disequilibrium of the reaction components and does not affect the final results as long as the lower value of baseline range is reset.

19. If an abnormal amplification plot has been noted ($C_T$ value <15 cycles with amplification signal detected in early cycles), the upper value of the baseline range should be lowered and the samples should be diluted to increase the $C_T$ value (a high $C_T$ value may also be due to contamination).

20. A small ΔRn value (or greater than expected $C_T$ value) indicates either poor PCR efficiency or low copy number of the target.

21. A standard deviation >0.16 for $C_T$ value indicates inaccurate pipetting.

22. SYBR Green entry in the Pure Dye Setup should be abbreviated as "SYBR" in capitals. Any other abbreviation or lower case letters will cause problems.

23. The SDS software for ABI 7700 have conflicts with the Macintosh Operating System version 8.1. The data should not be analyzed on such computers.

24. The ABI 7700 should not be deactivated for extended periods of time. If it has ever been shutdown, it should be allowed to warm up for at least one hour before a run. Leaving the instrument on all times is recommended and is beneficial for the laser. If the machine has been switched on just before a run, an error box stating a firmware version conflict may appear. If this happens, choose the "Auto Download" option.

25. The ABI 7700 is only one of the real-time PCR systems available, others include systems from BioRad, Cepheid, Corbett Research, Roche and Stratagene.

EXAMPLE 2

## IDENTIFICATION OF DIAGNOSTIC MARKER GENES AND PRIORITY RANKING OF GENES

For experimental groups, differences in gene expression between animals before and after experimental induction of endotoxaemia were analysed using the empirical Bayes approach of Lonnstedt and Speed (Lonnstedt and Speed, 2002, Statistica Sinica 12: 31-46).

The objectives were to: (a) identify changes in gene expression during the acute endotoxaemic phase of disease, and (b) evaluate the diagnostic potential of these changes, for detecting enodtoxaemia.

Comparison between dosed and control horses involved some information which is within horses (i.e. some information is available from the longitudinal comparison of horses which were used both as controls and as treated animals), and some information which is between horses (involving cross-sectional comparisons between horses which were dosed and horses which were not). In addition, some planed samples were not available. The result is an unbalanced, non-orthogonal mixed effects study.

Gene expression data were generated, and quality metrics were generated for each chip. Only chips providing high quality data and passing all quality metrics were used in subsequent analyses. The chips were then processed using the RMA (Robust Multichip Analysis) algorithm as implemented in the R Bioconductor project. Following calculation of expression measures, the distribution of the chips was compared using Box and Whisker plots, kernel density estimates and MA plots. Outliers were removed from further analyses.

Results obtained were corroborated using Microarray Analysis Software 5.0 (provided by Affymetrix) and a list of "housekeeping" genes to scale the data. Housekeeping genes were determined *a priori* by identifying those genes that vary the least in gene expression across healthy horses of various breed, age, sex, and geographical location, and across horses with various diseases.

Positive horses at each time point were compared with all horses at time zero, and negative horses at the time point concerned. For example, horses which were positive at 24 hours were compared with all horses at day 0, and negative horses at 24 hours. Two approaches were used for each comparison: univariate comparisons made gene at a time, and multivariate comparisons using the entire gene set. For the univariate comparisons, the analysis of each gene was made on a linear mixed model, in which horse was a random effect and time (time 0 vs current time) and status (control or induced) were fixed effects. Individual p values were adjusted using the Holm step down procedure (Holm, S. 1979, Scandinavian Journal of Statistics 6: 65-70) to provide strong control of the Family-Wise Error Rate (FWER). For multivariate analyses, a composite strategy was employed involving, reduced space linear discriminate analysis, support vector machines and classification tree techniques. Genes that showed statistically significant differences before and after experimental induction of endotoxaemia were tabulated for each day post dosing.

A list of genes ranked by p value for comparisons made between hours 0 and 24, 48 and 72 post-dosing is shown in Table 5. This analysis is based on two-group comparisons (Hour 0 versus hours 24, 48, and 72) with p Values adjusted using Holm's and the FDR method. Results are based on the full outcome from the empirical Bayes method.

Using linear mixed models, and at 24 hours, 159 genes were statistically significant when Holm's correction was applied and 995 genes following FDR adjustment. Using classification and regression trees, 829 of the 3105 genes on the GeneChip™ separated the groups perfectly with a p value of 0.002.

Using linear mixed models, and at 72 hours, no genes were statistically significant when Holm's correction was applied and 62 genes following FDR adjustment. Using classification and regression trees, 125 of the 3105 genes on the GeneChip™ separated the groups perfectly with a p value of 0.001.

Using linear mixed models, and at 120 hours, no genes were statistically significant when either Holm's correction or FDR adjustment were applied. Using classification and regression trees, 7 of the 3105 genes on the GeneChip™ separated the groups perfectly with a p value of 0.019.

The genes listed in Table 5 are ranked in order of their t statistic or value-which may be interpreted as a signal-to-noise ratio. The tabulation also displays the log 2 fold change (M value), and the adjusted p values. Genes with a negative t value (and hence a negative M value) are down regulated. Genes with positive t and M values are up-regulated. The priority ranking of significant genes ($p<0.05$) is based on increasing t value for the first time point (24 hours) followed by ranking on increasing t value at 72 hours. Note, some genes are significant for both 24 and 72 hours, others are significant for either 24 or 72 hours.

EXAMPLE 3

## DEMONSTRATION OF DIAGNOSTIC POTENTIAL TO DETERMINE ENDOTOXAEMIA

In addition, the diagnostic potential of the entire set of genes was assessed using discriminant analysis (Venables and Ripley, 2002, Modem Applied Statistics in S, Springer) on the principal component scores (Jolliffe, I.T. Principal components analysis, Springer-Verlag, 1986) calculated from gene expression. The entire process was cross-validated. Sensitivity and specificity were calculated for a uniform prior. This may be interpreted as a form of shrinkage regularization, where the estimates are shrunken to lie in a reduced space.

Cross-validated discriminant function scores were used to estimate a receiver operator curve. The receiver operator curve was calculated by moving a critical threshold along the axis of the discriminant function scores. Both raw empirical ROCs were calculated, and smoothed ROCs using Lloyd's method (Lloyd, C.J. 1998, Journal of the American Statistical Association 93: 1356-1364). Curves were calculated for the comparison of clinically normal and clinically affected animals. Separate curves were calculated, using gene expression at each day post-inoculation. The area under the receiver operator curve was calculated by the trapezoidal rule, applied to both the empirical ROC and the smoothed ROC.

The ROC curve provides a useful summary of the diagnostic potential of an assay. A perfect diagnostic assay has an ROC curve which is a horizontal line passing through the point with sensitivity and specificity both equal to one. The area under the ROC curve for such a perfect diagnostic is 1. A useless diagnostic assay has a ROC curve which is given by a 45 degree line through the origin. The area for such an uninformative diagnostic is 0.5.

The ROC curves for the analysis based on comparisons between time point 0 and time points 24 hours and 72 hours are presented Figures 1-2, respectively. The diagnostic capability is very high.

EXAMPLE 4

## PREDICTIVE GENE SETS

Although about 180 genes have been identified as having diagnostic potential, a much fewer number are generally required for acceptable diagnostic performance.

Table 6 shows the cross-validated classification success, sensitivity and specificity obtained from a linear discriminant analysis, based on two genes selected from the set of potential diagnostic genes. The pairs presented are those producing the highest prediction success, many other pairs of genes produce acceptable classification success. The identification of alternate pairs of genes would be readily apparent to those skilled in the art. Techniques for identifying pairs include (but are not limited to) forward variable selection (Venables W.N. and Ripley B.D. Modem Applied Statistics in S 4th Edition 2002. Springer), best subsets selection, backwards elimination (Venables W.N. and Ripley B.D., 2002, *supra),* stepwise selection (Venables W.N. and Ripley B.D., 2002, *supra)* and stochastic variable elimination (Figueirodo M.A. Adeaptive Sparseness for Supervised Learning).

Table 7 shows the cross-validated classification success obtained from a linear discriminant analysis based on three genes selected from the diagnostic set. Only twenty sets of three genes are presented. It will be readily apparent to those of skill in the art that other suitable diagnostic selections based on three endotoxemia marker genes can be made.

Table 8 shows the cross-validated classification success obtained from a linear discriminant analysis based on four genes selected from the diagnostic set. Only twenty sets of four genes are presented. It will be readily apparent to practitioners in the art that other suitable diagnostic selections based on four endotoxemia marker genes can be made.

Table 9 shows the cross-validated classification success obtained from a linear discriminant analysis based on five genes selected from the diagnostic set. Only twenty sets of five genes are presented. It will be readily apparent to practitioners in the art that other suitable diagnostic selections based on five endotoxemia marker genes can be made.

Table 10 shows the cross-validated classification success obtained from a linear discriminant analysis based on six genes selected from the diagnostic set. Only twenty sets of six genes are presented. It will be readily apparent to practitioners in the art that other suitable diagnostic selections based on six endotoxemia marker genes can be made.

Table 11 shows the cross-validated classification success obtained from a linear discriminant analysis based on seven genes selected from the diagnostic set. Only twenty sets of seven genes are presented. It will be readily apparent to practitioners in the art that other suitable diagnostic selections based on seven endotoxemia marker genes can be made.

Table 12 shows the cross-validated classification success obtained from a linear discriminant analysis based on eight genes selected from the diagnostic set. Only twenty sets of eight genes are presented. It will be readily apparent to practitioners in the art that other suitable diagnostic selections based on eight endotoxemia marker genes can be made.

Table 13 shows the cross-validated classification success obtained from a linear discriminant analysis based on nine genes selected from the diagnostic set. Only twenty sets of nine genes are presented. It will be readily apparent to practitioners in the art that other suitable diagnostic selections based on nine endotoxemia marker genes can be made.

Table 14 shows the cross-validated classification success obtained from a linear discriminant analysis based on ten genes selected from the diagnostic set. Only twenty sets of ten genes are presented. It will be readily apparent to practitioners in the art that other suitable diagnostic selections based on ten endotoxemia marker genes can be made.

Table 15 shows the cross-validated classification success obtained from a linear discriminant analysis based on 12 genes selected from the diagnostic set. Only 20 sets of twenty genes are presented. It will be readily apparent to practitioners in the art that other suitable diagnostic selections based on twenty endotoxemia marker genes can be made.

Table 16 shows the cross-validated classification success obtained from a linear discriminant analysis based on 13 genes selected from the diagnostic set. Only 20 sets of twenty genes are presented. It will be readily apparent to practitioners in the art that other suitable diagnostic selections based on twenty endotoxemia marker genes can be made.

Further numbers of genes introduced noise (and subsequently lower specificity and sensitivity) through observational overload compared to the number of variables.

The genes listed in Table 5 are ranked in order of their t statistic - which may be interpreted as a signal-to-noise ratio. The tabulation also displays the log 2 fold change (M value), and the adjusted p values. Genes with a negative t value (and hence a negative M value) are down regulated.

EXAMPLE 5

## DEMONSTRATION OF SPECIFICITY

The specificity of the endotoxemia signature was examined by training a classifier on the trial data only and running the classifier over a large gene expression dataset of over 850 GeneChips®. Gene expression results in the database were obtained from samples from horses with various diseases and conditions including; clinical, induced acute and chronic EPM, herpes virus infection, degenerative osteoarthritis, stress, *Rhodococcus* infection, endotoxemia, laminitis, gastric ulcer syndrome, animals in athletic training and clinically normal animals.

Three classifiers were generated. All were based on the comparison of positives at 24 hours with all horses at time zero, and negative horses at 24 hours. The first used all the genes on the GeneChip™. The second used only those genes that were statistically significant (Holm's adjusted p value <0.05). The third was based on all of the genes except for 45 that had been identified as being involved in at least one other gene signature for disease. The latter was the most specific. It was able to identify all eight endotoxemic horses in the database. It also identified five other horses, one with severe gastritis, one with botulism, another with Wobbler syndrome and two others with an unknown diagnosis.

Using this method and a gene signature of 159 genes, a specificity of 99% for endotoxemia was obtained from a population sample size of over 850.

EXAMPLE 7

**GENE ONTOLOGY**

Gene sequences were compared against the GenBank database using the BLAST algorithm (Altschul, S.F., Gish, W., Miller, W., Myers, E.W. & Lipman, D.J. (1990) "Basic local alignment search tool." J. Mol. Biol. 215:403-410), and gene homology and gene ontology searches were performed in order to group genes based on function, metabolic processes or cellular component. Table 17 lists and groups the genes based on these criteria. See also Table 1, which contains sequence information for each gene.

The disclosure of every patent, patent application, and publication cited herein is hereby incorporated herein by reference in its entirety.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

# TABLE 1

| Gene Name | GenBank Homology | DNA SEQUENCE / DEDUCED AMINO ACID SEQUENCE | SEQUENCE IDENTIFIER: |
|---|---|---|---|
| WBC001D02.bFSP_20 012908.abd | Homo sapiens potassium channel tetramerisation domain containing 12 (KCTD12). | 1 GCGGGCAAGGCGGGCGGAGCGCACTGGAACTCAAGGGGGCGCACAGCGGCGCGCTCGCAC<br>61 CGCTCGGCTCCGCGCGGCTCTAGGAGGTGGCGGCGGTGGCGGTGGCGGCGGTGGCGGCGG<br>121 CGGCGGCGGCGGGGCGCAGGGCTGAGCGAGCGTCCGGGTTCCGGGGCTCCGGGGAAGGCG<br>181 GTTGCAGCTCCTGAGTGCAGCGCGGCTTCCTGCCACTGTCCCGGCCCGGCCACCTCTCTG<br>241 TCATGGCTCTGGCGGACAGCACACGTGGATTACCCAACGGGGGCGGCGGCGGGGGCGGCA<br>301 GTGGCTCCTCGTCGTCCTCCGCGGAGCCACCGCTCTTCCCCGACATCGTGGAGCTGAACG<br>361 TGGGGGGCCAGGTGTACGTGACCCGGCGCTGCACGGTGGTGTCGGTGCCCGACTCGCTGC<br>421 TCTGGCGCATGTTCACGCAGCAGCAGCCGCAGGAGCTGGCCCGGGACAGCAAAGGCCGCT<br>481 TCTTTCTGGACCGGGACGGCTTCCTCTTCCGCTACATCCTGGATTACCTGCGGGACTTGC<br>541 AGCTCGTGCTGCCCGACTACTTCCCCGAGCGCAGCCGGCTGCAGCGCGAGGCCGAGTACT<br>601 TCGAGCTGCCAGAGCTCGTGCGCCGCCTCGGGGCGCCCCAGCAGCCCGGCCCGGGCCGC<br>661 CGCCCTCGCGGCGCGGGGTGCACAAGGAGGGCTCGCTGGGTGACGAGCTGCTGCCGCTTG<br>721 GCTACTCGGAGCCCGAACAGCAGGAGGGCGCCTCTGCCGGGGCGCCGTCGCCCACGCTGG<br>781 AGCTGGCTAGCCGCAGTCCGTCCGGGGGCGCGGCGGGCCCGCTGCTCACGCCGTCCCAGT<br>841 CGCTGGACGGCAGCCGGCGCTCGGGCTACATCACCATCGGCTACCGCGGCTCCTACACCA<br>901 TCGGGCGGGACGCGCAGGCGGACGCCAAGTTCCGGCGAGTGGCGCGCATCACCGTTTGCG<br>961 GAAAGACGTCGCTGGCCAAGGAGGTGTTTGGGGACACCCTGAACGAAAGCCGGGACCCCG<br>1021 ACCGTCCCCCGGAGCGCTACACCTCGCGCTATTACCTCAAGTTCAACTTCCTGGAGCAGG<br>1081 CCTTCGACAAGCTGTCCGAGTCGGGCTTCCACATGGTGGCGTGCAGCTCCACGGGCACCT<br>1141 GCGCCTTTGCCAGCAGCACCGACCAGAGCGAGGACAAGATCTGGACCAGCTACACCGAGT<br>1201 ACGTCTTCTGCAGGGAGTGAGCTCCCCAGACCCCCTCGCCACTCCAGCGCCCAGTCCTTC<br>1261 TCCTGCCCGAGAGATGATTACAGAGCCTCTTGTCCCACCTTTGTCCCCTGGCTGCTGCCC<br>1321 TCCCATTCTCCCCCTCCAGTAGTAGCTGGGTGAGACCTGTCCGCCCACCTTCCCTCCACT<br>1381 ACAGAACCTGCAGCCGCAAATCCTCTGGGCTGCTTCGTCTTCTTTGGACCTCCTGAACCG<br>1441 AGAGAACCCAGAGGAACCCCCACCCCACCCCCACCTACCACTCCATGCTTTCTCTACTCC<br>1501 CTGCCTCAAACCACCCCTCCCCCAGATGGTACTTCAGTTTGGATCTATTGGGGGAGTGTG<br>1561 GCCACAGACCGGGGGATGATTGAATTGTTCAGAACCTGATTGGACCGTGTCCAATGTGCG<br>1621 GAAGATTTCCTTGAAATCTTCTCAAGCTCTTATGACTCACTGGGGGTTTAAGAGATCAGG<br>1681 ATTGGTTCCACTGTCTGGGGTTAGTGTTTTACAAGGTCATTACACAGTCTTTTTGACCTC<br>1741 TTTTGAAGGTAGAGTTTTAGAAGGCTGGATGGAAGATTCTGAGCCTGGAATTAGGACCCC<br>1801 ATGGAGGCAGTTCAGTAACTAAACTAATAAAGTTTTGAAAAGTTACACGTAAAGTAGAAG<br>1861 AATCTAGTGCGTGGGACAGTAAAGGATCCTTTCTCGTACAGAATAAAAGGTCTCAGCCTG<br>1921 TAGCTTAAACTTATAGAAAGTGATCCGCCTGCCTGCAGAGGCGCCCTTTTCAGCTGCTGC<br>1981 TCGCCAGAAGCCCTTGATTCCACTGGTTGACATGGCAGCAGTTACTGGCAAGAGGGAGAA<br>2041 AGGACGCTGCCGCCTAAGAGTGCAAGGCTGCTCAGGTCTCCAAGCGCCGTAGGAGGTCAC<br>2101 CTGGCAGTGACTGTAGGGAGCTGGGTCATAGTGCACGTCGTGGGTATTAGGAAAGCCTGT<br>2161 ATTCTTTCAATGAATGTCAGTAGGACCTTCCTTTAGCTGTAAGACTTGGTGGGCGGGGTG<br>2221 GGGTGGGGAGGGAGGAAAGGGTAGGAAGGGTGGGAAGGGAGAAGCAGACATAGTCATTTA | SEQ ID NO:1 |

| Gene Name | GenBank Homology | DNA SEQUENCE / DEDUCED AMINO ACID SEQUENCE | SEQUENCE IDENTIFIER: |
|---|---|---|---|
| | | 2281 TGATTTGAAAGTTGGAAGTTTGTACCATCTGTTTGAGTATATGCACATTTAAAAAATATC<br>2341 ATATAGTAAATGCAAACATGCCAAGTATTTTATAAAGATTAATAACAGACCTACTCTTAA<br>2401 CTGGCAGTTTGAACTAACTGTGTTGAGTCCTAAACTTAGAAGTTGCTGATACTTACATAA<br>2461 CCTAACCAAAGAGCTATCCAGTAGGGTTTTAGTTTGTGAACTTTATTTTCCTGTTGATTA<br>2521 TAAATCCTGATTTTTAAGATCTATTTACTTGAGCAAAATATGTTTGATTTCGGTTAGTTA<br>2581 GACTTAAGATCTCTTATTAAAAGTCCTTACTTTCTCCAAGCCCAGTCAATGTTGACTTCT<br>2641 GGGCAAACCTGAAAATGCCACTTCTTTCATGCAGATTCTTTGAAGTTAAGATGGAATCTT<br>2701 TTCGAGTGATAGAGCTGCAGAGATATAGAAGCTCCATGGGCTGCCCATCTGTAGATAGCT<br>2761 GTAAAATGGAATATTTTTAATTGAAGGCAAAAAAGTGCTTAAAAGTGAGCTGAGCAATAA<br>2821 AATGGTCCAATAATAGGTAAATGCAACAGAAACAGAAGGAGACCTGGTTGCCTTATACCT<br>2881 TTACGCGAACACGGAATAAACTCCCACTGCACATCCTGTCTACACCATAAGTGGAGGGAA<br>2941 ATAAACCTCGTCATGCTCCATGCTGTGAGGCTCCTTTGGATATTCTGTGATGACCGAGAA<br>3001 GCCTATTTTGTTTTGTTTTCAACATCTTTATCCGAAGTACGTTTTTAAAGATTTTGTAAG<br>3061 AGTCGTTTTCAGTGTTTAAATTAGCGCTATTTTTCCTTCTTTTTAAAAATGAATCTTGTA<br>3121 CTGTATCTTACTATGTCTGTAACAGGTGTTAAGAATTGAAACAGTTTTATTCTTAGATTC<br>3181 ATGCGATCCAGTCTGTATATACCATATATAAACATTTTACATGAATCATTTAGTTTTTTA<br>3241 ATTCATTTACTAATGCTATAAAATTTCCTATATTACCCCAGTAATTTGCATCAGCTGGTT<br>3301 TATATACTAAAGCAACATGTTTTGATGAGTTTCTTACATCCTTATCGAGGAATTGGGTTA<br>3361 GGAAAAAAATACATAATTGTAAAACTGAGTTTGCTGTATTATACTTTTTTTCTTGAGTATT<br>3421 AGTTGTATTACTAATCATATGTTGATTAACTGTCTACTTAAAGTCAAGGTACCTGTATTT<br>3481 TTAATCCACTAATTTTTTTTTAGTTGGGAAATAGATTTCAAGTCTTTTATTAGACTAACA<br>3541 TTTTTTGAGAAGTAAAATTGACTTTATATACAAAGCCTGTAATTTTAGGCGAAATGGAAG<br>3601 CAGAAATCTAGGAAGTTGTGCTTGCTTGTATGGTCTCAGACTAAGTAATGCATCAGAATT<br>3661 CATCTGTTTGAAGCCTGAAATAATTTAGGACTCTGATTCACTGACCAAAAGTCAGTGTTG<br>3721 CAGAGATTTCTCTACCCCGTATGGTATTTTGTTAGATTGTTCAACAGGAAGCACATGATT<br>3781 GAGAACATCTTGGGACAGACCAAAACCACTGACAGATGGCAAGGCTCGGCGATTCTGATT<br>3841 TCCCTTCTCAAATCTGCTCAACTCCAAGAGTCTTGAGAAACTGCTAAAATTTTGCCTCTG<br>3901 TCACTCAAGTCTTACAAATGTTATCTTGTAAACCTTTGAGGTGAACTATTCCACTGTCTT<br>3961 GTACATAGGCATCTTATTCACTGCACCCTGTCACACCCAGCACCCCCCGCCCCGCACATT<br>4021 ATTTGAAAGACTGGAATTTAATGGTTAGGGACAGTAAATCTACTTCTTTTTCCAGGGAC<br>4081 GACTGTCCCCTCTAAAGTTAAAGTCAATACAAGAAAACTGTCTATTTTTAGCCTAAAGTA<br>4141 AAGGCTGTGAAGAAAATTCATTTTACATTGGGTAGACAGTAAAAAACAAGTAAAATAACT<br>4201 TGACATGAGCACCTTTAGATCCCTTCCCCTCCATGGGCTTTGGGCCACAGAATGAACCTT<br>4261 TGAGGCCTGTAAAGTGGATTGTAATTTCCTATAAGCTGTAATAGTGGAGGTATTGTGGGT<br>4321 TCATTTGAGTAAGCCCTCCAAAGATACCATTCAAATAACCTGGGAGAATGTCATAAATTA<br>4381 TTCAGATAATTATCACTGCATGAATCTGATTCAGAGGCATGCATTTACATATGTTGCCCT<br>4441 AATTACCATTTGATGATCATAAATACAAGTGAATGACATTGGACTTTTAGTAACAAACTT<br>4501 AATTTTTAAAAAAGTGTAGACAATGGTGGTTAAAAAAAAAAAAAACAGGTGCCAGGTTCT<br>4561 GTGTGTTTGCACCAAGTAATTGACATGTTTTTTGTTTAATACATGTGGACCATGAACAGT<br>4621 ATTCATTCTACTTTTTCAAATGATATGCTGTAGAAAATATTCCTTGAAGATGTGAGATTT<br>4681 AAAAATTTTTCCCTTTCAATGTTGTTTTAATTGTATTTCTTACTTGGTTTTTTTGATTGA<br>4741 TAGCACAGTGATAAATCATAATACTAGACAAAATTGTCTTCTCTTTCAAACCAGAGCCAT<br>4801 ATATATGTCTGTATATATGGACCTACTGCTTCTCTGAGGAAATGCATAATCTGTTAATA | |

| Gene Name | GenBank Homology | DNA SEQUENCE / DEDUCED AMINO ACID SEQUENCE | SEQUENCE IDENTIFIER: |
|---|---|---|---|
| | | 4861 TCAGACAAAATGAGCAATTGGCAGTGCTCATAATATATTCCAATTTTTATTGGAATTTTC<br>4921 GATGGAATGTTATTTCAATAAAGCCATGTAAGGTGAAACTTTGATAACTTTTTACTCTTC<br>4981 AAGTTAGGGTAAATTCTGATCCAATATTCAATTCATTTGTGTACTCCCACATGCAAAATG<br>5041 CTAAATTACAATGCAGACATTAAGAAAAAGTATTGACTGGAGGGGTTGAATTCCTTGAGA<br>5101 ATTTATTTTATAGTCTAAATCACAAATACTTTACTCAATTTAGTTTTTAAAATAGTAAAC<br>5161 TGAATATTTTTGTTGTAAGCCTATCAGAGTCAATCCTTCGTTTGGAATTGTTTTCCTGTT<br>5221 TTTCCTTACTATAAATCATTTAAAAACTGAATTCATTTTCTTAGATGGCATAAGTCTGTC<br>5281 TCTTGAGAAATAAGTAAAATACTCCTATTTTCAGTATCTGTAGCACCTGAAATAGGTCTT<br>5341 TGTATAGCCAGAAACAAGTTATGTTGAAGTTAGCTTTTCTTTGTCAACAGTTTTGGACAA<br>5401 TAAAAATCTGAAAGTATTAACACTTGATTTTCTACTGGGGCCCTTCAAACTTGGTTGGAA<br>5461 GAAATTCAACCAGAATATCTACATTAGAGTATAATCATGTGTGGTAGGAAGATGGACTAG<br>5521 TTAATCAAGATTTGTTGTCACTTAAATTTTTTGTGATTTTTTTCCAAGCCAGTTTTTTTA<br>5581 AATTCTAAATGTGTTTTGAGGTATGGGTACATTAATTGTAATGTAAACTATTATACAACT<br>5641 GTTTTTGCGACTTTATAGGCAGGTAAATTTTGCTATTACTATTGAATACAAATGACAATT<br>5701 CATTTATGACCACTCAAACAGCGTTAGTAACCATTTAGTGACAAAGGATTAAAACATCCA<br>5761 TCTGGATGTTAATTTTGAAGATGTAAATTATATGTTGTTTAAATTTTTCCAGGCATCTGA<br>5821 AAACCTTATCTGCTAGACAATGTAAGATTCACACAGAGTTATCTGGGATTCTGATTTTTT<br>5881 AAATAGTACATATCATTAAACCATTTTCTCTAAATGTAAGAAGAGCAGAAAAAATCTTAT<br>5941 AAGATTATCAGATTTTTCTAATGACACAGAAATGTAAGAAAAAAATCCCTTTATATTGAA<br>6001 AAAAGATGCAGTCAAAGTCTTTTCAGACATGCCCAAACTTTGAGAATTCCTTCAACCATC<br>6061 TAATGCTATAAAGATTTTTGTTCTTCCTGTTCACAACCAGTTGTATAACAGAAATACTAG<br>6121 CTACTGTTTTCCTTCCTGTGTGTGAAGTAATGAATCATTGATTATGTGACTTGTTATGTA<br>6181 TTCAATTAAACACTAAAGAATAAAACATTCACTCCTTT | |
| | | 1 M A L A D S T R G L P N G G G G G G G S<br>1 ATGGCTCTGGCGGACAGCACACGTGGATTACCCAACGGGGGCGGCGGCGGGGGCGGCAGT<br><br>21 G S S S S S A E P P L F P D I V E L N V<br>61 GGCTCCTCGTCGTCCTCCGCGGAGCCACCGCTCTTCCCCGACATCGTGGAGCTGAACGTG<br><br>41 G G Q V Y V T R R C T V V S V P D S L L<br>121 GGGGGCCAGGTGTACGTGACCCGGCGCTGCACGGTGGTGTCGGTGCCCGACTCGCTGCTC<br><br>61 W R M F T Q Q Q P Q E L A R D S K G R F<br>181 TGGCGCATGTTCACGCAGCAGCAGCCGCAGGAGCTGGCCCGGGACAGCAAAGGCCGCTTC<br><br>81 F L D R D G F L F R Y I L D Y L R D L Q<br>241 TTTCTGGACCGGGACGGCTTCCTCTTCCGCTACATCCTGGATTACCTGCGGGACTTGCAG<br><br>101 L V L P D Y F P E R S R L Q R E A E Y F<br>301 CTCGTGCTGCCCGACTACTTCCCCGAGCGCAGCCGGCTGCAGCGCGAGGCCGAGTACTTC<br><br>121 E L P E L V R R L G A P Q Q P G P G P P | SEQ ID NO:2 |

| Gene Name | GenBank Homology | DNA SEQUENCE / DEDUCED AMINO ACID SEQUENCE | SEQUENCE IDENTIFIER: |
|---|---|---|---|
| | | 361 GAGCTGCCAGAGCTCGTGCGCCGCCTCGGGGCGCCCCAGCAGCCCGGCCCGGGGCCGCCG<br><br>141 P S R R G V H K E G S L G D E L L P L G<br>421 CCCTCGCGGCGCGGGGTGCACAAGGAGGGCTCGCTGGGTGACGAGCTGCTGCCGCTTGGC<br><br>161 Y S E P E Q Q E G A S A G A P S P T L E<br>481 TACTCGGAGCCCGAACAGCAGGAGGGCGCCTCTGCCGGGGCGCCGTCGCCCACGCTGGAG<br><br>181 L A S R S P S G G A A G P L L T P S Q S<br>541 CTGGCTAGCCGCAGTCCGTCCGGGGGCGCGGCGGGCCCGCTGCTCACGCCGTCCCAGTCG<br><br>201 L D G S R R S G Y I T I G Y R G S Y T I<br>601 CTGGACGGCAGCCGGCGCTCGGGCTACATCACCATCGGCTACCGCGGCTCCTACACCATC<br><br>221 G R D A Q A D A K F R R V A R I T V C G<br>661 GGGCGGGACGCGCAGGCGGACGCCAAGTTCCGGCGAGTGGCGCGCATCACCGTTTGCGGA<br><br>241 K T S L A K E V F G D T L N E S R D P D<br>721 AAGACGTCGCTGGCCAAGGAGGTGTTTGGGGACACCCTGAACGAAAGCCGGGACCCCGAC<br><br>261 R P P E R Y T S R Y Y L K F N F L E Q A<br>781 CGTCCCCCGGAGCGCTACACCTCGCGCTATTACCTCAAGTTCAACTTCCTGGAGCAGGCC<br><br>281 F D K L S E S G F H M V A C S S T G T C<br>841 TTCGACAAGCTGTCCGAGTCGGGCTTCCACATGGTGGCGTGCAGCTCCACGGGCACCTGC<br><br>301 A F A S S T D Q S E D K I W T S Y T E Y<br>901 GCCTTTGCCAGCAGCACCGACCAGAGCGAGGACAAGATCTGGACCAGCTACACCGAGTAC<br><br>321 V F C R E -<br>961 GTCTTCTGCAGGGAGTGA | |
| WBC001E10.bFSP_20 012908.abd | No Homology | 1 GACTGGGAACAGCAAGTGCAAAGTTCCTGTGGCAGAAATGTGCCTGGCAAGGAGGCCTG<br>61 CATGGCTGGAGCATTGTGAGCTGGTGGGAGAATGGAGAACAGGAAGAGATGATGGGGTAC<br>121 CTCGCGTCTTCATCGGTAAAGACTGTATAGGTGGATGCACTGACCTACTCAACATGCATC<br>181 AGAGTGGGCAGCTGTTGACACGGCTAAAGCAAATTGGAGCTCTAGAATAATTACAGAGCT<br>241 GATCCCAGACTGATGTCCCCCTGAGAGCTGAATAGCATTGCAATGATGACAGCACTTCCT<br>301 GGTGGATGGATCAGGGGCTACCTTTACCCAGCTACAGCAGCTGTTTACTTCTGAAATATG<br>361 TTAACCAAAAAAGGAGGGGTGGGGGTTGGCAAAAATAGCTTTTTCTTTTTTGATTTAGTG<br>421 TTAAAAGTGGCTCAACTTGCCCCAAATCACAGATCTGAGAAGGTTGATGGATGTCTTGGG<br>481 TTTCTTCTGGATCGGCCCTTTGGGTTCCAAAAGACCGTGACAGTCATGGCCTAGAATTCA<br>541 CTCACTGACTCAAAAGATGTTCTCTCTCTTTGGCACATGTTTCTTATGATTGTCCTCCAT | SEQ ID NO:3 |

| Gene Name | GenBank Homology | DNA SEQUENCE / DEDUCED AMINO ACID SEQUENCE | SEQUENCE IDENTIFIER: |
|---|---|---|---|
| | | 601 GCGCCGGGCCTCGTCTACCTCTAAGCCAGTGTTTCGCAACCAAGTTTATCCCAAGCCCCT<br>661 CAGGATGAATCTGTAGTTGGTTTTCTGCTACTACCTGAAAATTATTTTGTTGAGTTTGGC<br>721 TTTATGGAGTTTGTACT<br><br>1 GCATGTGTCTCCCTGGTTACCATCCTCACTCCACCCCACTCCTATCTTGAGAATCGTTTG<br>61 CTCTGAAACAGTGTTCTCAACCTTGACAAATTCACAGCTCTCACAGGAAATGTTCAACAG<br>121 TTAAGAAATTGGAGAAGTGAATTAAGAAATGAAGGGCCTGAGGGCCCCATTTGAGAAAGT<br>181 CCGCAGGAAACATTAAGCACTGTTGGCCTCCTGGTGCCCAGTGATCGGAGGTCCAGGTGG<br>241 AGATGCCGTGATCATCTCTTGCCTAAGATGCTCATCCAGGTCATCCTGAACAAGTATGTG<br>301 TTAATTTGCTTAGATTATGAACCAAAGTAAAACATGAAATTTAAAACGTTATCACAGAAA<br>361 ACTTACAACTCCCTCCCCCAAGGGTATGTCTGTGAAAGTAGCCAAATTTAAGAAACACGG<br>421 TCCTAAATTATTTTCTCTCTCCTTTTTTTCCCTACTCTCTGAAAATTTAAGGATCCCTTA<br>481 CTGTGATGTTTGTTCTAGCATTGCAAGAGTAAGTTAATTTCCTTCTGCAGTCTTTAGGGT<br>541 GAATGTTAATTTCTTTTCTAATAAAGAAGTACCCAGAGATGAAAAAAAAAAAAAAAAAAA | SEQ ID NO:4 |
| WBC001F10.bFSP_20 012908.abd | No Homology | 1 CTTTTTCAAAATAGGTCTCTAAGGAAAACCAGCAGAACATTAGCCTGTGCAGAGCCATCG<br>61 TTTGGGGAGCACACTCTTCCGTTACGCTTGGCACGCAGATCTCCCTGTGGTGGGATTTCT<br>121 TTCTTTTTTGTTGTTTTTCCTTTTTGTTTCAAGGGGGTACTTCCCCCCTATTTTCTCTTT<br>181 TCCCTTGATACAAGTTGTAGATGAAATAGGAGAAAGCCCTTGTTGCTATAGATGTGTGTG<br>241 CAGTCTGGCAGCCTTAAGCCCACCTGGGCACTTTTAGAAAAAAAACAAAAAACACCAAAA<br>301 AAAACAGTGTCATATGTATTAATGATCCAGGTGGTTTTTAGTCTTTCCTGATGATGGGGT<br>361 GTTCATGTTAGTTTCTTTTCTTGAAAACCCTATTCAACATTAGGCAAAGTAGCTAAGAGC<br>421 CTTTTGTTTTGGTAAATTAGTGGGGAAATGGCATTTTAAGAGGACTCTCTTCTCAAAGAA<br>481 CTTAGCTGAGAGTCGAATTCTTCTCTTTTCCAGCTGATGAAGCTAAGTGGGTATCCCAGA<br>541 AATGTTTGTTTTCTGAAGGGGAGTACTCCAGGCCATCACTGAAGCTGTGTCCAGGCAGAG<br>601 AGTTAGCACACTTTCTACACCTTGTAGAATTCTGGGCTGTAGCATTACTCTGATTTTCTG<br>661 TCTAATGAAGGCAGAGAGTGCTGGTAGTTTAAAATTTTTATTTTAGGACTTATCTGTACT<br>721 CTGAATTTTCA<br><br>1 TAAATGAAAATCTGTAGATTCTCTCCTCCCCTAATCCCAGAAAACGAAACCAAATAATGT<br>61 TTTTTGAAATTGTTTCTAGGATTTCAAAGTGAAAAATGATGGTACTATCCAAAATTCTTT<br>121 ATTTCAGAACATGACAATAGATTCCTTTAATGTAGGCTCAAGATCAAAACAGCATGTCCT<br>181 GTAAGCTCAGATAGACTCTGTTGACTCCAAGGGTTTTGATCAGTATCATAACAAACTTTT<br>241 CCCTTTGACATGCTCTGATTTTTGTTGTTTGGGGGGAGGGCGTGTGTGTGTGTGCGTGTG<br>301 TGTGTGTGTGCGCGCGCGCGTGTGTGTGCGGTGTCCACCAGTATCAGTGCCTGCCTGCGT<br>361 TAGGAGAATTACATTCCTGGTTCTGTATGAGGAGAAGGGTGTATAAAGCAACATGAAACA<br>421 TTACCCCTCATTTTGTTTTAAAGGACAAACTAATGTTAATTGTTCTCAGAACTGGATTTT<br>481 CTTCCTCAAAATTAAAAGTTTTCTTTTCTTTTGGATTTAATGAAGTATTGCTAGCTGAAG<br>541 CCAGTTTGACATGGTGAGAGAGATGTCAGACTGATGAAAGGTGTGCAGCCTGATTTAAAA<br>601 CCAACCCCGAACCCTTTTAAAGAACATAAAACATATT | SEQ ID NO:5<br><br><br><br><br><br><br><br><br><br><br><br><br><br>SEQ ID NO:6 |
| WBC001F11.bFSP_20 | Homo sapiens | | |

| Gene Name | GenBank Homology | DNA SEQUENCE / DEDUCED AMINO ACID SEQUENCE | SEQUENCE IDENTIFIER: |
|---|---|---|---|
| 012908.abd | retinoblastoma-like 2 (p130), mRNA | 1 AGCGGGCAGGCGCGGTGCGGGCGGCGGACGGGCGGGCGCTTCGCCGTTTGAATGGCTGCG<br>61 GGCCCGGGCCCTCACCTCACCTGAGGTCCGGCCGCCCAGGGGTGCGCTATGCCGTCGGGA<br>121 GGTGACCAGTCGCCACCGCCCCCGCCTCCCCCTCCGGCGGCGGCAGCCTCGGATGAGGAG<br>181 GAGGAGGACGACGGCGAGGCGGAAGACGCCGCGCCGCCTGCCGAGTCGCCCACCCCTCAG<br>241 ATCCAGCAGCGGTTCGACGAGCTGTGCAGCCGCCTCAACATGGACGAGGCGGCGCGGGCC<br>301 GAGGCCTGGGACAGCTACCGCAGCATGAGCGAAAGCTACACGCTGGAGGGAAATGATCTT<br>361 CATTGGTTAGCATGTGCCTTATATGTGGCTTGCAGAAAATCTGTTCCAACTGTAAGCAAA<br>421 GGGACAGTGGAAGGAAACTATGTATCTTTAACTAGAATCCTGAAATGTTCAGAGCAGAGC<br>481 TTAATCGAATTTTTAATAAGATGAAGAAGTGGGAAGACATGGCAAATCTACCCCCACAT<br>541 TTCAGAGAACGTACTGAGAGATTAGAAAGAAACTTCACTGTTTCTGCTGTAATTTTTAAG<br>601 AAATATGAACCCATTTTTCAGGACATCTTTAAATACCCTCAAGAGGAGCAACCTCGTCAG<br>661 CAGCGAGGAAGGAAACAGCGGCGACAGCCCTGTACTGTGTCTGAAATTTTCCATTTTTGT<br>721 TGGGTGCTTTTTATATATGCAAAAGGTAATTTCCCCATGATTAGTGATGATTTGGTCAAT<br>781 TCTTATCACCTGCTGCTGTGTGCTTTGGACTTAGTTTATGGAAATGCACTTCAGTGTTCT<br>841 AATCGTAAAGAACTTGTGAACCCTAATTTTAAAGGCTTATCTGAAGATTTTCATGCTAAA<br>901 GATTCTAAACCTTCCTCTGACCCCCCTTGTATCATTGAGAAACTGTGTTCCTTACATGAT<br>961 GGCCTAGTTTTGGAAGCAAAGGGGATAAAGGAACATTTCTGGAAACCCTATATTAGGAAA<br>1021 CTTTATGAAAAAAAGCTCCTTAAGGGAAAAGAAGAAAATCTCACTGGGTTTCTAGAACCT<br>1081 GGGAACTTTGGAGAGAGTTTTAAAGCCATCAATAAGGCCTATGAGGAGTATGTTTTATCT<br>1141 GTTGGGAATTTAGATGAGCGGATATTTCTTGGAGAGGATGCTGAGGAGGAAATTGGGACT<br>1201 CTCTCAAGGTGTCTGAACGCTGGTTCAGGAACAGAGACTGCTGAAAGGGTGCAGATGAAA<br>1261 AACATCTTACAGCAGCATTTTGACAAGTCCAAAGCACTTAGAATCTCCACACCACTAACT<br>1321 GGTGTTAGGTACATTAAGGAGAATAGCCCTTGTGTGACTCCAGTTTCTACAGCTACGCAT<br>1381 AGCTTGAGTCGTCTTCACACCATGCTGACAGGCCTCAGGAATGCACCAAGTGAGAAACTG<br>1441 GAACAGATTCTCAGGACATGTTCCAGAGATCCAACCCAGGCTATTGCTAACAGACTGAAA<br>1501 GAAATGTTTGAAATATATTCTCAGCATTTCCAGCCAGACGAGGATTTCAGTAATTGTGCT<br>1561 AAAGAAATTGCCAGCAAACATTTTCGTTTTGCGGAGATGCTTTACTATAAAGTATTAGAA<br>1621 TCTGTTATTGAGCAGGAACAAAAAAGACTAGGAGACATGGATTTATCTGGTATTCTGGAA<br>1681 CAAGATGCGTTCCACAGATCTCTCTTGGCCTGCTGCCTTGAGGTCGTCACTTTTTCTTAT<br>1741 AAGCCTCCTGGGAATTTTCCATTTATTACTGAAATATTTGATGTGCCTCTTTATCATTTT<br>1801 TATAAGGTGATAGAAGTATTCATTAGAGCAGAAGATGGCCTTTGTAGAGAGGTGGTAAAA<br>1861 CACCTTAATCAGATTGAAGAACAGATCTTAGATCATTTGGCATGGAAACCAGAGTCTCCA<br>1921 CTCTGGGAAAAAATTAGAGACAATGAAAACAGAGTTCCTACATGTGAAGAGGTCATGCCA<br>1981 CCTCAGAACCTGGAAAGGGCAGATGAAATTTGCATTGCTGGCTCCCCTTTGACTCCCAGA<br>2041 AGGGTGACTGAAGTTCGTGCTGATACTGGAGGACTTGGAAGGAGCATAACATCTCCAACC<br>2101 ACATTATACGATAGGTACAGCTCCCCACCAGCCAGCACTACCAGAAGGCGGCTATTTGTT<br>2161 GAGAATGATAGCCCCTCTGATGGAGGGACGCCTGGGCGCATGCCCCCACAGCCCCTAGTC<br>2221 AATGCTGTCCCTGTGCAGAATGTATCTGGGGAGACTGTTTCTGTCACACCAGTTCCTGGA<br>2281 CAGACTTTGGTCACCATGGCAACCGCCACTGTCACAGCCAACAATGGGCAAACGGTAACC<br>2341 ATTCCTGTGCAAGGTATTGCCAATGAAAATGGAGGGATAACATTCTTCCCTGTCCAAGTC<br>2401 AATGTTGGGGGGCAGGCACAAGCTGTGACAGGCTCCATCCAGCCCCTCAGTGCTCAGGCC<br>2461 CTGGCTGGAAGTCTGAGCTCTCAACAGGTGACAGGAACAACTTTGCAAGTCCCTGGTCAA<br>2521 GTGGCCATTCAACAGATTTCCCCAGGTGGCCAACAGCAGAAGCAAGGCCAGTCTGTAACC | SEQ ID NO:7 |

| Gene Name | GenBank Homology | DNA SEQUENCE / DEDUCED AMINO ACID SEQUENCE | SEQUENCE IDENTIFIER: |
|---|---|---|---|
| | | 2581 AGCAGTAGTAATAGACCCAGGAAGACCAGCTCTTTATCGCTTTTCTTTAGAAAGGTATAC<br>2641 CATTTAGCAGCTGTCCGCCTTCGGGATCTCTGTGCCAAACTAGATATTTCAGATGAATTG<br>2701 AGGAAAAAAATCTGGACCTGCTTTGAATTCTCCATAATTCAGTGTCCTGAACTTATGATG<br>2761 GACAGACATCTGGACCAGTTATTAATGTGTGCCATTTATGTGATGGCAAAGGTCACAAAA<br>2821 GAAGATAAGTCCTTCCAGAACATTATGCGTTGTTATAGGACTCAGCCGCAGGCCCGGAGC<br>2881 CAGGTGTATAGAAGTGTTTTGATAAAAGGAAAAAGAAAAAGAAGAAATTCTGGCAGCAGT<br>2941 GATAGCAGAAGCCATCAGAATTCTCCAACAGAACTAAACAAAGATAGAACCAGTAGAGAC<br>3001 TCCAGTCCAGTTATGAGGTCAAGCAGCACCTTGCCAGTTCCACAGCCCAGCAGTGCTCCT<br>3061 CCCACACCTACTCGCCTCACAGGTGCCAACAGTGACATGGAAGAAGAGGAGAGGGGAGAC<br>3121 CTCATTCAGTTCTACAACAACATCTACATCAAACAGATTAAGACATTTGCCATGAAGTAC<br>3181 TCACAGGCAAATATGGATGCTCCTCCGCTCTCTCCCTATCCATTTGTAAGAACAGGCTCT<br>3241 CCTCGCCGAATACAGTTGTCTCAAAATCATCCTGTCTACATTTCCCCACATAAAAATGAA<br>3301 ACAATGCTTTCTCCTCGTGAAAAGATTTTCTACTACTTCAGCAACAGTCCTTCAAAGAGA<br>3361 CTAAGAGAAATTAACAGTATGATCCGGACAGGAGAAACTCCAACCAAAAAGCGAGGGATT<br>3421 CTTTTGGAAGACGGAAGTGAATCACCTGCAAAAAGAATTTGCCCAGAAAATCATTCTGCC<br>3481 TTACTGCGCCGTCTACAAGATGTAGCTAATGACCGAGGTTCCCACTGAGGTTAGTCCATG<br>3541 GTATTGAAACTGTCTTCTCACAAACTCTGTTTAGCAGCATCATTTAATGCATGCTCGATT<br>3601 ATGGGGCTCTTTCCCTTAATCCTTCCAGTATCCATAGGATATTCTGACTCATCCCAACTG<br>3661 CCTTTTTTCCTACCATTCAGTGCTTGCCCTCAAGGCTGCTTAGAATCCAAACTTGGATTT<br>3721 TTGACTCTGGCAAAGCTTTTAGAAATACTGCAAGAAAATGATGTGTACCCAAACGTGAGC<br>3781 ATAGGAGGCTTCTGTTGACGTACTCCAACAGAAGAACTGTTTTCAAGTTCATTCCTACCC<br>3841 GTTTTGTGGTCACCTTTGGCCCTCACAAAAAGTAAAACAAAAACTAGGTATTTTGTCCTA<br>3901 AAACACCTGGTAGGAGTGTGTGATTTTTTGCATTCCTGACAAAGGAGAGCACACCCAGGT<br>3961 TTGGAGGTCCTAGGTCATTAGCCCTCGTCTCCCGTTCCCTTTGTGCACATCTTCCCTCTC<br>4021 CCCATTCGGTGTGGTGCAGTGTGAAAAGTCCTTGATTGTTCGGGTGTGCAATGTCTGAGT<br>4081 GAACCTGTATAAGTGGAGGCACTTTAGGGCTGTAAAATGCATGATTTTGTAACCCAGATT<br>4141 TTGCTGTATATTTGCGATGGCACTTTCTACAATGTGAACTTTATTAAGTATAAAACTTCT<br>4201 AGGCTAAACATTCAATATCTTCTTTGATGCTTTTGTACTTTTTTAAAATTGTTAAAGCCC<br>4261 CAGTAGCCACCTTTTGGGCATATTTGAAATTCTCCAGTTTTTGATTATTAAGAGGGATCA<br>4321 GCTGGCTAAGTAAAGATTTTAAATCAAGTTGAATTAAGGGGATTAATAGGAAAATTATGA<br>4381 CCTCTTCCTTTAGGAGGCAGTTATCCNAAAGACATGTCTATTAAGGTGATATATTTTAAA<br>4441 ATATCTTTGAGTGTGTTCCTGGCAGTTTAAGAAAATAGTTGGAGAATTTAGGTTTTTATT<br>4501 AGTACACAGTACCATTTATACAAATTAGAAAATGTTATTTAACAGCTATTGAATTATCTA<br>4561 TATACACCTTTATTAATCGCTATTGTTCCAGCAGTTTTAACGTTGAACGAATAATCTTAT<br>4621 TAGGGAGAAAATTCAATTGTAAATTGAATCAGTATAAACAAAGTTACTAGGTAACTTCAC<br>4681 ATTGCTCTGAAAGAAATATGGAACTTACACTGTTCAATTAGAATAGTGTTCTGCAAAAAT<br>4741 ATTTATAAAACCTCTCAGATACTGCTACTGTAATTTTATATGAAAATAAGTGTATTTTTC<br>4801 AATAAAGCATTTATAAATTAAAAAAAAAAAAAAAAAAAACAAAAAAAAAAAAAAA | |
| | | 1 M P S G G D Q S P P P P P P P P A A A A<br>1 ATGCCGTCGGGAGGTGACCAGTCGCCACCGCCCCCGCCTCCCCCTCCGGCGGCGGCAGCC<br><br>21 S D E E E E D D G E A E D A A P P A E S | SEQ ID NO:8 |

| Gene Name | GenBank Homology | DNA SEQUENCE / DEDUCED AMINO ACID SEQUENCE | SEQUENCE IDENTIFIER: |
|---|---|---|---|
| | | 61 TCGGATGAGGAGGAGGAGGACGACGGCGAGGCGGAAGACGCCGCGCCGCCTGCCGAGTCG<br><br>41 P T P Q I Q Q R F D E L C S R L N M D E<br>121 CCCACCCCTCAGATCCAGCAGCGGTTCGACGAGCTGTGCAGCCGCCTCAACATGGACGAG<br><br>61 A A R A E A W D S Y R S M S E S Y T L E<br>181 GCGGCGCGGGCCGAGGCCTGGGACAGCTACCGCAGCATGAGCGAAAGCTACACGCTGGAG<br><br>81 G N D L H W L A C A L Y V A C R K S V P<br>241 GGAAATGATCTTCATTGGTTAGCATGTGCCTTATATGTGGCTTGCAGAAAATCTGTTCCA<br><br>101 T V S K G T V E G N Y V S L T R I L K C<br>301 ACTGTAAGCAAAGGGACAGTGGAAGGAAACTATGTATCTTTAACTAGAATCCTGAAATGT<br><br>121 S E Q S L I E F F N K M K K W E D M A N<br>361 TCAGAGCAGAGCTTAATCGAATTTTTAATAAGATGAAGAAGTGGGAAGACATGGCAAAT<br><br>141 L P P H F R E R T E R L E R N F T V S A<br>421 CTACCCCCACATTTCAGAGAACGTACTGAGAGATTAGAAAGAAACTTCACTGTTTCTGCT<br><br>161 V I F K K Y E P I F Q D I F K Y P Q E E<br>481 GTAATTTTTAAGAAATATGAACCCATTTTTCAGGACATCTTTAAATACCCTCAAGAGGAG<br><br>181 Q P R Q Q R G R K Q R R Q P C T V S E I<br>541 CAACCTCGTCAGCAGCGAGGAAGGAAACAGCGGCGACAGCCCTGTACTGTGTCTGAAATT<br><br>201 F H F C W V L F I Y A K G N F P M I S D<br>601 TTCCATTTTTGTTGGGTGCTTTTTATATATGCAAAAGGTAATTTCCCCATGATTAGTGAT<br><br>221 D L V N S Y H L L L C A L D L V Y G N A<br>661 GATTTGGTCAATTCTTATCACCTGCTGCTGTGTGCTTTGGACTTAGTTTATGGAAATGCA<br><br>241 L Q C S N R K E L V N P N F K G L S E D<br>721 CTTCAGTGTTCTAATCGTAAAGAACTTGTGAACCCTAATTTTAAAGGCTTATCTGAAGAT<br><br>261 F H A K D S K P S S D P P C I I E K L C<br>781 TTTCATGCTAAAGATTCTAAACCTTCCTCTGACCCCCCTTGTATCATTGAGAAACTGTGT<br><br>281 S L H D G L V L E A K G I K E H F W K P<br>841 TCCTTACATGATGGCCTAGTTTTGGAAGCAAAGGGGATAAAGGAACATTTCTGGAAACCC<br><br>301 Y I R K L Y E K K L L K G K E E N L T G<br>901 TATATTAGGAAACTTTATGAAAAAAAGCTCCTTAAGGGAAAAGAAGAAAATCTCACTGGG | |

| Gene Name | GenBank Homology | DNA SEQUENCE / DEDUCED AMINO ACID SEQUENCE | SEQUENCE IDENTIFIER: |
|---|---|---|---|
| | | 321 F L E P G N F G E S F K A I N K A Y E E<br>961 TTTCTAGAACCTGGGAACTTTGGAGAGAGTTTTAAAGCCATCAATAAGGCCTATGAGGAG<br><br>341 Y V L S V G N L D E R I F L G E D A E E<br>1021 TATGTTTTATCTGTTGGGAATTTAGATGAGCGGATATTTCTTGGAGAGGATGCTGAGGAG<br><br>361 E I G T L S R C L N A G S G T E T A E R<br>1081 GAAATTGGGACTCTCTCAAGGTGTCTGAACGCTGGTTCAGGAACAGAGACTGCTGAAAGG<br><br>381 V Q M K N I L Q Q H F D K S K A L R I S<br>1141 GTGCAGATGAAAAACATCTTACAGCAGCATTTTGACAAGTCCAAAGCACTTAGAATCTCC<br><br>401 T P L T G V R Y I K E N S P C V T P V S<br>1201 ACACCACTAACTGGTGTTAGGTACATTAAGGAGAATAGCCCTTGTGTGACTCCAGTTTCT<br><br>421 T A T H S L S R L H T M L T G L R N A P<br>1261 ACAGCTACGCATAGCTTGAGTCGTCTTCACACCATGCTGACAGGCCTCAGGAATGCACCA<br><br>441 S E K L E Q I L R T C S R D P T Q A I A<br>1321 AGTGAGAAACTGGAACAGATTCTCAGGACATGTTCCAGAGATCCAACCCAGGCTATTGCT<br><br>461 N R L K E M F E I Y S Q H F Q P D E D F<br>1381 AACAGACTGAAAGAAATGTTTGAAATATATTCTCAGCATTTCCAGCCAGACGAGGATTTC<br><br>481 S N C A K E I A S K H F R F A E M L Y Y<br>1441 AGTAATTGTGCTAAAGAAATTGCCAGCAAACATTTTCGTTTTGCGGAGATGCTTTACTAT<br><br>501 K V L E S V I E Q E Q K R L G D M D L S<br>1501 AAAGTATTAGAATCTGTTATTGAGCAGGAACAAAAAAGACTAGGAGACATGGATTTATCT<br><br>521 G I L E Q D A F H R S L L A C C L E V V<br>1561 GGTATTCTGGAACAAGATGCGTTCCACAGATCTCTCTTGGCCTGCTGCCTTGAGGTCGTC<br><br>541 T F S Y K P P G N F P F I T E I F D V P<br>1621 ACTTTTTCTTATAAGCCTCCTGGGAATTTTCCATTTATTACTGAAATATTTGATGTGCCT<br><br>561 L Y H F Y K V I E V F I R A E D G L C R<br>1681 CTTTATCATTTTTATAAGGTGATAGAAGTATTCATTAGAGCAGAAGATGGCCTTTGTAGA<br><br>581 E V V K H L N Q I E E Q I L D H L A W K<br>1741 GAGGTGGTAAAACACCTTAATCAGATTGAAGAACAGATCTTAGATCATTTGGCATGGAAA | |

| Gene Name | GenBank Homology | DNA SEQUENCE / DEDUCED AMINO ACID SEQUENCE | SEQUENCE IDENTIFIER: |
|---|---|---|---|
| | | 601 P E S P L W E K I R D N E N R V P T C E<br>1801 CCAGAGTCTCCACTCTGGGAAAAAATTAGAGACAATGAAAACAGAGTTCCTACATGTGAA<br><br>621 E V M P P Q N L E R A D E I C I A G S P<br>1861 GAGGTCATGCCACCTCAGAACCTGGAAAGGGCAGATGAAATTTGCATTGCTGGCTCCCCT<br><br>641 L T P R R V T E V R A D T G G L G R S I<br>1921 TTGACTCCCAGAAGGGTGACTGAAGTTCGTGCTGATACTGGAGGACTTGGAAGGAGCATA<br><br>661 T S P T T L Y D R Y S S P P A S T T R R<br>1981 ACATCTCCAACCACATTATACGATAGGTACAGCTCCCCACCAGCCAGCACTACCAGAAGG<br><br>681 R L F V E N D S P S D G G T P G R M P P<br>2041 CGGCTATTTGTTGAGAATGATAGCCCCTCTGATGGAGGGACGCCTGGGCGCATGCCCCCA<br><br>701 Q P L V N A V P V Q N V S G E T V S V T<br>2101 CAGCCCCTAGTCAATGCTGTCCCTGTGCAGAATGTATCTGGGGAGACTGTTTCTGTCACA<br><br>721 P V P G Q T L V T M A T A T V T A N N G<br>2161 CCAGTTCCTGGACAGACTTTGGTCACCATGGCAACCGCCACTGTCACAGCCAACAATGGG<br><br>741 Q T V T I P V Q G I A N E N G G I T F F<br>2221 CAAACGGTAACCATTCCTGTGCAAGGTATTGCCAATGAAAATGGAGGGATAACATTCTTC<br><br>761 P V Q V N V G G Q A Q A V T G S I Q P L<br>2281 CCTGTCCAAGTCAATGTTGGGGGGCAGGCACAAGCTGTGACAGGCTCCATCCAGCCCCTC<br><br>781 S A Q A L A G S L S S Q Q V T G T T L Q<br>2341 AGTGCTCAGGCCCTGGCTGGAAGTCTGAGCTCTCAACAGGTGACAGGAACAACTTTGCAA<br><br>801 V P G Q V A I Q Q I S P G G Q Q Q K Q G<br>2401 GTCCCTGGTCAAGTGGCCATTCAACAGATTTCCCCAGGTGGCCAACAGCAGAAGCAAGGC<br><br>821 Q S V T S S S N R P R K T S S L S L F F<br>2461 CAGTCTGTAACCAGCAGTAGTAATAGACCCAGGAAGACCAGCTCTTTATCGCTTTTCTTT<br><br>841 R K V Y H L A A V R L R D L C A K L D I<br>2521 AGAAAGGTATACCATTTAGCAGCTGTCCGCCTTCGGGATCTCTGTGCCAAACTAGATATT<br><br>861 S D E L R K K I W T C F E F S I I Q C P<br>2581 TCAGATGAATTGAGGAAAAAAATCTGGACCTGCTTTGAATTCTCCATAATTCAGTGTCCT<br><br>881 E L M M D R H L D Q L L M C A I Y V M A | |

| Gene Name | GenBank Homology | DNA SEQUENCE / DEDUCED AMINO ACID SEQUENCE | SEQUENCE IDENTIFIER: |
|---|---|---|---|
| | | 2641 GAACTTATGATGGACAGACATCTGGACCAGTTATTAATGTGTGCCATTTATGTGATGGCA<br><br>901 K V T K E D K S F Q N I M R C Y R T Q P<br>2701 AAGGTCACAAAAGAAGATAAGTCCTTCCAGAACATTATGCGTTGTTATAGGACTCAGCCG<br><br>921 Q A R S Q V Y R S V L I K G K R K R R N<br>2761 CAGGCCCGGAGCCAGGTGTATAGAAGTGTTTTGATAAAAGGAAAAAGAAAAAGAAGAAAT<br><br>941 S G S S D S R S H Q N S P T E L N K D R<br>2821 TCTGGCAGCAGTGATAGCAGAAGCCATCAGAATTCTCCAACAGAACTAAACAAAGATAGA<br><br>961 T S R D S S P V M R S S S T L P V P Q P<br>2881 ACCAGTAGAGACTCCAGTCCAGTTATGAGGTCAAGCAGCACCTTGCCAGTTCCACAGCCC<br><br>981 S S A P P T P T R L T G A N S D M E E E<br>2941 AGCAGTGCTCCTCCCACACCTACTCGCCTCACAGGTGCCAACAGTGACATGGAAGAAGAG<br><br>1001 E R G D L I Q F Y N N I Y I K Q I K T F<br>3001 GAGAGGGGAGACCTCATTCAGTTCTACAACAACATCTACATCAAACAGATTAAGACATTT<br><br>1021 A M K Y S Q A N M D A P P L S P Y P F V<br>3061 GCCATGAAGTACTCACAGGCAAATATGGATGCTCCTCCGCTCTCTCCCTATCCATTTGTA<br><br>1041 R T G S P R R I Q L S Q N H P V Y I S P<br>3121 AGAACAGGCTCTCCTCGCCGAATACAGTTGTCTCAAAATCATCCTGTCTACATTTCCCCA<br><br>1061 H K N E T M L S P R E K I F Y Y F S N S<br>3181 CATAAAAATGAAACAATGCTTTCTCCTCGTGAAAAGATTTTCTACTACTTCAGCAACAGT<br><br>1081 P S K R L R E I N S M I R T G E T P T K<br>3241 CCTTCAAAGAGACTAAGAGAAATTAACAGTATGATCCGGACAGGAGAAACTCCAACCAAA<br><br>1101 K R G I L L E D G S E S P A K R I C P E<br>3301 AAGCGAGGGATTCTTTTGGAAGACGGAAGTGAATCACCTGCAAAAAGAATTTGCCCAGAA<br><br>1121 N H S A L L R R L Q D V A N D R G S H -<br>3361 AATCATTCTGCCTTACTGCGCCGTCTACAAGATGTAGCTAATGACCGAGGTTCCCACTGA | |
| WBC003D11.bFSP_20 011309.abd | No Homology | 1 GGACCTTTGGCACAAACACTGAAAAGAGTTATGTTGATAATCAGAAGTGATTGATGGAGC<br>61 AGAATTCCAGCGTTTCATGGAGAGTTGAAGTACATGATGACAAAACCCTTGGGAAAACCT<br>121 TCTAATTTCTTGGGACATTTCAAGGAAGCAACATCCCCCTTTCTTCCAGGAACTTTTTTG<br>181 TGACTTGACTAGGACTCGCACTGCTCCCAAAGTTATCGCCATCCCACCTTGCTCTCCGTC | SEQ ID NO:9 |

| Gene Name | GenBank Homology | DNA SEQUENCE / DEDUCED AMINO ACID SEQUENCE | SEQUENCE IDENTIFIER: |
|---|---|---|---|
| | | 241 TCCCATAAAATCTTGAGGGCCACGGAGAGAAGGAGGCGAATTTTAAATGTAGATCTCCTC<br>301 CCTTCCACAGCCAGTCTCAAACACACCTACTCAAGGTGTGGTCCATGGACCAGAAGCATG<br>361 AGCATCATTTTGTTAGAAAAGCAGCATCAAAGGTACCACCGCAGATCTTCGGAACCAGAA<br>421 CCTGCATTTTAACAAGATCCCCAGGGAATTTGTGTTGTACACAGGAGTGAATGTTCTGGC<br>481 ATATTCTAGAGTATTCTTTTGTTTTCCATGCTGACAGAAGAGCTTGTCTTTAAAACTAGG<br>541 CAGGAAAACATATCCTCGACTTTGGGACTTTGCATCTGCCATTCTCTCTTCCTAGGACAA<br>601 CTTTCCTCANAATCGGACTAATTTCTACCCTCTCTTCGAGTCTCAGCATAGTCATCAATT<br>661 TCACCAGGAAGCATTCTCTGAATCTCTTGAAGTAGGATTAGGTGTCACCTCTGTGTGTCC<br>721 TCTAAGGCTTCATGATTTCTAGCCTTGTGTGATCATTTCTTCTCTTCTTGAGTGTCTCCA<br>781 ACTGGACTGTGAGCTCCATAATTACAGGGAATGGCATTCAGTTGATTCTCCATAAATAAG<br>841 TTGAATGAATGAATGAATGATTACTTTCCATTTTGAAGATGAAGAATTTGAGGC<br><br>1 ACCATTCCTGCTCTCACTATGGCTTGGCCTTGAGACTTGCTTTGGCTAATGGAATGTCAG<br>61 CAAGTGTGACTCAGACAGAGTGTGCATTCCTTCTTGCCCGTTACACGATAAGTCTCGCTT<br>121 GTTCTTGCACCCCTGCCTCCGCCATGAGAATGTGCCCAGGCTGAACTGCTGGAGGGATGT<br>181 GAAAAATGTATGGAAGACAGTTAAGTCATTCCATTATACCAGCTGAGGTTGTCCTTAATC<br>241 AGCCATCGGACCAACTTGTAGCTGACTACAGTCACATCCATGAGCCCAGTCAAGATCAGC<br>301 AGACCTGCCCGGGCAACCCAGACTCAGGAGCCACAATCAAGTACCATTATTTTAGGGCAC<br>361 AGAGTTTCAGGAGTGGTTTGTTAACGAAGTATTCTTGGTGGAAGTTGCTAACTGATTCAG<br>421 CAACCAATGGATTGTCAATGTTTTGCATTTGACTGCAACAATTTGGAACATACAAAGAGG<br>481 GTGATTCGCATCTTCATTTCCTCTTAGCTGTCAGGATCTGTAAATCTAAAACGCTTTGAA<br>541 TAAAGTTCTTTCTGACAGAAAAAAAAAAA | SEQ ID NO:10 |
| WBC004E04.bFSP_20 011309.abd | Homo sapiens TRAF-interacting protein with a forkhead-associated domain, mRNA (cDNA clone MGC:20791 IMAGE:4763971) | 1 gagaggacgt gctctgccag ccagtgggaa ggcaggccgc gcgcgcggga gcgcgggagg<br>61 atcggcggct cgcggtcact ggtccctggc tcggttcccc gcaccccggg gctcacactt<br>121 acccgcgcgg aggagcagcg gccgggtgtc cacccccatc ctgcgcccag tctcctcgat<br>181 tcccctcgct ctgagccggg agagccgaac agctgaagag agttcactga ctccccagcc<br>241 ccaggtgggc cttgtgcaca tcatgaccag ttttgaagat gctgacacag aagagacagt<br>301 aacttgtctc cagatgacgg tttaccatcc tggccagttg cagtgtggaa tatttcagtc<br>361 aataagtttt aacagagaga aactcccttc cagcgaagtg gtgaaatttg gccgaaattc<br>421 caacatctgt cattatactt ttcaggacaa acaggtttcc cgagttcagt tttctctgca<br>481 gctgtttaaa aaattcaaca gctcagttct ctcctttgaa ataaaaaata tgagtaaaaa<br>541 gaccaatctg atcgtggaca gcagagagct gggctaccta aataaaatgg acctgccata<br>601 caggtgcatg gtcagattcg gagagtatca gtttctgatg gagaaggaag atggcgagtc<br>661 attggaattt tttgagactc aatttatttt atctccaaga tcactcttgc aagaaaacaa<br>721 ctggccacca cacaggccca taccggagta tggcacttac tcgctctgct cctcccaaag<br>781 cagttctccg acagaaatgg atgaaaatga gtcatgaaca cagaaagtct aagaggagaa<br>841 atatgatgga tgaagagctc tgtagatgct gtatagacac taaataagag ttgattaggg<br>901 tagtatatta tagtcatctg ttatgctgtg aaatttggaa ttcaaaattt tgaagtctgt<br>961 aaattgtgtt agtcattaac ttagtcacct gttgtattct ggatctacac aaaattattt<br>1021 taagtgctct tattaatctg tgaggattaa tatacaaaaa gtatcctttg agatgaagtc<br>1081 gtgttctcaa aataaggtta tattattttc tttttctgct tgattttcat cttgtgtttt | SEQ ID NO:11 |

| Gene Name | GenBank Homology | DNA SEQUENCE / DEDUCED AMINO ACID SEQUENCE | SEQUENCE IDENTIFIER: |
|---|---|---|---|
| | | 1141 gctttgtttt tgtaaggaac catctcttgg tttggtcaca tcagttcaca acagccattt<br>1201 gttttcaagg tcaaggctcc aggcaggttg ttactggtgt ttgcagcctg tcagtacttg<br>1261 cagtactgga ataggttcta ggctagtgtc tgcgcgtcac tgtggtttta gcatgggagg<br>1321 acttatttga gaaatactac cttacttttc tatgatttct ttttacagag ttatagtgtg<br>1381 tttactccta agatgacagt tctctttgtc tatattcagc atctaagaca aatatttaaa<br>1441 cattttaaag aaccactgtg ttaagtttag gattatttac ttaccaaatt agaagtttga<br>1501 cttttatgtg ttatacacaa tcttaaaatt tcacgaattc accttttttaa tagtatccat<br>1561 gtacataata aaatcaaagt ttaattaaaa aaaaaaaaaa aaaa | |
| | | 1 M T S F E D A D T E E T V T C L Q M T V<br>1 ATGACCAGTTTTGAAGATGCTGACACAGAAGAGACAGTAACTTGTCTCCAGATGACGGTT<br><br>21 Y H P G Q L Q C G I F Q S I S F N R E K<br>61 TACCATCCTGGCCAGTTGCAGTGTGGAATATTTCAGTCAATAAGTTTTAACAGAGAGAAA<br><br>41 L P S S E V V K F G R N S N I C H Y T F<br>121 CTCCCTTCCAGCGAAGTGGTGAAATTTGGCCGAAATTCCAACATCTGTCATTATACTTTT<br><br>61 Q D K Q V S R V Q F S L Q L F K K F N S<br>181 CAGGACAAACAGGTTTCCCGAGTTCAGTTTTCTCTGCAGCTGTTTAAAAAATTCAACAGC<br><br>81 S V L S F E I K N M S K K T N L I V D S<br>241 TCAGTTCTCTCCTTTGAAATAAAAAATATGAGTAAAAAGACCAATCTGATCGTGGACAGC<br><br>101 R E L G Y L N K M D L P Y R C M V R F G<br>301 AGAGAGCTGGGCTACCTAAATAAAATGGACCTGCCATACAGGTGCATGGTCAGATTCGGA<br><br>121 E Y Q F L M E K E D G E S L E F F E T Q<br>361 GAGTATCAGTTTCTGATGGAGAAGGAAGATGGCGAGTCATTGGAATTTTTTGAGACTCAA<br><br>141 F I L S P R S L L Q E N N W P P H R P I<br>421 TTTATTTTATCTCCAAGATCACTCTTGCAAGAAAACAACTGGCCACCACACAGGCCCATA<br><br>161 P E Y G T Y S L C S S Q S S S P T E M D<br>481 CCGGAGTATGGCACTTACTCGCTCTGCTCCTCCCAAAGCAGTTCTCCGACAGAAATGGAT<br><br>181 E N E S -<br>541 GAAAATGAGTCATGA | SEQ ID NO:12 |
| WBC004G01.bFSP_20 011309.abd | Homo sapiens neural precursor cell expressed, developmentally | 1 agcggagggg ggagccggaa gcccagcgcg gagccggccg cggccccctg ttgtgttgct<br>61 gcggagaggt acttggatgc attttacagg ttagcctcac ttgagctgtt gtcctgcaag<br>121 taaagtgtat ttttggtgat tgaaagttgg agaactttca tttcagctga gtggtgtagt | SEQ ID NO:13 |

| Gene Name | GenBank Homology | DNA SEQUENCE / DEDUCED AMINO ACID SEQUENCE | SEQUENCE IDENTIFIER: |
|---|---|---|---|
| | down-regulated 1 | 181 tgaattggtt cctgtagccg ctgtccctaa acccaggccg acgttaccgc cttgtgtcct<br>241 gactgctagc tttcgacggg accgtctttg agggactcat gtaaagtctc tcccttaatg<br>301 ctcagttctt agaagaccga ggcgcagtca tgcaggaaaa cctcagattt gcttcatcag<br>361 gagatgatat taaaatatgg gatgcttcat ctatgacatt ggtggataaa ttcaacccac<br>421 acacatcacc acatggaatc agctcaatat gttggagcag caataataac tttttagtaa<br>481 cagcatcttc cagtggcgac aaaatagttg tctcaagttg caaatgtaaa cctgttccac<br>541 ttttagagct tgctgaaggg caaaagcaga catgtgtcaa tttaaattct acatctatgt<br>601 atttggtaag cggaggccta aataacactg ttaatatttg ggatttaaaa tcaaaaagag<br>661 ttcatcgatc tcttaaggat taaagatcaa gtaacttgtg taacatacaa ttggaatgat<br>721 tgctacattg cttctggatc tcttagtggt gaaattattt tacacagtgt aaccactaat<br>781 ttatctagta ctccttttgg ccatggtagt aaccagtctg ttcggcactt gaagtactcc<br>841 ttgtttaaga aatcactact gggcagtgtt tcggataatg gaatagtaac tctctgggat<br>901 gtaaatagtc agagtccata ccataacttt gacagtgtac acaaagctcc agcgtcaggc<br>961 atctgttttt ctcctgtcaa tgaattgctc tttgtaacca taggcttgga taaaagaatc<br>1021 atcctctatg acacttcaag taagaagcta gtgaaaactt tagtggctga cactcctcta<br>1081 actgcggtag atttcatgcc tgatggagcc actttggcta ttggatcttc ccgggggaaa<br>1141 atatatcaat atgatttaag aatgttgaaa tcaccagtta agaccatcag tgctcacaag<br>1201 acatctgtgc agtgtatagc atttcagtac tccactgttc ttactaagtc aagtttaaat<br>1261 aaaggctgtt caaataagcc cacaacagtg aacaaacgaa gtgttaatgt gaatgctgct<br>1321 agtggaggag ttcagaattc cggaattgtc agagaagcac ctgccacgtc cattgccaca<br>1381 gttctaccac aacctatgac atcagctatg gggaaaggaa cagttgctgt tcaagaaaaa<br>1441 gcaggtttgc ctcgaagcat aaacacagac actttatcta aggaaacaga cagtggaaaa<br>1501 aatcaggatt tctccagctt tgatgatact gggaaaagta gtttaggtga catgttctca<br>1561 cctatcagag atgatgctgt agttaacaag ggaagtgatg agtccatagg caaaggagat<br>1621 ggctttgact ttctaccgca gttgaactca gtgtttcctc caagaaaaaa tccagtaact<br>1681 tcaagtactt cagtattgca ttctagtcct cttaatgttt tatgggatct ccagggaaag<br>1741 aggaaaatga aaaccgtgat ctaacagctg agtctaagaa aatatatatg ggaaaacagg<br>1801 aatctaaaga ctccttcaaa cagttagcaa agttggtcac atctggtgct gaaagtggaa<br>1861 atctaaatac ctctccatca tctaaccaaa caagaaattc tgagaaattt gaaaagccag<br>1921 agaatgaaat tgaagcccag ttgatatgtg aaccccccaat caatggatcc tcaactccaa<br>1981 atccaaagat agcatcttct gtcactgctg gagttgccag ttcactctca gaaaaaatag<br>2041 ccgacagcat tggaaataac cggcaaaatg caccattgac ttccattcaa attcgtttta<br>2101 ttcagaacat gatacaggaa acgttggatg actttagaga agcatgccat agggacattg<br>2161 tgaatttgca agtggagatg attaaacagt ttcatatgca actgaatgaa atgcattctt<br>2221 tgctggaaag atactcagtg aatgaaggtt tagtggctga aattgaaaga ctacgagaag<br>2281 aaaacaaaag attacgggcc cacttttgaa atttcagtga ataccttaat gttctgtaat<br>2341 ttgggaagtt tctggcaaca cagaactaca tagaatcagt attgttttca tggcctccag<br>2401 ggaaaaaatg tttttcaagt aagagtaaaa gggtgatggg attttatacc aacaactgtt<br>2461 tcatcttaaa aatatgtata tttttatatt aaaaattgta cagtatgtca tctacccaat<br>2521 aggaaagtca acaggatctt tatttttgga aagctttagc catccactaa gtgccctttt<br>2581 tcataagaga agaaaattgt gcataaaaat tggttatgtt tgttttttag tcatcttttt<br>2641 taacatatat ttttgattga caaattgcct ttcaaatttt tggggctagt tgagatttaa<br>2701 agagtttgat atgccttcta tttttatgga gaaagtaatt ttaaaatggc aattggtgtt | |

| Gene Name | GenBank Homology | DNA SEQUENCE / DEDUCED AMINO ACID SEQUENCE | SEQUENCE IDENTIFIER: |
|---|---|---|---|
| | | 2761 tctaagccat tgactaataa aacatagggt tggctagtaa ttattttgtt aacttgatga<br>2821 agtcaagtat gactattatt tattgtacat ttgataagac aatttttgga attttgaatt<br>2881 gcacaaatta catgatatct tttgcattta tgttactata ttgtacttct gacaaatctt<br>2941 tattcctggg tggtattttt aagatatctt tacctataaa aagtgtttaa ggttcatagg<br>3001 actcgacaag agctatctgg tgatttctc attagtaaca tgcaacgttg tactgcaaaa<br>3061 tttcaatcaa catgacaact tataatgagt ggagatttca tattaggtac taaatattat<br>3121 agtattattt ctattttctt tttccaaata agaagcttgg attattttat tttgtggtct<br>3181 ttatcattaa ctttaattct ttctgtactg tgtataatat ttttatatta ttggccttac<br>3241 cataaaatta tttagaaagg ttgtcaaaat aagttatacc tctttggcaa tagatagatg<br>3301 tgtacatcta cctactatga tctacaattt taggttaagt gaagcttggg ggggctactg<br>3361 acttggttac cttcttgtct cttgtcccaa agatttaaac tatgtacctt tgtatagctc<br>3421 ttctgcccca ttttgacttc tgagatgaaa gtatttacta aaattaaaaa aaaaaaaaca<br>3481 aaaaacaaac ctttagctca ctaactttat gggtttctga agtgatggaa atttttaagg<br>3541 atatatttaa taagcataaa cttactaata attacttcca aaaataaaaa caggaatatt<br>3601 acttttaaag aaaaaaaaaa aaa | |
| | | 1 M Q E N L R F A S S G D D I K I W D A S<br>1 ATGCAGGAAAACCTCAGATTTGCTTCATCAGGAGATGATATTAAAATATGGGATGCTTCA<br><br>21 S M T L V D K F N P H T S P H G I S S I<br>61 TCTATGACATTGGTGGATAAATTCAACCCACACACATCACCACATGGAATCAGCTCAATA<br><br>41 C W S S N N N F L V T A S S S G D K I V<br>121 TGTTGGAGCAGCAATAATAACTTTTTAGTAACAGCATCTTCCAGTGGCGACAAAATAGTT<br><br>61 V S S C K C K P V P L L E L A E G Q K Q<br>181 GTCTCAAGTTGCAAATGTAAACCTGTTCCACTTTTAGAGCTTGCTGAAGGGCAAAAGCAG<br><br>81 T C V N L N S T S M Y L V S G G L N N T<br>241 ACATGTGTCAATTTAAATTCTACATCTATGTATTTGGTAAGCGGAGGCCTAAATAACACT<br><br>101 V N I W D L K S K R V H R S L K D H K D<br>301 GTTAATATTTGGGATTTAAAATCAAAAAGAGTTCATCGATCTCTTAAGGATCATAAAGAT<br><br>121 Q V T C V T Y N W N D C Y I A S G S L S<br>361 CAAGTAACTTGTGTAACATACAATTGGAATGATTGCTACATTGCTTCTGGATCTCTTAGT<br><br>141 G E I I L H S V T T N L S S T P F G H G<br>421 GGTGAAATTATTTTACACAGTGTAACCACTAATTTATCTAGTACTCCTTTTGGCCATGGT<br><br>161 S N Q S V R H L K Y S L F K K S L L G S<br>481 AGTAACCAGTCTGTTCGGCACTTGAAGTACTCCTTGTTTAAGAAATCACTACTGGGCAGT | SEQ ID NO:14 |

| Gene Name | GenBank Homology | DNA SEQUENCE / DEDUCED AMINO ACID SEQUENCE | SEQUENCE IDENTIFIER: |
|---|---|---|---|
| | | 181 V S D N G I V T L W D V N S Q S P Y H N<br>541 GTTTCGGATAATGGAATAGTAACTCTCTGGGATGTAAATAGTCAGAGTCCATACCATAAC<br><br>201 F D S V H K A P A S G I C F S P V N E L<br>601 TTTGACAGTGTACACAAAGCTCCAGCGTCAGGCATCTGTTTTTCTCCTGTCAATGAATTG<br><br>221 L F V T I G L D K R I I L Y D T S S K K<br>661 CTCTTTGTAACCATAGGCTTGGATAAAAGAATCATCCTCTATGACACTTCAAGTAAGAAG<br><br>241 L V K T L V A D T P L T A V D F M P D G<br>721 CTAGTGAAAACTTTAGTGGCTGACACTCCTCTAACTGCGGTAGATTTCATGCCTGATGGA<br><br>261 A T L A I G S S R G K I Y Q Y D L R M L<br>781 GCCACTTTGGCTATTGGATCTTCCCGGGGGAAAATATATCAATATGATTTAAGAATGTTG<br><br>281 K S P V K T I S A H K T S V Q C I A F Q<br>841 AAATCACCAGTTAAGACCATCAGTGCTCACAAGACATCTGTGCAGTGTATAGCATTTCAG<br><br>301 Y S T V L T K S S L N K G C S N K P T T<br>901 TACTCCACTGTTCTTACTAAGTCAAGTTTAAATAAAGGCTGTTCAAATAAGCCCACAACA<br><br>321 V N K R S V N V N A A S G G V Q N S G I<br>961 GTGAACAAACGAAGTGTTAATGTGAATGCTGCTAGTGGAGGAGTTCAGAATTCCGGAATT<br><br>341 V R E A P A T S I A T V L P Q P M T S A<br>1021 GTCAGAGAAGCACCTGCCACGTCCATTGCCACAGTTCTACCACAACCTATGACATCAGCT<br><br>361 M G K G T V A V Q E K A G L P R S I N T<br>1081 ATGGGGAAAGGAACAGTTGCTGTTCAAGAAAAAGCAGGTTTGCCTCGAAGCATAAACACA<br><br>381 D T L S K E T D S G K N Q D F S S F D D<br>1141 GACACTTTATCTAAGGAAACAGACAGTGGAAAAAATCAGGATTTCTCCAGCTTTGATGAT<br><br>401 T G K S S L G D M F S P I R D D A V V N<br>1201 ACTGGGAAAAGTAGTTTAGGTGACATGTTCTCACCTATCAGAGATGATGCTGTAGTTAAC<br><br>421 K G S D E S I G K G D G F D F L P Q L N<br>1261 AAGGGAAGTGATGAGTCCATAGGCAAAGGAGATGGCTTTGACTTTCTACCGCAGTTGAAC<br><br>441 S V F P P R K N P V T S S T S V L H S S<br>1321 TCAGTGTTTCCTCCAAGAAAAAATCCAGTAACTTCAAGTACTTCAGTATTGCATTCTAGT | |

| Gene Name | GenBank Homology | DNA SEQUENCE / DEDUCED AMINO ACID SEQUENCE | SEQUENCE IDENTIFIER: |
|---|---|---|---|
| | | 461 P L N V F M G S P G K E E N E N R D L T<br>1381 CCTCTTAATGTTTTTATGGGATCTCCAGGGAAAGAGGAAAATGAAAACCGTGATCTAACA<br><br>481 A E S K K I Y M G K Q E S K D S F K Q L<br>1441 GCTGAGTCTAAGAAAATATATATGGGAAAACAGGAATCTAAAGACTCCTTCAAACAGTTA<br><br>501 A K L V T S G A E S G N L N T S P S S N<br>1501 GCAAAGTTGGTCACATCTGGTGCTGAAAGTGGAAATCTAAATACCTCTCCATCATCTAAC<br><br>521 Q T R N S E K F E K P E N E I E A Q L I<br>1561 CAAACAAGAAATTCTGAGAAATTTGAAAAGCCAGAGAATGAAATTGAAGCCCAGTTGATA<br><br>541 C E P P I N G S S T P N P K I A S S V T<br>1621 TGTGAACCCCCAATCAATGGATCCTCAACTCCAAATCCAAAGATAGCATCTTCTGTCACT<br><br>561 A G V A S S L S E K I A D S I G N N R Q<br>1681 GCTGGAGTTGCCAGTTCACTCTCAGAAAAAATAGCCGACAGCATTGGAAATAACCGGCAA<br><br>581 N A P L T S I Q I R F I Q N M I Q E T L<br>1741 AATGCACCATTGACTTCCATTCAAATTCGTTTTATTCAGAACATGATACAGGAAACGTTG<br><br>601 D D F R E A C H R D I V N L Q V E M I K<br>1801 GATGACTTTAGAGAAGCATGCCATAGGGACATTGTGAATTTGCAAGTGGAGATGATTAAA<br><br>621 Q F H M Q L N E M H S L L E R Y S V N E<br>1861 CAGTTTCATATGCAACTGAATGAAATGCATTCTTTGCTGGAAAGATACTCAGTGAATGAA<br><br>641 G L V A E I E R L R E E N K R L R A H F<br>1921 GGTTTAGTGGCTGAAATTGAAAGACTACGAGAAGAAAACAAAAGATTACGGGCCCACTTT<br><br>661 -<br>1981 TGA | |
| WBC004G09.bFSP_20 011309.abd | No homology | 1 GGACGAAAAATTTACCAAATTTGCAGATAGCAAGCCTAGGAGGAATAGCTAATTCACAGG<br>61 TGATAGAATTAAGAGTTTAAAAGATTTTTTGACAAACTGGAATACTATCTGGAAATGACT<br>121 AGGATAGAAATTTGCCAAGAACAATTGAGTGGTTAGGTTTTTAAAATATTAATCAAAGAA<br>181 TATCTGTTGTGTGCTGAGTACCTGACATACATTATAGGCAGCATCTCTTGCAGGATTAGT<br>241 GATAGCCCAGTTTACAGCTGGAGAAATTAAAGTTTGGAGAAATAAGGTAACTTGTCCAAA<br>301 GCCAGAATATAGTAAATCGAAGGTACAAGTTTAATATGGAGAGTTTCTAACTTGAAAAGC<br>361 TTTCGAGTGAAAAGAATCTAGACTTCACCAGTCAGTTGCTCACAAAGTCAGTGGAAACCA<br>421 ATTAGAAAAGCTGGTAGAAAAGCTAATAAATTATAGACTGTTTGAACAGTTGTGTCCAGA<br>481 TTGAGGGAGATAATAGCCCTCCCACCATTCTCCATGCAGATCATACCACCCCAAAGTAGC | SEQ ID NO:15 |

| Gene Name | GenBank Homology | DNA SEQUENCE / DEDUCED AMINO ACID SEQUENCE | SEQUENCE IDENTIFIER: |
|---|---|---|---|
| | | 541 TCTTTAATCAGTAGGTACAAAAATCCATTATCATTATAGTATATGCTTAAAATATTTTTA<br>601 TGCACTTAGAAATGCTAAAAATTTAGATATAGCCTAAAGCAAATAAAACTTTGGCTTTTT<br>661 AAAGATTAAAGTAGGCTAGTCTTTTTTTAAAAAAAAGTAGAAAACTAGTATGCTGGCTTG<br>721 AAATATAGAGTTATGTGTCATTTCAAGTATTTTAATTGCACCTTAGTGAAATTACATATT<br>781 TTATGTAGATTTATTTGAGTACTATTGAATGTATTTCTTTATTACAATATTACTGGAAT | |
| | | 1 TTTGGAGAAATAAGGTAACTTGTCCAAAGCCAGAATATAGTAAATCGAAGGTACAAGTTT<br>61 AATATGGAGAGTTTCTAACTTGAAAAGCTTTCGAGTGAAAAGAATCTAGACTTCACCAGT<br>121 CAGTTGCTCACAAAGTCAGTGGAAACCAATTAGAAAAGCTGGTAGAAAAGCTAATAAATT<br>181 ATAGACTGTTTGAACAGTTGTGTCCAGATTGAGGGAGATAATAGCCCTCCCACCATTCTC<br>241 CATGCAGATCATACCACCCCAAAGTAGCTCTTTAATCAGTAGGTACAAAAATCCATTATC<br>301 ATTATAGTATATGCTTAAAATATTTTTATGCACTTAGAAATGCTAAAAATTTAGATATAG<br>361 CCTAAAGCAAATAAAACTTTGGCTTTTTAAAGATTAAAGTAGGCTAGTCTTTTTTTAAAA<br>421 AAAAGTAGAAAACTAGTATGCTGGCTTGAAATATAGAGTTATGTGTCATTTCAAGTATTT<br>481 TAATTGCACCTTAGTGAAATTACATATTTTATGTAGATTTATTTGAGTACTATTGAATGT<br>541 ATTTCTTTATTACAATATTACTGGAATCCTTTACACTGTTACCTGCTTAGGAATAAATAA<br>601 TTCTAAAAGAAAATGTAAAAAAAAAAAAAAAAAACTCGAG | SEQ ID NO:16 |
| WBC005D09.bFSP_20 012109.abd | No homology | 1 GCGTAAAGCATTTTTCTTAGCCTTTCCTTTGTCTTGATCTGTTGTTAACAAGATTAATAC<br>61 TGCCAGACTTAAAATATTCTACTGTTGGGCTAAAAGGAATACAACTTAGATTGCACAAAA<br>121 CTTTTGCTTTTTAGAGAAAATATAACTCAGCTGTCTTTTAAAAGAGCTGTGTTGTTATCT<br>181 ACAAGACTATTTGCAGTCTTTTTTTCAGAGCAGATTCTAACAGTTGTGAATGGTTTAAAA<br>241 AATAGAAAATTACTAAAATATTAGTTTTGGGGGTTTTATAGAGGGAGAAAAACAAAACAG<br>301 GACCAAAGTTTATGTGCCTTCTTCAGTAGTCTTAATTGACCTTTTCTTCCTATTTGAGAC<br>361 TGAGGTAGCGTCAGTATTCTGGTTTTCAGGAAATATGTACTATATAGTTTTCGAAGAATG<br>421 TTATCCCACAGGCTGTTCATAGAAGCAAAGAACTGTAACTGTGTCCATGAAGTCTCGGTT<br>481 ACACTTTTGCCTTTGTCACATAATATTCAGTGTATAGCATCGTGCAAGTCCAGGACAGAG<br>541 CTGCTCTGAAGCTTTGTGGTAGTCATCCCAGTTTTTGTAACCTGAGGCGTATGTTCCAGA<br>601 GAACAGGGATATGTGTGGTCCCGTGACCTTGGTGATGATAGTCATGATTGCAAGCTCTTT<br>661 ATGGCAAGCTTAAATCAGCACTGTAAACAGATTTTTGTTGTGTTATTTTCGGTTCTCGAA<br>721 TCTATGTAGTAGTTGTAATAATAAATATTTAAATAGCTATTTTTGATGTCATTTAAAAAA<br>781 AAATCATCCCCTGGCCTTTTTCCCCCTCTTACCATGTTAC | SEQ ID NO:17 |
| | | 1 CCTGGTCTGCCACTGTCAGCGTGACCTTGGCCTCAGTTCTCCTAGTCTCTAAGGTCCCTT<br>61 CTAACTTTCATGTCAGACAAGGTTCTTTTCATTTGACATTTTGAAAGGATAATGAGCTTT<br>121 AAAGTAAGTCTTGGTAAGGTGATATTTTAGGCAATCTTTCTTCTCCTTATCGCTTACAGT<br>181 GTTCTTAGAATTCTTTGAATATCATGAGAACCTCGTGTCTGTTCTGATTCCCTCCTCCTT<br>241 CCCTGATGCTAAATTTCCAATTGATATCAAACTGTCAACCTACCAAAAGAGATATTGTGG<br>301 CTTGTGGGTATTGCTGTCTTGTTTTTGATATGTTCTTGTATTGACTGCCCATTGGCCTGA<br>361 AAATACTCATTGTTAAACCTAAAAAAAAAATCACTTTTCTTTCCCACTGACTGTTTTTTC<br>421 TGCTTGTTGTAAGAATCAAACGAGATATTGTATGTGAAAGCACCTTGTAAACTGTAACCT<br>481 ATCAATGTAAACTGTTAAGGTGTGTTGTTATTTCATTAATTACTTCTTTGTTCAGAATGA | SEQ ID NO:18 |

| Gene Name | GenBank Homology | DNA SEQUENCE / DEDUCED AMINO ACID SEQUENCE | SEQUENCE IDENTIFIER: |
|---|---|---|---|
| | | 541 AATTTCCTACGCACTACTGTAGCTAGGAAATGCTGAAAACAACTGTGTTTTTTAATTAAT<br>601 CATAACTGCAAATTAAAGTATCTTCAAAGGATAGAAAAAAAATAAAAAAAAAAAAAAAA<br>661 AAAAAC | |
| WBC005E06.bFSP_20 012109.abd | *Homo sapiens* modulator of estrogen induced transcription, mRNA (cDNA clone IMAGE:4864860) | 1 caagtggcca aacatcagaa tcgattaaaa aaagtgaaga aaagaagcga ataagttcca<br>61 agagtccagg acatatggta atactagacc aaactaaagg agatcattgt agaccatcaa<br>121 gaagaggaag atatgagaaa attcatggaa gaagtaagga aaaggagaga gctagtctag<br>181 ataaaaaaag agataaagac tacagaagga aagagatctt gccttttgaa aagatgaagg<br>241 aacaaaggtt gagagaacat ttagttcgtt ttgaaaggct gcgacgagca atggaacttc<br>301 gaagacgaag agagattgca gagagagagc gtcgagagcg agaacgcatt agaataattc<br>361 gtgaacggga agaacgggaa cgcttacaga gagagagaga gcgcctagaa attgaaaggc<br>421 aaaaactaga gagagagaga atggaacgcg aacgcttgga aagggaacgc attcgtattg<br>481 aacaggaacg tcgtaaggaa gctgaacgga ttgctcgaga aagagaggaa ctcagaaggc<br>541 aacaacagca gcttcgttat gaacaagaaa aaaggaattc cttgaaacgc ccacgtgatg<br>601 tagatcatag gcgagatgat ccttactgga gcgagaataa aaagttgtct ctagatacag<br>661 atgcacgatt tggccatgga tccgactact ctcgccaaca gaacagattt aatgactttg<br>721 atcaccgaga gaggggcagg tttcctgaga gttcagcagt acagtcttca tcttttgaaa<br>781 ggcgggatcg ctttgttggt caaagtgagg ggaaaaaagc acgacctact gcacgaaggg<br>841 aagatccaag cttcgaaaga tatcccaaaa atttcagtga ctccagaaga aatgagcctc<br>901 caccaccaag aaatgaactt agagaatcag acaggcgaga agtacgaggg gagcgagacg<br>961 aaaggagaac ggtgattatt catgacaggc ctgatatcac tcatcctaga catcctcgag<br>1021 aggcagggcc caatccttcc agacccacca gctggaaaag tgaaggaagc atgtccactg<br>1081 acaaacggga aacaagagtt gaaaggccag aacgatctgg gagagaagta tcagggcaca<br>1141 gtgtgagagg cgctccccct gggaatcgta gcagcgcttc ggggtacggg agcagagagg<br>1201 gagacagagg agtcatcaca gaccgaggag gtggatcaca gcactatcct gaggagcgac<br>1261 atgtggttga acgccatgga cgggacacaa gcggaccaag gaaagagtgg catggtccac<br>1321 cctctcaagg gcctagctat catgatacga ggcgaatggg tgacggccgg gcaggagcag<br>1381 gcatgataac ccaacattca agtaacgcat ccccaattaa tagaattgta caaatcagtg<br>1441 gcaattccat gccaagagga agtggctccg gatttaagcc atttaagggt ggacctccgc<br>1501 gacgattctg aaaatgagct ctctgccaag gttttaagat aatttattga aatctcctgt<br>1561 aaactttact tgactactta tgaagaggac ctctgacttg cttgagagtt ctgtcagact<br>1621 tttcttttta aaaatttaac atgattgctt ttctcaattt tggagaagat gtttaaatag<br>1681 ttctgttgta acttttaata gttttgtgta tcattcaact ttttttcttg cagcaccgag<br>1741 gcacatttga aaagatggaa ttgaagtcgt tttgtttaac gctgtgtgaa tataaagagt<br>1801 agtttgcagc tgtgtggtag tggtttaatt tgcagcctta gctctgtggt gtctggctct<br>1861 agagttactt ctttttacca agcattttca gcctccattt tgaaggctgt ctacacttaa<br>1921 gaagtcttag ctgtctaatt tttagagaat aagattgttc attgcatttc tgagtattat<br>1981 gtaacctatt tttgcagaag gtactgttac attaagtgca tctgtgtatc ctggtttaaa<br>2041 aaaatgtaat ctttttgaa ataaaccttc atattctgta tagttgctaa agtgttgaga<br>2101 accttttaa ttgtaaaatg agaaccgatt ttcagtttag tgtagcagca cacttgttca<br>2161 ggtttgcatg gtatgaaacc aaatagattc atgaaacctt ggccatgagg tttgtttcac<br>2221 aaggttctta gaccgagttg tgcaggtaag tgcactttta ggtaatctgc actgtttgtt | SEQ ID NO:19 |

| Gene Name | GenBank Homology | DNA SEQUENCE / DEDUCED AMINO ACID SEQUENCE | SEQUENCE IDENTIFIER: |
|---|---|---|---|
| | | 2281 tgatggataa attccatctc tgggaattgt gtgggtatta atgtttccat gttcccaact<br>2341 atgttgagaa gtggaaaaaa acccaggttc tagatgggtg aatcagttgg gttttgtaaa<br>2401 tacttgtatg tggggaagac attgttgtct ttttgtgaaa ataaaaatcc acacctggaa<br>2461 aaaaaaaaaa aaa | |
| | | 1 M V I L D Q T K G D H C R P S R R G R Y<br>1 ATGGTAATACTAGACCAAACTAAAGGAGATCATTGTAGACCATCAAGAAGAGGAAGATAT<br><br>21 E K I H G R S K E K E R A S L D K K R D<br>61 GAGAAAATTCATGGAAGAAGTAAGGAAAAGGAGAGAGCTAGTCTAGATAAAAAAAGAGAT<br><br>41 K D Y R R K E I L P F E K M K E Q R L R<br>121 AAAGACTACAGAAGGAAAGAGATCTTGCCTTTTGAAAAGATGAAGGAACAAAGGTTGAGA<br><br>61 E H L V R F E R L R R A M E L R R R R E<br>181 GAACATTTAGTTCGTTTTGAAAGGCTGCGACGAGCAATGGAACTTCGAAGACGAAGAGAG<br><br>81 I A E R E R R E R E R I R I I R E R E E<br>241 ATTGCAGAGAGAGAGCGTCGAGAGCGAGAACGCATTAGAATAATTCGTGAACGGGAAGAA<br><br>101 R E R L Q R E R E R L E I E R Q K L E R<br>301 CGGGAACGCTTACAGAGAGAGAGAGAGCGCCTAGAAATTGAAAGGCAAAAACTAGAGAGA<br><br>121 E R M E R E R L E R E R I R I E Q E R R<br>361 GAGAGAATGGAACGCGAACGCTTGGAAAGGGAACGCATTCGTATTGAACAGGAACGTCGT<br><br>141 K E A E R I A R E R E E L R R Q Q Q Q L<br>421 AAGGAAGCTGAACGGATTGCTCGAGAAAGAGAGGAACTCAGAAGGCAACAACAGCAGCTT<br><br>161 R Y E Q E K R N S L K R P R D V D H R R<br>481 CGTTATGAACAAGAAAAAAGGAATTCCTTGAAACGCCCACGTGATGTAGATCATAGGCGA<br><br>181 D D P Y W S E N K K L S L D T D A R F G<br>541 GATGATCCTTACTGGAGCGAGAATAAAAAGTTGTCTCTAGATACAGATGCACGATTTGGC<br><br>201 H G S D Y S R Q Q N R F N D F D H R E R<br>601 CATGGATCCGACTACTCTCGCCAACAGAACAGATTTAATGACTTTGATCACCGAGAGAGG<br><br>221 G R F P E S S A V Q S S S F E R R D R F<br>661 GGCAGGTTTCCTGAGAGTTCAGCAGTACAGTCTTCATCTTTTGAAAGGCGGGATCGCTTT<br><br>241 V G Q S E G K K A R P T A R R E D P S F<br>721 GTTGGTCAAAGTGAGGGGAAAAAAGCACGACCTACTGCACGAAGGGAAGATCCAAGCTTC | SEQ ID NO:20 |

| Gene Name | GenBank Homology | DNA SEQUENCE / DEDUCED AMINO ACID SEQUENCE | SEQUENCE IDENTIFIER: |
|---|---|---|---|
| | | 261 E R Y P K N F S D S R R N E P P P P R N<br>781 GAAAGATATCCCAAAAATTTCAGTGACTCCAGAAGAAATGAGCCTCCACCACCAAGAAAT<br><br>281 E L R E S D R R E V R G E R D E R R T V<br>841 GAACTTAGAGAATCAGACAGGCGAGAAGTACGAGGGGAGCGAGACGAAAGGAGAACGGTG<br><br>301 I I H D R P D I T H P R H P R E A G P N<br>901 ATTATTCATGACAGGCCTGATATCACTCATCCTAGACATCCTCGAGAGGCAGGGCCCAAT<br><br>321 P S R P T S W K S E G S M S T D K R E T<br>961 CCTTCCAGACCCACCAGCTGGAAAAGTGAAGGAAGCATGTCCACTGACAAACGGGAAACA<br><br>341 R V E R P E R S G R E V S G H S V R G A<br>1021 AGAGTTGAAAGGCCAGAACGATCTGGGAGAGAAGTATCAGGGCACAGTGTGAGAGGCGCT<br><br>361 P P G N R S S A S G Y G S R E G D R G V<br>1081 CCCCCTGGGAATCGTAGCAGCGCTTCGGGGTACGGGAGCAGAGAGGGAGACAGAGGAGTC<br><br>381 I T D R G G G S Q H Y P E E R H V V E R<br>1141 ATCACAGACCGAGGAGGTGGATCACAGCACTATCCTGAGGAGCGACATGTGGTTGAACGC<br><br>401 H G R D T S G P R K E W H G P P S Q G P<br>1201 CATGGACGGGACACAAGCGGACCAAGGAAAGAGTGGCATGGTCCACCCTCTCAAGGGCCT<br><br>421 S Y H D T R R M G D G R A G A G M I T Q<br>1261 AGCTATCATGATACGAGGCGAATGGGTGACGGCCGGGCAGGAGCAGGCATGATAACCCAA<br><br>441 H S S N A S P I N R I V Q I S G N S M P<br>1321 CATTCAAGTAACGCATCCCCAATTAATAGAATTGTACAAATCAGTGGCAATTCCATGCCA<br><br>461 R G S G S G F K P F K G G P P R R F -<br>1381 AGAGGAAGTGGCTCCGGATTTAAGCCATTTAAGGGTGGACCTCCGCGACGATTCTGA | |
| WBC005F01.bFSP_20 012109.abd | Homo sapiens vaccinia related kinase 2, mRNA (cDNA clone MGC:34431 IMAGE:5221315). | 1 GGGCCGCTGCACTGCGAGGCCGACGCAGCTGGAGAGAAGTTAGGCAGGTCCTAGGGAGGG<br>61 CAGGCTCGAGTGCTGGGCCCGCCTCCCCGCGGACTGTAGGCCCGGGGGCTCCGCCTCGT<br>121 CGCAGCGGCAGGTAGGAGGCGGTGCGCGCGGCCCGGCGACGGGGATCCTGAGGCCCGAA<br>181 GTGATGCCACCAAAAAGAAATGAAAAATACAAACTTCCTATTCCATTTCCAGAAGGCAAG<br>241 GTTCTGGATGATATGGAAGGCAATCAGTGGGTACTGGGCAAGAAGATTGGCTCTGGAGGA<br>301 TTTGGATTGATATATTTAGCTTTCCCCACAAATAAACCAGAGAAAGATGCAAGACATGTA<br>361 GTAAAAGTGGAATATCAAGAAAATGGCCCGTTATTTTCAGAACTTAAATTTTATCAGAGA | SEQ ID NO:21 |

| Gene Name | GenBank Homology | DNA SEQUENCE / DEDUCED AMINO ACID SEQUENCE | SEQUENCE IDENTIFIER: |
|---|---|---|---|
| | | 421 GTTGCAAAAAAAGACTGTATCAAAAAGTGGATAGAACGCAAACAACTTGATTATTTAGGA<br>481 ATTCCTCTGTTTTATGGATCTGGTCTGACTGAATTCAAGGGAAGAAGTTACAGATTTATG<br>541 GTAATGGAAAGACTAGGAATAGATTTACAGAAGATCTCAGGCCAGAATGGTACCTTTAAA<br>601 AAGTCAACTGTCCTGCAATTAGGTATCCGAATGTTGGATGTACTGGAATATATACATGAA<br>661 AATGAATATGTTCATGGTGATGTAAAAGCAGCAAATCTACTTTTGGGTTACAAAAATCCA<br>721 GACCAGGTTTATCTTGCAGATTATGGACTTTCCTACAGATATTGTCCCAATGGAACCAC<br>781 AAACAGTATCAGGAAAATCCTAGAAAAGGCCATAATGGGACAATAGAGTTTACCAGCTTG<br>841 GATGCCCACAAGGGAGTAGCCTTGTCCAGACGAAGTGACGTTGAGATCCTCGGCTACTGC<br>901 ATGCTGCGGTGGTTGTGTGGGAAACTTCCCTGGGAACAGAACCTGAAGGACCCTGTGGCT<br>961 GTGCAGACTGCTAAAACAAATCTGTTGGACGAGCTCCCCCAGTCAGTGCTTAAATGGGCT<br>1021 CCTTCTGGAAGCAGTTGCTGTGAAATAGCCCAATTTTTGGTATGTGCTCATAGTTTAGCA<br>1081 TATGATGAAAAGCCAAACTATCAAGCCCTCAAGAAAATTTTGAACCCTCATGGAATACCT<br>1141 TTAGGACCACTGGACTTTTCCACAAAAGGACAGAGTATAAATGTCCATACTCCAAACAGT<br>1201 CAAAAAGTTGATTCACAAAAGGCTGCAACAAAGCAAGTCAACAAGGCACACAATAGGTTA<br>1261 ATCGAAAAAAAAGTCCACAGTGAGAGAAGCGCTGAGTCCTGTGCAACATGGAAAGTGCAG<br>1321 AAAGAGGAGAAACTGATTGGATTGATGAACAATGAAGCAGCTCAGGAAAACACAAGGAGA<br>1381 AGACAAAAATATTGTAAGTCTCAAGAATTTCTGAATGAACGAAAAAGTTCTCCACAAAAA<br>1441 TCTAGTGGCATACAATTCCCAAATTCATTTTATGAAACCCATCCAGATTTTACCAGTGAA<br>1501 GATATATTCAACAAGTCAAGATCTCCAACTTGGTATACATATGCTTCTACGCCTGGTATG<br>1561 GAGGTCACAGACTTAGAAAGTTCTACAGGATTTTGGCTTACTCAGTTTGCTCTTAGTGAA<br>1621 GAGACAAAGGCAGATGTGTATTATTATGGCTTCATCATTCTTTTTCTTTTGGTATTAGTA<br>1681 TGTCTTGCTTTATATTTTCTCTGAAGATGATACCAAAATTCCTTTTGATAATTTTTTAAG<br>1741 TTTCCAGCTCTTCACCGAAATGTTGTATTCTTATTTCAGTGTTTCCTTCCAGACATTTTT<br>1801 AAGGTAATTGGCTTTAAAAAGAGAACATATTTTAACAAAGTTTGTGGACACTCTAAAAAA<br>1861 TAAAATTGCTTTGTACTAGAAAAAAAAAAAAAAAAAAAAAAAAAAAAAAAAAAAAAAAAA<br>1921 AAAAAAAAAAAAAAAAAAAAAAAAAAAAAAAAAAAAAAAAAAAAAAAAA | |
| | | 1 M P P K R N E K Y K L P I P F P E G K V<br>1 ATGCCACCAAAAAGAAATGAAAAATACAAACTTCCTATTCCATTTCCAGAAGGCAAGGTT<br><br>21 L D D M E G N Q W V L G K K I G S G G F<br>61 CTGGATGATATGGAAGGCAATCAGTGGGTACTGGGCAAGAAGATTGGCTCTGGAGGATTT<br><br>41 G L I Y L A F P T N K P E K D A R H V V<br>121 GGATTGATATATTTAGCTTTCCCCACAAATAAACCAGAGAAAGATGCAAGACATGTAGTA<br><br>61 K V E Y Q E N G P L F S E L K F Y Q R V<br>181 AAAGTGGAATATCAAGAAAATGGCCCGTTATTTTCAGAACTTAAATTTTATCAGAGAGTT<br><br>81 A K K D C I K K W I E R K Q L D Y L G I<br>241 GCAAAAAAAGACTGTATCAAAAAGTGGATAGAACGCAAACAACTTGATTATTTAGGAATT<br><br>101 P L F Y G S G L T E F K G R S Y R F M V | SEQ ID NO:22 |

| Gene Name | GenBank Homology | DNA SEQUENCE / DEDUCED AMINO ACID SEQUENCE | SEQUENCE IDENTIFIER: |
|---|---|---|---|
| | | 301 CCTCTGTTTTATGGATCTGGTCTGACTGAATTCAAGGGAAGAAGTTACAGATTTATGGTA<br><br>121 M E R L G I D L Q K I S G Q N G T F K K<br>361 ATGGAAAGACTAGGAATAGATTTACAGAAGATCTCAGGCCAGAATGGTACCTTTAAAAAG<br><br>141 S T V L Q L G I R M L D V L E Y I H E N<br>421 TCAACTGTCCTGCAATTAGGTATCCGAATGTTGGATGTACTGGAATATATACATGAAAAT<br><br>161 E Y V H G D V K A A N L L L G Y K N P D<br>481 GAATATGTTCATGGTGATGTAAAAGCAGCAAATCTACTTTTGGGTTACAAAAATCCAGAC<br><br>181 Q V Y L A D Y G L S Y R Y C P N G N H K<br>541 CAGGTTTATCTTGCAGATTATGGACTTTCCTACAGATATTGTCCCAATGGGAACCACAAA<br><br>201 Q Y Q E N P R K G H N G T I E F T S L D<br>601 CAGTATCAGGAAAATCCTAGAAAAGGCCATAATGGGACAATAGAGTTTACCAGCTTGGAT<br><br>221 A H K G V A L S R R S D V E I L G Y C M<br>661 GCCCACAAGGGAGTAGCCTTGTCCAGACGAAGTGACGTTGAGATCCTCGGCTACTGCATG<br><br>241 L R W L C G K L P W E Q N L K D P V A V<br>721 CTGCGGTGGTTGTGTGGGAAACTTCCCTGGGAACAGAACCTGAAGGACCCTGTGGCTGTG<br><br>261 Q T A K T N L L D E L P Q S V L K W A P<br>781 CAGACTGCTAAAACAAATCTGTTGGACGAGCTCCCCCAGTCAGTGCTTAAATGGGCTCCT<br><br>281 S G S S C C E I A Q F L V C A H S L A Y<br>841 TCTGGAAGCAGTTGCTGTGAAATAGCCCAATTTTTGGTATGTGCTCATAGTTTAGCATAT<br><br>301 D E K P N Y Q A L K K I L N P H G I P L<br>901 GATGAAAAGCCAAACTATCAAGCCCTCAAGAAAATTTTGAACCCTCATGGAATACCTTTA<br><br>321 G P L D F S T K G Q S I N V H T P N S Q<br>961 GGACCACTGGACTTTTCCACAAAAGGACAGAGTATAAATGTCCATACTCCAAACAGTCAA<br><br>341 K V D S Q K A A T K Q V N K A H N R L I<br>1021 AAAGTTGATTCACAAAAGGCTGCAACAAAGCAAGTCAACAAGGCACACAATAGGTTAATC<br><br>361 E K K V H S E R S A E S C A T W K V Q K<br>1081 GAAAAAAAAGTCCACAGTGAGAGAAGCGCTGAGTCCTGTGCAACATGGAAAGTGCAGAAA<br><br>381 E E K L I G L M N N E A A Q E N T R R R<br>1141 GAGGAGAAACTGATTGGATTGATGAACAATGAAGCAGCTCAGGAAAACACAAGGAGAAGA | |

| Gene Name | GenBank Homology | DNA SEQUENCE / DEDUCED AMINO ACID SEQUENCE | SEQUENCE IDENTIFIER: |
|---|---|---|---|
| | | 401 Q K Y C K S Q E F L N E R K S S P Q K S<br>1201 CAAAAATATTGTAAGTCTCAAGAATTTCTGAATGAACGAAAAAGTTCTCCACAAAAATCT<br><br>421 S G I Q F P N S F Y E T H P D F T S E D<br>1261 AGTGGCATACAATTCCCAAATTCATTTTATGAAACCCATCCAGATTTTACCAGTGAAGAT<br><br>441 I F N K S R S P T W Y T Y A S T P G M E<br>1321 ATATTCAACAAGTCAAGATCTCCAACTTGGTATACATATGCTTCTACGCCTGGTATGGAG<br><br>461 V T D L E S S T G F W L T Q F A L S E E<br>1381 GTCACAGACTTAGAAAGTTCTACAGGATTTTGGCTTACTCAGTTTGCTCTTAGTGAAGAG<br><br>481 T K A D V Y Y Y G F I I L F L L V L V C<br>1441 ACAAAGGCAGATGTGTATTATTATGGCTTCATCATTCTTTTTCTTTTGGTATTAGTATGT<br><br>501 L A L Y F L -<br>1501 CTTGCTTTATATTTTCTCTGA | |
| WBC005F10.bFSP_20 012109.abd | Polymeric immunoglobulin receptor 3 precursor (PIGR3) | 1 ggtgcgacca agccctggct ccagctctag cgggtatctg cccaccatgg ccctggtgct<br>61 gatcctccag ctgctgaccc tctggcctct gtgtcacaca gacatcactc cgtctgtccc<br>121 cccagcttca taccacccta agccatggct gggagctcag ccggctacag ttgtgacccc<br>181 tggggtcaac gtgaccttga gatgccgggc accccaaccc gcttggagat ttggactttt<br>241 caagcctgga gagatcgctc cccttctctt ccgggatgtg tcctccgagc tggcagaatt<br>301 ctttctggag gaggtgactc cagcccaagg gggaagttac cgctgctgct accgaaggcc<br>361 agactggggg ccgggtgtct ggtcccagcc cagcgatgtc ctggagctgc tggtgacaga<br>421 ggagctgccg cggccgtcgc tggtggcgct gcccgggccg gtggtgggtc ctggcgccaa<br>481 cgtgagcctg cgctgcgcgg gccgcctgcg gaacatgaac ttcgtgctgt accgcgaggg<br>541 cgtggcggcc ccgctgcagt accgccactc cgcgcagccc tgggccgact tcacgctgct<br>601 gggcgcccgc gcccccggca cctacagctg ctactatcac acgccctccg cgccctacgt<br>661 gctgtcgcag cgcagcgagg tgctggtcat cagctgggaa gactctggct cctccgacta<br>721 cacccggggg aacctagtcc gcctggggct ggccgggctg gtcctcatct ccctgggcgc<br>781 gctggtcact tttgactggc gcagtcagaa ccgcgctcct gctggtatcc gcccctgagc<br>841 cccaggagca ctgcagcccg agacttccaa cctgagtggc ggagaagctg ggaccctggg<br>901 ctggactgtc ctttcctgca gccccacagt cctgctggct gagctccgcg gaacggtcct<br>961 tagaccccgc tgtgccctgt gctgtagctt ctttccaggc ctttcccaag gagtagctga<br>1021 aaggaagacg cgattagtgg ttaagacttc caagccagaa gacagagggt tcgaatccca<br>1081 gcactgccgt ctactcactg tagtagtagc agctacagaa aggtagtagt gagacgtgaa<br>1141 gccagctgga cttcctgggt tgaatgggga cctggagaac ttttctgtct tacaagagga<br>1201 ttgtaaaatg gaccaatcag cactctgtaa gatggaccaa tcagcgctct gtaaaatgga<br>1261 ccaatcagca ggacacgggc ggggacaata agggaataaa agctggcgag cgcggcaccc<br>1321 caccagagtc tgcttccacg ttgtgggagc tttgttctct tgctctacac aataaatctt | SEQ ID NO:23 |

| Gene Name | GenBank Homology | DNA SEQUENCE / DEDUCED AMINO ACID SEQUENCE | SEQUENCE IDENTIFIER: |
|---|---|---|---|
| | | 1381 gctgctgctt aaaaaaaaaa aaaaaaaaaa aaaaaaaaaa aaaaaa<br><br>1 M A L V L I L Q L L T L W P L C H T D I<br>1 ATGGCCCTGGTGCTGATCCTCCAGCTGCTGACCCTCTGGCCTCTGTGTCACACAGACATC<br><br>21 T P S V P P A S Y H P K P W L G A Q P A<br>61 ACTCCGTCTGTCCCCCCAGCTTCATACCACCCTAAGCCATGGCTGGGAGCTCAGCCGGCT<br><br>41 T V V T P G V N V T L R C R A P Q P A W<br>121 ACAGTTGTGACCCCTGGGGTCAACGTGACCTTGAGATGCCGGGCACCCCAACCCGCTTGG<br><br>61 R F G L F K P G E I A P L L F R D V S S<br>181 AGATTTGGACTTTTCAAGCCTGGAGAGATCGCTCCCCTTCTCTTCCGGGATGTGTCCTCC<br><br>81 E L A E F F L E E V T P A Q G G S Y R C<br>241 GAGCTGGCAGAATTCTTTCTGGAGGAGGTGACTCCAGCCCAAGGGGGAAGTTACCGCTGC<br><br>101 C Y R R P D W G P G V W S Q P S D V L E<br>301 TGCTACCGAAGGCCAGACTGGGGGCCGGGTGTCTGGTCCCAGCCCAGCGATGTCCTGGAG<br><br>121 L L V T E E L P R P S L V A L P G P V V<br>361 CTGCTGGTGACAGAGGAGCTGCCGCGGCCGTCGCTGGTGGCGCTGCCCGGGCCGGTGGTG<br><br>141 G P G A N V S L R C A G R L R N M N F V<br>421 GGTCCTGGCGCCAACGTGAGCCTGCGCTGCGCGGGCCGCCTGCGGAACATGAACTTCGTG<br><br>161 L Y R E G V A A P L Q Y R H S A Q P W A<br>481 CTGTACCGCGAGGGCGTGGCGGCCCCGCTGCAGTACCGCCACTCCGCGCAGCCCTGGGCC<br><br>181 D F T L L G A R A P G T Y S C Y Y H T P<br>541 GACTTCACGCTGCTGGGCGCCCGCGCCCCCGGCACCTACAGCTGCTACTATCACACGCCC<br><br>201 S A P Y V L S Q R S E V L V I S W E D S<br>601 TCCGCGCCCTACGTGCTGTCGCAGCGCAGCGAGGTGCTGGTCATCAGCTGGGAAGACTCT<br><br>221 G S S D Y T R G N L V R L G L A G L V L<br>661 GGCTCCTCCGACTACACCCGGGGGAACCTAGTCCGCCTGGGGCTGGCCGGGCTGGTCCTC<br><br>241 I S L G A L V T F D W R S Q N R A P A G<br>721 ATCTCCCTGGGCGCGCTGGTCACTTTTGACTGGCGCAGTCAGAACCGCGCTCCTGCTGGT<br><br>261 I R P -<br>781 ATCCGCCCCTGA | SEQ ID NO:24 |

| Gene Name | GenBank Homology | DNA SEQUENCE / DEDUCED AMINO ACID SEQUENCE | SEQUENCE IDENTIFIER: |
|---|---|---|---|
| | | | |
| WBC007A08.bFSP_20010110.abd | No Homology | 1 GTTTCATCCGTTCTGTGAGGCTCTGCGGCATCTGCCAGATAGCAAACAGGCCTCATGCAG<br>61 GCAGATTAGGGCCTAAAGCAGATCAGAAGGCTTTGTAAAAGACCTCCCTGAGCCATCCCT<br>121 CTGCAGTGTTGGCTCAAGGCCACATGTCGTTTAGTCACAGCAAAGAGTAGACAGTTAGGA<br>181 AAAGCAAATGTCTTTTTATTTTCTAACATCTCCTCCCCTCAGATAGGGGAGCTATGACTC<br>241 AGTTGCACCTAAGAATACTGTAATTTGACTCTGTAATTGCCTTTGCTTCCCAAACCTGGG<br>301 AATCAATGGAAAGGCAGGGAATGTTCCTCTTCTGTGGCCAGGTTCTGTTCTTTGCAATTA<br>361 CAGCAAATTTTTAAAAATGCAAAGGCAGTTGTTGGTCCGCAGGGCTTGGCCAGTTATGTG<br>421 GTAGATACAGAGGCTAGCAGACAATTTGAGGATCCCGCTGTAATAATAAATGGCAGCTAG<br>481 CATTCGTTGACCATGTATTTATATGTCAGGCACTGAGCTAGGCAGGCTCTCTCTATATGG<br>541 CAGCTCCAAACTTCACAATAACTGTGAGTTAAGTACTTTATCTTCATTTCATAGATGAAG<br>601 AAACAGGTTCGGAGAGAAAAAGTTCTTTCCCAAGCCAGTACAGCTAGTAAGTGGTAGAAT<br>661 CAGGATTCATAGTGTCACCATATGGACCAATGTTTATAGGGAAAAGGAAAATTACAAGT<br>721 CTTCTTAAAATGAAGTGGCCAGCTTTTCTTGCACAGACTAAATAACTCTAACTGGCATTT<br>781 TTCGGTTGGGAAACAGCTGAATTAGTAGTTAAGTCTGACAGCCAAGTGAGTTTTAGAAAC<br>841 CGCAGCGTGGAGCATGCACTCCCCCACACCATCTGCTGTGTTGATTAGCAGTCCTGCCTC<br>901 AGTCTCTTTCTTT | SEQ ID NO:25 |
| | | 1 AAGAAGAAATAGATAATCTGAATAGCGCTATGTCTATTAAAACTTAATAGCTCTGGGGGG<br>61 TGTAGGTATTATTGTCCTCATTTTACAGATAAGGAAACTGAGGCTCAGAGAGAGAAAGTC<br>121 TGTTGCTCAAGGTAGCAAAGCTAAAATATGGCAGAGCTAGGATTCAAATCCAGGTCTTCT<br>181 GATTCTCATTCCAGTGCCCTTTCAGTATACCGTGTTGCCTCTAAAGATTGCAGCTCCTTA<br>241 TTTACTGGAAAGTTGTTCCTGCCCAGTCCACACCCTCCCACCCCATCTTCCTCGAGCCCT<br>301 CTGTCTGTGTTTTCATCAGGATTATATTCATTGTATTTGGAATCCGTGTTCTTTTTTGTG<br>361 TTGGGAAAAACAAATCTCTTCTACCAGCTTGCACTCAGACCAACTTGGAAAAAAAAAGTT<br>421 TAAATGCTGTTGCAGATGTACAACTTAAAAATGTGAAGTTTGTAGCTTTAACTTTTGTAA<br>481 CTAAAATTAATAACACTGGCTTAAGTGCTGACTAGAAATGCTATTTTATAAAGTTTGGAT<br>541 GTAAATAATCAGTCGAGGTCAGCAGTTTGTATATGTATGAGACAGCTTCCTCCATTGCCT<br>601 TTTTTTTTAAATTTGAAATGCTTTCTCTGTGCTTTCGTTAGAATTGTTGTTCTTCCTCTT<br>661 TCCTACACCGTGTCACCTTTGTTTTAAATAAACTGTCCTTCGGAAAAAAAAAAAAAAAAA<br>721 A | SEQ ID NO:26 |
| WBC007F03.bFSP_20010110.abd | Homo sapiens mRNA for enolase 1 variant, clone: adSE00169 | 1 agtatctgtg ggtacccgga gcacggagat ctcgccggct ttacgttcac ctcggtgtct<br>61 gcagcaccct ccgcttcctc tcctaggcga cgagacccag tggctagaag ttcaccatgt<br>121 ctattctcaa gatccatgcc agggagatct ttgactctcg cgggaatccc actgttgagg<br>181 ttgatctctt cacctcaaaa ggtctcttca gagctgctgt gcccagtggt gcttcaactg<br>241 gtatctatga ggccctagag ctccgggaca atgataagac tcgctatatg ggaagggtg<br>301 tctcaaaggc tgttgagcac atcaataaaa ctattgcgcc tgccctggct agcaagaaac<br>361 tgaacgtcac agaacaagag aagattgaca aactgatgat cgagatggat ggaacagaaa<br>421 ataaatctaa gtttggtgcg aacgccattc tgggggtgtc ccttgccgtc tgcaaagctg<br>481 gtgccgttga gaagggggtc cccctgtacc gccacatcgc tgacttggct ggcaactctg | SEQ ID NO:27 |

| Gene Name | GenBank Homology | DNA SEQUENCE / DEDUCED AMINO ACID SEQUENCE | SEQUENCE IDENTIFIER: |
|---|---|---|---|
| | | 541 aagtcatcct gccagtcccg gcgttcaatg tcatcaatgg cggttctcat gctggcaaca<br>601 agctggccat gcaggagttc atgatcctcc cagtcggtgc agcaaacttc agggaagcca<br>661 tgcgcattgg agcagaggtt taccacaacc tgaagaatgt catcaaggag aaatatggga<br>721 aagatgccac caatgtgggg gatgaaggcg ggtttgctcc caacatcctg gagaataaag<br>781 aaggcctgga gctgctgaag actgctattg ggaaagctgg ctacactgat aaggtggtca<br>841 tcggcatgga cgtagcggcc tccgagttct tcaggtctgg gaagtatgac ctggacttca<br>901 agtctcccga tgaccccagc aggtacatct cgcctgacca gctggctgac ctgtacaagt<br>961 ccttcatcaa ggactaccca gtggtgtcta tcgaagatcc ctttgaccag gatgactggg<br>1021 gagcttggca gaagttcaca gccagtgcag gaatccaggt agtgggggat gatctcacag<br>1081 tgaccaaccc aaagaggatc gccaaggccg tgaacgagaa gtcctgcaac tgcctcctgc<br>1141 tcaaagtcaa ccagattggc tccgtgaccg agtctcttca ggcgtgcaag ctggcccagg<br>1201 ccaatggttg gggcgtcatg gtgtctcatc gttcggggga gactgaagat accttcatcg<br>1261 ctgacctggt tgtggggctg tgcactgggc agatcaagac tggtgcccct tgccgatctg<br>1321 agcgcttggc caagtacaac cagctcctca gaattgaaga ggagctgggc agcaaggcta<br>1381 agtttgccgg caggaacttc agaaacccct tggccaagta agctgtgggc aggcaagccc<br>1441 ttcggtcacc tgttggctac acagacccct cccctcgtgt cagctcaggc agctcgaggc<br>1501 ccccgaccaa cacttgcagg ggtccctgct agttagcgcc ccaccgccgt ggagttcgta<br>1561 ccgcttcctt agaacttcta cagaagccaa gctccctgga gccctgttgg cagctctagc<br>1621 tttgcagtcg tgtaattggc ccaagtcatt gtttttctcg cctcactttc caccaagtgt<br>1681 ctagagtcat gtgagcctcg tgtcatctcc ggggtggcca caggctagat ccccggtggt<br>1741 tttgtgctca aaataaaaag cctcagtgac ccatgaaaaa aaaaaaaaaa aaa<br><br>1 M S I L K I H A R E I F D S R G N P T V<br>1 ATGTCTATTCTCAAGATCCATGCCAGGGAGATCTTTGACTCTCGCGGGAATCCCACTGTT<br><br>21 E V D L F T S K G L F R A A V P S G A S<br>61 GAGGTTGATCTCTTCACCTCAAAAGGTCTCTTCAGAGCTGCTGTGCCCAGTGGTGCTTCA<br><br>41 T G I Y E A L E L R D N D K T R Y M G K<br>121 ACTGGTATCTATGAGGCCCTAGAGCTCCGGGACAATGATAAGACTCGCTATATGGGGAAG<br><br>61 G V S K A V E H I N K T I A P A L A S K<br>181 GGTGTCTCAAAGGCTGTTGAGCACATCAATAAAACTATTGCGCCTGCCCTGGCTAGCAAG<br><br>81 K L N V T E Q E K I D K L M I E M D G T<br>241 AAACTGAACGTCACAGAACAAGAGAAGATTGACAAACTGATGATCGAGATGGATGGAACA<br><br>101 E N K S K F G A N A I L G V S L A V C K<br>301 GAAAATAAATCTAAGTTTGGTGCGAACGCCATTCTGGGGGTGTCCCTTGCCGTCTGCAAA<br><br>121 A G A V E K G V P L Y R H I A D L A G N<br>361 GCTGGTGCCGTTGAGAAGGGGGTCCCCCTGTACCGCCACATCGCTGACTTGGCTGGCAAC | SEQ ID NO:28 |

| Gene Name | GenBank Homology | DNA SEQUENCE / DEDUCED AMINO ACID SEQUENCE | SEQUENCE IDENTIFIER: |
|---|---|---|---|
| | | 141 S E V I L P V P A F N V I N G G S H A G<br>421 TCTGAAGTCATCCTGCCAGTCCCGGCGTTCAATGTCATCAATGGCGGTTCTCATGCTGGC<br><br>161 N K L A M Q E F M I L P V G A A N F R E<br>481 AACAAGCTGGCCATGCAGGAGTTCATGATCCTCCCAGTCGGTGCAGCAAACTTCAGGGAA<br><br>181 A M R I G A E V Y H N L K N V I K E K Y<br>541 GCCATGCGCATTGGAGCAGAGGTTTACCACAACCTGAAGAATGTCATCAAGGAGAAATAT<br><br>201 G K D A T N V G D E G G F A P N I L E N<br>601 GGGAAAGATGCCACCAATGTGGGGGATGAAGGCGGGTTTGCTCCCAACATCCTGGAGAAT<br><br>221 K E G L E L L K T A I G K A G Y T D K V<br>661 AAAGAAGGCCTGGAGCTGCTGAAGACTGCTATTGGGAAAGCTGGCTACACTGATAAGGTG<br><br>241 V I G M D V A A S E F F R S G K Y D L D<br>721 GTCATCGGCATGGACGTAGCGGCCTCCGAGTTCTTCAGGTCTGGGAAGTATGACCTGGAC<br><br>261 F K S P D D P S R Y I S P D Q L A D L Y<br>781 TTCAAGTCTCCCGATGACCCCAGCAGGTACATCTCGCCTGACCAGCTGGCTGACCTGTAC<br><br>281 K S F I K D Y P V V S I E D P F D Q D D<br>841 AAGTCCTTCATCAAGGACTACCCAGTGGTGTCTATCGAAGATCCCTTTGACCAGGATGAC<br><br>301 W G A W Q K F T A S A G I Q V V G D D L<br>901 TGGGGAGCTTGGCAGAAGTTCACAGCCAGTGCAGGAATCCAGGTAGTGGGGGATGATCTC<br><br>321 T V T N P K R I A K A V N E K S C N C L<br>961 ACAGTGACCAACCCAAAGAGGATCGCCAAGGCCGTGAACGAGAAGTCCTGCAACTGCCTC<br><br>341 L L K V N Q I G S V T E S L Q A C K L A<br>1021 CTGCTCAAAGTCAACCAGATTGGCTCCGTGACCGAGTCTCTTCAGGCGTGCAAGCTGGCC<br><br>361 Q A N G W G V M V S H R S G E T E D T F<br>1081 CAGGCCAATGGTTGGGGCGTCATGGTGTCTCATCGTTCGGGGGAGACTGAAGATACCTTC<br><br>381 I A D L V V G L C T G Q I K T G A P C R<br>1141 ATCGCTGACCTGGTTGTGGGGCTGTGCACTGGGCAGATCAAGACTGGTGCCCCTTGCCGA<br><br>401 S E R L A K Y N Q L L R I E E E L G S K<br>1201 TCTGAGCGCTTGGCCAAGTACAACCAGCTCCTCAGAATTGAAGAGGAGCTGGGCAGCAAG<br><br>421 A K F A G R N F R N P L A K - | |

| Gene Name | GenBank Homology | DNA SEQUENCE / DEDUCED AMINO ACID SEQUENCE | SEQUENCE IDENTIFIER: |
|---|---|---|---|
| | | 1261 GCTAAGTTTGCCGGCAGGAACTTCAGAAACCCCTTGGCCAAGTAA | |
| WBC008A10.bFSP_20 010110.abd | Homo sapiens matrix metalloproteinase 8 (neutrophil collagenase) (MMP8) , mRNA. | 1 GCTCGCCAGGGAAGGGCCCTACCCAGAGGACAGAAAGAAAGCCAGGAGGGGTAGAGTTTG<br>61 AAGAGAAGATCATGTTCTCCCTGAAGACGCTTCCATTTCTGCTCTTACTCCATGTGCAGA<br>121 TTTCCAAGGCCTTTCCTGTATCTTCTAAAGAGAAAAATACAAAAACTGTTCAGGACTACC<br>181 TGGAAAAGTTCTACCAATTACCAAGCAACCAGTATCAGTCTACAAGGAAGAATGGCACTA<br>241 ATGTGATCGTTGAAAAGCTTAAAGAAATGCAGCGATTTTTTGGGTTGAATGTGACGGGGA<br>301 AGCCAAATGAGGAAACTCTGGACATGATGAAAAAGCCTCGCTGTGGAGTGCCTGACAGTG<br>361 GTGGTTTTATGTTAACCCCAGGAAACCCCAAGTGGGAACGCACTAACTTGACCTACAGGA<br>421 TTCGAAACTATACCCCACAGCTGTCAGAGGCTGAGGTAGAAAGAGCTATCAAGGATGCCT<br>481 TTGAACTCTGGAGTGTTGCATCACCTCTCATCTTCACCAGGATCTCACAGGGAGAGGCAG<br>541 ATATCAACATTGCTTTTTACCAAAGAGATCACGGTGACAATTCTCCATTTGATGGACCCA<br>601 ATGGAATCCTTGCTCATGCCTTTCAGCCAGGCCAAGGTATTGGAGGAGATGCTCATTTTG<br>661 ATGCCGAAGAAACATGGACCAACACCTCCGCAAATTACAACTTGTTTCTTGTTGCTGCTC<br>721 ATGAATTTGGCCATTCTTTGGGGCTCGCTCACTCCTCTGACCCTGGTGCCTTGATGTATC<br>781 CCAACTATGCTTTCAGGGAAACCAGCAACTACTCACTCCCTCAAGATGACATCGATGGCA<br>841 TTCAGGCCATCTATGGACTTTCAAGCAACCCTATCCAACCTACTGGACCAAGCACACCCA<br>901 AACCCTGTGACCCCAGTTTGACATTTGATGCTATCACCACACTCCGTGGAGAAATACTTT<br>961 TCTTTAAAGACAGGTACTTCTGGAGAAGGCATCCTCAGCTACAAAGAGTCGAACTCAATT<br>1021 TCATTTCTCTGTTCTGGCCATCCTTGCCAAATGGTATACAGGCGGCTTATGAGGATTTTG<br>1081 ACAGGGACCTAATTTTCCTATTTAAAGGCAACCAATACTGGGCTCTGAATGGCTATGACA<br>1141 TTCAGCAAGGTTATCCCAAGGACATATCAAACTACGGCTTCCCAAGCAGTGTCCAAGCAA<br>1201 TTGATGCAGCTGTTTCCTATAGGAGTAAAACATACTTCTTTGTAAATGATCAATTCTGGA<br>1261 GATATGATAACCAAAGACGATCCATGGAAGCAGGTTATCCCCAAAGCATAGCAAGTACCT<br>1321 TTCCAGGAATAGGAAGCAGAATTGATGCAGTTTTCCAGCAAGATGACTTCTTTCTTTTCT<br>1381 TCAGCGGACCAGTTTATTATGCATTTGATCTTAATACTCAGAGAGTTACTAGGGTTGCAA<br>1441 GAATCAATAAATGGCTTAACTGTAGACGAAGTTGAAGCAAAATCAAATGTGGCTGTATCC<br>1501 ACTTTCAGAATGTTGAAGGGAAGTTCAGCATGCATTTTCGTTACATTGTGTCCTGCTTAT<br>1561 ACTTTTCTCAATATTAAGTCATTGTTTCCCATCACTGTATCCATTCTACCTGTCCTCCGT<br>1621 GAAAATATGTTTGGAATATTCCACTATTTGCAGAGGCTTATTCAGTTCTTACACATTCCA<br>1681 TCTTACATTAGTGATTCCATCAAAGAGAAGGAAAGTAAGCCTTTTTGTCACCTCAATATT<br>1741 TACTATTTCAATACTTACATATCTGACTTCTAGGATTTATTGTTATATTACTTGCCTATC<br>1801 TGACTTCATACATCCCTCAGTTTCTTAAAATGTCCTATGTATATCTGCTACATGCAATTT<br>1861 AGCACTGGATTTAGGTTATAAGTAAGGATTATAAACAAGATAGATAGTACTCCTTGCGTT<br>1921 TATTGAAAATCTGGTGCTATTTTCCACTCTTGGAAAGAAATATTGTTGATATGTATTGTG<br>1981 AGTTGAATTGTGTTCCCCAAAAAAGCTATGTGCAAGTCCCAACCTCGGGTACCTATGAAT<br>2041 GTGAGCTTACCAGGGTCTTTGCAGATGTAAATAGTTAAGTTAAGGTGAGATCACACTGAA<br>2101 TTAGGGTGGGCTCTAAATCCATTATGACTGTTGTTCTTATAAGAAGAAGAGAGCATAGCC<br>2161 ACCTAGGGGAGGAGGCCGTGTGAAGACAGAGGCAGAGATTGGAGTGACGCATCTCCAAGC<br>2221 CAA | SEQ ID NO:29 |
| | | 1 M F S L K T L P F L L L L H V Q I S K A | SEQ ID NO:30 |

| Gene Name | GenBank Homology | DNA SEQUENCE / DEDUCED AMINO ACID SEQUENCE | SEQUENCE IDENTIFIER: |
|---|---|---|---|
| | | 1 ATGTTCTCCCTGAAGACGCTTCCATTTCTGCTCTTACTCCATGTGCAGATTTCCAAGGCC<br><br>21 F P V S S K E K N T K T V Q D Y L E K F<br>61 TTTCCTGTATCTTCTAAAGAGAAAAATACAAAAACTGTTCAGGACTACCTGGAAAAGTTC<br><br>41 Y Q L P S N Q Y Q S T R K N G T N V I V<br>121 TACCAATTACCAAGCAACCAGTATCAGTCTACAAGGAAGAATGGCACTAATGTGATCGTT<br><br>61 E K L K E M Q R F F G L N V T G K P N E<br>181 GAAAAGCTTAAAGAAATGCAGCGATTTTTTGGGTTGAATGTGACGGGGAAGCCAAATGAG<br><br>81 E T L D M M K K P R C G V P D S G G F M<br>241 GAAACTCTGGACATGATGAAAAAGCCTCGCTGTGGAGTGCCTGACAGTGGTGGTTTTATG<br><br>101 L T P G N P K W E R T N L T Y R I R N Y<br>301 TTAACCCCAGGAAACCCCAAGTGGGAACGCACTAACTTGACCTACAGGATTCGAAACTAT<br><br>121 T P Q L S E A E V E R A I K D A F E L W<br>361 ACCCCACAGCTGTCAGAGGCTGAGGTAGAAAGAGCTATCAAGGATGCCTTTGAACTCTGG<br><br>141 S V A S P L I F T R I S Q G E A D I N I<br>421 AGTGTTGCATCACCTCTCATCTTCACCAGGATCTCACAGGGAGAGGCAGATATCAACATT<br><br>161 A F Y Q R D H G D N S P F D G P N G I L<br>481 GCTTTTTACCAAAGAGATCACGGTGACAATTCTCCATTTGATGGACCCAATGGAATCCTT<br><br>181 A H A F Q P G Q G I G G D A H F D A E E<br>541 GCTCATGCCTTTCAGCCAGGCCAAGGTATTGGAGGAGATGCTCATTTTGATGCCGAAGAA<br><br>201 T W T N T S A N Y N L F L V A A H E F G<br>601 ACATGGACCAACACCTCCGCAAATTACAACTTGTTTCTTGTTGCTGCTCATGAATTTGGC<br><br>221 H S L G L A H S S D P G A L M Y P N Y A<br>661 CATTCTTTGGGGCTCGCTCACTCCTCTGACCCTGGTGCCTTGATGTATCCCAACTATGCT<br><br>241 F R E T S N Y S L P Q D D I D G I Q A I<br>721 TTCAGGGAAACCAGCAACTACTCACTCCCTCAAGATGACATCGATGGCATTCAGGCCATC<br><br>261 Y G L S S N P I Q P T G P S T P K P C D<br>781 TATGGACTTTCAAGCAACCCTATCCAACCTACTGGACCAAGCACACCCAAACCCTGTGAC<br><br>281 P S L T F D A I T T L R G E I L F F K D<br>841 CCCAGTTTGACATTTGATGCTATCACCACACTCCGTGGAGAAATACTTTTCTTTAAAGAC | |

| Gene Name | GenBank Homology | DNA SEQUENCE / DEDUCED AMINO ACID SEQUENCE | SEQUENCE IDENTIFIER: |
|---|---|---|---|
| | | 301 R Y F W R R H P Q L Q R V E L N F I S L<br>901 AGGTACTTCTGGAGAAGGCATCCTCAGCTACAAAGAGTCGAACTCAATTTCATTTCTCTG<br><br>321 F W P S L P N G I Q A A Y E D F D R D L<br>961 TTCTGGCCATCCTTGCCAAATGGTATACAGGCGGCTTATGAGGATTTTGACAGGGACCTA<br><br>341 I F L F K G N Q Y W A L N G Y D I Q Q G<br>1021 ATTTTCCTATTTAAAGGCAACCAATACTGGGCTCTGAATGGCTATGACATTCAGCAAGGT<br><br>361 Y P K D I S N Y G F P S S V Q A I D A A<br>1081 TATCCCAAGGACATATCAAACTACGGCTTCCCAAGCAGTGTCCAAGCAATTGATGCAGCT<br><br>381 V S Y R S K T Y F F V N D Q F W R Y D N<br>1141 GTTTCCTATAGGAGTAAAACATACTTCTTTGTAAATGATCAATTCTGGAGATATGATAAC<br><br>401 Q R R S M E A G Y P Q S I A S T F P G I<br>1201 CAAAGACGATCCATGGAAGCAGGTTATCCCCAAAGCATAGCAAGTACCTTTCCAGGAATA<br><br>421 G S R I D A V F Q Q D D F F L F F S G P<br>1261 GGAAGCAGAATTGATGCAGTTTTCCAGCAAGATGACTTCTTTCTTTTCTTCAGCGGACCA<br><br>441 V Y Y A F D L N T Q R V T R V A R I N K<br>1321 GTTTATTATGCATTTGATCTTAATACTCAGAGAGTTACTAGGGTTGCAAGAATCAATAAA<br><br>461 W L N C R R S -<br>1381 TGGCTTAACTGTAGACGAAGTTGA | |
| WBC008A12.bFSP_20 010110.abd | Homo sapiens cDNA FLJ33312 fis, clone BNGH42005968, *weakly similar to* DIPZ PROTEIN. | 1 AGTTTAATTACGTCCCCGGGAACTGCGCCGATTTGGACTTTTGGCACTTGGACCTATGCT<br>61 TTAAAAAGAAAAAAGTGTCATTGGCGTGGAGTGGGGCTAGTGGAGGGTGAGGTGAATGCG<br>121 CCGTTTGGAAACCACAGGACAGTGAACGTTTCGTCTCTCCCAGCGAGACTCTCCCGCGGG<br>181 CCCGGCGGCCGCATCGGGAGCCCGGCGGAAACATGGCGGCGCCCGGAGGCCGGGGCCGCA<br>241 GCCTCTCCGGCCTGCTCCCCGCGCAGACCTCGCTAGAGTACGCCCTGCTCGACGCCGTTA<br>301 CCCAGCAGGAGAAGGACAGCCTGGTCTACCAGTATCTGCAGAAGGTGGACGGCTGGGAGC<br>361 AGGACTTGTCAGTACCCGAGTTTCCGGAAGGATTAGAATGGCTGAACACAGAAGAACCTA<br>421 TTTCTGTCTACAAGGATCTATGTGGAAAAATAGTCGTCCTTGATTTCTTCACCTACTGCT<br>481 GCATAAACTGTATTCACCTATTGCCTGATCTCCATGCATTAGAACACACATACTCTGATA<br>541 AAGATGGTCTTCTTATTATTGGTGTTCACTCGGCTAAGTTTCCAAATGAAAAAGTCCTGG<br>601 ATAACATTAAGAGTGCTGTTCTTCGATACAACATCACCCACCCTATGGTTAATGATGCAG<br>661 ATGCCAGCCTTTGGCAAGAACTAGAAGTTTCCTGCTGGCCAACTCTAGTCATACTTGGAC<br>721 CTCGTGGAAACATGTTGTTTTCTTTGATTGGAGAGGGACACAAAGATAAATTATTTTTAT | *SEQ ID NO:31* |

| Gene Name | GenBank Homology | DNA SEQUENCE / DEDUCED AMINO ACID SEQUENCE | SEQUENCE IDENTIFIER: |
|---|---|---|---|
| | | 781 ATACTTCAATTGCTTTAAAGTATTACAAAGACAGGGGGCAGATCAGAGATAATAAAATTG<br>841 GAATAAAACTCTATAAAGATTCTTTGCCACCTTCACCATTGCTATTTCCTGGCAAAGTAA<br>901 CAGTAGACCAAGTTACTGATAGATTGGTAATAGCAGACACTGGACATCATAGAATTTTGG<br>961 TCGTTTGGAAGAATGGACAAATTCAATATAGCATTGGAGGACCCAACCCTGGAAGAAAAG<br>1021 ATGGAATATTTTCAGAATCAACTTTTAATTCTCCACAGGGTGTAGCCATAATGAATAATA<br>1081 TCATATATGTGGCAGACACTGAAAACCACCTTATAAGAAAGATTGACCTAGAAGCTGAGA<br>1141 AGGTGAGCACTGTAGCTGGTATTGGAATTCAAGGTACAGATAAAGAAGGTGGAGCAAAAG<br>1201 GAGAACAGCAGCCCATTAGTTCCCCTTGGGATGTGGTTTTTGGAACATCAGGTTCAGAGG<br>1261 TCCAACGAGATGACATTCTGTGGATAGCCATGGCAGGGACTCATCAGATATGGGCACTCC<br>1321 TGTTGGACTCTGGCAGACTACCAAAGAAAAATGAGTTAAAAAAAGGAACCTGCCTTCAGT<br>1381 TTGCTGGAAGTGGAAATGAAGAGAATCGAAATAATGCATATCCTCACAAGGCAGGTTTTG<br>1441 CCCAACCTTCAGGTCTTTCTGTGGCCTCAGAAGATCCCTGGAGCTGCCTGTTCGTAGCAG<br>1501 ATAGTGAGAGCAGCACCGTGAGAACCGTCTCACTGAAGGATGGAGCCGTGAAGCACCTTG<br>1561 TGGGAGGAGAAAGAGACCCCATGGACTTATTTGCTTTTGGTGATGTTGATGGAGTAGGAA<br>1621 TCAATGCAAAGCTTCAGCACCCACTTGGAGTAACTTGGGACAAGAAAAGGAATTTAGTTT<br>1681 ATGTGGCAGACTCTTATAATCACAAGATTAAAGTTGTGGATCCCAAAACGAAAACCTGTA<br>1741 CAACATTAGCAGGAACTGGAGATGCAAGTAATGTTATCAGTTCCAGTTTTACTCAGTCAA<br>1801 CTTTTAATGAGCCAGGAGGCTTGTGTATTGGAGAGAATGGTCAGTTATTATATGTAGCAG<br>1861 ACACCAATAATCATCAGATTAAAGTGATGGATTTAGAAACAAAAATGATTTCTGTGCTCC<br>1921 CACTCTTCACATCTGAAAACACTGTGGTAGATGGCCCATTCCCAGAAGAAAAACAGAAGA<br>1981 CATTACCCAGACTACCTAAATCTGCTCCAGCCGTTAAACTCTCCCCAGTGGCTGCATCTC<br>2041 CAGGACAGACTCTTCAGTTCAAGCTGAGATTAGACCTCCCATCGGGAGCAAAACTAACTG<br>2101 AAGGAGCACCCAGTTGCTGGTTTCTAACAGCTGAAGGCAATGAATGGCTTCTTCAAGGAC<br>2161 AGATACCATCTGGAGAAATAGAGAGCATTTCCAATCAACCCACAATCTCACTACAGATTC<br>2221 CTGGAGATTGTGTATCGCTTGAAGCTGTTATATCTGTCAGCGTGTTTCTTTACTACTGTA<br>2281 GCGCAGACAGCAGTGCCTGTATGATGAAGGGAATTTTGTTCAGTCAGCCTTTACAAATAA<br>2341 GCGCTACGCAACAAGGTTGCATAGCTCCAGTAGAGCTCCAATATGTATTTTAGCAAGCAA<br>2401 GCTAGCTAGCAACCCATTGCCACCACCTACTGTCTCCCATCCTGACTATCACTGTAATTT<br>2461 AAGGAAAGAAAACTTTATTTCTGCCTCTGGATACCAAGAAACCCATTGCTGAGTTCTAGC<br>2521 CACTGATCTTAAAATAAAGCTATGCTCCTT | |
| | | 1 M A A P G G R G R S L S G L L P A Q T S<br>1 ATGGCGGCGCCCGGAGGCCGGGGCCGCAGCCTCTCCGGCCTGCTCCCCGCGCAGACCTCG<br><br>21 L E Y A L L D A V T Q Q E K D S L V Y Q<br>61 CTAGAGTACGCCCTGCTCGACGCCGTTACCCAGCAGGAGAAGGACAGCCTGGTCTACCAG<br><br>41 Y L Q K V D G W E Q D L S V P E F P E G<br>121 TATCTGCAGAAGGTGGACGGCTGGGAGCAGGACTTGTCAGTACCCGAGTTTCCGGAAGGA<br><br>61 L E W L N T E E P I S V Y K D L C G K I<br>181 TTAGAATGGCTGAACACAGAAGAACCTATTTCTGTCTACAAGGATCTATGTGGAAAAATA | SEQ ID NO:32 |

| Gene Name | GenBank Homology | DNA SEQUENCE / DEDUCED AMINO ACID SEQUENCE | SEQUENCE IDENTIFIER: |
|---|---|---|---|
| | | 81 V V L D F F T Y C C I N C I H L L P D L<br>241 GTCGTCCTTGATTTCTTCACCTACTGCTGCATAAACTGTATTCACCTATTGCCTGATCTC<br><br>101 H A L E H T Y S D K D G L L I I G V H S<br>301 CATGCATTAGAACACACATACTCTGATAAAGATGGTCTTCTTATTATTGGTGTTCACTCG<br><br>121 A K F P N E K V L D N I K S A V L R Y N<br>361 GCTAAGTTTCCAAATGAAAAAGTCCTGGATAACATTAAGAGTGCTGTTCTTCGATACAAC<br><br>141 I T H P M V N D A D A S L W Q E L E V S<br>421 ATCACCCACCCTATGGTTAATGATGCAGATGCCAGCCTTTGGCAAGAACTAGAAGTTTCC<br><br>161 C W P T L V I L G P R G N M L F S L I G<br>481 TGCTGGCCAACTCTAGTCATACTTGGACCTCGTGGAAACATGTTGTTTTCTTTGATTGGA<br><br>181 E G H K D K L F L Y T S I A L K Y Y K D<br>541 GAGGGACACAAAGATAAATTATTTTTATATACTTCAATTGCTTTAAAGTATTACAAAGAC<br><br>201 R G Q I R D N K I G I K L Y K D S L P P<br>601 AGGGGGCAGATCAGAGATAATAAAATTGGAATAAAACTCTATAAAGATTCTTTGCCACCT<br><br>221 S P L L F P G K V T V D Q V T D R L V I<br>661 TCACCATTGCTATTTCCTGGCAAAGTAACAGTAGACCAAGTTACTGATAGATTGGTAATA<br><br>241 A D T G H H R I L V V W K N G Q I Q Y S<br>721 GCAGACACTGGACATCATAGAATTTTGGTCGTTTGGAAGAATGGACAAATTCAATATAGC<br><br>261 I G G P N P G R K D G I F S E S T F N S<br>781 ATTGGAGGACCCAACCCTGGAAGAAAAGATGGAATATTTTCAGAATCAACTTTTAATTCT<br><br>281 P Q G V A I M N N I I Y V A D T E N H L<br>841 CCACAGGGTGTAGCCATAATGAATAATATCATATATGTGGCAGACACTGAAAACCACCTT<br><br>301 I R K I D L E A E K V S T V A G I G I Q<br>901 ATAAGAAAGATTGACCTAGAAGCTGAGAAGGTGAGCACTGTAGCTGGTATTGGAATTCAA<br><br>321 G T D K E G G A K G E Q Q P I S S P W D<br>961 GGTACAGATAAAGAAGGTGGAGCAAAAGGAGAACAGCAGCCCATTAGTTCCCCTTGGGAT<br><br>341 V V F G T S G S E V Q R D D I L W I A M<br>1021 GTGGTTTTTGGAACATCAGGTTCAGAGGTCCAACGAGATGACATTCTGTGGATAGCCATG<br><br>361 A G T H Q I W A L L L D S G R L P K K N | |

| Gene Name | GenBank Homology | DNA SEQUENCE / DEDUCED AMINO ACID SEQUENCE | SEQUENCE IDENTIFIER: |
|---|---|---|---|
| | | 1081 GCAGGGACTCATCAGATATGGGCACTCCTGTTGGACTCTGGCAGACTACCAAAGAAAAAT<br><br>381 E L K K G T C L Q F A G S G N E E N R N<br>1141 GAGTTAAAAAAAGGAACCTGCCTTCAGTTTGCTGGAAGTGGAAATGAAGAGAATCGAAAT<br><br>401 N A Y P H K A G F A Q P S G L S V A S E<br>1201 AATGCATATCCTCACAAGGCAGGTTTTGCCCAACCTTCAGGTCTTTCTGTGGCCTCAGAA<br><br>421 D P W S C L F V A D S E S S T V R T V S<br>1261 GATCCCTGGAGCTGCCTGTTCGTAGCAGATAGTGAGAGCAGCACCGTGAGAACCGTCTCA<br><br>441 L K D G A V K H L V G G E R D P M D L F<br>1321 CTGAAGGATGGAGCCGTGAAGCACCTTGTGGGAGGAGAAAGAGACCCCATGGACTTATTT<br><br>461 A F G D V D G V G I N A K L Q H P L G V<br>1381 GCTTTTGGTGATGTTGATGGAGTAGGAATCAATGCAAAGCTTCAGCACCCACTTGGAGTA<br><br>481 T W D K K R N L V Y V A D S Y N H K I K<br>1441 ACTTGGGACAAGAAAAGGAATTTAGTTTATGTGGCAGACTCTTATAATCACAAGATTAAA<br><br>501 V V D P K T K T C T T L A G T G D A S N<br>1501 GTTGTGGATCCCAAAACGAAAACCTGTACAACATTAGCAGGAACTGGAGATGCAAGTAAT<br><br>521 V I S S S F T Q S T F N E P G G L C I G<br>1561 GTTATCAGTTCCAGTTTTACTCAGTCAACTTTTAATGAGCCAGGAGGCTTGTGTATTGGA<br><br>541 E N G Q L L Y V A D T N N H Q I K V M D<br>1621 GAGAATGGTCAGTTATTATATGTAGCAGACACCAATAATCATCAGATTAAAGTGATGGAT<br><br>561 L E T K M I S V L P L F T S E N T V V D<br>1681 TTAGAAACAAAAATGATTTCTGTGCTCCCACTCTTCACATCTGAAAACACTGTGGTAGAT<br><br>581 G P F P E E K Q K T L P R L P K S A P A<br>1741 GGCCCATTCCCAGAAGAAAAACAGAAGACATTACCCAGACTACCTAAATCTGCTCCAGCC<br><br>601 V K L S P V A A S P G Q T L Q F K L R L<br>1801 GTTAAACTCTCCCCAGTGGCTGCATCTCCAGGACAGACTCTTCAGTTCAAGCTGAGATTA<br><br>621 D L P S G A K L T E G A P S C W F L T A<br>1861 GACCTCCCATCGGGAGCAAAACTAACTGAAGGAGCACCCAGTTGCTGGTTTCTAACAGCT<br><br>641 E G N E W L L Q G Q I P S G E I E S I S<br>1921 GAAGGCAATGAATGGCTTCTTCAAGGACAGATACCATCTGGAGAAATAGAGAGCATTTCC | |

| Gene Name | GenBank Homology | DNA SEQUENCE / DEDUCED AMINO ACID SEQUENCE | SEQUENCE IDENTIFIER: |
|---|---|---|---|
| | | 661 N Q P T I S L Q I P G D C V S L E A V I<br>1981 AATCAACCCACAATCTCACTACAGATTCCTGGAGATTGTGTATCGCTTGAAGCTGTTATA<br><br>681 S V S V F L Y Y C S A D S S A C M M K G<br>2041 TCTGTCAGCGTGTTTCTTTACTACTGTAGCGCAGACAGCAGTGCCTGTATGATGAAGGGA<br><br>701 I L F S Q P L Q I S A T Q Q G C I A P V<br>2101 ATTTTGTTCAGTCAGCCTTTACAAATAAGCGCTACGCAACAAGGTTGCATAGCTCCAGTA<br><br>721 E L Q Y V F -<br>2161 GAGCTCCAATATGTATTTTAG | |
| WBC008D09.bFSP_20 010110.abd | Homo sapiens cDNA: FLJ21472 fis, clone COL04936. | 1 CATCACCCTTTTTAACATTGTTTACACATCACTGCCTGTTTTAGCCATGGGGATTTTTGA<br>61 CCAGGATGTGAGTGACCAGAACAGCGTGGACTGTCCCCAGCTCTACAAACCAGGACAGCT<br>121 GAATCTGCTTTTTAACAAGCGTAAATTTTCCATTTGCGTGTTGCATGGAATCTACACCTC<br>181 ATTAGTCCTTTTCTTCATCCCCTATGGGGCCTTTTACAACGTGGCTGGAGAAGATGGGCA<br>241 ACATATTGCTGACTACCAGTCCTCTGCAGTTACATGGCCACATCTTTGGTCATTGTGGTC<br>301 AGTGTGCAGATAGCCTTGGATACCAGTTACTGGACTTTCATTAATCACGTCTTCATCTGG<br>361 GGGAGCATTGCCATTTATTTCTCCATTTTATTTACAATGCACAGTAATGGCATCTTTGGC<br>421 ATCTTCCCAAACCAGTTTCCATTTGTTGGTAATGCACGACATTCCCTGACCCAGAAGTGC<br>481 ATCTGGCTTGTAATTCTCTTAACAACAGTGGCTTCAGTTATGCCAGTGGTGGCATTCAGA<br>541 TTTTTGAAGGTGGATTTATACCCAACCCTGAGTGATCAGATCCGCCGGTGGCAGAAGGCT<br>601 CAAAAGAAGGCAAGGCCTCCAAGTAGCCGAAGGCCTCGGACCCGCAGGTCAAGCTCAAGA<br>661 AGGTCTGGATATGCTTTTGCTCACCAAGAAGGCTATGGAGAGCTTATCACATCTGGAAAA<br>721 AATATGCGAGCTAAAAATCCACCCCCAACATCAGGGCTGGAAAAGACACATTATAATAGC<br>781 ACTAGCTGGATTGAAAATTTATGTAAGAAAACCACAGACACCGTGAGCAGCTTTAGCCAG<br>841 GATAAAACAGTGAAACTGTGAGTCAATATGAATTTAAACCACGTAGTTATCTTTTCACTT<br>901 CAGGTGGAGCTGAAATTCTGCTGGCTCCAGAGTTTGAGATTTGAGGCAAGAGGTGGGGCA<br>961 GGCAGATTGCCTCACTTAACTTAAATCTGCGGCAGACAACTGCCAGTGCCCATCAAACAG<br>1021 GAGTGTGCGCTATGGAAAACCAGGCCAGATGGTCACTGTCGAATTCGTGATTTGGTGGAC<br>1081 AAAACACTCGCTGTTACAAGTACAGATTTTTTTTTTTTTAAATCAACCTAGATACCAAT<br>1141 GGACCTGAACTTCAGAATCTTATTTATGGAGAAAAACTTGTAAGTCTGCATATACATTGA<br>1201 ATGAATCTTCAGATGGATAAAACGGTGCAATCAAAAGGTAATCAAAATATCCCAGCTGAA<br>1261 GAGTGTGCTTGTTGACAGATAAACACGTATAGGTAAGCTCAGCCTTTCCCAGGATATGCT<br>1321 TTTAAGATTTAAAATGCGTCCTAAGGTGCTTTCAGACTAAGAGCAGCTTGTCACTTTCCA<br>1381 GTCTTGATATCTACCTGTGTGTTCAACTGAGACAACACATCCCCTTCCGGCCTGAAGAGT<br>1441 AGAGGAATGTGTTGGTGTTCCTCATTTTTCTACTAGTTTAGAGATGAAGTAGAGTGAGCA<br>1501 TCTTTACAGTGGGATGGGCTGGTAATGTGGTCAAATTTATTCAAAAAGCTCTCAGTCTGT<br>1561 GTCATGTAAGGACATGCTTATGAAATGTGAGAGAGGCTCGCCACTAAGTATTCTAAATAC<br>1621 TTTTCAATGGCTTTTCTAACAACCTCAGTAGTAATTTGCTGAGCATCATCCAGACCATTA<br>1681 ATAGAACCAGCAAAGCACTGGAATTTCACCCTTTAATGATTGTTTTTCATATAGTATATG | SEQ ID NO:33 |

| Gene Name | GenBank Homology | DNA SEQUENCE / DEDUCED AMINO ACID SEQUENCE | SEQUENCE IDENTIFIER: |
|---|---|---|---|
| | | 1741 GGCAAATACCTTCCAAGTTTCCAATTTTAAAAAGTTCAGATTTGAAACCAAACAGATTAA<br>1801 TAAACAATCTCTTTCATTACTATTTAAAAACTCAGGTTTAGATTGTTTAAAATTAGTTGC<br>1861 TTTTGATACTCAGCTGTCATGTTTATAATTCAAACATGTAGTAAACATATGTAGGTAAGG<br>1921 TTGTTTTTTTGGAGATGTTGCAGCTCAAATTTCAGTCCACATATGAATCATCAGTGTATT<br>1981 TTCCATAAAGTGATTCGGGCATATTTGTGTGAAAACCTCAGTTCTGTCACTTCTTACCTC<br>2041 TATAAACTTGGACGATAATGAAAAAAAAAAAAAAAAAA<br><br>1 M A T S L V I V V S V Q I A L D T S Y W<br>1 ATGGCCACATCTTTGGTCATTGTGGTCAGTGTGCAGATAGCCTTGGATACCAGTTACTGG<br><br>21 T F I N H V F I W G S I A I Y F S I L F<br>61 ACTTTCATTAATCACGTCTTCATCTGGGGGAGCATTGCCATTTATTTCTCCATTTTATTT<br><br>41 T M H S N G I F G I F P N Q F P F V G N<br>121 ACAATGCACAGTAATGGCATCTTTGGCATCTTCCCAAACCAGTTTCCATTTGTTGGTAAT<br><br>61 A R H S L T Q K C I W L V I L L T T V A<br>181 GCACGACATTCCCTGACCCAGAAGTGCATCTGGCTTGTAATTCTCTTAACAACAGTGGCT<br><br>81 S V M P V V A F R F L K V D L Y P T L S<br>241 TCAGTTATGCCAGTGGTGGCATTCAGATTTTTGAAGGTGGATTTATACCCAACCCTGAGT<br><br>101 D Q I R R W Q K A Q K K A R P P S S R R<br>301 GATCAGATCCGCCGGTGGCAGAAGGCTCAAAAGAAGGCAAGGCCTCCAAGTAGCCGAAGG<br><br>121 P R T R R S S S R R S G Y A F A H Q E G<br>361 CCTCGGACCCGCAGGTCAAGCTCAAGAAGGTCTGGATATGCTTTTGCTCACCAAGAAGGC<br><br>141 Y G E L I T S G K N M R A K N P P P T S<br>421 TATGGAGAGCTTATCACATCTGGAAAAAATATGCGAGCTAAAAATCCACCCCCAACATCA<br><br>161 G L E K T H Y N S T S W I E N L C K K T<br>481 GGGCTGGAAAAGACACATTATAATAGCACTAGCTGGATTGAAAATTTATGTAAGAAAACC<br><br>181 T D T V S S F S Q D K T V K L -<br>541 ACAGACACCGTGAGCAGCTTTAGCCAGGATAAAACAGTGAAACTGTGA | SEQ ID NO:34 |
| WBC009F04.bFSP_20 010310.abd | Homo sapiens integrin, beta 1 (fibronectin receptor, beta polypeptide, antigen CD29 | 1 AGCGGGAGTCGCGGAACAGCAGGCCCGAGCCCACCGCGCCGGGCCCCGGACGCCGCGCGG<br>61 AAAAGATGAATTTACAACCAATTTCTGGATTGGACTGATCAGTTCAGTTTGCTGTGTGT<br>121 TTGCTCAAACAGATGAAAATAGATGTTTAAAAGCAAATGCCAAATCATGTGGAGAATGTA<br>181 TACAAGCAGGGCCAAATTGTGGGTGGTGCACAAATTCAACATTTTTACAGGAAGGAATGC<br>241 CTACTTCTGCACGATGTGATGATTTAGAAGCCTTAAAAAAGAAGGGTTGCCCTCCAGATG | SEQ ID NO:35 |

| Gene Name | GenBank Homology | DNA SEQUENCE / DEDUCED AMINO ACID SEQUENCE | SEQUENCE IDENTIFIER: |
|---|---|---|---|
| | includes MDF2, MSK12), transcript variant 1A, mRNA (cDNA clone MGC:17220 IMAGE:3924411). | 301 ACATAGAAAATCCCAGAGGCTCCAAAGATATAAAGAAAAATAAAAATGTAACCAACCGTA<br>361 GCAAAGGAACAGCAGAGAAGCTCAAGCCAGAGGATATTACTCAGATCCAACCACAGCAGT<br>421 TGGTTTTGCGATTAAGATCAGGGGAGCCACAGACATTTACATTAAAATTCAAGAGAGCTG<br>481 AAGACTATCCCATTGACCTCTACTACCTTATGGACCTGTCTTACTCAATGAAAGACGATT<br>541 TGGAGAATGTAAAAAGTCTTGGAACAGATCTGATGAATGAAATGAGGAGGATTACTTCGG<br>601 ACTTCAGAATTGGATTTGGCTCATTTGTGGAAAAGACTGTGATGCCTTACATTAGCACAA<br>661 CACCAGCTAAGCTCAGGAACCCTTGCACAAGTGAACAGAACTGCACCAGCCCATTTAGCT<br>721 ACAAAAATGTGCTCAGTCTTACTAATAAAGGAGAAGTATTTAATGAACTTGTTGGAAAAC<br>781 AGCGCATATCTGGAAATTTGGATTCTCCAGAAGGTGGTTTCGATGCCATCATGCAAGTTG<br>841 CAGTTTGTGGATCACTGATTGGCTGGAGGAATGTTACACGGCTGCTGGTGTTTTCCACAG<br>901 ATGCCGGGTTTCACTTTGCTGGAGATGGGAAACTTGGTGGCATTGTTTTACCAAATGATG<br>961 GACAATGTCACCTGGAAAAATAATATGTACACAATGAGCCATTATTATGATTATCCTTCTA<br>1021 TTGCTCACCTTGTCCAGAAACTGAGTGAAAATAATATTCAGACAATTTTTGCAGTTACTG<br>1081 AAGAATTTCAGCCTGTTTACAAGGAGCTGAAAAACTTGATCCCTAAGTCAGCAGTAGGAA<br>1141 CATTATCTGCAAATTCTAGCAATGTAATTCAGTTGATCATTGATGCATACAATTCCCTTT<br>1201 CCTCAGAAGTCATTTTGGAAAACGGCAAATTGTCAGAAGGAGTAACAATAAGTTACAAAT<br>1261 CTTACTGCAAGAACGGGGTGAATGGAACAGGGGAAAATGGAAGAAAATGTTCCAATATTT<br>1321 CCATTGGAGATGAGGTTCAATTTGAAATTAGCATAACTTCAAATAAGTGTCCAAAAAAGG<br>1381 ATTCTGACAGCTTTAAAATTAGGCCTCTGGGCTTTACGGAGGAAGTAGAGGTTATTCTTC<br>1441 AGTACATCTGTGAATGTGAATGCCAAAGCGAAGGCATCCCTGAAAGTCCCAAGTGTCATG<br>1501 AAGGAAATGGGACATTTGAGTGTGGCGCGTGCAGGTGCAATGAAGGGCGTGTTGGTAGAC<br>1561 ATTGTGAATGCAGCACAGATGAAGTTAACAGTGAAGACATGGATGCTTACTGCAGGAAAG<br>1621 AAAACAGTTCAGAAATCTGCAGTAACAATGGAGAGTGCGTCTGCGGACAGTGTGTTTGTA<br>1681 GGAAGAGGGATAATACAAATGAAATTTATTCTGGCAAATTCTGCGAGTGTGATAATTTCA<br>1741 ACTGTGATAGATCCAATGGCTTAATTTGTGGAGGAAATGGTGTTTGCAAGTGTCGTGTGT<br>1801 GTGAGTGCAACCCCAACTACACTGGCAGTGCATGTGACTGTTCTTTGGATACTAGTACTT<br>1861 GTGAAGCCAGCAACGGACAGATCTGCAATGGCCGGGGCATCTGTGAGTGTGGTGTCTGTA<br>1921 AGTGTACAGATCCGAAGTTTCAAGGGCAAACGTGTGAGATGTGTCAGACCTGCCTTGGTG<br>1981 TCTGTGCTGAGCATAAAGAATGTGTTCAGTGCAGAGCCTTTAATAAAGGAGAAAAGAAAG<br>2041 ACACATGCGCCCAGGAATGTTCACATTTCAACATTACCAAGGTTGAAAATCGGGACAAGT<br>2101 TACCTCAGCCAGGCCAGGTTGATCCCCTGTCTCACTGTAAGGAGAAGGATGTGGATGACT<br>2161 GCTGGTTCTATTTCACGTATTCAGTGAATGGGAACAATGAGGCCATTGTTCACGTTGTGG<br>2221 AGACTCCAGAGTGCCCCACTGGTCCAGACATCATTCCAATTGTAGCTGGTGTGGTTGCTG<br>2281 GAATTGTTCTTATTGGCCTTGCATTGCTGCTGATTTGGAAGCTTTTAATGATAATTCATG<br>2341 ACAGAAGGGAATTTGCCAAATTTGAGAAGGAAAAAATGAATGCCAAATGGGACACGGGTG<br>2401 AAAATCCTATTTATAAGAGTGCTGTGACAACTGTTGTCAATCCGAAGTACGAGGGAAAAT<br>2461 GAGCACTCGCTGTGCAAATTTCACAACACTGAATGCAAAGCAGTACTTTTCCTAGTCACA<br>2521 GTAAGGTAGCTGTAGGGCTGTACCGCCATGGGGTTACTCATGTGCCGGTTTTGAAAGTGT<br>2581 ACAATATGTATAATTTTAAAAATTTGTTATTATTTTGAAAATAATGTTATCATCAATGCC<br>2641 AGGGACTGACCAGAGACTTGAGACAGGATGGTTGTCCTCATCATAAGGTCACGTGGTGCC<br>2701 TTTTTGACCTTTTACTCCTGGGCTGTTGAAATCAAGTTCTTATTGGATCGAGTGGTGTGT<br>2761 CTACAGTGATGGAAGGGCAGTAGTTAAAATAATGAGTATGATGAGAGTTTCTATTAATCA<br>2821 CGTATTAAAACTGATCTTTAGCTTTACAGATATGTCAGTTTCAGTTATACAGAATCCAC | |

| Gene Name | GenBank Homology | DNA SEQUENCE / DEDUCED AMINO ACID SEQUENCE | SEQUENCE IDENTIFIER: |
|---|---|---|---|
| | | 2881 AGTAAATTTCTGCTACCTAGTTTAGAATTGTTTTAAATCTGTTATTTTGCTATTTGCCTG<br>2941 TTAGACATGACTGATGACATATCTGAAAGACAAGTATGTTGAGAGTTGCTGGTGTAAAAT<br>3001 ACGTTTGAAATAGTTGATCTACAGAGGCCATGGGACAAAGTCAGAGAGTTAGAAAGGGAA<br>3061 ACCATAGCTTTAAAACCTGTGTGCCATTTTGAGAGTTACTTAATCTTGGTAACTTTTATG<br>3121 CCTTCTTTTTATAAATTCAAGCCTTCAGGAAAAGTATTGGGAAATTTTCTGCAAAAAAGT<br>3181 CCTTGATTTAGCATTATTTACATAAAGGCCTTAATTTCCATAGTACTTGCTGAATTGGGG<br>3241 ACCTTTGGGTGAATTTGTTTTATTCATTTTATTTGTTTGAAGCCTGGTGCTTTTTACCA<br>3301 CCTCTTCTAATCATTTAATGTATTTGTTTGCAATTTGGGGGTAAGACTTTTTTTATGAGT<br>3361 ACTTTTTCTTTGAAGTTTTAGCGGTCAATTTGCCTTTTTAATGAACGTGTGAAGTTGTAC<br>3421 TGTGGCTTACGCAACAGCTGTCACTTAAGCGACTCTTATTTTCAGGCAGTGCCGTAATAG<br>3481 ACCGCTGTATAGTTACTTCTCACTGTCAGAGCTGCCCATTTTGTTTCATACCAGTCACAT<br>3541 CCTTGTTTTAAGTGCCTTTTAATTTTAACAGTTCACTTTTTACATGCTGTTTACTGAAGT<br>3601 TATTTATTAAATATGCCTAAAATACTTAAATCGGAAAAAAAAAAAAAAAAA<br><br>1 M N L Q P I F W I G L I S S V C C V F A<br>1 ATGAATTTACAACCAATTTTCTGGATTGGACTGATCAGTTCAGTTTGCTGTGTGTTTGCT<br><br>21 Q T D E N R C L K A N A K S C G E C I Q<br>61 CAAACAGATGAAAATAGATGTTTAAAAGCAAATGCCAAATCATGTGGAGAATGTATACAA<br><br>41 A G P N C G W C T N S T F L Q E G M P T<br>121 GCAGGGCCAAATTGTGGGTGGTGCACAAATTCAACATTTTTACAGGAAGGAATGCCTACT<br><br>61 S A R C D D L E A L K K K G C P P D D I<br>181 TCTGCACGATGTGATGATTTAGAAGCCTTAAAAAAGAAGGGTTGCCCTCCAGATGACATA<br><br>81 E N P R G S K D I K K N K N V T N R S K<br>241 GAAAATCCCAGAGGCTCCAAAGATATAAAGAAAAATAAAAATGTAACCAACCGTAGCAAA<br><br>101 G T A E K L K P E D I T Q I Q P Q Q L V<br>301 GGAACAGCAGAGAAGCTCAAGCCAGAGGATATTACTCAGATCCAACCACAGCAGTTGGTT<br><br>121 L R L R S G E P Q T F T L K F K R A E D<br>361 TTGCGATTAAGATCAGGGGAGCCACAGACATTTACATTAAAATTCAAGAGAGCTGAAGAC<br><br>141 Y P I D L Y Y L M D L S Y S M K D D L E<br>421 TATCCCATTGACCTCTACTACCTTATGGACCTGTCTTACTCAATGAAAGACGATTTGGAG<br><br>161 N V K S L G T D L M N E M R R I T S D F<br>481 AATGTAAAAAGTCTTGGAACAGATCTGATGAATGAAATGAGGAGGATTACTTCGGACTTC<br><br>181 R I G F G S F V E K T V M P Y I S T T P<br>541 AGAATTGGATTTGGCTCATTTGTGGAAAAGACTGTGATGCCTTACATTAGCACAACACCA | SEQ ID NO:36 |

| Gene Name | GenBank Homology | DNA SEQUENCE / DEDUCED AMINO ACID SEQUENCE | SEQUENCE IDENTIFIER: |
|---|---|---|---|
| | | 201 A K L R N P C T S E Q N C T S P F S Y K<br>601 GCTAAGCTCAGGAACCCTTGCACAAGTGAACAGAACTGCACCAGCCCATTTAGCTACAAA<br><br>221 N V L S L T N K G E V F N E L V G K Q R<br>661 AATGTGCTCAGTCTTACTAATAAAGGAGAAGTATTTAATGAACTTGTTGGAAAACAGCGC<br><br>241 I S G N L D S P E G G F D A I M Q V A V<br>721 ATATCTGGAAATTTGGATTCTCCAGAAGGTGGTTTCGATGCCATCATGCAAGTTGCAGTT<br><br>261 C G S L I G W R N V T R L L V F S T D A<br>781 TGTGGATCACTGATTGGCTGGAGGAATGTTACACGGCTGCTGGTGTTTTCCACAGATGCC<br><br>281 G F H F A G D G K L G G I V L P N D G Q<br>841 GGGTTTCACTTTGCTGGAGATGGGAAACTTGGTGGCATTGTTTTACCAAATGATGGACAA<br><br>301 C H L E N N M Y T M S H Y Y D Y P S I A<br>901 TGTCACCTGGAAAATAATATGTACACAATGAGCCATTATTATGATTATCCTTCTATTGCT<br><br>321 H L V Q K L S E N N I Q T I F A V T E E<br>961 CACCTTGTCCAGAAACTGAGTGAAAATAATATTCAGACAATTTTTGCAGTTACTGAAGAA<br><br>341 F Q P V Y K E L K N L I P K S A V G T L<br>1021 TTTCAGCCTGTTTACAAGGAGCTGAAAAACTTGATCCCTAAGTCAGCAGTAGGAACATTA<br><br>361 S A N S S N V I Q L I I D A Y N S L S S<br>1081 TCTGCAAATTCTAGCAATGTAATTCAGTTGATCATTGATGCATACAATTCCCTTTCCTCA<br><br>381 E V I L E N G K L S E G V T I S Y K S Y<br>1141 GAAGTCATTTTGGAAAACGGCAAATTGTCAGAAGGAGTAACAATAAGTTACAAATCTTAC<br><br>401 C K N G V N G T G E N G R K C S N I S I<br>1201 TGCAAGAACGGGGTGAATGGAACAGGGGAAAATGGAAGAAAATGTTCCAATATTTCCATT<br><br>421 G D E V Q F E I S I T S N K C P K K D S<br>1261 GGAGATGAGGTTCAATTTGAAATTAGCATAACTTCAAATAAGTGTCCAAAAAAGGATTCT<br><br>441 D S F K I R P L G F T E E V E V I L Q Y<br>1321 GACAGCTTTAAAATTAGGCCTCTGGGCTTTACGGAGGAAGTAGAGGTTATTCTTCAGTAC<br><br>461 I C E C E C Q S E G I P E S P K C H E G<br>1381 ATCTGTGAATGTGAATGCCAAAGCGAAGGCATCCCTGAAAGTCCCAAGTGTCATGAAGGA | |

| Gene Name | GenBank Homology | DNA SEQUENCE / DEDUCED AMINO ACID SEQUENCE | SEQUENCE IDENTIFIER: |
|---|---|---|---|
| | | 481 N G T F E C G A C R C N E G R V G R H C<br>1441 AATGGGACATTTGAGTGTGGCGCGTGCAGGTGCAATGAAGGGCGTGTTGGTAGACATTGT<br><br>501 E C S T D E V N S E D M D A Y C R K E N<br>1501 GAATGCAGCACAGATGAAGTTAACAGTGAAGACATGGATGCTTACTGCAGGAAAGAAAAC<br><br>521 S S E I C S N N G E C V C G Q C V C R K<br>1561 AGTTCAGAAATCTGCAGTAACAATGGAGAGTGCGTCTGCGGACAGTGTGTTTGTAGGAAG<br><br>541 R D N T N E I Y S G K F C E C D N F N C<br>1621 AGGGATAATACAAATGAAATTTATTCTGGCAAATTCTGCGAGTGTGATAATTTCAACTGT<br><br>561 D R S N G L I C G G N G V C K C R V C E<br>1681 GATAGATCCAATGGCTTAATTTGTGGAGGAAATGGTGTTTGCAAGTGTCGTGTGTGTGAG<br><br>581 C N P N Y T G S A C D C S L D T S T C E<br>1741 TGCAACCCCAACTACACTGGCAGTGCATGTGACTGTTCTTTGGATACTAGTACTTGTGAA<br><br>601 A S N G Q I C N G R G I C E C G V C K C<br>1801 GCCAGCAACGGACAGATCTGCAATGGCCGGGGCATCTGTGAGTGTGGTGTCTGTAAGTGT<br><br>621 T D P K F Q G Q T C E M C Q T C L G V C<br>1861 ACAGATCCGAAGTTTCAAGGGCAAACGTGTGAGATGTGTCAGACCTGCCTTGGTGTCTGT<br><br>641 A E H K E C V Q C R A F N K G E K K D T<br>1921 GCTGAGCATAAAGAATGTGTTCAGTGCAGAGCCTTTAATAAAGGAGAAAAGAAAGACACA<br><br>661 C A Q E C S H F N I T K V E N R D K L P<br>1981 TGCGCCCAGGAATGTTCACATTTCAACATTACCAAGGTTGAAAATCGGGACAAGTTACCT<br><br>681 Q P G Q V D P L S H C K E K D V D D C W<br>2041 CAGCCAGGCCAGGTTGATCCCCTGTCTCACTGTAAGGAGAAGGATGTGGATGACTGCTGG<br><br>701 F Y F T Y S V N G N N E A I V H V V E T<br>2101 TTCTATTTCACGTATTCAGTGAATGGGAACAATGAGGCCATTGTTCACGTTGTGGAGACT<br><br>721 P E C P T G P D I I P I V A G V V A G I<br>2161 CCAGAGTGCCCCACTGGTCCAGACATCATTCCAATTGTAGCTGGTGTGGTTGCTGGAATT<br><br>741 V L I G L A L L L I W K L L M I I H D R<br>2221 GTTCTTATTGGCCTTGCATTGCTGCTGATTTGGAAGCTTTTAATGATAATTCATGACAGA<br><br>761 R E F A K F E K E K M N A K W D T G E N | |

| Gene Name | GenBank Homology | DNA SEQUENCE / DEDUCED AMINO ACID SEQUENCE | SEQUENCE IDENTIFIER: |
|---|---|---|---|
| | | 2281 AGGGAATTTGCCAAATTTGAGAAGGAAAAAATGAATGCCAAATGGGACACGGGTGAAAAT<br><br>781 P I Y K S A V T T V V N P K Y E G K -<br>2341 CCTATTTATAAGAGTGCTGTGACAACTGTTGTCAATCCGAAGTACGAGGGAAAATGA | |
| WBC009H07.bFSP_20 010310.abd | No Homology | 1 ATCCTCGTGCCGGGAGTGCAGATCCTTTGAACCATTGTTATGGTAGGACAGGCCCCTTCA<br>61 ACGGCCTGGGGAGCTGGAATAAGCTGGCTTACTGTTTGGAATCGTGCTGGTGGGTCAGAT<br>121 GTCTCTAGTTCTAGTAAAAAGCAACTGGAGTCTCCTGGGAGAAACTTCGGGAAGCAGAGT<br>181 TGTGTTAGGTAAGGCTCTGCTGACATTGCACAGTTTTAGACGAACGTTGGCACAGGATGT<br>241 TAGATTTCTGTGACAAGTTCATCTACCTATTTTAGTAAGCACATAGTTCTCCAGCTAAAA<br>301 TTTGAATTCTTCTTGAAATAAAAGAAGATGAAATGAAAGAATAATTCTTGAAATATCACT<br>361 CAAATACCCCCAGCATGTTGTGAGCTTCCTGTAAGTGGTGGGTTACAAATCTGCTGGGTG<br>421 CCAAGTGCTGGATAGTGCTGGGTGGCTGTGTAGGAACCCCCTTGGCTTGCGTGTGAAGGC<br>481 TTGCAGATTGCCAGGAGGCTCCAGCTAATGAGTTTTAGGTAGAGCCTGGAAAGCTGTGTG<br>541 TTAAACTTCTTTCCAAGGAATTCTGGTCACCAGGCGGCTTGGGAACGATGAGGGCAGGCC<br>601 ACATAGGATATGTTAGGCAGGAAAGAAACACCCTTTTAAAAGACAAGGGTAGAAGAGGAG<br>661 TGAAATACTTGGAACTCAGAACTAGCAAGAGAGTTGACAGATTTCCTATGATCTTTGGAA<br>721 ACTGTCTTCACTGATGAAAGTACACCGCTTTTGTAGACTATCTGATTAGAGGGCATTGTG<br>781 CTGCTTTTGTGAAGGGAAAGTGGCCGTGGTGGTGGAGATGTCAAAGACTTGGACAAGGGA<br>841 GCAGGGGCTGCCTAATTTCAAAGTCTGGTTTT<br><br>1 ACTTGTAGTGGTGACAGTTTAGAGAGTCTAAAACTCTGTAAGGTTGTAAAGGAGGCGTGA<br>61 CAAAACCAGCTGGAGTCAGGAACTGCTTCCACTCTGCTCATGCAACAGCCGAGTTAAGAA<br>121 ATAAGTGTTCCCCAGAATGCACACTGTGCTGCCACCCCCAGGTGACATTCTTTCCAGGTA<br>181 CATTTGACACCTCTTCTCGCACATCCTGTTCACCTGTTGGTGACCACACCCNTGGTCTCA<br>241 TGCGAGATTTAGTACACCTGCACAGTCCCTTCTCAGGGGCAGACCTCCTCCTGACGGCCT<br>301 CCGCCAGTGAGTTGCAGCCACCCGATGTTGCGTTTGGAAGTGTTTGTTGCTCCCGTTGGA<br>361 GAAAGTATTTGCATAAGCATTTCATGTGTCTAGTGTGGCATTTCTGGATGCGGCTGTCAC<br>421 AGCAGTGTTATTGGTAGTTAATCTTACTGCAAGGTGAACAGTATGGCTGTTTGCTTTGTC<br>481 CTTGCCATGGCGTTCTTAGAAAGTACCCTGTCTTTCAATATTGGTTTTTTCAACATGAAT<br>541 ATATTCTACTTACAAACTCGTTTTGCTTCTTTGTATATAATTTGCCTTGGGTTTATTGTG<br>601 CACAGTTTATTTAGTTATGTTTTCGTGTATTATCAGGAGCAATTTGATAAGTACATGTGA<br>661 ATAACCTCCTGTAAATGAATTTTATAACAAAATGTACTAAAATATTTTCTCAAAAAAAAA<br>721 AAAAAAAAA | SEQ ID NO:37<br><br><br><br><br><br><br><br><br><br><br><br><br><br><br><br>SEQ ID NO:38 |
| WBC011D03.bFSP_20 010510.abd | Homo sapiens nuclear autoantigen GS2NA mRNA. | 1 GGGGAAGGGCAGATGGAGGAGGGATTGTAATGGCGGCAGCCGGCAGCTCCCTGCTCTGAC<br>61 CCACGGCAGGCACACAGCAACGACCCCTTCCCCGCCCCTCTCCCGGCCGGCCTCCGCCCC<br>121 GCAGCCAGCGCGGGGCCACCCTCTCCGGCCCTTCCCCCAGCTGTCGGCGTCTCGCCCTGC<br>181 GCCCCGGCCGGGGCCCCACACACAATGGACGAGCTTGCCGGAGGCGGTGGTGGCGGCCCG<br>241 GGGATGGCGGCCCCTCCCCGGCAGCAGCAGGGACCTGGGGGGAACCTGGGCCTTTCGCCC<br>301 GGGGGGAACGGAGCGGCGGGCGGCGGGGGTCCTCCGGCCTCCGAGGGAGCGGGTCCCGCG | SEQ ID NO:39 |

| Gene Name | GenBank Homology | DNA SEQUENCE / DEDUCED AMINO ACID SEQUENCE | SEQUENCE IDENTIFIER: |
|---|---|---|---|
| | | 361 GCAGGCCCCGAGCTGTCCCGGCCGCAGCAGAACACTATCCCGGGGATACTGCACTACATC<br>421 CAGCACGAGTGGGCTCGGTTCGAGATGGAGCGGGCGCACTGGGAGGTGGAACGGGCCGAA<br>481 CTGCAGGCCCGGATTGCATTTCTACAAGGCGAAAGAAAAGGTCAAGAGAACCTGAAGAAG<br>541 GACTTAGTAAGAAGAATAAAGATGTTAGAGTATGCATTAAAACAAGAAAGGGCAAAATAT<br>601 CACAAATTAAAATATGGCACGGAACTGAACCAAGGTGACTTGAAAATGCCAACCTTTGAG<br>661 TCAGAAGAAACCAAAGACACAGAGGCTCCCACAGCACCTCAGAATAGCCAGTTAACGTGG<br>721 AAGCAAGGCAGACAGCTTTTAAGACAGTATCTTCAGGAAGTAGGTTATACAGATACAATA<br>781 TTAGATGTCCGGTCTCAGCGGGTAAGGTCATTACTTGGACTATCTAATTCAGAACCAAAT<br>841 GGATCAGTAGAAACAAAGAATTTAGAACAGATCCTGAATGGAGGTGAATCTCCTAAGCAA<br>901 AAGGGACAAGAAATAAAAAGGCCCTCTGGTGATGTTCTTGAGACATTCAATTTCTTAGAA<br>961 AATGCTGATGACAGTGATGAAGAAGAGGAAAATGACATGATTGAAGGCATCCCAGAAGGA<br>1021 AAGGACAAACATCGGATGAATAAACACAAAATAGGTAATGAAGGTTTAGCTGCTGACCTA<br>1081 ACTGATGATCCTGATACTGAAGAAGCACTTAAAGAGTTTGATTTTTAGTCACTGCTGAA<br>1141 GATGGTGAAGGAGCTGGAGAAGCACGGAGTTCAGGGGATGGCACAGAATGGGCTGAACCA<br>1201 ATAACGTTTCCATCTGGAGGAGGCAAGTCATTTATTATGGGTTCTGATGATGTTTTGTTA<br>1261 AGTGTACTGGGCCTTGGAGACCTTGCAGACTTGACGGTAACAAATGATGCAGACTATAGT<br>1321 TATGATTTGCCTGCCAATAAAGATGCCTTTCGAAAGACATGGAACCCCAAGTATACACTA<br>1381 CGTAGCCATTTTGATGGAGTACGGGCATTAGCTTTCCATCCTGTAGAACCTGTGCTGGTT<br>1441 ACTGCCTCTGAGGACCATACTCTGAAACTTTGGAACTTGCAAAAAACAGTTCCTGCCAAA<br>1501 AAGAGTGCCTCTTTGGATGTAGAGCCTATCTACACATTTAGGGCCCACATTGGTCCTGTT<br>1561 CTATCATTAGCTCTTAGTTCTAATGGAGAACAATGTTTTAGTGGTGGTATTGATGCAACC<br>1621 ATCCAGTGGTGGAATATGCCTAGCCCTAACGTGGATCCATATGACACATATGAGCCAAAT<br>1681 GTTCTAGCTGGCACTTTAGTTGCTCATACAGATGCTGTCTGGGGTCTTGCTTATAGTGGC<br>1741 ATAAAAAATCAGTTACTGTCTTGTTCAGCAGATGGCACTGTTAGGTTATGGAATCCGCAA<br>1801 GAAAAATTGCCGTGTATTTGCACTTACAATGGAGATAAAAAGCATGGAATACCTACATCA<br>1861 GTTGACTTTATAGGCTGTGATCCAGCTCATATGGTAACCTCTTTCAACACTGGTAGTGCA<br>1921 GTAATTTATGATTTAGAAACATCACAGTCATTGGTGATACTTTCATCACAGGTAGATTCT<br>1981 GGTTTACAATCTAATAATCATATCAACAGAGTAGTAAGTCATCCCACACTTCCTGTTACA<br>2041 ATAACTGCTCATGAAGATAGACACATCAAATTTTTTGACAATAAAACGGGTAAAATGATC<br>2101 CATTCTATGGTAGCTCACTTGGATGCTGTTACAAGTCTAGCAGTAGATCCTAATGGAATC<br>2161 TATTAAATGTCTGGAAGCCATGATTGTTCTATTAGATTGTGGAATTTGGACAGCAAGACA<br>2221 TGTGTGCAAGAAATAACAGCACATAGGAAGAAATTAGATGAATCAATTTATGATGTTGCT<br>2281 TTCCATCCATCAAAAGCATATATTGCCAGTGCAGGGGCTGATGCCCTGGCCAAAGTGTTT<br>2341 GTGTGAATCAACAGAAGCTCACATTCTAACAGCAGACTTGCGTGGACAGAAAGAGGGTCT<br>2401 GCATCACTGCCATCAGAAAGCTTCCTGATATGACACTACATGTGATCTGCCTGGTGAAGG<br>2461 CTATTGAGGACAGGCATAAATTGGTAGAAATCACATCTTAATGTCAGGCTCATTAATTTA<br>2521 ATTGTAATTACTGTATTTTGTGGACAAAAAAAAGGACTCCAGTATTTGTGGCCTGTACT<br>2581 GGATATGAACTGAGCGTATGTCTGTTTTTTAGGTATCTTTAAGCCAATGTGGAGTCTGAC<br>2641 CTTACAAAAAAGTTTTTTTTTCTCTTCTTCTTCTTCTTTTTTTTTTTTTTTTTTTTTTT<br>2701 ATTTTGGACTCTGTGTGCTGCCTTAGGGTTAGGAATGTGGTGCTCTTGTTGGGGGGAAGT<br>2761 GAGCTCCAAGAGTAGCTTTTTTTCATCTCTTAGTGATCTTCTGTTTATCAGTTGAATGCC<br>2821 ACAGATTCCCTTTTAAACTTATTTTGCTTTAAAAAAAAAAAAAAAAGAAAAAGAAAATTT<br>2881 AACTTAAAATATTTTAGCATTAGTTGCACAAAATGTTTGGATAAAATTCTAGTTTTTATA | |

| Gene Name | GenBank Homology | DNA SEQUENCE / DEDUCED AMINO ACID SEQUENCE | SEQUENCE IDENTIFIER: |
|---|---|---|---|
| | | 2941 AGTCTTTTTATATATTTAGCCTGTCACAACAGTGCTCAAGATTGTATTGAAGTTTTTCTG<br>3001 CATTATACAACCCTAAAACACAGAGATCTCACTGACCCGTTGCCCTGTAACCACTTTCTT<br>3061 TCCTTCTTTTGCCTAATACAGCTCAGCTAAGTCCTTCCATAAAGATGCTAAATAACTTTC<br>3121 ATCATCACATAAATGTCTTCATAAACCAAGGACTATTAAGTGTATTAAAATGAAACAGTG<br>3181 GGGTTTAGTACTCCAAATGAACTCTTAAAGAAAAAAACTAATCTTGGCATCCTATCACTA<br>3241 TGTGATATTTTGTACATCTTACTGTATTCACAAGTTTATTTATCAAGGATTATCCATCTT<br>3301 GCTAATGGCCTATTATTTATTTCACTTTTTGTTGGTCTTTATATCCTTTTATTAATGTGC<br>3361 TAAAGGAACCTGTATATCTATTTTGGAACTCTTTGAATAGTCTAAAATAGACTAAAAATG<br>3421 GAGTATTTTATAGTTTAAATTACAAACCAAGGTGGTTCCCCCTAAGATATATATCTTGGT<br>3481 TTACCATGATTCCAAACAAAACTAACTTGTTTAAAAATATTTGGAGTAAAGTTTTATTTG<br>3541 CATTACAAATAGGATACAAAAATAGTTGAATCAGGATACAGAAATCTGCTGTGTTTTGAC<br>3601 TAGTTATCCCTGTTTTATTTTTTTAGATGTGCTTAATTTATGGTGTAACTAAAGATTTTG<br>3661 TTTGTGTAATGCATGATTAAGACAATAAAGTATTTTTTCTAGTCTTCCGAGAAACTTTTC<br>3721 TGATCAGTTTGCGAGTTTTGATGAGTTTTGTAAGGTTTTTGTTTTACAAACTATGAATCA<br>3781 GCAAATTTTTAAGATTGTACCACATAGCAAACATACAGCTGTTGAAAAATAATGTATATA<br>3841 AAATGCATATAATAAATATTAAATTGTGTACCTGTA | |
| | | 1 M D E L A G G G G G G P G M A A P P R Q<br>1 ATGGACGAGCTTGCCGGAGGCGGTGGTGGCGGCCCGGGGATGGCGGCCCCTCCCCGGCAG<br><br>21 Q Q G P G G N L G L S P G G N G A A G G<br>61 CAGCAGGGACCTGGGGGGAACCTGGGCCTTTCGCCCGGGGGGAACGGAGCGGCGGGCGGC<br><br>41 G G P P A S E G A G P A A G P E L S R P<br>121 GGGGGTCCTCCGGCCTCCGAGGGAGCGGGTCCCGCGGCAGGCCCCGAGCTGTCCCGGCCG<br><br>61 Q Q N T I P G I L H Y I Q H E W A R F E<br>181 CAGCAGAACACTATCCCGGGGATACTGCACTACATCCAGCACGAGTGGGCTCGGTTCGAG<br><br>81 M E R A H W E V E R A E L Q A R I A F L<br>241 ATGGAGCGGGCGCACTGGGAGGTGGAACGGGCCGAACTGCAGGCCCGGATTGCATTTCTA<br><br>101 Q G E R K G Q E N L K K D L V R R I K M<br>301 CAAGGCGAAAGAAAAGGTCAAGAGAACCTGAAGAAGGACTTAGTAAGAAGAATAAAGATG<br><br>121 L E Y A L K Q E R A K Y H K L K Y G T E<br>361 TTAGAGTATGCATTAAAACAAGAAAGGGCAAAATATCACAAATTAAAATATGGCACGGAA<br><br>141 L N Q G D L K M P T F E S E E T K D T E<br>421 CTGAACCAAGGTGACTTGAAAATGCCAACCTTTGAGTCAGAAGAAACCAAAGACACAGAG<br><br>161 A P T A P Q N S Q L T W K Q G R Q L L R<br>481 GCTCCCACAGCACCTCAGAATAGCCAGTTAACGTGGAAGCAAGGCAGACAGCTTTTAAGA | SEQ ID NO:40 |

| Gene Name | GenBank Homology | DNA SEQUENCE / DEDUCED AMINO ACID SEQUENCE | SEQUENCE IDENTIFIER: |
|---|---|---|---|
| | | 181 Q Y L Q E V G Y T D T I L D V R S Q R V<br>541 CAGTATCTTCAGGAAGTAGGTTATACAGATACAATATTAGATGTCCGGTCTCAGCGGGTA<br><br>201 R S L L G L S N S E P N G S V E T K N L<br>601 AGGTCATTACTTGGACTATCTAATTCAGAACCAAATGGATCAGTAGAAACAAAGAATTTA<br><br>221 E Q I L N G G E S P K Q K G Q E I K R P<br>661 GAACAGATCCTGAATGGAGGTGAATCTCCTAAGCAAAAGGGACAAGAAATAAAAAGGCCC<br><br>241 S G D V L E T F N F L E N A D D S D E E<br>721 TCTGGTGATGTTCTTGAGACATTCAATTTCTTAGAAAATGCTGATGACAGTGATGAAGAA<br><br>261 E E N D M I E G I P E G K D K H R M N K<br>781 GAGGAAAATGACATGATTGAAGGCATCCCAGAAGGAAAGGACAAACATCGGATGAATAAA<br><br>281 H K I G N E G L A A D L T D D P D T E E<br>841 CACAAAATAGGTAATGAAGGTTTAGCTGCTGACCTAACTGATGATCCTGATACTGAAGAA<br><br>301 A L K E F D F L V T A E D G E G A G E A<br>901 GCACTTAAAGAGTTTGATTTTTTAGTCACTGCTGAAGATGGTGAAGGAGCTGGAGAAGCA<br><br>321 R S S G D G T E W A E P I T F P S G G G<br>961 CGGAGTTCAGGGGATGGCACAGAATGGGCTGAACCAATAACGTTTCCATCTGGAGGAGGC<br><br>341 K S F I M G S D D V L L S V L G L G D L<br>1021 AAGTCATTTATTATGGGTTCTGATGATGTTTTGTTAAGTGTACTGGGCCTTGGAGACCTT<br><br>361 A D L T V T N D A D Y S Y D L P A N K D<br>1081 GCAGACTTGACGGTAACAAATGATGCAGACTATAGTTATGATTTGCCTGCCAATAAAGAT<br><br>381 A F R K T W N P K Y T L R S H F D G V R<br>1141 GCCTTTCGAAAGACATGGAACCCCAAGTATACACTACGTAGCCATTTTGATGGAGTACGG<br><br>401 A L A F H P V E P V L V T A S E D H T L<br>1201 GCATTAGCTTTCCATCCTGTAGAACCTGTGCTGGTTACTGCCTCTGAGGACCATACTCTG<br><br>421 K L W N L Q K T V P A K K S A S L D V E<br>1261 AAACTTTGGAACTTGCAAAAAACAGTTCCTGCCAAAAAGAGTGCCTCTTTGGATGTAGAG<br><br>441 P I Y T F R A H I G P V L S L A L S S N<br>1321 CCTATCTACACATTTAGGGCCCACATTGGTCCTGTTCTATCATTAGCTCTTAGTTCTAAT | |

| Gene Name | GenBank Homology | DNA SEQUENCE / DEDUCED AMINO ACID SEQUENCE | SEQUENCE IDENTIFIER: |
|---|---|---|---|
| | | 461 G E Q C F S G G I D A T I Q W W N M P S<br>1381 GGAGAACAATGTTTTAGTGGTGGTATTGATGCAACCATCCAGTGGTGGAATATGCCTAGC<br><br>481 P N V D P Y D T Y E P N V L A G T L V A<br>1441 CCTAACGTGGATCCATATGACACATATGAGCCAAATGTTCTAGCTGGCACTTTAGTTGCT<br><br>501 H T D A V W G L A Y S G I K N Q L L S C<br>1501 CATACAGATGCTGTCTGGGGTCTTGCTTATAGTGGCATAAAAAATCAGTTACTGTCTTGT<br><br>521 S A D G T V R L W N P Q E K L P C I C T<br>1561 TCAGCAGATGGCACTGTTAGGTTATGGAATCCGCAAGAAAAATTGCCGTGTATTTGCACT<br><br>541 Y N G D K K H G I P T S V D F I G C D P<br>1621 TACAATGGAGATAAAAAGCATGGAATACCTACATCAGTTGACTTTATAGGCTGTGATCCA<br><br>561 A H M V T S F N T G S A V I Y D L E T S<br>1681 GCTCATATGGTAACCTCTTTCAACACTGGTAGTGCAGTAATTTATGATTTAGAAACATCA<br><br>581 Q S L V I L S S Q V D S G L Q S N N H I<br>1741 CAGTCATTGGTGATACTTTCATCACAGGTAGATTCTGGTTTACAATCTAATAATCATATC<br><br>601 N R V V S H P T L P V T I T A H E D R H<br>1801 AACAGAGTAGTAAGTCATCCCACACTTCCTGTTACAATAACTGCTCATGAAGATAGACAC<br><br>621 I K F F D N K T G K M I H S M V A H L D<br>1861 ATCAAATTTTTTGACAATAAAACGGGTAAAATGATCCATTCTATGGTAGCTCACTTGGAT<br><br>641 A V T S L A V D P N G I Y -<br>1921 GCTGTTACAAGTCTAGCAGTAGATCCTAATGGAATCTATTAA | |
| WBC011F05.bFSP_20 010510.abd | No Homology | 1 GGCCGCCGCCACTGTTGGGCTCTCCTTTCAGTCGTCATTTTTAAGGAAAGAAGGGTACTA<br>61 TGTTTATTGTTAAAATTTCACAGAAAATTTTTGTCTGATGAATTTATTAGTCTTCATTTT<br>121 GAGGGTAAAAGAATTTTAGAAAATGCAAATATTCTGTAAAAAATTTAGATTTTTGATATT<br>181 TTGGGAAAAGTTGATGAAAGCAACCTCAGATAAAAATTTTCTGAAAACCATCAATGTGGT<br>241 CACGATACAGACTTCAGTGTGCTGTTCCTGGTCGCGGGTGTAACGGAGGAGGGGTTCCAA<br>301 CGCAGTTTGTGCCGGGAAATCCGAGTGCGCGCCCAAAGGAGAGGTTGGCCGCGGTGGCGG<br>361 CAGCAGCACCTCCCTGCACCTCACTTCTTAAAGCCAAGATTAGTTTTATGCCCTCCTCCT<br>421 TTCTGGGGTATTTGACTTTCTAGTTCATGATAGAAAATCCTCTTTGCTTTGCCAGTCGAT<br>481 CCCACTTGGTTAGATGACTTGGTAAGGGGGATGGATGTCCAGGAGTAAATGAACCTTGAC<br>541 TGTGTCTTACACTTGCAATGATATTTATAATTTGGGTCATTAAAAACATTTTAATTAGTG<br>601 GCCAGAATATAAAACTTCCTTTTCTCTTGGGAAAAGTCTTGTAGCCACTGGGAGTTGGGC<br>661 AGGTAGTCACACGGAACTCAGCAGCAGTCACTAGATGTTCTTCCTTCTCACCAGTGCTTG | SEQ ID NO:41 |

| Gene Name | GenBank Homology | DNA SEQUENCE / DEDUCED AMINO ACID SEQUENCE | SEQUENCE IDENTIFIER: |
|---|---|---|---|
| | | 721 AGCTTTCACAGTGGACTTGATTGAGTTGTCAATTTAGTGGTAATAAATGAGCTGTACGTT<br>781 GGTCCC | |
| | | 1 GTGACTTGAGCACGGTTTAGTTTAGTCTTCACCTTGGCTGTCACCTTCCAGAGGGTGTGT<br>61 GCACAGACCCTGGGGCACACACAGCACTGAGCACCACGAGGATGCCCAAGACCGAGCAAC<br>121 AGCAAGGGCTGGTTTCCTCCCGGTGTTACAAACCACACAAGCAGACGTCTCTGAGCGTTT<br>181 CCAGTGTTTTATCCAAATAAAAACTCTACTAGGGCTCTTGCTGACTTCAGTGCCTTTTGT<br>241 AAATCATTTTTGATTTTCCTCAACTTGCTTTTTGGGAATAGGCAGTGATGCTAAGTGGAG<br>301 GTAAGTGAGATTCTTTCTCCTCGTTAAGTTTTGTTCTTAAAACTTCTACATTGAAACTTG<br>361 TATTTAAAATAGCTTATTTCTTTCACGTGATAACAAGACAGGTCAGGAATATGGAATCAA<br>421 GATTTAGATTTTAAAATTCAAAAGGTTGTTACACAGGGTGTTTCCACTTGTGTAGCAGTG<br>481 GAATCTAGATATAAGACAGAAAAACACATTCTCTTCTAAAGACCAACGGCATTTTTAAAA<br>541 GGCATCTTTGTTCTCAACAGAAATCGGAATACAGATGTTATATTCACAGTCATAACCTGT<br>601 TTCCTCTAAGATCTTGCTATACTGTCCTGTATATTGTTGTAAATATTCAGATGGAAATTA<br>661 AAATGGTGTTTTGCCCTAAAAAAAAAAAAAAAAAA | SEQ ID NO:42 |
| WBC012A03.bFSP_20<br>010810.abd | No Homology | 1 GGAAGCTTTGAACTGTGCTGACTCTTCTGTTTGGGGAAGGAGGTTGTTTTCTGTGTGCCT<br>61 CAGAATATGACGGCTGCCAAACCATTACCTCCACCTAAAGCAGTGTAACACCAGAGCGTT<br>121 CAGTCCCAGATCAGATCCTTTTAGGACAGAAGTGTGTTTCCTCATTTTGGAGTGGAGCTG<br>181 TGAGTGGATGCATGCTCTGTCAGCTTGCATTTGTCCACTCTCTGTGCAGGCTCCGTGAGA<br>241 GTCTCAGAGCTGGGCCGGGCCACATGCATCAGGTCCTTCTCTCCTCTGGGTGTCCTCGGG<br>301 GGCACCTTGGGCCTGAGTCTCCCTCCAAGAGGCAGAAAGTGAGTCTGGTGGACTAGAGAT<br>361 CTCACCCATTCTGTGGGCTGGGGTTCCAGGCAGCCCAGCTGTCACCTGGGAACATCTGGA<br>421 GGAGGGTTGGCCCTCAGCACTCAGCGGGTGCCCGTGGTGTGGAGGATCCACAGTCAGCCC<br>481 ATGTGAATGAGCTACAGGAAGTGGTAATCACGTGTAGAAAAAATAAAAGCCTGGGAGGCT<br>541 GCAAACAGAACAGAATTCTTGTTTCATGAGGCACAGGAGCATTGCTACCACCAGTCTCAG<br>601 AGCACTGTGAAGGGACACACAGATCCCAGAATAGTCAGGAAAAGGTTTCACTATAACCAA<br>661 GAAGTCATGTTAGTGATGTGTTAGTCACAGACAGCACCCACAAACCTGGGCACTTGGCAG<br>721 ACGCGTACCAGCTTTGCGTACGTCCTGGGCAGAGAGCTGGTTCCACACCATTTGCATGGT<br>781 CTGCAGGAGCTTCTGCTACTGAATCCATGTTGGGTGGTTGATGGGGAGCAGCCCCTGGGA<br>841 TGGGCCTGCTGGGATTGTTCATCCTATCAGTGTATTTGGTGTCCTCCTCTTCTCTGTT | SEQ ID NO:43 |
| | | 1 TGCATGCGCAGAGCCCCTCTCAATGGGAGGAGAGGTGAGTAGGAGGAGGAAGGTTAGCAG<br>61 TGCCCTGAGTGATGTCCAGTTCCATTAGACACTCTGTCCCCATCTTCCCAACAGTCTATG<br>121 TGGAAGGAAAGGAAAGAATTTACTCTGTTAGGAATCTTCTCGAAGAAAATTTGAAACCTG<br>181 TGAGTCACAGTCAAAGAAACTTATCCACGAAACATAATTCATGTTTTCATGAGTCCTATA<br>241 GATGTTTGAGTCAGAAGAAGAAGGAGGGAGGTGACTGTGAGAAATCGCCCCTTTTTATTC<br>301 AACATCTGGGGATTGCAAATGCAGAAGGAAATCTATTTGGTTAGGCCTAGGCCATGCCTT<br>361 GGCTGAGTATAAAATAGCTTAGTCGTGAAGGCTCAGATAGTCTGTACGTGAACTGCTCCA<br>421 AGCGGGGTTACTGGCACATGTACAGAGGCATTCAGCGTGGACTGTGCCTGAGACCGACAG<br>481 GGCGGAGGATTCGATCCCAGTGAGAGCTGAGTCATTTGTTACAGGTATGAACGCCCAAGG<br>541 AAATTACCTTTTAGCAAAAGAAAGTTAAATGATTTAACAGTCCCCATATGTGTTAATGTC | SEQ ID NO:44 |

| Gene Name | GenBank Homology | DNA SEQUENCE / DEDUCED AMINO ACID SEQUENCE | SEQUENCE IDENTIFIER: |
|---|---|---|---|
| | | 601 TCACATGTATTTGTATCATAACGTGTAACATAATTGTGGAACAAAAATATCTACAATAAA<br>661 TCTTTTAGTATTTGTGAAAAAAAAAAAAAAAAAA | |
| WBC012B04.bFSP_20 010810.abd | Homo sapiens EGF-like-domain, multiple 5 (EGFL5) | 1 ATGGCGGCAGAGACCTCGCAAGGCTGCGACCAGGCTCTCTGGAGCAGCAGAGGGCATGCC<br>61 AAGCTCTACATCGTCGCTGCCGCTGCCCTCTCCCATCGCGGGGTTCCCGCGCGGTCCCCG<br>121 CCCGAGCTGGGCCCGGTGGAGCAGATGACTAGGCCCGGCGTCTGCCGCGCGGGGCGCATC<br>181 CTTTGTCTGCTCCGCGGCGCCCGGTGCCCGAGGCCCGCGCCCCACTCTTTGGGCAGACAA<br>241 AGCCGGGGCCGCCGCGAGCCCGGCGGGCTAGAGCTGGGCTCGCACCACCCTTCTCCCGCC<br>301 TCACGGACCGCCCCCTCTCCTGGACGTCCTCTGATTGGCCTACGGGGCTACAAAGAGGCC<br>361 GGGAAGCGGGCGGATCACGTGGGCGACTGTGGCTTCCCATTGGGCGACGGCTCCCACGGC<br>421 CCAGGCCCCGAGGACCGGGCCCCCGCGCGCCACCGTCCACCGACCCCTGGCTGCGACTTC<br>481 TCCAGCCCAGTCCCCGGAGACCACCCCTCTTTGGGCGACTGCTGGACCCTCTTCCACCAC<br>541 CTTTCAGGCGCCGCTCGGCCCCTCGCCGACCACCCCTCCGGCGGCGGAACGCACTTCGAC<br>601 CACCTCTCAGGCGCCGACCAGACCCGCGCCGACCACCCTTTCGACGACCACTGGCCCGGC<br>661 GCCGACCACCCCTGTAGCGACCACCGTACCGGCGCCCACGACTCCCCGGACCCCGACCCC<br>721 CGATCTCCCCAGCAGCAGCAACAGCAGCGTCCTCCCCACCCCACCTGCCACCGAGGCCCC<br>781 CTCTTCGCCTCCTCCAGAGACCAGAATGGGCGCAAACTTTTGGGTTGGGTTCGCGCACCC<br>841 AGCCTCTCTGCCTGGGTTATTTGGGGAAGTTTGAAAGCCCGCATAGTACCTCCTTCAACC<br>901 ACTGGGTCAAAAGCTGAGGAGTATGTATGTAACTGCTCTGTGGTTGGAAGCCTGAATGTG<br>961 AATCGCTGCAACCAGACCACAGGGCAGTGTGAGTGTCGGCCAGGTTATCAGGGGCTTCAC<br>1021 TGTGAAACCTGCAAAGAGGGCTTTTACCTAAATTACACTTCTGGGCTCTGTCAGCCATGT<br>1081 GACTGTAGTCCACATGGAGCTCTCAGCATACCGTGCAACAGTTCTGGGAAATGCCAGTGC<br>1141 AAAGTGGGTGTCATTGGCTCTATATGTGACCGATGCCAAGATGGATATTATGGCTTTAGT<br>1201 AAGAATGGCTGCTTGCCCTGCCAATGCAATAATCGGTCTGCCAGTTGCGATGCCCTCACA<br>1261 GGTGCTTGTTTAAACTGCCAGGAAAATAGCAAAGGAAATCACTGTGAAGAATGTAAAGAA<br>1321 GGATTTTATCAGAGTCCTGATGCCACTAAAGAATGTCTTCGCTGCCCTTGTTCAGCAGTG<br>1381 ACATCTACAGGCAGCTGCTCTATAAAATCGAGTGAATTGGAACCTGAATGTGACCAGTGT<br>1441 AAAGATGGTTACATAGGCCCGAACTGCAATAAATGTGAAAATGGCTATTACAATTTTGAC<br>1501 AGCATCTGTAGAAAGTGCCAATGTCACGGCCATGTGGACCCAGTTAAAACTCCAAAGATT<br>1561 TGTAAGCCCGAGAGTGGTGAGTGCATCAACTGCCTCCATAACACCACTGGGTTTTGGTGT<br>1621 GAGAACTGCCTAGAAGGTTATGTTCACGACCTCGAGGGAAATTGCATCAAGAAAGAAGTT<br>1681 ATTCTTCCAACACCTGAAGGTTCTACCATTTTGGTTTCCAATGCCTCTTTGACCACATCA<br>1741 GTGCCCACCCCTGTTATAAATAGTACTTTTACCCCTACAACCCTGCAGACTATCTTTTCA<br>1801 GTAAGCACTTCTGAAAACAGCACTTCAGCTTTAGCTGATGTATCATGGACCCAATTTAAC<br>1861 ATCATCATTTTGACAGTCATCATCATTGTTGTGGTGCTGCTAATGGGATTTGTGGGGGCT<br>1921 GTATATATGTACCGCGAGTACCAAAACCGGAAACTCAATGCCCCCTTTTGGACCATCGAG<br>1981 CTGAAAGAAGACAATATCAGTTTCAGCAGCTACCATGACAGCATTCCCAATGCAGATGTT<br>2041 TCGGGATTGTTGGAAGATGATGGCAATGAAGTGGCTCCCAATGGGCAGCTGACCCTGACG<br>2101 ACGCCCATACATAACTACAAAGCCTAAGGAGCTAGAACTGTTCTGAATTGTTAAACCACA<br>2161 GTGCTTGCTAAGACAGAGTCAGCCCCTGGGCCAGACAAAGCCTGGCTAGAGTTTGCTGAG<br>2221 AAAGCAAAGGCAAATAGTGCATCTGAAATTTTCAGGTACAAATTTGTAATGCGCTTAGCC | SEQ ID NO:45 |

| Gene Name | GenBank Homology | DNA SEQUENCE / DEDUCED AMINO ACID SEQUENCE | SEQUENCE IDENTIFIER: |
|---|---|---|---|
| | | 2281 TATCTATTGCTCTTAGTTTTCCCATGAAGATCTGAAGCGTTCACTGTCATTAGGACTTGA | |
| | | 2341 AGTAAAGTATATTTTTGTACCAAACCACATGGGAGTATGGAAAGGCTGAACCCCATAGTG | |
| | | 2401 CAGCCCAAAACCACTTTGACCTCCAGATAGACTCCTGGGGAAGCATTCACCAAGCCAGTG | |
| | | 2461 GTAACAAGATGATGAATGTGGAGGGGGAAAAGACTGCTGGAAGACTTCATCTCCATTCCT | |
| | | 2521 GAAACATTTTTTTAAAACAGTGTCAGGGATTATATTCGTTTTACTATACAAAAGGACAT | |
| | | 2581 CATTGAGGAAAAGCTTGTTTCTAGGCTGTATCACAGTAAATAATCTTGATTGTTTCTAGA | |
| | | 2641 ACAGGGGTAGAAAAGTCCCAAGATCTTCAGAAGGGCTTGGTTTTTTCCTCCCAGGATTCT | |
| | | 2701 TGCTCAGTGATGAGTTGAAAGCACAGGATAAGCTTCTAAGGGAGGAAGGCAGCATTGGGG | |
| | | 2761 AGCAGGAGGCTGATGGTCCTCCTCAGGGTCTCAGTGTAAATGATAAATTGTCTAAAAATA | |
| | | 2821 AGGAATATTCCTGCCTCTAGAGAAATAAGGTGTACATAGTTAATGTAGCATATCTGGTCG | |
| | | 2881 ACGTTGTATTTGTATCTATTTGTAAAAAAAAAATCATGTCATATTTTATCATCTGGAATT | |
| | | 2941 TATTTGTACAGCAGTTAATGGTGTTGTAAAAAGAAAAATATGGGGATTGCTTAAAATACT | |
| | | 3001 GTAAAATAGATGGCAATATTGCTAGAGCTGGGACTCTGGTGAGCATCCGTCATTTTTAGT | |
| | | 3061 CCTTCTTGAGGACATTGCTGAAATTTATAAGAGGTCGAATGTTTCTTTCTTTCCTTTCTC | |
| | | 3121 AGTCTAAAAGTAGAGTTATATGCTCTTTGACTTACTATGATTCAAGCAATAGATTTTGAA | |
| | | 3181 GTCAGTAATCGTTAAGCTGGGACATAGGATGCATTCTCCAAAAACATTAATCTAATTAGA | |
| | | 3241 CATTAGCCACCCTATATATTTTACATAGATTAATATAAAACATATCCTCATTAAAAAATG | |
| | | 3301 TTTTTCTTGAATGTAAAAGATAGTCTTCCAAAGGATAGAAATATTTTAGTTCCAAGAGAA | |
| | | 3361 AAGCCATCATATTACTTGGATCTGTAGTTTTCATGCCTATGAAATATTTATAATCCTCC | |
| | | 3421 CTCACCCCAAATAAAAAATCTCCTACATGTGAACATAGCCTGTCACTCATTTGCCATGAG | |
| | | 3481 GTATGTGCCATCTCCCTGCAGTGCTATAGTTCTCCCATTGTCTTCCTGTCTGGTTAATGA | |
| | | 3541 GCAGCTTTGTAGATGGGTGTGTCTAGTTAGTTCTTGCTTTTTGATGGAGTTTTTTCCTAG | |
| | | 3601 AGGCCAACTACTGTTGCTCTCGTTTATTATGATATTGACATTTTGGGTTAAGCAAGGTGG | |
| | | 3661 GTGGGAAAGGTGGTCAGATGTTAAATATAACTTATTAGGAGGAACAGTGAACTGTGAAGT | |
| | | 3721 GGTTAGAAAAAAAATAAAAACTTCTTATTAGATTAGACCTAGGAAATTCAAGCTGACCCC | |
| | | 3781 TTTGCCTAACTTCCCTAGAGAAAAACCTAGAACTTGGTCATCCCTTTATAGGAGACCCCT | |
| | | 3841 AAGTTGTTCTCAGGGTTTGGAGATGATGTTCTGGAGGGAGTACAAGCAAATAATCTACTT | |
| | | 3901 CTGGTAATCTGAGGGTGCTTTGCGTATTTGGCCATTGCTCAGAAGAATAGAGGTTACATT | |
| | | 3961 ATATTACATTATATGAAAATTATTTAGTTTTTCTATAGCCCTTTGTAGTTTGCAAAGCAC | |
| | | 4021 TTTTCCATATGTGTGTCCTCTCTTCCACACCATAGCCCCCTGAGGAAGTAATTGTTGTCC | |
| | | 4081 CCATTTTATAGATGAAAAGGCTGAGGCACAGAGGAGCTAAATATCTTGTTCAAGGCTATA | |
| | | 4141 CAATGTGTGTGATTATTAGGCTTGGAATCAGGGCCTCTAACTCTAAAGCACATGTTTTTT | |
| | | 4201 CAACTATATGCAGAAATTGCCTAGCCATGGACACAAGAAAGCTGGGAGGAAGAGTTCTAC | |
| | | 4261 TTGTGGATGTTTTTAATTTTTTTTAAATATCAGGAGCAATCCAGGGCCATACAGCTATGA | |
| | | 4321 AGCACTTAGTGAAAAGAACACAAGAAGATTTACTAACTGAAGTTAGTATTAGTGATTAGA | |
| | | 4381 AAACAAAACTGCCAGTTTGTGCTTCATTAAGGTGAACTCATCTCTCCAAGCAGGAAGGGA | |
| | | 4441 AAATTTGCTTTCCCTAGCAGTTGCTGCATATGTGTGTACAGACTGCACAATTCTAAGAAA | |
| | | 4501 TTGTGCAAAATGGCATACCTGTCTTTCTCCCAGATACACCGCCCCCTGCCCCCCCCAAAA | |
| | | 4561 AAGGTCAGGATTAAAGGTGGTGAGAAGTGAACATTTATTAAACAGAAGTTAAACCAATAG | |
| | | 4621 AGAGAAAGGAGGTTTGTTGGAGTCTAAGAGGATTTTGACCATGTAGAAATTCCTTGAAAC | |
| | | 4681 AGACCTTAAAAGGTATTGAAAAATTGAATCACAAAGAAAGTTTACCTTTAAATACTTGTT | |
| | | 4741 AATGGCTCCTAGATCATGGTTATAATTTTTCTCAATGGAAAAAAAGTCTTAAAATTTGT | |
| | | 4801 TTTAAATAAGATCCTCTAATGTCTCGAGCTTTTTGATTCTGGAAATAATTTGCTTTAAAA | |

| Gene Name | GenBank Homology | DNA SEQUENCE / DEDUCED AMINO ACID SEQUENCE | SEQUENCE IDENTIFIER: |
|---|---|---|---|
| | | 4861 TATAAATCAATCAGAAAGTGATCTACCACTGAAATCATTCTAGGAAACTAGCATGGGATG<br>4921 CACTGCACAATGTTCTTTCCTCTAAAGGCATGGGCCCCATAAACTGTGGCATCTGGCAGC<br>4981 AGGATGCCATCAGTCCTTTAGAAAGCCTAATTTTGGGCCGGGTGCGGTGGCTCACGCCTG<br>5041 TAATCCCAGCACTTTGGGAGGCCGAGGCGGGTGGATCACAAGGTCAGGAGTTCGAGACCA<br>5101 GCCTGGTCAACATGGCGAAATCCCGTCTCTACTAAAAACACAAAAATTAGCCGGGTGTAC<br>5161 GTGCCTGTAGTCTCAGCTACTTGGGAGGCTGAGGCAGGAGAATCACTTGAACCTGGGAGG<br>5221 CAGAGGTTACAGTGAGCCGAGATGGCGCCATTTCACTCCAGCCTGGGCGACAGAGCAAGA<br>5281 CTCTGTCTCAAAAAAAAGGAAGAAAAAGAAAGCCTAATATTGGGAGAATGTTTGTGTAAG<br>5341 GATTGGAAATCTGTTTATTTTTAAATAAGATAGCACTGATGTGAATCTTATTTGGAAATT<br>5401 ACAGATACTGCCATTAAGCGATGCTTTTATCTTCATTAATTCATTTTGGTAAGCGCCTAT<br>5461 GCATTTCTGATGGTTAGGTGCCCTAGGTAGTGTTAAGTATGTCCGTGTCCTCCCTTACTC<br>5521 CCCTCTCGCTCCCTCTACCCTCTATCCCCTAGGGTTCTTTTATTAATGGAATTTCTCCAT<br>5581 TTATAGAGAAAAAATGACATTTGAAAACTAAAGCTCATTTATATTAATACTAAGGTCCTG<br>5641 TGGGCCTAGTGCGTTACTCTTCCCAGTGATACCTAGATTTTAAGAGTAGAGAACAGAGGA<br>5701 AACATGGGATGAGAGGAGAGAGGTTTTTTAGACAACATTTGTAGACACCTCAAATTATAT<br>5761 CACTGTTCTCTAGCTCCAATATGCCTGGGAAAATTGGCCTTTAAAGCAATGCTCACTGAC<br>5821 CTGCTAGTTCGTTTTGGTCAGCTGAGTACCATCAGGATATTTAACCCTTTAAGTGCTGTT<br>5881 TTGGGAGTAGAAAACTAAAGCAACAATACTTCCTCTTGACAGCTTTGATTGGAATGGGGT<br>5941 TATTAGATCATTCACCTTGGTCCTACACTTTTTAGGATGCTTGGTGAACATCACACCACT<br>6001 TATCATGAACATTCCCTGGTTCCTATATTTTGGGCTATGTGGGTAGGAATTGTTACTTGT<br>6061 TACTGCAGCAGCAGCCCTAGAAAGTAAGCCCAGGGCTTCAGATCTAAGTTAGTCCAAAAG<br>6121 CTAAATGGTTTAAAGTTAAGTTTTAATACTAGGCACAAGCACTCTGTAATACGTTAAATT<br>6181 TCAGCCCAAGCAATTAGGAAATGTTTCTGAAACATTAGACTTATATTTACGTCACTAAAA<br>6241 TTCTAACACAAATTTTAAAACCGTGTCTCATACATATGCTGTACTAGGCTTCCTGATGCA<br>6301 TTTCTAAATTTGTGTATGATTTGAATATATGAAAGAATTTATACAAGAGTGTTATTTAAA<br>6361 ATTATTAAAAATAAATGTATATAATTTGTACCTATTGTAGATTTGTTGTTTACTTTTTTG<br>6421 TGTGGTCAGAGTTGTTTCATGTCTTCAAATACATGCCAAGGATCATTTACTTTCAGCCCA<br>6481 CTTCATAAAAATCTTTGAACTCTT | |
| | | 1 M A A E T S Q G C D Q A L W S S R G H A<br>1 ATGGCGGCAGAGACCTCGCAAGGCTGCGACCAGGCTCTCTGGAGCAGCAGAGGGCATGCC<br><br>21 K L Y I V A A A A L S H R G V P A R S P<br>61 AAGCTCTACATCGTCGCTGCCGCTGCCCTCTCCCATCGCGGGTTCCCGCGCGGTCCCCG<br><br>41 P E L G P V E Q M T R P G V C R A G R I<br>121 CCCGAGCTGGGCCCGGTGGAGCAGATGACTAGGCCCGGCGTCTGCCGCGCGGGCGCATC<br><br>61 L C L L R G A R C P R P A P H S L G R Q<br>181 CTTTGTCTGCTCCGCGGCGCCCGGTGCCCGAGGCCCGCGCCCCACTCTTTGGGCAGACAA<br><br>81 S R G R R E P G G L E L G S H H P S P A<br>241 AGCCGGGGCCGCCGCGAGCCCGGCGGGCTAGAGCTGGGCTCGCACCACCCTTCTCCCGCC | SEQ ID NO:46 |

| Gene Name | GenBank Homology | DNA SEQUENCE / DEDUCED AMINO ACID SEQUENCE | SEQUENCE IDENTIFIER: |
|---|---|---|---|
| | | 101 S R T A P S P G R P L I G L R G Y K E A<br>301 TCACGGACCGCCCCCTCTCCTGGACGTCCTCTGATTGGCCTACGGGGCTACAAAGAGGCC<br><br>121 G K R A D H V G D C G F P L G D G S H G<br>361 GGGAAGCGGGCGGATCACGTGGGCGACTGTGGCTTCCCATTGGGCGACGGCTCCCACGGC<br><br>141 P G P E D R A P A R H R P P T P G C D F<br>421 CCAGGCCCCGAGGACCGGGCCCCCGCGCGCCACCGTCCACCGACCCCTGGCTGCGACTTC<br><br>161 S S P V P G D H P S L G D C W T L F H H<br>481 TCCAGCCCAGTCCCCGGAGACCACCCCTCTTTGGGCGACTGCTGGACCCTCTTCCACCAC<br><br>181 L S G A A R P L A D H P S G G G T H F D<br>541 CTTTCAGGCGCCGCTCGGCCCCTCGCCGACCACCCCTCCGGCGGCGGAACGCACTTCGAC<br><br>201 H L S G A D Q T R A D H P F D D H W P G<br>601 CACCTCTCAGGCGCCGACCAGACCCGCGCCGACCACCCTTTCGACGACCACTGGCCCGGC<br><br>221 A D H P C S D H R T G A H D S P D P D P<br>661 GCCGACCACCCCTGTAGCGACCACCGTACCGGCGCCCACGACTCCCCGGACCCCGACCCC<br><br>241 R S P Q Q Q Q Q Q R P P H P T C H R G P<br>721 CGATCTCCCCAGCAGCAGCAACAGCAGCGTCCTCCCCACCCCACCTGCCACCGAGGCCCC<br><br>261 L F A S S R D Q N G R K L L G W V R A P<br>781 CTCTTCGCCTCCTCCAGAGACCAGAATGGGCGCAAACTTTTGGGTTGGGTTCGCGCACCC<br><br>281 S L S A W V I W G S L K A R I V P P S T<br>841 AGCCTCTCTGCCTGGGTTATTTGGGGAAGTTTGAAAGCCCGCATAGTACCTCCTTCAACC<br><br>301 T G S K A E E Y V C N C S V V G S L N V<br>901 ACTGGGTCAAAAGCTGAGGAGTATGTATGTAACTGCTCTGTGGTTGGAAGCCTGAATGTG<br><br>321 N R C N Q T T G Q C E C R P G Y Q G L H<br>961 AATCGCTGCAACCAGACCACAGGGCAGTGTGAGTGTCGGCCAGGTTATCAGGGGCTTCAC<br><br>341 C E T C K E G F Y L N Y T S G L C Q P C<br>1021 TGTGAAACCTGCAAAGAGGGCTTTTACCTAAATTACACTTCTGGGCTCTGTCAGCCATGT<br><br>361 D C S P H G A L S I P C N S S G K C Q C<br>1081 GACTGTAGTCCACATGGAGCTCTCAGCATACCGTGCAACAGTTCTGGGAAATGCCAGTGC | |

| Gene Name | GenBank Homology | DNA SEQUENCE / DEDUCED AMINO ACID SEQUENCE | SEQUENCE IDENTIFIER: |
|---|---|---|---|
| | | 381 K V G V I G S I C D R C Q D G Y Y G F S<br>1141 AAAGTGGGTGTCATTGGCTCTATATGTGACCGATGCCAAGATGGATATTATGGCTTTAGT<br><br>401 K N G C L P C Q C N N R S A S C D A L T<br>1201 AAGAATGGCTGCTTGCCCTGCCAATGCAATAATCGGTCTGCCAGTTGCGATGCCCTCACA<br><br>421 G A C L N C Q E N S K G N H C E E C K E<br>1261 GGTGCTTGTTTAAACTGCCAGGAAAATAGCAAAGGAAATCACTGTGAAGAATGTAAAGAA<br><br>441 G F Y Q S P D A T K E C L R C P C S A V<br>1321 GGATTTTATCAGAGTCCTGATGCCACTAAAGAATGTCTTCGCTGCCCTTGTTCAGCAGTG<br><br>461 T S T G S C S I K S S E L E P E C D Q C<br>1381 ACATCTACAGGCAGCTGCTCTATAAAATCGAGTGAATTGGAACCTGAATGTGACCAGTGT<br><br>481 K D G Y I G P N C N K C E N G Y Y N F D<br>1441 AAAGATGGTTACATAGGCCCGAACTGCAATAAATGTGAAAATGGCTATTACAATTTTGAC<br><br>501 S I C R K C Q C H G H V D P V K T P K I<br>1501 AGCATCTGTAGAAAGTGCCAATGTCACGGCCATGTGGACCCAGTTAAAACTCCAAAGATT<br><br>521 C K P E S G E C I N C L H N T T G F W C<br>1561 TGTAAGCCCGAGAGTGGTGAGTGCATCAACTGCCTCCATAACACCACTGGGTTTTGGTGT<br><br>541 E N C L E G Y V H D L E G N C I K K E V<br>1621 GAGAACTGCCTAGAAGGTTATGTTCACGACCTCGAGGGAAATTGCATCAAGAAAGAAGTT<br><br>561 I L P T P E G S T I L V S N A S L T T S<br>1681 ATTCTTCCAACACCTGAAGGTTCTACCATTTTGGTTTCCAATGCCTCTTTGACCACATCA<br><br>581 V P T P V I N S T F T P T T L Q T I F S<br>1741 GTGCCCACCCCTGTTATAAATAGTACTTTTACCCCTACAACCCTGCAGACTATCTTTTCA<br><br>601 V S T S E N S T S A L A D V S W T Q F N<br>1801 GTAAGCACTTCTGAAAACAGCACTTCAGCTTTAGCTGATGTATCATGGACCCAATTTAAC<br><br>621 I I I L T V I I I V V V L L M G F V G A<br>1861 ATCATCATTTTGACAGTCATCATCATTGTTGTGGTGCTGCTAATGGGATTTGTGGGGGCT<br><br>641 V Y M Y R E Y Q N R K L N A P F W T I E<br>1921 GTATATATGTACCGCGAGTACCAAAACCGGAAACTCAATGCCCCCTTTTGGACCATCGAG<br><br>661 L K E D N I S F S S Y H D S I P N A D V | |

| Gene Name | GenBank Homology | DNA SEQUENCE / DEDUCED AMINO ACID SEQUENCE | SEQUENCE IDENTIFIER: |
|---|---|---|---|
| | | 1981 CTGAAAGAAGACAATATCAGTTTCAGCAGCTACCATGACAGCATTCCCAATGCAGATGTT<br><br>681 S G L L E D D G N E V A P N G Q L T L T<br>2041 TCGGGATTGTTGGAAGATGATGGCAATGAAGTGGCTCCCAATGGGCAGCTGACCCTGACG<br><br>701 T P I H N Y K A -<br>2101 ACGCCCATACATAACTACAAAGCCTAA | |
| WBC012C10.bFSP_20 010810.abd | H.sapiens mRNA for inwardly rectifing potassium channel Kir4.2. | 1 GCAGTAGCAGAATCCCATGGTAGCCAGGTGGGTGAAGGGGAGCGAGGACGTCCCACCTGC<br>61 CTTGAAGAAGACGCCTTCTGACCTGCTGAGTGAGTGACCAGGTGGTCCCAAGAGCCTGAC<br>121 GATGGACGCCATGCACATCAACATGTCCAGTGCTCCTCTGGTGAAGCACACTGCTGGAGC<br>181 TGGACTCAAGACCAACAGGCCCCGAGTCATGTCCAAGAGTGGACACAGCAACGTGAGAAT<br>241 CGACAAGGTGGATGGCATCTACTTACTCTACCTTCAAGACTTGTGGACGACCGTGATCGA<br>301 CATGAAGTGGAGGTACAAGCTCACCCTCTTTGCAGCCACCTTTGTGATGACCTGGTTCCT<br>361 GTTCGGAGTAATCTATTACGCCATTGCATTTATTCACGGGGACTTAGAGCCCAGTGAGCG<br>421 TATTTCAAATCACACCCCCTGCATCATGAAAGTGGACTCTCTCACAGGGGCATTTCTCTT<br>481 TTCCCTGGAATCCCAGACAACCATTGGCTATGGAGTTCGCTCCATCACGGAGGAATGTCC<br>541 TCATGCCATTTTCCTGTTGGTCGCTCAGTTGGTCATCACGACCTTGATTGAGATCTTCAT<br>601 CACGGGCACCTTCCTGGCAAAAATCGCCAGACCCAAAAAGCGGGCAGAGACCATCAAGTT<br>661 CAGCCACTGTGCTGTCATCACCAAGCAGAATGGGAAGCTGTGCTTGGTGATTCAGGTAGC<br>721 CAATATGAGGAAGAGCCTCTTGATTCAGTGCCAGCTCTCTGGCAAGCTCCTGCAGACCCA<br>781 CGTCACCAAGGAGGGGGAGCGGATTCTCCTCAACCAAGCCACTGCCAAATTCCACGTGGA<br>841 CTCCTCCTCTGAGAGCCCCTTCCTCATTCTGCCCATGACATTCTACCATGTGCTGGATGA<br>901 GACGAGCCCCCTGAGAGACCTCACACCCCAAAACCTAAAGGAGAAGGAGTTTGAGCTTGT<br>961 GGTCCTCCTCAATGCCACTGTGGAATCCACCAGCGCTGTCTGCCAGAGCCGAACATCTTA<br>1021 TATCCCAGAGGAAATCTACTGGGGTTTTGAGTTTGTGCCTGTGGTATCTCTCTCCAAAAA<br>1081 TGGAAAATATGTGGCTGATTTCAGTCAGTTCGAACAGATCCGGAAGAGCCCGGACTGCAC<br>1141 CTTTTATTGCGCAGATGCTGAGAAGCAGAAACTTGAGGAGAAGTACAGGCAGGAGGACCA<br>1201 GAGGGAAAGAGAACTGAGGACCCTTTTGTTACAGCAGAGCAACGTCTGACGGCAGTGGTG<br>1261 TCACTGCTGTTTAACTCTGCGAGCTGTTTCCACATCTGAGCTCGCTCCAAAAGCAAAGGT<br>1321 CGCTGTGAAAGCAAAACTGTGTGGATGCACTCTCAAAAAACCTCACAGGCTACAAACTTG<br>1381 ATCTTTTCCTTTGATCTGGTGGCTAAACAAGCATTTCCATGTTTGAGAGGCTTCCTTTTA<br>1441 AATGCTTTGTCTGAAGCAAACTCTCAAAATTCAGATTTTCTAAAGTGGGGCTACATTTCG<br>1501 AAGAACTTGGTGTAGAGCAATTGAAAAAATGTCCTGCTGGATGGACAGACATTGATGCTG<br>1561 ACATTGAAAGGAACGGCATTTCTATACAAGATTATTAGCTGTAATACAAGATATTTATTT<br>1621 AACCAATGACCTTATGGCTGAGAGTTGAATTGTGGTTCAGTATTCATTGATCTCACTGCT<br>1681 TTAAAACATGCTCCTTTTGTTCAAGCAG | SEQ ID NO:47 |
| | | 1 M D A M H I N M S S A P L V K H T A G A<br>1 ATGGACGCCATGCACATCAACATGTCCAGTGCTCCTCTGGTGAAGCACACTGCTGGAGCT<br><br>21 G L K T N R P R V M S K S G H S N V R I | SEQ ID NO:48 |

| Gene Name | GenBank Homology | DNA SEQUENCE / DEDUCED AMINO ACID SEQUENCE | SEQUENCE IDENTIFIER: |
|---|---|---|---|
| | | 61 GGACTCAAGACCAACAGGCCCCGAGTCATGTCCAAGAGTGGACACAGCAACGTGAGAATC<br><br>41 D K V D G I Y L L Y L Q D L W T T V I D<br>121 GACAAGGTGGATGGCATCTACTTACTCTACCTTCAAGACTTGTGGACGACCGTGATCGAC<br><br>61 M K W R Y K L T L F A A T F V M T W F L<br>181 ATGAAGTGGAGGTACAAGCTCACCCTCTTTGCAGCCACCTTTGTGATGACCTGGTTCCTG<br><br>81 F G V I Y Y A I A F I H G D L E P S E R<br>241 TTCGGAGTAATCTATTACGCCATTGCATTTATTCACGGGGACTTAGAGCCCAGTGAGCGT<br><br>101 I S N H T P C I M K V D S L T G A F L F<br>301 ATTTCAAATCACACCCCCTGCATCATGAAAGTGGACTCTCTCACAGGGGCATTTCTCTTT<br><br>121 S L E S Q T T I G Y G V R S I T E E C P<br>361 TCCCTGGAATCCCAGACAACCATTGGCTATGGAGTTCGCTCCATCACGGAGGAATGTCCT<br><br>141 H A I F L L V A Q L V I T T L I E I F I<br>421 CATGCCATTTTCCTGTTGGTCGCTCAGTTGGTCATCACGACCTTGATTGAGATCTTCATC<br><br>161 T G T F L A K I A R P K K R A E T I K F<br>481 ACGGGCACCTTCCTGGCAAAAATCGCCAGACCCAAAAAGCGGGCAGAGACCATCAAGTTC<br><br>181 S H C A V I T K Q N G K L C L V I Q V A<br>541 AGCCACTGTGCTGTCATCACCAAGCAGAATGGGAAGCTGTGCTTGGTGATTCAGGTAGCC<br><br>201 N M R K S L L I Q C Q L S G K L L Q T H<br>601 AATATGAGGAAGAGCCTCTTGATTCAGTGCCAGCTCTCTGGCAAGCTCCTGCAGACCCAC<br><br>221 V T K E G E R I L L N Q A T A K F H V D<br>661 GTCACCAAGGAGGGGGAGCGGATTCTCCTCAACCAAGCCACTGCCAAATTCCACGTGGAC<br><br>241 S S S E S P F L I L P M T F Y H V L D E<br>721 TCCTCCTCTGAGAGCCCCTTCCTCATTCTGCCCATGACATTCTACCATGTGCTGGATGAG<br><br>261 T S P L R D L T P Q N L K E K E F E L V<br>781 ACGAGCCCCCTGAGAGACCTCACACCCCAAAACCTAAAGGAGAAGGAGTTTGAGCTTGTG<br><br>281 V L L N A T V E S T S A V C Q S R T S Y<br>841 GTCCTCCTCAATGCCACTGTGGAATCCACCAGCGCTGTCTGCCAGAGCCGAACATCTTAT<br><br>301 I P E E I Y W G F E F V P V V S L S K N<br>901 ATCCCAGAGGAAATCTACTGGGGTTTTGAGTTTGTGCCTGTGGTATCTCTCTCCAAAAAT | |

| Gene Name | GenBank Homology | DNA SEQUENCE / DEDUCED AMINO ACID SEQUENCE | SEQUENCE IDENTIFIER: |
|---|---|---|---|
| | | 321 G K Y V A D F S Q F E Q I R K S P D C T<br>961 GGAAAATATGTGGCTGATTTCAGTCAGTTCGAACAGATCCGGAAGAGCCCGGACTGCACC<br><br>341 F Y C A D A E K Q K L E E K Y R Q E D Q<br>1021 TTTTATTGCGCAGATGCTGAGAAGCAGAAACTTGAGGAGAAGTACAGGCAGGAGGACCAG<br><br>361 R E R E L R T L L L Q Q S N V -<br>1081 AGGGAAAGAGAACTGAGGACCCTTTTGTTACAGCAGAGCAACGTCTGA | |
| WBC012E05.bFSP_20 010810.abd | No homology. | 1 GAAAACAGTGGCTGCAAAGCACCCCAAAAAGCCTTTCAAGTGCCTTCTGTTAATATTGAT<br>61 TTATACGGTTTAAATGTGCCTGATTCTTCCAGGAAAATCTCAATTATTGCACTCAGCTCC<br>121 ATTTCTACCTGTTCCATTACCCAAGATAATAAATTAGTTACTGCGTTGTAAAGACAGTGG<br>181 TCTCTTCTTTGTTCGTTAAAATAAGGTTGTGTTTCTCTGTTTACATTAATGAAGTCTGAA<br>241 TTTAAAATGGTGATAAAACCAAGATTTAGTACTTTCTAAACTCAAGGAAATTATTTCTAG<br>301 ACATGAGGTAATTCTTTTAAATTTAAACTGTTGTACAGATCCCTTAAATGTTCTTGTTAA<br>361 GTACCTACTATTCTGCTGAACTTTAAAAGTTAATTCCCACATTTACAGGAATCTATGATA<br>421 TTTAAACATTTTTTATTGCTCATTAATGTTTCTGAGTATAATATATATGCCTTATTTTTA<br>481 GACTTGTAAAATGTATGAAATCTCACTAGTGCACGGACATCTGTGAAGTTAGCAGTGAGT<br>541 TGGAATTTTAAATGACTATGTGAAGGATACGCAAAATATGTTAACTCTTGTTACTTTTGT<br>601 ATGTGATTGTATAAGCATGTGACTGTATTATAACATTGTTCACTTTGAAAACTAATATAG<br>661 ATATGCTTGATTCTGAACAATAAGTGTGACTTGTATTGAACAGTTTATAGACTGTGTTCC<br>721 TTATTTAACACAATTATGAATTCTTCCACTCGTTCATAATTTGTACATCTCACTGCTTAG<br>781 CTTAGATTCAAGTCTGTGTTTATGCATTTATGTACCTGCTGATCATCACTGTGTACTCAG<br>841 AATTACTGTAGGAGTATTCTGACTCTAACATGGACTTTTAAAAATCTTTCAATAACCTGC<br>901 CAGTGGGATATGCTCACTGATCTGTGCTTAAGTTGAAGATATAACTTGTCTTGAAGGATA<br>961 TTTAAATATTTAGAGATCATGCTTGCGTTACTAGGACGTTGTCTGTGATGGTGTCCACGT<br>1021 GTGGAGGAGCCGTGTGCTGGTGACAGCCAGCTTTGTCTGTCCTGCCTTCTCTTCAGGTGC<br>1081 AGCCTTACTGGTCCTCACCTTCATGACCGTCCTGACTGTATATTTTCAAGTCAGCCTTGC<br>1141 TGGATTTGGAGTCTTACTGACTTTGTGTTTTTGTAGTTGAAGTTATGGAAATGCTTTTTG<br>1201 ATTTGTATTTAGATATTGAAGTCATTCATCAGATAGCACATTTGTATGTCTGAGCCTCAT<br>1261 GTACCTATTTGAAGTCATATTTTTAAAAATATATTTACAGGTTTATTTGTGACTTTTTAT<br>1321 CATTTCTTCAAATATTTCATGTTTACATTAAAAAAATTCCCAGAGAACCAAAAAA | SEQ ID NO:49 |
| WBC012E07.bFSP_20 010810.abd | Pinin, desmosome associated protein (PNN) | 1 GGCTGTCAGTCCTTTCGCGCCTCGGCGGCGCGGCATAGCCCGGCTCGGCCTGTAAAGCAG<br>61 TCTCAAGCCTGCCGCAGGGAGAAGATGGCGGTCGCCGTGAGAACTTTGCAGGAACAGCTG<br>121 GAAAAGGCCAAAGAGAGTCTTAAGAACGTGGATGAGAACATTCGCAAGCTCACCGGGCGG<br>181 GATCCGAATGACGTGAGGCCCATCCAAGCCAGATTGCTGGCCCTTTCTGGTCCTGGTGGA<br>241 GGTAGAGGACGTGGTAGTTTATTACTGAGGCGTGGATTCTCAGATAGTGGAGGAGGACCC<br>301 CCAGCCAAACAGAGAGACCTTGAAGGGGCAGTCAGTAGGCTGGGCGGGGAGCGTCGGACC<br>361 AGAAGAGAATCACGCCAGGAAAGCGACCCGGAGGATGATGATGTTAAAAAGCCAGCATTG | SEQ ID NO:50 |

| Gene Name | GenBank Homology | DNA SEQUENCE / DEDUCED AMINO ACID SEQUENCE | SEQUENCE IDENTIFIER: |
|---|---|---|---|
| | | 421 CAGTCTTCAGTTGTAGCTACCTCCAAAGAGCGCACACGTAGAGACCTTATCCAGGATCAA<br>481 AATATGGATGAAAAGGGAAAGCAAAGGAACCGGCGAATATTTGGCTTGTTGATGGGTACC<br>541 CTTCAAAAATTTAAACAAGAATCCACTGTTGCTACTGAAAGGCAAAAGCGGCGCCAGGAA<br>601 ATTGAACAAAAACTTGAAGTTCAGGCAGAAGAAGAGAGAAAGCAGGTTGAAAATGAAAGG<br>661 AGAGAACTGTTTGAAGAGAGGCGTGCTAAACAGACAGAACTGCGGCTTTTGGAACAGAAA<br>721 GTTGAGCTTGCGCAGCTGCAAGAAGAATGGAATGAACATAATGCCAAAATAATTAAATAT<br>781 ATAAGAACTAAGACAAAGCCCCATTTGTTTTATATTCCTGGAAGAATGTGTCCAGCTACC<br>841 CAAAAACTAATAGAAGAGTCACAGAGAAAAATGAACGCTTTATTTGAAGGTAGACGCATC<br>901 GAATTTGCAGAACAAATAAATAAAATGGAGGCTAGGCCTAGAAGACAATCAATGAAGGAA<br>961 AAAGAGCATCAGGTGGTGCGTAATGAAGAACAGAAGGCGGAACAAGAAGAGGGTAAGGTG<br>1021 GCTCAGCGAGAGGAAGAGTTGGAGGAGACAGGTAATCAGCACAATGATGTAGAAATAGAG<br>1081 GAAGCAGGAGAGGAAGAGGAAAAGGAAATAGCGATTGTTCATAGTGATGCAGAGAAAGAA<br>1141 CAGGAGGAGGAAGAACAAAAACAGGAAATGGAGGTTAAGATGGAGGAGGAAACTGAGGTA<br>1201 AGGGAAAGTGAGAAGCAGCAGGATAGTCAGCCTGAAGAAGTTATGGATGTGCTAGAGATG<br>1261 GTTGAGAATGTCAAACATGTAATTGCTGACCAGGAGGTAATGGAAACTAATCGAGTTGAA<br>1321 AGTGTAGAACCTTCAGAAAATGAAGCTAGCAAAGAATTGGAACCAGAAATGGAATTTGAA<br>1381 ATTGAGCCAGATAAAGAATGTAAATCCCTTTCTCCTGGGAAAGAGAATGTCAGTGCTTTA<br>1441 GACATGGAAAAGGAGTCTGAGGAAAAAGAAGAAAAAGAATCTGAGCCCCAACCTGAGCCT<br>1501 GTGGCTCAACCTCAGCCTCAGTCTCAGCCCCAGCTTCAGCTTCAATCCCAGTCCCAACCA<br>1561 GTACTCCAGTCCCAGCCTCCCTCTCAGCCTGAGGATTTGTCATTAGCTGTTTTACAGCCA<br>1621 ACACCCCAAGTTACTCAGGAGCAAGGGCATTTACTACCTGAGAGGAAGGATTTTCCTGTA<br>1681 GAGTCTGTAAAACTCACTGAGGTACCAGTAGAGCCAGTCTTGACAGTACATCCAGAGAGC<br>1741 AAGAGCAAAACCAAAACTAGGAGCAGAAGTAGAGGTCGAGCTAGAAATAAAACAAGCAAG<br>1801 AGTAGAAGTCGAAGCAGTAGCAGTAGCAGTTCTAGTAGCAGTTCAACCAGTAGCAGCAGT<br>1861 GGAAGTAGTTCCAGCAGTGGAAGTAGTAGCAGTCGCAGTAGTTCCAGTAGCAGCTCCAGT<br>1921 ACAAGTGGCAGCAGCAGCAGAGATAGTAGCAGTAGCACTAGTAGTAGTAGTGAGAGTAGA<br>1981 AGTCGGAGTAGGGGCCGGGGACATAACAGAGATAGAAAGCACAGAAGGAGCGTGGATCGG<br>2041 AAGCGAAGGGATACTTCAGGACTAGAAAGAAGTCACAAATCTTCAAAAGGTGGTAGTAGT<br>2101 AGAGATACGAAAGGATCAAAGGATAAGAATTCCCGGTCCGACAGGAAAAGGTCTATATCC<br>2161 GAGAGTAGTCGATCAGGCAAAAGATCGTCGAGAAGTGAAAGAGACCGAAAATCAGACAGG<br>2221 AAAGACAAAAGGCGTTAATGGAAGAAGCCAGGCTTTCTTAGCCTATTCTTTGCAGCAGAA<br>2281 GATTTCTTGATGAGTAAAAGGATTACCTTTCCTTGTAAGGAGGATGCTGCCTTAAGAATT<br>2341 GCATGTTGTAAAAAATCTTTTTGGAAAATACAGACTGTTTGTTTACCAGACATTCTTGTA<br>2401 CTTTTTGCATAATTTTGTAAGAGTTATTTATCAAAATTATGTGAGGTTCCAAAATATGTA<br>2461 AAATAATAATAATAAAAAAAGATTAACATCCCTTGTCATCTTTTTTAAATATCCTATACT<br>2521 CTGCAGTAAGAATGTATATTTTAATAGGTAAATCTTTAAGTCTGTTCCCTTCTAATTCTG<br>2581 TATCATACATTGCTTTTGTAGAAATAAATGTGCATTTCTTTCATTAGTTTTGAGATGTCC<br>2641 TCGTTGACGCTTGTATAATAAATATCCTCTTGATACCATTTTCAGCTTTTATCACTAGAT<br>2701 ACTGAACGTGATTAGAATGTCTTTGAAAGTTTCCTCACTTTTATTTGCCTTAGGCAGTTA<br>2761 TTTTGAGTTGTCAAAATCAGATCTTTGCAGCTTTGAGGGGGAACATAATAGTTCTCCGTG<br>2821 AAATCATTTACTGTTTTTTCTAATCTCCCTTGTTATTTTAATCTAAGCATTTTCCCCTCC<br>2881 TCATCTTTAAACCACGTATTTGGTAGATAACTTAAAATAGTATAATTTGGTTGGCATTTT<br>2941 CTTCATGATTATGGAGCATCATTCTTTTGTCTCCATGGTTACTTGTGTGATACAGCATA | |

| Gene Name | GenBank Homology | DNA SEQUENCE / DEDUCED AMINO ACID SEQUENCE | SEQUENCE IDENTIFIER: |
|---|---|---|---|
| | | 3001 TATATCTGTTAAAGAAAATAATCACTTCTTTCTAGGGGAGGGAGGTAGAAAAGTATATTC<br>3061 TAAATTTGGTTTTTGAGTTTGTGGTCTTGTCTTAACTTTTGTGTTGGCTCTAACTCTGAA<br>3121 ACATGCCAATAATGTGTTTTCAAGAATTTTTGTTTAAGTATTGTATGAAAGTTTACAAAA<br>3181 TGAAGGAAGTTAATCTACACTTGAATCTGTGAGCAAGATAACACGCAAGTGTACCAAGTG<br>3241 ATTATTAACTTTGTTTTATAAATTTGTATGAATTTGGAGTATCTGTTGCCCATTACTATA<br>3301 CATGTGCAAATAAATGTGGCTTAGACTTGTGTGACTGCTTAAAAAAAAAAAAAAAA | |
| | | 1 M A V A V R T L Q E Q L E K A K E S L K<br>1 ATGGCGGTCGCCGTGAGAACTTTGCAGGAACAGCTGGAAAAGGCCAAAGAGAGTCTTAAG<br><br>21 N V D E N I R K L T G R D P N D V R P I<br>61 AACGTGGATGAGAACATTCGCAAGCTCACCGGGCGGGATCCGAATGACGTGAGGCCCATC<br><br>41 Q A R L L A L S G P G G G R G R G S L L<br>121 CAAGCCAGATTGCTGGCCCTTTCTGGTCCTGGTGGAGGTAGAGGACGTGGTAGTTTATTA<br><br>61 L R R G F S D S G G G P P A K Q R D L E<br>181 CTGAGGCGTGGATTCTCAGATAGTGGAGGAGGACCCCCAGCCAAACAGAGAGACCTTGAA<br><br>81 G A V S R L G G E R R T R R E S R Q E S<br>241 GGGGCAGTCAGTAGGCTGGGCGGGGAGCGTCGGACCAGAAGAGAATCACGCCAGGAAAGC<br><br>101 D P E D D D V K K P A L Q S S V V A T S<br>301 GACCCGGAGGATGATGATGTTAAAAAGCCAGCATTGCAGTCTTCAGTTGTAGCTACCTCC<br><br>121 K E R T R R D L I Q D Q N M D E K G K Q<br>361 AAAGAGCGCACACGTAGAGACCTTATCCAGGATCAAAATATGGATGAAAAGGGAAAGCAA<br><br>141 R N R R I F G L L M G T L Q K F K Q E S<br>421 AGGAACCGGCGAATATTTGGCTTGTTGATGGGTACCCTTCAAAAATTTAAACAAGAATCC<br><br>161 T V A T E R Q K R R Q E I E Q K L E V Q<br>481 ACTGTTGCTACTGAAAGGCAAAAGCGGCGCCAGGAAATTGAACAAAAACTTGAAGTTCAG<br><br>181 A E E E R K Q V E N E R R E L F E E R R<br>541 GCAGAAGAAGAGAGAAAGCAGGTTGAAAATGAAAGGAGAGAACTGTTTGAAGAGAGGCGT<br><br>201 A K Q T E L R L L E Q K V E L A Q L Q E<br>601 GCTAAACAGACAGAACTGCGGCTTTTGGAACAGAAAGTTGAGCTTGCGCAGCTGCAAGAA<br><br>221 E W N E H N A K I I K Y I R T K T K P H<br>661 GAATGGAATGAACATAATGCCAAAATAATTAAATATATAAGAACTAAGACAAAGCCCCAT | *SEQ ID NO:51* |

| Gene Name | GenBank Homology | DNA SEQUENCE / DEDUCED AMINO ACID SEQUENCE | SEQUENCE IDENTIFIER: |
|---|---|---|---|
| | | 241 L F Y I P G R M C P A T Q K L I E E S Q<br>721 TTGTTTTATATTCCTGGAAGAATGTGTCCAGCTACCCAAAAACTAATAGAAGAGTCACAG<br><br>261 R K M N A L F E G R R I E F A E Q I N K<br>781 AGAAAAATGAACGCTTTATTTGAAGGTAGACGCATCGAATTTGCAGAACAAATAAATAAA<br><br>281 M E A R P R R Q S M K E K E H Q V V R N<br>841 ATGGAGGCTAGGCCTAGAAGACAATCAATGAAGGAAAAAGAGCATCAGGTGGTGCGTAAT<br><br>301 E E Q K A E Q E E G K V A Q R E E E L E<br>901 GAAGAACAGAAGGCGGAACAAGAAGAGGGTAAGGTGGCTCAGCGAGAGGAAGAGTTGGAG<br><br>321 E T G N Q H N D V E I E E A G E E E E K<br>961 GAGACAGGTAATCAGCACAATGATGTAGAAATAGAGGAAGCAGGAGAGGAAGAGGAAAAG<br><br>341 E I A I V H S D A E K E Q E E E E Q K Q<br>1021 GAAATAGCGATTGTTCATAGTGATGCAGAGAAAGAACAGGAGGAGGAAGAACAAAAACAG<br><br>361 E M E V K M E E E T E V R E S E K Q Q D<br>1081 GAAATGGAGGTTAAGATGGAGGAGGAAACTGAGGTAAGGGAAAGTGAGAAGCAGCAGGAT<br><br>381 S Q P E E V M D V L E M V E N V K H V I<br>1141 AGTCAGCCTGAAGAAGTTATGGATGTGCTAGAGATGGTTGAGAATGTCAAACATGTAATT<br><br>401 A D Q E V M E T N R V E S V E P S E N E<br>1201 GCTGACCAGGAGGTAATGGAAACTAATCGAGTTGAAAGTGTAGAACCTTCAGAAAATGAA<br><br>421 A S K E L E P E M E F E I E P D K E C K<br>1261 GCTAGCAAAGAATTGGAACCAGAAATGGAATTTGAAATTGAGCCAGATAAAGAATGTAAA<br><br>441 S L S P G K E N V S A L D M E K E S E E<br>1321 TCCCTTTCTCCTGGGAAAGAGAATGTCAGTGCTTTAGACATGGAAAAGGAGTCTGAGGAA<br><br>461 K E E K E S E P Q P E P V A Q P Q P Q S<br>1381 AAAGAAGAAAAAGAATCTGAGCCCCAACCTGAGCCTGTGGCTCAACCTCAGCCTCAGTCT<br><br>481 Q P Q L Q L Q S Q S Q P V L Q S Q P P S<br>1441 CAGCCCCAGCTTCAGCTTCAATCCCAGTCCCAACCAGTACTCCAGTCCCAGCCTCCCTCT<br><br>501 Q P E D L S L A V L Q P T P Q V T Q E Q<br>1501 CAGCCTGAGGATTTGTCATTAGCTGTTTTACAGCCAACACCCCAAGTTACTCAGGAGCAA<br><br>521 G H L L P E R K D F P V E S V K L T E V | |

| Gene Name | GenBank Homology | DNA SEQUENCE / DEDUCED AMINO ACID SEQUENCE | SEQUENCE IDENTIFIER: |
|---|---|---|---|
| | | 1561 GGGCATTTACTACCTGAGAGGAAGGATTTTCCTGTAGAGTCTGTAAAACTCACTGAGGTA<br><br>541 P V E P V L T V H P E S K S K T K T R S<br>1621 CCAGTAGAGCCAGTCTTGACAGTACATCCAGAGAGCAAGAGCAAAACCAAAACTAGGAGC<br><br>561 R S R G R A R N K T S K S R S R S S S S<br>1681 AGAAGTAGAGGTCGAGCTAGAAATAAAACAAGCAAGAGTAGAAGTCGAAGCAGTAGCAGT<br><br>581 S S S S S S S T S S S S G S S S S S G S<br>1741 AGCAGTTCTAGTAGCAGTTCAACCAGTAGCAGCAGTGGAAGTAGTTCCAGCAGTGGAAGT<br><br>601 S S S R S S S S S S S S T S G S S S R D<br>1801 AGTAGCAGTCGCAGTAGTTCCAGTAGCAGCTCCAGTACAAGTGGCAGCAGCAGCAGAGAT<br><br>621 S S S S T S S S S E S R S R S R G R G H<br>1861 AGTAGCAGTAGCACTAGTAGTAGTAGTGAGAGTAGAAGTCGGAGTAGGGGCCGGGGACAT<br><br>641 N R D R K H R R S V D R K R R D T S G L<br>1921 AACAGAGATAGAAAGCACAGAAGGAGCGTGGATCGGAAGCGAAGGGATACTTCAGGACTA<br><br>661 E R S H K S S K G G S S R D T K G S K D<br>1981 GAAAGAAGTCACAAATCTTCAAAAGGTGGTAGTAGTAGAGATACGAAAGGATCAAAGGAT<br><br>681 K N S R S D R K R S I S E S S R S G K R<br>2041 AAGAATTCCCGGTCCGACAGGAAAAGGTCTATATCCGAGAGTAGTCGATCAGGCAAAAGA<br><br>701 S S R S E R D R K S D R K D K R R -<br>2101 TCGTCGAGAAGTGAAAGAGACCGAAAATCAGACAGGAAAGACAAAAGGCGTTAA | |
| WBC013A09.bFSP_20010810.abd | Homo sapiens sialyltransferase 1 (beta-galactoside alpha-2,6-sialyltransferase), transcript variant 2. | 1 GAGGAGGTTCCTGTAGGGCGGCACAACCAGGGAGGGCGTGGAAGCTCTGCATCCCTTCTC<br>61 CCATACCTTGCTCTACACATCTCTTCATCTGTATCCTCTGCAGCATCCTTGATGATAAAC<br>121 CAGTAAATATGAGTTTTGATCATCCTGAGAAAAATGGGCCTTGGCCTGCAGACCCAATAA<br>181 ACCTTCCCTCCCATGGATAATAGTGCTAATTCCTGAGGACCTGAAGGGCCTGCCGCCCCT<br>241 GGGGGATTAGCCAGAAGCAGGCTTGTTTTCCTGCTCAGAACAAAGTGACTTCCCTGAACA<br>301 CATCTTCATTATGATTCACACCAACCTGAAGAAAAAGTTCAGCTGCTGCGTCCTGGTCTT<br>361 TCTTCTGTTTGCAGTCATCTGTGTGTGGAAGGAAAAGAAGAAAGGGAGTTACTATGATTC<br>421 CTTTAAATTGCAAACCAAGGAATTCCAGGTGTTAAAGAGTCTGGGGAAATTGGCCATGGG<br>481 GTCTGATTCCCAGTCTGTATCCTCAAGCAGCACCCAGGACCCCCACAGGGGCCGCCAGAC<br>541 CCTCGGCAGTCTCAGAGGCCTAGCCAAGGCCAAACCAGAGGCCTCCTTCCAGGTGTGGAA<br>601 CAAGGACAGCTCTTCCAAAAACCTTATCCCTAGGCTGCAAAAGATCTGGAAGAATTACCT<br>661 AAGCATGAACAAGTACAAAGTGTCCTACAAGGGGCCAGGACCAGGCATCAAGTTCAGTGC<br>721 AGAGGCCCTGCGCTGCCACCTCCGGGACCATGTGAATGTATCCATGGTAGAGGTCACAGA | SEQ ID NO:52 |

| Gene Name | GenBank Homology | DNA SEQUENCE / DEDUCED AMINO ACID SEQUENCE | SEQUENCE IDENTIFIER: |
|---|---|---|---|
| | | 781 TTTTCCCTTCAATACCTCTGAATGGGAGGGTTATCTGCCCAAGGAGAGCATTAGGACCAA<br>841 GGCTGGGCCTTGGGGCAGGTGTGCTGTTGTGTCGTCAGCGGGATCTCTGAAGTCCTCCCA<br>901 ACTAGGCAGAGAAATCGATGATCATGACGCAGTCCTGAGGTTTAATGGGGCACCCACAGC<br>961 CAACTTCCAACAAGATGTGGGCACAAAAACTACCATTCGCCTGATGAACTCTCAGTTGGT<br>1021 TACCACAGAGAAGCGCTTCCTCAAAGACAGTTTGTACAATGAAGGAATCCTAATTGTATG<br>1081 GGACCCATCTGTATACCACTCAGATATCCCAAAGTGGTACCAGAATCCGGATTATAATTT<br>1141 CTTTAACAACTACAAGACTTATCGTAAGCTGCACCCCAATCAGCCCTTTTACATCCTCAA<br>1201 GCCCCAGATGCCTTGGGAGCTATGGGACATTCTTCAAGAAATCTCCCCAGAAGAGATTCA<br>1261 GCCAAACCCCCCATCCTCTGGGATGCTTGGTATCATCATCATGATGACGCTGTGTGACCA<br>1321 GGTGGATATTTATGAGTTCCTCCCATCCAAGCGCAAGACTGACGTGTGCTACTACTACCA<br>1381 GAAGTTCTTCGATAGTGCCTGCACGATGGGTGCCTACCACCCGCTGCTCTATGAGAAGAA<br>1441 TTTGGTGAAGCATCTCAACCAGGGCACAGATGAGGACATCTACCTGCTTGGAAAAGCCAC<br>1501 ACTGCCTGGCTTCCGGACCATTCACTGCTAAGCACAGGCTCCTCACTCTTCTCCATCAGG<br>1561 CATTAAATGAATGGTCTCTTGGCCACCCCAGCCTGGGAAGAACATTTTCCTGAACAATTC<br>1621 CAGCCTGCTCCTTTTACTCTAGGGGCCTCTGTCAGCAAGACCATGGGGGCTTCAAGAGCC<br>1681 TGTGGTCAGGAAATCAGGTCCAGCCTTCCCTGTAGCCAGACAGTTTATGAGCCCAGAGCC<br>1741 TCCTGCCACACACATGCACACATATCTAGCATTCTTTCCAGACAGCATCCTCCCCGCCTT<br>1801 CCACCTTGGTAGATGCAAGGTCTATCTCTCCCATCAGGGCTGCCAAAGCTGGGCTTTGTT<br>1861 TTTCCCAGCAGAATGATGCCATTCTCACAAACCAATGCTCTATATTGCTTGAAGTCTGCA<br>1921 TCTAAATATTGATTTCACGTTTTAAAGAAATTCTCTTAAATTACAATTGTGCCCAATGCA<br>1981 GGGTGGCTCTGGGGGGCAAGTAGGTGGTACAGGGGATTGGAAACATGCTCCGCGCCTCCA<br>2041 GAGAAAAGTTGCTCCCGAGGTCCATGCCCCTGGAACGTGTTCCTATCACTCTGGCTGGTT<br>2101 GGGCTGGTCCTTAGACTGGGTGCTTATGATTAAAGGGTCTTGGTTAGCCCACTTTCCCTC<br>2161 TCCATGTGGAGATGGAAGGTAGAGAAGGATACAGTGTCTATCCTCAAGTTGCTACGGTTC<br>2221 AGTGAGAGAGGCAGACATCTGAACAGGCAGGTAGGATTCAGTGTGCTCAGTGCACTGGGG<br>2281 ATTTGGAGAGAGATGGGCTTGCTCTCTCTGTGCACCCAGGAGGGCCACGCACTTAAAACT<br>2341 GTGTTTGTGGATCAGAGAAGGCTTTATAGCACAGGGGGCATTCAGATGAGTCTTAGAGGA<br>2401 AGAGAAGAAACATGGCAAGCAGATTACATCTGAGCCGTTTGAATTGTGTTTTTCTTTCTT<br>2461 CCCATGTTTATTTTCTAAGATCTACCTGAACTTAGAGACTCAAGATATTTTTTTAGGAAA<br>2521 CCTCCTACCCATGTCTGAGGTAGCAAGTGCAGCCTCACGACAGATACCAGGCAATCCAGA<br>2581 GCCACAAAACGTGATTCCTCCAGGCTCTGCCTGGCCTGACCCTGTCCTGTCAGCTGGGTT<br>2641 TACATACCAGTCCCATTCTTCCTTTTCAATACCTACCCCCAAATCTTCTCCTAACCACCA<br>2701 TCTGTTTTTTTTTTTTAAAGCATTTTTTGCTTTAAAAGCATCCTGACCCCAATTTCTTTG<br>2761 AGCTCACGGGCCTTTTGCTGAAGGTCTCTCAGGGTGTAGTGGTGTGGCTCTCTGGACTTA<br>2821 ACGTCACTCTCAGAGGTCAGAACCTTGGAGATCAGAACTGATTCTCACCAGGTGTGAGAG<br>2881 GTGTGGTAGCAGATTGCAATGCTCTGCACCTCTTCCTTGCAAGTGAGCAACTTCAGGCTC<br>2941 TCTGGGCAGAGGCTGGCCCACTGTAGTTTGCAGACATGCTCTCCAGATGGTTTTACTAAG<br>3001 TCCCCTCTCCCTGATAGGGAATCCTGCTGGACCAGCGCAGCCCTGGTGTGGAGAGGGTAA<br>3061 AGGACTTGCACAGGATCACCAAGTCATGCTGTAGAGCCAGGATTCCTAGACCCAGGGCTC<br>3121 TGCACTCTCAAGGCTGGCCCCATGTGCTCAAGGGCCTCCAATGTTTGCGCTCCAGACCAG<br>3181 CCGTCTGCAAGCTGGACTTCTCATTTGCTGCCAAACCCTTGGCTTACATATGCAGTAGAA<br>3241 AGAGCCCTGGAGTTAGAAGCCCTGGGTTTTCAAGTTGATGTTCCTCTACTTACTAGCTGT<br>3301 GCCACTCTGGGCAAATGCTCTTCCTTGAGCCTGTTTCCACACCTGTAAAGTGGGATGAT | |

| Gene Name | GenBank Homology | DNA SEQUENCE / DEDUCED AMINO ACID SEQUENCE | SEQUENCE IDENTIFIER: |
|---|---|---|---|
| | | 3361 GATCCTATCTCACTGCTTTTGTGAGGATTACAGGAAAGCACCTGTCCTGGTTCTGCACCT<br>3421 GGCTCATGGCAGGTGCTCAGTACATGGTGGTGTCCTTCCTTCGTTCACTAAGGACCTGCT<br>3481 CTGAGCTCACACCTCGGCTGCATGCACCCTGCTGTGACGGAGGCTAGTGTGGAAGAGGTC<br>3541 CTGTCCTCAGGGAATTAACTGTCTTATTGGGAGACAACAACTACCCTGCCTGGGGCACCC<br>3601 AGGAAACCATCCGAGGCAGTGGACAACACAGAACACGCCCTCCTCCTCGCTGCCTGCAGC<br>3661 TCCAATCTGATTCTGCTTGGGAATGGGCTGAGCACGTGGGCTGCTTAACTGCTGTATAGG<br>3721 ACAAGCCCCTTACCCCTCTCTGGGCCCATGAATTCCTGGCTCTCTTTATGTTCTGATTTG<br>3781 ACACATTCATTGCAATCTTTGAATCATGAACATGAGTGCAGAGGAAGGTGGCATTCCATC<br>3841 AGCAGAAGAGAGATGTTGGTGTGGGACCCTGAGACTACACTGGGTAGATGCTAGCTTTTA<br>3901 ATTATGATTAATATAAGGATCAAATTAATTTAAATATGATTCTGAAGTCTACAGAACTT<br>3961 TTAGTTCTGTGCTGTCTATGTGGACACTTTGGTAAAATGCAAATTATGATATGGACGTTA<br>4021 TCATTGGTCTGGTGAGCTGTTTAATATTTGTGACAGATAATTTAAAAATTATGACTTAAT<br>4081 GCTGCCTGTGTCTATGGGGTTCTGTCTTCTTTGATAGCCATCTATTCATCTGGATCATGG<br>4141 GACCCTCTCTAATCCTTCCAACAATCAAATAAGCTATCGATATTGTTTTGGAGTTGAGAT<br>4201 ATCAGTCTCGGAAACTTCTGAAAAATGCTAATAATTACCCAAGGATTATGTCAAATTTTA<br>4261 AAATAAATGTGTGTGTGTTTCTTTAAAAAAAAAAAAAAAA | |
| | | 1 M I H T N L K K K F S C C V L V F L L F<br>1 ATGATTCACACCAACCTGAAGAAAAAGTTCAGCTGCTGCGTCCTGGTCTTTCTTCTGTTT<br><br>21 A V I C V W K E K K K G S Y Y D S F K L<br>61 GCAGTCATCTGTGTGTGGAAGGAAAAGAAGAAAGGGAGTTACTATGATTCCTTTAAATTG<br><br>41 Q T K E F Q V L K S L G K L A M G S D S<br>121 CAAACCAAGGAATTCCAGGTGTTAAAGAGTCTGGGGAAATTGGCCATGGGGTCTGATTCC<br><br>61 Q S V S S S S T Q D P H R G R Q T L G S<br>181 CAGTCTGTATCCTCAAGCAGCACCCAGGACCCCCACAGGGGCCGCCAGACCCTCGGCAGT<br><br>81 L R G L A K A K P E A S F Q V W N K D S<br>241 CTCAGAGGCCTAGCCAAGGCCAAACCAGAGGCCTCCTTCCAGGTGTGGAACAAGGACAGC<br><br>101 S S K N L I P R L Q K I W K N Y L S M N<br>301 TCTTCCAAAAACCTTATCCCTAGGCTGCAAAAGATCTGGAAGAATTACCTAAGCATGAAC<br><br>121 K Y K V S Y K G P G P G I K F S A E A L<br>361 AAGTACAAAGTGTCCTACAAGGGGCCAGGACCAGGCATCAAGTTCAGTGCAGAGGCCCTG<br><br>141 R C H L R D H V N V S M V E V T D F P F<br>421 CGCTGCCACCTCCGGGACCATGTGAATGTATCCATGGTAGAGGTCACAGATTTTCCCTTC<br><br>161 N T S E W E G Y L P K E S I R T K A G P<br>481 AATACCTCTGAATGGGAGGGTTATCTGCCCAAGGAGAGCATTAGGACCAAGGCTGGGCCT | SEQ ID NO:53 |

| Gene Name | GenBank Homology | DNA SEQUENCE / DEDUCED AMINO ACID SEQUENCE | SEQUENCE IDENTIFIER: |
|---|---|---|---|
| | | 181 W G R C A V V S S A G S L K S S Q L G R<br>541 TGGGGCAGGTGTGCTGTTGTGTCGTCAGCGGGATCTCTGAAGTCCTCCCAACTAGGCAGA<br><br>201 E I D D H D A V L R F N G A P T A N F Q<br>601 GAAATCGATGATCATGACGCAGTCCTGAGGTTTAATGGGGCACCCACAGCCAACTTCCAA<br><br>221 Q D V G T K T T I R L M N S Q L V T T E<br>661 CAAGATGTGGGCACAAAAACTACCATTCGCCTGATGAACTCTCAGTTGGTTACCACAGAG<br><br>241 K R F L K D S L Y N E G I L I V W D P S<br>721 AAGCGCTTCCTCAAAGACAGTTTGTACAATGAAGGAATCCTAATTGTATGGGACCCATCT<br><br>261 V Y H S D I P K W Y Q N P D Y N F F N N<br>781 GTATACCACTCAGATATCCCAAAGTGGTACCAGAATCCGGATTATAATTTCTTTAACAAC<br><br>281 Y K T Y R K L H P N Q P F Y I L K P Q M<br>841 TACAAGACTTATCGTAAGCTGCACCCCAATCAGCCCTTTTACATCCTCAAGCCCCAGATG<br><br>301 P W E L W D I L Q E I S P E E I Q P N P<br>901 CCTTGGGAGCTATGGGACATTCTTCAAGAAATCTCCCCAGAAGAGATTCAGCCAAACCCC<br><br>321 P S S G M L G I I I M M T L C D Q V D I<br>961 CCATCCTCTGGGATGCTTGGTATCATCATCATGATGACGCTGTGTGACCAGGTGGATATT<br><br>341 Y E F L P S K R K T D V C Y Y Y Q K F F<br>1021 TATGAGTTCCTCCCATCCAAGCGCAAGACTGACGTGTGCTACTACTACCAGAAGTTCTTC<br><br>361 D S A C T M G A Y H P L L Y E K N L V K<br>1081 GATAGTGCCTGCACGATGGGTGCCTACCACCCGCTGCTCTATGAGAAGAATTTGGTGAAG<br><br>381 H L N Q G T D E D I Y L L G K A T L P G<br>1141 CATCTCAACCAGGGCACAGATGAGGACATCTACCTGCTTGGAAAAGCCACACTGCCTGGC<br><br>401 F R T I H C -<br>1201 TTCCGGACCATTCACTGCTAA | |
| WBC013A11.bFSP_20 010810.abd | Homo sapiens nucleosome assembly protein 1-like 2. | 1 ATGGCAGACATTGACAACAAAGAACAGTCTGAACTTGATCAAGATTTGGATGATGTTGAA<br>61 GAAGTAGAAGAAGAAGAAACTGGTGAAGAAACAAAAATCAAAGCGCGTCAGCTGACTGTT<br>121 CAGATGATGCAAAATCCTCAGATTCTTGCAGCCCTTCAAGAAAGACTTGATGGTCTGGTA<br>181 GAAACACCAACAGGATACATTGAAAGCTTGCCTAGAGTAGTTAAAAGACGAGTGAATGCT<br>241 CTCAAAAACCTACAAGTTAAATGTGCACAGATAGAAGCCAAATTCTATGAGGAAGTTCAT | SEQ ID NO:54 |

| Gene Name | GenBank Homology | DNA SEQUENCE / DEDUCED AMINO ACID SEQUENCE | SEQUENCE IDENTIFIER: |
|---|---|---|---|
| | | 301 GATCTTGAAAGAAAGTATGCTGTTCTCTATCAGCCTCTATTTGATAAGCGGTTTGAGATT<br>361 ATTAATGCAATTTATGAACCTACGGAAGAAGAATGTGAGTGGAAACCAGATGAGGAAGAT<br>421 GAAATTTCGGAGGAATTGAAAGAAAAGGCCAAGATTGAAGATGAGAAAAAGGATGAAGAA<br>481 AAAGAAGACCCCAAGGGAATTCCTGAATTTTGGTTGACTGTTTTTAAGAATGTTGACTTG<br>541 CTCAGTGATATGGTTCAGGAACATGATGAACCTATTCTGAAGCACTTGAAAGATATTAAA<br>601 GTGAAGTTCTCAGATGCTGGCCAACCTATGAGCTTTGTCTTAGAATTTCACTTTGAACCC<br>661 AATGAATATTTCACAAATGAAGTGCTGACAAAGACATATAGGATGAGGTCAGAGCCAGAT<br>721 GATTCTGATCCCTTTTCTTTTGATGGACCAGAAATTATGGGTTGTACAGGGTGCCAGATA<br>781 GACTGGAAAAAAGGAAAGAATGTCACTTTGAAAACCATTAAGAAGAAGCAGAAACACAAG<br>841 GGACGTGGGACAGTTCGTACTGTGACCAAAACAGTTTCCAATGACTCTTTCTTTAATTTT<br>901 TTTGCCCCTCCTGAAGTTCCTGAGAGCGGAGATCTGGATGATGATGCTGAAGCTATCCTT<br>961 GCTGCAGACTTTGAAATTGGTCACTTTTTACGTGAGCGTATAATCCCAAGATCAGTGTTA<br>1021 TACTTTACTGGAGAAGCTATTGAAGATGATGACGACGATTATGATGAAGAAGGTGAAGAA<br>1081 GCGGATGAGGAAGGGGAAGAAGAAGGAGATGAGGAAAATGATCCAGACTATGACTCAAAG<br>1141 AAGGATCAAAATCCAGCAGAGTGCAAGCAGCAGTAG<br><br>1 M A D I D N K E Q S E L D Q D L D D V E<br>1 ATGGCAGACATTGACAACAAAGAACAGTCTGAACTTGATCAAGATTTGGATGATGTTGAA<br><br>21 E V E E E E T G E E T K I K A R Q L T V<br>61 GAAGTAGAAGAAGAAGAAACTGGTGAAGAAACAAAAATCAAAGCGCGTCAGCTGACTGTT<br><br>41 Q M M Q N P Q I L A A L Q E R L D G L V<br>121 CAGATGATGCAAAATCCTCAGATTCTTGCAGCCCTTCAAGAAAGACTTGATGGTCTGGTA<br><br>61 E T P T G Y I E S L P R V V K R R V N A<br>181 GAAACACCAACAGGATACATTGAAAGCTTGCCTAGAGTAGTTAAAAGACGAGTGAATGCT<br><br>81 L K N L Q V K C A Q I E A K F Y E E V H<br>241 CTCAAAAACCTACAAGTTAAATGTGCACAGATAGAAGCCAAATTCTATGAGGAAGTTCAT<br><br>101 D L E R K Y A V L Y Q P L F D K R F E I<br>301 GATCTTGAAAGAAAGTATGCTGTTCTCTATCAGCCTCTATTTGATAAGCGGTTTGAGATT<br><br>121 I N A I Y E P T E E E C E W K P D E E D<br>361 ATTAATGCAATTTATGAACCTACGGAAGAAGAATGTGAGTGGAAACCAGATGAGGAAGAT<br><br>141 E I S E E L K E K A K I E D E K K D E E<br>421 GAAATTTCGGAGGAATTGAAAGAAAAGGCCAAGATTGAAGATGAGAAAAAGGATGAAGAA<br><br>161 K E D P K G I P E F W L T V F K N V D L<br>481 AAAGAAGACCCCAAGGGAATTCCTGAATTTTGGTTGACTGTTTTTAAGAATGTTGACTTG | SEQ ID NO:55 |

| Gene Name | GenBank Homology | DNA SEQUENCE / DEDUCED AMINO ACID SEQUENCE | SEQUENCE IDENTIFIER: |
|---|---|---|---|
| | | 181 L S D M V Q E H D E P I L K H L K D I K<br>541 CTCAGTGATATGGTTCAGGAACATGATGAACCTATTCTGAAGCACTTGAAAGATATTAAA<br><br>201 V K F S D A G Q P M S F V L E F H F E P<br>601 GTGAAGTTCTCAGATGCTGGCCAACCTATGAGCTTTGTCTTAGAATTTCACTTTGAACCC<br><br>221 N E Y F T N E V L T K T Y R M R S E P D<br>661 AATGAATATTTCACAAATGAAGTGCTGACAAAGACATATAGGATGAGGTCAGAGCCAGAT<br><br>241 D S D P F S F D G P E I M G C T G C Q I<br>721 GATTCTGATCCCTTTTCTTTTGATGGACCAGAAATTATGGGTTGTACAGGGTGCCAGATA<br><br>261 D W K K G K N V T L K T I K K K Q K H K<br>781 GACTGGAAAAAAGGAAAGAATGTCACTTTGAAAACCATTAAGAAGAAGCAGAAACACAAG<br><br>281 G R G T V R T V T K T V S N D S F F N F<br>841 GGACGTGGGACAGTTCGTACTGTGACCAAAACAGTTTCCAATGACTCTTTCTTTAATTTT<br><br>301 F A P P E V P E S G D L D D D A E A I L<br>901 TTTGCCCCTCCTGAAGTTCCTGAGAGCGGAGATCTGGATGATGATGCTGAAGCTATCCTT<br><br>321 A A D F E I G H F L R E R I I P R S V L<br>961 GCTGCAGACTTTGAAATTGGTCACTTTTTACGTGAGCGTATAATCCCAAGATCAGTGTTA<br><br>341 Y F T G E A I E D D D D D Y D E E G E E<br>1021 TACTTTACTGGAGAAGCTATTGAAGATGATGACGACGATTATGATGAAGAAGGTGAAGAA<br><br>361 A D E E G E E E G D E E N D P D Y D S K<br>1081 GCGGATGAGGAAGGGGAAGAAGAAGGAGATGAGGAAAATGATCCAGACTATGACTCAAAG<br><br>381 K D Q N P A E C K Q Q -<br>1141 AAGGATCAAAATCCAGCAGAGTGCAAGCAGCAGTAG | |
| WBC013C09.bFSP_20 010810.abd | Human lymphoid-restricted membrane protein (Jaw1). | 1 TATATTGGCAGTTATTGAGGGTAAAGCAATATATTGTAACAGAATGTATAAATATTTTTG<br>61 ATAAAACAGTCTATATTTTATTAAAAAATGAATTATAACCCATTTTCAGTTTTGCCTGCA<br>121 TCATAAGAGTGAGCACTCCATTGCTTTCTTTCCTGGCCACACTGCTACAATCCAGCACTA<br>181 ACTATCCATGTCCAGGTTGAGGATCGAGATCGGGCAGCCCACGGTGCCAGTGAAAAAGCT<br>241 AAGTCTTTGCTGCATAAATTAGAGGAAGCAATTTCGGAACAACGGACTCTGCAGGCTATA<br>301 AATACTGAGTTAGTGAACACTTGCCAGACACTTGAGCAGAAGACAAGAAAACTGAAGAAG<br>361 CTTTTTAGATGAGGAATTTCCTCACTATGATTCCCTGTCCTGCGCAGATGCAATTCAACA<br>421 ACCTCTTCAAGAAAAATTGAAGCAGTGTTGCCATAAACTATATAGTGGTCAAGACGCCAG<br>481 AATACATCAGACGCCCCTGACCTTCAAACATACATGCTGGTACACACCTCTGCTGGATGC | SEQ ID NO:56 |

| Gene Name | GenBank Homology | DNA SEQUENCE / DEDUCED AMINO ACID SEQUENCE | SEQUENCE IDENTIFIER: |
|---|---|---|---|
| | | 541 CTTGCATCTGGATAGTCTTACAACGATTCCAAGGCTGGAATTGACACCTTTCTCATGTTT<br>601 GGCCAACCGAATCCATGCTTCATGTGAAAGACCTAAATATGGAGAAATAGAGGATGGTGC<br>661 TTTGGATGTAAAAAGACAACACAAGTGCCCAGGCCCCACAAGTGGCCCCAGCCCAGGAAC<br>721 GAATCTTTCAGGTTGCATCAGGATGAGTGATGACCTAAGTATGAACGAGAATGGTGTTGA<br>781 ACGCGTGTGTCCTGAGAGCCTGCTGCAGTCCAGGGGATATTCCTCACTACCATTACCCAG<br>841 ACACACTTCATCGACAGACGGTACTATAACTTCAAGTGATCCTGGATTAGAAATTCTGAA<br>901 TATGGCTTCTTGTGACCTTGACAGAAACTCGCTCTGTAAGAAAGAGGAGGATACAAGATC<br>961 AGCTTCTCCCACGATAGAGGCCCAAGGCACAAGTCCAGCTCATGATAATATTGCATTCCA<br>1021 AGACTCTACGAGTAAGGATAAAACCATATTAAATCTGGAAGCCAAAGAGGAACCAGAAAC<br>1081 AATAGAAGAACATAAAAAAGAACATGCTTCAGGAGACTCTGTGGTTTCCCCTCTTCCTGT<br>1141 AACCACTGTGAAATCGGTTAACGTTAGACAAAGTGAGAACACTTCTGCTAATGAGAAGGA<br>1201 GGTGGAGGCAGAATTTCTCAGATTATCTTTGGGATTTAAGTGTGACTGGTTTACCTTGGA<br>1261 GAAGAGAGTGAAGCTTGAAGAGAGGTCCCGTGACTGGGCAGAAGAAAATTTGAAGAAAGA<br>1321 AATCACTAACTCTTTAAAACTATTAGAGTCTTTAACACCTCTGTGTGAAGATGACAACCA<br>1381 GGCACAGGAAATCATTAAGAAGCTGGAGAAGAGTATAAAGTTTCTTAGCCAGTGTGCAGC<br>1441 ACGAGTGGCCAGTAGGGCTGAGATGTTGGGAGCCATCAATCAGGAAAGCCGGGTTAGTAA<br>1501 AGCAGTTGAAGTGATGATTCAGCACGTAGAAAACTTGAAGAGGATGTATGCCAAAGAGCA<br>1561 CGCTGAATTAGAAGAACTGAAACAGGTTCTTCTGCAGAATGAAAGGTCTTTCAATCCTCT<br>1621 TGAAGATGATGATGACTGCCAAATTAAAAAACGTTCAGCTTCTCTAAACTCCAAGCCATC<br>1681 TTCTCTACGAAGAGTGACTATTGCCTCTTTACCCAGAAATATTGGAAATGCAGGAATGGT<br>1741 GGCTGGGATGGAAAATAATGATCGATTCAGTAGAAGGTCAAGCAGTTGGCGTATTTTGGG<br>1801 GTCAAAGCAGAGTGAACACCGTCCCTCATTACCTCGATTTATTAGCACCTATTCCTGGGC<br>1861 AGATGCTGAAGAAGAAAAATGTGAACTAAAAACTAAAGATGACTCAGAACCACCTGAAGA<br>1921 AGAAATAGTAGAAAGAACAAGAAAGCCAAGTCTTTCTGAAAAGAGAAATAATCCATCGAA<br>1981 ATGGGATGTCTCTTCAGCTTATGACACAATAGCTTCCTGGACAACAAATCTCAAGACTTC<br>2041 CATCGGAAAGGCTAACAAGGCACTCTGTTTGTCTGTTGCCTTCATTGTACTGTTTGCAGC<br>2101 TTTGATGAGCTTCCTCACAGGCCTGTTTTTCCAAAAGTCCGTGGATGCCGCTCCCACACA<br>2161 GGATGGGGACTCCTGGATGTCTCTAGAACATATCTTGTGGCCATTTACCAGACTTAGACA<br>2221 CAATGGGCCACCACCAGTGTGACTGCAGCACATCCCAATGTGTGCATCTTGATTTTTAAG<br>2281 GTTTCAGTATCTGAACTTTGTAAATTAGTAAATTTTAGCTGGAAAATTATAGCATGGAAC<br>2341 CAAAGGTTCTCAGGATGCCTGTAAGATATTTTACATTCCCTCTTTTTGTGATTATCTCCC<br>2401 CAACTGAAATACAGTGGG | |
| | | 1 L E L T P F S C L A N R I H A S C E R P<br>1 CTGGAATTGACACCTTTCTCATGTTTGGCCAACCGAATCCATGCTTCATGTGAAAGACCT<br><br>21 K Y G E I E D G A L D V K R Q H K C P G<br>61 AAATATGGAGAAATAGAGGATGGTGCTTTGGATGTAAAAAGACAACACAAGTGCCCAGGC<br><br>41 P T S G P S P G T N L S G C I R M S D D<br>121 CCCACAAGTGGCCCCAGCCCAGGAACGAATCTTTCAGGTTGCATCAGGATGAGTGATGAC<br><br>61 L S M N E N G V E R V C P E S L L Q S R | SEQ ID NO:57 |

| Gene Name | GenBank Homology | DNA SEQUENCE / DEDUCED AMINO ACID SEQUENCE | SEQUENCE IDENTIFIER: |
|---|---|---|---|
| | | 181 CTAAGTATGAACGAGAATGGTGTTGAACGCGTGTGTCCTGAGAGCCTGCTGCAGTCCAGG<br><br>81 G Y S S L P L P R H T S S T D G T I T S<br>241 GGATATTCCTCACTACCATTACCCAGACACACTTCATCGACAGACGGTACTATAACTTCA<br><br>101 S D P G L E I L N M A S C D L D R N S L<br>301 AGTGATCCTGGATTAGAAATTCTGAATATGGCTTCTTGTGACCTTGACAGAAACTCGCTC<br><br>121 C K K E E D T R S A S P T I E A Q G T S<br>361 TGTAAGAAAGAGGAGGATACAAGATCAGCTTCTCCCACGATAGAGGCCCAAGGCACAAGT<br><br>141 P A H D N I A F Q D S T S K D K T I L N<br>421 CCAGCTCATGATAATATTGCATTCCAAGACTCTACGAGTAAGGATAAAACCATATTAAAT<br><br>161 L E A K E E P E T I E E H K K E H A S G<br>481 CTGGAAGCCAAAGAGGAACCAGAAACAATAGAAGAACATAAAAAGAACATGCTTCAGGA<br><br>181 D S V V S P L P V T T V K S V N V R Q S<br>541 GACTCTGTGGTTTCCCCTCTTCCTGTAACCACTGTGAAATCGGTTAACGTTAGACAAAGT<br><br>201 E N T S A N E K E V E A E F L R L S L G<br>601 GAGAACACTTCTGCTAATGAGAAGGAGGTGGAGGCAGAATTTCTCAGATTATCTTTGGGA<br><br>221 F K C D W F T L E K R V K L E E R S R D<br>661 TTTAAGTGTGACTGGTTTACCTTGGAGAAGAGAGTGAAGCTTGAAGAGAGGTCCCGTGAC<br><br>241 W A E E N L K K E I T N S L K L L E S L<br>721 TGGGCAGAAGAAAATTTGAAGAAAGAAATCACTAACTCTTTAAAACTATTAGAGTCTTTA<br><br>261 T P L C E D D N Q A Q E I I K K L E K S<br>781 ACACCTCTGTGTGAAGATGACAACCAGGCACAGGAAATCATTAAGAAGCTGGAGAAGAGT<br><br>281 I K F L S Q C A A R V A S R A E M L G A<br>841 ATAAAGTTTCTTAGCCAGTGTGCAGCACGAGTGGCCAGTAGGGCTGAGATGTTGGGAGCC<br><br>301 I N Q E S R V S K A V E V M I Q H V E N<br>901 ATCAATCAGGAAAGCCGGGTTAGTAAAGCAGTTGAAGTGATGATTCAGCACGTAGAAAAC<br><br>321 L K R M Y A K E H A E L E E L K Q V L L<br>961 TTGAAGAGGATGTATGCCAAAGAGCACGCTGAATTAGAAGAACTGAAACAGGTTCTTCTG<br><br>341 Q N E R S F N P L E D D D D C Q I K K R<br>1021 CAGAATGAAAGGTCTTTCAATCCTCTTGAAGATGATGATGACTGCCAAATTAAAAAACGT | |

| Gene Name | GenBank Homology | DNA SEQUENCE / DEDUCED AMINO ACID SEQUENCE | SEQUENCE IDENTIFIER: |
|---|---|---|---|
| | | 361 S A S L N S K P S S L R R V T I A S L P<br>1081 TCAGCTTCTCTAAACTCCAAGCCATCTTCTCTACGAAGAGTGACTATTGCCTCTTTACCC<br><br>381 R N I G N A G M V A G M E N N D R F S R<br>1141 AGAAATATTGGAAATGCAGGAATGGTGGCTGGGATGGAAAATAATGATCGATTCAGTAGA<br><br>401 R S S S W R I L G S K Q S E H R P S L P<br>1201 AGGTCAAGCAGTTGGCGTATTTTGGGGTCAAAGCAGAGTGAACACCGTCCCTCATTACCT<br><br>421 R F I S T Y S W A D A E E E·K C E L K T<br>1261 CGATTTATTAGCACCTATTCCTGGGCAGATGCTGAAGAAGAAAAATGTGAACTAAAAACT<br><br>441 K D D S E P P E E E I V E R T R K P S L<br>1321 AAAGATGACTCAGAACCACCTGAAGAAGAAATAGTAGAAAGAACAAGAAAGCCAAGTCTT<br><br>461 S E K R N N P S K W D V S S A Y D T I·A<br>1381 TCTGAAAAGAGAAATAATCCATCGAAATGGGATGTCTCTTCAGCTTATGACACAATAGCT<br><br>481 S W T T N L K T S I G K A N K A L C L S<br>1441 TCCTGGACAACAAATCTCAAGACTTCCATCGGAAAGGCTAACAAGGCACTCTGTTTGTCT<br><br>501 V A F I V L F A A L M S F L T G L F F Q<br>1501 GTTGCCTTCATTGTACTGTTTGCAGCTTTGATGAGCTTCCTCACAGGCCTGTTTTTCCAA<br><br>521 K S V D A A P T Q D G D S W M S L E H I<br>1561 AAGTCCGTGGATGCCGCTCCCACACAGGATGGGGACTCCTGGATGTCTCTAGAACATATC<br><br>541 L W P F T R L R H N G P P P V -<br>1621 TTGTGGCCATTTACCAGACTTAGACACAATGGGCCACCACCAGTGTGA | |
| WBC013E05.bFSP_20 010810.abd | Equus caballus MHC class II DQ-alpha chain (DQalpha). | 1 AAAGCTGAGAAGACAATGATCCTAAACAGAGCTCTGATTCTGGGGGCCCTCACCCTGACC<br>61 ACCATGATGAGTGCCTGCGGAGGTGAAGACATTGTGGCTGATCACTTTGCCTCCTACGGC<br>121 ACAAACGTCTACCAGTCTTATGGTGATTTTGGTCAGTACACCCACGAATTTGATGGAGAT<br>181 GAGAAGTTCCATGTGGACCTGGAGAAGAAGGAGACTGTGTGGCGGCTGCCTGTGTTTAGC<br>241 GAGTTTACAAGTTTTGACCCACAGGGCGCGCTGCAAAACATTGCTACGGCAAAATACAAC<br>301 TTGGACATCTTGATTAAACGTTCCAACTCTACTGCTGCTACCAATGAGGTTCCTGAGGTG<br>361 ACTGTGTTTCCCAAGTCTCCTGTGGTGCTGGGTCAGCCCAACACCCTCATCTGTCTTGTG<br>421 GACAACATCTTCCCTCCTGTGATCAATGTCACATGGTTGAGGAATGGGCACTCAGTCAGT<br>481 GAAGGTGTTTTTGAGATGATTTTCCTCCCCAAGACTGATGATTCCTTCCTGAAGATCAGT<br>541 TACCTCACCTTCCTCCCTTCTGCTGATGATATTTATGATTGCAAGGTGGAGCACTGGGGG<br>601 CTGGATGAGCCACTTCTGAAACACTGGGAACCTGAGATTCCAACCCCAATGTCAGAGCTG | SEQ ID NO:58 |

| Gene Name | GenBank Homology | DNA SEQUENCE / DEDUCED AMINO ACID SEQUENCE | SEQUENCE IDENTIFIER: |
|---|---|---|---|
| | | 661 ACAGAGACTGTGGTCTGTGCCCTGGGGTTGGCTGTTGGCTTTGTGGGCATCGTGGTGGGC<br>721 ACCTTCCTCATCATCCGAGCCCTACGCTCAGGTGGTGCCTTAAGACGCCAAGGATGCTTG<br>781 TGAGTTGCATCATAGAAGGGAAGGCACATTGCCCATCTACAAGAGCAGAAGAGTGGATGT<br>841 GCTAGATGACCTCGCACTATTTACTGGCCCAGTTCATCATACTCCTTCTCTTCTTCATCG<br>901 TTTTTCCTCTAACCTTTTCCCTAGGACTTAAGGTGCTGTATCTCCTAAGAGCTCTCATAC<br>961 TCTTCAGAATTCTCCCCCTGACTGCCGGATTATTTTTTCTCTTCTCACTTGTTACCTACT<br>1021 ATGGGATCCNTGGGACATTCCACCTAGTTACCTAATCTCTAGGGACCTGATCCATGTCTC<br>1081 CATGGAAGCAATAATTCTCCTTTATGAGATCTTTTATT | |
| | | 1 M I L N R A L I L G A L T L T T M M S A<br>1 ATGATCCTAAACAGAGCTCTGATTCTGGGGGCCCTCACCCTGACCACCATGATGAGTGCC<br><br>21 C G G E D I V A D H F A S Y G T N V Y Q<br>61 TGCGGAGGTGAAGACATTGTGGCTGATCACTTTGCCTCCTACGGCACAAACGTCTACCAG<br><br>41 S Y G D F G Q Y T H E F D G D E K F H V<br>121 TCTTATGGTGATTTTGGTCAGTACACCCACGAATTTGATGGAGATGAGAAGTTCCATGTG<br><br>61 D L E K K E T V W R L P V F S E F T S F<br>181 GACCTGGAGAAGAAGGAGACTGTGTGGCGGCTGCCTGTGTTTAGCGAGTTTACAAGTTTT<br><br>81 D P Q G A L Q N I A T A K Y N L D I L I<br>241 GACCCACAGGGCGCGCTGCAAAACATTGCTACGGCAAAATACAACTTGGACATCTTGATT<br><br>101 K R S N S T A A T N E V P E V T V F P K<br>301 AAACGTTCCAACTCTACTGCTGCTACCAATGAGGTTCCTGAGGTGACTGTGTTTCCCAAG<br><br>121 S P V V L G Q P N T L I C L V D N I F P<br>361 TCTCCTGTGGTGCTGGGTCAGCCCAACACCCTCATCTGTCTTGTGGACAACATCTTCCCT<br><br>141 P V I N V T W L R N G H S V S E G V F E<br>421 CCTGTGATCAATGTCACATGGTTGAGGAATGGGCACTCAGTCAGTGAAGGTGTTTTTGAG<br><br>161 M I F L P K T D D S F L K I S Y L T F L<br>481 ATGATTTTCCTCCCCAAGACTGATGATTCCTTCCTGAAGATCAGTTACCTCACCTTCCTC<br><br>181 P S A D D I Y D C K V E H W G L D E P L<br>541 CCTTCTGCTGATGATATTTATGATTGCAAGGTGGAGCACTGGGGGCTGGATGAGCCACTT<br><br>201 L K H W E P E I P T P M S E L T E T V V<br>601 CTGAAACACTGGGAACCTGAGATTCCAACCCCAATGTCAGAGCTGACAGAGACTGTGGTC<br><br>221 C A L G L A V G F V G I V V G T F L I I | SEQ ID NO:59 |

| Gene Name | GenBank Homology | DNA SEQUENCE / DEDUCED AMINO ACID SEQUENCE | SEQUENCE IDENTIFIER: |
|---|---|---|---|
| | | 661 TGTGCCCTGGGGTTGGCTGTTGGCTTTGTGGGCATCGTGGTGGGCACCTTCCTCATCATC<br><br>241 R A L R S G G A L R R Q G C L -<br>721 CGAGCCCTACGCTCAGGTGGTGCCTTAAGACGCCAAGGATGCTTGTGA | |
| WBC013E10.bFSP_20 010810.abd | Homo sapiens cDNA FLJ45679 fis, clone ERLTF2001835 | 1 aaaaacccca gcaggttagc ttctcattaa atagcttctc ctgagggcag accttgttaa<br>61 gaagaacaaa atgctctggc gtatttcaaa atggctcctt ttccccaccc actaccagag<br>121 gcatggataa tcaccctgag gacctgtgag agatccagga gcttaaaaaa aattggggag<br>181 cggggaagag ggaatggcta ggtccctctg acttgtccac actgatcctt caggtttacc<br>241 tatcctggta cttgttccat tggaggcttc agcggctagt ttctgctctg tggttaccaa<br>301 tgacacagga gctgagcttc caaaaattta ttgaacaatc tgacttacta ggagaactta<br>361 aatatgactt caatgaaaaa gatgaattca gacatactga gacacaaagg ccttttgtct<br>421 ttaactatta tgaaaatgtc cttgagaaaa atagcaagcg ctaccaggcc cttggccatt<br>481 tgcttgaaca atacatttat gagcttttgg agaaagtgtg caaattagaa aaagtatata<br>541 tcccacctga ggctgataaa gaagaaccaa gaagcttctt tttcatgagc gagaaagcat<br>601 taacaaatca ccattctgct cttcttatcc ttcttcaaga ccatggggtc tttcgagctg<br>661 gtcagtggag tcaacaggca ataatacatc atggtctcca acatggaagt cagataccat<br>721 gtattcaaat ggcattgcag gcacactatg atgtaattgt gctaaacccc aatgataatt<br>781 ttgtggaacc aaaggtggaa aaagagtgga aaggcctttt aacacaaaat attgagtcat<br>841 cttctctaaa aatggttcaa ggtgggagct ttttctctct ccagcatcct cccaaatgca<br>901 ttccaaaaag atgcagcaac acccccgaag aacacacggc ttacatatgg gattacttca<br>961 tttcaaagac tgaaggcaag gatattgcct tcattgtaca tggttatgga ggcttggttt<br>1021 ttatggactt gcttgttcgt agaaggtggg aagtgatgag caaagtatat gctgttgcac<br>1081 ttatcgactc tgaacatcac gtaggacacc agctgggaag tgatgtacaa ttattagcat<br>1141 ggataaagca ccactgccgt gaatgggtga caagtcctaa gcctttggat aaacctgcag<br>1201 ctactgtttt caaaaaggaa tttcctatgg tttctgctgg cacagaaaag tacatcttag<br>1261 ccccttcctc tagccttcag tcaattttta agtactttaa aaaagcttta aaagccagaa<br>1321 caaccattaa tttctctcga atgccaatag tgactagaag ctccacaaaa agaaagcaaa<br>1381 gtgcttaaac tacttttgtc atctaagatt tttttgtcct tctgcaactt tgctaataca<br>1441 gattctggaa gaaaaaaaaa cactttcaac tcacacacaa tatgatgttc tggcttgagg<br>1501 tttgatttgt tactttttat tatttggttt ttatgaggca gggtctgact ttgctgccca<br>1561 gggtggtatg gaaatctggc ctcaagcgaa cctcccacct cagcctccca aagtgctgag<br>1621 attataggca ttagccactg cacatggcca tggccaggtt tgttgctttt tttttttttg<br>1681 gaggaggagg aggaggagtc tctgtcaccc aggttggagt gcagtggcac catctcagct<br>1741 cactgcaacc tccacctcct ggtttcaagc agttctcctg cctcagcctc ccaagtagct<br>1801 gggactacag gcacatgcca ccacgcccag ctaatttttg tatttttact agagacgggc<br>1861 tgttcgccag gctggtctag gactcttgac ctcaggtgat ccacccacct tggcctccca<br>1921 aagtgctggg cttacaggca taagccactg cacctggcca ttttgttgtt tttttgaaaa<br>1981 gtgaacatat tctctctttt gtagcctgtt gatgaactgg gaattcagtg tcatcgttaa<br>2041 tattttacag tgttagttaa gtgtgaagat ctttctttta aaaagctgtg gtttgatata<br>2101 atgacaacaa aatggcaatt gataaaaatt agatattgaa tcaatctttg atttctataa<br>2161 taataccata gtaaaattac aagtgaacat aaaaattaaa catcataagc aaactgcatt | SEQ ID NO:60 |

| Gene Name | GenBank Homology | DNA SEQUENCE / DEDUCED AMINO ACID SEQUENCE | SEQUENCE IDENTIFIER: |
|---|---|---|---|
| | | 2221 ctttgtgtgt ttatgttttt tgttaagatt tgttgtactc cctgttcacc aggtctgaag<br>2281 aaaaataaat tttaaat | |
| WBC014B05.bFSP_20 010910.abd | Homo sapiens neuronal apoptosis inhibitory protein mRNA | 1 ACAAAAGGTCCTGTGCTCACCTGGGACCCTTCTGGACGTTGCCCTGTGTACCTCTTCGAC<br>61 TGCCTGTTCATCTACGACGAACCCCGGGTATTGACCCCAGACAACAATGCCACTTCATAT<br>121 TGGGGACTTCGTCTGGGATTCCAAGGTGCATTCATTGCAAAGTTCCTTAAATATTTTCTC<br>181 ACTGCTTCCTACTAAAGGACGGACAGAGCATTTGTTCTTCAGCCACATACTTTCCTTCCA<br>241 CTGGCCAGCATTCTCCTCTATTAGACTAGAACTGTGGATAAACCTCAGAAAATGGCCACC<br>301 CAGCAGAAAGCCTCTGACGAGAGGATCTCCCAGTTTGATCACAATTTGCTGCCAGAGCTG<br>361 TCTGCTCTTCTGGGCCTAGATGCAGTTCAGTTGGCAAAGGAACTAGAAGAAGAGGAGCAG<br>421 AAGGAGCGAGCAAAAATGCAGAAAGGCTACAACTCTCAAATGCGCAGTGAAGCAAAAAGG<br>481 TTAAAGACTTTTGTGACTTATGAGCCGTACAGCTCATGGATACCACAGGAGATGGCGGCC<br>541 GCTGGGTTTTACTTCACTGGGGTAAAATCTGGGATTCAGTGCTTCTGCTGTAGCCTAATC<br>601 CTCTTTGGTGCCGGCCTCACGAGACTCCCCATAGAAGACCACAAGAGGTTTCATCCAGAT<br>661 TGTGGGTTCCTTTTGAACAAGGATGTTGGTAACATTGCCAAGTACGACATAAGGGTGAAG<br>721 AATCTGAAGAGCAGGCTGAGAGGAGGTAAAATGAGGTACCAAGAAGAGGAGGCTAGACTT<br>781 GCGTCCTTCAGGAACTGGCCATTTTATGTCCAAGGGATATCCCCTTGTGTGCTCTCAGAG<br>841 GCTGGCTTTGTCTTTACAGGTAAACAGGACACGGTACAGTGTTTTTCCTGTGGTGGATGT<br>901 TTAGGAAATTGGGAAGAAGGAGATGATCCTTGGAAGGAACATGCCAAATGGTTCCCCAAA<br>961 TGTGAATTTCTTCGGAGTAAGAAATCCTCAGAGGAAATTACCCAGTATATTCAAAGCTAC<br>1021 AAGGGATTTGTTGACATAACGGGAGAACATTTTGTGAATTCCTGGGTCCAGAGAGAATTA<br>1081 CCTATGGCATCAGCTTATTGCAATGACAGCATCTTTGCTTACGAAGAACTACGGCTGGAC<br>1141 TCTTTTAAGGACTGGCCCCGGGAATCAGCTGTGGGAGTTGCAGCACTGGCCAAAGCAGGT<br>1201 CTTTTCTACACAGGTATAAAGGACATCGTCCAGTGCTTTTCCTGTGGAGGGTGTTTAGAG<br>1261 AAATGGCAGGAAGGTGATGACCCATTAGACGATCACACCAGATGTTTTCCCAATTGTCCA<br>1321 TTTCTCCAAAATATGAAGTCCTCTGCGGAAGTGACTCCAGACCTTCAGAGCCGTGGTGAA<br>1381 CTTTGTGAATTACTGGAAACCACAAGTGAAAGCAATCTTGAAGATTCAATAGCAGTTGGT<br>1441 CCTATAGTGCCAGAAATGGCACAGGGTGAAGCCCAGTGGTTTCAAGAGGCAAAGAATCTG<br>1501 AATGAGCAGCTGAGAGCAGCTTATACCAGCGCCAGTTTCCGCCACATGTCTTTGCTTGAT<br>1561 ATCTCTTCCGATCTGGCCACGGACCACTTGCTGGGCTGTGATCTGTCTATTGCTTCAAAA<br>1621 CACATCAGCAAACCTGTGCAAGAACCTCTGGTGCTGCCTGAGGTCTTTGGCAACTTGAAC<br>1681 TCTGTCATGTGTGTGGAGGGTGAAGCTGGAAGTGGAAAGACGGTCCTCCTGAAGAAAATA<br>1741 GCTTTTCTGTGGGCATCTGGATGCTGTCCCCTGTTAAACAGGTTCCAGCTGGTTTTCTAC<br>1801 CTCTCCCTTAGTTCCACCAGACCAGACGAGGGGCTGGCCAGTATCATCTGTGACCAGCTC<br>1861 CTAGAGAAAGAAGGATCTGTTACTGAAATGTGCATGAGGAACATTATCCAGCAGTTAAAG<br>1921 AATCAGGTCTTATTCCTTTTAGATGACTACAAAGAAATATGTTCAATCCCTCAAGTCATA<br>1981 GGAAAACTGATTCAAAAAAACCACTTATCCCGGACCTGCCTATTGATTGCTGTCCGTACA<br>2041 AACAGGGCCAGGGACATCCGCCGATACCTAGAGACCATTCTAGAGATCAAAGCATTTCCC<br>2101 TTTTATAATACTGTCTGTATATTACGGAAGCTCTTTTCACATAATATGACTCGTCTGCGA<br>2161 AAGTTTATGGTTTACTTTGGAAAGAACCAAAGTTTGCAGAAGATACAGAAAACTCCTCTC<br>2221 TTTGTGGCGGCGATCTGTGCTCATTGGTTTCAGTATCCTTTTGACCCATCCTTTGATGAT<br>2281 GTGGCTGTTTTCAAGTCCTATATGGAACGCCTTTCCTTAAGGAACAAAGCGACAGCTGAA | SEQ ID NO:61 |

| Gene Name | GenBank Homology | DNA SEQUENCE / DEDUCED AMINO ACID SEQUENCE | SEQUENCE IDENTIFIER: |
|---|---|---|---|
| | | 2341 ATTCTCAAAGCAACTGTGTCCTCCTGTGGTGAGCTGGCCTTGAAAGGGTTTTTTCATGT | |
| | | 2401 TGCTTTGAGTTTAATGATGATGATCTCGCAGAAGCAGGGGTTGATGAAGATGAAGATCTA | |
| | | 2461 ACCATGTGCTTGATGAGCAAATTTACAGCCCAGAGACTAAGACCATTCTACCGGTTTTTA | |
| | | 2521 AGTCCTGCCTTCCAAGAATTTCTTGCGGGGATGAGGCTGATTGAACTCCTGGATTCAGAT | |
| | | 2581 AGGCAGGAACATCAAGATTTGGGACTGTATCATTTGAAACAAATCAACTCACCCATGATG | |
| | | 2641 ACTGTAAGCGCCTACAACAATTTTTTCAACTATGTCTCCTGTCACGCTTCAACAAAGGCA | |
| | | 2701 GGGCCAAAAGTTGTCTCTCATTTGCTTCGTTTGGTGGATAAGAAAGAGTCCTTGGAGAAT | |
| | | 2761 ATACTTGAATCTGATGACTACCTGAAACACCGTCCAGAAATTTCAGTGCACATGGAATAT | |
| | | 2821 TTTAGGGTGTTGTGGCAATTATCTCCAAAATATTACTTTTCCCAGGTTTCAGAACATTTA | |
| | | 2881 CTTGCTCTTGCCATAAAAATCGCTTATCAAAGCAATGCTCTTGCTGCATGTTCTCCATTT | |
| | | 2941 ATTTTGAGATTCCTTCAAGGGAGAACCCTGACTTTCAATATGCTTAAGTCACAGTACTTT | |
| | | 3001 TTTGACCACCCCGAAAGCCTATTGTTGTTGAGAAGCATCCAGTTCTCGATAAAAGGAAGG | |
| | | 3061 AAGTCAAGCAGACTAGATCTTTCAGTCCTGGAAACGTGTTTGGACAAATCACAGGCACCG | |
| | | 3121 ACTATCGATCAGGACTATGCTTCTGCCTTTGAACCGGCGAAAGAATGGGAGCAGAATTTA | |
| | | 3181 GCAGAAAAAGAGGAAAACGTGAAGAGTTTTCTGGATATGCAACTCAATGCACCACCAGAC | |
| | | 3241 ATCAGCACTGGCTATTGGAAACTTTCTCCAAAGCCATACAAGATTCCCCTTCTGGAAGTC | |
| | | 3301 CACGTGACTGATATCGACGCTGTGGACCAGGAGATGCTCAGGGTTCTAACGGCAGTTTTC | |
| | | 3361 TCAGCTTCACAGCACATCGAACTCCACCTAAACGACTGCAGGGGCTTTCCGGAAAGCGTC | |
| | | 3421 CGCCCAGCTCTTGAGCTGTCTAAGGCCTCTGTCACCAAGTGCTCCATAAGCAAGTTGGAA | |
| | | 3481 CTCAGCGCAGCCGAACAGGAACTGCTTCTCACCCTGCCTTCCCTGGAATCTCTTGAAGTC | |
| | | 3541 TCAGGGACAATCCAGTCACAAGACCAAATCTTTCCTAATCTGGATAAGTTCCTGTGCCTG | |
| | | 3601 AAAGAACTGTCTGTGGATCTGGAGGGCAATATAAATGTTTTTTCAGTCATTCCTGAAGAA | |
| | | 3661 TTTCCAAACTTCCACCATATGGAGAAATTATTGATCCAAATTTCAGCTGAGTATGATCCT | |
| | | 3721 TCCAAACTAGTAAAATTAATTCAAAATTCTCCAAACCTTCATGTTTTCCATCTGAAGTGT | |
| | | 3781 AACTTCTTTTCGGATTTTGGGTCTCTCATGACTATGCTTGTTTCCTGTAAGAAACTCACA | |
| | | 3841 GAAATTAAGTTTTCGGATTCATTTTTTCAAGCCGTCCCATTTGTTGCCAGTTTGCCAAAT | |
| | | 3901 TTTATTTCTCTGAAGATATTAAATCTTGAAGGCCAGCAATTTCCTGATGAGGAAACATCA | |
| | | 3961 GAAAAATTTGCCTACATTTTAGGTTCTCTTAGTAACCTGGAAGAATTGATCCTTCCTACT | |
| | | 4021 GGGGATGGAATTTATCGAGTGGCCAAACTGATCATCCAGCAGTGTCAGCAGCTTCATTGT | |
| | | 4081 CTCCGAGTCCTCTCATTTTTCAAGACTTTGAATGATGACAGCGTGGTGGAAATTGCCAAA | |
| | | 4141 GTAGCAATCAGTGGAGGTTTCCAGAAACTTGAGAACCTAAAGCTTTCAATCAATCACAAG | |
| | | 4201 ATTACAGAGGAAGGATACAGAAATTTCTTTCAAGCACTGGACAACATGCCAAACTTGCAG | |
| | | 4261 GAGTTGGACATCTCCAGGCATTTCACAGAGTGTATCAAAGCTCAGGCCACAACAGTCAAG | |
| | | 4321 TCTTTGAGTCAATGTGTGTTACGACTACCAAGGCTCATTAGACTGAACATGTTAAGTTGG | |
| | | 4381 CTCTTGGATGCAGATGATATTGCATTGCTTAATGTCATGAAAGAAAGACATCCTCAATCT | |
| | | 4441 AAGTACTTAACTATTCTCCAGAAATGGATACTGCCGTTCTCTCCAATCATTCAGAAATAA | |
| | | 4501 AAGATTCAGCTAAAAACTGCTGAATCAATAATTTGTCTTGGGGCATATTGAGGATGTAAA | |
| | | 4561 AAAAGTTGTTGATTAATGCTAAAAACCAAATTATCCAAAATTATTTTATTAAATATTGCA | |
| | | 4621 TACAAAAGAAAATGTGTAAGGCTTGCTAAAAAACAAAACAAAACAAAACACAGTCCTGCA | |
| | | 4681 TACTCACCACCAAGCTCAAGAAATAAATCATCACCAATACCTTTGAGGTCCCTGAGTAAT | |
| | | 4741 CCACCCCAGCTAAAGGCAAACCCTTCAATCAAGTTTATACAGCAAACCCTCCATTGTCCA | |
| | | 4801 TGGTCAACAGGGAAGGGGTTGGGGACAGGTCTGCCAATCTATCTAAAAGCCACAATATGG | |
| | | 4861 AAGAAGTATTCAATTTATATAATAAATGGCTAACTTAACGGTTGAATCACTTTCATACAT | |

| Gene Name | GenBank Homology | DNA SEQUENCE / DEDUCED AMINO ACID SEQUENCE | SEQUENCE IDENTIFIER: |
|---|---|---|---|
| | | 4921 GGATGAAACGGGTTTAACACAGGATCCACATGAATCTTCTGTGGGCCAAGAGATGTTCCT<br>4981 TAATCCTTGTAGAACCTGTTTTCTATATTGAACTAGCTTTGGTACAGTAGAGTTAACTTA<br>5041 CTTTCCATTTATCCACTGCCAATATAAAGAGGAAACAGGGGTTAGGGAAAAATGACTTCA<br>5101 TTCCAGAGGCTTCTCAGAGTTCAACATATGCTATAATTTAGAATTTTCTTATGAATCCAC<br>5161 TCTACTTGGGTAGAAAATATTTTATCTCTAGTGATTGCATATTATTTCCATATCATAGTA<br>5221 TTTCATAGTATTATATTTGATATGAGTGTCTATATCAATGTCAGTGTCCAGAATTTCGTT<br>5281 CCTACCAGTTAAGTAGTTTTCTGAACGGCCAGAAGACCATTCGAAATTCATGATACTACT<br>5341 ATAAGTTGGTAAACAACCATACTTTTATCCTCATTTTTATTCTCACTAAGAAAAAAGTCA<br>5401 ACTCCCCTCCCCTTGCCCAAGTATGAAATATAGGGACAGTATGTATGGTGTGGTCTCATT<br>5461 TGTTTAGAAAACCACTTATGACTGGGTGCGGTGGCTCACACCTGTAATCCCAGCACTTTG<br>5521 GGAGGCTGAGGCGGGCGAATCATTTGAGGTGAGGAATTCGAGACCAGCCTGGCCAGCATG<br>5581 GTGAAACCCCATCTCTACTAAAAATACAAAAATTAGCCAGGTGTGGTGGCACATGCCTGT<br>5641 AGTCCCAGCCACTAGGGCGGCTGAGACGCAAGACTTGCTTGAACCCGGGAGGCAGAGGTT<br>5701 GCAGTGAGCCAAGATGGCGCCACTGCATTCCAGCCTGGGCAACAGAGCAAGACCCTGTCT<br>5761 GTCTCAAAACAAAAAACAAAACCACTTATATTGCTAGCTACATTAAGAATTTCTGAATAT<br>5821 GTTACTGAGCTTGCTTGTGGTAACCATTTATAATATCAGAAAGTATATGTACACCAAAAC<br>5881 ATGTTGAACATCCATGTTGTACAACTGAAATATAAATAATTTTGTCAATTATACCTAAAT<br>5941 AAAACTGGAAAAAAATTTCTGGAAGTTTATATCTAAAAATGTTAATAGTGCGTACCTCTA<br>6001 GGAAGTGGGCCTGGAAGCCATTCTTACTTTTCAGTCTCTCCCATTCTGTACTGTTTTTTG<br>6061 TTTTACTTTCGTGCCTGCATTATTTTTCTATTTAAAACAAAAATAAATCTAGTTTAGCAC<br>6121 T | |
| | | 1 M A T Q Q K A S D E R I S Q F D H N L L<br>1 ATGGCCACCCAGCAGAAAGCCTCTGACGAGAGGATCTCCCAGTTTGATCACAATTTGCTG<br><br>21 P E L S A L L G L D A V Q L A K E L E E<br>61 CCAGAGCTGTCTGCTCTTCTGGGCCTAGATGCAGTTCAGTTGGCAAAGGAACTAGAAGAA<br><br>41 E E Q K E R A K M Q K G Y N S Q M R S E<br>121 GAGGAGCAGAAGGAGCGAGCAAAAATGCAGAAAGGCTACAACTCTCAAATGCGCAGTGAA<br><br>61 A K R L K T F V T Y E P Y S S W I P Q E<br>181 GCAAAAAGGTTAAAGACTTTTGTGACTTATGAGCCGTACAGCTCATGGATACCACAGGAG<br><br>81 M A A A G F Y F T G V K S G I Q C F C C<br>241 ATGGCGGCCGCTGGGTTTTACTTCACTGGGGTAAAATCTGGGATTCAGTGCTTCTGCTGT<br><br>101 S L I L F G A G L T R L P I E D H K R F<br>301 AGCCTAATCCTCTTTGGTGCCGGCCTCACGAGACTCCCCATAGAAGACCACAAGAGGTTT<br><br>121 H P D C G F L L N K D V G N I A K Y D I<br>361 CATCCAGATTGTGGGTTCCTTTTGAACAAGGATGTTGGTAACATTGCCAAGTACGACATA | SEQ ID NO:62 |

| Gene Name | GenBank Homology | DNA SEQUENCE / DEDUCED AMINO ACID SEQUENCE | SEQUENCE IDENTIFIER: |
|---|---|---|---|
| | | 141 R V K N L K S R L R G G K M R Y Q E E E<br>421 AGGGTGAAGAATCTGAAGAGCAGGCTGAGAGGAGGTAAAATGAGGTACCAAGAAGAGGAG<br><br>161 A R L A S F R N W P F Y V Q G I S P C V<br>481 GCTAGACTTGCGTCCTTCAGGAACTGGCCATTTTATGTCCAAGGGATATCCCCTTGTGTG<br><br>181 L S E A G F V F T G K Q D T V Q C F S C<br>541 CTCTCAGAGGCTGGCTTTGTCTTTACAGGTAAACAGGACACGGTACAGTGTTTTTCCTGT<br><br>201 G G C L G N W E E G D D P W K E H A K W<br>601 GGTGGATGTTTAGGAAATTGGGAAGAAGGAGATGATCCTTGGAAGGAACATGCCAAATGG<br><br>221 F P K C E F L R S K K S S E E I T Q Y I<br>661 TTCCCCAAATGTGAATTTCTTCGGAGTAAGAAATCCTCAGAGGAAATTACCCAGTATATT<br><br>241 Q S Y K G F V D I T G E H F V N S W V Q<br>721 CAAAGCTACAAGGGATTTGTTGACATAACGGGAGAACATTTTGTGAATTCCTGGGTCCAG<br><br>261 R E L P M A S A Y C N D S I F A Y E E L<br>781 AGAGAATTACCTATGGCATCAGCTTATTGCAATGACAGCATCTTTGCTTACGAAGAACTA<br><br>281 R L D S F K D W P R E S A V G V A A L A<br>841 CGGCTGGACTCTTTTAAGGACTGGCCCCGGGAATCAGCTGTGGGAGTTGCAGCACTGGCC<br><br>301 K A G L F Y T G I K D I V Q C F S C G G<br>901 AAAGCAGGTCTTTTCTACACAGGTATAAAGGACATCGTCCAGTGCTTTTCCTGTGGAGGG<br><br>321 C L E K W Q E G D D P L D D H T R C F P<br>961 TGTTTAGAGAAATGGCAGGAAGGTGATGACCCATTAGACGATCACACCAGATGTTTTCCC<br><br>341 N C P F L Q N M K S S A E V T P D L Q S<br>1021 AATTGTCCATTTCTCCAAAATATGAAGTCCTCTGCGGAAGTGACTCCAGACCTTCAGAGC<br><br>361 R G E L C E L L E T T S E S N L E D S I<br>1081 CGTGGTGAACTTTGTGAATTACTGGAAACCACAAGTGAAAGCAATCTTGAAGATTCAATA<br><br>381 A V G P I V P E M A Q G E A Q W F Q E A<br>1141 GCAGTTGGTCCTATAGTGCCAGAAATGGCACAGGGTGAAGCCCAGTGGTTTCAAGAGGCA<br><br>401 K N L N E Q L R A A Y T S A S F R H M S<br>1201 AAGAATCTGAATGAGCAGCTGAGAGCAGCTTATACCAGCGCCAGTTTCCGCCACATGTCT<br><br>421 L L D I S S D L A T D H L L G C D L S I | |

| Gene Name | GenBank Homology | DNA SEQUENCE / DEDUCED AMINO ACID SEQUENCE | SEQUENCE IDENTIFIER: |
|---|---|---|---|
| | | 1261 TTGCTTGATATCTCTTCCGATCTGGCCACGGACCACTTGCTGGGCTGTGATCTGTCTATT<br><br>441 A S K H I S K P V Q E P L V L P E V F G<br>1321 GCTTCAAAACACATCAGCAAACCTGTGCAAGAACCTCTGGTGCTGCCTGAGGTCTTTGGC<br><br>461 N L N S V M C V E G E A G S G K T V L L<br>1381 AACTTGAACTCTGTCATGTGTGTGGAGGGTGAAGCTGGAAGTGGAAAGACGGTCCTCCTG<br><br>481 K K I A F L W A S G C C P L L N R F Q L<br>1441 AAGAAAATAGCTTTTCTGTGGGCATCTGGATGCTGTCCCCTGTTAAACAGGTTCCAGCTG<br><br>501 V F Y L S L S S T R P D E G L A S I I C<br>1501 GTTTTCTACCTCTCCCTTAGTTCCACCAGACCAGACGAGGGGCTGGCCAGTATCATCTGT<br><br>521 D Q L L E K E G S V T E M C M R N I I Q<br>1561 GACCAGCTCCTAGAGAAAGAAGGATCTGTTACTGAAATGTGCATGAGGAACATTATCCAG<br><br>541 Q L K N Q V L F L L D D Y K E I C S I P<br>1621 CAGTTAAAGAATCAGGTCTTATTCCTTTTAGATGACTACAAAGAAATATGTTCAATCCCT<br><br>561 Q V I G K L I Q K N H L S R T C L L I A<br>1681 CAAGTCATAGGAAAACTGATTCAAAAAAACCACTTATCCCGGACCTGCCTATTGATTGCT<br><br>581 V R T N R A R D I R R Y L E T I L E I K<br>1741 GTCCGTACAAACAGGGCCAGGGACATCCGCCGATACCTAGAGACCATTCTAGAGATCAAA<br><br>601 A F P F Y N T V C I L R K L F S H N M T<br>1801 GCATTTCCCTTTTATAATACTGTCTGTATATTACGGAAGCTCTTTTCACATAATATGACT<br><br>621 R L R K F M V Y F G K N Q S L Q K I Q K<br>1861 CGTCTGCGAAAGTTTATGGTTTACTTTGGAAAGAACCAAAGTTTGCAGAAGATACAGAAA<br><br>641 T P L F V A A I C A H W F Q Y P F D P S<br>1921 ACTCCTCTCTTTGTGGCGGCGATCTGTGCTCATTGGTTTCAGTATCCTTTTGACCCATCC<br><br>661 F D D V A V F K S Y M E R L S L R N K A<br>1981 TTTGATGATGTGGCTGTTTTCAAGTCCTATATGGAACGCCTTTCCTTAAGGAACAAAGCG<br><br>681 T A E I L K A T V S S C G E L A L K G F<br>2041 ACAGCTGAAATTCTCAAAGCAACTGTGTCCTCCTGTGGTGAGCTGGCCTTGAAAGGGTTT<br><br>701 F S C C F E F N D D D L A E A G V D E D<br>2101 TTTTCATGTTGCTTTGAGTTTAATGATGATGATCTCGCAGAAGCAGGGGTTGATGAAGAT | |

| Gene Name | GenBank Homology | DNA SEQUENCE / DEDUCED AMINO ACID SEQUENCE | SEQUENCE IDENTIFIER: |
|---|---|---|---|
| | | 721 E D L T M C L M S K F T A Q R L R P F Y<br>2161 GAAGATCTAACCATGTGCTTGATGAGCAAATTTACAGCCCAGAGACTAAGACCATTCTAC<br><br>741 R F L S P A F Q E F L A G M R L I E L L<br>2221 CGGTTTTTAAGTCCTGCCTTCCAAGAATTTCTTGCGGGGATGAGGCTGATTGAACTCCTG<br><br>761 D S D R Q E H Q D L G L Y H L K Q I N S<br>2281 GATTCAGATAGGCAGGAACATCAAGATTTGGGACTGTATCATTTGAAACAAATCAACTCA<br><br>781 P M M T V S A Y N N F F N Y V S C H A S<br>2341 CCCATGATGACTGTAAGCGCCTACAACAATTTTTTCAACTATGTCTCCTGTCACGCTTCA<br><br>801 T K A G P K V V S H L L R L V D K K E S<br>2401 ACAAAGGCAGGGCCAAAAGTTGTCTCTCATTTGCTTCGTTTGGTGGATAAGAAAGAGTCC<br><br>821 L E N I L E S D D Y L K H R P E I S V H<br>2461 TTGGAGAATATACTTGAATCTGATGACTACCTGAAACACCGTCCAGAAATTTCAGTGCAC<br><br>841 M E Y F R V L W Q L S P K Y Y F S Q V S<br>2521 ATGGAATATTTTAGGGTGTTGTGGCAATTATCTCCAAAATATTACTTTTCCCAGGTTTCA<br><br>861 E H L L A L A I K I A Y Q S N A L A A C<br>2581 GAACATTTACTTGCTCTTGCCATAAAAATCGCTTATCAAAGCAATGCTCTTGCTGCATGT<br><br>881 S P F I L R F L Q G R T L T F N M L K S<br>2641 TCTCCATTTATTTTGAGATTCCTTCAAGGGAGAACCCTGACTTTCAATATGCTTAAGTCA<br><br>901 Q Y F F D H P E S L L L L R S I Q F S I<br>2701 CAGTACTTTTTTGACCACCCCGAAAGCCTATTGTTGTTGAGAAGCATCCAGTTCTCGATA<br><br>921 K G R K S S R L D L S V L E T C L D K S<br>2761 AAAGGAAGGAAGTCAAGCAGACTAGATCTTTCAGTCCTGGAAACGTGTTTGGACAAATCA<br><br>941 Q A P T I D Q D Y A S A F E P A K E W E<br>2821 CAGGCACCGACTATCGATCAGGACTATGCTTCTGCCTTTGAACCGGCGAAAGAATGGGAG<br><br>961 Q N L A E K E E N V K S F L D M Q L N A<br>2881 CAGAATTTAGCAGAAAAAGAGGAAAACGTGAAGAGTTTTCTGGATATGCAACTCAATGCA<br><br>981 P P D I S T G Y W K L S P K P Y K I P L<br>2941 CCACCAGACATCAGCACTGGCTATTGGAAACTTTCTCCAAAGCCATACAAGATTCCCCTT | |

| Gene Name | GenBank Homology | DNA SEQUENCE / DEDUCED AMINO ACID SEQUENCE | SEQUENCE IDENTIFIER: |
|---|---|---|---|
| | | 1001 L E V H V T D I D A V D Q E M L R V L T<br>3001 CTGGAAGTCCACGTGACTGATATCGACGCTGTGGACCAGGAGATGCTCAGGGTTCTAACG<br><br>1021 A V F S A S Q H I E L H L N D C R G F P<br>3061 GCAGTTTTCTCAGCTTCACAGCACATCGAACTCCACCTAAACGACTGCAGGGGCTTTCCG<br><br>1041 E S V R P A L E L S K A S V T K C S I S<br>3121 GAAAGCGTCCGCCCAGCTCTTGAGCTGTCTAAGGCCTCTGTCACCAAGTGCTCCATAAGC<br><br>1061 K L E L S A A E Q E L L L T L P S L E S<br>3181 AAGTTGGAACTCAGCGCAGCCGAACAGGAACTGCTTCTCACCCTGCCTTCCCTGGAATCT<br><br>1081 L E V S G T I Q S Q D Q I F P N L D K F<br>3241 CTTGAAGTCTCAGGGACAATCCAGTCACAAGACCAAATCTTTCCTAATCTGGATAAGTTC<br><br>1101 L C L K E L S V D L E G N I N V F S V I<br>3301 CTGTGCCTGAAAGAACTGTCTGTGGATCTGGAGGGCAATATAAATGTTTTTTCAGTCATT<br><br>1121 P E E F P N F H H M E K L L I Q I S A E<br>3361 CCTGAAGAATTTCCAAACTTCCACCATATGGAGAAATTATTGATCCAAATTTCAGCTGAG<br><br>1141 Y D P S K L V K L I Q N S P N L H V F H<br>3421 TATGATCCTTCCAAACTAGTAAAATTAATTCAAAATTCTCCAAACCTTCATGTTTTCCAT<br><br>1161 L K C N F F S D F G S L M T M L V S C K<br>3481 CTGAAGTGTAACTTCTTTTCGGATTTTGGGTCTCTCATGACTATGCTTGTTTCCTGTAAG<br><br>1181 K L T E I K F S D S F F Q A V P F V A S<br>3541 AAACTCACAGAAATTAAGTTTTCGGATTCATTTTTTCAAGCCGTCCCATTTGTTGCCAGT<br><br>1201 L P N F I S L K I L N L E G Q Q F P D E<br>3601 TTGCCAAATTTTATTTCTCTGAAGATATTAAATCTTGAAGGCCAGCAATTTCCTGATGAG<br><br>1221 E T S E K F A Y I L G S L S N L E E L I<br>3661 GAAACATCAGAAAAATTTGCCTACATTTTAGGTTCTCTTAGTAACCTGGAAGAATTGATC<br><br>1241 L P T G D G I Y R V A K L I I Q Q C Q Q<br>3721 CTTCCTACTGGGGATGGAATTTATCGAGTGGCCAAACTGATCATCCAGCAGTGTCAGCAG<br><br>1261 L H C L R V L S F F K T L N D D S V V E<br>3781 CTTCATTGTCTCCGAGTCCTCTCATTTTTCAAGACTTTGAATGATGACAGCGTGGTGGAA<br><br>1281 I A K V A I S G G F Q K L E N L K L S I | |

| Gene Name | GenBank Homology | DNA SEQUENCE / DEDUCED AMINO ACID SEQUENCE | SEQUENCE IDENTIFIER: |
|---|---|---|---|
| | | 3841 ATTGCCAAAGTAGCAATCAGTGGAGGTTTCCAGAAACTTGAGAACCTAAAGCTTTCAATC<br><br>1301 N H K I T E E G Y R N F F Q A L D N M P<br>3901 AATCACAAGATTACAGAGGAAGGATACAGAAATTTCTTTCAAGCACTGGACAACATGCCA<br><br>1321 N L Q E L D I S R H F T E C I K A Q A T<br>3961 AACTTGCAGGAGTTGGACATCTCCAGGCATTTCACAGAGTGTATCAAAGCTCAGGCCACA<br><br>1341 T V K S L S Q C V L R L P R L I R L N M<br>4021 ACAGTCAAGTCTTTGAGTCAATGTGTGTTACGACTACCAAGGCTCATTAGACTGAACATG<br><br>1361 L S W L L D A D D I A L L N V M K E R H<br>4081 TTAAGTTGGCTCTTGGATGCAGATGATATTGCATTGCTTAATGTCATGAAAGAAAGACAT<br><br>1381 P Q S K Y L T I L Q K W I L P F S P I I<br>4141 CCTCAATCTAAGTACTTAACTATTCTCCAGAAATGGATACTGCCGTTCTCTCCAATCATT<br><br>1401 Q K -<br>4201 CAGAAATAA | |
| WBC014E06.bFSP_20010910.abd | No Homology | 1 TTAAAGAATGAAAGAAAGCCGTTTCACAAAGAAGTAGTGGTGCTTGGAGTCACATTGGGA<br>61 TAGATTTTCTGGTGATTCCCAGTCTGTAAACATCACAGATGTTAGTTTAATGGAATAGGA<br>121 TACATACTGCATTGGCTCCTGTTGAAATTTGAGGAGGAGAATTGGCTTGAAGCTTAGGTT<br>181 TCCTTACCGAAAGCGGCTACTCACTTTCTTAAGTGAGGGTCCTTTCATTCAAGACAAAAA<br>241 TTTTCCTGCCCCTTCTGTTTGTAGTCTCCAAAATTGGAAGGAGAGCTTTATCAACTGCAA<br>301 AGTACAAATTAAGTGCCCTTGCCAAGCTGACTTCTCTTAATTACACTATTGTTCACACTG<br>361 ATTGCCGTCCTTTAGTTCTTTCCTGCTAAACAATGGACTAGTTTCCTCCTCAGTCACTTA<br>421 GAGCTGATTAATATCAGCCTTGGTAAACCAAAAATGTGAGGCTACACCTAATCCAGATTG<br>481 TCTGCAGGACTTTTCTCCTGCATCCCTGCTTGAGGAAGCTTTCTCTAGATAGCAGGTGGC<br>541 CATCGTTACTTTATTGGTTCAGATAACGTTTTATACCGTCTTAGGGGCGCTGATCTGCTA<br>601 TTCTGGAAAGGTTTTATCTTAATAAAGGTGAACTAACAGTGCAGCCAACTACTTCTACCT<br>661 GGATACTTTTCTTCCATCCTACCTCTGGATCTCATACTTCTTCATCACTCAAGACCTGGA<br>721 TGCCACCTCTGTGAAACGCTCCCCAACTCACCGTTAGTAGGAGAAGGGCCGTTATTTTGG<br>781 TTTCCGTGGTCCTTTCCAGAGAGCTGTGCTGTTCATACTAACTGTTGTAATGGAATGATT<br>841 TGATAGCATGTCTCCATTTTCTTTCAAACTGAAAATCTGTGTATCTCCTTGCGTATGCTC<br>901 AGCACCTAATGCACATAGAACTTTACAGGTGCTTGGTAAATTAACCACACTTCTGTTGTC<br>961 TTTTCAAAGACTTTCAAAAGTAATACCAGTTTACAAAGAGTTAATTCTCTAGGGTTCTAG<br>1021 AAGCACAAATCAGGAACGGTACTATTTATTTTTGGTTCTCTGCTGTAAAAAATATCAGGT<br>1081 TACCTGCCTCCAGAGTTCTAAAAAGAGTTGTGTATTTTCAAACAGCTTTTCAGGATGTAA<br>1141 ATACTCACAAAGCAACATATGTATATAGGAATAGATTTAAAATGGAATTTAAAATCACCT<br>1201 TTACTGACGGTTGTTGGTTGTTTTTTTGTTTTGTTTTGTTTTTTTTTACTGTTTCTCTGA<br>1261 GTAACTTTGTATCTAAATGTTTGTTTTGATTGTGGAAGAAAAGACGTGGTGGGTACAAAT | SEQ ID NO:63 |

| Gene Name | GenBank Homology | DNA SEQUENCE / DEDUCED AMINO ACID SEQUENCE | SEQUENCE IDENTIFIER: |
|---|---|---|---|
| | | 1321 TACAGATGTATGCGTGAGAAGGCCCCGTGATATAATAGTGCTTAGCATTCTCTGTTGTAA<br>1381 TTGCGTGTCTGTCCTTTCCTCTTGTCCTTAAACTCCATAGGGCAGGGGTCAGCTCTGTAT<br>1441 TATTGACCATTTTATTCCTTATGTATAACATAATACATGATAAATAATAGGCCTTCAATA<br>1501 AATATTT | |
| WBC014G03.bFSP_20 010910.abd | Homo sapiens non-POU domain containing, octamer-binding, mRNA | 1 GAGAGGCCGTGTAGCGTCGCCGTTACTCCGAGGAGATACCAGTCGGTAGAGGGGTGCAAA<br>61 AATGCAGAGTAATAAAACTTTTAACTTGGAGAAGCAAAACCATACTCCAAGAAAGCATCA<br>121 TCAACATCACCACCAGCAGCAGCACCACCAGCAGCAACAGCAGCAGCCGCCACCACCGCC<br>181 AATACCTGCAAATGGGCAACAGGCCAGCAGCCAAAATGAAGGCTTGACTATTGACCTGAA<br>241 GAATTTTAGAAAACCAGGAGAGAAGACCTTCACCCAACGAAGCCGTCTTTTTGTGGGAAA<br>301 TCTTCCTCCCGACATCACTGAGGAAGAAATGAGGAAACTATTTGAGAAATATGGAAAGGC<br>361 AGGCGAAGTCTTCATTCATAAGGATAAAGGATTTGGCTTTATCCGCTTGGAAACCCGAAC<br>421 CCTAGCGGAGATTGCCAAAGTGGAGCTGGACAATATGCCACTCCGTGGAAAGCAGCTGCG<br>481 TGTGCGCTTTGCCTGCCATAGTGCATCCCTTACAGTTCGAAACCTTCCTCAGTATGTGTC<br>541 CAACGAACTGCTGGAAGAAGCCTTTTCTGTGTTTGGCCAGGTAGAGAGGGCTGTAGTCAT<br>601 TGTGGATGATCGAGGAAGGCCCTCAGGAAAAGGCATTGTTGAGTTCTCAGGGAAGCCAGC<br>661 TGCTCGGAAAGCTCTGGACAGATGCAGTGAAGGCTCCTTCCTGCTAACCACATTTCCTCG<br>721 TCCTGTGACTGTGGAGCCCATGGACCAGTTAGATGATGAAGAGGGACTTCCAGAGAAGCT<br>781 GGTTATAAAAAACCAGCAATTTCACAAGGAACGAGAGCAGCCACCCAGATTTGCACAGCC<br>841 TGGCTCCTTTGAGTATGAATATGCCATGCGCTGGAAGGCACTCATTGAGATGGAGAAGCA<br>901 GCAGCAGGACCAAGTGGACCGCAACATCAAGGAGGCTCGTGAGAAGCTGGAGATGGAGAT<br>961 GGAAGCTGCACGCCATGAGCACCAGGTCATGCTAATGAGACAGGATTTGATGAGGCGCCA<br>1021 AGAAGAACTTCGGAGGATGGAAGAGCTGCACAACCAAGAGGTGCAAAAACGAAAGCAACT<br>1081 GGAGCTCAGGCAGGAGGAAGAGCGCAGGCGCCGTGAAGAAGAGATGCGGCGGCAGCAAGA<br>1141 AGAAATGATGCGGCGACAGCAGGAAGGATTCAAGGGAACCTTCCCTGATGCGAGAGAGCA<br>1201 GGAGATACGGATGGGCCAGATGGCTATGGGAGGTGCTATGGGCATAAACAACAGAGGCGC<br>1261 CATGCCCCCTGCTCCTGTGCCAGCTGGTACCCCAGCTCCTCCAGGACCTGCTACTATGAT<br>1321 GCCAGATGGAACCTTGGGATTGACCCCACCAACAACTGAACGCTTTGGTCAAGCTGCTAC<br>1381 AATGGAAGGAATTGGGGCAATTGGTGGAACCCCTCCTGCGTTCAACCGTGCAGCTCCTGG<br>1441 AGCTGAATTTGCTCCGAACAAACGTCGCCGATACTAATAAAATTGCAGTGTCTAGTTTCC<br>1501 CAAAACCCCTAAAAGAAGGACCCTTTTTGGACTAGCCAGAATTCTACCCTGGAAAAGTGT<br>1561 TAGGGATTCCTCCCAATAGTTAGGTCTTCCCTGCCTGTACAACTTACTCTAGGGAGTATG<br>1621 CTGGAGGCAGAGGGCAAGGGAGGGGCGATATGTAACAAATCAGTTCTGTGTGGTATTTCG<br>1681 TTTAATCAATTCTGTGTGGTGCATTCCTGAAGTCTCATGTGATTGTTGAGGGCCTGGGGG<br>1741 GGAACCATGGCAAAATGGATCCAGTTAGAGCCCCCATTAATCTTAATCATTCCGGTTTTT<br>1801 TTTTTTTTGTCCATCTTGTTTCATTTGCTTGCCCCGCCCCCGAGACGGAGTCTTACTCT<br>1861 GTCGCCCAGGCTGGAGTGTAGTGGCATGATCTCGGCTCACTGCAATCTCTGCCTCCCGGG<br>1921 TTCAAGCTTGTCCAGGTTGATCTTGAACTCCTGACCTCGTGATCTACCCACCTCGGCCTC<br>1981 CCAAAATGCTGGGATTACAGGGGTGAGCCACCGTGCCCAACCTCACTTGCTTCTTATCCT<br>2041 TACACTCCCCCAGCCCCAGAGACACTGCCACATATACCACAAAAAACCAAATGTCCCCCA<br>2101 ATGACCTTAGCCCCATTGCTCCATTCACTCCCAGGTGGGAATTCAGGCAAATGTCCGCAG<br>2161 AGGTCACAGACAACGTGCATACGGTTCTGTTATATCCCATATATTACCCCTTCATGTCCT | SEQ ID NO:64 |

| Gene Name | GenBank Homology | DNA SEQUENCE / DEDUCED AMINO ACID SEQUENCE | SEQUENCE IDENTIFIER: |
|---|---|---|---|
| | | 2221 AAGGAAGACGTTTTCTCTTAGTGATTTTCATTTTAGTATATCTTTTAAAAAATCTTGTGT<br>2281 TAACTTGCCTCCATCTTTTTCTTGGGTGAAGACAACCCAGGAATGGCCCTTTTGTGTCTA<br>2341 TGATGTTGCTGTTCACAGCTTTTCTTGGTAGGCTTAGTACAATCTTGGGAACAGGGTTGC<br>2401 TGTGTGCTGAAGGTGGGACAGTAACTCTTAGCCTTGCCTATCTTAGGTAGTTACGCTGTG<br>2461 CATTTTTTATTGGTGTACCATGTTTGATTTGTCCCTGATACCTTTGGAGTTTTTCTGAAA<br>2521 AATGGAGCAGTAATGCAGCATCAACTTATTAAAATACAGTTTTAAGCCTTTTAAAAAAAA<br>2581 AAAAAAAAAAAAAAAAAAAA | |
| | | 1 M Q S N K T F N L E K Q N H T P R K H H<br>1 ATGCAGAGTAATAAAACTTTTAACTTGGAGAAGCAAAACCATACTCCAAGAAAGCATCAT<br><br>21 Q H H H Q Q Q H H Q Q Q Q Q Q P P P P P<br>61 CAACATCACCACCAGCAGCAGCACCACCAGCAGCAACAGCAGCAGCCGCCACCACCGCCA<br><br>41 I P A N G Q Q A S S Q N E G L T I D L K<br>121 ATACCTGCAAATGGGCAACAGGCCAGCAGCCAAAATGAAGGCTTGACTATTGACCTGAAG<br><br>61 N F R K P G E K T F T Q R S R L F V G N<br>181 AATTTTAGAAAACCAGGAGAGAAGACCTTCACCCAACGAAGCCGTCTTTTTGTGGGAAAT<br><br>81 L P P D I T E E E M R K L F E K Y G K A<br>241 CTTCCTCCCGACATCACTGAGGAAGAAATGAGGAAACTATTTGAGAAATATGGAAAGGCA<br><br>101 G E V F I H K D K G F G F I R L E T R T<br>301 GGCGAAGTCTTCATTCATAAGGATAAAGGATTTGGCTTTATCCGCTTGGAAACCCGAACC<br><br>121 L A E I A K V E L D N M P L R G K Q L R<br>361 CTAGCGGAGATTGCCAAAGTGGAGCTGGACAATATGCCACTCCGTGGAAAGCAGCTGCGT<br><br>141 V R F A C H S A S L T V R N L P Q Y V S<br>421 GTGCGCTTTGCCTGCCATAGTGCATCCCTTACAGTTCGAAACCTTCCTCAGTATGTGTCC<br><br>161 N E L L E E A F S V F G Q V E R A V V I<br>481 AACGAACTGCTGGAAGAAGCCTTTTCTGTGTTTGGCCAGGTAGAGAGGGCTGTAGTCATT<br><br>181 V D D R G R P S G K G I V E F S G K P A<br>541 GTGGATGATCGAGGAAGGCCCTCAGGAAAAGGCATTGTTGAGTTCTCAGGGAAGCCAGCT<br><br>201 A R K A L D R C S E G S F L L T T F P R<br>601 GCTCGGAAAGCTCTGGACAGATGCAGTGAAGGCTCCTTCCTGCTAACCACATTTCCTCGT<br><br>221 P V T V E P M D Q L D D E E G L P E K L<br>661 CCTGTGACTGTGGAGCCCATGGACCAGTTAGATGATGAAGAGGGACTTCCAGAGAAGCTG | SEQ ID NO:65 |

| Gene Name | GenBank Homology | DNA SEQUENCE / DEDUCED AMINO ACID SEQUENCE | SEQUENCE IDENTIFIER: |
|---|---|---|---|
| | | 241 V I K N Q Q F H K E R E Q P P R F A Q P<br>721 GTTATAAAAAACCAGCAATTTCACAAGGAACGAGAGCAGCCACCCAGATTTGCACAGCCT<br><br>261 G S F E Y E Y A M R W K A L I E M E K Q<br>781 GGCTCCTTTGAGTATGAATATGCCATGCGCTGGAAGGCACTCATTGAGATGGAGAAGCAG<br><br>281 Q Q D Q V D R N I K E A R E K L E M E M<br>841 CAGCAGGACCAAGTGGACCGCAACATCAAGGAGGCTCGTGAGAAGCTGGAGATGGAGATG<br><br>301 E A A R H E H Q V M L M R Q D L M R R Q<br>901 GAAGCTGCACGCCATGAGCACCAGGTCATGCTAATGAGACAGGATTTGATGAGGCGCCAA<br><br>321 E E L R R M E E L H N Q E V Q K R K Q L<br>961 GAAGAACTTCGGAGGATGGAAGAGCTGCACAACCAAGAGGTGCAAAAACGAAAGCAACTG<br><br>341 E L R Q E E E R R R R E E E M R R Q Q E<br>1021 GAGCTCAGGCAGGAGGAAGAGCGCAGGCGCCGTGAAGAAGAGATGCGGCGGCAGCAAGAA<br><br>361 E M M R R Q Q E G F K G T F P D A R E Q<br>1081 GAAATGATGCGGCGACAGCAGGAAGGATTCAAGGGAACCTTCCCTGATGCGAGAGAGCAG<br><br>381 E I R M G Q M A M G G A M G I N N R G A<br>1141 GAGATACGGATGGGCCAGATGGCTATGGGAGGTGCTATGGGCATAAACAACAGAGGCGCC<br><br>401 M P P A P V P A G T P A P P G P A T M M<br>1201 ATGCCCCCTGCTCCTGTGCCAGCTGGTACCCCAGCTCCTCCAGGACCTGCTACTATGATG<br><br>421 P D G T L G L T P P T T E R F G Q A A T<br>1261 CCAGATGGAACCTTGGGATTGACCCCACCAACAACTGAACGCTTTGGTCAAGCTGCTACA<br><br>441 M E G I G A I G G T P P A F N R A A P G<br>1321 ATGGAAGGAATTGGGGCAATTGGTGGAACCCCTCCTGCGTTCAACCGTGCAGCTCCTGGA<br><br>461 A E F A P N K R R R Y -<br>1381 GCTGAATTTGCTCCGAACAAACGTCGCCGATACTAA | |
| WBC014H06.bFSP_20010910.abd | No Homology | 1 TTAAATTCTGACTCTGTAAATGCATAAGAGATGTTAAAAATAACAATGCACTTTGAGGAA<br>61 TGTTTGCATATGCCTCTGATTAGGAAGAAAACATTTGTCTATGCTCTGCAGTGGCCAATG<br>121 AAGAATTACTAATGCCTTATTTTCTAGGCATAGATTAGAGAATGTAGACCAGACAATTTG<br>181 TTTACTATTACAAGAAGACTACCAGTTTGCTTACAGGAGATTATTTTTTGTGAACAGTAG<br>241 GTTTGAGTCCAAGACCATTTGAAGAATTACAAACTCTTTCTTAAAAAAAGGAAGAAGTCT | SEQ ID NO:66 |

| Gene Name | GenBank Homology | DNA SEQUENCE / DEDUCED AMINO ACID SEQUENCE | SEQUENCE IDENTIFIER: |
|---|---|---|---|
| | | 301 GCTTGAAATGAATGCAAAATTTCCTTTTAGTCCATCACATTCAAATGTCTTGGTTGTGAC<br>361 CTGTTACCAGTATAGGAGAGAAGCCCAAATCACTGTTTAGATCAAAACATTCTTCATGTA<br>421 GGTAACTATAAGAGGCTAGTGCCTACAAATTACTTCTTCATGCATTGTTGGGAGTCTCTG<br>481 AAAGCACTGGCAATTTTAAGAGTCTTTCTCAGGGTAACTTAATGTTTTCTTAATGAACAA<br>541 TGTTTTCAGCTAAAAAGTCCTTCCAAGTATATTATCTGCCCTTTCAAAAAGTAATCTTAT<br>601 AAAAGAACTTTAGCACTTTCTCAATTGATTTAGGCTATTTTCAGTATTCAGAAATGATTT<br>661 TGATCTCTGTAGAATTAATTGTCTGCTTGAAAATTATTTTTCAAATCCCTTTTAGTGGCA<br>721 ACATTTTTTTCTATTGAAGGTCAAAGGATTGGAAACTGGTTCTTGTATCCATGTAACGTG<br>781 TTATGTGAAAAAGTATTCATGTTGGCAATTTTAGTTAAGCATTATGGTGTGGGTGTAATT<br>841 TTTAAAACTTAATTCAGTGTCTCGTCTGATTAAAGCAGGCACTGATCAGTGTATCTCCTA<br>901 AGAGGTAGTTTACCTCCTATTCCCTTCCTATAATCCTTACATTCTAAATTT<br><br>1 GCTTTTGCAAAAACGGTCTCGACCCTGGTCTGGCTCCTTTTGTACCTCACCTCTCTGCCA<br>61 AACGTGATCTTAAGCAACAAGGAAGCCACACCGTCGTCTGTGAGAAAGTGCGCCTCCTTA<br>121 AAGGGTCCTCTGGGGCTCAAATGGCACCAAGTGGTGAATAACATATCCCAGTTCATTTTC<br>181 TGGACCGTTTTTGTCCTAATGCTTCTGTTTTATGTGGTGATCGCAAAACAAGTATATAAT<br>241 TCTTACAGAAAGTCCAAAAGCAAGGAGAGCGGAAACAACAAAAAGCTGGGAGGTAAAGTG<br>301 TTCGTTGTGGTGGCCGTCTTCTTTGTGTGCTTTGCTCCATTTCATTTTGCCAGAGTGCCA<br>361 TATACACAGAGCCAGACCAACAGCAGGACTGATTGCCGGGTGCAAAACCACCTGTTTATT<br>421 GCTAAGGAAACAACTCTCTTTTTGGCAGCAACTAACATTTGTATGGACCCCTTCATATAT<br>481 ATATTCTTATGTAAAAAATTCACAGAAAGGCTACCATGCATGAGAGGCAGAAAGATGGCA<br>541 ACATCAACCCAAGAAAATCACACCATCCAGACTGACAATAACATAACCCTAGGCTGATAA<br>601 CTTTATTATGGTTAGCTTCTATTTATCAACAAGACTTTAAGAGACAATTTATTTGGAAAT<br>661 AAAATTAAGCATGAAA | SEQ ID NO:67 |
| WBC015B08.bFSP_20010910.abd | No Homology | 1 TGGAGCCTTTGCTGTCTCAGGTGTCCTGATCTCACTTCTCTGTGTGTGTTTGGCAACAGC<br>61 AATTTTATGTCGCTGTGGTTTTGAGAATTTTCTTTTTTAAAAACATGAAGCCTTTAAAAA<br>121 AAAAATGATTTGCGCCTGTCCTCACTGTCTGGTGAAGCAGACAGAAGGAGAGCCCGCCTG<br>181 AAGCGTGTGCAGGGCTCCTGCTCTCCGTGACCAGCCCAGCTGCTCGCAGCACCCACGTCA<br>241 CCTCTCAGGGCTTGGGGACCCCTGGCCACGAGCATTCACTGTTTTCACGGCCAAATAACA<br>301 GGACTTCTGTCACCACTGTCAGACTGATTTAGTCTTCATAATTTTATGTGCTAGTTTTAA<br>361 TCGGAATTGTTCCCTTTTTAAAATTAATTTTTTATTATTCATTCTTGATCATGTTCATCA<br>421 GTAGAGGCTGTTCATAAGAACTCTGATTCTAGCGAATTAGGGTAATTGATACCTTTCTGC<br>481 AGTTTTGAATAAAAGCATTTACAATTCTAAATTATTGTTTTATAAAATTCTTACATTTCA<br>541 GCATTCCCCTCATCACATGCTGATGTAATATTGTTTTATATTAAAATCGTCCTTTTTGCT<br>601 GTTGTGCCACCATTCACGCAAGTACCGTTAGTTCTTAAAATGCGTTTAAAGAAAAAGTGA<br>661 CCAAGTCAACTTTCACACCTCCAGTAATCAGGTAGATTACAAACCCATCCTGGGGGACAG<br>721 TGCCTGGGCAGATGTAGTTTCTCCCCGCTGCCTGTACTGAATAGTGTGCAGTTAGCGTGG<br>781 TCCCCTCACCTCGTGCTTGGAGTGGAAGTCGTCACGATCGCGAGAGTTGACACTCAGATC<br>841 AGGGGTTCGCCGCTGCTCGCTGTGGCCGTGCCAGAGGCAGCCTTTCCAGAGCAAATCCAG<br>901 GGCCCCACGGCGCTCATGCCTTCTCTGACTGCTTTGCCTTTTTCGTTCTGTAAATTGACT<br>961 GGAAAGCCATCTTTAATAAACTCCTCCTGTCTCTTTATGTGTGCGGTTTATGGCCATGGA | SEQ ID NO:68 |

| Gene Name | GenBank Homology | DNA SEQUENCE / DEDUCED AMINO ACID SEQUENCE | SEQUENCE IDENTIFIER: |
|---|---|---|---|
| | | 1021 AGTGACACTTGAGGGAAGCCAGAAGTTTGGTTGAGATTGTGAGAGGCTCTTCTGTGCTTC<br>1081 TATGATATCATACCCGATAACACGTGTGTAAGTTACTAATATGTGAATTGTGTTAAATGT<br>1141 ATCTTCCGTTTGAATTCCCTTTGGATGAAATTATTTCTTGATGTGATACNATAGTGCCTT<br>1201 GTTCTGATTTTTGTTCTTTCCCTAAATGTTATCAAATCAGTAGAAAAGTTAGAAATAAAG<br>1261 TGAATATAGCATTTTTAAAAAAAAAAAAAAAAAAAAAAAAAA | |
| WBC016A12.bFSP_20 011010.abd | No Homology | 1 AAGGGACGAAGCCCCTGTAGCCAACCCTGGCCACCGGTGTATTTGAGCCATGAGTTAAGA<br>61 GAACAACCTTCTATGCATGTAGAGACTGCCCCAACCCATGTTCATCCACAACTGTCCAGA<br>121 GTTCCACAGCCATGGATGTGCCCACAGACTGCAGGGAGAAGGAGTCATAGCACATACAGT<br>181 GAGTGAACAGACATGATACAGAGAAGAGACATGTTATGCACATTTGACACGAGTCCTCTT<br>241 GCGTTTCTTGGTCTAGATGAATTGCTAATGGCTATCACGATCTGCGTCCAGGCAGCTGAG<br>301 CTCAGGGGAAGGCAGCTGACCCCTCTCCCACAGGCCCCCAGACCCGGTGTACCCAGAATC<br>361 AGGCAGGGCAGAGGCAGGACACAAAGGTAATGAAGGACATAGGTTCAGGGATTTGTTTTC<br>421 AGGCTGTTGTTGTTGTTTACATTTTGCATTTTTAAATTTGCTCTCTTTTTAATTTGTTTA<br>481 GAGGGAAAGCTATGTGTTTTTAATTTTTTAATTTTCTTGTTTGTTTTGGTTTGTGATATC<br>541 TGTGGAGGGAGTTTTAAGTAGTGAGCTAATGGACCTTATCTGTTGGCTTAGAGGCCTAAT<br>601 AATAATCAGACCATTTTATTCCTTGTTTGGGTGTTATACTTTCCAAGTCCTTTCCTCTGA<br>661 ATCAGTGGTAATGAATATATTTACATAATCCCTTAGATTTGCTTAGTGCTTTTGTGATTT<br>721 TCAAAGCACTTCCATGAGCATTATCCCTTGCATTCCCTTGACAAGTATTTATGGAAAGTC<br>781 TGCTGTGTGCCAACCATAGTGTTAGGCATGGGGCATCTAAAACAAGTAAGCCTGGGACCC<br>841 TGCCCTCACAAAACGAAAAAATCTAACCAAAGAGGCAGGTTTGTAAACAAATGATTATAA<br>901 TAAACACAA | SEQ ID NO:69 |
| | | 1 GAGGCATTTAAAATTCATGTATGATACTCTTATAATCATAACTGCTGCTAATGCTTGATT<br>61 ATATACTCAGGGACAATGTATAACGTAAGATTACAAATTTGTTCTCACCAACTCCCTTTT<br>121 GGATTTCATTAAGAAAGAAAAACTCCTGGTCAGAGATAATGTATAAATCAGAGATGTAAA<br>181 TGGCTAGTTGTCTATAGCATTAAATGAAGGGAAAACTGATAATTAGGCAGTTAATTTGGT<br>241 CAATTAAACAACCATGTCTGCATCTAGGGGAGCCTGGCTTATTTGATTGATTTCCTACTA<br>301 GTCTTTGACTCTCCCTTCATTCTGGTTTCCATGTGAATTTGCTTTAGAAAAGCCAAGTAT<br>361 GGGCTGTTTCACGTGTTCAGACTTGTGTTTTCATAGCATTCATGGCGGGGCTGCAAGGCT<br>421 TGGAAGTGACCAGGAAGACAGTGAGAACGCGTGGTTGGACAACTGAAATGATGCAGATCA<br>481 GATGGCGTTTCTCCCAGCTCAGTGGATTTTCTTTGGGGTTATTCTTAAAAATATCCATCA<br>541 CTACACTGGAAGTCCCTGTTCTGACTATGTACTGTCCACCTGCATTACANACATTCTGCA<br>601 GAGCTGCTAAGTATATACATATCCGATGTGTGTTACATCTTGTATTTAATTAAATGCTAT<br>661 GAAACTGAAAAAAAAAAAAAA | SEQ ID NO:70 |
| WBC016G11.bFSP_20 011010.abd | Homo sapiens mRNA for HsMcm6. | 1 GGAAAAAAGCGACTTGTGGCGGTCGAGCGTGGCGCAGGCGAATCCTCGGCACTAAGCAAA<br>61 TATGGACCTCGCGGCGGCAGCGGAGCCGGGCGCCGGCAGCCAGCACCTGGAGGTCCGCGA<br>121 CGAGGTGGCCGAGAAGTGCCAGAAACTGTTCCTGGACTTCTTGGAGGAGTTTCAGAGCAG<br>181 CGATGGAGAAATTAAATACTTGCAATTAGCAGAGGAACTGATTCGTCCTGAGAGAAACAC<br>241 ATTGGTTGTGAGTTTTGTGGACCTGGAACAATTTAACCAGCAACTTTCCACCACCATTCA | SEQ ID NO:71 |

| Gene Name | GenBank Homology | DNA SEQUENCE / DEDUCED AMINO ACID SEQUENCE | SEQUENCE IDENTIFIER: |
|---|---|---|---|
| | | 301 AGAGGAGTTCTATAGAGTTTACCCTTACCTGTGTCGGGCCTTGAAAACATTCGTCAAAGA<br>361 CCGTAAAGAGATCCCTCTTGCCAAGGATTTTTATGTTGCATTCCAAGACCTGCCTACCAG<br>421 ACACAAGATTCGAGAGCTCACCTCATCCAGAATTGGTTTGCTCACTCGCATCAGTGGGCA<br>481 GGTGGTGCGGACTCACCCAGTTCACCCAGAGCTTGTGAGCGGAACTTTTCTGTGCTTGGA<br>541 CTGTCAGACAGTGATCAGGGATGTAGAACAGCAGTTCAAATACACACAGCCAAACATCTG<br>601 CCGAAATCCAGTTTGTGCCAACAGGAGGAGATTCTTACTGGATACAAATAAATCAAGATT<br>661 TGTTGATTTTCAAAAGGTTCGTATTCAAGAGACCCAAGCTGAGCTTCCTCGAGGGAGTAT<br>721 CCCCCGCAGTTTAGAAGTAATTTTAAGGGCTGAAGCTGTGGAATCAGCTCAAGCTGGTGA<br>781 CAAGTGTGACTTTACAGGGACACTGATTGTTGTGCCTGACGTCTCCAAGCTTAGCACACC<br>841 AGGAGCACGTGCAGAAACTAATTCCCGTGTCAGTGGTGTTGATGGATATGAGACAGAAGG<br>901 CATTCGAGGACTCCGGGCCCTTGGTGTTAGGGACCTTTCTTATAGGCTGGTCTTTCTTGC<br>961 CTGCTGTGTTGCGCCAACCAACCCAAGGTTTGGGGGGAAAGAGCTCAGAGATGAGGAACA<br>1021 GACAGCTGAGAGCATTAAGAACCAAATGACTGTGAAAGAATGGGAGAAAGTGTTTGAGAT<br>1081 GAGTCAAGATAAAAATCTATACCACAATCTTTGTACCAGCCTGTTCCCTACTATACATGG<br>1141 CAATGATGAAGTAAAACGGGGTGTCCTGCTGATGCTCTTTGGTGGCGTTCCAAAGACAAC<br>1201 AGGAGAAGGGACCTCTCTTCGAGGGGACATAAATGTTTGCATTGTTGGTGACCCAAGTAC<br>1261 AGCTAAGAGCCAATTTCTCAAGCACGTGGAGGAGTTCAGCCCCAGAGCTGTCTACACCAG<br>1321 TGGTAAAGCGTCCAGTGCTGCTGGCTTAACAGCAGCTGTTGTGAGAGATGAAGAATCTCA<br>1381 TGAGTTTGTCATTGAGGCTGGAGCTTTGATGTTGGCTGATAATGGTGTGTGTTGTATTGA<br>1441 TGAATTTGATAAGATGGACGTGCGGGATCAAGTTGCTATTCATGAAGCTATGGAACAGCA<br>1501 GACCATATCCATCACTAAAGCAGGAGTGAAGGCTACTCTGAACGCCCGGACGTCCATTTT<br>1561 GGCAGCAGCAAACCCAATCAGTGGACACTATGACAGATCAAAATCATTGAAACAGAATAT<br>1621 AAATTTGTCAGCTCCCATCATGTCCCGATTCGATCTCTTCTTTATCCTTGTGGATGAATG<br>1681 TAATGAGGTTACAGATTATGCCATTGCCAGGCGCATAGTAGATTTGCATTCAAGAATTGA<br>1741 GGAATCAATTGATCGTGTCTATTCCCTCGATGATATCAGAAGATATCTTCTCTTTGCAAG<br>1801 ACAGTTTAAACCCAAGATTTCCAAAGAGTCAGAGGACTTCATTGTGGAGCAATATAAACA<br>1861 TCTCCGCCAGAGAGATGGTTCTGGAGTGACCAAGTCTTCATGGAGGATTACAGTGCGACA<br>1921 GCTTGAGAGCATGATTCGTCTCTCTGAAGCTATGGCTCGGATGCACTGCTGTGATGAGGT<br>1981 CCAACCTAAACATGTGAAGGAAGCTTTCCGGTTACTGAATAAATCAATCATCCGTGTGGA<br>2041 AACACCTGATGTCAATCTAGATCAAGAGGAAGAGATCCAGATGGAGGTAGATGAGGGTGC<br>2101 TGGTGGCATCAATGGTCATGCTGACAGCCCTGCTCCTGTGAACGGGATCAATGGCTACAA<br>2161 TGAAGACATAAATCAAGAGTCTGCTCCCAAAGCCTCCTTAAGGCTGGGCTTCTCTGAGTA<br>2221 CTGCCGAATCTCTAACCTTATTGTGCTTCACCTCAGAAAGGTGGAAGAAGAAGAGGACGA<br>2281 GTCAGCATTAAAGAGGAGCGAGCTTGTTAACTGGTACTTGAAGGAAATCGAATCAGAAAT<br>2341 AGATTCTGAAGAGGAACTTATAAATAAAAAGAGAATCATAGAGAAAGTCATTCATCGACT<br>2401 CACCCACTACGATCATGTTCTAATTGAGCTCACACAAGCTGGATTGAAAGGCTCGACAGA<br>2461 AGGAAGTGAGAGCTATGAAGAAGATCCCTACCTGGTGGTTAACCCTAACTACCTGCTTGA<br>2521 AGATTGAGGTATTGAAAGTAACTGACCAGAGCTGAGGAACTGTGGCACAGCACCTCGTGG<br>2581 CCTGGAGCCTGGCTGGAGCTCTGCTAGGGACAGAAGTGTTTCTGGAAGTGATGCTTCCAG<br>2641 GATTTGTTTTCAGAAACAAGAATTGAGTTGATGGTCCTATGTGTCACATTCATCACAGGT<br>2701 TTCATACCAACACAGGCTTCAGCACTTCCTTTGGTGTGTTTCCTGTCCCAGTGAAGTTGG<br>2761 AACCAAATAATGTGTAGTCTCTATAACCAATACCTTTGTTTTCATGTGTAAGAAAAGGCC<br>2821 CATTACTTTTAAGGTATGTGCTGTCCTATTGAGCAAATAACTTTTTTTCAATTGCCAGCT | |

| Gene Name | GenBank Homology | DNA SEQUENCE / DEDUCED AMINO ACID SEQUENCE | SEQUENCE IDENTIFIER: |
|---|---|---|---|
| | | 2881 ACTGCTTTTATTCATCAAAATAAAATAACTTGTTCTG<br><br>1 M D L A A A A E P G A G S Q H L E V R D<br>1 ATGGACCTCGCGGCGGCAGCGGAGCCGGGCGCCGGCAGCCAGCACCTGGAGGTCCGCGAC<br><br>21 E V A E K C Q K L F L D F L E E F Q S S<br>61 GAGGTGGCCGAGAAGTGCCAGAAACTGTTCCTGGACTTCTTGGAGGAGTTTCAGAGCAGC<br><br>41 D G E I K Y L Q L A E E L I R P E R N T<br>121 GATGGAGAAATTAAATACTTGCAATTAGCAGAGGAACTGATTCGTCCTGAGAGAAACACA<br><br>61 L V V S F V D L E Q F N Q Q L S T T I Q<br>181 TTGGTTGTGAGTTTTGTGGACCTGGAACAATTTAACCAGCAACTTTCCACCACCATTCAA<br><br>81 E E F Y R V Y P Y L C R A L K T F V K D<br>241 GAGGAGTTCTATAGAGTTTACCCTTACCTGTGTCGGGCCTTGAAAACATTCGTCAAAGAC<br><br>101 R K E I P L A K D F Y V A F Q D L P T R<br>301 CGTAAAGAGATCCCTCTTGCCAAGGATTTTTATGTTGCATTCCAAGACCTGCCTACCAGA<br><br>121 H K I R E L T S S R I G L L T R I S G Q<br>361 CACAAGATTCGAGAGCTCACCTCATCCAGAATTGGTTTGCTCACTCGCATCAGTGGGCAG<br><br>141 V V R T H P V H P E L V S G T F L C L D<br>421 GTGGTGCGGACTCACCCAGTTCACCCAGAGCTTGTGAGCGGAACTTTTCTGTGCTTGGAC<br><br>161 C Q T V I R D V E Q Q F K Y T Q P N I C<br>481 TGTCAGACAGTGATCAGGGATGTAGAACAGCAGTTCAAATACACACAGCCAAACATCTGC<br><br>181 R N P V C A N R R R F L L D T N K S R F<br>541 CGAAATCCAGTTTGTGCCAACAGGAGGAGATTCTTACTGGATACAAATAAATCAAGATTT<br><br>201 V D F Q K V R I Q E T Q A E L P R G S I<br>601 GTTGATTTTCAAAAGGTTCGTATTCAAGAGACCCAAGCTGAGCTTCCTCGAGGGAGTATC<br><br>221 P R S L E V I L R A E A V E S A Q A G D<br>661 CCCCGCAGTTTAGAAGTAATTTTAAGGGCTGAAGCTGTGGAATCAGCTCAAGCTGGTGAC<br><br>241 K C D F T G T L I V V P D V S K L S T P<br>721 AAGTGTGACTTTACAGGGACACTGATTGTTGTGCCTGACGTCTCCAAGCTTAGCACACCA<br><br>261 G A R A E T N S R V S G V D G Y E T E G<br>781 GGAGCACGTGCAGAAACTAATTCCCGTGTCAGTGGTGTTGATGGATATGAGACAGAAGGC | SEQ ID NO:72 |

| Gene Name | GenBank Homology | DNA SEQUENCE / DEDUCED AMINO ACID SEQUENCE | SEQUENCE IDENTIFIER: |
|---|---|---|---|
| | | 281 I R G L R A L G V R D L S Y R L V F L A<br>841 ATTCGAGGACTCCGGGCCCTTGGTGTTAGGGACCTTTCTTATAGGCTGGTCTTTCTTGCC<br><br>301 C C V A P T N P R F G G K E L R D E E Q<br>901 TGCTGTGTTGCGCCAACCAACCCAAGGTTTGGGGGAAAGAGCTCAGAGATGAGGAACAG<br><br>321 T A E S I K N Q M T V K E W E K V F E M<br>961 ACAGCTGAGAGCATTAAGAACCAAATGACTGTGAAAGAATGGGAGAAAGTGTTTGAGATG<br><br>341 S Q D K N L Y H N L C T S L F P T I H G<br>1021 AGTCAAGATAAAAATCTATACCACAATCTTTGTACCAGCCTGTTCCCTACTATACATGGC<br><br>361 N D E V K R G V L L M L F G G V P K T T<br>1081 AATGATGAAGTAAAACGGGGTGTCCTGCTGATGCTCTTTGGTGGCGTTCCAAAGACAACA<br><br>381 G E G T S L R G D I N V C I V G D P S T<br>1141 GGAGAAGGGACCTCTCTTCGAGGGGACATAAATGTTTGCATTGTTGGTGACCCAAGTACA<br><br>401 A K S Q F L K H V E E F S P R A V Y T S<br>1201 GCTAAGAGCCAATTTCTCAAGCACGTGGAGGAGTTCAGCCCCAGAGCTGTCTACACCAGT<br><br>421 G K A S S A A G L T A A V V R D E E S H<br>1261 GGTAAAGCGTCCAGTGCTGCTGGCTTAACAGCAGCTGTTGTGAGAGATGAAGAATCTCAT<br><br>441 E F V I E A G A L M L A D N G V C C I D<br>1321 GAGTTTGTCATTGAGGCTGGAGCTTTGATGTTGGCTGATAATGGTGTGTGTTGTATTGAT<br><br>461 E F D K M D V R D Q V A I H E A M E Q Q<br>1381 GAATTTGATAAGATGGACGTGCGGGATCAAGTTGCTATTCATGAAGCTATGGAACAGCAG<br><br>481 T I S I T K A G V K A T L N A R T S I L<br>1441 ACCATATCCATCACTAAAGCAGGAGTGAAGGCTACTCTGAACGCCCGGACGTCCATTTTG<br><br>501 A A A N P I S G H Y D R S K S L K Q N I<br>1501 GCAGCAGCAAACCCAATCAGTGGACACTATGACAGATCAAAATCATTGAAACAGAATATA<br><br>521 N L S A P I M S R F D L F F I L V D E C<br>1561 AATTTGTCAGCTCCCATCATGTCCCGATTCGATCTCTTCTTTATCCTTGTGGATGAATGT<br><br>541 N E V T D Y A I A R R I V D L H S R I E<br>1621 AATGAGGTTACAGATTATGCCATTGCCAGGCGCATAGTAGATTTGCATTCAAGAATTGAG | |

| Gene Name | GenBank Homology | DNA SEQUENCE / DEDUCED AMINO ACID SEQUENCE | SEQUENCE IDENTIFIER: |
|---|---|---|---|
| | | 561 E S I D R V Y S L D D I R R Y L L F A R<br>1681 GAATCAATTGATCGTGTCTATTCCCTCGATGATATCAGAAGATATCTTCTCTTTGCAAGA<br><br>581 Q F K P K I S K E S E D F I V E Q Y K H<br>1741 CAGTTTAAACCCAAGATTTCCAAAGAGTCAGAGGACTTCATTGTGGAGCAATATAAACAT<br><br>601 L R Q R D G S G V T K S S W R I T V R Q<br>1801 CTCCGCCAGAGAGATGGTTCTGGAGTGACCAAGTCTTCATGGAGGATTACAGTGCGACAG<br><br>621 L E S M I R L S E A M A R M H C C D E V<br>1861 CTTGAGAGCATGATTCGTCTCTCTGAAGCTATGGCTCGGATGCACTGCTGTGATGAGGTC<br><br>641 Q P K H V K E A F R L L N K S I I R V E<br>1921 CAACCTAAACATGTGAAGGAAGCTTTCCGGTTACTGAATAAATCAATCATCCGTGTGGAA<br><br>661 T P D V N L D Q E E E I Q M E V D E G A<br>1981 ACACCTGATGTCAATCTAGATCAAGAGGAAGAGATCCAGATGGAGGTAGATGAGGGTGCT<br><br>681 G G I N G H A D S P A P V N G I N G Y N<br>2041 GGTGGCATCAATGGTCATGCTGACAGCCCTGCTCCTGTGAACGGGATCAATGGCTACAAT<br><br>701 E D I N Q E S A P K A S L R L G F S E Y<br>2101 GAAGACATAAATCAAGAGTCTGCTCCCAAAGCCTCCTTAAGGCTGGGCTTCTCTGAGTAC<br><br>721 C R I S N L I V L H L R K V E E E E D E<br>2161 TGCCGAATCTCTAACCTTATTGTGCTTCACCTCAGAAAGGTGGAAGAAGAAGAGGACGAG<br><br>741 S A L K R S E L V N W Y L K E I E S E I<br>2221 TCAGCATTAAAGAGGAGCGAGCTTGTTAACTGGTACTTGAAGGAAATCGAATCAGAAATA<br><br>761 D S E E E L I N K K R I I E K V I H R L<br>2281 GATTCTGAAGAGGAACTTATAAATAAAAAGAGAATCATAGAGAAAGTCATTCATCGACTC<br><br>781 T H Y D H V L I E L T Q A G L K G S T E<br>2341 ACCCACTACGATCATGTTCTAATTGAGCTCACACAAGCTGGATTGAAAGGCTCGACAGAA<br><br>801 G S E S Y E E D P Y L V V N P N Y L L E<br>2401 GGAAGTGAGAGCTATGAAGAAGATCCCTACCTGGTGGTTAACCCTAACTACCTGCTTGAA<br><br>821 D -<br>2461 GATTGA | |
| WBC017B09.bFSP_20 | Homo sapiens G | | |

| Gene Name | GenBank Homology | DNA SEQUENCE / DEDUCED AMINO ACID SEQUENCE | SEQUENCE IDENTIFIER: |
|---|---|---|---|
| 011010.abd | protein-coupled receptor 155, mRNA (cDNA clone MGC:33390 IMAGE:4813561) | 1 agcggacttt atcggaacgt gcctaagctg ctgcagctgg cacccggttg cgctcggcga<br>61 agagggctgg gggcgggaga tgacggtggt cttctccctg cttggcaccc tgcgagcacc<br>121 agctcccttc tcctcgccac tccaaggttg cagacgaagc aggtactgtc ctagatgtta<br>181 aggacacaac attgagcaaa aacatgcata gtccctgctt ccgtgacact tgagtgaact<br>241 gaatgtgcaa agtccctgtg gcacaaagtc cctggggcca gtgtgtgtgg cgggtgtaga<br>301 aggagctaaa gagatctggt tggagttgga gggtgagaga aaatgaattc taatttacct<br>361 gcagagaact taaccattgc agtcaatatg accaagactt tgcctacagc agtaacgcat<br>421 ggatttaatt ccactaatga cccaccttca atgtcaatta caaggctttt tccagcctta<br>481 ctggaatgct ttggcattgt cctttgtggc tacatagcag gaagggccaa tgtcataaca<br>541 tcaacccagg ccaaaggact aggaaatttt gtctccagat ttgcacttcc agctttatta<br>601 ttcaaaaaca tggttgtact taatttttcc aatgtggact ggtccttcct atatagtatc<br>661 ttaattgcca aagcttctgt atttttcatt gtatgtgtat taaccttatt ggttgccagt<br>721 cctgatagtc gatttagcaa agctggacta ttccctattt ttgctacaca aagtaatgac<br>781 tttgcattgg gataccctat agttgaagct ttatatcaaa ctacataccc agaatatctc<br>841 cagtacattt atttggtggc accaatatct cttatgatgt taaaccctat agggtttatt<br>901 ttctgtgaaa tccaaaagtg ggaagacact caaaatgctt ctcaaaataa aataaaaatt<br>961 gtgggactcg gactcctgcg tgtattacag aacccaatag tatttatggt cttcattggc<br>1021 atcgccttca attttattct tgatcgaaag gtacctgtat atgtcgaaaa ttttcttgat<br>1081 ggacttggaa attctttttc tggatcagcc ctattttatc ttggtctcac gatggtggga<br>1141 aaaataaaga gactgaagaa gtcggcattt gtagtactaa ttcttctcat cacagctaaa<br>1201 cttctggtgc tgccacttct gtgcagagaa atggtggaac tcttggacaa gggcgacagt<br>1261 gtggtgaacc atacaagttt atcaaattat gcatttctgt atggtgtatt tcctgtagca<br>1321 ccaggagtgg ctatctttgc aacacaattc aacatggaag tagaaattat aacctcaggg<br>1381 atggtgataa gcacatttgt gtctgctccc atcatgtacg tttctgcctg gttactgacc<br>1441 tttcccacta tggaccctaa gccattggca tatgccatcc agaatgttag ttttgatata<br>1501 agtattgtca gcctgatctc cttgatctgg tctctggcta ttcttctttt gagtaagaaa<br>1561 tataaacagc ttcctcatat gcttacaact aatttactca ttgctcagtc tattgtctgt<br>1621 gctggaatga tgatatggaa ttttgttaaa gaaaaaaatt ttgttggaca aattttggtg<br>1681 tttgttctat tgtacagctc cctctatagc acctacctgt ggacaggcct tctagcaatt<br>1741 tctttgtttc ttttgaaaaa gcgagagagg gtacaaattc ctgttggaat aatcataata<br>1801 tctggctggg gaattcctgc tctccttgtt ggtgttcttt tgataactgg aaaacacaat<br>1861 ggagatagca ttgactcagc cttcttttat ggaaaagaac agatgatcac cacagcagtc<br>1921 accctgttct gcagcatcct gatagctggc atatccctca tgtgcatgaa ccagactgcc<br>1981 caagcaggag gctatgaagg tttcgatcag tctcagagcc acaaagtggt ggagcctgga<br>2041 aatactgctt ttgaggagag tccagcacca gtaaatgaac cagaactttt tacaagctct<br>2101 attccagaaa caagttgctg ctcctgctcc atgggaaatg gtgaattaca ctgcccatca<br>2161 atagagccaa tagcaaacac aagcaccagt gagcctgtga ttccttcgtt tgagaaaaac<br>2221 aatcattgtg tgagtcgctg taactcccag agctgcatat tagcccagga agaagaacag<br>2281 tatctacaga gtggagacca gcaactgacc cgacatgtgt tgctgtgttt acttctcatc<br>2341 attggcctgt tcgctaatct ttacagttgt ttatggtggc tattcaacca agagcctgga<br>2401 agactttatg ttgagttaca gtttttctgt gccgtgttta actttggtca gggatttatt<br>2461 tcatttggaa tctttggatt agataaacat ttaatcatcc tgcctttcaa aagaagactt<br>2521 gaattcctat ggaacaataa agacacagca gaaaacaggg attctcctgt ttcagaggaa | SEQ ID NO:73 |

| Gene Name | GenBank Homology | DNA SEQUENCE / DEDUCED AMINO ACID SEQUENCE | SEQUENCE IDENTIFIER: |
|---|---|---|---|
| | | 2581 ataaaaatga cctgtcaaca atttatccat tatcaccgtg acctctgtat ccgaaacatt<br>2641 gtcaaagaaa gaaggtgtgg tgcaaagact tctgctggaa ctttctgtgg ctgtgacctg<br>2701 gtgagctggc taattgaagt cggccttgcc tccgaccgtg gtgaagctgt gatatacgga<br>2761 gacaggctgg tacaaggggg agtcatccaa catattacca atgagtatga attccgggat<br>2821 gagtacttgt tttacagatt tcttcaaaag agtcctgaac agagtcctcc tgctattaat<br>2881 gcaaacactc tccaacagga aagatataaa gaaattgagc attcatcccc accctcacat<br>2941 tcccctaaga cctaaattat gcaggggaga accctacatg gaatcatatt ctagccgcgt<br>3001 attcattagt ttttagctgg gtgaccttgg gcaagttaac agaggcaacc tctctgagcc<br>3061 tcagttgtct cttctgtaaa atgtgaaaag atgtgctccc accacatgcg cagggtctgt<br>3121 gattttacat gtatgtaaaa cacataggaa gctgacttag gaaaaagaga aaaccaaatt<br>3181 aaagttctga taatgaatat aatatagtca tcctttagta cccaagggga ttggttccag<br>3241 gactcctgaa gatcccaaaa tctgaggata ctcaagtccc ttatataaaa tggtgtaggc<br>3301 cgggcatggt ggctcacgcc tgtaatccca gtactttggg aggccgagtc aggtggatca<br>3361 cctgaggtca ggagttcaag accagcctgg ccaacatggc gaaaccccgt ctctactata<br>3421 aaatacaaaa aattagttgg gtgtggtggc gggtgcctgt aatcccagct actcaggagg<br>3481 ctgaggcagg agaatcgctt gaatctggga ggcagaggtt gctgtgagcc gagatcgtgc<br>3541 cactgcactc cagcctgagc aacagagcaa gactctatct caaaaataaa ataaaataaa<br>3601 ataaaataaa ataattaata aaatggtgta gtatttgcat ataacctatg cacattctcc<br>3661 catatagttt aatcatcttt agatacttat aatgcctaat acaatgtaaa tgctatgtaa<br>3721 atagttgttg ttatactgta ttgtttaggg aataacaata agaaaaacag tctgtacatg<br>3781 ttcactacag atgcaaccat tgttaagcct gactacatct ttttatctgc agttgattga<br>3841 atctatggat gtggaacctg tgcatatgga gggtcaactg tactataaat aatacgaata<br>3901 tgccaacatt atataatcat tgctttctgc aaaaaaaaaa aaaaa | |
| | | 1 M N S N L P A E N L T I A V N M T K T L<br>1 ATGAATTCTAATTTACCTGCAGAGAACTTAACCATTGCAGTCAATATGACCAAGACTTTG<br><br>21 P T A V T H G F N S T N D P P S M S I T<br>61 CCTACAGCAGTAACGCATGGATTTAATTCCACTAATGACCCACCTTCAATGTCAATTACA<br><br>41 R L F P A L L E C F G I V L C G Y I A G<br>121 AGGCTTTTTCCAGCCTTACTGGAATGCTTTGGCATTGTCCTTTGTGGCTACATAGCAGGA<br><br>61 R A N V I T S T Q A K G L G N F V S R F<br>181 AGGGCCAATGTCATAACATCAACCCAGGCCAAAGGACTAGGAAATTTTGTCTCCAGATTT<br><br>81 A L P A L L F K N M V V L N F S N V D W<br>241 GCACTTCCAGCTTTATTATTCAAAAACATGGTTGTACTTAATTTTTCCAATGTGGACTGG<br><br>101 S F L Y S I L I A K A S V F F I V C V L<br>301 TCCTTCCTATATAGTATCTTAATTGCCAAAGCTTCTGTATTTTTCATTGTATGTGTATTA<br><br>121 T L L V A S P D S R F S K A G L F P I F | SEQ ID NO:74 |

| Gene Name | GenBank Homology | DNA SEQUENCE / DEDUCED AMINO ACID SEQUENCE | SEQUENCE IDENTIFIER: |
|---|---|---|---|
| | | 361 ACCTTATTGGTTGCCAGTCCTGATAGTCGATTTAGCAAAGCTGGACTATTCCCTATTTTT<br><br>141 A T Q S N D F A L G Y P I V E A L Y Q T<br>421 GCTACACAAAGTAATGACTTTGCATTGGGATACCCTATAGTTGAAGCTTTATATCAAACT<br><br>161 T Y P E Y L Q Y I Y L V A P I S L M M L<br>481 ACATACCCAGAATATCTCCAGTACATTTATTTGGTGGCACCAATATCTCTTATGATGTTA<br><br>181 N P I G F I F C E I Q K W E D T Q N A S<br>541 AACCCTATAGGGTTTATTTTCTGTGAAATCCAAAAGTGGGAAGACACTCAAAATGCTTCT<br><br>201 Q N K I K I V G L G L L R V L Q N P I V<br>601 CAAAATAAAATAAAAATTGTGGGACTCGGACTCCTGCGTGTATTACAGAACCCAATAGTA<br><br>221 F M V F I G I A F N F I L D R K V P V Y<br>661 TTTATGGTCTTCATTGGCATCGCCTTCAATTTTATTCTTGATCGAAAGGTACCTGTATAT<br><br>241 V E N F L D G L G N S F S G S A L F Y L<br>721 GTCGAAAATTTTCTTGATGGACTTGGAAATTCTTTTTCTGGATCAGCCCTATTTTATCTT<br><br>261 G L T M V G K I K R L K K S A F V V L I<br>781 GGTCTCACGATGGTGGGAAAAATAAAGAGACTGAAGAAGTCGGCATTTGTAGTACTAATT<br><br>281 L L I T A K L L V L P L L C R E M V E L<br>841 CTTCTCATCACAGCTAAACTTCTGGTGCTGCCACTTCTGTGCAGAGAAATGGTGGAACTC<br><br>301 L D K G D S V V N H T S L S N Y A F L Y<br>901 TTGGACAAGGGCGACAGTGTGGTGAACCATACAAGTTTATCAAATTATGCATTTCTGTAT<br><br>321 G V F P V A P G V A I F A T Q F N M E V<br>961 GGTGTATTTCCTGTAGCACCAGGAGTGGCTATCTTTGCAACACAATTCAACATGGAAGTA<br><br>341 E I I T S G M V I S T F V S A P I M Y V<br>1021 GAAATTATAACCTCAGGGATGGTGATAAGCACATTTGTGTCTGCTCCCATCATGTACGTT<br><br>361 S A W L L T F P T M D P K P L A Y A I Q<br>1081 TCTGCCTGGTTACTGACCTTTCCCACTATGGACCCTAAGCCATTGGCATATGCCATCCAG<br><br>381 N V S F D I S I V S L I S L I W S L A I<br>1141 AATGTTAGTTTTGATATAAGTATTGTCAGCCTGATCTCCTTGATCTGGTCTCTGGCTATT<br><br>401 L L L S K K Y K Q L P H M L T T N L L I<br>1201 CTTCTTTTGAGTAAGAAATATAAACAGCTTCCTCATATGCTTACAACTAATTTACTCATT | |

| Gene Name | GenBank Homology | DNA SEQUENCE / DEDUCED AMINO ACID SEQUENCE | SEQUENCE IDENTIFIER: |
|---|---|---|---|
| | | 421 A Q S I V C A G M M I W N F V K E K N F<br>1261 GCTCAGTCTATTGTCTGTGCTGGAATGATGATATGGAATTTTGTTAAAGAAAAAAATTTT<br><br>441 V G Q I L V F V L L Y S S L Y S T Y L W<br>1321 GTTGGACAAATTTTGGTGTTTGTTCTATTGTACAGCTCCCTCTATAGCACCTACCTGTGG<br><br>461 T G L L A I S L F L L K K R E R V Q I P<br>1381 ACAGGCCTTCTAGCAATTTCTTTGTTTCTTTTGAAAAAGCGAGAGAGGGTACAAATTCCT<br><br>481 V G I I I I S G W G I P A L L V G V L L<br>1441 GTTGGAATAATCATAATATCTGGCTGGGGAATTCCTGCTCTCCTTGTTGGTGTTCTTTTG<br><br>501 I T G K H N G D S I D S A F F Y G K E Q<br>1501 ATAACTGGAAAACACAATGGAGATAGCATTGACTCAGCCTTCTTTTATGGAAAAGAACAG<br><br>521 M I T T A V T L F C S I L I A G I S L M<br>1561 ATGATCACCACAGCAGTCACCCTGTTCTGCAGCATCCTGATAGCTGGCATATCCCTCATG<br><br>541 C M N Q T A Q A G G Y E G F D Q S Q S H<br>1621 TGCATGAACCAGACTGCCCAAGCAGGAGGCTATGAAGGTTTCGATCAGTCTCAGAGCCAC<br><br>561 K V V E P G N T A F E E S P A P V N E P<br>1681 AAAGTGGTGGAGCCTGGAAATACTGCTTTTGAGGAGAGTCCAGCACCAGTAAATGAACCA<br><br>581 E L F T S S I P E T S C C S C S M G N G<br>1741 GAACTTTTTACAAGCTCTATTCCAGAAACAAGTTGCTGCTCCTGCTCCATGGGAAATGGT<br><br>601 E L H C P S I E P I A N T S T S E P V I<br>1801 GAATTACACTGCCCATCAATAGAGCCAATAGCAAACACAAGCACCAGTGAGCCTGTGATT<br><br>621 P S F E K N N H C V S R C N S Q S C I L<br>1861 CCTTCGTTTGAGAAAAACAATCATTGTGTGAGTCGCTGTAACTCCCAGAGCTGCATATTA<br><br>641 A Q E E E Q Y L Q S G D Q Q L T R H V L<br>1921 GCCCAGGAAGAAGAACAGTATCTACAGAGTGGAGACCAGCAACTGACCCGACATGTGTTG<br><br>661 L C L L L I I G L F A N L Y S C L W W L<br>1981 CTGTGTTTACTTCTCATCATTGGCCTGTTCGCTAATCTTTACAGTTGTTTATGGTGGCTA<br><br>681 F N Q E P G R L Y V E L Q F F C A V F N<br>2041 TTCAACCAAGAGCCTGGAAGACTTTATGTTGAGTTACAGTTTTTCTGTGCCGTGTTTAAC | |

| Gene Name | GenBank Homology | DNA SEQUENCE / DEDUCED AMINO ACID SEQUENCE | SEQUENCE IDENTIFIER: |
|---|---|---|---|
| | | 701 F G Q G F I S F G I F G L D K H L I I L<br>2101 TTTGGTCAGGGATTTATTTCATTTGGAATCTTTGGATTAGATAAACATTTAATCATCCTG<br><br>721 P F K R R L E F L W N N K D T A E N R D<br>2161 CCTTTCAAAAGAAGACTTGAATTCCTATGGAACAATAAAGACACAGCAGAAAACAGGGAT<br><br>741 S P V S E E I K M T C Q Q F I H Y H R D<br>2221 TCTCCTGTTTCAGAGGAAATAAAAATGACCTGTCAACAATTTATCCATTATCACCGTGAC<br><br>761 L C I R N I V K E R R C G A K T S A G T<br>2281 CTCTGTATCCGAAACATTGTCAAAGAAAGAAGGTGTGGTGCAAAGACTTCTGCTGGAACT<br><br>781 F C G C D L V S W L I E V G L A S D R G<br>2341 TTCTGTGGCTGTGACCTGGTGAGCTGGCTAATTGAAGTCGGCCTTGCCTCCGACCGTGGT<br><br>801 E A V I Y G D R L V Q G G V I Q H I T N<br>2401 GAAGCTGTGATATACGGAGACAGGCTGGTACAAGGGGGAGTCATCCAACATATTACCAAT<br><br>821 E Y E F R D E Y L F Y R F L Q K S P E Q<br>2461 GAGTATGAATTCCGGGATGAGTACTTGTTTTACAGATTTCTTCAAAAGAGTCCTGAACAG<br><br>841 S P P A I N A N T L Q Q E R Y K E I E H<br>2521 AGTCCTCCTGCTATTAATGCAAACACTCTCCAACAGGAAAGATATAAAGAAATTGAGCAT<br><br>861 S S P P S H S P K T -<br>2581 TCATCCCCACCCTCACATTCCCCTAAGACCTAA | |
| WBC017C08.bFSP_20011010.abd | No homology | 1 TTACAGTGTGAAGCCAGCTTCTGTTTCTGGGTCAGTTACCAGCAGCAGCCACAAGGTCTG<br>61 AAAGGTGTGAGGTGGATCAGTGATGCTGTACCACCTTCTGAATGAATCTGTCCAGCAGAG<br>121 TAATGTCTTTTTGTCTTATTCTTTGAGAATTTTTTTTCTTTTTCCCTTTGATTTTGGTAT<br>181 TTTCGTTTGAAGTCTGAATGCTTTTCAAAGTTCAAGGTTCTAAATCAATACTTGAGGATT<br>241 TGTAGAATAAAGTAACGGAACTGTGTTTGGCTCCCTAGATTTTCTGTTTATAAAAATAGA<br>301 TCTCAAAGATCAATAAAGATGAATGCCAGCATTTGCATAGTACTTTACGTGTGAAAGATA<br>361 AGTCACTGTGCTGCATCTTTAGTTATTTTAAGGCAGAGGATTCCTTTTTTAGTTTAATAA<br>421 TGAAAGTTTGAGTTAATTGGATATGGAAAACAGGAATCATTTCAGCAAAAATTTTTCCTG<br>481 TGGGAACACTATCATTTTAAGAAGATTAGTGATTTAATGTGTATTCTCTTACTCTTGAGC<br>541 TTTAAAAAAAGTCAAAAACATTTTCTGTATGGGGAGGATATTAATACCCGTATTTTAGTT<br>601 GTCATAGAGTAATTTATACATACTGTACAAAGATATAGTTACACTCCCATATGAAGATAT<br>661 AGTTAATGCCATCTTTGGCCAAGTGTTAGGAGTTTTGGCTTTGTAGTCAGGCAAGCCTAT<br>721 GTTGGGTCCTGGCTGTTGCCACTTAATAGCGGTGTGACTTTGCTTAGGTTTCTCAACTTC<br>781 GCTGAGCCTCATTATCCACATCTGTAAATATAAGAATGCTACCTTTTTAGGTTGTCCTGA<br>841 AGATTTAAATGAATAAATGTGAAGTATCTAACCTCAAATAATAATAGCAGTTACTTAACA | SEQ ID NO:75 |

| Gene Name | GenBank Homology | DNA SEQUENCE / DEDUCED AMINO ACID SEQUENCE | SEQUENCE IDENTIFIER: |
|---|---|---|---|
| | | 901 TAGCTTATA<br><br>1 TTCCTTTTTTAGTTTAATAATGAAAGTTTGAGTTAATTGGATATGGAAAACAGGAATCAT<br>61 TTCAGCGAAAATTTTTCCTGTGGGAACACTATCATTTTAAGAAGATTAGTGATTTAATGT<br>121 GTATTCTCTTACTCTTGAGCTTTAAAAAAAGTCAAAAACATTTTCTGTATGGGGAGGATA<br>181 TTAATACCCGTATTTTAGTTGTCATAGAGTAATTTATACATACTGTACAAAGATATAGTT<br>241 ACACTCCCATATGAAGATATAGTTAATGCCATCTTTGGCCAAGTGTTAGGAGTTTTGGCT<br>301 TTGTAGTCAGGCAAGCCTATGTTGGGTCCTGGCTGTTGCCACTTAATAGCGGTGTGACTT<br>361 TGCTTAGGTTTCTCAACTTCGCTGAGCCTCATTATCCACATCTGTAAATATAAGAATGCT<br>421 ACCTTTTTAGGTTGTCCTGAGGATTTAAATGAATAAATGTGAAGTATCTAACTCAAATAA<br>481 TAATAGCAGTTACTTAACATAGCTTATATGCCAGACAATTTTATTAATTTATTTAATCCT<br>541 TATAACAATCCTGTGAAGTTGGTACAGTTATTTTCCTCATTTTTACAGATGAAGAAACTG<br>601 AGGCTAATGAAAGGTTATATAACTTATGCAAGCTCGTACAGCTAATAAGTACATAACACT<br>661 CGACAAATGCTGACTGTTGTCATAAAAAAAAAA | SEQ ID NO:76 |
| WBC018B05.bFSP_20 011110.abd | Human transcriptional repressor (CTCF) mRNA. | 1 TGTGTCTGAGCCTGTGGAGCGATTAAACCGTGCGCGGAGCTGCTTCTTTGGCGGCAGCGG<br>61 CGGCGGCGGTGGCCGGTGCGGACGCGCGGAGCTCGCCGGAGACGCCGGGTGGCCGGAGCC<br>121 GTGGAGCGGCGGCGGAGCGGGCGCCGCGGGGGGTGTGGCGCGGAGAATGATTACGGACCT<br>181 GAAGCCAAAGAACAAGATGCGCTAGTGGACAGATTGCTGACCAGGGGCTTGAGAGCTGGG<br>241 TTCTATTTTCCCTCCTCAAACTGACTTTGCAGCCACGGAGAGGCAGGGGAAATGGAAGGT<br>301 GATGCAGTCGAAGCCATTGTGGAGGAGTCCGAAACTTTTATTAAAGGAAAGGAGAGAAAG<br>361 ACTTACCAGAGACGCCGGGAAGGGGGCCAGGAAGAAGATGCCTGCCACTTACCCCAGAAC<br>421 CAGACGGATGGGGGTGAGGTGGTCCAGGATGTCAACAGCAGTGTACAGATGGTGATGATG<br>481 GAACAGCTGGACCCCACCCTTCTTCAGATGAAGACTGAAGTAATGGAGGGCACAGTGGCT<br>541 CCAGAAGCAGAGGCTGCTGTGGACGATACCCAGATTATAACTTTACAGGTTGTAAATATG<br>601 GAGGAACAGCCCATAAACATAGGAGAACTTCAGCTTGTTCAAGTACCTGTTCCTGTGACT<br>661 GTACCTGTTGCTACCACTTCAGTAGAAGAACTTCAGGGGGCTTATGAAAATGAAGTGTCT<br>721 AAAGAGGGCCTTGCGGAAAGTGAACCCATGATATGCCACACCCTACCTTTGCCTGAAGGG<br>781 TTTCAGGTGGTTAAAGTGGGGGCCAATGGAGAGGTGGAGACACTAGAACAAGGGGAACTT<br>841 CCACCCCAGGAAGATCCTAGTTGGCAAAAAGACCCAGACTATCAGCCACCAGCCAAAAAA<br>901 ACAAAGAAAACCAAAAAGAGCAAACTGCGTTATACAGAGGAGGGCAAAGATGTAGATGTG<br>961 TCTGTCTACGATTTTGAGGAAGAACAGCAGGAGGGTCTGCTATCAGAGGTTAATGCGGAG<br>1021 AAAGTGGTTGGTAATATGAAGCCTCCAAAGCCAACAAAAATTAAAAAGAAAGGTGTAAAG<br>1081 AAGACATTCCAGTGTGAGCTTTGCAGTTACACGTGTCCACGGCGTTCAAATTTGGATCGT<br>1141 CACATGAAAAGCCACACTGATGAGAGACCACACAAGTGCCATCTCTGTGGCAGGGCATTC<br>1201 AGAACAGTCACCCTCCTGAGGAATCACCTTAACACACACACAGGTACTCGTCCTCACAAG<br>1261 TGCCCAGACTGCGACATGGCCTTTGTGACCAGTGGAGAATTGGTTCGGCATCGTCGTTAC<br>1321 AAACACACCCACGAGAAGCCATTCAAGTGTTCCATGTGCGATTACGCCAGTGTAGAAGTC<br>1381 AGCAAATTAAAACGTCACATTCGCTCTCATACTGGAGAGCGTCCGTTTCAGTGCAGTTTG<br>1441 TGCAGTTATGCCAGCAGGGACACATACAAGCTGAAAAGGCACATGAGAACCCATTCAGGG<br>1501 GAAAAGCCTTATGAATGTTATATTTGTCATGCTCGGTTTACCCAAAGTGGTACCATGAAG<br>1561 ATGCACATTTTACAGAAGCACACAGAAAATGTGGCCAAATTTCACTGTCCCCACTGTGAC | SEQ ID NO:77 |

| Gene Name | GenBank Homology | DNA SEQUENCE / DEDUCED AMINO ACID SEQUENCE | SEQUENCE IDENTIFIER: |
|---|---|---|---|
| | | 1621 ACAGTCATAGCCCGAAAAAGTGATTTGGGTGTCCACTTGAGAAAGCAGCATTCCTATATT<br>1681 GAGCAAGGCAAGAAATGCCGATACTGCGATGCTGTTTTTCATGAGCGTTATGCCCTCATC<br>1741 CAGCATCAGAAGTCACACAAGAACGAGAAGCGCTTTAAGTGTGACCAGTGTGATTATGCT<br>1801 TGCAGACAGGAGAGGCACATGATAATGCACAAGCGCACTCACACTGGGGAGAAGCCTTAT<br>1861 GCCTGCAGCCACTGCGACAAGACCTTCCGCCAGAAACAGCTCCTCGACATGCACTTCAAA<br>1921 CGCTATCATGACCCCAACTTTGTTCCTGCGGCCTTCGTCTGTTCTAAGTGTGGAAAACA<br>1981 TTTACACGCCGGAATACCATGGCAAGACATGCTGATAATTGTGCTGGTCCAGATGGCGTA<br>2041 GAGGGGGAAAATGGAGGAGAAACGAAGAAGAGTAAACGTGGAAGAAAAAGAAAGATGCGA<br>2101 TCTAAAAAAGAAGATTCCTCTGACAGTGAAAATGCTGAGCCAGACCTGGATGACAATGAG<br>2161 GATGAGGAGGAGCCTGCCGTGGAAATTGAACCTGAGCCAGAGCCTCAGCCTGTTACCCCA<br>2221 GCCCCACCACCCGCCAAGAAGCGGAGAGGACGCCCCCCTGGCAGAACCAACCAGCCCAAA<br>2281 CAGACCCAGCCCATCATTCAGGTTGAAGACCAGAGTACAGGTGCAATTGAGAACATTATA<br>2341 GTTGAAGTCAAAAAAGAGCCAGATGCAGAGCCCGTGGAGGGGGAAGAAGAGGAGGCCCAG<br>2401 CCAGCTGCCACAGACGCCCCCAATGGAGACCTCACATCCAAGATGATCCTCACCATGAT<br>2461 GGACCGGTGATGGCGGAGCCTTGTGCGTCGCCAGGACTTCTCTGGGCTGTGTTTAAACGG<br>2521 CCCGCATCTTAATTTTTCTCCCTTCTTTCTTTTTTTGGCTTTGGGAAAAGCATCATTTTA<br>2581 CCAAACATACCGAGAACGAAAACTTCAAGGATGATGTTAGAAAAAAATGTGATTTAACTA<br>2641 GAACTTGCTGTCTGATGTTAGCAAATCATGGAATGTTCTGAGTCCCTGAGGGTTTACTGT<br>2701 GAAGTGCTGAGGACAGTGTTGACAACTAACTCGTTTTCCTAGATGGAAACGGAGACATTG<br>2761 ACCCCTCCCTCCATGTGGTAAACCACTCCAGAATGGCCACCAGGCTTCCCAGAGTTCTAT<br>2821 GGTCTTCTTCCCAAGAGAGTTTTTAATTGTAAATGCATACTTGGGAAGGACTTAGAGTTT<br>2881 TAAACTGTTTTTTGCTTTTGCTTTTCCCTGACTCCCTTTGCTTGGAGTCAGCTGCACACC<br>2941 AGTAGTATGGCATGCTACGATCAGGTTCTGTCCTGAAAGCTTTGCCTCTTTCTTGGCAAA<br>3001 GTTTCTGGTATGGTCAAGCTTGTAAATAACTTTTTTTACATTTTAATCTTTTCCATTAAT<br>3061 TAAGAGGTTGAAAAGAAGTGCAGTGTAAGAAAACCCAGCATTTTAATTACTTGCAAATTA<br>3121 AGTTACCACAGACTCTGTAGTGTGTCAGTGTTGACAAGAAATTGGATCACAATCATGTAG<br>3181 CAGAACGGCACCCAGACCACTGCCCACCAGTGACGGACATGCACGTGGCAGATCGTGAAT<br>3241 TCCAGCCCGCGGAGCCAGCATTTGAACCTTGTACAATTAACTTTCAGTTATGATTTCCCA<br>3301 TCGACATTTTCTTTGCTCTGTTTGTAGCTGAATTGTTTGTGTTCTGTAAATCCTCTGTTA<br>3361 AAGCAGAAGGGTGATTATGAGCGGGTCACAGCAGCCCTTAAAAGCTGGGCCAGGAAATTC<br>3421 ACTAGGTCAGTAATTTAAACTTTGGATCTTCAAAAAAAAATAATAATGTGAAGCAAAACC<br>3481 AACTAAAAAGTGATTCTTGCACATGAACTGTCACACGTTTAAAATGTGTTTTTTAGAGAG<br>3541 CCTCAGTCTTGCTGATTTCAAACACTTTTTTCTTCTGTGTATTGCTTTTAAGAGAGCCAT<br>3601 CAGTTAGCTATCAGACTCTAGGTTGATGCATTTTGTACTTAGCTGTACTGTGTGATATTT<br>3661 TTCATTATTTTAGGATGCCAACAAGAGACCTGTAATAAAATATGTGATGGGGTTGAGAGC<br>3721 TGGGGAGGAGGATCTACTGCTGTACAGCTAATAAATCATACGGATTAACAAGTGA | |
| | | 1 M E G D A V E A I V E E S E T F I K G K<br>1 ATGGAAGGTGATGCAGTCGAAGCCATTGTGGAGGAGTCCGAAACTTTTATTAAAGGAAAG<br><br>21 E R K T Y Q R R R E G G Q E E D A C H L<br>61 GAGAGAAAGACTTACCAGAGACGCCGGGAAGGGGGCCAGGAAGAAGATGCCTGCCACTTA | SEQ ID NO:78 |

| Gene Name | GenBank Homology | DNA SEQUENCE / DEDUCED AMINO ACID SEQUENCE | SEQUENCE IDENTIFIER: |
|---|---|---|---|
| | | 41 P Q N Q T D G G E V V Q D V N S S V Q M<br>121 CCCCAGAACCAGACGGATGGGGGTGAGGTGGTCCAGGATGTCAACAGCAGTGTACAGATG<br><br>61 V M M E Q L D P T L L Q M K T E V M E G<br>181 GTGATGATGGAACAGCTGGACCCCACCCTTCTTCAGATGAAGACTGAAGTAATGGAGGGC<br><br>81 T V A P E A E A A V D D T Q I I T L Q V<br>241 ACAGTGGCTCCAGAAGCAGAGGCTGCTGTGGACGATACCCAGATTATAACTTTACAGGTT<br><br>101 V N M E E Q P I N I G E L Q L V Q V P V<br>301 GTAAATATGGAGGAACAGCCCATAAACATAGGAGAACTTCAGCTTGTTCAAGTACCTGTT<br><br>121 P V T V P V A T T S V E E L Q G A Y E N<br>361 CCTGTGACTGTACCTGTTGCTACCACTTCAGTAGAAGAACTTCAGGGGGCTTATGAAAAT<br><br>141 E V S K E G L A E S E P M I C H T L P L<br>421 GAAGTGTCTAAAGAGGGCCTTGCGGAAAGTGAACCCATGATATGCCACACCCTACCTTTG<br><br>161 P E G F Q V V K V G A N G E V E T L E Q<br>481 CCTGAAGGGTTTCAGGTGGTTAAAGTGGGGGCCAATGGAGAGGTGGAGACACTAGAACAA<br><br>181 G E L P P Q E D P S W Q K D P D Y Q P P<br>541 GGGGAACTTCCACCCCAGGAAGATCCTAGTTGGCAAAAAGACCCAGACTATCAGCCACCA<br><br>201 A K K T K K T K K S K L R Y T E E G K D<br>601 GCCAAAAAAACAAAGAAAACCAAAAAGAGCAAACTGCGTTATACAGAGGAGGGCAAAGAT<br><br>221 V D V S V Y D F E E E Q Q E G L L S E V<br>661 GTAGATGTGTCTGTCTACGATTTTGAGGAAGAACAGCAGGAGGGTCTGCTATCAGAGGTT<br><br>241 N A E K V V G N M K P P K P T K I K K K<br>721 AATGCGGAGAAAGTGGTTGGTAATATGAAGCCTCCAAAGCCAACAAAAATTAAAAAGAAA<br><br>261 G V K K T F Q C E L C S Y T C P R R S N<br>781 GGTGTAAAGAAGACATTCCAGTGTGAGCTTTGCAGTTACACGTGTCCACGGCGTTCAAAT<br><br>281 L D R H M K S H T D E R P H K C H L C G<br>841 TTGGATCGTCACATGAAAAGCCACACTGATGAGAGACCACACAAGTGCCATCTCTGTGGC<br><br>301 R A F R T V T L L R N H L N T H T G T R<br>901 AGGGCATTCAGAACAGTCACCCTCCTGAGGAATCACCTTAACACACACACAGGTACTCGT<br><br>321 P H K C P D C D M A F V T S G E L V R H | |

| Gene Name | GenBank Homology | DNA SEQUENCE / DEDUCED AMINO ACID SEQUENCE | SEQUENCE IDENTIFIER: |
|---|---|---|---|
| | | 961 CCTCACAAGTGCCCAGACTGCGACATGGCCTTTGTGACCAGTGGAGAATTGGTTCGGCAT | |
| | | 341 R R Y K H T H E K P F K C S M C D Y A S | |
| | | 1021 CGTCGTTACAAACACACCCACGAGAAGCCATTCAAGTGTTCCATGTGCGATTACGCCAGT | |
| | | 361 V E V S K L K R H I R S H T G E R P F Q | |
| | | 1081 GTAGAAGTCAGCAAATTAAAACGTCACATTCGCTCTCATACTGGAGAGCGTCCGTTTCAG | |
| | | 381 C S L C S Y A S R D T Y K L K R H M R T | |
| | | 1141 TGCAGTTTGTGCAGTTATGCCAGCAGGGACACATACAAGCTGAAAAGGCACATGAGAACC | |
| | | 401 H S G E K P Y E C Y I C H A R F T Q S G | |
| | | 1201 CATTCAGGGGAAAAGCCTTATGAATGTTATATTTGTCATGCTCGGTTTACCCAAAGTGGT | |
| | | 421 T M K M H I L Q K H T E N V A K F H C P | |
| | | 1261 ACCATGAAGATGCACATTTTACAGAAGCACACAGAAAATGTGGCCAAATTTCACTGTCCC | |
| | | 441 H C D T V I A R K S D L G V H L R K Q H | |
| | | 1321 CACTGTGACACAGTCATAGCCCGAAAAAGTGATTTGGGTGTCCACTTGAGAAAGCAGCAT | |
| | | 461 S Y I E Q G K K C R Y C D A V F H E R Y | |
| | | 1381 TCCTATATTGAGCAAGGCAAGAAATGCCGATACTGCGATGCTGTTTTTCATGAGCGTTAT | |
| | | 481 A L I Q H Q K S H K N E K R F K C D Q C | |
| | | 1441 GCCCTCATCCAGCATCAGAAGTCACACAAGAACGAGAAGCGCTTTAAGTGTGACCAGTGT | |
| | | 501 D Y A C R Q E R H M I M H K R T H T G E | |
| | | 1501 GATTATGCTTGCAGACAGGAGAGGCACATGATAATGCACAAGCGCACTCACACTGGGGAG | |
| | | 521 K P Y A C S H C D K T F R Q K Q L L D M | |
| | | 1561 AAGCCTTATGCCTGCAGCCACTGCGACAAGACCTTCCGCCAGAAACAGCTCCTCGACATG | |
| | | 541 H F K R Y H D P N F V P A A F V C S K C | |
| | | 1621 CACTTCAAACGCTATCATGACCCCAACTTTGTTCCTGCGGCCTTCGTCTGTTCTAAGTGT | |
| | | 561 G K T F T R R N T M A R H A D N C A G P | |
| | | 1681 GGGAAAACATTTACACGCCGGAATACCATGGCAAGACATGCTGATAATTGTGCTGGTCCA | |
| | | 581 D G V E G E N G G E T K K S K R G R K R | |
| | | 1741 GATGGCGTAGAGGGGGAAAATGGAGGAGAAACGAAGAAGAGTAAACGTGGAAGAAAAAGA | |
| | | 601 K M R S K K E D S S D S E N A E P D L D | |
| | | 1801 AAGATGCGATCTAAAAAAGAAGATTCCTCTGACAGTGAAAATGCTGAGCCAGACCTGGAT | |

| Gene Name | GenBank Homology | DNA SEQUENCE / DEDUCED AMINO ACID SEQUENCE | SEQUENCE IDENTIFIER: |
|---|---|---|---|
| | | 621 D N E D E E E P A V E I E P E P E P Q P<br>1861 GACAATGAGGATGAGGAGGAGCCTGCCGTGGAAATTGAACCTGAGCCAGAGCCTCAGCCT<br><br>641 V T P A P P P A K K R R G R P P G R T N<br>1921 GTTACCCCAGCCCCACCACCCGCCAAGAAGCGGAGAGGACGCCCCCCTGGCAGAACCAAC<br><br>661 Q P K Q T Q P I I Q V E D Q S T G A I E<br>1981 CAGCCCAAACAGACCCAGCCCATCATTCAGGTTGAAGACCAGAGTACAGGTGCAATTGAG<br><br>681 N I I V E V K K E P.D A E P V E G E E E<br>2041 AACATTATAGTTGAAGTCAAAAAAGAGCCAGATGCAGAGCCCGTGGAGGGGGAAGAAGAG<br><br>701 E A Q P A A T D A P N G R P H I Q D D P<br>2101 GAGGCCCAGCCAGCTGCCACAGACGCCCCCAATGGGAGACCTCACATCCAAGATGATCCT<br><br>721 H H D G P V M A E P C A S P G L L W A V<br>2161 CACCATGATGGACCGGTGATGGCGGAGCCTTGTGCGTCGCCAGGACTTCTCTGGGCTGTG<br><br>741 F K R P A S -<br>2221 TTTAAACGGCCCGCATCTTAA | |
| WBC018D03.bFSP_20 011110.abd | No homology | 1 ATCTGTATTAGCCAGAGCATTGGGCTTCCAAAATACAGCCTTCTGTTCATTAGATCTTGA<br>61 GGATTTTAATAGATTGACACCCCATACCTTATAGAATTGGAGTTGGAATATGATTTCTCA<br>121 AAAATGAATTTGATAATTTCAAGTATAATATGCATATGTGCCAAAAGAATGCTGCAAATA<br>181 TAATTATGGGTAGCCAAATTTGGGGTTATGTGAACATTTTATATTGCTCATTTTGTTTAT<br>241 GTAATTTTTTTTCTCGGAAAAACAGTTGTTTTTCCTCTCAGAAGTGTTTAGGCATTATTG<br>301 CACGTGAGTTTCAGTTCTGCTACATGAACTGTTAGCTGTTTTGTTTGAAGCAACACAGCA<br>361 CTAAAGAACCAGTATTGTTGACTAATATGCTTCATCTTTCAGTTTGTATGGTTGTAATTC<br>421 AGTTTACCACATCTAGTTTGGTGTTTGATGATCTGAAAAATATTTCACAGTGAGGGCAAT<br>481 GCTCAGTGCCTGGAAGAAGCAGACAGTCTTTATTGGTCTGACAATATTTGATGCAACTAC<br>541 AAGAGCTCCATGGACATGACCCTTGCACATTTGTCTGTAATACACAATGCTGTAATTGAA<br>601 TTGTTTCCTATGTTGATTCTCTCATGTACCCAAGTATAAAAAATTGGTAAGTTTCTCTTG<br>661 TCTTCAAAGTACCATTAGGATTTTATTTCCTGAATTTAAAATGAATATTAAAATTTTTGT<br>721 AATGGCGTTCTTTAACCTGCCCTAAATTTTTATAGAAAAGCCTTGCCAAATATTGTTGTC<br>781 CTCATACTTAAAAGTTTCCACA | SEQ ID NO:79 |
| | | 1 CCCATTCTAATTAAGAAAGAGCTTTCCCAAAATAGTCTCTAATTTCCAGTTTTTGTAAAA<br>61 GAAGTTCGTTTTTGGAGTAAAAAGTATTTTCATATCTGTTTGGGGGAAGTTAACGCATCA<br>121 GGAAAGAATATTATTAGGTGTTTAAACCCTGAAACAAACATATACTGATGCATAACTTAG<br>181 TAGATCACCGAAACTAACTTGCTGAAATCTTTGGTTTTGTCCTGTGAGACCAGTGCTTGA<br>241 CATCGTCTGTTCGCCTGAGCCCAGTTGGTGGCTCCAGTTTGCTTTTCTCTTCGGTCTCGT | SEQ ID NO:80 |

| Gene Name | GenBank Homology | DNA SEQUENCE / DEDUCED AMINO ACID SEQUENCE | SEQUENCE IDENTIFIER: |
|---|---|---|---|
| | | 301 GGTAGCAGCCTTGGTCGTTTTCGTTGGAGGTTGGAGAGGAGCTGAGGAGAAAGTGCTCTT<br>361 CGGGGTTTTGATTCCTCTGCTAGGACGCTGCCTAAACCAGCTCATCTCAATGTCCTGTTG<br>421 ACTTTATTATTATTCCGGGTAGTTGAAAGAAAAGAAAATGGATGGACGTCATTAAAACCA<br>481 GCTCAAAAATATATTCTTGTCAGTAAAGATTTCTTGTCGAGATGTCTTGGATTGCACTTT<br>541 CGTGGAGGGAAAACAGTGTAAATAGTTAAAGAATGTTGATAAAATTGAAACATTTGGTTA<br>601 TGGAATTGTGTGTGGTGTTAGAAGGTTTCTGTTTGTGAAATGTCTGTATTAAAATAATAA<br>661 ATTTCAGAAACT | |
| WBC018F03.bFSP_20 011110.abd | Human mRNA for integrin alpha-4 subunit | 1 GATTGTTGAAGCTTCTTTAAACCATCCTGAGTCAGTTAATAGAACGAATTTTGACTGTAT<br>61 TGAAAATGGATTGCCTTCTGTGTGCATGGATCTGAAACTCTGTTTCTCATACAAGGGCAA<br>121 AGAGGTTCCAGGTTATATTGTTTTGTTTTATAACATGAGTCTGGATGTGAACCGAAAGGC<br>181 GGAGTCTCCATCGAGATTTTACTTTTCTTCAAATGGAACCTCTGATGTGATTACAGGAAG<br>241 CATGAAAGTATCAAGCAGAGCGGCTAACTGCAGAACCCATCAAGCATTTATGCGGAAAGA<br>301 TGTGCGGGACATCCTCACCCCAATTCAGATCAAAGCTGCTTACCACCTTGGGCATCACGT<br>361 CATCAGTAAACGAAGCACAGAGGAATTCCCGCCCCTTCAGCCAATTCTTCAGCAGAAGAG<br>421 AGAAAAGGATGTGATTGAAAAAACAATAAACTTTGCAAGGTTTTGTGCCCATGAAAATTG<br>481 TTCTGCAGATTTACAGCTTTCTGCAAAAATTGGATTTTTGAAGCCACATGAAAATAAAAC<br>541 CTATCTTGCTGTTGGAAATATGAAGACGTTAATGTTGAATGTGTCCTTGTTTAATGCTGG<br>601 AGACGATGCATTATGAAACGACTCTGCATATCAAACTACCCACTGGTCTTTATTTCATTA<br>661 AGATTTTAGATCTGGAAGAGAAACAAATAAACTGTGAAGTCACAGACAGCTCTTGCATAG<br>721 TAAAACTTGACTGCAATGTTGGTTATATATATGTAGATCATCTCTCAAGGATAGACGTTA<br>781 GCTTTCTCCT | SEQ ID NO:81 |
| | | 1 GTAGGATCCATGGTAGAAAATCAAACCCAAATTCAGAATCGCTTTCTCAAAGATGATCCC<br>61 TGAGGAACATTAGTTCTTTCTTATTCAGAAATGATGGCATTTCAGATTGCTGGATAACAT<br>121 GACTTTCAGGAGCAAGAGGAACACAGCTCCCAATTAGGCCAGATTCTGCCCAGTTAGATA<br>181 TTTTGCACAGTTTAAAATAAAATTTGTTATAAAGTCTTAAATTACCTGTATTATGAATTT<br>241 TAAAACCCTACCACTTTAAGAAAATCAACTTTCAGAGAAGATTCACAAAATTGGAACCAT<br>301 AACTTTTAAAAAAAAAAAGATTGGGTTTCTTTTTGGGCAAGTAGTCTAGGTATTGGAGAG<br>361 ATTTTGAAAAAAAAAATAGTTCTATTTCTGGTTATATCATTAACTAGCTGTGTAATTTTG<br>421 AAATTTAACCTCACTGGATTGGCACCTCCCCAGGAAAATCAGGGCATTCTTATGTGAACT<br>481 TGTGGTTTGTCTGTACCAGGCCATAGGAAACTTAACATTAGAATTGAAAGACTCAAATCC<br>541 TGAAAAATCAAAAAATCCAAATTATTTCACTTTGGAATCATCTAGTTAAAATTGATATAC<br>601 TGATGTTTAAATTTTAAGTACCTGCAATGTTTACTTTCAGTTCTAATAAATACCTTCTAA<br>661 AAAACCAAAAAAAAAAAAAAAA | SEQ ID NO:82 |
| WBC019B05.bFSP_20 011110.abd | No Homology | 1 TCCTTCCCCTCCTACCTGAGCCATGAAAAGTACGGAGACGTCCACTGTCATGGCAGAACA<br>61 TGGGCCATAAAGCAAATTCAACAAGGGGTGTTCTGTTTTAATAGGACCTCAACCAGTTCC<br>121 ATGAACTGAGTGAAGGACGTTCATTTTAAAAGTCATTTAATAATAAGCTTAGTTAAACTC<br>181 TTTCACCTATTCTCCCAAGACCTATTGGCATTGTTAAAAACCAAGTATATCAAATATCTA<br>241 ACTAAGGATGTAGTTAACCTTATTAAATATTGATTAGAATTGTTCTGTAATATTACTGAA | SEQ ID NO:83 |

| Gene Name | GenBank Homology | DNA SEQUENCE / DEDUCED AMINO ACID SEQUENCE | SEQUENCE IDENTIFIER: |
|---|---|---|---|
| | | 301 TTTGTAAGATCTTTAGCAAAGATTTTTGAGCAATTTATAAATATAGAGCAAATGTTTCTG<br>361 TTTACTGCACTTTTTGTAATTGAAGGTGATAAATTCTCAAGCCATGGTTATTGGCTTCCA<br>421 TGCACTGCATATTTACCCACAATTCTAGACAGTTTCCATTTTTATGGAAGAGTTGCTATT<br>481 ACCTTAATCATAAATGCAATCATGTGGTTAATGAGAGCTAATGCTAATAGTTAACCTTTT<br>541 AAAGTGAATTGGCCACAGTTTAGGAAGAAATCTCTTTTAATACAGAGTATGTCATTCCTT<br>601 ATTGCTTCACTTAGAAGGAGACTGAAAGCTTTGTTTTTTTAAGCACTATTTAGTATCCT<br>661 AAGCTTCCTGAGGGTAGAGATTGTATCTCTTTTTGTCTGCACAATGGCCAGCACATGTCA<br>721 GCATTTGATCATTGTTAAATGACAACAAGTGTTCCCCAATTCCAACTTTTTTCCTGGATT<br>781 CTTTTGGTAAATTTATAAAATATGGCAAGCTGTATGGTTAACACTTTCTTCTACAACCAA<br>841 GTATTAAAGCCACATTTGAAAAGACCACATGAAATGCTGATTCCTAATTGTGTGTAGGTC<br>901 TTGAGGATTAAGCACACAAATTTCACCAAACTCTGTGAGTAACAAACTCAGCCTTCTGTA<br>961 AATATACATGCAAGTCTGGAAACGATAATACTGTACCTATAAATATATGCTGTCTGTTTT<br>1021 GTGTACAGTATGTAAAAACTCCTTTTCTGCCACACTAAAAATGCAAGCCATTTGTGGGAA<br>1081 TCCTAAAAATAGTATTATACTAAAACTTTGCTAATGATCTTTACTAGAGGATCATCTGAC<br>1141 TTTTCACTTACATTGGGTTTTCTTTTCAACTCACTCTTACAGTAGTCTGCTTATTTCCAG<br>1201 CTGTTTATTTTATTGAGTCCTGAATTTAAAAAAAAAATATTTTGATTCATTTTGTAAATA<br>1261 CAAGCTGTAAAAAAAGAGAGATTTAATGTTGTCTTTTAAATACTCCGATTTTCATTCTAA<br>1321 TATGAATGTTGTTATATTGTACTTAGAAACTGTACCTTTAATATTACATTACCTTTATTA<br>1381 AAAGTGCATTGAACACATCAATTTTAGATGTGCTTTATGTACTGTTATCCTATAATAAAA<br>1441 CTTCAGCTTCTAATGGAAAAAAAAAAAAAAAA | |
| WBC019C11.bFSP_20 011110.abd | Human proline-rich protein (PRP) mRNA. | 1 AAAACTCTGATCTGGGGAGGAACCAGGACTACATAGATCAAGGCAGTTTTCTTCTTTGAG<br>61 AAACTATCCCAGATATCATCATAGAGTCTTCTGCTCTTCCTCAACTACCAAAGAAAAACA<br>121 TCAGCGAAGCAGCAGGCCATGCACCCCCCAAAAACTCCATCTGGGGCTCTTCATAGAAAA<br>181 AGGAAAATGGCAGCCTGGCCCTTCTCCAGGCTGTGGAAAGTCTCTGATCCAATTCTCTTC<br>241 CAAATGACCTTGATCGCTGCTCTGTTGCCTGCTGTTCTTGGCAATTGTGGTCCTCCACCC<br>301 ACTTTATCATTTGCTGCCCCGATGGATATTACGTTGACTGAGACACGCTTCAAAACTGGA<br>361 ACTACTCTGAAATACACCTGCCTCCCTGGCTACGTCAGATCCCATTCAACTCAGACGCTT<br>421 ACCTGTAATTCTGATGGCGAATGGGTGTATAACACCTTCTGTATCTACAAACGATGCAGA<br>481 CACCCAGGAGAGTTACGTAATGGGCAAGTAGAGATTAAGACAGATTTATCTTTTGGATCA<br>541 CAAATAGAATTCAGCTGTTCAGAAGGATTTTTCTTAATTGGCTCAACCACTAGTCGTTGT<br>601 GAAGTCCAAGATAGAGGAGTTGACTGGAGTGATCCTCTCCCAGTATGTGTAATTGCCAAG<br>661 TGTGAGGCCCCTCCAGCCATCAGCAATGGGAAGCACAGTGGTGGAGATGAAGACGTCTAC<br>721 ACATATGGCTCTTCTGTCACCTACAGCTGTGACCCTCACTTCTCAATGATAGGCAAAGCC<br>781 TCCATTTCCTGCACAGTGGAGAATAAAACGATAGGTGTCTGGAGCCCAAGCCCTCCTACC<br>841 TGTGAAAAAATCACCTGTCGCAAGCCAGATGTTTCACATGGGGAAATGGTCTCTGGATTT<br>901 GGACCCCTCTATACTTACAAAGACGCTATTGTGTTGGACTGCAAGAAGGGTTATGTCCTC<br>961 ACAGGCAGCAGTTTAATCCACTGTGAAGCTGATAACAACTGGGACCCTCCTCCTCCTGTT<br>1021 TGTGAGCTCAATGGTTGTACCAACTTACCAGACATCCCAAATGCCTTCTGGGAACGATAC<br>1081 AGGTATCAGAGGCCAACAAAAGAGGACGTGTATAATGTTGGGACTGTGTTGAAGTACCAT<br>1141 TGCCTGACTGGCTATAAACCTGCTTCAGATAAACCCACGGCTGTGATTTGTCAGGAAGAT<br>1201 TTCAGGTGGACTCCATACACGGAATGTGAGGAGGTATGTTGCCCAGTACCAGAGCTGAAG | SEQ ID NO:84 |

| Gene Name | GenBank Homology | DNA SEQUENCE / DEDUCED AMINO ACID SEQUENCE | SEQUENCE IDENTIFIER: |
|---|---|---|---|
| | | 1261 AACGGTGAAATCACTCAACACAGGAAAAGTCGTCCTGCCAATCACTGTGTTTATTTCTAT<br>1321 GGAGATGAGATTTCATTTTCATGTCATGAGACCAGTAGGTTTTCAGCTATATGCCAAGGA<br>1381 GATGGCACGTGGAGTCCCCGAACACCATCATGTGGAGACATTTGCAATTTTCCTCCTAAA<br>1441 ATTGCCCATGGGCATTATAAACAATCTAGTTCATACAGCTTTTTCAAAGAAGAGATTATA<br>1501 TATGAATGTGATAAAGGCTACATTCTGGTCGGACAGGCGAAACTCTCCTGCAGTTATTCA<br>1561 CACTGGTCATCTCCAGCTCCTCAATGTAAAGCTCTGTGTCCGAAACCACAGATAGAGCAT<br>1621 GGACGGCTGTCTGTGGTTAAGGATCAGTATATTACACCTGAAAATGTCACCATCCAGTGT<br>1681 GACCCTCACTATCGTTTGGTTGGTCTCCAAAGTATCACTTGCTCAGAGAACAGCACCTGG<br>1741 TACCCCGAGGTGCCCATGTGTGAGTGGGAGATCGCTGAAGGCTGTGAGCAAGTGCTCGCA<br>1801 GGCAGAAAAATCATGCAGTGTCTCCCAAAGCCGGAGGATGTGAGAACGGCCCTGGAGCTG<br>1861 TATAAGCTGTCTCTGGAAATTGAACAACTGGAACTACAGAGAGACAGCGCAAGACAATCC<br>1921 ACTTTGGATAAAGAACTATAATTTTTCTCAAAAGAAGGAGGAAAAGGTGTCTTGCTGGCT<br>1981 TGCCTCTTGCAATTCAATACAGATCAGTTTAGCAAATCTACTGTCAATTTGGCAGTGATA<br>2041 TTCATCATAATAAATATCTAGAAAGGATAATTGGTTAATGTTTAATGCTTTGAGATTGTG<br>2101 AAATTATTAATCATCCTCTGTGTGGCTCATGTTTTTGCTTTTCAACACACAAAGCACAAA<br>2161 TTTTTTTTCGATTAAAAATGTATGTAT | |
| | | 1 M H P P K T P S G A L H R K R K M A A W<br>1 ATGCACCCCCCAAAAACTCCATCTGGGGCTCTTCATAGAAAAAGGAAAATGGCAGCCTGG<br><br>21 P F S R L W K V S D P I L F Q M T L I A<br>61 CCCTTCTCCAGGCTGTGGAAAGTCTCTGATCCAATTCTCTTCCAAATGACCTTGATCGCT<br><br>41 A L L P A V L G N C G P P P T L S F A A<br>121 GCTCTGTTGCCTGCTGTTCTTGGCAATTGTGGTCCTCCACCCACTTTATCATTTGCTGCC<br><br>61 P M D I T L T E T R F K T G T T L K Y T<br>181 CCGATGGATATTACGTTGACTGAGACACGCTTCAAAACTGGAACTACTCTGAAATACACC<br><br>81 C L P G Y V R S H S T Q T L T C N S D G<br>241 TGCCTCCCTGGCTACGTCAGATCCCATTCAACTCAGACGCTTACCTGTAATTCTGATGGC<br><br>101 E W V Y N T F C I Y K R C R H P G E L R<br>301 GAATGGGTGTATAACACCTTCTGTATCTACAAACGATGCAGACACCCAGGAGAGTTACGT<br><br>121 N G Q V E I K T D L S F G S Q I E F S C<br>361 AATGGGCAAGTAGAGATTAAGACAGATTTATCTTTTGGATCACAAATAGAATTCAGCTGT<br><br>141 S E G F F L I G S T T S R C E V Q D R G<br>421 TCAGAAGGATTTTTCTTAATTGGCTCAACCACTAGTCGTTGTGAAGTCCAAGATAGAGGA<br><br>161 V D W S D P L P V C V I A K C E A P P A<br>481 GTTGACTGGAGTGATCCTCTCCCAGTATGTGTAATTGCCAAGTGTGAGGCCCCTCCAGCC | SEQ ID NO:85 |

| Gene Name | GenBank Homology | DNA SEQUENCE / DEDUCED AMINO ACID SEQUENCE | SEQUENCE IDENTIFIER: |
|---|---|---|---|
| | | 181 I S N G K H S G G D E D V Y T Y G S S V<br>541 ATCAGCAATGGGAAGCACAGTGGTGGAGATGAAGACGTCTACACATATGGCTCTTCTGTC<br><br>201 T Y S C D P H F S M I G K A S I S C T V<br>601 ACCTACAGCTGTGACCCTCACTTCTCAATGATAGGCAAAGCCTCCATTTCCTGCACAGTG<br><br>221 E N K T I G V W S P S P P T C E K I T C<br>661 GAGAATAAAACGATAGGTGTCTGGAGCCCAAGCCCTCCTACCTGTGAAAAAATCACCTGT<br><br>241 R K P D V S H G E M V S G F G P L Y T Y<br>721 CGCAAGCCAGATGTTTCACATGGGGAAATGGTCTCTGGATTTGGACCCCTCTATACTTAC<br><br>261 K D A I V L D C K K G Y V L T G S S L I<br>781 AAAGACGCTATTGTGTTGGACTGCAAGAAGGGTTATGTCCTCACAGGCAGCAGTTTAATC<br><br>281 H C E A D N N W D P P P P V C E L N G C<br>841 CACTGTGAAGCTGATAACAACTGGGACCCTCCTCCTCCTGTTTGTGAGCTCAATGGTTGT<br><br>301 T N L P D I P N A F W E R Y R Y Q R P T<br>901 ACCAACTTACCAGACATCCCAAATGCCTTCTGGGAACGATACAGGTATCAGAGGCCAACA<br><br>321 K E D V Y N V G T V L K Y H C L T G Y K<br>961 AAAGAGGACGTGTATAATGTTGGGACTGTGTTGAAGTACCATTGCCTGACTGGCTATAAA<br><br>341 P A S D K P T A V I C Q E D F R W T P Y<br>1021 CCTGCTTCAGATAAACCCACGGCTGTGATTTGTCAGGAAGATTTCAGGTGGACTCCATAC<br><br>361 T E C E E V C C P V P E L K N G E I T Q<br>1081 ACGGAATGTGAGGAGGTATGTTGCCCAGTACCAGAGCTGAAGAACGGTGAAATCACTCAA<br><br>381 H R K S R P A N H C V Y F Y G D E I S F<br>1141 CACAGGAAAAGTCGTCCTGCCAATCACTGTGTTTATTTCTATGGAGATGAGATTTCATTT<br><br>401 S C H E T S R F S A I C Q G D G T W S P<br>1201 TCATGTCATGAGACCAGTAGGTTTTCAGCTATATGCCAAGGAGATGGCACGTGGAGTCCC<br><br>421 R T P S C G D I C N F P P K I A H G H Y<br>1261 CGAACACCATCATGTGGAGACATTTGCAATTTTCCTCCTAAAATTGCCCATGGGCATTAT<br><br>441 K Q S S S Y S F F K E E I I Y E C D K G<br>1321 AAACAATCTAGTTCATACAGCTTTTTCAAAGAAGAGATTATATATGAATGTGATAAAGGC | |

| Gene Name | GenBank Homology | DNA SEQUENCE / DEDUCED AMINO ACID SEQUENCE | SEQUENCE IDENTIFIER: |
|---|---|---|---|
| | | 461 Y I L V G Q A K L S C S Y S H W S S P A<br>1381 TACATTCTGGTCGGACAGGCGAAACTCTCCTGCAGTTATTCACACTGGTCATCTCCAGCT<br><br>481 P Q C K A L C P K P Q I E H G R L S V V<br>1441 CCTCAATGTAAAGCTCTGTGTCCGAAACCACAGATAGAGCATGGACGGCTGTCTGTGGTT<br><br>501 K D Q Y I T P E N V T I Q C D P H Y R L<br>1501 AAGGATCAGTATATTACACCTGAAAATGTCACCATCCAGTGTGACCCTCACTATCGTTTG<br><br>521 V G L Q S I T C S E N S T W Y P E V P M<br>1561 GTTGGTCTCCAAAGTATCACTTGCTCAGAGAACAGCACCTGGTACCCCGAGGTGCCCATG<br><br>541 C E W E I A E G C E Q V L A G R K I M Q<br>1621 TGTGAGTGGGAGATCGCTGAAGGCTGTGAGCAAGTGCTCGCAGGCAGAAAAATCATGCAG<br><br>561 C L P K P E D V R T A L E L Y K L S L E<br>1681 TGTCTCCCAAAGCCGGAGGATGTGAGAACGGCCCTGGAGCTGTATAAGCTGTCTCTGGAA<br><br>581 I E Q L E L Q R D S A R Q S T L D K E L<br>1741 ATTGAACAACTGGAACTACAGAGAGACAGCGCAAGACAATCCACTTTGGATAAAGAACTA<br><br>601 -<br>1801 TAA | |
| WBC020F05.bFSP_20 011110.abd | Human translation initiation factor 3 large subunit mRNA. | 1 GGCGACTGCTGGCGAGGCGCGTGGGACCTTACGCTGGTTCCCCTTCGTCTCCTCTCCCGG<br>61 CCCGGGCCACTAGAGAGTTCGCTGACGCCGGGTGAGCTGAGCCTGCCGCCAAGATGCCGG<br>121 CCTATTTTCAGAGGCCGGAAAATGCCCTCAAACGCGCCAACGAATTTCTTGAGGTTGGCA<br>181 AAAAGCAGCCTGCTCTGGATGTTCTTTATGATGTTATGAAAAGTAAAAAACATAGAACAT<br>241 GGCAAAAGATACACGAACCAATTATGTTGAAATACTTGGAACTTTGCGTGGATCTTCGCA<br>301 AGAGCCACTTGGCAAAGGAGGGGTTATACCAGTATAAGAACATTTGTCAACAGGTGAACA<br>361 TAAAATCTCTGGAGGATGTTGTTAGGGCATATTTGAAAATGGCAGAGGAAAAAACTGAAG<br>421 CTGCTAAAGAAGAATCTCAGCAGATGGTCTTAGATATAGAGGATCTAGATAATATTCAAA<br>481 CTCCTGAGAGTGTTCTCCTAAGTGCTGTAAGTGGTGAAGACACTCAGGATCGTACTGACA<br>541 GATTACTTTTAACTCCATGGGTTAAATTCCTGTGGGAGTCTTACAGGCAGTGTTTGGACC<br>601 TTCTTAGAAACAATTCTAGAGTAGAGCGCCTGTACCATGATATTGCCCAGCAAGCTTTCA<br>661 AATTCTGCCTCCAATACACGCGTAAGGCTGAATTCCGTAAACTGTGTGACAATTTGAGAA<br>721 TGCACTTATCGCAGATTCAGCGCCACCATAACCAAAGTACGGCAATCAATCTTAATAATC<br>781 CAGAGAGCCAGTCCATGCATTTGGAAACCAGACTTGTTCAGCTGGACAGTGCTATCAGCA<br>841 TGGAATTGTGGCAGGAAGCATTCAAAGCTGTGGAAGATATTCACGGGCTATTCTCCTTGT<br>901 CTAAAAAACCACCTAAACCTCAGTTGATGGCAAATTACTATAACAAAGTCTCAACTGTGT<br>961 TTTGGAAATCTGGAAATGCTCTTTTTCATGCATCTACACTCCATCGTCTTTACCATCTCT<br>1021 CTAGAGAAATGAGAAAGAATCTCACACAAGACGAGATGCAAAGAATGTCTACTAGAGTCC | SEQ ID NO:86 |

| Gene Name | GenBank Homology | DNA SEQUENCE / DEDUCED AMINO ACID SEQUENCE | SEQUENCE IDENTIFIER: |
|---|---|---|---|
| | | 1081 TTTTAGCCACTCTTTCCATCCCTATTACTCCTGAGCGTACGGATATTGCTCGACTTCTGG<br>1141 ATATGGATGGCATTATAGTTGAAAAACAGCGTCGCCTTGCAACACTACTAGGTCTTCAAG<br>1201 CCCCACCGACACGAATTGGCCTTATTAATGATATGGTCAGATTTAATGTACTACAATATG<br>1261 TTGTCCCAGAAGTGAAAGACCTTTACAATTGGCTTGAAGTAGAATTTAACCCATTAAAAC<br>1321 TCTGTGAGAGAGTCACAAAGGTTCTAAATTGGGTTAGAGAACAACCTGAAAAGGAACCAG<br>1381 AGTTGCAGCAGTATGTCCCACAGCTGCAAAACAACACCATACTCCGTCTTCTGCAGCAGG<br>1441 TGGCACAAATTTATCAGAGCATTGAGTTTTCTCGTTTGACTTCTCTGGTTCCTTTTGTTG<br>1501 ATGCTTTCCAACTGGAACGGGCCATAGTAGATGCAGCCAGGCACTGTGACCTGCAGGTTC<br>1561 GTATTGACCACACTTCTCGGACCCTTAGTTTTGGATCCGATTTGAATTATGCTACTCGAG<br>1621 AAGATGCTCCAATTGGTCCTCATTTGCAAAGCATGCCTTCAGAACAGATAAGAAATCAGC<br>1681 TGACAGCCATGTCCTCAGTACTTGCAAAAGCACTTGAAGTCATTAAGCCAGCTCATATAC<br>1741 TGCAAGAGAAAGAAGAACAGCATCAGTTGGCTGTTACCGCGTACCTTAAAAATTCACGAA<br>1801 AAGAGCATCAGCGCATCCTGGCTCGCCGTCAGACCATTGAGGAAAGGAAAGAGCGGCTTG<br>1861 AGAGTCTGAATATCCAGCGGGAGAAAGAAGAATTGGAGCAGAGGGAAGCTGAACTCCAGA<br>1921 AAGTACGGAAGGCTGAAGAAGAGAGGCTGCGACAGGAGGCAAAGGAGAGAGAGAAGGAGC<br>1981 GTATCTTACAGGAACATGAACAAATCAAAAAGAAAACTGTTCGTGATCGTTTGGAGCAGA<br>2041 TCAAGAAAACGGAACTGGGTGCCAAAGCATTCAAAGATATCGATATTGAAGACCTTGAAG<br>2101 AATTGGATCCAGATTTCATCATGGCTAAACAGGTTGAACAACTGGAGAAAAAAAAGAAGG<br>2161 AACTTCAGGAACGCCTAAAGAATCAAGAAAAGAAGATTGACTATTTTGAAAGAGCTAAGC<br>2221 GTTTGGAAGAAATTCCTTTGATAAAGAGCGCTTACGAGGAACAGAGAATTAAAGACATGG<br>2281 ATCTGTGGGAGCAACAAGAGGAAGAAAGAATTACTACAATGCAGCTAGAACGTGAAAAGG<br>2341 CTCTTGAACATAAGAATCGAATGTCACGAATGCTTGAAGACAGAGATTTATTCGTAATGC<br>2401 GACTCAAAGCTGCACGGCAGTCTGTTTATGAGGAAAAACTTAAACAGTTTGAAGAGCGAT<br>2461 TAGCAGAAGAAAGGCATAATCGATTGGAAGAACGGAAAAGGCAGCGTAAAGAAGAACGCA<br>2521 GGATAACATACTATAGAGAAAAAGAAGAGGAGGAGCAGAGAAGGGCAGAAGAACAAATGC<br>2581 TAAAAGAGCGGGAAGAGAGAGAGCGCGCCGAACGAGCAAAACGCGAGGAAGAGCTACGAG<br>2641 AGTATCAGGAGCGGGTGAAGAAATTAGAAGAAGTGGAAAGGAAAAAACGCCAAAGGGAGT<br>2701 TGGAAATTGAAGAACGAGAACGGCGTAGAGAGGAAGAGAGAAGACTTGGCGATAGTTCCC<br>2761 TTTCTAGAAAGGACTCTCGTTGGGGAGATAGAGATTCAGAAGGCACCTGGAGAAAAGGAC<br>2821 CTGAAGCAGATTCTGAGTGGAGAAGAGGCCCGCCAGAGAAGGAGTGGAGACGTGGAGAAG<br>2881 GGCGAGATGAGGACAGGTCTCATAGAAGAGATGAAGAGCGGCCCCGGCGTCTGGGGATG<br>2941 ATGAAGATAGAGAGCCCTCTCTTAGACCAGACGATGATCGGGTTCCCCGGCGTGGCATGG<br>3001 ATGATGACAGAGGCCCTAGACGTGGTCCTGAGGAAGATAGGTTCTCTCGTCGTGGGGCAG<br>3061 ACGATGACCGGCCTTCCTGGCGTAACACAGATGATGACAGGCCTCCCAGACGAATTGCCG<br>3121 ATGAAGACAGGGGAAACTGGCGTCATGCGGATGATGACAGACCACCTAGACGAGGACTGG<br>3181 ATGAGGACAGAGGAAGCTGGCGAACAGCTGATGAGGACAGAGGACCAAGACGTGGGATGG<br>3241 ATGATGACCGGGGGCCGAGGCGAGGAGGCGCTGATGATGAGCGATCATCCTGGCGTAATG<br>3301 CTGATGATGACCGGGGTCCCAGGCGAGGGTTGGATGATGATCGGGGTCCCAGGCGAGGCA<br>3361 TGGATGATGACCGGGGTCCCAGGCGAGGCATGGATGATGACCGGGGTCCCAGGCGAGGCA<br>3421 TGGATGATGACCGGGGTCCCAGGCGAGGGTTGGATGATGATCGAGGACCTTGGAGGAACG<br>3481 CCGATGATGACAGAATTCCCAGGCGTGGTGCAGAGGATGACAGGGGCCCTTGGAGAAACA<br>3541 TGGATGATGATCGCCTTTCAAGACGTGCTGATGATGATCGGTTTCCCAGACGGGGTGATG<br>3601 ACTCAAGACCTGGTCCTTGGAGACCATTAGTCAAGCCAGGTGGATGGAGAGAGAAAGAAA | |

| Gene Name | GenBank Homology | DNA SEQUENCE / DEDUCED AMINO ACID SEQUENCE | SEQUENCE IDENTIFIER: |
|---|---|---|---|
| | | 3661 AAGCCAGAGAGGAGAGCTGGGGTCCACCTCGAGAATCAAGGCCATCAGAAGAACGTGAAT<br>3721 GGGACAGAGAAAAAGAAAGGGACAGAGATAATCAAGATCGGGAGGAGAATGACAAGGACC<br>3781 CTGAGAGAGAAAGGGACAGAGAGAGAGATGTGGATCGAGAGGATCGCTTCAGACGACCCA<br>3841 GGGATGAAGGTGGCTGGAGAAGAGGACCAGCTGAAGAATCTTCTAGCTGGAGAGATTCGA<br>3901 GTCGCCGTGACGATAGGGATCGGGATGACCGTCGTCGTGAGCGAGATGATCGCCGTGATC<br>3961 TGAGAGAAAGACGAGATGACAGAGACCGAAGAGGACCTCCTTTGAGATCAGAACGTGAAG<br>4021 AAGCAAGTTCTTGGAGACGTGCTGATGACAGGAAAGATGAGCGGGCGGAAGAGCGGGACG<br>4081 CCCCTCGTCGTGTTCCTCCCCCAGCTCTTTCAAGAGACCGAGAAAGAGACCGAGACCGAG<br>4141 AAAGAGAAGGTGAAAAAGAGAAGGCCTCATGGAGAGCTGAGAAAGATAGGGAATCTCTTC<br>4201 GTCGTACTAAAAATGAGACTGATGAAGATGGATGGACCACGGTACGACGTTAAGTCTCAA<br>4261 GATAATGGGTTCGAACTCATGTGTCACATAGGTTTGATCACATTCGCGGATTATTAGACT<br>4321 TGTGCTTCAACCAGTCTAAATTGGATTCTTTAATGTTGTCTCACCATAACACAAAAAGCA<br>4381 TGAACTTGTATTAATCCTATATAATAGATTGATCATGCACCGTATCCACAGAAAGCTGGA<br>4441 AAACCATGCCATTTTCTGGAATTTAAGGTGTTGCATTATTTCATCAATCATTTGTTGACA<br>4501 AAAAAGAAAAACTAAAAAATAAATTTAAAATGTGAACCTTCAGGTATTGAGTAACACCTT<br>4561 TATCTTGGTATAGAACTGATACCATTTTTTGATTTTGAAATATCTGATAATAATTTGGAA<br>4621 TGAAGTAAGGTTCTGTTAAAATATATTTGAAGACCCTTTAAAGCAGTGAATCTGAAACAA<br>4681 TTTTCACACCCTTAAGTGGTTGATACGTACCTATTTTAGGTATTTTGAGGTATTTACCAT<br>4741 AAACTAAATTTAGAAATTTTTTAGATTCACTTGAAGTAAACATTACAAACATTGGATACG<br>4801 GTGGGGTTTTCTTTAGATTTTACTTGAGAGAAGGTGAGTACAAAGCAATTTGCAGTTGTT<br>4861 GTAATGACAAGATTACTGCGCAAGTGTGAATCCAAACAGTATAGCTTTTAAATTTTAAAG<br>4921 CATTTGGTAAATTATCGCTGAGTTTTTTTCTGTTGCCAATAGCAAACTGCTTTTCCATTA<br>4981 ATGGAGAATTCATGCCTTTCAAGCATTTTAAATATGACAATATTTATAAATGTATGGTTT<br>5041 GGAGGAATCGTTTAAATTCTCTTTCCTAATTTTCTTTCTTTTGAAGATAGATTCTTTCAA<br>5101 CAAGTAATTTGTAGTAATGACTGTGTTGACTTCAATTTTGGAGCGCAGTAGCTATGTTAA<br>5161 AGATGAACTATTTGGTCTCATTGAAGCCAACACAGAACTTGCTGCTGTGTTTTTTCTTCA<br>5221 GTGATAAATAAAATACTTACAGAAA | |
| | | 1 M P A Y F Q R P E N A L K R A N E F L E<br>1 ATGCCGGCCTATTTTCAGAGGCCGGAAAATGCCCTCAAACGCGCCAACGAATTTCTTGAG<br><br>21 V G K K Q P A L D V L Y D V M K S K K H<br>61 GTTGGCAAAAAGCAGCCTGCTCTGGATGTTCTTTATGATGTTATGAAAAGTAAAAAACAT<br><br>41 R T W Q K I H E P I M L K Y L E L C V D<br>121 AGAACATGGCAAAAGATACACGAACCAATTATGTTGAAATACTTGGAACTTTGCGTGGAT<br><br>61 L R K S H L A K E G L Y Q Y K N I C Q Q<br>181 CTTCGCAAGAGCCACTTGGCAAAGGAGGGGTTATACCAGTATAAGAACATTTGTCAACAG<br><br>81 V N I K S L E D V V R A Y L K M A E E K<br>241 GTGAACATAAAATCTCTGGAGGATGTTGTTAGGGCATATTTGAAAATGGCAGAGGAAAAA | SEQ ID NO:87 |

| Gene Name | GenBank Homology | DNA SEQUENCE / DEDUCED AMINO ACID SEQUENCE | SEQUENCE IDENTIFIER: |
|---|---|---|---|
| | | 101 T E A A K E E S Q Q M V L D I E D L D N<br>301 ACTGAAGCTGCTAAAGAAGAATCTCAGCAGATGGTCTTAGATATAGAGGATCTAGATAAT<br><br>121 I Q T P E S V L L S A V S G E D T Q D R<br>361 ATTCAAACTCCTGAGAGTGTTCTCCTAAGTGCTGTAAGTGGTGAAGACACTCAGGATCGT<br><br>141 T D R L L L T P W V K F L W E S Y R Q C<br>421 ACTGACAGATTACTTTTAACTCCATGGGTTAAATTCCTGTGGGAGTCTTACAGGCAGTGT<br><br>161 L D L L R N N S R V E R L Y H D I A Q Q<br>481 TTGGACCTTCTTAGAAACAATTCTAGAGTAGAGCGCCTGTACCATGATATTGCCCAGCAA<br><br>181 A F K F C L Q Y T R K A E F R K L C D N<br>541 GCTTTCAAATTCTGCCTCCAATACACGCGTAAGGCTGAATTCCGTAAACTGTGTGACAAT<br><br>201 L R M H L S Q I Q R H H N Q S T A I N L<br>601 TTGAGAATGCACTTATCGCAGATTCAGCGCCACCATAACCAAAGTACGGCAATCAATCTT<br><br>221 N N P E S Q S M H L E T R L V Q L D S A<br>661 AATAATCCAGAGAGCCAGTCCATGCATTTGGAAACCAGACTTGTTCAGCTGGACAGTGCT<br><br>241 I S M E L W Q E A F K A V E D I H G L F<br>721 ATCAGCATGGAATTGTGGCAGGAAGCATTCAAAGCTGTGGAAGATATTCACGGGCTATTC<br><br>261 S L S K K P P K P Q L M A N Y Y N K V S<br>781 TCCTTGTCTAAAAAACCACCTAAACCTCAGTTGATGGCAAATTACTATAACAAAGTCTCA<br><br>281 T V F W K S G N A L F H A S T L H R L Y<br>841 ACTGTGTTTTGGAAATCTGGAAATGCTCTTTTTCATGCATCTACACTCCATCGTCTTTAC<br><br>301 H L S R E M R K N L T Q D E M Q R M S T<br>901 CATCTCTCTAGAGAAATGAGAAAGAATCTCACACAAGACGAGATGCAAAGAATGTCTACT<br><br>321 R V L L A T L S I P I T P E R T D I A R<br>961 AGAGTCCTTTTAGCCACTCTTTCCATCCCTATTACTCCTGAGCGTACGGATATTGCTCGA<br><br>341 L L D M D G I I V E K Q R R L A T L L G<br>1021 CTTCTGGATATGGATGGCATTATAGTTGAAAAACAGCGTCGCCTTGCAACACTACTAGGT<br><br>361 L Q A P P T R I G L I N D M V R F N V L<br>1081 CTTCAAGCCCCACCGACACGAATTGGCCTTATTAATGATATGGTCAGATTTAATGTACTA<br><br>381 Q Y V V P E V K D L Y N W L E V E F N P | |

| Gene Name | GenBank Homology | DNA SEQUENCE / DEDUCED AMINO ACID SEQUENCE | SEQUENCE IDENTIFIER: |
|---|---|---|---|
| | | 1141 CAATATGTTGTCCCAGAAGTGAAAGACCTTTACAATTGGCTTGAAGTAGAATTTAACCCA<br><br>401 L K L C E R V T K V L N W V R E Q P E K<br>1201 TTAAAACTCTGTGAGAGAGTCACAAAGGTTCTAAATTGGGTTAGAGAACAACCTGAAAAG<br><br>421 E P E L Q Q Y V P Q L Q N N T I L R L L<br>1261 GAACCAGAGTTGCAGCAGTATGTCCCACAGCTGCAAAACAACACCATACTCCGTCTTCTG<br><br>441 Q Q V A Q I Y Q S I E F S R L T S L V P<br>1321 CAGCAGGTGGCACAAATTTATCAGAGCATTGAGTTTTCTCGTTTGACTTCTCTGGTTCCT<br><br>461 F V D A F Q L E R A I V D A A R H C D L<br>1381 TTTGTTGATGCTTTCCAACTGGAACGGGCCATAGTAGATGCAGCCAGGCACTGTGACCTG<br><br>481 Q V R I D H T S R T L S F G S D L N Y A<br>1441 CAGGTTCGTATTGACCACACTTCTCGGACCCTTAGTTTTGGATCCGATTTGAATTATGCT<br><br>501 T R E D A P I G P H L Q S M P S E Q I R<br>1501 ACTCGAGAAGATGCTCCAATTGGTCCTCATTTGCAAAGCATGCCTTCAGAACAGATAAGA<br><br>521 N Q L T A M S S V L A K A L E V I K P A<br>1561 AATCAGCTGACAGCCATGTCCTCAGTACTTGCAAAAGCACTTGAAGTCATTAAGCCAGCT<br><br>541 H I L Q E K E E Q H Q L A V T A Y L K N<br>1621 CATATACTGCAAGAGAAAGAAGAACAGCATCAGTTGGCTGTTACCGCGTACCTTAAAAAT<br><br>561 S R K E H Q R I L A R R Q T I E E R K E<br>1681 TCACGAAAAGAGCATCAGCGCATCCTGGCTCGCCGTCAGACCATTGAGGAAAGGAAAGAG<br><br>581 R L E S L N I Q R E K E E L E Q R E A E<br>1741 CGGCTTGAGAGTCTGAATATCCAGCGGGAGAAAGAAGAATTGGAGCAGAGGGAAGCTGAA<br><br>601 L Q K V R K A E E E R L R Q E A K E R E<br>1801 CTCCAGAAAGTACGGAAGGCTGAAGAAGAGAGGCTGCGACAGGAGGCAAAGGAGAGAGAG<br><br>621 K E R I L Q E H E Q I K K K T V R D R L<br>1861 AAGGAGCGTATCTTACAGGAACATGAACAAATCAAAAAGAAAACTGTTCGTGATCGTTTG<br><br>641 E Q I K K T E L G A K A F K D I D I E D<br>1921 GAGCAGATCAAGAAAACGGAACTGGGTGCCAAAGCATTCAAAGATATCGATATTGAAGAC<br><br>661 L E E L D P D F I M A K Q V E Q L E K K<br>1981 CTTGAAGAATTGGATCCAGATTTCATCATGGCTAAACAGGTTGAACAACTGGAGAAAAAA | |

| Gene Name | GenBank Homology | DNA SEQUENCE / DEDUCED AMINO ACID SEQUENCE | SEQUENCE IDENTIFIER: |
|---|---|---|---|
| | | 681 K K E L Q E R L K N Q E K K I D Y F E R<br>2041 AAGAAGGAACTTCAGGAACGCCTAAAGAATCAAGAAAAGAAGATTGACTATTTTGAAAGA<br><br>701 A K R L E E I P L I K S A Y E E Q R I K<br>2101 GCTAAGCGTTTGGAAGAAATTCCTTTGATAAAGAGCGCTTACGAGGAACAGAGAATTAAA<br><br>721 D M D L W E Q Q E E E R I T T M Q L E R<br>2161 GACATGGATCTGTGGGAGCAACAAGAGGAAGAAAGAATTACTACAATGCAGCTAGAACGT<br><br>741 E K A L E H K N R M S R M L E D R D L F<br>2221 GAAAAGGCTCTTGAACATAAGAATCGAATGTCACGAATGCTTGAAGACAGAGATTTATTC<br><br>761 V M R L K A A R Q S V Y E E K L K Q F E<br>2281 GTAATGCGACTCAAAGCTGCACGGCAGTCTGTTTATGAGGAAAAACTTAAACAGTTTGAA<br><br>781 E R L A E E R H N R L E E R K R Q R K E<br>2341 GAGCGATTAGCAGAAGAAAGGCATAATCGATTGGAAGAACGGAAAAGGCAGCGTAAAGAA<br><br>801 E R R I T Y Y R E K E E E E Q R R A E E<br>2401 GAACGCAGGATAACATACTATAGAGAAAAAGAAGAGGAGGAGCAGAGAAGGGCAGAAGAA<br><br>821 Q M L K E R E E R E R A E R A K R E E E<br>2461 CAAATGCTAAAAGAGCGGGAAGAGAGAGAGCGCGCCGAACGAGCAAAACGCGAGGAAGAG<br><br>841 L R E Y Q E R V K K L E E V E R K K R Q<br>2521 CTACGAGAGTATCAGGAGCGGGTGAAGAAATTAGAAGAAGTGGAAAGGAAAAAACGCCAA<br><br>861 R E L E I E E R E R R R E E E R R L G D<br>2581 AGGGAGTTGGAAATTGAAGAACGAGAACGGCGTAGAGAGGAAGAGAGAAGACTTGGCGAT<br><br>881 S S L S R K D S R W G D R D S E G T W R<br>2641 AGTTCCCTTTCTAGAAAGGACTCTCGTTGGGGAGATAGAGATTCAGAAGGCACCTGGAGA<br><br>901 K G P E A D S E W R R G P P E K E W R R<br>2701 AAAGGACCTGAAGCAGATTCTGAGTGGAGAAGAGGCCCGCCAGAGAAGGAGTGGAGACGT<br><br>921 G E G R D E D R S H R R D E E R P R R L<br>2761 GGAGAAGGGCGAGATGAGGACAGGTCTCATAGAAGAGATGAAGAGCGGCCCCGGCGTCTG<br><br>941 G D D E D R E P S L R P D D D R V P R R<br>2821 GGGGATGATGAAGATAGAGAGCCCTCTCTTAGACCAGACGATGATCGGGTTCCCCGGCGT | |

| Gene Name | GenBank Homology | DNA SEQUENCE / DEDUCED AMINO ACID SEQUENCE | SEQUENCE IDENTIFIER: |
|---|---|---|---|
| | | 961 G M D D D R G P R R G P E E D R F S R R<br>2881 GGCATGGATGATGACAGAGGCCCTAGACGTGGTCCTGAGGAAGATAGGTTCTCTCGTCGT<br><br>981 G A D D D R P S W R N T D D D R P P R R<br>2941 GGGGCAGACGATGACCGGCCTTCCTGGCGTAACACAGATGATGACAGGCCTCCCAGACGA<br><br>1001 I A D E D R G N W R H A D D D R P P R R<br>3001 ATTGCCGATGAAGACAGGGGAAACTGGCGTCATGCGGATGATGACAGACCACCTAGACGA<br><br>1021 G L D E D R G S W R T A D E D R G P R R<br>3061 GGACTGGATGAGGACAGAGGAAGCTGGCGAACAGCTGATGAGGACAGAGGACCAAGACGT<br><br>1041 G M D D D R G P R R G G A D D E R S S W<br>3121 GGGATGGATGATGACCGGGGGCCGAGGCGAGGAGGCGCTGATGATGAGCGATCATCCTGG<br><br>1061 R N A D D D R G P R R G L D D D R G P R<br>3181 CGTAATGCTGATGATGACCGGGGTCCCAGGCGAGGGTTGGATGATGATCGGGGTCCCAGG<br><br>1081 R G M D D D R G P R R G M D D D R G P R<br>3241 CGAGGCATGGATGATGACCGGGGTCCCAGGCGAGGCATGGATGATGACCGGGGTCCCAGG<br><br>1101 R G M D D D R G P R R G L D D D R G P W<br>3301 CGAGGCATGGATGATGACCGGGGTCCCAGGCGAGGGTTGGATGATGATCGAGGACCTTGG<br><br>1121 R N A D D D R I P R R G A E D D R G P W<br>3361 AGGAACGCCGATGATGACAGAATTCCCAGGCGTGGTGCAGAGGATGACAGGGGCCCTTGG<br><br>1141 R N M D D D R L S R R A D D D R F P R R<br>3421 AGAAACATGGATGATGATCGCCTTTCAAGACGTGCTGATGATGATCGGTTTCCCAGACGG<br><br>1161 G D D S R P G P W R P L V K P G G W R E<br>3481 GGTGATGACTCAAGACCTGGTCCTTGGAGACCATTAGTCAAGCCAGGTGGATGGAGAGAG<br><br>1181 K E K A R E E S W G P P R E S R P S E E<br>3541 AAAGAAAAAGCCAGAGAGGAGAGCTGGGGTCCACCTCGAGAATCAAGGCCATCAGAAGAA<br><br>1201 R E W D R E K E R D R D N Q D R E E N D<br>3601 CGTGAATGGGACAGAGAAAAAGAAAGGGACAGAGATAATCAAGATCGGGAGGAGAATGAC<br><br>1221 K D P E R E R D R E R D V D R E D R F R<br>3661 AAGGACCCTGAGAGAGAAAGGGACAGAGAGAGAGATGTGGATCGAGAGGATCGCTTCAGA<br><br>1241 R P R D E G G W R R G P A E E S S S W R | |

| Gene Name | GenBank Homology | DNA SEQUENCE / DEDUCED AMINO ACID SEQUENCE | SEQUENCE IDENTIFIER: |
|---|---|---|---|
| | | 3721 CGACCCAGGGATGAAGGTGGCTGGAGAAGAGGACCAGCTGAAGAATCTTCTAGCTGGAGA<br><br>1261 D S S R R D D R D R D D R R R E R D D R<br>3781 GATTCGAGTCGCCGTGACGATAGGGATCGGGATGACCGTCGTCGTGAGCGAGATGATCGC<br><br>1281 R D L R E R R D D R D R R G P P L R S E<br>3841 CGTGATCTGAGAGAAAGACGAGATGACAGAGACCGAAGAGGACCTCCTTTGAGATCAGAA<br><br>1301 R E E A S S W R R A D D R K D E R A E E<br>3901 CGTGAAGAAGCAAGTTCTTGGAGACGTGCTGATGACAGGAAAGATGAGCGGGCGGAAGAG<br><br>1321 R D A P R R V P P P A L S R D R E R D R<br>3961 CGGGACGCCCCTCGTCGTGTTCCTCCCCCAGCTCTTTCAAGAGACCGAGAAAGAGACCGA<br><br>1341 D R E R E G E K E K A S W R A E K D R E<br>4021 GACCGAGAAAGAGAAGGTGAAAAAGAGAAGGCCTCATGGAGAGCTGAGAAAGATAGGGAA<br><br>1361 S L R R T K N E T D E D G W T T V R R -<br>4081 TCTCTTCGTCGTACTAAAAATGAGACTGATGAAGATGGATGGACCACGGTACGACGTTAA | |
| WBC020H01.bFSP_20 011110.abd | Human CSaids binding protein (CSBP1) mRNA | 1 GGAACCGCGACCACTGGAGCCTTAGCGGGCGCAGCAGCTGGAACGGGAGTACTGCGACGC<br>61 AGCCCGGAGTCGGCCTTGTAGGGGCGAAGGTGCAGGGAGATCGCGGCGGGCGCAGTCTTG<br>121 AGCGCCGGAGCGCGTCCCTGCCCTTAGCGGGGCTTGCCCCAGTCGCAGGGGCACATCCAG<br>181 CCGCTGCGGCTGACAGCAGCCGCGCGCGGGAGTCTGCGGGGTCGCGGCAGCCGCACCT<br>241 GCGCGGGCGACCAGCGCAAGGTCCCCGCCCGGCTGGGCGGGCAGCAAGGGCCGGGGAGAG<br>301 GGTGCGGGTGCAGGCGGGGGCCCCACAGGGCCACCTTCTTGCCCGGCGGCTGCCGCTGGA<br>361 AAATGTCTCAGGAGAGGCCCACGTTCTACCGGCAGGAGCTGAACAAGACAATCTGGGAGG<br>421 TGCCCGAGCGTTACCAGAACCTGTCTCCAGTGGGCTCTGGCGCCTATGGCTCTGTGTGTG<br>481 CTGCTTTTGACACAAAAACGGGGTTACGTGTGGCAGTGAAGAAGCTCTCCAGACCATTTC<br>541 AGTCCATCATTCATGCGAAAAGAACCTACAGAGAACTGCGGTTACTTAAACATATGAAAC<br>601 ATGAAAATGTGATTGGTCTGTTGGACGTTTTTACACCTGCAAGGTCTCTGGAGGAATTCA<br>661 ATGATGTGTATCTGGTGACCCATCTCATGGGGGCAGATCTGAACAACATTGTGAAATGTC<br>721 AGAAGCTTACAGATGACCATGTTCAGTTCCTTATCTACCAAATTCTCCGAGGTCTAAAGT<br>781 ATATACATTCAGCTGACATAATTCACAGGGACCTAAAACCTAGTAATCTAGCTGTGAATG<br>841 AAGACTGTGAGCTGAAGATTCTGGATTTTGGACTGGCTCGGCACACAGATGATGAAATGA<br>901 CAGGCTACGTGGCCACTAGGTGGTACAGGGCTCCTGAGATCATGCTGAACTGGATGCATT<br>961 ACAACCAGACAGTTGATATTTGGTCAGTGGGATGCATAATGGCCGAGCTGTTGACTGGAA<br>1021 GAACATTGTTTCCTGGTACAGACCATATTAACCAGCTTCAGCAGATTATGCGTCTGACAG<br>1081 GAACACCCCCCGCTTATCTCATTAACAGGATGCCAAGCCATGAGGCAAGAAACTATATTC<br>1141 AGTCTTTGACTCAGATGCCGAAGATGAACTTTGCGAATGTATTTATTGGTGCCAATCCCC<br>1201 TGGCTGTCGACTTGCTGGAGAAGATGCTTGTATTGGACTCAGATAAGAGAATTACAGCGG | SEQ ID NO:88 |

| Gene Name | GenBank Homology | DNA SEQUENCE / DEDUCED AMINO ACID SEQUENCE | SEQUENCE IDENTIFIER: |
|---|---|---|---|
| | | 1261 CCCAAGCCCTTGCACATGCCTACTTTGCTCAGTACCACGATCCTGATGATGAACCAGTGG<br>1321 CCGATCCTTATGATCAGTCCTTTGAAAGCAGGGACCTCCTTATAGATGAGTGGAAAAGCC<br>1381 TGACCTATGATGAAGTCATCAGCTTTGTGCCACCACCCCTTGACCAAGAAGAGATGGAGT<br>1441 CCTGAGCACCTGGTTTCTGTTCTGTTGATCCCACTTCACTGTGAGGGGAAGGCCTTTTCA<br>1501 CGGGAACTCTCCAAATATTATTCAAGTGCCTCTTGTTGCAGAGATTTCCTCCATGGTGGA<br>1561 AGGGGGTGTGCGTGCGTGTGCGTGCGTGTTAGTGTGTGCATGTGTGTGTCTGTCTTTG<br>1621 TGGGAGGGTAAGACAATATGAACAAACTATGATCACAGTGACTTTACAGGAGGTTGTGGA<br>1681 TGCTCCAGGGCAGCCTCCACCTTGCTCTTCTTTCTGAGAGTTGGCTCAGGCAGACAAGAG<br>1741 CTGCTGTCCTTTTAGGAATATGTTCAATGCAAAGTAAAAAAATATGAATTGTCCCCAATC<br>1801 CCGGTCATGCTTTTGCCACTTTGGCTTCTCCTGTGACCCCACCTTGATGGTGGGAGGTAG<br>1861 AATTGACCATATCCCCCAGCGGCACAGAGAGAAGGCGCACACCTTCTGGCTGCTTCAGAT<br>1921 CTGAAGCCGTCCATCAGCGATGCATACAGCCAAAAAGGACCAACTGGCTTCTGTGCTCTA<br>1981 GTCTATGATTAACTTGCTTAATATGATTCTCAGAACTCAACAGGTGTATCTGAAACTGTA<br>2041 AATATGTTTGTGCCTTAAAAGGAGAGAAGGGAGTGTAGAGAGTTAAGAGAGTGCAGCAGA<br>2101 TGAAGTTCTGAGCCAGGCGCGTCGTCAGGTTGTCGGACGGCTACTTGTGAAGCTGAAGTA<br>2161 ACCAGGGAGCCTTCAGAGGCGCCGTGTATGCATGTGTGAGCATGCGTATATGTGCTTCTC<br>2221 TCTCTTTCTTCCTCGCCCCCCAGGTGGTGTTGCCGTTTCTCTGCTTACCCCCACCTTCAG<br>2281 TGCAGAGGTTTCTTGAGTATTGGCCCCGGTAGTCAGAAGCCGGTTCTTGCTGTCAGTGTA<br>2341 CTTCCTGTGTGCTCTTTATTCCTAGCAGAGTGACATGTGTTTTGCACGTCTTGCTATTTG<br>2401 AGCATGCACAGCTGCTGTCCTGTTCTCTTCGGGAGGCCCTGGTGCCAGGCAGGTTTGCCA<br>2461 GTGAAGACTTTTTGGGTAGTTCGGAACCCATGTCACCTTGGCTCATGCTTTGGGCAGGTG<br>2521 ACATCATCCTCTCTTCAGCCCCCAGTGCTATTTTGTGTTGAACACAATTAATACTCCAGG<br>2581 TGCTTTTGATGTGAAAACTATGAAAAAATGGAACAGATGGACATATAGCATTTTCATTCT<br>2641 TGCCATCTGGTTTTTCAACAAACTATTTTTGGGGAGCTGTCATGGGAAAGATCAGGGCTT<br>2701 TTCCTAGGAATATCTCAAATTTTGGAAAACAAATTGTTCTCTTCCCTCCCAATAACTAAT<br>2761 GCTAAGAAATGCTGAAAATCAAAGTAAAAAATTAAAGCCCATAAGGCCAGAAACTCCTTT<br>2821 TGCTGTCTTTCTCTAAATATGATTACTTTAAAATAAAAAAGTAACAAGGTGTCTTTTCCA<br>2881 CTCCTATGGAAAAGGGTCTTCTTGGCAGCTTAACATTGACTTCTTGGTTTGGGGAGAAAT<br>2941 AAATTTTGTTTCAGAATTTTGTATATTGTAGGAATCCCTTTGAGAATGTGATTCCTTTTG<br>3001 ATGGGGAGAAAGGGCAAATTATTTTAATATTTTGTATTTTCAACTTTATAAAGATAAAAT<br>3061 ATCCTCAGGGGTGGAGAAGTGTCGTTTTCATAACTTGCTGAATTTCAGGCATTTTGTTCT<br>3121 ACATGAGGACTCATATATTTAAGCCTTTTGTGTAATAAGAAAGTATAAAGCTACTTCCAG<br>3181 TGTTGGCTATGTGACAGAATCTTGTATTTGGGCCAAGGTGTTTCCATTTCTCCATCACAG<br>3241 TGCGGTGATGCATGCGCTCCAGAGGGGCAGGTAGGACCCTGGGTCAACTGGAGTGAGAAG<br>3301 GAACAGGCAGACTGATGGCGATTCCCTCTCACCCGGGACTCTCCCCCTTTCAAGGAAAGT<br>3361 GAACCTTTAAAGTAAAGGCCTCATCTCCTTTATATGCAGGTCAAATCCTCACGATGCACA<br>3421 GCAAGATGAATTTTATCGGCCATGTTTGGTTGTAAATGCTAGTGTGATTTCCTACAGAAA<br>3481 TACTGCTCTGACTATTTTCATAAAGGTCTTTGCACATGTGACCATATCTGTGTTAGGAGG<br>3541 CTGAATGCTCCGGGAGCCTGAACTCAGCTGGAGCCTGTGGAAGAGCTCCTTGGTTTCTGA<br>3601 GCATCACGCTCCCATCTCCTGATTTCTCTGAACGGAAAACAGAAGAGAGAATGAGGGATA<br>3661 CTGCTATGTTATTTGTACTCATGAACTTGGCTGTAATCAGT<br><br>1 M S Q E R P T F Y R Q E L N K T I W E V | SEQ ID NO:89 |

| Gene Name | GenBank Homology | DNA SEQUENCE / DEDUCED AMINO ACID SEQUENCE | SEQUENCE IDENTIFIER: |
|---|---|---|---|
| | | 1 ATGTCTCAGGAGAGGCCCACGTTCTACCGGCAGGAGCTGAACAAGACAATCTGGGAGGTG<br><br>21 P E R Y Q N L S P V G S G A Y G S V C A<br>61 CCCGAGCGTTACCAGAACCTGTCTCCAGTGGGCTCTGGCGCCTATGGCTCTGTGTGTGCT<br><br>41 A F D T K T G L R V A V K K L S R P F Q<br>121 GCTTTTGACACAAAAACGGGGTTACGTGTGGCAGTGAAGAAGCTCTCCAGACCATTTCAG<br><br>61 S I I H A K R T Y R E L R L L K H M K H<br>181 TCCATCATTCATGCGAAAAGAACCTACAGAGAACTGCGGTTACTTAAACATATGAAACAT<br><br>81 E N V I G L L D V F T P A R S L E E F N<br>241 GAAAATGTGATTGGTCTGTTGGACGTTTTTACACCTGCAAGGTCTCTGGAGGAATTCAAT<br><br>101 D V Y L V T H L M G A D L N N I V K C Q<br>301 GATGTGTATCTGGTGACCCATCTCATGGGGGCAGATCTGAACAACATTGTGAAATGTCAG<br><br>121 K L T D D H V Q F L I Y Q I L R G L K Y<br>361 AAGCTTACAGATGACCATGTTCAGTTCCTTATCTACCAAATTCTCCGAGGTCTAAAGTAT<br><br>141 I H S A D I I H R D L K P S N L A V N E<br>421 ATACATTCAGCTGACATAATTCACAGGGACCTAAAACCTAGTAATCTAGCTGTGAATGAA<br><br>161 D C E L K I L D F G L A R H T D D E M T<br>481 GACTGTGAGCTGAAGATTCTGGATTTTGGACTGGCTCGGCACACAGATGATGAAATGACA<br><br>181 G Y V A T R W Y R A P E I M L N W M H Y<br>541 GGCTACGTGGCCACTAGGTGGTACAGGGCTCCTGAGATCATGCTGAACTGGATGCATTAC<br><br>201 N Q T V D I W S V G C I M A E L L T G R<br>601 AACCAGACAGTTGATATTTGGTCAGTGGGATGCATAATGGCCGAGCTGTTGACTGGAAGA<br><br>221 T L F P G T D H I N Q L Q Q I M R L T G<br>661 ACATTGTTTCCTGGTACAGACCATATTAACCAGCTTCAGCAGATTATGCGTCTGACAGGA<br><br>241 T P P A Y L I N R M P S H E A R N Y I Q<br>721 ACACCCCCCGCTTATCTCATTAACAGGATGCCAAGCCATGAGGCAAGAAACTATATTCAG<br><br>261 S L T Q M P K M N F A N V F I G A N P L<br>781 TCTTTGACTCAGATGCCGAAGATGAACTTTGCGAATGTATTTATTGGTGCCAATCCCCTG<br><br>281 A V D L L E K M L V L D S D K R I T A A<br>841 GCTGTCGACTTGCTGGAGAAGATGCTTGTATTGGACTCAGATAAGAGAATTACAGCGGCC | |

| Gene Name | GenBank Homology | DNA SEQUENCE / DEDUCED AMINO ACID SEQUENCE | SEQUENCE IDENTIFIER: |
|---|---|---|---|
| | | 301 Q A L A H A Y F A Q Y H D P D D E P V A<br>901 CAAGCCCTTGCACATGCCTACTTTGCTCAGTACCACGATCCTGATGATGAACCAGTGGCC<br><br>321 D P Y D Q S F E S R D L L I D E W K S L<br>961 GATCCTTATGATCAGTCCTTTGAAAGCAGGGACCTCCTTATAGATGAGTGGAAAAGCCTG<br><br>341 T Y D E V I S F V P P P L D Q E E M E S<br>1021 ACCTATGATGAAGTCATCAGCTTTGTGCCACCACCCCTTGACCAAGAAGAGATGGAGTCC<br><br>361 -<br>1081 TGA | |
| WBC020H11.bFSP_20 011110.abd | Homo sapiens nuclear factor (erythroid-derived 2)-like 2, mRNA | 1 GCAGCCGCCACCGCCGCCGCCGCCGCCACCAGAGCCGCCCTGTCCGCGCCGCGCCTCGGC<br>61 AGCCGGAACAGGGCCGCCGTCGGGGAGCCCCAGAGCACGGTCCCCACCTCAGTATGATGG<br>121 ACTTGGAGGTGCCGCCGCCCGCACAGCCGTCCCAGCAGGACATGGATTTGATTGACATAC<br>181 TATGGAGGCAAGATATAGATCTTGGTGTAAGTCGAGAAGTATTTGACTTCACTCAACGAC<br>241 TACAGCATGAGCTGGAAAAACAGAAAACACTTGAAAGACAAGAAAGACAAGAACAACTCC<br>301 AAAAGGAGCAAGAGAAAGCCTTTTTCGCTCAGTTGCAACTAGATGAAGAGACAGGTGAAT<br>361 TCCTCCCGATTCAGCCAGCCCAACACATCTCATCAGAAACCAGTGGGTCTGCCAACTTCT<br>421 CCCAGGTTTCCCACATTCCCAAACCAGATGCTCTGAACTTCGATGACTGCATGCAGCTTT<br>481 TGGCAGAGACATTCCCGTTTGTAGATGACAATGAGGTTTCTCCAGCTACGTTTCAATCAC<br>541 TTGTTCCTGATAGTCCCAGCAACATCGAGAGCCCAGACTTCATTGCTCCTAACCAGGCCC<br>601 AGTCACCTGAAACTCTTGTCCTTCAGCCAGCCGTTGCTGATTCAGACAATATGCAGCAGG<br>661 ACCTTGAGCAAGTTTGGGAGGAGCTATTATCCATTCCAGAATTACAGTGTCTTAACATTC<br>721 AAAACGACAAGCTGGTTGAGACTAGCACGGTTCCAAGTTCAGAAACCAAAGTGACAGAAA<br>781 TTGACAACAATTACCATTTCTACTCATCGATCCCCTCACTGGAAAAAGAAGTAGGTAACT<br>841 GCAGTCCACAGCTTCTCAATGCTTTTGACGATTCCTTCAGCAGCATCCTCTCCACAGAAG<br>901 ACACCACCCAGTTGACAATGAACTCATTAAATTCAGATGCCACAGTCAACACAGATTTTG<br>961 GTGATGAATTTTATTCTGCTTTCATAGCTGAGCCCAGTATCAGCAACAGCATGCCCTCAC<br>1021 CTGCTACTTTAAGCCATTCACTCTCTGAACTTCTAAATGGGCCCATTGATGTTTCTGATC<br>1081 TATCACTTTGCAAAGCTTTCAACCAAAACCACCCTGAAAGCACAGCAGAATTCAATGATT<br>1141 CTGACTCCGGCATTTCACTAAACACAAGTCCCAGTGTGGCATCACCAGAACACTCAGTGG<br>1201 AATCTTCCAGCTATGGAGACACACTACTTGGCCTCAGTGATTCTGAAGTGGAAGAGCTAG<br>1261 ATAGTGCCCCTGGAAGTGTCAAACAGAATGGTCCTAAAACACCAGTACATTCTTCTGGGG<br>1321 ATATGGTACAACCCTTGTCACCATCTCAGGGGCAGAGCACTCACGTGCATGATGCCCAAT<br>1381 GTGAGAACACACCAGAGAAAGAATTGCCTGTAAGTCCTGGTCATCGGAAAACCCCATTCA<br>1441 CAAAAGACAAACATTCAAGCCGCTTGGAGGCTCATCTCACAAGAGATGAACTTAGGGCAA<br>1501 AAGCTCTCCATATCCCATTCCCTGTAGAAAAAATCATTAACCTCCCTGTTGTTGACTTCA<br>1561 ACGAAATGATGTCCAAAGAGCAGTTCAATGAAGCTCAACTTGCATTAATTCGGGATATAC<br>1621 GTAGGAGGGGTAAGAATAAAGTGGCTGCTCAGAATTGCAGAAAAAGAAAACTGGAAAATA<br>1681 TAGTAGAACTAGAGCAAGATTTAGATCATTTGAAAGATGAAAAAGAAAAATTGCTCAAAG | SEQ ID NO:90 |

| Gene Name | GenBank Homology | DNA SEQUENCE / DEDUCED AMINO ACID SEQUENCE | SEQUENCE IDENTIFIER: |
|---|---|---|---|
| | | 1741 AAAAAGGAGAAAATGACAAAAGCCTTCACCTATTGAAAAAACGACTCAGCACCTTGTATC<br>1801 TCGAAGTCTTCAGCATGCTACGTGACGAAGATGGAAGACCTTACTCCCCCAGCGAATACT<br>1861 CCCTGCAGCAGACGAGAGACGGCAGCGTGTTCCTTGTTCCCAAAAGTAAGAAGCCAGATG<br>1921 TTAAGAAAAACTAGATTTAGGAGGATTTGACCTTTTCTGAGCTAGTTTTTTTGTACTGTT<br>1981 ATACTAAAAGCTCCTACTGTGATGTGAAATGCAGAAATATACTTTATAAGTAATTCTATG<br>2041 CAAATTTATAGTCAAAACTAGTGTAGAAAATATAAAACTTTAAAAAGCATTGGAGTGTCA<br>2101 GTATGCTGAATCAGTAGTTTCACTTTAACTGCAAACTTAATTTCTTAGAACACCATTTGG<br>2161 GCTAGTTTCTGTGTAAGTGTAAATACTACAAAAACTTTATTTATACTGTTCTTATCTCAT<br>2221 TTGTTACGTTCCTAGATTTATATGATACATCTGGCTAAAAAGCAAATTGTTGCAAAACTA<br>2281 ACCACTATGTACTTTTTTATAAATACTGTATGGACAAAAAATGGCATTTTTTATATTAAA<br>2341 TTGTTTAGCTCTGGCAAAAAAAAAAAATTTTAAGAGCTGGTACTAATAAAGGATTATTAT<br>2401 GACTGTTAAAAAAAAAAAAAAAAAAAAAAAAAAAAAAAAA | |
| | | 1 M M D L E V P P P A Q P S Q Q D M D L I<br>1 ATGATGGACTTGGAGGTGCCGCCGCCCGCACAGCCGTCCCAGCAGGACATGGATTTGATT<br><br>21 D I L W R Q D I D L G V S R E V F D F T<br>61 GACATACTATGGAGGCAAGATATAGATCTTGGTGTAAGTCGAGAAGTATTTGACTTCACT<br><br>41 Q R L Q H E L E K Q K T L E R Q E R Q E<br>121 CAACGACTACAGCATGAGCTGGAAAAACAGAAAACACTTGAAAGACAAGAAAGACAAGAA<br><br>61 Q L Q K E Q E K A F F A Q L Q L D E E T<br>181 CAACTCCAAAAGGAGCAAGAGAAAGCCTTTTTCGCTCAGTTGCAACTAGATGAAGAGACA<br><br>81 G E F L P I Q P A Q H I S S E T S G S A<br>241 GGTGAATTCCTCCCGATTCAGCCAGCCCAACACATCTCATCAGAAACCAGTGGGTCTGCC<br><br>101 N F S Q V S H I P K P D A L N F D D C M<br>301 AACTTCTCCCAGGTTTCCCACATTCCCAAACCAGATGCTCTGAACTTCGATGACTGCATG<br><br>121 Q L L A E T F P F V D D N E V S P A T F<br>361 CAGCTTTTGGCAGAGACATTCCCGTTTGTAGATGACAATGAGGTTTCTCCAGCTACGTTT<br><br>141 Q S L V P D S P S N I E S P D F I A P N<br>421 CAATCACTTGTTCCTGATAGTCCCAGCAACATCGAGAGCCCAGACTTCATTGCTCCTAAC<br><br>161 Q A Q S P E T L V L Q P A V A D S D N M<br>481 CAGGCCCAGTCACCTGAAACTCTTGTCCTTCAGCCAGCCGTTGCTGATTCAGACAATATG<br><br>181 Q Q D L E Q V W E E L L S I P E L Q C L<br>541 CAGCAGGACCTTGAGCAAGTTTGGGAGGAGCTATTATCCATTCCAGAATTACAGTGTCTT | SEQ ID NO:91 |

| Gene Name | GenBank Homology | DNA SEQUENCE / DEDUCED AMINO ACID SEQUENCE | SEQUENCE IDENTIFIER: |
|---|---|---|---|
| | | 201 N I Q N D K L V E T S T V P S S E T K V<br>601 AACATTCAAAACGACAAGCTGGTTGAGACTAGCACGGTTCCAAGTTCAGAAACCAAAGTG<br><br>221 T E I D N N Y H F Y S S I P S L E K E V<br>661 ACAGAAATTGACAACAATTACCATTTCTACTCATCGATCCCCTCACTGGAAAAAGAAGTA<br><br>241 G N C S P Q L L N A F D D S F S S I L S<br>721 GGTAACTGCAGTCCACAGCTTCTCAATGCTTTTGACGATTCCTTCAGCAGCATCCTCTCC<br><br>261 T E D T T Q L T M N S L N S D A T V N T<br>781 ACAGAAGACACCACCCAGTTGACAATGAACTCATTAAATTCAGATGCCACAGTCAACACA<br><br>281 D F G D E F Y S A F I A E P S I S N S M<br>841 GATTTTGGTGATGAATTTTATTCTGCTTTCATAGCTGAGCCCAGTATCAGCAACAGCATG<br><br>301 P S P A T L S H S L S E L L N G P I D V<br>901 CCCTCACCTGCTACTTTAAGCCATTCACTCTCTGAACTTCTAAATGGGCCCATTGATGTT<br><br>321 S D L S L C K A F N Q N H P E S T A E F<br>961 TCTGATCTATCACTTTGCAAAGCTTTCAACCAAAACCACCCTGAAAGCACAGCAGAATTC<br><br>341 N D S D S G I S L N T S P S V A S P E H<br>1021 AATGATTCTGACTCCGGCATTTCACTAAACACAAGTCCCAGTGTGGCATCACCAGAACAC<br><br>361 S V E S S S Y G D T L L G L S D S E V E<br>1081 TCAGTGGAATCTTCCAGCTATGGAGACACACTACTTGGCCTCAGTGATTCTGAAGTGGAA<br><br>381 E L D S A P G S V K Q N G P K T P V H S<br>1141 GAGCTAGATAGTGCCCCTGGAAGTGTCAAACAGAATGGTCCTAAAACACCAGTACATTCT<br><br>401 S G D M V Q P L S P S Q G Q S T H V H D<br>1201 TCTGGGGATATGGTACAACCCTTGTCACCATCTCAGGGGCAGAGCACTCACGTGCATGAT<br><br>421 A Q C E N T P E K E L P V S P G H R K T<br>1261 GCCCAATGTGAGAACACACCAGAGAAAGAATTGCCTGTAAGTCCTGGTCATCGGAAAACC<br><br>441 P F T K D K H S S R L E A H L T R D E L<br>1321 CCATTCACAAAAGACAAACATTCAAGCCGCTTGGAGGCTCATCTCACAAGAGATGAACTT<br><br>461 R A K A L H I P F P V E K I I N L P V V<br>1381 AGGGCAAAAGCTCTCCATATCCCATTCCCTGTAGAAAAAATCATTAACCTCCCTGTTGTT<br><br>481 D F N E M M S K E Q F N E A Q L A L I R | |

| Gene Name | GenBank Homology | DNA SEQUENCE / DEDUCED AMINO ACID SEQUENCE | SEQUENCE IDENTIFIER: |
|---|---|---|---|
| | | 1441 GACTTCAACGAAATGATGTCCAAAGAGCAGTTCAATGAAGCTCAACTTGCATTAATTCGG<br><br>501 D I R R R G K N K V A A Q N C R K R K L<br>1501 GATATACGTAGGAGGGGTAAGAATAAAGTGGCTGCTCAGAATTGCAGAAAAAGAAAACTG<br><br>521 E N I V E L E Q D L D H L K D E K E K L<br>1561 GAAAATATAGTAGAACTAGAGCAAGATTTAGATCATTTGAAAGATGAAAAAGAAAAATTG<br><br>541 L K E K G E N D K S L H L L K K R L S T<br>1621 CTCAAAGAAAAAGGAGAAAATGACAAAAGCCTTCACCTATTGAAAAAACGACTCAGCACC<br><br>561 L Y L E V F S M L R D E D G R P Y S P S<br>1681 TTGTATCTCGAAGTCTTCAGCATGCTACGTGACGAAGATGGAAGACCTTACTCCCCCAGC<br><br>581 E Y S L Q Q T R D G S V F L V P K S K K<br>1741 GAATACTCCCTGCAGCAGACGAGAGACGGCAGCGTGTTCCTTGTTCCCAAAAGTAAGAAG<br><br>601 P D V K K N -<br>1801 CCAGATGTTAAGAAAAACTAG | |
| WBC021E02.bFSP_20 011210.abd | No homology. | 1 GGACCTTACCTTCAAGCACCAATGAGAGCTCAGAATGGAGGAAAAAACTATGAACGGAAG<br>61 GAATGTTCAAGATCTTTTATTCATTCCGCAAGCCTCGGTGCGCATGTACAAACTCACACT<br>121 GCTAACAACCATTACAAATGTAAGGAATGCAGAAAATCCTTTAAACGATCTGCATACCTT<br>181 AACGCTCATGTTCGAATTCACACTGGAAGAAAACCCTTTAAATGTAAGGAATGCAGGAAA<br>241 GGCTTTAAACGTTCTATGCACCTTAAGGTTCACATGCGAACCCACACTGGAGAGAAATCC<br>301 TATGAATGTAAGGAATGTGGGAAAACCTTCACTCAATCCTCAGGCCTCATTTACCACAAT<br>361 AAGATTCACACTGGAGAGAAGCCTTTTAAATGTGACACATGTGGGAAAGCCTATGCTAGT<br>421 TTCTCAAATCTTACTGCCCATTTTAGAACTCACACTGGAGAGAAGCGTTTTGAGTGTAAT<br>481 GTATGCAGGAAAAGATTTAGCAGTTCCTCATACTTAGTCGTTCACAACCGCACTCACACT<br>541 GGAGAGAAACCCTATAAATGTGAGGAATGTGGGAAGGGCTTTAAATGTTCTGTGTCCCTT<br>601 AAAGTTCACATGCGGTTTCACACTGGAGAGAAACCCTATGAATGCAAGAAATGTGGGAGA<br>661 GCCTTCACTCAGTCCTCAAGCCTTACTGACCATAGGAAAACTCATACTGGAGAGAATCCT<br>721 TTTAAATGTGACGCATGTGGGAAAGCCTTTGCTCTTTCCTCACATCTTAACAGACAT | SEQ ID NO:92 |
| | | 1 GCCTATTGAGTGTAATGTATGTGGGAAGACATTTAGCAGTTTTTTGTACTTGATCGTTCA<br>61 CAAGCGTACTCACGCTGGAGAGAAACCTTAGAAACGTAAAGAATGTAGGGAAAGCCTTCA<br>121 GTTTTCCCATTTCCTTTTGGTACCATGAAAGCACTTACCTGCAGAGAAATCCTGTCAGTG<br>181 TAAAGAATGTGGTAAAGCCCTCAGTTGTCCCAATTCACTTTGAAGACGTGAAAGAACCAC<br>241 ACTGGAGAGATGCTCTAGAAATGTAAGGAATGAGGGAAAGCCATGAGAATCTCATCACAA<br>301 CCTGGCATGTATGGACTAACAGTGAAAGCATACCATTAAGAAACATGGGAAGCCCTTCAC<br>361 TGTTTCCTCAGTGAAATATATTTATTTTTATAAATGAGACACCTTGTAATGGAGAGAAAT<br>421 CCAAGTGGTGTAATGTGGGAAAATTGTTCTAGTAATGTTCACTTGTTTTTTGTGTGGAGA | SEQ ID NO:93 |

| Gene Name | GenBank Homology | DNA SEQUENCE / DEDUCED AMINO ACID SEQUENCE | SEQUENCE IDENTIFIER: |
|---|---|---|---|
| | | 481 AAAACTTTATGAATGTAAGAAATGTTGTATAGTCATTATCATTTTTCGTAAGATTGTTTC<br>541 TCTTCCTTAATAAACGTATGGTAATTCAAATTAAAAAAAAAAAAAAAA | |
| WBC021E07.bFSP_20 011210.abd | Homo sapiens vav 1 oncogene, mRNA (cDNA clone MGC:15356 IMAGE:3842829) | 1 gtggtgcacg cctatagtct cagctactca ggaagttgag gcaggaggat cgcttgagcc<br>61 caggagtttg atgctgcagt gagctatgat tgtgctacca ctgtactcca gactggacaa<br>121 aagattcctg tgccttaaga acattagaac cttcctgtcc acctgctgtg agaagttcgg<br>181 cctcaagcgg agcgagctct tcgaagcctt tgacctcttc gatgtgcagg attttggcaa<br>241 ggtcatctac accctgtctg ctctgtcctg gaccccgatc gcccagaaca gggggatcat<br>301 gcccttcccc accgaggagg agagtgtagg tgatgaagac atctacagtg gcctgtccga<br>361 ccagatcgac gacacggtgg aggaggatga ggacctgtat gactgcgtgg agaatgagga<br>421 ggcggaaggc gacgagatct atgaggacct catgcgctcg gagcccgtgt ccatgccgcc<br>481 caagatgaca gagtatgaca agcgctgctg ctgcctgcgg gagatccagc agacggagga<br>541 gaagtacact gacacgctgg gctccatcca gcagcatttc ttgaagcccc tgcaacggtt<br>601 cctgaaacct caagacattg agatcatctt tatcaacatt gaggacctgc ttcgtgttca<br>661 tactcacttc ctaaaggaga tgaaggaagc cctgggcacc cctggcgcag ccaatctcta<br>721 ccaggtcttc atcaaataca aggagaggtt cctcgtctat ggccgctact gcagccaggt<br>781 ggagtcagcc agcaaacacc tggaccgtgt ggccgcagcc cgggaggacg tgcagatgaa<br>841 gctggaggaa tgttctcaga gagccaacaa cgggaggttc accctgcggg acctgctgat<br>901 ggtgcctatg cagcgagttc tcaaatatca cctccttctc caggagctgg tgaaacacac<br>961 gcaggaggcg atggagaagg agaacctgcg gctggccctg gatgccatga gggacctggc<br>1021 tcagtgcgtg aacgaggtca agcgagacaa cgagacactg cgacagatca ccaatttcca<br>1081 gctgtccatt gagaacctgg accagtctct ggctcactat ggccggccca agatcgacgg<br>1141 ggaactcaag atcacctcgg tggaacggcg ctccaagatg gacaggtatg ccttcctgct<br>1201 cgacaaagct ctactcatct gtaagcgcag gggagactcc tatgacctca aggactttgt<br>1261 aaacctgcac agcttccagg ttcgggatga ctcttcagga gaccgagaca acaagaagtg<br>1321 gagccacatg ttcctcctga tcgaggacca aggtgcccag ggctatgagc tgttcttcaa<br>1381 gacaagagaa ttgaagaaga agtggatgga gcagtttgag atggccatct ccaacatcta<br>1441 tccggagaat gccaccgcca acgggcatga cttccagatg ttctcctttg aggagaccac<br>1501 atcctgcaag gcctgtcaga tgctgcttag aggtaccttc tatcagggct accgctgcca<br>1561 tcggtgccgg gcatctgcac acaaggagtg tctggggagg gtccctccat gtggccgaca<br>1621 tgggcaagat ttcccaggaa ctatgaagaa ggacaaacta catcgcaggg ctcaggacaa<br>1681 aaagaggaat gagctgggtc tgcccaagat ggaggtgttt caggaatact acgggcttcc<br>1741 tccacccccct ggagccattg gaccctttct acggctcaac cctggagaca ttgtggagct<br>1801 cacgaaggct gaggctgaac agaactggtg ggagggcaga aatacatcta ctaatgaaat<br>1861 tggctggttt ccttgtaaca gggtgaagcc ctatgtccat ggccctcctc aggacctgtc<br>1921 tgttcatctc tggtacgcag gccccatgga gcgggcaggg gcagagagca tcctggccaa<br>1981 ccgctcggac gggactttct tggtgcggca gagggtgaag gatgcagcag aatttgccat<br>2041 cagcattaaa tataacgtcg aggtcaagca cattaaaatc atgacagcag aaggactgta<br>2101 ccggatcaca gagaaaaagg ctttccgggg gcttacggag ctggtggagt tttaccagca<br>2161 gaactctcta aaggattgct tcaagtctct ggacaccacc ttgcagttcc ccttcaagga<br>2221 gcctgaaaag agaaccatca gcaggccagc agtgggaagc acaaagtatt ttggcacagc<br>2281 caaagcccgc tatgacttct gcgcccgaga ccgatcagag ctgtcgctca aggagggtga | SEQ ID NO:94 |

| Gene Name | GenBank Homology | DNA SEQUENCE / DEDUCED AMINO ACID SEQUENCE | SEQUENCE IDENTIFIER: |
|---|---|---|---|
| | | 2341 catcatcaag atccttaaca agaagggaca gcaaggctgg tggcgagggg agatctatgg<br>2401 ccgggttggc tggttccctg ccaactacgt ggaggaagat tattctgaat actgctgagc<br>2461 cctggtgcct tggcagagag acgagaaact ccaggctctg agcccggcgt gggcaggcag<br>2521 cggagccagg ggctgtgaca gctcccggcg ggtggagact ttgggatgga ctggaggagg<br>2581 ccagcgtcca gctggcggtg ctcccgggat gtgccctgac atggttaatt tataacaccc<br>2641 cgatttcctc ttgggtcccc tcaagcagac ggggctcaag ggggttacat ttaataaaag<br>2701 gatgaagatg gaaaaaaaaa aaaaaaaaaa aaaaaaaaaa aaaaaaaaaa aaaaaaaaaa<br>2761 aaaaaaaaaa aaaaaa | |
| | | 1 M I V L P L Y S R L D K R F L C L K N I<br>1 ATGATTGTGCTACCACTGTACTCCAGACTGGACAAAAGATTCCTGTGCCTTAAGAACATT<br><br>21 R T F L S T C C E K F G L K R S E L F E<br>61 AGAACCTTCCTGTCCACCTGCTGTGAGAAGTTCGGCCTCAAGCGGAGCGAGCTCTTCGAA<br><br>41 A F D L F D V Q D F G K V I Y T L S A L<br>121 GCCTTTGACCTCTTCGATGTGCAGGATTTTGGCAAGGTCATCTACACCCTGTCTGCTCTG<br><br>61 S W T P I A Q N R G I M P F P T E E E S<br>181 TCCTGGACCCCGATCGCCCAGAACAGGGGGATCATGCCCTTCCCCACCGAGGAGGAGAGT<br><br>81 V G D E D I Y S G L S D Q I D D T V E E<br>241 GTAGGTGATGAAGACATCTACAGTGGCCTGTCCGACCAGATCGACGACACGGTGGAGGAG<br><br>101 D E D L Y D C V E N E E A E G D E I Y E<br>301 GATGAGGACCTGTATGACTGCGTGGAGAATGAGGAGGCGGAAGGCGACGAGATCTATGAG<br><br>121 D L M R S E P V S M P P K M T E Y D K R<br>361 GACCTCATGCGCTCGGAGCCCGTGTCCATGCCGCCCAAGATGACAGAGTATGACAAGCGC<br><br>141 C C C L R E I Q Q T E E K Y T D T L G S<br>421 TGCTGCTGCCTGCGGGAGATCCAGCAGACGGAGGAGAAGTACACTGACACGCTGGGCTCC<br><br>161 I Q Q H F L K P L Q R F L K P Q D I E I<br>481 ATCCAGCAGCATTTCTTGAAGCCCCTGCAACGGTTCCTGAAACCTCAAGACATTGAGATC<br><br>181 I F I N I E D L L R V H T H F L K E M K<br>541 ATCTTTATCAACATTGAGGACCTGCTTCGTGTTCATACTCACTTCCTAAAGGAGATGAAG<br><br>201 E A L G T P G A A N L Y Q V F I K Y K E<br>601 GAAGCCCTGGGCACCCCTGGCGCAGCCAATCTCTACCAGGTCTTCATCAAATACAAGGAG<br><br>221 R F L V Y G R Y C S Q V E S A S K H L D | SEQ ID NO:95 |

| Gene Name | GenBank Homology | DNA SEQUENCE / DEDUCED AMINO ACID SEQUENCE | SEQUENCE IDENTIFIER: |
|---|---|---|---|
| | | 661 AGGTTCCTCGTCTATGGCCGCTACTGCAGCCAGGTGGAGTCAGCCAGCAAACACCTGGAC<br><br>241 R V A A A R E D V Q M K L E E C S Q R A<br>721 CGTGTGGCCGCAGCCCGGGAGGACGTGCAGATGAAGCTGGAGGAATGTTCTCAGAGAGCC<br><br>261 N N G R F T L R D L L M V P M Q R V L K<br>781 AACAACGGGAGGTTCACCCTGCGGGACCTGCTGATGGTGCCTATGCAGCGAGTTCTCAAA<br><br>281 Y H L L L Q E L V K H T Q E A M E K E N<br>841 TATCACCTCCTTCTCCAGGAGCTGGTGAAACACACGCAGGAGGCGATGGAGAAGGAGAAC<br><br>301 L R L A L D A M R D L A Q C V N E V K R<br>901 CTGCGGCTGGCCCTGGATGCCATGAGGGACCTGGCTCAGTGCGTGAACGAGGTCAAGCGA<br><br>321 D N E T L R Q I T N F Q L S I E N L D Q<br>961 GACAACGAGACACTGCGACAGATCACCAATTTCCAGCTGTCCATTGAGAACCTGGACCAG<br><br>341 S L A H Y G R P K I D G E L K I T S V E<br>1021 TCTCTGGCTCACTATGGCCGGCCCAAGATCGACGGGGAACTCAAGATCACCTCGGTGGAA<br><br>361 R R S K M D R Y A F L L D K A L L I C K<br>1081 CGGCGCTCCAAGATGGACAGGTATGCCTTCCTGCTCGACAAAGCTCTACTCATCTGTAAG<br><br>381 R R G D S Y D L K D F V N L H S F Q V R<br>1141 CGCAGGGGAGACTCCTATGACCTCAAGGACTTTGTAAACCTGCACAGCTTCCAGGTTCGG<br><br>401 D D S S G D R D N K K W S H M F L L I E<br>1201 GATGACTCTTCAGGAGACCGAGACAACAAGAAGTGGAGCCACATGTTCCTCCTGATCGAG<br><br>421 D Q G A Q G Y E L F F K T R E L K K K W<br>1261 GACCAAGGTGCCCAGGGCTATGAGCTGTTCTTCAAGACAAGAGAATTGAAGAAGAAGTGG<br><br>441 M E Q F E M A I S N I Y P E N A T A N G<br>1321 ATGGAGCAGTTTGAGATGGCCATCTCCAACATCTATCCGGAGAATGCCACCGCCAACGGG<br><br>461 H D F Q M F S F E E T T S C K A C Q M L<br>1381 CATGACTTCCAGATGTTCTCCTTTGAGGAGACCACATCCTGCAAGGCCTGTCAGATGCTG<br><br>481 L R G T F Y Q G Y R C H R C R A S A H K<br>1441 CTTAGAGGTACCTTCTATCAGGGCTACCGCTGCCATCGGTGCCGGGCATCTGCACACAAG<br><br>501 E C L G R V P P C G R H G Q D F P G T M<br>1501 GAGTGTCTGGGGAGGGTCCCTCCATGTGGCCGACATGGGCAAGATTTCCCAGGAACTATG | |

| Gene Name | GenBank Homology | DNA SEQUENCE / DEDUCED AMINO ACID SEQUENCE | SEQUENCE IDENTIFIER: |
|---|---|---|---|
| | | 521 K K D K L H R R A Q D K K R N E L G L P<br>1561 AAGAAGGACAAACTACATCGCAGGGCTCAGGACAAAAAGAGGAATGAGCTGGGTCTGCCC<br><br>541 K M E V F Q E Y Y G L P P P P G A I G P<br>1621 AAGATGGAGGTGTTTCAGGAATACTACGGGCTTCCTCCACCCCCTGGAGCCATTGGACCC<br><br>561 F L R L N P G D I V E L T K A E A E Q N<br>1681 TTTCTACGGCTCAACCCTGGAGACATTGTGGAGCTCACGAAGGCTGAGGCTGAACAGAAC<br><br>581 W W E G R N T S T N E I G W F P C N R V<br>1741 TGGTGGGAGGGCAGAAATACATCTACTAATGAAATTGGCTGGTTTCCTTGTAACAGGGTG<br><br>601 K P Y V H G P P Q D L S V H L W Y A G P<br>1801 AAGCCCTATGTCCATGGCCCTCCTCAGGACCTGTCTGTTCATCTCTGGTACGCAGGCCCC<br><br>621 M E R A G A E S I L A N R S D G T F L V<br>1861 ATGGAGCGGGCAGGGGCAGAGAGCATCCTGGCCAACCGCTCGGACGGGACTTTCTTGGTG<br><br>641 R Q R V K D A A E F A I S I K Y N V E V<br>1921 CGGCAGAGGGTGAAGGATGCAGCAGAATTTGCCATCAGCATTAAATATAACGTCGAGGTC<br><br>661 K H I K I M T A E G L Y R I T E K K A F<br>1981 AAGCACATTAAAATCATGACAGCAGAAGGACTGTACCGGATCACAGAGAAAAAGGCTTTC<br><br>681 R G L T E L V E F Y Q Q N S L K D C F K<br>2041 CGGGGGCTTACGGAGCTGGTGGAGTTTTACCAGCAGAACTCTCTAAAGGATTGCTTCAAG<br><br>701 S L D T T L Q F P F K E P E K R T I S R<br>2101 TCTCTGGACACCACCTTGCAGTTCCCCTTCAAGGAGCCTGAAAAGAGAACCATCAGCAGG<br><br>721 P A V G S T K Y F G T A K A R Y D F C A<br>2161 CCAGCAGTGGGAAGCACAAAGTATTTTGGCACAGCCAAAGCCCGCTATGACTTCTGCGCC<br><br>741 R D R S E L S L K E G D I I K I L N K K<br>2221 CGAGACCGATCAGAGCTGTCGCTCAAGGAGGGTGACATCATCAAGATCCTTAACAAGAAG<br><br>761 G Q Q G W W R G E I Y G R V G W F P A N<br>2281 GGACAGCAAGGCTGGTGGCGAGGGGAGATCTATGGCCGGGTTGGCTGGTTCCCTGCCAAC<br><br>781 Y V E E D Y S E Y C -<br>2341 TACGTGGAGGAAGATTATTCTGAATACTGCTGA | |

| Gene Name | GenBank Homology | DNA SEQUENCE / DEDUCED AMINO ACID SEQUENCE | SEQUENCE IDENTIFIER: |
|---|---|---|---|
| WBC022B03.bFSP_20011210.abd | Homo sapiens T-cell receptor interacting molecule, mRNA (cDNA clone MGC:34314 IMAGE:5227396). | 1 GATAAAGATAAGTTTTACTGTCATGCTGCTTTTAACATAACAGAGCAACATCACCTAGGA<br>61 AAAAAGTTTGTAGGAGGATTTTTAATCCATACACTTGCCTTATGATTACATTAAGATTTC<br>121 TTTGAGAAAAGAAGCATGTCAGGAAGCTCTGGATGCCCTTTTTCTGTCTGGGCAATTCTG<br>181 GCCTTCTTGGGTTTAGCTCTTGTTGTATCGCTGATCTTCAATATTTCCTGCTATGTGGAA<br>241 AAGCAGCGACAAGGTATAACATACAGGTTCTCTGATGACTACATTTCCCGGGAGGATGAG<br>301 TATTACATTGAAGACACACCCATTTATGGTAACTTAGATAATGTGATCCCAGAACCAATG<br>361 GATGAAAACTGCTATGAGCAAATGAAAGCCCGACCAGAGAGATCTGTGAATCAACTGCGA<br>421 GCAGCTCCCACACCTGCACAGGCAACCAATGAAGCACAGATGTTCTACTCCTCCCTGGAG<br>481 CACAGCCTTGAGGGGAAGCGCAGAAAGCCCAGGAAACACGATCCTTATTTGCCAGACAAG<br>541 GTTGAAGATGAGCAGTTACACGCGACGGATGCCAGCCTTTCTATGACTGCTTTGGTTGAT<br>601 AGTTTTGCACCTGAGGACCAGGAAGAAGAGGAAAACATCCATGATGATCCCATCAGGCTG<br>661 TTCGGATTGATTCGTGCGAAGAAAGAACCTATGAACTAGCTGGACTCTGATCTAGCTCAA<br>721 TGATTCAGCTCTTATTGGAGATGGCTTCTAAGAAAACAAGATGCACAGAGGACACAGAAG<br>781 GACTTGGCAGCAGGGTGATGACCTGATCATTTGTTGATGGGATGGTGGCTTACCTCTTAT<br>841 TCACAGCTTACACTTATGCATGCCAAATGTAAGGCCATGAAAATCAGTATTTCAAATAAC<br>901 TTAAAAAATGCTTTACTACTAAAATGTAAAAAATTAATGTGCTCACCTCGGCAGCACATA<br>961 TACTAAAAATTAATAAGACCCAGCTTGAAAATTGAGCCTGATAACAAGATTACAAATTCA<br>1021 CAATACCTAATACTTAGGGAAATATAAAAATTTAAGCATGAATGTGTTCTGGAACACGTT<br>1081 AGAAGAAAAATAAAAGCCAATGAGTTTTTTTTAATTCTCCTTTCTCACCAATGGGCAAT<br>1141 AGCCCATAATTGAAATAAATTTCTGATTGAAAGGTATAGGAAACATTAAAATGCATTACT<br>1201 AAGAGAAGTAATATAATTTTCTTACAAAGTATTTTTCCCAAAGATAGCTTTACTATTTCA<br>1261 AAAATTGTCAAATTAATGCATGCTCCTTACAACAAACAAATATCAAAAAGAGTTTAGGAA<br>1321 TTCTACTAGCCAGAGATAGTCACTTGGAGAAACTTTCTATATATCCTTCTAAATATTTTT<br>1381 CTGGGCATGCTTATGTATGTACATCAGTTGTTTCTTTTTATTTTGAACCAAAAATGTGGT<br>1441 TTCTTTTGTACACATTACTTAAACTTTCTTTCCAGTCAACAATATATTGTGGATTTATTT<br>1501 TCACTGTTATATTTAACTATATATAAATACGCATATATTGTAATTTTAATGTCTGCTTAG<br>1561 CACCCCACTGATAACCAAATCACAGTTTATTTAAATAATTTTAATGACTTTTCAAAAACA<br>1621 ATTTATTGATGCAAAAAGCAAGGTTGAGATGACAATGTTTCTTTCAATAATTAAAAAATA<br>1681 CTGCTTCAAAAAAAAAAAAAAAAAAAAAAAAAAAAAAAAAA | SEQ ID NO:96 |
| | | 1 M S G S S G C P F S V W A I L A F L G L<br>1 ATGTCAGGAAGCTCTGGATGCCCTTTTTCTGTCTGGGCAATTCTGGCCTTCTTGGGTTTA<br><br>21 A L V V S L I F N I S C Y V E K Q R Q G<br>61 GCTCTTGTTGTATCGCTGATCTTCAATATTTCCTGCTATGTGGAAAAGCAGCGACAAGGT<br><br>41 I T Y R F S D D Y I S R E D E Y Y I E D<br>121 ATAACATACAGGTTCTCTGATGACTACATTTCCCGGGAGGATGAGTATTACATTGAAGAC<br><br>61 T P I Y G N L D N V I P E P M D E N C Y<br>181 ACACCCATTTATGGTAACTTAGATAATGTGATCCCAGAACCAATGGATGAAAACTGCTAT | SEQ ID NO:97 |

| Gene Name | GenBank Homology | DNA SEQUENCE / DEDUCED AMINO ACID SEQUENCE | SEQUENCE IDENTIFIER: |
|---|---|---|---|
| | | 81 E Q M K A R P E R S V N Q L R A A P T P<br>241 GAGCAAATGAAAGCCCGACCAGAGAGATCTGTGAATCAACTGCGAGCAGCTCCCACACCT<br><br>101 A Q A T N E A Q M F Y S S L E H S L E G<br>301 GCACAGGCAACCAATGAAGCACAGATGTTCTACTCCTCCCTGGAGCACAGCCTTGAGGGG<br><br>121 K R R K P R K H D P Y L P D K V E D E Q<br>361 AAGCGCAGAAAGCCCAGGAAACACGATCCTTATTTGCCAGACAAGGTTGAAGATGAGCAG<br><br>141 L H A T D A S L S M T A L V D S F A P E<br>421 TTACACGCGACGGATGCCAGCCTTTCTATGACTGCTTTGGTTGATAGTTTTGCACCTGAG<br><br>161 D Q E E E E N I H D D P I R L F G L I R<br>481 GACCAGGAAGAAGAGGAAAACATCCATGATGATCCCATCAGGCTGTTCGGATTGATTCGT<br><br>181 A K K E P M N -<br>541 GCGAAGAAAGAACCTATGAACTAG | |
| WBC022C04.bFSP_20 011210.abd | PREDICTED: Bos taurus similar to Caspase recruitment domain protein 8 (Apoptotic protein NDPP1) (DACAR) (CARD-inhibitor of NF-kappaB activating ligand) (CARDINAL) (Tumor-upregulated CARD-containing antagonist of CASP9) (TUCAN) (LOC511229), partial mRNA | < 1 gtgaggacag cagctttgag agcggttctg aagctgaatc tgcccatgtc actgatattg<br>61 ctcgaggaca tagtaattac aagtgtcctt ttggtgaagt accgaagagt acttatatgg<br>121 atagcggctg tggaataagg ttgaaagatt ttctggtgca ggacttggat cggtgtttca<br>181 gaaccttcag atctatgggg tccccaaccc ctgggccact gtctggtacc attaggagcc<br>241 gggccacaca gcaggaggtg agcggtgagc tagcgagcaa agcctcatct gtatttacag<br>301 ccacttcccg ttgcttgcgt taccgcctga gtgccacctc ctgtcagagc agcggtggga<br>361 caagattgtc atgggaatac agaccctact gtgaactgcc catggaacta ggttgcccac<br>421 tcgggaaaca caatgaactg gtgacccccca ggcttctttc ccgaggacag tttttgctga<br>481 aggtggatgg agagggaact taccagtgca cggagactgg tctgatattt gaggttaacg<br>541 ggaaagttga tatcaagtat tgcgtcttgt cctggagcaa atattcagac ctcattgtga<br>601 agccctgggc cgtcggtgga cccttgtttg atgttaaatg tgacccgctc tgtcttacct<br>661 ccattcagtt tccacattcc ctctgcttgg gtcaccgaga tgccaacatg acgttcaaag<br>721 tcttccatgt taaaagtact ggggcctcat tagaatatac tgtggaccat tctgcaaccc<br>781 acgtcaaatg gcgtgtgagc tctctttctc ctgtgggacc cgttgttcaa agcgaagaga<br>841 cggtattcca ccacggggca gtgattctgt acaaagccat cgaccataac ccgtccttgt<br>901 cttttcgagt gtatgtagca accaataatg aatccttcat caaggatatt tccaagtctg<br>961 taaagcactc ctctaagaaa ttcatgaaaa tcgacaagcc tccggtttgc cagaagttgc<br>1021 tgcagaatgg gaagaagtac aggctgatca gtgagccaga agctgagatc accccagagg<br>1081 aaattgaatt tgtggatggt tcacttttaa aattgaaaag ttatattgaa gtgtatctgg<br>1141 agcaaccagt ggagtttaaa ttactcttgg ttgaaatgga ttctgaggaa attgtatgga<br>1201 aagcaaaact gagagagtgt gactgggttc agcatcatca gaaccagaac atttcaaaaa<br>1261 gcagcacaag tggtaacagg cgacgaaaaa tgagtagcag cttacctgat gagatggtct<br>1321 atgataaaag aatgaagcag attgacagtt caggtttgta aacatgtcct ctgtggctct | SEQ ID NO:98 |

| Gene Name | GenBank Homology | DNA SEQUENCE / DEDUCED AMINO ACID SEQUENCE | SEQUENCE IDENTIFIER: |
|---|---|---|---|
| | | 1 E D S S F E S G S E A E S A H V T D I A<br>3 GAGGACAGCAGCTTTGAGAGCGGTTCTGAAGCTGAATCTGCCCATGTCACTGATATTGCT<br><br>21 R G H S N Y K C P F G E V P K S T Y M D<br>63 CGAGGACATAGTAATTACAAGTGTCCTTTTGGTGAAGTACCGAAGAGTACTTATATGGAT<br><br>41 S G C G I R L K D F L V Q D L D R C F R<br>123 AGCGGCTGTGGAATAAGGTTGAAAGATTTTCTGGTGCAGGACTTGGATCGGTGTTTCAGA<br><br>61 T F R S M G S P T P G P L S G T I R S R<br>183 ACCTTCAGATCTATGGGGTCCCCAACCCCTGGGCCACTGTCTGGTACCATTAGGAGCCGG<br><br>81 A T Q Q E V S G E L A S K A S S V F T A<br>243 GCCACACAGCAGGAGGTGAGCGGTGAGCTAGCGAGCAAAGCCTCATCTGTATTTACAGCC<br><br>101 T S R C L R Y R L S A T S C Q S S G G T<br>303 ACTTCCCGTTGCTTGCGTTACCGCCTGAGTGCCACCTCCTGTCAGAGCAGCGGTGGGACA<br><br>121 R L S W E Y R P Y C E L P M E L G C P L<br>363 AGATTGTCATGGGAATACAGACCCTACTGTGAACTGCCCATGGAACTAGGTTGCCCACTC<br><br>141 G K H N E L V T P R L L S R G Q F L L K<br>423 GGGAAACACAATGAACTGGTGACCCCCAGGCTTCTTTCCCGAGGACAGTTTTTGCTGAAG<br><br>161 V D G E G T Y Q C T E T G L I F E V N G<br>483 GTGGATGGAGAGGGAACTTACCAGTGCACGGAGACTGGTCTGATATTTGAGGTTAACGGG<br><br>181 K V D I K Y C V L S W S K Y S D L I V K<br>543 AAAGTTGATATCAAGTATTGCGTCTTGTCCTGGAGCAAATATTCAGACCTCATTGTGAAG<br><br>201 P W A V G G P L F D V K C D P L C L T S<br>603 CCCTGGGCCGTCGGTGGACCCTTGTTTGATGTTAAATGTGACCCGCTCTGTCTTACCTCC<br><br>221 I Q F P H S L C L G H R D A N M T F K V<br>663 ATTCAGTTTCCACATTCCCTCTGCTTGGGTCACCGAGATGCCAACATGACGTTCAAAGTC<br><br>241 F H V K S T G A S L E Y T V D H S A T H<br>723 TTCCATGTTAAAAGTACTGGGGCCTCATTAGAATATACTGTGGACCATTCTGCAACCCAC<br><br>261 V K W R V S S L S P V G P V V Q S E E T<br>783 GTCAAATGGCGTGTGAGCTCTCTTTCTCCTGTGGGACCCGTTGTTCAAAGCGAAGAGACG<br><br>281 V F H H G A V I L Y K A I D H N P S L S | SEQ ID NO:99 |

| Gene Name | GenBank Homology | DNA SEQUENCE / DEDUCED AMINO ACID SEQUENCE | SEQUENCE IDENTIFIER: |
|---|---|---|---|
| | | 843 GTATTCCACCACGGGGCAGTGATTCTGTACAAAGCCATCGACCATAACCCGTCCTTGTCT<br><br>301 F R V Y V A T N N E S F I K D I S K S V<br>903 TTTCGAGTGTATGTAGCAACCAATAATGAATCCTTCATCAAGGATATTTCCAAGTCTGTA<br><br>321 K H S S K K F M K I D K P P V C Q K L L<br>963 AAGCACTCCTCTAAGAAATTCATGAAAATCGACAAGCCTCCGGTTTGCCAGAAGTTGCTG<br><br>341 Q N G K K Y R L I S E P E A E I T P E E<br>1023 CAGAATGGGAAGAAGTACAGGCTGATCAGTGAGCCAGAAGCTGAGATCACCCCAGAGGAA<br><br>361 I E F V D G S L L K L K S Y I E V Y L E<br>1083 ATTGAATTTGTGGATGGTTCACTTTTAAAATTGAAAAGTTATATTGAAGTGTATCTGGAG<br><br>381 Q P V E F K L L L V E M D S E E I V W K<br>1143 CAACCAGTGGAGTTTAAATTACTCTTGGTTGAAATGGATTCTGAGGAAATTGTATGGAAA<br><br>401 A K L R E C D W V Q H H Q N Q N I S K S<br>1203 GCAAAACTGAGAGAGTGTGACTGGGTTCAGCATCATCAGAACCAGAACATTTCAAAAAGC<br><br>421 S T S G N R R R K M S S S L P D E M V Y<br>1263 AGCACAAGTGGTAACAGGCGACGAAAAATGAGTAGCAGCTTACCTGATGAGATGGTCTAT<br><br>441 D K R M K Q I D S S G L -<br>1323 GATAAAAGAATGAAGCAGATTGACAGTTCAGGTTTGTAA | |
| WBC022D03.bFSP_20 011210.abd | No homology | 1 GCTGCGAAGCTCCTTTACATTGGGTCTGTAAGAAGGTCAGACTTTGACAGATGGTCAGAT<br>61 CTGTCATATGACTCCCTTAAAAGGGGGAGCTGGCCTCTGGGTGTTGGGATAGCCATGAGT<br>121 ATATATATATATATATATAGTGAATGCTGAACCTTTGAGGATATGGCATTAGATGCAAGA<br>181 TTAAATGCAGCTTCCCCAGGGTTGATGGTTTATTGGTGGACTTTGAGAGTATTTAGATAT<br>241 TATCTTAACAATTTAAAAACCAGATCTAGGCAAATTTGTGAAATAGATAAAGTTTTTTTT<br>301 AGTATTTTTCACTCTAGTGACTAATGCTGCTTCCTTCATCCTCACCCCCACCATCTCAGT<br>361 CAAAAATATACCTTTTATATGCTATATTCTGAGCTGATCTAATGAAATCTGTGCAAATAT<br>421 ATACTATTGCTAATGTAGCAGAGGGTTTTTATGTGCATTAATGGGTTGACATGTTACTTT<br>481 TCTCTGATAATATATTTTTATCCTAACAGTGGCCCTTTGCAGATAAAGCATTATTCTAAA<br>541 GAATAATGCTAATGGTGAGAAAATAAATTATGAAAGTAGATGAGAGCAGGGTGATGCTGT<br>601 CTGGTCTTCAGCTCTTCACACTTACTTAAGACCTGGCCTTGACTGAATTTAGTTTTTCTG<br>661 AATATGCGTAGATGTGGTTGAATGAAACAATGTGTGACTATATTTTTTCCATGTTGTGAA<br>721 CATGTGATTTGCATGTTGATTTTACAACAGTGGTCTCTTTGACAAGGCATTTTTGTAGAG<br>781 GAAAATTTACGGAAGCAGTTTATGTGGTTCATTCTCAAATTATTTTAGGCACTAATGCCC<br>841 TCAAAAATGAGAAAAACTTATTAATTTTTCTTTA | SEQ ID NO:100 |

| Gene Name | GenBank Homology | DNA SEQUENCE / DEDUCED AMINO ACID SEQUENCE | SEQUENCE IDENTIFIER: |
|---|---|---|---|
| | | 1 GGGTTTTTATGTGCATTAATGGGTTGACATGTTACTTTTCTCTGATAATATATTTTTATC<br>61 CTAACAGTGGCCCTTTGCAGATAAAGCATTATTCTAAAGAATAATGCTAATGGTGAGAAA<br>121 ATAAATTATGAAAGTAGATGAGAGCAGGGTGATGCTGTCTGGTCTTCAGCTCTTCACACT<br>181 TACTTAAGACCTGGCCTTGACTGAATTTAGTTTTTCTGAATATGCGTAGATGTGGTTGAA<br>241 TGAAACAATGTGTGACTATATTTTTTCCATGTTGTGAACATGTGATTTGCATGTTGATTT<br>301 TACAACAGTGGTCTCTTTGACAAGGCATTTTTGTAGAGGAAAATTTACGGAAGCAGTTTA<br>361 TGTGGTTGATTCTCAAATTATTTTAGGCACTAATGCCTCAAAAATGAGAAAAACTTATTA<br>421 TTTTTCTTTAGTAGGATTTGGACATAGATAAGGAGGGAAGATGTTGTTTAAACATATTGG<br>481 AGGGAGTAATGAAAGGGACGATACTTCTTACTGTTGTTGACGCTGGATTGTAGTCTGTTT<br>541 ACTTGTCTTTCTCTTTCGTAGTTTTTAAGGACGTGGACTGTATCTTATCCACCATTGTAT<br>601 CTCAGACACCTAAAATAGTGTTTGTATCAATGAATATTAAATAAATGAATGGATTAATAA<br>661 AAAAAAAAGGGGGGCCCGTACCCAT | SEQ ID NO:101 |
| WBC023F12.bFSP_20 011510.abd | No significant homology | 1 GTTAAACATTATTTCCTTAAAAAAATTTATTTTTACATGAAAACAAAATATATTTGGAGT<br>61 GTACTTCCATCAAAAATTTCACTTCTATATCTGAGGTCTACTTATTTCAAAGTAAAAATT<br>121 TTCACTTGTCAGCTGAAATTTCTGATTAGAACTAATATTTGTGTATTTTTGTGCTTAGTC<br>181 AGAATGTAAATTTCACAGTGGATTTAAATTTATCCTTAATTTGGATAAAAGCAAGGATA<br>241 TATTTAAAAGTTATTATAGGGATGAAAGTGGAAATTTCTGTTTTTGGTTTTTTTTTAACT<br>301 ACTAATGAGATCACGTTTGTAGTTCTTTTTACATGTGTTTAGCACAGGCTCTAAATTAAA<br>361 AAATACAGTATAGTCTGTGTTGACCTTAAAAATATTCCATAAATTAATAAATATATGGCA<br>421 AGTTTTTCATAGCTAAATCTAACTCTTGTAGAAGGAAGCTGTGCCTTCTGTTTAATTACA<br>481 TTAATTGAAATGTGTTTAAGAGAAATGTTTTCAGCATATTTTGTATACTAAAAAACAAAA<br>541 AGGATTAGTATTGGGCCAATGGCCAAGAGGTAATATTACTACCATGTAGACTGTTACAGT<br>601 TCAAATTGTCCCACTTCCCCCAGAGTTTTAGAAGCTAGAAGTCTGGGAGATACTGTATCA<br>661 GCTGTAGTTGGGTGATTCTAAGTGCTATAAGTACTATTCATCTTTTAATTGTGTGAGATG<br>721 GAACAAATGCCAAGTTGCACAAGATGCATACTTTGAATATGTAATGGTTTGTTTCAGGTG<br>781 AAAAATACCAGCAAGTCATGTCTTGTGTATTTTACTTATATTTCCTTGTAAAACTAAATT<br>841 AAGTAAGATTTTGGAAAATGATTTGAATTGATTGCTGACTTGACCTGCCTGGTCAAATAT<br>901 CTGAATGATGGCCTTACTGGGTAAAT | SEQ ID NO:102 |
| | | 1 AATATTACTACCATGTAGACTGTTACAGTTCAAATTGTCCCACTTCCCCCAGAGTTTTAG<br>61 AAGCTAGAAGTCTGGGAGATACTGTATCAGCTGTAGTTGGGTGATTCTAAGTGCTATAAG<br>121 TACTATTCATCTTTTAATTGTGTGAGATGGAACAAATGCCAAGTTGCACAAGATGCATAC<br>181 TTTGAATATGTAATGGTTTGTTTCAGGTGAAAAATACCAGCAAGTCATGTCTTGTGTATT<br>241 TTACTTATATTTCCTTGTAGAACTAAATTAAGTAAGATTTTGGAAAATGATTTGAATTGA<br>301 TTGCTGACTTGACCTGCCTGGTCAAATATCTGAATGATGGCATTACTGGGTAGATTCTAA<br>361 CCCATGATTCTAACTGTGGAATAAAGAGTAGTATAGACCGTAAAACTATGTTCAGGCACT<br>421 TACTCTTCGTTTATATTATGTATTACCCTCTATCATAACACACTTATTGAATTTCCAAGT<br>481 TGAACAGTTCTGTAACTATTGTTGCTATTCATGTAATAAAACATGCTTATGATAGTAAAA<br>541 TGAATAGAAGTGCCCCCAAATGCTATTTTTTTTAATTCACTTGTAACTGTTACTCTTGTA<br>601 ATTGTGTATGACATAAAATTTGTAAAAATTTTAGCATGAAAAAAAAAAAA | SEQ ID NO:103 |

| Gene Name | GenBank Homology | DNA SEQUENCE / DEDUCED AMINO ACID SEQUENCE | SEQUENCE IDENTIFIER: |
|---|---|---|---|
| WBC024B05.bFSP_20011510.abd | Adducin 3 (gamma) (ADD3), transcript variant 2 | 1 GAGAGAAGCTGCTGCTGCTAATGCTGCTGCTGCTGCTGCCGCCGCCGCTGCTGCTGC<br>61 TGCTGTTGGTCTGAGGCTGCAGTAGGTTTCTGTGCAGCATTGCAGAATCCACACCTAGAG<br>121 AACAGAAGACACAGACACGTACGTCTACTACCCTTGTTAGAAGGAAGCTTTGGATCTTCG<br>181 GTGGATAACAAGAGTAATCCACAGACTTAAAACATGAGCTCAGATGCCAGCCAAGGCGTG<br>241 ATTACCACTCCTCCTCCTCCCAGCATGCCTCACAAAGAGAGATATTTTGACCGCATCAAT<br>301 GAAAATGACCCAGAATACATTAGGGAGAGGAACATGTCTCCTGATCTACGACAAGACTTC<br>361 AACATGATGGAGCAGAGGAAACGAGTTACTCAGATCCTGCAAAGTCCTGCCTTTCGGGAA<br>421 GACTTGGAATGCCTTATTCAAGAACAGATGAAGAAAGGCCACAACCCAACTGGATTACTA<br>481 GCATTACAGCAGATTGCAGATTACATCATGGCCAATTCTTTCTCGGGTTTTTCTTCACCT<br>541 CCTCTCAGTCTTGGCATGGTCACACCTATCAATGACCTTCCTGGTGCAGATACATCCTCA<br>601 TATGTGAAGGGAGAAAAACTTACTCGCTGTAAACTTGCCAGCCTGTACAGACTTGTAGAC<br>661 TTGTTTGGATGGGCACACCTGGCAAATACCTATATCTCAGTAAGAATAAGTAAGGAGCAA<br>721 GACCACATTATAATAATTCCCAGAGGCCTATCTTTTTCTGAAGCTACAGCCTCCAATTTG<br>781 GTGAAAGTCAATATAATAGGAGAAGTGGTTGACCAGGGAAGTACCAATTTGAAAATTGAC<br>841 CATACAGGATTCAGTCCCCATGCTGCAATCTATTCAACACGTCCTGATGTTAAGTGTGTG<br>901 ATACACATCCATACCCTTGCAACAGCAGCTGTATCCTCCATGAAATGTGGGATCCTTCCA<br>961 ATTTCTCAAGAGTCTCTTCTTCTGGGAGATGTTGCCTATTATGACTACCAAGGGTCACTT<br>1021 GAAGAACAGGAGGAGAGAATTCAACTGCAGAAGGTTCTGGGACCAAGTTGTAAGGTGCTG<br>1081 GTACTCAGGAATCATGGTGTGGTTGCACTTGGAGAAACATTAGAGGAGGCTTTTCATTAT<br>1141 ATTTTTAATGTGCAACTAGCCTGTGAGATTCAGGTGCAGGCCCTAGCAGGTGCAGGTGGA<br>1201 GTAGACAATCTCCATGTACTGGACTTTCAGAAGTATAAAGCTTTCACTTACACTGTAGCA<br>1261 GCGTCTGGTGGAGGAGGTGTGAATATGGGTTCCCATCAAAAATGGAAGGTTGGCGAAATT<br>1321 GAGTTTGAAGGGCTTATGAGGACTCTGGACAACTTGGGGTATAGAACAGGCTATGCTTAC<br>1381 AGGCATCCTCTCATTCGAGAGAAGCCTAGGCACAAGAGTGATGTGGAAATCCCAGCAACT<br>1441 GTGACTGCTTTTTCCTTTGAAGACGATACAGTGCCACTCTCTCCTCTCAAATACATGGCA<br>1501 CAGAGGCAACAGCGTGAAAAAACAAGATGGCTGAACTCACCAAATACTTACATGAAAGTG<br>1561 AATGTGCCTGAGGAGTCTCGGAACGGAGAAACCAGTCCCCGAACCAAAATCACGTGGATG<br>1621 AAAGCAGAAGACTCATCTAAAGTTAGTGGTGGAACACCTATCAAAATTGAAGATCCAAAT<br>1681 CAGTTTGTTCCTTTAAACACAAACCCGAATGAGGTACTAGAAAAGAGAAATAAGATTCGG<br>1741 GAACAAAATCGATATGACTTGAAAACAGCAGGACCACAATCTCAGTTGCTTGCTGGAATT<br>1801 GTTGTGGATAAGCCACCTTCTACTATGCAATTTGAAGATGATGATCATGGCCCACCAGCT<br>1861 CCTCCTAACCCATTTAGTCATCTCACAGAAGGAGAACTTGAAGAGTATAAGAGGACAATC<br>1921 GAACGTAAACAACAAGGCCTAGAAGAAAACCATGAGCTGTTTTCCAAGAGCTTCATCTCC<br>1981 ATGGAAGTGCCTGTCATGGTAGTAAATGGCAAGGATGATATGCATGATGTTGAAGATGAG<br>2041 CTTGCTAAGCGAGTGAGTAGGTTAAGCACAAGTACAACCATAGAAAACATCGAGATTACT<br>2101 ATTAAGTCTCCAGAGAAAATCGAAGAAGTCCTGTCACCTGAAGGCTCCCCTTCAAAATCG<br>2161 CCATCCAAGAAAAAGAAGAAATTCCGCACTCCTTCTTTTCTGAAAAAGAACAAAAAAAAG<br>2221 GAGAAAGTTGAGGCCTAAATAAAGTCTTTTTATAATTATTATTATAACAATGTGACATTG<br>2281 CACATCTAAATACCACATTTAAGTTGATCATTAATATGCAATGGTAGATCAGATTGGGGG<br>2341 ATGTAGCAAACTGGACTTTAAGAACTGGAAAGAGGTTTTACAAAAGAAAAACTTTCAGAT<br>2401 TCATCTCTCATTTTATATGTCCAGAAATGGCTTTGAATTTTAAGCAATTACTAGTTTTAA<br>2461 TTAGCTCTGCCCTCATGAAGTATTATTATAATTCACCATAAACAGCTATCTGTCTGAATT | SEQ ID NO:104 |

| Gene Name | GenBank Homology | DNA SEQUENCE / DEDUCED AMINO ACID SEQUENCE | SEQUENCE IDENTIFIER: |
|---|---|---|---|
| | | 2521 ACTTCAGGCCTTCTCCATAATATCTGTTAGAAAGAAATTGCCAGTGAGCAAGTGAGAATT<br>2581 TTTATTTCTCAATACCTGCTTCACTTGATAATCATATTATAATTTTTTATCATGATTATT<br>2641 GACTATATTTTTGGAGTCCCATTGTTTCAGTGGGCATTAACAGAATGCTTTAAAAACTTC<br>2701 TAAGACAAGAATCTATAGCATTAGTATACACTGGCACATAATTTTTTAAAAAGTTTTAAG<br>2761 AAAAGATTCATTTGGAATTTTATTCACAGTATAAAATTTCCTCACCTGAAGTAACTTTGT<br>2821 TTGCCAAAAAAGTTGTTTTAATAAACTATAATTTTTGAAAACTTCCTTTTTTATTAGTTT<br>2881 AGAAAGCCCCTTATTTTTCAACAAAGGGGATTTTGTACACATAACATGGGTTATTTAGTT<br>2941 TAACTCTGGCAAAAAAAAAAAAAAAAAATTTTGTATGTTGATGTTTGTATACCGTTCAGTA<br>3001 TAAAAGTGTCCTAAGCATATTAGCCAATCTTTTCACAGTAGAGCATACTTAAGGCTGCTT<br>3061 GGTACTGAGTATACTTAAATATAACTCCAGAATCCAGGGACTTGGTGTTAAAACAGGATT<br>3121 AGAGCATGTAAAGGTACATCTAGATTCATATTTGAATCTTAAACTGTATTTTTCTCTTAG<br>3181 TATTGCTAATGAGTAAAGAAAAGTCTCATAAGGTAGCCAAATGAAAAAGAATGAAAGGGA<br>3241 AAGTGAAAAATTAAGGGGACAAAAGATGGGATGTGAAAAGAAGAATTCTAGTTTGATGGT<br>3301 GACTCATATTCACGATAGGATACAAAGTGTGATTTGTTGGAAACATGTCCCAAATTTCTA<br>3361 AAATTCTGCTTCTCTGCCAAAAGCAATGTCTTTCTTGGTTGATATTTGAGTTTTAAAAGG<br>3421 GTCAAATCTTTCTAATTTTTTGTATCTTTAGAGGGCAGCACTAGAAGAAATCAGCAGGTC<br>3481 TAATCCCACCAGTAAGAAAACTACCACTTCTTGATTTTTACAGATTTAAAAAAATCTTTT<br>3541 CAGTGACCTTTCTTTTTAATGTAAATACAAATTTAAACCTAGGCTTAATATAGGCGTTTC<br>3601 CCCTTTCACCCAAGTGATGTCACAGTTCGATGCAAAATCAATGATCCAGAATGATCGTGG<br>3661 GTAAAAATAACTCAAAGTGTTTCTTAAGGGTGAGTTGGCATGCAAAAAATTACATTGATT<br>3721 ACAGTGTGTTTTGGAGCTGGCTCTGTTTGTGTGCATATGATAATGCAGAGTTGAGCCAGA<br>3781 GCCTGGAAATGTCATTCTAGATCTCACTAACTACTGGAATCAGTGTTTTAATCTCTTGGT<br>3841 GGAAACTTTCAGTTGCTTAACTCTCTATTGGAAGATTTTTTTAATGTTCTACATCATTTA<br>3901 TGTTGTATTACAATGTATGTAGAAATAGTAACCTGTGAACTATGCTTTTCCATAACTTTT<br>3961 TAAAAATATATATATCTAAATGAATGCAATGTGCATAAATATTTTTTAAACATAACAGTG<br>4021 AACTATTGCACCTTTTGCTAATGCCTCTATTTACTTGCTTTGGCATAAAGAATGAGCCAA<br>4081 TGAACCTCTGTGTCCTGTGGAAAAATGTATAAATGTTATCTGATATTGCTCTTAGATGTA<br>4141 ATGCTAATTAATGTTAAATCACAAATAAACAGTATTTTAAATATAAAAAAAAAAA | |
| | | 1 M S S D A S Q G V I T T P P P P S M P H<br>1 ATGAGCTCAGATGCCAGCCAAGGCGTGATTACCACTCCTCCTCCTCCCAGCATGCCTCAC<br><br>21 K E R Y F D R I N E N D P E Y I R E R N<br>61 AAAGAGAGATATTTTGACCGCATCAATGAAAATGACCCAGAATACATTAGGGAGAGGAAC<br><br>41 M S P D L R Q D F N M M E Q R K R V T Q<br>121 ATGTCTCCTGATCTACGACAAGACTTCAACATGATGGAGCAGAGGAAACGAGTTACTCAG<br><br>61 I L Q S P A F R E D L E C L I Q E Q M K<br>181 ATCCTGCAAAGTCCTGCCTTTCGGGAAGACTTGGAATGCCTTATTCAAGAACAGATGAAG<br><br>81 K G H N P T G L L A L Q Q I A D Y I M A<br>241 AAAGGCCACAACCCAACTGGATTACTAGCATTACAGCAGATTGCAGATTACATCATGGCC | SEQ ID NO:105 |

| Gene Name | GenBank Homology | DNA SEQUENCE / DEDUCED AMINO ACID SEQUENCE | SEQUENCE IDENTIFIER: |
|---|---|---|---|
| | | 101 N S F S G F S S P P L S L G M V T P I N<br>301 AATTCTTTCTCGGGTTTTTCTTCACCTCCTCTCAGTCTTGGCATGGTCACACCTATCAAT<br><br>121 D L P G A D T S S Y V K G E K L T R C K<br>361 GACCTTCCTGGTGCAGATACATCCTCATATGTGAAGGGAGAAAAACTTACTCGCTGTAAA<br><br>141 L A S L Y R L V D L F G W A H L A N T Y<br>421 CTTGCCAGCCTGTACAGACTTGTAGACTTGTTTGGATGGGCACACCTGGCAAATACCTAT<br><br>161 I S V R I S K E Q D H I I I I P R G L S<br>481 ATCTCAGTAAGAATAAGTAAGGAGCAAGACCACATTATAATAATTCCCAGAGGCCTATCT<br><br>181 F S E A T A S N L V K V N I I G E V V D<br>541 TTTTCTGAAGCTACAGCCTCCAATTTGGTGAAAGTCAATATAATAGGAGAAGTGGTTGAC<br><br>201 Q G S T N L K I D H T G F S P H A A I Y<br>601 CAGGGAAGTACCAATTTGAAAATTGACCATACAGGATTCAGTCCCCATGCTGCAATCTAT<br><br>221 S T R P D V K C V I H I H T L A T A A V<br>661 TCAACACGTCCTGATGTTAAGTGTGTGATACACATCCATACCCTTGCAACAGCAGCTGTA<br><br>241 S S M K C G I L P I S Q E S L L L G D V<br>721 TCCTCCATGAAATGTGGGATCCTTCCAATTTCTCAAGAGTCTCTTCTTCTGGGAGATGTT<br><br>261 A Y Y D Y Q G S L E E Q E E R I Q L Q K<br>781 GCCTATTATGACTACCAAGGGTCACTTGAAGAACAGGAGGAGAGAATTCAACTGCAGAAG<br><br>281 V L G P S C K V L V L R N H G V V A L G<br>841 GTTCTGGGACCAAGTTGTAAGGTGCTGGTACTCAGGAATCATGGTGTGGTTGCACTTGGA<br><br>301 E T L E E A F H Y I F N V Q L A C E I Q<br>901 GAAACATTAGAGGAGGCTTTTCATTATATTTTTAATGTGCAACTAGCCTGTGAGATTCAG<br><br>321 V Q A L A G A G G V D N L H V L D F Q K<br>961 GTGCAGGCCCTAGCAGGTGCAGGTGGAGTAGACAATCTCCATGTACTGGACTTTCAGAAG<br><br>341 Y K A F T Y T V A A S G G G G V N M G S<br>1021 TATAAAGCTTTCACTTACACTGTAGCAGCGTCTGGTGGAGGAGGTGTGAATATGGGTTCC<br><br>361 H Q K W K V G E I E F E G L M R T L D N<br>1081 CATCAAAAATGGAAGGTTGGCGAAATTGAGTTTGAAGGGCTTATGAGGACTCTGGACAAC | |

| Gene Name | GenBank Homology | DNA SEQUENCE / DEDUCED AMINO ACID SEQUENCE | SEQUENCE IDENTIFIER: |
|---|---|---|---|
| | | 381 L G Y R T G Y A Y R H P L I R E K P R H<br>1141 TTGGGGTATAGAACAGGCTATGCTTACAGGCATCCTCTCATTCGAGAGAAGCCTAGGCAC<br><br>401 K S D V E I P A T V T A F S F E D D T V<br>1201 AAGAGTGATGTGGAAATCCCAGCAACTGTGACTGCTTTTTCCTTTGAAGACGATACAGTG<br><br>421 P L S P L K Y M A Q R Q Q R E K T R W L<br>1261 CCACTCTCTCCTCTCAAATACATGGCACAGAGGCAACAGCGTGAAAAAACAAGATGGCTG<br><br>441 N S P N T Y M K V N V P E E S R N G E T<br>1321 AACTCACCAAATACTTACATGAAAGTGAATGTGCCTGAGGAGTCTCGGAACGGAGAAACC<br><br>461 S P R T K I T W M K A E D S S K V S G G<br>1381 AGTCCCCGAACCAAAATCACGTGGATGAAAGCAGAAGACTCATCTAAAGTTAGTGGTGGA<br><br>481 T P I K I E D P N Q F V P L N T N P N E<br>1441 ACACCTATCAAAATTGAAGATCCAAATCAGTTTGTTCCTTTAAACACAAACCCGAATGAG<br><br>501 V L E K R N K I R E Q N R Y D L K T A G<br>1501 GTACTAGAAAAGAGAAATAAGATTCGGGAACAAAATCGATATGACTTGAAAACAGCAGGA<br><br>521 P Q S Q L L A G I V V D K P P S T M Q F<br>1561 CCACAATCTCAGTTGCTTGCTGGAATTGTTGTGGATAAGCCACCTTCTACTATGCAATTT<br><br>541 E D D D H G P P A P P N P F S H L T E G<br>1621 GAAGATGATGATCATGGCCCACCAGCTCCTCCTAACCCATTTAGTCATCTCACAGAAGGA<br><br>561 E L E E Y K R T I E R K Q Q G L E E N H<br>1681 GAACTTGAAGAGTATAAGAGGACAATCGAACGTAAACAACAAGGCCTAGAAGAAAACCAT<br><br>581 E L F S K S F I S M E V P V M V V N G K<br>1741 GAGCTGTTTTCCAAGAGCTTCATCTCCATGGAAGTGCCTGTCATGGTAGTAAATGGCAAG<br><br>601 D D M H D V E D E L A K R V S R L S T S<br>1801 GATGATATGCATGATGTTGAAGATGAGCTTGCTAAGCGAGTGAGTAGGTTAAGCACAAGT<br><br>621 T T I E N I E I T I K S P E K I E E V L<br>1861 ACAACCATAGAAAACATCGAGATTACTATTAAGTCTCCAGAGAAAATCGAAGAAGTCCTG<br><br>641 S P E G S P S K S P S K K K K K F R T P<br>1921 TCACCTGAAGGCTCCCCTTCAAAATCGCCATCCAAGAAAAAGAAGAAATTCCGCACTCCT<br><br>661 S F L K K N K K K E K V E A - | |

| Gene Name | GenBank Homology | DNA SEQUENCE / DEDUCED AMINO ACID SEQUENCE | SEQUENCE IDENTIFIER: |
|---|---|---|---|
| | | 1981 TCTTTTCTGAAAAAGAACAAAAAAAAGGAGAAAGTTGAGGCCTAA | |
| WBC024F08.bFSP_20<br>011510.abd | No homology | 1 GGATAGTGCAGCATCCTAGAGCTTTGGTGTCACACAGACCCAGGTTCGAGTCTTCACTTT<br>61 GCCACCTCTTGCCTGCCGAGGGGCTGTGAGCTGAATTTCCTACCTCATAGGGTTGTAATG<br>121 AGCAGTGGATGCTGAAGCAGACACCTCTTGGTCAAGGTGACTGCTCATCCCAGATTCTCC<br>181 CTTTTTCTGAGAATTTGTTCCCCTCAGTCCTCATATAGGAGCTGAATGGATGAGAGGCGA<br>241 GCATCTGGCTGTACTTGGGCCAGTCTGGTTCTCTCTCTCAGGAATAAGCAGCGAGACTCA<br>301 GGGATGCTGTTTCCAGCTCTGCAAGTCACTTACCTGAAGATATACAAACTCTGGAGCTGT<br>361 GAGCGTGTGTCTACATGTGTGTCTCTATGTACCCAAGCCTTGGGGGTGGTGGCGGCAGGG<br>421 GGTGGCCACCTGCATGAGTGTATTAGAGAAAATGGCTCGACTCAGAGAGAAGATGGAGAT<br>481 GTGCAGAGCAAATCAGAGACCAGAGGGAGACCCCAGGAGCCTGGAGAGGCCAGGGGGAGG<br>541 CGCTTCCCGGAGCCTGCCGGCGCTCCTCCTCTGGGCCAAACCCTTCTCGAGCCTCAAAGT<br>601 CCGCCCTCCTTCCTTTGAGTCCTGTGAGATGCCCTTGTTAGGATGCATTCTCTTGTTTTG<br>661 CTTGAGCTGATTTGAAGTATTATCTATTACTTGCAACCAAAAGAGCTCTGAATAAGATAA<br>721 AATCTTGTTGCTAAAGCACAGTGTCTCTCGAATTTCGGTCATCCCTGTACCCTCCTCACA<br>781 ATTTTTGCATATCTATGTACCATCTGTATGTTTTACTAATAAATATTGTTCTTTAAATCT<br>841 TTAAAAAAAAAAAAAAAAA | *SEQ ID NO:106* |
| WBC024G03.bFSP_20<br>011510.abd | Horse serpin mRNA. | 1 CCCCCGCCGCCGGGACCGTCAGCCGCGCGCCGCCTTCGCCATGGAGCAGCTGAGCACAGC<br>61 CAACACCCACTTTGCGGTGGACTTGTTCCGCGCTTTGAATGAGAGCGATCCTACTGGAAA<br>121 CATCTTCATCTCTCCCTTAAGCATTTCCTCAGCGCTGGCCATGATCTTTCTGGGGACCAG<br>181 AGGCAACACAGCGGCACAGGTGTCCAAGGCACTTTATTTTGATACTGTTGAGGATATTCA<br>241 TTCAAGATTTCAGAGTCTGAATGCTGATATCAACAAACCTGGAGCTCCCTATATTCTGAA<br>301 ACTCGCTAATAGATTATATGGAGAGAAAACCTATAATTTCCTCGCTGATTTCTTAGCTTC<br>361 AACTCAGAAAATGTATGGTGCTGAATTGGCCAGTGTGGATTTTCAGCAAGCCCCTGAAGA<br>421 TGCAAGAAAGGAGATAAATGAATGGGTCAAAGGACAGACAGAGGGGAAAATTCCGGAACT<br>481 GTTGGTCAAGGGTATGGTTGATAACATGACTAAACTTGTACTGGTGAATGCCATCTACTT<br>541 CAAGGGAAACTGGCAGGAGAAATTCATGAAAGAGGCCACCAGAGACGCCCCATTCCGATT<br>601 GAATAAGAAAGACACAAAAACCGTGAAAATGATGTATCAGAAGAAGAAATTTCCATACAA<br>661 CTACATCGAGGACCTCAAGTGCCGTGTGCTGGAGCTGCCTTACCAAGGCAAAGAGCTCAG<br>721 CATGATCATCCTGCTGCCAGACGACATTGAGGATGAGTCCACGGGCCTGGAGAAGATTGA<br>781 GAAACAGTTGACTTTGGAAAAACTGCGTGAGTGGACCAAACCTGAGAATCTGTATCTCGC<br>841 TGAAGTCAATGTCCACTTGCCCAGGTTCAAGCTGGAAGAGAGCTACGATCTCACCTCCCA<br>901 TCTAGCCCGCCTAGGTGTACAAGACCTCTTTAACAGGGGCAAAGCTGATCTGTCTGGCAT<br>961 GTCAGGGGCCAGAGATCTTTTCGTATCGAAAATTATCCACAAATCCTTTGTGGACCTGAA<br>1021 TGAGGAGGGTACAGAGGCTGCGGCAGCCACAGCAGGCACGATCATGCTTGCCATGCTGAT<br>1081 GCCAGAGGAAAATTTCAATGCCGACCATCCATTCATTTTCTTCATTCGGCACAATCCCTC<br>1141 AGCTAACATCCTGTTCTTAGGCAGATTTTCTTCCCCTTAGAGAAGGTGGAAGTACAAGGT<br>1201 GAAGCTTAAGCTTAGAGCTTTATTACCTGCTAGTATTATTACCATTACTTTTAATAGTGA<br>1261 CAGTTTTCATATATCTTTACCAATAAAACTACTATCCAAAAACAAATCTTTAATTTCCTT | SEQ ID NO:107 |

<table>
<tr><th>Gene Name</th><th>GenBank Homology</th><th>DNA SEQUENCE / DEDUCED AMINO ACID SEQUENCE</th><th>SEQUENCE IDENTIFIER:</th></tr>
<tr><td></td><td></td><td>1321 TGTAAGTTCAGCTCTATTGGCTGTTGCACCCCTTAACTTTGGCGTTTTTTTATGTTGAAA<br>1381 AAATCTAGTTATTGCTTTTGGATGCATCAAGTTAAAAAATAAAAAGACTAAATCAAATTT<br>1441 ATAGATTCTTGATAATGCAGTAAGCTATGAAACTACTATACTCTCCTGATAGTGGTGGGA<br>1501 AACATTATAGTACTGTAATTTATTTTACAGATAGACTTTTGGAAGCCATAATAGAGAGAC<br>1561 GCTCTGATTGTTGAAGGAAGGCTTAAAAACAGATATTCAATTGAAATATAGTAAAGCACC<br>1621 CCAATTAAGGCCTGGGTTGTGCAGACAAACTCGTCTTGACCGTTGTTTCTAACCCTTTGT<br>1681 TCCACAGGGTTAGTGTCCTAGGGCAGCACTCCAATTTCAAACTGTTAGCCACCTGGTGAT<br>1741 ACCCCGCCTTCTCTTTTTTGTTCTCCTTTATCCTACCTGACATGTATTTTGTGTAGGATC<br>1801 ACATGGGGTCAGAAAGTAGGTGTTCTATCATAAAAGATACCAAGAAACGACAAAAAGGAA<br>1861 AATGCGGTAGGAGAGACTGTTAAGCAAAGGGGCACAAAGATCTCACTGTTACCCAGCTTC<br>1921 TTGCGTTTTCTGATGAAGGCTCTTGCCTTCCATTGTCTGGTAAGGTTGTTACACCAGTGC<br>1981 GATGGCTTTATGCAATGTTGTGGTGGGAGATGGGGGGTTCTTTTATTAATATAAACCTT<br>2041 TGTCAAATGTTTGAATGGGGCTGAAAATGCTGCATTTTCTTGCTCCCACAAAGCTGGTAT<br>2101 GCTGGTTAATAAAGTTTCCTAAGATTCTGCA</td><td></td></tr>
<tr><td></td><td></td><td>1 M E Q L S T A N T H F A V D L F R A L N<br>1 ATGGAGCAGCTGAGCACAGCCAACACCCACTTTGCGGTGGACTTGTTCCGCGCTTTGAAT<br><br>21 E S D P T G N I F I S P L S I S S A L A<br>61 GAGAGCGATCCTACTGGAAACATCTTCATCTCTCCCTTAAGCATTTCCTCAGCGCTGGCC<br><br>41 M I F L G T R G N T A A Q V S K A L Y F<br>121 ATGATCTTTCTGGGGACCAGAGGCAACACAGCGGCACAGGTGTCCAAGGCACTTTATTTT<br><br>61 D T V E D I H S R F Q S L N A D I N K P<br>181 GATACTGTTGAGGATATTCATTCAAGATTTCAGAGTCTGAATGCTGATATCAACAAACCT<br><br>81 G A P Y I L K L A N R L Y G E K T Y N F<br>241 GGAGCTCCCTATATTCTGAAACTCGCTAATAGATTATATGGAGAGAAAACCTATAATTTC<br><br>101 L A D F L A S T Q K M Y G A E L A S V D<br>301 CTCGCTGATTTCTTAGCTTCAACTCAGAAAATGTATGGTGCTGAATTGGCCAGTGTGGAT<br><br>121 F Q Q A P E D A R K E I N E W V K G Q T<br>361 TTTCAGCAAGCCCCTGAAGATGCAAGAAAGGAGATAAATGAATGGGTCAAAGGACAGACA<br><br>141 E G K I P E L L V K G M V D N M T K L V<br>421 GAGGGGAAAATTCCGGAACTGTTGGTCAAGGGTATGGTTGATAACATGACTAAACTTGTA<br><br>161 L V N A I Y F K G N W Q E K F M K E A T<br>481 CTGGTGAATGCCATCTACTTCAAGGGAAACTGGCAGGAGAAATTCATGAAAGAGGCCACC<br><br>181 R D A P F R L N K K D T K T V K M M Y Q</td><td>SEQ ID NO:108</td></tr>
</table>

| Gene Name | GenBank Homology | DNA SEQUENCE / DEDUCED AMINO ACID SEQUENCE | SEQUENCE IDENTIFIER: |
|---|---|---|---|
| | | 541 AGAGACGCCCCATTCCGATTGAATAAGAAAGACACAAAAACCGTGAAAATGATGTATCAG<br><br>201 K K K F P Y N Y I E D L K C R V L E L P<br>601 AAGAAGAAATTTCCATACAACTACATCGAGGACCTCAAGTGCCGTGTGCTGGAGCTGCCT<br><br>221 Y Q G K E L S M I I L L P D D I E D E S<br>661 TACCAAGGCAAAGAGCTCAGCATGATCATCCTGCTGCCAGACGACATTGAGGATGAGTCC<br><br>241 T G L E K I E K Q L T L E K L R E W T K<br>721 ACGGGCCTGGAGAAGATTGAGAAACAGTTGACTTTGGAAAAACTGCGTGAGTGGACCAAA<br><br>261 P E N L Y L A E V N V H L P R F K L E E<br>781 CCTGAGAATCTGTATCTCGCTGAAGTCAATGTCCACTTGCCCAGGTTCAAGCTGGAAGAG<br><br>281 S Y D L T S H L A R L G V Q D L F N R G<br>841 AGCTACGATCTCACCTCCCATCTAGCCCGCCTAGGTGTACAAGACCTCTTTAACAGGGGC<br><br>301 K A D L S G M S G A R D L F V S K I I H<br>901 AAAGCTGATCTGTCTGGCATGTCAGGGGCCAGAGATCTTTTCGTATCGAAAATTATCCAC<br><br>321 K S F V D L N E E G T E A A A A T A G T<br>961 AAATCCTTTGTGGACCTGAATGAGGAGGGTACAGAGGCTGCGGCAGCCACAGCAGGCACG<br><br>341 I M L A M L M P E E N F N A D H P F I F<br>1021 ATCATGCTTGCCATGCTGATGCCAGAGGAAAATTTCAATGCCGACCATCCATTCATTTTC<br><br>361 F I R H N P S A N I L F L G R F S S P -<br>1081 TTCATTCGGCACAATCCCTCAGCTAACATCCTGTTCTTAGGCAGATTTTCTTCCCCTTAG | |
| WBC024G08.bFSP_20 011510.abd | No homology | 1 CAGACCTGTCCTGGATTCCCACCCACTCTTCTTACACTTCTTGCAAAATGATGACGCATC<br>61 ACTTGGTTCTCTGCCACCCAGTTTTAGGACTGGCACTCCACAAATGATGCTGACTTTGTA<br>121 TGGAAGGATTTAGCTGAAAAAAAGCCCAGACATTATCACTATAACTAGCCCAGCTGGTTC<br>181 TTTTCTTTCATTTTATAAAAAGAATCATTTAAAAGCATTAATTAAAATTTATTTCTATAA<br>241 TTAAATTTGTGTTGGTATTGCCAGCAAAATAATTGAAGCCCCAAGGCTCTTGTCTTTTTT<br>301 AAAAGCAAATTAAAATATTTTGAAGATGGTGTCTTTAAAAAGAGAGGTTAGATCAGACAC<br>361 TAAAATAACAGTGCCTTTCACCTTGAGGCATGGCACTGGCTATGTGACCTTCACCACCTA<br>421 TATTCTGTAGCCTATCAGCAAAGCAAAGGAAACCTCTCTTTTTAAAGTGCTACAGCAATG<br>481 CATCAATATTTATGTAGCAATTCCATCCCGTAATGCATGACTGTCCTGAAAACACTGACA<br>541 GCCACCATTGGCAGACACAACTTTCCATCCTGTTCCAGCCACCTCAGGGAGTCTGAGCCA<br>601 TGTCACCATGAATATCCCAGGACGTGAGGACACGTTAAAGGTAATCAAATTAGATGTTTA<br>661 CCAGATGAGCTCATCATGATTATTCTCTTGGTAACTGGTAAGAGGAAAAATTTTAAGTGT<br>721 ATACCCAGCCCAAACTAAATGAATTCACAGGGCTTTCAGCAGAAAGGCTGCTCAAAAGCA | SEQ ID NO:109 |

| Gene Name | GenBank Homology | DNA SEQUENCE / DEDUCED AMINO ACID SEQUENCE | SEQUENCE IDENTIFIER: |
|---|---|---|---|
| | | 781 CGAAGCCAGAGTGCGGAGCACCTGTGTGTGCATTTCCGCTCATTCCAGTTGTTTCCATTT<br>841 TTTTCTCCTAACACCTTCTT<br><br>1 GACTTGTCACATCTCATGGAGACACTCAGAGGTGCCTGGGCTGGGTTTGGCTGTAGGATG<br>61 GGCTTTGGTATGACAGACAAGACCAGTGAACTTGTCCTTGCCAGATGGGGTCCCTAAAAC<br>121 AGATTTTTATTTCCGCAATCAAGCTGGAAACAAGCTGTGCCTTCAAAAAAGTGGCTTTTC<br>181 TAACTTTAGTTTTATGTTAAGTATGCTACTAACTTAAAAATAAGTGCATTCTCCTCAGAG<br>241 GAAACTATTTCTAACATCTGGTGATGTGTAACTCAGCAGTGAGTTGCCTGTGGCTGTCTT<br>301 TTCTTGTATTCTGTAGGAAAGGAGACCTGAATTTAACTCAGGTGGCAAGAAAAAAAATAC<br>361 TAAGTGCTTTTTAATTTCTCTAACTTCTTTTTGTGAATATGTAATTTTAAGTGTACACTA<br>421 CACCAACTTAGTAAAACTCTTATATTTTGCCTATGTAGGCCTTTTCTGCAGAATTTTTAA<br>481 AGTTACCAATGTCTTTAAGCATCTTCAGCTCTGATCAATGAAATGTCTTGCATATGGAAT<br>541 GATGAGCTAGTCATTGGAAGTGCTCATGGAGGGGGCTTTTTGTTCAGTCTGTATCAGAA<br>601 CAGCGTTTGTGCTCCATGCCTTCAACTCAACAAATGACCATAATCCATAAAATAAAATGT<br>661 GAAGTTTTTAAATCAGAAAAAAAAAAAAAAAA | SEQ ID NO:110 |
| WBC024H07.bFSP_20 011510.abd | Homo sapiens interferon gamma receptor 2 (interferon gamma transducer 1), mRNA | 1 GCGGGCCCTGCGCGCCCTGCGCTCGCCATGGCGGTTTGGGCGGCGACGTGAGCGGCTCCG<br>61 CGGACCCCGAGCGGGGCCCCGGCCGCGACCTGAGCCGCCGCCGAGCGCCCGGGGCCATGC<br>121 GACCGACGCTGCTGTGGTCGCTGCTGCTGCTGCTCGGAGTCTTCGCCGCCGCCGCCGCGG<br>181 CCCCGCCAGACCCTCTTTCCCAGCTGCCTGCTCCCCAAAACCCGAAGATTCGCCTCTACA<br>241 ACGCTGAGCAGCTTCTGAGCTGGGAGCCGGTGCCCCCTGGCAATGGCACGGGGCCGGTGG<br>301 TCTACCACGTGCAGTTTAAATACACCGACAGTAAATGGTTCACGGCCGACATCATGTCCA<br>361 TAGGGGTGAATTGTACACAGATCACAGCAACAGAGTGTGACTTCACTGCCGCCAGTCCCT<br>421 CAGCAGGCTTCCCAATGGATTTCAATGTCACTCTACGCCTTCGAGCTGAGCTGGGAGCAC<br>481 TCCATTCTGCCTGGGTGACGGTACCTTGGTTCCAGCACTTTCGGAATGTTACCATTGGGC<br>541 CTCCAGAAAACATTTGGGTGACCCCAGGAGAAGGCTCCCTTATCATCAAGTTGTCCCCTC<br>601 CCTTTGACATCGCTGATACCTCCACGGCCTTTTTTTGTTATTATGTCCATTACTGGGAAA<br>661 AAGGAGGAATCCAACAGGTCAAAGGCCCTTTCAGAAGCAACTCCATTTCATTGGATAACT<br>721 TAAAACCCTCCAGAGTGTACTGTTTACAAGTCAAGGCACAACTACTTTGGACAATGCTAA<br>781 ACATCTCTAGACCCGGGCATTTAAGCAGCATATCTTGCTACGAAACGATGGCAGATGCCT<br>841 CCACCAAGCTTCAGCAAGTGATTCTCATCGCCTTTGGAACCTTTCTGTTGCTGTTCGCGA<br>901 TGGCAGGGGCCTGTCTCTTCCTGGTCCTGAAATACAGAGGCCTGGTTAAATACTGGTTTC<br>961 ATTCTCCGCCAAGCATCCCATTACAGATAGAAGAGTATTTAAAGGACCCGGCTCAGCCTA<br>1021 TCTTAGACGCCTTGGACAAGGACAGCTCCCCAACGGAAGATGCCTGGGACTCTGTGTCTG<br>1081 TCGTTGTGTTTCCAGAGAAGGAGTAAGAAGATGTTCTCCAAAGCACTTTGAAGCAAAGCA<br>1141 TTGGCCTAGCCCACTGGCTCCCTGGAAGAGATCAAGCCATCGGAGCTGCTAGAGTTCTGT<br>1201 CTGGACTTTCCAGAGACCAGTATTCCCTTTTGCTGCCTCTAAAAGGCCTGTCCCTGCAGA<br>1261 CATGAGAGACAGCAGGTCTCATGGGGGTGACAAGCTTTTTTTTTTTTTCTTAAAGAATTTT<br>1321 CAAAATCAAATTCCAGAATGATTCTACAGAGATCTCTCAGAATAATTAAGATTTCTCTTA<br>1381 AACACTAAACAAGCATGTAATTATTTGTTAGCAAAATGGCTCTGGCACACCTGTGATATT<br>1441 TTGTTCATTGTCGGTTGGGCTGAGCAGTCAGAAGACCTGGTCGTCGTCTTGACTTTGGCA<br>1501 AATGAGCCGGAGCCCCTTGGGCAGGTCACACAACCTGTCCCAGCGAGGGACACCGAGTGG | SEQ ID NO:111 |

| Gene Name | GenBank Homology | DNA SEQUENCE / DEDUCED AMINO ACID SEQUENCE | SEQUENCE IDENTIFIER: |
|---|---|---|---|
| | | 1561 CCCTTCATGTACATCCATGGTGTGCTGGCTTAAAATGTAATTAATCTTGTAAATATACTC<br>1621 CTAGTAATTTAAGATTTTGTTTTTAAACTGGAAATAAAAGATTGTATAGTGCATGTTTTT<br>1681 TAAAAAAAAAAAAAAAAAAAAAAAAAAAAAAAAA<br><br>1 M R P T L L W S L L L L L G V F A A A A<br>1 ATGCGACCGACGCTGCTGTGGTCGCTGCTGCTGCTGCTCGGAGTCTTCGCCGCCGCCGCC<br><br>21 A A P P D P L S Q L P A P Q N P K I R L<br>61 GCGGCCCCGCCAGACCCTCTTTCCCAGCTGCCTGCTCCCCAAAACCCGAAGATTCGCCTC<br><br>41 Y N A E Q L L S W E P V P P G N G T G P<br>121 TACAACGCTGAGCAGCTTCTGAGCTGGGAGCCGGTGCCCCCTGGCAATGGCACGGGGCCG<br><br>61 V V Y H V Q F K Y T D S K W F T A D I M<br>181 GTGGTCTACCACGTGCAGTTTAAATACACCGACAGTAAATGGTTCACGGCCGACATCATG<br><br>81 S I G V N C T Q I T A T E C D F T A A S<br>241 TCCATAGGGGTGAATTGTACACAGATCACAGCAACAGAGTGTGACTTCACTGCCGCCAGT<br><br>101 P S A G F P M D F N V T L R L R A E L G<br>301 CCCTCAGCAGGCTTCCCAATGGATTTCAATGTCACTCTACGCCTTCGAGCTGAGCTGGGA<br><br>121 A L H S A W V T V P W F Q H F R N V T I<br>361 GCACTCCATTCTGCCTGGGTGACGGTACCTTGGTTCCAGCACTTTCGGAATGTTACCATT<br><br>141 G P P E N I W V T P G E G S L I I K L S<br>421 GGGCCTCCAGAAAACATTTGGGTGACCCCAGGAGAAGGCTCCCTTATCATCAAGTTGTCC<br><br>161 P P F D I A D T S T A F F C Y Y V H Y W<br>481 CCTCCCTTTGACATCGCTGATACCTCCACGGCCTTTTTTTGTTATTATGTCCATTACTGG<br><br>181 E K G G I Q Q V K G P F R S N S I S L D<br>541 GAAAAAGGAGGAATCCAACAGGTCAAAGGCCCTTTCAGAAGCAACTCCATTTCATTGGAT<br><br>201 N L K P S R V Y C L Q V K A Q L L W T M<br>601 AACTTAAAACCCTCCAGAGTGTACTGTTTACAAGTCAAGGCACAACTACTTTGGACAATG<br><br>221 L N I S R P G H L S S I S C Y E T M A D<br>661 CTAAACATCTCTAGACCCGGGCATTTAAGCAGCATATCTTGCTACGAAACGATGGCAGAT<br><br>241 A S T K L Q Q V I L I A F G T F L L L F<br>721 GCCTCCACCAAGCTTCAGCAAGTGATTCTCATCGCCTTTGGAACCTTTCTGTTGCTGTTC | SEQ ID NO:112 |

| Gene Name | GenBank Homology | DNA SEQUENCE / DEDUCED AMINO ACID SEQUENCE | SEQUENCE IDENTIFIER: |
|---|---|---|---|
| | | 261 A M A G A C L F L V L K Y R G L V K Y W<br>781 GCGATGGCAGGGGCCTGTCTCTTCCTGGTCCTGAAATACAGAGGCCTGGTTAAATACTGG<br><br>281 F H S P P S I P L Q I E E Y L K D P A Q<br>841 TTTCATTCTCCGCCAAGCATCCCATTACAGATAGAAGAGTATTTAAAGGACCCGGCTCAG<br><br>301 P I L D A L D K D S S P T E D A W D S V<br>901 CCTATCTTAGACGCCTTGGACAAGGACAGCTCCCCAACGGAAGATGCCTGGGACTCTGTG<br><br>321 S V V V F P E K E -<br>961 TCTGTCGTTGTGTTTCCAGAGAAGGAGTAA | |
| WBC025A06.bFSP_20 011710.abd | No homology | 1 TTCCTGAAGCTTTGTCTGATGAATGTCTTCCATTCTTCAAAGTTTCTCTGTTTTAACTAC<br>61 ACAACTTGATTTGCCACATCATCAGTGTCTTTATTTCTTTAGTTCTGTATTTCTTTGCAA<br>121 TGTCAGAAAGTCATACATCTGTGATTCTCTTGGTGTTTGGAACCTGCTTGGAGGTGTAGT<br>181 AAAAAGTCATAAAGCAGAGAATAGAGTCAGAATCACCAGTAACTTCTTTGCTGCAGTGTG<br>241 AACATGCCAGTGGTTTCACTCTAGCATGGAGTGTTATTTTCCTAAGATCAAGTGAATAAG<br>301 AGAAGTAATCATGCATGCCTCCACCTTGCCTGAAATGGGAATGCCTTAGTGAATGTAGTG<br>361 TTGTGTGGTGTGTGCGTTCTTTCACATATATGCGTACGCACATACGTATATATGCATATA<br>421 TTTCAATCACAAGCGCTGCTGATACTGTGGTTTAGAGCAGCCAGTAGTACTGGTTTTTGA<br>481 ATTCTTGAGAAGAGAAATTAAGTGACACAAATCATTATATCACCTGCCTCCAGCTGTAGC<br>541 TGAACAGATGCCAGCATTATAAATGTCACACCTTTCTTTACCACTAAACATTTGCCTTCA<br>601 ACAGGGCAACAGTGAGCTTTTCCTATATATTATGGTAAGTTATCAGAAATTACAGTTTAA<br>661 AAATATTTTCACATTTCCTGAAGTTTGAGGCAGTCTAAACATGACATTTTAGGTCTTCAT<br>721 TAGCTTTGTTTAAATAACATTTTGCCCTTATCAAAGCTTGCTCTTGTCTTAGATATAAAT<br>781 TGGAAGTTTCCTGAATGCAAATCTAAGATGCCAAATTCTTGTTTTCCAGAATTAAAAAAA<br>841 ACTGTTTAAAAATTGCAGTCATTAAAATATTGATTGTTTTTCAGCTTTATCATAATTTTT<br>901 ACTTCCTATAGAAAAATGTTTATTAAGAAAAATTTAATTTTTCTTTTTC<br><br>1 TTGTTACTCAAAATTGCGGTTCTGGACTTTGTTTTTATTAGTTGCTTGATTGATTTTTAG<br>61 TCTTCTGAATAATAGTGAAAATATCGGTAAGGTGTGAAAGACTCCTGCTATTATAAGCAC<br>121 TCAGTCCGCTTCCCACGGAACTGAGCAGAGAATTAGTCACTGGCCAGGCGAGCCCGAGC<br>181 AGGTCCCGGGCGCTCCAGTGTGGTGGTTAGTCTGCCTTGGTGAAGGGCAGCCCACTGGCA<br>241 TCGTGTCAGCACAGAAACCTTCTCAGGTACAGGCTTTGTTTTAGATTGTAGGCTCTGGAA<br>301 CAATTCAGAAGTGTCCAGCCCAGCCCTCCTTTCCCTGTGTCGGTTACCACTCAAGACGCA<br>361 CATCGTCGGGGCCGGCCGGGTGGCGCAGCGGTTAAGTGCGCACGTTCCACTTCTCCACGG<br>421 CCCGGGGTTCGCCGGTTTGGATTCCGGCTGCGGACATGGCACTGCTTGGCAAAAGCCATG<br>481 CTGTGGTAGGCATCCCACGTATAAAGTAGAGGAAGATGGGCACAGATGTTAGCTCAGGGC<br>541 CGTCTTCCTCAGCAAAAGAGGAGGACTGGCAGTAGTTAGCTCAGGGCTAATCTTCGTCAA<br>601 AAAAAAAAAAAAAA | SEQ ID NO:113<br><br><br><br><br><br><br><br><br><br><br><br><br><br><br><br><br>SEQ ID NO:114 |
| WBC025B03.bFSP_20 | Homo sapiens pro- | | |

| Gene Name | GenBank Homology | DNA SEQUENCE / DEDUCED AMINO ACID SEQUENCE | SEQUENCE IDENTIFIER: |
|---|---|---|---|
| 011710.abd | oncosis receptor inducing membrane injury gene, mRNA | 1 CCACGCGTCCGGGAAGCGGAGCCGGCGCCGGCTGCGCAGAGGAGCCGCTCTCGCCGCCGC<br>61 CACCTCGGCTGGGAGCCCACGAGGCTGCCGCATCCTGCCCTCGGAACAATGGGACTCGGC<br>121 GCGCGAGGTGCTTGGGCCGCGCTGCTCCTGGGGACGCTGCAGGTGCTAGCGCTGCTGGGG<br>181 GCCGCCCATGAAAGCGCAGCCATGGCGGCATCTGCAAACATAGAGAATTCTGGGCTTCCA<br>241 CACAACTCCAGTGCTAACTCAACAGAGACTCTCCAACATGTGCCTTCTGACCATACAAAT<br>301 GAAACTTCCAACAGTACTGTGAAACCACCAACTTCAGTTGCCTCAGACTCCAGTAATACA<br>361 ACGGTCACCACCATGAAACCTACAGCGGCATCTAATACAACAACACCAGGGATGGTCTCA<br>421 ACAAATATGACTTCTACCACCTTAAAGTCTACACCCAAAACAACAAGTGTTTCACAGAAC<br>481 ACATCTCAGATATCAACATCCACAATGACCGTAACCCACAATAGTTCAGTGACATCTGCT<br>541 GCTTCATCAGTAACAATCACAACAACTATGCATTCTGAAGCAAAGAAAGGATCAAAATTT<br>601 GATACTGGGAGCTTTGTTGGTGGTATTGTATTAACGCTGGGAGTTTTATCTATTCTTTAC<br>661 ATTGGATGCAAAATGTATTACTCAAGAAGAGGCATTCGGTATCGAACCATAGATGAACAT<br>721 GATGCCATCATTTAAGGAAATCCATGGACCAAGGATGGAATACAGATTGATGCTGCCCTA<br>781 TCAATTAATTTTGGTTTATTAATAGTTTAAAACAATATTCTCTTTTTGAAAATAGTATAA<br>841 ACAGGCCATGCATATAATGTACAGTGTATTACGTAAATATGTAAAGATTCTTCAAGGTAA<br>901 CAAGGGTTTGGGTTTTGAAATAAACATCTGGATCTTATAGACCGTTCATACAATGGTTTT<br>961 AGCAAGTTCATAGTAAGACAAACAAGTCCTATCTTTTTTTTTTTGGCTGGGGTGGGGGCA<br>1021 TTGGTCACATATGACCAGTAATTGAAAGACGTCATCACTGAAAGACAGAATGCCATCTGG<br>1081 GCATACAAATAAGAAGTTTGTCACAGCACTCAGGATTTTGGGTATCTTTTGTAGCTCACA<br>1141 TAAAGAACTTCAGTGCTTTTCAGAGCTGGATATATCTTAATTACTAATGCCACACAGAAA<br>1201 TTATACAATCAAACTAGATCTGAAGCATAATTTAAGAAAAACATCAACATTTTTTGTGCT<br>1261 TTAAACTGTAGTAGTTGGTCTAGAAACAAAATACTCCAAGAAAAAGAAAATTTTCAAATA<br>1321 AAACCCAAAATAATAGCTTTGCTTAGCCCTGTTAGGGATCCATTGGAGCATTAAGGAGCA<br>1381 CATATTTTTATTAACTTCTTTTGAGCTTTCAATGTTGATGTAATTTTTGTTCTCTGTGTA<br>1441 ATTTAGGTAAACTGCAGTGTTTAACATAATAATGTTTTAAAAACTTAGTTGTTAGTATTA<br>1501 AATAAGTTAGATTATTTGCTACTGTGAGAATATTGTCACCACTGGAAGTTACTTTAGTTC<br>1561 ATTTAATTTTAATTTTATCTTTAGTGAATATTTTTAAGAGATAGAGAGCTGCTTTCAATA<br>1621 TCTAGAAATGTTTAATGTAGTGTAAACATGCCTAACTTTAAAAAAAAGAGCCCCTTATAT<br>1681 GATTTCCAAAACAACATTTAGAACTGTATACACAGAACTATAGATTAATGCTCTGTTTAT<br>1741 CAAGCCAGGGATTGTGGGGGCCCCCCTCAGGCTACTAAACCCACAGGATGAAAATGCTAT<br>1801 ATTTTCTTTCATGCACTGTCGATATTACTCAGATTTGGGGAAATGACATTTTTATACTAA<br>1861 AACAAACACCAAAATATTTTAGAATAAATTCTTAGAAAGTTTTGAGAGGAATTTTTAGAG<br>1921 AGGACATTTCCTCCTTCCTGATTTGGATATTCCCTCAAATCCCTCCTCTTACTCCATGCT<br>1981 GAAGGAGAAGTACTCTCAGATGCATTATGTTAATGGAGAGAAAAAGCACAGTATTTTAGG<br>2041 GATACCAATATTAGCCAAGGTATTTTGAAGTGTTTTCGGTTTTAGTTTATATTACAACAC<br>2101 TCAAAACTCAGGGTCAAGTTTTGACAAAAGATGTATTTAGTCATACTGAATACTGAGATA<br>2161 GTATTTCTATCTTAGGTGGTCAAAGTAAATCACACCAATTTCTGATAGTCCAAAAATCAG<br>2221 CTGAGAAGTCCTAATGAACATGTACCTACAATTAATAGGAGTACAATAAAACTGTTGTCA<br>2281 GCTTTTGTTTTACAGAGAACGCTAGATATTAAGAATTTTGAAATGGATCATTTCTACTTG<br>2341 CTGTGCATTTTAACCAATAATCTGATGAATATAGAAAAAAATGATCCAAAATATGGATAT<br>2401 GATTGGATGTATGTAACACATACATGGAGTATGGAGGAAATTTTCTGAAAAATACATTTA<br>2461 GATTAGTTTAGTTTGAAGGAGAGGTGGGCTGATGGCTGAGTTGTATGTTACTAACTTGGC<br>2521 CCTGACTGGTTGTGCAACCATTGCTTCATTTCTTTGCAAAATGTAGTTAAGATATACTTT | SEQ ID NO:115 |

| Gene Name | GenBank Homology | DNA SEQUENCE / DEDUCED AMINO ACID SEQUENCE | SEQUENCE IDENTIFIER: |
|---|---|---|---|
| | | 2581 ATTCTAATGAAGGCCTTTTAAATTTGTCCACTGCATTCTTGGTATTTCAGTACTTCAAGT<br>2641 CAGTCAGAACTTTGTAGACAGGCCTGAAACTTTCTTCTATACTTGCTTCTTTAGCACTTT<br>2701 GAAGATATAAAACCACTTTTTAAGTACTAAATGTCGAAATTTGCCTTTCAAGTTTCCTGT<br>2761 GCTTTGGGTTTGAAATAATTTAGTTATGTCTTTTCTCTGTATGTAAATTGTATAATTTGT<br>2821 TATTTTCAAATACCCGTACTTTGAATGTAGGTTTTTTTGTTGTTGTTATCTATAAAAATT<br>2881 GAGGGAACTGGTGATGCAAACCAATATTCTGCTTTGGAGCACATTTCCTAAGCATAAAAA<br>2941 AAATGCTCAGTTTTGCTTGCATTCCTTGAGAATGTATTTATCTGAAGATCAAAACAAACA<br>3001 ATCCAGATGTATAAGTACTAGGCAGAAGCCAATTTTAAAATTTCTTTGGATAATCCATAA<br>3061 AATGAATAATTCAAATACAGATAAGAGTGGTAGCATATTACAGTGATCATTTTGTATTTT<br>3121 TTCACAAAAAAAAAGTTAAACTCTTCTTTTCTTTTTATTATAAGGATCAGCTTTTGGTAT<br>3181 TTCATTGTTACTGAGATCTATTTTTAGAATAAAATTGTTCTCCTTCCAAAAAAAAAAAAA<br>3241 AAAA | |
| | | 1 M G L G A R G A W A A L L L G T L Q V L<br>1 ATGGGACTCGGCGCGCGAGGTGCTTGGGCCGCGCTGCTCCTGGGGACGCTGCAGGTGCTA<br><br>21 A L L G A A H E S A A M A A S A N I E N<br>61 GCGCTGCTGGGGGCCGCCCATGAAAGCGCAGCCATGGCGGCATCTGCAAACATAGAGAAT<br><br>41 S G L P H N S S A N S T E T L Q H V P S<br>121 TCTGGGCTTCCACACAACTCCAGTGCTAACTCAACAGAGACTCTCCAACATGTGCCTTCT<br><br>61 D H T N E T S N S T V K P P T S V A S D<br>181 GACCATACAAATGAAACTTCCAACAGTACTGTGAAACCACCAACTTCAGTTGCCTCAGAC<br><br>81 S S N T T V T T M K P T A A S N T T T P<br>241 TCCAGTAATACAACGGTCACCACCATGAAACCTACAGCGGCATCTAATACAACAACACCA<br><br>101 G M V S T N M T S T T L K S T P K T T S<br>301 GGGATGGTCTCAACAAATATGACTTCTACCACCTTAAAGTCTACACCCAAAACAACAAGT<br><br>121 V S Q N T S Q I S T S T M T V T H N S S<br>361 GTTTCACAGAACACATCTCAGATATCAACATCCACAATGACCGTAACCCACAATAGTTCA<br><br>141 V T S A A S S V T I T T T M H S E A K K<br>421 GTGACATCTGCTGCTTCATCAGTAACAATCACAACAACTATGCATTCTGAAGCAAAGAAA<br><br>161 G S K F D T G S F V G G I V L T L G V L<br>481 GGATCAAAATTTGATACTGGGAGCTTTGTTGGTGGTATTGTATTAACGCTGGGAGTTTTA<br><br>181 S I L Y I G C K M Y Y S R R G I R Y R T<br>541 TCTATTCTTTACATTGGATGCAAAATGTATTACTCAAGAAGAGGCATTCGGTATCGAACC | SEQ ID NO:116 |

| Gene Name | GenBank Homology | DNA SEQUENCE / DEDUCED AMINO ACID SEQUENCE | SEQUENCE IDENTIFIER: |
|---|---|---|---|
| | | 201 I D E H D A I I -<br>601 ATAGATGAACATGATGCCATCATTTAA | |
| WBC025C03.bFSP_20 011710.abd | Homo sapiens TBP-interacting protein, mRNA | 1 GGCGTCGCGCTGCGACCCTGGAAGCGGGAGCCGCCGCGAGCGAGAGGAGGAGCTCCAGTG<br>61 GCGGCGGCGGCGGCGGCAGCGGCAGCGGGCAGCAGCTCCAGCAGCGCCAGCAGGCGGGAT<br>121 CGAGGCCGTCAACATGGCGAGCGCCTCGTACCACATTTCCAATTTGCTGGAAAAAATGAC<br>181 ATCCAGCGACAAGGACTTTAGGTTTATGGCTACAAATGATTTGATGACGGAACTGCAGAA<br>241 AGATTCCATCAAGTTGGATGATGATAGTGAAAGGAAAGTAGTGAAAATGATTTTGAAGTT<br>301 ATTGGAAGATAAAAATGGAGAGGTACAGAATTTAGCTGTCAAATGTCTTGGTCCTTTAGT<br>361 GAGTAAAGTGAAAGAATACCAAGTAGAGACAATTGTAGATACCCTCTGCACTAACATGCT<br>421 TTCTGATAAAGAACAACTTCGAGACATTTCAAGTATTGGTCTTAAAACAGTAATTGGAGA<br>481 ACTTCCTCCAGCTTCCAGTGGCTCTGCATTAGCTGCTAATGTATGTAAAAAGATTACTGG<br>541 ACGTCTTACAAGTGCAATAGCAAAACAGGAAGATGTCTCTGTTCAGCTAGAAGCCTTGGA<br>601 TATTATGGCTGATATGTTGAGCAGGCAAGGAGGACTTCTTGTTAATTTCCATCCTTCAAT<br>661 TCTGACCTGTCTACTTCCCCAGTTGACCAGCCCTAGACTTGCAGTGAGGAAAAGAACCAT<br>721 TATCGCTCTTGGCCATCTGGTTATGAGCTGTGGAAATATAGTTTTTGTAGATCTTATTGA<br>781 ACATCTGTTGTCAGAGTTGTCCAAAAATGATTCTATGTCAACAACAAGAACCTACATACA<br>841 ATGTATTGCTGCTATTAGTAGGCAAGCTGGTCATAGAATAGGTGAATACCTTGAGAAGAT<br>901 AATTCCTTTGGTGGTAAAATTTTGCAATGTAGATGATGATGAATTAAGAGAGTACTGTAT<br>961 TCAAGCCTTTGAATCATTTGTAAGAAGATGTCCTAAGGAAGTATATCCTCATGTTTCTAC<br>1021 CATTATAAATATTTGTCTTAAATATCTTACCTATGATCCAAATTATAATTACGATGATGA<br>1081 AGATGAAGATGAAAATGCAATGGATGCTGATGGTGGTGATGATGATGATCAAGGGAGTGA<br>1141 TGATGAATACAGTGATGATGATGACATGAGTTGGAAAGTGAGACGTGCAGCTGCGAAGTG<br>1201 CTTGGATGCTGTAGTTAGCACAAGGCATGAAATGCTTCCAGAATTCTACAAGACCGTCTC<br>1261 TCCTGCACTGATATCCAGATTTAAAGAACGAGAAGAGAACGTAAAGGCAGATGTTTTTCA<br>1321 TGCATACCTTTCTCTTTTGAAGCAAACTCGTCCTGTGCAAAGTTGGCTGTGTGACCCTGA<br>1381 TGCAATGGAACAAGGAGAAACACCTTTAACAATGCTTCAGAGTCAGGTTCCCAACATTGT<br>1441 TAAAGCTCTGCACAAACAGATGAAAGAAAAAAGTGTGAAGACTCGACAGTGCTGTTTTAA<br>1501 CATGTTAACTGAACTGGTAAATGTGTTACCTGGGGCCCTAACACAACATGTTCCTGTGCT<br>1561 TGTACCAGGAATCATTTTCTCACTGAATGATAAATCAAGCTCATCAAATTTGAAGATTGA<br>1621 TGCTCTGTCGTGTCTCTACGTAATCCTCTGTAACCACTCTCCTCAAGTCTTCCATCTTCA<br>1681 TGTCCAGGCTTTGGTCCCTCCAGTGGTGGCTTGTGTTGGATACCCATTTTACAAGATTAC<br>1741 GTCTGAAGCACTTCTTGTTACTCAACAGCTTGTGAAAGTCATTCGTCCTTTAGATCAGCC<br>1801 TTCCTCATTTGATGCCACTCCTTACATCAAAGATTTGTTTACCTGTACCATTAAGAGGTT<br>1861 AAAAGCAGCTGACATTGATCAGGAAGTCAAGGAAAGGCTATTTCCTGTATGGGACAAAT<br>1921 TATTCTGCACCTTGGAGACATTTTGGGTTCTGACTTGCCTAATACACTTCAGATTTTCTT<br>1981 GGAGAGACTAAAGAATGAAATTACCAGGTTAACTACAGTAAAGGCATTGACACTGATTGC<br>2041 TGGGTCACCTTTGAAGATAGATTTGAGGCCTGTTCTGGGAGAAGGGGTTCCTATCCTTGC<br>2101 TTCATTTCTTAGAAAAAACCAGAGAGCTTTGAAACTGGGTACTCTTTCTGCCCTTGATAT<br>2161 TCTAATAAAAAACTATAGTGACAGCTTGACAGCTGCCATGATTGATGCAGTTCTAGATGA<br>2221 GCTCCCACCTCTTATCAGCGAAAGTGATATGCATGTTTCACAAATGGCCATCAGTTTTCT | SEQ ID NO:117 |

| Gene Name | GenBank Homology | DNA SEQUENCE / DEDUCED AMINO ACID SEQUENCE | SEQUENCE IDENTIFIER: |
|---|---|---|---|
| | | 2281 TACCACTTTGGCAAAAGTATATCCCTCCTCCCTTTCAAAGATAAGTGGATCCATTCTCAA<br>2341 TGAACTTATTGGACTTGTGAGATCACCCTTATTGCAGGGGGGAGCTCTTAGTGCCATGCT<br>2401 AGACTTTTTCCAAGCTCTGGTTGTCACTGGAACAAATAATTTAGGATACATGGATTTGTT<br>2461 GCGCATGCTGACTGGTCCAGTTTACTCTCAGAGCACAGCTCTTACTCATAAGCAGTCTTA<br>2521 TTATTCCATTGCCAAATGTGTAGCTGCCCTTACTCGAGCATGCCCTAAAGAGGGACCAGC<br>2581 TGTAGTAGGTCAGTTTATTCAAGATGTCAAGAACTCAAGGTCTACAGATTCCATTCGTCT<br>2641 CTTAGCTCTACTTTCTCTTGGAGAAGTTGGGCATCATATTGACTTAAGTGGACAGTTGGA<br>2701 ACTAAAATCTGTAATACTAGAAGCTTTCTCATCTCCTAGTGAAGAAGTCAAATCAGCTGC<br>2761 ATCCTATGCATTAGGCAGCATTAGTGTGGGCAACCTTCCTGAATATCTACCGTTTGTCCT<br>2821 GCAAGAAATAACTAGTCAACCCAAAAGGCAGTATCTTTTACTTCATTCCTTGAAGGAAAT<br>2881 TATTAGCTCTGCATCAGTGGTGGGCCTTAAACCATATGTTGAAAACATCTGGGCCTTATT<br>2941 ACTAAAGCACTGTGAGTGTGCAGAGGAAGGAACCAGAAATGTTGTTGTTGAATGTCTAGG<br>3001 AAAACTCACTCTAATTGATCCAGAAACTCTCCTTCCACGGCTTAAGGGGTACTTGATATC<br>3061 AGGCTCATCATATGCCCGAAGCTCAGTGGTTACGGCTGTGAAATTTACAATTTCTGACCA<br>3121 TCCACAACCTATTGATCCACTGTTAAAGAACTGCATAGGTGATTTCCTAAAAACTTTGGA<br>3181 AGACCCAGATTTGAATGTGAGAAGAGTAGCCTTGGTCACATTTAATTCAGCAGCACATAA<br>3241 CAAGCCATCATTAATAAGGGATCTATTGGATACTGTTCTTCCACATCTTTACAATGAAAC<br>3301 AAAAGTTAGAAAGGAGCTTATAAGAGAGGTAGAAATGGGTCCATTTAAACATACGGTTGA<br>3361 TGATGGTCTGGATATTAGAAAGGCAGCATTTGAGTGTATGTACACACTTCTAGACAGTTG<br>3421 TCTTGATAGACTTGATATCTTTGAATTTCTAAATCATGTTGAAGATGGTTTGAAGGACCA<br>3481 TTATGATATTAAGATGCTGACATTTTTAATGTTGGTGAGACTGTCTACCCTTTGTCCAAG<br>3541 TGCAGTATTGCAGAGGTTGGACCGACTTGTTGAGCCGTTACGCGCCACGTGTACAACGAA<br>3601 GGTGAAGGCAAACTCAGTAAAGCAGGAGTTTGAAAAGCAGGACGAACTGAAGCGATCGGC<br>3661 CATGAGAGCAGTCGCGGCCCTGCTGACCATTCCCGAGGCCGAGAAGAGCCCGCTGATGAG<br>3721 CGAGTTCCAGTCGCAGATCAGCTCCAACCCTGAGCTGGCAGCCATCTTTGAGAGCATCCA<br>3781 GAAAGACTCATCGTCCACTAACTTGGAATCAATGGACACTAGTTAGATGTTTGCTCCATA<br>3841 CGGGACCATTACGTTGACCATACGATGCACTGAATTGACAGGTTAATCATAAGACATGGA<br>3901 AAGAGAAGTGTCTAAAAGCTTCAAAATGTTCCACTTTTTTTTCCTTCATGGAGACAGTTT<br>3961 GGCTTTCTTCCATTGTTGTTTTTGTAGCATTTATTTCAGAAATGTGTATTTCCATAATCC<br>4021 AGAGGTTGTAAAACCACTAATGTCTTAGTGGTTGGGGCAACATTTAAAATGGAAACTAAA<br>4081 AGTTAGGATTTATGGAGATAGGGTCCAGTATCTATTTGCCCTGTAATGTTTAGGATTAAA<br>4141 ATGTTAAAATTTAAAAAAAAAAAAAAAAA<br><br>1 M A S A S Y H I S N L L E K M T S S D K<br>1 ATGGCGAGCGCCTCGTACCACATTTCCAATTTGCTGGAAAAAATGACATCCAGCGACAAG<br><br>21 D F R F M A T N D L M T E L Q K D S I K<br>61 GACTTTAGGTTTATGGCTACAAATGATTTGATGACGGAACTGCAGAAAGATTCCATCAAG<br><br>41 L D D D S E R K V V K M I L K L L E D K<br>121 TTGGATGATGATAGTGAAAGGAAAGTAGTGAAAATGATTTTGAAGTTATTGGAAGATAAA<br><br>61 N G E V Q N L A V K C L G P L V S K V K | SEQ ID NO:118 |

| Gene Name | GenBank Homology | DNA SEQUENCE / DEDUCED AMINO ACID SEQUENCE | SEQUENCE IDENTIFIER: |
|---|---|---|---|
| | | 181 AATGGAGAGGTACAGAATTTAGCTGTCAAATGTCTTGGTCCTTTAGTGAGTAAAGTGAAA<br><br>81 E Y Q V E T I V D T L C T N M L S D K E<br>241 GAATACCAAGTAGAGACAATTGTAGATACCCTCTGCACTAACATGCTTTCTGATAAAGAA<br><br>101 Q L R D I S S I G L K T V I G E L P P A<br>301 CAACTTCGAGACATTTCAAGTATTGGTCTTAAAACAGTAATTGGAGAACTTCCTCCAGCT<br><br>121 S S G S A L A A N V C K K I T G R L T S<br>361 TCCAGTGGCTCTGCATTAGCTGCTAATGTATGTAAAAAGATTACTGGACGTCTTACAAGT<br><br>141 A I A K Q E D V S V Q L E A L D I M A D<br>421 GCAATAGCAAAACAGGAAGATGTCTCTGTTCAGCTAGAAGCCTTGGATATTATGGCTGAT<br><br>161 M L S R Q G G L L V N F H P S I L T C L<br>481 ATGTTGAGCAGGCAAGGAGGACTTCTTGTTAATTTCCATCCTTCAATTCTGACCTGTCTA<br><br>181 L P Q L T S P R L A V R K R T I I A L G<br>541 CTTCCCCAGTTGACCAGCCCTAGACTTGCAGTGAGGAAAAGAACCATTATCGCTCTTGGC<br><br>201 H L V M S C G N I V F V D L I E H L L S<br>601 CATCTGGTTATGAGCTGTGGAAATATAGTTTTTGTAGATCTTATTGAACATCTGTTGTCA<br><br>221 E L S K N D S M S T T R T Y I Q C I A A<br>661 GAGTTGTCCAAAAATGATTCTATGTCAACAACAAGAACCTACATACAATGTATTGCTGCT<br><br>241 I S R Q A G H R I G E Y L E K I I P L V<br>721 ATTAGTAGGCAAGCTGGTCATAGAATAGGTGAATACCTTGAGAAGATAATTCCTTTGGTG<br><br>261 V K F C N V D D D E L R E Y C I Q A F E<br>781 GTAAAATTTTGCAATGTAGATGATGATGAATTAAGAGAGTACTGTATTCAAGCCTTTGAA<br><br>281 S F V R R C P K E V Y P H V S T I I N I<br>841 TCATTTGTAAGAAGATGTCCTAAGGAAGTATATCCTCATGTTTCTACCATTATAAATATT<br><br>301 C L K Y L T Y D P N Y N Y D D E D E D E<br>901 TGTCTTAAATATCTTACCTATGATCCAAATTATAATTACGATGATGAAGATGAAGATGAA<br><br>321 N A M D A D G G D D D D Q G S D D E Y S<br>961 AATGCAATGGATGCTGATGGTGGTGATGATGATGATCAAGGGAGTGATGATGAATACAGT<br><br>341 D D D D M S W K V R R A A A K C L D A V<br>1021 GATGATGATGACATGAGTTGGAAAGTGAGACGTGCAGCTGCGAAGTGCTTGGATGCTGTA | |

| Gene Name | GenBank Homology | DNA SEQUENCE / DEDUCED AMINO ACID SEQUENCE | SEQUENCE IDENTIFIER: |
|---|---|---|---|
| | | 361 V S T R H E M L P E F Y K T V S P A L I<br>1081 GTTAGCACAAGGCATGAAATGCTTCCAGAATTCTACAAGACCGTCTCTCCTGCACTGATA<br><br>381 S R F K E R E E N V K A D V F H A Y L S<br>1141 TCCAGATTTAAAGAACGAGAAGAGAACGTAAAGGCAGATGTTTTTCATGCATACCTTTCT<br><br>401 L L K Q T R P V Q S W L C D P D A M E Q<br>1201 CTTTTGAAGCAAACTCGTCCTGTGCAAAGTTGGCTGTGTGACCCTGATGCAATGGAACAA<br><br>421 G E T P L T M L Q S Q V P N I V K A L H<br>1261 GGAGAAACACCTTTAACAATGCTTCAGAGTCAGGTTCCCAACATTGTTAAAGCTCTGCAC<br><br>441 K Q M K E K S V K T R Q C C F N M L T E<br>1321 AAACAGATGAAAGAAAAAAGTGTGAAGACTCGACAGTGCTGTTTTAACATGTTAACTGAA<br><br>461 L V N V L P G A L T Q H V P V L V P G I<br>1381 CTGGTAAATGTGTTACCTGGGGCCCTAACACAACATGTTCCTGTGCTTGTACCAGGAATC<br><br>481 I F S L N D K S S S S N L K I D A L S C<br>1441 ATTTTCTCACTGAATGATAAATCAAGCTCATCAAATTTGAAGATTGATGCTCTGTCGTGT<br><br>501 L Y V I L C N H S P Q V F H L H V Q A L<br>1501 CTCTACGTAATCCTCTGTAACCACTCTCCTCAAGTCTTCCATCTTCATGTCCAGGCTTTG<br><br>521 V P P V V A C V G Y P F Y K I T S E A L<br>1561 GTCCCTCCAGTGGTGGCTTGTGTTGGATACCCATTTTACAAGATTACGTCTGAAGCACTT<br><br>541 L V T Q Q L V K V I R P L D Q P S S F D<br>1621 CTTGTTACTCAACAGCTTGTGAAAGTCATTCGTCCTTTAGATCAGCCTTCCTCATTTGAT<br><br>561 A T P Y I K D L F T C T I K R L K A A D<br>1681 GCCACTCCTTACATCAAAGATTTGTTTACCTGTACCATTAAGAGGTTAAAAGCAGCTGAC<br><br>581 I D Q E V K E R A I S C M G Q I I L H L<br>1741 ATTGATCAGGAAGTCAAGGAAAGGGCTATTTCCTGTATGGGACAAATTATTCTGCACCTT<br><br>601 G D I L G S D L P N T L Q I F L E R L K<br>1801 GGAGACATTTTGGGTTCTGACTTGCCTAATACACTTCAGATTTTCTTGGAGAGACTAAAG<br><br>621 N E I T R L T T V K A L T L I A G S P L<br>1861 AATGAAATTACCAGGTTAACTACAGTAAAGGCATTGACACTGATTGCTGGGTCACCTTTG | |

| Gene Name | GenBank Homology | DNA SEQUENCE / DEDUCED AMINO ACID SEQUENCE | SEQUENCE IDENTIFIER: |
|---|---|---|---|
| | | 641 K I D L R P V L G E G V P I L A S F L R<br>1921 AAGATAGATTTGAGGCCTGTTCTGGGAGAAGGGGTTCCTATCCTTGCTTCATTTCTTAGA<br><br>661 K N Q R A L K L G T L S A L D I L I K N<br>1981 AAAAACCAGAGAGCTTTGAAACTGGGTACTCTTTCTGCCCTTGATATTCTAATAAAAAAC<br><br>681 Y S D S L T A A M I D A V L D E L P P L<br>2041 TATAGTGACAGCTTGACAGCTGCCATGATTGATGCAGTTCTAGATGAGCTCCCACCTCTT<br><br>701 I S E S D M H V S Q M A I S F L T T L A<br>2101 ATCAGCGAAAGTGATATGCATGTTTCACAAATGGCCATCAGTTTTCTTACCACTTTGGCA<br><br>721 K V Y P S S L S K I S G S I L N E L I G<br>2161 AAAGTATATCCCTCCTCCCTTTCAAAGATAAGTGGATCCATTCTCAATGAACTTATTGGA<br><br>741 L V R S P L L Q G G A L S A M L D F F Q<br>2221 CTTGTGAGATCACCCTTATTGCAGGGGGGAGCTCTTAGTGCCATGCTAGACTTTTTCCAA<br><br>761 A L V V T G T N N L G Y M D L L R M L T<br>2281 GCTCTGGTTGTCACTGGAACAAATAATTTAGGATACATGGATTTGTTGCGCATGCTGACT<br><br>781 G P V Y S Q S T A L T H K Q S Y Y S I A<br>2341 GGTCCAGTTTACTCTCAGAGCACAGCTCTTACTCATAAGCAGTCTTATTATTCCATTGCC<br><br>801 K C V A A L T R A C P K E G P A V V G Q<br>2401 AAATGTGTAGCTGCCCTTACTCGAGCATGCCCTAAAGAGGGACCAGCTGTAGTAGGTCAG<br><br>821 F I Q D V K N S R S T D S I R L L A L L<br>2461 TTTATTCAAGATGTCAAGAACTCAAGGTCTACAGATTCCATTCGTCTCTTAGCTCTACTT<br><br>841 S L G E V G H H I D L S G Q L E L K S V<br>2521 TCTCTTGGAGAAGTTGGGCATCATATTGACTTAAGTGGACAGTTGGAACTAAAATCTGTA<br><br>861 I L E A F S S P S E E V K S A A S Y A L<br>2581 ATACTAGAAGCTTTCTCATCTCCTAGTGAAGAAGTCAAATCAGCTGCATCCTATGCATTA<br><br>881 G S I S V G N L P E Y L P F V L Q E I T<br>2641 GGCAGCATTAGTGTGGGCAACCTTCCTGAATATCTACCGTTTGTCCTGCAAGAAATAACT<br><br>901 S Q P K R Q Y L L L H S L K E I I S S A<br>2701 AGTCAACCCAAAAGGCAGTATCTTTTACTTCATTCCTTGAAGGAAATTATTAGCTCTGCA<br><br>921 S V V G L K P Y V E N I W A L L L K H C | |

| Gene Name | GenBank Homology | DNA SEQUENCE / DEDUCED AMINO ACID SEQUENCE | SEQUENCE IDENTIFIER: |
|---|---|---|---|
| | | 2761 TCAGTGGTGGGCCTTAAACCATATGTTGAAAACATCTGGGCCTTATTACTAAAGCACTGT<br><br>941 E C A E E G T R N V V V E C L G K L T L<br>2821 GAGTGTGCAGAGGAAGGAACCAGAAATGTTGTTGTTGAATGTCTAGGAAAACTCACTCTA<br><br>961 I D P E T L L P R L K G Y L I S G S S Y<br>2881 ATTGATCCAGAAACTCTCCTTCCACGGCTTAAGGGGTACTTGATATCAGGCTCATCATAT<br><br>981 A R S S V V T A V K F T I S D H P Q P I<br>2941 GCCCGAAGCTCAGTGGTTACGGCTGTGAAATTTACAATTTCTGACCATCCACAACCTATT<br><br>1001 D P L L K N C I G D F L K T L E D P D L<br>3001 GATCCACTGTTAAAGAACTGCATAGGTGATTTCCTAAAAACTTTGGAAGACCCAGATTTG<br><br>1021 N V R R V A L V T F N S A A H N K P S L<br>3061 AATGTGAGAAGAGTAGCCTTGGTCACATTTAATTCAGCAGCACATAACAAGCCATCATTA<br><br>1041 I R D L L D T V L P H L Y N E T K V R K<br>3121 ATAAGGGATCTATTGGATACTGTTCTTCCACATCTTTACAATGAAACAAAAGTTAGAAAG<br><br>1061 E L I R E V E M G P F K H T V D D G L D<br>3181 GAGCTTATAAGAGAGGTAGAAATGGGTCCATTTAAACATACGGTTGATGATGGTCTGGAT<br><br>1081 I R K A A F E C M Y T L L D S C L D R L<br>3241 ATTAGAAAGGCAGCATTTGAGTGTATGTACACACTTCTAGACAGTTGTCTTGATAGACTT<br><br>1101 D I F E F L N H V E D G L K D H Y D I K<br>3301 GATATCTTTGAATTTCTAAATCATGTTGAAGATGGTTTGAAGGACCATTATGATATTAAG<br><br>1121 M L T F L M L V R L S T L C P S A V L Q<br>3361 ATGCTGACATTTTTAATGTTGGTGAGACTGTCTACCCTTTGTCCAAGTGCAGTATTGCAG<br><br>1141 R L D R L V E P L R A T C T T K V K A N<br>3421 AGGTTGGACCGACTTGTTGAGCCGTTACGCGCCACGTGTACAACGAAGGTGAAGGCAAAC<br><br>1161 S V K Q E F E K Q D E L K R S A M R A V<br>3481 TCAGTAAAGCAGGAGTTTGAAAAGCAGGACGAACTGAAGCGATCGGCCATGAGAGCAGTC<br><br>1181 A A L L T I P E A E K S P L M S E F Q S<br>3541 GCGGCCCTGCTGACCATTCCCGAGGCCGAGAAGAGCCCGCTGATGAGCGAGTTCCAGTCG<br><br>1201 Q I S S N P E L A A I F E S I Q K D S S<br>3601 CAGATCAGCTCCAACCCTGAGCTGGCAGCCATCTTTGAGAGCATCCAGAAAGACTCATCG | |

| Gene Name | GenBank Homology | DNA SEQUENCE / DEDUCED AMINO ACID SEQUENCE | SEQUENCE IDENTIFIER: |
|---|---|---|---|
| | | 1221 S T N L E S M D T S -<br>3661 TCCACTAACTTGGAATCAATGGACACTAGTTAG | |
| WBC025E03.bFSP_20011710.abd | Homo sapiens v-ets erythroblastosis virus E26 oncogene homolog 2 (avian) (ETS2), mRNA. | 1 GCCCGGTTACTTCCTCCAGAGACTGACGAGTGCGGTGTCGCTCCAGCTCAGAGCTCCCGG<br>61 AGCCGCCCGGCCAGCGTCCGGCCTCCCTGATCGTCTCTGGCCGGCGCCCTCGCCCTCGCC<br>121 CGGCGCGCACCGAGCAGCCGCGGGCGCCGAGCAGCCACCGTCCCGACCAAGCGCCGGCCC<br>181 TGCCCGCAGCGGCAGGATGAATGATTTCGGAATCAAGAATATGGACCAGGTAGCCCCTGT<br>241 GGCTAACAGTTACAGAGGGACACTCAAGCGCCAGCCAGCCTTTGACACCTTTGATGGGTC<br>301 CCTGTTTGCTGTTTTTCCTTCTCTAAATGAAGAGCAAACACTGCAAGAAGTGCCAACAGG<br>361 CTTGGATTCCATTTCTCATGACTCCGCCAACTGTGAATTGCCTTTGTTAACCCCGTGCAG<br>421 CAAGGCTGTGATGAGTCAAGCCTTAAAAGCTACCTTCAGTGGCTTCAAAAAGGAACAGCG<br>481 GCGCCTGGGCATTCCAAAGAACCCCTGGCTGTGGAGTGAGCAACAGGTATGCCAGTGGCT<br>541 TCTCTGGGCCACCAATGAGTTCAGTCTGGTGAACGTGAATCTGCAGAGGTTCGGCATGAA<br>601 TGGCCAGATGCTGTGTAACCTTGGCAAGGAACGCTTTCTGGAGCTGGCACCTGACTTTGT<br>661 GGGTGACATTCTCTGGGAACATCTGGAGCAAATGATCAAAGAAAACCAAGAAAAGACAGA<br>721 AGATCAATATGAAGAAAATTCACACCTCACCTCCGTTCCTCATTGGATTAACAGCAATAC<br>781 ATTAGGTTTTGGCACAGAGCAGGCGCCCTATGGAATGCAGACACAGAATTACCCCAAAGG<br>841 CGGCCTCCTGGACAGCATGTGTCCGGCCTCCACACCCAGCGTACTCAGCTCTGAGCAGGA<br>901 GTTTCAGATGTTCCCCAAGTCTCGGCTCAGCTCCGTCAGCGTCACCTACTGCTCTGTCAG<br>961 TCAGGACTTCCCAGGCAGCAACTTGAATTTGCTCACCAACAATTCTGGGACTCCCAAAGA<br>1021 CCACGACTCCCCTGAGAACGGTGCGGACAGCTTCGAGAGCTCAGACTCCCTCCTCCAGTC<br>1081 CTGGAACAGCCAGTCGTCCTTGCTGGATGTGCAACGGGTTCCTTCCTTCGAGAGCTTCGA<br>1141 AGATGACTGCAGCCAGTCTCTCTGCCTCAATAAGCCAACCATGTCTTTCAAGGATTACAT<br>1201 CCAAGAGAGGAGTGACCCAGTGGAGCAAGGCAAACCAGTTATACCTGCAGCTGTGCTGGC<br>1261 CGGCTTCACAGGAAGTGGACCTATTCAGCTGTGGCAGTTTCTCCTGGAGCTGCTATCAGA<br>1321 CAAATCCTGCCAGTCATTCATCAGCTGGACTGGAGACGGATGGGAGTTTAAGCTCGCCGA<br>1381 CCCCGATGAGGTGGCCCGCCGGTGGGGAAAGAGGAAAAATAAGCCCAAGATGAACTACGA<br>1441 GAAGCTGAGCCGGGGCTTACGCTACTATTACGACAAGAACATCATCCACAAGACGTCGGG<br>1501 GAAGCGCTACGTGTACCGCTTCGTGTGCGACCTCCAGAACTTGCTGGGGTTCACGCCCGA<br>1561 GGAACTGCACGCCATCCTGGGCGTCCAGCCCGACACGGAGGACTGAGGTCGCCGGGACCA<br>1621 CCCTGAGCCGGCCCCAGGCTCGTGGACTGAGTGGGAAGCCCATCCTGACCAGCTGCTCCG<br>1681 AGGACCCAGGAAAGGCAGGATTGAAAATGTCCAGGAAAGTGGCCAAGAAGCAGTGGCCTT<br>1741 ATTGCATCCCAAACCACGCCTCTTGACCAGGCTGCCTCCCTTGTGGCAGCAACGGCACAG<br>1801 CTAATTCTACTCACAGTGCTTTTAAGTGAAAATGGTCGAGAAAGAGGCACCAGGAAGCCG<br>1861 TCCTGGCGCCTGGCAGTCCGTGGGACGGATGGTTCTGGCTGTTTGAGATTCTCAAAGGA<br>1921 GCGAGCATGTCGTGGACACACACAGACTATTTTTAGATTTTCTTTTGCCTTTTGCAACCA<br>1981 GGAACAGCAAATGCAAAAACTCTTTGAGAGGGTAGGAGGGTGGAAGGAAACAACCATGT<br>2041 CATTTCAGAAGTTAGTTTGTATATATTATTATAATCTTATAATTGTTCTCAGAATCCCTT<br>2101 AACAGTTGTATTTAACAGAAATTGTATATTGTAATTTAAAATAATTATATAACTGTATTT<br>2161 GAAATAAGAATTCAGACATCTGAGGTTTTATTTCATTTTTCAATAGCACATATGGAATTT<br>2221 TGCAAAGATTTAATCTGCCAAGGGCCGACTAAGAGAAGTTGTAAAGTATGTATTATTTAC | SEQ ID NO:119 |

| Gene Name | GenBank Homology | DNA SEQUENCE / DEDUCED AMINO ACID SEQUENCE | SEQUENCE IDENTIFIER: |
|---|---|---|---|
| | | 2281 ATTTAATAGACTTACAGGGATAAGGCCTGTGGGGGGTAATCCCTGCTTTTTGTGTTTTTT<br>2341 TGTTTGTTTGTTTGTTTGTTTTTGGGGGGTTTTCTTGCCTTGGTTCTGGCAAAGACTTTG<br>2401 TACATTTGGGAATTTTTATAAGAAACTTAATGTTATTATCTGGGGGCACATTGGCCCCTG<br>2461 TGCTTTCTCCTTTAATTGTAATGTAAAAGCTATAAAGCGGTATTTTTCTTGACAAATGGC<br>2521 ATATGTTTTCCATTTCTATGCATGCATTTAAATCAGTTTATACAAAAAACTTATTTTATT<br>2581 TTTTAGTTTTAACTGTGTTTCTCCGACAGCTCACCTCTCTCTGACCACCCAGCCATTTCC<br>2641 TTCCTGTGCTCCACGTTCTTCTGTATGATGAAAATAAGAATACTATTTTTGGAAATATGC<br>2701 GACTCCTTTTCAGAGACCAGGAGGGATTTATGTAGCAGCTATTTTTACTGCAAAAGTAAT<br>2761 TCACTGGAAAAAAATGTAATTTGTAAGAAAGCTTTATTTTTATCTCAGCTCTATGTAAAG<br>2821 TTAAAGTTACTGTACAGAGCTGAAGGACGGGGGGCGGTAGGGGTCTTGATGAAACCTCTT<br>2881 GAACGAAGCACAGTTTGTCCCATCTTTGTTCACTCGTGTGTCTCAACCATCTTAATAGCA<br>2941 TGCTGCTCCTTTTTGCTCAGTGTCCACAGCAAGATGACGTGATTCTTATTTTCTTGGACA<br>3001 CAGACTATTCTGAGGCACAGAGCGGGGACTTAAGATGGGAAAGAGAAAGCATCGGAGCCA<br>3061 TTCATTCGGAGAAAACGTTTTGATCAAAATGGAGACTTTTGTAGTCGTTTCAAAAGAGCA<br>3121 CCTGAGTCATGTGTATTCCCGGCCTTTATAAATGACCCGGTCAAGTTGGTTTCAAAGTCC<br>3181 GACAGGCTTGTCTGTTTACTAGCTGCGTGGCCTTGGACGGGTGGCTGACATCTGTAAAGA<br>3241 ATCCTCCTGTGATGAAACTGAGGAATCGGGTGGCCGGGCAAGCTGGGAAGAGCAAAGCCA<br>3301 GAGCTGCGCTGCCTCAATACCCACACGAGACGATTTCCAGTATCTCAGCACAGGATGTTT<br>3361 TTCTCAAGCAGGCCCTGTTTATCTCTCGGACATCTGTTTATAACATCAACAGACATGCGA<br>3421 CTGGGAACATCTTGTTGCTGAAAGAAGTCATGAACCATTGGCTCATTTTATATGGGCTCG<br>3481 AGCCACATGAAATTGCCAATATTTGACTTTTTTATCTACGAAGTTGGCAGTTTTGTGTGG<br>3541 TCCAACCTAATAGTTATGGAATCTGAAGTCCAGTATAAACCTCTGGCGTGGTAATGAACA<br>3601 GGACTTAAGTATAAAAATTTTGTAATTGGGCCTCTTCTTTCTCAAATAATAAAGTATTTT<br>3661 GTTTATATAAAAAAAAAAAAAAAA | |
| | | 1 M N D F G I K N M D Q V A P V A N S Y R<br>1 ATGAATGATTTCGGAATCAAGAATATGGACCAGGTAGCCCCTGTGGCTAACAGTTACAGA<br><br>21 G T L K R Q P A F D T F D G S L F A V F<br>61 GGGACACTCAAGCGCCAGCCAGCCTTTGACACCTTTGATGGGTCCCTGTTTGCTGTTTTT<br><br>41 P S L N E E Q T L Q E V P T G L D S I S<br>121 CCTTCTCTAAATGAAGAGCAAACACTGCAAGAAGTGCCAACAGGCTTGGATTCCATTTCT<br><br>61 H D S A N C E L P L L T P C S K A V M S<br>181 CATGACTCCGCCAACTGTGAATTGCCTTTGTTAACCCCGTGCAGCAAGGCTGTGATGAGT<br><br>81 Q A L K A T F S G F K K E Q R R L G I P<br>241 CAAGCCTTAAAAGCTACCTTCAGTGGCTTCAAAAAGGAACAGCGGCGCCTGGGCATTCCA<br><br>101 K N P W L W S E Q Q V C Q W L L W A T N<br>301 AAGAACCCCTGGCTGTGGAGTGAGCAACAGGTATGCCAGTGGCTTCTCTGGGCCACCAAT | SEQ ID NO:120 |

| Gene Name | GenBank Homology | DNA SEQUENCE / DEDUCED AMINO ACID SEQUENCE | SEQUENCE IDENTIFIER: |
|---|---|---|---|
| | | 121 E F S L V N V N L Q R F G M N G Q M L C<br>361 GAGTTCAGTCTGGTGAACGTGAATCTGCAGAGGTTCGGCATGAATGGCCAGATGCTGTGT<br><br>141 N L G K E R F L E L A P D F V G D I L W<br>421 AACCTTGGCAAGGAACGCTTTCTGGAGCTGGCACCTGACTTTGTGGGTGACATTCTCTGG<br><br>161 E H L E Q M I K E N Q E K T E D Q Y E E<br>481 GAACATCTGGAGCAAATGATCAAAGAAAACCAAGAAAAGACAGAAGATCAATATGAAGAA<br><br>181 N S H L T S V P H W I N S N T L G F G T<br>541 AATTCACACCTCACCTCCGTTCCTCATTGGATTAACAGCAATACATTAGGTTTTGGCACA<br><br>201 E Q A P Y G M Q T Q N Y P K G G L L D S<br>601 GAGCAGGCGCCCTATGGAATGCAGACACAGAATTACCCCAAAGGCGGCCTCCTGGACAGC<br><br>221 M C P A S T P S V L S S E Q E F Q M F P<br>661 ATGTGTCCGGCCTCCACACCCAGCGTACTCAGCTCTGAGCAGGAGTTTCAGATGTTCCCC<br><br>241 K S R L S S V S V T Y C S V S Q D F P G<br>721 AAGTCTCGGCTCAGCTCCGTCAGCGTCACCTACTGCTCTGTCAGTCAGGACTTCCCAGGC<br><br>261 S N L N L L T N N S G T P K D H D S P E<br>781 AGCAACTTGAATTTGCTCACCAACAATTCTGGGACTCCCAAAGACCACGACTCCCCTGAG<br><br>281 N G A D S F E S S D S L L Q S W N S Q S<br>841 AACGGTGCGGACAGCTTCGAGAGCTCAGACTCCCTCCTCCAGTCCTGGAACAGCCAGTCG<br><br>301 S L L D V Q R V P S F E S F E D D C S Q<br>901 TCCTTGCTGGATGTGCAACGGGTTCCTTCCTTCGAGAGCTTCGAAGATGACTGCAGCCAG<br><br>321 S L C L N K P T M S F K D Y I Q E R S D<br>961 TCTCTCTGCCTCAATAAGCCAACCATGTCTTTCAAGGATTACATCCAAGAGAGGAGTGAC<br><br>341 P V E Q G K P V I P A A V L A G F T G S<br>1021 CCAGTGGAGCAAGGCAAACCAGTTATACCTGCAGCTGTGCTGGCCGGCTTCACAGGAAGT<br><br>361 G P I Q L W Q F L L E L L S D K S C Q S<br>1081 GGACCTATTCAGCTGTGGCAGTTTCTCCTGGAGCTGCTATCAGACAAATCCTGCCAGTCA<br><br>381 F I S W T G D G W E F K L A D P D E V A<br>1141 TTCATCAGCTGGACTGGAGACGGATGGGAGTTTAAGCTCGCCGACCCCGATGAGGTGGCC<br><br>401 R R W G K R K N K P K M N Y E K L S R G | |

| Gene Name | GenBank Homology | DNA SEQUENCE / DEDUCED AMINO ACID SEQUENCE | SEQUENCE IDENTIFIER: |
|---|---|---|---|
| | | 1201 CGCCGGTGGGGAAAGAGGAAAAATAAGCCCAAGATGAACTACGAGAAGCTGAGCCGGGGC<br><br>421 L R Y Y Y D K N I I H K T S G K R Y V Y<br>1261 TTACGCTACTATTACGACAAGAACATCATCCACAAGACGTCGGGGAAGCGCTACGTGTAC<br><br>441 R F V C D L Q N L L G F T P E E L H A I<br>1321 CGCTTCGTGTGCGACCTCCAGAACTTGCTGGGGTTCACGCCCGAGGAACTGCACGCCATC<br><br>461 L G V Q P D T E D -<br>1381 CTGGGCGTCCAGCCCGACACGGAGGACTGA | |
| WBC027B01.bFSP_20 012210.abd | No Homology. | 1 CCTTATTTAACCAGTCAAGTAACCAACCAAAGTGTTAAGTTTAGGCATTTAGGAAAAGCT<br>61 GTAAACTCTCACATTGCTGAAATTCCCCCTCATTGTGTCCCCTGGCCAGGTGCCTCCCGA<br>121 GGGTTGGTCTTGTTCCATTTTGTTCTGGTCACCGGATGATTTCTTCCTTCGCCATCAAAT<br>181 ACTTCTTCGTAATGGTCACCATCTGAGGGTTTGTCCCATTTCTGGATTCTTCCCAAGCTC<br>241 TAACCCAACACTCCTCCTATTTAAAACATCCCACCGTGGTCAGACCTCTGTGGTGAAGCT<br>301 GACTCTCACCCCTCACTGTTCTGTCTACAGAGCTGCTCGCTAGAGTGGCCTGCCCTGCTG<br>361 CTCGAGCGCGAGGGCGGGGCACCGTGGAAGAACTCTGACTGCTTGCAGACTGTCTCTGCT<br>421 TGGGTTCTTATTGCTTTCTGCTTTTCGAAATTATGCCAGCCTCTGCCTGGGTCTGGGTCT<br>481 AGATTAGGATTTAAGCTGGTAAAGGAAAATGTTGGTAAATCCTCTGCTCAGAAGTCATTT<br>541 ATCATGTTTCTATTTAAGGATTAAAGTTATTAACTTACCCTATTTAGAAGCTGACCCATT<br>601 TAAATATAACTCTTCGAAGTTCTACTTCTCTTTTATTCTTGATGTCTTAAGATGATTCTA<br>661 ACTGGCATCTGGGGCTTGGGGTCTCCCTGTCCTTCCATTATCTTTTGTTTTTCCCAAGAA<br>721 ATTCTGAACACATACCTCTTTGCTAAACCAGGATCCTCTGTTTTGACTCTGTTTTCGTAC<br>781 CAGGTAAGAGGACTTCTTTCAAAGAGCTTTTTACTGCTTGACCAAAGAACAAATCTGAAA<br>841 ATCACCAAGTTAACGTTGTTACTTTTTCTGTTGACCAAAGCTTAAGTGAGGAGAATTTTT<br>901 GGCTT<br><br>1 ACTCTCAGCATCTGAGAGCCTTAAGCCCCCGAAATATTCCAAGGTTTTATTGTACTTTTT<br>61 TAAAGAACAGAAAAGGGTGAATAAAAGAACCAAGTAGAAATGCAGGGACTATATTTAGCA<br>121 TGTACAAGTTGAAGTAAAAAAGAGATTGTGGCAACTAAGTAACTAGTCCTGTAAAATAAA<br>181 ATACATTGCTCACCTTTCCTGTACATCTAGTTCCTCACCATAGAACCCACCTGGGCAAAC<br>241 CCCAACTCTAGAACTCTCAGACGCTGCGACAGCGTCCGAACAGGCAAAGAGCTAGATTTC<br>301 AAAGGCTCGCTTCGCAAAAATACTCCATCTTGGCACCTTGCTCTTTTAGAAAGGCCTTAA<br>361 TGATTGAAAAAAATTACTTGGCCCCTGAGAGTATAATTTTCAGCCCCTGAAGCTTGCTAA<br>421 TATAAAGAACAACTTTTAATTATGAGAAATAAAAACTGAGATGCTGTAAAATCCTGTCAG<br>481 ACCACCGATAGACTGATCGGAAAGACAGTGTCAGCTCAGAAGGGGTCAGCGGACACTGTA<br>541 CGGAGCGTGGGTGTTTGGATTCTCAGGGAAACTCCGACTGGATTTATTTTTCTACCTGTG<br>601 TATGTCTTATATAGTGCTGGTGCTCTATAGTTTTGTTCTCTATTTAATCTGTATGTATTA<br>661 ACGATAACACGTTTGATAGTTGTCACATAAAAAAAAAAAAAA | SEQ ID NO:121<br><br><br><br><br><br><br><br><br><br><br><br><br><br><br><br><br>SEQ ID NO:122 |
| WBC027D09.bFSP_20 | No homology | | |

| Gene Name | GenBank Homology | DNA SEQUENCE / DEDUCED AMINO ACID SEQUENCE | SEQUENCE IDENTIFIER: |
|---|---|---|---|
| 012210.abd | | 1 TGCATCCTGTCAGGATAGAATCCTATCAGACAACCAATTTGGATTTGTCCTCTTATGGAG<br>61 AACGTTCATTTCCGTCCCTTGATTAAGATGGTGTGTGCTATGCTTCTCCATCGCAAAGTC<br>121 ACTCTTTTATCCTTTGAAATTAACAAATATTTGTTTGGGGATGTGCTTGAGATGATGTAA<br>181 ATGTCCTATTCCTCATTAAATCTCTAATTTATCCATTTATTCCTGATATAGACTTAGTGA<br>241 TTCTTATTTTATTCGGTGGGCTGTAATCTGTTATCATTATCATCTCATTTATCTATTCTC<br>301 ATGTGCCCATTTTTCTTGATTTGGCCAATAGGACACCTTCAAGCTGACTTCCGTGTCTTT<br>361 TTTGACTTGTCCCTACTTCCTTACTTTCTGACAGCAATATGATTTTCCTGGCTCATCTCA<br>421 TTCTTTCCCTTCCCCAGCCCAGGAATCTACCATTTCTCTAAGGAGCCTTGCTTTCTTTTA<br>481 GTGGAGAATAGTATTAAGAAAACCAAGATCTGGGTACTAAATGATCTCATTGCTATTGGA<br>541 GTCTTGCTACTCCTAGATCCTTTCCATGGACAGAGGTTGGGAATGTGTACACACACACAT<br>601 ACACATATGCACACATACACATGCAAACATAATTTGTTCATGTTCAACATCTATATTTCT<br>661 ATATTTATCTATATTAAAAATCATGATGACACACTCACATCTCTGATTTCAGTCCAACAC<br>721 TACAGGGTTCATTCTGGTTCTCCACTTTCATATTTGTAATTTCCTTCCCCACCAGTGAGA<br>781 AACCGGGCTTCCATTATCCTCCACATATATCCTTATTTGGCCAGTTCTTCTGTATGTATC<br>841 CAGTATCCCAT<br><br>1 TGGTAGAAATGAGTCAAAGTTCTGAGTGTTAGGATAGTTGAGATGAACTGGAGCTACTTC<br>61 ACAATTTTGCCTGCACTCTTCATTCTTACCTGCACCCTAACCCCTTGCACCATGACTGAT<br>121 GTGTGGCAAATACTCAATGTTAGTGAATAGAACACGTTAGTTCTTGTGTTATTGCATTAG<br>181 CTCCCTTCGATGGGCATCTCTATGAATTAGATGGATGGAAACTATTTCCAATTAACTTTA<br>241 TTAGAGGATGCCACAGAGGTTTGCAAGAAACTTATGGAATGTGACCCTGATGAATTAGGA<br>301 TTCATTGTGATCACTCTTTCTGACGCATAGCTTGTCAATAATGGAAGCACCAAATACTGT<br>361 CTTATTTGCAACAGAAATTAAATTTTTGCTGCCATACACTAACTCAAAAATTTTGATATT<br>421 TTCATTAACTTGACTTGATAAACTTTATGTGAATTAAACCTTGTGAATTAAACCTTGTCT<br>481 TTAAAAAAAAAAAA | SEQ ID NO:123<br><br>SEQ ID NO:124 |
| WBC027E07.bFSP_20 012210.abd | No Homology | 1 ACTCATTTATATCTTGTAGTTTACTATTTTACCTCTAGAAATAGACTTAAAAGTAGTTTG<br>61 TGTGTGAATATTTAACTACACGAAAGTTAGCAAAATTAACATTTGGAAACCTTCTATTCC<br>121 ATATGTTAGCATTTATTCCAACATCTTTTTAGCCTTATTTAGTCTAATTTAAAATTTTCA<br>181 AGCAACCCTATCTTATCATCTTCACATGGTGTTTACAATTTTGCAACGTGGATTATTCTC<br>241 TGTATAATATGTGAAATTCATTGCTTGATACTTTTGACCCAAAATTATGTTTGTTACAGT<br>301 TGAACTTAAATAAAGATCTTTAAACAAAAAAAAAAAAAAAAAACGCGAGACTAGTTCTCT<br>361 CGTGACACCCACTCTTCCACCTTCGATGCTGGGGCTGGCATTGCCCTCAACGACCACTTT<br>421 GTCAAGCTCATTTCCTGGTATGACAATGAATTTGGCTACAGCAATAGGGTGGTGGACCTT<br>481 ATGGCCCACATGGCCTCCAAGGAGTAAGAGCCCCCTGGACCACCAATCACCCAGCAAGAG<br>541 AAGGAGAAAGGCCCTCAGCTGCTGGGAATGCTTGTCCCAACTTGACCCCCTAACATATTG<br>601 AGAGTCTCCTGACCTCCACAGTTTCCATCTCAGACCCCCCTGAAGAAGGGGAGGGGCTTG<br>661 GGGAGCCCTACCTTGTCATGTACCATCAATAAAGTACACTGTACCCAAAAAAAAAAAAAA<br>721 AAAAAAAAA | SEQ ID NO:125 |
| WBC027F05.bFSP_20 012210.abd | Homo sapiens collagenase mRNA. | 1 GCTCGCCAGGGAAGGGCCCTACCCAGAGGACAGAAAGAAAGCCAGGAGGGGTAGAGTTTG | SEQ ID NO:126 |

| Gene Name | GenBank Homology | DNA SEQUENCE / DEDUCED AMINO ACID SEQUENCE | SEQUENCE IDENTIFIER: |
|---|---|---|---|
| | | 61 AAGAGAAGATCATGTTCTCCCTGAAGACGCTTCCATTTCTGCTCTTACTCCATGTGCAGA<br>121 TTTCCAAGGCCTTTCCTGTATCTTCTAAAGAGAAAAATACAAAAACTGTTCAGGACTACC<br>181 TGGAAAAGTTCTACCAATTACCAAGCAACCAGTATCAGTCTACAAGGAAGAATGGCACTA<br>241 ATGTGATCGTTGAAAAGCTTAAAGAAATGCAACGATTCTTTGGGTTGAATGTGACAGGGA<br>301 AGCCAAATGAGGAAACTCTGGAAATGATGCAGAAACCTCGCTGTGGAGTGCCTGACACTG<br>361 GAGAGTTTATGATAACCCCAGGGAACCCCAAGTGGGAACGAAATAACCTGACCTACAGGA<br>421 TTATTAACTATACCCCACAGCTGTCAGAGGCTGAGGTAGAAAGAGCTATCAAGGATGCCT<br>481 TTGAACTCTGGAGTGTTGCATCACCTCTCATCTTCACCAGGATCTCACAGGGAGAAGCAG<br>541 ACATCAGGATTGCTTTTTACCAAGGAGATCATGGTGACAATTCTCCATTTGATGGACCCA<br>601 ATGGAATCCTTGCTCATGCCTTTCAGCCAGGCCCAGGTATTGGAGGAGATGCTCATTTTG<br>661 ATGCAGAAGAAACATGGACCGACAACAGAACAAATTACAACTTGCTTATTGTTGCTGCCC<br>721 ATGAATTTGGCCATTCCTTGGGGCTCGCTCACTCCACAAACCCTGCTGCCTTGATGTATC<br>781 CCACCTACGCTTTCAGGGACCCCAGCACCTACTTGCTCTCTCAGGATGACATCAATGGCA<br>841 TTCAGGCCATCTATGGACCTTCAAATGACCCTATCCAACCAACTGGATCAACCGCGCCCA<br>901 CCGCCTGTGACCCCAGACTGACATTTGATGCTATCGTCACACTCCGCGGAGAAACGCTTT<br>961 TCTTTAAAGACAGGTACTTTTGGAGAAGGCATCCCCAACTGCCAACAGTCGAACTCAATT<br>1021 TCATTTCTCTGTTCTGGCCATCCTTGCCAAATGGTATACAGGCGGCTTATGAGGATTTTG<br>1081 ACAGGGACCTAATTTTCCTATTTAAAGGCAACCAATACTGGGCTCTGAATGGCTATGACA<br>1141 TTCAGCAAGGTTATCCCAAGGACATATCAAACTACGGCTTCCCAAGCAGTGTCCAAGCAA<br>1201 TTGATGCAGCTGTTTCCTATAGGAGTAAAACATACTTCTTTGTAAATGATCAATTCTGGA<br>1261 GATATGATAACCAAAGACGATCCATGGAAGCAGGTTATCCCCAAAGCATAGCAAGTACCT<br>1321 TTCCAGGAATAGGAAGCAGAATTGATGCAGTTTTCCAGCAAGATGACTTCTTTCTTTTCT<br>1381 TCAGCGGACCAGTTTATTATGCATTTGATCTTAATACTCAGAGAGTTACTAGGGTTGCAA<br>1441 GAATCAATAAATGGCTTAACTGTAGATATGGCTGAAGCAAAATCAAATGTGGCTGTATCC<br>1501 ACTTTCAGAATGTTGAAGGGAAGTTCAGCATGCATTTTCGTTACATTGTGTCCTGCTTAT<br>1561 ACTTTTCTCAATATTAAGTCATTGTTTCCCATCACTGTATCCATTCTACCTGTCCTCCGT<br>1621 GAAAATATGTTTGGAATATTCCACTATTTGCAGAGGCTTATTCAGTTCTTACACATTCCA<br>1681 TCTTACATTAGTGATTCCATCAAAGAGAAGGAAAGTAAGCCTTTTTGTCACCTCAATATT<br>1741 TACTATTTCAATACTTACATATCTGACTTCTAGGATTTATTGTTATATTACTTGCCTATC<br>1801 TGACTTCATACATCCCTCAGTTTCTTAAAATGTCCTATGTATATCTTCTACATGCAATTT<br>1861 AGAACTAGATTTTGGTTAGAAGTAAGGATTATAAACAACCTAGACAGTACCCTTGGCCTT<br>1921 TACAGAAAATATGGTGCTGTTTTCTACCCTTGGAAAGAAATGTAGATGATATGTTTCGTG<br>1981 GGTTGAATTGTGTCCCCCATAAAAGATATGTTGAAGTTCTAACCCCAGGTACCCATGAAT<br>2041 GTGAGCTTACCAGGGTCTTTGCAGATGTAATTAGTTAAGTTAAGGTGAGATCACACTGAA<br>2101 TTAGGGTGGGCTCTAAATCCATTATGACTGTTGTTCTTATAAGAAGAAGAGAGCATAGCC<br>2161 ACCTAGGGGAGGAGGCCGTGTGAAGACAGAGGCAGAGATTGGAGTGACGCATCTCCAAGC<br>2221 CAA | |
| | | 1 M F S L K T L P F L L L L H V Q I S K A<br>1 ATGTTCTCCCTGAAGACGCTTCCATTTCTGCTCTTACTCCATGTGCAGATTTCCAAGGCC<br><br>21 F P V S S K E K N T K T V Q D Y L E K F<br>61 TTTCCTGTATCTTCTAAAGAGAAAAATACAAAAACTGTTCAGGACTACCTGGAAAAGTTC | SEQ ID NO:127 |

| Gene Name | GenBank Homology | DNA SEQUENCE / DEDUCED AMINO ACID SEQUENCE | SEQUENCE IDENTIFIER: |
|---|---|---|---|
| | | 41 Y Q L P S N Q Y Q S T R K N G T N V I V<br>121 TACCAATTACCAAGCAACCAGTATCAGTCTACAAGGAAGAATGGCACTAATGTGATCGTT<br><br>61 E K L K E M Q R F F G L N V T G K P N E<br>181 GAAAAGCTTAAAGAAATGCAACGATTCTTTGGGTTGAATGTGACAGGGAAGCCAAATGAG<br><br>81 E T L E M M Q K P R C G V P D T G E F M<br>241 GAAACTCTGGAAATGATGCAGAAACCTCGCTGTGGAGTGCCTGACACTGGAGAGTTTATG<br><br>101 I T P G N P K W E R N N L T Y R I I N Y<br>301 ATAACCCCAGGGAACCCCAAGTGGGAACGAAATAACCTGACCTACAGGATTATTAACTAT<br><br>121 T P Q L S E A E V E R A I K D A F E L W<br>361 ACCCCACAGCTGTCAGAGGCTGAGGTAGAAAGAGCTATCAAGGATGCCTTTGAACTCTGG<br><br>141 S V A S P L I F T R I S Q G E A D I R I<br>421 AGTGTTGCATCACCTCTCATCTTCACCAGGATCTCACAGGGAGAAGCAGACATCAGGATT<br><br>161 A F Y Q G D H G D N S P F D G P N G I L<br>481 GCTTTTTACCAAGGAGATCATGGTGACAATTCTCCATTTGATGGACCCAATGGAATCCTT<br><br>181 A H A F Q P G P G I G G D A H F D A E E<br>541 GCTCATGCCTTTCAGCCAGGCCCAGGTATTGGAGGAGATGCTCATTTTGATGCAGAAGAA<br><br>201 T W T D N R T N Y N L L I V A A H E F G<br>601 ACATGGACCGACAACAGAACAAATTACAACTTGCTTATTGTTGCTGCCCATGAATTTGGC<br><br>221 H S L G L A H S T N P A A L M Y P T Y A<br>661 CATTCCTTGGGGCTCGCTCACTCCACAAACCCTGCTGCCTTGATGTATCCCACCTACGCT<br><br>241 F R D P S T Y L L S Q D D I N G I Q A I<br>721 TTCAGGGACCCCAGCACCTACTTGCTCTCTCAGGATGACATCAATGGCATTCAGGCCATC<br><br>261 Y G P S N D P I Q P T G S T A P T A C D<br>781 TATGGACCTTCAAATGACCCTATCCAACCAACTGGATCAACCGCGCCCACCGCCTGTGAC<br><br>281 P R L T F D A I V T L R G E T L F F K D<br>841 CCCAGACTGACATTTGATGCTATCGTCACACTCCGCGGAGAAACGCTTTTCTTTAAAGAC<br><br>301 R Y F W R R H P Q L P T V E L N F I S L<br>901 AGGTACTTTTGGAGAAGGCATCCCCAACTGCCAACAGTCGAACTCAATTTCATTTCTCTG | |

| Gene Name | GenBank Homology | DNA SEQUENCE / DEDUCED AMINO ACID SEQUENCE | SEQUENCE IDENTIFIER: |
|---|---|---|---|
| | | 321 F W P S L P N G I Q A A Y E D F D R D L<br>961 TTCTGGCCATCCTTGCCAAATGGTATACAGGCGGCTTATGAGGATTTTGACAGGGACCTA<br><br>341 I F L F K G N Q Y W A L N G Y D I Q Q G<br>1021 ATTTTCCTATTTAAAGGCAACCAATACTGGGCTCTGAATGGCTATGACATTCAGCAAGGT<br><br>361 Y P K D I S N Y G F P S S V Q A I D A A<br>1081 TATCCCAAGGACATATCAAACTACGGCTTCCCAAGCAGTGTCCAAGCAATTGATGCAGCT<br><br>381 V S Y R S K T Y F F V N D Q F W R Y D N<br>1141 GTTTCCTATAGGAGTAAAACATACTTCTTTGTAAATGATCAATTCTGGAGATATGATAAC<br><br>401 Q R R S M E A G Y P Q S I A S T F P G I<br>1201 CAAAGACGATCCATGGAAGCAGGTTATCCCCAAAGCATAGCAAGTACCTTTCCAGGAATA<br><br>421 G S R I D A V F Q Q D D F F L F F S G P<br>1261 GGAAGCAGAATTGATGCAGTTTTCCAGCAAGATGACTTCTTTCTTTTCTTCAGCGGACCA<br><br>441 V Y Y A F D L N T Q R V T R V A R I N K<br>1321 GTTTATTATGCATTTGATCTTAATACTCAGAGAGTTACTAGGGTTGCAAGAATCAATAAA<br><br>461 W L N C R Y G -<br>1381 TGGCTTAACTGTAGATATGGCTGA | |
| WBC027H11.bFSP_20 012210.abd | Homo sapiens ribosomal protein S8, mRNA (cDNA clone MGC:90249 IMAGE:6527505). | 1 GCGCCGAGCGATGGGCATCTCTCGGGACAACTGGCACAAGCGCCGCAAGACCGGGGGCAA<br>61 GAGAAAGCCCTACCACAAGAAGCGGAAGTATGAGCTGGGCCGGCCCGCGGCCAACACGAA<br>121 GATTGGCCCCCGCCGCATACACACAGTCCGCGTGCGGGGAGGCAACAAGAAGTACCGGGC<br>181 CTTGAGGTTGGACGTGGGAACTTCTCCTGGGGCTCCGAGTGCTGTACGCGCAAAACAAG<br>241 GATTATTGATGTTGTCTACAATGCATCCAACAACGAATTGGTCCGCACCAAGACCCTGGT<br>301 GAAGAACTGCATCGTGCTGGTTGACAGCACACCGTACCGACAGTGGTACGAGTCCCACTA<br>361 CGCACTGCCCCTGGGCCGCAAGAAGGGGGCCAAGCTGACTCCTGAGGAGGAAGAAATTTT<br>421 AAACAAAAAACGATCTAAAAAAATTCAGAAGAAATATGATGAAAGGAAAAAGAATGCCAA<br>481 AATCAGCAGTCTCCTGGAGGAGCAGTTCCAGCAGGGCAAGCTTCTCGCATGTATCGCTTC<br>541 CAGACCAGGCCAGTGTGGCCGAGCAGACGGCTATGTGCTCGAGGGCAAGGAGCTGGAGTT<br>601 CTATCTGAGGAAAATCAAGGCCCGGAAAGGCAAATAAATCCTTGTTTTGTCTTCACCCAT<br>661 GTAATAAAGGTGTTTATTGTTTTGTTCCCAAAAAAAAAAAAAAA<br><br>1 M G I S R D N W H K R R K T G G K R K P<br>1 ATGGGCATCTCTCGGGACAACTGGCACAAGCGCCGCAAGACCGGGGGCAAGAGAAAGCCC<br><br>21 Y H K K R K Y E L G R P A A N T K I G P<br>61 TACCACAAGAAGCGGAAGTATGAGCTGGGCCGGCCCGCGGCCAACACGAAGATTGGCCCC | SEQ ID NO:128<br><br><br><br><br><br><br><br><br><br><br><br><br>SEQ ID NO:129 |

| Gene Name | GenBank Homology | DNA SEQUENCE / DEDUCED AMINO ACID SEQUENCE | SEQUENCE IDENTIFIER: |
|---|---|---|---|
| | | 41 R R I H T V R V R G G N K K Y R A L R L<br>121 CGCCGCATACACACAGTCCGCGTGCGGGGAGGCAACAAGAAGTACCGGGCCTTGAGGTTG<br><br>61 D V G N F S W G S E C C T R K T R I I D<br>181 GACGTGGGGAACTTCTCCTGGGGCTCCGAGTGCTGTACGCGCAAAACAAGGATTATTGAT<br><br>81 V V Y N A S N N E L V R T K T L V K N C<br>241 GTTGTCTACAATGCATCCAACAACGAATTGGTCCGCACCAAGACCCTGGTGAAGAACTGC<br><br>101 I V L V D S T P Y R Q W Y E S H Y A L P<br>301 ATCGTGCTGGTTGACAGCACACCGTACCGACAGTGGTACGAGTCCCACTACGCACTGCCC<br><br>121 L G R K K G A K L T P E E E E I L N K K<br>361 CTGGGCCGCAAGAAGGGGGCCAAGCTGACTCCTGAGGAGGAAGAAATTTTAAACAAAAAA<br><br>141 R S K K I Q K K Y D E R K K N A K I S S<br>421 CGATCTAAAAAAATTCAGAAGAAATATGATGAAAGGAAAAAGAATGCCAAAATCAGCAGT<br><br>161 L L E E Q F Q Q G K L L A C I A S R P G<br>481 CTCCTGGAGGAGCAGTTCCAGCAGGGCAAGCTTCTCGCATGTATCGCTTCCAGACCAGGC<br><br>181 Q C G R A D G Y V L E G K E L E F Y L R<br>541 CAGTGTGGCCGAGCAGACGGCTATGTGCTCGAGGGCAAGGAGCTGGAGTTCTATCTGAGG<br><br>201 K I K A R K G K -<br>601 AAAATCAAGGCCCGGAAAGGCAAATAA | |
| WBC028D04.bFSP_20 012310.abd | Homo sapiens hypothetical protein FLJ25421, mRNA (cDNA clone MGC:33348 IMAGE:4831324). | 1 GGGGACCGGCCTGGGCCCTTGAGCCTGGCTGGAGTAGGTGGAATATCCTGGGGAGAGGAA<br>61 GTTTGGTCGTTGGGCAGCGGCCGACTTTCCTCTCAGGAAATTGATACTTTATCAACATAG<br>121 AGATTAATTATTTGTATTTGGGAGCAGCAGAACTGGGGACCAATTACCTGTCAAGAGTTT<br>181 TATAAACCCGTGACATAAACTGTTATTCCATTGCTTTCATCCTCCATCTCACCTTGGAAC<br>241 TTGTATTACTTATGGCGTTCAGGAGACAAGTGAAAAACTTTGTGAAAAATTACTCAGATG<br>301 CTGAAATAAAAGTCAGGGAAGCAACTTCTAACGACCCTTGGGGTCCCTCTAGTTCTCTGA<br>361 TGTTAGATATCAGTGACTTGACTTTCAACACAATTTCTCTCTCAGAGATTATGAATATGC<br>421 TGTGGCACAGACTCAATGACCATGGGAAGAACTGGCGCCACGTGTATAAATCCCTTACCC<br>481 TAATGGATTATCTCATCAAGAATGGATCAAAGAAAGTTATTCAGCATTGCAGAGAGGGGT<br>541 TCTGTAACCTTCAAACACTAAAAGATTTTCAGCACATAGATGAAGCTGGAAAAGACCAAG<br>601 GTTATTATATCCGGGAAAAATCTAAGCAAGTCATCACATTGCTGATGGATGAACCATTGC<br>661 TGTGTAAAGAGAGGGAAGTGGCATGTCGGACTAGACAGCGTACCTCCCACTCTATATTGT<br>721 TTTCTAAAAGACAACTTGGTTCAAGTAACTCACTGACAGCGTGCACTTCTGCCCCCACAC<br>781 CGGATATTTCTGCTTCAGAGAAGAAGTATAAGCTTCCTAAGTTTGGAAGGTTACATAATA | SEQ ID NO:130 |

| Gene Name | GenBank Homology | DNA SEQUENCE / DEDUCED AMINO ACID SEQUENCE | SEQUENCE IDENTIFIER: |
|---|---|---|---|
| | | 841 AAAGAAATGTGTGCAAGGCAGGATTGAAACAAGAGCATTGCCAAGATGTTCATTTGCCTA<br>901 CAGAAACTATGTTGTCCCAGGAAACACTTCCACTCAAGATTCATGGTTGGAAATCAACAG<br>961 AGGACCTGATGACATTTTTGGATGATGATCCAGAGCTGCCTTTACTAGCAACACCTCCTT<br>1021 CCATTGTCTCTCCAATCACTTGCTTGTCAGAAGCAGAAGAAGTTTGTAATCTTTCGGGTG<br>1081 CAGATGCTGTGCCTACTCTCTCAGAAAATAGTCCTTCTGGGCAGAGAGATGTGAGTTTGG<br>1141 ACAAGAGATCAGATGGTATCTTTACAAATACTGTCACAGAAAACCTCTTGGAAACACCTT<br>1201 TAGAAAAGCAATCAGCTGCAGAAGGTCTTAAAACATTGACAATTTTACCAGCATGTTGGT<br>1261 CAAGTAAAGAGGAGTTTATCAGCCCCGACTTAAGGGTATCAAAGTCAGATTCTACTTTCC<br>1321 ATAACCAGGCCTCTGTAGAAACACTCTGTCTCTCTCCCTCATTCAAAATATTTGACCGAG<br>1381 TGAAGGAGATTGTAATCAACAAGGCCTACCAGAAACCAGCACAATCCAGCATTCAGATGG<br>1441 ATGATAAAATCCTCAAGACAACCACACGGGTTTCAACTGCTTCTGAGGGAGCATCTTCCT<br>1501 TTTCTCCTTTATCCATGTCATCTCCTGATTTAGCCTCTCCAGAGAAGTCAGCTCATCTCT<br>1561 TATCACCAATTCTGGCCGGACCTTCCTTCTGGACTCTGTCCCATCAACAGTTGTCTTCTA<br>1621 CCTCCTTTAAAGATGAAGATAAGACAGCCAAGCTGCATCACCCCTTTGCTCCTGGAGGCC<br>1681 CAGTGTCTTCTGATGAAGAGGAAAATGACAACCTCAATCTACTGGAAATTCTTCCAGATA<br>1741 ACTCTGATTCTGCTCAAAAGAAAAGTCACATTTCCAGCAGTAACTGGGTAGAGTTTTCCA<br>1801 CCCAAACTGTAGACCCCTTCACCTCTATGTCCTGTTCTGGTTTTCAGACAAGCAAAGGCC<br>1861 TGCCTAGGGAGCCTGAAGTGAAGAATTCCATTAAGGGTCTTCTAGGGGAGGTAAAGAATG<br>1921 CGATAGTCAAATTACACGAAGATCTGAGCATGGTGATCCAGGAGCTGAACGTCATCAATA<br>1981 ACATCTTGATGAGCATGAGTCTGAATAGTTCACAAATAAGCCAGTCTTCCCAGGTCCCCC<br>2041 AGTCTTCTGAGGGGGGCTCAGATCCAGTCTAATCATCACAATATCTATTTTTGATAGAAC<br>2101 TCGTGTGGTTCCACTTCCCCAAGACTTACGTTAGCATTGTATAAATTCATATTATTATGG<br>2161 CAAAAAAGGGGATGGTTTGATTTCTCTTTGTCAGGTTTGTTAACAAGTATCTTTCAAACC<br>2221 AGTAAATTCATTCAGGTGGTTTATTTTTTAAAGAGAAAAAGTATCCCCTTTGATAATATC<br>2281 ATCAGATTTAGATATTAGTTCATTTGAAACTGAAAAGAATTTTATTCTTTCATGCTCTGT<br>2341 ACCTCAAATGGGTTAAGCATTAGTGAAATTTGTGGTAAGACCTCAAACTGCAAGTGACTA<br>2401 TTGCAGAGCCTTTGGATAAGGGAGGCAGGAAGAAGCTCTTTATCTTTCAACTGTATGGAG<br>2461 CTTGTTAACAAGCCTCTGAGGTGACCTAGGAGGATTGAGTTTGGGGGTATACATTTCACA<br>2521 CATTCTTTTGCAAGTTTTAGGTTCTGCAACCAAGGGCCAGAAATCTTTTCTTTCTTCCAG<br>2581 ATGCTAGTGTTAGGAGTTAAATGGAAACAAGAAGGACATGGGGCAAATGTTTTTACTGTC<br>2641 AATGTATATTGCAAAGTGAATTCAAATGCATCCTTTTATTCTACTTCAGAAAATAAATTT<br>2701 GATATTTTTCAAAAAAAAAAAAAAAA | |
| | | 1 M A F R R Q V K N F V K N Y S D A E I K<br>1 ATGGCGTTCAGGAGACAAGTGAAAAACTTTGTGAAAAATTACTCAGATGCTGAAATAAAA<br><br>21 V R E A T S N D P W G P S S S L M L D I<br>61 GTCAGGGAAGCAACTTCTAACGACCCTTGGGGTCCCTCTAGTTCTCTGATGTTAGATATC<br><br>41 S D L T F N T I S L S E I M N M L W H R<br>121 AGTGACTTGACTTTCAACACAATTTCTCTCTCAGAGATTATGAATATGCTGTGGCACAGA<br><br>61 L N D H G K N W R H V Y K S L T L M D Y | SEQ ID NO:131 |

| Gene Name | GenBank Homology | DNA SEQUENCE / DEDUCED AMINO ACID SEQUENCE | SEQUENCE IDENTIFIER: |
|---|---|---|---|
| | | 181 CTCAATGACCATGGGAAGAACTGGCGCCACGTGTATAAATCCCTTACCCTAATGGATTAT<br><br>81 L I K N G S K K V I Q H C R E G F C N L<br>241 CTCATCAAGAATGGATCAAAGAAAGTTATTCAGCATTGCAGAGAGGGGTTCTGTAACCTT<br><br>101 Q T L K D F Q H I D E A G K D Q G Y Y I<br>301 CAAACACTAAAAGATTTTCAGCACATAGATGAAGCTGGAAAAGACCAAGGTTATTATATC<br><br>121 R E K S K Q V I T L L M D E P L L C K E<br>361 CGGGAAAAATCTAAGCAAGTCATCACATTGCTGATGGATGAACCATTGCTGTGTAAAGAG<br><br>141 R E V A C R T R Q R T S H S I L F S K R<br>421 AGGGAAGTGGCATGTCGGACTAGACAGCGTACCTCCCACTCTATATTGTTTTCTAAAAGA<br><br>161 Q L G S S N S L T A C T S A P T P D I S<br>481 CAACTTGGTTCAAGTAACTCACTGACAGCGTGCACTTCTGCCCCCACACCGGATATTTCT<br><br>181 A S E K K Y K L P K F G R L H N K R N V<br>541 GCTTCAGAGAAGAAGTATAAGCTTCCTAAGTTTGGAAGGTTACATAATAAAAGAAATGTG<br><br>201 C K A G L K Q E H C Q D V H L P T E T M<br>601 TGCAAGGCAGGATTGAAACAAGAGCATTGCCAAGATGTTCATTTGCCTACAGAAACTATG<br><br>221 L S Q E T L P L K I H G W K S T E D L M<br>661 TTGTCCCAGGAAACACTTCCACTCAAGATTCATGGTTGGAAATCAACAGAGGACCTGATG<br><br>241 T F L D D D P E L P L L A T P P S I V S<br>721 ACATTTTTGGATGATGATCCAGAGCTGCCTTTACTAGCAACACCTCCTTCCATTGTCTCT<br><br>261 P I T C L S E A E E V C N L S G A D A V<br>781 CCAATCACTTGCTTGTCAGAAGCAGAAGAAGTTTGTAATCTTTCGGGTGCAGATGCTGTG<br><br>281 P T L S E N S P S G Q R D V S L D K R S<br>841 CCTACTCTCTCAGAAAATAGTCCTTCTGGGCAGAGAGATGTGAGTTTGGACAAGAGATCA<br><br>301 D G I F T N T V T E N L L E T P L E K Q<br>901 GATGGTATCTTTACAAATACTGTCACAGAAAACCTCTTGGAAACACCTTTAGAAAAGCAA<br><br>321 S A A E G L K T L T I L P A C W S S K E<br>961 TCAGCTGCAGAAGGTCTTAAAACATTGACAATTTTACCAGCATGTTGGTCAAGTAAAGAG<br><br>341 E F I S P D L R V S K S D S T F H N Q A<br>1021 GAGTTTATCAGCCCCGACTTAAGGGTATCAAAGTCAGATTCTACTTTCCATAACCAGGCC | |

| Gene Name | GenBank Homology | DNA SEQUENCE / DEDUCED AMINO ACID SEQUENCE | SEQUENCE IDENTIFIER: |
|---|---|---|---|
| | | 361 S V E T L C L S P S F K I F D R V K E I<br>1081 TCTGTAGAAACACTCTGTCTCTCTCCCTCATTCAAAATATTTGACCGAGTGAAGGAGATT<br><br>381 V I N K A Y Q K P A Q S S I Q M D D K I<br>1141 GTAATCAACAAGGCCTACCAGAAACCAGCACAATCCAGCATTCAGATGGATGATAAAATC<br><br>401 L K T T T R V S T A S E G A S S F S P L<br>1201 CTCAAGACAACCACACGGGTTTCAACTGCTTCTGAGGGAGCATCTTCCTTTTCTCCTTTA<br><br>421 S M S S P D L A S P E K S A H L L S P I<br>1261 TCCATGTCATCTCCTGATTTAGCCTCTCCAGAGAAGTCAGCTCATCTCTTATCACCAATT<br><br>441 L A G P S F W T L S H Q Q L S S T S F K<br>1321 CTGGCCGGACCTTCCTTCTGGACTCTGTCCCATCAACAGTTGTCTTCTACCTCCTTTAAA<br><br>461 D E D K T A K L H H P F A P G G P V S S<br>1381 GATGAAGATAAGACAGCCAAGCTGCATCACCCCTTTGCTCCTGGAGGCCCAGTGTCTTCT<br><br>481 D E E E N D N L N L L E I L P D N S D S<br>1441 GATGAAGAGGAAAATGACAACCTCAATCTACTGGAAATTCTTCCAGATAACTCTGATTCT<br><br>501 A Q K K S H I S S S N W V E F S T Q T V<br>1501 GCTCAAAAGAAAAGTCACATTTCCAGCAGTAACTGGGTAGAGTTTTCCACCCAAACTGTA<br><br>521 D P F T S M S C S G F Q T S K G L P R E<br>1561 GACCCCTTCACCTCTATGTCCTGTTCTGGTTTTCAGACAAGCAAAGGCCTGCCTAGGGAG<br><br>541 P E V K N S I K G L L G E V K N A I V K<br>1621 CCTGAAGTGAAGAATTCCATTAAGGGTCTTCTAGGGGAGGTAAAGAATGCGATAGTCAAA<br><br>561 L H E D L S M V I Q E L N V I N N I L M<br>1681 TTACACGAAGATCTGAGCATGGTGATCCAGGAGCTGAACGTCATCAATAACATCTTGATG<br><br>581 S M S L N S S Q I S Q S S Q V P Q S S E<br>1741 AGCATGAGTCTGAATAGTTCACAAATAAGCCAGTCTTCCCAGGTCCCCCAGTCTTCTGAG<br><br>601 G G S D P V -<br>1801 GGGGGCTCAGATCCAGTCTAA | |
| WBC028E11.bFSP_20 012310.abd | Homo sapiens regulator of G-protein signaling | 1 AAGAAACGCAGCTCTTGACTGCTTTTTTGTAAACATTACTGTATTATTGTGATAACTTTT<br>61 TATTCTACTATGTATATGTATGGAATAGTATTAATAAATGAACTAGGGAAGGATGTAATA | SEQ ID NO:132 |

| Gene Name | GenBank Homology | DNA SEQUENCE / DEDUCED AMINO ACID SEQUENCE | SEQUENCE IDENTIFIER: |
|---|---|---|---|
| | 13 mRNA. | 121 AATTAGACGTCTCTTCATTTTAGAGAAAGAAGATGGAAACATCATTGGTTTTCTTTTCTC<br>181 AATTAAATATGTGCGAATCAAAAGAAAAAACTTTTTTCAAGTTAATACATGGTTCAGGAA<br>241 AAGAAGAAACAAGCAAAGAAGCCAAACTCAGAGCTAAGGAAAAAAGAAACAGGTTAAGTC<br>301 TGCTCTTGCAGAAACCTGAATTTCATGAAGAGACCCACTCCGGTAGATCCGGGCACTTAG<br>361 CCAAAGAAACGAGAATCTCCCCTGCAGAAGCCGTGATATGGGGTGAATCATTTGACAAAC<br>421 TGCTCTCCCATAAAGGTGGACTGGAGACTTTTACCAGATTTCTTAAAACCGAATTCAGTG<br>481 AGGAAAACATTGAATTTTGGGTAGCCTGTGAAGATTTCAAGAAAAGCAAAGACCCTCAAC<br>541 AAATTATCCTTAAAGCAAAAGCAATATATGAGAAATTTATACAGAACGATGCTCCGCAAG<br>601 AGGTTAACCTTGATTTCCACACCAAAGAAATCATTGCCAAGAGCATCACCAAACCCACTC<br>661 TCCACAGTTTTGATGCTGCACAAAGCAGAGTATATCAGCTTATGGAACAAGACAGTTACA<br>721 CACGTTTTCTGAAATCTGACATCTATTTAGACTTGACAGAAGGAAGACCTCAGAGGCCAA<br>781 CAAATCTTAAAAGACGGTCACGTTCATTTACCTACAATGAATTCCAGGATGTAAAGTCAG<br>841 ATGTTGCCATTTGGTTATAAAGAAAATTAATTTTGCTCGTTTTTATGACAAACTTATACA<br>901 TCTGCTTCTAACATATCGCATGTTTATGTTAAGATTTGGTCCCATCCTTTAAACTGAAAT<br>961 ATGTCATGTGAAATTATTTTAAAAATGTAAAAACAAAACTTTCTGCTAACAAAATACATA<br>1021 CAGTATCTGCCAGTATATTCTGTAAAACCTTCTATTTGATGTCATTCCATTTATAATCAG<br>1081 AAAAAAAACTTATTTCTTAATCAAAAGGCAGTACAAAAAAAGTAATAATGTTTTATAAGA<br>1141 TTGTAGAGTTAAGTAAAAGTTAAGCTTTTGCAAAGTTGTCAAAAGTTCAAACAAAAGTCT<br>1201 AGTTGGGATTTTTTACCAAAGCAGCATAATATGTGTTATATAAACATAATAATACTCAGA<br>1261 TATCCAAATGTTCAGATAGCATTTTTCATAATGAATGTTCTCTTTTTTTTGGTAATAGTG<br>1321 TAGAAGTGATCTGGTTCTTACAATGGGAGATGAAGAACATTTATTATTGGGTTACTACTA<br>1381 ACCCTGTCCCAAGAATAGTAATATCACCTCTAGTTATAAGCCAGCAACAGGAACTTTTGT<br>1441 GAAGACACATTCATCTCTACAGAACTTCAGATTAAATATAATCTAGATTAATGACTGAGA<br>1501 ATAAGATCCACATTTGAACTCATTCCTAAGTGAACATGGACGTACCCAGTTATACAAAGT<br>1561 ACTTCTGTTGGTCACAGAAACATGACCAGATTTTGCATATCTCCAGGTAGGGAACTAAGT<br>1621 AGACTACCTTATCACCGGCTAAGAAAACTTGCTACTAAACTATTAGGCCATCAATGGCTT<br>1681 GAATAAAAACCAGAGAAGGTTTTTCCCAGGACGTCTCATGTTTGGCCCTTTAGAATTGGG<br>1741 GTAGAAATCAGAAATGAGATGAGGGGAAGAAGCAAGGAGTCTAAGGCCCTAGCGATTTGG<br>1801 GCATCTGCCACATTGGTTCATATTCAGAAAGTGTTATCTCATTGATTATATTCTTGTTAA<br>1861 GCAAATCTCCTTAAGTAATTATTATTCAAATAAGATTATACTCATACATCTATATGTCAC<br>1921 TGTTTTAAAGAGATATTTAATTTTTAATGTGTGTTACATGGTCTGTAAATATTTGTATTT<br>1981 AAAAATGCCATGCATTAGGCTTTGGAAATTTAATGTTAGTTGAAATGTAAAATGTGAAAA<br>2041 CTTTAGATCATTTGTAGTAATAAATATTTTTAACTTCATTCATACAGTTAAGTTTATCTG<br>2101 ACAATAAAAGCTCTGACTGAAAAAAAAAAAAAAAAAAAAA<br><br>1 M E T S L V F F S Q L N M C E S K E K T<br>1 ATGGAAACATCATTGGTTTTCTTTTCTCAATTAAATATGTGCGAATCAAAAGAAAAAACT<br><br>21 F F K L I H G S G K E E T S K E A K L R<br>61 TTTTTCAAGTTAATACATGGTTCAGGAAAAGAAGAAACAAGCAAAGAAGCCAAACTCAGA<br><br>41 A K E K R N R L S L L L Q K P E F H E E<br>121 GCTAAGGAAAAAAGAAACAGGTTAAGTCTGCTCTTGCAGAAACCTGAATTTCATGAAGAG | SEQ ID NO:133 |

| Gene Name | GenBank Homology | DNA SEQUENCE / DEDUCED AMINO ACID SEQUENCE | SEQUENCE IDENTIFIER: |
|---|---|---|---|
| | | 61 T H S G R S G H L A K E T R I S P A E A<br>181 ACCCACTCCGGTAGATCCGGGCACTTAGCCAAAGAAACGAGAATCTCCCCTGCAGAAGCC<br><br>81 V I W G E S F D K L L S H K G G L E T F<br>241 GTGATATGGGGTGAATCATTTGACAAACTGCTCTCCCATAAAGGTGGACTGGAGACTTTT<br><br>101 T R F L K T E F S E E N I E F W V A C E<br>301 ACCAGATTTCTTAAAACCGAATTCAGTGAGGAAAACATTGAATTTTGGGTAGCCTGTGAA<br><br>121 D F K K S K D P Q Q I I L K A K A I Y E<br>361 GATTTCAAGAAAAGCAAAGACCCTCAACAAATTATCCTTAAAGCAAAAGCAATATATGAG<br><br>141 K F I Q N D A P Q E V N L D F H T K E I<br>421 AAATTTATACAGAACGATGCTCCGCAAGAGGTTAACCTTGATTTCCACACCAAAGAAATC<br><br>161 I A K S I T K P T L H S F D A A Q S R V<br>481 ATTGCCAAGAGCATCACCAAACCCACTCTCCACAGTTTTGATGCTGCACAAAGCAGAGTA<br><br>181 Y Q L M E Q D S Y T R F L K S D I Y L D<br>541 TATCAGCTTATGGAACAAGACAGTTACACACGTTTTCTGAAATCTGACATCTATTTAGAC<br><br>201 L T E G R P Q R P T N L K R R S R S F T<br>601 TTGACAGAAGGAAGACCTCAGAGGCCAACAAATCTTAAAAGACGGTCACGTTCATTTACC<br><br>221 Y N E F Q D V K S D V A I W L -<br>661 TACAATGAATTCCAGGATGTAAAGTCAGATGTTGCCATTTGGTTATAA | |
| WBC028G09.bFSP_20012310.abd | Homo sapiens mRNA for KIAA1689 protein, partial cds | 1 tgcctccgcc atgttccgca gggcacgcct tagcgtgaag ccgaatgtca ggcctggtgt<br>61 aggcgccagg ggctccacag cttccaatcc ccagcgtgga cgggagtctc ccaggccgcc<br>121 ggagcctgcc acggactctg cttccaagcc cgcggagccc acagatgtgc ccacagtcga<br>181 tttcggtgga gcggagcccc aagaaaaggc tcctaggagc agtactgaaa agactggtgg<br>241 tgacaatgat gttgaagaat ccagtagatc ttcctctact gtttcacaga gaagaaagcg<br>301 aatatcaagt acttctagcc tggttaagtc tagtgtcagt gttccttcag aatctcatcc<br>361 cttatctaca attaatcaag aggctccaca gccaactgcc acttcaacaa aagagaaaca<br>421 gccatgctca gacagatacc gaatatacaa agcccagaaa ctgagggaaa tgttaaaaga<br>481 agaattgaga aaagagaaga aacaatggaa aaacaaatat gctataaatg aaagtcagag<br>541 gccaccagat cgttcaaaaa tgactatgag agacttcata tattatctac cagataataa<br>601 tccaatgact tcttcactgg aacaagaaaa gaaaactgaa aagccatcga ctccagtcca<br>661 gacaagagag caagaaggta agagtactcc taatgctgaa gataatgaaa tggaagaaga<br>721 gacagatgat gggccattac tggttcctcg agtaaaagtg gcagaagatg gttccattat<br>781 tttggatgaa gaaagtttaa ctgtagaagt tttaagaaca aaaggccctt gtgttgttga | SEQ ID NO:134 |

| Gene Name | GenBank Homology | DNA SEQUENCE / DEDUCED AMINO ACID SEQUENCE | SEQUENCE IDENTIFIER: |
|---|---|---|---|
| | | 841 agaaaatgac cccatatttg agcgcggttc tacaactaca tactccagct ttaggaaaaa<br>901 ctattactct aaaccatggt caaataaaga aacagatatg tttttttttag ccatcagcat<br>961 ggtaggaact gacttttcta tgatcggaca actttttcct cacagagcaa ggatagaaat<br>1021 taagaataaa tttaaacggg aagagaaaac aaatggatgg agaatagaca aagcattcca<br>1081 ggaaaagcgc ccttttgact tcgatttttt tgctcatttg cttcagaaag ttcttgctga<br>1141 agaagagaaa agaaaacaaa aatctgttaa aaatcacagt ttaaaggaga agaaatccac<br>1201 caaaccacgg aaaaatgtaa aagtgaaaaa agttgcctgt gaaggagtga ataatgatcc<br>1261 agatgagtct atgagttcta gaatttcaga cacggaaaga tctcagaagg atgctcagac<br>1321 agttgaagaa gagtctctga ccttatcaag ggaggatgca gagcaggttg cattagaagt<br>1381 agacctaaat caaaagaaaa gaaggaggaa gaagcaagat ggagctaatg aactgggagt<br>1441 aaacaatctt ttagaaaatg ccactgttca ggcgggtcct tctaaaggag aaaaacacaa<br>1501 gaataaatgt caggctataa ggcctgagct aaaggaaggt gaatgcagta aggagcagat<br>1561 gctttcctgc acacaaaaca tagatggcat tgtgggtttt gcctccactg aaaaagttga<br>1621 gaaaagaact gaccccatcc tttcattaag taatcaacaa gatgccacat cagtagcaac<br>1681 tgagtcttca gaatcaagca cttcagattt gccttcattc gaagttggaa ttagagcatt<br>1741 gtgtgaggtg aataatgctg agggtagttg tatagaagaa agaaatgttg acctaaaaaa<br>1801 taattcactg gaaattgatc aaacagaaaa tgttaaacca atgttgagag gtcgcttcca<br>1861 aagacctaaa cccaatttgt caagggctgg gaagaaatca gttctttcac aaggcaaaac<br>1921 agagtcagag agcaagaatt cacattcaaa aacttcagtt gaaaagaacc acgtggaaaa<br>1981 agataaaatg aatacattgg acattttgag aatggagact acagagagag agaatccaga<br>2041 agctgaaact gtatctgttt tgggtgaaaa aaattgtctg caggaaggga gtcaactaaa<br>2101 ggctttaaga cctgtacaag tgaggggccg attgcaaaag ccaaagccaa atgcaggtaa<br>2161 agctgctgaa agaaaagaaa ttctcatatc acaggaagaa attggggcca atgtagagaa<br>2221 gaatgaaaat gaatcctgtg ctgatagaga tactcctcaa cacatggaag atcaatcgtg<br>2281 taaagatttt gaagaggaag atgtcatatt acagcctgag aaaaatgatt cttttcaaaa<br>2341 tgtgcagcca gatgagccca aggttcttaa tgaatgtcta agcgttcaag agaataataa<br>2401 ggcaaataaa cttaaccaag tcccaattct aaggactcga tttcagaaac caaagccaaa<br>2461 tataggaaga ggaactggaa ggagagaaat ttcctcaaag gaagaggtac tagagaagat<br>2521 tcttgtctct ggggaaatgg cggcagcatt gagagaaact gtaagactag acacctcacc<br>2581 aaaggagatg gtaccagcag agattaatac taaagaaatg cagtcagatt taaaagaaac<br>2641 tggaagaaga gccatttctc ccagggagaa gattctagat gtgattgatg acaccataga<br>2701 aatggagaca ggtctgaaag caatgggaag agagatttgt ctaagggaga agacgccaga<br>2761 ggtgattgat gccactgagg aaatagacaa agatttggaa gaagctggaa gaagagaaat<br>2821 atccccacag aaaaatggcc cagaggaggt taagcctcta ggtgaagtgg agacagattt<br>2881 gaaagcaact ggaaatgaga gttccccaag ggagaagaca ccagaggtga ctgatgccac<br>2941 tgaggaaata gacaaaaatt tggaagaaac tggaagaaga aaaatatccc caagggaaaa<br>3001 tggcccagag gaggtcaagc ctgtagatga aatggagaca gatttgaacg caactggaag<br>3061 agagagttct ccaagggaga agacaccaga ggtgattgat gctactgagg aaatagattt<br>3121 ggaagaaact gaaagagaag tatccccaca ggaaaatgga ctagaggagg tcaagcctct<br>3181 aggtgaaatg gagacggatt tgaaagcaac tggaagagac agtttcccaa gggggaagac<br>3241 accagaggtg attgatgcca ttgaggaaat agagatagat ttggaagaaa ctgaaagaga<br>3301 aatatcccca caggaaaatg gcctagagga ggttaagcct ctaggtgaaa tgcaaacaga<br>3361 tttgaaagca accggaaggg agatttcccc aagggagaag acaccagagg tgattgatgc | |

| Gene Name | GenBank Homology | DNA SEQUENCE / DEDUCED AMINO ACID SEQUENCE | SEQUENCE IDENTIFIER: |
|---|---|---|---|
| | | 3421 cactgaggaa atagacaaag atctggaaga aactggaaga agagaaatat ccccagagga<br>3481 aaatggccca gaggaggtca agcctgtaga tgaaatggag acagacttga aaacaactgg<br>3541 aagagagggt tcctcaaggg agaagacacg agaggtgatt gatgctgctg aggtaataga<br>3601 gacagatttg gaagaaactg aaagagaaat atcgccacag gaaaatggcc cagaggaggt<br>3661 caagcctgta ggtaaaatgg agacagattt gaaagaaatt agagaagaaa tttcccaaag<br>3721 ggaaaaggtg ctagcagaga tcagtgctat aagggaaaag gagattgatt tgaaagaaac<br>3781 tggaaaaaga gacattccca tgatggagaa agtatcagga aagatggctg ttgttgaaga<br>3841 aatggaggca gatttgaaag aaactggaaa agaaaatttt agagagagag gatctgaaga<br>3901 gatctgtgtt actgaggaaa aggtggcaga attgaaacaa actggaaaaa cagacatttc<br>3961 tccaagggaa aacgagctag aggagaccag tacctcaaga caaactgaca cacatttaat<br>4021 gcagagcggt agcaatgact tcagtgctat gccttcacta gatattcaga acattagcag<br>4081 tgaagtactg tcgatgatgc atacacctgt agaagaaaaa agaaattctg aaaaagaagt<br>4141 atcaagtcac ttcagtcatt tcaagatttc ttcacagact catgaatctg ataaaacaga<br>4201 agtccagggg attcaatctc cagatgttcc agagcagttt tcagatatta atttaagcaa<br>4261 atctcttcct caagaacaga agccacttga aattaaacca gcaccttttg tgaggagccg<br>4321 attcaaaaga ccaaaaccaa acttagcaag agcagctttg aagagagaga ctacagaatc<br>4381 agaaaaatat atatatgaga agaaatcaga aaccgagaaa atggagacta ttgtgatgca<br>4441 agaaaataat gaacaaactg atactctccc ttctcaacat gatgaagctt ccctaatgat<br>4501 atcaagagaa aaagacacat taggtcacag gaatgaggag gctgtgatat tgccatgtac<br>4561 acagactgaa aggaaccttt caccttcaaa ttcttgtgaa cctaaagagg agtctcagtc<br>4621 agcaccagtc cagaaaaatg actcagttgt ttctgtgggg actaataatg taaacacttt<br>4681 ccagcaagaa atgaaggaaa gtgttatcca aactgctcga caagtaaggg gccgacttca<br>4741 gagaccgaga ccaaatataa gaaagacagg acagaggcaa atagtagaca aaggtgaagc<br>4801 caaaggcata attaaggaag gaagaacgat attaccaaaa gatgaaactg aaaagaaagt<br>4861 cttaactgtg tcaaattctc aaattgaaac tgaaattgaa gttccatcgt ccgcagttcc<br>4921 agaacacaga atgtatgaaa atcaaagtca ggtggttctt gtagaaaacc ttcacgttaa<br>4981 caaaacaaat gaaacaatca gacatgaaaa taaaccgtat gttcctagtt cagcacaaat<br>5041 gacaagaagg aaattccaaa aggctaagcc aaatttggga agagcacaca gtaagaaaga<br>5101 ggaaccagtt ttagaaaaag tcacaacaga tcagagcaag gaaggcaagc cagaagatca<br>5161 tttgctgcag aaaggagctt ccaacaccca gctccttcta aaagaaaaag ctgagcttct<br>5221 gacatctctg gaggtttcag caagaaaaga ttgtgtaggt tccaaagagt ctgctttggc<br>5281 aaaaatagat gcggaattag aagaagttgg accatcaaga agggttggag aggaaactgt<br>5341 aggagataat tcaccatctt cagttgttga agagcaatat ctcaataaac taacaagctg<br>5401 tccacaaccg ttaaacgaaa caagttactc taaaattgcc ctggatggga aaacaactat<br>5461 ctcttctaca tctgagtatg agagaaatcg tggtgaaagg agaagtcata aaaagttcaa<br>5521 accaaatgtc accagaggtc gtggatcaaa acgagttcgg ggtaagacct ctaagaagga<br>5581 acctagagct tccaaggcca tgctggtgac tcttcgggct tcccaggaag aagatgatga<br>5641 tgctgacgat tttgagtctg actatgagga agaaagctat catcttgctc ccgaagaagt<br>5701 aaacaaagct ccagtatttg tacctgttgg tctcagatct cctgaacctg tttctgctca<br>5761 gattgaggaa acaatggaag agcttgaaat aactgtgaat gtcccagatg taggatgcat<br>5821 agctgttgtt gaacatgagc taccaaacac agatgtgact actgaagaaa tgaaacaaga<br>5881 agaaaatttg agtgtaccgt ttgaaatgac cacaagtgaa catatccaag atgaaccagg<br>5941 taccaatgat ggaagcaccg aagctgcaat aactttactt acaatgggag atctagtatt | |

| Gene Name | GenBank Homology | DNA SEQUENCE / DEDUCED AMINO ACID SEQUENCE | SEQUENCE IDENTIFIER: |
|---|---|---|---|
| | | 6001 gcagtcagag atcagtagtg aacagggtga tgtaggagta tgtataattc ctcatgttca | |
| | | 6061 ttcaaaggat aaaagccata ttccttctag cctagataat gtaaatcaca aaattgttca | |
| | | 6121 tgaatgtcag gaactttctt cacctgtcat tactacatct cctgcatcat ttgaagaaaa | |
| | | 6181 caagattgta ttggaggaac aaagttccag agaagaaata agtttaatgg agaaagtaaa | |
| | | 6241 ggagaatgct acaccaacca ggaatacaat ttctaaagtg accagtaatt tgagaataag | |
| | | 6301 aagtaggctt gctaagccta aaccaaatct tgagaagact ttagggacca acaggcttga | |
| | | 6361 tgattatcag gaagtttcca gtttgtgtgt aaccaaaggg gcagaaatgg aaactcaaag | |
| | | 6421 agaaacagag aaaaatgctt ccaaagcaac agaattggaa aataaaaacc tcggaccagt | |
| | | 6481 tacaacagca gagaataagg atcagagcaa attggcatgt gtacatggta tcaaagggac | |
| | | 6541 cagtatttct tcagaagtaa acctaactga aagaaacgaa aatcaagaag agagctctca | |
| | | 6601 ggaggttcac atgttgtcag ttgctccagt tgcttcctct gagacagggc cctgcacact | |
| | | 6661 tggtttggat aggggtcttg gtgaaaattc tgttgaagag ccccagataa aggactctaa | |
| | | 6721 aggagacagt gtgcttacac ttcctgtgcc agagtataca ccaacaagta ttccagaagt | |
| | | 6781 ccaacaagag aatataatca atcctcaaga cctaacagtg aatctagttg ctaatgtacc | |
| | | 6841 tcaagatgga gaagatgaac aagcctttat tttaactctg gtggaaatcc cagccaatgc | |
| | | 6901 agtagaagaa tttactgatg ccactgcaca gttcatgcca aaccctttac tgccagctcc | |
| | | 6961 catattggtc aaatcagtga ataccgaaga aaggggtgac atgagtattt gtttaccagc | |
| | | 7021 aacttcagtt ggtcaagatg ccatgggttt atctatttct ggaagagata attctaaaaa | |
| | | 7081 gccgcctgat aatttggatc ttgtatctag gaagagattt caatgcaggc ttgataaaaa | |
| | | 7141 tgaccacatt cctcctgcca aaaaacgttc actcacttta agagatgact gtcaagaata | |
| | | 7201 taccactgag gtgcactcaa aggaattaac aaatgttttt gaggaaacag gggagtctca | |
| | | 7261 caagggacaa gatatttttc ttacctcagg aagcacactg acaactccag aacctcaaag | |
| | | 7321 acagcaagtt gaagcagctt ttcagagtag aggatctaga tctcctgatg catgcatgga | |
| | | 7381 caagaatgtg cctcagttac ctcaggatga aatgattgtg tctgataagg aagaaagaac | |
| | | 7441 tgatgctgct cctaagtctc agcaaatgga tagcagaaca tcgtcttcta aagcctcact | |
| | | 7501 atccagacct ggcagaagac ccctgggatt tttatcttta atatgctcaa agaatagttt | |
| | | 7561 ggagtctgat gaacctatgc aagtccatag taagaaacgc ctaaaacctc ttatacctgg | |
| | | 7621 attaagaaag aaattgaaaa gatctaatcc attcaatgaa agccaggaaa aaaatcgaga | |
| | | 7681 gtcctctgat ctgcttccat ctccaagtgt tattactact caatctgaga atattagcag | |
| | | 7741 ctcagcaact caggtttctt gtgatcagcc cttactgaaa gaaggatata aagtgcccca | |
| | | 7801 aaagcgggcc cctcaagggg aggcaaccac agtctctgaa tatttcttca atgatatctt | |
| | | 7861 cattgaagtg gatgaaacag aataaaacaa tcttttctct ttttcttttt taaattaggt | |
| | | 7921 ctaggatttc cagagtcaat tacatcaaca aaacagtatt tagagcaaaa tatcactgtc | |
| | | 7981 ttatttttct ttaggttgat tttgaatact taatgagctt gatttgaagc ttttataatc | |
| | | 8041 agtggaaaac atttctgagg ttcctttcat tctgactgat tcagcatttt gcaaatagca | |
| | | 8101 agcaattaag actgctttct cagacagaaa taacaactct tgtttacatt ttgactcttc | |
| | | 8161 ctgtgctaag cacacatgga catttgggaa tgttgtggat atatgtctct gtatgaattg | |
| | | 8221 cagtgcagac agatttgggg gttaattgta tcatatttaa catttagcaa cttcttttgt | |
| | | 8281 gaagattttt tatttttagg gggagatgat gaaggatgaa ggctttttca tttgcttcta | |
| | | 8341 agtacagtca tgtatcacat aatgaagttt aagtcaatga tggaccacat atattacagt | |
| | | 8401 ggtcccatgc aattaaaatg gagataaatt cctatcaact agtgacatta tagccatcat | |
| | | 8461 aacacagtgc attgcttttt tatgtttaga tatgtttaga tacacaaata cttactattg | |
| | | 8521 tgttacaact gcctacagta ttcagcatag taacacattg tagaggtttg tagccgagga | |

| Gene Name | GenBank Homology | DNA SEQUENCE / DEDUCED AMINO ACID SEQUENCE | SEQUENCE IDENTIFIER: |
|---|---|---|---|
| | | 8581 gcaataggct ataccacata gcctcagtct gtacggtgtg tagcagcctg taccatctca<br>8641 gtgtttgtaa gtacactctg cgttgtttca cacaagatca cctaacaagg caattcttag<br>8701 aacatgtagt taagggacac gtgactatat gtgaaaacaa attgtcaaac ttcactttag<br>8761 tatggcaaat gttaaagaac ttttctatta tcagctgttt gtctgactga aaaatacata<br>8821 tttttctaat tcatggtgat gtaacattag tcctaattga aaaactagta tttaaacata<br>8881 attatttata aagatgacac atcaaagggc ttatttattt ttaaattttt tatttaaaag<br>8941 gatattcagt tttagctatt tttacccctc agattgtaga taaagaaggc attaaattta<br>9001 tgtttgtctc ctttttctgt ttaattttaa agatatttaa aatgatataa ctaaaaatgt<br>9061 ttagctggtg tatatgtttt gaataccttt ttcccctcag tttagtatat tgactttgta<br>9121 ctgtaaattt ttctgctttt cttggataat ctcaggattt tcatgaggat agggggaact<br>9181 caactttatt tatgagacaa aatttccata caaagctcaa tctcctttat acacccacag<br>9241 acataatatt agtttttaaa aagccaattt cttcatagtt ttttcccatt aaattctcaa<br>9301 ggaacacttg gatctttaag cacggaacat aatcatgatg ttaaaaacag agaaaataag<br>9361 gctttataaa gttaatgatt atcatcagta tgaatttaag gtttgtttta atttcaagat<br>9421 ttgatttttt atacgtgtaa ttctattatc tacccaagca gatctgtagt ggttccaatt<br>9481 agacttctca aacagcaaat ttatcctgat tttatttgaa aagcctcttg gattgatagt<br>9541 atagtagctc aggcattgga aactttctgc aaactgtttt gggtttgcag gccagatggt<br>9601 ctctgtggca gctactcagc tctgcaattt cagtgtgaaa gaagccatag acagtacttg<br>9661 aatgaaggac tgtggctgga ttggcctttt agtttgaccc cctacattag gccccaaatt<br>9721 ttcttaccct gaggtgctga tatctgtatg gatgagttat ttgtcactaa agttatgagt<br>9781 tgtgcctaaa agttaaaact gttgactgta ttatgtaatg atcagtattt cagttgggaa<br>9841 gatattttag agtctagata attatgtttg tatattgaaa aaatggtggc cagtttttaa<br>9901 gttccttaat agaagagaat tatgtctcag cacatataac agtaatgcta atttattgaa<br>9961 actactgctg ttagagcact tcttattcat tgtcttttag tgaaatttat ggcgtaacac<br>10021 tttgtcagag aggaggctat ataattcgga gcggaaattg tctataagta ggcatttatt<br>10081 tcatgattga tatgtcacag aaatcatggt agtaaatcac attgctattt gaataccctg<br>10141 tttttgtaag tttttaaaac tcatattctg aaaagatttc attctcttag tgttagcttg<br>10201 ggagttagat tgccatgatt aaactattat ttatccttgt gtaatattag tttttaactt<br>10261 taacatctgt ttctttttaa tctataatga gctagtttta tggaaaatgg aatttcttac<br>10321 tatataaaga atacagagac tcattgtatt agagaatcaa gtcagccagc taaagtatcc<br>10381 tactgttaaa tccttaaacc taattttgga aaagagaaag ttaatcaatg tatttacctt<br>10441 acatgttgga aagaactatg ttaggtctga ttcatgtgaa gaagatgttg caaaggattt<br>10501 atttcacaaa ttttaaagga gatatgagta aaagttttta tctttttcttg actttttctc<br>10561 ctgaacactt atgtcttagc aagtggtcaa catgaggatt tgaacgccta attgttggta<br>10621 aatggttgag gcatgacaaa aatattaata tccactgttt accatcatgt tatttgaaac<br>10681 aaaagtgacc atgtatacta tcttgcttga agaagtcttt gacagaaaaa gcaatatcat<br>10741 gtcatttata aattttcttg ttctaaagaa agcagttata tatatatata aattatgtaa<br>10801 ataaaagtta ttttatatc | |
| | | 1 A S A M F R R A R L S V K P N V R P G V<br>2 GCCTCCGCCATGTTCCGCAGGGCACGCCTTAGCGTGAAGCCGAATGTCAGGCCTGGTGTA<br><br>21 G A R G S T A S N P Q R G R E S P R P P | SEQ ID NO:135 |

| Gene Name | GenBank Homology | DNA SEQUENCE / DEDUCED AMINO ACID SEQUENCE | SEQUENCE IDENTIFIER: |
|---|---|---|---|
| | | 62 GGCGCCAGGGGCTCCACAGCTTCCAATCCCCAGCGTGGACGGGAGTCTCCCAGGCCGCCG<br><br>41 E P A T D S A S K P A E P T D V P T V D<br>122 GAGCCTGCCACGGACTCTGCTTCCAAGCCCGCGGAGCCCACAGATGTGCCCACAGTCGAT<br><br>61 F G G A E P Q E K A P R S S T E K T G G<br>182 TTCGGTGGAGCGGAGCCCCAAGAAAAGGCTCCTAGGAGCAGTACTGAAAAGACTGGTGGT<br><br>81 D N D V E E S S R S S S T V S Q R R K R<br>242 GACAATGATGTTGAAGAATCCAGTAGATCTTCCTCTACTGTTTCACAGAGAAGAAAGCGA<br><br>101 I S S T S S L V K S S V S V P S E S H P<br>302 ATATCAAGTACTTCTAGCCTGGTTAAGTCTAGTGTCAGTGTTCCTTCAGAATCTCATCCC<br><br>121 L S T I N Q E A P Q P T A T S T K E K Q<br>362 TTATCTACAATTAATCAAGAGGCTCCACAGCCAACTGCCACTTCAACAAAAGAGAAACAG<br><br>141 P C S D R Y R I Y K A Q K L R E M L K E<br>422 CCATGCTCAGACAGATACCGAATATACAAAGCCCAGAAACTGAGGGAAATGTTAAAAGAA<br><br>161 E L R K E K K Q W K N K Y A I N E S Q R<br>482 GAATTGAGAAAAGAGAAGAAACAATGGAAAAACAAATATGCTATAAATGAAAGTCAGAGG<br><br>181 P P D R S K M T M R D F I Y Y L P D N N<br>542 CCACCAGATCGTTCAAAAATGACTATGAGAGACTTCATATATTATCTACCAGATAATAAT<br><br>201 P M T S S L E Q E K K T E K P S T P V Q<br>602 CCAATGACTTCTTCACTGGAACAAGAAAAGAAAACTGAAAAGCCATCGACTCCAGTCCAG<br><br>221 T R E Q E G K S T P N A E D N E M E E E<br>662 ACAAGAGAGCAAGAAGGTAAGAGTACTCCTAATGCTGAAGATAATGAAATGGAAGAAGAG<br><br>241 T D D G P L L V P R V K V A E D G S I I<br>722 ACAGATGATGGGCCATTACTGGTTCCTCGAGTAAAAGTGGCAGAAGATGGTTCCATTATT<br><br>261 L D E E S L T V E V L R T K G P C V V E<br>782 TTGGATGAAGAAAGTTTAACTGTAGAAGTTTTAAGAACAAAAGGCCCTTGTGTTGTTGAA<br><br>281 E N D P I F E R G S T T T Y S S F R K N<br>842 GAAAATGACCCCATATTTGAGCGCGGTTCTACAACTACATACTCCAGCTTTAGGAAAAAC<br><br>301 Y Y S K P W S N K E T D M F F L A I S M<br>902 TATTACTCTAAACCATGGTCAAATAAAGAAACAGATATGTTTTTTTTAGCCATCAGCATG | |

| Gene Name | GenBank Homology | DNA SEQUENCE / DEDUCED AMINO ACID SEQUENCE | SEQUENCE IDENTIFIER: |
|---|---|---|---|
| | | 321 V G T D F S M I G Q L F P H R A R I E I<br>962 GTAGGAACTGACTTTTCTATGATCGGACAACTTTTTCCTCACAGAGCAAGGATAGAAATT<br><br>341 K N K F K R E E K T N G W R I D K A F Q<br>1022 AAGAATAAATTTAAACGGGAAGAGAAAACAAATGGATGGAGAATAGACAAAGCATTCCAG<br><br>361 E K R P F D F D F F A H L L Q K V L A E<br>1082 GAAAAGCGCCCTTTTGACTTCGATTTTTTTGCTCATTTGCTTCAGAAAGTTCTTGCTGAA<br><br>381 E E K R K Q K S V K N H S L K E K K S T<br>1142 GAAGAGAAAAGAAAACAAAAATCTGTTAAAAATCACAGTTTAAAGGAGAAGAAATCCACC<br><br>401 K P R K N V K V K K V A C E G V N N D P<br>1202 AAACCACGGAAAAATGTAAAAGTGAAAAAAGTTGCCTGTGAAGGAGTGAATAATGATCCA<br><br>421 D E S M S S R I S D T E R S Q K D A Q T<br>1262 GATGAGTCTATGAGTTCTAGAATTTCAGACACGGAAAGATCTCAGAAGGATGCTCAGACA<br><br>441 V E E E S L T L S R E D A E Q V A L E V<br>1322 GTTGAAGAAGAGTCTCTGACCTTATCAAGGGAGGATGCAGAGCAGGTTGCATTAGAAGTA<br><br>461 D L N Q K K R R R K K Q D G A N E L G V<br>1382 GACCTAAATCAAAAGAAAAGAAGGAGGAAGAAGCAAGATGGAGCTAATGAACTGGGAGTA<br><br>481 N N L L E N A T V Q A G P S K G E K H K<br>1442 AACAATCTTTTAGAAAATGCCACTGTTCAGGCGGGTCCTTCTAAAGGAGAAAAACACAAG<br><br>501 N K C Q A I R P E L K E G E C S K E Q M<br>1502 AATAAATGTCAGGCTATAAGGCCTGAGCTAAAGGAAGGTGAATGCAGTAAGGAGCAGATG<br><br>521 L S C T Q N I D G I V G F A S T E K V E<br>1562 CTTTCCTGCACACAAAACATAGATGGCATTGTGGGTTTTGCCTCCACTGAAAAAGTTGAG<br><br>541 K R T D P I L S L S N Q Q D A T S V A T<br>1622 AAAAGAACTGACCCCATCCTTTCATTAAGTAATCAACAAGATGCCACATCAGTAGCAACT<br><br>561 E S S E S S T S D L P S F E V G I R A L<br>1682 GAGTCTTCAGAATCAAGCACTTCAGATTTGCCTTCATTCGAAGTTGGAATTAGAGCATTG<br><br>581 C E V N N A E G S C I E E R N V D L K N<br>1742 TGTGAGGTGAATAATGCTGAGGGTAGTTGTATAGAAGAAAGAAATGTTGACCTAAAAAAT | |

| Gene Name | GenBank Homology | DNA SEQUENCE / DEDUCED AMINO ACID SEQUENCE | SEQUENCE IDENTIFIER: |
|---|---|---|---|
| | | 601 N S L E I D Q T E N V K P M L R G R F Q<br>1802 AATTCACTGGAAATTGATCAAACAGAAAATGTTAAACCAATGTTGAGAGGTCGCTTCCAA<br><br>621 R P K P N L S R A G K K S V L S Q G K T<br>1862 AGACCTAAACCCAATTTGTCAAGGGCTGGGAAGAAATCAGTTCTTTCACAAGGCAAAACA<br><br>641 E S E S K N S H S K T S V E K N H V E K<br>1922 GAGTCAGAGAGCAAGAATTCACATTCAAAAACTTCAGTTGAAAAGAACCACGTGGAAAAA<br><br>661 D K M N T L D I L R M E T T E R E N P E<br>1982 GATAAAATGAATACATTGGACATTTTGAGAATGGAGACTACAGAGAGAGAGAATCCAGAA<br><br>681 A E T V S V L G E K N C L Q E G S Q L K<br>2042 GCTGAAACTGTATCTGTTTTGGGTGAAAAAAATTGTCTGCAGGAAGGGAGTCAACTAAAG<br><br>701 A L R P V Q V R G R L Q K P K P N A G K<br>2102 GCTTTAAGACCTGTACAAGTGAGGGGCCGATTGCAAAAGCCAAAGCCAAATGCAGGTAAA<br><br>721 A A E R K E I L I S Q E E I G A N V E K<br>2162 GCTGCTGAAAGAAAAGAAATTCTCATATCACAGGAAGAAATTGGGGCCAATGTAGAGAAG<br><br>741 N E N E S C A D R D T P Q H M E D Q S C<br>2222 AATGAAAATGAATCCTGTGCTGATAGAGATACTCCTCAACACATGGAAGATCAATCGTGT<br><br>761 K D F E E E D V I L Q P E K N D S F Q N<br>2282 AAAGATTTTGAAGAGGAAGATGTCATATTACAGCCTGAGAAAAATGATTCTTTTCAAAAT<br><br>781 V Q P D E P K V L N E C L S V Q E N N K<br>2342 GTGCAGCCAGATGAGCCCAAGGTTCTTAATGAATGTCTAAGCGTTCAAGAGAATAATAAG<br><br>801 A N K L N Q V P I L R T R F Q K P K P N<br>2402 GCAAATAAACTTAACCAAGTCCCAATTCTAAGGACTCGATTTCAGAAACCAAAGCCAAAT<br><br>821 I G R G T G R R E I S S K E E V L E K I<br>2462 ATAGGAAGAGGAACTGGAAGGAGAGAAATTTCCTCAAAGGAAGAGGTACTAGAGAAGATT<br><br>841 L V S G E M A A A L R E T V R L D T S P<br>2522 CTTGTCTCTGGGGAAATGGCGGCAGCATTGAGAGAAACTGTAAGACTAGACACCTCACCA<br><br>861 K E M V P A E I N T K E M Q S D L K E T<br>2582 AAGGAGATGGTACCAGCAGAGATTAATACTAAAGAAATGCAGTCAGATTTAAAAGAAACT<br><br>881 G R R A I S P R E K I L D V I D D T I E | |

| Gene Name | GenBank Homology | DNA SEQUENCE / DEDUCED AMINO ACID SEQUENCE | SEQUENCE IDENTIFIER: |
|---|---|---|---|
| | | 2642 GGAAGAAGAGCCATTTCTCCCAGGGAGAAGATTCTAGATGTGATTGATGACACCATAGAA<br><br>901 M E T G L K A M G R E I C L R E K T P E<br>2702 ATGGAGACAGGTCTGAAAGCAATGGGAAGAGAGATTTGTCTAAGGGAGAAGACGCCAGAG<br><br>921 V I D A T E E I D K D L E E A G R R E I<br>2762 GTGATTGATGCCACTGAGGAAATAGACAAAGATTTGGAAGAAGCTGGAAGAAGAGAAATA<br><br>941 S P Q K N G P E E V K P L G E V E T D L<br>2822 TCCCCACAGAAAAATGGCCCAGAGGAGGTTAAGCCTCTAGGTGAAGTGGAGACAGATTTG<br><br>961 K A T G N E S S P R E K T P E V T D A T<br>2882 AAAGCAACTGGAAATGAGAGTTCCCCAAGGGAGAAGACACCAGAGGTGACTGATGCCACT<br><br>981 E E I D K N L E E T G R R K I S P R E N<br>2942 GAGGAAATAGACAAAAATTTGGAAGAAACTGGAAGAAGAAAAATATCCCCAAGGGAAAAT<br><br>1001 G P E E V K P V D E M E T D L N A T G R<br>3002 GGCCCAGAGGAGGTCAAGCCTGTAGATGAAATGGAGACAGATTTGAACGCAACTGGAAGA<br><br>1021 E S S P R E K T P E V I D A T E E I D L<br>3062 GAGAGTTCTCCAAGGGAGAAGACACCAGAGGTGATTGATGCTACTGAGGAAATAGATTTG<br><br>1041 E E T E R E V S P Q E N G L E E V K P L<br>3122 GAAGAAACTGAAAGAGAAGTATCCCCACAGGAAAATGGACTAGAGGAGGTCAAGCCTCTA<br><br>1061 G E M E T D L K A T G R D S F P R G K T<br>3182 GGTGAAATGGAGACGGATTTGAAAGCAACTGGAAGAGACAGTTTCCCAAGGGGGAAGACA<br><br>1081 P E V I D A I E E I E I D L E E T E R E<br>3242 CCAGAGGTGATTGATGCCATTGAGGAAATAGAGATAGATTTGGAAGAAACTGAAAGAGAA<br><br>1101 I S P Q E N G L E E V K P L G E M Q T D<br>3302 ATATCCCCACAGGAAAATGGCCTAGAGGAGGTTAAGCCTCTAGGTGAAATGCAAACAGAT<br><br>1121 L K A T G R E I S P R E K T P E V I D A<br>3362 TTGAAAGCAACCGGAAGGGAGATTTCCCCAAGGGAGAAGACACCAGAGGTGATTGATGCC<br><br>1141 T E E I D K D L E E T G R R E I S P E E<br>3422 ACTGAGGAAATAGACAAAGATCTGGAAGAAACTGGAAGAAGAGAAATATCCCCAGAGGAA<br><br>1161 N G P E E V K P V D E M E T D L K T T G<br>3482 AATGGCCCAGAGGAGGTCAAGCCTGTAGATGAAATGGAGACAGACTTGAAAACAACTGGA | |

| Gene Name | GenBank Homology | DNA SEQUENCE / DEDUCED AMINO ACID SEQUENCE | SEQUENCE IDENTIFIER: |
|---|---|---|---|
| | | 1181 R E G S S R E K T R E V I D A A E V I E<br>3542 AGAGAGGGTTCCTCAAGGGAGAAGACACGAGAGGTGATTGATGCTGCTGAGGTAATAGAG<br><br>1201 T D L E E T E R E I S P Q E N G P E E V<br>3602 ACAGATTTGGAAGAAACTGAAAGAGAAATATCGCCACAGGAAAATGGCCCAGAGGAGGTC<br><br>1221 K P V G K M E T D L K E I R E E I S Q R<br>3662 AAGCCTGTAGGTAAAATGGAGACAGATTTGAAAGAAATTAGAGAAGAAATTTCCCAAAGG<br><br>1241 E K V L A E I S A I R E K E I D L K E T<br>3722 GAAAAGGTGCTAGCAGAGATCAGTGCTATAAGGGAAAAGGAGATTGATTTGAAAGAAACT<br><br>1261 G K R D I P M M E K V S G K M A V V E E<br>3782 GGAAAAAGAGACATTCCCATGATGGAGAAAGTATCAGGAAAGATGGCTGTTGTTGAAGAA<br><br>1281 M E A D L K E T G K E N F R E R G S E E<br>3842 ATGGAGGCAGATTTGAAAGAAACTGGAAAAGAAAATTTTAGAGAGAGAGGATCTGAAGAG<br><br>1301 I C V T E E K V A E L K Q T G K T D I S<br>3902 ATCTGTGTTACTGAGGAAAAGGTGGCAGAATTGAAACAAACTGGAAAAACAGACATTTCT<br><br>1321 P R E N E L E E T S T S R Q T D T H L M<br>3962 CCAAGGGAAAACGAGCTAGAGGAGACCAGTACCTCAAGACAAACTGACACACATTTAATG<br><br>1341 Q S G S N D F S A M P S L D I Q N I S S<br>4022 CAGAGCGGTAGCAATGACTTCAGTGCTATGCCTTCACTAGATATTCAGAACATTAGCAGT<br><br>1361 E V L S M M H T P V E E K R N S E K E V<br>4082 GAAGTACTGTCGATGATGCATACACCTGTAGAAGAAAAAAGAAATTCTGAAAAAGAAGTA<br><br>1381 S S H F S H F K I S S Q T H E S D K T E<br>4142 TCAAGTCACTTCAGTCATTTCAAGATTTCTTCACAGACTCATGAATCTGATAAAACAGAA<br><br>1401 V Q G I Q S P D V P E Q F S D I N L S K<br>4202 GTCCAGGGGATTCAATCTCCAGATGTTCCAGAGCAGTTTTCAGATATTAATTTAAGCAAA<br><br>1421 S L P Q E Q K P L E I K P A P F V R S R<br>4262 TCTCTTCCTCAAGAACAGAAGCCACTTGAAATTAAACCAGCACCTTTTGTGAGGAGCCGA<br><br>1441 F K R P K P N L A R A A L K R E T T E S<br>4322 TTCAAAAGACCAAAACCAAACTTAGCAAGAGCAGCTTTGAAGAGAGAGACTACAGAATCA | |

| Gene Name | GenBank Homology | DNA SEQUENCE / DEDUCED AMINO ACID SEQUENCE | SEQUENCE IDENTIFIER: |
|---|---|---|---|
| | | 1461 E K Y I Y E K K S E T E K M E T I V M Q<br>4382 GAAAAATATATATATGAGAAGAAATCAGAAACCGAGAAAATGGAGACTATTGTGATGCAA<br><br>1481 E N N E Q T D T L P S Q H D E A S L M I<br>4442 GAAAATAATGAACAAACTGATACTCTCCCTTCTCAACATGATGAAGCTTCCCTAATGATA<br><br>1501 S R E K D T L G H R N E E A V I L P C T<br>4502 TCAAGAGAAAAAGACACATTAGGTCACAGGAATGAGGAGGCTGTGATATTGCCATGTACA<br><br>1521 Q T E R N L S P S N S C E P K E E S Q S<br>4562 CAGACTGAAAGGAACCTTTCACCTTCAAATTCTTGTGAACCTAAAGAGGAGTCTCAGTCA<br><br>1541 A P V Q K N D S V V S V G T N N V N T F<br>4622 GCACCAGTCCAGAAAAATGACTCAGTTGTTTCTGTGGGGACTAATAATGTAAACACTTTC<br><br>1561 Q Q E M K E S V I Q T A R Q V R G R L Q<br>4682 CAGCAAGAAATGAAGGAAAGTGTTATCCAAACTGCTCGACAAGTAAGGGGCCGACTTCAG<br><br>1581 R P R P N I R K T G Q R Q I V D K G E A<br>4742 AGACCGAGACCAAATATAAGAAAGACAGGACAGAGGCAAATAGTAGACAAAGGTGAAGCC<br><br>1601 K G I I K E G R T I L P K D E T E K K V<br>4802 AAAGGCATAATTAAGGAAGGAAGAACGATATTACCAAAAGATGAAACTGAAAAGAAAGTC<br><br>1621 L T V S N S Q I E T E I E V P S S A V P<br>4862 TTAACTGTGTCAAATTCTCAAATTGAAACTGAAATTGAAGTTCCATCGTCCGCAGTTCCA<br><br>1641 E H R M Y E N Q S Q V V L V E N L H V N<br>4922 GAACACAGAATGTATGAAAATCAAAGTCAGGTGGTTCTTGTAGAAAACCTTCACGTTAAC<br><br>1661 K T N E T I R H E N K P Y V P S S A Q M<br>4982 AAAACAAATGAAACAATCAGACATGAAAATAAACCGTATGTTCCTAGTTCAGCACAAATG<br><br>1681 T R R K F Q K A K P N L G R A H S K K E<br>5042 ACAAGAAGGAAATTCCAAAAGGCTAAGCCAAATTTGGGAAGAGCACACAGTAAGAAAGAG<br><br>1701 E P V L E K V T T D Q S K E G K P E D H<br>5102 GAACCAGTTTTAGAAAAAGTCACAACAGATCAGAGCAAGGAAGGCAAGCCAGAAGATCAT<br><br>1721 L L Q K G A S N T Q L L L K E K A E L L<br>5162 TTGCTGCAGAAAGGAGCTTCCAACACCCAGCTCCTTCTAAAAGAAAAAGCTGAGCTTCTG<br><br>1741 T S L E V S A R K D C V G S K E S A L A | |

| Gene Name | GenBank Homology | DNA SEQUENCE / DEDUCED AMINO ACID SEQUENCE | SEQUENCE IDENTIFIER: |
|---|---|---|---|
| | | 5222 ACATCTCTGGAGGTTTCAGCAAGAAAAGATTGTGTAGGTTCCAAAGAGTCTGCTTTGGCA<br><br>1761 K I D A E L E E V G P S R R V G E E T V<br>5282 AAAATAGATGCGGAATTAGAAGAAGTTGGACCATCAAGAAGGGTTGGAGAGGAAACTGTA<br><br>1781 G D N S P S S V V E E Q Y L N K L T S C<br>5342 GGAGATAATTCACCATCTTCAGTTGTTGAAGAGCAATATCTCAATAAACTAACAAGCTGT<br><br>1801 P Q P L N E T S Y S K I A L D G K T T I<br>5402 CCACAACCGTTAAACGAAACAAGTTACTCTAAAATTGCCCTGGATGGAAAACAACTATC<br><br>1821 S S T S E Y E R N R G E R R S H K K F K<br>5462 TCTTCTACATCTGAGTATGAGAGAAATCGTGGTGAAAGGAGAAGTCATAAAAAGTTCAAA<br><br>1841 P N V T R G R G S K R V R G K T S K K E<br>5522 CCAAATGTCACCAGAGGTCGTGGATCAAAACGAGTTCGGGGTAAGACCTCTAAGAAGGAA<br><br>1861 P R A S K A M L V T L R A S Q E E D D D<br>5582 CCTAGAGCTTCCAAGGCCATGCTGGTGACTCTTCGGGCTTCCCAGGAAGAAGATGATGAT<br><br>1881 A D D F E S D Y E E E S Y H L A P E E V<br>5642 GCTGACGATTTTGAGTCTGACTATGAGGAAGAAAGCTATCATCTTGCTCCCGAAGAAGTA<br><br>1901 N K A P V F V P V G L R S P E P V S A Q<br>5702 AACAAAGCTCCAGTATTTGTACCTGTTGGTCTCAGATCTCCTGAACCTGTTTCTGCTCAG<br><br>1921 I E E T M E E L E I T V N V P D V G C I<br>5762 ATTGAGGAAACAATGGAAGAGCTTGAAATAACTGTGAATGTCCCAGATGTAGGATGCATA<br><br>1941 A V V E H E L P N T D V T T E E M K Q E<br>5822 GCTGTTGTTGAACATGAGCTACCAAACACAGATGTGACTACTGAAGAAATGAAACAAGAA<br><br>1961 E N L S V P F E M T T S E H I Q D E P G<br>5882 GAAAATTTGAGTGTACCGTTTGAAATGACCACAAGTGAACATATCCAAGATGAACCAGGT<br><br>1981 T N D G S T E A A I T L L T M G D L V L<br>5942 ACCAATGATGGAAGCACCGAAGCTGCAATAACTTTACTTACAATGGGAGATCTAGTATTG<br><br>2001 Q S E I S S E Q G D V G V C I I P H V H<br>6002 CAGTCAGAGATCAGTAGTGAACAGGGTGATGTAGGAGTATGTATAATTCCTCATGTTCAT<br><br>2021 S K D K S H I P S S L D N V N H K I V H<br>6062 TCAAAGGATAAAAGCCATATTCCTTCTAGCCTAGATAATGTAAATCACAAAATTGTTCAT | |

| Gene Name | GenBank Homology | DNA SEQUENCE / DEDUCED AMINO ACID SEQUENCE | SEQUENCE IDENTIFIER: |
|---|---|---|---|
| | | 2041 E C Q E L S S P V I T T S P A S F E E N<br>6122 GAATGTCAGGAACTTTCTTCACCTGTCATTACTACATCTCCTGCATCATTTGAAGAAAAC<br><br>2061 K I V L E E Q S S R E E I S L M E K V K<br>6182 AAGATTGTATTGGAGGAACAAAGTTCCAGAGAAGAAATAAGTTTAATGGAGAAAGTAAAG<br><br>2081 E N A T P T R N T I S K V T S N L R I R<br>6242 GAGAATGCTACACCAACCAGGAATACAATTTCTAAAGTGACCAGTAATTTGAGAATAAGA<br><br>2101 S R L A K P K P N L E K T L G T N R L D<br>6302 AGTAGGCTTGCTAAGCCTAAACCAAATCTTGAGAAGACTTTAGGGACCAACAGGCTTGAT<br><br>2121 D Y Q E V S S L C V T K G A E M E T Q R<br>6362 GATTATCAGGAAGTTTCCAGTTTGTGTGTAACCAAAGGGGCAGAAATGGAAACTCAAAGA<br><br>2141 E T E K N A S K A T E L E N K N L G P V<br>6422 GAAACAGAGAAAAATGCTTCCAAAGCAACAGAATTGGAAAATAAAAACCTCGGACCAGTT<br><br>2161 T T A E N K D Q S K L A C V H G I K G T<br>6482 ACAACAGCAGAGAATAAGGATCAGAGCAAATTGGCATGTGTACATGGTATCAAAGGGACC<br><br>2181 S I S S E V N L T E R N E N Q E E S S Q<br>6542 AGTATTTCTTCAGAAGTAAACCTAACTGAAAGAAACGAAAATCAAGAAGAGAGCTCTCAG<br><br>2201 E V H M L S V A P V A S S E T G P C T L<br>6602 GAGGTTCACATGTTGTCAGTTGCTCCAGTTGCTTCCTCTGAGACAGGGCCCTGCACACTT<br><br>2221 G L D R G L G E N S V E E P Q I K D S K<br>6662 GGTTTGGATAGGGGTCTTGGTGAAAATTCTGTTGAAGAGCCCCAGATAAAGGACTCTAAA<br><br>2241 G D S V L T L P V P E Y T P T S I P E V<br>6722 GGAGACAGTGTGCTTACACTTCCTGTGCCAGAGTATACACCAACAAGTATTCCAGAAGTC<br><br>2261 Q Q E N I I N P Q D L T V N L V A N V P<br>6782 CAACAAGAGAATATAATCAATCCTCAAGACCTAACAGTGAATCTAGTTGCTAATGTACCT<br><br>2281 Q D G E D E Q A F I L T L V E I P A N A<br>6842 CAAGATGGAGAAGATGAACAAGCCTTTATTTTAACTCTGGTGGAAATCCCAGCCAATGCA<br><br>2301 V E E F T D A T A Q F M P N P L L P A P<br>6902 GTAGAAGAATTTACTGATGCCACTGCACAGTTCATGCCAAACCCTTTACTGCCAGCTCCC | |

| Gene Name | GenBank Homology | DNA SEQUENCE / DEDUCED AMINO ACID SEQUENCE | SEQUENCE IDENTIFIER: |
|---|---|---|---|
| | | 2321 I L V K S V N T E E R G D M S I C L P A<br>6962 ATATTGGTCAAATCAGTGAATACCGAAGAAAGGGGTGACATGAGTATTTGTTTACCAGCA<br><br>2341 T S V G Q D A M G L S I S G R D N S K K<br>7022 ACTTCAGTTGGTCAAGATGCCATGGGTTTATCTATTTCTGGAAGAGATAATTCTAAAAAG<br><br>2361 P P D N L D L V S R K R F Q C R L D K N<br>7082 CCGCCTGATAATTTGGATCTTGTATCTAGGAAGAGATTTCAATGCAGGCTTGATAAAAAT<br><br>2381 D H I P P A K K R S L T L R D D C Q E Y<br>7142 GACCACATTCCTCCTGCCAAAAAACGTTCACTCACTTTAAGAGATGACTGTCAAGAATAT<br><br>2401 T T E V H S K E L T N V F E E T G E S H<br>7202 ACCACTGAGGTGCACTCAAAGGAATTAACAAATGTTTTTGAGGAAACAGGGGAGTCTCAC<br><br>2421 K G Q D I F L T S G S T L T T P E P Q R<br>7262 AAGGGACAAGATATTTTTCTTACCTCAGGAAGCACACTGACAACTCCAGAACCTCAAAGA<br><br>2441 Q Q V E A A F Q S R G S R S P D A C M D<br>7322 CAGCAAGTTGAAGCAGCTTTTCAGAGTAGAGGATCTAGATCTCCTGATGCATGCATGGAC<br><br>2461 K N V P Q L P Q D E M I V S D K E E R T<br>7382 AAGAATGTGCCTCAGTTACCTCAGGATGAAATGATTGTGTCTGATAAGGAAGAAAGAACT<br><br>2481 D A A P K S Q Q M D S R T S S S K A S L<br>7442 GATGCTGCTCCTAAGTCTCAGCAAATGGATAGCAGAACATCGTCTTCTAAAGCCTCACTA<br><br>2501 S R P G R R P L G F L S L I C S K N S L<br>7502 TCCAGACCTGGCAGAAGACCCCTGGGATTTTTATCTTTAATATGCTCAAAGAATAGTTTG<br><br>2521 E S D E P M Q V H S K K R L K P L I P G<br>7562 GAGTCTGATGAACCTATGCAAGTCCATAGTAAGAAACGCCTAAAACCTCTTATACCTGGA<br><br>2541 L R K K L K R S N P F N E S Q E K N R E<br>7622 TTAAGAAAGAAATTGAAAAGATCTAATCCATTCAATGAAAGCCAGGAAAAAAATCGAGAG<br><br>2561 S S D L L P S P S V I T T Q S E N I S S<br>7682 TCCTCTGATCTGCTTCCATCTCCAAGTGTTATTACTACTCAATCTGAGAATATTAGCAGC<br><br>2581 S A T Q V S C D Q P L L K E G Y K S A Q<br>7742 TCAGCAACTCAGGTTTCTTGTGATCAGCCCTTACTGAAAGAAGGATATAAAAGTGCCCAA<br><br>2601 K R A P Q G E A T T V S E Y F F N D I F | |

| Gene Name | GenBank Homology | DNA SEQUENCE / DEDUCED AMINO ACID SEQUENCE | SEQUENCE IDENTIFIER: |
|---|---|---|---|
| | | 7802 AAGCGGGCCCCTCAAGGGGAGGCAACCACAGTCTCTGAATATTTCTTCAATGATATCTTC<br><br>2621 I E V D E T E -<br>7862 ATTGAAGTGGATGAAACAGAATAA | |
| WBC030C04.bFSP_20 012410.abd | No Homology | 1 GAAGGTTTCCCTGAGCGTCTCTAAGCTCGCCCAGCCAGACACCCTGTGCCCCTCCATTTG<br>61 TGTCCTCTGTCCCCCTTTCCTGCTTCCCTCCTTCCCCACCGTGGCTGGTCTAGGTGTCTG<br>121 TAATAGTCAGAATGCAGGTTTGGCAACTTGAACAAAACTTTTAAATAACAGTGGCTTAAA<br>181 CCACAAAAAAAAATTGATTTCTCTGGTAATATCTAAGCACAAGCAGGCCAGGCAATATGG<br>241 GGACTCCCTGGTTTTGAGATCCAGGATTCCCAGGTCTTGGCGCTACGAGGTCCACATTCT<br>301 AGGCCTCAAGACCAGGGAAGAAGGCAGTGAAAGGGAGCATGACCCTCCCCTTCAAGGGCA<br>361 CAGCTAGGAATCACCCACATCACCTCTAATTGGCTGGAATGTGGTCACACGGTCACACCT<br>421 AGCTGCAAGGAAGGCAGGGAAATGTAGTCTTTATTCTGACCACAACGTGCCCAGGTGAAA<br>481 TTTGTCACTAGAGAACCGGTGGGCAGATATGTGTAGATAATCAATAATCTCCACCTCAGG<br>541 GGCATTGAGGCAGCCTCTTACTTCCTCTGCGTGACACCAGAGTTCGATTCCCATCTGAAA<br>601 CGACCCCTCTTTGTACCACCAGCTCCACTAGGGCTGCGTAAGGGAGGGTGGTAGTTCTCA<br>661 AGAAGGGGAGATTTTGCCCCCACAGGGGACATTTGGCAAAGTCCAGAGACATTTTCCGTT<br>721 GTCACAACTTGGGGGGTGCTATCGCCATCTAGTGGGGAGAGGTCACCGATGCTGCTAAAC<br>781 ATCATATGATGCCCCCACCAACATAGAGTTGTCTGGCCCAAAGCATCAGCAGAAGCCCTG<br>841 ATGCAGGGAAAGCGTGCGTTCCCCGGGGTCAGACACGCCTGACTTCTAACCCTGAAGCCC<br>901 CGCCTCCCTCCCCGTGTGGCCTTAGTGACCAATCAGTCACTTCACCCCTCGGAACATCGA<br>961 TGATGGTAACATGGACTTCCCTGCATTGTATGAGAACCAATTCAGGTCGTTTACTGAGGG<br>1021 CTTACCATGTGCTGTGTGCTCTGTGTACCCTCAGATCACGTGAACTCCCTTTCCATGCGC<br>1081 ACATTTACTAGCACCTACTATGCGGGGAAGTGGATTCCTCTGGATGATTTTGTTATAATA<br>1141 AACAGAAAAACAGCTTGAAAAAAA | SEQ ID NO:136 |
| WBC030C11.bFSP_20 012410.abd | Homo sapiens protein associated with Myc mRNA. | 1 TCTTGGAGCGTTCTCAGTTTCTCAACAGATCTTCACTTGCTAGGCAGCCAGAAGCCGGCG<br>61 GCAGTGGCGGCACCGCCTCCTCCTCACATTCCCGGGGTGGCGGGGTTAGATGAGCGGCCC<br>121 CAGTCGCGGCGCCGGGGGCGCTGTTCATGCCGGTTCCCGACGGCTCCGTGGCTGCTGCGG<br>181 GGCTGGGGCTGGGGCTACCCGCCGCGGACTCCCCGGGTCACTACCAGCTGCTGCTGTCAG<br>241 GCCGGGCCCTGGCCGACCGCTACCGGAGGATTTATACCGCTGCGCTCAATGACAGGGACC<br>301 AGGGGGGCGGCAGCGCTGGACACCCAGCCTCCAGGAATAAGAAAATTTTAAATAAGAAGA<br>361 AATTGAAAAGAAAACAGAAGAGCAAATCAAAAGTGAAGACAAGAAGCAAGTCTGAAAACT<br>421 TAGAGAATACAGTAATCATACCAGATATCAAACTACATAGCAATCCTTCTGCTTTCAATA<br>481 TTTACTGTAATGTACGCCATTGCGTTCTGGAATGGCAGAAAAAGGAAATATCATTGGCAG<br>541 CCGCATCTAAGAACTCTGTGCAGAGTGGAGAATCAGATAGTGATGAAGAAGAGGAATCCA<br>601 AAGAGCCCCCTATCAAGCTTCCAAAGATTATTGAGGTTGGCCTTTGTGAAGTTTTTGAAT<br>661 TGATCAAAGAGACACGATTTTCTCATCCATCCCTGTGTCTCAGGAGTCTCCAAGCCCTGC<br>721 TCAACGTGCTGCAGGGCCAGCAGCCAGAAGTGCTCCAGTCTGAGCCACCTGAGGTCCTAG<br>781 AGTCTCTCTTCCAGCTTCTTTTGGAATCACCGTTCGAAGTACTGGGATGAATGACAGCA<br>841 CAGGACAGTCCTTAACAGCACTTTCCTGTGCTTGCCTCTTTAGTCTGGTGGCTTCTTGGG | SEQ ID NO:137 |

| Gene Name | GenBank Homology | DNA SEQUENCE / DEDUCED AMINO ACID SEQUENCE | SEQUENCE IDENTIFIER: |
|---|---|---|---|
| | | 901 GAGAAACAGGAAGGACACTTCAGGCCATCTCTGCTATCCTCACCAACAATGGAAGCCATG<br>961 CTTGCCAAACTATTCAGGTGCCAACAATTCTAAATTCGCTACAGAGAAGTGTACAAGCAG<br>1021 TTTTGGTGGGAAAAATTCAAATTCAGGACTGGTTTAGTAATGGCATTAAGAAAGCAGCTT<br>1081 TAATGCACAAGTGGCCATTAAAAGAAATATCTGTTGATGAAGATGACCAATGTCTACTTC<br>1141 AGAATGATGGATTTTTTCTTTATCTATTATGCAAGGATGGATTATATAAAATAGGCTCTG<br>1201 GATACAGTGGAACAGTTAGGGGCCATATATACAATTCTACATCCCGTATTAGAAACAGAA<br>1261 AAGAAAAAAAGTCTTGGTTAGGGTATGCTCAGGGTTATTTATTATATAGAGATGTGAATA<br>1321 ACCACAGCATGACAGCCATAAGGATAAGCCCTGAAACACTGGAGCAAGATGGTACTGTGA<br>1381 TGTTACCAGATTGCCACACTGAAGGTCAAAATATTTTATTCACTGATGGAGAATATATTA<br>1441 ATCAGATAGCTGCTTCAAGAGATGATGGCTTTGTTGTCAGAATATTTGCCACAAGCACTG<br>1501 AACCTGTTCTACAGCAAGAATTGCAACTTAAACTGGCTAGAAAATGCTTACATGCCTGTC<br>1561 GTATCTCATTATTCGATCTGGAAAAGGACTTGCATATTATAAGTACAGGATTTGATGAGG<br>1621 AGTCAGCAATTCTTGGTGCAGGACGAGAGTTTGCGCTAATGAAAACAGCAAATGGAAAGA<br>1681 TATATTACACTGGCAAATACCAGAGTCTTGGAATCAAACAAGGTGGTCCTTCAGCAGGAA<br>1741 AATGGGTTGAGCTACCAATTACAAAATCTCCAAAGATAGTACACTTCTCAGTTGGACACG<br>1801 ATGGCTCTCACGCCCTTTTAGTTGCAGAAGATGGGAGCATATTCTTTACAGGATCTGCTA<br>1861 GTAAAGGAGAAGATGGAGAATCAATTAAGAGCAGACGGCAATCCAAACCTTATAAACCTA<br>1921 AAAAGATAATTAAGATGGAAGGAAAGATTGTGGTATATACAGCCTGCAATAATGGAAGTA<br>1981 GTTCTGTTATTTCTAAAGATGGAGAACTCTACATGTTTGGAAAAGATGCCATTTACTCTG<br>2041 ATAGTTCAAGTTTGGTAACTGATTTGAAGGGCCATTTTGTAACTCAGGTAGCTATGGGCA<br>2101 AAGCTCACACTTGTGTTTTAATGAAGAATGGAGAGGTGTGGACATTTGGTGTAAATAATA<br>2161 AAGGACAGTGTGGACGAGATACTGGTGCCATGAACCAAGGTGGGAAAGGGTTTGGAGTTG<br>2221 AAAATATGGCAACAGCAATGGATGAAGACCTGGAAGAAGAACTAGATGAAAAAGATGAGA<br>2281 AGTCTATGATGTGCCCTCCAGGCATGCACAAATGGAAGCTGGAGCAGTGCATGGTTTGCA<br>2341 CTGTCTGTGGAGACTGTACAGGTTATGGAGCCAGCTGTGTCAGTAGTGGACGGCCAGACA<br>2401 GAGTCCCCGGAGGGATCTGTGGTTGTGGTTCCGGAGAATCTGGTTGTGCTGTGTGTGGAT<br>2461 GTTGCAAGGCCTGTGCAAGAGAGTTAGATGGTCAAGAGGCAAGACAAAGAGGAATTCTTG<br>2521 ATGCAGTGAAAGAAATGATACCTTTAGATCTTCTTTTAGCTGTCCCAGTGCCCGGGGTTA<br>2581 ACATTGAAGAACACCTTCAGTTACGACAAGAAGAAAAACGGCAACGTGTAATCAGAAGGC<br>2641 ACAGATTAGAGGAAGGAAGAGGCCCCCTTGTATTTGCTGGTCCTATTTTTATGAACCATC<br>2701 GAGAACAGGCTCTAGCCAGACTCAGATCCCATCCAGCACACGTAAAGCATAAACGGGACA<br>2761 AGCACAAAGATGGAAGTGGAGAAAGAGGCGAAAAGGATGCAAGCAAAATCACAACATACC<br>2821 CTCCAGGCTCTGTGCGATTTGACTGTGAGCTCCGGGCAGTCCAAGTCAGCTGTGGATTTC<br>2881 ACCATTCAGTGGTTTTAATGGAAAATGGAGATGTCTATACATTTGGTTATGGGCAGCATG<br>2941 GGCAGCTAGGACATGGAGATGTCAACTCCAGGGGATGTCCCACTCTTGTTCAAGCATTGC<br>3001 CAGGCCCTAGCACACAAGTCACTGCAGGCAGCAACCATACGGCAGTACTTTTAATGGATG<br>3061 GACAGGTCTTCACATTTGGAAGTTTTTCTAAAGGACAACTGGGCAGACCAATTTTGGATG<br>3121 TGCCATATTGGAATGCAAAGCCAGCTCCCATGCCTAACATTGGATCAAAATATGGAAGAA<br>3181 AAGCTACTTGGATAGGTGCAAGTGGGGACCAAACTTTTTTACGAATTGATGAAGCACTTA<br>3241 TTAATTCTCATGTACTTGCTACATCAGAAATTTTTGCCAGTAAACACATAATAGGCTTGG<br>3301 TACCTGCTTCTATATCAGAACCTCCTCCATTTAAATGCCTTCTGATAAATAAAGTGGATG<br>3361 GGAGTTGTAAAACTTTTAATGACTCAGAACAAGAGGATCTGCAAGGATTTGGTGTGTGTC<br>3421 TTGATCCTGTATATGATGTAATTTGGAGGTTTCGACCAAATACTAGAGAGCTGTGGTGTT | |

| Gene Name | GenBank Homology | DNA SEQUENCE / DEDUCED AMINO ACID SEQUENCE | SEQUENCE IDENTIFIER: |
|---|---|---|---|
| | | 3481 ACAATGCGGTGGTTGCTGATGCCAGGCTTCCCTCTGCAGCAGACATGCAGTCCAGATGTA<br>3541 GTATCCTAAGTCCTGAACTTGCCTTACCAACAGGATCAAGGGCCCTCACTACCCGATCTC<br>3601 ATGCAGCTTTGCACATTTTAGGTTGTCTTGATACCTTGGCAGCTATGCAGGACTTAAAAA<br>3661 TGGGTGTTGCAAGTACAGAGGAAGAGACTCAAGCAGTAATGAAGGTTTATTCTAAAGAAG<br>3721 ATTATAGTGTGGTAAACAGGTTTGAAAGTCATGGAGGAGGCTGGGGTTATTCTGCCCATT<br>3781 CAGTAGAAGCTATACGTTTCAGTGCCGACACTGATATTTTACTTGGTGGTCTTGGTCTGT<br>3841 TTGGAGGTAGAGGAGAATATACTGCTAAAATTAAGCTGTTTGAATTGGGTCCTGATGGAG<br>3901 GAGATCATGAAACTGATGGTGACCTTCTTGCAGAGACTGATGTATTGGCTTATGACTGTG<br>3961 CTGCTAGAGAAAAATATGCAATGATGTTTGATGAGCCTGTTCTCCTGCAAGCTGGGTGGT<br>4021 GGTATGTGGCATGGGCCCGAGTGTCAGGACCCAGCAGTGACTGTGGATCTCATGGACAGG<br>4081 CATCTATTACCACAGATGATGGGGTTGTTTTCCAGTTCAAGAGTTCAAAGAAATCAAATA<br>4141 ATGGTACAGATGTTAATGCGGGTCAGATACCTCAGTTATTATACAGACTTCCAACCAGTG<br>4201 ATGGCAGTGCTTCAAAAGGCAAACAGCAAACCAGTGAACCTGTACACATTTTAAAGAGGT<br>4261 CTTTTGCAAGAACTGTCTCAGTGGAATGTTTTGAGTCATTGTTGAGTATTCTTCACTGGA<br>4321 GCTGGACCACCTTAGTCTTAGGAGTTGAAGAACTTAGAGGATTAAAAGGATTCCAGTTCA<br>4381 CAGCTACACTCCTAGATTTAGAGAGACTGCGCTTTGTGGGTACCTGTTGTCTGAGGTTAT<br>4441 TGCGTGTCTATACCTGTGAAATTTACCCAGTGTCAGCTACAGGAAAAGCAGTTGTAGAAG<br>4501 AAACTAGCAAATTAGCAGAGTGTATTGGAAAAACCAGAACTTTGTTAAGAAAAATTTTAT<br>4561 CAGAACCACTTGATCACTGCATGGTGAAATTGGATAATGATCCTCAAGGATATCTCAGTC<br>4621 AACCCTTGAGTCTTCTAGAAGCTGTCCTTCAGGAATGTCATAATACTTTCACTGCCTGCT<br>4681 TTCATTCTTTCTACCCAACTCCTGCCTTACAGTGGGCTTGCCTTTGTGATCTGCTGAATT<br>4741 GTTTGGATCAGGATATCCAAGAAGCAAACTTCAAGACATCAAGTAGCCGACTCCTTGCAG<br>4801 CTGTTATGTCAGCTCTGTGTCACACGTCTGTTAAGCTGACTTCCATCTTCCCGATTGCGT<br>4861 ATGATGGAGAAGTATTACTACGATCAATTGTTAAACAAGTTAGTACAGAGAACGACTCAA<br>4921 CACTAGTTCATCGTTTTCCCCTTTTGGTGGCACATATGGAAAAACTCAGCCAGAGTGAAG<br>4981 AGAATATCTCAGGGATGACAAGCTTCCGTGAAGTTCTGGAGAAAATGCTGGTCATTGTTG<br>5041 TGCTACCAGTCAGGAACAGCCTGAGGAGAGAAAATGAACTCTTCTCCTCCCACCTCGTCT<br>5101 CTAACACCTGTGGATTACTGGCCAGCATTGTCAGTGAACTGACAGCGTCAGCCCTGGGAT<br>5161 CTGAGGTTGATGGACTTAATTCTCTTCACTCTGTAAAAGCTAGTGCTAACCGATTTACAA<br>5221 AAACAAGTCAGGGCAGAAGTTGGAACACTGGGAACGGGTCCCCTGATGCAATCTGTTTTT<br>5281 CAGTAGACAAACCTGGAATAGTTGTGGTTGGTTTCTCTGTCTATGGAGGAGGTGGAATTC<br>5341 ATGAATATGAATTAGAGGTGTTGGTTGATGATAGTGAACATGCAGGAGATTCAACTCATT<br>5401 CCCACAGATGGACATCTCTGGAATTAGTGAAAGGAACGTACACAACGGATGACTCACCCA<br>5461 GTGATATAGCTGAGATCAGACTTGACAAAGTGGTTCCTTTAAAGGAAAATGTTAAATATG<br>5521 CTGTGCGCTTGAGGAACTATGGAAGCCGTACAGCCAATGGAGATGGAGGAATGACCACAG<br>5581 TTCAGTGCCCTGATGGTGTGACATTCACATTCAGCACGTGCAGCTTGAGCAGTAACGGCA<br>5641 CAAACCAAACCAGAGGACAGATCCCACAGATACTCTACTATAGGAGTGAATTTGATGGAG<br>5701 ATTTACAATCCCAACTTCTGAGTAAAGCCAATGAAGAAGATAAAAACTGTAGCAGAGCAT<br>5761 TGTCTGTTGTAAGCACTGTCGTTCGAGCCTCTAAGGACCTCCTGCACAGAGCTCTTGCTG<br>5821 TGGATGCTGATGACATTCCAGAACTGCTGAGTTCTTCCAGTCTGTTTTCCATGCTGCTCC<br>5881 CCCTTATTATAGCCTACATAGGACCAGTAGCTGCTGCTATTCCCAAGGTGGCTGTAGAAG<br>5941 TCTTTGGCCTTGTCCAACAATTGCTTCCGTCAGTTGCCATTTTGAATCAGAAGTATGCAC<br>6001 CGCCTGCCTTCAACCCTAATCAGTCGACAGATAGCACCACAGGAAACCAGCCTGAACAGG | |

| Gene Name | GenBank Homology | DNA SEQUENCE / DEDUCED AMINO ACID SEQUENCE | SEQUENCE IDENTIFIER: |
|---|---|---|---|
| | | 6061 GCCTCTCTGCTTGTACAACCTCCAGTCACTATGCTGTCATAGAGAGTGAGCACCCGTATA<br>6121 AACCTGCCTGTGTGATGCATTACAAGGTGACATTCCCAGAATGTGTGAGGTGGATGACAA<br>6181 TCGAATTTGACCCTCAGTGTGGTACTGCACAGTCAGAAGATGTCCTTCGTTTGTTGATTC<br>6241 CTGTCAGAACTGTTCAGAATTCAGGATATGGACCAAAATTGACATCTGTTCATGAAAATC<br>6301 TTAATTCATGGATAGAATTAAAGAAATTTTCAGGATCCTCTGGGTGGCCTACTATGGTTT<br>6361 TGGTGTTGCCAGGAAATGAGGCCCTTTTTTCATTGGAGACTGCATCAGATTATGTGAAAG<br>6421 ATGACAAAGCTTCTTTCTATGGTTTTATGTGTTTTGCAATTGGATATGAATTTAGCCCTG<br>6481 GACCTGATGAGGGAGTCATCCAATTGGAAAAAGAATTAGCCAATCTTGGTGGGGTTTGTG<br>6541 CAGCAGCTCTGATGAAGAAGGACCTAGCACTTCCTATTGGTAATGAATTAGAAGAAGACC<br>6601 TTGAAATTCTTGAGGAGGCTGCATTGCAGGTGTGCAAAACCCATTCTGGAATTCTTGGAA<br>6661 AGGGTCTAGCTCTTTCTCATTCACCAACTATATTAGAAGCACTTGAGGGAAATTTACCAC<br>6721 TCCAAATCCAAAGCAATGAACAGTCTTTTCTGGATGATTTTATTGCCTGTGTCCCAGGAT<br>6781 CAAGTGGTGGAAGGCTTGCAAGGTGGCTTCAGCCAGATTCATATGCGGATCCTCAGAAAA<br>6841 CATCTTTGATCCTGAATAAGGATGATATTCGTTGTGGTTGGCCTACCACCATAACTGTTC<br>6901 AAACAAAAGACCAGTATGGGGATGTGGTACATGTTCCCAATATGAAGGTGGAAGTGAAAG<br>6961 CTGTCCCTGTTTCTCAGAAAAAAATGTCTTTACAACAAGATCAAGCAAAGAAACCTCAAA<br>7021 GGATTCCTGGCAGTCCTGCAGTAACAGCTGCATCTTCTAATACTGACATGACTTATGGAG<br>7081 GGCTGGCATCACCAAAGCTAGATGTTTCATATGAACCAATGATAGTGAAGGAAGCTCGAT<br>7141 ATATTGCCATAACAATGATGAAGGTTTATGAAAATTATTCATTTGAAGAACTACGTTTTG<br>7201 CATCACCAACTCCTAAGAGACCCAGTGAGAATATGCTGATCCGTGTCAATAATGATGGGA<br>7261 CTTATTGTGCAAATTGGACTCCAGGGGCTATTGGACTCTACACTCTTCATGTTACCATTG<br>7321 ATGGCATTGAAATCGATGCTGGTCTGGAAGTAAAAGTAAAAGACCCACCAAAAGGGATGA<br>7381 TACCACCAGGAACTCAGTTGGTCAAACCAAAGTCTGAACCTCAGCCTAATAAGGTTCGAA<br>7441 AATTTGTGGCCAAGGACAGTGCGGGGCTTCGCATCCGTAGCCACCCTTCCCTTCAGAGTG<br>7501 AGCAGATAGGCATAGTGAAAGTCAATGGAACTATCACTTTTATTGATGAGATCCATAATG<br>7561 ATGATGGTGTGTGGCTGAGGCTGAATGATGAGACAATAAAGAAGTATGTCCCTAACATGA<br>7621 ATGGTTACACTGAAGCCTGGTGCCTCTCTTTTAATCAACATCTTGGCAAGAGTCTTCTGG<br>7681 TCCCTGTTGACGAATCTAAAACTAATACTGATGACTTTTTCAAAGACATAAACTCCTGCT<br>7741 GCCCACAGGAAGCAACAATGCAAGAACAAGATATGCCATTCTTGCGAGGAGGGCCAGGCA<br>7801 TGTACAAGGTAGTGAAGACGGGACCTTCAGGTCACAACATCAGAAGCTGCCCTAACCTTA<br>7861 GAGGTATCCCAATTGGAATGTTAGTTCTGGGAAACAAAGTCAAAGCAGTGGGAGAGGTAA<br>7921 CCAATTCTGAAGGGACATGGGTGCAACTGGATCAGAACAGCATGGTAGAGTTCTGTGAGA<br>7981 GTGATGAAGGAGAGGCATGGTCCTTAGCTAGAGACAGAGGCGGAAACCAGTACCTCCGAC<br>8041 ATGAAGATGAACAAGCTCTTCTGGATCAGAATTCTCAAACTCCTCCTCCAAGCCCTTTCT<br>8101 CAGTGCAAGCTTTTAATAAAGGGGCAAGTTGCAGTGCCCAAGGATTTGATTATGGACTCG<br>8161 GAAATAGCAAAGGTGATCGAGGAAACATCTCAACATCTTCTAAACCAGCCTCTACATCAG<br>8221 GAAAATCAGAGCTGTCCTCTAAACACAGCAGATCGCTTAAACCTGATGGACGTATGAGCC<br>8281 GGACTACTGCTGATCAGAAGAAGCCAAGGGGCACAGAAAGTTTATCTGCTAGTGAATCCC<br>8341 TCATCTTAAAATCTGATGCTGCAAAGTTGAGGTCAGATTCCCACAGTAGGTCATTATCCC<br>8401 CCAACCATAACACCTTGCAGACATTGAAATCTGATGGGAGGATGCCTTCTAGCTCCAGAG<br>8461 CTGAATCCCCAGGACCAGGTTCTCGGTTGTCATCTCCTAAGCCAAAGACTCTCCCAGCCA<br>8521 ATAGGTCTAGCCCATCGGGTGCTAGTTCTCCACGCTCCTCCTCACCACATGATAAAAATC<br>8581 TACCTCAAAAAAGTACTGCTCCTGTTAAGACAAAGCTTGATCCTCCTCGGGAACGTTCTA | |

| Gene Name | GenBank Homology | DNA SEQUENCE / DEDUCED AMINO ACID SEQUENCE | SEQUENCE IDENTIFIER: |
|---|---|---|---|
| | | 8641 AATCAGACTCTTACACACTTGATCCAGATACCCTCCGCAAGAAGAAAATGCCCCTCACAG<br>8701 AACCTTTGAGAGGACGGTCAACGTCACCAAAACCAAAATCAGTACCAAAGGATTCTACAG<br>8761 ATTCCCCTGGATCTGAAAATAGAGCTCCCTCTCCCCATGTGGTACAGGAAAACCTCCACA<br>8821 GTGAGGTGGTCGAAGTCTGCACCTCAAGTACTTTAAAAACAAATAGTCTAACAGACAGCA<br>8881 CCTGCGATGACAGCAGTGAATTTAAGAGTGTGGATGAAGGTTCAAATAAAGTTCATTTTA<br>8941 GCATTGGAAAAGCACCACTGAAAGATGAACAGGAAATGAGAGCATCTCCCAAAATAAGTC<br>9001 GAAAATGTGCTAATAGACACACCAGGCCCAAAAAAGAAAAATCGAGTTTTCTTTTCAAAG<br>9061 GAGATGGATCCAAGCCTTTAGAGCCAGCCAAGCAAGCCATGTCTCCTTCTGTGGCCGAAT<br>9121 GTGCCAGAGCTGTGTTTGCTTCCTTCCTCTGGCATGAAGGCATAGTACATGATGCAATGG<br>9181 CTTGTTCTTCTTTCCTAAAGTTTCATCCTGAACTTTCCAAAGAACATGCTCCTATAAGGA<br>9241 GTAGTTTAAATAGCCAACAACCTACAGAAGAAAAAGAAACCAAGTTAAAAAATAGACATT<br>9301 CATTAGAAATATCATCTGCACTGAATATGTTTAATATTGCACCCCATGGACCAGATATAT<br>9361 CTAAGATGGGTAGCATCAACAAAAACAAGGTATTGTCTATGCTTAAGGAACCACCTCTGC<br>9421 ATGAAAAATGTGAGGATGGAAAACCGAGACCACTTTTGAAATGTCCATGCATAACACAA<br>9481 TGAAGTCTAAGTCTCCTCTTCCCTTAACTTTACAACATTTAGTGGCTTTTTGGGAAGACA<br>9541 TCTCTTTGGCTACTATCAAAGCTGCTTCCCAGAATATGATTTTTCCAAGTCCTGGTTCCT<br>9601 GTGCAGTTCTTAAAAAGAAAGAGTGTGAGAAAGGAAGGAATAAGAAGTCCAAAAAGGAAA<br>9661 AAAAGAAAAAAGAAAAGGCAGAAGTTAGGCCCAGGGGTAATTTGTTTGGAGAGATGGCCC<br>9721 AGCTGGCAGTAGGAGGACCAGAGAAAGATACCATCTGTGAACTGTGTGGGGAGTCACATC<br>9781 CATACCCGGTGACCTATCACATGAGACAAGCTCACCCAGGTTGTGGCCGATATGCTGGTG<br>9841 GACAAGGTTACAATAGCATTGGGCATTTTTGTGGAGGATGGGCTGGTAACTGTGGTGATG<br>9901 GTGGCATAGGAGGAAGCACTTGGTATCTGGTATGTGATCGCTGTAGAGAAAAATACCTCC<br>9961 GCGAAAAACAGGCTGCTGCAAGGGAGAAGGTCAAACAATCTAGGAGAAAACCAATGCAAG<br>10021 TCAAGACCCCTCGTGCCTTGCCCACCATGGAAGCTCACCAGGTGATTAAAGCCAATGCAC<br>10081 TCTTCCTGCTGTCCCTGAGCAGTGCAGCAGAACCGAGCATTCTGTGTTACCATCCTGCAA<br>10141 AGCCATTCCAATCTCAGTTGCCCAGTGTAAAAGAAGGCATTTCTGAGGATCTTCCTGTGA<br>10201 AAATGCCTTGTCTGTACCTGCAGACATTAGCTAGGCATCATCATGAAAATTTTGTGGCT<br>10261 ATCAAGATGACAATCTATTCCAGGATGAAATGAGATATCTACGTTCAACATCTGTACCTG<br>10321 CCCCGTATATATCAGTAACTCCTGATGCAAGTCCTAATGTATTTGAAGAGCCAGAGAGCA<br>10381 ATATGAAGTCTATGCCACCAAGTTTAGAAACCAGTCCCATAACTGATACTGATCTTGCAA<br>10441 AGAGAACTGTCTTCCAAAGATCATACTCAGTTGTTGCTTCCGAATATGATAAACAACACT<br>10501 CCATTTTACCTGCACGAGTTAAAGCTATTCCTAGAAGAAGAGTTAACAGTGGAGACACTG<br>10561 AAGTTGGTTCTTCCCTTTTGAGACATCCGTCTCCTGAGCTTTCTCGGCTAATCTCAGCCC<br>10621 ACAGCTCTCTTTCTAAAGGAGAACGAAATTTCCAGTGGCCAGTTTTAGCTTTTGTTATAC<br>10681 AACATCATGATCTAGAAGGTCTTGAAATAGCAATGAAACAGGCCCTAAGGAAATCTGCTT<br>10741 GTCGAGTTTTTGCTATGGAGGCTTTCAACTGGCTTCTGTGTAATGTCATCCAAACCACTT<br>10801 CTCTCCATGATATTCTGTGGCATTTTGTGGCATCACTGACTCCTGCACCAGTGGAACCAG<br>10861 AGGAAGAAGAGGATGAAGAAAATAAAACAAGCAAAGAAAATTCAGAACAAGAGAAAGATA<br>10921 CAAGAGTATGTGAACATCCACTCTCAGACATAGTGATTGCCGGGGAACGTGCTCATCCTT<br>10981 TACCACACACCTTTCACCGCTTGCTGCAGACCATCTCAGACCTTATGATGTCTCTCCCCA<br>11041 GCGGCAGTTCATTACAGCAAATGGCCCTGAGGTGCTGGAGTCTCAAATTCAAGCAATCTG<br>11101 ATCACCAGTTCCTTCATCAGAGCAACGTCTTTCATCACATTAACAATATTTTGTCAAAGT<br>11161 CAGATGATGGCGATAGTGAAGAGAGTTTTAGCATCAGTATACAGTCTGGCTTTGAAGCTA | |

| Gene Name | GenBank Homology | DNA SEQUENCE / DEDUCED AMINO ACID SEQUENCE | SEQUENCE IDENTIFIER: |
|---|---|---|---|
| | | 11221 TGAGTCAGGAATTATGCATAGTAATGTGCTTAAAGGACTTAACCAGCATTGTTGACATAA<br>11281 AAACTTCAAGCCGACCTGCCATGATTGGCAGTTTGACAGACGGCTCCACAGAAACCTTTT<br>11341 GGGAATCAGGAGATGAAGATAAAAACAAAACTAAGAACATCACCATCAACTGTGTAAAAG<br>11401 GAATCAATGCCCGCTATGTGTCTGTTCACGTGGACAATTCCCGAGATCTTGGGAATAAAG<br>11461 TTACCTCAATGACCTTCTTAACTGGCAAAGCAGTAGAAGATTTGTGCAGAATAAAGCAAG<br>11521 TTGATCTGGATTCCAGGCACATTGGCTGGGTAACAAGTGAACTTCCAGGAGGAGATAATC<br>11581 ACATCATCAAAATTGAATTAAAAGGCCCAGAAAATACACTGAGAGTTCGACAAGTTAAGG<br>11641 TTCTGGGCTGGAAGGATGGTGAAAGCACTAAAATTGCTGGCCAGATTTCAGCCAGTGTGG<br>11701 CCCAGCAAAGGAACTGTGAAGCTGAGACTCTGCGAGTGTTCAGACTTATTACATCTCAAG<br>11761 TATTTGGAAAGCTCATCTCTGGAGATGCTGAACCCACACCAGAACAAGAGGAAAAAGCAC<br>11821 TGTTGTCATCACCTGAAGGAGAGGAAAAAGTATACAATGCAACATCAGATGCTGACCTGA<br>11881 AAGAACATATGGTTGGAATCATATTCAGCAGGAGTAAACTGACTAACTTACAAAAACAGG<br>11941 TGTGTGCTCACATAGTCCAGGCTATTCGCATGGAAGCTACCAGAGTTCGTGAAGAATGGG<br>12001 AACATGCCATATCAAGCAAAGAAAATGCCAATTCTCAGCCCAATGATGAAGATGCCTCCT<br>12061 CTGATGCTTACTGCTTTGAGCTGCTCTCGATGGTTTTAGCACTGAGTGGCTCTAACGTGG<br>12121 GCCGGCAGTATCTGGCTCAGCAACTAACTCTGCTCCAGGATCTCTTTTCACTGCTCCACA<br>12181 CAGCCTCCCCTCGAGTCCAGAGACAGGTGACCTCCTTACTAAGACGAGTTTTACCTGAAG<br>12241 TGACCCCTAATCGGCTGGCCAGCATCATAGGAGTGAAATCCCTTCCCCCACCAGATATCA<br>12301 GTGATATCATTCACTCAACAGAGAAAGGAGATTGGAATAAGCTAGGTATCTTGGACATGT<br>12361 TTCTAGGATGCATTGCCAAAGCACTCACAGTACAGCTAAAAGCCAAAGGAACCACCATCA<br>12421 CTGGAACAGCTGGTACCACTGTGGGCAAAGGAGTTACAACAGTTACTCTTCCGATGATTT<br>12481 TCAATTCCAGTTATCTCCGACGAGGTGAAAGTCATTGGTGGATGAAGGGCTCAACCCCTA<br>12541 CCCAGATCTCAGAGATCATCATTAAACTTATCAAGGATATGGCAGCAGGTCATCTGTCAG<br>12601 AAGCTTGGTCCCGAGTGACAAAAAATGCTATTGCAGAAACCATCATTGCCTTGACCAAGA<br>12661 TGGAAGAAGAATTTAGGTCTCCAGTGAGATGTATTGCAACAACTAGACTCTGGCTTGCTC<br>12721 TCGCATCCCTATGTGTTCTTGATCAGGACCACGTAGATCGTCTCTCCTCGGGGAGATGGA<br>12781 TGGGAAAGGATGGACAACAAAAACAAATGCCTATGTGTGATAACCATGATGATGGTGAAA<br>12841 CTGCAGCAATCATTTTATGCAATGTCTGTGGAAATTTATGTACAGACTGTGACAGATTCC<br>12901 TTCACCTTCATCGAAGAACCAAAACTCATCAAAGACAGGTCTTCAAAGAAGAAGAAGAAG<br>12961 CTATAAAGGTTGACCTTCATGAAGGTTGTGGTAGAACCAAATTGTTCTGGTTGATGGCAC<br>13021 TGGCAGATTCTAAAACAATGAAGGCAATGGTGGAATTCCGAGAACACACAGGCAAACCCA<br>13081 CCACGAGTAGCTCAGAAGCATGTCGCTTCTGTGGTTCCAGGAGTGGAACAGAGTTATCTG<br>13141 CTGTTGGCAGTGTTTGTTCTGATGCAGATTGCCAGGAATACGCTAAGATAGCCTGTAGTA<br>13201 AGACGCATCCTTGTGGCCATCCATGCGGGGGTGTTAAAAACGAAGAGCACTGTCTGCCCT<br>13261 GTCTACACGGCTGTGACAAAAGTGCCACAAGCCTGAAGCAAGACGCCGATGACATGTGCA<br>13321 TGATATGTTTCACCGAAGCGCTCTCGGCAGCACCAGCCATTCAGCTGGATTGTAGTCACA<br>13381 TATTCCACTTACAGTGCTGTCGGCGAGTATTAGAAAATCGATGGCTTGGCCCAAGGATAA<br>13441 CATTTGGATTTATATCTTGTCCCATTTGCAAGAACAAAATTAATCACATAGTACTAAAAG<br>13501 ACCTACTTGATCCAATAAAAGAACTCTATGAGGATGTCAGAAGAAAAGCCTTAATGAGAT<br>13561 TGGAATATGAAGGTCTGCATAAGAGTGAAGCTATCACAACTCCTGGTGTGAGGTTTTATA<br>13621 ATGACCCAGCTGGCTATGCAATGAATAGATATGCATATTATGTGTGCTACAAATGCAGAA<br>13681 AGGCATATTTTGGTGGTGAAGCTCGCTGCGATGCTGAGGCTGGACGGGGAGATGATTATG<br>13741 ATCCCAGAGAGCTCATTTGTGGTGCCTGTTCTGATGTTTCCAGGGCTCAGATGTGTCCCA | |

| Gene Name | GenBank Homology | DNA SEQUENCE / DEDUCED AMINO ACID SEQUENCE | SEQUENCE IDENTIFIER: |
|---|---|---|---|
| | | 13801 AACATGGCACAGACTTTTTGGAATATAAATGTCGCTACTGCTGTTCAGTGGCTGTTTTTT<br>13861 TCTGTTTTGGAACAACACATTTTTGTAATGCTTGTCATGATGATTTTCAAAGAATGACTA<br>13921 GCATTCCTAAGGAAGAACTACCACACTGTCCTGCAGGTCCCAAAGGCAAGCAGTTAGAAG<br>13981 GAACTGAATGTCCACTCCATGTTGTTCATCCACCCACTGGGGAAGAGTTTGCTCTGGGAT<br>14041 GTGGAGTGTGCAGAAATGCCCACACTTTTTAGAACACGCAGATCCTTTGTCTACAGAGAG<br>14101 AAAAATTGCCTTCATCCCCCAAGAGGATGCGGTGAAGTTTAAACTCTGCTCAGGATAAGG<br>14161 ACGGGACCATTTTGACATCCATGAAAATGAACCATTCACAGTGCAAGAAGGATGCCAAAT<br>14221 ACCATGTACATAATTCTTGCTATGGAAAGTTTCCCCATTATTTCGGTTTATCTTCTTTTG<br>14281 AACCAAGACATCAAACTTGTGAGGTGTTTGCATGTGGCCATTACCGTCATTGGCCTGTGA<br>14341 AGCATTGGACATTTATAGATAATTGATATAAAAGAATTGCCATACCTATGGACTAAGAAT<br>14401 GATGCTGGCTTTCAAGCAAAAAAGAAAAATAATCATTGTTTATTGTATACTACCTTTTTG<br>14461 TAATCCTGTACAATTGCATCACGGGTGGGGATAAAAAAAGGAATATTCTGGTTTATTTCC<br>14521 TAGACTGTTATTTAAAAAAAAAAAAAACATTGTGTTAGGACGGCATATAAATGTAATAAG<br>14581 TATCACACTGTATATAAAGATATCAATGTTTGTCCTGTATAAGAATTACTAAATTACAAA<br>14641 TGCAGTTTCATTTAAACTTCTAGGTTAAGTTTGAGCCTGAAATTTTAATGAAGTGCAATA<br>14701 CTGAGTGTGCCTCATTATCTTGCAGCTGTAAACATATTGGAATGACATGTCAATAAACCA<br>14761 CTGTACATTTTTATACAGTGATAAAGTCTAAAAAAAAAAAAAAA<br><br>1 M P V P D G S V A A A G L G L G L P A A<br>1 ATGCCGGTTCCCGACGGCTCCGTGGCTGCTGCGGGGCTGGGGCTGGGGCTACCCGCCGCG<br><br>21 D S P G H Y Q L L L S G R A L A D R Y R<br>61 GACTCCCCGGGTCACTACCAGCTGCTGCTGTCAGGCCGGGCCCTGGCCGACCGCTACCGG<br><br>41 R I Y T A A L N D R D Q G G G S A G H P<br>121 AGGATTTATACCGCTGCGCTCAATGACAGGGACCAGGGGGGCGGCAGCGCTGGACACCCA<br><br>61 A S R N K K I L N K K K L K R K Q K S K<br>181 GCCTCCAGGAATAAGAAAATTTTAAATAAGAAGAAATTGAAAAGAAAACAGAAGAGCAAA<br><br>81 S K V K T R S K S E N L E N T V I I P D<br>241 TCAAAAGTGAAGACAAGAAGCAAGTCTGAAAACTTAGAGAATACAGTAATCATACCAGAT<br><br>101 I K L H S N P S A F N I Y C N V R H C V<br>301 ATCAAACTACATAGCAATCCTTCTGCTTTCAATATTTACTGTAATGTACGCCATTGCGTT<br><br>121 L E W Q K K E I S L A A A S K N S V Q S<br>361 CTGGAATGGCAGAAAAAGGAAATATCATTGGCAGCCGCATCTAAGAACTCTGTGCAGAGT<br><br>141 G E S D S D E E E E S K E P P I K L P K<br>421 GGAGAATCAGATAGTGATGAAGAAGAGGAATCCAAAGAGCCCCCTATCAAGCTTCCAAAG<br><br>161 I I E V G L C E V F E L I K E T R F S H | SEQ ID NO:138 |

| Gene Name | GenBank Homology | DNA SEQUENCE / DEDUCED AMINO ACID SEQUENCE | SEQUENCE IDENTIFIER: |
|---|---|---|---|
| | | 481 ATTATTGAGGTTGGCCTTTGTGAAGTTTTTGAATTGATCAAAGAGACACGATTTTCTCAT<br><br>181 P S L C L R S L Q A L L N V L Q G Q Q P<br>541 CCATCCCTGTGTCTCAGGAGTCTCCAAGCCCTGCTCAACGTGCTGCAGGGCCAGCAGCCA<br><br>201 E V L Q S E P P E V L E S L F Q L L L E<br>601 GAAGTGCTCCAGTCTGAGCCACCTGAGGTCCTAGAGTCTCTCTTCCAGCTTCTTTTGGAA<br><br>221 I T V R S T G M N D S T G Q S L T A L S<br>661 ATCACCGTTCGAAGTACTGGGATGAATGACAGCACAGGACAGTCCTTAACAGCACTTTCC<br><br>241 C A C L F S L V A S W G E T G R T L Q A<br>721 TGTGCTTGCCTCTTTAGTCTGGTGGCTTCTTGGGGAGAAACAGGAAGGACACTTCAGGCC<br><br>261 I S A I L T N N G S H A C Q T I Q V P T<br>781 ATCTCTGCTATCCTCACCAACAATGGAAGCCATGCTTGCCAAACTATTCAGGTGCCAACA<br><br>281 I L N S L Q R S V Q A V L V G K I Q I Q<br>841 ATTCTAAATTCGCTACAGAGAAGTGTACAAGCAGTTTTGGTGGGAAAAATTCAAATTCAG<br><br>301 D W F S N G I K K A A L M H K W P L K E<br>901 GACTGGTTTAGTAATGGCATTAAGAAAGCAGCTTTAATGCACAAGTGGCCATTAAAAGAA<br><br>321 I S V D E D D Q C L L Q N D G F F L Y L<br>961 ATATCTGTTGATGAAGATGACCAATGTCTACTTCAGAATGATGGATTTTTTCTTTATCTA<br><br>341 L C K D G L Y K I G S G Y S G T V R G H<br>1021 TTATGCAAGGATGGATTATATAAAATAGGCTCTGGATACAGTGGAACAGTTAGGGGCCAT<br><br>361 I Y N S T S R I R N R K E K K S W L G Y<br>1081 ATATACAATTCTACATCCCGTATTAGAAACAGAAAAGAAAAAAAGTCTTGGTTAGGGTAT<br><br>381 A Q G Y L L Y R D V N N H S M T A I R I<br>1141 GCTCAGGGTTATTTATTATATAGAGATGTGAATAACCACAGCATGACAGCCATAAGGATA<br><br>401 S P E T L E Q D G T V M L P D C H T E G<br>1201 AGCCCTGAAACACTGGAGCAAGATGGTACTGTGATGTTACCAGATTGCCACACTGAAGGT<br><br>421 Q N I L F T D G E Y I N Q I A A S R D D<br>1261 CAAAATATTTTATTCACTGATGGAGAATATATTAATCAGATAGCTGCTTCAAGAGATGAT<br><br>441 G F V V R I F A T S T E P V L Q Q E L Q<br>1321 GGCTTTGTTGTCAGAATATTTGCCACAAGCACTGAACCTGTTCTACAGCAAGAATTGCAA | |

| Gene Name | GenBank Homology | DNA SEQUENCE / DEDUCED AMINO ACID SEQUENCE | SEQUENCE IDENTIFIER: |
|---|---|---|---|
| | | 461 L K L A R K C L H A C R I S L F D L E K<br>1381 CTTAAACTGGCTAGAAAATGCTTACATGCCTGTCGTATCTCATTATTCGATCTGGAAAAG<br><br>481 D L H I I S T G F D E E S A I L G A G R<br>1441 GACTTGCATATTATAAGTACAGGATTTGATGAGGAGTCAGCAATTCTTGGTGCAGGACGA<br><br>501 E F A L M K T A N G K I Y Y T G K Y Q S<br>1501 GAGTTTGCGCTAATGAAAACAGCAAATGGAAAGATATATTACACTGGCAAATACCAGAGT<br><br>521 L G I K Q G G P S A G K W V E L P I T K<br>1561 CTTGGAATCAAACAAGGTGGTCCTTCAGCAGGAAAATGGGTTGAGCTACCAATTACAAAA<br><br>541 S P K I V H F S V G H D G S H A L L V A<br>1621 TCTCCAAAGATAGTACACTTCTCAGTTGGACACGATGGCTCTCACGCCCTTTTAGTTGCA<br><br>561 E D G S I F F T G S A S K G E D G E S I<br>1681 GAAGATGGGAGCATATTCTTTACAGGATCTGCTAGTAAAGGAGAAGATGGAGAATCAATT<br><br>581 K S R R Q S K P Y K P K K I I K M E G K<br>1741 AAGAGCAGACGGCAATCCAAACCTTATAAACCTAAAAAGATAATTAAGATGGAAGGAAAG<br><br>601 I V V Y T A C N N G S S S V I S K D G E<br>1801 ATTGTGGTATATACAGCCTGCAATAATGGAAGTAGTTCTGTTATTTCTAAAGATGGAGAA<br><br>621 L Y M F G K D A I Y S D S S S L V T D L<br>1861 CTCTACATGTTTGGAAAAGATGCCATTTACTCTGATAGTTCAAGTTTGGTAACTGATTTG<br><br>641 K G H F V T Q V A M G K A H T C V L M K<br>1921 AAGGGCCATTTTGTAACTCAGGTAGCTATGGGCAAAGCTCACACTTGTGTTTTAATGAAG<br><br>661 N G E V W T F G V N N K G Q C G R D T G<br>1981 AATGGAGAGGTGTGGACATTTGGTGTAAATAATAAAGGACAGTGTGGACGAGATACTGGT<br><br>681 A M N Q G G K G F G V E N M A T A M D E<br>2041 GCCATGAACCAAGGTGGGAAAGGGTTTGGAGTTGAAAATATGGCAACAGCAATGGATGAA<br><br>701 D L E E E L D E K D E K S M M C P P G M<br>2101 GACCTGGAAGAAGAACTAGATGAAAAAGATGAGAAGTCTATGATGTGCCCTCCAGGCATG<br><br>721 H K W K L E Q C M V C T V C G D C T G Y<br>2161 CACAAATGGAAGCTGGAGCAGTGCATGGTTTGCACTGTCTGTGGAGACTGTACAGGTTAT | |

| Gene Name | GenBank Homology | DNA SEQUENCE / DEDUCED AMINO ACID SEQUENCE | SEQUENCE IDENTIFIER: |
|---|---|---|---|
| | | 741 G A S C V S S G R P D R V P G G I C G C<br>2221 GGAGCCAGCTGTGTCAGTAGTGGACGGCCAGACAGAGTCCCCGGAGGGATCTGTGGTTGT<br><br>761 G S G E S G C A V C G C C K A C A R E L<br>2281 GGTTCCGGAGAATCTGGTTGTGCTGTGTGTGGATGTTGCAAGGCCTGTGCAAGAGAGTTA<br><br>781 D G Q E A R Q R G I L D A V K E M I P L<br>2341 GATGGTCAAGAGGCAAGACAAAGAGGAATTCTTGATGCAGTGAAAGAAATGATACCTTTA<br><br>801 D L L L A V P V P G V N I E E H L Q L R<br>2401 GATCTTCTTTTAGCTGTCCCAGTGCCCGGGGTTAACATTGAAGAACACCTTCAGTTACGA<br><br>821 Q E E K R Q R V I R R H R L E E G R G P<br>2461 CAAGAAGAAAAACGGCAACGTGTAATCAGAAGGCACAGATTAGAGGAAGGAAGAGGCCCC<br><br>841 L V F A G P I F M N H R E Q A L A R L R<br>2521 CTTGTATTTGCTGGTCCTATTTTTATGAACCATCGAGAACAGGCTCTAGCCAGACTCAGA<br><br>861 S H P A H V K H K R D K H K D G S G E R<br>2581 TCCCATCCAGCACACGTAAAGCATAAACGGGACAAGCACAAAGATGGAAGTGGAGAAAGA<br><br>881 G E K D A S K I T T Y P P G S V R F D C<br>2641 GGCGAAAAGGATGCAAGCAAAATCACAACATACCCTCCAGGCTCTGTGCGATTTGACTGT<br><br>901 E L R A V Q V S C G F H H S V V L M E N<br>2701 GAGCTCCGGGCAGTCCAAGTCAGCTGTGGATTTCACCATTCAGTGGTTTTAATGGAAAAT<br><br>921 G D V Y T F G Y G Q H G Q L G H G D V N<br>2761 GGAGATGTCTATACATTTGGTTATGGGCAGCATGGGCAGCTAGGACATGGAGATGTCAAC<br><br>941 S R G C P T L V Q A L P G P S T Q V T A<br>2821 TCCAGGGGATGTCCCACTCTTGTTCAAGCATTGCCAGGCCCTAGCACACAAGTCACTGCA<br><br>961 G S N H T A V L L M D G Q V F T F G S F<br>2881 GGCAGCAACCATACGGCAGTACTTTTAATGGATGGACAGGTCTTCACATTTGGAAGTTTT<br><br>981 S K G Q L G R P I L D V P Y W N A K P A<br>2941 TCTAAAGGACAACTGGGCAGACCAATTTTGGATGTGCCATATTGGAATGCAAAGCCAGCT<br><br>1001 P M P N I G S K Y G R K A T W I G A S G<br>3001 CCCATGCCTAACATTGGATCAAAATATGGAAGAAAAGCTACTTGGATAGGTGCAAGTGGG<br><br>1021 D Q T F L R I D E A L I N S H V L A T S | |

| Gene Name | GenBank Homology | DNA SEQUENCE / DEDUCED AMINO ACID SEQUENCE | SEQUENCE IDENTIFIER: |
|---|---|---|---|
| | | 3061 GACCAAACTTTTTACGAATTGATGAAGCACTTATTAATTCTCATGTACTTGCTACATCA<br><br>1041 E I F A S K H I I G L V P A S I S E P P<br>3121 GAAATTTTTGCCAGTAAACACATAATAGGCTTGGTACCTGCTTCTATATCAGAACCTCCT<br><br>1061 P F K C L L I N K V D G S C K T F N D S<br>3181 CCATTTAAATGCCTTCTGATAAATAAAGTGGATGGGAGTTGTAAAACTTTTAATGACTCA<br><br>1081 E Q E D L Q G F G V C L D P V Y D V I W<br>3241 GAACAAGAGGATCTGCAAGGATTTGGTGTGTGTCTTGATCCTGTATATGATGTAATTTGG<br><br>1101 R F R P N T R E L W C Y N A V V A D A R<br>3301 AGGTTTCGACCAAATACTAGAGAGCTGTGGTGTTACAATGCGGTGGTTGCTGATGCCAGG<br><br>1121 L P S A A D M Q S R C S I L S P E L A L<br>3361 CTTCCCTCTGCAGCAGACATGCAGTCCAGATGTAGTATCCTAAGTCCTGAACTTGCCTTA<br><br>1141 P T G S R A L T T R S H A A L H I L G C<br>3421 CCAACAGGATCAAGGGCCCTCACTACCCGATCTCATGCAGCTTTGCACATTTTAGGTTGT<br><br>1161 L D T L A A M Q D L K M G V A S T E E E<br>3481 CTTGATACCTTGGCAGCTATGCAGGACTTAAAAATGGGTGTTGCAAGTACAGAGGAAGAG<br><br>1181 T Q A V M K V Y S K E D Y S V V N R F E<br>3541 ACTCAAGCAGTAATGAAGGTTTATTCTAAAGAAGATTATAGTGTGGTAAACAGGTTTGAA<br><br>1201 S H G G G W G Y S A H S V E A I R F S A<br>3601 AGTCATGGAGGAGGCTGGGGTTATTCTGCCCATTCAGTAGAAGCTATACGTTTCAGTGCC<br><br>1221 D T D I L L G G L G L F G G R G E Y T A<br>3661 GACACTGATATTTTACTTGGTGGTCTTGGTCTGTTTGGAGGTAGAGGAGAATATACTGCT<br><br>1241 K I K L F E L G P D G G D H E T D G D L<br>3721 AAAATTAAGCTGTTTGAATTGGGTCCTGATGGAGGAGATCATGAAACTGATGGTGACCTT<br><br>1261 L A E T D V L A Y D C A A R E K Y A M M<br>3781 CTTGCAGAGACTGATGTATTGGCTTATGACTGTGCTGCTAGAGAAAAATATGCAATGATG<br><br>1281 F D E P V L L Q A G W W Y V A W A R V S<br>3841 TTTGATGAGCCTGTTCTCCTGCAAGCTGGGTGGTGGTATGTGGCATGGGCCCGAGTGTCA<br><br>1301 G P S S D C G S H G Q A S I T T D D G V<br>3901 GGACCCAGCAGTGACTGTGGATCTCATGGACAGGCATCTATTACCACAGATGATGGGGTT | |

| Gene Name | GenBank Homology | DNA SEQUENCE / DEDUCED AMINO ACID SEQUENCE | SEQUENCE IDENTIFIER: |
|---|---|---|---|
| | | 1321 V F Q F K S S K K S N N G T D V N A G Q<br>3961 GTTTTCCAGTTCAAGAGTTCAAAGAAATCAAATAATGGTACAGATGTTAATGCGGGTCAG<br><br>1341 I P Q L L Y R L P T S D G S A S K G K Q<br>4021 ATACCTCAGTTATTATACAGACTTCCAACCAGTGATGGCAGTGCTTCAAAAGGCAAACAG<br><br>1361 Q T S E P V H I L K R S F A R T V S V E<br>4081 CAAACCAGTGAACCTGTACACATTTTAAAGAGGTCTTTTGCAAGAACTGTCTCAGTGGAA<br><br>1381 C F E S L L S I L H W S W T T L V L G V<br>4141 TGTTTTGAGTCATTGTTGAGTATTCTTCACTGGAGCTGGACCACCTTAGTCTTAGGAGTT<br><br>1401 E E L R G L K G F Q F T A T L L D L E R<br>4201 GAAGAACTTAGAGGATTAAAAGGATTCCAGTTCACAGCTACACTCCTAGATTTAGAGAGA<br><br>1421 L R F V G T C C L R L L R V Y T C E I Y<br>4261 CTGCGCTTTGTGGGTACCTGTTGTCTGAGGTTATTGCGTGTCTATACCTGTGAAATTTAC<br><br>1441 P V S A T G K A V V E E T S K L A E C I<br>4321 CCAGTGTCAGCTACAGGAAAAGCAGTTGTAGAAGAAACTAGCAAATTAGCAGAGTGTATT<br><br>1461 G K T R T L L R K I L S E P L D H C M V<br>4381 GGAAAAACCAGAACTTTGTTAAGAAAAATTTTATCAGAACCACTTGATCACTGCATGGTG<br><br>1481 K L D N D P Q G Y L S Q P L S L L E A V<br>4441 AAATTGGATAATGATCCTCAAGGATATCTCAGTCAACCCTTGAGTCTTCTAGAAGCTGTC<br><br>1501 L Q E C H N T F T A C F H S F Y P T P A<br>4501 CTTCAGGAATGTCATAATACTTTCACTGCCTGCTTTCATTCTTTCTACCCAACTCCTGCC<br><br>1521 L Q W A C L C D L L N C L D Q D I Q E A<br>4561 TTACAGTGGGCTTGCCTTTGTGATCTGCTGAATTGTTTGGATCAGGATATCCAAGAAGCA<br><br>1541 N F K T S S S R L L A A V M S A L C H T<br>4621 AACTTCAAGACATCAAGTAGCCGACTCCTTGCAGCTGTTATGTCAGCTCTGTGTCACACG<br><br>1561 S V K L T S I F P I A Y D G E V L L R S<br>4681 TCTGTTAAGCTGACTTCCATCTTCCCGATTGCGTATGATGGAGAAGTATTACTACGATCA<br><br>1581 I V K Q V S T E N D S T L V H R F P L L<br>4741 ATTGTTAAACAAGTTAGTACAGAGAACGACTCAACACTAGTTCATCGTTTTCCCCTTTTG | |

| Gene Name | GenBank Homology | DNA SEQUENCE / DEDUCED AMINO ACID SEQUENCE | SEQUENCE IDENTIFIER: |
|---|---|---|---|
| | | 1601 V A H M E K L S Q S E E N I S G M T S F<br>4801 GTGGCACATATGGAAAAACTCAGCCAGAGTGAAGAGAATATCTCAGGGATGACAAGCTTC<br><br>1621 R E V L E K M L V I V V L P V R N S L R<br>4861 CGTGAAGTTCTGGAGAAAATGCTGGTCATTGTTGTGCTACCAGTCAGGAACAGCCTGAGG<br><br>1641 R E N E L F S S H L V S N T C G L L A S<br>4921 AGAGAAAATGAACTCTTCTCCTCCCACCTCGTCTCTAACACCTGTGGATTACTGGCCAGC<br><br>1661 I V S E L T A S A L G S E V D G L N S L<br>4981 ATTGTCAGTGAACTGACAGCGTCAGCCCTGGGATCTGAGGTTGATGGACTTAATTCTCTT<br><br>1681 H S V K A S A N R F T K T S Q G R S W N<br>5041 CACTCTGTAAAAGCTAGTGCTAACCGATTTACAAAAACAAGTCAGGGCAGAAGTTGGAAC<br><br>1701 T G N G S P D A I C F S V D K P G I V V<br>5101 ACTGGGAACGGGTCCCCTGATGCAATCTGTTTTTCAGTAGACAAACCTGGAATAGTTGTG<br><br>1721 V G F S V Y G G G G I H E Y E L E V L V<br>5161 GTTGGTTTCTCTGTCTATGGAGGAGGTGGAATTCATGAATATGAATTAGAGGTGTTGGTT<br><br>1741 D D S E H A G D S T H S H R W T S L E L<br>5221 GATGATAGTGAACATGCAGGAGATTCAACTCATTCCCACAGATGGACATCTCTGGAATTA<br><br>1761 V K G T Y T T D D S P S D I A E I R L D<br>5281 GTGAAAGGAACGTACACAACGGATGACTCACCCAGTGATATAGCTGAGATCAGACTTGAC<br><br>1781 K V V P L K E N V K Y A V R L R N Y G S<br>5341 AAAGTGGTTCCTTTAAAGGAAAATGTTAAATATGCTGTGCGCTTGAGGAACTATGGAAGC<br><br>1801 R T A N G D G G M T T V Q C P D G V T F<br>5401 CGTACAGCCAATGGAGATGGAGGAATGACCACAGTTCAGTGCCCTGATGGTGTGACATTC<br><br>1821 T F S T C S L S S N G T N Q T R G Q I P<br>5461 ACATTCAGCACGTGCAGCTTGAGCAGTAACGGCACAAACCAAACCAGAGGACAGATCCCA<br><br>1841 Q I L Y Y R S E F D G D L Q S Q L L S K<br>5521 CAGATACTCTACTATAGGAGTGAATTTGATGGAGATTTACAATCCCAACTTCTGAGTAAA<br><br>1861 A N E E D K N C S R A L S V V S T V V R<br>5581 GCCAATGAAGAAGATAAAAACTGTAGCAGAGCATTGTCTGTTGTAAGCACTGTCGTTCGA<br><br>1881 A S K D L L H R A L A V D A D D I P E L | |

| Gene Name | GenBank Homology | DNA SEQUENCE / DEDUCED AMINO ACID SEQUENCE | SEQUENCE IDENTIFIER: |
|---|---|---|---|
| | | 5641 GCCTCTAAGGACCTCCTGCACAGAGCTCTTGCTGTGGATGCTGATGACATTCCAGAACTG<br><br>1901 L S S S S L F S M L L P L I I A Y I G P<br>5701 CTGAGTTCTTCCAGTCTGTTTTCCATGCTGCTCCCCCTTATTATAGCCTACATAGGACCA<br><br>1921 V A A A I P K V A V E V F G L V Q Q L L<br>5761 GTAGCTGCTGCTATTCCCAAGGTGGCTGTAGAAGTCTTTGGCCTTGTCCAACAATTGCTT<br><br>1941 P S V A I L N Q K Y A P P A F N P N Q S<br>5821 CCGTCAGTTGCCATTTTGAATCAGAAGTATGCACCGCCTGCCTTCAACCCTAATCAGTCG<br><br>1961 T D S T T G N Q P E Q G L S A C T T S S<br>5881 ACAGATAGCACCACAGGAAACCAGCCTGAACAGGGCCTCTCTGCTTGTACAACCTCCAGT<br><br>1981 H Y A V I E S E H P Y K P A C V M H Y K<br>5941 CACTATGCTGTCATAGAGAGTGAGCACCCGTATAAACCTGCCTGTGTGATGCATTACAAG<br><br>2001 V T F P E C V R W M T I E F D P Q C G T<br>6001 GTGACATTCCCAGAATGTGTGAGGTGGATGACAATCGAATTTGACCCTCAGTGTGGTACT<br><br>2021 A Q S E D V L R L L I P V R T V Q N S G<br>6061 GCACAGTCAGAAGATGTCCTTCGTTTGTTGATTCCTGTCAGAACTGTTCAGAATTCAGGA<br><br>2041 Y G P K L T S V H E N L N S W I E L K K<br>6121 TATGGACCAAAATTGACATCTGTTCATGAAAATCTTAATTCATGGATAGAATTAAAGAAA<br><br>2061 F S G S S G W P T M V L V L P G N E A L<br>6181 TTTTCAGGATCCTCTGGGTGGCCTACTATGGTTTTGGTGTTGCCAGGAAATGAGGCCCTT<br><br>2081 F S L E T A S D Y V K D D K A S F Y G F<br>6241 TTTTCATTGGAGACTGCATCAGATTATGTGAAAGATGACAAAGCTTCTTTCTATGGTTTT<br><br>2101 M C F A I G Y E F S P G P D E G V I Q L<br>6301 ATGTGTTTTGCAATTGGATATGAATTTAGCCCTGGACCTGATGAGGGAGTCATCCAATTG<br><br>2121 E K E L A N L G G V C A A A L M K K D L<br>6361 GAAAAAGAATTAGCCAATCTTGGTGGGGTTTGTGCAGCAGCTCTGATGAAGAAGGACCTA<br><br>2141 A L P I G N E L E E D L E I L E E A A L<br>6421 GCACTTCCTATTGGTAATGAATTAGAAGAAGACCTTGAAATTCTTGAGGAGGCTGCATTG<br><br>2161 Q V C K T H S G I L G K G L A L S H S P<br>6481 CAGGTGTGCAAAACCCATTCTGGAATTCTTGGAAAGGGTCTAGCTCTTTCTCATTCACCA | |

| Gene Name | GenBank Homology | DNA SEQUENCE / DEDUCED AMINO ACID SEQUENCE | SEQUENCE IDENTIFIER: |
|---|---|---|---|
| | | 2181 T I L E A L E G N L P L Q I Q S N E Q S<br>6541 | |
| WBC030E04.bFSP_20 012410.abd | Equus caballus matrix metalloproteinase 1 precursor (MMP1) | 1 AGGGAAGTAGGGAGCTATCAGACTGCTTTATCGGAGAAGGAAGACAGAGGCCAGCATGCT<br>61 CAGCCTTCCTCTGCTGCTGCTGCTACTCTGGGGCATGGGCTCTCACAGCTTCCCAACAGT<br>121 GCCTTCAGAAACACGAGAAGAAGATGTGGAGATGGTCCAGAAATACCTGGAAAACTACTA<br>181 CAACCTGAAGAGTGATGGAGAGCAAATTGAAAAGCAGAGACACAGGAGCCCAGTCGTTGA<br>241 AAAGCTGAAGCAAATGCAGGAATTCTTTGGGCTGAAAGTGACTGGGAAGCCAGATGCTGA<br>301 AACCCTGAATGTGATGAAGCAGCCCAGATGCGGGGTGCCTGATGTGGCTGAGTTTGTCCT<br>361 CACCGAAGGGAACCCTCGGTGGGAGAACACACACCTGACCTACAGGATTGAAAATTACAC<br>421 ACCAGATTTACCAAGAGCAGATGTGGACCAGGCCATTGAGAAAGCCTTTCAACTCTGGAG<br>481 TAATGTCTCACCCCTGACCTTCACTAAAGTCTCTGAGGGTCAAGCGGACATAATGATATC<br>541 CTTTGTCAGGGGAGATCATCGTGACAATTCTCCCTTTGATGGACCTGGAGGAAACCTCGC<br>601 CCATGCTTTTCAGCCAGGCCCACGTATTGGAGGGGATGCTCATTTTGATGAAGATGAAAC<br>661 ATGGACCAGCAATTTCAGAAATTACAACTTGTATCGAGTTGCAGCTCATGAATTTGGCCA<br>721 TTCCCTTGGACTCTCTCATTCTACTGATATTGGGGCTTTGATGTACCCCAACTACTTCTT<br>781 CACTGGTGATGTTCAGCTATCTCAGGATGACATCAATGGCATCCAGGCCATCTATGGACC<br>841 TTCCCAAAATCCCATCCAGCCAATTGGCCCACAAACCCCAGAAGTGTGTGATAGTAAGCT<br>901 AACCTTTGATGCTATAACTACGATTCGGGGAGAAGTGATGTTCTTTAAAGACAGGTTCTA<br>961 CATGCGCATAAATCCTTACTACCCAGAAGCTGAGCTCAATTTCATTTCTATTTTCTGGCC<br>1021 ACAACTGCCAAATGGACTTCAAGCTGCTTATGAGGTTTCCCATAGAGATGAAGTCCGGTT<br>1081 TTTCAAAGGTAATAAGTACTGGGCTGTTAAGGGGCAGGATGTGCTCTACGGATACCCCAA<br>1141 GGACATCCACAGATCCTTCGGCTTTCCTAGCACTGTGAAGAATATCGATGCTGCTGTTTC<br>1201 TGAGGAAGATACTGGGAAAACGTACTTCTTTGTTGCTGACAAGTACTGGAGGTACGATGA<br>1261 ATATAAACGATCTATGGATGCAGGTTATCCCAAAATGATAGCAGATGACTTTCCTGGAAT<br>1321 TGGCGACAAAGTTGATGCTGTTTTTCAGAAAGATGGATTTTTCTATTTCTTTCACGGAAC<br>1381 AAGGCAATACAAATTTGATCCTAAAACTAAGAGAATTTTGACTCTCCAGAAAGCCAATAG<br>1441 CTGGTTCAACTGCAGAAAAAATTGACCATTACAAAGTTGAACAGAAAAATATATTTTGAG<br>1501 AACCCAAGGAAGATGTTTTCCTGAAGAGCCATACATTTAAATAAAATACAGAGCTGTTAC<br>1561 GTATAATCTTATTTATATCTCATTCTGCAAATATTTTTTACAATTTAGAATGCAATGTTT<br>1621 CATGTACTGCTATAATTTACTTCCACCAATGAGGGTGAAGGAAGGTCAAGTTCTGATTTA<br>1681 CGGATTCATATGGGCCAATTTTGCAAAGAGAGAAGCTCTTTTCCAAAGTAGAAGACTCTG<br>1741 ACATTGATACTGGAGAGCAGCCTCAGAGATGGAGAGAAAGAAACAAGAGATACGAGTCTT<br>1801 CACCAGAGGAAAAGCAGCTCAAGAACATCTGAGCTGCTACTAGTATTATCCTAATATGCA<br>1861 AGGGATAATTTTTGTTACGGAAAATAAAATCTTTTATGTTTGGAATAAAAAAAAAAAAAA<br>1921 AAA | SEQ ID NO:139 |
| | | 1 M L S L P L L L L L L W G M G S H S F P<br>1 ATGCTCAGCCTTCCTCTGCTGCTGCTGCTACTCTGGGGCATGGGCTCTCACAGCTTCCCA | SEQ ID NO:140 |

| Gene Name | GenBank Homology | DNA SEQUENCE / DEDUCED AMINO ACID SEQUENCE | SEQUENCE IDENTIFIER: |
|---|---|---|---|
| | | 21 T V P S E T R E E D V E M V Q K Y L E N<br>61 ACAGTGCCTTCAGAAACACGAGAAGAAGATGTGGAGATGGTCCAGAAATACCTGGAAAAC<br><br>41 Y Y N L K S D G E Q I E K Q R H R S P V<br>121 TACTACAACCTGAAGAGTGATGGAGAGCAAATTGAAAAGCAGAGACACAGGAGCCCAGTC<br><br>61 V E K L K Q M Q E F F G L K V T G K P D<br>181 GTTGAAAAGCTGAAGCAAATGCAGGAATTCTTTGGGCTGAAAGTGACTGGGAAGCCAGAT<br><br>81 A E T L N V M K Q P R C G V P D V A E F<br>241 GCTGAAACCCTGAATGTGATGAAGCAGCCCAGATGCGGGGTGCCTGATGTGGCTGAGTTT<br><br>101 V L T E G N P R W E N T H L T Y R I E N<br>301 GTCCTCACCGAAGGGAACCCTCGGTGGGAGAACACACACCTGACCTACAGGATTGAAAAT<br><br>121 Y T P D L P R A D V D Q A I E K A F Q L<br>361 TACACACCAGATTTACCAAGAGCAGATGTGGACCAGGCCATTGAGAAAGCCTTTCAACTC<br><br>141 W S N V S P L T F T K V S E G Q A D I M<br>421 TGGAGTAATGTCTCACCCCTGACCTTCACTAAAGTCTCTGAGGGTCAAGCGGACATAATG<br><br>161 I S F V R G D H R D N S P F D G P G G N<br>481 ATATCCTTTGTCAGGGGAGATCATCGTGACAATTCTCCCTTTGATGGACCTGGAGGAAAC<br><br>181 L A H A F Q P G P R I G G D A H F D E D<br>541 CTCGCCCATGCTTTTCAGCCAGGCCCACGTATTGGAGGGGATGCTCATTTTGATGAAGAT<br><br>201 E T W T S N F R N Y N L Y R V A A H E F<br>601 GAAACATGGACCAGCAATTTCAGAAATTACAACTTGTATCGAGTTGCAGCTCATGAATTT<br><br>221 G H S L G L S H S T D I G A L M Y P N Y<br>661 GGCCATTCCCTTGGACTCTCTCATTCTACTGATATTGGGGCTTTGATGTACCCCAACTAC<br><br>241 F F T G D V Q L S Q D D I N G I Q A I Y<br>721 TTCTTCACTGGTGATGTTCAGCTATCTCAGGATGACATCAATGGCATCCAGGCCATCTAT<br><br>261 G P S Q N P I Q P I G P Q T P E V C D S<br>781 GGACCTTCCCAAAATCCCATCCAGCCAATTGGCCCACAAACCCCAGAAGTGTGTGATAGT<br><br>281 K L T F D A I T T I R G E V M F F K D R<br>841 AAGCTAACCTTTGATGCTATAACTACGATTCGGGGAGAAGTGATGTTCTTTAAAGACAGG<br><br>301 F Y M R I N P Y Y P E A E L N F I S I F | |

| Gene Name | GenBank Homology | DNA SEQUENCE / DEDUCED AMINO ACID SEQUENCE | SEQUENCE IDENTIFIER: |
|---|---|---|---|
| | | 901 TTCTACATGCGCATAAATCCTTACTACCCAGAAGCTGAGCTCAATTTCATTTCTATTTTC<br><br>321 W P Q L P N G L Q A A Y E V S H R D E V<br>961 TGGCCACAACTGCCAAATGGACTTCAAGCTGCTTATGAGGTTTCCCATAGAGATGAAGTC<br><br>341 R F F K G N K Y W A V K G Q D V L Y G Y<br>1021 CGGTTTTTCAAAGGTAATAAGTACTGGGCTGTTAAGGGGCAGGATGTGCTCTACGGATAC<br><br>361 P K D I H R S F G F P S T V K N I D A A<br>1081 CCCAAGGACATCCACAGATCCTTCGGCTTTCCTAGCACTGTGAAGAATATCGATGCTGCT<br><br>381 V S E E D T G K T Y F F V A D K Y W R Y<br>1141 GTTTCTGAGGAAGATACTGGGAAAACGTACTTCTTTGTTGCTGACAAGTACTGGAGGTAC<br><br>401 D E Y K R S M D A G Y P K M I A D D F P<br>1201 GATGAATATAAACGATCTATGGATGCAGGTTATCCCAAAATGATAGCAGATGACTTTCCT<br><br>421 G I G D K V D A V F Q K D G F F Y F F H<br>1261 GGAATTGGCGACAAAGTTGATGCTGTTTTTCAGAAAGATGGATTTTTCTATTTCTTTCAC<br><br>441 G T R Q Y K F D P K T K R I L T L Q K A<br>1321 GGAACAAGGCAATACAAATTTGATCCTAAAACTAAGAGAATTTTGACTCTCCAGAAAGCC<br><br>461 N S W F N C R K N -<br>1381 AATAGCTGGTTCAACTGCAGAAAAAATTGA | |
| WBC031B06.bFSP_20 012510.abd | Homo sapiens progestin and adipoQ receptor family member XI (PAQR11) | 1 atggcggagg agccggggag gcgggaggcg ggaggcggga ggtgttgggg ccgttgaagc<br>61 ggcctccctc ccgcccccag ccgcccggtc tggccccagc cctgtcccga cccccggcct<br>121 ggcccactcc gaccctaccc ggccgaaggg ttccgctgga cacgcaggcg gcctccggag<br>181 cagcccaagc ccatgagggc cgcgcgcccg gccgccggtg ctgacgagac ggagctcctg<br>241 gcccccgagg aggagcagag gatcaatgcg gttcaagaat cgattccagc ggttcatgaa<br>301 ccatcgagct ccagccaatg gccgctacaa gccaacttgc tatgaacatg ctgctaactg<br>361 ttacacacac gcattcctca ttgttccggc catcgtgggc agtgccctcc tccatcggct<br>421 gtctgatgac tgctgggaaa agataacagc atggatttat ggaatgggac tctgtgccct<br>481 cttcatcgtt tctacagtat ttcacattgt atcatggaaa aagagccact taaggacagt<br>541 ggagcattgt tttcacatgt gtgatagaat ggttatctat ttcttcattg ctgcttctta<br>601 tgctccatgg ttaaatcttc gtgaacttgg acccctggca tctcatatgc gttggtttat<br>661 ctggctcatg gcagctggag gaaccattta tgtatttctc taccatgaaa aatataaggt<br>721 ggttgaactc tttttctatc tcacaatggg attctctcca gccttggtgg tgacatcaat<br>781 gaacaacacc gatggacttc aggaacttgc ctgtgggggc ttaatttatt gcttgggagt<br>841 tgtgttcttc aagagtgatg gcatcattcc atttgcccac gccatctggc acctgtttgt<br>901 ggccacggca gctgcagtgc attactacgc catttggaaa tacctttacc gaagtcctac | SEQ ID NO:141 |

| Gene Name | GenBank Homology | DNA SEQUENCE / DEDUCED AMINO ACID SEQUENCE | SEQUENCE IDENTIFIER: |
|---|---|---|---|
| | | 961 ggactttatg cggcatttat gaccaatctg tactaattct ccaaaccagt attatttcaa<br>1021 ttatggcact tgggagtggg gtgagagcta aacattgcac agggaaagaa aaaaaataac<br>1081 tgcactgact ttatatcttt tgaatataat tactgtgaaa gtataaaggc tgtgttctgg<br>1141 aattttctgc ctcacagcaa ataaataagg tagtgaatta attattcatt ccattccact<br>1201 atcatgaagg actctgaata gacttggcca actgatgttt acaaaccaga cttttatatt<br>1261 ttaattttac agattttact acatgatttt tctaaattac tatgtcaggt tgtaaaagtc<br>1321 agtgcaataa caaaccttcc tttttaagaa gaaaattgtt tctattactt tcccattcac<br>1381 taggtaaaga atcatggaca gaacttacac tactttttac catgtttcat cttggcataa<br>1441 catggttctt ttttaaatag aaactttagt tttttgtaaa tttttaaaaa aatatttcat<br>1501 tgatatgcat ctctgcaggt cctcattcat gttgtaaatt tttgcagcaa gcagtcaaca<br>1561 ttccacaaac gaacaaacat tatacctctt ctgatagttt tattaagcat ggagaaattg<br>1621 ccaattttta aaaactgcag ttttccaaac ttttctgcca acctcttact ctgaattcag<br>1681 tgctgctttg ggacatatac ttgacctagc ttggtttacc agtgatggaa aagtattttg<br>1741 atatcattaa ctttttcaaa agatccaact ttttctctat gcctttgcca cattctcttc<br>1801 agggtctctt tccacagtgg ataaatgttt tttctgtatt atgacagtat tgttgtgatg<br>1861 gccatctgct ggaaactcct gaagagcatt atgtattaca gtgagcagtt gtattgcctg<br>1921 tttggtgccc aatggttaag tcattgtcac ttagctttat attgtcagtt tgatatttat<br>1981 tttaaattgt ggaactagat gcataaattc acatttctgc ctttcatttg catcttctca<br>2041 tatattgtgt tttttttttt tttcctagaa aaaatattta aagcattgtt tgacaggtag<br>2101 aaactcatgt atctgtagtc catgagttat atcctggctc agtggagtga tatttatgta<br>2161 ttatttttac ttttctctca gtgtcttata ttaagattaa catgttgtta atagttgctt<br>2221 tgttgattaa tctctcttgt tggtgtttta ataaatgaaa taggcttgcc tttagatcgg<br>2281 gtgctgatat tgcctgtttc ctagtaatgg gctgatcaaa tgatcagtgg aattcttggt<br>2341 ttgatgataa ccttattaat tgaaattttt tactgatgtg gctttaaaag aggtttattt<br>2401 tgtatatgtt tagaactctc tgattttgat gaattatatg ggagtgagaa acagaagaag<br>2461 tggtatttgc tggcgagtta aataggcaag gtacccagtg ataacaccaa ccaaaccact<br>2521 cctatctgca tgattctgaa catctggatg cctgttgttt tactgtgtat attttatttt<br>2581 taatatatta actttgtgga ttcatttaag gtctactcaa aagtaacact gtccaaacca<br>2641 ctaatatgta tgtaaaaatt gtgctgtata ctacaataaa gttgttactt ggatttgttc<br>2701 caaaaaaaaa aaaaaa | |
| | | 1 M R F K N R F Q R F M N H R A P A N G R<br>1 ATGCGGTTCAAGAATCGATTCCAGCGGTTCATGAACCATCGAGCTCCAGCCAATGGCCGC<br><br>21 Y K P T C Y E H A A N C Y T H A F L I V<br>61 TACAAGCCAACTTGCTATGAACATGCTGCTAACTGTTACACACACGCATTCCTCATTGTT<br><br>41 P A I V G S A L L H R L S D D C W E K I<br>121 CCGGCCATCGTGGGCAGTGCCCTCCTCCATCGGCTGTCTGATGACTGCTGGGAAAAGATA<br><br>61 T A W I Y G M G L C A L F I V S T V F H<br>181 ACAGCATGGATTTATGGAATGGGACTCTGTGCCCTCTTCATCGTTTCTACAGTATTTCAC | SEQ ID NO:142 |

| Gene Name | GenBank Homology | DNA SEQUENCE / DEDUCED AMINO ACID SEQUENCE | SEQUENCE IDENTIFIER: |
|---|---|---|---|
| | | 81 I V S W K K S H L R T V E H C F H M C D<br>241 ATTGTATCATGGAAAAAGAGCCACTTAAGGACAGTGGAGCATTGTTTTCACATGTGTGAT<br><br>101 R M V I Y F F I A A S Y A P W L N L R E<br>301 AGAATGGTTATCTATTTCTTCATTGCTGCTTCTTATGCTCCATGGTTAAATCTTCGTGAA<br><br>121 L G P L A S H M R W F I W L M A A G G T<br>361 CTTGGACCCCTGGCATCTCATATGCGTTGGTTTATCTGGCTCATGGCAGCTGGAGGAACC<br><br>141 I Y V F L Y H E K Y K V V E L F F Y L T<br>421 ATTTATGTATTTCTCTACCATGAAAAATATAAGGTGGTTGAACTCTTTTTCTATCTCACA<br><br>161 M G F S P A L V V T S M N N T D G L Q E<br>481 ATGGGATTCTCTCCAGCCTTGGTGGTGACATCAATGAACAACACCGATGGACTTCAGGAA<br><br>181 L A C G G L I Y C L G V V F F K S D G I<br>541 CTTGCCTGTGGGGGCTTAATTTATTGCTTGGGAGTTGTGTTCTTCAAGAGTGATGGCATC<br><br>201 I P F A H A I W H L F V A T A A A V H Y<br>601 ATTCCATTTGCCCACGCCATCTGGCACCTGTTTGTGGCCACGGCAGCTGCAGTGCATTAC<br><br>221 Y A I W K Y L Y R S P T D F M R H L -<br>661 TACGCCATTTGGAAATACCTTTACCGAAGTCCTACGGACTTTATGCGGCATTTATGA | |
| WBC031C11.bFSP_20 012510.abd | Homo sapiens ribosomal protein L4, mRNA (cDNA clone MGC:87190 IMAGE:5259747). | 1 CTTTTCCTGTGGCAGCAGCCGGGCTGAGAGGAGCGTGGCCTTCTCCTCTCCCTGCCATGG<br>61 CGTGTGCTCGTCCACTGATATCAGTGTACTCCGAAAAGGGGAGTCCTCTGGCAAAAATG<br>121 TCACTTTGCCTGCTGTGTTCAAGGCTCCCATTCGGCCAGATATTGTCAACTTTGTTCACA<br>181 CCAACTTGCGCAAAAACAACAGACAGCCTTATGCCGTCAGCGAATTAGCAGGTCATCAAA<br>241 CCAGTGCTGAGTCTTGGGGTACTGGCAGAGCTGTGGCTCGGATTCCCAGAGTTCGAGGCG<br>301 GTGGTACTCACCGTTCTGGCCAGGGTGCTTTTGGAAACATGTGTCGTGGGGGCCGCATGT<br>361 TTGCACCAACCAAAACCTGGCGCCGTTGGCACCGCAGAGTGAACACAACACAGAAGCGAT<br>421 ATGCCATCTGCTCTGCCCTGGCTGCCTCAGCCTTACCAGCGTTGGTCATGTCTAAAGGTC<br>481 ATCGTATTGAGGAAGTTCCTGAACTTCCTTTGGTAGTTGAAGATAAAGTTGAAGGCTACA<br>541 AGAAGACCAAGGAAGCTGTTTTGCTCCTTAAGAAACTTAAAGCCTGGAATGATATCAAAA<br>601 AGGTCTATGCCTCTCAGCGAATGAGAGCTGGCAAAGGCAAAATGAGAAACCGTCGCCGTA<br>661 TCCAGCGCAGGGGCCCGTGCATCATCTATAATGAGGATAATGGTATCATCAAGGCCTTCA<br>721 GAAACATCCCTGGAATTACTCTGCTTAATGTAAGCAAGCTGAACATTTTGAAGCTTGCTC<br>781 CTGGTGGGCACGTGGACGTTTTTGCATTTGGACTGAAAGCGCTTTCCGCAAGTTAGATG<br>841 ATCTTTATGGCACTTGGCGTAAAGCTGCTTCCCTCAAGAGTAACTACAACCTTCCCATGC<br>901 ACAAGATGCTCAATACAGACCTTAGCCGAATATTGAAGAGCCCAGAGATCCAAAGAGCCC<br>961 TCCGAGCACCACGCAAGAAGATTCATCGCAGGGTCCTGAAGAAGAATCCACTGAAAAACC<br>1021 TGAGAATCATGTTGAAGCTAAACCCGTATGCGAAGACCATGCGCAGGAACACCATTCTTC | SEQ ID NO:143 |

| Gene Name | GenBank Homology | DNA SEQUENCE / DEDUCED AMINO ACID SEQUENCE | SEQUENCE IDENTIFIER: |
|---|---|---|---|
| | | 1081 GCCAGGCCAAGAATCACAAACTCCGGGTGGATAAGGCAGCAGCAGCACTAGAAGCCAAAT<br>1141 CAGATGAGAAGGGGGTTCCAGGCAAGAAGCCTGTGGTAGGGAAGAAAGCAAAGAAGGCTG<br>1201 TTGACGTTAAGAAGCAGAAGAAGCCTTTGGTGGAAAAAAGGCTGCAGCTACTAAGAAGC<br>1261 CAGCAGCTGAGAAGAAGCCTGCAGAAAAGAAACCCGCTGCAGAAGAAAAGAAGCCTGCTG<br>1321 CGTAAACTTGTTTGTTTATTTCATAAAGGTCACATCATTTTGGACAGCTCCTTTTGAATA<br>1381 AAGACCTGATTATACAGGCAGTGAGAAACAAAAAAAAAAAAAAAA | |
| | | 1 M A C A R P L I S V Y S E K G E S S G K<br>1 ATGGCGTGTGCTCGTCCACTGATATCAGTGTACTCCGAAAAGGGGGAGTCCTCTGGCAAA<br><br>21 N V T L P A V F K A P I R P D I V N F V<br>61 AATGTCACTTTGCCTGCTGTGTTCAAGGCTCCCATTCGGCCAGATATTGTCAACTTTGTT<br><br>41 H T N L R K N N R Q P Y A V S E L A G H<br>121 CACACCAACTTGCGCAAAAACAACAGACAGCCTTATGCCGTCAGCGAATTAGCAGGTCAT<br><br>61 Q T S A E S W G T G R A V A R I P R V R<br>181 CAAACCAGTGCTGAGTCTTGGGGTACTGGCAGAGCTGTGGCTCGGATTCCCAGAGTTCGA<br><br>81 G G G T H R S G Q G A F G N M C R G G R<br>241 GGCGGTGGTACTCACCGTTCTGGCCAGGGTGCTTTTGGAAACATGTGTCGTGGGGGCCGC<br><br>101 M F A P T K T W R R W H R R V N T T Q K<br>301 ATGTTTGCACCAACCAAAACCTGGCGCCGTTGGCACCGCAGAGTGAACACAACACAGAAG<br><br>121 R Y A I C S A L A A S A L P A L V M S K<br>361 CGATATGCCATCTGCTCTGCCCTGGCTGCCTCAGCCTTACCAGCGTTGGTCATGTCTAAA<br><br>141 G H R I E E V P E L P L V V E D K V E G<br>421 GGTCATCGTATTGAGGAAGTTCCTGAACTTCCTTTGGTAGTTGAAGATAAAGTTGAAGGC<br><br>161 Y K K T K E A V L L L K K L K A W N D I<br>481 TACAAGAAGACCAAGGAAGCTGTTTTGCTCCTTAAGAAACTTAAAGCCTGGAATGATATC<br><br>181 K K V Y A S Q R M R A G K G K M R N R R<br>541 AAAAAGGTCTATGCCTCTCAGCGAATGAGAGCTGGCAAAGGCAAAATGAGAAACCGTCGC<br><br>201 R I Q R R G P C I I Y N E D N G I I K A<br>601 CGTATCCAGCGCAGGGGCCCGTGCATCATCTATAATGAGGATAATGGTATCATCAAGGCC<br><br>221 F R N I P G I T L L N V S K L N I L K L<br>661 TTCAGAAACATCCCTGGAATTACTCTGCTTAATGTAAGCAAGCTGAACATTTTGAAGCTT | SEQ ID NO:144 |

| Gene Name | GenBank Homology | DNA SEQUENCE / DEDUCED AMINO ACID SEQUENCE | SEQUENCE IDENTIFIER: |
|---|---|---|---|
| | | 241 A P G G H V G R F C I W T E S A F R K L<br>721 GCTCCTGGTGGGCACGTGGGACGTTTTTGCATTTGGACTGAAAGCGCTTTCCGCAAGTTA<br><br>261 D D L Y G T W R K A A S L K S N Y N L P<br>781 GATGATCTTTATGGCACTTGGCGTAAAGCTGCTTCCCTCAAGAGTAACTACAACCTTCCC<br><br>281 M H K M L N T D L S R I L K S P E I Q R<br>841 ATGCACAAGATGCTCAATACAGACCTTAGCCGAATATTGAAGAGCCCAGAGATCCAAAGA<br><br>301 A L R A P R K K I H R R V L K K N P L K<br>901 GCCCTCCGAGCACCACGCAAGAAGATTCATCGCAGGGTCCTGAAGAAGAATCCACTGAAA<br><br>321 N L R I M L K L N P Y A K T M R R N T I<br>961 AACCTGAGAATCATGTTGAAGCTAAACCCGTATGCGAAGACCATGCGCAGGAACACCATT<br><br>341 L R Q A K N H K L R V D K A A A A L E A<br>1021 CTTCGCCAGGCCAAGAATCACAAACTCCGGGTGGATAAGGCAGCAGCAGCACTAGAAGCC<br><br>361 K S D E K G V P G K K P V V G K K A K K<br>1081 AAATCAGATGAGAAGGGGGTTCCAGGCAAGAAGCCTGTGGTAGGGAAGAAAGCAAAGAAG<br><br>381 A V D V K K Q K K P L V G K K A A A T K<br>1141 GCTGTTGACGTTAAGAAGCAGAAGAAGCCTTTGGTGGGAAAAAAGGCTGCAGCTACTAAG<br><br>401 K P A A E K K P A E K K P A A E E K K P<br>1201 AAGCCAGCAGCTGAGAAGAAGCCTGCAGAAAAGAAACCCGCTGCAGAAGAAAAGAAGCCT<br><br>421 A A -<br>1261 GCTGCGTAA | |
| WBC031D02.bFSP_20 012510.abd | Homo sapiens syntenin (sycl) mRNA. | 1 GGCACGAGGCGGGGGCGGTGCATGACGCGCCTCGGGGGCGGTCCTCGGGCGCGCACCGCT<br>61 CTCTTACACTCGGGTCTCGGAAGTCCGTGCCAGCGACCGAGGCGACGGCGAGCGGATCCC<br>121 AGCGGCCTAGAAAAATCCTGCAAAAATGTCTCTGTACCCATCTCTTGAAGACCTGAAGGT<br>181 AGACAAAGTAATTCAGGCTCAGACTGCCTATTCTGCAAACCCTGCCAACCCAGCAGTTTT<br>241 GTCTGAAGCTTCTGCTCCTCCCGTCTCTCAAGATGGAAATCTCTATCCTAAGTTGTATCC<br>301 GGAGCTCTCTCAGTACATGGGCCTGAGTTTAAACGATGAAGAAATACGTGCAAATATGGC<br>361 CATGATCCCTGGAGCACCAATTCAGGGGCAATTGGTAGCAAGACCTTCCAGTATGAACTA<br>421 TATGGTGGCTCCTGTAACCGGCAATGATGTTGGGATTCGTAGAGCCGAAATTAAGCAAGG<br>481 GATTCGTGAAGTCATTCTGTGTAAGGATCAAGATGGAAAAATTGGGCTCCGGCTTAAATC<br>541 AATAGATAACGGAGTATTTGTGCAGCTAGTCCAGGCAAATTCTCCAGCCTCGCTGGTTGG<br>601 CCTGAGGTTTGGGGACCAAGTACTCCAGATCAACGGGGAAAACTGTGCAGGCTGGAGCTC<br>661 TGATAAATCGCACAAGGTGCTCAAACAGGCTTTTGGAGAGAAGATTACTATGACTATTCG | SEQ ID NO:145 |

| Gene Name | GenBank Homology | DNA SEQUENCE / DEDUCED AMINO ACID SEQUENCE | SEQUENCE IDENTIFIER: |
|---|---|---|---|
| | | 721 TGACAGGCCCTTCGAACGGACAATTACCATGCATAAGGATAGTACTGGACATGTTGGTTT<br>781 TATCTTTAAAAATGGAAAAATAACATCCATAGTGAAAGATAGTTCTGCAGCCAGAAATGG<br>841 TCTCCTCACAAAACATAACATCTGCCGAGATCAATGGATAGAATGTCATTGGGCTGAAGG<br>901 ACTCTCAAATTGCAGACATACTGTCAACATCTGGGACTGTAGTTACTATTACAATCTGCC<br>961 CTGCTTTTATCTTTGAACATATTATTAAGCGGATGGCACCAAGCATTATGAAAAGCCTAA<br>1021 TGGACCACACCATTCCTGAGGTTTAAAATTCACGGCACCATGGAAATGTAGCTGAACGTC<br>1081 TCCAGTTTCCTTCTTTGGCAACTTCTGTATTATGCACGTGAAGCCTTCCCGGAGCCAGCG<br>1141 AGCATATGCTGCATGAGGACCTTTCTATCTTACATTATGGCTGGGGATCTTACTCTTTCA<br>1201 TCTGATACCTTGTTCAGATTTCAAAATAGTTGTAGCCTTATCCTGGTTTTACAGATGTGA<br>1261 ACTTTCAAGAGATTTACTGACTTTCCTAGAATAGTTTCTCTACTGGAAACCTGATGCTTT<br>1321 TATAAGCCATTGTGATTAGGATGACTGTTACAGGCTTAGCTTTGTGTGAAAACCAGTCAC<br>1381 CTTTCTCCTAGGTAATGAGTAGTGCTGTCATATTACTTTAGTTCTATAGCATACTTGCAT<br>1441 CTTTAACATGCTATCATAGTACATTTAGAATGATTGCCTTTGATTTTTTTTTTTAAATTC<br>1501 TGTGTGTGTGTGTGTAAAATGCCAATTAAGAACACTGGTTTCATTCCATTGTAAGCATTA<br>1561 GACAGTGTATGTAGGTTGCAAGAGATTGTGTAGATTATCACTAAATTTTAACTACCTTCA<br>1621 CTTAATATGCTTAAACTGTCGCCTTAACTATGCTAAGCATCTAGAATAAAAGCCAAAATG<br>1681 TAACTACTGCTGCCTTTCTGAAACCCACCATGCAGTTCTGTATTAACTGAAATGTTCAGC<br>1741 AGCCCAGAGCAGTTTACTGGGAGATACTGAGCTATAGCATAGCTGCTTAGTTGCATTTGA<br>1801 GATTTTCTAGTCATTGTGTAATGGAAACTTCTTTCTTCTAAAAGTTACTGGTATCACTTT<br>1861 CTGAGAAATGAATCACTATATATTTAATGTAAAAATTCTTATAGGAAATAGGACAAACTT<br>1921 CTGACTCCCTTCTTTCATTTTTAGATTAAGTGGGATAATACCTAATTTGGGCATTCTACT<br>1981 CTTAGCATTATGTAACCGTGCTACTTTGGCATGGTCATAGAATGTTTNCCTGGTAATTTG<br>2041 TTCCATGTCCTGACTCCTCCCTGCAAACAAAATGATAGTTGACACTTTATCCTGATTTTT<br>2101 TTCTTTTTTTGGTTTATGTCTATTCTAATTAAATATGTATAAATAAAGTTACATTTTAG<br>2161 TCTGTCAAAAAAAAAAAAAAAAAAAAAAAAAAAAAAA | |
| | | 1 M S L Y P S L E D L K V D K V I Q A Q T<br>1 ATGTCTCTGTACCCATCTCTTGAAGACCTGAAGGTAGACAAAGTAATTCAGGCTCAGACT<br><br>21 A Y S A N P A N P A V L S E A S A P P V<br>61 GCCTATTCTGCAAACCCTGCCAACCCAGCAGTTTTGTCTGAAGCTTCTGCTCCTCCCGTC<br><br>41 S Q D G N L Y P K L Y P E L S Q Y M G L<br>121 TCTCAAGATGGAAATCTCTATCCTAAGTTGTATCCGGAGCTCTCTCAGTACATGGGCCTG<br><br>61 S L N D E E I R A N M A M I P G A P I Q<br>181 AGTTTAAACGATGAAGAAATACGTGCAAATATGGCCATGATCCCTGGAGCACCAATTCAG<br><br>81 G Q L V A R P S S M N Y M V A P V T G N<br>241 GGGCAATTGGTAGCAAGACCTTCCAGTATGAACTATATGGTGGCTCCTGTAACCGGCAAT<br><br>101 D V G I R R A E I K Q G I R E V I L C K<br>301 GATGTTGGGATTCGTAGAGCCGAAATTAAGCAAGGGATTCGTGAAGTCATTCTGTGTAAG | SEQ ID NO:146 |

| Gene Name | GenBank Homology | DNA SEQUENCE / DEDUCED AMINO ACID SEQUENCE | SEQUENCE IDENTIFIER: |
|---|---|---|---|
| | | 121 D Q D G K I G L R L K S I D N G V F V Q<br>361 GATCAAGATGGAAAAATTGGGCTCCGGCTTAAATCAATAGATAACGGAGTATTTGTGCAG<br><br>141 L V Q A N S P A S L V G L R F G D Q V L<br>421 CTAGTCCAGGCAAATTCTCCAGCCTCGCTGGTTGGCCTGAGGTTTGGGGACCAAGTACTC<br><br>161 Q I N G E N C A G W S S D K S H K V L K<br>481 CAGATCAACGGGGAAAACTGTGCAGGCTGGAGCTCTGATAAATCGCACAAGGTGCTCAAA<br><br>181 Q A F G E K I T M T I R D R P F E R T I<br>541 CAGGCTTTTGGAGAGAAGATTACTATGACTATTCGTGACAGGCCCTTCGAACGGACAATT<br><br>201 T M H K D S T G H V G F I F K N G K I T<br>601 ACCATGCATAAGGATAGTACTGGACATGTTGGTTTTATCTTTAAAAATGGAAAAATAACA<br><br>221 S I V K D S S A A R N G L L T K H N I C<br>661 TCCATAGTGAAAGATAGTTCTGCAGCCAGAAATGGTCTCCTCACAAAACATAACATCTGC<br><br>241 R D Q W I E C H W A E G L S N C R H T V<br>721 CGAGATCAATGGATAGAATGTCATTGGGCTGAAGGACTCTCAAATTGCAGACATACTGTC<br><br>261 N I W D C S Y Y Y N L P C F Y L -<br>781 AACATCTGGGACTGTAGTTACTATTACAATCTGCCCTGCTTTTATCTTTGA | |
| WBC031F04.bFSP_20<br><br>012510.abd | Homo sapiens mRNA; cDNA DKFZp686K06110 (from clone DKFZp686K06110). | 1 CTGATGAAACAGCAAAAGCTAGATATTGTTTCTTCTATACATAGAAGGAAATAGCTTCTT<br>61 GCAACAGGAACAATTCAGGAGAAAACAGTTGTTTTCTCCCCTTTAGATGATAGGTTGGGA<br>121 AGCAGGGTTGGCTCCGTCATAATGATTTCTCCTAAACACTTCTCAGGTTTGGCCTATGCA<br>181 TGTGTGTTATAGATTGGGGATAGTTTGAAAACTCATTCTGTAGTATTATTCCTATGGGCA<br>241 GGGTGGTGAGTAGTGTGTAAATTCTTTGTCTTCTTTATGGGTCAGTATATCCACAGGCAC<br>301 ATTTTCATGCTGCTTTGTCACTGAAAGTAAGGCAGCTGCAGTGGCAGGACCCTGTTCTAG<br>361 GACAGTTGATTGCCTCTATGATGAGACCTCAGAATTTCCTCTTTCCTAGTGCCCAAGACA<br>421 AGGAGCTTTGCTGATGTATTTCTGATAGCAGAAAAGGCCATTGAGCTTAGTGGTTGGTAC<br>481 CATATGGCAATTTACAATCTGGGAAATATTCTGATGATACATACTTTTCGAAGAATGCTA<br>541 GTTAGAATATTTAGATAGGACTCCTGAACTTTCTTTGAGAAGATGTTGATGACAAGAACT<br>601 AAAATCAGCCTAATCTGGGTCACATCCCAGGAAGGATAAAGGTCTATAGGTGTGGAAGGC<br>661 TCTTTCTGGTCCTTTCAGTGAGCTATTTCCAATAGAGCCCGCCTATGCAGAGCTTCCCAC<br>721 TCTTGTGCATAGGATGTGGTTAAGCTCATGTCTCAGTGCCACAGTAGGTAGAGGATGCCA<br>781 TTGCAATGTAAAAGGGCTATAAATAAAGCTCTCTTGTTTTTCCATAAAACAGTCTGCTCT<br>841 AGAACAGATTATAAAGGGGCCAGAAGGAATCTCTTCTCATTTTCTTATTGAATAGAGAGT<br>901 GTATAAATTATTCCCTTACATAAAGGACAATTACTTTTTCTATATAGATCATATATAGAA<br>961 ATAGAGCTATAGAAAAAGTAATTAATTCTATATGATATGGCCTCAATTTTTAATTTTTAA | SEQ ID NO:147 |

| Gene Name | GenBank Homology | DNA SEQUENCE / DEDUCED AMINO ACID SEQUENCE | SEQUENCE IDENTIFIER: |
|---|---|---|---|
| | | 1021 ATTGTTTTTATATGAAATAGTCTTAATTTTACTCTTTTTGCAGTTCACACAAAGATGAAT<br>1081 TCCTGCACTTCAAGATTACATTGTATATGTGATTCCTTGCTTCATTTTAAGGACTATGTT<br>1141 GTTTTCTTAAATGTGTTGATGAGTCTTTATAACCTGTTTTAATAATGTTAAATTGAATTC<br>1201 ATTTTTCTTATTGTTAATACAAAAACAGTAAATTTGAAGACTGAGAACCATATAAGAGGG<br>1261 TGATTGCACTTATTATGAAAAGATTTGAATCCTTCTTTGTGTGTGTGGGTGTGACCAGCC<br>1321 TTGCTATCAATTTCTTCTGTCATACACTGCCAAATTTTCTATTTAAGTATTGTGAAAACT<br>1381 CTGTTTTTTTCAGGTCGAATGAGTGATTTGAGTGTAATTGGTCATCCAATAGATTCAGAA<br>1441 TCTAAAGAAGATGAACCTTGTAGTGAAGAAACAGATCCAGTGCATGATCTAATGGCTGAA<br>1501 ATTTTACCTGAATTCCCTGACATAATTGAAATAGACCTATACCACAGTGAAGAAAATGAA<br>1561 GAAGAAGAAGAAGAGTGTGCAAATGCTACTGATGTGACAACCACCCCATCTGTGCAGTAC<br>1621 ATAAATGGGAAGCATCTCGTTACCACTGTGCCCAAGGACCCAGAAGCTGCAGAAGCTAGG<br>1681 CGTGGCCAGTTTGAAAGTGTTGCACCTTCTCAAAATTTCTCGGACAGCTCTGAAAGTGAT<br>1741 ACTCATCCATTTGTAATAGCCAAAACGGAATTGTCTACTGCTGTGCAACCTAATGAATCT<br>1801 ACAGAAACAACTGAGTCTCTTGAAGTTACATGGAAGCCTGAGACTTACCCTGAAACATCA<br>1861 GAACATTTTTCAGGTGGTGAGCCTGATGTTTTCCCCACAGTCCCATTCCATGAGGAATTT<br>1921 GAAAGTGGAACAGCCAGAAAAGGGGCAGAATCAGTCACAGAGAGAGATACTGAAGTTGGT<br>1981 CATCAGGCACATGAACATACTGAACCTGTATCTCTGTTTCCTGAAGAGTCTTCAGGGGAG<br>2041 ATTGCCATTGACCAAGAATCTCAGAAAATAGCCTTTGCAAGGGCTACAGAAGTAACATTT<br>2101 GGTGAAGAGGTAGAAAAAAGTACTTCTGTCACATACACTCCCACTATAGTTCCAAGTTCT<br>2161 GCATCAGCATATGTTTCAGAGGAAGAAGCAGTTACCCTAATAGGAAATCCTTGGCCAGAT<br>2221 GACCTGTTGTCTACCAAAGAAAGCTGGGTAGAAGCAACTCCTAGACAAGTTGTAGAGCTC<br>2281 TCAGGGAGTTCTTCGATTCCAATTACAGAAGGCTCTGGAGAAGCAGAAGAAGATGAAGAT<br>2341 ACAATGTTCACCATGGTAACTGATTTATCACAGAGAAATACTACTGATACACTCATTACT<br>2401 TTAGACACTAGCAGGATAATCACAGAAAGCTTTTTTGAGGTTCCTGCAACCACCATTTAT<br>2461 CCAGTTTCTGAACAACCTTCTGCAAAAGTGGTGCCTACCAAGTTTGTAAGTGAAACAGAC<br>2521 ACTTCTGAGTGGATTTCCAGTACCACTGTTGAGGAAAAGAAAAGGAAGGAGGAGGAGGGA<br>2581 ACTACAGGTACGGCTTCTACATTTGAGGTATATTCATCTACACAGAGATCGGATCAATTA<br>2641 ATTTTACCCTTTGAATTAGAAAGTCCAAATGTAGCTACATCTAGTGATTCAGGTACCAGG<br>2701 AAAAGTTTTATGTCCTTGACAACACCAACACGGTCTGAAAGGGAAATGACAGATTCTACT<br>2761 CCTGTCTTTACAGAAACAAATACATTAGAAAATTTGGGGGCACAGACCACTGAGCACAGC<br>2821 AGTATCCATCAACCTGGGGTTCAGGAAGGGCTGACCACTCTCCCACATAGTCCTGCCTCT<br>2881 GTCTTTATGGAGCAGGGCTCTGGAGAAGCTGCTGCCGACCCAGAAACCACCACTGTTTCT<br>2941 TCATTTTCATTAAACGTAGAGTATGCAATTCAAGCCGAAAAGGAAGTAGCTGGCACTTTG<br>3001 TCTCCGCATGTGGAAACTACATTCTCCACTGAGCCAACAGGACTGGTTTTGAGTACAGTA<br>3061 ATGGACAGAGTAGTTGCTGAAAATATAACCCAAACATCCAGGGAAATAGCGATTTCAGAG<br>3121 CGATTAGGAGAACCAAATTATGGGGCAGAAATAAGGGGCTTTTCCACAGGTTTTCCTTTG<br>3181 GAGGAAGATTTCAGTGGCGACTTTAGAGAATACTCAACAGTGTCTCATCCCATAGCAAAA<br>3241 GAAGAAACGGTAATGATGGAAGGCTCTGGAGATGCAGCATTTAGGGACACCCAGACTTCA<br>3301 CCATCTACAGTACCTACTTCAGTTCACATCAGTCACATATCTGACTCAGAAGGACCCAGT<br>3361 AGCACCATGGTCAGCACTTCAGCCTTCCCCTGGGAAGAGTTTACATCCTCAGCTGAGGGC<br>3421 TCAGGTGAGCAACTGGTCACAGTCAGCAGCTCTGTTGTTCCAGTGCTTCCCAGTGCTGTG<br>3481 CAAAAGTTTTCTGGTACAGCTTCCTCCATTATCGACGAAGGATTGGGAGAAGTGGGTACT<br>3541 GTCAATGAAATTGATAGAAGATCCACCATTTTACCAACAGCAGAAGTGGAAGGTACGAAA | |

| Gene Name | GenBank Homology | DNA SEQUENCE / DEDUCED AMINO ACID SEQUENCE | SEQUENCE IDENTIFIER: |
|---|---|---|---|
| | | 3601 GCTCCAGTAGAGAAGGAGGAAGTAAAGGTCAGTGGCACAGTTTCAACAAACTTTCCCCAA<br>3661 ACTATAGAGCCAGCCAAATTATGGTCTAGGCAAGAAGTCAACCCTGTAAGACAAGAAATT<br>3721 GAAAGTGAAACAACATCAGAGGAACAAATTCAAGAAGAAAAGTCATTTGAATCCCCTCAA<br>3781 AACTCTCCTGCAACAGAACAAACAATCTTTGATTCACAGACATTTACTGAAACTGAACTC<br>3841 AAAACCACAGATTATTCTGTACTAACAACAAAGAAAACTTACAGTGATGATAAAGAAATG<br>3901 AAGGAGGAAGACACTTCTTTAGTTAACATGTCTACTCCAGATCCAGATGCAAATGGCTTG<br>3961 GAATCTTACACAACTCTCCCTGAAGCTACTGAAAAGTCACATTTTTTCTTAGCTACTGCA<br>4021 TTAGTAACTGAATCTATACCAGCTGAACATGTAGTCACAGATTCACCAATCAAAAAGGAA<br>4081 GAAAGTACAAAACATTTTCCGAAAGGCATGAGGCCAACAATTCAAGAGTCAGATACTGAG<br>4141 CTCTTATTCTCTGGACTGGGATCAGGAGAAGAAGTTTTACCTACTCTACCAACAGAGTCA<br>4201 GTGAATTTTACTGAAGTGGAACAAATCAATAACACATTATATCCCCACACTTCTCAAGTG<br>4261 GAAAGTACCTCAAGTGACAAAATTGAAGACTATAACAGAATGGAAAATGTGGCAAAAGAA<br>4321 GTTGGACCACTCGTATCTCAAACAGACATCTTTGAAGGTAGTGGGTCAGTAACCAGCACA<br>4381 ACATTAATAGAAATTTTAAGTGACACTGGAGCAGAAGGACCCACGGTGGCACCTCTCCCT<br>4441 TTCTCCACGGACATCGGACATCCTCAAAATCAGACTGTCAGGTGGGCAGAAGAAATCCAG<br>4501 ACTAGTAGACCACAAACCATAACTGAACAAGACTCTAACAAGAATTCTTCAACAGCAGAA<br>4561 ATTAACGAAACAACAACCTCATCTACTGATTTTCTGGCTAGAGCTTATGGTTTTGAAATG<br>4621 GCCAAAGAATTTGTTACATCAGCACCAAAACCATCTGACTTGTATTATGAACCTTCTGGA<br>4681 GAAGGATCTGGAGAAGTGGATATTGTTGATTCATTTCACACTTCTGCAACTACTCAGGCA<br>4741 ACCAGACAAGAAAGCAGCACCACATTTGTTTCTGATGGGTCCCTGGAAAAACATCCTGAG<br>4801 GTGCCAAGCGCTAAAGCTGTTACTGCTGATGGATTCCCAACAGTTTCAGTGATGCTGCCT<br>4861 CTTCATTCAGAGCAGAACAAAAGCTCCCCTGATCCAACTAGCACACTGTCAAATACAGTG<br>4921 TCATATGAGAGGTCCACAGACGGTAGTTTCCAAGACCGTTTCAGGGAATTCGAGGATTCC<br>4981 ACCTTAAAACCTAACAGAAAAAAACCCACTGAAAATATTATCATAGACCTGGACAAAGAG<br>5041 GACAAGGATTTAATATTGACAATTACAGAGAGTACCATCCTTGAAATTCTACCTGAGCTG<br>5101 ACATCGGATAAAAATACTATCATAGATATTGATCATACTAAACCTGTGTATGAAGACATT<br>5161 CTTGGAATGCAAACAGATATAGATACAGAGGTACCATCAGAACCACATGACAGTAATGAT<br>5221 GAAAGTAATGATGACAGCACTCAAGTTCAAGAGATCTATGAGGCAGCTGTCAACCTTTCT<br>5281 TTAACTGAGGAAACATTTGAGGGCTCTGCTGATGTTCTGGCTAGCTACACTCAGGCAACA<br>5341 CATGATGAATCAATGACTTATGAAGATAGAAGCCAACTAGATCACATGGGCTTTCACTTC<br>5401 ACAACTGGGATCCCTGCTCCTAGCACAGAAACAGAATTAGACGTTTTACTTCCCACGGCA<br>5461 ACATCCCTGCCAATTCCTCGTAAGTCTGCCACAGTTATTCCAGAGATTGAAGGAATAAAA<br>5521 GCTGAAGCAAAAGCCCTGGATGACATGTTTGAATCAAGCACTTTGTCTGATGGTCAAGCT<br>5581 ATTGCAGACCAAAGTGAAATAATACCAACATTGGGCCAATTTGAAAGGACTCAGGAGGAG<br>5641 TATGAAGACAAAAAACATGCTGGTCCTTCTTTTCAGCCAGAATTCTCTTCAGGAGCTGAG<br>5701 GAGGCATTAGTAGACCATACTCCCTATCTAAGTATTGCTACTACCCACCTTATGGATCAG<br>5761 AGTGTAACAGAGGTGCCTGATGTGATGGAAGGATCCAATCCCCCATATTACACTGATACA<br>5821 ACATTAGCAGTTTCAACATTTGCGAAGTTGTCTTCTCAGACACCATCATCTCCCCTCACT<br>5881 ATCTACTCAGGCAGTGAAGCCTCTGGACACACAGAGATCCCCCAGCCCAGTGCTCTGCCA<br>5941 GGAATAGACGTCGGCTCATCTGTAATGTCCCCACAGGATTCTTTTAAGGAAATTCATGTA<br>6001 AATATTGAAGCGACTTTCAAACCATCAAGTGAGGAATACCTTCACATAACTGAGCCTCCC<br>6061 TCTTTATCTCCTGACACAAAATTAGAACCTTCAGAAGATGATGGTAAACCTGAGTTATTA<br>6121 GAAGAAATGGAAGCTTCTCCCACAGAACTTATTGCTGTGGAAGGAACTGAGATTCTCCAA | |

| Gene Name | GenBank Homology | DNA SEQUENCE / DEDUCED AMINO ACID SEQUENCE | SEQUENCE IDENTIFIER: |
|---|---|---|---|
| | | 6181 GATTTCCAAAACAAAACCTATGGTCAAGTTTCTGGAGAAGCAATCAAGATGTTTCCCACC<br>6241 ATTAAAACACCTGAGGCTGGAACTGTTATTACAACTGCCGATGAAATTGAATTAGAAGGT<br>6301 GCTACACAGTGGCCACACTCTACTTCTGCTTCTGCCACCTATGGGGTCGAGGCAGGTGTG<br>6361 GTGCCTTGGCTAAGTCCACAGACTTCTGAGAGGCCCACGCTTTCTTCTTCTCCAGAAATA<br>6421 AACCCTGAAACTCAAGCAGCTTTAATCAGAGGGCAGGATTCCACGATAGCAGCATCAGAA<br>6481 CAGCAAGTGGCAGCGAGAATTCTTGATTCCAATAATCAGGCAACTGTAAGCACTGCAGAG<br>6541 TTAAATACTGAGCTTGCAACACCATCATTTTCCCTTCTGGAAACTTCTAATGAAACCGAT<br>6601 TTCCTGATTGGCATTAATGAAGAGTCAGTGGAGGGCACAGCAGTCTATTTACCAGGACCA<br>6661 GATCGTTGCAAAACGAACCCGTGCCTTAACGGAGGCACCTGTTATCCTACTGAAACTTCC<br>6721 TACGTGTGCACCTGTGTGCCAGGATACAGCGGCGACCAGTGTGAGCTTGATTTTGATGAA<br>6781 TGTCACTCTAACCCCTGTCGCAATGGAGCCACGTGCATTGACGGTTTCAATACGTTCAGG<br>6841 TGCCTCTGCCTTCCGAGCTACATTGGTGCACTTTGTGAGCAAGACACCGAGACATGTGAC<br>6901 TACGGCTGGCACAAATTCCAAGGGCAGTGCTACAAGTACTTTGCCCACCGCCGCACGTGG<br>6961 GATGCAGCTGAGCGAGAATGCCGGCTGCAGGGCGCCCATCTCACAAGCATCCTGTCTCAT<br>7021 GAAGAACAAATGTTTGTGAATCGTGTGGGCCATGACTATCAGTGGATAGGCCTCAATGAC<br>7081 AAGATGTTTGAGCATGACTTCCGTTGGACTGATGGCAGCACCCTGCAATATGAGAACTGG<br>7141 AGGCCCAACCAGCCAGACAGCTTTTTTTCTGCTGGAGAAGATTGTGTCGTAATCATTTGG<br>7201 CACGAGAACGGCCAGTGGAATGATGTTCCCTGCAATTACCATCTCACCTATACTTGCAAG<br>7261 AAAGGAACAGTTGCTTGCGGCCAGCCCCCAGTTGTAGAAAATGCCAAGACCTTTGGAAAG<br>7321 ATGAAACCTCGTTATGAAATCAACTCCCTGATTAGATATCACTGTAAAGATGGTTTCATT<br>7381 CAACGCCACCCTTCCACTATCCGTTGCCTAGGAAATGGAAAATGGGCCATGCCTAAAATT<br>7441 ACCTGCATGAACCCATCTGCATACCAAAGGACTTATTCTATGAAATACTTTAAAAATTCC<br>7501 TCATCAGCAAAGGACAATTCAATAAATACATCCAAACATGATCATCGTTGGAGCCGGAGG<br>7561 TGGCAGGAGTCGAGGCGCTGATCCCTAAAATGGCGAACATGTGTTTTCATCATTTCAGCC<br>7621 AAAGTCCTAACTTCCTGTGCCTTTCCTATCACCTCGAGAAGTAATTATCAGTTGGTTTGG<br>7681 ATTTTTGGACCACCGTTCAGTCATTTTGGGTTGCCGTGCTCCCAAAACATTTTAAATGAA<br>7741 AGTATTGGCATTCAAAAAGACAGCAGACAAAATGAAAGAAAATGAGAGCAGAAAGTAAGC<br>7801 ATTTCTAGCCTATCTAATTTCTTTAGTTTTCTATTTGCCTCCAGTGCAGTCCATTTCCTA<br>7861 ATGTATACCAGCCTACTGTACTATTTAAAAAGCTCAATTTCAGCACCAATGGCAATGTAA<br>7921 ATAAGATGATTTAATGTTGATTTTAATCCTGTATATAAAATAAAAAGTCATACTGAGTTC<br>7981 GGGCGTATTTAATGATGATTATGGAGCCTTAGAGGTCTTTAATCATTGGTTCGGCTGCTT<br>8041 TTTGTACTTTGTTTAGGCTGGAAACGGTTTCACTTGCCCTTTGTCAGCAAGACTGAGGAC<br>8101 GGCTTTTCCTGGACAACTAACAAACAAAAGTCTTGTAGGTCACTGCGCCATCTCAGCCAT<br>8161 AGTAGTTGCAGTTTGCTTCCATGTGATTCTGAAGCCTGGCCAATGGGATCCGGACAAAAG<br>8221 TAGGGACTGCAATGTGAAACACCTCTTTGCTGCATTCTTTTTCTTCGCTTACAAGAAAGG<br>8281 CCTGAATGGAGGACTTTTTTATGACCAGGAGCAGTTTTTAGGGGTCAAAGTGCTAATTAT<br>8341 TAACTCAACCAGGTCTACTTTTTAATGGCTTTCATAACACTAACTGGCAAGATTACAGAT<br>8401 CAACACATTTCAAATGGATAGAGACCTAGGACTCTGGAGGGTGGGGGATTGTTAAAAACA<br>8461 C | |
| | | 1 M S D L S V I G H P I D S E S K E D E P<br>1 ATGAGTGATTTGAGTGTAATTGGTCATCCAATAGATTCAGAATCTAAAGAAGATGAACCT | SEQ ID NO:148 |

| Gene Name | GenBank Homology | DNA SEQUENCE / DEDUCED AMINO ACID SEQUENCE | SEQUENCE IDENTIFIER: |
|---|---|---|---|
| | | 21 C S E E T D P V H D L M A E I L P E F P<br>61 TGTAGTGAAGAAACAGATCCAGTGCATGATCTAATGGCTGAAATTTTACCTGAATTCCCT<br><br>41 D I I E I D L Y H S E E N E E E E E E C<br>121 GACATAATTGAAATAGACCTATACCACAGTGAAGAAAATGAAGAAGAAGAAGAAGAGTGT<br><br>61 A N A T D V T T T P S V Q Y I N G K H L<br>181 GCAAATGCTACTGATGTGACAACCACCCCATCTGTGCAGTACATAAATGGGAAGCATCTC<br><br>81 V T T V P K D P E A A E A R R G Q F E S<br>241 GTTACCACTGTGCCCAAGGACCCAGAAGCTGCAGAAGCTAGGCGTGGCCAGTTTGAAAGT<br><br>101 V A P S Q N F S D S S E S D T H P F V I<br>301 GTTGCACCTTCTCAAAATTTCTCGGACAGCTCTGAAAGTGATACTCATCCATTTGTAATA<br><br>121 A K T E L S T A V Q P N E S T E T T E S<br>361 GCCAAAACGGAATTGTCTACTGCTGTGCAACCTAATGAATCTACAGAAACAACTGAGTCT<br><br>141 L E V T W K P E T Y P E T S E H F S G G<br>421 CTTGAAGTTACATGGAAGCCTGAGACTTACCCTGAAACATCAGAACATTTTTCAGGTGGT<br><br>161 E P D V F P T V P F H E E F E S G T A R<br>481 GAGCCTGATGTTTTCCCCACAGTCCCATTCCATGAGGAATTTGAAAGTGGAACAGCCAGA<br><br>181 K G A E S V T E R D T E V G H Q A H E H<br>541 AAAGGGGCAGAATCAGTCACAGAGAGAGATACTGAAGTTGGTCATCAGGCACATGAACAT<br><br>201 T E P V S L F P E E S S G E I A I D Q E<br>601 ACTGAACCTGTATCTCTGTTTCCTGAAGAGTCTTCAGGGGAGATTGCCATTGACCAAGAA<br><br>221 S Q K I A F A R A T E V T F G E E V E K<br>661 TCTCAGAAAATAGCCTTTGCAAGGGCTACAGAAGTAACATTTGGTGAAGAGGTAGAAAAA<br><br>241 S T S V T Y T P T I V P S S A S A Y V S<br>721 AGTACTTCTGTCACATACACTCCCACTATAGTTCCAAGTTCTGCATCAGCATATGTTTCA<br><br>261 E E E A V T L I G N P W P D D L L S T K<br>781 GAGGAAGAAGCAGTTACCCTAATAGGAAATCCTTGGCCAGATGACCTGTTGTCTACCAAA<br><br>281 E S W V E A T P R Q V V E L S G S S S I<br>841 GAAAGCTGGGTAGAAGCAACTCCTAGACAAGTTGTAGAGCTCTCAGGGAGTTCTTCGATT<br><br>301 P I T E G S G E A E E D E D T M F T M V | |

| Gene Name | GenBank Homology | DNA SEQUENCE / DEDUCED AMINO ACID SEQUENCE | SEQUENCE IDENTIFIER: |
|---|---|---|---|
| | | 901 CCAATTACAGAAGGCTCTGGAGAAGCAGAAGAAGATGAAGATACAATGTTCACCATGGTA<br><br>321 T D L S Q R N T T D T L I T L D T S R I<br>961 ACTGATTTATCACAGAGAAATACTACTGATACACTCATTACTTTAGACACTAGCAGGATA<br><br>341 I T E S F F E V P A T T I Y P V S E Q P<br>1021 ATCACAGAAAGCTTTTTTGAGGTTCCTGCAACCACCATTTATCCAGTTTCTGAACAACCT<br><br>361 S A K V V P T K F V S E T D T S E W I S<br>1081 TCTGCAAAAGTGGTGCCTACCAAGTTTGTAAGTGAAACAGACACTTCTGAGTGGATTTCC<br><br>381 S T T V E E K K R K E E E G T T G T A S<br>1141 AGTACCACTGTTGAGGAAAAGAAAAGGAAGGAGGAGGAGGGAACTACAGGTACGGCTTCT<br><br>401 T F E V Y S S T Q R S D Q L I L P F E L<br>1201 ACATTTGAGGTATATTCATCTACACAGAGATCGGATCAATTAATTTTACCCTTTGAATTA<br><br>421 E S P N V A T S S D S G T R K S F M S L<br>1261 GAAAGTCCAAATGTAGCTACATCTAGTGATTCAGGTACCAGGAAAAGTTTTATGTCCTTG<br><br>441 T T P T R S E R E M T D S T P V F T E T<br>1321 ACAACACCAACACGGTCTGAAAGGGAAATGACAGATTCTACTCCTGTCTTTACAGAAACA<br><br>461 N T L E N L G A Q T T E H S S I H Q P G<br>1381 AATACATTAGAAAATTTGGGGGCACAGACCACTGAGCACAGCAGTATCCATCAACCTGGG<br><br>481 V Q E G L T T L P H S P A S V F M E Q G<br>1441 GTTCAGGAAGGGCTGACCACTCTCCCACATAGTCCTGCCTCTGTCTTTATGGAGCAGGGC<br><br>501 S G E A A A D P E T T T V S S F S L N V<br>1501 TCTGGAGAAGCTGCTGCCGACCCAGAAACCACCACTGTTTCTTCATTTTCATTAAACGTA<br><br>521 E Y A I Q A E K E V A G T L S P H V E T<br>1561 GAGTATGCAATTCAAGCCGAAAAGGAAGTAGCTGGCACTTTGTCTCCGCATGTGGAAACT<br><br>541 T F S T E P T G L V L S T V M D R V V A<br>1621 ACATTCTCCACTGAGCCAACAGGACTGGTTTTGAGTACAGTAATGGACAGAGTAGTTGCT<br><br>561 E N I T Q T S R E I A I S E R L G E P N<br>1681 GAAAATATAACCCAAACATCCAGGGAAATAGCGATTTCAGAGCGATTAGGAGAACCAAAT<br><br>581 Y G A E I R G F S T G F P L E E D F S G<br>1741 TATGGGGCAGAAATAAGGGGCTTTTCCACAGGTTTTCCTTTGGAGGAAGATTTCAGTGGC | |

| Gene Name | GenBank Homology | DNA SEQUENCE / DEDUCED AMINO ACID SEQUENCE | SEQUENCE IDENTIFIER: |
|---|---|---|---|
| | | 601 D F R E Y S T V S H P I A K E E T V M M<br>1801 GACTTTAGAGAATACTCAACAGTGTCTCATCCCATAGCAAAAGAAGAAACGGTAATGATG<br><br>621 E G S G D A A F R D T Q T S P S T V P T<br>1861 GAAGGCTCTGGAGATGCAGCATTTAGGGACACCCAGACTTCACCATCTACAGTACCTACT<br><br>641 S V H I S H I S D S E G P S S T M V S T<br>1921 TCAGTTCACATCAGTCACATATCTGACTCAGAAGGACCCAGTAGCACCATGGTCAGCACT<br><br>661 S A F P W E E F T S S A E G S G E Q L V<br>1981 TCAGCCTTCCCCTGGGAAGAGTTTACATCCTCAGCTGAGGGCTCAGGTGAGCAACTGGTC<br><br>681 T V S S S V V P V L P S A V Q K F S G T<br>2041 ACAGTCAGCAGCTCTGTTGTTCCAGTGCTTCCCAGTGCTGTGCAAAAGTTTTCTGGTACA<br><br>701 A S S I I D E G L G E V G T V N E I D R<br>2101 GCTTCCTCCATTATCGACGAAGGATTGGGAGAAGTGGGTACTGTCAATGAAATTGATAGA<br><br>721 R S T I L P T A E V E G T K A P V E K E<br>2161 AGATCCACCATTTTACCAACAGCAGAAGTGGAAGGTACGAAAGCTCCAGTAGAGAAGGAG<br><br>741 E V K V S G T V S T N F P Q T I E P A K<br>2221 GAAGTAAAGGTCAGTGGCACAGTTTCAACAAACTTTCCCCAAACTATAGAGCCAGCCAAA<br><br>761 L W S R Q E V N P V R Q E I E S E T T S<br>2281 TTATGGTCTAGGCAAGAAGTCAACCCTGTAAGACAAGAAATTGAAAGTGAAACAACATCA<br><br>781 E E Q I Q E E K S F E S P Q N S P A T E<br>2341 GAGGAACAAATTCAAGAAGAAAAGTCATTTGAATCCCCTCAAAACTCTCCTGCAACAGAA<br><br>801 Q T I F D S Q T F T E T E L K T T D Y S<br>2401 CAAACAATCTTTGATTCACAGACATTTACTGAAACTGAACTCAAAACCACAGATTATTCT<br><br>821 V L T T K K T Y S D D K E M K E E D T S<br>2461 GTACTAACAACAAAGAAAACTTACAGTGATGATAAAGAAATGAAGGAGGAAGACACTTCT<br><br>841 L V N M S T P D P D A N G L E S Y T T L<br>2521 TTAGTTAACATGTCTACTCCAGATCCAGATGCAAATGGCTTGGAATCTTACACAACTCTC<br><br>861 P E A T E K S H F F L A T A L V T E S I<br>2581 CCTGAAGCTACTGAAAAGTCACATTTTTTCTTAGCTACTGCATTAGTAACTGAATCTATA | |

| Gene Name | GenBank Homology | DNA SEQUENCE / DEDUCED AMINO ACID SEQUENCE | SEQUENCE IDENTIFIER: |
|---|---|---|---|
| | | 881 P A E H V V T D S P I K K E E S T K H F<br>2641 CCAGCTGAACATGTAGTCACAGATTCACCAATCAAAAAGGAAGAAAGTACAAAACATTTT<br><br>901 P K G M R P T I Q E S D T E L L F S G L<br>2701 CCGAAAGGCATGAGGCCAACAATTCAAGAGTCAGATACTGAGCTCTTATTCTCTGGACTG<br><br>921 G S G E E V L P T L P T E S V N F T E V<br>2761 GGATCAGGAGAAGAAGTTTTACCTACTCTACCAACAGAGTCAGTGAATTTTACTGAAGTG<br><br>941 E Q I N N T L Y P H T S Q V E S T S S D<br>2821 GAACAAATCAATAACACATTATATCCCCACACTTCTCAAGTGGAAAGTACCTCAAGTGAC<br><br>961 K I E D Y N R M E N V A K E V G P L V S<br>2881 AAAATTGAAGACTATAACAGAATGGAAAATGTGGCAAAAGAAGTTGGACCACTCGTATCT<br><br>981 Q T D I F E G S G S V T S T T L I E I L<br>2941 CAAACAGACATCTTTGAAGGTAGTGGGTCAGTAACCAGCACAACATTAATAGAAATTTTA<br><br>1001 S D T G A E G P T V A P L P F S T D I G<br>3001 AGTGACACTGGAGCAGAAGGACCCACGGTGGCACCTCTCCCTTTCTCCACGGACATCGGA<br><br>1021 H P Q N Q T V R W A E E I Q T S R P Q T<br>3061 CATCCTCAAAATCAGACTGTCAGGTGGGCAGAAGAAATCCAGACTAGTAGACCACAAACC<br><br>1041 I T E Q D S N K N S S T A E I N E T T T<br>3121 ATAACTGAACAAGACTCTAACAAGAATTCTTCAACAGCAGAAATTAACGAAACAACAACC<br><br>1061 S S T D F L A R A Y G F E M A K E F V T<br>3181 TCATCTACTGATTTTCTGGCTAGAGCTTATGGTTTTGAAATGGCCAAAGAATTTGTTACA<br><br>1081 S A P K P S D L Y Y E P S G E G S G E V<br>3241 TCAGCACCAAAACCATCTGACTTGTATTATGAACCTTCTGGAGAAGGATCTGGAGAAGTG<br><br>1101 D I V D S F H T S A T T Q A T R Q E S S<br>3301 GATATTGTTGATTCATTTCACACTTCTGCAACTACTCAGGCAACCAGACAAGAAAGCAGC<br><br>1121 T T F V S D G S L E K H P E V P S A K A<br>3361 ACCACATTTGTTTCTGATGGGTCCCTGGAAAAACATCCTGAGGTGCCAAGCGCTAAAGCT<br><br>1141 V T A D G F P T V S V M L P L H S E Q N<br>3421 GTTACTGCTGATGGATTCCCAACAGTTTCAGTGATGCTGCCTCTTCATTCAGAGCAGAAC<br><br>1161 K S S P D P T S T L S N T V S Y E R S T | |

| Gene Name | GenBank Homology | DNA SEQUENCE / DEDUCED AMINO ACID SEQUENCE | SEQUENCE IDENTIFIER: |
|---|---|---|---|
| | | 3481 AAAAGCTCCCCTGATCCAACTAGCACACTGTCAAATACAGTGTCATATGAGAGGTCCACA<br><br>1181 D G S F Q D R F R E F E D S T L K P N R<br>3541 GACGGTAGTTTCCAAGACCGTTTCAGGGAATTCGAGGATTCCACCTTAAAACCTAACAGA<br><br>1201 K K P T E N I I I D L D K E D K D L I L<br>3601 AAAAAACCCACTGAAAATATTATCATAGACCTGGACAAAGAGGACAAGGATTTAATATTG<br><br>1221 T I T E S T I L E I L P E L T S D K N T<br>3661 ACAATTACAGAGAGTACCATCCTTGAAATTCTACCTGAGCTGACATCGGATAAAAATACT<br><br>1241 I I D I D H T K P V Y E D I L G M Q T D<br>3721 ATCATAGATATTGATCATACTAAACCTGTGTATGAAGACATTCTTGGAATGCAAACAGAT<br><br>1261 I D T E V P S E P H D S N D E S N D D S<br>3781 ATAGATACAGAGGTACCATCAGAACCACATGACAGTAATGATGAAAGTAATGATGACAGC<br><br>1281 T Q V Q E I Y E A A V N L S L T E E T F<br>3841 ACTCAAGTTCAAGAGATCTATGAGGCAGCTGTCAACCTTTCTTTAACTGAGGAAACATTT<br><br>1301 E G S A D V L A S Y T Q A T H D E S M T<br>3901 GAGGGCTCTGCTGATGTTCTGGCTAGCTACACTCAGGCAACACATGATGAATCAATGACT<br><br>1321 Y E D R S Q L D H M G F H F T T G I P A<br>3961 TATGAAGATAGAAGCCAACTAGATCACATGGGCTTTCACTTCACAACTGGGATCCCTGCT<br><br>1341 P S T E T E L D V L L P T A T S L P I P<br>4021 CCTAGCACAGAAACAGAATTAGACGTTTTACTTCCCACGGCAACATCCCTGCCAATTCCT<br><br>1361 R K S A T V I P E I E G I K A E A K A L<br>4081 CGTAAGTCTGCCACAGTTATTCCAGAGATTGAAGGAATAAAAGCTGAAGCAAAAGCCCTG<br><br>1381 D D M F E S S T L S D G Q A I A D Q S E<br>4141 GATGACATGTTTGAATCAAGCACTTTGTCTGATGGTCAAGCTATTGCAGACCAAAGTGAA<br><br>1401 I I P T L G Q F E R T Q E E Y E D K K H<br>4201 ATAATACCAACATTGGGCCAATTTGAAAGGACTCAGGAGGAGTATGAAGACAAAAAACAT<br><br>1421 A G P S F Q P E F S S G A E E A L V D H<br>4261 GCTGGTCCTTCTTTTCAGCCAGAATTCTCTTCAGGAGCTGAGGAGGCATTAGTAGACCAT<br><br>1441 T P Y L S I A T T H L M D Q S V T E V P<br>4321 ACTCCCTATCTAAGTATTGCTACTACCCACCTTATGGATCAGAGTGTAACAGAGGTGCCT | |

| Gene Name | GenBank Homology | DNA SEQUENCE / DEDUCED AMINO ACID SEQUENCE | SEQUENCE IDENTIFIER: |
|---|---|---|---|
| | | 1461 D V M E G S N P P Y Y T D T T L A V S T<br>4381 GATGTGATGGAAGGATCCAATCCCCCATATTACACTGATACAACATTAGCAGTTTCAACA<br><br>1481 F A K L S S Q T P S S P L T I Y S G S E<br>4441 TTTGCGAAGTTGTCTTCTCAGACACCATCATCTCCCCTCACTATCTACTCAGGCAGTGAA<br><br>1501 A S G H T E I P Q P S A L P G I D V G S<br>4501 GCCTCTGGACACACAGAGATCCCCCAGCCCAGTGCTCTGCCAGGAATAGACGTCGGCTCA<br><br>1521 S V M S P Q D S F K E I H V N I E A T F<br>4561 TCTGTAATGTCCCCACAGGATTCTTTTAAGGAAATTCATGTAAATATTGAAGCGACTTTC<br><br>1541 K P S S E E Y L H I T E P P S L S P D T<br>4621 AAACCATCAAGTGAGGAATACCTTCACATAACTGAGCCTCCCTCTTTATCTCCTGACACA<br><br>1561 K. L E P S E D D G K P E L L E E M E A S<br>4681 AAATTAGAACCTTCAGAAGATGATGGTAAACCTGAGTTATTAGAAGAAATGGAAGCTTCT<br><br>1581 P T E L I A V E G T E I L Q D F Q N K T<br>4741 CCCACAGAACTTATTGCTGTGGAAGGAACTGAGATTCTCCAAGATTTCCAAAACAAAACC<br><br>1601 Y G Q V S G E A I K M F P T I K T P E A<br>4801 TATGGTCAAGTTTCTGGAGAAGCAATCAAGATGTTTCCCACCATTAAAACACCTGAGGCT<br><br>1621 G T V I T T A D E I E L E G A T Q W P H<br>4861 GGAACTGTTATTACAACTGCCGATGAAATTGAATTAGAAGGTGCTACACAGTGGCCACAC<br><br>1641 S T S A S A T Y G V E A G V V P W L S P<br>4921 TCTACTTCTGCTTCTGCCACCTATGGGGTCGAGGCAGGTGTGGTGCCTTGGCTAAGTCCA<br><br>1661 Q T S E R P T L S S S P E I N P E T Q A<br>4981 CAGACTTCTGAGAGGCCCACGCTTTCTTCTTCTCCAGAAATAAACCCTGAAACTCAAGCA<br><br>1681 A L I R G Q D S T I A A S E Q Q V A A R<br>5041 GCTTTAATCAGAGGGCAGGATTCCACGATAGCAGCATCAGAACAGCAAGTGGCAGCGAGA<br><br>1701 I L D S N N Q A T V S T A E L N T E L A<br>5101 ATTCTTGATTCCAATAATCAGGCAACTGTAAGCACTGCAGAGTTAAATACTGAGCTTGCA<br><br>1721 T P S F S L L E T S N E T D F L I G I N<br>5161 ACACCATCATTTTCCCTTCTGGAAACTTCTAATGAAACCGATTTCCTGATTGGCATTAAT | |

| Gene Name | GenBank Homology | DNA SEQUENCE / DEDUCED AMINO ACID SEQUENCE | SEQUENCE IDENTIFIER: |
|---|---|---|---|
| | | 1741 E E S V E G T A V Y L P G P D R C K T N<br>5221 GAAGAGTCAGTGGAGGGCACAGCAGTCTATTTACCAGGACCAGATCGTTGCAAAACGAAC<br><br>1761 P C L N G G T C Y P T E T S Y V C T C V<br>5281 CCGTGCCTTAACGGAGGCACCTGTTATCCTACTGAAACTTCCTACGTGTGCACCTGTGTG<br><br>1781 P G Y S G D Q C E L D F D E C H S N P C<br>5341 CCAGGATACAGCGGCGACCAGTGTGAGCTTGATTTTGATGAATGTCACTCTAACCCCTGT<br><br>1801 R N G A T C I D G F N T F R C L C L P S<br>5401 CGCAATGGAGCCACGTGCATTGACGGTTTCAATACGTTCAGGTGCCTCTGCCTTCCGAGC<br><br>1821 Y I G A L C E Q D T E T C D Y G W H K F<br>5461 TACATTGGTGCACTTTGTGAGCAAGACACCGAGACATGTGACTACGGCTGGCACAAATTC<br><br>1841 Q G Q C Y K Y F A H R R T W D A A E R E<br>5521 CAAGGGCAGTGCTACAAGTACTTTGCCCACCGCCGCACGTGGGATGCAGCTGAGCGAGAA<br><br>1861 C R L Q G A H L T S I L S H E E Q M F V<br>5581 TGCCGGCTGCAGGGCGCCCATCTCACAAGCATCCTGTCTCATGAAGAACAAATGTTTGTG<br><br>1881 N R V G H D Y Q W I G L N D K M F E H D<br>5641 AATCGTGTGGGCCATGACTATCAGTGGATAGGCCTCAATGACAAGATGTTTGAGCATGAC<br><br>1901 F R W T D G S T L Q Y E N W R P N Q P D<br>5701 TTCCGTTGGACTGATGGCAGCACCCTGCAATATGAGAACTGGAGGCCCAACCAGCCAGAC<br><br>1921 S F F S A G E D C V V I I W H E N G Q W<br>5761 AGCTTTTTTTCTGCTGGAGAAGATTGTGTCGTAATCATTTGGCACGAGAACGGCCAGTGG<br><br>1941 N D V P C N Y H L T Y T C K K G T V A C<br>5821 AATGATGTTCCCTGCAATTACCATCTCACCTATACTTGCAAGAAAGGAACAGTTGCTTGC<br><br>1961 G Q P P V V E N A K T F G K M K P R Y E<br>5881 GGCCAGCCCCCAGTTGTAGAAAATGCCAAGACCTTTGGAAAGATGAAACCTCGTTATGAA<br><br>1981 I N S L I R Y H C K D G F I Q R H P S T<br>5941 ATCAACTCCCTGATTAGATATCACTGTAAAGATGGTTTCATTCAACGCCACCCTTCCACT<br><br>2001 I R C L G N G K W A M P K I T C M N P S<br>6001 ATCCGTTGCCTAGGAAATGGAAAATGGGCCATGCCTAAAATTACCTGCATGAACCCATCT<br><br>2021 A Y Q R T Y S M K Y F K N S S S A K D N | |

| Gene Name | GenBank Homology | DNA SEQUENCE / DEDUCED AMINO ACID SEQUENCE | SEQUENCE IDENTIFIER: |
|---|---|---|---|
| | | 6061 GCATACCAAAGGACTTATTCTATGAAATACTTTAAAAATTCCTCATCAGCAAAGGACAAT<br><br>2041 S I N T S K H D H R W S R R W Q E S R R<br>6121 TCAATAAATACATCCAAACATGATCATCGTTGGAGCCGGAGGTGGCAGGAGTCGAGGCGC<br><br>2061 -<br>6181 TGA | |
| WBC031G11.bFSP_20 012510.abd | Homo sapiens solute carrier family 2 (facilitated glucose transporter), member 14, mRNA | 1 GCCGGCGGGGGCGCAGCGGCCCGGGAGGTGCGTGGTTGAGCGAAGCCGCGGGGCCCAACC<br>61 GCAGTCGCGGGGTCTGGGAGGAAGCAGTACCTTGAAGAGAAATTGGAGAGGGAGTCAATT<br>121 CCTAGGATAGCAGAGAGATGGACAACAGACAGAATGTCACCCCAGCTCTGATCTTTGCCA<br>181 TCACAGTTGCTACAATCGGCTCTTTCCAGTTTGGCTACAACACTGGGGTCATCAATGCTC<br>241 CTGAGACGATCATAAAGGAATTTATCAATAAAACTTTGACGGACAAGGCAAATGCCCCTC<br>301 CCTCTGAGGTGCTGCTCACGAATCTCTGGTCCTTGTCTGTGGCCATATTTTCCGTCGGGG<br>361 GTATGATCGGCTCCTTTTCCGTCGGACTCTTTGTTAACCGCTTTGGCAGGCGCAATTCAA<br>421 TGCTGATTGTCAACCTGTTGGCTGCCACTGGTGGCTGCCTTATGGGACTGTGTAAAATAG<br>481 CTGAGTCAGTTGAAATGCTGATCCTGGGCCGCTTGGTTATTGGCCTCTTCTGCGGACTCT<br>541 GCACAGGTTTTGTGCCCATGTACATTGGAGAGATCTCGCCTACTGCCCTGAGGGGTGCCT<br>601 TTGGCACTCTCAACCAGCTGGGCATAGTTATTGGAATTCTGGTGGCCCAGATCTTTGGTC<br>661 TGGAACTCATCCTTGGGTCTGAAGAGCTATGGCCGGTGCTATTAGGCTTTACCATCCTTC<br>721 CAGCTATCCTGCAAAGTGCAGCCCTTCCATGTTGCCCTGAAAGTCCCAGATTTTTGCTCA<br>781 TTAACAGAAAAAAAGAGGAGAATGCTACGCGGATCCTCCAGCGGTTGTGGGGCACCCAGG<br>841 ATGTATCCCAAGACATCCAGGAGATGAAAGATGAGAGTGCAAGGATGTCACAAGAAAAGC<br>901 AAGTCACCGTGCTGGAGCTCTTTAGAGTGTCCAGCTACCGACAGCCCATCATCATTTCCA<br>961 TTGTGCTCCAGCTCTCTCAGCAGCTCTCTGGGATCAATGCTGTGTTCTATTACTCAACAG<br>1021 GAATCTTCAAGGATGCAGGTGTTCAACAGCCCATCTATGCCACCATCAGCGCGGGTGTGG<br>1081 TTAATACTATCTTCACTTTACTTTCTCTATTTCTGGTGGAAAGGGCAGGAAGAAGGACTC<br>1141 TGCATATGATAGGCCTTGGAGGGATGGCTTTTTGTTCCACGCTCATGACTGTTTCTTTGT<br>1201 TATTAAAGAATCACTATAATGGGATGAGCTTTGTCTGTATTGGGGCTATCTTGGTCTTTG<br>1261 TGGCCTGTTTTGAAATTGGACCAGGCCCCATTCCCTGGTTTATTGTGGCCGAACTCTTCA<br>1321 GCCAGGGCCCCCGCCCAGCTGCGATGGCAGTGGCCGGCTGCTCCAACTGGACCTCCAACT<br>1381 TCCTAGTCGGATTGCTCTTCCCCTCTGCTGCTTACTATTTAGGAGCCTACGTTTTTATTA<br>1441 TCTTCACCGGCTTCCTCATTACCTTCTTGGCCTTTACCTTCTTCAAAGTCCCTGAGACCC<br>1501 GTGGCAGGACTTTTGAGGATATCACACGGGCCTTTGAAGGGCAGGCACACGGTGCAGATA<br>1561 GATCTGGGAAGGACGGCGTCATGGGGATGAACAGCATCGAGCCTGCTAAGGAGACCACCA<br>1621 CCAATGTCTAAGTCATGCCTCCTTCCACCTCCCTCCCGGCATGGGAAAGCCACCTCTCCC<br>1681 TCAACAAGGGAGAGACTTTATCAGGATGAACCCAGGACTGCTTCTGAATGCTGCTACTTG<br>1741 ATTTCTTTCTCATCCCACGCACTCCATGAGCACCCCAAGGCTGCAGTTTGTTGGATCTTC<br>1801 AATGGCTTTTTAAATTTTATTTCCTGGACATCCTCTTCTGCTTAGGAGAGACCGAGTGAA<br>1861 CCTACCTTCATTTCAGGAGGGATTGGCCGCTTGGCACATGACAACTTTGCCAGCTTTTCC<br>1921 TCCCTTGGGTTCTGATATTGCCGCACTAGAGGATATAGGAGAGGAAAAGTAAGGTGCAGT<br>1981 TGCCCCAACCTCAGACTTACCAGGAAGCAGATACATATGAGTGTGGAAGCCGGAGGGTGT | SEQ ID NO:149 |

| Gene Name | GenBank Homology | DNA SEQUENCE / DEDUCED AMINO ACID SEQUENCE | SEQUENCE IDENTIFIER: |
|---|---|---|---|
| | | 2041 TTATGTAAGAGCACCTTCCTCACTTCCATACAGCTCTACGCGGCAAATTAACTTGAGTTT<br>2101 TATTTATCTTATCCTCTGGTTTAATTACATAAATATTTATTTTTTAAGTGTAATTTTGCC<br>2161 AAATAATAAAAACAGAAGGAAATTGAGATTAGAGGGAGGTGTTTAAAGAGAGGTTATAGA<br>2221 GTAAAAGATTTGATGCTGGAGAGGTTAAGGTGCAATAAGAATTCAGGGAGAAATGTTGTT<br>2281 CATTATTGGAGGGTAAATGATGTGGTGCCTGAGGTCTGTACATTACCTCTTAACAATTTC<br>2341 TGTCCTTCAGATGGAAACTCTTTGATTTCTCAGAAAAGTTGTATGCCTATTTAATAAAGC<br>2401 TACTCATTTCCTTTGGAACTTTATCTTTAAGATAATAGTTTACATGTAGTAGTACTTGAA<br>2461 ATCTAGGATTATTAACTAATATGGGCATTGTAGTTAATGGCGGTTGATGGGTTCTAATTT<br>2521 TGGATGGAGTCCAGGGAAGAGAAAGTGATTTCTAGAAAGCCTGTTCCCCTCACTGGACGA<br>2581 AATAACTCCTTGTAGTAGTCTCATTACTTTTGAAGTAATCCCGCCACCTATCTAGTGGGA<br>2641 GAGCCATCCAAATGAGAAACCTAAAATAATTGGTTCTTGGTAGAGATTCATTATTTCTCC<br>2701 ACTTTGTTCTTTAGGAGATTTTAGGTGTTGATTTTCTGTTTTATTTTAACTCATACCTTT<br>2761 AAAGGAATTCCCCAAAGAATGTTTATAGCAAACTTGGAATTTGTAACCTCAGCTCTGGGA<br>2821 GAGGATTTTTTTCTGAGCGATTATTATCTAAAGTGTGTTGTTGCTTTAGGCTCACGGCAC<br>2881 GCTTGCGTATGTCTGTTACCATGTCACTGTGGTCCTATGCCGAATGCCCTCAGGGGACTT<br>2941 GAATCTTTCCAATAAACCAGGTTTAGACAGTATGAGTAAATGTGCGGTGTAGCCCATACT<br>3001 TGAGAGTATGAATGTATGTGCACTGTCACTTTGCTCTGGGTGGAAGTATGTTATTGTTGA<br>3061 CTTATTTTCTCTGTGTTTGTTCCTACAGCCCCTTTTTCATATGTTGCTTGGTCTCCCTTT<br>3121 CCCTCCTTGGTGCTTACACATTTCAGGCCCTTTAGCCAAACCCTTGCCTACGGCAGTATT<br>3181 TTAGTTCTCAGTTCTCACCATTCCCTCTGGTCATTGAGCCTTTGGATAAAAACTCATAGA<br>3241 GCTATGGACTGGGGCTTTGCTGGAAAGGTGGCCTTCCAACTTCACGTCAACTTCTGGCTC<br>3301 CTCAGTTTGGCAGTAAGGCAGGGAAGTTGTTTTCCCGTTTCTCAGTGACCAGATTGTGAA<br>3361 TATTTCCCAATGGATTTTCTATTATTAATGTTACTGTAGTTCTTTGTTTTGAAATAAAAA<br>3421 TTCTGAATGTAAAAAAAAAAAAAAAAA<br><br>1 M D N R Q N V T P A L I F A I T V A T I<br>1 ATGGACAACAGACAGAATGTCACCCCAGCTCTGATCTTTGCCATCACAGTTGCTACAATC<br><br>21 G S F Q F G Y N T G V I N A P E T I I K<br>61 GGCTCTTTCCAGTTTGGCTACAACACTGGGGTCATCAATGCTCCTGAGACGATCATAAAG<br><br>41 E F I N K T L T D K A N A P P S E V L L<br>121 GAATTTATCAATAAAACTTTGACGGACAAGGCAAATGCCCCTCCCTCTGAGGTGCTGCTC<br><br>61 T N L W S L S V A I F S V G G M I G S F<br>181 ACGAATCTCTGGTCCTTGTCTGTGGCCATATTTTCCGTCGGGGGTATGATCGGCTCCTTT<br><br>81 S V G L F V N R F G R R N S M L I V N L<br>241 TCCGTCGGACTCTTTGTTAACCGCTTTGGCAGGCGCAATTCAATGCTGATTGTCAACCTG<br><br>101 L A A T G G C L M G L C K I A E S V E M<br>301 TTGGCTGCCACTGGTGGCTGCCTTATGGGACTGTGTAAAATAGCTGAGTCAGTTGAAATG | SEQ ID NO:150 |

| Gene Name | GenBank Homology | DNA SEQUENCE / DEDUCED AMINO ACID SEQUENCE | SEQUENCE IDENTIFIER: |
|---|---|---|---|
| | | 121 L I L G R L V I G L F C G L C T G F V P<br>361 CTGATCCTGGGCCGCTTGGTTATTGGCCTCTTCTGCGGACTCTGCACAGGTTTTGTGCCC<br><br>141 M Y I G E I S P T A L R G A F G T L N Q<br>421 ATGTACATTGGAGAGATCTCGCCTACTGCCCTGAGGGGTGCCTTTGGCACTCTCAACCAG<br><br>161 L G I V I G I L V A Q I F G L E L I L G<br>481 CTGGGCATAGTTATTGGAATTCTGGTGGCCCAGATCTTTGGTCTGGAACTCATCCTTGGG<br><br>181 S E E L W P V L L G F T I L P A I L Q S<br>541 TCTGAAGAGCTATGGCCGGTGCTATTAGGCTTTACCATCCTTCCAGCTATCCTGCAAAGT<br><br>201 A A L P C C P E S P R F L L I N R K K E<br>601 GCAGCCCTTCCATGTTGCCCTGAAAGTCCCAGATTTTTGCTCATTAACAGAAAAAAAGAG<br><br>221 E N A T R I L Q R L W G T Q D V S Q D I<br>661 GAGAATGCTACGCGGATCCTCCAGCGGTTGTGGGGCACCCAGGATGTATCCCAAGACATC<br><br>241 Q E M K D E S A R M S Q E K Q V T V L E<br>721 CAGGAGATGAAAGATGAGAGTGCAAGGATGTCACAAGAAAAGCAAGTCACCGTGCTGGAG<br><br>261 L F R V S S Y R Q P I I I S I V L Q L S<br>781 CTCTTTAGAGTGTCCAGCTACCGACAGCCCATCATCATTTCCATTGTGCTCCAGCTCTCT<br><br>281 Q Q L S G I N A V F Y Y S T G I F K D A<br>841 CAGCAGCTCTCTGGGATCAATGCTGTGTTCTATTACTCAACAGGAATCTTCAAGGATGCA<br><br>301 G V Q Q P I Y A T I S A G V V N T I F T<br>901 GGTGTTCAACAGCCCATCTATGCCACCATCAGCGCGGGTGTGGTTAATACTATCTTCACT<br><br>321 L L S L F L V E R A G R R T L H M I G L<br>961 TTACTTTCTCTATTTCTGGTGGAAAGGGCAGGAAGAAGGACTCTGCATATGATAGGCCTT<br><br>341 G G M A F C S T L M T V S L L L K N H Y<br>1021 GGAGGGATGGCTTTTTGTTCCACGCTCATGACTGTTTCTTTGTTATTAAAGAATCACTAT<br><br>361 N G M S F V C I G A I L V F V A C F E I<br>1081 AATGGGATGAGCTTTGTCTGTATTGGGGCTATCTTGGTCTTTGTGGCCTGTTTTGAAATT<br><br>381 G P G P I P W F I V A E L F S Q G P R P<br>1141 GGACCAGGCCCCATTCCCTGGTTTATTGTGGCCGAACTCTTCAGCCAGGGCCCCCGCCCA<br><br>401 A A M A V A G C S N W T S N F L V G L L | |

| Gene Name | GenBank Homology | DNA SEQUENCE / DEDUCED AMINO ACID SEQUENCE | SEQUENCE IDENTIFIER: |
|---|---|---|---|
| | | 1201 GCTGCGATGGCAGTGGCCGGCTGCTCCAACTGGACCTCCAACTTCCTAGTCGGATTGCTC<br><br>421 F P S A A Y Y L G A Y V F I I F T G F L<br>1261 TTCCCCTCTGCTGCTTACTATTTAGGAGCCTACGTTTTTATTATCTTCACCGGCTTCCTC<br><br>441 I T F L A F T F F K V P E T R G R T F E<br>1321 ATTACCTTCTTGGCCTTTACCTTCTTCAAAGTCCCTGAGACCCGTGGCAGGACTTTTGAG<br><br>461 D I T R A F E G Q A H G A D R S G K D G<br>1381 GATATCACACGGGCCTTTGAAGGGCAGGCACACGGTGCAGATAGATCTGGGAAGGACGGC<br><br>481 V M G M N S I E P A K E T T T N V -<br>1441 GTCATGGGGATGAACAGCATCGAGCCTGCTAAGGAGACCACCACCAATGTCTAA | |
| WBC032D03.bFSP_20 012510.abd | Homo sapiens hypothetical protein MGC12966, mRNA | 1 CGGCCAGGCTGGTACAGGCGGCTGCTCGCGGAGGCACTTCTCCGAGCGTGACGTGCGGAC<br>61 TCCGCTCCACTGCGGCCCGCCCGCCCGCCGGTCTAGCCGCCGCCTCCTTCGCTCCCTGAG<br>121 CACGCCAGGCGAGGGTCAGCTGCCGGGGCGACGCGGCGCGGAGAGGGCGGGCGGGGGGC<br>181 TGTGGGAGCGCCGGTCTCTATATGGCGGCGGCTCTGTCGGGCCTGGCTGTCCGGCTCTCG<br>241 CGCTCGGCCGCCGCCCGCTCCTATGGGGTCTTCTGCAAGGGGCTGACTCGCACGCTGCTC<br>301 ATCTTCTTCGACCTGGCCTGGCGGCTGCGTATCAACTTCCCCTACCTCTACATCGTGGCT<br>361 TCCATGATGCTCAACGTCCGCCTGCAGGTTCATATTGAGATCCATTGAAGGCCCTCCCAG<br>421 ACTGACGAAGGCGTGACCTCAGGCCCACCGCATCCAAGAACACGAAGCATGAGGGACAGA<br>481 AGAGGGCCCCTCGGCACCTGCCTGGCACAAGTGCAGCAGGCCGGAGGAGGTGACTCGGAC<br>541 AAACTATCATGCAGCCTTAAGAAAAGAATGCCGGAGGGCCCTTGGCCTGCAGATGCACCC<br>601 TCCTGGATGAATAAGCCTGTGGTTGATGGAAATTCACAAAGTGAGGCATTATCACTGGAA<br>661 ATGAGAAAGGATCCGAGCGGGGCTGGCCTCTGGCTTCACAGTGGCGGCCCAGTGCTTCCA<br>721 TATGTGAGAGAATCAGTAAGAAGAAATCCAGCCTCAGCAGCCACTCCGAGCACAGCCGTG<br>781 GGTTTGTTCCCTGCTCCAACAGAGTGTTTTGCTCGGGTGTCCTGCAGTGGTGTTGAAGCT<br>841 CTGGGGCGGCGAGACTGGCTGGGAGGAGGGCCCAGGGCCACTGACGGCCACAGAGGACAG<br>901 TGCCCCAAAGGAGAGCCTCGGGTGTCACGACTGCCACGCCATCAAAAAGTGCCGGAAATG<br>961 GGAAGTTTTCAGGATGACCCACCAAGTGCTTTTCCCAAGGGTCTGGGCTCTGAGTTGGAA<br>1021 CCCGCTTGCCTGCACTCCATCCTGTCTGCAACGCTGCACGTGTATCCCGAAGTGCTCCTG<br>1081 AGTGAGGAGACAAAACGCATTTTCCTTGACCGTTTAAAGCCCATGTTTTCAAAGCAAACA<br>1141 ATAGAATTCAAGAAAATGCTTAAAAGCACCTCAGATGGTCTGCAGATGACACTGGGCTTG<br>1201 CTGGCTCTACAAGCTTTTGAATTAGCAAAGCCGTCACGCCATAGTTAAGGTACAAGCAGA<br>1261 ACAATAAAAATAGTGAGATTAATTTTAAAAATTGTCCTAGAATGATTTCTTTTGCATTAA<br>1321 ATCTGGCTGCAGACAGTGCAGCCCTTAGGTTCCTGCTGTAGTTTTGTACGACCTGGCAGA<br>1381 CTTAAAGTAAATTGAGTTTAAATTCAAAGCCAGTTGATGCGGAAGGAACTTTTTTGGCAT<br>1441 GTGTTAAATTGTGCTTTAAAAGACATATAAAGAATTGGGAAACATTTCAGGAGACGATCA<br>1501 TAGCCTGTATAAATACCAGATTAGAACATACGGATTTACCATGAAGTTCTGTCTTCAACA<br>1561 TCCATTCTAAAGGGCTACTGTCCCAAATCCTGTGTGTCCTTTTGACTTGTCTGATCACCC<br>1621 AATGGAAGTGGATACTTGTAAAGTCTACACCACTGTACTTGGCGTTAAATCTTGCTGAGT | SEQ ID NO:151 |

| Gene Name | GenBank Homology | DNA SEQUENCE / DEDUCED AMINO ACID SEQUENCE | SEQUENCE IDENTIFIER: |
|---|---|---|---|
| | | 1681 TGCTGGTAGGCTGTTACCATTTCTGCGTTTTGTAAAATAATTTTGGTCTGCAGCAATACT<br>1741 TGATTTCTTTTCTCACATCCCTTTCCTTCCGCTTGAAATGTGATTTAAATGGAGGCCCTA<br>1801 CGGTGAAAGTTGCAATATTAAACTTACTTTTAGAATAGTCCTTCCTCAAACTAGGAACCC<br>1861 TAGCAGTGTTACCTTAAACAAAAATGAGCTCGAGAAAAAAGTAGCTCAGTTACAGAGAAG<br>1921 CAAATCGAGTTATTTCCCACATAAAAAGTTTCCCAGATTCTAAGAATTGCAGTATCCTGT<br>1981 ACCCTAAAATTTTTCAAGGTGACTCCTGTTGTCGTCTGTTGATAACTTTAATAAAGGTCA<br>2041 TTTAAGGACATAAGTTTTTAAAGACTCCCAAAGTGAAACTTAAACATTTTCGGGATTATC<br>2101 GATTGCATATATCAGTTTATGCTGTGTGCTGAATATTACTACACCGTGTGCTATTTTAGT<br>2161 GTTTTGGGAAAATGAAAAATAAAATCTGTTCTTTAGCTTAAAAAAAAAAAAAAAAAAAA<br>2221 AAAAAAAA | |
| | | 1 M A A A L S G L A V R L S R S A A A R S<br>1 ATGGCGGCGGCTCTGTCGGGCCTGGCTGTCCGGCTCTCGCGCTCGGCCGCCGCCCGCTCC<br><br>21 Y G V F C K G L T R T L L I F F D L A W<br>61 TATGGGGTCTTCTGCAAGGGGCTGACTCGCACGCTGCTCATCTTCTTCGACCTGGCCTGG<br><br>41 R L R I N F P Y L Y I V A S M M L N V R<br>121 CGGCTGCGTATCAACTTCCCCTACCTCTACATCGTGGCTTCCATGATGCTCAACGTCCGC<br><br>61 L Q V H I E I H -<br>181 CTGCAGGTTCATATTGAGATCCATTGA | SEQ ID NO:152 |
| WBC032H02.bFSP_20 012510.abd | Homo sapiens mRNA; cDNA DKFZp686H1588 | 1 TCACTTCCGGCGTGCCTACGCCTCCTCTTGCGCTGTCCTGTTAATGGCGGGCAGTAGCCG<br>61 CTGAGGGGATTGCAGATAACCGCTTCCCGCACGGGGAAAGTCTACCCTGCCTGCCACTTT<br>121 CTGCTCGCCGTCAGCGCCGGAGCTCGCCAGCATGTCTGTGGTACCGCCCAATCGCTCGCA<br>181 GACCGGCTGGCCCCGGGGGGTCACTCAGTTCGGCAACAAGTACATCCAGCAGACGAAGCC<br>241 CCTCACCCTGGAGCGCACCATCAACCTGTACCCTCTTACCAATTATACTTTTGGTACAAA<br>301 AGAGCCCCTCTACGAGAAGGACAGCTCTGTTGCAGCCAGATTTCAGCGCATGAGGGAAGA<br>361 ATTTGATAAAATTGGAATGAGGAGGACTGTAGAAGGGGTTCTGATTGTACATGAGCACCG<br>421 GCTACCCCATGTGTTACTGCTGCAGCTGGGAACAACTTTCTTCAAATTACCTGGTGATGA<br>481 ACTTAACCCAAGAGAATATGAAGTTGAAGGACTAAAACGCTTAGTGACTGATATACTGGG<br>541 TCGTCAAGATGGAGACCTGCAAGACTGGGTCACTGATGACCGCATTGGTAACTGGTGGAG<br>601 ACCAAATTTTGAACCTCCTCACTATCCATATATTCCTGCACACAGTACAAAACATAAGGA<br>661 GCATAAGAATTTGTTTCTGGTTCAGCTTCAAGAAAAAGCCTTGTTTGCAGTCCCTAAAAA<br>721 TTACAAGCTGGTAGCTGCACCATTGTTTGAATTGTATGACAATGCACCAGGATATGGACC<br>781 CATCATTTCTAGTCTCCCTCAGCCGTTGAGCAGGTTCAATTTTATTTACAACTGAATTCC<br>841 TGCGCAGTGGAGAAGTAAAAGAAGCCGCTTGTCTCTGTGAGCACAGCTATATACAGTGTA<br>901 GAATAAATGTGGTAGAAAAGTTTTTTTGGTTTTATCTCTTTTGCGATCCCTAAATTGCCA<br>961 CCTTTCTATTGTTTGAATAGTAAAATTAATATGAAGAACTAGATAGTGGTGTAAACAAAT<br>1021 GTGATAATGTTTATTTACTTTCGGTTCTGCTCATACTTTTTTGTACAACATTAAAGAAAA<br>1081 TGGACTTTTTTTATTTTAATTTCTCATTAAACTTCTAAAATTCTTATAGGTGAGGATCAT | SEQ ID NO:153 |

| Gene Name | GenBank Homology | DNA SEQUENCE / DEDUCED AMINO ACID SEQUENCE | SEQUENCE IDENTIFIER: |
|---|---|---|---|
| | | 1141 TTTTCCCCCCACCTTAGGATGGTGAATGTTGCAACACAATGACAGGTTTAAGTCAGTCAA<br>1201 GTTTATTGGACCCTTGCTTTGATACCATTCTTGGGCACATACTCCAAGATTGTATTAGAT<br>1261 TTTTGTGATGAAGAGCTTCCATTACTTCTGAAAACTATATTTATCTGAGTGAGTCCAAGG<br>1321 TGCAACTCCTAAATGAATTGTGTTGCAGAGAACTCCCAGTATAATTCACTGACCAGTACA<br>1381 TTTTATAACCATCCAGGCCTTGGTTTGCAAGCAACAGACCTTAAACATACAGGAAACTAT<br>1441 TAAAATTGGCTCGATCAGTAGTCATAGGAATTGGTATAAGAAAAGACTCATTTAGAGCTC<br>1501 AGAGTTTTCTTCACATAATGGGGGTATTAATTATTTGTGCTGTTGCGAAATTATGTGTCT<br>1561 TATTCTTAAAGCCATGGTAAAAATAGGGATCTGTGAAGGAAATTTCTAAAATTGGATGTA<br>1621 TTAGGTTTTGAACTCTGAGATTGCACAAATATTCAATTAACTTGAAGTTGTGTACATAGA<br>1681 GAAGAAAATTTGGTTTTAGCAAATGACAGAGCCTTCAAAAATATTTTTGGAATAATGTGA<br>1741 ATCAACCGAAAACTGGGGGCAAGGCAGAGGACAGGTTTTCTCAGGTTAAGAGAAAAACGA<br>1801 AATTTTAAAAACTTTAAAAAATACTGATAAATTCGGATCAAATTTGGGGGAATAAAAAAT<br>1861 ATTAGAGCAAAGGAGTTTGCTGGTTGTGTCATTATTTAATGATCAAAGTATAGCATGTAT<br>1921 GCCTTATTACAGACTTGTTGACTATAGGCTTAATGTAAAAAGGAATCTTGCCAGATGTAG<br>1981 CTACTTAAGGAAAAAAGGGTTTTTAATAGAAATGAACTTTTGATTAGTATGGTGCCAGTC<br>2041 ACAGGGCTATTTTCCTGAATATTGGGTGATGTCAAAGGTATATGATACTTGAGGAAATCA<br>2101 GGCCAAGTGTAGCTGAGCAATTAATAAACACTCAAGTTTTTTTAGTTGGGGTCATGTCAA<br>2161 ACCCTCACATGGTGGTTAAGTAGTGACTAACTGTGCATGTCCTTCATGGTAGGTGAGCAC<br>2221 CAGTCTCCATGATCAAAAATGCCCCAGTTTCCTTTGAAAAAGCCACTAGTTCTGTGAATT<br>2281 GTCCTTTTCCCTCCTGCCCCTAACAGTCCTACCCCTCTAGCCTACATTAGCATATTTCTC<br>2341 ATGTAATGTATTTTGCTGGTAAGCTCATCCATTAGCTGCGTCCTTCAGCTATTCCTTTAG<br>2401 ATTGGAGGAAGTGGATATGAAGATGGATTGATTCAGCTCACCTTCCATGATTGCTTTTGA<br>2461 GGGAAAAGCTACAGTGGCCACATTTCAGATGTTCACTTTTGCAATTTGTGGAGGGTGGAG<br>2521 AGAGGTAGACTTTTGTTGTGTGTTTTATAAAGTACTCTCAGTAAGGAGTGTTTGAGTTGA<br>2581 AGAGTCATCTGATTCGAGGCCACTCATGTTCTTTGTAACTTAAACTTTGACCAAGAAATT<br>2641 CTTCACTTCTCACTTCTTCACTTCTTCCCAATATACAGTAAGTACGTGAGCCAGTCATCC<br>2701 ATACACTAAGGCCTAGTTGAGAAAAACCTTTGATTCAGGATGGCTGGGTTACTAACCTTG<br>2761 AAATGTAAGAGATCTGGTTTTGGATGTAAAAGTTGCAACACACAAACGGAAGTCTTAAAA<br>2821 ACTTTTTGCTCTGGTCAGTTACAGGTGGATCCCCAATAATCTGTTTTTGGTTTTCTGATG<br>2881 GAAATAATAGAATTAGGGGAAATCAAATCTGGTTGGTAGGTGTCTACAGTATTAGAAGAG<br>2941 GGTATAAGGGCACTGTTTAACACTAAGTTCTAATACTTCCAGAAACTGTGCATTCCAGAT<br>3001 CTACATACTAAATGCTCTTATCATTTTGAAATGGGCTCTTGATTAATAGACCCATATTTT<br>3061 TTAGTGGCTTCTATGTTGTATATTTGTCTAAAATGAAAGCTCTTTTGCGTTCTAAAACTA<br>3121 CAATATATGTCATCTTATTTTCCCTGAGTATCCAAGTATAGTGCAGATTCTATGTAAAAC<br>3181 TACTAAATGACACTGGAATATGTTTAGTAGATTAGGGGGAAAAACTATAAAGGTTTATAC<br>3241 AATTGTTTGTAGTTACATTTAGGATGGACTTATCCCTTTGGAGAAGAGTGAAGTTTGTTT<br>3301 TTTCGCCATGTGATGAAGACCACTGTGATTTTTTAAAAAAGTAGATAATACTTAAAATGG<br>3361 CGTAATAATTCTGCACTTGAATTTGTACTGTTAACAGCACATTTGGAAGATTTTAAAACT<br>3421 TTTTATTGTCTTATAAATAGCATTCACTTATTATTTTGGATATTTAAGGGTTCCATTAAG<br>3481 TTAACACTGTATTTGGACAAAGTGTGACCAAATTAGCCAGTCTGTTTTCTTCCATGTTTA<br>3541 ATTAGAAGTGAGAGGTAGAAGTACTTCAAATTCAACAGGCCAGCAAGCAATCGGCTTAAA<br>3601 ATTCCCTTTCTTAAATGTTGTGCTCTTATGTTCTCGGCTTTTTAATGACTTTATTTTTAC<br>3661 AGTACTTGTTCAGTCACTTGAGATGAAATGCTTGGGGTAGCTTTTCCATCCTCAAACTTA | |

| Gene Name | GenBank Homology | DNA SEQUENCE / DEDUCED AMINO ACID SEQUENCE | SEQUENCE IDENTIFIER: |
|---|---|---|---|
| | | 3721 ATGTTTTTACTAGTTCATAGTGTTTGGAACAGTATATGCCAATCACTGAGACTGCATCAG<br>3781 AGTTTGCAATTTTGTATGTTTCATTGCCAAAGAAGGCTTAGTGGTTTGTCACTGTAATAC<br>3841 GAGTCAGCTTCTGTAGCATAAGGTTTGATTTCCTATAATTCTTCCACTTGTTTTACATCA<br>3901 CTTATTATTTGGGTTAAAGTGGCCTCACTTCAAGCAGTTTGCCTTTGTTCTTTAAATATG<br>3961 TGATGAGAAACAATGCTGTAATTTGACTTGAGCCATAAAACACAATTTTAATATTAGCTT<br>4021 GTATTATATTTATCAAGGTTGTTTTTGTGAAAAAACAGGCTAAAACCTAAGTAAGTCTAG<br>4081 ATTAAGCCAGGTTAGCTGTAATTTGTATTTTAGTGATGGATTGTATTGTAAGACTAGAGA<br>4141 TAAATTGGGAAAATGTATTATGCTTTTCTGTTGAGGGAGAGATCAGTGCATTTAAAAACT<br>4201 TCAACTTTTGCTTACTGTGCTCACATCATAGTGTACAAGCGTCATTTATAGTTTGGCTTT<br>4261 GAGGATTTTTCTGGCATTAAGTTTATAGAAATGTATAAAAGAAAGTGTTTGAAGTTTTGG<br>4321 TTATATTTTTATATTTGTAAAGTAAGTTTGGTTAAAGTGATCACTGTTCTTTTTTTATTT<br>4381 TATTGTCATTTCAATAAAAAATATTTGAAAGAGAAAAAAAAAAAAAAAAA | |
| | | 1 M S V V P P N R S Q T G W P R G V T Q F<br>1 ATGTCTGTGGTACCGCCCAATCGCTCGCAGACCGGCTGGCCCCGGGGGGTCACTCAGTTC<br><br>21 G N K Y I Q Q T K P L T L E R T I N L Y<br>61 GGCAACAAGTACATCCAGCAGACGAAGCCCCTCACCCTGGAGCGCACCATCAACCTGTAC<br><br>41 P L T N Y T F G T K E P L Y E K D S S V<br>121 CCTCTTACCAATTATACTTTTGGTACAAAAGAGCCCCTCTACGAGAAGGACAGCTCTGTT<br><br>61 A A R F Q R M R E E F D K I G M R R T V<br>181 GCAGCCAGATTTCAGCGCATGAGGGAAGAATTTGATAAAATTGGAATGAGGAGGACTGTA<br><br>81 E G V L I V H E H R L P H V L L L Q L G<br>241 GAAGGGGTTCTGATTGTACATGAGCACCGGCTACCCCATGTGTTACTGCTGCAGCTGGGA<br><br>101 T T F F K L P G D E L N P R E Y E V E G<br>301 ACAACTTTCTTCAAATTACCTGGTGATGAACTTAACCCAAGAGAATATGAAGTTGAAGGA<br><br>121 L K R L V T D I L G R Q D G D L Q D W V<br>361 CTAAAACGCTTAGTGACTGATATACTGGGTCGTCAAGATGGAGACCTGCAAGACTGGGTC<br><br>141 T D D R I G N W W R P N F E P P H Y P Y<br>421 ACTGATGACCGCATTGGTAACTGGTGGAGACCAAATTTTGAACCTCCTCACTATCCATAT<br><br>161 I P A H S T K H K E H K N L F L V Q L Q<br>481 ATTCCTGCACACAGTACAAAACATAAGGAGCATAAGAATTTGTTTCTGGTTCAGCTTCAA<br><br>181 E K A L F A V P K N Y K L V A A P L F E<br>541 GAAAAAGCCTTGTTTGCAGTCCCTAAAAATTACAAGCTGGTAGCTGCACCATTGTTTGAA | SEQ ID NO:154 |

| Gene Name | GenBank Homology | DNA SEQUENCE / DEDUCED AMINO ACID SEQUENCE | SEQUENCE IDENTIFIER: |
|---|---|---|---|
| | | 201 L Y D N A P G Y G P I I S S L P Q P L S<br>601 TTGTATGACAATGCACCAGGATATGGACCCATCATTTCTAGTCTCCCTCAGCCGTTGAGC<br><br>221 R F N F I Y N -<br>661 AGGTTCAATTTTATTTACAACTGA | |
| WBC033A01.bFSP_20 020801.esd | Homo sapiens mRNA; cDNA DKFZp686D0662 (from clone DKFZp686D0662). | 1 AGTTGGAAAGAGACCACAGACTTTGAGGGAAGCTCACTCAGGATCTGCTCTCCGGCAAAG<br>61 TAGTAAGTGAGGTGCTGAGAGCAGAATGAGCTACTTTGTGGATTCTGCTGGGAGCAGCCC<br>121 CGTCCCTTACTCAGCGCCTCGTCCTGCAGTGGTGAGGCAAGGACCTAGCAACACTTATGA<br>181 AGATCCTCGAATGAACTGTGGTTTCCAGTCCAATTATCACCAGCAAAGACCTTGCTACCC<br>241 CTTTTGGGATGAGATGGCAACTCAGGAAGTTCCTACTGGTCTTGAACACTGTGTCTCAGA<br>301 TATGGAATGTGCAGATGTCCCACTATTAACTCCAAGCAGCAAAGAAATGATGTCTCAAGC<br>361 ATTAAAAGCTACTTTCAGTGGTTTCACTAAAGAACAGCAACGACTGGGGATCCCAAAAGA<br>421 CCCCCGGCAGTGGACAGAAACCCATGTTCGGGACTGGGTGATGTGGGCTGTGAATGAATT<br>481 CAGCCTGAAAGGTGTAGACTTCCAGAAGTTCTGTATGAATGGAGCAGCCCTCTGCGCCCT<br>541 GGGTAAAGACTGCTTTCTCGAGCTGGCCCCAGACTTTGTTGGGGACATCTTATGGAACA<br>601 TCTAGAGATCCTGCAGAAAGAGGATGTGAAACCATATCAAGTTAATGGAGTCAACCCAGC<br>661 CTATCCAGAATCCCGCTATACCTCGGATTACTTCATTAGCTATGGTATTGAGCATGCCCA<br>721 GTGTGTTCCACCATCGGAGTTCTCAGAGCCCAGCTTCATCACAGAGTCCTATCAGACGCT<br>781 CCATCCCATCAGCTCGGAAGAGCTCCTCTCCCTCAAGTATGAGAATGACTACCCCTCGGT<br>841 CATTCTCCGAGACCCTCTCCAGACAGACACCTTGCAGAATGACTACTTTGCTATCAAACA<br>901 AGAAGTCGTCACCCCAGACAACATGTGCATGGGGAGGACCAGTCGTGGTAAACTCGGGGG<br>961 CCAGGACTCTTTTGAAAGCATAGAGAGCTACGATAGTTGTGATCGCCTCACCCAGTCCTG<br>1021 GAGCAGCCAGTCATCTTTCAACAGCCTGCAGCGTGTTCCCTCCTATGACAGCTTCGACTC<br>1081 AGAGGACTATCCGGCTGCCCTGCCCAACCACAAGCCCAAGGGCACCTTCAAGGACTATGT<br>1141 GCGGGACCGTGCTGACCTCAATAAGGACAAGCCTGTCATTCCTGCTGCTGCCCTAGCTGG<br>1201 CTACACAGGCAGTGGACCAATCCAGCTATGGCAGTTTCTTCTGGAATTACTCACTGATAA<br>1261 ATCCTGTCAGTCTTTTATCAGCTGGACAGGAGATGGCTGGGAATTCAAACTTTCTGACCC<br>1321 AGATGAGGTGGCCAGGAGATGGGGAAAGAGGAAAAACAAACCTAAGATGAATTATGAGAA<br>1381 ACTGAGCCGTGGCCTACGCTACTATTACGACAAAAACATCATCCACAAGACAGCGGGGAA<br>1441 ACGCTACGTGTACCGCTTTGTGTGTGACCTGCAGAGCCTGCTGGGGTACACCCCTGAGGA<br>1501 GCTGCACGCCATGCTGGACGTCAAGCCAGATGCCGACGAGTGATGGCACTGAAGGGGCTG<br>1561 GGGAAACCCTGCTGAGACCTTCCAAGGACAGCCGTGTTGGTTGGACTCTGAATTTTGAAT<br>1621 TGTTATTCTATTTTTTATTTTCCAGAACTCATTTTTTACCTTCAGGGGTGGGAGCTAAGT<br>1681 CAGTTGCAGCTGTAATCAATTGTGCGCAGTTGGGAAAGGAAAGCCAGGACTTGTGGGGTG<br>1741 GGTGGGACCAGAAATTCTTGAGCAAATTTTCAGGAGAGGGAGAAGGGCCTTCTCAGAAGC<br>1801 TTGAAGGCTCTGGCTTAACAGAGAAAGAGACTAATGTGTCCAATCATTTTTAAAAATCAT<br>1861 CCATGAAAAAGTGTCTTGAGTTGTGGACCCATTAGCAAGTGACATTGTCACATCAGAACT<br>1921 CATGGAACTGATGTAAGGCAATTAATTTGCTTCTGTTTTTAGGTCTGGGAGGGCAAAAAA<br>1981 GAGGTGGGTGGGATGAAACATGTTTTGGGGGGGGATGCACTGAAAATCTGAGAACTATTT<br>2041 ACCTATCACTCTAGTTTTGAAGCAAAGATGGACTTCAGTGGGGAGGGGCCAAAACCGTTG<br>2101 TTGTGTTAAAATTTATTTTATTAAATTTTGTGCCAGTATTTTTTTTCTTAAAAATCGTCT | SEQ ID NO:155 |

| Gene Name | GenBank Homology | DNA SEQUENCE / DEDUCED AMINO ACID SEQUENCE | SEQUENCE IDENTIFIER: |
|---|---|---|---|
| | | 2161 TAAGCTCTAAGGTGGTCTCAGTATTCGCGGTATCATGCAAGTTGGTTTTTATTTGCTGGC | |
| | | 2221 TGAGGATTCTGTCACAGTGAAAGAAAACTGTTTATATAGACCCCACTGGAAACGCAAAGG | |
| | | 2281 GCTGTCACTGAGATCGGGGATCCCAAATTCATGTGACTTATGTATGAAGGAAAAAATAAT | |
| | | 2341 GCTGATCTGAGTGCAAGGGAACTGTGCATGTGAATTTCATCTATAATTTAAAGAAGTTAT | |
| | | 2401 CACACCCTCTACTTATTTGCCATTAGTGAATTCTCGGGGTTTGGACTTAACGTTGAGCTA | |
| | | 2461 AGAAGCATTAAGTCTTTGAACTGAATGTATTTTGCAGCCCTGGTTTTGGACCACAGTAAA | |
| | | 2521 TGTAGGAGCTCTGTTGACACCATGGAAAGGAGGTCAAAGGAGGAAGATTGACTTGGTTGT | |
| | | 2581 TTATTGGTTCTATGCCTATAACATGTATGACTTGGAATAAAGGCTGTGTGCATGGGCATT | |
| | | 2641 ACCCCTCAGGTCTCAAGAAATAAGTCCTGAATGCATGTCATTCCGAACTAACACTCTACA | |
| | | 2701 CTTTCTCCTTTATGTTTTATTTTCCAAGAGTCCTCCATTTTTTGCACCCCCTCACCGCCA | |
| | | 2761 ACTCTGTTATTCAGTAGAGAGAAATGTACAGCTTTCTGATTGATGAGTGAAAAAGTAACT | |
| | | 2821 TGAGACACGACCTAAGTTGAAGAGTTTAGACTTGCTGAGTTTTAGAAGTGATGGAAATTA | |
| | | 2881 AGAGAGCATTTCAATAAAATGTGACTTGGCTGTCTTTGGAAGAGAAGTGCAAGGCTTTCC | |
| | | 2941 TTTGAAGAATTTAGGCTAGTTGAATTGGATGTCAGGTGAGAGTGTGTATGCAAAGTGAAT | |
| | | 3001 GGCACAGGTGATGCCAGGGCCTCTTGCTTGGGTCTGATGTCTTGGCACAGGGTAAGTGAA | |
| | | 3061 GGTTAATTCCAGAAGAGAGGAATGACTTGAAGGCAAAGGAAACTAAGGAAGGAGGTTCAG | |
| | | 3121 TGAGGAAAATAAGGTTGTCCATGAGATTTGAATAGATTTTTAGTTCCCCCAAGGTTTAAA | |
| | | 3181 TACAAACATAGTCAAGCAAGGTAGTCATCTTTCTGCTGGTTGTGAGGGGGAATCTGAAAA | |
| | | 3241 TGGAGTTTTAGAGGAAAAGTCAACATCTAACTAGTGAGGAAAAGTGCCTAATACAATTAG | |
| | | 3301 AATCTCCCTCACTCTATAGTTGCCCAGTTGAACGGATAAGGAGGAGGGGTGGCTTTTATG | |
| | | 3361 GACTTCCATGAGAGAAGGAAAGAAATATTTCAGGTAAGCTTCTCAGGGCTGGCCCTTTTT | |
| | | 3421 GGGATTTGGATGAGAAATTGGAAGTACTAACTACTTTCTAGCATATCTTTAAGAAAATTG | |
| | | 3481 ATTGTTATTTACTCCCAGATCCTCTTGCAGACCCAGAATTATCAGGAACATAGCTCTGTG | |
| | | 3541 ATTCATGAGTGTCCCCATACTGATGAATTGGAGCATCCATATGGAAAGCAAAGGCAGAAT | |
| | | 3601 TATCCCAGCTGTATTATTTTGATCTTTTGGATGCAGGTGCCTTAATGAAGCTCTCAAAAT | |
| | | 3661 ATTTTAGGAGCTGCTCAGGGAGTGTTGGGTGGAACTGTTTGGACTACATTGTTTTCTCTT | |
| | | 3721 AGATTATGTGATTTTTGTTGGGCACTGGCAAAAGGTGTGTGTGTGAATGTGTGCATGTGT | |
| | | 3781 GTGAATGTTGTGTGTGTGTGTGTGTGTGTGTGCGTGTTTGTGTGTGTGTGTGTGTGTTTG | |
| | | 3841 CAGACATGCAAAACTGCAGCTGAAATAATACCTTAGATTTCTAGGTAAGTCTTTCCACAT | |
| | | 3901 TTCAATAATGGGTAAGAGTAGAACCAGGGCCGGGTATCAATTATTGCTTGCTGTTTGCAA | |
| | | 3961 CCAGGCATAAAATCACTTTCTCAAATCATCCACCGTTCCTATTAAATTTATGCCGGAAAC | |
| | | 4021 ACTCCTTCTGTGAGTATAACTCCTGCAGTTCCTATAGCAGATAAGATATAAGAAAGTGCC | |
| | | 4081 TCCTAGTGCTCCTCCGCCCGCTTGTTTGCTAAAATTCCCTTTCTCTCTAAGTCCACCATT | |
| | | 4141 TTCAAGATTTGTAGATAGTGTATTAGTTAAGACAGCTTTGTCGATCTGGCCAGATGTTTT | |
| | | 4201 TTCTCCTTTGTCCAAAGGCCAGAGACCATCCCAGGAAGAGTGGTGGGTGGTTTATACACT | |
| | | 4261 GGAAATGTTGCGTTTATGCTTTTTAAAAACACACGTTAACTTCAGAGGAAGGATGGGCAA | |
| | | 4321 ATCTGGTCTAGCTGGGTGAAACCCTTATTTTCCCAGAGATGCCTTAACCTTTGTTGGTTT | |
| | | 4381 TGGCTTTAGGGTTCAGAGTCACTTTTGTTCCCTTCTCCATTCTGGAGAGGGACTTCCCCT | |
| | | 4441 ACATAGAGCCCTGATTTTTGTGGCTGTGGGGATTGGAGGTAGCATTCAAAGATCAGATGT | |
| | | 4501 GCTTTTCCTCACTTTGGAGATGAACACTCTGGGTTTTAGAGCATTAACCTGCCTAATCTT | |
| | | 4561 CATGGTGAAAATTACACCTTCTCTATTCTAGTCATGCTGTGCATGCTGCTGTCTCTGTTG | |
| | | 4621 GGGTCTATATAAATGTGTTGAAATGGTATCTACATTCCAAAGAAGTTTCAAGGAACCATA | |
| | | 4681 AATATATGTATACATATACATATATAAAATATATATATTAAAATAAAATTATCAGGAATA | |

| Gene Name | GenBank Homology | DNA SEQUENCE / DEDUCED AMINO ACID SEQUENCE | SEQUENCE IDENTIFIER: |
|---|---|---|---|
| | | 4741 CTGCCTCAGTTATTGAACTTTTTTTTTTAAGAATACTTTTTTTTTAAGCTGAGAAGTATA<br>4801 GGGGTGAAAAAGATGTTATATTGTGTTTGACTATTTTCCAACTTGTATTTTCATATAATT<br>4861 TATATTTTTTAAAAGCTGAAAATTTAAAAGCAAGATGAAAAAAGGAAAAGCAGGTGCTTT<br>4921 TTAAAAATCAGAACTGAGGTAGCTTAGAGATGTAGCGATGTAAGTGTCTATGTGTTTTTA<br>4981 AAAAAAAATGCAAAAAAATTCTTATGGGGGAGTTTTTTGTTTGTTTATTTTAGTAGCTGA<br>5041 TGCTGGCACATCATTTTGCTGGAGAGTTTTTTATATACTGTAGCCTGATTTCATATTGTA<br>5101 TTTTAAACTGTGTGAGATTAAAAACAAAGAATTTCATTCATAAAAAAAAAAAAAAAAAAA<br>5161 AAAAAAAAAAAAAAAAAAAAAAA | |
| | | 1 M S Y F V D S A G S S P V P Y S A P R P<br>1 ATGAGCTACTTTGTGGATTCTGCTGGGAGCAGCCCCGTCCCTTACTCAGCGCCTCGTCCT<br><br>21 A V V R Q G P S N T Y E D P R M N C G F<br>61 GCAGTGGTGAGGCAAGGACCTAGCAACACTTATGAAGATCCTCGAATGAACTGTGGTTTC<br><br>41 Q S N Y H Q Q R P C Y P F W D E M A T Q<br>121 CAGTCCAATTATCACCAGCAAAGACCTTGCTACCCCTTTTGGGATGAGATGGCAACTCAG<br><br>61 E V P T G L E H C V S D M E C A D V P L<br>181 GAAGTTCCTACTGGTCTTGAACACTGTGTCTCAGATATGGAATGTGCAGATGTCCCACTA<br><br>81 L T P S S K E M M S Q A L K A T F S G F<br>241 TTAACTCCAAGCAGCAAAGAAATGATGTCTCAAGCATTAAAAGCTACTTTCAGTGGTTTC<br><br>101 T K E Q Q R L G I P K D P R Q W T E T H<br>301 ACTAAAGAACAGCAACGACTGGGGATCCCAAAAGACCCCCGGCAGTGGACAGAAACCCAT<br><br>121 V R D W V M W A V N E F S L K G V D F Q<br>361 GTTCGGGACTGGGTGATGTGGGCTGTGAATGAATTCAGCCTGAAAGGTGTAGACTTCCAG<br><br>141 K F C M N G A A L C A L G K D C F L E L<br>421 AAGTTCTGTATGAATGGAGCAGCCCTCTGCGCCCTGGGTAAAGACTGCTTTCTCGAGCTG<br><br>161 A P D F V G D I L W E H L E I L Q K E D<br>481 GCCCCAGACTTTGTTGGGGACATCTTATGGGAACATCTAGAGATCCTGCAGAAAGAGGAT<br><br>181 V K P Y Q V N G V N P A Y P E S R Y T S<br>541 GTGAAACCATATCAAGTTAATGGAGTCAACCCAGCCTATCCAGAATCCCGCTATACCTCG<br><br>201 D Y F I S Y G I E H A Q C V P P S E F S<br>601 GATTACTTCATTAGCTATGGTATTGAGCATGCCCAGTGTGTTCCACCATCGGAGTTCTCA<br><br>221 E P S F I T E S Y Q T L H P I S S E E L | SEQ ID NO:156 |

<table>
<tr><th>Gene Name</th><th>GenBank Homology</th><th>DNA SEQUENCE / DEDUCED AMINO ACID SEQUENCE</th><th>SEQUENCE IDENTIFIER:</th></tr>
<tr><td></td><td></td><td>661 GAGCCCAGCTTCATCACAGAGTCCTATCAGACGCTCCATCCCATCAGCTCGGAAGAGCTC<br><br>241 L S L K Y E N D Y P S V I L R D P L Q T<br>721 CTCTCCCTCAAGTATGAGAATGACTACCCCTCGGTCATTCTCCGAGACCCTCTCCAGACA<br><br>261 D T L Q N D Y F A I K Q E V V T P D N M<br>781 GACACCTTGCAGAATGACTACTTTGCTATCAAACAAGAAGTCGTCACCCCAGACAACATG<br><br>281 C M G R T S R G K L G G Q D S F E S I E<br>841 TGCATGGGGAGGACCAGTCGTGGTAAACTCGGGGGCCAGGACTCTTTTGAAAGCATAGAG<br><br>301 S Y D S C D R L T Q S W S S Q S S F N S<br>901 AGCTACGATAGTTGTGATCGCCTCACCCAGTCCTGGAGCAGCCAGTCATCTTTCAACAGC<br><br>321 L Q R V P S Y D S F D S E D Y P A A L P<br>961 CTGCAGCGTGTTCCCTCCTATGACAGCTTCGACTCAGAGGACTATCCGGCTGCCCTGCCC<br><br>341 N H K P K G T F K D Y V R D R A D L N K<br>1021 AACCACAAGCCCAAGGGCACCTTCAAGGACTATGTGCGGGACCGTGCTGACCTCAATAAG<br><br>361 D K P V I P A A A L A G Y T G S G P I Q<br>1081 GACAAGCCTGTCATTCCTGCTGCTGCCCTAGCTGGCTACACAGGCAGTGGACCAATCCAG<br><br>381 L W Q F L L E L L T D K S C Q S F I S W<br>1141 CTATGGCAGTTTCTTCTGGAATTACTCACTGATAAATCCTGTCAGTCTTTTATCAGCTGG<br><br>401 T G D G W E F K L S D P D E V A R R W G<br>1201 ACAGGAGATGGCTGGGAATTCAAACTTTCTGACCCAGATGAGGTGGCCAGGAGATGGGGA<br><br>421 K R K N K P K M N Y E K L S R G L R Y Y<br>1261 AAGAGGAAAAACAAACCTAAGATGAATTATGAGAAACTGAGCCGTGGCCTACGCTACTAT<br><br>441 Y D K N I I H K T A G K R Y V Y R F V C<br>1321 TACGACAAAAACATCATCCACAAGACAGCGGGGAAACGCTACGTGTACCGCTTTGTGTGT<br><br>461 D L Q S L L G Y T P E E L H A M L D V K<br>1381 GACCTGCAGAGCCTGCTGGGGTACACCCCTGAGGAGCTGCACGCCATGCTGGACGTCAAG<br><br>481 P D A D E -<br>1441 CCAGATGCCGACGAGTGA</td><td></td></tr>
<tr><td>WBC033D12.bFSP_20020801.esd</td><td>No Homology</td><td>1 AGGGCCTGGGAAGCCCTAACAATCCTGCTGCTGTCACGGCAGCCCCACAGGGACCCCACA</td><td>SEQ ID NO:157</td></tr>
</table>

| Gene Name | GenBank Homology | DNA SEQUENCE / DEDUCED AMINO ACID SEQUENCE | SEQUENCE IDENTIFIER: |
|---|---|---|---|
| | | 61 CACTGCCCTGGGCAAGGACAGTGAGCTGTGCCCAAGGTCCAGATTGGCTGAGGCTCCTCC<br>121 CACCGCTCCTGTCCTCGCCAAACTCAGCTTCTCTGGACTGTGACGACAGCCCCAGGGCTG<br>181 GCTACCCTGACTGCCTCCCCGCCTCCAGTGTGATGCATTTTGTACCCCCTTACTTTTTAT<br>241 TTATTTATTCTTGCTGAGGAAGATTGGCCCTGAGCTAACATCTGTGCCAGTCTTCCTCTA<br>301 TTTTTGTATGTGGGTCGCTGCCACGGCACCACTGATGAATGGTGTAGGTCCGCACCCAGG<br>361 ATCTGAATCTGAACCGTGTGTACCCCTTTATTTTTCACAATACATGTCCCACATGGAAA<br>421 AACAGAACATTCTGGTAAGAAAAAAATAATTACAGTCAAACCTCCAAACCCAAAAAACCC<br>481 TACGCAGTGACTTAATATTTCTGTGTGAATCTTTCCAGGTTTTCACACTCTGAGTACACA<br>541 GACACACACACCTGTGCACGTGCACACACATGCCTTANAAAATTATAGTGGTTTGTTACC<br>601 TGTTAGGGTGTTTTAGCTTATTTAATCCTGGACGTGAAACAGGGTAAGCTGCCGTTTCTG<br>661 AGGGCTGCATGGCTTACACAGCGTGAACGTACCATAATTTTAAAATGCTCATGTCTTCAT<br>721 TTGCTTATCCAAGTGTAATTTAGAAGTGAAATTGCTGAGACAGAAGAAAAGAAGTATTTT<br>781 TAGAATTTTGATACTTACTGCCAAATTTTCTTTCAGGGAGTTTGCCCCAGTTTGTTGTCC<br>841 CTTCGGTGGTGTATGAAACTGTTTGTTTTCCCATAACTGAGCCTACGTGAGATATTTATA<br>901 TTGTCTTAATACCTTCCAATTTTATAAAAGGAAAATAGTCTCTTGTTTCATTATGCATTC<br>961 CTTTGGTTACTTGTGATTTGAATGTGTTTTTACTTGTTTAATGGCTTTTTTTCTTTTGGC<br>1021 AGATTGCTTCAGAGACATGTGAAATCTTCACATATGAAAATGTGTCACTCGCTTTCTTCT<br>1081 GCAACTCTCTAGTGTCAGCGTGTAGAGCCCAATACTTCCCAGTATTTTCTAAGACTTTAA<br>1141 CGTTTTCTTCCTACCTTTGATTTTTAAGTAGTATTTATTTTCAACCTACTTGAACTATAT<br>1201 TTTGGAATAAAATGTGAACTATAGAGCTAGATTGATTTGTTTATTTAAGTTAATAAATTA<br>1261 GAATGGCCCTTCTTTTGTTTTGGTATCAGTAAAAAAAAAAAA | |
| WBC035E01.bFSP_20 020901.abd | Homo sapiens splicing factor, arginine/serine-rich 2, mRNA (cDNA clone MGC:5480 IMAGE:3452024). | 1 CTGCGGAGCCCGTCCGTGCTGTTCTACGGCAAGGCCTTTCCCAGTGTCCCCACGCGGAAG<br>61 GCAACTGCCTGAGAGGCGCGGCGTCGCACCGCCCAGAGCTGAGGAAGCCGGCGCCAGTTC<br>121 GCGGGGCTCCGGGCCGCCACTCAGAGCTATGAGCTACGGCCGCCCCCCTCCCGATGTGGA<br>181 GGGTATGACCTCTCTCAAGGTGGACAACCTGACCTACCGCACCTCGCCCGACACGCTGAG<br>241 GCGCGTCTTCGAGAAGTACGGGCGCGTCGGCGACGTGTACATCCCGCGGGACCGCTACAC<br>301 CAAGGAGTCCCGCGGCTTCGCCTTCGTTCGCTTTCACGACAAGCGCGACGCTGAGGACGC<br>361 TATGGATGCCATGGACGGGGCCGTGCTGGACGGCCGCGAGCTGCGGGTGCAAATGGCGCG<br>421 CTACGGCCGCCCCCCGGACTCACACCACAGCCGCCGGGGACCGCCACCCCGCAGGTACGG<br>481 GGGCGGTGGCTACGGACGCCGGAGCCGCAGCCCTAGGCGGCGTCGCCGCAGCCGATCCCG<br>541 GAGTCGGAGCCGTTCCAGGTCTCGCAGCCGATCTCGCTACAGCCGCTCGAAGTCTCGGTC<br>601 CCGCACTCGTTCTCGATCTCGGTCGACCTCCAAGTCCAGATCCGCACGAAGGTCCAAGTC<br>661 CAAGTCCTCGTCGGTCTCCAGATCTCGTTCGCGGTCCAGGTCCCGGTCTCGGTCCAGGAG<br>721 TCCTCCCCCAGTGTCCAAGAGGGAATCCAAATCCAGGTCGCGATCGAAGAGTCCCCCCAA<br>781 GTCTCCTGAAGAGGAAGGAGCGGTGTCCTCTTAAGAAAATGATGTATCGGCAAGCAGTGT<br>841 AAACGGAGGACTTGGGGAAAAAGGACCACATAGTCCATCGAAGAAGAGTCCTTGGAGCGA<br>901 GCAGCTGGCTACCGAAAGGTTATTTTGTAACATTTGTCTAACTTCTTACTTGTTTTAAGC<br>961 TTTGCCTCAGGCGGCAAACTTCATTTTATGTGCCATTTTGTTGCTGTTATTCAAATTTCT<br>1021 TGTAATTTAGTGAGGTGAACGACTTCAGATTTCATTATTGGCTTGGATATTTGAGGTAAA<br>1081 ACTTCCTTTTTGTTTATATAGTGCTGACTTTTTTTGTTTGAAATTGAACAGATTGGTAAC<br>1141 CTAAATTGTGGCCTCCTGACTTTTTAAGGAAACGTGCAGCCATTACACACAGCCTAAAGC | SEQ ID NO:158 |

| Gene Name | GenBank Homology | DNA SEQUENCE / DEDUCED AMINO ACID SEQUENCE | SEQUENCE IDENTIFIER: |
|---|---|---|---|
| | | 1201 TGTCAAGAGATTGACTCAACATTGCCTTCATTCCTTAAAATTAAAACCTACGAAAGTTGG<br>1261 TGTAAATTTGTTTGTATATGTTATTTACCTGCAGATCTAAATGGTAATCTGAACCCAAAT<br>1321 TTGTGTAAGACTTTTCAGGTGAAAAGACTTGATTTTTTTGAGAGGATTGTTGACCAAACA<br>1381 CAATTCTAATCTCTTCTCTTATGTATTTTTGTGCACTAGGCGCAGTTGTGTAGCAGTTGA<br>1441 GTAATGCTGGTTAGCTGTTAAGGTGGCGTGTTGCAGTGCAGAGTGCTTGGCTGTTTCCTG<br>1501 TTTTCTCCTGATTGCTCCTGTGTAAAGATGCCTTGTCGTGCAGAAACAAATGGCTGTCCA<br>1561 GTTTATTAAAATGCCTGACAACTGCACTTCCAGTCACCCGGGCCTTGCATATAAATAACG<br>1621 GAGCATACAGTGAGCACATCTAGCTGATGATAAATACACCTTTTTTTCCCTCTTCCCCCT<br>1681 AAAAATGGTAAATCTGATCATATCTACATGTATGAACTTAACATGGAAAATGTTAAGGAA<br>1741 GCAAATGGTTGTAACTTTGTAAGTACTTATAACATGGTGTATCTTTTTGCTTATGAATAT<br>1801 TCTGTATTATAACCATTGTTTCTGTAGTTTAATTAAAACATTTTCTTGGTGTTAGCTTTT<br>1861 CTCAGAAAAAAAAAAAAAAA | |
| | | 1 M S Y G R P P P D V E G M T S L K V D N<br>1 ATGAGCTACGGCCGCCCCCCTCCCGATGTGGAGGGTATGACCTCTCTCAAGGTGGACAAC<br><br>21 L T Y R T S P D T L R R V F E K Y G R V<br>61 CTGACCTACCGCACCTCGCCCGACACGCTGAGGCGCGTCTTCGAGAAGTACGGGCGCGTC<br><br>41 G D V Y I P R D R Y T K E S R G F A F V<br>121 GGCGACGTGTACATCCCGCGGGACCGCTACACCAAGGAGTCCCGCGGCTTCGCCTTCGTT<br><br>61 R F H D K R D A E D A M D A M D G A V L<br>181 CGCTTTCACGACAAGCGCGACGCTGAGGACGCTATGGATGCCATGGACGGGGCCGTGCTG<br><br>81 D G R E L R V Q M A R Y G R P P D S H H<br>241 GACGGCCGCGAGCTGCGGGTGCAAATGGCGCGCTACGGCCGCCCCCCGGACTCACACCAC<br><br>101 S R R G P P P R R Y G G G G Y G R R S R<br>301 AGCCGCCGGGGACCGCCACCCCGCAGGTACGGGGGCGGTGGCTACGGACGCCGGAGCCGC<br><br>121 S P R R R R R S R S R S R S R S R S R S<br>361 AGCCCTAGGCGGCGTCGCCGCAGCCGATCCCGGAGTCGGAGCCGTTCCAGGTCTCGCAGC<br><br>141 R S R Y S R S K S R S R T R S R S R S T<br>421 CGATCTCGCTACAGCCGCTCGAAGTCTCGGTCCCGCACTCGTTCTCGATCTCGGTCGACC<br><br>161 S K S R S A R R S K S K S S S V S R S R<br>481 TCCAAGTCCAGATCCGCACGAAGGTCCAAGTCCAAGTCCTCGTCGGTCTCCAGATCTCGT<br><br>181 S R S R S R S R S R S P P P V S K R E S<br>541 TCGCGGTCCAGGTCCCGGTCTCGGTCCAGGAGTCCTCCCCCAGTGTCCAAGAGGGAATCC | SEQ ID NO:159 |

| Gene Name | GenBank Homology | DNA SEQUENCE / DEDUCED AMINO ACID SEQUENCE | SEQUENCE IDENTIFIER: |
|---|---|---|---|
| | | 201 K S R S R S K S P P K S P E E E G A V S<br>601 AAATCCAGGTCGCGATCGAAGAGTCCCCCCAAGTCTCCTGAAGAGGAAGGAGCGGTGTCC<br><br>221 S -<br>661 TCTTAA | |
| WBC036E08.bFSP_20 021001.abd | Homo sapiens annexin A2, mRNA | 1 GCTCAGCATTTGGGGACGCTCGCAGCCCTCGGCGTCCGGCCCAGCTTCTATCAAAATGTC<br>61 TACTGTTCACGAAATTCTGTGCAAGCTCAGCTTGGAGGGTGATCACTCTACCCCGGCAAG<br>121 TGCTTACGGGTCAGTCAAGGCGTACACCAACTTCGATGCCGAGCGGGATGCGCTGAACAT<br>181 TGAAACAGCCATCAAGACCAAAGGTGTGGACGAGGTCACCATCGTCAACATTCTGACCAA<br>241 CCGCAGCAATGAACAGAGACAAGATATTGCCTTCGCCTACCAGAGAAGGACCAAAAAGGA<br>301 GCTTGCAGCAGCGCTGAAGTCAGCCTTGTCTGGCCACCTGGAGACGGTGATTCTGGGCCT<br>361 GTTGAAAACACCTGCTCAGTATGATGCTTCTGAGCTGAAGGCTTCCATGAAGGGGCTGGG<br>421 GACCGATGAGGACTCCCTCATTGAGATCATCTGCTCAAGGACCAACCAGGAGCTGCAGGA<br>481 AATTAACAGAGTCTACAAGGAAATGTACAAGACTGATCTGGAGAAGGACATCGTTTCTGA<br>541 CACATCTGGTGACTTCCGCAAGCTGATGGTTGCCCTCGCAAAGGGTAGAAGAGCAGAGGA<br>601 TGGCTCTGTCATTGATTATGAACTGATTGACCAAGATGCCCGGGATCTCTATGATGCTGG<br>661 AGTGAAGAGGAAAGGAACTGATGTTCCAAAGTGGATCAGCATCATGACCGAGCGCAGTGT<br>721 CTGTCACCTCCAGAAAGTATTTGAAAGGTACAAGAGCTACAGCCCTTATGACATGTTGGA<br>781 GAGCATCAAGAAGGAGGTCAAAGGAGACCTGGAAAATGCTTTCCTGAACCTGGTCCAGTG<br>841 TATTCAGAACAAGCCCCTGTATTTTGCTGACCGACTGTACGACTCCATGAAGGGCAAGGG<br>901 CACGCGCGATAAGGTCCTGATCAGAATCATGGTCTCCCGCAGTGAAGTGGACATGTTGAA<br>961 AATTAGGTCTGAATTCAAGAGAAAGTACGGCAAGTCCCTGTACTACTACATCCAGCAAGA<br>1021 CACCAAGGGCGACTACCAGAAGGCGCTGCTGTACCTGTGTGGTGGGGATGACTGAAGCCC<br>1081 GACACGGCCTGAGCGTCAAGAAATGGTGCTCACCATGCTTCCTGCTAACAGTTCTAGAAA<br>1141 ACAGCTTGCGACTAACAGCCCCCTTGTGCCCATCCCTGTGAGGATGATGTTAGCCTTGTG<br>1201 CCCCCATCCTTATTTTAGATGCCTACGCGTTACCCGGCTTTCCCGTCTAGTCTCTCCTTT<br>1261 TAGCCAAAGAAATGAACATTCCAAGGAGTTGGCAGTGAAATCTATGATGTGAAACACTTT<br>1321 GCCTCTTGTGTACTGTGTCGTAAACAGATGAATAAACTGAGTTTTTACTTTAAAAAAAAA<br>1381 AAAAAAA<br><br>1 M S T V H E I L C K L S L E G D H S T P<br>1 ATGTCTACTGTTCACGAAATTCTGTGCAAGCTCAGCTTGGAGGGTGATCACTCTACCCCG<br><br>21 A S A Y G S V K A Y T N F D A E R D A L<br>61 GCAAGTGCTTACGGGTCAGTCAAGGCGTACACCAACTTCGATGCCGAGCGGGATGCGCTG<br><br>41 N I E T A I K T K G V D E V T I V N I L<br>121 AACATTGAAACAGCCATCAAGACCAAAGGTGTGGACGAGGTCACCATCGTCAACATTCTG<br><br>61 T N R S N E Q R Q D I A F A Y Q R R T K<br>181 ACCAACCGCAGCAATGAACAGAGACAAGATATTGCCTTCGCCTACCAGAGAAGGACCAAA | SEQ ID NO:160<br><br>SEQ ID NO:161 |

| Gene Name | GenBank Homology | DNA SEQUENCE / DEDUCED AMINO ACID SEQUENCE | SEQUENCE IDENTIFIER: |
|---|---|---|---|
| | | 81 K E L A A A L K S A L S G H L E T V I L<br>241 AAGGAGCTTGCAGCAGCGCTGAAGTCAGCCTTGTCTGGCCACCTGGAGACGGTGATTCTG<br><br>101 G L L K T P A Q Y D A S E L K A S M K G<br>301 GGCCTGTTGAAAACACCTGCTCAGTATGATGCTTCTGAGCTGAAGGCTTCCATGAAGGGG<br><br>121 L G T D E D S L I E I I C S R T N Q E L<br>361 CTGGGGACCGATGAGGACTCCCTCATTGAGATCATCTGCTCAAGGACCAACCAGGAGCTG<br><br>141 Q E I N R V Y K E M Y K T D L E K D I V<br>421 CAGGAAATTAACAGAGTCTACAAGGAAATGTACAAGACTGATCTGGAGAAGGACATCGTT<br><br>161 S D T S G D F R K L M V A L A K G R R A<br>481 TCTGACACATCTGGTGACTTCCGCAAGCTGATGGTTGCCCTCGCAAAGGGTAGAAGAGCA<br><br>181 E D G S V I D Y E L I D Q D A R D L Y D<br>541 GAGGATGGCTCTGTCATTGATTATGAACTGATTGACCAAGATGCCCGGGATCTCTATGAT<br><br>201 A G V K R K G T D V P K W I S I M T E R<br>601 GCTGGAGTGAAGAGGAAAGGAACTGATGTTCCAAAGTGGATCAGCATCATGACCGAGCGC<br><br>221 S V C H L Q K V F E R Y K S Y S P Y D M<br>661 AGTGTCTGTCACCTCCAGAAAGTATTTGAAAGGTACAAGAGCTACAGCCCTTATGACATG<br><br>241 L E S I K K E V K G D L E N A F L N L V<br>721 TTGGAGAGCATCAAGAAGGAGGTCAAAGGAGACCTGGAAAATGCTTTCCTGAACCTGGTC<br><br>261 Q C I Q N K P L Y F A D R L Y D S M K G<br>781 CAGTGTATTCAGAACAAGCCCCTGTATTTTGCTGACCGACTGTACGACTCCATGAAGGGC<br><br>281 K G T R D K V L I R I M V S R S E V D M<br>841 AAGGGCACGCGCGATAAGGTCCTGATCAGAATCATGGTCTCCCGCAGTGAAGTGGACATG<br><br>301 L K I R S E F K R K Y G K S L Y Y Y I Q<br>901 TTGAAAATTAGGTCTGAATTCAAGAGAAAGTACGGCAAGTCCCTGTACTACTACATCCAG<br><br>321 Q D T K G D Y Q K A L L Y L C G G D D -<br>961 CAAGACACCAAGGGCGACTACCAGAAGGCGCTGCTGTACCTGTGTGGTGGGGATGACTGA | |
| WBC037C09.bFSP_20021001.esd | Homo sapiens programmed cell death 4 | 1 AGTGACTCTCCGAGGCGGCGGCAGAGACAGAAGAGCGGGGTCGGGGCCGGCTGACCAGGA<br>61 ACCTGGGCGAGCAGCGGCGGGGGCCAGAGGGATTCTGAAGGAAGATTTCCATTAGGTAAT | SEQ ID NO:162 |

<table>
<tr><th>Gene Name</th><th>GenBank Homology</th><th>DNA SEQUENCE / DEDUCED AMINO ACID SEQUENCE</th><th>SEQUENCE IDENTIFIER:</th></tr>
<tr><td></td><td>(neoplastic transformation inhibitor), transcript variant 1, mRNA</td><td>121 TTGTTTAATCAGTGCAAGCGAAATTAAGGGAAAATGGATGTAGAAAATGAGCAGATACTG<br>181 AATGTAAACCCTGCAGATCCTGATAACTTAAGTGACTCTCTCTTTTCCGGTGATGAAGAA<br>241 AATGCTGGGACTGAGGAAGTAAAGAATGAAATAAATGGAAATTGGATTTCAGCATACTCC<br>301 ATTAACGAAGCTAGAATTAATGCCAAGGCAAAAAGGCGACTAAGGAAAAACTCATCCCGG<br>361 GACTCTGGCAGAGGCGATTCGGTCAGCGACAGTGGGAGTGACGCCCTTAGAAGTGGATTA<br>421 ACTGTGCCAACCAGTCCAAAGGGAAGGTTGCTGGATAGGCGATCCAGATCTGGGAAAGGA<br>481 AGGGGACTACCAAAGAAAGGTGGTGCAGGAGGCAAAGGTGTCTGGGGTACACCTGGACAG<br>541 GTGTATGATGTGGAGGAGGTGGATGTGAAGGATCCTAACTATGATGATGACCAGGAGAAC<br>601 TGTGTTTATGAAACTGTGGTTTTGCCTTTGGATGAAACGGCATTTGAGAAAACTTTAACA<br>661 CCAATCATACAGGAATATTTTGAGCATGGAGATACTAATGAAGTTGCGGAAATGCTAAGA<br>721 GATTTAAATCTTGGTGAAATGAAAAGTGGAGTACCAGTGTTGGCAGTGTCCTTGGCATTG<br>781 GAGGGAAAGGCTAGTCACAGAGAAATGACATCTAAGCTCCTTTCTGACCTTTGTGGGACG<br>841 GTAATGAGCACAAACGATGTAGAAAAGTCATTTGATAAATTGTTGAAAGATCTACCTGAA<br>901 TTAGCACTGGATACTCCTAGAGCACCACAGTTGGTGGGCCAGTTTATTGCTAGAGCTGTT<br>961 GGAGATGGAATTTTATGTAATACCTATATTGATAGTTACAAAGGAACTGTAGATTGTGTA<br>1021 CAGGCTAGAGCTGCCCTGGATAAGGCTACTGTGCTCCTGAGTATGTCTAAAGGTGGAAAG<br>1081 CGCAAAGACAGTGTGTGGGGCTCTGGAGGTGGGCAGCAGTCTGTTAACCACCTTGTTAAA<br>1141 GAGATTGATATGCTGCTGAAAGAATATTTACTCTCTGGAGACATATCTGAGGCTGAACAT<br>1201 TGCCTTAAGGAACTGGAAGTACCTCATTTTCACCATGAGCTTGTATATGAAGCCATTGTA<br>1261 ATGGTTTTAGAGTCAACTGGAGAAAGTACATTTAAGATGATTTTGGATTTATTAAAGTCC<br>1321 CTTTGGAAGTCTTCTACCATTACTGTAGACCAAATGAAAAGAGGTTATGAGAGAATTTAC<br>1381 AATGAAATTCCGGACATTAATCTGGATGTCCCACATTCATACTCTGTGCTGGAGCGGTTT<br>1441 GTAGAAGAATGTTTTCAGGCTGGAATAATTTCCAAACAACTCAGAGATCTTTGTCCTTCA<br>1501 AGGGGCAGAAAGCGTTTTGTAAGCGAAGGAGATGGAGGTCGTCTTAAACCAGAGAGCTAC<br>1561 TGAATATAAGAACTCTTGCAGTCTTAGATGTTATAAAAATATATATCTGAATTGTAAGAG<br>1621 TTGTTAGCACAAGTTTTTTTTTTTTTTTTTTTAAGCACTTGTTTTGGGTACAAGGCATTT<br>1681 CTGACATTTTATAAACCTACATTTAAGGGGAATTTTTAAAGGAAATGTTTTTTCTTTTTT<br>1741 TTTTGTTTTTCGAGGGGGCAAGGAGGGACAGAAAAGTAACCACTTCTTAAGTGGAATATT<br>1801 CTCATAAGCTACCTTTTGTAAGTGCCATGTTTATGACCTAATCATTCCAAGTTTTGCATT<br>1861 GATGTCTGACTGCCACTCCTTTCTTTCAAGGACAGTGTTTTTGTAGTAAAATCACTGGTT<br>1921 TATACAAAGCTTTATTTAGGGGGTAAAGTTAAGCTGCTAAAACCCCATGTTGGCTGCTGC<br>1981 TGTTGAAATACTGTGCTTTGGGAGTAAAAAAAGAAAGTTATTTCTTTGTCTTAAAGAATT<br>2041 TTAAGAAAACGAGTTAGTCACAAGACTTATTCATCTTTCCAGGGAGCATACTGATTGGTC<br>2101 TTAAAGACTAGACAGTTAAGTAAATGGTGGCCGGAACATCTATTTTTCTACAAACTGGAA<br>2161 AAATGAACCCAGTTCTAGAAGAATGTATGACAAAATAAAACATGTGAAGCAGTAAAAAAA<br>2221 AAAAAAAAA<br><br>1 M D V E N E Q I L N V N P A D P D N L S<br>1 ATGGATGTAGAAAATGAGCAGATACTGAATGTAAACCCTGCAGATCCTGATAACTTAAGT<br><br>21 D S L F S G D E E N A G T E E V K N E I<br>61 GACTCTCTCTTTTCCGGTGATGAAGAAAATGCTGGGACTGAGGAAGTAAAGAATGAAATA</td><td>SEQ ID NO:163</td></tr>
</table>

| Gene Name | GenBank Homology | DNA SEQUENCE / DEDUCED AMINO ACID SEQUENCE | SEQUENCE IDENTIFIER: |
|---|---|---|---|
| | | 41 N G N W I S A Y S I N E A R I N A K A K<br>121 AATGGAAATTGGATTTCAGCATACTCCATTAACGAAGCTAGAATTAATGCCAAGGCAAAA<br><br>61 R R L R K N S S R D S G R G D S V S D S<br>181 AGGCGACTAAGGAAAAACTCATCCCGGGACTCTGGCAGAGGCGATTCGGTCAGCGACAGT<br><br>81 G S D A L R S G L T V P T S P K G R L L<br>241 GGGAGTGACGCCCTTAGAAGTGGATTAACTGTGCCAACCAGTCCAAAGGGAAGGTTGCTG<br><br>101 D R R S R S G K G R G L P K K G G A G G<br>301 GATAGGCGATCCAGATCTGGGAAAGGAAGGGGACTACCAAAGAAAGGTGGTGCAGGAGGC<br><br>121 K G V W G T P G Q V Y D V E E V D V K D<br>361 AAAGGTGTCTGGGGTACACCTGGACAGGTGTATGATGTGGAGGAGGTGGATGTGAAGGAT<br><br>141 P N Y D D D Q E N C V Y E T V V L P L D<br>421 CCTAACTATGATGATGACCAGGAGAACTGTGTTTATGAAACTGTGGTTTTGCCTTTGGAT<br><br>161 E T A F E K T L T P I I Q E Y F E H G D<br>481 GAAACGGCATTTGAGAAAACTTTAACACCAATCATACAGGAATATTTTGAGCATGGAGAT<br><br>181 T N E V A E M L R D L N L G E M K S G V<br>541 ACTAATGAAGTTGCGGAAATGCTAAGAGATTTAAATCTTGGTGAAATGAAAAGTGGAGTA<br><br>201 P V L A V S L A L E G K A S H R E M T S<br>601 CCAGTGTTGGCAGTGTCCTTGGCATTGGAGGGAAAGGCTAGTCACAGAGAAATGACATCT<br><br>221 K L L S D L C G T V M S T N D V E K S F<br>661 AAGCTCCTTTCTGACCTTTGTGGGACGGTAATGAGCACAAACGATGTAGAAAAGTCATTT<br><br>241 D K L L K D L P E L A L D T P R A P Q L<br>721 GATAAATTGTTGAAAGATCTACCTGAATTAGCACTGGATACTCCTAGAGCACCACAGTTG<br><br>261 V G Q F I A R A V G D G I L C N T Y I D<br>781 GTGGGCCAGTTTATTGCTAGAGCTGTTGGAGATGGAATTTTATGTAATACCTATATTGAT<br><br>281 S Y K G T V D C V Q A R A A L D K A T V<br>841 AGTTACAAAGGAACTGTAGATTGTGTACAGGCTAGAGCTGCCCTGGATAAGGCTACTGTG<br><br>301 L L S M S K G G K R K D S V W G S G G G<br>901 CTCCTGAGTATGTCTAAAGGTGGAAAGCGCAAAGACAGTGTGTGGGGCTCTGGAGGTGGG<br><br>321 Q Q S V N H L V K E I D M L L K E Y L L | |

| Gene Name | GenBank Homology | DNA SEQUENCE / DEDUCED AMINO ACID SEQUENCE | SEQUENCE IDENTIFIER: |
|---|---|---|---|
| | | 961 CAGCAGTCTGTTAACCACCTTGTTAAAGAGATTGATATGCTGCTGAAAGAATATTTACTC<br><br>341 S G D I S E A E H C L K E L E V P H F H<br>1021 TCTGGAGACATATCTGAGGCTGAACATTGCCTTAAGGAACTGGAAGTACCTCATTTTCAC<br><br>361 H E L V Y E A I V M V L E S T G E S T F<br>1081 CATGAGCTTGTATATGAAGCCATTGTAATGGTTTTAGAGTCAACTGGAGAAAGTACATTT<br><br>381 K M I L D L L K S L W K S S T I T V D Q<br>1141 AAGATGATTTTGGATTTATTAAAGTCCCTTTGGAAGTCTTCTACCATTACTGTAGACCAA<br><br>401 M K R G Y E R I Y N E I P D I N L D V P<br>1201 ATGAAAAGAGGTTATGAGAGAATTTACAATGAAATTCCGGACATTAATCTGGATGTCCCA<br><br>421 H S Y S V L E R F V E E C F Q A G I I S<br>1261 CATTCATACTCTGTGCTGGAGCGGTTTGTAGAAGAATGTTTTCAGGCTGGAATAATTTCC<br><br>441 K Q L R D L C P S R G R K R F V S E G D<br>1321 AAACAACTCAGAGATCTTTGTCCTTCAAGGGGCAGAAAGCGTTTTGTAAGCGAAGGAGAT<br><br>461 G G R L K P E S Y -<br>1381 GGAGGTCGTCTTAAACCAGAGAGCTACTGA | |
| WBC038A02.bFSP_20 021101.esd | Homo sapiens ribosomal protein L3, mRNA (cDNA clone MGC:70878 IMAGE:3852576). | 1 GTTTGATGGCGTGATGTCTCACAGAAAGTTCTCCGCCCCCAGGCACGGGTCCCTGGGCTT<br>61 CTTGCCTCGGAAACGCAGCAGCCGGCACCGCGGCAAGGTGAAGAGTTTCCCCAAGGATGA<br>121 CGCTTCGAAGCCCGTGCACCTCACGGCCTTCCTTGGCTACCAGGGCGGCATGACCCACAT<br>181 CCTGCGTGAAGTCCATAGGCCCGGATTCCAAGTGAACCAGAAAGAAGTTGTGGAAGCTGT<br>241 GACCATTGTGGAGACGCCACCCATGGTGGTTGTGGGCATTGTGGGCTACGTGGAAACCCC<br>301 TCGGGGCCCTCGTACCTTCAAGACCATCTTTGCTGAGCACATAAGTGATGAGTGCAAAAG<br>361 GCGCTTCTACAAGAACTGGCATAAGTCTAAGAAGAAGGCCTTCACCAAATACTGCAAGAA<br>421 GTGGCAGGATGAGGACGGCAGGAAGCAGCTGGAGAAGGATTTCAGCAGCATGAAGAAGTA<br>481 CTGCCAGGTCATCCGTGTCATCGCCCACACCCAGATGCGCCTGCTCCCTCTGCGCCAGAA<br>541 GAAGGCGCATCTCATGGAGATCCAGGTGAACGGAGGCACAGTGGCCGAGAAACTGGACTG<br>601 GGCCCGCGAGAGGCTGGAGCAGCAGGTCCCTGTGAACCAGGTGTTTGGGCAGGACGAGAT<br>661 GATCGACGTCATCGGGGTGACCAAGGGCAAAGGCTACAAAGGGGTCACCAGCCGCTGGCA<br>721 CACCAAGAAGCTGCCCCGCAAGACCCACCGAGGGCTGCGCAAGGTTGCCTGCATTGGGGC<br>781 CTGGCATCCCGCCCGAGTGGCCTTCTCCGTGGCGCGGGCCGGGCAGAAGGGCTACCATCA<br>841 CCGCACGGAGATCAACAAGAAGATCTACAAGATTGGCCAGGGCTACCTCATCAAGGACGG<br>901 CAAGCTGATCAAGAACAATGCCTCCACTGATTACGACCTGTCTGACAAGAGCATCAACCC<br>961 TCTGGGTGGCTTTGTCCACTACGGTGAAGTGACCAACGACTTTGTCATGCTGAAAGGCTG<br>1021 TGTGGTGGGAACCAAGAAGCGAGTGCTGACCCTTCGCAAGTCCCTGCTGGTGCAGACGAA<br>1081 GCGGCGGGCCCTGGAGAAGGTTGACCTCAAGTTCATCGACACCACCTCCAAGTTTGGCCA | SEQ ID NO:164 |

| Gene Name | GenBank Homology | DNA SEQUENCE / DEDUCED AMINO ACID SEQUENCE | SEQUENCE IDENTIFIER: |
|---|---|---|---|
| | | 1141 TGGCCGCTTCCAGACTGTGGAGGAGAAGAAAGCATTCATGGGACCACTTAAGAAAGACCG<br>1201 AATTGCAAAGGAGGAAGGAGCTTGATGCCAGCATCAGATCGTGCAGCTGGTGGGGTCCCA<br>1261 ATAAAAGTTATTTTCCACTGAAAAAAAAAAAAAAAAAAA | |
| | | 1 M S H R K F S A P R H G S L G F L P R K<br>1 ATGTCTCACAGAAAGTTCTCCGCCCCCAGGCACGGGTCCCTGGGCTTCTTGCCTCGGAAA<br><br>21 R S S R H R G K V K S F P K D D A S K P<br>61 CGCAGCAGCCGGCACCGCGGCAAGGTGAAGAGTTTCCCCAAGGATGACGCTTCGAAGCCC<br><br>41 V H L T A F L G Y Q G G M T H I L R E V<br>121 GTGCACCTCACGGCCTTCCTTGGCTACCAGGGCGGCATGACCCACATCCTGCGTGAAGTC<br><br>61 H R P G F Q V N Q K E V V E A V T I V E<br>181 CATAGGCCCGGATTCCAAGTGAACCAGAAAGAAGTTGTGGAAGCTGTGACCATTGTGGAG<br><br>81 T P P M V V V G I V G Y V E T P R G P R<br>241 ACGCCACCCATGGTGGTTGTGGGCATTGTGGGCTACGTGGAAACCCCTCGGGGCCCTCGT<br><br>101 T F K T I F A E H I S D E C K R R F Y K<br>301 ACCTTCAAGACCATCTTTGCTGAGCACATAAGTGATGAGTGCAAAAGGCGCTTCTACAAG<br><br>121 N W H K S K K K A F T K Y C K K W Q D E<br>361 AACTGGCATAAGTCTAAGAAGAAGGCCTTCACCAAATACTGCAAGAAGTGGCAGGATGAG<br><br>141 D G R K Q L E K D F S S M K K Y C Q V I<br>421 GACGGCAGGAAGCAGCTGGAGAAGGATTTCAGCAGCATGAAGAAGTACTGCCAGGTCATC<br><br>161 R V I A H T Q M R L L P L R Q K K A H L<br>481 CGTGTCATCGCCCACACCCAGATGCGCCTGCTCCCTCTGCGCCAGAAGAAGGCGCATCTC<br><br>181 M E I Q V N G G T V A E K L D W A R E R<br>541 ATGGAGATCCAGGTGAACGGAGGCACAGTGGCCGAGAAACTGGACTGGGCCCGCGAGAGG<br><br>201 L E Q Q V P V N Q V F G Q D E M I D V I<br>601 CTGGAGCAGCAGGTCCCTGTGAACCAGGTGTTTGGGCAGGACGAGATGATCGACGTCATC<br><br>221 G V T K G K G Y K G V T S R W H T K K L<br>661 GGGGTGACCAAGGGCAAAGGCTACAAAGGGGTCACCAGCCGCTGGCACACCAAGAAGCTG<br><br>241 P R K T H R G L R K V A C I G A W H P A<br>721 CCCCGCAAGACCCACCGAGGGCTGCGCAAGGTTGCCTGCATTGGGGCCTGGCATCCCGCC | *SEQ ID NO:165* |

| Gene Name | GenBank Homology | DNA SEQUENCE / DEDUCED AMINO ACID SEQUENCE | SEQUENCE IDENTIFIER: |
|---|---|---|---|
| | | 261 R V A F S V A R A G Q K G Y H H R T E I<br>781 CGAGTGGCCTTCTCCGTGGCGCGGGCCGGGCAGAAGGGCTACCATCACCGCACGGAGATC<br><br>281 N K K I Y K I G Q G Y L I K D G K L I K<br>841 AACAAGAAGATCTACAAGATTGGCCAGGGCTACCTCATCAAGGACGGCAAGCTGATCAAG<br><br>301 N N A S T D Y D L S D K S I N P L G G F<br>901 AACAATGCCTCCACTGATTACGACCTGTCTGACAAGAGCATCAACCCTCTGGGTGGCTTT<br><br>321 V H Y G E V T N D F V M L K G C V V G T<br>961 GTCCACTACGGTGAAGTGACCAACGACTTTGTCATGCTGAAAGGCTGTGTGGTGGGAACC<br><br>341 K K R V L T L R K S L L V Q T K R R A L<br>1021 AAGAAGCGAGTGCTGACCCTTCGCAAGTCCCTGCTGGTGCAGACGAAGCGGCGGGCCCTG<br><br>361 E K V D L K F I D T T S K F G H G R F Q<br>1081 GAGAAGGTTGACCTCAAGTTCATCGACACCACCTCCAAGTTTGGCCATGGCCGCTTCCAG<br><br>381 T V E E K K A F M G P L K K D R I A K E<br>1141 ACTGTGGAGGAGAAGAAAGCATTCATGGGACCACTTAAGAAAGACCGAATTGCAAAGGAG<br><br>401 E G A -<br>1201 GAAGGAGCTTGA | |
| WBC038B01.bFSP_20 021101.esd | Homo sapiens ribosomal protein L15, mRNA. | 1 CCTTTCCGTCTGGCGGCAGCCATCAGGTAAGCCAAGATGGGCGCTTACAAGTACATCCAG<br>61 GAGCTATGGAGGAAGAAGCAGTCGGACGTGATGCGATTTCTTCTCAGGGTGCGCTGCTGG<br>121 CAGTACCGCCAGCTCTCGGCGCTCCACAGGGCGCCCCGCCCCACCCGGCCCGATAAGGCG<br>181 CGCAGACTGGGCTACAAGGCCAAGCAAGGTTATGTCATATATCGCATTCGTGTGCGCCGT<br>241 GGTGGCCGCAAACGCCCCGTTCCTAAGGGCGCGACCTACGGCAAGCCTGTGCATCATGGT<br>301 GTTAACCAGCTCAAGTTTGCTCGAAGCCTTCAGTCTGTTGCCGAGGAACGAGCTGGACGC<br>361 CACTGTGGGGCTCTGAGAGTTCTGAATTCTTACTGGGTTGGCGAAGATTCCACATACAAA<br>421 TTCTTTGAGGTGATCCTCATTGATCCATTCCATAAGGCTATTAGAAGAAATCCTGATACC<br>481 CAGTGGATCACCAAACCAGTCCACAAGCACAGGGAAATGAGAGGGCTGACATCTGCAGGC<br>541 CGCAAGAGCCGTGGCCTTGGAAAGGGCCACAAGTTCCACCACACTATTGGTGGTTCTCGC<br>601 CGTGCAGCTTGGAGAAGGCGCAATACTCTGCAGCTTCACCGTTACCGCTAATATAAGTAA<br>661 TGTTTGTAAAATTCTTACCTAATAAACAATTTAGGACAGTCATGTCTGCTTACAGGTGTT<br>721 ATTTGTCTGTTAAAACTAGTCTGCAGATGTTTCTTGAATGCTTTGTCAAATTAAGAAAGT<br>781 TAAAGTGCAATAATGTTTGAAGACAATAAGTGGTGGTGTATCTTGTTTCTAATAAGATAA<br>841 ACTTTTTTGTCTTTGCTTTATCTTATTAGGGAGTTGTATGTCAGTGTATAAAACATACTG<br>901 TGTGGTATAACAGGCTTAATAAATTCTCCAAAAAAAAAAAAAAAAAAAAAAAAAAAAAAA<br><br>1 M G A Y K Y I Q E L W R K K Q S D V M R | SEQ ID NO:166<br><br>SEQ ID NO:167 |

| Gene Name | GenBank Homology | DNA SEQUENCE / DEDUCED AMINO ACID SEQUENCE | SEQUENCE IDENTIFIER: |
|---|---|---|---|
| | | 1 ATGGGCGCTTACAAGTACATCCAGGAGCTATGGAGGAAGAAGCAGTCGGACGTGATGCGA<br><br>21 F L L R V R C W Q Y R Q L S A L H R A P<br>61 TTTCTTCTCAGGGTGCGCTGCTGGCAGTACCGCCAGCTCTCGGCGCTCCACAGGGCGCCC<br><br>41 R P T R P D K A R R L G Y K A K Q G Y V<br>121 CGCCCCACCCGGCCCGATAAGGCGCGCAGACTGGGCTACAAGGCCAAGCAAGGTTATGTC<br><br>61 I Y R I R V R R G G R K R P V P K G A T<br>181 ATATATCGCATTCGTGTGCGCCGTGGTGGCCGCAAACGCCCCGTTCCTAAGGGCGCGACC<br><br>81 Y G K P V H H G V N Q L K F A R S L Q S<br>241 TACGGCAAGCCTGTGCATCATGGTGTTAACCAGCTCAAGTTTGCTCGAAGCCTTCAGTCT<br><br>101 V A E E R A G R H C G A L R V L N S Y W<br>301 GTTGCCGAGGAACGAGCTGGACGCCACTGTGGGGCTCTGAGAGTTCTGAATTCTTACTGG<br><br>121 V G E D S T Y K F F E V I L I D P F H K<br>361 GTTGGCGAAGATTCCACATACAAATTCTTTGAGGTGATCCTCATTGATCCATTCCATAAG<br><br>141 A I R R N P D T Q W I T K P V H K H R E<br>421 GCTATTAGAAGAAATCCTGATACCCAGTGGATCACCAAACCAGTCCACAAGCACAGGGAA<br><br>161 M R G L T S A G R K S R G L G K G H K F<br>481 ATGAGAGGGCTGACATCTGCAGGCCGCAAGAGCCGTGGCCTTGGAAAGGGCCACAAGTTC<br><br>181 H H T I G G S R R A A W R R R N T L Q L<br>541 CACCACACTATTGGTGGTTCTCGCCGTGCAGCTTGGAGAAGGCGCAATACTCTGCAGCTT<br><br>201 H R Y R -<br>601 CACCGTTACCGCTAA | |
| WBC038D08.bFSP_20 021101.esd | No homology | 1 GGACGTCAGTGAGAGCAGAGATGGCTGTGGGTGTGGAGTGAGGGTTCTACTGTGAGGTGA<br>61 GCTGCTCACTGCCGAGGAGAGTTACCGCAACCTGCACTCCGCGGCCACCTCACACTCTCG<br>121 ACTTGTCCAAACTGAATTCTTTCTTTCCTTTACCCGAACTTGTTATCCCTTTCAGCGTGG<br>181 GTTAAGGTGGGCACAGGAGGTTTATGAACCCCCTGAAATTGTATATGGAAGTTGGTGTAT<br>241 ATATGGCAAATGTATTTTTTTTTCCCCTAAGGAAAGGGTCCATATTATTCATCAGATTCT<br>301 GAATGGGATCTGTAATAGAAAAGTTTAAAAATTCCTATTTTGTAGGGATATAGAAAATTT<br>361 AAAAGGGATTTTAGAAGAAATATACAAAGATAAATCTGGGGGAGTCAGTGAGGAAAAAA<br>421 TTAAGGAACTGTGATTTAATCTGGAGAATGGAATAAATTTAAGATATGTAGGGTATAAGG<br>481 AAGAACTTGAAAGATTCTAGAAAGGGTCAGAAAGTTAATATTTATATATGTGAAAAAGTC<br>541 TGAGGAGAGTAATTCTCATTTAATTCTCTACGTACCTTGCAACTTAAATTATCCCATTGC | SEQ ID NO:168 |

| Gene Name | GenBank Homology | DNA SEQUENCE / DEDUCED AMINO ACID SEQUENCE | SEQUENCE IDENTIFIER: |
|---|---|---|---|
| | | 601 TCCTAGTATAGAACATCCTTATCCAGAACAAGTTTGCACAAAATTTATCTCAGTGTGCAT<br>661 TTGTTTCTTTTATTTTTATCTAACTTATTTCTATATTATAAAATA | |
| | | 1 GTAGATGATCATTGGTGAGTGGGCATGGACAGGAAAAAAAGAGTCTGAAGTTAAGTGCAA<br>61 GAAGAAATGAGCAGGTAAAGCCAACGTTTTTGCTTCAAATGTTGCAATGAGATGGAAGAA<br>121 TAGGCTGCTTGATGTGTATTAAAAGAATGCTTGGTACAAGGGGAAAAAGTTGTATCTTGG<br>181 CTGCAAGATACCATTTAAGAATATCCTAGTGAAGTTATTTGACTATGGGTTTGGCAGGAC<br>241 TTCTGAAAAAAGAAAATAATAATAACCATGATATACATAGCCATGGTTAATCTGTTTGCT<br>301 TTTCACTTACTTTACAAGTATAATAAAACTTGCCAAAACAAGTCGTTATCCATCTTTCTT<br>361 TTTCTTTTTTTTTTTTGCAGGGACCTTGCTGCCTTTGATAAATCCCATGATCAAGCCATC<br>421 CGTACCTACCAAGAGCACAGAGCAAGTATGCACCCTGTGACTGCCATGCTGGTGGGAAAA<br>481 GATTTAAAAGTGGATTAAGGACCTGCCTATTGCTGATTCCACACATTTCTCTTTTTTACA<br>541 TTGAATTCATAAATAAATATGAAACTTCAGCTTGCA | SEQ ID NO:169 |
| WBC038E02.bFSP_20 021101.esd | Homo sapiens lysosomal-associated membrane protein 1, mRNA. | 1 GGCCCAACCGCCGCCCGCGCCCCCGCTCTCCGCACCGTACCCGGCCGCCTCGCGCCATGG<br>61 CGGCCCCCGGCAGCGCCCGGCGACCCCTGCTGCTGCTACTGCTGTTGCTGCTGCTCGGCC<br>121 TCATGCATTGTGCGTCAGCAGCAATGTTTATGGTGAAAAATGGCAACGGGACCGCGTGCA<br>181 TAATGGCCAACTTCTCTGCTGCCTTCTCAGTGAACTACGACACCAAGAGTGGCCCTAAGA<br>241 ACATGACCTTTGACCTGCCATCAGATGCCACAGTGGTGCTCAACCGCAGCTCCTGTGGAA<br>301 AAGAGAACACTTCTGACCCCAGTCTCGTGATTGCTTTTGGAAGAGGACATACACTCACTC<br>361 TCAATTTCACGAGAAATGCAACACGTTACAGCGTCCAGCTCATGAGTTTTGTTTATAACT<br>421 TGTCAGACACACACCTTTTCCCCAATGCGAGCTCCAAAGAAATCAAGACTGTGGAATCTA<br>481 TAACTGACATCAGGGCAGATATAGATAAAAAATACAGATGTGTTAGTGGCACCCAGGTCC<br>541 ACATGAACAACGTGACCGTAACGCTCCATGATGCCACCATCCAGGCGTACCTTTCCAACA<br>601 GCAGCTTCAGCAGGGGAGAGACACGCTGTGAACAAGACAGGCCTTCCCCAACCACAGCGC<br>661 CCCCTGCGCCACCCAGCCCCTCGCCCTCACCCGTGCCCAAGAGCCCCTCTGTGGACAAGT<br>721 ACAACGTGAGCGGCACCAACGGGACCTGCCTGCTGGCCAGCATGGGGCTGCAGCTGAACC<br>781 TCACCTATGAGAGGAAGGACAACACGACGGTGACAAGGCTTCTCAACATCAACCCCAACA<br>841 AGACCTCGGCCAGCGGGAGCTGCGGCGCCCACCTGGTGACTCTGGAGCTGCACAGCGAGG<br>901 GCACCACCGTCCTGCTCTTCCACTTTGGAATGAATGCAACTTCTAGCCAGTTTTTCCTGC<br>961 AAGAAATCCAGTTGGATATGACTCTTCCTGACGCCAGAGACCCCACCTTCAAGGCTGCCA<br>1021 ACAGCTCGCTGAGGGCACTTCAGGCCACCATCGGGAACTCCTACAGGTGTAATGCCGAGG<br>1081 AGCGCCTGCGCGTCACAGAGGCCCTTTCTGTCAACGTCTTCAGAGTGTGGGTTCAGGCTT<br>1141 TCCAGGTGGAAGGTGACAAGTTTGGGTCCGTGGAGGAATGCCAGCTGGATGAGAACAACA<br>1201 TGATGATCCCCATTGCCGTGGGCGGTGCCCTGGCCGGGCTGGTCCTCATCGTCCTCATTG<br>1261 CCTACCTCATCGGCAGGAAGAGGAGCCACGCGGGCTACCAGACTATCTAGCCTGGTGCAC<br>1321 GCAGGCACAGCAGCTGCAGGGGCCTCTGTTCCTTTCTCTGGGCTTAGGGTCCTGTCGAAG<br>1381 GGGAGGCACACTTGCCTGCACACGATTTCCAAATCTGCTTTATCCAATTTGAAGTTCATC<br>1441 TTGCAACATTTACTATGCACAAAAGAGTAACTATTGAAATGACGGTGTTAATTTTGCTAA<br>1501 CTGGGTTAAATATTTTGCTAACTGGTTAAATGTTAATATTTACCAAAGTAGGATTTTGAG<br>1561 GGTGGGGGTGCTCTCTCTGAGGGGGTGGGGGTGCCGCTGTCTCTGAGGGGTGGGGGTGCC<br>1621 GCTGTCTCTGAGGGGTGGGGGTGCCGCTCTCTCTGAGGGGGTGGGGGTGCCGCTTTCTCT | SEQ ID NO:170 |

| Gene Name | GenBank Homology | DNA SEQUENCE / DEDUCED AMINO ACID SEQUENCE | SEQUENCE IDENTIFIER: |
|---|---|---|---|
| | | 1681 GAGGGGGTGGGGGTGCCGCTCTCTCTGAGGGGGTGGGGGTGCTGCTCTCTCCGAGGGGTG<br>1741 GAATGCCGCTGTCTCTGAGGGGTGGGGGTGCCGCTCTAAATTGGCTCCATATCATTTGAG<br>1801 TTTAGGGTTCTGGTGTTTGGTTTCTTCATTCTTTACTGCACTCAGATCTAAGCCTTACAA<br>1861 AGGGAGAGTCTCTGGCCGTCACACGTAGGACGCATGAAGGTCACTCGTGGTGAGGCTGAC<br>1921 ATGCTCACACATTACAACAGTAGAGAGGGAAAATCCTAAGACAGAGGAACTCCAGAGATG<br>1981 AGTGTCTGGAGCGCTTCAGTTCAGCTTTAAAGGCCAGGACGGGCCACACGTGGCTGGCGG<br>2041 CCTCGTTCCAGTGGCGGCACGTCCTTGGGCGTCTCTAATGTCTGCAGCTCAAGAGCTGGC<br>2101 ACTTTTTTAAATAGCAAACTGGGTGTTATTTTTATTTACTTCTTTGTAAAGTGGTTTTCG<br>2161 GTCTTCTGTTGACATTCGGGGTGATCCTGTTCTGCGCTGTGTACAATGTGAGATCGGTGC<br>2221 GTTCTCCTGATGTTTTGCCGTGGCTTGGGGATTGTACACGGGACCAGCTCACGTAATGCA<br>2281 TTGCCTGTAACAATGTAATAAAAAGCCTCTTTCTTTTAAAAAAAAAAAAAAAAAAAAAAA<br>2341 A | |
| | | 1 M A A P G S A R R P L L L L L L L L L L<br>1 ATGGCGGCCCCCGGCAGCGCCCGGCGACCCCTGCTGCTGCTACTGCTGTTGCTGCTGCTC<br><br>21 G L M H C A S A A M F M V K N G N G T A<br>61 GGCCTCATGCATTGTGCGTCAGCAGCAATGTTTATGGTGAAAAATGGCAACGGGACCGCG<br><br>41 C I M A N F S A A F S V N Y D T K S G P<br>121 TGCATAATGGCCAACTTCTCTGCTGCCTTCTCAGTGAACTACGACACCAAGAGTGGCCCT<br><br>61 K N M T F D L P S D A T V V L N R S S C<br>181 AAGAACATGACCTTTGACCTGCCATCAGATGCCACAGTGGTGCTCAACCGCAGCTCCTGT<br><br>81 G K E N T S D P S L V I A F G R G H T L<br>241 GGAAAAGAGAACACTTCTGACCCCAGTCTCGTGATTGCTTTTGGAAGAGGACATACACTC<br><br>101 T L N F T R N A T R Y S V Q L M S F V Y<br>301 ACTCTCAATTTCACGAGAAATGCAACACGTTACAGCGTCCAGCTCATGAGTTTTGTTTAT<br><br>121 N L S D T H L F P N A S S K E I K T V E<br>361 AACTTGTCAGACACACACCTTTTCCCCAATGCGAGCTCCAAAGAAATCAAGACTGTGGAA<br><br>141 S I T D I R A D I D K K Y R C V S G T Q<br>421 TCTATAACTGACATCAGGGCAGATATAGATAAAAAATACAGATGTGTTAGTGGCACCCAG<br><br>161 V H M N N V T V T L H D A T I Q A Y L S<br>481 GTCCACATGAACAACGTGACCGTAACGCTCCATGATGCCACCATCCAGGCGTACCTTTCC<br><br>181 N S S F S R G E T R C E Q D R P S P T T<br>541 AACAGCAGCTTCAGCAGGGGAGAGACACGCTGTGAACAAGACAGGCCTTCCCCAACCACA | SEQ ID NO:171 |

| Gene Name | GenBank Homology | DNA SEQUENCE / DEDUCED AMINO ACID SEQUENCE | SEQUENCE IDENTIFIER: |
|---|---|---|---|
| | | 201 A P P A P P S P S P S P V P K S P S V D<br>601 GCGCCCCCTGCGCCACCCAGCCCCTCGCCCTCACCCGTGCCCAAGAGCCCCTCTGTGGAC<br><br>221 K Y N V S G T N G T C L L A S M G L Q L<br>661 AAGTACAACGTGAGCGGCACCAACGGGACCTGCCTGCTGGCCAGCATGGGGCTGCAGCTG<br><br>241 N L T Y E R K D N T T V T R L L N I N P<br>721 AACCTCACCTATGAGAGGAAGGACAACACGACGGTGACAAGGCTTCTCAACATCAACCCC<br><br>261 N K T S A S G S C G A H L V T L E L H S<br>781 AACAAGACCTCGGCCAGCGGGAGCTGCGGCGCCCACCTGGTGACTCTGGAGCTGCACAGC<br><br>281 E G T T V L L F H F G M N A T S S Q F F<br>841 GAGGGCACCACCGTCCTGCTCTTCCACTTTGGAATGAATGCAACTTCTAGCCAGTTTTTC<br><br>301 L Q E I Q L D M T L P D A R D P T F K A<br>901 CTGCAAGAAATCCAGTTGGATATGACTCTTCCTGACGCCAGAGACCCCACCTTCAAGGCT<br><br>321 A N S S L R A L Q A T I G N S Y R C N A<br>961 GCCAACAGCTCGCTGAGGGCACTTCAGGCCACCATCGGGAACTCCTACAGGTGTAATGCC<br><br>341 E E R L R V T E A L S V N V F R V W V Q<br>1021 GAGGAGCGCCTGCGCGTCACAGAGGCCCTTTCTGTCAACGTCTTCAGAGTGTGGGTTCAG<br><br>361 A F Q V E G D K F G S V E E C Q L D E N<br>1081 GCTTTCCAGGTGGAAGGTGACAAGTTTGGGTCCGTGGAGGAATGCCAGCTGGATGAGAAC<br><br>381 N M M I P I A V G G A L A G L V L I V L<br>1141 AACATGATGATCCCCATTGCCGTGGGCGGTGCCCTGGCCGGGCTGGTCCTCATCGTCCTC<br><br>401 I A Y L I G R K R S H A G Y Q T I -<br>1201 ATTGCCTACCTCATCGGCAGGAAGAGGAGCCACGCGGGCTACCAGACTATCTAG | |
| WBC038G11.bFSP_20 021101.esd | *No Homology* | 1 GATGCCTGCAATACCTCTTATCATCGTTGTAACATTTCTTCCAAAATTAATGACCCAACC<br>61 CGTCCTCTCTGGGCCAGAGTTAAAGCGAGGCTTGGACAAGAAGAATCGGCCTATGCAGAC<br>121 TCAAAAGAGTTTATCCTGTGCCAGCACGGGAAAATTGGACCACCTAAACTGGATATCAGA<br>181 AAGAAAGACGACCAGATCATTATTGACATATCTCACCCTTTATTTGCTGTAAATGGATTT<br>241 GACCTGGAAGCCATGTATGATGATGAAAATGCTTGTTACGAATTCGTGTACAAGGTGTAT<br>301 GTGAAAATAAACGGAAGTGGAGAGAGCATGGAGAGAGTGTATGAATGCAGAGACGAGGAC<br>361 TGCAATGAGACCCACTGCCGCCTCGGCATCCCAGCGTCCTCCCTGAACTCTCAGTGCTGC<br>421 GCTGCAGCAGAGGGAGTCTCGGAACGATGGGAGTTCACAACTGACAAGTCCGAAGAACTC<br>481 TGCCTGACCGTTTTCGATGATGGCAGCCCAGAAGGTTCGGTTTGGATTCCAGTTGTTGCT | SEQ ID NO:172 |

| Gene Name | GenBank Homology | DNA SEQUENCE / DEDUCED AMINO ACID SEQUENCE | SEQUENCE IDENTIFIER: |
|---|---|---|---|
| | | 541 GCTTTCCTACTCTTTCTAGTCATTCTGCTGGTTGCGTGTTGTTATTGTCGCATTAAGACG<br>601 AAGAATCTGTTTAAGAGAAGAAACAACCCTTTACCCAGTTCCTTGCTGTCTGTGGTAAGA<br>661 AGTGCCTCTTCAGAGCCAGACTCTGAATCAAAGCATATATCGCCTGTCACCTACCAGCCA<br>721 ATTGTCCCGGAGAACGATCACCTGTCTTCAGCAGCGGTGTCAGGGACGCAGGCTGAAGAC<br>781 AGTCC | |
| | | 1 GTGGACGAAGGTGGGAAGGAGTTCCCTGATCGGCTACAGACTGACAGCTGACTCCCAGGA<br>61 GTTCTCCTGAGCTCAGCCGAGGGGCCCGGCCCTGAAGGAAGGGCTCTGCCTGAGCAGTTC<br>121 TTCCTGAACGGCTTTGAGTTTGTGAAAAGATCTGATTTCTGGAATTGTGTCAATATTAAC<br>181 CACATGCAATGTCTGGTTCAGATCAAAATGTGAACTGCCTGTTGAACTTGGTCGCATTTG<br>241 GTCTGAAAACAGCAAACCAGTTTCAAAACCTGTCTTAGGAAAACAAGCGACATCAGCACC<br>301 TGGGAGCCTTTCTCCCGGCACGGCCAGCTGTGACAGTGGCACTCCCTGCCACCCTCTTCC<br>361 TGCGACAGCGGTGGCATCTGTGGCGGATGTTTCTTCTCTGCGCCTCCGTGTGGGATTTGG<br>421 TTTGGAAGTTTGTGTAGATAGTACTTTTAAATACCTGCTTTTGGAAGAAATCCTGGAAGC<br>481 TTTTCAAAACTGGCATTTAAAATCAGATTTGAGCCAGTGGAATTAATAGAGGGTGTAAAT<br>541 AGAGACGTGCATGTGTCTTCCAGGAGCATTGCACGTTGCCTGTAGAAAGTTTTAAATAAA<br>601 ACATTTTTAAAATTAAAAAAAAAAAAAAAAAA | SEQ ID NO:173 |
| WBC039D09.bFSP_20 021101.esd | Homo sapiens NPF/calponin-like protein, mRNA | 1 AAGGGAACGGGGCCAGACTCAGCCCGACAGCGGGATTAGAGCCCTCCAGAATACCCATCA<br>61 TATAGCCCCTGAGGTGGCATGGTGATGTCTCCATGAGGGAACCCCTTCCCACTCATCCTG<br>121 TCACGTATATCATAGTGTTCTTGACTGGGCCATTCATCTAAGATGGGATTTACCCTGTGA<br>181 AACAGGGAGAAGACTTATGGACCCCAAGCATCATTTCGAGTTGTAGTTGAGTTTTTAAAA<br>241 GACATACATGCAAAGTTCCTTTGCTTTGGACCCTCTGCATTATTAAAGCTGCTGTATTGC<br>301 TAACCCAGAACTGCTCCAGTGTCTTGACTGATCATCATGGCTTCAGTTTGGAAGAGACTG<br>361 CAGCGTGTGGGAAAACATGCATCCAAGTTCCAGTTTGTGGCCTCCTACCAGGAGCTCATG<br>421 GTTGAGTGTACGAAGAAATGGCAACCAGATAAACTGGTGGTAGTTTGGACCAGAAGAAGC<br>481 CGAAGGAAGTCTTCTAAGGCACATAGCTGGCAACCTGGAATAAAAAATCCCTATCGTGGT<br>541 GTTGTTGTGTGGCCTGTTCCTGAAAACATTGAAATCACTGTAACACTTTTTAAGGATCCT<br>601 CATGCGGAAGAATTTGAAGACAAAGAGTGGACATTTGTCATAGAAAATGAATCCCCTTCT<br>661 GGTCGAAGGAAAGCTCTTGCTACTAGCAGCATCAATATGAAACAGTATGCAAGCCCTATG<br>721 CCAACTCAGACTGATGTCAAGTTAAAATTCAAGCCATTATCTAAAAAAGTTGTATCTGCC<br>781 GCTCTTCAGTTTTCATTATCTTGCATTTTTCTGAGGGAAGGAAAAGCCACAGATGAAGAC<br>841 ATGCAAAGTTTGGCTAGTTTGATGAGTATGAAGCAGGCTGACATTGGCAATTTAGATGAC<br>901 TTCGAAGAAGATAATGAAGATGATGATGAGAACAGAGTGAACCAAGAAGAAAAGGCAGCT<br>961 AAAATTACAGAGCTTATCAACAAACTTAACTTTTTGGATGAAGCAGAAAAGGACTTGGCC<br>1021 ACCGTGAATTCAAATCCATTTGATGATCCTGATGCTGCAGAATTAAATCCATTTGGAGAT<br>1081 CCTGACTCAGAAGAACCTATCACTGAAACAGCTTCACCTAGAAAAACAGAAGACTCTTTT<br>1141 TATAATAACAGCTATAATCCCTTTAAAGAGGTGCAGACTCCACAGTATTTGAACCCATTC<br>1201 GATGAGCCAGAAGCATTTGTGACCATAAAGGATTCTCCTCCCCAGTCTACAAAAAGAAAA<br>1261 AATATAAGACCTGTGGATATGAGCAAGTACCTCTATGCTGATAGTTCTAAAACTGAAGAA<br>1321 GAAGAATTGGATGAATCAAATCCTTTTTATGAACCTAAATCAACTCCTCCTCCAAATAAT<br>1381 TTGGTAAATCCTGTTCAAGAACTAGAAACTGAAAGGCGAGTGAAAAGAAAGGCCCCGGCT | SEQ ID NO:174 |

| Gene Name | GenBank Homology | DNA SEQUENCE / DEDUCED AMINO ACID SEQUENCE | SEQUENCE IDENTIFIER: |
|---|---|---|---|
| | | 1441 CCACCAGTCCTCTCACCAAAAACAGGAGTATTAAATGAAAACACAGTTTCTGCAGGAAAA<br>1501 GATCTCTCTACTTCTCCTAAGCCAAGCCCTATACCAAGTCCTGTTTTGGGGCGAAAGCCA<br>1561 AATGCTAGTCAGTCTTTGCTTGTATGGTGTAAAGAAGTTACAAAGAACTACCGAGGAGTA<br>1621 AAAATCACCAATTTTACTACATCGTGGAGAAATGGTTTATCTTTTTGTGCAATATTACAC<br>1681 CACTTTAGACCAGATTTAATTGACTACAAGTCTCTGAATCCTCAAGATATTAAAGAGAAC<br>1741 AACAAAAAGGCATACGATGGATTTGCCAGCATAGGAATTTCCCGATTATTGGAACCTTCT<br>1801 GATATGGTATTATTAGCAATTCCTGATAAACTGACTGTTATGACTTATCTCTATCAAATA<br>1861 AGGGCACATTTCAGTGGCCAAGAACTAAATGTCGTTCAGATAGAGGAAAACAGCAGTAAA<br>1921 AGCACATATAAAGTTGGAAACTATGAAACAGATACAAACAGTTCTGTTGATCAAGAAAAA<br>1981 TTCTATGCAGAGCTTAGTGATCTGAAGCGGGAGCCTGAACTACAACAGCCTATCAGCGGA<br>2041 GCAGTAGACTTCTTATCACAGGATGACTCTGTATTTGTAAATGATAGCGGGGTTGGAGAG<br>2101 TCAGAAAGTGAGCATCAAACTCCTGATGATCACCTTAGTCCAAGCACAGCCTCCCCTTAC<br>2161 TGTCGCAGGACTAAAAGTGACACAGAACCCCAGAAGTCTCAGCAGAGCTCTGGAAGGACT<br>2221 TCAGGATCTGATGACCCTGGAATATGTTCCAATACAGATTCAACCCAAGCACAGGTTTTG<br>2281 TTAGGCAAAAAGAGACTATTGAAAGCTGAGACTTTAGAATTGAGTGACTTATATGTTAGT<br>2341 GATAAGAAGAAGGATATGTCTCCACCCTTTATTTGTGAGGAGACAGATGAACAAAAGCTT<br>2401 CAAACTCTAGACATCGGTAGTAACTTGGAGAAAGAAAAATTAGAGAATTCCAGATCCTTA<br>2461 GAATGCAGATCAGATCCAGAATCTCCTATCAAAAAAACAAGTTTATCTCCTACTTCTAAA<br>2521 CTTGGATACTCATATAGTAGAGATCTAGACCTTGCTAAGAAAAAACATGCTTCCCTGAGG<br>2581 CAGACGGAGTCTGATCCAGATGCTGATAGAACCACTTTAAATCATGCAGATCATTCATCA<br>2641 AAAATAGTCCAGCATCGATTGTTATCTAGACAAGAAGAACTTAAGGAAAGAGCAAGAGTT<br>2701 CTGCTTGAGCAAGCAAGAAGAGATGCAGCCTTAAAGGCGGGGAATAAGCACAATACCAAC<br>2761 ACAGCCACCCCATTCTGCAACAGGCAGCTAAGTGATCAGCAAGATGAAGAGCGACGTCGG<br>2821 CAGCTGAGAGAGAGAGCTCGTCAGCTAATAGCAGAAGCTCGATCTGGAGTGAAGATGTCA<br>2881 GAACTTCCCAGCTATGGTGAAATGGCTGCAGAAAAGTTGAAAGAAAGGTCAAAGGCATCT<br>2941 GGAGAACAAAACAGTAAGTTGGTGGACTTGAAGCTGAAGAAGCTCCTAGAAGTTCAGCCA<br>3001 CAGGTGGCAAATTCACCCTCCAGTGCTGCCCAGAAAGCTGTAACTGAGAGCTCAGAGCAG<br>3061 GACATGAAAAGTGGCACAGAAGATCTCCGGACTGAACGATTACAAAAAACAACAGAACGT<br>3121 TTTAGAAATCCTGTTGTGTTCAGCAAAGATTCTACAGTCAGAAAAACTCAACTTCAGTCT<br>3181 TTCAGCCAATATATTGAGAATAGACCAGAGATGAAAAGGCAGAGATCAATACAGGAAGAT<br>3241 ACAAAGAAAGGAAATGAGGAGAAGGCAGCGATAACTGAAACTCAGAGGAAGCCATCAGAA<br>3301 GATGAAGTGCTTAATAAAGGGTTCAAAGACACCAGTCAGTATGTTGTAGGAGAATTGGCA<br>3361 GCACTAGAGAATGAGCAAAAGCAAATTGACACCCGTGCCGCGCTGGTGGAGAAGCGCCTT<br>3421 CGCTATCTCATGGACACAGGAAGGAACACAGAAGAAGAAGAAGCTATGATGCAGGAATGG<br>3481 TTTATGTTAGTTAATAAGAAAAATGCCTTAATAAGGAGAATGAACCAGCTGTCTCTTCTG<br>3541 GAAAAAGAACATGATTTAGAACGCCGGTATGAACTGCTGAATCGGGAATTGCGGGCAATG<br>3601 CTGGCCATTGAAGACTGGCAGAAGACCGAGGCCCAGAAGCGACGCGAGCAACTCCTGCTG<br>3661 GATGAGCTGGTGGCCCTCGTGAAACAGCGCGACGCGCTAGTCAGGGACCTGGACGCGCAG<br>3721 GAGAAGCAGGCCGAAGAAGAAGATGAGCATTTGGAGCGAACTCTGGAGCAAAACAAAGGC<br>3781 AAGATGGCCAAGAAAGAGGAGAAATGTGTTCTTCAGTAGCCATCAGATCAGAAAGAATCT<br>3841 CTCCCAACATTTTAGAGTCTTGCTTCCCAAACCAGAAAAAGTCAGACTCATTGTTGATTT<br>3901 AAAACTTTTAACATTTTGTTTGGCTGGATTGTACTACTTTACCTCTACTTTACCACCACC<br>3961 ACCCTTTTCCTCCCTCCTTTCCAAATAATATACAGAACTCCAAAATAGCTTCATTTAAGG | |

| Gene Name | GenBank Homology | DNA SEQUENCE / DEDUCED AMINO ACID SEQUENCE | SEQUENCE IDENTIFIER: |
|---|---|---|---|
| | | 4021 ATTTTTTTGTGAGTTAACAATTTCCTTGAAATCCTGTGAAATAGATTTGCACAGACACCT<br>4081 TGTGAGTGATTGGTATTGGAGGTGTTCAAGAAACTGTTCGAAAAAGAACAAAAACACTTC<br>4141 CCTCGTTATTTTCTCTCATTTTTTGATGAGAGGAAAATTTGAAACATTATTCTTGTTGTT<br>4201 GTTGGTAATAGCATAATGACAGTGGGAGGGGTACAAGGGGATAAGAAAAATGTCATGATT<br>4261 TTTTTCCGGTCCTGCCATATCTAACACTGACTCTGTTACCTAAATTTTATAGTTAGATCA<br>4321 TATTCAATCTGCTTATTAAACTGTGTTCTATTTACCAGTGGGATTTTCTGCAGTTGTTTT<br>4381 GCGTTTCACTGTAAGGATAATAGAGCTCCTCTCCTCTGCTTTCTTCAGAGGATGGCTCTG<br>4441 TAACCTAGCCTGAGACAAGTCCTGTGATTTAAAGGTGAGCTGTGAGGATGGGACTAATTG<br>4501 ACATGCATCAGTTTACAAAGACGGGCTTATCATCTGAGAATGCGCCATCTTTTTCTCTGG<br>4561 ATAATTATTTATTACTTCTAGCTTGGTTCCTACAATTTTGTTGGGTCTCAACATTGGCTC<br>4621 AAGAATGCTGTTAATATTTATTCTGTATTGATAAAAGTCTGTCTTGCCACTATGAGTAAA<br>4681 TCCCCCAATTAATATTTTCTTCTCTAGCATCGCACTGTCATTTTTTGTGAAAATGGTTAT<br>4741 CTGTTTATTTATTACATATCTGAGTCATATATAAATTTTCAATAAAAGCAGAAACTTTCT<br>4801 TACCTTAAAAAAAAAAAAAAAA | |
| | | 1 M A S V W K R L Q R V G K H A S K F Q F<br>1 ATGGCTTCAGTTTGGAAGAGACTGCAGCGTGTGGGAAAACATGCATCCAAGTTCCAGTTT<br><br>21 V A S Y Q E L M V E C T K K W Q P D K L<br>61 GTGGCCTCCTACCAGGAGCTCATGGTTGAGTGTACGAAGAAATGGCAACCAGATAAACTG<br><br>41 V V V W T R R S R R K S S K A H S W Q P<br>121 GTGGTAGTTTGGACCAGAAGAAGCCGAAGGAAGTCTTCTAAGGCACATAGCTGGCAACCT<br><br>61 G I K N P Y R G V V V W P V P E N I E I<br>181 GGAATAAAAAATCCCTATCGTGGTGTTGTTGTGTGGCCTGTTCCTGAAAACATTGAAATC<br><br>81 T V T L F K D P H A E E F E D K E W T F<br>241 ACTGTAACACTTTTTAAGGATCCTCATGCGGAAGAATTTGAAGACAAAGAGTGGACATTT<br><br>101 V I E N E S P S G R R K A L A T S S I N<br>301 GTCATAGAAAATGAATCCCCTTCTGGTCGAAGGAAAGCTCTTGCTACTAGCAGCATCAAT<br><br>121 M K Q Y A S P M P T Q T D V K L K F K P<br>361 ATGAAACAGTATGCAAGCCCTATGCCAACTCAGACTGATGTCAAGTTAAAATTCAAGCCA<br><br>141 L S K K V V S A A L Q F S L S C I F L R<br>421 TTATCTAAAAAAGTTGTATCTGCCGCTCTTCAGTTTTCATTATCTTGCATTTTTCTGAGG<br><br>161 E G K A T D E D M Q S L A S L M S M K Q<br>481 GAAGGAAAAGCCACAGATGAAGACATGCAAAGTTTGGCTAGTTTGATGAGTATGAAGCAG<br><br>181 A D I G N L D D F E E D N E D D D E N R | SEQ ID NO:175 |

| Gene Name | GenBank Homology | DNA SEQUENCE / DEDUCED AMINO ACID SEQUENCE | SEQUENCE IDENTIFIER: |
|---|---|---|---|
| | | 541 GCTGACATTGGCAATTTAGATGACTTCGAAGAAGATAATGAAGATGATGATGAGAACAGA | |
| | | 201 V N Q E E K A A K I T E L I N K L N F L<br>601 GTGAACCAAGAAGAAAAGGCAGCTAAAATTACAGAGCTTATCAACAAACTTAACTTTTTG | |
| | | 221 D E A E K D L A T V N S N P F D D P D A<br>661 GATGAAGCAGAAAAGGACTTGGCCACCGTGAATTCAAATCCATTTGATGATCCTGATGCT | |
| | | 241 A E L N P F G D P D S E E P I T E T A S<br>721 GCAGAATTAAATCCATTTGGAGATCCTGACTCAGAAGAACCTATCACTGAAACAGCTTCA | |
| | | 261 P R K T E D S F Y N N S Y N P F K E V Q<br>781 CCTAGAAAAACAGAAGACTCTTTTTATAATAACAGCTATAATCCCTTTAAAGAGGTGCAG | |
| | | 281 T P Q Y L N P F D E P E A F V T I K D S<br>841 ACTCCACAGTATTTGAACCCATTCGATGAGCCAGAAGCATTTGTGACCATAAAGGATTCT | |
| | | 301 P P Q S T K R K N I R P V D M S K Y L Y<br>901 CCTCCCCAGTCTACAAAAAGAAAAAATATAAGACCTGTGGATATGAGCAAGTACCTCTAT | |
| | | 321 A D S S K T E E E E L D E S N P F Y E P<br>961 GCTGATAGTTCTAAAACTGAAGAAGAAGAATTGGATGAATCAAATCCTTTTTATGAACCT | |
| | | 341 K S T P P P N N L V N P V Q E L E T E R<br>1021 AAATCAACTCCTCCTCCAAATAATTTGGTAAATCCTGTTCAAGAACTAGAAACTGAAAGG | |
| | | 361 R V K R K A P A P P V L S P K T G V L N<br>1081 CGAGTGAAAAGAAAGGCCCCGGCTCCACCAGTCCTCTCACCAAAAACAGGAGTATTAAAT | |
| | | 381 E N T V S A G K D L S T S P K P S P I P<br>1141 GAAAACACAGTTTCTGCAGGAAAAGATCTCTCTACTTCTCCTAAGCCAAGCCCTATACCA | |
| | | 401 S P V L G R K P N A S Q S L L V W C K E<br>1201 AGTCCTGTTTTGGGGCGAAAGCCAAATGCTAGTCAGTCTTTGCTTGTATGGTGTAAAGAA | |
| | | 421 V T K N Y R G V K I T N F T T S W R N G<br>1261 GTTACAAAGAACTACCGAGGAGTAAAAATCACCAATTTTACTACATCGTGGAGAAATGGT | |
| | | 441 L S F C A I L H H F R P D L I D Y K S L<br>1321 TTATCTTTTTGTGCAATATTACACCACTTTAGACCAGATTTAATTGACTACAAGTCTCTG | |
| | | 461 N P Q D I K E N N K K A Y D G F A S I G<br>1381 AATCCTCAAGATATTAAAGAGAACAACAAAAAGGCATACGATGGATTTGCCAGCATAGGA | |

| Gene Name | GenBank Homology | DNA SEQUENCE / DEDUCED AMINO ACID SEQUENCE | SEQUENCE IDENTIFIER: |
|---|---|---|---|
| | | 481 I S R L L E P S D M V L L A I P D K L T<br>1441 ATTTCCCGATTATTGGAACCTTCTGATATGGTATTATTAGCAATTCCTGATAAACTGACT<br><br>501 V M T Y L Y Q I R A H F S G Q E L N V V<br>1501 GTTATGACTTATCTCTATCAAATAAGGGCACATTTCAGTGGCCAAGAACTAAATGTCGTT<br><br>521 Q I E E N S S K S T Y K V G N Y E T D T<br>1561 CAGATAGAGGAAAACAGCAGTAAAAGCACATATAAAGTTGGAAACTATGAAACAGATACA<br><br>541 N S S V D Q E K F Y A E L S D L K R E P<br>1621 AACAGTTCTGTTGATCAAGAAAAATTCTATGCAGAGCTTAGTGATCTGAAGCGGGAGCCT<br><br>561 E L Q Q P I S G A V D F L S Q D D S V F<br>1681 GAACTACAACAGCCTATCAGCGGAGCAGTAGACTTCTTATCACAGGATGACTCTGTATTT<br><br>581 V N D S G V G E S E S E H Q T P D D H L<br>1741 GTAAATGATAGCGGGGTTGGAGAGTCAGAAAGTGAGCATCAAACTCCTGATGATCACCTT<br><br>601 S P S T A S P Y C R R T K S D T E P Q K<br>1801 AGTCCAAGCACAGCCTCCCCTTACTGTCGCAGGACTAAAAGTGACACAGAACCCCAGAAG<br><br>621 S Q Q S S G R T S G S D D P G I C S N T<br>1861 TCTCAGCAGAGCTCTGGAAGGACTTCAGGATCTGATGACCCTGGAATATGTTCCAATACA<br><br>641 D S T Q A Q V L L G K K R L L K A E T L<br>1921 GATTCAACCCAAGCACAGGTTTTGTTAGGCAAAAAGAGACTATTGAAAGCTGAGACTTTA<br><br>661 E L S D L Y V S D K K K D M S P P F I C<br>1981 GAATTGAGTGACTTATATGTTAGTGATAAGAAGAAGGATATGTCTCCACCCTTTATTTGT<br><br>681 E E T D E Q K L Q T L D I G S N L E K E<br>2041 GAGGAGACAGATGAACAAAAGCTTCAAACTCTAGACATCGGTAGTAACTTGGAGAAAGAA<br><br>701 K L E N S R S L E C R S D P E S P I K K<br>2101 AAATTAGAGAATTCCAGATCCTTAGAATGCAGATCAGATCCAGAATCTCCTATCAAAAAA<br><br>721 T S L S P T S K L G Y S Y S R D L D L A<br>2161 ACAAGTTTATCTCCTACTTCTAAACTTGGATACTCATATAGTAGAGATCTAGACCTTGCT<br><br>741 K K K H A S L R Q T E S D P D A D R T T<br>2221 AAGAAAAAACATGCTTCCCTGAGGCAGACGGAGTCTGATCCAGATGCTGATAGAACCACT | |

| Gene Name | GenBank Homology | DNA SEQUENCE / DEDUCED AMINO ACID SEQUENCE | SEQUENCE IDENTIFIER: |
|---|---|---|---|
| | | 761 L N H A D H S S K I V Q H R L L S R Q E<br>2281 TTAAATCATGCAGATCATTCATCAAAAATAGTCCAGCATCGATTGTTATCTAGACAAGAA<br><br>781 E L K E R A R V L L E Q A R R D A A L K<br>2341 GAACTTAAGGAAAGAGCAAGAGTTCTGCTTGAGCAAGCAAGAAGAGATGCAGCCTTAAAG<br><br>801 A G N K H N T N T A T P F C N R Q L S D<br>2401 GCGGGGAATAAGCACAATACCAACACAGCCACCCCATTCTGCAACAGGCAGCTAAGTGAT<br><br>821 Q Q D E E R R R Q L R E R A R Q L I A E<br>2461 CAGCAAGATGAAGAGCGACGTCGGCAGCTGAGAGAGAGAGCTCGTCAGCTAATAGCAGAA<br><br>841 A R S G V K M S E L P S Y G E M A A E K<br>2521 GCTCGATCTGGAGTGAAGATGTCAGAACTTCCCAGCTATGGTGAAATGGCTGCAGAAAAG<br><br>861 L K E R S K A S G E Q N S K L V D L K L<br>2581 TTGAAAGAAAGGTCAAAGGCATCTGGAGAACAAAACAGTAAGTTGGTGGACTTGAAGCTG<br><br>881 K K L L E V Q P Q V A N S P S S A A Q K<br>2641 AAGAAGCTCCTAGAAGTTCAGCCACAGGTGGCAAATTCACCCTCCAGTGCTGCCCAGAAA<br><br>901 A V T E S S E Q D M K S G T E D L R T E<br>2701 GCTGTAACTGAGAGCTCAGAGCAGGACATGAAAAGTGGCACAGAAGATCTCCGGACTGAA<br><br>921 R L Q K T T E R F R N P V V F S K D S T<br>2761 CGATTACAAAAAACAACAGAACGTTTTAGAAATCCTGTTGTGTTCAGCAAAGATTCTACA<br><br>941 V R K T Q L Q S F S Q Y I E N R P E M K<br>2821 GTCAGAAAAACTCAACTTCAGTCTTTCAGCCAATATATTGAGAATAGACCAGAGATGAAA<br><br>961 R Q R S I Q E D T K K G N E E K A A I T<br>2881 AGGCAGAGATCAATACAGGAAGATACAAAGAAAGGAAATGAGGAGAAGGCAGCGATAACT<br><br>981 E T Q R K P S E D E V L N K G F K D T S<br>2941 GAAACTCAGAGGAAGCCATCAGAAGATGAAGTGCTTAATAAAGGGTTCAAAGACACCAGT<br><br>1001 Q Y V V G E L A A L E N E Q K Q I D T R<br>3001 CAGTATGTTGTAGGAGAATTGGCAGCACTAGAGAATGAGCAAAAGCAAATTGACACCCGT<br><br>1021 A A L V E K R L R Y L M D T G R N T E E<br>3061 GCCGCGCTGGTGGAGAAGCGCCTTCGCTATCTCATGGACACAGGAAGGAACACAGAAGAA<br><br>1041 E E A M M Q E W F M L V N K K N A L I R | |

| Gene Name | GenBank Homology | DNA SEQUENCE / DEDUCED AMINO ACID SEQUENCE | SEQUENCE IDENTIFIER: |
|---|---|---|---|
| | | 3121 GAAGAAGCTATGATGCAGGAATGGTTTATGTTAGTTAATAAGAAAAATGCCTTAATAAGG<br><br>1061 R M N Q L S L L E K E H D L E R R Y E L<br>3181 AGAATGAACCAGCTGTCTCTTCTGGAAAAAGAACATGATTTAGAACGCCGGTATGAACTG<br><br>1081 L N R E L R A M L A I E D W Q K T E A Q<br>3241 CTGAATCGGGAATTGCGGGCAATGCTGGCCATTGAAGACTGGCAGAAGACCGAGGCCCAG<br><br>1101 K R R E Q L L L D E L V A L V K Q R D A<br>3301 AAGCGACGCGAGCAACTCCTGCTGGATGAGCTGGTGGCCCTCGTGAAACAGCGCGACGCG<br><br>1121 L V R D L D A Q E K Q A E E E D E H L E<br>3361 CTAGTCAGGGACCTGGACGCGCAGGAGAAGCAGGCCGAAGAAGAAGATGAGCATTTGGAG<br><br>1141 R T L E Q N K G K M A K K E E K C V L Q<br>3421 CGAACTCTGGAGCAAAACAAAGGCAAGATGGCCAAGAAAGAGGAGAAATGTGTTCTTCAG<br><br>1161 -<br>3481 TAG | |
| WBC039F12.bFSP_20 021101.esd | Leu-8 pan leukocyte antigen | 1 ctccctttgg gcaaggacct gagacccttg tgctaagtca agaggctcaa tgggctgcag<br>61 aagaactaga gaaggaccaa gcaaagccat gatatttcca tggaaatgtc agagcaccca<br>121 gagggactta tggaacatct tcaagttgtg ggggtggaca atgctctgtt gtgatttcct<br>181 ggcacatcat ggaaccgact gctggactta ccattattct gaaaaaccca tgaactggca<br>241 aagggctaga agattctgcc gagacaatta cacagattta gttgccatac aaaacaaggc<br>301 ggaaattgag tatctggaga agactctgcc tttcagtcgt tcttactact ggataggaat<br>361 ccggaagata ggaggaatat ggacgtgggt gggaaccaac aaatctctca ctgaagaagc<br>421 agagaactgg ggagatggtg agcccaacaa caagaagaac aaggaggact gcgtggagat<br>481 ctatatcaag agaaacaaag atgcaggcaa atggaacgat gacgcctgcc acaaactaaa<br>541 ggcagccctc tgttacacag cttcttgcca gccctggtca tgcagtggcc atggagaatg<br>601 tgtagaaatc atcaataatt acacctgcaa ctgtgatgtg gggtactatg ggccccagtg<br>661 tcagtttgtg attcagtgtg agcctttgga ggccccagag ctgggtacca tggactgtac<br>721 tcactctttg ggaaacttca gcttcagctc acagtgtgcc ttcagctgct ctgaaggaac<br>781 aaacttaact gggattgaag aaaccacctg tggaccattt ggaaactggt catctccaga<br>841 accaacctgt caagtgattc agtgtgagcc tctatcagca ccagatttgg ggatcatgaa<br>901 ctgtagccat cccctggcca gcttcagctt tacctctgca tgtaccttca tctgctcaga<br>961 aggaactgag ttaattggga agaagaaaac catttgtgaa tcatctggaa tctggtcaaa<br>1021 tcctagtcca atatgtcaaa aattggacaa aagtttctca atgattaagg agggtgatta<br>1081 taaccccctc ttcattccag tggcagtcat ggttactgca ttctctgggt tggcatttat<br>1141 catttggctg gcaaggagat taaaaaaagg caagaaatcc aagagaagta tgaatgaccc<br>1201 atattaaatc gcccttggtg aaagaaaatt cttggaatac taaaaatcat gagatccttt<br>1261 aaatccttcc atgaaacgtt ttgtgtggtg gcacctccta cgtcaaacat gaagtgtgtt | SEQ ID NO:176 |

| Gene Name | GenBank Homology | DNA SEQUENCE / DEDUCED AMINO ACID SEQUENCE | SEQUENCE IDENTIFIER: |
|---|---|---|---|
| | | 1321 tccttcagtg catctgggaa gatttctacc tgaccaacag ttccttcagc ttccatttcg<br>1381 cccctcattt atccctcaac ccccagccca caggtgttta tacagctcag ctttttgtct<br>1441 tttctgagga gaaacaaata agaccataaa gggaaaggat tcatgtggaa tataaagatg<br>1501 gctgactttg ctctttcttg actcttgttt tcagtttcaa ttcagtgctg tacttgatga<br>1561 cagacacttc taaatgaagt gcaaatttga tacatatgtg aatatggact cagttttctt<br>1621 gcagatcaaa tttcacgtcg tcttctgtat actgtggagg tacactctta tagaaagttc<br>1681 aaaaagtcta cgctctcctt tctttctaac tccagtgaag taatggggtc ctgctcaagt<br>1741 tgaaagagtc ctatttgcac tgtagcctcg ccgtctgtga attggaccat cctatttaac<br>1801 tggcttcagc ctccccacct tcttcagcca cctctctttt tcagttggct gacttccaca<br>1861 cctagcatct catgagtgcc aagcaaaagg agagaagaga gaaatagcct gcgctgtttt<br>1921 ttagtttggg ggttttgctg tttcctttta tgagacccat tcctatttct tatagtcaat<br>1981 gtttctttta tcacgatatt attagtaaga aaacatcact gaaatgctag ctgcaagtga<br>2041 catctctttg atgtcatatg gaagagttaa aacaggtgga gaaattcctt gattcacaat<br>2101 gaaatgctct cctttcccct gcccccagac cttttatccg acttacctag attctacata<br>2161 ttctttaaat ttcatctcag gcctccctca accccaccac ttcttttata actagtcctt<br>2221 tactaatcca acccatgatg agctcctctt cctggcttct tactgaaagg ttaccctgta<br>2281 acatgcaatt ttgcatttga ataaagcctg ctttttaagt gtt | |
| | | 1 M G C R R T R E G P S K A M I F P W K C<br>1 ATGGGCTGCAGAAGAACTAGAGAAGGACCAAGCAAAGCCATGATATTTCCATGGAAATGT<br><br>21 Q S T Q R D L W N I F K L W G W T M L C<br>61 CAGAGCACCCAGAGGGACTTATGGAACATCTTCAAGTTGTGGGGGTGGACAATGCTCTGT<br><br>41 C D F L A H H G T D C W T Y H Y S E K P<br>121 TGTGATTTCCTGGCACATCATGGAACCGACTGCTGGACTTACCATTATTCTGAAAAACCC<br><br>61 M N W Q R A R R F C R D N Y T D L V A I<br>181 ATGAACTGGCAAAGGGCTAGAAGATTCTGCCGAGACAATTACACAGATTTAGTTGCCATA<br><br>81 Q N K A E I E Y L E K T L P F S R S Y Y<br>241 CAAAACAAGGCGGAAATTGAGTATCTGGAGAAGACTCTGCCTTTCAGTCGTTCTTACTAC<br><br>101 W I G I R K I G G I W T W V G T N K S L<br>301 TGGATAGGAATCCGGAAGATAGGAGGAATATGGACGTGGGTGGGAACCAACAAATCTCTC<br><br>121 T E E A E N W G D G E P N N K K N K E D<br>361 ACTGAAGAAGCAGAGAACTGGGGAGATGGTGAGCCCAACAACAAGAAGAACAAGGAGGAC<br><br>141 C V E I Y I K R N K D A G K W N D D A C<br>421 TGCGTGGAGATCTATATCAAGAGAAACAAAGATGCAGGCAAATGGAACGATGACGCCTGC | *SEQ ID NO:*177 |

| Gene Name | GenBank Homology | DNA SEQUENCE / DEDUCED AMINO ACID SEQUENCE | SEQUENCE IDENTIFIER: |
|---|---|---|---|
| | | 161 H K L K A A L C Y T A S C Q P W S C S G<br>481 CACAAACTAAAGGCAGCCCTCTGTTACACAGCTTCTTGCCAGCCCTGGTCATGCAGTGGC<br><br>181 H G E C V E I I N N Y T C N C D V G Y Y<br>541 CATGGAGAATGTGTAGAAATCATCAATAATTACACCTGCAACTGTGATGTGGGGTACTAT<br><br>201 G P Q C Q F V I Q C E P L E A P E L G T<br>601 GGGCCCCAGTGTCAGTTTGTGATTCAGTGTGAGCCTTTGGAGGCCCCAGAGCTGGGTACC<br><br>221 M D C T H S L G N F S F S S Q C A F S C<br>661 ATGGACTGTACTCACTCTTTGGGAAACTTCAGCTTCAGCTCACAGTGTGCCTTCAGCTGC<br><br>241 S E G T N L T G I E E T T C G P F G N W<br>721 TCTGAAGGAACAAACTTAACTGGGATTGAAGAAACCACCTGTGGACCATTTGGAAACTGG<br><br>261 S S P E P T C Q V I Q C E P L S A P D L<br>781 TCATCTCCAGAACCAACCTGTCAAGTGATTCAGTGTGAGCCTCTATCAGCACCAGATTTG<br><br>281 G I M N C S H P L A S F S F T S A C T F<br>841 GGGATCATGAACTGTAGCCATCCCCTGGCCAGCTTCAGCTTTACCTCTGCATGTACCTTC<br><br>301 I C S E G T E L I G K K K T I C E S S G<br>901 ATCTGCTCAGAAGGAACTGAGTTAATTGGGAAGAAGAAAACCATTTGTGAATCATCTGGA<br><br>321 I W S N P S P I C Q K L D K S F S M I K<br>961 ATCTGGTCAAATCCTAGTCCAATATGTCAAAAATTGGACAAAAGTTTCTCAATGATTAAG<br><br>341 E G D Y N P L F I P V A V M V T A F S G<br>1021 GAGGGTGATTATAACCCCCTCTTCATTCCAGTGGCAGTCATGGTTACTGCATTCTCTGGG<br><br>361 L A F I I W L A R R L K K G K K S K R S<br>1081 TTGGCATTTATCATTTGGCTGGCAAGGAGATTAAAAAAAGGCAAGAAATCCAAGAGAAGT<br><br>381 M N D P Y -<br>1141 ATGAATGACCCATATTAA | |
| WBC039G08.bFSP_20 021101.esd | Human Lyn B protein mRNA. | 1 GAAACTTTCACGCGAGCGGGAAATATGGGATGTATAAAATCAAAAGGGAAAGACAGCTTG<br>61 AGTGACGATGGAGTAGATTTGAAGACTCAACCAGTTCCAGAATCTCAGCTTTTACCTGGA<br>121 CAGAGGTTTCAAACTAAAGATCCAGAGGAACAAGGAGACATTGTGGTAGCCTTGTACCCC<br>181 TATGATGGCATCCACCCGGACGACTTGTCTTTCAAGAAAGGAGAGAAGATGAAAGTCCTG<br>241 GAGGAGCATGGAGAATGGTGGAAAGCAAAGTCCCTTTTAACAAAAAAAGAAGGCTTCATC<br>301 CCCAGCAACTATGTGGCCAAACTCAACACCTTAGAAACAGAAGAGTGGTTTTTCAAGGAT | SEQ ID NO:178 |

| Gene Name | GenBank Homology | DNA SEQUENCE / DEDUCED AMINO ACID SEQUENCE | SEQUENCE IDENTIFIER: |
|---|---|---|---|
| | | 361 ATAACCAGGAAGGACGCAGAAAGGCAGCTTTTGGCACCAGGAAATAGCGCTGGAGCTTTC<br>421 CTTATTAGAGAAAGTGAAACATTAAAAGGAAGCTTCTCTCTGTCTGTCAGAGACTTTGAC<br>481 CCTGTGCATGGTGATGTTATTAAGCACTACAAAATTAGAAGTCTGGATAATGGGGGCTAT<br>541 TACATCTCTCCACGAATCACTTTTCCCTGTATCAGCGACATGATTAAACATTACCAAAAG<br>601 CAGGCAGATGGCTTGTGCAGAAGATTGGAGAAGGCTTGTATTAGTCCCAAGCCACAGAAG<br>661 CCATGGGATAAAGATGCCTGGGAGATCCCCCGGGAGTCCATCAAGTTGGTGAAAAGGCTT<br>721 GGCGCTGGGCAGTTTGGGGAAGTCTGGATGGGTTACTATAACAACAGTACCAAGGTGGCT<br>781 GTGAAAACCCTGAAGCCAGGAACTATGTCTGTGCAAGCCTTCCTGGAAGAAGCCAACCTC<br>841 ATGAAGACCCTGCAGCATGACAAGCTCGTGAGGCTCTACGCTGTGGTCACCAGGGAGGAG<br>901 CCCATTTACATCATCACCGAGTACATGGCCAAGGGCAGTTTGCTGGATTTCCTGAAGAGC<br>961 GATGAAGGTGGCAAAGTGCTGCTTCCAAAGCTCATTGACTTTTCTGCTCAGATTGCAGAG<br>1021 GGAATGGCATACATTGAGAGGAAAAACTACATTCACCGGGACCTGCGAGCAGCAAACGTC<br>1081 CTGGTCTCCGAGTCGCTCATGTGCAAAATTGCGGATTTTGGGCTTGCTCGAGTGATTGAA<br>1141 GATAACGAGTACACAGCAAGGGAAGGTGCTAAATTCCCCATCAAGTGGACTGCTCCAGAA<br>1201 GCAATCAATTTTGGATGTTTCACTATTAAGTCTGACGTGTGGTCCTTCGGAATCCTCCTG<br>1261 TATGAAATCGTCACCTATGGGAAAATCCCCTACCCAGGGAGAACTAATGCCGACGTGATG<br>1321 ACTGCCCTGTCACAGGGCTACAGGATGCCCCGCATGGAGAACTGCCCAGATGAGCTCTAT<br>1381 GACATCATGAAAATGTGCTGGAAAGAAAAGGCGGAAGAGAGGCCGACGTTTGATTACTTA<br>1441 CAGAGCGTCCTGGATGATTTCTACACTGCCACGGAAGGGCAGTATCAGCAGCAGCCTTAG<br>1501 AGCGCAGGAAGACCCGTC | |
| | | 1 M G C I K S K G K D S L S D D G V D L K<br>1 ATGGGATGTATAAAATCAAAAGGGAAAGACAGCTTGAGTGACGATGGAGTAGATTTGAAG<br><br>21 T Q P V P E S Q L L P G Q R F Q T K D P<br>61 ACTCAACCAGTTCCAGAATCTCAGCTTTTACCTGGACAGAGGTTTCAAACTAAAGATCCA<br><br>41 E E Q G D I V V A L Y P Y D G I H P D D<br>121 GAGGAACAAGGAGACATTGTGGTAGCCTTGTACCCCTATGATGGCATCCACCCGGACGAC<br><br>61 L S F K K G E K M K V L E E H G E W W K<br>181 TTGTCTTTCAAGAAAGGAGAGAAGATGAAAGTCCTGGAGGAGCATGGAGAATGGTGGAAA<br><br>81 A K S L L T K K E G F I P S N Y V A K L<br>241 GCAAAGTCCCTTTTAACAAAAAAAGAAGGCTTCATCCCCAGCAACTATGTGGCCAAACTC<br><br>101 N T L E T E E W F F K D I T R K D A E R<br>301 AACACCTTAGAAACAGAAGAGTGGTTTTTCAAGGATATAACCAGGAAGGACGCAGAAAGG<br><br>121 Q L L A P G N S A G A F L I R E S E T L<br>361 CAGCTTTTGGCACCAGGAAATAGCGCTGGAGCTTTCCTTATTAGAGAAAGTGAAACATTA<br><br>141 K G S F S L S V R D F D P V H G D V I K | SEQ ID NO:179 |

| Gene Name | GenBank Homology | DNA SEQUENCE / DEDUCED AMINO ACID SEQUENCE | SEQUENCE IDENTIFIER: |
|---|---|---|---|
| | | 421 AAAGGAAGCTTCTCTCTGTCTGTCAGAGACTTTGACCCTGTGCATGGTGATGTTATTAAG<br><br>161 H Y K I R S L D N G G Y Y I S P R I T F<br>481 CACTACAAAATTAGAAGTCTGGATAATGGGGGCTATTACATCTCTCCACGAATCACTTTT<br><br>181 P C I S D M I K H Y Q K Q A D G L C R R<br>541 CCCTGTATCAGCGACATGATTAAACATTACCAAAAGCAGGCAGATGGCTTGTGCAGAAGA<br><br>201 L E K A C I S P K P Q K P W D K D A W E<br>601 TTGGAGAAGGCTTGTATTAGTCCCAAGCCACAGAAGCCATGGGATAAAGATGCCTGGGAG<br><br>221 I P R E S I K L V K R L G A G Q F G E V<br>661 ATCCCCCGGGAGTCCATCAAGTTGGTGAAAAGGCTTGGCGCTGGGCAGTTTGGGGAAGTC<br><br>241 W M G Y Y N N S T K V A V K T L K P G T<br>721 TGGATGGGTTACTATAACAACAGTACCAAGGTGGCTGTGAAAACCCTGAAGCCAGGAACT<br><br>261 M S V Q A F L E E A N L M K T L Q H D K<br>781 ATGTCTGTGCAAGCCTTCCTGGAAGAAGCCAACCTCATGAAGACCCTGCAGCATGACAAG<br><br>281 L V R L Y A V V T R E E P I Y I I T E Y<br>841 CTCGTGAGGCTCTACGCTGTGGTCACCAGGGAGGAGCCCATTTACATCATCACCGAGTAC<br><br>301 M A K G S L L D F L K S D E G G K V L L<br>901 ATGGCCAAGGGCAGTTTGCTGGATTTCCTGAAGAGCGATGAAGGTGGCAAAGTGCTGCTT<br><br>321 P K L I D F S A Q I A E G M A Y I E R K<br>961 CCAAAGCTCATTGACTTTTCTGCTCAGATTGCAGAGGGAATGGCATACATTGAGAGGAAA<br><br>341 N Y I H R D L R A A N V L V S E S L M C<br>1021 AACTACATTCACCGGGACCTGCGAGCAGCAAACGTCCTGGTCTCCGAGTCGCTCATGTGC<br><br>361 K I A D F G L A R V I E D N E Y T A R E<br>1081 AAAATTGCGGATTTTGGGCTTGCTCGAGTGATTGAAGATAACGAGTACACAGCAAGGGAA<br><br>381 G A K F P I K W T A P E A I N F G C F T<br>1141 GGTGCTAAATTCCCCATCAAGTGGACTGCTCCAGAAGCAATCAATTTTGGATGTTTCACT<br><br>401 I K S D V W S F G I L L Y E I V T Y G K<br>1201 ATTAAGTCTGACGTGTGGTCCTTCGGAATCCTCCTGTATGAAATCGTCACCTATGGGAAA<br><br>421 I P Y P G R T N A D V M T A L S Q G Y R<br>1261 ATCCCCTACCCAGGGAGAACTAATGCCGACGTGATGACTGCCCTGTCACAGGGCTACAGG | |

| Gene Name | GenBank Homology | DNA SEQUENCE / DEDUCED AMINO ACID SEQUENCE | SEQUENCE IDENTIFIER: |
|---|---|---|---|
| | | 441 M P R M E N C P D E L Y D I M K M C W K<br>1321 ATGCCCCGCATGGAGAACTGCCCAGATGAGCTCTATGACATCATGAAAATGTGCTGGAAA<br><br>461 E K A E E R P T F D Y L Q S V L D D F Y<br>1381 GAAAAGGCGGAAGAGAGGCCGACGTTTGATTACTTACAGAGCGTCCTGGATGATTTCTAC<br><br>481 T A T E G Q Y Q Q Q P -<br>1441 ACTGCCACGGAAGGGCAGTATCAGCAGCAGCCTTAG | |
| WBC040E12.bFSP_20 021101.esd | Homo sapiens arachidonate 5-lipoxygenase-activating protein (ALOX5AP). | 1 ACTTCCCCTTCCTGTACAGGGCAGGTTGTGCAGCTGGAGGCAGAGCAGTCCTCACTGCGG<br>61 GGAGCCTGGAGCGAACATGGATCAAGAAACCGTGGGCAATGTTGTCCTGCTGGCCATCGT<br>121 CACCCTCATTAGCGTGATCCAGAATGGGTTCTTCGCCCACAAGGTGGAACACGAAAGCAA<br>181 GACTCAGAATGGGCGGAGCTTCCAGAGGACGGGAACACTTGCCTTTGAGCGGGTCTACAC<br>241 TGCCAACCAAAACTGTGTAGACGCGTACCCCACTTTCCTTGTCATGCTCTGGAGCGCCGG<br>301 GCTACTCTGCAGCCAAGTTCCTGCCGCCTTTGCTGGGCTGATGTACCTGTTCGTGAGGCA<br>361 GAAGTACTTCGTGGGCTATCTGGGAGAGAGAAGGCAGAGCACACCCGGCTACATATTTGG<br>421 GAAACGCATCATACTGTTCCTGTTCCTCATGTCCCTTGCTGGAATATTCAACTATTATCT<br>481 CATCCTCTTTTTCGGAAGTGACTTTGAAAACTACATAAAGACGATAACCACCACCATCTC<br>541 CCCTCTACTTCTCATTCCCTAACTCTCTGCTGAATACAGGGTTGGTGCTCTCATCTAATC<br>601 AATACCTACAAGTCATCATAATTCAGCTCTTGAGAGCATTCTGCTCTTCTTTAGATTGCT<br>661 GTAAATCTAATGACCATCTGGGCTTCGCGTCTTGAGTTAACCTTGCTTTTCCTGGAACAA<br>721 AATGATGTCATGTCAGCTCCGCCCCTTGAACATGACCGTGGCCTCAGATTTGTTATTCAG<br>781 ATCCATTCTGTGTGTTTCTTGACTTACCCTGCTTTCTGAAGATATTTTGTGACCCAGATT<br>841 TATGTTTTCCTAAAATAAAATGCAGAGACATGTTTT | SEQ ID NO:180 |
| | | 1 M D Q E T V G N V V L L A I V T L I S V<br>1 ATGGATCAAGAAACCGTGGGCAATGTTGTCCTGCTGGCCATCGTCACCCTCATTAGCGTG<br><br>21 I Q N G F F A H K V E H E S K T Q N G R<br>61 ATCCAGAATGGGTTCTTCGCCCACAAGGTGGAACACGAAAGCAAGACTCAGAATGGGCGG<br><br>41 S F Q R T G T L A F E R V Y T A N Q N C<br>121 AGCTTCCAGAGGACGGGAACACTTGCCTTTGAGCGGGTCTACACTGCCAACCAAAACTGT<br><br>61 V D A Y P T F L V M L W S A G L L C S Q<br>181 GTAGACGCGTACCCCACTTTCCTTGTCATGCTCTGGAGCGCCGGGCTACTCTGCAGCCAA<br><br>81 V P A A F A G L M Y L F V R Q K Y F V G<br>241 GTTCCTGCCGCCTTTGCTGGGCTGATGTACCTGTTCGTGAGGCAGAAGTACTTCGTGGGC<br><br>101 Y L G E R R Q S T P G Y I F G K R I I L | SEQ ID NO:181 |

| Gene Name | GenBank Homology | DNA SEQUENCE / DEDUCED AMINO ACID SEQUENCE | SEQUENCE IDENTIFIER: |
|---|---|---|---|
| | | 301 TATCTGGGAGAGAGAAGGCAGAGCACACCCGGCTACATATTTGGGAAACGCATCATACTG<br><br>121 F L F L M S L A G I F N Y Y L I L F F G<br>361 TTCCTGTTCCTCATGTCCCTTGCTGGAATATTCAACTATTATCTCATCCTCTTTTTCGGA<br><br>141 S D F E N Y I K T I T T T I S P L L L I<br>421 AGTGACTTTGAAAACTACATAAAGACGATAACCACCACCATCTCCCCTCTACTTCTCATT<br><br>161 P -<br>481 CCCTAA | |
| WBC043B10.bFSP_20 021401.esd | H.sapiens mRNA for P1 protein (P1.h). | 1 CGGCACGAGGCGACTTTGGTGGAGGTAGTTCTTTGGCAGCGGGCATGGCGGGTACCGTGG<br>61 TGCTGGACGATGTGGAGCTGCGGGAGGCTCAGAGAGATTACCTGGACTTCCTGGACGACG<br>121 AGGAAGACCAGGGAATTTATCAGAGCAAAGTTCGGGAGCTGATCAGTGACAACCAATACC<br>181 GGCTGATTGTCAATGTGAATGACCTGCGCAGGAAAAACGAGAAGAGGGCTAACCGGCTTC<br>241 TGAACAATGCCTTTGAGGAGCTGGTTGCCTTCCAGCGGGCCTTAAAGGATTTTGTGGCCT<br>301 CCATTGATGCTACCTATGCCAAGCAGTATGAGGAGTTCTACGTAGGACTGGAAGGCAGCT<br>361 TTGGCTCCAAGCACGTCTCCCCGCGGACTCTTACCTCCTGCTTCCTCAGCTGTGTGGTCT<br>421 GTGTGGAGGGCATTGTCACTAAATGTTCTCTAGTTCGTCCCAAAGTCGTCCGCAGTGTCC<br>481 ACTACTGTCCTGCTACTAAGAAGACCATAGAGCGACGTTATTCTGATCTCACCACCCTGG<br>541 TGGCCTTTCCCTCCAGCTCTGTCTATCCTACCAAGGATGAGGAGAACAATCCCCTTGAGA<br>601 CAGAATATGGCCTTTCTGTCTACAAGGATCACCAGACCATCACCATCCAGGAGATGCCGG<br>661 AGAAGGCCCCAGCCGGCCAGCTCCCCCGCTCTGTGGACGTCATTCTGGATGATGACTTGG<br>721 TGGATAAAGCGAAGCCTGGTGACCGGGTTCAGGTGGTGGGAACCTACCGTTGCCTTCCTG<br>781 GAAAGAAGGGAGGCTACACCTCTGGGACCTTCAGGACTGTCCTGATTGCCTGTAATGTTA<br>841 AGCAGATGAGCAAGGATGCTCAGCCCTCTTTCTCTGCTGAGGATATAGCCAAGATCAAGA<br>901 AGTTCAGTAAAACCCGATCCAAGGATATCTTTGACCAGCTGGCCAAGTCATTGGCCCCAA<br>961 GTATCCATGGGCATGACTATGTCAAGAAAGCAATCCTCTGCTTGCTCTTGGGAGGGGTGG<br>1021 AACGAGACCTAGAAAATGGCAGCCACATCCGTGGGGACATCAATATTCTTCTAATAGGAG<br>1081 ACCCATCCGTTGCCAAGTCTCAGCTTCTGCGGTATGTGCTTTGCACTGCACCCCGAGCTA<br>1141 TCCCCACCACTGGCCGGGGCTCCTCTGGAGTGGGTCTGACGGCTGCTGTCACCACAGACC<br>1201 AGGAAACAGGAGAGCGCCGTCTGGAAGCAGGGGCCATGGTCCTGGCTGACCGAGGCGTGG<br>1261 TTTGCATCGATGAATTTGACAAGATGTCTGACATGGACCGCACGGCCATCCACGAAGTGA<br>1321 TGGAGCAGGGTCGAGTCACTATCGCCAAGGCTGGCATCCATGCTCGACTGAATGCCCGCT<br>1381 GCAGTGTTTTAGCAGCTGCCAACCCCGTCTATGGCAGGTATGATCAGTACAAGACCCCTA<br>1441 TGGAGAACATCGGGCTGCAGGACTCCCTGCTCTCCCGATTTGACTTGCTCTTCATCATGC<br>1501 TGGATCAGATGGACCCTGAGCAGGATCGGGAGATCTCGGACCATGTCCTTAGGATGCACC<br>1561 GTTACAGGGCACCTGGGGAGCAGGATGGCGATGCTATGCCTTTGGGTAGTGCTGTGGATA<br>1621 TCCTGGCTACAGACGATCCCAACTTTAGCCAGGAAGACCAGCAGGACACCCAGATTTATG<br>1681 AGAAGCATGACAACCTTCTGCATGGACCAAGAAGAAAAAGGAGAAGATGGTGAATGCAG<br>1741 CTTTCATGAAGAAGTACATCCACGTGGCCAAAATCATCAAGCCCATCCTAACACAGGAGT<br>1801 CAGCTGCTTACATTGCAGAAGAGTACTCCCGCCTGCGCAGCCAAGACAGCATGAGCTCGG | SEQ ID NO:182 |

| Gene Name | GenBank Homology | DNA SEQUENCE / DEDUCED AMINO ACID SEQUENCE | SEQUENCE IDENTIFIER: |
|---|---|---|---|
| | | 1861 ACACCGCCAGGACATCTCCAGTTACAGCCCGGACGCTGGAAACTCTGATTCGCTTAGCCA<br>1921 CAGCCCATGCCAAGGCTCGCATGAGCAAGACGGTGGACCTGCAGGACGCCGAGGAAGCTG<br>1981 TGGAGTTGGTCCAGTATGCTTACTTTAAGAAGGTTCTGGAGAAGGAGAAGAAACGTAAGA<br>2041 AGCGAAGTGAGGATGAATCAGAGACAGAAGATGAAGAGGAGAAAAGCCAAGAGGACCAGG<br>2101 AGCAGAAGAGGAAGAGAAGGAAGACTCGCCAGCCAGATGCCAAAGATGGGGATTCATACG<br>2161 ACCCCTATGACTTCAGTGACACAGAGGAGGAAATGCCTCAAGTACACACTCCAAAGACGG<br>2221 CAGACTCACAGGAGACCAAGGAATCCCAGAAAGTGGAGTTGAGTGAATCCAGGTTGAAGG<br>2281 CATTCAAGGTGGCCCTCTTGGATGTGTTCCGGGAAGCTCATGCGCAGTCAATCGGCATGA<br>2341 ATCGCCTCACAGAATCCATCAACCGGGACAGCGAAGAGCCCTTCTCTTCAGTTGAGATCC<br>2401 AGGCTGCTCTGAGCAAGATGCAGGATGACAATCAGGTCATGGTGTCTGAGGGCATCATCT<br>2461 TCCTCATCTGAGGAGGCCTCGTCTCTGAACTTGGGTTGTGCCGAGAGAGTTTGTTCTGTG<br>2521 TTTCCCACCCTCTCCCTGACCCAAGTCTTTGCCTCTACTCCCTTAACAGTGTTGAATTCA<br>2581 ACTGAAGGCGAGGAATGTTGGTGATGAAGCTGAGTTCAGGAGTCGGTGGAAGCCTTGGGA<br>2641 ATGGGCAATGAAAGCTGCCGTGAGGTAAAGAGAAGAAAGTAGGAAGGACCAAGACTATTG<br>2701 CTTCTTCATTGCAAAAGCACTGGCTCATCCGCCCTACTTCCCATCCCACACAAACCCAAT<br>2761 TGTAAATAACATATGACTTCTGATTAATTTCGGGGGCACAACTGTTTTCTGTTTGCTGTT<br>2821 TTTTTGTTTTGTTTTCTTCCTCCCGAGCACTTTGGTCCAGAATAGGCTTTGGATTGTTTT<br>2881 ATGCTGCTGGAGAGAAGCTCTGAGGCAAGTCATGGAGGCCAAAAAAGCTTATGTGCAGTA<br>2941 GCATCAGTTAAGGTGAATATGTATTGTATCACAAAACAAACCCAATATCCAGATGAATAT<br>3001 CCAAGACATTGAATAAACTTACCCATTTCGTACAAAAAAAGGGGGGCCCGGTAAAC<br><br>1 M A G T V V L D D V E L R E A Q R D Y L<br>1 ATGGCGGGTACCGTGGTGCTGGACGATGTGGAGCTGCGGGAGGCTCAGAGAGATTACCTG<br><br>21 D F L D D E E D Q G I Y Q S K V R E L I<br>61 GACTTCCTGGACGACGAGGAAGACCAGGGAATTTATCAGAGCAAAGTTCGGGAGCTGATC<br><br>41 S D N Q Y R L I V N V N D L R R K N E K<br>121 AGTGACAACCAATACCGGCTGATTGTCAATGTGAATGACCTGCGCAGGAAAAACGAGAAG<br><br>61 R A N R L L N N A F E E L V A F Q R A L<br>181 AGGGCTAACCGGCTTCTGAACAATGCCTTTGAGGAGCTGGTTGCCTTCCAGCGGGCCTTA<br><br>81 K D F V A S I D A T Y A K Q Y E E F Y V<br>241 AAGGATTTTGTGGCCTCCATTGATGCTACCTATGCCAAGCAGTATGAGGAGTTCTACGTA<br><br>101 G L E G S F G S K H V S P R T L T S C F<br>301 GGACTGGAAGGCAGCTTTGGCTCCAAGCACGTCTCCCCGCGGACTCTTACCTCCTGCTTC<br><br>121 L S C V V C V E G I V T K C S L V R P K<br>361 CTCAGCTGTGTGGTCTGTGTGGAGGGCATTGTCACTAAATGTTCTCTAGTTCGTCCCAAA<br><br>141 V V R S V H Y C P A T K K T I E R R Y S | SEQ ID NO:183 |

| Gene Name | GenBank Homology | DNA SEQUENCE / DEDUCED AMINO ACID SEQUENCE | SEQUENCE IDENTIFIER: |
|---|---|---|---|
| | | 421 GTCGTCCGCAGTGTCCACTACTGTCCTGCTACTAAGAAGACCATAGAGCGACGTTATTCT<br><br>161 D L T T L V A F P S S S V Y P T K D E E<br>481 GATCTCACCACCCTGGTGGCCTTTCCCTCCAGCTCTGTCTATCCTACCAAGGATGAGGAG<br><br>181 N N P L E T E Y G L S V Y K D H Q T I T<br>541 AACAATCCCCTTGAGACAGAATATGGCCTTTCTGTCTACAAGGATCACCAGACCATCACC<br><br>201 I Q E M P E K A P A G Q L P R S V D V I<br>601 ATCCAGGAGATGCCGGAGAAGGCCCCAGCCGGCCAGCTCCCCCGCTCTGTGGACGTCATT<br><br>221 L D D D L V D K A K P G D R V Q V V G T<br>661 CTGGATGATGACTTGGTGGATAAAGCGAAGCCTGGTGACCGGGTTCAGGTGGTGGGAACC<br><br>241 Y R C L P G K K G G Y T S G T F R T V L<br>721 TACCGTTGCCTTCCTGGAAAGAAGGGAGGCTACACCTCTGGGACCTTCAGGACTGTCCTG<br><br>261 I A C N V K Q M S K D A Q P S F S A E D<br>781 ATTGCCTGTAATGTTAAGCAGATGAGCAAGGATGCTCAGCCCTCTTTCTCTGCTGAGGAT<br><br>281 I A K I K K F S K T R S K D I F D Q L A<br>841 ATAGCCAAGATCAAGAAGTTCAGTAAAACCCGATCCAAGGATATCTTTGACCAGCTGGCC<br><br>301 K S L A P S I H G H D Y V K K A I L C L<br>901 AAGTCATTGGCCCCAAGTATCCATGGGCATGACTATGTCAAGAAAGCAATCCTCTGCTTG<br><br>321 L L G G V E R D L E N G S H I R G D I N<br>961 CTCTTGGGAGGGGTGGAACGAGACCTAGAAAATGGCAGCCACATCCGTGGGGACATCAAT<br><br>341 I L L I G D P S V A K S Q L L R Y V L C<br>1021 ATTCTTCTAATAGGAGACCCATCCGTTGCCAAGTCTCAGCTTCTGCGGTATGTGCTTTGC<br><br>361 T A P R A I P T T G R G S S G V G L T A<br>1081 ACTGCACCCCGAGCTATCCCCACCACTGGCCGGGGCTCCTCTGGAGTGGGTCTGACGGCT<br><br>381 A V T T D Q E T G E R R L E A G A M V L<br>1141 GCTGTCACCACAGACCAGGAAACAGGAGAGCGCCGTCTGGAAGCAGGGGCCATGGTCCTG<br><br>401 A D R G V V C I D E F D K M S D M D R T<br>1201 GCTGACCGAGGCGTGGTTTGCATCGATGAATTTGACAAGATGTCTGACATGGACCGCACG<br><br>421 A I H E V M E Q G R V T I A K A G I H A<br>1261 GCCATCCACGAAGTGATGGAGCAGGGTCGAGTCACTATCGCCAAGGCTGGCATCCATGCT | |

| Gene Name | GenBank Homology | DNA SEQUENCE / DEDUCED AMINO ACID SEQUENCE | SEQUENCE IDENTIFIER: |
|---|---|---|---|
| | | 441 R L N A R C S V L A A A N P V Y G R Y D<br>1321 CGACTGAATGCCCGCTGCAGTGTTTTAGCAGCTGCCAACCCCGTCTATGGCAGGTATGAT<br><br>461 Q Y K T P M E N I G L Q D S L L S R F D<br>1381 CAGTACAAGACCCCTATGGAGAACATCGGGCTGCAGGACTCCCTGCTCTCCCGATTTGAC<br><br>481 L L F I M L D Q M D P E Q D R E I S D H<br>1441 TTGCTCTTCATCATGCTGGATCAGATGGACCCTGAGCAGGATCGGGAGATCTCGGACCAT<br><br>501 V L R M H R Y R A P G E Q D G D A M P L<br>1501 GTCCTTAGGATGCACCGTTACAGGGCACCTGGGGAGCAGGATGGCGATGCTATGCCTTTG<br><br>521 G S A V D I L A T D D P N F S Q E D Q Q<br>1561 GGTAGTGCTGTGGATATCCTGGCTACAGACGATCCCAACTTTAGCCAGGAAGACCAGCAG<br><br>541 D T Q I Y E K H D N L L H G T K K K K E<br>1621 GACACCCAGATTTATGAGAAGCATGACAACCTTCTGCATGGGACCAAGAAGAAAAAGGAG<br><br>561 K M V N A A F M K K Y I H V A K I I K P<br>1681 AAGATGGTGAATGCAGCTTTCATGAAGAAGTACATCCACGTGGCCAAAATCATCAAGCCC<br><br>581 I L T Q E S A A Y I A E E Y S R L R S Q<br>1741 ATCCTAACACAGGAGTCAGCTGCTTACATTGCAGAAGAGTACTCCCGCCTGCGCAGCCAA<br><br>601 D S M S S D T A R T S P V T A R T L E T<br>1801 GACAGCATGAGCTCGGACACCGCCAGGACATCTCCAGTTACAGCCCGGACGCTGGAAACT<br><br>621 L I R L A T A H A K A R M S K T V D L Q<br>1861 CTGATTCGCTTAGCCACAGCCCATGCCAAGGCTCGCATGAGCAAGACGGTGGACCTGCAG<br><br>641 D A E E A V E L V Q Y A Y F K K V L E K<br>1921 GACGCCGAGGAAGCTGTGGAGTTGGTCCAGTATGCTTACTTTAAGAAGGTTCTGGAGAAG<br><br>661 E K K R K K R S E D E S E T E D E E E K<br>1981 GAGAAGAAACGTAAGAAGCGAAGTGAGGATGAATCAGAGACAGAAGATGAAGAGGAGAAA<br><br>681 S Q E D Q E Q K R K R R K T R Q P D A K<br>2041 AGCCAAGAGGACCAGGAGCAGAAGAGGAAGAGAAGGAAGACTCGCCAGCCAGATGCCAAA<br><br>701 D G D S Y D P Y D F S D T E E E M P Q V<br>2101 GATGGGGATTCATACGACCCCTATGACTTCAGTGACACAGAGGAGGAAATGCCTCAAGTA | |

| Gene Name | GenBank Homology | DNA SEQUENCE / DEDUCED AMINO ACID SEQUENCE | SEQUENCE IDENTIFIER: |
|---|---|---|---|
| | | 721 H T P K T A D S Q E T K E S Q K V E L S<br>2161 CACACTCCAAAGACGGCAGACTCACAGGAGACCAAGGAATCCCAGAAAGTGGAGTTGAGT<br><br>741 E S R L K A F K V A L L D V F R E A H A<br>2221 GAATCCAGGTTGAAGGCATTCAAGGTGGCCCTCTTGGATGTGTTCCGGGAAGCTCATGCG<br><br>761 Q S I G M N R L T E S I N R D S E E P F<br>2281 CAGTCAATCGGCATGAATCGCCTCACAGAATCCATCAACCGGGACAGCGAAGAGCCCTTC<br><br>781 S S V E I Q A A L S K M Q D D N Q V M V<br>2341 TCTTCAGTTGAGATCCAGGCTGCTCTGAGCAAGATGCAGGATGACAATCAGGTCATGGTG<br><br>801 S E G I I F L I -<br>2401 TCTGAGGGCATCATCTTCCTCATCTGA | |
| WBC044E01.bFSP_20<br>021501.esd | Human lymphocyte<br>dihydropyrimidine<br>dehydrogenase | 1 tttcgactcg cgctccggct gctgtcactt ggctctctgg ctggagcttg aggacgcaag<br>61 gagggtttgt cactggcaga ctcgagactg taggcactgc catggcccct gtgctcagta<br>121 aggactcggc ggacatcgag agtatcctgg ctttaaatcc tcgaacacaa actcatgcaa<br>181 ctctgtgttc cacttcggcc aagaaattag acaagaaaca ttggaaaaga aatcctgata<br>241 agaactgctt taattgtgag aagctggaga ataattttga tgacatcaag cacacgactc<br>301 ttggtgagcg aggagctctc cgagaagcaa tgagatgcct gaaatgtgca gatgccccgt<br>361 gtcagaagag ctgtccaact aatcttgata ttaaatcatt catcacaagt attgcaaaca<br>421 agaactatta tggagctgct aagatgatat tttctgacaa cccacttggt ctgacttgtg<br>481 gaatggtatg tccaacctct gatctttgtg taggtggatg caatttatat gccactgaag<br>541 agggacccat taatattggt ggattgcagc aatttgctac tgaggtattc aaagcaatga<br>601 gtatcccaca gatcagaaat ccttcgctgc ctcccccaga aaaaatgtct gaagcctatt<br>661 ctgcaaagat tgctcttttt ggtgctgggc ctgcaagtat aagttgtgct tcctttttgg<br>721 ctcgattggg gtactctgac atcactatat ttgaaaaaca agaatatgtt ggtggtttaa<br>781 gtacttctga aattcctcag ttccggctgc cgtatgatgt agtgaatttt gagattgagc<br>841 taatgaagga ccttggtgta aagataattt gcggtaaaag cctttcagtg aatgaaatga<br>901 ctcttagcac tttgaaagaa aaaggctaca aagctgcttt cattggaata ggtttgccag<br>961 aacccaataa agatgccatc ttccaaggcc tgacgcagga ccaggggttt tatacatcca<br>1021 aagacttttt gccacttgta gccaaaggca gtaaagcagg aatgtgcgcc tgtcactctc<br>1081 cattgccatc gatacgggga gtcgtgattg tacttggagc tggagacact gcctttgact<br>1141 gtgcaacatc tgctctacgt tgtggagctc gccgtgtgtt catcgtcttc agaaaaggct<br>1201 ttgttaatat aagagctgtc cctgaggaga tggaacttgc taaggaagaa aagtgtgaat<br>1261 ttctgccatt cctgtcccca cggaaggtta tagtaaaagg tgggagaatt gttgctatgc<br>1321 agtttgttcg gacagagcaa gatgaaactg gaaaatggaa tgaagatgaa gatcagatgg<br>1381 tccatctgaa agccgatgtg gtcatcagtg cctttggttc agttctgagt gatcctaaag<br>1441 taaaagaagc cttgagccct ataaaattta acagatgggg tctcccagaa gtagatccag<br>1501 aaactatgca aactagtgaa gcatgggtat ttgcaggtgg tgatgtcgtt ggtttggcta<br>1561 acactacagt ggaatcggtg aatgatggaa agcaagcttc ttggtacatt cacaaatacg | SEQ ID NO:184 |

| Gene Name | GenBank Homology | DNA SEQUENCE / DEDUCED AMINO ACID SEQUENCE | SEQUENCE IDENTIFIER: |
|---|---|---|---|
| | | 1621 tacagtcaca atatggagct tccgtttctg ccaagcctga actacccctc ttttacactc<br>1681 ctattgatct ggtggacatt agtgtagaaa tggccggatt gaagtttata aatccttttg<br>1741 gtcttgctag cgcaactcca gccaccagca catcaatgat tcgaagagct tttgaagctg<br>1801 gatggggttt tgccctcacc aaaactttct ctcttgataa ggacattgtg acaaatgttt<br>1861 cccccagaat catccgggga accacctctg gccccatgta tggccctgga caaagctcct<br>1921 ttctgaatat tgagctcatc agtgagaaaa cggctgcata ttggtgtcaa agtgtcactg<br>1981 aactaaaggc tgactttcca gacaacattg tgattgctag cattatgtgc agttacaata<br>2041 aaaatgactg gacggaactt gccaagaagt ctgaggattc tggagcagat gccctggagt<br>2101 taaatttatc atgtccacat ggcatgggag aaagaggaat gggcctggcc tgtgggcagg<br>2161 atccagagct ggtgcggaac atctgccgct gggttaggca agctgttcag attccttttt<br>2221 ttgccaagct gaccccaaat gtcactgata ttgtgagcat cgcaagagct gcaaaggaag<br>2281 gtggtgccaa tggcgttaca gccaccaaca ctgtctcagg tctgatggga ttaaaatctg<br>2341 atggcacacc ttggccagca gtggggattg caaagcgaac tacatatgga ggagtgtctg<br>2401 ggacagcaat cagacctatt gctttgagag ctgtgacctc cattgctcgt gctctgcctg<br>2461 gatttcccat tttggctact ggtggaattg actctgctga aagtggtctt cagtttctcc<br>2521 atagtggtgc ttccgtcctc caggtatgca gtgccattca gaatcaggat ttcactgtga<br>2581 tcgaagacta ctgcactggc ctcaaagccc tgctttatct gaaaagcatt gaagaactac<br>2641 aagactggga tggacagagt ccagctactg tgagtcacca gaaagggaaa ccagttccac<br>2701 gtatagctga actcatggac aagaaactgc caagttttgg accttatctg gaacagcgca<br>2761 agaaaatcat agcagaaaac aagattagac tgaaagaaca aaatgtagct tttcaccac<br>2821 ttaagagaaa ctgttttatc cccaaaaggc ctattcctac catcaaggat gtaataggaa<br>2881 aagcactgca gtaccttgga acatttggtg aattgagcaa cgtagagcaa gttgtggcta<br>2941 tgattgatga agaaatgtgt atcaactgtg gtaaatgcta catgacctgt aatgattctg<br>3001 gctaccaggc tatacagttt gatccagaaa cccacctgcc caccataacc gacacttgta<br>3061 caggctgtac tctgtgtctc agtgtttgcc ctattgtcga ctgcatcaaa atggtttcca<br>3121 ggacaacacc ttatgaacca aagagaggcg taccettatc tgtgaatccg gtgtgttaag<br>3181 gtgatttgtg aaacagttgc tgtgaacttt catgtcacct acatatgctg atcttttaaa<br>3241 atcatgatcc ttgtgttcag ctctttccaa attaaaacaa atatacattt tctaaataaa<br>3301 aatatgtaat ttcaaaatac atttgtaagt gtaaaaaatg tctcatgtca atgaccattc<br>3361 aattagtggt cataaaatag aataattctt ttctgaggat agtagttaaa taactgtgtg<br>3421 gcagttaatt ggatgttcac tgccagttgt cttatgtgaa aaattaactt ttttgtggca<br>3481 attagtgtga cagtttccaa attgccctat gctgtgctcc atatttgatt tctaattgta<br>3541 agtgaaatta agcattttga aacaaagtac tctttaacat acaagaaaat gtatccaagg<br>3601 aaacatttta tcattaaaaa ttacctttaa ttttaatgct gtttctaaga aaatgtagtt<br>3661 agctccataa agtacaaatg aagaaagtca aaaaattatt tgctatggca ggataagaaa<br>3721 gcctaaaatt gagtttgtag aactttatta agtaaaatcc ccttcgctga aattgcttat<br>3781 ttttggtgtt ggatagagga tagggagaat atttactaac taaataccat tcactactca<br>3841 tgcgtgagat gggtgtacaa actcatcctc ttttaatggc atttctcttt aaactatgtt<br>3901 cctaacaaaa tgagatgata ggatagatcc tggttaccac tcttttgctg tgcacatacg<br>3961 ggctctgact ggttttaata gtcaccttca tgattatagc aactaatgtt tgaacaaagc<br>4021 tcaaagtatg caatgcttca ttattcaaga atgaaaaata taatgttgat aatatatatt<br>4081 aagtgtgcca aatcagtttg actactctct gttttagtgt ttatgtttaa aagaaatata<br>4141 ttttttgtta ttattagata atatttttgt atttctctat tttcataatc agtaaatagt | |

| Gene Name | GenBank Homology | DNA SEQUENCE / DEDUCED AMINO ACID SEQUENCE | SEQUENCE IDENTIFIER: |
|---|---|---|---|
| | | 4201 gtcatataaa ctcatttatc tcctcttcat ggcatcttca atatgaatct ataagtagta<br>4261 aatcagaaag taacaatcta tggcttattt ctatgacaaa ttcaagagct agaaaaataa<br>4321 aatgtttcat tatgcacttt tagaaatgca tatttgccac aaaacctgta ttactgaata<br>4381 atatcaaata aaatatcata aagcatttt | |
| | | 1 M A P V L S K D S A D I E S I L A L N P<br>1 ATGGCCCCTGTGCTCAGTAAGGACTCGGCGGACATCGAGAGTATCCTGGCTTTAAATCCT<br><br>21 R T Q T H A T L C S T S A K K L D K K H<br>61 CGAACACAAACTCATGCAACTCTGTGTTCCACTTCGGCCAAGAAATTAGACAAGAAACAT<br><br>41 W K R N P D K N C F N C E K L E N N F D<br>121 TGGAAAAGAAATCCTGATAAGAACTGCTTTAATTGTGAGAAGCTGGAGAATAATTTTGAT<br><br>61 D I K H T T L G E R G A L R E A M R C L<br>181 GACATCAAGCACACGACTCTTGGTGAGCGAGGAGCTCTCCGAGAAGCAATGAGATGCCTG<br><br>81 K C A D A P C Q K S C P T N L D I K S F<br>241 AAATGTGCAGATGCCCCGTGTCAGAAGAGCTGTCCAACTAATCTTGATATTAAATCATTC<br><br>101 I T S I A N K N Y Y G A A K M I F S D N<br>301 ATCACAAGTATTGCAAACAAGAACTATTATGGAGCTGCTAAGATGATATTTTCTGACAAC<br><br>121 P L G L T C G M V C P T S D L C V G G C<br>361 CCACTTGGTCTGACTTGTGGAATGGTATGTCCAACCTCTGATCTTTGTGTAGGTGGATGC<br><br>141 N L Y A T E E G P I N I G G L Q Q F A T<br>421 AATTTATATGCCACTGAAGAGGGACCCATTAATATTGGTGGATTGCAGCAATTTGCTACT<br><br>161 E V F K A M S I P Q I R N P S L P P P E<br>481 GAGGTATTCAAAGCAATGAGTATCCCACAGATCAGAAATCCTTCGCTGCCTCCCCCAGAA<br><br>181 K M S E A Y S A K I A L F G A G P A S I<br>541 AAAATGTCTGAAGCCTATTCTGCAAAGATTGCTCTTTTTGGTGCTGGGCCTGCAAGTATA<br><br>201 S C A S F L A R L G Y S D I T I F E K Q<br>601 AGTTGTGCTTCCTTTTTGGCTCGATTGGGGTACTCTGACATCACTATATTTGAAAAACAA<br><br>221 E Y V G G L S T S E I P Q F R L P Y D V<br>661 GAATATGTTGGTGGTTTAAGTACTTCTGAAATTCCTCAGTTCCGGCTGCCGTATGATGTA<br><br>241 V N F E I E L M K D L G V K I I C G K S<br>721 GTGAATTTTGAGATTGAGCTAATGAAGGACCTTGGTGTAAAGATAATTTGCGGTAAAAGC | SEQ ID NO:185 |

| Gene Name | GenBank Homology | DNA SEQUENCE / DEDUCED AMINO ACID SEQUENCE | SEQUENCE IDENTIFIER: |
|---|---|---|---|
| | | 261 L S V N E M T L S T L K E K G Y K A A F<br>781 CTTTCAGTGAATGAAATGACTCTTAGCACTTTGAAAGAAAAAGGCTACAAAGCTGCTTTC<br><br>281 I G I G L P E P N K D A I F Q G L T Q D<br>841 ATTGGAATAGGTTTGCCAGAACCCAATAAAGATGCCATCTTCCAAGGCCTGACGCAGGAC<br><br>301 Q G F Y T S K D F L P L V A K G S K A G<br>901 CAGGGGTTTTATACATCCAAAGACTTTTTGCCACTTGTAGCCAAAGGCAGTAAAGCAGGA<br><br>321 M C A C H S P L P S I R G V V I V L G A<br>961 ATGTGCGCCTGTCACTCTCCATTGCCATCGATACGGGGAGTCGTGATTGTACTTGGAGCT<br><br>341 G D T A F D C A T S A L R C G A R R V F<br>1021 GGAGACACTGCCTTTGACTGTGCAACATCTGCTCTACGTTGTGGAGCTCGCCGTGTGTTC<br><br>361 I V F R K G F V N I R A V P E E M E L A<br>1081 ATCGTCTTCAGAAAAGGCTTTGTTAATATAAGAGCTGTCCCTGAGGAGATGGAACTTGCT<br><br>381 K E E K C E F L P F L S P R K V I V K G<br>1141 AAGGAAGAAAAGTGTGAATTTCTGCCATTCCTGTCCCCACGGAAGGTTATAGTAAAAGGT<br><br>401 G R I V A M Q F V R T E Q D E T G K W N<br>1201 GGGAGAATTGTTGCTATGCAGTTTGTTCGGACAGAGCAAGATGAAACTGGAAAATGGAAT<br><br>421 E D E D Q M V H L K A D V V I S A F G S<br>1261 GAAGATGAAGATCAGATGGTCCATCTGAAAGCCGATGTGGTCATCAGTGCCTTTGGTTCA<br><br>441 V L S D P K V K E A L S P I K F N R W G<br>1321 GTTCTGAGTGATCCTAAAGTAAAAGAAGCCTTGAGCCCTATAAAATTTAACAGATGGGGT<br><br>461 L P E V D P E T M Q T S E A W V F A G G<br>1381 CTCCCAGAAGTAGATCCAGAAACTATGCAAACTAGTGAAGCATGGGTATTTGCAGGTGGT<br><br>481 D V V G L A N T T V E S V N D G K Q A S<br>1441 GATGTCGTTGGTTTGGCTAACACTACAGTGGAATCGGTGAATGATGGAAAGCAAGCTTCT<br><br>501 W Y I H K Y V Q S Q Y G A S V S A K P E<br>1501 TGGTACATTCACAAATACGTACAGTCACAATATGGAGCTTCCGTTTCTGCCAAGCCTGAA<br><br>521 L P L F Y T P I D L V D I S V E M A G L<br>1561 CTACCCCTCTTTTACACTCCTATTGATCTGGTGGACATTAGTGTAGAAATGGCCGGATTG | |

| Gene Name | GenBank Homology | DNA SEQUENCE / DEDUCED AMINO ACID SEQUENCE | SEQUENCE IDENTIFIER: |
|---|---|---|---|
| | | 541 K F I N P F G L A S A T P A T S T S M I<br>1621 AAGTTTATAAATCCTTTTGGTCTTGCTAGCGCAACTCCAGCCACCAGCACATCAATGATT<br><br>561 R R A F E A G W G F A L T K T F S L D K<br>1681 CGAAGAGCTTTTGAAGCTGGATGGGGTTTTGCCCTCACCAAAACTTTCTCTCTTGATAAG<br><br>581 D I V T N V S P R I I R G T T S G P M Y<br>1741 GACATTGTGACAAATGTTTCCCCCAGAATCATCCGGGGAACCACCTCTGGCCCCATGTAT<br><br>601 G P G Q S S F L N I E L I S E K T A A Y<br>1801 GGCCCTGGACAAAGCTCCTTTCTGAATATTGAGCTCATCAGTGAGAAAACGGCTGCATAT<br><br>621 W C Q S V T E L K A D F P D N I V I A S<br>1861 TGGTGTCAAAGTGTCACTGAACTAAAGGCTGACTTTCCAGACAACATTGTGATTGCTAGC<br><br>641 I M C S Y N K N D W T E L A K K S E D S<br>1921 ATTATGTGCAGTTACAATAAAAATGACTGGACGGAACTTGCCAAGAAGTCTGAGGATTCT<br><br>661 G A D A L E L N L S C P H G M G E R G M<br>1981 GGAGCAGATGCCCTGGAGTTAAATTTATCATGTCCACATGGCATGGGAGAAAGAGGAATG<br><br>681 G L A C G Q D P E L V R N I C R W V R Q<br>2041 GGCCTGGCCTGTGGGCAGGATCCAGAGCTGGTGCGGAACATCTGCCGCTGGGTTAGGCAA<br><br>701 A V Q I P F F A K L T P N V T D I V S I<br>2101 GCTGTTCAGATTCCTTTTTTTGCCAAGCTGACCCCAAATGTCACTGATATTGTGAGCATC<br><br>721 A R A A K E G G A N G V T A T N T V S G<br>2161 GCAAGAGCTGCAAAGGAAGGTGGTGCCAATGGCGTTACAGCCACCAACACTGTCTCAGGT<br><br>741 L M G L K S D G T P W P A V G I A K R T<br>2221 CTGATGGGATTAAAATCTGATGGCACACCTTGGCCAGCAGTGGGGATTGCAAAGCGAACT<br><br>761 T Y G G V S G T A I R P I A L R A V T S<br>2281 ACATATGGAGGAGTGTCTGGGACAGCAATCAGACCTATTGCTTTGAGAGCTGTGACCTCC<br><br>781 I A R A L P G F P I L A T G G I D S A E<br>2341 ATTGCTCGTGCTCTGCCTGGATTTCCCATTTTGGCTACTGGTGGAATTGACTCTGCTGAA<br><br>801 S G L Q F L H S G A S V L Q V C S A I Q<br>2401 AGTGGTCTTCAGTTTCTCCATAGTGGTGCTTCCGTCCTCCAGGTATGCAGTGCCATTCAG<br><br>821 N Q D F T V I E D Y C T G L K A L L Y L | |

| Gene Name | GenBank Homology | DNA SEQUENCE / DEDUCED AMINO ACID SEQUENCE | SEQUENCE IDENTIFIER: |
|---|---|---|---|
| | | 2461 AATCAGGATTTCACTGTGATCGAAGACTACTGCACTGGCCTCAAAGCCCTGCTTTATCTG<br><br>841 K S I E E L Q D W D G Q S P A T V S H Q<br>2521 AAAAGCATTGAAGAACTACAAGACTGGGATGGACAGAGTCCAGCTACTGTGAGTCACCAG<br><br>861 K G K P V P R I A E L M D K K L P S F G<br>2581 AAAGGGAAACCAGTTCCACGTATAGCTGAACTCATGGACAAGAAACTGCCAAGTTTTGGA<br><br>881 P Y L E Q R K K I I A E N K I R L K E Q<br>2641 CCTTATCTGGAACAGCGCAAGAAAATCATAGCAGAAAACAAGATTAGACTGAAAGAACAA<br><br>901 N V A F S P L K R N C F I P K R P I P T<br>2701 AATGTAGCTTTTTCACCACTTAAGAGAAACTGTTTTATCCCCAAAAGGCCTATTCCTACC<br><br>921 I K D V I G K A L Q Y L G T F G E L S N<br>2761 ATCAAGGATGTAATAGGAAAAGCACTGCAGTACCTTGGAACATTTGGTGAATTGAGCAAC<br><br>941 V E Q V V A M I D E E M C I N C G K C Y<br>2821 GTAGAGCAAGTTGTGGCTATGATTGATGAAGAAATGTGTATCAACTGTGGTAAATGCTAC<br><br>961 M T C N D S G Y Q A I Q F D P E T H L P<br>2881 ATGACCTGTAATGATTCTGGCTACCAGGCTATACAGTTTGATCCAGAAACCCACCTGCCC<br><br>981 T I T D T C T G C T L C L S V C P I V D<br>2941 ACCATAACCGACACTTGTACAGGCTGTACTCTGTGTCTCAGTGTTTGCCCTATTGTCGAC<br><br>1001 C I K M V S R T T P Y E P K R G V P L S<br>3001 TGCATCAAAATGGTTTCCAGGACAACACCTTATGAACCAAAGAGAGGCGTACCCTTATCT<br><br>1021 V N P V C -<br>3061 GTGAATCCGGTGTGTTAA | |
| WBC047A01.bFSP_20021401.esd | H.sapiens mRNA for BS69 protein. | 1 GGAGCATAATGCTAAAGAAGTAAACAGGTCATGGCACGTTTAACAAAAAGACGACAGGCG<br>61 ATACAAAAGCTATCCAGCATCTTTGGGCAGCCATTGAGATTATACGGAACCAGAAGCAGA<br>121 TTGCCAACATTGACCGTATTACAAAATGTGAAACAACTACATTATTCTTGAACCTATGGT<br>181 GATTTTTACATCATTACACAGATATGTCATTTTCATTAGTTGTATCATTGTTATAAACTG<br>241 GTATATGTCTCGAGTCCACGGTATGCACCCTAAAGAGACCACCCGTCAGCTGAGCTTAGC<br>301 TGTGAAAGATGGTCTTATTGTCGAAACTCTAACAGTGGGCTGCAAAGGTTCAAAAGCTGG<br>361 TATTGAACAAGAAGGATATTGGTTGCCAGGAGATGAGATTGACTGGGAAACAGAAAATCA<br>421 TGACTGGTATTGTTTTGAATGCCATTTGCCTGGAGAGGTGTTGATATGTGACCTGTGTTT<br>481 TCGTGTGTATCATTCCAAGTGTTTGTCTGATGAGTTCAGGCTTAGAGACAGCAGTAGTCC<br>541 CTGGCAGTGCCCAGTTTGCAGGAGCATTAAGAAGAAGAATACAAACAAACAGGAGATGGG | SEQ ID NO:186 |

| Gene Name | GenBank Homology | DNA SEQUENCE / DEDUCED AMINO ACID SEQUENCE | SEQUENCE IDENTIFIER: |
|---|---|---|---|
| | | 601 CACATACCTCAGATTCATTGTCTCCCGCATGAAGGAGAGGGCTATAGATCTTAATAAAAA<br>661 GGGGAAGGACAATAAACACCCGATGTACAGGAGGCTGGTGCACTCAGCTGTGGACGTTCC<br>721 CACCATTCAAGAGAAAGTGAATGAAGGGAAATACCGAAGTTATGAAGAGTTCAAAGCTGA<br>781 TGCCCAATTGCTTCTCCACAATACCGTGATTTTCTATGGAGCAGACAGTGAGCAAGCTGA<br>841 CATTGCGAGGATGCTATATAAAGACACATGTCATGAGCTGGATGAACTGCAGCTTTGCAA<br>901 GAATTGCTTTTACTTGTCAAATGCTCGTCCTGACAACTGGTTCTGTTATCCTTGTATACC<br>961 TAATCATGAGCTGGTTTGGGCTAAAATGAAAGGTTTTGGGTTTTGGCCAGCCAAAGTCAT<br>1021 GCAGAAAGAAGACAATCAAGTCGACGTTCGCTTCTTTGGCCACCACCACCAGAGGGCCTG<br>1081 GATTCCTTCTGAAAACATTCAAGATATCACAGTCAACATTCATCGGCTGCACGTGAAGCG<br>1141 CAGTATGGGTTGGAAAAAGGCCTGTGATGAGCTGGAGCTGCATCAGCGTTTCCTACGAGA<br>1201 AGGGAGATTTTGGAAATCTAAGAATGAGGACCGAGGTGAGGAAGAGGCAGAATCCAGTAT<br>1261 CTCCTCCACCAGTAATGAGCAGCTAAAGGTCACTCAAGAACCAAGAGCAAAGAAAGGACG<br>1321 ACGTAATCAAAGTGTGGAGCCCAAAAAGGAAGAACCAGAGCCTGAAACAGAAGCAGTAAG<br>1381 TTCTAGCCAGGAAATACCCACGATGCCTCAGCCCATCGAAAAAGTCTCCGTGTCAACTCA<br>1441 GACAAAGAAGTTAAGTGCCTCTTCACCAAGAATGCTGCATCGGAGCACCCAGACCACAAA<br>1501 CGACGGCGTGTGTCAGAGCATGTGCCATGACAAATACACCAAGATCTTCAATGACTTCAA<br>1561 AGACCGGATGAAGTCGGACCACAAGCGGGAGACAGAGCGTGTTGTCCGAGAAGCTCTGGA<br>1621 GAAGCTGCGTTCTGAAATGGAAGAAGAAAAGAGACAAGCTGTAAATAAAGCTGTAGCCAA<br>1681 CATGCAGGGTGAGATGGACAGAAAATGTAAGCAAGTAAAGGAAAAGTGTAAGGAGGAATT<br>1741 TGTAGAAGAAATCAAGAAGCTGGCAACACAGCACAAGCAACTGATTTCTCAGACCAAGAA<br>1801 GAAGCAGTGGTGCTACAACTGTGAGGAGGAGGCCATGTACCACTGCTGCTGGAACACATC<br>1861 CTACTGCTCCATCAAGTGCCAGCAGGAGCACTGGCACGCCGAGCACAAGCGCACCTGCCG<br>1921 CCGGAAGAGATGAAGGCGGCTCTTCCTGGGAGCGCCGACGACGACTCTTCTCAGACACAG<br>1981 CGGTTTTTGTTTCCAAGAAGCCAAAATTGTTTAGAATTTGCTTCCCATTTTGCACCAGCC<br>2041 TTTAAACACTTTTCGTGAAGAAATTTTGCACAGTAGTTTAAATCTTTTGTTAATGCTCCT<br>2101 CCAGAGTTTTTCAGGGGGTAAAAGTAACATCAGTGGAGGGTATTATTTTAAATAAACTTT<br>2161 AGTGAGAATTTGTTGCATTTTCAGCAAATTTTAAAACATTTTTAGGTTTTACAGAGATTT<br>2221 TAACCTTTAAACAAAAGATCTTTAAAAAACAACAGGTGAATACAAGTGAGTTTAACAAAG<br>2281 AAGCATTTAAAGTAGATCTGAATGTGAGAACTGTTTCAGAAATAAAACATACTACCTTGA<br>2341 TGTGAAATTTATTTCTTAACCTTGTTGAGCTGGTTTTGTTCAGCTTAATTTACTGTTTAA<br>2401 AGGCATTATCTATTGGTTATGCCAGTGGGTATATGATTGAATTTAGGGAACAGGGTTGAC<br>2461 ACAGCAGGTGCTAGTCCTGCATATTTTTTCGTAAATATTTCCCAATTGTGTTTTTCATTA<br>2521 TTTCTTTTCAATATATAACTTTTATAACAAATTATTAGCTTTGATCTTGTAGTTTAAAAT<br>2581 TGCAGGGAACTGGGGTAATCTTTTACTGAGCTGGATCTTAGAGAAAATGAATATTTAAAT<br>2641 TTTAAAGTTTGCACATTTCATCTTTGTCCTAACATGAGTGCTTGTAACAAAATAAAACAA<br>2701 CAAAAACAAAGCCT | |
| | | 1 M S R V H G M H P K E T T R Q L S L A V<br>1 ATGTCTCGAGTCCACGGTATGCACCCTAAAGAGACCACCCGTCAGCTGAGCTTAGCTGTG<br><br>21 K D G L I V E T L T V G C K G S K A G I<br>61 AAAGATGGTCTTATTGTCGAAACTCTAACAGTGGGCTGCAAAGGTTCAAAAGCTGGTATT | SEQ ID NO:187 |

| Gene Name | GenBank Homology | DNA SEQUENCE / DEDUCED AMINO ACID SEQUENCE | SEQUENCE IDENTIFIER: |
|---|---|---|---|
| | | 41 E Q E G Y W L P G D E I D W E T E N H D<br>121 GAACAAGAAGGATATTGGTTGCCAGGAGATGAGATTGACTGGGAAACAGAAAATCATGAC<br><br>61 W Y C F E C H L P G E V L I C D L C F R<br>181 TGGTATTGTTTTGAATGCCATTTGCCTGGAGAGGTGTTGATATGTGACCTGTGTTTTCGT<br><br>81 V Y H S K C L S D E F R L R D S S S P W<br>241 GTGTATCATTCCAAGTGTTTGTCTGATGAGTTCAGGCTTAGAGACAGCAGTAGTCCCTGG<br><br>101 Q C P V C R S I K K K N T N K Q E M G T<br>301 CAGTGCCCAGTTTGCAGGAGCATTAAGAAGAAGAATACAAACAAACAGGAGATGGGCACA<br><br>121 Y L R F I V S R M K E R A I D L N K K G<br>361 TACCTCAGATTCATTGTCTCCCGCATGAAGGAGAGGGCTATAGATCTTAATAAAAAGGGG<br><br>141 K D N K H P M Y R R L V H S A V D V P T<br>421 AAGGACAATAAACACCCGATGTACAGGAGGCTGGTGCACTCAGCTGTGGACGTTCCCACC<br><br>161 I Q E K V N E G K Y R S Y E E F K A D A<br>481 ATTCAAGAGAAAGTGAATGAAGGGAAATACCGAAGTTATGAAGAGTTCAAAGCTGATGCC<br><br>181 Q L L L H N T V I F Y G A D S E Q A D I<br>541 CAATTGCTTCTCCACAATACCGTGATTTTCTATGGAGCAGACAGTGAGCAAGCTGACATT<br><br>201 A R M L Y K D T C H E L D E L Q L C K N<br>601 GCGAGGATGCTATATAAAGACACATGTCATGAGCTGGATGAACTGCAGCTTTGCAAGAAT<br><br>221 C F Y L S N A R P D N W F C Y P C I P N<br>661 TGCTTTTACTTGTCAAATGCTCGTCCTGACAACTGGTTCTGTTATCCTTGTATACCTAAT<br><br>241 H E L V W A K M K G F G F W P A K V M Q<br>721 CATGAGCTGGTTTGGGCTAAAATGAAAGGTTTTGGGTTTTGGCCAGCCAAAGTCATGCAG<br><br>261 K E D N Q V D V R F F G H H H Q R A W I<br>781 AAAGAAGACAATCAAGTCGACGTTCGCTTCTTTGGCCACCACCACCAGAGGGCCTGGATT<br><br>281 P S E N I Q D I T V N I H R L H V K R S<br>841 CCTTCTGAAAACATTCAAGATATCACAGTCAACATTCATCGGCTGCACGTGAAGCGCAGT<br><br>301 M G W K K A C D E L E L H Q R F L R E G<br>901 ATGGGTTGGAAAAAGGCCTGTGATGAGCTGGAGCTGCATCAGCGTTTCCTACGAGAAGGG<br><br>321 R F W K S K N E D R G E E E A E S S I S | |

| Gene Name | GenBank Homology | DNA SEQUENCE / DEDUCED AMINO ACID SEQUENCE | SEQUENCE IDENTIFIER: |
|---|---|---|---|
| | | 961 AGATTTTGGAAATCTAAGAATGAGGACCGAGGTGAGGAAGAGGCAGAATCCAGTATCTCC<br><br>341 S T S N E Q L K V T Q E P R A K K G R R<br>1021 TCCACCAGTAATGAGCAGCTAAAGGTCACTCAAGAACCAAGAGCAAAGAAAGGACGACGT<br><br>361 N Q S V E P K K E E P E P E T E A V S S<br>1081 AATCAAAGTGTGGAGCCCAAAAAGGAAGAACCAGAGCCTGAAACAGAAGCAGTAAGTTCT<br><br>381 S Q E I P T M P Q P I E K V S V S T Q T<br>1141 AGCCAGGAAATACCCACGATGCCTCAGCCCATCGAAAAAGTCTCCGTGTCAACTCAGACA<br><br>401 K K L S A S S P R M L H R S T Q T T N D<br>1201 AAGAAGTTAAGTGCCTCTTCACCAAGAATGCTGCATCGGAGCACCCAGACCACAAACGAC<br><br>421 G V C Q S M C H D K Y T K I F N D F K D<br>1261 GGCGTGTGTCAGAGCATGTGCCATGACAAATACACCAAGATCTTCAATGACTTCAAAGAC<br><br>441 R M K S D H K R E T E R V V R E A L E K<br>1321 CGGATGAAGTCGGACCACAAGCGGGAGACAGAGCGTGTTGTCCGAGAAGCTCTGGAGAAG<br><br>461 L R S E M E E E K R Q A V N K A V A N M<br>1381 CTGCGTTCTGAAATGGAAGAAGAAAAGAGACAAGCTGTAAATAAAGCTGTAGCCAACATG<br><br>481 Q G E M D R K C K Q V K E K C K E E F V<br>1441 CAGGGTGAGATGGACAGAAAATGTAAGCAAGTAAAGGAAAAGTGTAAGGAGGAATTTGTA<br><br>501 E E I K K L A T Q H K Q L I S Q T K K K<br>1501 GAAGAAATCAAGAAGCTGGCAACACAGCACAAGCAACTGATTTCTCAGACCAAGAAGAAG<br><br>521 Q W C Y N C E E E A M Y H C C W N T S Y<br>1561 CAGTGGTGCTACAACTGTGAGGAGGAGGCCATGTACCACTGCTGCTGGAACACATCCTAC<br><br>541 C S I K C Q Q E H W H A E H K R T C R R<br>1621 TGCTCCATCAAGTGCCAGCAGGAGCACTGGCACGCCGAGCACAAGCGCACCTGCCGCCGG<br><br>561 K R -<br>1681 AAGAGATGA | |
| WBC048H02.bFSP_20 021501.esd | Homo sapiens guanine nucleotide binding protein (G protein), beta polypeptide 2-like | 1 CTGCAAGGCGGCGGCAGGAGAGGTTGTGGTGCTAGTTTCTCTAAGCCATCCAGTGCCATC<br>61 CTCGTCGCTGCAGCGACACCGCTCTCGCCGCCGCCATGACTGAGCAGATGACCCTTCGTG<br>121 GCACCCTCAAGGGCCACAACGGCTGGGTAACCCAGATCGCTACCACCCCGCAGTTCCCAG<br>181 ACATGATACTGTCGGCCTCGCGAGACAAGACCATCATCATGTGGAAGCTGACCAGGGATG | SEQ ID NO:188 |

| Gene Name | GenBank Homology | DNA SEQUENCE / DEDUCED AMINO ACID SEQUENCE | SEQUENCE IDENTIFIER: |
|---|---|---|---|
| | 1 (GNB2L1), mRNA. | 241 AGACCAACTACGGCATCCCACAGCGTGCTCTTCGAGGTCACTCCCACTTTGTTAGTGACG | |
| | | 301 TGGTGATCTCTTCAGATGGCCAGTTTGCCCTCTCAGGCTCCTGGGATGGAACGCTTCGCC | |
| | | 361 TCTGGGATCTCACAACGGGCACCACCACACGCCGATTTGTGGGCCATACCAAGGATGTGC | |
| | | 421 TGAGTGTGGCATTCTCCTCTGACAACCGGCAGATTGTCTCTGGCTCCCGAGATAAAACCA | |
| | | 481 TCAAGCTATGGAATACTCTGGGTGTATGCAAATACACTGTCCAGGATGAGAGCCACTCGG | |
| | | 541 AGTGGGTGTCTTGTGTCCGCTTCTCACCCAACAGTAGCAATCCCATCATTGTCTCCTGTG | |
| | | 601 GCTGGGACAAGCTAGTCAAGGTGTGGAATTTGGCAAACTGCAAGCTGAAGACCAATCACA | |
| | | 661 TCGGCCACACAGGCTACCTGAACACTGTCACTGTCTCTCCGGATGGATCCCTCTGTGCTT | |
| | | 721 CTGGAGGCAAGGATGGCCAGGCCATGCTTTGGGATTTAAATGAAGGCAAGCACCTTTACA | |
| | | 781 CACTAGATGGTGGGGACATCATCAACGCCTTGTGCTTCAGTCCCAATCGCTACTGGCTCT | |
| | | 841 GTGCTGCCACAGGCCCCAGCATCAAGATCTGGGACTTGGAGGGCAAGATCATTGTAGATG | |
| | | 901 AACTGAAGCAAGAAGTTATCAGTACCAGCAGCAAGGCAGAGCCACCCCAGTGCACTTCTC | |
| | | 961 TTGCCTGGTCTGCTGATGGCCAGACTCTGTTTGCTGGCTACACGGACAACCTGGTGAGAG | |
| | | 1021 TGTGGCAGGTGACCATCGGCACCCGCTAGAAATACATGGCAAGCTTTAGAAATAAAAAAA | |
| | | 1081 AAATGGCTTTTC | |
| | | 1 M T E Q M T L R G T L K G H N G W V T Q | SEQ ID NO:189 |
| | | 1 ATGACTGAGCAGATGACCCTTCGTGGCACCCTCAAGGGCCACAACGGCTGGGTAACCCAG | |
| | | 21 I A T T P Q F P D M I L S A S R D K T I | |
| | | 61 ATCGCTACCACCCCGCAGTTCCCAGACATGATACTGTCGGCCTCGCGAGACAAGACCATC | |
| | | 41 I M W K L T R D E T N Y G I P Q R A L R | |
| | | 121 ATCATGTGGAAGCTGACCAGGGATGAGACCAACTACGGCATCCCACAGCGTGCTCTTCGA | |
| | | 61 G H S H F V S D V V I S S D G Q F A L S | |
| | | 181 GGTCACTCCCACTTTGTTAGTGACGTGGTGATCTCTTCAGATGGCCAGTTTGCCCTCTCA | |
| | | 81 G S W D G T L R L W D L T T G T T T R R | |
| | | 241 GGCTCCTGGGATGGAACGCTTCGCCTCTGGGATCTCACAACGGGCACCACCACACGCCGA | |
| | | 101 F V G H T K D V L S V A F S S D N R Q I | |
| | | 301 TTTGTGGGCCATACCAAGGATGTGCTGAGTGTGGCATTCTCCTCTGACAACCGGCAGATT | |
| | | 121 V S G S R D K T I K L W N T L G V C K Y | |
| | | 361 GTCTCTGGCTCCCGAGATAAAACCATCAAGCTATGGAATACTCTGGGTGTATGCAAATAC | |
| | | 141 T V Q D E S H S E W V S C V R F S P N S | |
| | | 421 ACTGTCCAGGATGAGAGCCACTCGGAGTGGGTGTCTTGTGTCCGCTTCTCACCCAACAGT | |
| | | 161 S N P I I V S C G W D K L V K V W N L A | |
| | | 481 AGCAATCCCATCATTGTCTCCTGTGGCTGGGACAAGCTAGTCAAGGTGTGGAATTTGGCA | |

| Gene Name | GenBank Homology | DNA SEQUENCE / DEDUCED AMINO ACID SEQUENCE | SEQUENCE IDENTIFIER: |
|---|---|---|---|
| | | 181 N C K L K T N H I G H T G Y L N T V T V<br>541 AACTGCAAGCTGAAGACCAATCACATCGGCCACACAGGCTACCTGAACACTGTCACTGTC<br><br>201 S P D G S L C A S G G K D G Q A M L W D<br>601 TCTCCGGATGGATCCCTCTGTGCTTCTGGAGGCAAGGATGGCCAGGCCATGCTTTGGGAT<br><br>221 L N E G K H L Y T L D G G D I I N A L C<br>661 TTAAATGAAGGCAAGCACCTTTACACACTAGATGGTGGGGACATCATCAACGCCTTGTGC<br><br>241 F S P N R Y W L C A A T G P S I K I W D<br>721 TTCAGTCCCAATCGCTACTGGCTCTGTGCTGCCACAGGCCCCAGCATCAAGATCTGGGAC<br><br>261 L E G K I I V D E L K Q E V I S T S S K<br>781 TTGGAGGGCAAGATCATTGTAGATGAACTGAAGCAAGAAGTTATCAGTACCAGCAGCAAG<br><br>281 A E P P Q C T S L A W S A D G Q T L F A<br>841 GCAGAGCCACCCCAGTGCACTTCTCTTGCCTGGTCTGCTGATGGCCAGACTCTGTTTGCT<br><br>301 G Y T D N L V R V W Q V T I G T R -<br>901 GGCTACACGGACAACCTGGTGAGAGTGTGGCAGGTGACCATCGGCACCCGCTAG | |
| WBC107.bFSP | Homo sapiens nuclear receptor subfamily 1, group D, member 2, mRNA (cDNA clone IMAGE:3912370) - Partial. | 1 ggggctgca ggaagccgcc gcgccgccgc ttttgttgtc agggacccag cgaggagcgc<br>61 cgctcgccgg ccgccgccac cctctctcgc tgcagcctgc tgtgcgctgc acggcctggg<br>121 gcccgggagc cccgcccgct ctgcccatga gggggccccg cgaccaccgc tgcttccagc<br>181 ccggggcggc gcggcgctga ggcggcggcg gcggcgctgc cccctctgcg ggaagcgggc<br>241 ggccccggcc gcctccgcga gggcaccatg gaggtgaatg caggaggtgt gattgcctat<br>301 atcagttctt ccagctcagc ctcaagccat gcctcttgtc acagtgaggg ttctgagaat<br>361 agtttccagt cctcctcctc ttctgttcca tcttctccaa atagctctaa ttctgatacc<br>421 aatggtaatc ccaagaatgg tgatctcgcc aatattgaag gcatcttgaa gaatgatcga<br>481 atagattgtt ctatgaaaac aagcaaatcg agtgcacctg ggatgacaaa aagtcatagt<br>541 ggtgtgacaa aatttagtgg catggttcta ctgtgtaaag tctgtgggga tgtggcgtca<br>601 ggattccact atggagttca tgcttgcgaa ggctgtaagg gtttctttcg gagaagtatt<br>661 caacaaaaca tccagtacaa gaagtgcctg aagaatgaaa actgttctat aatgagaatg<br>721 aataggaaca gatgtcagca atgtcgcttc aaaaagtgtc tgtctgttgg aatgtcaaga<br>781 gatgctgttc ggtttggtcg tattcctaag cgtgaaaaac agaggatgct aattgaaatg<br>841 caaagtgcaa tgaagaccat gatgaacagc cagttcagtg gtcacttgca aaatgacaca<br>901 ttagtagaac atcatgaaca gacagccttg ccagcccagg aacagctgcg acccaagccc<br>961 caactggagc aagaaaacat caaaagctct tctcctccat cttctgattt tgcaaaggaa<br>1021 gaagtgattg gcatggtgac cagagctcac aaggatacct ttatgtataa tcaagagcag<br>1081 caagaaaact cagctgagag catgcagccc aagagaggag aacggattcg caagaacatg<br>1141 gagcaatata atttaaatca tgatcattgc ggcaatgggc ttagcagcca ttttccctgt<br>1201 agtgagagcc agcagcatct caatggacag ttcaaaggga ggaatataat gcattaccca | SEQ ID NO:190 |

| Gene Name | GenBank Homology | DNA SEQUENCE / DEDUCED AMINO ACID SEQUENCE | SEQUENCE IDENTIFIER: |
|---|---|---|---|
| | | 1261 aatggtcatg ccatttgtat tgcaaatgga cattgtatga acttctccaa tgcttatact | |
| | | 1321 caaagagtat gtgatagagt tccgatagat ggattttctc agaatgagaa caagaatagt | |
| | | 1381 tacctgtgca acactggagg aagaatgcat ctggtttgtc caatgagtaa gtctccatat | |
| | | 1441 gtggatcctc ataaatcagg acatgaaatc tgggaagaat tttcgatgag cttcactcca | |
| | | 1501 gcagtgaaag aagtggtgga atttgcaaag cgtattcctg ggttcagaga tctctctcag | |
| | | 1561 catgaccagg tcaacctttt aaaggctggg actttgagg ttttaatggt acggttcgca | |
| | | 1621 tcattatttg atgcaaagga acgtactgtc accttttaa gtggaaagaa atatagtgtg | |
| | | 1681 gatgatttac actcaatggg agcaggggat ctgctaaact ctatgtttga atttagtgag | |
| | | 1741 aagctaaatg ccctccaact tagtgatgaa gagatgagtt tgtttacagc tgttgtcctg | |
| | | 1801 gtatctgcag atcgatctgg aatagaaaac gtcaactctg tggaggcttt gcaggaaact | |
| | | 1861 ctcattcgtg cactaaggac cttaataatg aaaaaccatc caaatgaggc ctctattttt | |
| | | 1921 acaaaactgc ttctaaagtt gccagatctt cgatctttaa acaacatgca ctctgaggag | |
| | | 1981 ctcttggcct ttaaagttca cccttaaggc ctttgtttat ttaaacatga actgatggta | |
| | | 2041 actgtacatt ttgtgctaaa atgcatattt atatgtgtat accatatgtg gagatagaaa | |
| | | 2101 agacctttaa gacaataaaa gattgtaggc tatctctgta atcatgcaat agctgttcgg | |
| | | 2161 attgagaact cttcagccat gattagacgt tgactgcatc tccctgatag accaatcagc | |
| | | 2221 tgtgtcgcac ttaaactgga gaagttacac tgaagtataa tcacactgaa tgttatactt | |
| | | 2281 tttcatctgc caaaaccaaa aaccattttg atctccctgt ggttatcaat ataacgcaca | |
| | | 2341 atcacaagtg tatgaggact tagaaattaa tcctttgtgg taggagttct gttgaatgat | |
| | | 2401 ggaaatctta ttactaccac aagactattt gttctggtaa ttggagactt cgggatttag | |
| | | 2461 gagatctcca tgtctgtatt tactctacca ctgctaaagt gtgtggtcct gggtagttta | |
| | | 2521 cttgcttgcg gaaaatgaga attgatggtg tccccaatgc cccacctcac agagttacta | |
| | | 2581 aaaaatgtct gtaaagcata tttacctctt gggagatagg cactatgtaa ataaggtaaa | |
| | | 2641 atttctgtta ttacaattat tcataataat attcttttct tatttctaag cctttctggg | |
| | | 2701 aaatcatttc agtccacacc aaccatatta ttcagggttc ctgccatatg tgtggggtat | |
| | | 2761 cctactgata cacacgtatt caaagtttat gggtacaaca aagacatagt acatgtacat | |
| | | 2821 aatatgtatg tgaatatagt taaatatatt tcttcacaat attttaaact gtgaagaact | |
| | | 2881 ttatcataca ggaaacttaa aacaagaggt gtcaaaagac ccaaattagg tgcattttac | |
| | | 2941 ttgtttatga tggcataacc attgctttaa aatgtttaga cagtagaata ttgaatttat | |
| | | 3001 gctctatttt tgtttattta agcaacactt aatgtaaaag tgcaacaggc aattgaatcc | |
| | | 3061 aaatttcaac gacaaaaaaa aaaaacatgt attttagagt tcatctttgg caaaatcttt | |
| | | 3121 ggttcagggt actagttgtt taaaagttga ttcatattct taccttgtgc tgagaaaggt | |
| | | 3181 tgcattgctg ccccttatac acatgctgca gcttgatgtt aaagaatttt tattctttct | |
| | | 3241 gaagaactaa ttaatgttta aagcaactgt ttaatatgat ggcatgtgtg tgtgtgcgtg | |
| | | 3301 cgtgtgtatg ttctgagtcc acttcttttt tcctaaataa cactacaggg attttgtcat | |
| | | 3361 attagattta atttataatt tgaaaaatca tctagtgtgt gacctacagg cttagaaatg | |
| | | 3421 gtatagtcaa agacatttta tccacatttc taatagtgga cttgattaag tagataagat | |
| | | 3481 cagcatctgt ttatggtatt aggagaaata gccaaagttg aggattttat gtatgttttc | |
| | | 3541 ctgtttacct ggaaaatagc aattaattgg attttttggt aaagattgcc ttctgtataa | |
| | | 3601 tgtttggatt atataaaatt gcaaaaatga taacagcccg ctttactgta ctaagcctgt | |
| | | 3661 tactctcatg acgtgtgagc agaatgcctt attttgtaat cttgtttaac ttgttgctac | |
| | | 3721 tgggacttga tttactgtgg cactagttaa gtaagttaaa aaaaagttaa accctctcat | |
| | | 3781 tattaaagag gaaaggcgat ggtgatgtct gtagtacaat ataaaccata attgtgattt | |

| Gene Name | GenBank Homology | DNA SEQUENCE / DEDUCED AMINO ACID SEQUENCE | SEQUENCE IDENTIFIER: |
|---|---|---|---|
| | | 3841 accttaagta ggtataactc ttatgggata tacagtatag tttttgtgaa tctttacatg<br>3901 atagcattat ctttttataa tttttttcc taagataaac aaatgcatag ttttcttcta<br>3961 tgggtgatag aaacagcttt ttgaagtaat gaaaacctca aaagatcatg ttgattctta<br>4021 atttttgcct tttgcataag cctctttata acatgtatct ttaaaacaat taagtcttta<br>4081 ggaatgtgta accagaacta tgttagtatt gcttataaaa ctttagttag gttcaatata<br>4141 tacatatata catctctata taggtatata gatttgcatt ttgtcttgta aaattttatt<br>4201 tgaataaatt cttcctgtag gtaatgggaa acaaaattaa tagttcatat gtcactcata<br>4261 gcatttctat atttgaaagt agcccaatat aaaacttttg attctaaaat taaaccagca<br>4321 gcctattaca agcaaaaaaa aaaaaaaaa | |
| | | 1 M E V N A G G V I A Y I S S S S S A S S<br>1 ATGGAGGTGAATGCAGGAGGTGTGATTGCCTATATCAGTTCTTCCAGCTCAGCCTCAAGC<br><br>21 H A S C H S E G S E N S F Q S S S S S V<br>61 CATGCCTCTTGTCACAGTGAGGGTTCTGAGAATAGTTTCCAGTCCTCCTCCTCTTCTGTT<br><br>41 P S S P N S S N S D T N G N P K N G D L<br>121 CCATCTTCTCCAAATAGCTCTAATTCTGATACCAATGGTAATCCCAAGAATGGTGATCTC<br><br>61 A N I E G I L K N D R I D C S M K T S K<br>181 GCCAATATTGAAGGCATCTTGAAGAATGATCGAATAGATTGTTCTATGAAAACAAGCAAA<br><br>81 S S A P G M T K S H S G V T K F S G M V<br>241 TCGAGTGCACCTGGGATGACAAAAAGTCATAGTGGTGTGACAAAATTTAGTGGCATGGTT<br><br>101 L L C K V C G D V A S G F H Y G V H A C<br>301 CTACTGTGTAAAGTCTGTGGGGATGTGGCGTCAGGATTCCACTATGGAGTTCATGCTTGC<br><br>121 E G C K G F F R R S I Q Q N I Q Y K K C<br>361 GAAGGCTGTAAGGGTTTCTTTCGGAGAAGTATTCAACAAAACATCCAGTACAAGAAGTGC<br><br>141 L K N E N C S I M R M N R N R C Q Q C R<br>421 CTGAAGAATGAAAACTGTTCTATAATGAGAATGAATAGGAACAGATGTCAGCAATGTCGC<br><br>161 F K K C L S V G M S R D A V R F G R I P<br>481 TTCAAAAAGTGTCTGTCTGTTGGAATGTCAAGAGATGCTGTTCGGTTTGGTCGTATTCCT<br><br>181 K R E K Q R M L I E M Q S A M K T M M N<br>541 AAGCGTGAAAAACAGAGGATGCTAATTGAAATGCAAAGTGCAATGAAGACCATGATGAAC<br><br>201 S Q F S G H L Q N D T L V E H H E Q T A<br>601 AGCCAGTTCAGTGGTCACTTGCAAAATGACACATTAGTAGAACATCATGAACAGACAGCC | SEQ ID NO:191 |

| Gene Name | GenBank Homology | DNA SEQUENCE / DEDUCED AMINO ACID SEQUENCE | SEQUENCE IDENTIFIER: |
|---|---|---|---|
| | | 221 L P A Q E Q L R P K P Q L E Q E N I K S<br>661 TTGCCAGCCCAGGAACAGCTGCGACCCAAGCCCCAACTGGAGCAAGAAAACATCAAAAGC<br><br>241 S S P P S S D F A K E E V I G M V T R A<br>721 TCTTCTCCTCCATCTTCTGATTTTGCAAAGGAAGAAGTGATTGGCATGGTGACCAGAGCT<br><br>261 H K D T F M Y N Q E Q Q E N S A E S M Q<br>781 CACAAGGATACCTTTATGTATAATCAAGAGCAGCAAGAAAACTCAGCTGAGAGCATGCAG<br><br>281 P K R G E R I R K N M E Q Y N L N H D H<br>841 CCCAAGAGAGGAGAACGGATTCGCAAGAACATGGAGCAATATAATTTAAATCATGATCAT<br><br>301 C G N G L S S H F P C S E S Q Q H L N G<br>901 TGCGGCAATGGGCTTAGCAGCCATTTTCCCTGTAGTGAGAGCCAGCAGCATCTCAATGGA<br><br>321 Q F K G R N I M H Y P N G H A I C I A N<br>961 CAGTTCAAAGGGAGGAATATAATGCATTACCCAAATGGTCATGCCATTTGTATTGCAAAT<br><br>341 G H C M N F S N A Y T Q R V C D R V P I<br>1021 GGACATTGTATGAACTTCTCCAATGCTTATACTCAAAGAGTATGTGATAGAGTTCCGATA<br><br>361 D G F S Q N E N K N S Y L C N T G G R M<br>1081 GATGGATTTTCTCAGAATGAGAACAAGAATAGTTACCTGTGCAACACTGGAGGAAGAATG<br><br>381 H L V C P M S K S P Y V D P H K S G H E<br>1141 CATCTGGTTTGTCCAATGAGTAAGTCTCCATATGTGGATCCTCATAAATCAGGACATGAA<br><br>401 I W E E F S M S F T P A V K E V V E F A<br>1201 ATCTGGGAAGAATTTTCGATGAGCTTCACTCCAGCAGTGAAAGAAGTGGTGGAATTTGCA<br><br>421 K R I P G F R D L S Q H D Q V N L L K A<br>1261 AAGCGTATTCCTGGGTTCAGAGATCTCTCTCAGCATGACCAGGTCAACCTTTTAAAGGCT<br><br>441 G T F E V L M V R F A S L F D A K E R T<br>1321 GGGACTTTTGAGGTTTTAATGGTACGGTTCGCATCATTATTTGATGCAAAGGAACGTACT<br><br>461 V T F L S G K K Y S V D D L H S M G A G<br>1381 GTCACCTTTTTAAGTGGAAAGAAATATAGTGTGGATGATTTACACTCAATGGGAGCAGGG<br><br>481 D L L N S M F E F S E K L N A L Q L S D<br>1441 GATCTGCTAAACTCTATGTTTGAATTTAGTGAGAAGCTAAATGCCCTCCAACTTAGTGAT<br><br>501 E E M S L F T A V V L V S A D R S G I E | |

| Gene Name | GenBank Homology | DNA SEQUENCE / DEDUCED AMINO ACID SEQUENCE | SEQUENCE IDENTIFIER: |
|---|---|---|---|
| | | 1501 GAAGAGATGAGTTTGTTTACAGCTGTTGTCCTGGTATCTGCAGATCGATCTGGAATAGAA<br><br>521 N V N S V E A L Q E T L I R A L R T L I<br>1561 AACGTCAACTCTGTGGAGGCTTTGCAGGAAACTCTCATTCGTGCACTAAGGACCTTAATA<br><br>541 M K N H P N E A S I F T K L L L K L P D<br>1621 ATGAAAAACCATCCAAATGAGGCCTCTATTTTTACAAAACTGCTTCTAAAGTTGCCAGAT<br><br>561 L R S L N N M H S E E L L A F K V H P -<br>1681 CTTCGATCTTTAAACAACATGCACTCTGAGGAGCTCTTGGCCTTTAAAGTTCACCCTTAA | |
| WBC117.gRSP | No homology | 1 GACTTTATTTGAGGTAAGAAGGCAGAGCAGGATCAGAGAGTTCGAGAACAGGCATGGCAT<br>61 TATCTGGCTTGTATTTTTAAAGGGTGCCTCTGGTAGAATTATCTATTTTGTTTTCATGAA<br>121 GAGTCCTGTAATTATTGCCACGTTGCAGGTGAAGAATCTGAGGCTCAGAGAGGTTGAGTC<br>181 ATTGCTCAGGTTGGCACAGCTGGTGAGCGGCAGAGCTGGGTTTCCTAGTTACCTCCGTCT<br>241 GACTGCAGAATCCAAATCTTCGTCCACCATACTGGGCTTGCCCTGGTCCCTTCCTAATTC<br>301 CTTTAGCTTTGTAAAACATGTCTAAATTTGGAGTATCCAGAGAGTTGTGACTGTGTCGCC<br>361 TTCTATCACTGTTGACTTCAACCCCATCTCTTCCTGATGCATCCCCTAGGATGTGAGAGC<br>421 TCACTCTTATGGCTCCACCTGGGTTTCTCATGTTTGGTGCTACCATCCTTAACCTTTCAC<br>481 CAAGGGGCTCAAGATGTTTCCCATGATGATCCAGCCTTTCCGTTCTTCCCTTTGCTGGCA<br>541 ATTATACGTGCTTACCTTTCTCGGACAGGACCCACTTTCCGGGTGACTGCATGCAAACTC<br>601 TCCCTTTCTCTTTTTAGATGTTAATAGCAGAGTTATCAAGAACGAATGCTCTGAAGAGAA<br>661 CATGGCCTTTAGCTTGAAAGCCAAAGGGGATAAGATGACGATTTCATGCTCCACAATTAA<br>721 AGAATCTGGATCCACAGGTACAGACCCACTCCGGAGAATCAGGGTGGGTAGTTATGGTAC<br>781 ACTCAGTCAACAAACACTTATCGATCACCTCTTAGGTGCCAAGACCTATGCCAGGTGGTG<br>841 GGGAAATCAATGATGGGAGAGGCAAAAATGG | SEQ ID NO:192 |
| | | 1 GTTATGGTACACTCAGCCACCAACACTTATCGGATCCCCTTTTTAGGGGCCAAGACCTAT<br>61 GCCAGGTGGGGGAAATCAATGATGGAAGAGGCAGAAATGGCCTCAGGAAAGTTTCCCAC<br>121 CTGCTAGGAAAGGCAGACATTGAAAAAATAATCTTGCAGTACCGGAGTATTGAAAGCTTA<br>181 CCTATCTAATACAGCAGCCACTGGCCACTTTTGTCATTCAGCACTTGAAATGTGGCCAGG<br>241 TTGTGAATTTAAAATACCTACCAGATTTCAAAGACTTGGTCCAAAAAAAAAAATGGAGGA<br>301 TGTAAAATAGCTCACTCATAATTTTTTCTATTGATCAAGTGTTGAAACGATACTATTTTG<br>361 CATGTATTGAATTAATATATTATTAAAATGAATTTTACCTGTTTTCTACTCTAAAATGTG<br>421 GCTATTAGAAAATTTAAAATTGCGTATGTGGCTCATAGTATATTTCTAGCAGATAGTGCT<br>481 GATCTGTATCAGGGGCTGGGAAACTTTTCCTGTAGAGGGCCGCTTTGTAAATATTTTAGG<br>541 CTTTGAGGGCAACACAATTTCTGTCGCAACCATTCGACCCTGCCGTCATCATGCAAAAGC<br>601 ATTCATCAGCAGCGTGTAAATGAATGGGTGTGTCTGTGTTGCAATAGAGCTTTTATTTAA<br>661 AAAAAAAAAAAA | SEQ ID NO:193 |
| WBC21.bFSP | No Homology | 1 TTCGCATACCACTTGTGGACATTATAAATTGCTGATTTCCAGTCTCCATTCCCAGAGATT | SEQ ID NO:194 |

| Gene Name | GenBank Homology | DNA SEQUENCE / DEDUCED AMINO ACID SEQUENCE | SEQUENCE IDENTIFIER: |
|---|---|---|---|
| | | 61 CTGATTCTGTAGGGGTGACCTGGGGCCCAGGATCAAATCTTTCTAACCAGTTCCACAGGT<br>121 GATTCTGATGTACGTATACAGAGTTTGAGAACTATCACTTTAGAGCACGGGTTGGAAAAT<br>181 TACAACCCACAGATCAAATCCGGCCTGCTGCTTTTTTGGTGTGATAAAGTTTTATTGGAA<br>241 TGCAGCTACACTTATTTGTTTGCATATTGTCTATGTCTGTTTCAGCACTGCAACAGCAGA<br>301 GTTGAGTAGTTACAGCAGACACCATACGGCCATAAAGCTGAAGATATTTACTATCTGGAC<br>361 CTTTACAGAAAAAGTTTGCAAACTCTTGCTCTAAAGCTAAATGAACATTTCTCCTTAAAT<br>421 AATTTAAACTCTGTTGCAATCCTGATCATTCCTGCCAAACACAATTGTAGATAGAATAAC<br>481 TGTTTTTTCAGAATTTAGTCTGAGAATGTTAGTTCTTGTATAGTCCACACTTTCTCTAGG<br>541 ACCTTTGGCAAATCAAAGAATGCTGTGACTCCTGAGTGGTCAATAGAAGATATACATTTC<br>601 CTTGGCCTTACCACAAGTGATTGAAAGTGAAGCATATGGTTCTATAGTCTGCGTATCTCA<br>661 TAATAGTCCTTAGTAATCTCATGCATTAGGAGGCAATCATCAAGTAGAGACTCTTTCATC<br>721 CATAAGCCCCCAAACCAAACTACTTACCTGATTGTCAAGAAGAAAAGCATTGCTGTATCT<br>781 ATTTTTGAAGAGAGGCATTAGTGGCTTTGGCTAAAGTTTAACAAAGCTAACCACATCCCC<br>841 TCCCTTCAGCCAGCCAGCCATCTGATTCCCCTAAATGCGTAGAAATAAAGATGTAGTGCT<br>901 GCAAATGTATTTTGACTTTTTCAAGTTATGCTGTTTCCTCTATTCTTGGATAAATCATAG<br>961 TAGGTTAATAATACAGAAATGAGATTGCATATTTCTCTTTAGTAGAAATTAGCAAAATTA<br>1021 GCTTTTTTTGCTACAACTTAAGACACTTGATGTATTCCTCTGTGTTTTATGTTACTGTAA<br>1081 ACTCAACATACGGTGAGTTTTGTGGTATTTGACCTGTTAGGCAAAATCACTATTTTCACT<br>1141 TTGACTGACATTTATAATGTTCTGTTCATGAAATAAAATTCTTCTGTCTAAAAAAAAAAA<br>1201 AAAAAAA | |
| WBC215.gRSP | Homo sapiens CDC14 cell division cycle 14 homolog A (S. cerevisiae) (CDC14A), transcript variant 1, mRNA | 1 cgaagaggat ccggagcagc tgctgccagc ccgcgggcac tgaagtcctc ccggctgccg<br>61 ctcgagtagc cacgggcgcg atcgggacca gaagtctcct cctccatgat cactttggaa<br>121 gccgggggggg aagactttgc cctgccctga gagctggtct gcgtttccca ggcgcggcgg<br>181 cggcggagca gcagctgcag cagccgagtc caaataggag cggccacagc caggggcgtg<br>241 tgcgccccgc gcggagcgag ctcgggttcc cctcggaatg tccccggggc gcccggcgcg<br>301 ctgaccccga agccgcctcc gccttcggcg cctgctgcct ccctcggcca ggcttgttgt<br>361 tcgggactgt gagcttcctg gctcctgggc agtggggaag cccccggggg cgagtgacct<br>421 cagctggcca cgacccagcc ctcccccgtg cgtatctcgc ttaagatggc agcggagtca<br>481 ggggaactaa tcggggcttg tgagttcatg aaagatcggt tatattttgc tactttaagg<br>541 aatagaccaa aaagcacagt aaatacccac tatttctcca tcgatgagga gctggtctat<br>601 gaaaatttct atgcagattt tggaccgctg aacttggcaa tggtgtacag atattgctgc<br>661 aaactaaaca agaaactaaa atcatacagt ttgtcaagaa agaaaatagt gcactacacc<br>721 tgttttgacc aacggaaaag agcaaatgca gcatttttga taggtgccta tgcagtaatc<br>781 tatttaaaga agacaccaga agaagcctac agagcactcc tgtctggctc aaaccccccc<br>841 tatcttccat tcagggatgc ttcctttgga aattgcactt acaatctcac cattctcgac<br>901 tgtttgcagg gaatcagaaa gggattacaa catggatttt ttgactttga gacatttgat<br>961 gtggatgaat atgaacatta tgagcgagtt gaaaatggtg acttcaactg gattgttcca<br>1021 ggaaaatttt tagcatttag tggaccacat cctaaaagca aaattgagaa tggttatcct<br>1081 cttcacgccc ctgaagccta ctttccttat ttcaaaaagc ataatgtgac tgcagttgtg<br>1141 aggctaaaca aaaagattta tgaggcaaag cgcttcacag acgctggctt cgagcactat<br>1201 gacctcttct tcatagatgg cagcacaccc agtgacaaca tcgtgcgaag gttcctgaac | SEQ ID NO:195 |

| Gene Name | GenBank Homology | DNA SEQUENCE / DEDUCED AMINO ACID SEQUENCE | SEQUENCE IDENTIFIER: |
|---|---|---|---|
| | | 1261 atctgtgaga acaccgaagg ggccatcgcc gttcactgca aagctggtct tggaagaaca<br>1321 gggacattga tagcctgtta tgtaatgaaa cactacaggt ttacacatgc tgaaataatt<br>1381 gcttggatta gaatatgccg gccaggctct attataggac cccagcagca cttcctggaa<br>1441 gaaaaacaag catcgttgtg ggtccaagga gacattttcc gatccaaact gaaaaatcga<br>1501 ccatccagtg aaggaagtat taataaaatt ctttctggcc tagatgatat gtctattggt<br>1561 ggaaatcttt caaaaacaca aaacatggaa cgatttggag aggataactt agaagatgat<br>1621 gatgtggaaa tgaaaaatgg tataacccag ggagacaaac tacgtgcctt aaaaagtcag<br>1681 agacagccac gtacctcacc atcctgtgca tttaggtcag atgatacaaa aggacatcca<br>1741 agagcagtgt cccagccttt cagattaagt tcatccctgc aaggatctgc agttactttg<br>1801 aagacatcaa aaatggcact gtccccttca gcaacggcca agaggatcaa cagaacttct<br>1861 ttgtcttcgg gtgccactgt aagaagcttt tccataaact cccggctagc cagttctcta<br>1921 gggaacttga atgctgcaac agatgatcca gagaacaaaa agacctcctc atcctctaag<br>1981 gcaggcttca cagccagccc gtttaccaac ctcttgaatg gcagctccca gccaactacc<br>2041 agaaattacc ctgagctcaa caataatcag tacaacagaa gcagcaacag caacgggggc<br>2101 aacctgaaca gccccccagg cccccacagc gccaagacag aggagcacac caccatcctc<br>2161 cgaccctcct acaccgggct ttcttcttct tcagcgagat tcctgagccg ttctatccct<br>2221 tcccttcagt ctgaatatgt tcattactaa ggccttgcca ctccagtgaa agctgttctt<br>2281 ctcttagaca caatttcttc atctggacga gcagtggaga gggaaagcaa cttcttgctg<br>2341 gaagaatatc tctgccttct taccttaaat taaaaagagc actaagataa caccttcaag<br>2401 agacttgaaa acagaaaact ggttaatgac tactataaat gcactgaaac tatgttatgg<br>2461 agatttccat acttttaaag acagttttaa tgttgaattt ggtattttga agggttattt<br>2521 ttaatgtatt ttggtaatac atttattatt atatttacat gtacagtgtt acattatata<br>2581 tgtattgtga actttaaaag actattttga taaatttata aatatataaa attatgtaaa<br>2641 aactacacta tattttgatt tagattttcc tgctgtttgc taccaaaaat ttgtatttta<br>2701 aatctgttta gttttagtat ggttttgtct ctaatgaata aataattcct tcttattaag<br>2761 aagaagtaag ggagaaagtt tttagaaagt gatttttatg ctcgcactat aaatatggca<br>2821 ggtcagttca ttcttttggg aagtcagttt agttacactg agtttatcca agtttatctc<br>2881 taccaagagt ataatggcat gggatggctt atttaggaca attccctttc ctattgtttt<br>2941 tgttgctgag ccaatttgag ttagttttgc atcctggggg gctttaaaat acagcatgca<br>3001 gtgaaagatc agaattcact gaatatttct tctgagagca tggtttcatg gtttttctct<br>3061 atgaaatgac tcaatattcc aaatgttttt ttttccttcc tcctttcaaa agagttctta<br>3121 acccaattag gatatcctgc tttgggtatg aggttgttgt tgcctgtaat cacacatggt<br>3181 ttgacatcag ttttaaatca atggagagaa aaaactgaaa aagatgctgc taagtagttc<br>3241 tctgtattaa aggagatatt tttaaaacag ggtacaaccc cctgctgcac acgctagcat<br>3301 atctggaacc tactatgaaa atgaaaggac ccttataggt actcacagcc ctttcatgta<br>3361 agtatgatct gatatttagg tcttcagaag cctgtaggtt tcatttctat gaggaatcga<br>3421 ggagcgttac atcctgatat ccttccaggc tgcttaagaa tggactgctt cgacactgaa<br>3481 agtgctagtt aaatggattc atatgaagtg ctttactccc aaccattgag ttatttataa<br>3541 tgtatttatt aggggagggt accttgagtc tattatatat gcttcatcaa aacatcttgt<br>3601 tcatgtttta tgtttttaaa aaaggcattt gaatgaatgt ttgactcagg tttgttaaat<br>3661 taacttcagt aactgcagta ccaaaaatta cactcaactg atgaaaaaaa cgaattgtat<br>3721 gatttaggaa tcaaaaacta aaataagtgg aattatgtat cttttctaaa gttaaaaaag<br>3781 taaaatattt tattatgagt tattataaaa attggttaat tgtataggaa gatgacagta | |

| Gene Name | GenBank Homology | DNA SEQUENCE / DEDUCED AMINO ACID SEQUENCE | SEQUENCE IDENTIFIER: |
|---|---|---|---|
| | | 3841 tttttttcaa gttatcataa aaagtaattc agatgacatt tgagaagtag gggaaaggga<br>3901 atcatgttga cagttttagt tctgtgaaca ctaatttgtg tgaagctatt aaaatgattg<br>3961 taaagttgac tactgtaaat ttcccataat tatgtgtgta tatgtgtcat atgtatgtac<br>4021 atgtatatgt ctaaaaatta ctttacacat gtgcctacat agacacacca agaagtggat<br>4081 gtatataata tagaaagtat atagcaaagt aattttactc tgataataaa aattgtttga<br>4141 catgtatttt gttatgaata gtttatcttc caaaagatat tttgctctat tttaaagtgt<br>4201 agaagaatac actgctaata aataataaaa gttttattca atttaaaaaa aaaaaaaaaa<br>4261 aa | |
| | | 1 M A A E S G E L I G A C E F M K D R L Y<br>1 ATGGCAGCGGAGTCAGGGGAACTAATCGGGGCTTGTGAGTTCATGAAAGATCGGTTATAT<br><br>21 F A T L R N R P K S T V N T H Y F S I D<br>61 TTTGCTACTTTAAGGAATAGACCAAAAAGCACAGTAAATACCCACTATTTCTCCATCGAT<br><br>41 E E L V Y E N F Y A D F G P L N L A M V<br>121 GAGGAGCTGGTCTATGAAAATTTCTATGCAGATTTTGGACCGCTGAACTTGGCAATGGTG<br><br>61 Y R Y C C K L N K K L K S Y S L S R K K<br>181 TACAGATATTGCTGCAAACTAAACAAGAAACTAAAATCATACAGTTTGTCAAGAAAGAAA<br><br>81 I V H Y T C F D Q R K R A N A A F L I G<br>241 ATAGTGCACTACACCTGTTTTGACCAACGGAAAAGAGCAAATGCAGCATTTTTGATAGGT<br><br>101 A Y A V I Y L K K T P E E A Y R A L L S<br>301 GCCTATGCAGTAATCTATTTAAAGAAGACACCAGAAGAAGCCTACAGAGCACTCCTGTCT<br><br>121 G S N P P Y L P F R D A S F G N C T Y N<br>361 GGCTCAAACCCCCCCTATCTTCCATTCAGGGATGCTTCCTTTGGAAATTGCACTTACAAT<br><br>141 L T I L D C L Q G I R K G L Q H G F F D<br>421 CTCACCATTCTCGACTGTTTGCAGGGAATCAGAAAGGGATTACAACATGGATTTTTTGAC<br><br>161 F E T F D V D E Y E H Y E R V E N G D F<br>481 TTTGAGACATTTGATGTGGATGAATATGAACATTATGAGCGAGTTGAAAATGGTGACTTC<br><br>181 N W I V P G K F L A F S G P H P K S K I<br>541 AACTGGATTGTTCCAGGAAAATTTTTAGCATTTAGTGGACCACATCCTAAAAGCAAAATT<br><br>201 E N G Y P L H A P E A Y F P Y F K K H N<br>601 GAGAATGGTTATCCTCTTCACGCCCCTGAAGCCTACTTTCCTTATTTCAAAAAGCATAAT<br><br>221 V T A V V R L N K K I Y E A K R F T D A | SEQ ID NO:196 |

| Gene Name | GenBank Homology | DNA SEQUENCE / DEDUCED AMINO ACID SEQUENCE | SEQUENCE IDENTIFIER: |
|---|---|---|---|
| | | 661 GTGACTGCAGTTGTGAGGCTAAACAAAAAGATTTATGAGGCAAAGCGCTTCACAGACGCT<br><br>241 G F E H Y D L F F I D G S T P S D N I V<br>721 GGCTTCGAGCACTATGACCTCTTCTTCATAGATGGCAGCACACCCAGTGACAACATCGTG<br><br>261 R R F L N I C E N T E G A I A V H C K A<br>781 CGAAGGTTCCTGAACATCTGTGAGAACACCGAAGGGGCCATCGCCGTTCACTGCAAAGCT<br><br>281 G L G R T G T L I A C Y V M K H Y R F T<br>841 GGTCTTGGAAGAACAGGGACATTGATAGCCTGTTATGTAATGAAACACTACAGGTTTACA<br><br>301 H A E I I A W I R I C R P G S I I G P Q<br>901 CATGCTGAAATAATTGCTTGGATTAGAATATGCCGGCCAGGCTCTATTATAGGACCCCAG<br><br>321 Q H F L E E K Q A S L W V Q G D I F R S<br>961 CAGCACTTCCTGGAAGAAAAACAAGCATCGTTGTGGGTCCAAGGAGACATTTTCCGATCC<br><br>341 K L K N R P S S E G S I N K I L S G L D<br>1021 AAACTGAAAAATCGACCATCCAGTGAAGGAAGTATTAATAAAATTCTTTCTGGCCTAGAT<br><br>361 D M S I G G N L S K T Q N M E R F G E D<br>1081 GATATGTCTATTGGTGGAAATCTTTCAAAAACACAAAACATGGAACGATTTGGAGAGGAT<br><br>381 N L E D D D V E M K N G I T Q G D K L R<br>1141 AACTTAGAAGATGATGATGTGGAAATGAAAAATGGTATAACCCAGGGAGACAAACTACGT<br><br>401 A L K S Q R Q P R T S P S C A F R S D D<br>1201 GCCTTAAAAAGTCAGAGACAGCCACGTACCTCACCATCCTGTGCATTTAGGTCAGATGAT<br><br>421 T K G H P R A V S Q P F R L S S S L Q G<br>1261 ACAAAAGGACATCCAAGAGCAGTGTCCCAGCCTTTCAGATTAAGTTCATCCCTGCAAGGA<br><br>441 S A V T L K T S K M A L S P S A T A K R<br>1321 TCTGCAGTTACTTTGAAGACATCAAAAATGGCACTGTCCCCTTCAGCAACGGCCAAGAGG<br><br>461 I N R T S L S S G A T V R S F S I N S R<br>1381 ATCAACAGAACTTCTTTGTCTTCGGGTGCCACTGTAAGAAGCTTTTCCATAAACTCCCGG<br><br>481 L A S S L G N L N A A T D D P E N K K T<br>1441 CTAGCCAGTTCTCTAGGGAACTTGAATGCTGCAACAGATGATCCAGAGAACAAAAAGACC<br><br>501 S S S S K A G F T A S P F T N L L N G S<br>1501 TCCTCATCCTCTAAGGCAGGCTTCACAGCCAGCCCGTTTACCAACCTCTTGAATGGCAGC | |

| Gene Name | GenBank Homology | DNA SEQUENCE / DEDUCED AMINO ACID SEQUENCE | SEQUENCE IDENTIFIER: |
|---|---|---|---|
| | | 521 S Q P T T R N Y P E L N N N Q Y N R S S<br>1561 TCCCAGCCAACTACCAGAAATTACCCTGAGCTCAACAATAATCAGTACAACAGAAGCAGC<br><br>541 N S N G G N L N S P P G P H S A K T E E<br>1621 AACAGCAACGGGGGCAACCTGAACAGCCCCCCAGGCCCCCACAGCGCCAAGACAGAGGAG<br><br>561 H T T I L R P S Y T G L S S S S A R F L<br>1681 CACACCACCATCCTCCGACCCTCCTACACCGGGCTTTCTTCTTCTTCAGCGAGATTCCTG<br><br>581 S R S I P S L Q S E Y V H Y -<br>1741 AGCCGTTCTATCCCTTCCCTTCAGTCTGAATATGTTCATTACTAA | |
| WBC309.bFSP | Homo sapiens hypothetical protein MGC5576, mRNA | 1 GACATGACACAAGTGGCACATCCCGGACATGGGGTCCCAGCATTCCGCCTCTGCTCGCCC<br>61 CTCTTCCTGCAGGCGAAAGAAAGATGACAGGGAGGATTTTCTGGCTGAACGGGAGCAGGA<br>121 AGAAGCCATCGCTCAGTTTCCGTATGTGGAGTTCACCGGTCGAGATAGCATCACCTGTCT<br>181 CACGTGCCAAGGGACCGGCTACATTCCAACAGAGCAAGTAAATGAATTGGTGGCTTTGAT<br>241 CCCACACAGTGATCAGAGGTTGCGCCCTCAGAGAACTAAGCAATATGTACTCCTGTCTGT<br>301 CTTGCTGTGTCTCCTGGCATCTGGGTTGGTGGTTTTCTTCCTCTTTCCACATTCAGTCCT<br>361 TGTGGATGACGATGGCATCAAAGTGGTGAAAGTCACGTTTAATAAGCAGGACTCCCTGGT<br>421 GATCCTCGCCATCACGGCCACCCTGAAAATCAGGAACTCCAACTTCTACTCTGTGGCCGT<br>481 GACCAGCCTGTCCACCCAGGTTCAGTACATGAACACCGTGGTTGGGTCATATGTGACAGC<br>541 AAATGTCTCCCTTATTCCACCTCGGAGCGAGCACCTGGTGAATTTTACTGTGAAGGCTGA<br>601 GATGGGAGGACCGTATTCCTATGTGTACTTCTTCTGCACATTGCCGGATATCCTGATTCA<br>661 CAACATAGTGATCTTCATGCGAACTTCAGTGAAGATTTCATACATTGGCCTCATGACCCA<br>721 GAGCTCCTTGGAGACACATCACTATGTGGATTGTGGAGGAAATTCCACAGCTATTTAACA<br>781 ACTGCTATTGGTTCTTCCACACAGCGCCTGTAGAAGAGAGCACAGCATATGTTCCCAAGG<br>841 CCTGAGTTCTGGACCTACCCCCACGTGGTGTAAGCAGAGGAGGAATTGGTTCACTTAACT<br>901 CCCAGCAAACATCCTCCTACCACTTGGGAGGAAAACTCCTCTCTGTAGCACCATTTCTGT<br>961 TTCTGAGAACCAGCAGAATCACTGCCCAGTGCCTGGCCATATGCCCAGCAAATTAATAGG<br>1021 CACTCCAAGAAGGTTTGAAGAATGTAATTCAGATCTTTTCAGCTGTTCTTGGGCCGTTAT<br>1081 AAATTGAAATCATACTGTGGTTCTAGGTTATCAGACCATGGAGTGATGTGGAGCTAGGAT<br>1141 TGTGAGTGACCTGCAGGCCATTATCAGTGCCTCATCTGTGCAGAAGTGGCAGCAGAGAGG<br>1201 GACCATCCAAATACCTAAGAGAAAACAGACCTAGTCAGGATATGAATTTGTTTCAGCTGT<br>1261 TCCCAAAGGCCTGGGAGCTTTTTGAAAAGAAAGAAAAAAGTGTGTTGGCTTTTTTTTTTT<br>1321 TTTAGAGAGTTACACTTGTTTTTACCAAGAACCTTTATGGGCTTTGATTTACCCTTCATC<br>1381 CATTGGCTGGAACATGGATTGGGGATTTGGTAGAAAAATAAACCCTGCTTTTGATTCAAA<br>1441 AAAAAAAAAAAAAAAA | SEQ ID NO:197 |
| | | 1 M G S Q H S A S A R P S S C R R K K D D<br>1 ATGGGGTCCCAGCATTCCGCCTCTGCTCGCCCCTCTTCCTGCAGGCGAAAGAAAGATGAC | SEQ ID NO:198 |

| Gene Name | GenBank Homology | DNA SEQUENCE / DEDUCED AMINO ACID SEQUENCE | SEQUENCE IDENTIFIER: |
|---|---|---|---|
| | | 21 R E D F L A E R E Q E E A I A Q F P Y V<br>61 AGGGAGGATTTTCTGGCTGAACGGGAGCAGGAAGAAGCCATCGCTCAGTTTCCGTATGTG<br><br>41 E F T G R D S I T C L T C Q G T G Y I P<br>121 GAGTTCACCGGTCGAGATAGCATCACCTGTCTCACGTGCCAAGGGACCGGCTACATTCCA<br><br>61 T E Q V N E L V A L I P H S D Q R L R P<br>181 ACAGAGCAAGTAAATGAATTGGTGGCTTTGATCCCACACAGTGATCAGAGGTTGCGCCCT<br><br>81 Q R T K Q Y V L L S V L L C L L A S G L<br>241 CAGAGAACTAAGCAATATGTACTCCTGTCTGTCTTGCTGTGTCTCCTGGCATCTGGGTTG<br><br>101 V V F F L F P H S V L V D D D G I K V V<br>301 GTGGTTTTCTTCCTCTTTCCACATTCAGTCCTTGTGGATGACGATGGCATCAAAGTGGTG<br><br>121 K V T F N K Q D S L V I L A I T A T L K<br>361 AAAGTCACGTTTAATAAGCAGGACTCCCTGGTGATCCTCGCCATCACGGCCACCCTGAAA<br><br>141 I R N S N F Y S V A V T S L S T Q V Q Y<br>421 ATCAGGAACTCCAACTTCTACTCTGTGGCCGTGACCAGCCTGTCCACCCAGGTTCAGTAC<br><br>161 M N T V V G S Y V T A N V S L I P P R S<br>481 ATGAACACCGTGGTTGGGTCATATGTGACAGCAAATGTCTCCCTTATTCCACCTCGGAGC<br><br>181 E H L V N F T V K A E M G G P Y S Y V Y<br>541 GAGCACCTGGTGAATTTTACTGTGAAGGCTGAGATGGGAGGACCGTATTCCTATGTGTAC<br><br>201 F F C T L P D I L I H N I V I F M R T S<br>601 TTCTTCTGCACATTGCCGGATATCCTGATTCACAACATAGTGATCTTCATGCGAACTTCA<br><br>221 V K I S Y I G L M T Q S S L E T H H Y V<br>661 GTGAAGATTTCATACATTGGCCTCATGACCCAGAGCTCCTTGGAGACACATCACTATGTG<br><br>241 D C G G N S T A I -<br>721 GATTGTGGAGGAAATTCCACAGCTATTTAA | |
| WBC310.bFSP | Homo sapiens lymphoid enhancer-binding factor 1, mRNA (cDNA clone MGC:60112 IMAGE:6054813). | 1 CCCACGCGTCCGCCCACGCGTCCGCCCCTCTGTCTTTCCTGCTGTTGCGCGGGTGCTCCC<br>61 ACAGCGGAGCGGAGATTACAGAGCCGCCGGGATGCCCCAACTCTCCGGAGGAGGTGGCGG<br>121 CGGCGGGGGGGACCCGGAACTCTGCGCCACGGACGAGATGATCCCCTTCAAGGACGAGGG<br>181 CGATCCTCAGAAGGAAAAGATCTTCGCCGAGATCAGTCATCCCGAAGAGGAAGGCGATTT<br>241 AGCTGACATCAAGTCTTCCTTGGTGAACGAGTCTGAAATCATCCCGGCCAGCAACGGACA | SEQ ID NO:199 |

| Gene Name | GenBank Homology | DNA SEQUENCE / DEDUCED AMINO ACID SEQUENCE | SEQUENCE IDENTIFIER: |
|---|---|---|---|
| | | 301 CGAGGTGGCCAGACAAGCACAAACCTCTCAGGAGCCCTACCACGACAAGGCCAGAGAACA<br>361 CCCCGATGACGGAAAGCATCCAGATGGAGGCCTCTACAACAAGGGACCCTCCTACTCGAG<br>421 TTATTCCGGGTACATAATGATGCCAAATATGAATAACGACCCATACATGTCAAATGGATC<br>481 TCTTTCTCCACCCATCCCGAGAACATCAAATAAAGTGCCCGTGGTGCAGCCATCCCATGC<br>541 GGTCCATCCTCTCACCCCCCTCATCACTTACAGTGACGAGCACTTTTCTCCAGGATCACA<br>601 CCCGTCACACATCCCATCAGATGTCAACTCCAAACAAGGCATGTCCAGACATCCTCCAGC<br>661 TCCTGATATCCCTACTTTTTATCCCTTGTCTCCGGGTGGTGTTGGACAGATCACCCCACC<br>721 TCTTGGCTGGCAAGGTCAGCCTGTATATCCCATCACGGGTGGATTCAGGCAACCCTACCC<br>781 ATCCTCACTGTCAGTCGACACTTCCATGTCCAGGTTTTCCCATCATATGATTCCCGGTCC<br>841 TCCTGGTCCCCACACAACTGGCATCCCTCATCCAGCTATTGTAACACCTCAGGTCAAACA<br>901 GGAACATCCCCACACTGACAGTGACCTAATGCACGTGAAGCCTCAGCATGAACAGAGAAA<br>961 GGAGCAGGAGCCAAAAAGACCTCACATTAAGAAGCCTCTGAATGCTTTTATGTTATACAT<br>1021 GAAAGAAATGAGAGCGAATGTCGTTGCTGAGTGTACTCTAAAAGAAAGTGCAGCTATCAA<br>1081 CCAGATTCTTGGCAGAAGGTGGCATGCCCTCTCCCGTGAAGAGCAGGCTAAATATTATGA<br>1141 ATTAGCACGGAAAGAAAGACAGCTACATATGCAGCTTTATCCAGGCTGGTCTGCAAGAGA<br>1201 CAATTATGGTAAGAAAAAGAAGAGGAAGAGAGAGAAACTACAGGAATCTGCATCAGGTAC<br>1261 AGGTCCAAGAATGACAGCTGCCTACATCTGAAACATGGTGGAAAACGAAGCTCATTCCCG<br>1321 ACGTGCAAAGCCAAGGCAGCGACCCCGGGGCCTCTACTGGAGATGGAAGCTTGTTGAAAA<br>1381 CCCAGACTGTGTCCAGGGCTTGCCCAGTCGACCCCAAAGGAGAATTGACACCAACTTCAC<br>1441 CCTGAGGTCACTGCTAGAGACGCTGATCCACAAAGACAATCACTGCCAACCCCCCTTCAT<br>1501 CTACTGCAAGAGCCAAGTTCCAAAATAAAGCGTAAAGGATTTTTTAGAAGAAAAGTAAAA<br>1561 AGCACATGAGAATGCTGGCAGGCCCTGGGTCAGCTGAGCAGCTTTCCCCCTCAGCTCCGC<br>1621 CGTGCACTTCCCAGAGCATCTCGCGTCCATACCTGAACCCTTGGGCGAGGACAGTAACTT<br>1681 GGCTGCATTTGCCTGTCATGCGCAACTGGAGCCAGCAACCAGCACATCCATCAGCACCCC<br>1741 AGTGGAGGAGTTCATGGAAGAGTTCCCTCTTTGTTTCTGCTTCATTTTTCTTTCTTTTCT<br>1801 TTTCTCCTAAAGCTTTTCTTTAACAGTGCAAAAGGATCGTTTTTTTTTGCTTTTTTAAAC<br>1861 TTGAATTTTTTTAATTTACACTTTTTAGTTTTAACTTTCTCTGTTGTATATTTTGCTAGC<br>1921 TATGAGCTTTGAAGTAAAATTGAAAGTTCTGGAAAAGTTTGAAATAATGACATAAAAAGA<br>1981 AGCCTTCTTTTTCTGAGACAGCTTGTCTGGTAAGTGGCTTCTCTGTGAATTGCCTGTAAC<br>2041 ACATAGTGGCTTCTCCGCCCTTGTAAGGTGTTCAGTAGAGCTAAATAAATGTAATAGCCA<br>2101 AACCCACTCTGTTGGTAGCAATTGGCAGCCCTATTTCAGTTTATTTTTTCTTCTGTTTTC<br>2161 TTCTTTTCTTTTTTTAAACAGTAAACCTTAACAGATGCGTTCAGCAGACTGGTTTGCAGT<br>2221 GAATTTTCATTTCCTTCCTTCTTGCCCCTTGTTGTAAAAAGCCCAGCACTTGAATTGTTC<br>2281 TTACTTTAAATGTTCTGTATTTGTATCTGTTTTTATTAACCGATTAGTGGATTTTATGC<br>2341 CAGTTGTTAAAATGAGCAATGATGTACCCATTTTTTTTAAATAGCAAGGCACAGCCTTCG<br>2401 CCCCAAACTGTCATCTAACGTTTGTCATTCCAGTTTGAGTTAATGTGCTGAGCATTTTTT<br>2461 AAAAGAAGCTTTGTAATAAAACATTTTTAAAAATTGTCAAAAAAAAAAAAAAAAAA | |
| | | 1 M P Q L S G G G G G G G G D P E L C A T<br>1 ATGCCCCAACTCTCCGGAGGAGGTGGCGGCGGCGGGGGGGACCCGGAACTCTGCGCCACG<br><br>21 D E M I P F K D E G D P Q K E K I F A E<br>61 GACGAGATGATCCCCTTCAAGGACGAGGGCGATCCTCAGAAGGAAAAGATCTTCGCCGAG | SEQ ID NO:200 |

| Gene Name | GenBank Homology | DNA SEQUENCE / DEDUCED AMINO ACID SEQUENCE | SEQUENCE IDENTIFIER: |
|---|---|---|---|
| | | 41 I S H P E E E G D L A D I K S S L V N E<br>121 ATCAGTCATCCCGAAGAGGAAGGCGATTTAGCTGACATCAAGTCTTCCTTGGTGAACGAG<br><br>61 S E I I P A S N G H E V A R Q A Q T S Q<br>181 TCTGAAATCATCCCGGCCAGCAACGGACACGAGGTGGCCAGACAAGCACAAACCTCTCAG<br><br>81 E P Y H D K A R E H P D D G K H P D G G<br>241 GAGCCCTACCACGACAAGGCCAGAGAACACCCCGATGACGGAAAGCATCCAGATGGAGGC<br><br>101 L Y N K G P S Y S S Y S G Y I M M P N M<br>301 CTCTACAACAAGGGACCCTCCTACTCGAGTTATTCCGGGTACATAATGATGCCAAATATG<br><br>121 N N D P Y M S N G S L S P P I P R T S N<br>361 AATAACGACCCATACATGTCAAATGGATCTCTTTCTCCACCCATCCCGAGAACATCAAAT<br><br>141 K V P V V Q P S H A V H P L T P L I T Y<br>421 AAAGTGCCCGTGGTGCAGCCATCCCATGCGGTCCATCCTCTCACCCCCCTCATCACTTAC<br><br>161 S D E H F S P G S H P S H I P S D V N S<br>481 AGTGACGAGCACTTTTCTCCAGGATCACACCCGTCACACATCCCATCAGATGTCAACTCC<br><br>181 K Q G M S R H P P A P D I P T F Y P L S<br>541 AAACAAGGCATGTCCAGACATCCTCCAGCTCCTGATATCCCTACTTTTTATCCCTTGTCT<br><br>201 P G G V G Q I T P P L G W Q G Q P V Y P<br>601 CCGGGTGGTGTTGGACAGATCACCCCACCTCTTGGCTGGCAAGGTCAGCCTGTATATCCC<br><br>221 I T G G F R Q P Y P S S L S V D T S M S<br>661 ATCACGGGTGGATTCAGGCAACCCTACCCATCCTCACTGTCAGTCGACACTTCCATGTCC<br><br>241 R F S H H M I P G P P G P H T T G I P H<br>721 AGGTTTTCCCATCATATGATTCCCGGTCCTCCTGGTCCCCACACAACTGGCATCCCTCAT<br><br>261 P A I V T P Q V K Q E H P H T D S D L M<br>781 CCAGCTATTGTAACACCTCAGGTCAAACAGGAACATCCCCACACTGACAGTGACCTAATG<br><br>281 H V K P Q H E Q R K E Q E P K R P H I K<br>841 CACGTGAAGCCTCAGCATGAACAGAGAAAGGAGCAGGAGCCAAAAAGACCTCACATTAAG<br><br>301 K P L N A F M L Y M K E M R A N V V A E<br>901 AAGCCTCTGAATGCTTTTATGTTATACATGAAAGAAATGAGAGCGAATGTCGTTGCTGAG | |

| Gene Name | GenBank Homology | DNA SEQUENCE / DEDUCED AMINO ACID SEQUENCE | SEQUENCE IDENTIFIER: |
|---|---|---|---|
| | | 321 C T L K E S A A I N Q I L G R R W H A L<br>961 TGTACTCTAAAAGAAAGTGCAGCTATCAACCAGATTCTTGGCAGAAGGTGGCATGCCCTC<br><br>341 S R E E Q A K Y Y E L A R K E R Q L H M<br>1021 TCCCGTGAAGAGCAGGCTAAATATTATGAATTAGCACGGAAAGAAAGACAGCTACATATG<br><br>361 Q L Y P G W S A R D N Y G K K K K R K R<br>1081 CAGCTTTATCCAGGCTGGTCTGCAAGAGACAATTATGGTAAGAAAAAGAAGAGGAAGAGA<br><br>381 E K L Q E S A S G T G P R M T A A Y I -<br>1141 GAGAAACTACAGGAATCTGCATCAGGTACAGGTCCAAGAATGACAGCTGCCTACATCTGA | |
| WBC311.bFSP | Homo sapiens THAP domain containing 5, mRNA (cDNA clone MGC:61780 IMAGE:5165075), complete cds | 1 gtgaggcctt ggacctgtgg cctttgggct tgggtgtctc ctgtccgctc tgccagtgag<br>61 tcctcagctc tccgtacgtg gcccgtcgcc cgcggcaaga cggagggaag gcgctagttc<br>121 agtttagcta gcaatgccct tcttgcgcga ggactgctgg ctggtggcga ggcccaggag<br>181 cctgtttggg tgttaacagt taaaacagta aagtttgcgc gggcttctgt ggttttcagt<br>241 cttttcccca cttcacgtga tctcatttca ttgagcaaca ttaaccacca tttaagatac<br>301 gtgaaacctg cgaagtggaa aggtgcacgc aaagttaatt tacacccccc gcgagatttc<br>361 ctctacatga caaagaaaga ctggaaaagt ggttaaagaa tatgaagcga gattcatggg<br>421 ttcccagtaa ataccagttt ctatgtagtg accattttac tcctgactct cttgacatca<br>481 gatggggtat tcgatattta aaacaaactg cagttccaac aatatttttct ttgcctgaag<br>541 acaatcaggg aaaagaccct tctaaaaaaa aatcccagaa gaaaaacttg gaagatgaga<br>601 aagaagtatg cccaaaagcc aagtcagaag aatcatttgt attaaatgag acaaagaaaa<br>661 atatagttaa cacagatgtg ccccatcaac atccagaatt acttcattca tcttccttgg<br>721 taaagccacc agctcccaaa acaggaagta tacaaaataa catgttaact cttaatctag<br>781 ttaaacaaca tactgggaaa ccagaatcta ccttggaaac atcagttaac caagatacag<br>841 gtagaggtgg ttttcacaca tgttttgaga atctaaattc tacaactatt actttgacaa<br>901 cttcaaattc agaaagtatt catcaatctt tggaaactca agaagttctt gaagtaacta<br>961 ccagtcatct tgctaatcca aactttacaa gtaattccat ggaaataaag tcagcacagg<br>1021 aaaatccatt cttattcagc acaattaatc aaacagttga agaattaaac acaaataaag<br>1081 aatctgttat tgccattttt gtacctgctg aaaattctaa accctcagtt aattctttta<br>1141 tatctgcaca aaaagaaacc acggaaatgg aagacacaga cattgaagac tccttgtata<br>1201 aggatgtaga ctatgggaca gaagttttac aaatcgaaca ttcttactgc agacaagata<br>1261 taaataagga acatctttgg cagaaagtct ctaagctaca ttcaaagata actcttctag<br>1321 agttaaaaga gcaacaaact ctaggtagat tgaagtcttt ggaagctctt ataaggcagc<br>1381 taaagcagga aaactggcta tctgaagaaa acgtcaagat tatagaaaac cattttacaa<br>1441 catatgaagt cactatgata tagaataact aggttttaaa actatggctg ttaaataagc<br>1501 tttttccagc caaaccaaac tacatgtaaa gtgaactgtt tcctgtataa agttctcatc<br>1561 ttaatgaaaa aaaaaaaaaa aaaaaaaaaa aaaaaaaaaa aaaaaaaaaa aaaaaaaaaa<br>1621 aaaaaaaaaa aaaaaaaaaa aaaaaaaaaa aaaaaaaaaa aaaaaaaaaa aaaaaaaaaa | SEQ ID NO:201 |
| | | 1 M L T L N L V K Q H T G K P E S T L E T | SEQ ID NO:202 |

| Gene Name | GenBank Homology | DNA SEQUENCE / DEDUCED AMINO ACID SEQUENCE | SEQUENCE IDENTIFIER: |
|---|---|---|---|
| | | 1 ATGTTAACTCTTAATCTAGTTAAACAACATACTGGGAAACCAGAATCTACCTTGGAAACA<br><br>21 S V N Q D T G R G G F H T C F E N L N S<br>61 TCAGTTAACCAAGATACAGGTAGAGGTGGTTTTCACACATGTTTTGAGAATCTAAATTCT<br><br>41 T T I T L T T S N S E S I H Q S L E T Q<br>121 ACAACTATTACTTTGACAACTTCAAATTCAGAAAGTATTCATCAATCTTTGGAAACTCAA<br><br>61 E V L E V T T S H L A N P N F T S N S M<br>181 GAAGTTCTTGAAGTAACTACCAGTCATCTTGCTAATCCAAACTTTACAAGTAATTCCATG<br><br>81 E I K S A Q E N P F L F S T I N Q T V. E<br>241 GAAATAAAGTCAGCACAGGAAAATCCATTCTTATTCAGCACAATTAATCAAACAGTTGAA<br><br>101 E L N T N K E S V I A I F V P A E N S K<br>301 GAATTAAACACAAATAAAGAATCTGTTATTGCCATTTTTGTACCTGCTGAAAATTCTAAA<br><br>121 P S V N S F I S A Q K E T T E M E D T D<br>361 CCCTCAGTTAATTCTTTTATATCTGCACAAAAAGAAACCACGGAAATGGAAGACACAGAC<br><br>141 I E D S L Y K D V D Y G T E V L Q I E H<br>421 ATTGAAGACTCCTTGTATAAGGATGTAGACTATGGGACAGAAGTTTTACAAATCGAACAT<br><br>161 S Y C R Q D I N K E H L W Q K V S K L H<br>481 TCTTACTGCAGACAAGATATAAATAAGGAACATCTTTGGCAGAAAGTCTCTAAGCTACAT<br><br>181 S K I T L L E L K E Q Q T L G R L K S L<br>541 TCAAAGATAACTCTTCTAGAGTTAAAAGAGCAACAAACTCTAGGTAGATTGAAGTCTTTG<br><br>201 E A L I R Q L K Q E N W L S E E N V K I<br>601 GAAGCTCTTATAAGGCAGCTAAAGCAGGAAAACTGGCTATCTGAAGAAAACGTCAAGATT<br><br>221 I E N H F T T Y E V T M I -<br>661 ATAGAAAACCATTTTACAACATATGAAGTCACTATGATATAG | |
| WBC327.bFSP | Homo sapiens maltase-glucoamylase mRNA. | 1 ATTGCTAAGCCATCCTTCAGACAGAGAGGGAGCGGCTGCAAGAGGTAATGAGAGATGGCA<br>61 AGAAAGAAGCTGAAAAAATTTACTACTTTGGAGATTGTGCTCAGTGTTCTTCTGCTTGTG<br>121 TTGTTTATCATCAGTATTGTTCTAATTGTGCTTTTAGCCAAAGAGTCACTGAAATCAACA<br>181 GCCCCAGATCCTGGGACAACTGGTACCCCAGATCCTGGGACAACTGGTACCCCAGATCCT<br>241 GGAACAACTGGTACCACACATGCTAGGACAACGGGTCCCCCAGATCCTGGAACAACTGGT<br>301 ACCACTCCTGTTTCTGCTGAATGTCCAGTGGTAAATGAATTGGAACGAATTAATTGCATC<br>361 CCTGACCAGCCGCCAACAAAGGCCACATGTGACCAACGTGGCTGTTGCTGGAATCCCCAG | SEQ ID NO:203 |

| Gene Name | GenBank Homology | DNA SEQUENCE / DEDUCED AMINO ACID SEQUENCE | SEQUENCE IDENTIFIER: |
|---|---|---|---|
| | | 421 GGAGCTGTAAGTGTTCCCTGGTGCTACTATTCCAAGAATCATAGCTACCATGTAGAGGGC<br>481 AACCTTGTCAACACAAATGCAGGATTCACAGCCCGGTTGAAAAATCTGCCTTCTTCACCA<br>541 GTGTTTGGAAGCAATGTTGACAATGTTCTTCTCACAGCAGAATATCAGACATCTAATCGT<br>601 TTCCACTTTAAGTTGACTGACCAAACCAATAACAGGTTTGAAGTGCCCCACGAACACGTG<br>661 CAGTCCTTCAGTGGAAATGCTGCTGCTTCTTTGACCTACCAAGTTGAAATCTCCAGACAG<br>721 CCATTTAGCATCAAAGTGACCAGAAGAAGCAACAATCGTGTTTTGTTTGACTCGAGCATT<br>781 GGGCCCCTACTGTTTGCTGACCAGTTCTTGCAGCTCTCCACTCGACTGCCTAGCACTAAC<br>841 GTGTATGGCCTGGGAGAGCATGTGCACCAGCAGTATCGGCATGATATGAATTGGAAGACC<br>901 TGGCCCATATTTAACAGAGACACAACTCCCAATGGAAACGGAACTAATTTGTATGGTGCG<br>961 CAGACATTCTTCTTGTGCCTTGAAGATGCTAGTGGATTGTCCTTTGGGGTGTTTCTGATG<br>1021 AACAGCAATGCCATGGAGGTTGTCCTTCAGCCTGCGCCAGCCATCACTTACCGCACCATT<br>1081 GGGGGCATTCTCGACTTCTATGTGTTCTTGGGAAACACTCCAGAGCAAGTTGTTCAAGAA<br>1141 TATCTAGAGCTCATTGGGCGGCCAGCCCTTCCCTCCTACTGGGCGCTTGGATTTCACCTC<br>1201 AGTCGTTACGAATATGGAACCTTAGACAACATGAGGGAAGTCGTGGAGAGAAATCGCGCA<br>1261 GCACAGCTCCCTTATGATGTTCAGCATGCTGATATTGATTATATGGATGAGAGAAGGGAC<br>1321 TTCACTTATGATTCAGTGGATTTTAAAGGCTTCCCTGAATTTGTCAACGAGTTACACAAT<br>1381 AATGGACAGAAGCTTGTCATCATTGTGGATCCAGCCATCTCCAACAACTCTTCCTCAAGT<br>1441 AAACCCTATGGCCCATATGACAGGGGTTCAGATATGAAGATATGGGTGAATAGTTCAGAT<br>1501 GGAGTGACTCCACTCATTGGGGAGGTCTGGCCTGGACAAACTGTGTTTCCTGATTATACC<br>1561 AATCCCAACTGTGCTGTTTGGTGGACAAAGGAATTTGAGCTTTTTCACAATCAAGTAGAG<br>1621 TTTGATGGAATCTGGATTGATATGAATGAAGTCTCCAACTTTGTTGATGGTTCGGTCTCA<br>1681 GGATGTTCCACAAACAACCTAAATAATCCCCCATTCACTCCCAGAATCCTGGATGGGTAC<br>1741 CTGTTCTGCAAGACTCTCTGTATGGATGCAGTGCAGCACTGGGGCAAGCAGTATGACATT<br>1801 CACAATCTGTATGGCTACTCCATGGCGGTCGCCACAGCAGAAGCTGCCAAGACTGTGTTC<br>1861 CCTAATAAGAGAAGCTTCATTCTGACCCGTTCTACCTTTGCGGGCTCTGGCAAGTTTGCA<br>1921 GCACATTGGTTAGGAGACAACACTGCCACCTGGGATGACCTGAGATGGTCCATCCCTGGC<br>1981 GTGCTTGAGTTCAACCTTTTTGGCATCCCAATGGTGGGTCCTGACATATGTGGCTTTGCT<br>2041 TTGGACACCCCTGAGGAGCTCTGTAGGCGGTGGATGCAGTTGGGTGCATTTTATCCGTTT<br>2101 TCTAGAAATCACAATGGCCAAGGCTACAAGGACCAGGATCCTGCCTCCTTTGGAGCTGAC<br>2161 TCCCTGCTGTTGAATTCCTCCAGGCACTACCTTAACATCCGCTATACTCTATTGCCCTAC<br>2221 CTATACACCCTTTTCTTCCGTGCTCACAGCCGAGGGGACACGGTGGCCAGGCCCCTTTTG<br>2281 CATGAGTTCTACGAGGACAACAGCACTTGGGATGTGCACCAACAGTTCTTATGGGGGCCC<br>2341 GGCCTCCTCATCACTCCAGTTCTGGATGAAGGTGCAGAGAAAGTGATGGCATATGTGCCT<br>2401 GATGCTGTCTGGTATGACTACGAGACTGGGAGCCAAGTGAGATGGAGGAAGCAAAAAGTC<br>2461 GAGATGGAACTTCCTGGAGACAAAATTGGACTTCACCTTCGAGGAGGCTACATCTTCCCC<br>2521 ACACAGCAGCCAAATACAACCACTCTGGCCAGTCGAAAGAACCCTCTTGGTCTTATCATT<br>2581 GCCCTAGATGAGAACAAAGAAGCAAAAGGAGAACTTTTCTGGGATGATGGGGAAACGAAG<br>2641 GATACTGTGGCCAATAAAGTGTATCTTTTATGTGAGTTTTCTGTCACTCAAAACCGCTTG<br>2701 GAGGTGAATATTTCACAATCAACCTACAAGGACCCCAATAATTTAGCATTTAATGAGATT<br>2761 AAAATTCTTGGGACGGAGGAACCTAGCAATGTTACAGTGAAACACAATGGTGTCCCAAGT<br>2821 CAGACTTCTCCTACAGTCACTTATGATTCTAACCTGAAGGTTGCCATTATCACAGATATT<br>2881 GATCTTCTCCTGGGAGAAGCATACACAGTGGAATGGAGCATAAAGATAAGGGATGAAGAA<br>2941 AAAATAGACTGTTACCCTGATGAGAATGGTGCTTCTGCCGAAAACTGCACTGCCCGTGGC | |

| Gene Name | GenBank Homology | DNA SEQUENCE / DEDUCED AMINO ACID SEQUENCE | SEQUENCE IDENTIFIER: |
|---|---|---|---|
| | | 3001 TGTATCTGGGAGGCATCCAATTCTTCTGGAGTCCCTTTTTGCTATTTTGTCAACGACCTA<br>3061 TACTCTGTCAGTGATGTTCAGTATAATTCCCATGGGGCCACAGCTGACATCTCCTTAAAG<br>3121 TCTTCCGTTTATGCCAATGCCTTCCCCTCCACACCCGTGAACCCCCTTCGCCTGGATGTC<br>3181 ACTTACCATAAGAATGAAATGCTGCAGTTCAAGATTTATGATCCCAACAAGAATCGGTAT<br>3241 GAAGTTCCAGTCCCTCTGAACATACCCAGCATGCCATCCAGCACCCCTGAGGGTCAACTC<br>3301 TATGATGTGCTCATTAAGAAGAATCCATTTGGGATTGAAATTCGCCGGAAGAGTACAGGC<br>3361 ACTATAATTTGGGACTCTCAGCTCCTTGGCTTTACCTTCAGTGACATGTTTATCCGCATC<br>3421 TCCACCCGCCTTCCCTCCAAGTACCTCTATGGCTTTGGGGAAACTGAGCACAGGTCCTAT<br>3481 AGGAGAGACTTGGAGTGGCACACTTGGGGGATGTTCTCCCGAGACCAGCCCCCAGGGTAC<br>3541 AAGAAGAATTCCTATGGTGTCCACCCCTACTACATGGGGCTGGAGGAGGACGGCAGTGCC<br>3601 CATGGAGTGCTCCTGCTGAACAGCAATGCCATGGATGTGACGTTCCAGCCCCTGCCTGCC<br>3661 TTGACATACCGCACCACAGGGGGAGTTCTGGACTTTTATGTGTTCTTGGGGCCGACTCCA<br>3721 GAGCTTGTCACCCAGCAGTACACTGAGTTGATTGGCCGGCCTGTGATGGTACCTTACTGG<br>3781 TCTTTGGGGTTCCAGCTGTGTCGCTATGGCTACCAGAATGACTCTGAGATCGCCAGCTTG<br>3841 TATGATGAGATGGTGGCTGCCCAGATCCCTTATGATGTGCAGTACTCAGACATCGACTAC<br>3901 ATGGAGCGGCAGCTGGACTTCACCCTCAGCCCCAAGTTTGCTGGGTTTCCAGCTCTGATC<br>3961 AATCGCATGAAGGCTGATGGGATGCGGGTCATCCTCATTCTGGATCCAGCCATTTCTGGC<br>4021 AATGAGACACAGCCTTATCCTGCCTTCACTCGGGGCGTGGAGGATGACGTCTTCATCAAA<br>4081 TACCCAAATGATGGAGACATTGTCTGGGGAAAGGTCTGGCCTGATTTTCCTGATGTTGTT<br>4141 GTGAATGGGTCTCTAGACTGGGACAGCCAAGTGGAGCTATATCGAGCTTATGTGGCCTTC<br>4201 CCAGACTTTTTCCGTAATTCAACTGCCAAGTGGTGGAAGAGGGAAATAGAAGAACTATAC<br>4261 AACAATCCACAGAATCCAGAGAGGAGCTTGAAGTTTGATGGCATGTGGATTGATATGAAT<br>4321 GAACCATCAAGCTTCGTGAATGGGGCAGTTTCTCCAGGCTGCAGGGACGCCTCTCTGAAC<br>4381 CACCCTCCCTACATGCCACATTTGGAGTCCAGGGACAGGGGCCTGAGCAGCAAGACCCTT<br>4441 TGTATGGAGAGTCAGCAGATCCTCCCAGACGGCTCCCTGGTGCAGCACTACAACGTGCAC<br>4501 AACCTGTATGGGTGGTCCCAGACCAGACCCACATACGAAGCCGTGCAGGAGGTGACGGGA<br>4561 CAGCGAGGGGTCGTCATCACCCGCTCCACATTTCCCTCTTCTGGCCGCTGGGCAGGACAT<br>4621 TGGCTGGGAGACAACACGGCCGCATGGGATCAGCTGAAGAAGTCTATCATTGGCATGATG<br>4681 GAGTTCAGCCTCTTCGGCATATCCTATACGGGAGCAGATATCTGTGGGTTCTTTCAAGAT<br>4741 GCTGAATATGAGATGTGTGTTCGCTGGATGCAGCTGGGGGCCTTTTACCCCTTCTCAAGA<br>4801 AACCACAACACCATTGGGACCAGGAGACAAGACCCTGTGTCCTGGGATGTTGCTTTTGTG<br>4861 AATATTTCCAGAACTGTCCTGCAGACCAGATACACCCTGTTGCCATATCTGTATACCTTG<br>4921 ATGCATAAGGCCCACACGGAGGGCGTCACTGTTGTGCGGCCTCTGCTCCATGAATTTGTG<br>4981 TCAGACCAGGTGACATGGGACATAGACAGTCAGTTCCTGCTGGGCCCAGCCTTCCTGGTC<br>5041 AGCCCTGTCCTGGAGCGTAATGCCAGAAATGTCACTGCATATTTCCCTAGAGCCCGCTGG<br>5101 TATGATTACTACACGGGTGTGGATATTAATGCAAGAGGAGAGTGGAAGACCTTGCCAGCC<br>5161 CCTCTTGACCACATTAATCTTCATGTCCGTGGGGGCTACATCCTGCCCTGGCAAGAGCCT<br>5221 GCACAGAACACCCACTTAAGCCGTCAGAAATTCATAGGTTTCAAGGTTGCATTGGATGAC<br>5281 GATGGGACTGCTGAAGGTTGTCTCTTCTGGGATGATGGGCAAAGCATTGATACCTATGAA<br>5341 AAAGGACTCTATTATCTGGCCAACTTTTCTGCCAGCCAGAATACGATGCAGAGTCATGTA<br>5401 ATTTTCAACCAATACATCGTTGACACAAACCCTTTGAAACTGGGCTACATTGAGATCTGG<br>5461 GGAGTGGGCAGTGCTCCTGTTTCCAATGTCAGTGTCTCTCTGAGCAGCACAGTTATAACA<br>5521 CCCGCCTTCACCTACAACCCTACAACACAGGTGTTAAACATCGATGTGACCAGCAGAGAC | |

| Gene Name | GenBank Homology | DNA SEQUENCE / DEDUCED AMINO ACID SEQUENCE | SEQUENCE IDENTIFIER: |
|---|---|---|---|
| | | 5581 ATCAGCCTACATAATTTCATCTCATTGACATGGAGCAGCACTCTGTGAATTTTTAGAGCA<br>5641 AGATCCTAATTATGAAAGGCTTTGAAACTCCTTGTACATCATACCCATAAAATTACTGTG<br>5701 TGTTGCTAATTGGTTCAAGCCCAATATGGTTAAAATATTTCATTAATTTTGTTGTATGTT<br>5761 TTTTGTGTGAACCCTAAAGGTTAAACCTTAGCCCTGTAATATAGGCAGTAGGGAAGTGTG<br>5821 AAAAATCTATGGGGTACCTTAATGTCTCTGTGTGATCAGTATGGTACTGATCACTCATCA<br>5881 TACAACATTTGTTGAAGATGCTCTGGGCCCATGATGAAGTGTGTGTATGTCCACGTTTGT<br>5941 AATCATAGAATGGACCCCATTCTTTTGTTAAATACACAAGAGAAAGCTTTCTGTGACAGT<br>6001 TCCAGGTCTTGAAGCTAATCAGCATCTCAAGAAAGTATCCAGAAAGAACATCTGCTAGTT<br>6061 GGTTATAGGCGGTGGGAGGAATAATATACCTAATTGGTTATAGGTGGGGGGAGCATGATA<br>6121 AGCAAAGAAAAGGCAAACACAAGGAAAGATCAGATGAAACAGAAGATGATAGTAAAAGTG<br>6181 ATCCTAAGTAAGAACATAATGTAAAATTGTCAGCAGCCTCATGGGGAGGTGAAAGGAAGA<br>6241 GCCAACTCACCTGAAGAAGAGGGTCTTGAGAAATCCTTAGCATAAAGGGCTACTGGTGAG<br>6301 ATTGAGATCTGAGCAGGCAAAGCTCAAAAGAGAGTTTGGAGGTTAAAAATAATTTATTTT<br>6361 TGCAGTGGTGTGCTTTGAAATTTGTCAGTCTTATTTCTAATGTATATAACCACAGTTCCC<br>6421 ATAAATGCATGAAATTTATATGCCAGAGAAAAATAAAACAAGTGAATTTGCAAGTGAAAA<br>6481 AAAAAAAAAAAAAAAAAAAAAAAAAA | |
| | | 1 M A R K K L K K F T T L E I V L S V L L<br>1 ATGGCAAGAAAGAAGCTGAAAAAATTTACTACTTTGGAGATTGTGCTCAGTGTTCTTCTG<br><br>21 L V L F I I S I V L I V L L A K E S L K<br>61 CTTGTGTTGTTTATCATCAGTATTGTTCTAATTGTGCTTTTAGCCAAAGAGTCACTGAAA<br><br>41 S T A P D P G T T G T P D P G T T G T P<br>121 TCAACAGCCCCAGATCCTGGGACAACTGGTACCCCAGATCCTGGGACAACTGGTACCCCA<br><br>61 D P G T T G T T H A R T T G P P D P G T<br>181 GATCCTGGAACAACTGGTACCACACATGCTAGGACAACGGGTCCCCCAGATCCTGGAACA<br><br>81 T G T T P V S A E C P V V N E L E R I N<br>241 ACTGGTACCACTCCTGTTTCTGCTGAATGTCCAGTGGTAAATGAATTGGAACGAATTAAT<br><br>101 C I P D Q P P T K A T C D Q R G C C W N<br>301 TGCATCCCTGACCAGCCGCCAACAAAGGCCACATGTGACCAACGTGGCTGTTGCTGGAAT<br><br>121 P Q G A V S V P W C Y Y S K N H S Y H V<br>361 CCCCAGGGAGCTGTAAGTGTTCCCTGGTGCTACTATTCCAAGAATCATAGCTACCATGTA<br><br>141 E G N L V N T N A G F T A R L K N L P S<br>421 GAGGGCAACCTTGTCAACACAAATGCAGGATTCACAGCCCGGTTGAAAAATCTGCCTTCT<br><br>161 S P V F G S N V D N V L L T A E Y Q T S<br>481 TCACCAGTGTTTGGAAGCAATGTTGACAATGTTCTTCTCACAGCAGAATATCAGACATCT | SEQ ID NO:204 |

| Gene Name | GenBank Homology | DNA SEQUENCE / DEDUCED AMINO ACID SEQUENCE | SEQUENCE IDENTIFIER: |
|---|---|---|---|
| | | 181 N R F H F K L T D Q T N N R F E V P H E<br>541 AATCGTTTCCACTTTAAGTTGACTGACCAAACCAATAACAGGTTTGAAGTGCCCCACGAA<br><br>201 H V Q S F S G N A A A S L T Y Q V E I S<br>601 CACGTGCAGTCCTTCAGTGGAAATGCTGCTGCTTCTTTGACCTACCAAGTTGAAATCTCC<br><br>221 R Q P F S I K V T R R S N N R V L F D S<br>661 AGACAGCCATTTAGCATCAAAGTGACCAGAAGAAGCAACAATCGTGTTTTGTTTGACTCG<br><br>241 S I G P L L F A D Q F L Q L S T R L P S<br>721 AGCATTGGGCCCCTACTGTTTGCTGACCAGTTCTTGCAGCTCTCCACTCGACTGCCTAGC<br><br>261 T N V Y G L G E H V H Q Q Y R H D M N W<br>781 ACTAACGTGTATGGCCTGGGAGAGCATGTGCACCAGCAGTATCGGCATGATATGAATTGG<br><br>281 K T W P I F N R D T T P N G N G T N L Y<br>841 AAGACCTGGCCCATATTTAACAGAGACACAACTCCCAATGGAAACGGAACTAATTTGTAT<br><br>301 G A Q T F F L C L E D A S G L S F G V F<br>901 GGTGCGCAGACATTCTTCTTGTGCCTTGAAGATGCTAGTGGATTGTCCTTTGGGGTGTTT<br><br>321 L M N S N A M E V V L Q P A P A I T Y R<br>961 CTGATGAACAGCAATGCCATGGAGGTTGTCCTTCAGCCTGCGCCAGCCATCACTTACCGC<br><br>341 T I G G I L D F Y V F L G N T P E Q V V<br>1021 ACCATTGGGGGCATTCTCGACTTCTATGTGTTCTTGGGAAACACTCCAGAGCAAGTTGTT<br><br>361 Q E Y L E L I G R P A L P S Y W A L G F<br>1081 CAAGAATATCTAGAGCTCATTGGGCGGCCAGCCCTTCCCTCCTACTGGGCGCTTGGATTT<br><br>381 H L S R Y E Y G T L D N M R E V V E R N<br>1141 CACCTCAGTCGTTACGAATATGGAACCTTAGACAACATGAGGGAAGTCGTGGAGAGAAAT<br><br>401 R A A Q L P Y D V Q H A D I D Y M D E R<br>1201 CGCGCAGCACAGCTCCCTTATGATGTTCAGCATGCTGATATTGATTATATGGATGAGAGA<br><br>421 R D F T Y D S V D F K G F P E F V N E L<br>1261 AGGGACTTCACTTATGATTCAGTGGATTTTAAAGGCTTCCCTGAATTTGTCAACGAGTTA<br><br>441 H N N G Q K L V I I V D P A I S N N S S<br>1321 CACAATAATGGACAGAAGCTTGTCATCATTGTGGATCCAGCCATCTCCAACAACTCTTCC | |

| Gene Name | GenBank Homology | DNA SEQUENCE / DEDUCED AMINO ACID SEQUENCE | SEQUENCE IDENTIFIER: |
|---|---|---|---|
| | | 461 S S K P Y G P Y D R G S D M K I W V N S<br>1381 TCAAGTAAACCCTATGGCCCATATGACAGGGGTTCAGATATGAAGATATGGGTGAATAGT<br><br>481 S D G V T P L I G E V W P G Q T V F P D<br>1441 TCAGATGGAGTGACTCCACTCATTGGGGAGGTCTGGCCTGGACAAACTGTGTTTCCTGAT<br><br>501 Y T N P N C A V W W T K E F E L F H N Q<br>1501 TATACCAATCCCAACTGTGCTGTTTGGTGGACAAAGGAATTTGAGCTTTTTCACAATCAA<br><br>521 V E F D G I W I D M N E V S N F V D G S<br>1561 GTAGAGTTTGATGGAATCTGGATTGATATGAATGAAGTCTCCAACTTTGTTGATGGTTCG<br><br>541 V S G C S T N N L N N P P F T P R I L D<br>1621 GTCTCAGGATGTTCCACAAACAACCTAAATAATCCCCCATTCACTCCCAGAATCCTGGAT<br><br>561 G Y L F C K T L C M D A V Q H W G K Q Y<br>1681 GGGTACCTGTTCTGCAAGACTCTCTGTATGGATGCAGTGCAGCACTGGGGCAAGCAGTAT<br><br>581 D I H N L Y G Y S M A V A T A E A A K T<br>1741 GACATTCACAATCTGTATGGCTACTCCATGGCGGTCGCCACAGCAGAAGCTGCCAAGACT<br><br>601 V F P N K R S F I L T R S T F A G S G K<br>1801 GTGTTCCCTAATAAGAGAAGCTTCATTCTGACCCGTTCTACCTTTGCGGGCTCTGGCAAG<br><br>621 F A A H W L G D N T A T W D D L R W S I<br>1861 TTTGCAGCACATTGGTTAGGAGACAACACTGCCACCTGGGATGACCTGAGATGGTCCATC<br><br>641 P G V L E F N L F G I P M V G P D I C G<br>1921 CCTGGCGTGCTTGAGTTCAACCTTTTTGGCATCCCAATGGTGGGTCCTGACATATGTGGC<br><br>661 F A L D T P E E L C R R W M Q L G A F Y<br>1981 TTTGCTTTGGACACCCCTGAGGAGCTCTGTAGGCGGTGGATGCAGTTGGGTGCATTTTAT<br><br>681 P F S R N H N G Q G Y K D Q D P A S F G<br>2041 CCGTTTTCTAGAAATCACAATGGCCAAGGCTACAAGGACCAGGATCCTGCCTCCTTTGGA<br><br>701 A D S L L L N S S R H Y L N I R Y T L L<br>2101 GCTGACTCCCTGCTGTTGAATTCCTCCAGGCACTACCTTAACATCCGCTATACTCTATTG<br><br>721 P Y L Y T L F F R A H S R G D T V A R P<br>2161 CCCTACCTATACACCCTTTTCTTCCGTGCTCACAGCCGAGGGGACACGGTGGCCAGGCCC<br><br>741 L L H E F Y E D N S T W D V H Q Q F L W | |

| Gene Name | GenBank Homology | DNA SEQUENCE / DEDUCED AMINO ACID SEQUENCE | SEQUENCE IDENTIFIER: |
|---|---|---|---|
| | | 2221 CTTTTGCATGAGTTCTACGAGGACAACAGCACTTGGGATGTGCACCAACAGTTCTTATGG<br><br>761 G P G L L I T P V L D E G A E K V M A Y<br>2281 GGGCCCGGCCTCCTCATCACTCCAGTTCTGGATGAAGGTGCAGAGAAAGTGATGGCATAT<br><br>781 V P D A V W Y D Y E T G S Q V R W R K Q<br>2341 GTGCCTGATGCTGTCTGGTATGACTACGAGACTGGGAGCCAAGTGAGATGGAGGAAGCAA<br><br>801 K V E M E L P G D K I G L H L R G G Y I<br>2401 AAAGTCGAGATGGAACTTCCTGGAGACAAAATTGGACTTCACCTTCGAGGAGGCTACATC<br><br>821 F P T Q Q P N T T T L A S R K N P L G L<br>2461 TTCCCCACACAGCAGCCAAATACAACCACTCTGGCCAGTCGAAAGAACCCTCTTGGTCTT<br><br>841 I I A L D E N K E A K G E L F W D D G E<br>2521 ATCATTGCCCTAGATGAGAACAAAGAAGCAAAAGGAGAACTTTTCTGGGATGATGGGGAA<br><br>861 T K D T V A N K V Y L L C E F S V T Q N<br>2581 ACGAAGGATACTGTGGCCAATAAAGTGTATCTTTTATGTGAGTTTTCTGTCACTCAAAAC<br><br>881 R L E V N I S Q S T Y K D P N N L A F N<br>2641 CGCTTGGAGGTGAATATTTCACAATCAACCTACAAGGACCCCAATAATTTAGCATTTAAT<br><br>901 E I K I L G T E E P S N V T V K H N G V<br>2701 GAGATTAAAATTCTTGGGACGGAGGAACCTAGCAATGTTACAGTGAAACACAATGGTGTC<br><br>921 P S Q T S P T V T Y D S N L K V A I I T<br>2761 CCAAGTCAGACTTCTCCTACAGTCACTTATGATTCTAACCTGAAGGTTGCCATTATCACA<br><br>941 D I D L L L G E A Y T V E W S I K I R D<br>2821 GATATTGATCTTCTCCTGGGAGAAGCATACACAGTGGAATGGAGCATAAAGATAAGGGAT<br><br>961 E E K I D C Y P D E N G A S A E N C T A<br>2881 GAAGAAAAAATAGACTGTTACCCTGATGAGAATGGTGCTTCTGCCGAAAACTGCACTGCC<br><br>981 R G C I W E A S N S S G V P F C Y F V N<br>2941 CGTGGCTGTATCTGGGAGGCATCCAATTCTTCTGGAGTCCCTTTTTGCTATTTTGTCAAC<br><br>1001 D L Y S V S D V Q Y N S H G A T A D I S<br>3001 GACCTATACTCTGTCAGTGATGTTCAGTATAATTCCCATGGGGCCACAGCTGACATCTCC<br><br>1021 L K S S V Y A N A F P S T P V N P L R L<br>3061 TTAAAGTCTTCCGTTTATGCCAATGCCTTCCCCTCCACACCCGTGAACCCCCTTCGCCTG | |

| Gene Name | GenBank Homology | DNA SEQUENCE / DEDUCED AMINO ACID SEQUENCE | SEQUENCE IDENTIFIER: |
|---|---|---|---|
| | | 1041 D V T Y H K N E M L Q F K I Y D P N K N<br>3121 GATGTCACTTACCATAAGAATGAAATGCTGCAGTTCAAGATTTATGATCCCAACAAGAAT<br><br>1061 R Y E V P V P L N I P S M P S S T P E G<br>3181 CGGTATGAAGTTCCAGTCCCTCTGAACATACCCAGCATGCCATCCAGCACCCCTGAGGGT<br><br>1081 Q L Y D V L I K K N P F G I E I R R K S<br>3241 CAACTCTATGATGTGCTCATTAAGAAGAATCCATTTGGGATTGAAATTCGCCGGAAGAGT<br><br>1101 T G T I I W D S Q L L G F T F S D M F I<br>3301 ACAGGCACTATAATTTGGGACTCTCAGCTCCTTGGCTTTACCTTCAGTGACATGTTTATC<br><br>1121 R I S T R L P S K Y L Y G F G E T E H R<br>3361 CGCATCTCCACCCGCCTTCCCTCCAAGTACCTCTATGGCTTTGGGGAAACTGAGCACAGG<br><br>1141 S Y R R D L E W H T W G M F S R D Q P P<br>3421 TCCTATAGGAGAGACTTGGAGTGGCACACTTGGGGGATGTTCTCCCGAGACCAGCCCCCA<br><br>1161 G Y K K N S Y G V H P Y Y M G L E E D G<br>3481 GGGTACAAGAAGAATTCCTATGGTGTCCACCCCTACTACATGGGGCTGGAGGAGGACGGC<br><br>1181 S A H G V L L L N S N A M D V T F Q P L<br>3541 AGTGCCCATGGAGTGCTCCTGCTGAACAGCAATGCCATGGATGTGACGTTCCAGCCCCTG<br><br>1201 P A L T Y R T T G G V L D F Y V F L G P<br>3601 CCTGCCTTGACATACCGCACCACAGGGGGAGTTCTGGACTTTTATGTGTTCTTGGGGCCG<br><br>1221 T P E L V T Q Q Y T E L I G R P V M V P<br>3661 ACTCCAGAGCTTGTCACCCAGCAGTACACTGAGTTGATTGGCCGGCCTGTGATGGTACCT<br><br>1241 Y W S L G F Q L C R Y G Y Q N D S E I A<br>3721 TACTGGTCTTTGGGGTTCCAGCTGTGTCGCTATGGCTACCAGAATGACTCTGAGATCGCC<br><br>1261 S L Y D E M V A A Q I P Y D V Q Y S D I<br>3781 AGCTTGTATGATGAGATGGTGGCTGCCCAGATCCCTTATGATGTGCAGTACTCAGACATC<br><br>1281 D Y M E R Q L D F T L S P K F A G F P A<br>3841 GACTACATGGAGCGGCAGCTGGACTTCACCCTCAGCCCCAAGTTTGCTGGGTTTCCAGCT<br><br>1301 L I N R M K A D G M R V I L I L D P A I<br>3901 CTGATCAATCGCATGAAGGCTGATGGGATGCGGGTCATCCTCATTCTGGATCCAGCCATT | |

| Gene Name | GenBank Homology | DNA SEQUENCE / DEDUCED AMINO ACID SEQUENCE | SEQUENCE IDENTIFIER: |
|---|---|---|---|
| | | 1321 S G N E T Q P Y P A F T R G V E D D V F<br>3961 TCTGGCAATGAGACACAGCCTTATCCTGCCTTCACTCGGGGCGTGGAGGATGACGTCTTC<br><br>1341 I K Y P N D G D I V W G K V W P D F P D<br>4021 ATCAAATACCCAAATGATGGAGACATTGTCTGGGGAAAGGTCTGGCCTGATTTTCCTGAT<br><br>1361 V V V N G S L D W D S Q V E L Y R A Y V<br>4081 GTTGTTGTGAATGGGTCTCTAGACTGGGACAGCCAAGTGGAGCTATATCGAGCTTATGTG<br><br>1381 A F P D F F R N S T A K W W K R E I E E<br>4141 GCCTTCCCAGACTTTTTCCGTAATTCAACTGCCAAGTGGTGGAAGAGGGAAATAGAAGAA<br><br>1401 L Y N N P Q N P E R S L K F D G M W I D<br>4201 CTATACAACAATCCACAGAATCCAGAGAGGAGCTTGAAGTTTGATGGCATGTGGATTGAT<br><br>1421 M N E P S S F V N G A V S P G C R D A S<br>4261 ATGAATGAACCATCAAGCTTCGTGAATGGGGCAGTTTCTCCAGGCTGCAGGGACGCCTCT<br><br>1441 L N H P P Y M P H L E S R D R G L S S K<br>4321 CTGAACCACCCTCCCTACATGCCACATTTGGAGTCCAGGGACAGGGGCCTGAGCAGCAAG<br><br>1461 T L C M E S Q Q I L P D G S L V Q H Y N<br>4381 ACCCTTTGTATGGAGAGTCAGCAGATCCTCCCAGACGGCTCCCTGGTGCAGCACTACAAC<br><br>1481 V H N L Y G W S Q T R P T Y E A V Q E V<br>4441 GTGCACAACCTGTATGGGTGGTCCCAGACCAGACCCACATACGAAGCCGTGCAGGAGGTG<br><br>1501 T G Q R G V V I T R S T F P S S G R W A<br>4501 ACGGGACAGCGAGGGGTCGTCATCACCCGCTCCACATTTCCCTCTTCTGGCCGCTGGGCA<br><br>1521 G H W L G D N T A A W D Q L K K S I I G<br>4561 GGACATTGGCTGGGAGACAACACGGCCGCATGGGATCAGCTGAAGAAGTCTATCATTGGC<br><br>1541 M M E F S L F G I S Y T G A D I C G F F<br>4621 ATGATGGAGTTCAGCCTCTTCGGCATATCCTATACGGGAGCAGATATCTGTGGGTTCTTT<br><br>1561 Q D A E Y E M C V R W M Q L G A F Y P F<br>4681 CAAGATGCTGAATATGAGATGTGTGTTCGCTGGATGCAGCTGGGGGCCTTTTACCCCTTC<br><br>1581 S R N H N T I G T R R Q D P V S W D V A<br>4741 TCAAGAAACCACAACACCATTGGGACCAGGAGACAAGACCCTGTGTCCTGGGATGTTGCT<br><br>1601 F V N I S R T V L Q T R Y T L L P Y L Y | |

| Gene Name | GenBank Homology | DNA SEQUENCE / DEDUCED AMINO ACID SEQUENCE | SEQUENCE IDENTIFIER: |
|---|---|---|---|
| | | 4801 TTTGTGAATATTTCCAGAACTGTCCTGCAGACCAGATACACCCTGTTGCCATATCTGTAT<br><br>1621 T L M H K A H T E G V T V V R P L L H E<br>4861 ACCTTGATGCATAAGGCCCACACGGAGGGCGTCACTGTTGTGCGGCCTCTGCTCCATGAA<br><br>1641 F V S D Q V T W D I D S Q F L L G P A F<br>4921 TTTGTGTCAGACCAGGTGACATGGGACATAGACAGTCAGTTCCTGCTGGGCCCAGCCTTC<br><br>1661 L V S P V L E R N A R N V T A Y F P R A<br>4981 CTGGTCAGCCCTGTCCTGGAGCGTAATGCCAGAAATGTCACTGCATATTTCCCTAGAGCC<br><br>1681 R W Y D Y Y T G V D I N A R G E W K T L<br>5041 CGCTGGTATGATTACTACACGGGTGTGGATATTAATGCAAGAGGAGAGTGGAAGACCTTG<br><br>1701 P A P L D H I N L H V R G G Y I L P W Q<br>5101 CCAGCCCCTCTTGACCACATTAATCTTCATGTCCGTGGGGGCTACATCCTGCCCTGGCAA<br><br>1721 E P A Q N T H L S R Q K F I G F K V A L<br>5161 GAGCCTGCACAGAACACCCACTTAAGCCGTCAGAAATTCATAGGTTTCAAGGTTGCATTG<br><br>1741 D D D G T A E G C L F W D D G Q S I D T<br>5221 GATGACGATGGGACTGCTGAAGGTTGTCTCTTCTGGGATGATGGGCAAAGCATTGATACC<br><br>1761 Y E K G L Y Y L A N F S A S Q N T M Q S<br>5281 TATGAAAAAGGACTCTATTATCTGGCCAACTTTTCTGCCAGCCAGAATACGATGCAGAGT<br><br>1781 H V I F N Q Y I V D T N P L K L G Y I E<br>5341 CATGTAATTTTCAACCAATACATCGTTGACACAAACCCTTTGAAACTGGGCTACATTGAG<br><br>1801 I W G V G S A P V S N V S V S L S S T V<br>5401 ATCTGGGGAGTGGGCAGTGCTCCTGTTTCCAATGTCAGTGTCTCTCTGAGCAGCACAGTT<br><br>1821 I T P A F T Y N P T T Q V L N I D V T S<br>5461 ATAACACCCGCCTTCACCTACAACCCTACAACACAGGTGTTAAACATCGATGTGACCAGC<br><br>1841 R D I S L H N F I S L T W S S T L -<br>5521 AGAGACATCAGCCTACATAATTTCATCTCATTGACATGGAGCAGCACTCTGTGA | |
| WBC33.gRSP | No Homology | 1 GGGACGAAGTTTCCATGACATCATAAGCAGAGATGGCAGAAACATCAAGGATTCTGGGGA<br>61 TTACTTCTTATCAAGACTCAGAGAGTGTGGTGACCACAAAGTCCCCCTTCTGCTAAGGGA<br>121 CTGGCCCTGGATTTATGACCCCTGTGGAGTGGTAGCCACTAGCTGTGAAGAGGTCTCAGC<br>181 CCAGGCCCCTTCAGTACTCTTTTCTCCTGTATAGAAGGCATCATTTGTCTTTCTTTCCCC | SEQ ID NO:205 |

| Gene Name | GenBank Homology | DNA SEQUENCE / DEDUCED AMINO ACID SEQUENCE | SEQUENCE IDENTIFIER: |
|---|---|---|---|
| | | 241 AGAACCTTCCAAGACCTGGCCCTGACTTCTGCATGATAAACTGCTATCAGGAACCCTATA<br>301 TCAGTTAAAACACATTATCCCTCTCCACTCTCTTGGGAAGCCGGAATTTGAGACTGGGTG<br>361 GGGGCTTGGGATATTTTTAATAGAGCTTATCTTCAGCTAGTGGGTTAGTTATGATTCTAA<br>421 AACTTTGGGAGCCTGAGAGATTTGTTAAAAATATAGACACTTGGGCCCCACTCTAGGCAT<br>481 GCAGTCCCCAGGTGATTCTGAAGCAGCCCCTATTCAGAGTCTCTGGTTTAGAGGAAGAAC<br>541 TGAGGCCACTTAGACTATTGAGTCCTTCATGCCTGTTCCTGTCTACCATAGGTGGATAA<br>601 GAGTTTGTTCTTAGAGTAAGAGTAATTTAATGAACCTCAAGACAGTTTAATGAGAGTGGG<br>661 AGTTTCCTAGACCAGGCTTCCCAAAGGAGAAAAGGATT | |
| | | 1 TTTTTTTTTTTTTTGAAAAGTTAAACTGTCTTTATTCACAACCAACATTATCATCTACGT<br>61 AGGATGTCTAACAGGATGTACCAAAAAAAGCTACCAGCATTAATAAGTGAGTTTAGGAAG<br>121 TTTTCAGGGTACAAGATCAATGTACTAAAGTAACTGTATTTCTATATATTATCAATGAAC<br>181 AATTGGAAATTGAAATTTTAAAAATACCATATACAATAGCATCAAAAAATATGAAATATT<br>241 TAGGGCTAAATCTGACAGAAGATGTGTTCATGGATTGGAACACTCAATATCAGTAAGTTG<br>301 TCTGTTCTCCCCAAGTTGATCTATAAATTTGATGCAATCCTGATCATGATTCCAGGAGAC<br>361 CTTTTGTAGTAATGAGAAGTCAATTCGTTTGGGAGGGCAAAGGACTGAGAATAGCCAAAC<br>421 TGCTTTCTTTAAAAGTTGTAACCCAGCCTTTCCAATCCTGAGTATTTATCCAATAGAAAT<br>481 GAAAGCATACGCTCACATGATGACTTATACACCGAATGTTCGTAGCACTTTATTTGTAAT<br>541 AACCAAAACAGGAGACAACCCAAAAGACTGTCAACAGGTGAATGGATAAAGCAGCTTGTG<br>601 GTATATCCATACAATGAAATACTAATGAACAATAAAAAGGAATGAACTATTGAT | SEQ ID NO:206 |
| WBC335.bFSP | Homo sapiens radixin, mRNA (cDNA clone MGC:48283 IMAGE:5284438) | 1 ATTGCGGAGCTGTCTGCGGTGGCGGCGGCGCCTCTCGTCTCCCGCGGCCCAGCGCTCGCA<br>61 CCACCGCTTCTCCCTCCCTGTCGCAGCCGCGCCGCCGCGCAGCGCCCCAGCCACACGCCG<br>121 GCGGGCAGAAGCCGCCCGCTCTCCGGAAAGTGATAACAGAATTCATTGAAGTGGAGAATT<br>181 TTTAAAGAAGGTAACAAAAAGAGAAAGAAAATGCCGAAACCAATCAACGTAAGAGTAACT<br>241 ACAATGGATGCTGAGCTGGAATTTGCCATTCAGCCCAATACAACTGGCAAACAACTTTTT<br>301 GACCAGGTGGTGAAAACAGTTGGTTTGCGTGAGGTCTGGTTTTTTGGGCTGCAGTATGTA<br>361 GACAGCAAAGGTTATTCTACATGGCTTAAACTAAATAAAAAGGTAACACAGCAGGATGTT<br>421 AAAAAAGAGAATCCTTTACAGTTCAAGTTTAGAGCTAAATTCTTTCCTGAAGATGTTTCT<br>481 GAGGAATTAATTCAAGAAATAACCCAGAGACTCTTCTTCTTGCAAGTTAAAGAAGCCATC<br>541 TTAAATGATGAGATATATTGCCCGCCAGAAACTGCAGTTCTTTTGGCTTCCTATGCTGTC<br>601 CAAGCCAAGTATGGAGATTACAATAAAGAGATTCATAAGCCAGGCTACCTGGCTAATGAT<br>661 AGACTCCTACCCCAGCGTGTATTGGAACAACACAAACTAACAAAAGAACAGTGGGAAGAA<br>721 AGAATACAGAACTGGCATGAAGAACATAGAGGAATGTTAAGGGAGGATTCTATGATGGAA<br>781 TACCTGAAGATTGCACAAGATCTAGAAATGTATGGAGTCAACTATTTTGAAATAAAAAAT<br>841 AAAAAAGGAACTGAATTGTGGCTAGGTGTTGATGCTTTGGGTCTGAATATTTATGAGCAT<br>901 GACGACAAGTTAACACCTAAAATTGGTTTTCCCTGGAGTGAAATCAGAAATATTTCATTT<br>961 AATGACAAAAAATTTGTTATAAAGCCAATCGACAAAAAGGCACCTGATTTTGTGTTTTAT<br>1021 GCACCTCGTCTGAGAATCAATAAGAGGATTTTGGCCTTATGTATGGGAAACCATGAACTA<br>1081 TACATGCGAAGAAGGAAGCCTGATACTATTGAAGTACAACAGATGAAGGCTCAGGCTAGG<br>1141 GAGGAGAAACATCAGAAGCAGTTGGAAAGGGCACAATTAGAGAATGAAAAGGAGAAAAGA<br>1201 GAAATAGCAGAAAAGGAAAAGGAAAGAATAGAACGTGAAAAGGAAGAGCTAATGGAACGT | SEQ ID NO:207 |

| Gene Name | GenBank Homology | DNA SEQUENCE / DEDUCED AMINO ACID SEQUENCE | SEQUENCE IDENTIFIER: |
|---|---|---|---|
| | | 1261 CTAAAACAAATTGAAGAGCAGACAATTAAAGCTCAGAAAGAACTAGAAGAACAGACTCGA<br>1321 AAAGCTCTAGAACTGGATCAAGAACGAAAACGAGCAAAAGAAGAAGCAGAACGACTTGAA<br>1381 AAGGAGCGTCGAGCTGCTGAAGAGGCAAAGTCTGCCATAGCAAAACAAGCTGCCGACCAG<br>1441 ATGAAGAATCAGGAGCAGCTAGCAGCAGAACTTGCTGAATTCACTGCCAAGATTGCACTT<br>1501 CTAGAGGAAGCCAAGAAGAAAAAGGAAGAGGAAGCTACTGAGTGGCAACACAAAGCTTTT<br>1561 GCAGCCCAGGAAGACTTGGAAAAGACCAAAGAAGAGTTAAAAACTGTGATGTCTGCCCCC<br>1621 CCTCCACCTCCACCACCACCAGTCATTCCTCCAACAGAAAACGAACATGATGAACACGAT<br>1681 GAGAATAATGCTGAAGCTAGTGCTGAATTATCAAATGAAGGGGTAATGAACCATAGAAGC<br>1741 GAGGAAGAACGTGTAACCGAAACACAGAAAAATGAGCGTGTTAAGAAGCAACTTCAGGCA<br>1801 TTAAGTTCAGAATTAGCCCAAGCCAGAGATGAAACCAAGAAAACACAAAATGATGTTCTT<br>1861 CATGCTGAGAATGTTAAAGCAGGCCGTGATAAGTACAAGACTCTGCGACAGATTCGACAA<br>1921 GGCAATACAAAGCAGCGTATCGATGAGTTTGAAGCAATGTGAGAGCTGTTATTTTGCATA<br>1981 TATGTTCTTCATAAGCTGAACCACCAACAGAGAAAAGCAGGCCTTTGCAGATATGATGGA<br>2041 ATGCATCCCACCTTGCCAAAGCACTTACACCAGTTTGACTGTGCTAGCTAAAAGACAAAT<br>2101 TTAAGGGGAGCTCTTCAACATTAAGGCAGTATGATATCATGCTTGGTTTTCTTTTTTCTT<br>2161 TTGGTCCAGGGAATGGAGAATGGTGTTCCATTGCCTCTTTTCACATTTTTTTTCTTTTTC<br>2221 TTTTTTTTTTCTGTTGAAGATTAACACTAATTATCACGTCTGACAAATGTGTATGTGTGG<br>2281 TTTCAGTTCTGTGTACATTTTAAAGGATAATGGTGAACATTTTAATGGGTTTCCCTTGCC<br>2341 CTTTCCATATTTAACCTATTTCCACATTCTCTCTCACTCACATTTTCTCAGTGTGCCCTT<br>2401 CTCTTATCTGCCGTGTCCATAGCCATAATTCCACCATACAGATCATTGGCAGTGTTTAAA<br>2461 ATGAGGGTAGGTGACAGGGTGGACAGTGTTTGATCATCATGTAGAATATGGCTATGAATC<br>2521 AGGAAAGAGATTAGAACATTTAATAATGTATGTACAGCTGGTGCTTAGTTTTTTTTTAAT<br>2581 CTAAATTTAATTACATTATTGGATATTTGATGTTTGCTTACTAAATTACAGTCATCTTTG<br>2641 ACAGCTGAATTGATTGGTGTTTCATCTCCTGTCACCCTTTGGCTTTCTTTACTTACCAAT<br>2701 TCACAAAGGTGTAGACAGCAGGTAGTAGGTCCCTAGGAAAATTTATTGATGAAACAGCCT<br>2761 CTGCAAGATTTTAAAAGTTTTGTTCTTTCATAGATTGATTTAGAAAAACAAATGATTAGT<br>2821 TAAAAAAAGAAAATATACATTTGTTGGTAACTAATGTTATTTTTTAAAACCTGGACCTTT<br>2881 CTGGAAGGGCAGCCTATGAAAACATTAGTCCCAGAGAAGGGACAATAAATACTACCTCAC<br>2941 TACTACACCTGCGATGACTGGTTGTTCAAACACAACAGAGTGTGTCAGCTGTATGTTTTA<br>3001 TAATACATAACCATAGCCTCGATCATCAAGAAATACTTTCGAAATTTCATTTTCCTTCAG<br>3061 AATATCTTAAGAGTGCTAAATTTTTAACTGCCTTTTTGTCGAGTCAAACTGTGGGATTCT<br>3121 GATTTGTATTAAAATTGTAAGCTCCTCACTGGTATACTATCATCCTGGAGGGGTGTTGTA<br>3181 TGGCTGAGCAAGAGAGAGAGAGAATGAGAGAGAGACTGTGTGTGTGTGTGTGTGTGTGTG<br>3241 TACTCTGTGTGTGTATGAGAGAGAGAGAGAAATGCTAACCTTGTAGCATGTGAAGAATGT<br>3301 CTGTACCTTGATACGATAGTTACATAATCAGTATATTTGGTTTCTAGATCACTGTGCTTA<br>3361 TTTTGTTTCAATCTCTGACTAAAAATACTTAAATTTGGTTTGTTAATTCTTATTTTAGAA<br>3421 ATTATAATTTTAGTTTATATTAATTTCGGTTATAGCTTACTGAAGAAATCTTTCCAGTTA<br>3481 GAAGGAGGTTCTAATATTCACATGTTCTAATACTTTGTTTATTGTAAAACAGCTAAATTT<br>3541 GGAGATACGTAAAGCCTTGTTTTCTCTGTGTGGTTCAGCTACTTTCCATTTGGTATTACA<br>3601 CAGTCAAATTTACATTTATCTATTAAAATTGCCATTTTATTAAACATTTTCATGCACAGT<br>3661 AGATTCAAGTTGTGTCTGAAAATATCTCTTGTGCTTTTTTGATTTTGCTGACTTTAAAAG<br>3721 GATTAATCTGGGCAGACATTATGTAAAAGAAAGGTTGCGTTTAATATATTTTTTGAACTT<br>3781 TGTAGGACAAAACATAGCTGGTTAACCTTGAAGTGACTGTTGTACCATGGTTGTGCACAT | |

| Gene Name | GenBank Homology | DNA SEQUENCE / DEDUCED AMINO ACID SEQUENCE | SEQUENCE IDENTIFIER: |
|---|---|---|---|
| | | 3841 GCTTCAGAATCCTATGGAAGAGAATATTCCTACTTGCAGTACATCAAAGGAATGGATGGT<br>3901 GGACCCTACTATTCATGTTTTGAGACATAAATGTTCACTTTAAAGCAATTGCATAATAGA<br>3961 TAAAAACCTGAACTTTCATTGGATTTTTATTAATTTTCTTCATTTTGAATTGTGTGCACT<br>4021 ACCATTGTCACATCAGTTTGATATCAGTGTTGAAAAATTACCAGTTATATTTGCTGTTTA<br>4081 TAATCTATTTGTAGATTAGGATTAAAATGGATTTAATCCATTTTTAAAGCTGTGTGAATT<br>4141 TTTCTAAACAGGAACAACTATTTGCAATATAGGTTTTCATAGTGATTAAACCAATTATAA<br>4201 CTTTTATTATTAGCAATTGAGAGCACATGTTCATATAGCAATGTAAAAATATATTAATGA<br>4261 GTATTTGGTAAATTCCAACAGGCTTTTACCATTAGCATAATTTTGTGTTGTACAATTAAG<br>4321 TTACAATTACATCTATAATTTTGGATAACATTCATTGGTTAACAATAAAGTGACAAAAGC<br>4381 TCAAAAAAAAAAAAAAAAAGAAAAAAAAAAAAAAAAA<br><br>1 M P K P I N V R V T T M D A E L E F A I<br>1 ATGCCGAAACCAATCAACGTAAGAGTAACTACAATGGATGCTGAGCTGGAATTTGCCATT<br><br>21 Q P N T T G K Q L F D Q V V K T V G L R<br>61 CAGCCCAATACAACTGGCAAACAACTTTTTGACCAGGTGGTGAAAACAGTTGGTTTGCGT<br><br>41 E V W F F G L Q Y V D S K G Y S T W L K<br>121 GAGGTCTGGTTTTTTGGGCTGCAGTATGTAGACAGCAAAGGTTATTCTACATGGCTTAAA<br><br>61 L N K K V T Q Q D V K K E N P L Q F K F<br>181 CTAAATAAAAAGGTAACACAGCAGGATGTTAAAAAAGAGAATCCTTTACAGTTCAAGTTT<br><br>81 R A K F F P E D V S E E L I Q E I T Q R<br>241 AGAGCTAAATTCTTTCCTGAAGATGTTTCTGAGGAATTAATTCAAGAAATAACCCAGAGA<br><br>101 L F F L Q V K E A I L N D E I Y C P P E<br>301 CTCTTCTTCTTGCAAGTTAAAGAAGCCATCTTAAATGATGAGATATATTGCCCGCCAGAA<br><br>121 T A V L L A S Y A V Q A K Y G D Y N K E<br>361 ACTGCAGTTCTTTTGGCTTCCTATGCTGTCCAAGCCAAGTATGGAGATTACAATAAAGAG<br><br>141 I H K P G Y L A N D R L L P Q R V L E Q<br>421 ATTCATAAGCCAGGCTACCTGGCTAATGATAGACTCCTACCCCAGCGTGTATTGGAACAA<br><br>161 H K L T K E Q W E E R I Q N W H E E H R<br>481 CACAAACTAACAAAAGAACAGTGGGAAGAAAGAATACAGAACTGGCATGAAGAACATAGA<br><br>181 G M L R E D S M M E Y L K I A Q D L E M<br>541 GGAATGTTAAGGGAGGATTCTATGATGGAATACCTGAAGATTGCACAAGATCTAGAAATG<br><br>201 Y G V N Y F E I K N K K G T E L W L G V<br>601 TATGGAGTCAACTATTTTGAAATAAAAAATAAAAAAGGAACTGAATTGTGGCTAGGTGTT | SEQ ID NO:208 |

| Gene Name | GenBank Homology | DNA SEQUENCE / DEDUCED AMINO ACID SEQUENCE | SEQUENCE IDENTIFIER: |
|---|---|---|---|
| | | 221 D A L G L N I Y E H D D K L T P K I G F<br>661 GATGCTTTGGGTCTGAATATTTATGAGCATGACGACAAGTTAACACCTAAAATTGGTTTT<br><br>241 P W S E I R N I S F N D K K F V I K P I<br>721 CCCTGGAGTGAAATCAGAAATATTTCATTTAATGACAAAAAATTTGTTATAAAGCCAATC<br><br>261 D K K A P D F V F Y A P R L R I N K R I<br>781 GACAAAAAGGCACCTGATTTTGTGTTTTATGCACCTCGTCTGAGAATCAATAAGAGGATT<br><br>281 L A L C M G N H E L Y M R R R K P D T I<br>841 TTGGCCTTATGTATGGGAAACCATGAACTATACATGCGAAGAAGGAAGCCTGATACTATT<br><br>301 E V Q Q M K A Q A R E E K H Q K Q L E R<br>901 GAAGTACAACAGATGAAGGCTCAGGCTAGGGAGGAGAAACATCAGAAGCAGTTGGAAAGG<br><br>321 A Q L E N E K E K R E I A E K E K E R I<br>961 GCACAATTAGAGAATGAAAAGGAGAAAAGAGAAATAGCAGAAAAGGAAAAGGAAAGAATA<br><br>341 E R E K E E L M E R L K Q I E E Q T I K<br>1021 GAACGTGAAAAGGAAGAGCTAATGGAACGTCTAAAACAAATTGAAGAGCAGACAATTAAA<br><br>361 A Q K E L E E Q T R K A L E L D Q E R K<br>1081 GCTCAGAAAGAACTAGAAGAACAGACTCGAAAAGCTCTAGAACTGGATCAAGAACGAAAA<br><br>381 R A K E E A E R L E K E R R A A E E A K<br>1141 CGAGCAAAAGAAGAAGCAGAACGACTTGAAAAGGAGCGTCGAGCTGCTGAAGAGGCAAAG<br><br>401 S A I A K Q A A D Q M K N Q E Q L A A E<br>1201 TCTGCCATAGCAAAACAAGCTGCCGACCAGATGAAGAATCAGGAGCAGCTAGCAGCAGAA<br><br>421 L A E F T A K I A L L E E A K K K K E E<br>1261 CTTGCTGAATTCACTGCCAAGATTGCACTTCTAGAGGAAGCCAAGAAGAAAAAGGAAGAG<br><br>441 E A T E W Q H K A F A A Q E D L E K T K<br>1321 GAAGCTACTGAGTGGCAACACAAAGCTTTTGCAGCCCAGGAAGACTTGGAAAAGACCAAA<br><br>461 E E L K T V M S A P P P P P P P P V I P<br>1381 GAAGAGTTAAAAACTGTGATGTCTGCCCCCCCTCCACCTCCACCACCACCAGTCATTCCT<br><br>481 P T E N E H D E H D E N N A E A S A E L<br>1441 CCAACAGAAAACGAACATGATGAACACGATGAGAATAATGCTGAAGCTAGTGCTGAATTA | |

| Gene Name | GenBank Homology | DNA SEQUENCE / DEDUCED AMINO ACID SEQUENCE | SEQUENCE IDENTIFIER: |
|---|---|---|---|
| | | 501 S N E G V M N H R S E E E R V T E T Q K<br>1501 TCAAATGAAGGGGTAATGAACCATAGAAGCGAGGAAGAACGTGTAACCGAAACACAGAAA<br><br>521 N E R V K K Q L Q A L S S E L A Q A R D<br>1561 AATGAGCGTGTTAAGAAGCAACTTCAGGCATTAAGTTCAGAATTAGCCCAAGCCAGAGAT<br><br>541 E T K K T Q N D V L H A E N V K A G R D<br>1621 GAAACCAAGAAAACACAAAATGATGTTCTTCATGCTGAGAATGTTAAAGCAGGCCGTGAT<br><br>561 K Y K T L R Q I R Q G N T K Q R I D E F<br>1681 AAGTACAAGACTCTGCGACAGATTCGACAAGGCAATACAAAGCAGCGTATCGATGAGTTT<br><br>581 E A M -<br>1741 GAAGCAATGTGA | |
| WBC374.bFSP | No Homology | 1 AGTTTCTGCTTTTTGTTTTTAGAATAAATCGTCAGGGAAATGAAGAGAGGATGCTTTTGT<br>61 TTTGGGTTATGGTCAAGAACTGATTAAATAATTTAGTGTCTCTGGCACACACTAAAACCA<br>121 TATACTTCAGTTGTGATCTTGGTGACATATCTGTTGGCTTAGGTTTTATTACATTTATTA<br>181 TCGCAGATGTTCGGTTGACCAAGTAGTTCATTACTTTCTCTTAATTTTTTTAAATTTTAA<br>241 TTTGGGCTTCTAACTGCAGCGTTTGAGAACTCAGCATCCTTGTTTTCTGCAAATACTGAT<br>301 TAAAATAAAATGCTGTAAAACGTGTTGTAAAACATTGTAATTACCTTCCCATGTCTTTGT<br>361 TGTACTTGTGCAGAAATGTTTTCACATTCCTTTGTCCGGAGAAAAAATCTTTGCTTTCAT<br>421 TTTGATTATGTTGCTTACCTTGAAATCAATAGGTTTTGATATTTTCTCTTGGAAGATTTT<br>481 ATATCTTTTTGGGAATATGTAATATATGATCTCTAATAAAAGATAAATCTCAAAAAAAAA<br>541 AAAAAAAAAACTCG | SEQ ID NO:209 |
| WBC964.gRSP | No Homology | 1 GGGACCAAGGCAGAGGCTGAGCTGGGGATGTTCCCCAGTACATTCAGGAAACTCCTGCAC<br>61 ACAGTCAACGTACCCTCCTTCAGCAAGGCGCCCTCCAGCAGGCCCCAGCAGACCAGCTAC<br>121 GAACCCCCGTCTTGTTCCGGACCAAAGACCACTTTCCCTTGTTGCAGTGAAAGACCTTGG<br>181 GTGTGAGTCTGCTAGCCAACACTGCAAGAGGACGCTGAGATTATCTCGAGTATTAAATCC<br>241 ATTGAAAATCCTACTAATAGACATCCATGATGTTGGAGAGTATGGAATTTTACAGTTTAT<br>301 TTTTTGCACACCCTATTTCTCACTGTCTTCATTATTTCACGTAGGATGTATGATTTTCTA<br>361 TGTCTTCCACTTCTAACCTGAAAGCTGCTTGGTGGGGGACTGGAGAAGCAAAATGGATC<br>421 TGTGCGCTGTGGCAGAACTGTTATTTTTATGGATTTTACAGCTCAACTAACAGTGTTACC<br>481 TTTTCAAAATTTATATATACTCCTAAAGCCTTGTTATTATTTGATCCTAATAGTGATTCA<br>541 GTTTTCTATAGCTTACTTTTAGGGTAGATGGTAAATTATTTAACATTATATTAAACTTTC<br>601 CATATCATGGATAGTAAACCGAAGGAAATTATACAATTTCTGAGAAAATGTATTATTTAA<br>661 ACTAATTCTGAAGTTGTATTTGAAAATATGACCTCACAATCTTAATTTGTCCATAGCAAA<br>721 CATGTCTATATATGGTTGCAGCATTTGGTTTATAATTTAAATATTAATAAAGGTTTTAAA<br>781 TTTTGTTGGAAAAAAAAAAAAAAAAAAAA | SEQ ID NO:210 |

| Gene Name | GenBank Homology | DNA SEQUENCE / DEDUCED AMINO ACID SEQUENCE | SEQUENCE IDENTIFIER: |
|---|---|---|---|
| | | 1 TTTTTTTTTTTTTTTTTTTTCCAACAAAATTTAAAACCTTTATTAATATTTAAATTATAAA<br>61 CCAAATGCTGCAACCATATATAGACATGTTTGCTATGGACAAATTAAGATTGTGAGGTCA<br>121 TATTTTCAAATACAACTTCAGAATTAGTTTAAATAATACATTTTCTCAGAAATTGTATAA<br>181 TTTCCTTCGGTTTACTATCCATGATATGGAAAGTTTAATATAATGTTAAATAATTTACCA<br>241 TCTACCCTAAAAGTAAGCTATAGAAAACTGAATCACTATTAGGATCAAATAATAACAAGG<br>301 CTTTAGGAGTATATATAAATTTTGAAAAGGTAACACTGTTAGTTGAGCTGTAAAATCCAT<br>361 AAAAATAACAGTTCTGCCACAGCGCACAGATCCATTTTGCTTCTCCAGTCCCCCCACCAA<br>421 GCAGCTTTCAGGTTAGAAGTGGAAGACATAGAAAATCATACATCCTACGTGAAATAATGA<br>481 AGACAGTGAGAAATAGGGTGTGCAAAAAATAAACTGTAAAATTCCATACTCTCCAACATC<br>541 ATGGATGTCTATTAAGTAGGGATTTTCAATGGATTTAATACTCGAGATAATCTCAGCGTC<br>601 CTCTTGCAGTGGTGGC | SEQ ID NO:211 |
| B1961827 | Homo sapiens actin related protein 2/3 complex subunit 1B (41 kD) (ARPC1B) mRNA. | 1 gcgcgtcgac tgcccagagt ccgcggccgg ggcgcgggag gagccaagcc gccatggcct<br>61 accacagctt cctggtggag cccatcagct gccacgcctg gaacaaggac cgcacccaga<br>121 ttgccatctg ccccaacaac catgaggtgc atatctatga aaagagcggt gccaaatgga<br>181 ccaaggtgca cgagctcaag gagcacaacg ggcaggtgac aggcatcgac tgggcccccg<br>241 agagtaaccg tattgtgacc tgcggcacag accgcaacgc ctacgtgtgg acgctgaagg<br>301 gccgcacatg gaagcccacg ctggtcatcc tgcggatcaa ccgggctgcc cgctgcgtgc<br>361 gctgggcccc caacgagaac aagtttgctg tgggcagcgg ctctcgtgtg atctccatct<br>421 gttatttcga gcaggagaat gactggtggg tttgcaagca catcaagaag cccatccgct<br>481 ccaccgtcct cagcctggac tggcacccca acaatgtgct gctggctgcc ggctcctgtg<br>541 acttcaagtg tcggatcttt tcagcctaca tcaaggaggt ggaggaacgg ccggcaccca<br>601 ccccgtgggg ctccaagatg ccctttgggg aactgatgtt cgaatccagc agtagctgcg<br>661 gctgggtaca tggcgtctgt ttctcagcca gcgggagccg cgtggcctgg gtaagccacg<br>721 acagcaccgt ctgcctggct gatgccgaca agaagatggc cgtcgcgact ctggcctctg<br>781 aaacactacc actgctggcg ctgaccttca tcacagacaa cagcctggtg gcagcgggcc<br>841 acgactgctt cccggtgctg ttcacctatg acgccgccgc ggggatgctg agcttcggcg<br>901 ggcggctgga cgttcctaag cagagctcgc agcgtggctt gacggcccgc gagcgcttcc<br>961 agaacctgga caagaaggcg agctccgagg gtggcacggc tgcgggcgcg ggcctagact<br>1021 cgctgcacaa gaacagcgtc agccagatct cggtgctcag cggcggcaag gccaagtgct<br>1081 cgcagttctg caccactggc atggatggcg gcatgagtat ctgggatgtg aagagcttgg<br>1141 agtcagcctt gaaggacctc aagatcaaat gacctgtgag gaatatgttg ccttcatcct<br>1201 agctgctggg gaagcgggga gaggggtcag ggaggctaat ggttgctttg ctgaatgttt<br>1261 ctggggtacc aatacgagtt cccatagggg ctgctccctc aaaaagggag gggacagatg<br>1321 gggagctttt cttacctatt caaggaatac gtgccttttt cttaaatgct ttcatttatt<br>1381 gaaaaaaaaa aaaaatgccc ccaaagcact atgctggtca tgaactgctt caaaatgtgg<br>1441 aggtaataaa atgcaactgt gtaaaaaaaa aaaaaaaaaa aaaaa | SEQ ID NO:212 |
| | | 1 M A Y H S F L V E P I S C H A W N K D R<br>1 ATGGCCTACCACAGCTTCCTGGTGGAGCCCATCAGCTGCCACGCCTGGAACAAGGACCGC<br><br>21 T Q I A I C P N N H E V H I Y E K S G A | SEQ ID NO:213 |

| Gene Name | GenBank Homology | DNA SEQUENCE / DEDUCED AMINO ACID SEQUENCE | SEQUENCE IDENTIFIER: |
|---|---|---|---|
| | | 61 ACCCAGATTGCCATCTGCCCCAACAACCATGAGGTGCATATCTATGAAAAGAGCGGTGCC<br><br>41 K W T K V H E L K E H N G Q V T G I D W<br>121 AAATGGACCAAGGTGCACGAGCTCAAGGAGCACAACGGGCAGGTGACAGGCATCGACTGG<br><br>61 A P E S N R I V T C G T D R N A Y V W T<br>181 GCCCCCGAGAGTAACCGTATTGTGACCTGCGGCACAGACCGCAACGCCTACGTGTGGACG<br><br>81 L K G R T W K P T L V I L R I N R A A R<br>241 CTGAAGGGCCGCACATGGAAGCCCACGCTGGTCATCCTGCGGATCAACCGGGCTGCCCGC<br><br>101 C V R W A P N E N K F A V G S G S R V I<br>301 TGCGTGCGCTGGGCCCCCAACGAGAACAAGTTTGCTGTGGGCAGCGGCTCTCGTGTGATC<br><br>121 S I C Y F E Q E N D W W V C K H I K K P<br>361 TCCATCTGTTATTTCGAGCAGGAGAATGACTGGTGGGTTTGCAAGCACATCAAGAAGCCC<br><br>141 I R S T V L S L D W H P N N V L L A A G<br>421 ATCCGCTCCACCGTCCTCAGCCTGGACTGGCACCCCAACAATGTGCTGCTGGCTGCCGGC<br><br>161 S C D F K C R I F S A Y I K E V E E R P<br>481 TCCTGTGACTTCAAGTGTCGGATCTTTTCAGCCTACATCAAGGAGGTGGAGGAACGGCCG<br><br>181 A P T P W G S K M P F G E L M F E S S S<br>541 GCACCCACCCCGTGGGGCTCCAAGATGCCCTTTGGGGAACTGATGTTCGAATCCAGCAGT<br><br>201 S C G W V H G V C F S A S G S R V A W V<br>601 AGCTGCGGCTGGGTACATGGCGTCTGTTTCTCAGCCAGCGGGAGCCGCGTGGCCTGGGTA<br><br>221 S H D S T V C L A D A D K K M A V A T L<br>661 AGCCACGACAGCACCGTCTGCCTGGCTGATGCCGACAAGAAGATGGCCGTCGCGACTCTG<br><br>241 A S E T L P L L A L T F I T D N S L V A<br>721 GCCTCTGAAACACTACCACTGCTGGCGCTGACCTTCATCACAGACAACAGCCTGGTGGCA<br><br>261 A G H D C F P V L F T Y D A A A G M L S<br>781 GCGGGCCACGACTGCTTCCCGGTGCTGTTCACCTATGACGCCGCCGCGGGGATGCTGAGC<br><br>281 F G G R L D V P K Q S S Q R G L T A R E<br>841 TTCGGCGGGCGGCTGGACGTTCCTAAGCAGAGCTCGCAGCGTGGCTTGACGGCCCGCGAG<br><br>301 R F Q N L D K K A S S E G G T A A G A G<br>901 CGCTTCCAGAACCTGGACAAGAAGGCGAGCTCCGAGGGTGGCACGGCTGCGGGCGCGGGC | |

| Gene Name | GenBank Homology | DNA SEQUENCE / DEDUCED AMINO ACID SEQUENCE | SEQUENCE IDENTIFIER: |
|---|---|---|---|
| | | 321 L D S L H K N S V S Q I S V L S G G K A<br>961 CTAGACTCGCTGCACAAGAACAGCGTCAGCCAGATCTCGGTGCTCAGCGGCGGCAAGGCC<br><br>341 K C S Q F C T T G M D G G M S I W D V K<br>1021 AAGTGCTCGCAGTTCTGCACCACTGGCATGGATGGCGGCATGAGTATCTGGGATGTGAAG<br><br>361 S L E S A L K D L K I K -<br>1081 AGCTTGGAGTCAGCCTTGAAGGACCTCAAGATCAAATGA | |
| B1961438 | No homology | 1 GCACGAGGCAGCCAGCAGCTTTGGAGCCCCCACAACCTGGACACTCACATCGAAGTCTCT<br>61 GCCTAGCCACCCTCTCCCTGAGCCCATCTCAGATGCCCCTCCCCCCAGGCTGCCAGGTG<br>121 GCTGGGGTGAGGTCAGGTGGCAGGCCCAGAATAGGCCCAGTCATCCACTCCCCTCCCTCT<br>181 CAGGATTGCAGGAGTCTGAGCTATTCCCGGCCACCTCTATCTCCCACCCCACCTTCTCCA<br>241 CCATAGCCAGGGGCTGGGCCCCGTAGGTGAAACAAGCGGGTGAAACCCCTCTGGGGCCCA<br>301 GACATGCAAACCTCTGGCACCACTGGGTCCTGGCTGAACCCCTGGGTGGTGTGCAGCTTC<br>361 CCCTCTGGTCTCAGTTTCCTTTCCTGAAAAGCGAGGAGTTGGAGGCAACGTTCTCCTCCT<br>421 GGCCTGTGGCCCTCAGAGGAGAAGGACTGGAAGGAACTTCAGAGAATCCTCACTCCCCTC<br>481 ATTTTACAGATGAGGAAACT | SEQ ID NO:214 |
| | | 1 CCTTTCCTGAAAAGCGAGGAGTTGGAGGCAACGTTCTCCTCCTGGCCTGTGGCCCTCAGA<br>61 GGAGAAGGACTGGAAGGAACTTCAGAGAATCCTCACTCCCCTCATTTTACAGATGAGGAA<br>121 ACTGAGGCCCAGAGTGGGCAGAGACTTGGCATCATTGTCATCCAGCAAATAACAGAAGGG<br>181 AAGTTCCTTGGGGAAGAACCAGGAGCAGAGGTCTGGGGAGAGGTGGGCAAGGAGGGGGTC<br>241 AGTCTCCCCTAGTCCAGAAAAGGGCCTCACCTGCCAGGGGCCTCCAGTCTCTGGCCCTCT<br>301 GTCCTGCGCATTGCTTTCCAAGGAGCCTTCTTGAGGTCACTGCATTTGATCTTCATGGCG<br>361 GCACCTGGAAGAGAGACATGAGCGTTTGTGTTTGCTTTAAAAAAAGCATTTTTCATTTTA<br>421 AAAAATCAAGGTGTGCTTTGTAGACACTTTGGAAAGTTCAGGAGAGTAATCCAGGGTGGA<br>481 GTCTATGCAGAAATGACCCATAATCCAGCAACGCCAGGCCCCTGAGTGGTGGCTTCTATG<br>541 TAGCTGTAATCGTACTATACAT | SEQ ID NO:215 |
| B1961499 | Homo sapiens SUI1 isolog | 1 cggcacgagc gccgccgagg attcagcagc ctcccccttg agccccctcg cttcccgacg<br>61 ttccgttccc ccctgcccgc cttctcccgc caccgccgcc gccgccttcc gcaggccgtt<br>121 tccaccgagg aaaaggaatc gtatcgtatg tccgctatcc agaacctcca ctctttcgac<br>181 ccctttgctg atgcaagtaa gggtgatgac ctgcttcctg ctggcactga ggattatatc<br>241 catataagaa ttcaacagag aaacggcagg aagaccctta ctactgtcca agggatcgct<br>301 gatgattacg ataaaaagaa actagtgaag gcgtttaaga aaaagtttgc ctgcaatggt<br>361 actgtaattg agcatccgga atatggagaa gtaattcagc tacagggtga ccaacgcaag<br>421 aacatatgcc agttcctcgt agagattgga ctggctaagg acgatcagct gaaggttcat | SEQ ID NO:216 |

| Gene Name | GenBank Homology | DNA SEQUENCE / DEDUCED AMINO ACID SEQUENCE | SEQUENCE IDENTIFIER: |
|---|---|---|---|
| | | 481 gggttttaag tgcttgtggc tcactgaagc ttaagtgagg atttccttgc aatgagtaga<br>541 atttcccttc tctcccttgt cacaggttta aaaacctcac agcttgtata atgtaaccat<br>601 ttggggtccg cttttaactt ggactagtgt aactccttca tgcaataaac tgaaaagagc<br>661 catgctgtct agtcttgaag tccctcattt aaacagaggt caagcaatag gcgcctggca<br>721 gtgtcaagcc tgaaaccaag caataccgtc atgtttcagc caagcccaga gccctaagat<br>781 tacaaacaac tatggccgga acctcctcag ctctccctct gcagagttcc ctaccctaag<br>841 agaatgttac cacctgaaca gtcctcggtg aatctgagag gagaggatgg ggtaaggcag<br>901 aagcaccagc tgtactacta gaagggagct tttggtggta gatcccctgg tgtctccaac<br>961 ctgactaggt ggacagagct caaagaggcc ctcttaccgc tagcgaggtg ataggacatc<br>1021 tggcttgcca caaaggtctg ttcgaccaga catatcctag ctaagggatg tccaaacatc<br>1081 agaatgtgag gccaaccttc tatcagagtt aaacttttga caagggaaca aatctcaaac<br>1141 tgatccatca gtcatgtagc tagctgtaga gcttgcaact taatagcagc agctgcccaa<br>1201 tgccatgtga agtaacaaac tggtttttgg ttttttttc cccttcagtt ttaatgttat<br>1261 gtgtaatgta tttaaaccct tatttaaata aaacttgttt tcagaaaaaa aaaaaaaaaa<br>1321 aaaa | |
| | | 1 M S A I Q N L H S F D P F A D A S K G D<br>1 ATGTCCGCTATCCAGAACCTCCACTCTTTCGACCCCTTTGCTGATGCAAGTAAGGGTGAT<br><br>21 D L L P A G T E D Y I H I R I Q Q R N G<br>61 GACCTGCTTCCTGCTGGCACTGAGGATTATATCCATATAAGAATTCAACAGAGAAACGGC<br><br>41 R K T L T T V Q G I A D D Y D K K K L V<br>121 AGGAAGACCCTTACTACTGTCCAAGGGATCGCTGATGATTACGATAAAAAGAAACTAGTG<br><br>61 K A F K K K F A C N G T V I E H P E Y G<br>181 AAGGCGTTTAAGAAAAAGTTTGCCTGCAATGGTACTGTAATTGAGCATCCGGAATATGGA<br><br>81 E V I Q L Q G D Q R K N I C Q F L V E I<br>241 GAAGTAATTCAGCTACAGGGTGACCAACGCAAGAACATATGCCAGTTCCTCGTAGAGATT<br><br>101 G L A K D D Q L K V H G F -<br>301 GGACTGGCTAAGGACGATCAGCTGAAGGTTCATGGGTTTTAA | SEQ ID NO:217 |
| B1961539 | Human mRNA for calcium-binding protein in macrophages (MRP-14) macrophage migration inhibitory factor (MIF)-related protein. | 1 AAAACACTCTGTGTGGCTCCTCGGCTTTGACAGAGTGCAAGACGATGACTTGCAAAATGT<br>61 CCCAGATGGAGCGCGACATAGAGACCATCATCAATGTCTTCCACCAGTACTCTGTGCGAC<br>121 TGGGGCACCCGGACACCCTGAACCGGAAGGAATTCAAACAGCTGGTTCAAAAAGAGCTGG<br>181 CGAACTTCCTCAAGAGTAAGAAGAAGGATGAGAAAGCCATAAATCATATCATGGAGGACC<br>241 TGGATACCAATGAAGACAAGCAGCTGAGTTTCGAGGAGTTCATCATCCTGGTGGCCAGGC<br>301 TGACGCATGCCTCCCATGAGAAGATGCACGAGCATGACCAAGGCCACGGCCACTGCCATG<br>361 GGCCAGGCCTTGGGGAGGGCACCCCCTAAGACCACAGTGGCCAAGATCACAGTGGCCACG<br>421 GCCACGGCCACAGTCATGGTGGCCACGGCCACAGCCACCCAT | SEQ ID NO:218 |

| Gene Name | GenBank Homology | DNA SEQUENCE / DEDUCED AMINO ACID SEQUENCE | SEQUENCE IDENTIFIER: |
|---|---|---|---|
| | | 1 M T C K M S Q M E R D I E T I I N V F H<br>1 ATGACTTGCAAAATGTCCCAGATGGAGCGCGACATAGAGACCATCATCAATGTCTTCCAC<br><br>21 Q Y S V R L G H P D T L N R K E F K Q L<br>61 CAGTACTCTGTGCGACTGGGGCACCCGGACACCCTGAACCGGAAGGAATTCAAACAGCTG<br><br>41 V Q K E L A N F L K S K K K D E K A I N<br>121 GTTCAAAAAGAGCTGGCGAACTTCCTCAAGAGTAAGAAGAAGGATGAGAAAGCCATAAAT<br><br>61 H I M E D L D T N E D K Q L S F E E F I<br>181 CATATCATGGAGGACCTGGATACCAATGAAGACAAGCAGCTGAGTTTCGAGGAGTTCATC<br><br>81 I L V A R L T H A S H E K M H E H D Q G<br>241 ATCCTGGTGGCCAGGCTGACGCATGCCTCCCATGAGAAGATGCACGAGCATGACCAAGGC<br><br>101 H G H C H G P G L G E G T P -<br>301 CACGGCCACTGCCATGGGCCAGGCCTTGGGGAGGGCACCCCCTAA | SEQ ID NO:219 |
| B1961620 | ILT11A mRNA for immunoglobulin-like transcript 11 protein | 1 cacggctgga gaccatggga agcagtgccg ggacaccttc cctcattgcc tttcagtgac<br>61 ttgggctgag tatggacccg aggaccccag tgcaggcaga cactctcccc aaacccacca<br>121 tctgggctga gccaggctct gtgatccctc gggggaagcc tgtggccatc aggtgtcaag<br>181 ggacactgga gacccaagag tatcgtctgc ataaagagcg aaatccagtg ccttgggacc<br>241 aaaagagccc tctggagccc ggggaaaagg ccaatttctc catcccacac atgacagaga<br>301 aatattcagg gacatatcac tgttactatc tcggccccac tggctggtca gagcacagtg<br>361 accccctcga gctggtggtg acaggattct acaccaaacc caccctctca gccctgccga<br>421 gcccactggt gatctcagga gggaatgtga ccctccagtg tggctcaaag cttggatttg<br>481 ccaggtttgt tctcactaag gaaggagaaa acaagacctc ctggaccctg gattcacagc<br>541 gacaacccaa tgggcagttc caggccctgt tccctgtggg ccctgtgatc cccttccaca<br>601 agtggatgtt cagctgctat ggcttttaca ggaactaccc tcacgtgtgg tcacatccta<br>661 gtgacccctt ggagctcctg gtctcagcct cacaccctca agattacaca gtggagaatc<br>721 tcatccggat gagtgcagct ggcttgatcc tggtggtcct tgggattctg ctatttcagg<br>781 ctcaacacag ccagaaaaga ccccaagagg cagacaggaa gtaaacacaa gtgagaaaaa<br>841 tgcacagttc agagtagtag agccttgaaa gtaaatcttg tgcacccagg aggttcctga<br>901 agagcatctg tggtgtatga tcagtgaact gtctgcaggt cattgtgagg tgtagtagtt<br>961 atcgttcatg tgcagggaca atgtccgctc ctttctgcca ttaaactgag ggacattcct<br>1021 tcacaagcac ttttgagtta tcttggtggg tttgtaactt tctctctttt tgtgaaatca<br>1081 gcatgtatcc caaaggacag atttgggtcc acacttcctt gcactttcat gctgtggtac<br>1141 aatttcatgt aatacatata tttttaaatt | SEQ ID NO:220 |
| | | 1 M D P R T P V Q A G F Y T K P T L S A L | SEQ ID NO:221 |

| Gene Name | GenBank Homology | DNA SEQUENCE / DEDUCED AMINO ACID SEQUENCE | SEQUENCE IDENTIFIER: |
|---|---|---|---|
| | | 1 ATGGACCCGAGGACCCCAGTGCAGGCAGGATTCTACACCAAACCCACCCTCTCAGCCCTG<br><br>21 P S P L V I S G G N V T L Q C G S K L G<br>61 CCGAGCCCACTGGTGATCTCAGGAGGGAATGTGACCCTCCAGTGTGGCTCAAAGCTTGGA<br><br>41 F A R F V L T K E G E N K T S W T L D S<br>121 TTTGCCAGGTTTGTTCTCACTAAGGAAGGAGAAAACAAGACCTCCTGGACCCTGGATTCA<br><br>61 Q R Q P N G Q F Q A L F P V G P V I P F<br>181 CAGCGACAACCCAATGGGCAGTTCCAGGCCCTGTTCCCTGTGGGCCCTGTGATCCCCTTC<br><br>81 H K W M F S C Y G F Y R N Y P H V W S H<br>241 CACAAGTGGATGTTCAGCTGCTATGGCTTTTACAGGAACTACCCTCACGTGTGGTCACAT<br><br>101 P S D P L E L L V S A S H P Q D Y T V E<br>301 CCTAGTGACCCCTTGGAGCTCCTGGTCTCAGCCTCACACCCTCAAGATTACACAGTGGAG<br><br>121 N L I R M S A A G L I L V V L G I L L F<br>361 AATCTCATCCGGATGAGTGCAGCTGGCTTGATCCTGGTGGTCCTTGGGATTCTGCTATTT<br><br>141 Q A Q H S Q K R P Q E A D R K -<br>421 CAGGCTCAACACAGCCAGAAAAGACCCCAAGAGGCAGACAGGAAGTAA | |
| B1961856 | No Homology | 1 GCACGAGGGAAGAGGGTCTCCTTCCACCCCAAGGAATTTTTTTAATCAGAAGACCTTGA<br>61 AAAGGGGGATATAACTTTGAACAATTTAATTTCTTGGCCGTGTTTAGTAACAGTTCCTAT<br>121 GCATGGTTTTTAACGTATAAATTTGCTTTCTTCCCTCTCCCACCTGATTCTGCCTCTTTA<br>181 ATGAATATTTTTACTGGGGTTGAAACTCAATGCCTTATGTGCTCACTCAGTTTCCCTTCT<br>241 GCAGCTTGTTTTTTCTCACTATCTGTATGTGTTCAGATTGTGTCAAATGCTGGCAAAACC<br>301 TCTAAGACTGTCAAGAAAGGCTTGAAAGTTGATTTGTCCTAGTGCAAATTCAAGATAAAT<br>361 GTCTTAATGTTTTTTGGCTATGTAGTACATAGGAACAGAAAATAACTTAAATAAAGTCAT<br>421 TTTTGTAATTTAAAAAAAAAAAAAAAAAAA | SEQ ID NO:222 |
| | | 1 GCACGAGGGAAGAGGGTCTCCTTCCACCCCAAGGAATTTTTTTAATCAGAAGACCTTGA<br>61 AAAGGGGGATATAACTTTGAACAATTTAATTTCTTGGCCGTGTTTAGTAACAGTTCCTAT<br>121 GCATGGTTTTTAACGTATAAATTTGCTTTCTTCCCTCTCCCACCTGATTCTGCCTCTTTA<br>181 ATGAATATTTTTACTGGGGTTGAAACTCAATGCCTTATGTGCTCACTCAGTTTCCCTTCT<br>241 GCAGCTTGTTTTTTCTCACTATCTGTATGTGTTCAGATTGTGTCAAATGCTGGCAAAACC<br>301 TCTAAGACTGTCAAGAAAGGCTTGAAAGTTGATTTGTCCTAGTGCAAATTCAAGATAAAT<br>361 GTCTTAATGTTTTTTGGCTATGTAGTACATAGGAACAGAAAATAACTT | SEQ ID NO:223 |
| B1961872 | No homology | 1 GCACGAGCCGAGCCCTGAGCAAAACCCTCCCCATCCGCTGTATTTATCTCTATTTAATAT | SEQ ID NO:224 |

| Gene Name | GenBank Homology | DNA SEQUENCE / DEDUCED AMINO ACID SEQUENCE | SEQUENCE IDENTIFIER: |
|---|---|---|---|
| | | 61 TTATGCTCTGTGTATAAGGATGAATTAGAGTTGTCATTTTCTCTTCACTGGATGTTTATT<br>121 TATAAAGATTTGACCTGTTCATGCGTCTGTGGAGCAGCCCTCCGTCTCCCGGCTATATAG<br>181 TAATCTTAGGTAGAGTGTTGCCTTGTGGGTTACCGTTTGCTCTGAGACTTCTCGGATGGA<br>241 CCCACCCTCCTCCAGCCCTGGCTGCCAGCGCCGCGGGGCCGCGTGCTGGGCCTGCCGGGG<br>301 CCTGGCGCCGAGCCGTGTCCAATAAAGTTCTCGGATGTGACTGGAAAAAAAAAAAAAAAA<br>361 AA | |
| B1961880 | Equus caballus tissue inhibitor of metalloproteinase-1 (TIMP-1) mRNA, complete cds | 1 aggcagagca cccaccatgg ccccctttgc accccctggtc tccggcattc tgttgttgct<br>61 gtggctcaca gcccccagca gggcctgtac gtgtgtccca ccccacccgc agacggcctt<br>121 ctgcagctct gagttcgtca tcagggccaa gttcgtgggg acctcagaag tcaaccagac<br>181 caccttacag cggcgttatg agatcaagat gaccaagatg ttcaaagggt tcagcgcctt<br>241 gggggatgcc cctgacacct ggtttgtcta caccccccgct atggagagcc tctgcggata<br>301 cttccacagg tcggagaacc gcagcgagga gtttctcatc gccggacaac tattggacga<br>361 gaagctgtac atcaccacct gcagtttcgt ggctccctgg aacagtctga gctccgctca<br>421 gcgccagggc ttcaccaaga cctacgccgc cggctgtggg gaatgctcag tgtttccctg<br>481 ttcatccatc ccctgcaaac tgcagagtga cactgattgc ttgtggacgg accagctcct<br>541 cacaggctct gacaagggct tccagagccg ctacctcgcc tgcctgcccc gggagccagg<br>601 gctgtgcacc tggcagtccc ttcggccccg gacggcctga atcctgtctc cagcagaagc<br>661 tgaagcttgc acagtgttca ccctctttcc cattcctatc tttctttctc caagacagtg<br>721 aaataaagaa ctaccaccca aa | SEQ ID NO:225 |
| | | 1 M A P F A P L V S G I L L L L W L T A P<br>1 ATGGCCCCCTTTGCACCCCTGGTCTCCGGCATTCTGTTGTTGCTGTGGCTCACAGCCCCC<br><br>21 S R A C T C V P P H P Q T A F C S S E F<br>61 AGCAGGGCCTGTACGTGTGTCCCACCCCACCCGCAGACGGCCTTCTGCAGCTCTGAGTTC<br><br>41 V I R A K F V G T S E V N Q T T L Q R R<br>121 GTCATCAGGGCCAAGTTCGTGGGGACCTCAGAAGTCAACCAGACCACCTTACAGCGGCGT<br><br>61 Y E I K M T K M F K G F S A L G D A P D<br>181 TATGAGATCAAGATGACCAAGATGTTCAAAGGGTTCAGCGCCTTGGGGGATGCCCCTGAC<br><br>81 T W F V Y T P A M E S L C G Y F H R S E<br>241 ACCTGGTTTGTCTACACCCCCGCTATGGAGAGCCTCTGCGGATACTTCCACAGGTCGGAG<br><br>101 N R S E E F L I A G Q L L D E K L Y I T<br>301 AACCGCAGCGAGGAGTTTCTCATCGCCGGACAACTATTGGACGAGAAGCTGTACATCACC<br><br>121 T C S F V A P W N S L S S A Q R Q G F T<br>361 ACCTGCAGTTTCGTGGCTCCCTGGAACAGTCTGAGCTCCGCTCAGCGCCAGGGCTTCACC | SEQ ID NO:226 |

| Gene Name | GenBank Homology | DNA SEQUENCE / DEDUCED AMINO ACID SEQUENCE | SEQUENCE IDENTIFIER: |
|---|---|---|---|
| | | 141 K T Y A A G C G E C S V F P C S S I P C<br>421 AAGACCTACGCCGCCGGCTGTGGGGAATGCTCAGTGTTTCCCTGTTCATCCATCCCCTGC<br><br>161 K L Q S D T D C L W T D Q L L T G S D K<br>481 AAACTGCAGAGTGACACTGATTGCTTGTGGACGGACCAGCTCCTCACAGGCTCTGACAAG<br><br>181 G F Q S R Y L A C L P R E P G L C T W Q<br>541 GGCTTCCAGAGCCGCTACCTCGCCTGCCTGCCCCGGGAGCCAGGGCTGTGCACCTGGCAG<br><br>201 S L R P R T A -<br>601 TCCCTTCGGCCCCGGACGGCCTGA | |
| B1961900 | PREDICTED: Homo sapiens zinc finger, SWIM domain containing 6 (ZSWIM6), mRNA | 1 atggcatcgt ggcgtcgcga cggcgtgtgc gtgtcgcgct tcctagtgcc gtttataggg<br>61 tcccggcact tccgctgtcg gacgcggctg cctttccgcc ggggcatcgc gctgttggaa<br>121 agcggctgcg tagacaacgt cctgcaagtc gttccgcgcg cgcccgcacc cctgcgggtg<br>181 tcctcactgg cccggacggt cctcctgagc ggagcgccca tttcctccct cccttccctg<br>241 ccccccgtcg ccccggacgg gccagcgcga gtgggaaatg aatcagcagg acgcgcccct<br>301 ccgtgggctc cgcgcccccg gcccgcgctc ccctacccgc cctcctccgg ggagcacatc<br>361 ctggagggct gttcgccggt ttcgggggtg gatgtggaca aaggcgcggg cggacggccg<br>421 gcctccggcg agggtgtgtg tggcggggct ggctccgacg ggcgagcggc cgcggcgctc<br>481 ccgggtcgct gtggcgtctg gcccgggcga gctccactgg ccgcccgggg tttaaatgtc<br>541 gtttctttg cgcctcctca gtctccaagc ggcgcagagg agaggtggcg gcggaccgga<br>601 ctccgtctcc ggagggacat ctcgcccgta ggacgcttac taattaaagg gcgcgagcct<br>661 gaggaggagc tgctagcgct tccctttctt ctgaagctgc ctccagacac ccctgtgcgg<br>721 gccgcggcgc agcccttgtg ccccgccgcc cgagttttgt ttgcgctgtg tggtcaggtc<br>781 gtggtgcccc gggcgagccg cggtcggcag ttgcggattt gccgctcgca gccaaagggg<br>841 cgagcgggct gcaggagcgc gaggcagagg gctcggcggc cgctcccgcg tccccgcggc<br>901 gcggcagccc ggcacgaccc agcactttgt tctcattttc aggaaactct tctgtttgct<br>961 gatgaggaaa ggttatcttt ccaggcagca tccaggaatg gtcttttgaa tgtcttcatg<br>1021 ccttcttgtg acctcattcc tgctgccatc gtacttatcc ctgctcagat taactgcctg<br>1081 cgtgttcaac tttattccat agttggatct acctgtctgc tggagatctt cattggttct<br>1141 gctgctttgg agccaccaca gatgatgatg gagcctacag ttatctgttg ctgctgttgg<br>1201 agggtaagtc tctccggccc ggtttccctc ggtgtgctac tgtgcgcgcg atccagcacc<br>1261 atggggaagc gggacaatcg ggtggcctat atgaacccaa tagcaatggc gagatcaagg<br>1321 ggtccaatcc agtcttcagg gccaacaata caggattatc tgaatcgacc aaggcctacc<br>1381 tgggaagaag taaaagagca actagaaaag gaaaagaaag gctccaaggc tttggctgaa<br>1441 tttgaagaaa aaatgaatga gaactggaag aaagaactgg aaaaacacag agagaaattg<br>1501 ttaagtggaa gtgagagctc atccaaaaaa agacagagaa agaaaaaaga aaagaagaaa<br>1561 tctggtaggt attcatcttc ttcttcatca agctctgatt cttccagcag ttcttctgat<br>1621 tctgaagatg aggataagaa acaaggaaaa cagagaaaga aaaagaagaa ccgttcacat<br>1681 aaatcttctg aaagctccat gtcagaaact gaatcagaca gtaaggatag tttaaaaaag | SEQ ID NO:227 |

| Gene Name | GenBank Homology | DNA SEQUENCE / DEDUCED AMINO ACID SEQUENCE | SEQUENCE IDENTIFIER: |
|---|---|---|---|
| | | 1741 aaaaagaagt caaaagatgg aactgagaaa gaaaaggata ttaaaggact cagcaaaaag<br>1801 agaaagatgt attctgaaga taaaccttta tcatctgagt ccttgtcaga atcagagtat<br>1861 attgaggagc tgcttatcat gggcacagct gacaaaacca gaagagcctt tctatggtgc<br>1921 catgaatgct gtcttctcct ccatggtctg gaagcagcca tggccaaact tggaggtggt<br>1981 gtcaatgaac ttaaggtcaa tcttctccag aggccgccgt ttggttggca ctggcaagga<br>2041 cttgtgaagg ccattgccct tggtcaacct gatgacgtcg atcatcttgt cctgctcaaa<br>2101 cacttggttc acaggtacct gctgctcaag cctctcgtgg gcccagtcca gcttcttggc<br>2161 catggtgcct ccctttacct ggatctccat caggtgggcc ttcctctggt gcagaggaag<br>2221 caggcacatc tgggtgtagg caatgatgca gatgacttgg cggtacttct tcatgctgct<br>2281 gaagtccttc tccagctgct tcttgccatc ctcatcctga catttcttgc agtacttggt<br>2341 aaaggccttc ttcttagatt tatgccggtt tttatagaaa tgtctcttgc atgcatcgct<br>2401 gatgtgctca gcgaagacag tattgaaggt ctggaggcct cgaggggttt ccacgaagcc<br>2461 cacaatggcc acaaccacca tgggtggtat ctccacaatg gtcacagcct ctatcacttc<br>2521 cttcttgttc accttggatc ctggcctgtc gacttcccgc atgatgttgg tcatgccagc<br>2581 cttgtatccc aggaaggctg tgaggtggac cagcttggaa gggtcatcct taggaaagct<br>2641 cttcaccttc ccatgatgct tcctgctgcg ctgctgaggc aggaagccga gggacccatg<br>2701 tctgggagcg gagaaccttc tctgagggct ttcgaagctt cccaccagtg ggtttgtctg<br>2761 aactgtttag gaatgcggcc gcggtcgcca gctgacgtca gcgccgccgg ttcagccgca<br>2821 ggccagagtc agagactgac agcgcgaggg gcggggttgg gagtcaggat ggggcgaggc<br>2881 ctgcgtgtga ctgatggcgc gggggcgggg cggcgtttcc acttgagcgg cacagtgaca<br>2941 gaacctgcaa tacaatcgga gccagaaact gtttgcaacg tggccatcag ctttgatcgt<br>3001 tgcaagatta cctcagtgac ctgcagctgt ggaaacaagg acatatttta ttgtgcccat<br>3061 gttgtggcac tgtctttata ccgcatccgc aagccagatc aggtcaaact gcatcttcct<br>3121 atttcagaga ctctctttca aatgaataga gaccaactgc aaaagtttgt acagtatttg<br>3181 atcacagtgc accacacaga agttttgcca actgctcaaa aattagcaga tgaaattctt<br>3241 tcccaaaatt cagaaatcaa ccaagttcat ggtgctcctg atccaacagc aggtgctagt<br>3301 atagatgatg aaaactgctg gcacttagat gaagagcagg ttcaagaaca ggttaaactg<br>3361 ttcctttccc agggcgggta ccacggatca gggaagcagc ttaatttgct ctttgcaaag<br>3421 gtgcgggaga tgttaaagat gagggactcc aatggggccc gcatgttgac cttgataaca<br>3481 gagcaattca tggctgaccc tcgcctgtca ctttggcggc aacaaggcac tgcaatgact<br>3541 gacaaataca ggcagctctg ggatgagctg ggtgctctgt ggatgtgtat agttttaaac<br>3601 ccccactgca agttggagca aaaggccagt tggctaaaac agctgaagaa atggaatagt<br>3661 gttgatgtct gtccatggga agatggaaat catggcagtg aattacccaa cttaaccaat<br>3721 gctctgcctc agggtgcaaa tgccaaccaa gattcatcga acaggccaca tcggacagtg<br>3781 ttcacccgag ccatcgaggc atgcgatctc cactggcagg atagccactt gcagcacatt<br>3841 atcagcagtg acctatacac caactactgt taccatgacg acactgaaaa ctccctcttc<br>3901 gactcccgcg ggtggcccct ctggcatgaa catgttccta cagcctgtgc aagagtggac<br>3961 gcattacgtt ctcatgggta ccccagagaa gcactgagac tagcaatagc tattgttaat<br>4021 acattaagac gacagcagca gaaacagttg gaaatgttcc gaacccaaaa aaaagagcta<br>4081 ccccataaaa acataacctc gataaccaat ctggagggct gggttggaca tcccctggac<br>4141 cctgtgggca ctctcttcag tagccttatg gaagcctgcc gcattgatga tgagaacctc<br>4201 tctgggttct cagattttac agagaatatg ggacagtgca agtctctgga ataccagcat<br>4261 ctacctgcac acaaattctt agaagaaggg gaatcctatt taacgctggc tgtggaagta | |

| Gene Name | GenBank Homology | DNA SEQUENCE / DEDUCED AMINO ACID SEQUENCE | SEQUENCE IDENTIFIER: |
|---|---|---|---|
| | | 4321 gccctgatag ggctaggaca gcagcgtatc atgcctgatg ggctgtacac acaagagaaa<br>4381 gtttgccgga atgaggagca gctcatttct aagcttcagg aaattgaatt ggatgacaca<br>4441 ctggtgaaaa tttttcgcaa gcaagcagtc ttcctattag aagccggacc atatagtggt<br>4501 ttaggtgaaa taatccatcg ggagagcgtt ccaatgcaca catttgccaa gtatctcttc<br>4561 acctctctcc tacctcacga tgctgaattg gcatacaaaa ttgcactgag agcaatgcgg<br>4621 ttactagtat tggaatctac tgctccatca ggagacctca cccgcccaca ccacattgca<br>4681 tcagttgttc ccaaccgcta ccctcgctgg ttcactctaa gccacattga gtcccagcag<br>4741 tgtgagctgg catccaccat gctaactgca gccaaaggcg atgttcggag gctggaaaca<br>4801 gtattagaat ccatccagaa aaacattcac tcctcatcac acatcttcaa gcttgcccaa<br>4861 gatgcattta aaatagcaac tctcatggac agtttgccag acatcactct tttgaaagtg<br>4921 tctctggagc tgggcctgca ggttatgcga atgacactgt caaccttaaa ttggcgacgg<br>4981 cgggagatgg tgaggtggct ggtaacgtgt gctactgaag tcggggttta tgccctggac<br>5041 agcatcatgc agacctggtt tacactcttt actcccaccg aggccacaag tatagttgca<br>5101 actaccgtga tgtccaacag caccatcgtc cgcctccacc tggactgcca ccagcaggaa<br>5161 aagctggcca gcagcgcccg gacacttgca ctgcagtgtg ccatgaagga tccacagaac<br>5221 tgtgccctct ctgcgctaac cctttgtgaa aaggatcaca tagcttttga gacggcgtac<br>5281 caaattgttc tcgacgctgc tacgactggc atgagctata cacagctctt tacaatagca<br>5341 cggtacatgg agcaccgcgg gtaccccatg agggcctaca agctggccac cctggccatg<br>5401 acccatctca acctgagcta caatcaggac acacaccctg ccattaatga tgttttgtgg<br>5461 gcctgtgcgc ttagccactc ccttggtaaa aatgagcttg cagctataat acctctggtg<br>5521 gtcaagagtg tcaagtgtgc aacggtactg tcagacattt tgcgcagatg cactctgacc<br>5581 actcctggca tggtgggact tcatgggagg aggaactctg gtaagctcat gtcactggac<br>5641 aaagcccccct tgaggcaact cttggatgcc acgatcgggg cctacatcaa cacaacgcac<br>5701 tcacggctca cacacatcag tcctcggcac tatagtgagt ttatagagtt cctcagcaaa<br>5761 gcccgagaga ccttcttaat ggcgcatgat ggacacattc agtttacaca gtttattgac<br>5821 aacctgaaac aaatctacaa aggcaaaaag aaactgatga tgttggttcg ggagaggttt<br>5881 ggttgataga tcttgtatga atggggtggg gggtggggat gggagggatg gtttgttttt<br>5941 acttgagcct gcctttgtac cctttttaac ttaaagaaca gagccacacc ggtattatat<br>6001 gtgtatagtt atattgcgtt tgcagactaa attgtcatgt tgtgaaagtt tgtgtgtttt<br>6061 ttattttttc cctatttctt tctttccttt atttttattat ttttttttaat tttttttttc<br>6121 tggttttgta tgagagagag gttaaaaagg tttggtttac actgagtata tgttgtcaag<br>6181 tggcaaaagt ccacatagct ctcctgtttt ctgtatacgt tcacagcctc aaaaaaaata<br>6241 attgaaatgg ctttaaaaac caaacaaaac acctccatcc tgtgataagt acctcgaatg<br>6301 gattcagctt tactcctttg taactcatct ttacattttc agcatattta aacaaaccaa<br>6361 caaaatgaaa tactaatagt aaaaaggctg acccatgtgg ctttgcagtg ctgttcgtcc<br>6421 agaagcatgg cacacgatgc ttgtgcatgt ggaaacttag cgactgtcaa catacattct<br>6481 cagggattta tccaaaaaaa ttaaaaaaag aatgagagca tttattgtac tgtatatata<br>6541 ttatagtata tgtctgaata ttgaaaatat aacattaact aatttataaa aaatattcta<br>6601 tgtaatgcaa aatacttgaa gctgcagtag cttggtttta aacaaaaaca aaaaaaaaac<br>6661 tgagagaaaa cctatcagaa ggactaaaag tacgccttgc ttcagggttg gctcaggtgg<br>6721 tgaacttcat gctgggcatc tatgcagagc cacctttttgg attgcatggt tggactgaga<br>6781 tctattggga gaaattatat atgtatatat atttatacaa cttatgtata catatatata<br>6841 tgtatacaca cagacacaca cacacaccac caacaaccac cactacacca cacatgctta | |

| Gene Name | GenBank Homology | DNA SEQUENCE / DEDUCED AMINO ACID SEQUENCE | SEQUENCE IDENTIFIER: |
|---|---|---|---|
| | | 6901 taacaggcac tagaataaag agggacaaca aaatacacag ccagagcagc aagccttagc<br>6961 attaagaata tacaatatgc cggaattggg gttcgtgcct cctagcctag gaaacttaaa<br>7021 agaaatatcc ttttgacaca aaacacaaaa tgttttccaa aacaattgac ataatgatac<br>7081 attacgcctt tgcagtgagc taataataag ctaacctttg tgcacaaata acattatata<br>7141 tattatatat ctattctgca taggtatttt gactttgtgc aggacagaaa gttgtgtagg<br>7201 tatgactgtt ctacttttca gttttctttt tttttaatat attttatttt ctctagaaat<br>7261 tactcaaaca aaagcagcct tctatcttgc cttgtcttaa tgctttaaaa taaccaaact<br>7321 ggagatctaa ctaccaaact gttcattata ttattaaata ccaactttgg ttacagtata<br>7381 gtgtctttac tttagctgat ggttctgtaa ccttgtgctt tttaaagcaa tttttatgtt<br>7441 ttggtgcaaa agttgtccag tgtctcttgt tcccttcact agagaacatg cttagaggta<br>7501 tgtttgtagg tatttttgtt tagaagaact atttcatgcg ctccatttta tttattataa<br>7561 taggtaaaaa gaaaaaaaaa ggttgtaatt catcacccat gtaaacatgc tcatgaagtt<br>7621 gaaagtaatt aagatttgat acactgatat caatttattt atgtaactaa atcactgttt<br>7681 tataaacttg ttaatgatca acaattttg ttttgattaa aattagtttt ttgaaagtt<br><br>1 M A S W R R D G V C V S R F L V P F I G<br>1 ATGGCATCGTGGCGTCGCGACGGCGTGTGCGTGTCGCGCTTCCTAGTGCCGTTTATAGGG<br><br>21 S R H F R C R T R L P F R R G I A L L E<br>61 TCCCGGCACTTCCGCTGTCGGACGCGGCTGCCTTTCCGCCGGGGCATCGCGCTGTTGGAA<br><br>41 S G C V D N V L Q V V P R A P A P L R V<br>121 AGCGGCTGCGTAGACAACGTCCTGCAAGTCGTTCCGCGCGCGCCCGCACCCCTGCGGGTG<br><br>61 S S L A R T V L L S G A P I S S L P S L<br>181 TCCTCACTGGCCCGGACGGTCCTCCTGAGCGGAGCGCCCATTTCCTCCCTCCCTTCCCTG<br><br>81 P P V A P D G P A R V G N E S A G R A P<br>241 CCCCCCGTCGCCCCGGACGGGCCAGCGCGAGTGGGAAATGAATCAGCAGGACGCGCCCCT<br><br>101 P W A P R P R P A L P Y P P S S G E H I<br>301 CCGTGGGCTCCGCGCCCCCGGCCCGCGCTCCCCTACCCGCCCTCCTCCGGGGAGCACATC<br><br>121 L E G C S P V S G V D V D K G A G G R P<br>361 CTGGAGGGCTGTTCGCCGGTTTCGGGGGTGGATGTGGACAAAGGCGCGGGCGGACGGCCG<br><br>141 A S G E G V C G G A G S D G R A A A A L<br>421 GCCTCCGGCGAGGGTGTGTGTGGCGGGGCTGGCTCCGACGGGCGAGCGGCCGCGGCGCTC<br><br>161 P G R C G V W P G R A P L A A R G L N V<br>481 CCGGGTCGCTGTGGCGTCTGGCCCGGGCGAGCTCCACTGGCCGCCCGGGGTTTAAATGTC<br><br>181 V F F A P P Q S P S G A E E R W R R T G | SEQ ID NO:228 |

| Gene Name | GenBank Homology | DNA SEQUENCE / DEDUCED AMINO ACID SEQUENCE | SEQUENCE IDENTIFIER: |
|---|---|---|---|
| | | 541 GTTTTCTTTGCGCCTCCTCAGTCTCCAAGCGGCGCAGAGGAGAGGTGGCGGCGGACCGGA<br><br>201 L R L R R D I S P V G R L L I K G R E P<br>601 CTCCGTCTCCGGAGGGACATCTCGCCCGTAGGACGCTTACTAATTAAAGGGCGCGAGCCT<br><br>221 E E E L L A L P F S L K L P P D T P V R<br>661 GAGGAGGAGCTGCTAGCGCTTCCCTTTTCTCTGAAGCTGCCTCCAGACACCCCTGTGCGG<br><br>241 A A A Q P L C P A A R V L F A L C G Q V<br>721 GCCGCGGCGCAGCCCTTGTGCCCCGCCGCCCGAGTTTTGTTTGCGCTGTGTGGTCAGGTC<br><br>261 V V P R A S R G R Q L R I C R S Q P K G<br>781 GTGGTGCCCCGGGCGAGCCGCGGTCGGCAGTTGCGGATTTGCCGCTCGCAGCCAAAGGGG<br><br>281 R A G C R S A R Q R A R R P L P R P R G<br>841 CGAGCGGGCTGCAGGAGCGCGAGGCAGAGGGCTCGGCGGCCGCTCCCGCGTCCCCGCGGC<br><br>301 A A A R H D P A L C S H F Q E T L L F A<br>901 GCGGCAGCCCGGCACGACCCAGCACTTTGTTCTCATTTCAGGAAACTCTTCTGTTTGCT<br><br>321 D E E R L S F Q A A S R N G L L N V F M<br>961 GATGAGGAAAGGTTATCTTTCCAGGCAGCATCCAGGAATGGTCTTTTGAATGTCTTCATG<br><br>341 P S C D L I P A A I V L I P A Q I N C L<br>1021 CCTTCTTGTGACCTCATTCCTGCTGCCATCGTACTTATCCCTGCTCAGATTAACTGCCTG<br><br>361 R V Q L Y S I V G S T C L L E I F I G S<br>1081 CGTGTTCAACTTTATTCCATAGTTGGATCTACCTGTCTGCTGGAGATCTTCATTGGTTCT<br><br>381 A A L E P P Q M M M E P T V I C C C C W<br>1141 GCTGCTTTGGAGCCACCACAGATGATGATGGAGCCTACAGTTATCTGTTGCTGCTGTTGG<br><br>401 R V S L S G P V S L G V L L C A R S S T<br>1201 AGGGTAAGTCTCTCCGGCCCGGTTTCCCTCGGTGTGCTACTGTGCGCGCGATCCAGCACC<br><br>421 M G K R D N R V A Y M N P I A M A R S R<br>1261 ATGGGGAAGCGGGACAATCGGGTGGCCTATATGAACCCAATAGCAATGGCGAGATCAAGG<br><br>441 G P I Q S S G P T I Q D Y L N R P R P T<br>1321 GGTCCAATCCAGTCTTCAGGGCCAACAATACAGGATTATCTGAATCGACCAAGGCCTACC<br><br>461 W E E V K E Q L E K E K K G S K A L A E<br>1381 TGGGAAGAAGTAAAAGAGCAACTAGAAAAGGAAAAGAAAGGCTCCAAGGCTTTGGCTGAA | |

| Gene Name | GenBank Homology | DNA SEQUENCE / DEDUCED AMINO ACID SEQUENCE | SEQUENCE IDENTIFIER: |
|---|---|---|---|
| | | 481 F E E K M N E N W K K E L E K H R E K L<br>1441 TTTGAAGAAAAAATGAATGAGAACTGGAAGAAAGAACTGGAAAAACACAGAGAGAAATTG<br><br>501 L S G S E S S S K K R Q R K K K E K K K<br>1501 TTAAGTGGAAGTGAGAGCTCATCCAAAAAAAGACAGAGAAAGAAAAAAGAAAAGAAGAAA<br><br>521 S G R Y S S S S S S S S D S S S S S S D<br>1561 TCTGGTAGGTATTCATCTTCTTCTTCATCAAGCTCTGATTCTTCCAGCAGTTCTTCTGAT<br><br>541 S E D E D K K Q G K Q R K K K K N R S H<br>1621 TCTGAAGATGAGGATAAGAAACAAGGAAAACAGAGAAAGAAAAAGAAGAACCGTTCACAT<br><br>561 K S S E S S M S E T E S D S K D S L K K<br>1681 AAATCTTCTGAAAGCTCCATGTCAGAAACTGAATCAGACAGTAAGGATAGTTTAAAAAAG<br><br>581 K K K S K D G T E K E K D I K G L S K K<br>1741 AAAAAGAAGTCAAAAGATGGAACTGAGAAAGAAAAGGATATTAAAGGACTCAGCAAAAAG<br><br>601 R K M Y S E D K P L S S E S L S E S E Y<br>1801 AGAAAGATGTATTCTGAAGATAAACCTTTATCATCTGAGTCCTTGTCAGAATCAGAGTAT<br><br>621 I E E L L I M G T A D K T R R A F L W C<br>1861 ATTGAGGAGCTGCTTATCATGGGCACAGCTGACAAAACCAGAAGAGCCTTTCTATGGTGC<br><br>641 H E C C L L L H G L E A A M A K L G G G<br>1921 CATGAATGCTGTCTTCTCCTCCATGGTCTGGAAGCAGCCATGGCCAAACTTGGAGGTGGT<br><br>661 V N E L K V N L L Q R P P F G W H W Q G<br>1981 GTCAATGAACTTAAGGTCAATCTTCTCCAGAGGCCGCCGTTTGGTTGGCACTGGCAAGGA<br><br>681 L V K A I A L G Q P D D V D H L V L L K<br>2041 CTTGTGAAGGCCATTGCCCTTGGTCAACCTGATGACGTCGATCATCTTGTCCTGCTCAAA<br><br>701 H L V H R Y L L L K P L V G P V Q L L G<br>2101 CACTTGGTTCACAGGTACCTGCTGCTCAAGCCTCTCGTGGGCCCAGTCCAGCTTCTTGGC<br><br>721 H G A S L Y L D L H Q V G L P L V Q R K<br>2161 CATGGTGCCTCCCTTTACCTGGATCTCCATCAGGTGGGCCTTCCTCTGGTGCAGAGGAAG<br><br>741 Q A H L G V G N D A D D L A V L L H A A<br>2221 CAGGCACATCTGGGTGTAGGCAATGATGCAGATGACTTGGCGGTACTTCTTCATGCTGCT | |

| Gene Name | GenBank Homology | DNA SEQUENCE / DEDUCED AMINO ACID SEQUENCE | SEQUENCE IDENTIFIER: |
|---|---|---|---|
| | | 761 E V L L Q L L L A I L I L T F L A V L G<br>2281 GAAGTCCTTCTCCAGCTGCTTCTTGCCATCCTCATCCTGACATTTCTTGCAGTACTTGGT<br><br>781 K G L L L R F M P V F I E M S L A C I A<br>2341 AAAGGCCTTCTTCTTAGATTTATGCCGGTTTTTATAGAAATGTCTCTTGCATGCATCGCT<br><br>801 D V L S E D S I E G L E A S R G F H E A<br>2401 GATGTGCTCAGCGAAGACAGTATTGAAGGTCTGGAGGCCTCGAGGGGTTTCCACGAAGCC<br><br>821 H N G H N H H G W Y L H N G H S L Y H F<br>2461 CACAATGGCCACAACCACCATGGGTGGTATCTCCACAATGGTCACAGCCTCTATCACTTC<br><br>841 L L V H L G S W P V D F P H D V G H A S<br>2521 CTTCTTGTTCACCTTGGATCCTGGCCTGTCGACTTCCCGCATGATGTTGGTCATGCCAGC<br><br>861 L V S Q E G C E V D Q L G R V I L R K A<br>2581 CTTGTATCCCAGGAAGGCTGTGAGGTGGACCAGCTTGGAAGGGTCATCCTTAGGAAAGCT<br><br>881 L H L P M M L P A A L L R Q E A E G P M<br>2641 CTTCACCTTCCCATGATGCTTCCTGCTGCGCTGCTGAGGCAGGAAGCCGAGGGACCCATG<br><br>901 S G S G E P S L R A F E A S H Q W V C L<br>2701 TCTGGGAGCGGAGAACCTTCTCTGAGGGCTTTCGAAGCTTCCCACCAGTGGGTTTGTCTG<br><br>921 N C L G M R P R S P A D V S A A G S A A<br>2761 AACTGTTTAGGAATGCGGCCGCGGTCGCCAGCTGACGTCAGCGCCGCCGGTTCAGCCGCA<br><br>941 G Q S Q R L T A R G A G L G V R M G R G<br>2821 GGCCAGAGTCAGAGACTGACAGCGCGAGGGGCGGGGTTGGGAGTCAGGATGGGGCGAGGC<br><br>961 L R V T D G A G A G R R F H L S G T V T<br>2881 CTGCGTGTGACTGATGGCGCGGGGGCGGGGCGGCGTTTCCACTTGAGCGGCACAGTGACA<br><br>981 E P A I Q S E P E T V C N V A I S F D R<br>2941 GAACCTGCAATACAATCGGAGCCAGAAACTGTTTGCAACGTGGCCATCAGCTTTGATCGT<br><br>1001 C K I T S V T C S C G N K D I F Y C A H<br>3001 TGCAAGATTACCTCAGTGACCTGCAGCTGTGGAAACAAGGACATATTTTATTGTGCCCAT<br><br>1021 V V A L S L Y R I R K P D Q V K L H L P<br>3061 GTTGTGGCACTGTCTTTATACCGCATCCGCAAGCCAGATCAGGTCAAACTGCATCTTCCT<br><br>1041 I S E T L F Q M N R D Q L Q K F V Q Y L | |

| Gene Name | GenBank Homology | DNA SEQUENCE / DEDUCED AMINO ACID SEQUENCE | SEQUENCE IDENTIFIER: |
|---|---|---|---|
| | | 3121 ATTTCAGAGACTCTCTTTCAAATGAATAGAGACCAACTGCAAAAGTTTGTACAGTATTTG | |
| | | 1061 I T V H H T E V L P T A Q K L A D E I L | |
| | | 3181 ATCACAGTGCACCACACAGAAGTTTTGCCAACTGCTCAAAAATTAGCAGATGAAATTCTT | |
| | | 1081 S Q N S E I N Q V H G A P D P T A G A S | |
| | | 3241 TCCCAAAATTCAGAAATCAACCAAGTTCATGGTGCTCCTGATCCAACAGCAGGTGCTAGT | |
| | | 1101 I D D E N C W H L D E E Q V Q E Q V K L | |
| | | 3301 ATAGATGATGAAAACTGCTGGCACTTAGATGAAGAGCAGGTTCAAGAACAGGTTAAACTG | |
| | | 1121 F L S Q G G Y H G S G K Q L N L L F A K | |
| | | 3361 TTCCTTTCCCAGGGCGGGTACCACGGATCAGGGAAGCAGCTTAATTTGCTCTTTGCAAAG | |
| | | 1141 V R E M L K M R D S N G A R M L T L I T | |
| | | 3421 GTGCGGGAGATGTTAAAGATGAGGGACTCCAATGGGGCCCGCATGTTGACCTTGATAACA | |
| | | 1161 E Q F M A D P R L S L W R Q Q G T A M T | |
| | | 3481 GAGCAATTCATGGCTGACCCTCGCCTGTCACTTTGGCGGCAACAAGGCACTGCAATGACT | |
| | | 1181 D K Y R Q L W D E L G A L W M C I V L N | |
| | | 3541 GACAAATACAGGCAGCTCTGGGATGAGCTGGGTGCTCTGTGGATGTGTATAGTTTTAAAC | |
| | | 1201 P H C K L E Q K A S W L K Q L K K W N S | |
| | | 3601 CCCCACTGCAAGTTGGAGCAAAAGGCCAGTTGGCTAAAACAGCTGAAGAAATGGAATAGT | |
| | | 1221 V D V C P W E D G N H G S E L P N L T N | |
| | | 3661 GTTGATGTCTGTCCATGGGAAGATGGAAATCATGGCAGTGAATTACCCAACTTAACCAAT | |
| | | 1241 A L P Q G A N A N Q D S S N R P H R T V | |
| | | 3721 GCTCTGCCTCAGGGTGCAAATGCCAACCAAGATTCATCGAACAGGCCACATCGGACAGTG | |
| | | 1261 F T R A I E A C D L H W Q D S H L Q H I | |
| | | 3781 TTCACCCGAGCCATCGAGGCATGCGATCTCCACTGGCAGGATAGCCACTTGCAGCACATT | |
| | | 1281 I S S D L Y T N Y C Y H D D T E N S L F | |
| | | 3841 ATCAGCAGTGACCTATACACCAACTACTGTTACCATGACGACACTGAAAACTCCCTCTTC | |
| | | 1301 D S R G W P L W H E H V P T A C A R V D | |
| | | 3901 GACTCCCGCGGGTGGCCCCTCTGGCATGAACATGTTCCTACAGCCTGTGCAAGAGTGGAC | |
| | | 1321 A L R S H G Y P R E A L R L A I A I V N | |
| | | 3961 GCATTACGTTCTCATGGGTACCCCAGAGAAGCACTGAGACTAGCAATAGCTATTGTTAAT | |

| Gene Name | GenBank Homology | DNA SEQUENCE / DEDUCED AMINO ACID SEQUENCE | SEQUENCE IDENTIFIER: |
|---|---|---|---|
| | | 1341 T L R R Q Q Q K Q L E M F R T Q K K E L<br>4021 ACATTAAGACGACAGCAGCAGAAACAGTTGGAAATGTTCCGAACCCAAAAAAAAGAGCTA<br><br>1361 P H K N I T S I T N L E G W V G H P L D<br>4081 CCCCATAAAAACATAACCTCGATAACCAATCTGGAGGGCTGGGTTGGACATCCCCTGGAC<br><br>1381 P V G T L F S S L M E A C R I D D E N L<br>4141 CCTGTGGGCACTCTCTTCAGTAGCCTTATGGAAGCCTGCCGCATTGATGATGAGAACCTC<br><br>1401 S G F S D F T E N M G Q C K S L E Y Q H<br>4201 TCTGGGTTCTCAGATTTTACAGAGAATATGGGACAGTGCAAGTCTCTGGAATACCAGCAT<br><br>1421 L P A H K F L E E G E S Y L T L A V E V<br>4261 CTACCTGCACACAAATTCTTAGAAGAAGGGGAATCCTATTTAACGCTGGCTGTGGAAGTA<br><br>1441 A L I G L G Q Q R I M P D G L Y T Q E K<br>4321 GCCCTGATAGGGCTAGGACAGCAGCGTATCATGCCTGATGGGCTGTACACACAAGAGAAA<br><br>1461 V C R N E E Q L I S K L Q E I E L D D T<br>4381 GTTTGCCGGAATGAGGAGCAGCTCATTTCTAAGCTTCAGGAAATTGAATTGGATGACACA<br><br>1481 L V K I F R K Q A V F L L E A G P Y S G<br>4441 CTGGTGAAAATTTTTCGCAAGCAAGCAGTCTTCCTATTAGAAGCCGGACCATATAGTGGT<br><br>1501 L G E I I H R E S V P M H T F A K Y L F<br>4501 TTAGGTGAAATAATCCATCGGGAGAGCGTTCCAATGCACACATTTGCCAAGTATCTCTTC<br><br>1521 T S L L P H D A E L A Y K I A L R A M R<br>4561 ACCTCTCTCCTACCTCACGATGCTGAATTGGCATACAAAATTGCACTGAGAGCAATGCGG<br><br>1541 L L V L E S T A P S G D L T R P H H I A<br>4621 TTACTAGTATTGGAATCTACTGCTCCATCAGGAGACCTCACCCGCCCACACCACATTGCA<br><br>1561 S V V P N R Y P R W F T L S H I E S Q Q<br>4681 TCAGTTGTTCCCAACCGCTACCCTCGCTGGTTCACTCTAAGCCACATTGAGTCCCAGCAG<br><br>1581 C E L A S T M L T A A K G D V R R L E T<br>4741 TGTGAGCTGGCATCCACCATGCTAACTGCAGCCAAAGGCGATGTTCGGAGGCTGGAAACA<br><br>1601 V L E S I Q K N I H S S S H I F K L A Q<br>4801 GTATTAGAATCCATCCAGAAAAACATTCACTCCTCATCACACATCTTCAAGCTTGCCCAA | |

| Gene Name | GenBank Homology | DNA SEQUENCE / DEDUCED AMINO ACID SEQUENCE | SEQUENCE IDENTIFIER: |
|---|---|---|---|
| | | 1621 D A F K I A T L M D S L P D I T L L K V<br>4861 GATGCATTTAAAATAGCAACTCTCATGGACAGTTTGCCAGACATCACTCTTTTGAAAGTG<br><br>1641 S L E L G L Q V M R M T L S T L N W R R<br>4921 TCTCTGGAGCTGGGCCTGCAGGTTATGCGAATGACACTGTCAACCTTAAATTGGCGACGG<br><br>1661 R E M V R W L V T C A T E V G V Y A L D<br>4981 CGGGAGATGGTGAGGTGGCTGGTAACGTGTGCTACTGAAGTCGGGGTTTATGCCCTGGAC<br><br>1681 S I M Q T W F T L F T P T E A T S I V A<br>5041 AGCATCATGCAGACCTGGTTTACACTCTTTACTCCCACCGAGGCCACAAGTATAGTTGCA<br><br>1701 T T V M S N S T I V R L H L D C H Q Q E<br>5101 ACTACCGTGATGTCCAACAGCACCATCGTCCGCCTCCACCTGGACTGCCACCAGCAGGAA<br><br>1721 K L A S S A R T L A L Q C A M K D P Q N<br>5161 AAGCTGGCCAGCAGCGCCCGGACACTTGCACTGCAGTGTGCCATGAAGGATCCACAGAAC<br><br>1741 C A L S A L T L C E K D H I A F E T A Y<br>5221 TGTGCCCTCTCTGCGCTAACCCTTTGTGAAAAGGATCACATAGCTTTTGAGACGGCGTAC<br><br>1761 Q I V L D A A T T G M S Y T Q L F T I A<br>5281 CAAATTGTTCTCGACGCTGCTACGACTGGCATGAGCTATACACAGCTCTTTACAATAGCA<br><br>1781 R Y M E H R G Y P M R A Y K L A T L A M<br>5341 CGGTACATGGAGCACCGCGGGTACCCCATGAGGGCCTACAAGCTGGCCACCCTGGCCATG<br><br>1801 T H L N L S Y N Q D T H P A I N D V L W<br>5401 ACCCATCTCAACCTGAGCTACAATCAGGACACACACCCTGCCATTAATGATGTTTTGTGG<br><br>1821 A C A L S H S L G K N E L A A I I P L V<br>5461 GCCTGTGCGCTTAGCCACTCCCTTGGTAAAAATGAGCTTGCAGCTATAATACCTCTGGTG<br><br>1841 V K S V K C A T V L S D I L R R C T L T<br>5521 GTCAAGAGTGTCAAGTGTGCAACGGTACTGTCAGACATTTTGCGCAGATGCACTCTGACC<br><br>1861 T P G M V G L H G R R N S G K L M S L D<br>5581 ACTCCTGGCATGGTGGGACTTCATGGGAGGAGGAACTCTGGTAAGCTCATGTCACTGGAC<br><br>1881 K A P L R Q L L D A T I G A Y I N T T H<br>5641 AAAGCCCCCTTGAGGCAACTCTTGGATGCCACGATCGGGGCCTACATCAACACAACGCAC<br><br>1901 S R L T H I S P R H Y S E F I E F L S K | |

| Gene Name | GenBank Homology | DNA SEQUENCE / DEDUCED AMINO ACID SEQUENCE | SEQUENCE IDENTIFIER: |
|---|---|---|---|
| | | 5701 TCACGGCTCACACACATCAGTCCTCGGCACTATAGTGAGTTTATAGAGTTCCTCAGCAAA<br><br>1921 A R E T F L M A H D G H I Q F T Q F I D<br>5761 GCCCGAGAGACCTTCTTAATGGCGCATGATGGACACATTCAGTTTACACAGTTTATTGAC<br><br>1941 N L K Q I Y K G K K K L M M L V R E R F<br>5821 AACCTGAAACAAATCTACAAAGGCAAAAAGAAACTGATGATGTTGGTTCGGGAGAGGTTT<br><br>1961 G -<br>5881 GGTTGA | |
| B1961932 | Equus caballus interleukin-4 receptor alpha-chain mRNA. | 1 GGGCGCTGCCGAGCCTGGCTGCCCTGGATCCCGCACTTCCCGCTCGGGCGCTGGACGGCG<br>61 AATGGGCCAGGGGCGCGCAGGTGCAGTAGGGTCTCCCAATGGGGTGCCTTTGCCCCGGGC<br>121 TCACGCTCCCTGTGAGCTGCCTGATCCTGGTGTGGGCGGCAGGCTCTGGGAGCGTTAAGG<br>181 TCCTGCGTCTCACCGCCTGCTTCTCCGACTACATCAGCGCCTCCACCTGTGAGTGGAAGA<br>241 TGGACCGTCCCACCAACTGCAGTGCCCAGCTCCGTCTGTCCTACCAGCTGAACGACGAGT<br>301 TCTCTGACAACCTCACGTGTATCCCCGAGAACAGAGAAGATGAAGTGTGCGTGTGCCGTA<br>361 TGCTGATGGACAACATCGTCAGCGAGGACGTCTATGAGCTGGACCTGTGGGCTGGAACC<br>421 AACTGCTGTGGAACAGCTCCTTCAAGCCCAGCCGGCACGTGAAACCCAGGGCCCCTCAAA<br>481 ACCTCACGGTTCACGCCATCTCCCACACGTGGCTGCTGACGTGGAGCAACCCGTACCCTT<br>541 TGAAGAATCACCTGTGGTCTGAGCTTACCTACCTGGTCAACATCTCCAAGGAGGACGACC<br>601 CCACGGACTTCAAAATCTACAACGTGACCTACATGGACCCCACCCTCCGCGTCACAGCCA<br>661 GCACCCTGAAGTCCAGGGCTACGTACAGCGCACGGGTGAAGGCCAGGGCTCAGAACTACA<br>721 ACAGCACCTGGAGTGAGTGGAGCCCCAGCACCACGTGGCATAACTACTACGAGCAGCCCT<br>781 TGGAGCAGCGCCTCCCGCTTGGTGTCAGCATCTCCTGCGTTGTCATCCTGGCCATCTGCC<br>841 TGTCCTGCTATTTCAGCATCATCAAGATTAAGAAAGAATGGTGGGACCAGATTCCCAACC<br>901 CAGCGCACAGCCCCCTCGTGGCTATCGTCCTCCAGGATTCTCAGGTGTCACTGTGGGGA<br>961 AGCAGTCCCGAGGCCAGGAGCCAGCCAAGTGCCCACGCTGGAAGACTTGTCTTACCAAGC<br>1021 TCCTGCCCTGTTTACTGGAGCATGGCCTGCAAAAGGAGGAGGATTCCTCCAAGACTGTCA<br>1081 GAAATGGGCCTTTCCAGAGTCCTGGAAAATCAGCATGGCACACTGTGGAGGTCAACCACA<br>1141 CGATCCTCCGGCCAGAGATCATCAGCGTGGTGCCGTGTGTGGAGCTGTGTGAGGCCCAGG<br>1201 TGGAGAGCGAGGAGGAGGAAGTGGAGGAAGATAGAGGGAGCTTCTGCCCGTCGCCTGAGA<br>1261 GCAGCGGGAGCGGCTTCCAGGAAGGCAGGGAGGGCGTCGCGGCCCGGCTGACAGAGAGCC<br>1321 TGTTCCTGGGCCTCCTCGGGGCTGAGAATGGGGCCTTGGGGGAGTCATGCCTTCTTCCCC<br>1381 CTTTAGGAAGTGCTCACATGCCCTGGGCCAGGATCTCAAGTGCAGGGCCCCAGGAGGCAG<br>1441 CGTCCCAGGGTGAGGAGCAGCCTCTCAACCCAGAGTCAAATCCTCTGGCCACTCTGACCC<br>1501 AGAGCCCAGGCAGCCTGGCTTTCACAGAGGCGCCTGCTGTGGTTGCAGACAACCCCGCCT<br>1561 ACCGCAGCTTCAGCAACTCCCTGAGCCAGCCCCGAGGTCCTGGAGAGCTTGACTCAGACC<br>1621 CACAGCTGGCCGAACACCTGGGCCAAGTGGACCCCAGCATCCCCTCCGCCCCCCAGCCCT<br>1681 CTGAACCACCCACTGCACTCCAGCCTGAACCAGAAACCTGGGAGCAGATGCTCCGCCAGA<br>1741 GTGTCCTCCAGCAGGGGGCAGCCCCAGCCCCCGCCTCGGCCCCCACTGGCGGCTACCGGG<br>1801 AGTTTGCGCAGGTGGTGAAGCAGGGTGGCGGGGCGGCGGGCTCCGGCCCTTCTGGGGAGG | SEQ ID NO:229 |

| Gene Name | GenBank Homology | DNA SEQUENCE / DEDUCED AMINO ACID SEQUENCE | SEQUENCE IDENTIFIER: |
|---|---|---|---|
| | | 1861 CTGGGTACAAGGCCTTCTCCAGCCTGCTCGCTGGCAGTGCCGTCTGCCCAGGGCAATCTG<br>1921 GGGTTGAGGCCAGCAGTGGGGAGGGGGGCTACAGGCCCTATGAGAGCCCCGACCCTGGAG<br>1981 CCCCTGCCCCGGTCCCCGTCCCCCTGTTCACCTTTGGACTGGATGTGGAGCCACCTCACA<br>2041 GCCCTCAGAACTCCCTCCTGCCAGGCGGCTCCCCAGAGCTCCCTGGCCCAGAGCCGACGG<br>2101 TGAAGGGAGAGGACCCACGGAAGCCCCTGCTTTCCGCACAGCAGGCCACAGACTCCCTCA<br>2161 GGGACGACCTGGGCAGCGGCATTGTCTACTCGGCCCTCACCTGCCACCTTTGTGGCCACC<br>2221 TGAAGCAGTGTCATGGCCAGGAGGAGCATGGCGAGGCCCACACTGTGGCCAGCCCCTGCT<br>2281 GTGGCTGCTGTTGTGGGGACAGGTCCTCCCCCCCAGTGAGCCCCGTGAGGGCCCTGGACC<br>2341 CCCCGCCAGGTGGGGTTCCCCTGGAGGCGGGCCTCTCTCTTGCCTCCCTGGGATCCTTGG<br>2401 GGCTCTCTGAGGAGCGCAAACCCTCCCTCTTCTTCCAGCCTGCTCCCGGCAATGCTCAGA<br>2461 GCTCGAGCCAGACCCCTCTCACGGTGGCCATGCTCTCCACAGGGCCCACATGCACGAGCG<br>2521 CTTCCTAGGGGCGTGCCCTCTGGTTGCTGCGGTCCACAGGTGAGGACCGGGTCTTCGAAA<br>2581 TGCCTCCCCCCACATTTTGGGGCAGCCAGGCTGGCAGACTTCTGAAAGACTCAGAGAACC<br>2641 CTGGTAGGAAGCTGTGAGGTTGTCCAACCTGGGGCTACAGAGACTGGACCCCCTTGCTCC<br>2701 CAGCCGTGGCCCAAGCTCCCCCCATCCCACGGGAGTGGAGCCTGCAGGGCAGCCATGCCC<br>2761 ACGGCAGGCACCTGCGGGCATCGGGAGGTCCCTGGGCAGCTGAGCTTGTGAACGAGCCGT<br>2821 TGGCCGCTTCGTTGGTGCACAGCTTCTCCAGCATGCTGTCCCTGTCACGCCTGCCCAAGG<br>2881 CTTGTTTTGTCCACCTAATCTCTCTGTTACCCGAGTCTGACCCAGTCCTGGGTTAGCTGC<br>2941 CGCCATATCACTGGATTGGATGCTGAGCCTAGAAACTGATCAAGCTCATGGGGGAATGAC<br>3001 TTAGGAGGCCCCAGGAAATTCAAGGGAAGTCGGGGTCCAGGAGGATAGGATTTGCCTAGA<br>3061 GAGGCCCGTTCCTTCAACAGAGCTTCATCTAGCTGGCACCAGAGGCAGGATTGCACCTGT<br>3121 GGTGGGTGCTTAGCCAAGTCGGGGTCACAGAGAAGGACATGAGAAATTGTGATTAGCCGG<br>3181 TAGTGACAGTTTGCTGTCAGGTCCCCCCACAACTGTAGGCCTGGGCCTCCTCTTAGGCAT<br>3241 GGGATCCCCAGAGTGGACCTGCCCAGCTACCCAGGGCCAGTTTGTGCACCCATGGAGAGC<br>3301 GTTGCTGGCAGCCATAGAGACCAGAGGGAGTGGGTCACAGCCCATGACCCAGCCGAATGG<br>3361 GGCATTCCAGACAGCTGACCCGGCACGTTTTGCCTGCACATGGCTCAGACCTTGGGTCGA<br>3421 GTAACGCTTGTTTGTGTGTATCTCAAAGATATATTTTATCTCACTGGTATTTGTGTTTGC<br>3481 TGAGGACGGTGGAATGGGGGGTCTGGAGTCTTGTATGAATAAAGATTCTTTCTCTCAAA<br>3541 AAAAAAAAAAAAAAA | |
| | | 1 M G C L C P G L T L P V S C L I L V W A<br>1 ATGGGGTGCCTTTGCCCCGGGCTCACGCTCCCTGTGAGCTGCCTGATCCTGGTGTGGGCG<br><br>21 A G S G S V K V L R L T A C F S D Y I S<br>61 GCAGGCTCTGGGAGCGTTAAGGTCCTGCGTCTCACCGCCTGCTTCTCCGACTACATCAGC<br><br>41 A S T C E W K M D R P T N C S A Q L R L<br>121 GCCTCCACCTGTGAGTGGAAGATGGACCGTCCCACCAACTGCAGTGCCCAGCTCCGTCTG<br><br>61 S Y Q L N D E F S D N L T C I P E N R E<br>181 TCCTACCAGCTGAACGACGAGTTCTCTGACAACCTCACGTGTATCCCCGAGAACAGAGAA<br><br>81 D E V C V C R M L M D N I V S E D V Y E | SEQ ID NO:230 |

| Gene Name | GenBank Homology | DNA SEQUENCE / DEDUCED AMINO ACID SEQUENCE | SEQUENCE IDENTIFIER: |
|---|---|---|---|
| | | 241 GATGAAGTGTGCGTGTGCCGTATGCTGATGGACAACATCGTCAGCGAGGACGTCTATGAG<br><br>101 L D L W A G N Q L L W N S S F K P S R H<br>301 CTGGACCTGTGGGCTGGGAACCAACTGCTGTGGAACAGCTCCTTCAAGCCCAGCCGGCAC<br><br>121 V K P R A P Q N L T V H A I S H T W L L<br>361 GTGAAACCCAGGGCCCCTCAAAACCTCACGGTTCACGCCATCTCCCACACGTGGCTGCTG<br><br>141 T W S N P Y P L K N H L W S E L T Y L V<br>421 ACGTGGAGCAACCCGTACCCTTTGAAGAATCACCTGTGGTCTGAGCTTACCTACCTGGTC<br><br>161 N I S K E D D P T D F K I Y N V T Y M D<br>481 AACATCTCCAAGGAGGACGACCCCACGGACTTCAAAATCTACAACGTGACCTACATGGAC<br><br>181 P T L R V T A S T L K S R A T Y S A R V<br>541 CCCACCCTCCGCGTCACAGCCAGCACCCTGAAGTCCAGGGCTACGTACAGCGCACGGGTG<br><br>201 K A R A Q N Y N S T W S E W S P S T T W<br>601 AAGGCCAGGGCTCAGAACTACAACAGCACCTGGAGTGAGTGGAGCCCCAGCACCACGTGG<br><br>221 H N Y Y E Q P L E Q R L P L G V S I S C<br>661 CATAACTACTACGAGCAGCCCTTGGAGCAGCGCCTCCCGCTTGGTGTCAGCATCTCCTGC<br><br>241 V V I L A I C L S C Y F S I I K I K K E<br>721 GTTGTCATCCTGGCCATCTGCCTGTCCTGCTATTTCAGCATCATCAAGATTAAGAAAGAA<br><br>261 W W D Q I P N P A H S P L V A I V L Q D<br>781 TGGTGGGACCAGATTCCCAACCCAGCGCACAGCCCCCTCGTGGCTATCGTCCTCCAGGAT<br><br>281 S Q V S L W G K Q S R G Q E P A K C P R<br>841 TCTCAGGTGTCACTGTGGGGGAAGCAGTCCCGAGGCCAGGAGCCAGCCAAGTGCCCACGC<br><br>301 W K T C L T K L L P C L L E H G L Q K E<br>901 TGGAAGACTTGTCTTACCAAGCTCCTGCCCTGTTTACTGGAGCATGGCCTGCAAAAGGAG<br><br>321 E D S S K T V R N G P F Q S P G K S A W<br>961 GAGGATTCCTCCAAGACTGTCAGAAATGGGCCTTTCCAGAGTCCTGGAAAATCAGCATGG<br><br>341 H T V E V N H T I L R P E I I S V V P C<br>1021 CACACTGTGGAGGTCAACCACACGATCCTCCGGCCAGAGATCATCAGCGTGGTGCCGTGT<br><br>361 V E L C E A Q V E S E E E E V E E D R G<br>1081 GTGGAGCTGTGTGAGGCCCAGGTGGAGAGCGAGGAGGAGGAAGTGGAGGAAGATAGAGGG | |

| Gene Name | GenBank Homology | DNA SEQUENCE / DEDUCED AMINO ACID SEQUENCE | SEQUENCE IDENTIFIER: |
|---|---|---|---|
| | | 381 S F C P S P E S S G S G F Q E G R E G V<br>1141 AGCTTCTGCCCGTCGCCTGAGAGCAGCGGGAGCGGCTTCCAGGAAGGCAGGGAGGGCGTC<br><br>401 A A R L T E S L F L G L L G A E N G A L<br>1201 GCGGCCCGGCTGACAGAGAGCCTGTTCCTGGGCCTCCTCGGGGCTGAGAATGGGGCCTTG<br><br>421 G E S C L L P P L G S A H M P W A R I S<br>1261 GGGGAGTCATGCCTTCTTCCCCCTTTAGGAAGTGCTCACATGCCCTGGGCCAGGATCTCA<br><br>441 S A G P Q E A A S Q G E E Q P L N P E S<br>1321 AGTGCAGGGCCCCAGGAGGCAGCGTCCCAGGGTGAGGAGCAGCCTCTCAACCCAGAGTCA<br><br>461 N P L A T L T Q S P G S L A F T E A P A<br>1381 AATCCTCTGGCCACTCTGACCCAGAGCCCAGGCAGCCTGGCTTTCACAGAGGCGCCTGCT<br><br>481 V V A D N P A Y R S F S N S L S Q P R G<br>1441 GTGGTTGCAGACAACCCCGCCTACCGCAGCTTCAGCAACTCCCTGAGCCAGCCCCGAGGT<br><br>501 P G E L D S D P Q L A E H L G Q V D P S<br>1501 CCTGGAGAGCTTGACTCAGACCCACAGCTGGCCGAACACCTGGGCCAAGTGGACCCCAGC<br><br>521 I P S A P Q P S E P P T A L Q P E P E T<br>1561 ATCCCCTCCGCCCCCCAGCCCTCTGAACCACCCACTGCACTCCAGCCTGAACCAGAAACC<br><br>541 W E Q M L R Q S V L Q Q G A A P A P A S<br>1621 TGGGAGCAGATGCTCCGCCAGAGTGTCCTCCAGCAGGGGGCAGCCCCAGCCCCCGCCTCG<br><br>561 A P T G G Y R E F A Q V V K Q G G G A A<br>1681 GCCCCCACTGGCGGCTACCGGGAGTTTGCGCAGGTGGTGAAGCAGGGTGGCGGGGCGGCG<br><br>581 G S G P S G E A G Y K A F S S L L A G S<br>1741 GGCTCCGGCCCTTCTGGGGAGGCTGGGTACAAGGCCTTCTCCAGCCTGCTCGCTGGCAGT<br><br>601 A V C P G Q S G V E A S S G E G G Y R P<br>1801 GCCGTCTGCCCAGGGCAATCTGGGGTTGAGGCCAGCAGTGGGGAGGGGGGCTACAGGCCC<br><br>621 Y E S P D P G A P A P V P V P L F T F G<br>1861 TATGAGAGCCCCGACCCTGGAGCCCCTGCCCCGGTCCCCGTCCCCCTGTTCACCTTTGGA<br><br>641 L D V E P P H S P Q N S L L P G G S P E<br>1921 CTGGATGTGGAGCCACCTCACAGCCCTCAGAACTCCCTCCTGCCAGGCGGCTCCCCAGAG | |

| Gene Name | GenBank Homology | DNA SEQUENCE / DEDUCED AMINO ACID SEQUENCE | SEQUENCE IDENTIFIER: |
|---|---|---|---|
| | | 661 L P G P E P T V K G E D P R K P L L S A<br>1981 CTCCCTGGCCCAGAGCCGACGGTGAAGGGAGAGGACCCACGGAAGCCCCTGCTTTCCGCA<br><br>681 Q Q A T D S L R D D L G S G I V Y S A L<br>2041 CAGCAGGCCACAGACTCCCTCAGGGACGACCTGGGCAGCGGCATTGTCTACTCGGCCCTC<br><br>701 T C H L C G H L K Q C H G Q E E H G E A<br>2101 ACCTGCCACCTTTGTGGCCACCTGAAGCAGTGTCATGGCCAGGAGGAGCATGGCGAGGCC<br><br>721 H T V A S P C C G C C C G D R S S P P V<br>2161 CACACTGTGGCCAGCCCCTGCTGTGGCTGCTGTTGTGGGGACAGGTCCTCCCCCCCAGTG<br><br>741 S P V R A L D P P P G G V P L E A G L S<br>2221 AGCCCCGTGAGGGCCCTGGACCCCCCGCCAGGTGGGGTTCCCCTGGAGGCGGGCCTCTCT<br><br>761 L A S L G S L G L S E E R K P S L F F Q<br>2281 CTTGCCTCCCTGGGATCCTTGGGGCTCTCTGAGGAGCGCAAACCCTCCCTCTTCTTCCAG<br><br>781 P A P G N A Q S S S Q T P L T V A M L S<br>2341 CCTGCTCCCGGCAATGCTCAGAGCTCGAGCCAGACCCCTCTCACGGTGGCCATGCTCTCC<br><br>801 T G P T C T S A S -<br>2401 ACAGGGCCCACATGCACGAGCGCTTCCTAG | |
| B1961941 | Fibroblast mRNA for aldolase A | 1 GGGGAACCACACTCCGTCCACGGACTCTCCGTTATTTTAGGAGGTCCCTGGCCAAAGATT<br>61 TATTTCTCTTGACAACCAAGGGCCTCCGTCTGGATTTCCAAGGAAGAATTTCCTCTGAAG<br>121 CACCGGAACTTGCTACTACCAGCACCATGCCCTACCAATATCCAGCACTGACCCCGGAGC<br>181 AGAAGAAGGAGCTGTCTGACATCGCTCACCGCATCGTGGCACCTGGCAAGGGCATCCTGG<br>241 CTGCAGATGAGTCCACTGGGAGCATTGCCAAGCGGCTGCAGTCCATTGGCACCGAGAACA<br>301 CCGAGGAGAACCGGCGCTTCTACCGCCAGCTGCTGCTGACAGCTGACGACCGCGTGAACC<br>361 CCTGCATTGGGGGTGTCATCCTCTTCCATGAGACACTCTACCAGAAGGCGGATGATGGGC<br>421 GTCCCTTCCCCCAAGTTATCAAATCCAAGGGCGGTGTTGTGGGCATCAAGGTAGACAAGG<br>481 GCGTGGTCCCCCTGGCAGGGACAAATGGCGAGACTACCACCCAAGGGTTGGATGGGCTGT<br>541 CTGAGCGCTGTGCCCAGTACAAGAAGGACGGAGCTGACTTTGCCAAGTGGCGCTGTGTGC<br>601 TGAAGATTGGGGAGCACACTCCCTCATCCCTTGCCATCATGGAAAATGCCAATGTTCTGG<br>661 CCCGTTATGCCAGCATCTGCCAGCAGAATGGCATTGTGCCCATCGTGGAGCCTGAGATCC<br>721 TCCCTGATGGGGACCATGACTTGAAGCGCTGTCAGTATGTAACCGAGAAGGTGCTGGCTG<br>781 CTGTTTACAAGGCTCTGAGTGACCACCACATCTACCTGGAAGGCACCTTGCTGAAGCCCA<br>841 ATATGGTAACCCCAGGCCACGCCTGCACCCACAAATATTCTCATGAGGAGATTGCCATGG<br>901 CAACTGTCACGGCACTGCGTCGCACGGTGCCCCCTGCTGTCACTGGGATCACCTTCCTAT<br>961 CTGGAGGCCAGAGTGAGGAGGAGGCATCCATCAACCTCAACGCCATCAACAAGTGCCCCC<br>1021 TGCTGAAGCCGTGGGCCCTGACCTTCTCCTATGGCCGAGCCCTGCAGGCCTCTGCCCTGA | SEQ ID NO:231 |

| Gene Name | GenBank Homology | DNA SEQUENCE / DEDUCED AMINO ACID SEQUENCE | SEQUENCE IDENTIFIER: |
|---|---|---|---|
| | | 1081 AGGCCTGGGGTGGGAAGAAGGAGAACCTGAAGGCTGCCCAGGAGGAATATGTCAAGCGAG<br>1141 CCCTGGCCAACAGCCTCGCCTGCCAAGGAAAATACACCCCAAGTGGTCACGCTGGGGCTG<br>1201 CAGCCAGCGAGTCCCTCTTCATCTCTAACCATGCCTACTAAGTGGAGGTATTCTAAGGCT<br>1261 GCCCCCTCAACACTCCAGGCCCTGCCTCCTCCCACTCTCACTCTTGAAGAGGGGGCCTCA<br>1321 TCCAGGGCTTTAGGCTGTTCTTTCCCATTCTCCTTGCCTCCCTGGTGACATTGGTCTGTG<br>1381 GTATTGTCTGTGTATGCTAACTCCATCACCCTTTCGAGCCCACTGCCAAATAAACAGCTA<br>1441 TTTAAGGGGGAAAAAAAAAAAAAAAAAAAAAAAAAAAAAAAAAATAAAAAAAAAAAAAAA<br>1501 AAAAAAAAAAAAAAA | |
| | | 1 M P Y Q Y P A L T P E Q K K E L S D I A<br>1 ATGCCCTACCAATATCCAGCACTGACCCCGGAGCAGAAGAAGGAGCTGTCTGACATCGCT<br><br>21 H R I V A P G K G I L A A D E S T G S I<br>61 CACCGCATCGTGGCACCTGGCAAGGGCATCCTGGCTGCAGATGAGTCCACTGGGAGCATT<br><br>41 A K R L Q S I G T E N T E E N R R F Y R<br>121 GCCAAGCGGCTGCAGTCCATTGGCACCGAGAACACCGAGGAGAACCGGCGCTTCTACCGC<br><br>61 Q L L L T A D D R V N P C I G G V I L F<br>181 CAGCTGCTGCTGACAGCTGACGACCGCGTGAACCCCTGCATTGGGGGTGTCATCCTCTTC<br><br>81 H E T L Y Q K A D D G R P F P Q V I K S<br>241 CATGAGACACTCTACCAGAAGGCGGATGATGGGCGTCCCTTCCCCCAAGTTATCAAATCC<br><br>101 K G G V V G I K V D K G V V P L A G T N<br>301 AAGGGCGGTGTTGTGGGCATCAAGGTAGACAAGGGCGTGGTCCCCCTGGCAGGGACAAAT<br><br>121 G E T T T Q G L D G L S E R C A Q Y K K<br>361 GGCGAGACTACCACCCAAGGGTTGGATGGGCTGTCTGAGCGCTGTGCCCAGTACAAGAAG<br><br>141 D G A D F A K W R C V L K I G E H T P S<br>421 GACGGAGCTGACTTTGCCAAGTGGCGCTGTGTGCTGAAGATTGGGGAGCACACTCCCTCA<br><br>161 S L A I M E N A N V L A R Y A S I C Q Q<br>481 TCCCTTGCCATCATGGAAAATGCCAATGTTCTGGCCCGTTATGCCAGCATCTGCCAGCAG<br><br>181 N G I V P I V E P E I L P D G D H D L K<br>541 AATGGCATTGTGCCCATCGTGGAGCCTGAGATCCTCCCTGATGGGGACCATGACTTGAAG<br><br>201 R C Q Y V T E K V L A A V Y K A L S D H<br>601 CGCTGTCAGTATGTAACCGAGAAGGTGCTGGCTGCTGTTTACAAGGCTCTGAGTGACCAC<br><br>221 H I Y L E G T L L K P N M V T P G H A C | SEQ ID NO:232 |

| Gene Name | GenBank Homology | DNA SEQUENCE / DEDUCED AMINO ACID SEQUENCE | SEQUENCE IDENTIFIER: |
|---|---|---|---|
| | | 661 CACATCTACCTGGAAGGCACCTTGCTGAAGCCCAATATGGTAACCCCAGGCCACGCCTGC<br><br>241 T H K Y S H E E I A M A T V T A L R R T<br>721 ACCCACAAATATTCTCATGAGGAGATTGCCATGGCAACTGTCACGGCACTGCGTCGCACG<br><br>261 V P P A V T G I T F L S G G Q S E E E A<br>781 GTGCCCCCTGCTGTCACTGGGATCACCTTCCTATCTGGAGGCCAGAGTGAGGAGGAGGCA<br><br>281 S I N L N A I N K C P L L K P W A L T F<br>841 TCCATCAACCTCAACGCCATCAACAAGTGCCCCCTGCTGAAGCCGTGGGCCCTGACCTTC<br><br>301 S Y G R A L Q A S A L K A W G G K K E N<br>901 TCCTATGGCCGAGCCCTGCAGGCCTCTGCCCTGAAGGCCTGGGGTGGAAGAAGGAGAAC<br><br>321 L K A A Q E E Y V K R A L A N S L A C Q<br>961 CTGAAGGCTGCCCAGGAGGAATATGTCAAGCGAGCCCTGGCCAACAGCCTCGCCTGCCAA<br><br>341 G K Y T P S G H A G A A A S E S L F I S<br>1021 GGAAAATACACCCCAAGTGGTCACGCTGGGGCTGCAGCCAGCGAGTCCCTCTTCATCTCT<br><br>361 N H A Y -<br>1081 AACCATGCCTACTAA | |
| BM734452 | Equus caballus adenosine A2A receptor mRNA | 1 gtgccccggg tgctgacgct gagctgagcc aagatgccgc gggagccgct agagagggcc<br>61 tggtttctgg agactcggag ttggctgtga aaaaaccctt ggggagcccc ccactcccca<br>121 gcacggacgc tcttggctcc tgtgagaagg gggctccggg gcctgggcag ggccggctgc<br>181 gctgcagcta tggaccgtga gctggcctgg cccgcgtcct tgctgaccct gcccgtctgc<br>241 agccatgccc accgtgggct ccttggtgta catcatggtg gagctggcca tcgctctgct<br>301 ggccatcctg ggcaacatgc tggtgtgctg ggccgtgtgg ctgaacagca acctgcagaa<br>361 cgtcaccaac tactttgtgg tgtcactggc agcggccgac atcgctgtgg gggtccttgc<br>421 catccccttc gccatcacca tcagcacagg gttctgtgcc gcctgccacg gctgcctctt<br>481 catcgcctgc ttcgtgctgg tcctcacgca gagctccatc ttcagcctcc tggccatcgc<br>541 cattgaccgc tacatcgcca tccgcatccc actccgatac aatggcttgg tgaccggcac<br>601 gagggccaag ggcgtcattg cagtctgctg ggtgctgtcg ttcgccatcg gcctgacgcc<br>661 catgctgggc tggaacaact gccatcactg gggggagggc gagaaccagt cgcagggctg<br>721 cggcgagggc caggtggcct gcctctttga ggacgtggtc cccatgaact acatggtgta<br>781 ctacaacttc tttgcttgcg ttctggtgcc cctgctgctc atgctgggcg tctacttgcg<br>841 catcttcctg gcggcgcggc gacagctgaa gcagatggag acccagcctc tgccggggga<br>901 gcgggctcgg tccacgctgc agaaggaggt ccacgccgcc aagtcgctgg ccatcatcgt<br>961 ggggctcttc gccctctgct ggctgcccct gcacatcatc aactgcttca ccttcttctg<br>1021 ccccgagtgc ccgcacgccc cgctctggct catgtacccg gccatcatcc tctcccactt<br>1081 caattccgtc gtgaacccct tcatctacgc ctaccgcatc cgcgagttcc gccacacctt | SEQ ID NO:233 |

| Gene Name | GenBank Homology | DNA SEQUENCE / DEDUCED AMINO ACID SEQUENCE | SEQUENCE IDENTIFIER: |
|---|---|---|---|
| | | 1141 ccacaagatc attcgcagtc acatcctgag gcggcaggag cccttcaagg cagggggcac<br>1201 cagtgcccgg gccttggcag ctcatggcag cgatgcagag caggtcagcc tccgcctcaa<br>1261 cggccaccct tctggggtgt gccccaatgg cagcgccccc catcctgagc ggcggcccaa<br>1321 tggctatgcc ctggggctgg tgagtcgggc gagtgcccgc gagtcccctg gagacacagg<br>1381 cctcccagat gtggaactcc tcagccacga gctccatggg gcgagcccag agtcccctgg<br>1441 cctcgagggg cccctggccc aagacggagc gggagtgtcc tgacgactcc ccgtgtctgc<br>1501 cccttcctaa gggaaggggg cctttctctt tctggttgga ggaaccgctc acgttgggag<br>1561 atgagtgcat gtgccgggag actcttctcg aggccagttc ctgccgcgga ctgagagaag<br>1621 ggagccccgg ctggagcagc atgaggtcca gcaggaaggt ctggcatcca aggaggtgga<br>1681 cgcatcatgc tgggagggcc tgcacgaggc cagggccgcg gcaccagggc atcttggctg<br>1741 ggtaggcccg gtccccgact ccagcagcac gtagaagtgc cccctggtct ctgcagagca<br>1801 gccgtggcgg agcaggctgg ccggccctga ggctggggag tggccccgaa ggccccccgc<br>1861 cacacacgca ccaccctccc cagactttcc caggttcggg agctgctgtg tccagaggtg<br>1921 gcatttgact atttttgacc aggagaaaat gtaagcgcga ggaaatcctt tttattgtat<br>1981 tatcttccct ccctggctgc tgggtctgtt gtcagtcctg ctgctaacct ggcaccagga<br>2041 gtggccaggg agtcccaggc agccctctcc ggctgccgtg agctgccatg tgcttctcac<br>2101 ccgtcagtcc tgaggccacc tcttggggtg acaaagctgg gcttggtgac aggaagttgt<br>2161 aatggagcgg tgccagagtg cgggctcagg gagcaggttg gggctggctg gcatggggtg<br>2221 tgcctgggcg gctcagagct gcccaagtgg aggctctagc tgactgcctt tccttctgag<br>2281 gggaatgttt ttttctgaga tacaataaaa atgagccaca ttgtgtttta aaaaaaaaaa<br>2341 aaaaaaaaaa aa | |
| | | 1 M P T V G S L V Y I M V E L A I A L L A<br>1 ATGCCCACCGTGGGCTCCTTGGTGTACATCATGGTGGAGCTGGCCATCGCTCTGCTGGCC<br><br>21 I L G N M L V C W A V W L N S N L Q N V<br>61 ATCCTGGGCAACATGCTGGTGTGCTGGGCCGTGTGGCTGAACAGCAACCTGCAGAACGTC<br><br>41 T N Y F V V S L A A A D I A V G V L A I<br>121 ACCAACTACTTTGTGGTGTCACTGGCAGCGGCCGACATCGCTGTGGGGGTCCTTGCCATC<br><br>61 P F A I T I S T G F C A A C H G C L F I<br>181 CCCTTCGCCATCACCATCAGCACAGGGTTCTGTGCCGCCTGCCACGGCTGCCTCTTCATC<br><br>81 A C F V L V L T Q S S I F S L L A I A I<br>241 GCCTGCTTCGTGCTGGTCCTCACGCAGAGCTCCATCTTCAGCCTCCTGGCCATCGCCATT<br><br>101 D R Y I A I R I P L R Y N G L V T G T R<br>301 GACCGCTACATCGCCATCCGCATCCCACTCCGATACAATGGCTTGGTGACCGGCACGAGG<br><br>121 A K G V I A V C W V L S F A I G L T P M<br>361 GCCAAGGGCGTCATTGCAGTCTGCTGGGTGCTGTCGTTCGCCATCGGCCTGACGCCCATG, | SEQ ID NO:234 |

| Gene Name | GenBank Homology | DNA SEQUENCE / DEDUCED AMINO ACID SEQUENCE | SEQUENCE IDENTIFIER: |
|---|---|---|---|
| | | 141 L G W N N C H H W G E G E N Q S Q G C G<br>421 CTGGGCTGGAACAACTGCCATCACTGGGGGGAGGGCGAGAACCAGTCGCAGGGCTGCGGC<br><br>161 E G Q V A C L F E D V V P M N Y M V Y Y<br>481 GAGGGCCAGGTGGCCTGCCTCTTTGAGGACGTGGTCCCCATGAACTACATGGTGTACTAC<br><br>181 N F F A C V L V P L L L M L G V Y L R I<br>541 AACTTCTTTGCTTGCGTTCTGGTGCCCCTGCTGCTCATGCTGGGCGTCTACTTGCGCATC<br><br>201 F L A A R R Q L K Q M E T Q P L P G E R<br>601 TTCCTGGCGGCGCGGCGACAGCTGAAGCAGATGGAGACCCAGCCTCTGCCGGGGGAGCGG<br><br>221 A R S T L Q K E V H A A K S L A I I V G<br>661 GCTCGGTCCACGCTGCAGAAGGAGGTCCACGCCGCCAAGTCGCTGGCCATCATCGTGGGG<br><br>241 L F A L C W L P L H I I N C F T F F C P<br>721 CTCTTCGCCCTCTGCTGGCTGCCCCTGCACATCATCAACTGCTTCACCTTCTTCTGCCCC<br><br>261 E C P H A P L W L M Y P A I I L S H F N<br>781 GAGTGCCCGCACGCCCCGCTCTGGCTCATGTACCCGGCCATCATCCTCTCCCACTTCAAT<br><br>281 S V V N P F I Y A Y R I R E F R H T F H<br>841 TCCGTCGTGAACCCCTTCATCTACGCCTACCGCATCCGCGAGTTCCGCCACACCTTCCAC<br><br>301 K I I R S H I L R R Q E P F K A G G T S<br>901 AAGATCATTCGCAGTCACATCCTGAGGCGGCAGGAGCCCTTCAAGGCAGGGGGCACCAGT<br><br>321 A R A L A A H G S D A E Q V S L R L N G<br>961 GCCCGGGCCTTGGCAGCTCATGGCAGCGATGCAGAGCAGGTCAGCCTCCGCCTCAACGGC<br><br>341 H P S G V C P N G S A P H P E R R P N G<br>1021 CACCCTTCTGGGGTGTGCCCCAATGGCAGCGCCCCCCATCCTGAGCGGCGGCCCAATGGC<br><br>361 Y A L G L V S R A S A R E S P G D T G L<br>1081 TATGCCCTGGGGCTGGTGAGTCGGGCGAGTGCCCGCGAGTCCCCTGGAGACACAGGCCTC<br><br>381 P D V E L L S H E L H G A S P E S P G L<br>1141 CCAGATGTGGAACTCCTCAGCCACGAGCTCCATGGGGCGAGCCCAGAGTCCCCTGGCCTC<br><br>401 E G P L A Q D G A G V S -<br>1201 GAGGGGCCCCTGGCCCAAGACGGAGCGGGAGTGTCCTGA | |
| BM734511 | No Homology | | |

| Gene Name | GenBank Homology | DNA SEQUENCE / DEDUCED AMINO ACID SEQUENCE | SEQUENCE IDENTIFIER: |
|---|---|---|---|
| | | 1 GTGGTCCTCCTCATCTCTTTCCTCATTTTCCTGATAGTGGGATGAGGATGGCCTTCCTGT<br>61 TTCACCATCTTCTCAATCTTTCTTTACCGGGTTGGGGAAGGGCTGTCTACCTGCGGCCCT<br>121 TTCAGTGGTGGTGTCTCCTTGCTTCTCTCTTCTCACCCGTAGGCTAATACGCTTGGCACT<br>181 GATGGGCACTGGGGAATGCAGAGCAGACCCCGGATGCTATTTATTCAAGCCCTGTTTGAA<br>241 TGAGTCCTTCGTGGGCCAGTGCTGGGCTGGGAAGAGGGTAACAGCAAATAATCATTGGTT<br>301 GAGCTAGGCTTATCTTTTTGTCTAGTGCAACAGATTGAGGGTAAAACAGTTCTCAAAACA<br>361 GCCTTCAAATACCTTTACCTAAATACTTCCAGCTCCTTCTTCCAGCTAAAGCTCAGTACA<br>421 CTAATGCCTCATCTTAGCAGTGATTTTGTAGCAATTCGAAGAAATTTTCTCATCCGTTTT<br>481 AAGAAAACCTGACTGAGTTTTCCTGTCTAAACAGCATTTCTGCCAAACTTAAAAGGAGGC<br>541 CCACATGATACCCATTC | SEQ ID NO:235 |
| BM734531 | No Homology | 1 TCCTCATCAGAGTCACCCAAGGAACTTATGCAGATGCCATGTCCTCACTCCGAATCCTCA<br>61 CTCTGGGAGTGGAGCCAGGTGTCTGCATTTGAACAGGGTGAGGGGGTGGGTTGCCTGCTG<br>121 GACAAGGGAGCTGAGCCAGCAGCTCCGGACTCTGTCCTGTGGCTTTTGTGTGTCTCTCCC<br>181 ATGTTCTCCCTCCCAGGGGCCCACCACTTCATGTTCTCTACAGAGCTAAGATGATGGCCT<br>241 TGCTTGAGAGAGGTCCATCCCCCGTGGTTGGGGTCACTTAGCAGCGACTTCTTGGACTGC<br>301 CATCCAGCCACAGTGGCCCCACAGAGCTGTCCAGAGCCGAGGCTGTGGGCTGCAGTCCCA<br>361 GTGAGCAGGGACGTTGCCCGCTGTTGGTCATGACAACCAAACGAGTCCGAGGGCACAGCC<br>421 AGGTGTCTTCCACCAGCCCATGAGCTGAGAATGCTAAGTCACCAGACAACCAGAAATTGA<br>481 GTCTCTGTCAGGATCCTTTTGAAGTGTAAGGAGAGAGAAACTTACACTTTGAAAGGTTTT<br>541 GAGCCTCATCCAGGACAATAAAAATAACTGT | SEQ ID NO:236 |
| BM734632 | Homo sapiens AT rich interactive domain 5A (MRF1-like), mRNA (cDNA clone MGC:75508 IMAGE:30409017) | 1 cggacagccg cgcgctgagg gtctcggggc gggcgccgcg ggacctctcc gggccatggc<br>61 agcccctgtc aaagggaaca ggaagcagtc cacggagggt gacgccctag acccacctgc<br>121 atcccccaaa cctgctggca agcagaacgg aatccagaac cccatctcgc tggagttaac<br>181 ctgtggaaga tctacaaagc agtggagaag ctgggggcct atgagctggt gaccgggcgc<br>241 cgcctctgga agaacgtgta cgacgagctg gggggcagcc caggcagcac cagcgcggcc<br>301 acgtgcacgc gccgccacta cgagaggctg gtcctgccat acgtgcggca cctgaagggg<br>361 gaggatgaca agccgctgcc cacctccaag cccaggaaac agtacaagat ggctaaggag<br>421 aacagggggg atgatggggc caccgagagg ccgaagaagg ccaaggagga gcggcgcatg<br>481 gaccagatga tgccaggaaa gaccaaagca gatgctgctg acccagcacc acttcccagc<br>541 caggagcccc ccaggaacag cacagaacag cagggcctgg cctctgggtc ttctgtgtcc<br>601 tttgtgggtg ccagcggctg tcctgaggcc tacaagcggc tcctatccag cttctactgc<br>661 aaggggacac acggcatcat gtcaccactg gccaaaaaga agctcctggc ccaggtgagc<br>721 aaggtggagg ccttgcagtg ccaggaggag ggctgccgcc atggggcaga gccccaggcg<br>781 tccccagctg ttcacctccc agagagtccc cagagcccca aagggctgac tgagaactcc<br>841 aggcaccggc tgacccctca ggagggattg caggccccag gtggcagcct cagagaggag<br>901 gcgcaggcag gcccctgccc ggcagccccc atcttcaagg gctgcttcta cacccacccc<br>961 accgaggtgc tgaagcctgt cagccagcac cccagggact tcttctctag acttaaagat<br>1021 ggggtgctat tggggcctcc tggcaaagag ggctgtcag tgaaagagcc ccagctggtg<br>1081 tggggcggag acgctaaccg cccttctgcg ttccataaag gtggctccag aaagggcatc | SEQ ID NO:237 |

| Gene Name | GenBank Homology | DNA SEQUENCE / DEDUCED AMINO ACID SEQUENCE | SEQUENCE IDENTIFIER: |
|---|---|---|---|
| | | 1141 ctctacccca agcccaaagc ctgctgggtg tcccccatgg ccaaggtccc agccgagagc<br>1201 cccacgctcc cgcccacctt ccccagtagc ccaggcctgg gcagcaagcg cagcctggag<br>1261 gaagagggtg ctgcccacag tgggaagaga ctgcgggccg tgtctccctt tcttaaggag<br>1321 gcggatgcca agaagtgtgg ggccaaacct gcagggtccg gcctggtctc ctgccttctg<br>1381 ggcccagccc tggggcctgt gcccccagag gcctacaggg gcaccatgct gcactgcccg<br>1441 ctgaacttca ctggcacccc gggccccttg aagggccagg ctgcactccc cttcagcccc<br>1501 ctggtcatcc cggccttccc ggcccacttc ctggccaccg caggcccctc gcccatggcc<br>1561 gctggcctga tgcacttccc cccaacgtcc ttcgacagtg ccctccgcca cagactttgc<br>1621 ccggcctcat ctgcctggca cgcaccacca gtcacaacct atgcagcgcc ccacttcttc<br>1681 cacctcaaca ccaagctgta ggccagccca tggtgttgtg tacactgtgg agtcgacagg<br>1741 ggcctacaac aggcaggtac tgctgccagg gggctctgaa ctagtgcctg ctacccagga<br>1801 caccccgggcc atgcccctgg ctgggcagcc tggcacaagt gaagaagaag gcagtgggaa<br>1861 aactgggttt atctcaaggc agcagcctga gcccaggagc agaggaccca gttgttataa<br>1921 ggcgctggga gaggatgggc agctcccact gccccagagc ggagctcgaa gcacccaggt<br>1981 tgcccacgga aaatccaata aaaagacacc agtgtgaatc caaaaaaaaa aaaaaaaaaa<br>2041 aaaaaaaaaa aaaaaaaaaa aaaaaaaaaa aaaaaaaaaa aaaaaaaaaa aaaaaaaaaa<br>2101 aaaaaaaaaa aaaaaaaaaa aaaaaaaaaa aaaaaaaa<br><br>1 M A K E N R G D D G A T E R P K K A K E<br>1 ATGGCTAAGGAGAACAGGGGGGATGATGGGGCCACCGAGAGGCCGAAGAAGGCCAAGGAG<br><br>21 E R R M D Q M M P G K T K A D A A D P A<br>61 GAGCGGCGCATGGACCAGATGATGCCAGGAAAGACCAAAGCAGATGCTGCTGACCCAGCA<br><br>41 P L P S Q E P P R N S T E Q Q G L A S G<br>121 CCACTTCCCAGCCAGGAGCCCCCCAGGAACAGCACAGAACAGCAGGGCCTGGCCTCTGGG<br><br>61 S S V S F V G A S G C P E A Y K R L L S<br>181 TCTTCTGTGTCCTTTGTGGGTGCCAGCGGCTGTCCTGAGGCCTACAAGCGGCTCCTATCC<br><br>81 S F Y C K G T H G I M S P L A K K K L L<br>241 AGCTTCTACTGCAAGGGGACACACGGCATCATGTCACCACTGGCCAAAAAGAAGCTCCTG<br><br>101 A Q V S K V E A L Q C Q E E G C R H G A<br>301 GCCCAGGTGAGCAAGGTGGAGGCCTTGCAGTGCCAGGAGGAGGGCTGCCGCCATGGGGCA<br><br>121 E P Q A S P A V H L P E S P Q S P K G L<br>361 GAGCCCCAGGCGTCCCCAGCTGTTCACCTCCCAGAGAGTCCCCAGAGCCCCAAAGGGCTG<br><br>141 T E N S R H R L T P Q E G L Q A P G G S<br>421 ACTGAGAACTCCAGGCACCGGCTGACCCCTCAGGAGGGATTGCAGGCCCCAGGTGGCAGC<br><br>161 L R E E A Q A G P C P A A P I F K G C F | SEQ ID NO:238 |

| Gene Name | GenBank Homology | DNA SEQUENCE / DEDUCED AMINO ACID SEQUENCE | SEQUENCE IDENTIFIER: |
|---|---|---|---|
| | | 481 CTCAGAGAGGAGGCGCAGGCAGGCCCCTGCCCGGCAGCCCCCATCTTCAAGGGCTGCTTC<br><br>181 Y T H P T E V L K P V S Q H P R D F F S<br>541 TACACCCACCCCACCGAGGTGCTGAAGCCTGTCAGCCAGCACCCCAGGGACTTCTTCTCT<br><br>201 R L K D G V L L G P P G K E G L S V K E<br>601 AGACTTAAAGATGGGGTGCTATTGGGGCCTCCTGGCAAAGAGGGGCTGTCAGTGAAAGAG<br><br>221 P Q L V W G G D A N R P S A F H K G G S<br>661 CCCCAGCTGGTGTGGGGCGGAGACGCTAACCGCCCTTCTGCGTTCCATAAAGGTGGCTCC<br><br>241 R K G I L Y P K P K A C W V S P M A K V<br>721 AGAAAGGGCATCCTCTACCCCAAGCCCAAAGCCTGCTGGGTGTCCCCCATGGCCAAGGTC<br><br>261 P A E S P T L P P T F P S S P G L G S K<br>781 CCAGCCGAGAGCCCCACGCTCCCGCCCACCTTCCCCAGTAGCCCAGGCCTGGGCAGCAAG<br><br>281 R S L E E E G A A H S G K R L R A V S P<br>841 CGCAGCCTGGAGGAAGAGGGTGCTGCCCACAGTGGGAAGAGACTGCGGGCCGTGTCTCCC<br><br>301 F L K E A D A K K C G A K P A G S G L V<br>901 TTTCTTAAGGAGGCGGATGCCAAGAAGTGTGGGGCCAAACCTGCAGGGTCCGGCCTGGTC<br><br>321 S C L L G P A L G P V P P E A Y R G T M<br>961 TCCTGCCTTCTGGGCCCAGCCCTGGGGCCTGTGCCCCCAGAGGCCTACAGGGGCACCATG<br><br>341 L H C P L N F T G T P G P L K G Q A A L<br>1021 CTGCACTGCCCGCTGAACTTCACTGGCACCCCGGGCCCCTTGAAGGGCCAGGCTGCACTC<br><br>361 P F S P L V I P A F P A H F L A T A G P<br>1081 CCCTTCAGCCCCCTGGTCATCCCGGCCTTCCCGGCCCACTTCCTGGCCACCGCAGGCCCC<br><br>381 S P M A A G L M H F P P T S F D S A L R<br>1141 TCGCCCATGGCCGCTGGCCTGATGCACTTCCCCCCAACGTCCTTCGACAGTGCCCTCCGC<br><br>401 H R L C P A S S A W H A P P V T T Y A A<br>1201 CACAGACTTTGCCCGGCCTCATCTGCCTGGCACGCACCACCAGTCACAACCTATGCAGCG<br><br>421 P H F F H L N T K L -<br>1261 CCCCACTTCTTCCACCTCAACACCAAGCTGTAG | |
| BM734654 | No homologies | 1 AGGGCGTCACGCTGACCGTGATGGTCAACACCAGCCAAACGCAGGTTTCGGAGAAGCTCG | SEQ ID NO:239 |

| Gene Name | GenBank Homology | DNA SEQUENCE / DEDUCED AMINO ACID SEQUENCE | SEQUENCE IDENTIFIER: |
|---|---|---|---|
| | | 61 TCTACACCAACCCAGGTGGTGAGGGCACCGCCGCCCAGAACTTCTCATGCTTCATCTACA<br>121 TCGTGCACTTCATGATCTGCACTTGGGCCCCGGGCCGCGCGGCCCCCGACGACGTCCAGT<br>181 ATTTCCTGTATATACGAGACTCCAAGAAGCGGGAGAGGGAATGTCCCCGTTACATGAGCG<br>241 ACTCGGGGACCCACGTGGGCTGCCACCTAGACGACCTCTCGGGATTGACCTCTTACAATT<br>301 ACTTCGTGGTGAACGGGACCAGCCAAACGACGGGGATCCAGTTCTTCGATTCCGTTATGC<br>361 GGTCCAAGCAAATAGAGAGGTACAGCCCGCCCGCCAACATCAGCGTGCAGGTAAAC | |
| BM734661 | Human UbA52 adrenal mRNA for ubiquitin-52 amino acid fusion protein. | 1 GACGCAGACATGCAGATCTTTGTGAAGACCCTGACGGGCAAGACCATCACCCTCGAGGTT<br>61 GAGCCCAGTGACACCATTGAGAATGTTAAAGCTAAAATCCAAGACAAGGAGGGCATCCCA<br>121 CCTGACCAGCAGCGTTTGATTTTTGCCGGCAAACAGCTGGAGGACGGCCGCACTCTCTCA<br>181 GACTACAATATCCAGAAAGAGTCCACCCTGCACTTGGTGCTCCGCCTGCGGGGCGGCATC<br>241 ATTGAGCCTTCCCTCCGCCAGCTCGCCCAGAAATACAACTGCGACAAGATGATTTGCCGC<br>301 AAGTGTTATGCTCGCCTGCACCCTCGTGCTGTCAACTGCCGCAAGAAGAAGTGCGGCCAC<br>361 ACCAACAACCTGCGCCCCAAGAAGAAGGTCAAATAAGGTTGTTCTTTCCTTGAAGGGCAG<br>421 CCTCCTGCCCAGGCCCCATGGCCCTGGGGCCTCAATAAAGTTTCCCTTTCATTGACTGGA<br>481 AAAAAAAAAAAAAAA | SEQ ID NO:240 |
| | | 1 M Q I F V K T L T G K T I T L E V E P S<br>1 ATGCAGATCTTTGTGAAGACCCTGACGGGCAAGACCATCACCCTCGAGGTTGAGCCCAGT<br><br>21 D T I E N V K A K I Q D K E G I P P D Q<br>61 GACACCATTGAGAATGTTAAAGCTAAAATCCAAGACAAGGAGGGCATCCCACCTGACCAG<br><br>41 Q R L I F A G K Q L E D G R T L S D Y N<br>121 CAGCGTTTGATTTTTGCCGGCAAACAGCTGGAGGACGGCCGCACTCTCTCAGACTACAAT<br><br>61 I Q K E S T L H L V L R L R G G I I E P<br>181 ATCCAGAAAGAGTCCACCCTGCACTTGGTGCTCCGCCTGCGGGGCGGCATCATTGAGCCT<br><br>81 S L R Q L A Q K Y N C D K M I C R K C Y<br>241 TCCCTCCGCCAGCTCGCCCAGAAATACAACTGCGACAAGATGATTTGCCGCAAGTGTTAT<br><br>101 A R L H P R A V N C R K K K C G H T N N<br>301 GCTCGCCTGCACCCTCGTGCTGTCAACTGCCGCAAGAAGAAGTGCGGCCACACCAACAAC<br><br>121 L R P K K K V K -<br>361 CTGCGCCCCAAGAAGAAGGTCAAATAA | SEQ ID NO:241 |
| BM734694 | Human hemopoietic cell protein-tyrosine kinase (HCK) gene. | 1 GAATTCCTTTCTAAAATCCAACCATTCCAGGAAATAGAAATATCAACTTGGGGGCTTCCT<br>61 GAGAATGTCAGATTGATGGGGTGCATGAAGTCCAAGTTCCTCCAGGTCGGAGGCAATACA<br>121 TTCTCAAAAACTGAAACCAGCGCCAGCCCACACTGTCCTGTGTACGTGCCGGATCCCACA | SEQ ID NO:242 |

| Gene Name | GenBank Homology | DNA SEQUENCE / DEDUCED AMINO ACID SEQUENCE | SEQUENCE IDENTIFIER: |
|---|---|---|---|
| | | 181 TCCACCATCAAGCCGGGGCCTAATAGCCACAACAGCAACACACCAGGAATCAGGGAGGCA<br>241 GGCTCTGAGGACATCATCGTGGTTGCCCTGTATGATTACGAGGCCATTCACCACGAAGAC<br>301 CTCAGCTTCCAGAAGGGGGACCAGATGGTGGTCCTAGAGGAATCCGGGGAGTGGTGGAAG<br>361 GCTCGATCCCTGGCCACCCGGAAGGAGGGCTACATCCCAAGCAACTATGTCGCCCGCGTT<br>421 GACTCTCTGGAGACAGAGGAGTGGTTTTTCAAGGGCATCAGCCGGAAGGACGCAGAGCGC<br>481 CAACTGCTGGCTCCCGGCAACATGCTGGGCTCCTTCATGATCCGGGATAGCGAGACCACT<br>541 AAAGGAAGCTACTCTTTGTCCGTGCGAGACTACGACCCTCGGCAGGGAGATACCGTGAAA<br>601 CATTACAAGATCCGGACCCTGGACAACGGGGGCTTCTACATATCCCCCCGAAGCACCTTC<br>661 AGCACTCTGCAGGAGCTGGTGGACCACTACAAGAAGGGGAACGACGGGCTCTGCCAGAAA<br>721 CTGTCGGTGCCCTGCATGTCTTCCAAGCCCCAGAAGCCTTGGGAGAAAGATGCCTGGGAG<br>781 ATCCCTCGGGAATCCCTCAAGCTGGAGAAGAAACTTGGAGCTGGGCAGTTTGGGGAAGTC<br>841 TGGATGGCCACCTACAACAAGCACACCAAGGTGGCAGTGAAGACGATGAAGCCAGGGAGC<br>901 ATGTCGGTGGAGGCCTTCCTGGCAGAGGCCAACGTGATGAAAACTCTGCAGCATGACAAG<br>961 CTGGTCAAACTTCATGCGGTGGTCACCAAGGAGCCCATCTACATCATCACGGAGTTCATG<br>1021 GCCAAAGGAAGCTTGCTGGACTTTCTGAAAAGTGATGAGGGCAGCAAGCAGCCATTGCCA<br>1081 AAACTCATTGACTTCTCAGCCCAGATTGCAGAAGGCATGGCCTTCATCGAGCAGAGGAAC<br>1141 TACATCCACCGAGACCTCCGAGCTGCCAACATCTTGGTCTCTGCATCCCTGGTGTGTAAG<br>1201 ATTGCTGACTTTGGCCTGGCCCGGGTCATTGAGGACAACGAGTACACGGCTCGGGAAGGG<br>1261 GCCAAGTTCCCCATCAAGTGGACAGCTCNTGAGGCCATCAACTTTGGCTCCTTCACCATC<br>1321 AAGTCGGATGTCTGGTCCTTTGGTGTCCTGCTGATGGAGATTGTCACCTATGGCAGGATC<br>1381 CCTTACCCAGGGATGTCGAACCCCGAGGTGATCCGAGCCCTGGAGCACGGATACAGGATG<br>1441 CCTCGACCAGAGCACTGCCCCGAGGAGCTTTACAACATCATGACCCGCTGCTGGAAGAAC<br>1501 AGCCCCGAGGAGCGGCCCACCTTCGAGTACATCCAGAGTGTGCTGGAGGACTTCTACACC<br>1561 GCCACCGAGAGCCAGTACCAACAGCAGCCGTGATGGGTGGACCAGGGCAGGGCAGGGGG<br>1621 TGCCCAGGTGGTGGCTGCAAGGTGGCTCCAGCACCATCTGCCCCGGGGCCCACTCACCCT<br>1681 TCCCACTCCCAGACACCCACTCTCGCTCCAGCCACAGTTCCTCATCTGTCAAGGGGGTGG<br>1741 GTTGGACTGGACAATCTCTTTTTGACTCTAGCAATCCACAATCTGCCATTCTCAGGAGAC<br>1801 CCCCAAGTTGACATTTCTATGGCCTGGGAGAGTTGGATTCCACTCACAGCTGTGGTTTGG<br>1861 ATGGGAAACTTTAAAAATAATGAAATAAATATTTAAATAAAAGATATAAATGCCAAAGTC<br>1921 TTTACC | |
| | | 1 M G C M K S K F L Q V G G N T F S K T E<br>1 ATGGGGTGCATGAAGTCCAAGTTCCTCCAGGTCGGAGGCAATACATTCTCAAAAACTGAA<br><br>21 T S A S P H C P V Y V P D P T S T I K P<br>61 ACCAGCGCCAGCCCACACTGTCCTGTGTACGTGCCGGATCCCACATCCACCATCAAGCCG<br><br>41 G P N S H N S N T P G I R E A G S E D I<br>121 GGGCCTAATAGCCACAACAGCAACACACCAGGAATCAGGGAGGCAGGCTCTGAGGACATC<br><br>61 I V V A L Y D Y E A I H H E D L S F Q K<br>181 ATCGTGGTTGCCCTGTATGATTACGAGGCCATTCACCACGAAGACCTCAGCTTCCAGAAG | SEQ ID NO:243 |

| Gene Name | GenBank Homology | DNA SEQUENCE / DEDUCED AMINO ACID SEQUENCE | SEQUENCE IDENTIFIER: |
|---|---|---|---|
| | | 81 G D Q M V V L E E S G E W W K A R S L A<br>241 GGGGACCAGATGGTGGTCCTAGAGGAATCCGGGGAGTGGTGGAAGGCTCGATCCCTGGCC<br><br>101 T R K E G Y I P S N Y V A R V D S L E T<br>301 ACCCGGAAGGAGGGCTACATCCCAAGCAACTATGTCGCCCGCGTTGACTCTCTGGAGACA<br><br>121 E E W F F K G I S R K D A E R Q L L A P<br>361 GAGGAGTGGTTTTTCAAGGGCATCAGCCGGAAGGACGCAGAGCGCCAACTGCTGGCTCCC<br><br>141 G N M L G S F M I R D S E T T K G S Y S<br>421 GGCAACATGCTGGGCTCCTTCATGATCCGGGATAGCGAGACCACTAAAGGAAGCTACTCT<br><br>161 L S V R D Y D P R Q G D T V K H Y K I R<br>481 TTGTCCGTGCGAGACTACGACCCTCGGCAGGGAGATACCGTGAAACATTACAAGATCCGG<br><br>181 T L D N G G F Y I S P R S T F S T L Q E<br>541 ACCCTGGACAACGGGGGCTTCTACATATCCCCCCGAAGCACCTTCAGCACTCTGCAGGAG<br><br>201 L V D H Y K K G N D G L C Q K L S V P C<br>601 CTGGTGGACCACTACAAGAAGGGGAACGACGGGCTCTGCCAGAAACTGTCGGTGCCCTGC<br><br>221 M S S K P Q K P W E K D A W E I P R E S<br>661 ATGTCTTCCAAGCCCCAGAAGCCTTGGGAGAAAGATGCCTGGGAGATCCCTCGGGAATCC<br><br>241 L K L E K K L G A G Q F G E V W M A T Y<br>721 CTCAAGCTGGAGAAGAAACTTGGAGCTGGGCAGTTTGGGGAAGTCTGGATGGCCACCTAC<br><br>261 N K H T K V A V K T M K P G S M S V E A<br>781 AACAAGCACACCAAGGTGGCAGTGAAGACGATGAAGCCAGGGAGCATGTCGGTGGAGGCC<br><br>281 F L A E A N V M K T L Q H D K L V K L H<br>841 TTCCTGGCAGAGGCCAACGTGATGAAAACTCTGCAGCATGACAAGCTGGTCAAACTTCAT<br><br>301 A V V T K E P I Y I I T E F M A K G S L<br>901 GCGGTGGTCACCAAGGAGCCCATCTACATCATCACGGAGTTCATGGCCAAAGGAAGCTTG<br><br>321 L D F L K S D E G S K Q P L P K L I D F<br>961 CTGGACTTTCTGAAAAGTGATGAGGGCAGCAAGCAGCCATTGCCAAAACTCATTGACTTC<br><br>341 S A Q I A E G M A F I E Q R N Y I H R D<br>1021 TCAGCCCAGATTGCAGAAGGCATGGCCTTCATCGAGCAGAGGAACTACATCCACCGAGAC<br><br>361 L R A A N I L V S A S L V C K I A D F G | |

| Gene Name | GenBank Homology | DNA SEQUENCE / DEDUCED AMINO ACID SEQUENCE | SEQUENCE IDENTIFIER: |
|---|---|---|---|
| | | 1081 CTCCGAGCTGCCAACATCTTGGTCTCTGCATCCCTGGTGTGTAAGATTGCTGACTTTGGC<br><br>381 L A R V I E D N E Y T A R E G A K F P I<br>1141 CTGGCCCGGGTCATTGAGGACAACGAGTACACGGCTCGGGAAGGGGCCAAGTTCCCCATC<br><br>401 K W T A X E A I N F G S F T I K S D V W<br>1201 AAGTGGACAGCTCNTGAGGCCATCAACTTTGGCTCCTTCACCATCAAGTCGGATGTCTGG<br><br>421 S F G V L L M E I V T Y G R I P Y P G M<br>1261 TCCTTTGGTGTCCTGCTGATGGAGATTGTCACCTATGGCAGGATCCCTTACCCAGGGATG<br><br>441 S N P E V I R A L E H G Y R M P R P E H<br>1321 TCGAACCCCGAGGTGATCCGAGCCCTGGAGCACGGATACAGGATGCCTCGACCAGAGCAC<br><br>461 C P E E L Y N I M T R C W K N S P E E R<br>1381 TGCCCCGAGGAGCTTTACAACATCATGACCCGCTGCTGGAAGAACAGCCCCGAGGAGCGG<br><br>481 P T F E Y I Q S V L E D F Y T A T E S Q<br>1441 CCCACCTTCGAGTACATCCAGAGTGTGCTGGAGGACTTCTACACCGCCACCGAGAGCCAG<br><br>501 Y Q Q Q P -<br>1501 TACCAACAGCAGCCGTGA | |
| BM734719 | No Homology | 1 AGTCCTCTCGATGAAGCTTCTTCCCACCTAGAAAGAAGTCCTCTCTGAGACTCAAGGGCT<br>61 AAGGCAAGGTCTTCCAGAAAACAAGGCTTTGACTCCAAGAACAGAGGTGATGGGGAGACT<br>121 TCTTGGCTCGGCGGGGAAAGGAGGACGCCAATGGGTCAAACTAACTCTGAATCCCTCCCT<br>181 CCATCTCTCTTTTTCCACTGTCGTCAGAGCCAACAAGAAATGACAGCCTCCAAGCCTTCC<br>241 TAAAAGCACACTTGCCCCCGCCTGCCACCTCTCCTCAGGCTGCTGCCCTCCCAGGCGCTC<br>301 CACAGCGAAGGGGTGTGGTGTTTCCTGCAGGCCAGGCCAGCTGCCTCAGCCCCGCCAAAA<br>361 CTGTGTTCCCAGCCAGCGCTCTGGAGCAGGGATGACGTGTGGCCAGCCCTTCTCAGGTCT<br>421 CAGCTCTGCTGGGATGGGAGGAGAGGCCAGGGAGAAAGGTTAGGGGCCCAGGGGTGGGTG<br>481 ACAACGCTGGCTGCTGAAAGCCCATGAGCTGCTTCTTTGTTCTCTGTCACGGGACAAAAA<br>541 TCTCTCATCCTATTCTGCTTTCAGTTCATTAAGAGAGCACATTTTACTCATACACAAATA<br>601 AAAGTTTTCCCTTGTGGAAACAACAGCTTTAAAAGAAAGG | SEQ ID NO:244 |
| BM734722 | No Homology | 1 ACCGCGACTTCCAGTACGAGCTGGTCATCCAGAGACAGAGGATCGAGGTGCCGGGCGACT<br>61 CGCAGAATCAATACATTTTCCCGAGTCTGGAGCCCAGACCGAAGCACACGGTGAAGATCA<br>121 GGACGGGGGACGCCCGGGACGCCCAGGACCCCCGCTGGGGCGCCTGGAGCCGGCCCGTCG<br>181 AGTTCGGCTCCGAGGACCCGGATGCGGGGGTCGTGCACGTGTACGTGCTCGTCGTGCTGG<br>241 GGACCCTGGGCTGCGCGCTGGCGCTCGGCTGCCTGGTGAAGAGGTTCCTCCGGAGACACA<br>301 GTCTGTTCCAGCCGGTCCCGCGCATCAAAGACAAACTGAACGATAACCATCCGACCGACC | SEQ ID NO:245 |

| Gene Name | GenBank Homology | DNA SEQUENCE / DEDUCED AMINO ACID SEQUENCE | SEQUENCE IDENTIFIER: |
|---|---|---|---|
| | | 361 TCCAGGACGTCTGGGAGGAATTCCCGGCGGGGGCCGGAAAAGCCGAGCACGAAGAGGTGG<br>421 TGACCGTCCAGGAGGTCGACAGAGCCCCGGTCAGCGGGTGAAAGGTCACGGGCACCTCCG<br>481 GCCCCCAGACACCCCGGAAGCCGCCCGGGTTTTCTCAAAACCGTTTTAAGCGTTTCTGCA<br>541 GCTATTTTTCTACTTTTTATTAAAAAAATAAATGTGAAGTTTGGCCAAGAGGCTTCCCGA<br>601 CGGGGGGCCGGAAAAAAGGCCCAAAGCTT | |
| BM734780 | Homo sapiens STAT4 mRNA. | 1 GCTTTCTCCTAGGGACTGTGAGGGGCGCTTCTGACTTTGGACTTGAGCACTGCCTGGGAC<br>61 CTGTGCTGAGAGAGCGCTAGCATGTCTCAGTGGAATCAAGTCCAACAGTTAGAAATCAAG<br>121 TTTTTGGAGCAGGTGGATCAATTCTATGATGACAACTTTCCCATGGAAATTCGGCATCTG<br>181 TTGGCCCAATGGATTGAAAATCAAGACTGGGAGGCAGCTTCTAACAATGAAACCATGGCA<br>241 ACGATTCTTCTTCAAAACTTGTTAATACAACTGGATGAACAGTTAGGTCGTGTTTCCAAA<br>301 GAGAAAAACCTACTCTTGATACACAATCTAAAAAGAATTAGGAAGGTCCTTCAGGGAAAA<br>361 TTTCATGGAAATCCAATGCATGTAGCTGTGGTTATTTCAAACTGTTTAAGGGAAGAGAGG<br>421 AGAATATTGGCTGCAGCCAACATGCCTGTCCAGGGGCCTCTAGAGAAATCCTTACAAAGT<br>481 TCTTCAGTTTCAGAAAGACAGAGGAATGTGGAGCACAAAGTGGCTGCCATTAAAAACAGT<br>541 GTGCAGATGACAGAACAAGATACCAAATACTTAGAAGATCTGCAAGACGAATTTGACTAC<br>601 AGGTATAAAACAATTCAGACAATGGATCAGAGTGACAAGAATAGTGCCATGGTGAATCAG<br>661 GAAGTTTTGACACTGCAGGAAATGCTTAACAGCCTCGATTTCAAGAGAAAGGAGGCTCTC<br>721 AGTAAAATGACCCAAATCATCCATGAGACAGACCTGTTAATGAACACCATGCTCATAGAA<br>781 GAGCTGCAAGACTGGAAGCGGCGGCAGCAAATCGCCTGCATCGGGGGTCCACTCCACAAT<br>841 GGGCTCGACCAGCTTCAGAACTGCTTTACACTATTGGCAGAAAGTCTTTTCCAACTGAGA<br>901 AGGCAATTGGAGAAACTAGAGGAGCAATCTACCAAAATGACATATGAAGGTGATCCCATT<br>961 CCAATGCAAAGAACTCACATGCTAGAAAGAGTCACCTTCTTGATCTACAACCTTTTCAAG<br>1021 AACTCATTTGTGGTTGAGCGACAGCCATGTATGCCAACCCACCCTCAGAGGCCGTTGGTA<br>1081 CTTAAAACCCTAATTCAGTTCACTGTAAAACTAAGGCTACTAATAAAATTGCCAGAACTA<br>1141 AACTATCAGGTAAAGGTTAAGGCATCAATTGACAAGAATGTTTCAACTCTAAGCAACCGA<br>1201 AGATTTGTACTTTGTGGAACTAATGTCAAAGCCATGTCTATTGAAGAATCTTCCAATGGG<br>1261 AGTCTCTCAGTAGAATTTCGACATTTGCAACCAAAGGAAATGAAGTCCAGTGCTGGAGGT<br>1321 AAAGGAAATGAGGGCTGTCACATGGTGACTGAAGAACTTCATTCCATAACGTTTGAAACA<br>1381 CAGATCTGCCTCTATGGCCTGACCATAGATTTGGAGACCAGCTCATTGCCTGTGGTGATG<br>1441 ATTTCCAATGTCAGTCAGTTACCTAATGCTTGGGCATCCATCATTTGGTACAACGTGTCA<br>1501 ACCAACGATTCCCAGAACTTGGTTTTCTTTAATAATCCTCCACCTGCCACATTGAGTCAA<br>1561 CTACTGGAGGTGATGAGCTGGCAGTTTTCATCGTACGTTGGTCGTGGTCTTAACTCAGAT<br>1621 CAACTCCATATGCTGGCAGAGAAGCTTACAGTCCAATCTAGCTACAGTGATGGTCACCTC<br>1681 ACCTGGGCCAAGTTCTGCAAGGAACATTTACCTGGTAAATCATTTACCTTTTGGACATGG<br>1741 CTTGAAGCAATATTGGATCTAATTAAGAAACACATTCTTCCCCTTTGGATTGATGGGTAT<br>1801 GTCATGGGCTTTGTTAGCAAAGAGAAGGAACGGCTGTTGCTAAAGGATAAAATGCCTGGC<br>1861 ACCTTTTTATTAAGATTCAGTGAAAGCCATCTCGGAGGAATAACTTTCACCTGGGTGGAC<br>1921 CATTCTGAAAGTGGGGAAGTGAGATTCCACTCTGTAGAGCCCTACAATAAAGGCCGCTTG<br>1981 TCTGCTTTGCCGTTTGCTGACATCCTTCGAGACTACAAGGTTATTATGGCTGAAAACATT<br>2041 CCTGAAAACCCTCTGAAGTACCTGTATCCTGACATTCCCAAAGACAAAGCCTTCGGTAAA<br>2101 CACTACAGCTCCCAGCCGTGCGAAGTTTCAAGACCAACAGAAAGAGGAGACAAAGGTTAT | SEQ ID NO:246 |

| Gene Name | GenBank Homology | DNA SEQUENCE / DEDUCED AMINO ACID SEQUENCE | SEQUENCE IDENTIFIER: |
|---|---|---|---|
| | | 2161 GTTCCTTCTGTTTTTATCCCCATCTCAACAATCCGAAGCGATTCCACTGAGCCGCAGTCT<br>2221 CCATCAGACCTTCTTCCCATGTCTCCAAGCGTATACGCAGTGCTGAGAGAAAACCTGAGT<br>2281 CCCACGACCATCGAAACTGCGATGAAGTCTCCATATTCTGCCGAATGACAGGATGACCTC<br>2341 TTGACACTCAAAAAATGGAAGCAAATGGAAAACTTTAAGGACTGATCTTTGCCAACAGTC<br>2401 ATCTCTTATTCTTCATCTTTGTATATACGGATTTCCAGGAAACGGTTGAAGTCTGAAGCT<br>2461 CTGCTCTCACTGGCATGACACTCCCAATTGGGTGTGTTGTGACTGAAATGTTAAAACCAA<br>2521 AGCTTCAGATAAACTTGCAAGATAAGACACTTTAAAAAATTAGTGTTAATATAATAACAA<br>2581 TATTAACAG | |
| | | 1 M S Q W N Q V Q Q L E I K F L E Q V D Q<br>1 ATGTCTCAGTGGAATCAAGTCCAACAGTTAGAAATCAAGTTTTTGGAGCAGGTGGATCAA<br><br>21 F Y D D N F P M E I R H L L A Q W I E N<br>61 TTCTATGATGACAACTTTCCCATGGAAATTCGGCATCTGTTGGCCCAATGGATTGAAAAT<br><br>41 Q D W E A A S N N E T M A T I L L Q N L<br>121 CAAGACTGGGAGGCAGCTTCTAACAATGAAACCATGGCAACGATTCTTCTTCAAAACTTG<br><br>61 L I Q L D E Q L G R V S K E K N L L L I<br>181 TTAATACAACTGGATGAACAGTTAGGTCGTGTTTCCAAAGAGAAAAACCTACTCTTGATA<br><br>81 H N L K R I R K V L Q G K F H G N P M H<br>241 CACAATCTAAAAAGAATTAGGAAGGTCCTTCAGGGAAAATTTCATGGAAATCCAATGCAT<br><br>101 V A V V I S N C L R E E R R I L A A A N<br>301 GTAGCTGTGGTTATTTCAAACTGTTTAAGGGAAGAGAGGAGAATATTGGCTGCAGCCAAC<br><br>121 M P V Q G P L E K S L Q S S S V S E R Q<br>361 ATGCCTGTCCAGGGGCCTCTAGAGAAATCCTTACAAAGTTCTTCAGTTTCAGAAAGACAG<br><br>141 R N V E H K V A A I K N S V Q M T E Q D<br>421 AGGAATGTGGAGCACAAAGTGGCTGCCATTAAAAACAGTGTGCAGATGACAGAACAAGAT<br><br>161 T K Y L E D L Q D E F D Y R Y K T I Q T<br>481 ACCAAATACTTAGAAGATCTGCAAGACGAATTTGACTACAGGTATAAAACAATTCAGACA<br><br>181 M D Q S D K N S A M V N Q E V L T L Q E<br>541 ATGGATCAGAGTGACAAGAATAGTGCCATGGTGAATCAGGAAGTTTTGACACTGCAGGAA<br><br>201 M L N S L D F K R K E A L S K M T Q I I<br>601 ATGCTTAACAGCCTCGATTTCAAGAGAAAGGAGGCTCTCAGTAAAATGACCCAAATCATC<br><br>221 H E T D L L M N T M L I E E L Q D W K R | SEQ ID NO:247 |

| Gene Name | GenBank Homology | DNA SEQUENCE / DEDUCED AMINO ACID SEQUENCE | SEQUENCE IDENTIFIER: |
|---|---|---|---|
| | | 661 CATGAGACAGACCTGTTAATGAACACCATGCTCATAGAAGAGCTGCAAGACTGGAAGCGG<br><br>241 R Q Q I A C I G G P L H N G L D Q L Q N<br>721 CGGCAGCAAATCGCCTGCATCGGGGGTCCACTCCACAATGGGCTCGACCAGCTTCAGAAC<br><br>261 C F T L L A E S L F Q L R R Q L E K L E<br>781 TGCTTTACACTATTGGCAGAAAGTCTTTTCCAACTGAGAAGGCAATTGGAGAAACTAGAG<br><br>281 E Q S T K M T Y E G D P I P M Q R T H M<br>841 GAGCAATCTACCAAAATGACATATGAAGGTGATCCCATTCCAATGCAAAGAACTCACATG<br><br>301 L E R V T F L I Y N L F K N S F V V E R<br>901 CTAGAAAGAGTCACCTTCTTGATCTACAACCTTTTCAAGAACTCATTTGTGGTTGAGCGA<br><br>321 Q P C M P T H P Q R P L V L K T L I Q F<br>961 CAGCCATGTATGCCAACCCACCCTCAGAGGCCGTTGGTACTTAAAACCCTAATTCAGTTC<br><br>341 T V K L R L L I K L P E L N Y Q V K V K<br>1021 ACTGTAAAACTAAGGCTACTAATAAAATTGCCAGAACTAAACTATCAGGTAAAGGTTAAG<br><br>361 A S I D K N V S T L S N R R F V L C G T<br>1081 GCATCAATTGACAAGAATGTTTCAACTCTAAGCAACCGAAGATTTGTACTTTGTGGAACT<br><br>381 N V K A M S I E E S S N G S L S V E F R<br>1141 AATGTCAAAGCCATGTCTATTGAAGAATCTTCCAATGGGAGTCTCTCAGTAGAATTTCGA<br><br>401 H L Q P K E M K S S A G G K G N E G C H<br>1201 CATTTGCAACCAAAGGAAATGAAGTCCAGTGCTGGAGGTAAAGGAAATGAGGGCTGTCAC<br><br>421 M V T E E L H S I T F E T Q I C L Y G L<br>1261 ATGGTGACTGAAGAACTTCATTCCATAACGTTTGAAACACAGATCTGCCTCTATGGCCTG<br><br>441 T I D L E T S S L P V V M I S N V S Q L<br>1321 ACCATAGATTTGGAGACCAGCTCATTGCCTGTGGTGATGATTTCCAATGTCAGTCAGTTA<br><br>461 P N A W A S I I W Y N V S T N D S Q N L<br>1381 CCTAATGCTTGGGCATCCATCATTTGGTACAACGTGTCAACCAACGATTCCCAGAACTTG<br><br>481 V F F N N P P P A T L S Q L L E V M S W<br>1441 GTTTTCTTTAATAATCCTCCACCTGCCACATTGAGTCAACTACTGGAGGTGATGAGCTGG<br><br>501 Q F S S Y V G R G L N S D Q L H M L A E<br>1501 CAGTTTTCATCGTACGTTGGTCGTGGTCTTAACTCAGATCAACTCCATATGCTGGCAGAG | |

| Gene Name | GenBank Homology | DNA SEQUENCE / DEDUCED AMINO ACID SEQUENCE | SEQUENCE IDENTIFIER: |
|---|---|---|---|
| | | 521 K L T V Q S S Y S D G H L T W A K F C K<br>1561 AAGCTTACAGTCCAATCTAGCTACAGTGATGGTCACCTCACCTGGGCCAAGTTCTGCAAG<br><br>541 E H L P G K S F T F W T W L E A I L D L<br>1621 GAACATTTACCTGGTAAATCATTTACCTTTTGGACATGGCTTGAAGCAATATTGGATCTA<br><br>561 I K K H I L P L W I D G Y V M G F V S K<br>1681 ATTAAGAAACACATTCTTCCCCTTTGGATTGATGGGTATGTCATGGGCTTTGTTAGCAAA<br><br>581 E K E R L L L K D K M P G T F L L R F S<br>1741 GAGAAGGAACGGCTGTTGCTAAAGGATAAAATGCCTGGCACCTTTTTATTAAGATTCAGT<br><br>601 E S H L G G I T F T W V D H S E S G E V<br>1801 GAAAGCCATCTCGGAGGAATAACTTTCACCTGGGTGGACCATTCTGAAAGTGGGGAAGTG<br><br>621 R F H S V E P Y N K G R L S A L P F A D<br>1861 AGATTCCACTCTGTAGAGCCCTACAATAAAGGCCGCTTGTCTGCTTTGCCGTTTGCTGAC<br><br>641 I L R D Y K V I M A E N I P E N P L K Y<br>1921 ATCCTTCGAGACTACAAGGTTATTATGGCTGAAAACATTCCTGAAAACCCTCTGAAGTAC<br><br>661 L Y P D I P K D K A F G K H Y S S Q P C<br>1981 CTGTATCCTGACATTCCCAAAGACAAAGCCTTCGGTAAACACTACAGCTCCCAGCCGTGC<br><br>681 E V S R P T E R G D K G Y V P S V F I P<br>2041 GAAGTTTCAAGACCAACAGAAAGAGGAGACAAAGGTTATGTTCCTTCTGTTTTTATCCCC<br><br>701 I S T I R S D S T E P Q S P S D L L P M<br>2101 ATCTCAACAATCCGAAGCGATTCCACTGAGCCGCAGTCTCCATCAGACCTTCTTCCCATG<br><br>721 S P S V Y A V L R E N L S P T T I E T A<br>2161 TCTCCAAGCGTATACGCAGTGCTGAGAGAAAACCTGAGTCCCACGACCATCGAAACTGCG<br><br>741 M K S P Y S A E -<br>2221 ATGAAGTCTCCATATTCTGCCGAATGA | |
| BM734795 | Homo sapiens granzyme A (granzyme 1, cytotoxic T-lymphocyte-associated serine | 1 cagattttca ggttgattga tgtgggacag cagccacaat gaggaactcc tatagatttc<br>61 tggcatcctc tctctcagtt gtcgtttctc tcctgctaat tcctgaagat gtctgtgaaa<br>121 aaattattgg aggaaatgaa gtaactcctc attcaagacc ctacatggtc ctacttagtc<br>181 ttgacagaaa aaccatctgt gctggggctt tgattgcaaa agactgggtg ttgactgcag<br>241 ctcactgtaa cttgaacaaa aggtcccagg tcattcttgg ggctcactca ataaccaggg | SEQ ID NO:248 |

| Gene Name | GenBank Homology | DNA SEQUENCE / DEDUCED AMINO ACID SEQUENCE | SEQUENCE IDENTIFIER: |
|---|---|---|---|
| | esterase 3) (GZMA) | 301 aagagccaac aaaacagata atgcttgtta agaaagagtt tccctatcca tgctatgacc<br>361 cagccacacg cgaaggtgac cttaaacttt tacagctgac ggaaaaagca aaaattaaca<br>421 aatatgtgac tatccttcat ctacctaaaa agggggatga tgtgaaacca ggaaccatgt<br>481 gccaagttgc agggtggggg aggactcaca atagtgcatc ttggtccgat actctgagag<br>541 aagtcaatat caccatcata gacagaaaag tctgcaatga tcgaaatcac tataatttta<br>601 accctgtgat tggaatgaat atggtttgtg ctggaagcct ccgaggtgga agagactcgt<br>661 gcaatggaga ttctggaagc cctttgttgt gcgagggtgt tttccgaggg gtcacttcct<br>721 ttggccttga aaataaatgc ggagaccctc gtgggcctgg tgtctatatt cttctctcaa<br>781 agaaacacct caactggata attatgacta tcaagggagc agtttaaata accgtttcct<br>841 ttcatttact gtggcttctt aatcttttca caaataaa | |
| | | 1 M R N S Y R F L A S S L S V V V S L L L<br>1 ATGAGGAACTCCTATAGATTTCTGGCATCCTCTCTCTCAGTTGTCGTTTCTCTCCTGCTA<br><br>21 I P E D V C E K I I G G N E V T P H S R<br>61 ATTCCTGAAGATGTCTGTGAAAAAATTATTGGAGGAAATGAAGTAACTCCTCATTCAAGA<br><br>41 P Y M V L L S L D R K T I C A G A L I A<br>121 CCCTACATGGTCCTACTTAGTCTTGACAGAAAAACCATCTGTGCTGGGGCTTTGATTGCA<br><br>61 K D W V L T A A H C N L N K R S Q V I L<br>181 AAAGACTGGGTGTTGACTGCAGCTCACTGTAACTTGAACAAAAGGTCCCAGGTCATTCTT<br><br>81 G A H S I T R E E P T K Q I M L V K K E<br>241 GGGGCTCACTCAATAACCAGGGAAGAGCCAACAAAACAGATAATGCTTGTTAAGAAAGAG<br><br>101 F P Y P C Y D P A T R E G D L K L L Q L<br>301 TTTCCCTATCCATGCTATGACCCAGCCACACGCGAAGGTGACCTTAAACTTTTACAGCTG<br><br>121 T E K A K I N K Y V T I L H L P K K G D<br>361 ACGGAAAAAGCAAAAATTAACAAATATGTGACTATCCTTCATCTACCTAAAAAGGGGGAT<br><br>141 D V K P G T M C Q V A G W G R T H N S A<br>421 GATGTGAAACCAGGAACCATGTGCCAAGTTGCAGGGTGGGGGAGGACTCACAATAGTGCA<br><br>161 S W S D T L R E V N I T I I D R K V C N<br>481 TCTTGGTCCGATACTCTGAGAGAAGTCAATATCACCATCATAGACAGAAAAGTCTGCAAT<br><br>181 D R N H Y N F N P V I G M N M V C A G S<br>541 GATCGAAATCACTATAATTTTAACCCTGTGATTGGAATGAATATGGTTTGTGCTGGAAGC<br><br>201 L R G G R D S C N G D S G S P L L C E G<br>601 CTCCGAGGTGGAAGAGACTCGTGCAATGGAGATTCTGGAAGCCCTTTGTTGTGCGAGGGT | *SEQ ID NO:249* |

| Gene Name | GenBank Homology | DNA SEQUENCE / DEDUCED AMINO ACID SEQUENCE | SEQUENCE IDENTIFIER: |
|---|---|---|---|
| | | 221 V F R G V T S F G L E N K C G D P R G P<br>661 GTTTTCCGAGGGGTCACTTCCTTTGGCCTTGAAAATAAATGCGGAGACCCTCGTGGGCCT<br><br>241 G V Y I L L S K K H L N W I I M T I K G<br>721 GGTGTCTATATTCTTCTCTCAAAGAAACACCTCAACTGGATAATTATGACTATCAAGGGA<br><br>261 A V -<br>781 GCAGTTTAA | |
| BM734844 | Homo sapiens phosphate cytidylyltransferase 1, choline, alpha isoform, mRNA (cDNA clone MGC:49915 IMAGE:5744747), | 1 cccgggcgtc tccccgcaac ctcctcaact tccctagtca gtgacgcggc gccggccaga<br>61 aatccgaccg gaccgggctc gggggagcgt gagttgcagc atgtgccgaa gcgccacctc<br>121 agaagataaa aagaaatgag tctcatattt ggcattcttt tagttaaaag aagatggatg<br>181 cacagtgttc agccaaggtc aatgcaagga agaggagaaa agaggcgccc ggacccaacg<br>241 gggcaacaga agaagatggg gttccttcca aagtgcagcg ctgtgcagtg ggcttacggc<br>301 aaccagctcc tttttctgat gaaattgaag ttgactttag taagccctat gtcagggtaa<br>361 ctatggaaga agccagcaga ggaactcctt gtgagcgacc tgtgagagtt tatgccgatg<br>421 gaatatttga cttatttcac tctggtcacg cccgagctct gatgcaagcg aagaaccttt<br>481 tccctaatac gtacctcatt gtgggagttt gcagtgatga gctcacacac aacttcaaag<br>541 gcttcacggt gatgaacgag aatgagcgct atgacgcagt ccagcactgc cgctacgtgg<br>601 atgaggtggt gaggaatgcg ccctggacgc tgacacccga gttcctggcc gaacaccgga<br>661 ttgattttgt agcccatgat gatattcctt attcatctgc tggcagtgat gatgtttata<br>721 agcacatcaa ggaggcaggc atgtttgctc caacacagag gacagaaggt atctccacat<br>781 cagacatcat cacccgaatt gtgcgggatt atgatgtgta tgcgaggcgg aacctgcaga<br>841 ggggctacac agcaaaggag ctcaatgtca gctttatcaa cgagaagaaa taccacttgc<br>901 aggagagggt tgacaaagta aagaagaaag tgaaagatgt ggaggaaaag tcaaaagaat<br>961 ttgttcagaa ggtggaggaa aaaagcattg acctcattca gaagtgggag gagaagtccc<br>1021 gagaattcat tggaagtttt ctggaaatgt ttggtccgga aggagcactg aaacatatgc<br>1081 tgaaagaggg gaagggccgg atgctgcagg ccatcagccc gaagcagagc cccagcagca<br>1141 gccctactcg cgagcgctcc ccctccccct ctttccgatg gcccttctcc ggcaagactt<br>1201 ccccaccttg ctccccagca aatctctcca ggcacaaggc tgcagcctat gatatcagtg<br>1261 aggatgaaga agactaatgt ttcctccctc ctttcctgtc ctccctttct gtcccattac<br>1321 cttcagaagc tctctgttga attccaaatt gtgaccccaa cactaaacct aaggacagct<br>1381 acaaaggaaa gacaactggg gaaagaagac ctaggactgg gggaacccaa acagcctgtc<br>1441 ctgcaagtga catcactcac ccacacgaag gagggggact gcaccttaga agcctgctct<br>1501 gcatccattt acccccccc aagggctttg tttcactgtt tatgttcacg tgatctcaac<br>1561 agggaaattg tcattttctt taaaaaaaaa aaaaaaaaaa aaaaaaaaaa aaaaaaaaaa<br>1621 aaaaaaaaaa aaaaaaaaaa aaaaaaaaaa aaaaaaaaaa aaaaaaaaaa aaaaaa<br><br>1 M D A Q C S A K V N A R K R R K E A P G<br>1 ATGGATGCACAGTGTTCAGCCAAGGTCAATGCAAGGAAGAGGAGAAAAGAGGCGCCCGGA | SEQ ID NO:250<br><br>SEQ ID NO:251 |

| Gene Name | GenBank Homology | DNA SEQUENCE / DEDUCED AMINO ACID SEQUENCE | SEQUENCE IDENTIFIER: |
|---|---|---|---|
| | | 21 P N G A T E E D G V P S K V Q R C A V G<br>61 CCCAACGGGGCAACAGAAGAAGATGGGGTTCCTTCCAAAGTGCAGCGCTGTGCAGTGGGC<br><br>41 L R Q P A P F S D E I E V D F S K P Y V<br>121 TTACGGCAACCAGCTCCTTTTTCTGATGAAATTGAAGTTGACTTTAGTAAGCCCTATGTC<br><br>61 R V T M E E A S R G T P C E R P V R V Y<br>181 AGGGTAACTATGGAAGAAGCCAGCAGAGGAACTCCTTGTGAGCGACCTGTGAGAGTTTAT<br><br>81 A D G I F D L F H S G H A R A L M Q A K<br>241 GCCGATGGAATATTTGACTTATTTCACTCTGGTCACGCCCGAGCTCTGATGCAAGCGAAG<br><br>101 N L F P N T Y L I V G V C S D E L T H N<br>301 AACCTTTTCCCTAATACGTACCTCATTGTGGGAGTTTGCAGTGATGAGCTCACACACAAC<br><br>121 F K G F T V M N E N E R Y D A V Q H C R<br>361 TTCAAAGGCTTCACGGTGATGAACGAGAATGAGCGCTATGACGCAGTCCAGCACTGCCGC<br><br>141 Y V D E V V R N A P W T L T P E F L A E<br>421 TACGTGGATGAGGTGGTGAGGAATGCGCCCTGGACGCTGACACCCGAGTTCCTGGCCGAA<br><br>161 H R I D F V A H D D I P Y S S A G S D D<br>481 CACCGGATTGATTTTGTAGCCCATGATGATATTCCTTATTCATCTGCTGGCAGTGATGAT<br><br>181 V Y K H I K E A G M F A P T Q R T E G I<br>541 GTTTATAAGCACATCAAGGAGGCAGGCATGTTTGCTCCAACACAGAGGACAGAAGGTATC<br><br>201 S T S D I I T R I V R D Y D V Y A R R N<br>601 TCCACATCAGACATCATCACCCGAATTGTGCGGGATTATGATGTGTATGCGAGGCGGAAC<br><br>221 L Q R G Y T A K E L N V S F I N E K K Y<br>661 CTGCAGAGGGGCTACACAGCAAAGGAGCTCAATGTCAGCTTTATCAACGAGAAGAAATAC<br><br>241 H L Q E R V D K V K K K V K D V E E K S<br>721 CACTTGCAGGAGAGGGTTGACAAAGTAAAGAAGAAAGTGAAAGATGTGGAGGAAAAGTCA<br><br>261 K E F V Q K V E E K S I D L I Q K W E E<br>781 AAAGAATTTGTTCAGAAGGTGGAGGAAAAAAGCATTGACCTCATTCAGAAGTGGGAGGAG<br><br>281 K S R E F I G S F L E M F G P E G A L K<br>841 AAGTCCCGAGAATTCATTGGAAGTTTTCTGGAAATGTTTGGTCCGGAAGGAGCACTGAAA<br><br>301 H M L K E G K G R M L Q A I S P K Q S P | |

| Gene Name | GenBank Homology | DNA SEQUENCE / DEDUCED AMINO ACID SEQUENCE | SEQUENCE IDENTIFIER: |
|---|---|---|---|
| | | 901 CATATGCTGAAAGAGGGGAAGGGCCGGATGCTGCAGGCCATCAGCCCGAAGCAGAGCCCC<br><br>321 S S S P T R E R S P S P S F R W P F S G<br>961 AGCAGCAGCCCTACTCGCGAGCGCTCCCCCTCCCCCTCTTTCCGATGGCCCTTCTCCGGC<br><br>341 K T S P P C S P A N L S R H K A A A Y D<br>1021 AAGACTTCCCCACCTTGCTCCCCAGCAAATCTCTCCAGGCACAAGGCTGCAGCCTATGAT<br><br>361 I S E D E E D -<br>1081 ATCAGTGAGGATGAAGAAGACTAA | |
| BM734862 | Homo sapiens triggering receptor expressed on myeloid cells 1, mRNA. | 1 GTCCCGGGAGCCCTCAGCAGCAGTTGGAGCTGGTGCACAGGAAGGATGAGGAAGACCAGG<br>61 CTCTGGGGGCTGCTGTGGATGCTCTTTGTCTCAGAACTCCGAGCTGCAACTAAATTAACT<br>121 GAGGAAAAGTATGAACTGAAAGAGGGGCAGACCCTGGATGTGAAATGTGACTACACGCTA<br>181 GAGAAGTTTGCCAGCAGCCAGAAAGCTTGGCAGATAATAAGGGACGGAGAGATGCCCAAG<br>241 ACCCTGGCATGCACAGAGAGGCCTTCAAAGAATTCCCATCCAGTCCAAGTGGGGAGGATC<br>301 ATACTAGAAGACTACCATGATCATGGTTTACTGCGCGTCCGAATGGTCAACCTTCAAGTG<br>361 GAAGATTCTGGACTGTATCAGTGTGTGATCTACCAGCCTCCCAAGGAGCCTCACATGCTG<br>421 TTCGATCGCATCCGCTTGGTGGTGACCAAGGGTTTTTCAGGGACCCCTGGCTCCAATGAG<br>481 AATTCTACCCAGAATGTGTATAAGATTCCTCCTACCACCACTAAGGCCTTGTGCCCACTC<br>541 TATACCAGCCCCAGAACTGTGACCCAACCCCCACCCAAGTCAACTGCCGGTGTCTCCCGC<br>601 CCTGGACTTGAAGTCAACCCCACACATGTGACAGACGTCACCAGGATCTCTGTGTTCAGC<br>661 ATTGTCATTCCTGTGGCGTGCGCACTCGTGACTAAGAGCCTGGTCCTTACTGTCCTGTTT<br>721 GCTGTCACACAGAAGTCATTTGGATCCTAGGCCCATGGACCCATGAGGATGACCTCTGAT<br>781 CTCCATCTACATCCATCTGGCAGTTGTGCCAAGGGAGGAGGGAGGAGGTAAAAGGCAGGG<br>841 AGTTAATAACATGAATTAAATCTGTAATCACCGCCAAAAAAAAAAAAAAAAAAAAAAAAA<br>901 AAAAA | SEQ ID NO:252 |
| | | 1 M R K T R L W G L L W M L F V S E L R A<br>1 ATGAGGAAGACCAGGCTCTGGGGGCTGCTGTGGATGCTCTTTGTCTCAGAACTCCGAGCT<br><br>21 A T K L T E E K Y E L K E G Q T L D V K<br>61 GCAACTAAATTAACTGAGGAAAAGTATGAACTGAAAGAGGGGCAGACCCTGGATGTGAAA<br><br>41 C D Y T L E K F A S S Q K A W Q I I R D<br>121 TGTGACTACACGCTAGAGAAGTTTGCCAGCAGCCAGAAAGCTTGGCAGATAATAAGGGAC<br><br>61 G E M P K T L A C T E R P S K N S H P V<br>181 GGAGAGATGCCCAAGACCCTGGCATGCACAGAGAGGCCTTCAAAGAATTCCCATCCAGTC<br><br>81 Q V G R I I L E D Y H D H G L L R V R M<br>241 CAAGTGGGGAGGATCATACTAGAAGACTACCATGATCATGGTTTACTGCGCGTCCGAATG | SEQ ID NO:253 |

| Gene Name | GenBank Homology | DNA SEQUENCE / DEDUCED AMINO ACID SEQUENCE | SEQUENCE IDENTIFIER: |
|---|---|---|---|
| | | 101 V N L Q V E D S G L Y Q C V I Y Q P P K<br>301 GTCAACCTTCAAGTGGAAGATTCTGGACTGTATCAGTGTGTGATCTACCAGCCTCCCAAG<br><br>121 E P H M L F D R I R L V V T K G F S G T<br>361 GAGCCTCACATGCTGTTCGATCGCATCCGCTTGGTGGTGACCAAGGGTTTTTCAGGGACC<br><br>141 P G S N E N S T Q N V Y K I P P T T T K<br>421 CCTGGCTCCAATGAGAATTCTACCCAGAATGTGTATAAGATTCCTCCTACCACCACTAAG<br><br>161 A L C P L Y T S P R T V T Q P P P K S T<br>481 GCCTTGTGCCCACTCTATACCAGCCCCAGAACTGTGACCCAACCCCCACCCAAGTCAACT<br><br>181 A G V S R P G L E V N P T H V T D V T R<br>541 GCCGGTGTCTCCCGCCCTGGACTTGAAGTCAACCCCACACATGTGACAGACGTCACCAGG<br><br>201 I S V F S I V I P V A C A L V T K S L V<br>601 ATCTCTGTGTTCAGCATTGTCATTCCTGTGGCGTGCGCACTCGTGACTAAGAGCCTGGTC<br><br>221 L T V L F A V T Q K S F G S -<br>661 CTTACTGTCCTGTTTGCTGTCACACAGAAGTCATTTGGATCCTAG | |
| BM734891 | No Homology | 1 AACGAAGTCATGTTCCTGGCCCCACTGGTATTCTCAGCTTCGTATGCTGCAGTACCTGTG<br>61 GACAAACACAGGTGGAGAGGTGTCCAGGGTGGGGCTGGGGAAGGATCCTCCTTCTGGACT<br>121 TGTTTCCTGTCAGACCCCCAAGCCCCTTAGACCCAAAGCCAGAGGAGCTTGCCACTGCCT<br>181 TTGGGATATAGGGACTGCAGCCTTTGTTTCTGTGTTCCCCAGAATTTGGGTGACTTTTGG<br>241 TTACCTGGGCACACTCCCTTGTTGGCCATAATGGGTCCCTTCAGTCAGTGGCATGGACAT<br>301 CTGGATGTCCGCACCTCTCTAGAACCTCAGGCCAGCGAGATCAGCAAGGCCTTTTCTCTC<br>361 TATGTCCAGAAGTCAGTCAAGAGCTGCTTGACCTGAATGTGTTACAAGGATTGGGATAGG<br>421 GCAGAGGGAAGTCTGGGGTCACATCCTGAGCTGGGGCGGCCCCTCCTTCCCTCTGCCCTG<br>481 GAGTGGTCTCTGCATGGGCAGCAACCCACCTGGTGGGCTGCCAGGTTTTAGCTGGGATGG<br>541 TCAATGTGCATGTTTCAGACCTTCTGCTCTCTCTGGAATGGGTAGTGTTGGATCTCAGCC<br>601 ACCCATCAGACTGTGTCCGGCATTCTTTGCAATAAATACTT | SEQ ID NO:254 |
| BM734900 | Homo sapiens matrix metalloproteinase 9 (gelatinase B, 92kDa gelatinase, 92kDa type IV collagenase), mRNA (cDNA clone | 1 tcaccatgag cctctggcag cccctggtcc tggtgctcct ggtgctgggc tgctgctttg<br>61 ctgcccccag acagcgccag tccacccttg tgctcttccc tggagacctg agaaccaatc<br>121 tcaccgacag gcagctggca gaggaatacc tgtaccgcta tggttacact cgggtggcag<br>181 agatgcgtgg agagtcgaaa tctctggggc ctgcgctgct gcttctccag aagcaactgt<br>241 ccctgcccga gaccggtgag ctggatagcg ccacgctgaa ggccatgcga accccacggt<br>301 gcggggtccc agacctgggc agattccaaa cctttgaggg cgacctcaag tggcaccacc<br>361 acaacatcac ctattggatc caaaactact cggaagactt gccgcgggcg gtgattgacg | SEQ ID NO:255 |

| Gene Name | GenBank Homology | DNA SEQUENCE / DEDUCED AMINO ACID SEQUENCE | SEQUENCE IDENTIFIER: |
|---|---|---|---|
| | MGC:12688<br>IMAGE:4054882) | 421 acgcctttgc ccgcgccttc gcactgtgga gcgcggtgac gccgctcacc ttcactcgcg<br>481 tgtacagccg ggacgcagac atcgtcatcc agtttggtgt cgcggagcac ggagacgggt<br>541 atcccttcga cgggaaggac gggctcctgg cacacgcctt tcctcctggc cccggcattc<br>601 agggagacgc ccatttcgac gatgacgagt tgtggtccct gggcaagggc gtcgtggttc<br>661 caactcggtt tggaaacgca gatggcgcgg cctgccactt ccccttcatc ttcgagggcc<br>721 gctcctactc tgcctgcacc accgacggtc gctccgacgg cttgccctgg tgcagtacca<br>781 cggccaacta cgacaccgac gaccggtttg gcttctgccc cagcgagaga ctctacaccc<br>841 gggacggcaa tgctgatggg aaaccctgcc agtttccatt catcttccaa ggccaatcct<br>901 actccgcctg caccacggac ggtcgctccg acggctaccg ctggtgcgcc accaccgcca<br>961 actacgaccg ggacaagctc ttcggcttct gcccgacccg agctgactcg acggtgatgg<br>1021 ggggcaactc ggcgggggag ctgtgcgtct tcccc ttcac tttcctgggt aaggagtact<br>1081 cgacctgtac cagcgagggc cgcggagatg ggcgcctctg gtgcgctacc acctcgaact<br>1141 ttgacagcga caagaagtgg ggcttctgcc cggaccaagg atacagtttg ttcctcgtgg<br>1201 cggcgcatga gttcggccac gcgctgggct tagatcattc ctcagtgccg gaggcgctca<br>1261 tgtaccctat gtaccgcttc actgaggggc ccccttgca taaggacgac gtgaatggca<br>1321 tccggcacct ctatggtcct cgccctgaac ctgagccacg gcctccaacc accaccacac<br>1381 cgcagcccac ggctcccccg acggtctgcc ccaccggacc ccccactgtc cacccctcag<br>1441 agcgccccac agctggcccc acaggtcccc cctcagctgg ccccacaggt ccccccactg<br>1501 ctggcccttc tacggccact actgtgcctt tgagtccggt ggacgatgcc tgcaacgtga<br>1561 acatcttcga cgccatcgcg gagattggga accagctgta tttgttcaag gatgggaagt<br>1621 actggcgatt ctctgagggc agggggagcc ggccgcaggg cccc ttcctt atcgccgaca<br>1681 agtggcccgc gctgccccgc aagctggact cggtctttga ggagccgctc tccaagaagc<br>1741 ttttcttctt ctctgggcgc caggtgtggg tgtacacagg cgcgtcggtg ctgggcccga<br>1801 ggcgtctgga caagctgggc ctgggagccg acgtggccca ggtgaccggg gccctccgga<br>1861 gtggcagggg gaagatgctg ctgttcagcg ggcggcgcct ctggaggttc gacgtgaagg<br>1921 cgcagatggt ggatccccgg agcgccagcg aggtggaccg gatgttcccc ggggtgcctt<br>1981 tggacacgca cgacgtcttc cagtaccgag agaaagccta tttctgccag gaccgcttct<br>2041 actggcgcgt gagttcccgg agtgagttga accaggtgga ccaagtgggc tacgtgacct<br>2101 atgacatcct gcagtgccct gaggactagg gctcccgtcc tgctttggca gtgccatgta<br>2161 aatccccact gggaccaacc ctggggaagg agccagtttg ccggatacaa actggtattc<br>2221 tgttctggag gaaagggagg agtggaggtg ggctgggccc tctcttctca cctttgtttt<br>2281 ttgttggagt gtttctaata aacttggatt ctctaacctt taaaaaaaaa aaaaaaaaaa<br>2341 aaaaaaaaaa aaaaaaaaaa aaaaaaaaaa aaa<br><br>1 M S L W Q P L V L V L L V L G C C F A A<br>1 ATGAGCCTCTGGCAGCCCCTGGTCCTGGTGCTCCTGGTGCTGGGCTGCTGCTTTGCTGCC<br><br>21 P R Q R Q S T L V L F P G D L R T N L T<br>61 CCCAGACAGCGCCAGTCCACCCTTGTGCTCTTCCCTGGAGACCTGAGAACCAATCTCACC<br><br>41 D R Q L A E E Y L Y R Y G Y T R V A E M<br>121 GACAGGCAGCTGGCAGAGGAATACCTGTACCGCTATGGTTACACTCGGGTGGCAGAGATG | SEQ ID NO:256 |

| Gene Name | GenBank Homology | DNA SEQUENCE / DEDUCED AMINO ACID SEQUENCE | SEQUENCE IDENTIFIER: |
|---|---|---|---|
| | | 61 R G E S K S L G P A L L L L Q K Q L S L<br>181 CGTGGAGAGTCGAAATCTCTGGGGCCTGCGCTGCTGCTTCTCCAGAAGCAACTGTCCCTG<br><br>81 P E T G E L D S A T L K A M R T P R C G<br>241 CCCGAGACCGGTGAGCTGGATAGCGCCACGCTGAAGGCCATGCGAACCCCACGGTGCGGG<br><br>101 V P D L G R F Q T F E G D L K W H H H N<br>301 GTCCCAGACCTGGGCAGATTCCAAACCTTTGAGGGCGACCTCAAGTGGCACCACCACAAC<br><br>121 I T Y W I Q N Y S E D L P R A V I D D A<br>361 ATCACCTATTGGATCCAAAACTACTCGGAAGACTTGCCGCGGGCGGTGATTGACGACGCC<br><br>141 F A R A F A L W S A V T P L T F T R V Y<br>421 TTTGCCCGCGCCTTCGCACTGTGGAGCGCGGTGACGCCGCTCACCTTCACTCGCGTGTAC<br><br>161 S R D A D I V I Q F G V A E H G D G Y P<br>481 AGCCGGGACGCAGACATCGTCATCCAGTTTGGTGTCGCGGAGCACGGAGACGGGTATCCC<br><br>181 F D G K D G L L A H A F P P G P G I Q G<br>541 TTCGACGGGAAGGACGGGCTCCTGGCACACGCCTTTCCTCCTGGCCCCGGCATTCAGGGA<br><br>201 D A H F D D D E L W S L G K G V V V P T<br>601 GACGCCCATTTCGACGATGACGAGTTGTGGTCCCTGGGCAAGGGCGTCGTGGTTCCAACT<br><br>221 R F G N A D G A A C H F P F I F E G R S<br>661 CGGTTTGGAAACGCAGATGGCGCGGCCTGCCACTTCCCCTTCATCTTCGAGGGCCGCTCC<br><br>241 Y S A C T T D G R S D G L P W C S T T A<br>721 TACTCTGCCTGCACCACCGACGGTCGCTCCGACGGCTTGCCCTGGTGCAGTACCACGGCC<br><br>261 N Y D T D D R F G F C P S E R L Y T R D<br>781 AACTACGACACCGACGACCGGTTTGGCTTCTGCCCCAGCGAGAGACTCTACACCCGGGAC<br><br>281 G N A D G K P C Q F P F I F Q G Q S Y S<br>841 GGCAATGCTGATGGGAAACCCTGCCAGTTTCCATTCATCTTCCAAGGCCAATCCTACTCC<br><br>301 A C T T D G R S D G Y R W C A T T A N Y<br>901 GCCTGCACCACGGACGGTCGCTCCGACGGCTACCGCTGGTGCGCCACCACCGCCAACTAC<br><br>321 D R D K L F G F C P T R A D S T V M G G<br>961 GACCGGGACAAGCTCTTCGGCTTCTGCCCGACCCGAGCTGACTCGACGGTGATGGGGGGC<br><br>341 N S A G E L C V F P F T F L G K E Y S T | |

| Gene Name | GenBank Homology | DNA SEQUENCE / DEDUCED AMINO ACID SEQUENCE | SEQUENCE IDENTIFIER: |
|---|---|---|---|
| | | 1021 AACTCGGCGGGGGAGCTGTGCGTCTTCCCCTTCACTTTCCTGGGTAAGGAGTACTCGACC<br><br>361 C T S E G R G D G R L W C A T T S N F D<br>1081 TGTACCAGCGAGGGCCGCGGAGATGGGCGCCTCTGGTGCGCTACCACCTCGAACTTTGAC<br><br>381 S D K K W G F C P D Q G Y S L F L V A A<br>1141 AGCGACAAGAAGTGGGGCTTCTGCCCGGACCAAGGATACAGTTTGTTCCTCGTGGCGGCG<br><br>401 H E F G H A L G L D H S S V P E A L M Y<br>1201 CATGAGTTCGGCCACGCGCTGGGCTTAGATCATTCCTCAGTGCCGGAGGCGCTCATGTAC<br><br>421 P M Y R F T E G P P L H K D D V N G I R<br>1261 CCTATGTACCGCTTCACTGAGGGGCCCCCCTTGCATAAGGACGACGTGAATGGCATCCGG<br><br>441 H L Y G P R P E P E P R P P T T T T P Q<br>1321 CACCTCTATGGTCCTCGCCCTGAACCTGAGCCACGGCCTCCAACCACCACCACACCGCAG<br><br>461 P T A P P T V C P T G P P T V H P S E R<br>1381 CCCACGGCTCCCCCGACGGTCTGCCCCACCGGACCCCCCACTGTCCACCCCTCAGAGCGC<br><br>481 P T A G P T G P P S A G P T G P P T A G<br>1441 CCCACAGCTGGCCCCACAGGTCCCCCCTCAGCTGGCCCCACAGGTCCCCCCACTGCTGGC<br><br>501 P S T A T T V P L S P V D D A C N V N I<br>1501 CCTTCTACGGCCACTACTGTGCCTTTGAGTCCGGTGGACGATGCCTGCAACGTGAACATC<br><br>521 F D A I A E I G N Q L Y L F K D G K Y W<br>1561 TTCGACGCCATCGCGGAGATTGGGAACCAGCTGTATTTGTTCAAGGATGGGAAGTACTGG<br><br>541 R F S E G R G S R P Q G P F L I A D K W<br>1621 CGATTCTCTGAGGGCAGGGGGAGCCGGCCGCAGGGCCCCTTCCTTATCGCCGACAAGTGG<br><br>561 P A L P R K L D S V F E E P L S K K L F<br>1681 CCCGCGCTGCCCCGCAAGCTGGACTCGGTCTTTGAGGAGCCGCTCTCCAAGAAGCTTTTC<br><br>581 F F S G R Q V W V Y T G A S V L G P R R<br>1741 TTCTTCTCTGGGCGCCAGGTGTGGGTGTACACAGGCGCGTCGGTGCTGGGCCCGAGGCGT<br><br>601 L D K L G L G A D V A Q V T G A L R S G<br>1801 CTGGACAAGCTGGGCCTGGGAGCCGACGTGGCCCAGGTGACCGGGGCCCTCCGGAGTGGC<br><br>621 R G K M L L F S G R R L W R F D V K A Q<br>1861 AGGGGGAAGATGCTGCTGTTCAGCGGGCGGCGCCTCTGGAGGTTCGACGTGAAGGCGCAG | |

| Gene Name | GenBank Homology | DNA SEQUENCE / DEDUCED AMINO ACID SEQUENCE | SEQUENCE IDENTIFIER: |
|---|---|---|---|
| | | 641 M V D P R S A S E V D R M F P G V P L D<br>1921 ATGGTGGATCCCCGGAGCGCCAGCGAGGTGGACCGGATGTTCCCCGGGGTGCCTTTGGAC<br><br>661 T H D V F Q Y R E K A Y F C Q D R F Y W<br>1981 ACGCACGACGTCTTCCAGTACCGAGAGAAAGCCTATTTCTGCCAGGACCGCTTCTACTGG<br><br>681 R V S S R S E L N Q V D Q V G Y V T Y D<br>2041 CGCGTGAGTTCCCGGAGTGAGTTGAACCAGGTGGACCAAGTGGGCTACGTGACCTATGAC<br><br>701 I L Q C P E D -<br>2101 ATCCTGCAGTGCCCTGAGGACTAG | |
| BM734933 | Homo sapiens S100 calcium binding protein P, mRNA. | 1 GCCTTATAAAGCACCAAGAGGCTGCCATGGGACATTTCCTCGGCCACGCCAGCCCCCAGC<br>61 AGGAAGGTGGGTCCACGCCAGCCCCCAGCAGGAAGGTGGGTCCAAATCCAGCACCATGAC<br>121 GGAACTGGAGACAGCCATGAGCATGATCATCGACGTCTTTGCCCGGTACTCGGGTGCCGA<br>181 GGGCAGCAAGCAGAGCCTGACCAAGGGGGAGCTGAAGACGCTCATGGAGAAGGAGCTCCC<br>241 GGGCTTCCTGCAGACCGGAAGGAACAAGGGTGCCGTGGACAAACTGCTCAAGGACCTGGA<br>301 CGCCAACGGAGACGCCGAGGTGGACTTCAGCGAGTTCATCGTGTTTGTGGCCGCGCTCAC<br>361 GTCTACCTGCCACAAGTACTTTGAGAAGGCAGGACTCAAATGATGCCCTGGAGATGTCAC<br>421 AGATTCCTGGCAGAGCCATGGTCCCAGGCTTCCCAAAAGTGTTTGTGGCAATTATTCCCC<br>481 TAGGCTGAGCCTGCTCATGTACCTCTGATTAATAAATGCTTATGAAATGAAAAAAAAAAA<br>541 AAAA | SEQ ID NO:257 |
| | | 1 M T E L E T A M S M I I D V F A R Y S G<br>1 ATGACGGAACTGGAGACAGCCATGAGCATGATCATCGACGTCTTTGCCCGGTACTCGGGT<br><br>21 A E G S K Q S L T K G E L K T L M E K E<br>61 GCCGAGGGCAGCAAGCAGAGCCTGACCAAGGGGGAGCTGAAGACGCTCATGGAGAAGGAG<br><br>41 L P G F L Q T G R N K G A V D K L L K D<br>121 CTCCCGGGCTTCCTGCAGACCGGAAGGAACAAGGGTGCCGTGGACAAACTGCTCAAGGAC<br><br>61 L D A N G D A E V D F S E F I V F V A A<br>181 CTGGACGCCAACGGAGACGCCGAGGTGGACTTCAGCGAGTTCATCGTGTTTGTGGCCGCG<br><br>81 L T S T C H K Y F E K A G L K -<br>241 CTCACGTCTACCTGCCACAAGTACTTTGAGAAGGCAGGACTCAAATGA | SEQ ID NO:258 |
| BM734943 | No Homoloy | 1 AACTCAGGGAGGGAGGGAGGTCCCTGGGAGCAGTTGCACATGGAGTAGGCACCGGGAAAG<br>61 GCTAGAGAGGAGCAGAGCCATGTAAGCAGAGGGCTCAGGACTCCCCAGACAGAGTCCTGC | SEQ ID NO:259 |

| Gene Name | GenBank Homology | DNA SEQUENCE / DEDUCED AMINO ACID SEQUENCE | SEQUENCE IDENTIFIER: |
|---|---|---|---|
| | | 121 TCCCAAGGGGGCTACTGGCCCTTCGCTCAGCATTCCTGTTTCCCTTATATCCAAAATAAA<br>181 CTTTCCTGAGCTTGTCTGAGTCTTGAATCGTTACCACGGAACAGCTTTATTTCCTGGTAA<br>241 TCTCAATTCCTGCCTCTTGCCATACGATGCCGTATGATCTGGGCCACAAGGCTCTTCAGT<br>301 CCTGGCCTGAGTGTCAGCCCCGAGCTCTGTGCCCAGACCTTTGCTGTGCGTCCTGGAGCA<br>361 AACAAGGAACCCTTTCTGCAAGTCAGTTTCCACTTTGAAAAGTTCCGGGGAGGGTGACAT<br>421 TGCTCAAGCCATGGTCATTTGTGTTACGTATATTTTTAATGATCTGTGTGTACTATCTTT<br>481 GCTGTTGTTTACTTAATATTTTTCTTTAAGCCAATTCACTTTAAAAAATATTTTTATTTA<br>541 AAGAAAAATCTTTATATTGCTTTTGTAAATGGAAAAATGGATATTCACTATAAATAAAAA<br>601 GTAACTGTAAAAAGTAAATACAATAAAACAAAATGTTTCAACTC | |
| BM734975 | Human mRNA for coupling protein G(s) alpha-subunit (alpha-S1) (stimulatory regulatory component Gs of adenylyl cyclase). | 1 GCCGCCGCCGCCATGGGCTGCCTCGGGAACAGTAAGACCGAGGACCAGCGCAACGAGGAG<br>61 AAGGCGCAGCGTGAGGCCAACAAAAAGATCGAGAAGCAGCTGCAGAAGGACAAGCAGGTC<br>121 TACCGGGCCACGCACCGCCTGCTGCTGCTGGGTGCTGGAGAATCTGGTAAAAGCACCATT<br>181 GTGAAGCAGATGAGGATCCTGCATGTTAATGGGTTTAATGGAGACAGTGAGAAGGCAACC<br>241 AAAGTGCAGGACATCAAAAACAACCTGAAAGAGGCGATTGAAACCATTGTGGCCGCCATG<br>301 AGCAACCTGGTGCCCCCCGTGGAGCTGGCCAACCCCGAGAACCAGTTCAGAGTGGACTAC<br>361 ATCCTGAGTGTGATGAACGTGCCTGACTTTGACTTCCCTCCCGAATTCTATGAGCATGCC<br>421 AAGGCTCTGTGGGAGGATGAAGGAGTGCGTGCCTGCTACGAACGCTCCAACGAGTACCAG<br>481 CTGATTGACTGTGCCCAGTACTTCCTGGACAAGATCGACGTGATCAAGCAGGCTGACTAT<br>541 GTGCCGAGCGATCAGGACCTGCTTCGCTGCCGTGTCCTGACTTCTGGAATCTTTGAGACC<br>601 AAGTTCCAGGTGGACAAAGTCAACTTCCACATGTTTGACGTGGGTGGCCAGCGCGATGAA<br>661 CGCCGCAAGTGGATCCAGTGCTTCAACGATGTGACTGCCATCATCTTCGTGGTGGCCAGC<br>721 AGCAGCTACAACATGGTCATCCGGGAGGACAACCAGACCAACCGCCTGCAGGAGGCTCTG<br>781 AACCTCTTCAAGAGCATCTGGAACAACAGATGGCTGCGCACCATCTCTGTGATTCTGTTC<br>841 CTCAACAAGCAAGACCTGCTCGCTGAGAAAGTCCTTGCTGGAAAATCGAAAATTGAGGAC<br>901 TACTTTCCAGAATTTGCTCGCTACACTACTCCTGAGGATGCTACTCCCGAGCCCGGAGAG<br>961 GACCCACGCGTGACCCGGGCCAAGTACTTCATCCGTGACGAATTTCTGAGAATCAGCCCC<br>1021 GCTAGTGGGGACGGGCGCCACTACTGCTACCCCCACTTCACCTGCGCTGTGGTCACCGAG<br>1081 AACATCCGCCGGGTGTTCAACGACTGCCGGGACATCATCCAGCGCATGCACCTCCGTCAG<br>1141 TATGAGCTGCTCTAAGAAGGGAACGCCCAGTTTAATTAAAGCCTTAAGCACAATTAATTA<br>1201 AAAGTGAAACGTAATTGTTCACGCAGTTAATCACCCACCATAGGGCATGATTAACAAAGC<br>1261 CACCTTTCCCTTCCCCCTTGAGTGATTTGGCGAAACCCCCTGCTCCCTTCAGCTTGGTTA<br>1321 AATATATTCCAAATTTAGAAAGCTTAAGGCGGCCTACAGAAAAAGAAACAGGCCACAAAA<br>1381 GTTCCCTCTCTCTTTCAGTAAAAATAAATAAAACAGCAGCAGCAAACAAATAAAATGAAA<br>1441 TAAAAGAAACAAATGAAATAAATATTGTGTTGTGCAGCATTAAAAAAAATCAAAATAAAA<br>1501 ATTAAATGTGAGCAAAG | SEQ ID NO:260 |
| | | 1 M G C L G N S K T E D Q R N E E K A Q R<br>1 ATGGGCTGCCTCGGGAACAGTAAGACCGAGGACCAGCGCAACGAGGAGAAGGCGCAGCGT<br><br>21 E A N K K I E K Q L Q K D K Q V Y R A T<br>61 GAGGCCAACAAAAAGATCGAGAAGCAGCTGCAGAAGGACAAGCAGGTCTACCGGGCCACG | SEQ ID NO:261 |

| Gene Name | GenBank Homology | DNA SEQUENCE / DEDUCED AMINO ACID SEQUENCE | SEQUENCE IDENTIFIER: |
|---|---|---|---|
| | | 41 H R L L L L G A G E S G K S T I V K Q M<br>121 CACCGCCTGCTGCTGCTGGGTGCTGGAGAATCTGGTAAAAGCACCATTGTGAAGCAGATG<br><br>61 R I L H V N G F N G D S E K A T K V Q D<br>181 AGGATCCTGCATGTTAATGGGTTTAATGGAGACAGTGAGAAGGCAACCAAAGTGCAGGAC<br><br>81 I K N N L K E A I E T I V A A M S N L V<br>241 ATCAAAAACAACCTGAAAGAGGCGATTGAAACCATTGTGGCCGCCATGAGCAACCTGGTG<br><br>101 P P V E L A N P E N Q F R V D Y I L S V<br>301 CCCCCCGTGGAGCTGGCCAACCCCGAGAACCAGTTCAGAGTGGACTACATCCTGAGTGTG<br><br>121 M N V P D F D F P P E F Y E H A K A L W<br>361 ATGAACGTGCCTGACTTTGACTTCCCTCCCGAATTCTATGAGCATGCCAAGGCTCTGTGG<br><br>141 E D E G V R A C Y E R S N E Y Q L I D C<br>421 GAGGATGAAGGAGTGCGTGCCTGCTACGAACGCTCCAACGAGTACCAGCTGATTGACTGT<br><br>161 A Q Y F L D K I D V I K Q A D Y V P S D<br>481 GCCCAGTACTTCCTGGACAAGATCGACGTGATCAAGCAGGCTGACTATGTGCCGAGCGAT<br><br>181 Q D L L R C R V L T S G I F E T K F Q V<br>541 CAGGACCTGCTTCGCTGCCGTGTCCTGACTTCTGGAATCTTTGAGACCAAGTTCCAGGTG<br><br>201 D K V N F H M F D V G G Q R D E R R K W<br>601 GACAAAGTCAACTTCCACATGTTTGACGTGGGTGGCCAGCGCGATGAACGCCGCAAGTGG<br><br>221 I Q C F N D V T A I I F V V A S S S Y N<br>661 ATCCAGTGCTTCAACGATGTGACTGCCATCATCTTCGTGGTGGCCAGCAGCAGCTACAAC<br><br>241 M V I R E D N Q T N R L Q E A L N L F K<br>721 ATGGTCATCCGGGAGGACAACCAGACCAACCGCCTGCAGGAGGCTCTGAACCTCTTCAAG<br><br>261 S I W N N R W L R T I S V I L F L N K Q<br>781 AGCATCTGGAACAACAGATGGCTGCGCACCATCTCTGTGATTCTGTTCCTCAACAAGCAA<br><br>281 D L L A E K V L A G K S K I E D Y F P E<br>841 GACCTGCTCGCTGAGAAAGTCCTTGCTGGAAAATCGAAAATTGAGGACTACTTTCCAGAA<br><br>301 F A R Y T T P E D A T P E P G E D P R V<br>901 TTTGCTCGCTACACTACTCCTGAGGATGCTACTCCCGAGCCCGGAGAGGACCCACGCGTG | |

| Gene Name | GenBank Homology | DNA SEQUENCE / DEDUCED AMINO ACID SEQUENCE | SEQUENCE IDENTIFIER: |
|---|---|---|---|
| | | 321 T R A K Y F I R D E F L R I S P A S G D<br>961 ACCCGGGCCAAGTACTTCATCCGTGACGAATTTCTGAGAATCAGCCCCGCTAGTGGGGAC<br><br>341 G R H Y C Y P H F T C A V V T E N I R R<br>1021 GGGCGCCACTACTGCTACCCCCACTTCACCTGCGCTGTGGTCACCGAGAACATCCGCCGG<br><br>361 V F N D C R D I I Q R M H L R Q Y E L L<br>1081 GTGTTCAACGACTGCCGGGACATCATCCAGCGCATGCACCTCCGTCAGTATGAGCTGCTC<br><br>381 -<br>1141 TAA | |
| BM735057 | Human mRNA for macrophage inflammatory protein-2alpha (MIP2alpha) | 1 ctctcctcct cgcacagccg ctcgaaccgc ctgctgagcc ccatggcccg cgccacgctc<br>61 tccgccgccc ccagcaatcc ccggctcctg cgggtggcgc tgctgctcct gctcctggtg<br>121 gccgccagcc ggcgcgcagc aggagcgccc ctggccactg aactgcgctg ccagtgcttg<br>181 cagaccctgc agggaattca cctcaagaac atccaaagtg tgaaggtgaa gtcccccgga<br>241 ccccactgcg cccaaaccga agtcatagcc acactcaaga atgggcagaa agcttgtctc<br>301 aaccccgcat cgcccatggt taagaaaatc atcgaaaaga tgctgaaaaa tggcaaatcc<br>361 aactgaccag aaggaaggag gaagcttatt ggtggctgtt cctgaaggag gccctgccct<br>421 tacaggaaca gaagaggaaa gagagacaca gctgcagagg ccacctggat tgcgcctaat<br>481 gtgtttgagc atcacttagg agaagtcttc tatttattta tttatttatt tatttgtttg<br>541 ttttagaaga ttctatgtta atattttatg tgtaaaataa ggttatgatt gaatctactt<br>601 gcacactctc ccattatatt tattgtttat tttaggtcaa acccaagtta gttcaatcct<br>661 gattcatatt taatttgaag atagaaggtt tgcagatatt ctctagtcat ttgttaatat<br>721 ttcttcgtga tgacatatca catgtcagcc actgtgatag aggctgagga atccaagaaa<br>781 atggccagtg agatcaatgt gacggcaggg aaatgtatgt gtgtctattt tgtaactgta<br>841 aagatgaatg tcagttgtta tttattgaaa tgatttcaca gtgtgtggtc aacatttctc<br>901 atgttgaagc tttaagaact aaaatgttct aaatatccct tggacatttt atgtctttct<br>961 tgtaaggcat actgccttgt ttaatgttaa ttatgcagtg tttccctctg tgttagagca<br>1021 gagaggtttc gatatttatt gatgttttca caaagaacag gaaaataaaa tatttaaaaa<br>1081 t | SEQ ID NO:262 |
| | | 1 M A R A T L S A A P S N P R L L R V A L<br>1 ATGGCCCGCGCCACGCTCTCCGCCGCCCCCAGCAATCCCCGGCTCCTGCGGGTGGCGCTG<br><br>21 L L L L L V A A S R R A A G A P L A T E<br>61 CTGCTCCTGCTCCTGGTGGCCGCCAGCCGGCGCGCAGCAGGAGCGCCCCTGGCCACTGAA<br><br>41 L R C Q C L Q T L Q G I H L K N I Q S V<br>121 CTGCGCTGCCAGTGCTTGCAGACCCTGCAGGGAATTCACCTCAAGAACATCCAAAGTGTG<br><br>61 K V K S P G P H C A Q T E V I A T L K N | SEQ ID NO:263 |

| Gene Name | GenBank Homology | DNA SEQUENCE / DEDUCED AMINO ACID SEQUENCE | SEQUENCE IDENTIFIER: |
|---|---|---|---|
| | | 181 AAGGTGAAGTCCCCCGGACCCCACTGCGCCCAAACCGAAGTCATAGCCACACTCAAGAAT<br><br>81 G Q K A C L N P A S P M V K K I I E K M<br>241 GGGCAGAAAGCTTGTCTCAACCCCGCATCGCCCATGGTTAAGAAAATCATCGAAAAGATG<br><br>101 L K N G K S N -<br>301 CTGAAAAATGGCAAATCCAACTGA | |
| BM735091 | Horse serpin mRNA. | 1 CCCCCGCCGCCGGGACCGTCAGCCGCGCGCCGCCTTCGCCATGGAGCAGCTGAGCACAGC<br>61 CAACACCCACTTTGCGGTGGACTTGTTCCGCGCTTTGAATGAGAGCGATCCTACTGGAAA<br>121 CATCTTCATCTCTCCCTTAAGCATTTCCTCAGCGCTGGCCATGATCTTTCTGGGGACCAG<br>181 AGGCAACACAGCGGCACAGGTGTCCAAGGCACTTTATTTGATACTGTTGAGGATATTCA<br>241 TTCAAGATTTCAGAGTCTGAATGCTGATATCAACAAACCTGGAGCTCCCTATATTCTGAA<br>301 ACTCGCTAATAGATTATATGGAGAGAAAACCTATAATTTCCTCGCTGATTTCTTAGCTTC<br>361 AACTCAGAAAATGTATGGTGCTGAATTGGCCAGTGTGGATTTTCAGCAAGCCCCTGAAGA<br>421 TGCAAGAAAGGAGATAAATGAATGGGTCAAAGGACAGACAGAGGGGAAAATTCCGGAACT<br>481 GTTGGTCAAGGGTATGGTTGATAACATGACTAAACTTGTACTGGTGAATGCCATCTACTT<br>541 CAAGGGAAACTGGCAGGAGAAATTCATGAAAGAGGCCACCAGAGACGCCCCATTCCGATT<br>601 GAATAAGAAAGACACAAAAACCGTGAAAATGATGTATCAGAAGAAGAAATTTCCATACAA<br>661 CTACATCGAGGACCTCAAGTGCCGTGTGCTGGAGCTGCCTTACCAAGGCAAAGAGCTCAG<br>721 CATGATCATCCTGCTGCCAGACGACATTGAGGATGAGTCCACGGGCCTGGAGAAGATTGA<br>781 GAAACAGTTGACTTTGGAAAAACTGCGTGAGTGGACCAAACCTGAGAATCTGTATCTCGC<br>841 TGAAGTCAATGTCCACTTGCCCAGGTTCAAGCTGGAAGAGAGCTACGATCTCACCTCCCA<br>901 TCTAGCCCGCCTAGGTGTACAAGACCTCTTTAACAGGGGCAAAGCTGATCTGTCTGGCAT<br>961 GTCAGGGGCCAGAGATCTTTTCGTATCGAAAATTATCCACAAATCCTTTGTGGACCTGAA<br>1021 TGAGGAGGGTACAGAGGCTGCGGCAGCCACAGCAGGCACGATCATGCTTGCCATGCTGAT<br>1081 GCCAGAGGAAAATTTCAATGCCGACCATCCATTCATTTTCTTCATTCGGCACAATCCCTC<br>1141 AGCTAACATCCTGTTCTTAGGCAGATTTTCTTCCCCTTAGAGAAGGTGGAAGTACAAGGT<br>1201 GAAGCTTAGAGCTTTATTACCTGCTAGTATTATTACCATTACTTTTAATAGTGACAGTTT<br>1261 TCATATATCTTTACCAATAAAACTACTATCCAAAAACAAATCTTTAATTTCCTTTGTAAG<br>1321 TTCAGCTCTATTGGCTGTTGCACCCCTTAACTTTGGCGTGGGTAGCTATTTTCCTTTTTT<br>1381 ATGTTGAAAAAATCTAGTTATTGCTTTTGGATGCATCAAGTTAAAAAATAAAAAGACTAA<br>1441 ATCAAATTTATAGATTCTTGATAATGCAGTAAGCTATGAAACTACTATACTCTCCTGATA<br>1501 GTGTGGAACATTATAGTACTGTAATTATTTACTAGATAGACTTTTGGAAGCCATAATAGA<br>1561 GACGCTCTGATTGTTGAAGGAAGGGCTTAAAAACAGATATTCAATTGAAATATAGTAAAG<br>1621 CACCCCAATTAAGGCCTGGGTTGTGCAGACAAACTCGTCTTGGCCGTTGTTTCTAACCCT<br>1681 TTGTTCCACAGGGTTAGTGTCCTAGGCAGCACTCCAATTTCAAACTGTTAGCCACCTGGT<br>1741 GATACCCCGCCTTCTCTTTTTTGTTCTCCTTTATCCTACCTGACATGTATTTTGTGTAGG<br>1801 ATCACATGGGGTCAGAAAGTAGGTGTTCTATCATAAAAGATACCAAGAACGACAAAAGGA<br>1861 AATGCGTAGGAGAGACTGTAGCAAGGGCACAAGATCTCACTGTTACCCAGCTTCTTGCGT<br>1921 TTTCTGATGAAGGCTCTTGCCTTCCATTGTCTGGTAAGGTTGTTACACCAGTGCGATGGC<br>1981 TTTATGCAATGTTGTGGTGGGAGATGGGGGGGTTCTTTTATTAATATAAACCTTTGTCAA | SEQ ID NO:264 |

| Gene Name | GenBank Homology | DNA SEQUENCE / DEDUCED AMINO ACID SEQUENCE | SEQUENCE IDENTIFIER: |
|---|---|---|---|
| | | 2041 ATGTTTGAATGGGGCTGAAAATGCTGCATTTTCTTGCTCCCACAAAGCTGGTATGCTGGT<br>2101 TAATAAAGTTTCCTAAGATTCTGCA<br><br>1 M E Q L S T A N T H F A V D L F R A L N<br>1 ATGGAGCAGCTGAGCACAGCCAACACCCACTTTGCGGTGGACTTGTTCCGCGCTTTGAAT<br><br>21 E S D P T G N I F I S P L S I S S A L A<br>61 GAGAGCGATCCTACTGGAAACATCTTCATCTCTCCCTTAAGCATTTCCTCAGCGCTGGCC<br><br>41 M I F L G T R G N T A A Q V S K A L Y F<br>121 ATGATCTTTCTGGGGACCAGAGGCAACACAGCGGCACAGGTGTCCAAGGCACTTTATTTT<br><br>61 D T V E D I H S R F Q S L N A D I N K P<br>181 GATACTGTTGAGGATATTCATTCAAGATTTCAGAGTCTGAATGCTGATATCAACAAACCT<br><br>81 G A P Y I L K L A N R L Y G E K T Y N F<br>241 GGAGCTCCCTATATTCTGAAACTCGCTAATAGATTATATGGAGAGAAAACCTATAATTTC<br><br>101 L A D F L A S T Q K M Y G A E L A S V D<br>301 CTCGCTGATTTCTTAGCTTCAACTCAGAAAATGTATGGTGCTGAATTGGCCAGTGTGGAT<br><br>121 F Q Q A P E D A R K E I N E W V K G Q T<br>361 TTTCAGCAAGCCCCTGAAGATGCAAGAAAGGAGATAAATGAATGGGTCAAAGGACAGACA<br><br>141 E G K I P E L L V K G M V D N M T K L V<br>421 GAGGGGAAAATTCCGGAACTGTTGGTCAAGGGTATGGTTGATAACATGACTAAACTTGTA<br><br>161 L V N A I Y F K G N W Q E K F M K E A T<br>481 CTGGTGAATGCCATCTACTTCAAGGGAAACTGGCAGGAGAAATTCATGAAAGAGGCCACC<br><br>181 R D A P F R L N K K D T K T V K M M Y Q<br>541 AGAGACGCCCCATTCCGATTGAATAAGAAAGACACAAAAACCGTGAAAATGATGTATCAG<br><br>201 K K K F P Y N Y I E D L K C R V L E L P<br>601 AAGAAGAAATTTCCATACAACTACATCGAGGACCTCAAGTGCCGTGTGCTGGAGCTGCCT<br><br>221 Y Q G K E L S M I I L L P D D I E D E S<br>661 TACCAAGGCAAAGAGCTCAGCATGATCATCCTGCTGCCAGACGACATTGAGGATGAGTCC<br><br>241 T G L E K I E K Q L T L E K L R E W T K<br>721 ACGGGCCTGGAGAAGATTGAGAAACAGTTGACTTTGGAAAAACTGCGTGAGTGGACCAAA<br><br>261 P E N L Y L A E V N V H L P R F K L E E | SEQ ID NO:265 |

| Gene Name | GenBank Homology | DNA SEQUENCE / DEDUCED AMINO ACID SEQUENCE | SEQUENCE IDENTIFIER: |
|---|---|---|---|
| | | 781 CCTGAGAATCTGTATCTCGCTGAAGTCAATGTCCACTTGCCCAGGTTCAAGCTGGAAGAG<br><br>281 S Y D L T S H L A R L G V Q D L F N R G<br>841 AGCTACGATCTCACCTCCCATCTAGCCCGCCTAGGTGTACAAGACCTCTTTAACAGGGGC<br><br>301 K A D L S G M S G A R D L F V S K I I H<br>901 AAAGCTGATCTGTCTGGCATGTCAGGGGCCAGAGATCTTTTCGTATCGAAAATTATCCAC<br><br>321 K S F V D L N E E G T E A A A A T A G T<br>961 AAATCCTTTGTGGACCTGAATGAGGAGGGTACAGAGGCTGCGGCAGCCACAGCAGGCACG<br><br>341 I M L A M L M P E E N F N A D H P F I F<br>1021 ATCATGCTTGCCATGCTGATGCCAGAGGAAAATTTCAATGCCGACCATCCATTCATTTTC<br><br>361 F I R H N P S A N I L F L G R F S S P -<br>1081 TTCATTCGGCACAATCCCTCAGCTAACATCCTGTTCTTAGGCAGATTTTCTTCCCCTTAG | |
| BM735286 | Equus caballus mRNA for ferritin light chain. | 1 ATGAGCTCCCAGATTCGTCAGAATTATTCTACTGAAGTGGAGGCCGCCGTCAACCGCCTG<br>61 GTCAACCTGTACCTGCGGGCCTCCTACACCTACCTCTCTCTGGGCTTCTATTTCGACCGC<br>121 GACGATGTGGCTCTGGAGGGCGTATGCCACTTCTTCCGCGAGTTGGCGGAGGAGAAGCGC<br>181 GAGGGTGCCGAGCGTCTCTTGAAGATGCAAAACCAGCGCGGCGGCCGCGCTCTCTTCCAG<br>241 GACTTGCAGAAGCCGTCCCAGGATGAATGGGGTACAACCCTGGATGCCATGAAAGCCGCC<br>301 ATTGTCCTGGAGAAGAGCCTGAACCAGGCCCTTTTGGATCTGCATGCCCTGGGTTCTGCC<br>361 CAGGCAGACCCCCATCTCTGTGACTTCTTGGAGAGCCACTTCCTAGACGAGGAGGTGAAA<br>421 CTCATCAAGAAGATGGGCGACCATCTGACCAACATCCAGAGGCTCGTTGGCTCCCAAGCT<br>481 GGGCTGGGCGAGTATCTCTTTGAAAGGCTCACCCTCAAGCACGACTAA<br><br>1 M S S Q I R Q N Y S T E V E A A V N R L<br>1 ATGAGCTCCCAGATTCGTCAGAATTATTCTACTGAAGTGGAGGCCGCCGTCAACCGCCTG<br><br>21 V N L Y L R A S Y T Y L S L G F Y F D R<br>61 GTCAACCTGTACCTGCGGGCCTCCTACACCTACCTCTCTCTGGGCTTCTATTTCGACCGC<br><br>41 D D V A L E G V C H F F R E L A E E K R<br>121 GACGATGTGGCTCTGGAGGGCGTATGCCACTTCTTCCGCGAGTTGGCGGAGGAGAAGCGC<br><br>61 E G A E R L L K M Q N Q R G G R A L F Q<br>181 GAGGGTGCCGAGCGTCTCTTGAAGATGCAAAACCAGCGCGGCGGCCGCGCTCTCTTCCAG<br><br>81 D L Q K P S Q D E W G T T L D A M K A A<br>241 GACTTGCAGAAGCCGTCCCAGGATGAATGGGGTACAACCCTGGATGCCATGAAAGCCGCC | SEQ ID NO:266<br><br><br><br><br><br><br><br><br><br>SEQ ID NO:267 |

| Gene Name | GenBank Homology | DNA SEQUENCE / DEDUCED AMINO ACID SEQUENCE | SEQUENCE IDENTIFIER: |
|---|---|---|---|
| | | 101 I V L E K S L N Q A L L D L H A L G S A<br>301 ATTGTCCTGGAGAAGAGCCTGAACCAGGCCCTTTTGGATCTGCATGCCCTGGGTTCTGCC<br><br>121 Q A D P H L C D F L E S H F L D E E V K<br>361 CAGGCAGACCCCCATCTCTGTGACTTCTTGGAGAGCCACTTCCTAGACGAGGAGGTGAAA<br><br>141 L I K K M G D H L T N I Q R L V G S Q A<br>421 CTCATCAAGAAGATGGGCGACCATCTGACCAACATCCAGAGGCTCGTTGGCTCCCAAGCT<br><br>161 G L G E Y L F E R L T L K H D -<br>481 GGGCTGGGCGAGTATCTCTTTGAAAGGCTCACCCTCAAGCACGACTAA | |
| BM735386 | No Homology | 1 TTTCCTTCATGTTCCATTGGAATAGTTAAGTATGTGGTTCCTTCTCAGAACCAAGGGCAA<br>61 TCCTGCTTCACTGGTGTGCCATTTGCTGACCTTGTGGTGCTCTCAGGGGCCGCCTGGGCC<br>121 CAGGGCGAGGCCTCGACGCCTGCCGGGCTCGTGAGATGCTCCAGGTCCTCTCCCCTCCCT<br>181 GGGCGGTTCTGGGCTGAGGGTTCAGGGAGCGTTTCCTTTACCTGGAAGTTGTCACCATGA<br>241 AGTTGTCACGTGCAGTGTCTTTCTCTCTTTCTCTCCGTCCCGTTGCTGGTGCCCCTGTAA<br>301 AGAGGCTTTTGGGAACGAGTAGAGGGCAGAGGTGGCACAGGAGGGTAAAGGATGTCCTGG<br>361 TGCTGGCTCAGCCTGGCAGGGGCTCTGCTGACCTCAGGGCCGGGCACAGCCAGGATGGCT<br>421 GTTTTCCTGTCCCAGAGACCTGTTGTGCTGTGTTATTTTGATTTCCCATTTATTCAAATG<br>481 TATGTATTTACAATTGTAAAGGGGGGGGATGTCTGACCTTAGTGTTCCAAACAGAACTGT<br>541 ATTTTTGCCTTTAAAATCCAGTGATATAACTGGAAGGTGAATAAATGACCTTATCTTTT | SEQ ID NO:268 |
| BM735402 | No Homology | 1 CCAGACTGGCAGACAGTCGCGCCCTGGGGCTTTTCTCACTTACTTTCCCTTTAGGAGGAC<br>61 AGTGAGTGAGCAAGAGCAGTGCTCTGGTACTCACCCATGGAGATGCTTAGAGAATCTTTT<br>121 CTCCATGCATGTAAATTTGTTTAATTCCGTTTTCACAGAAGGGCTCAATTTGTTCCTTAT<br>181 CGTCTCCCCATTGAAGTGTCGATGAAGAGTTCCTGGAAGATGGATCTCTCAGAACTCAAT<br>241 TAGGTGAAATGCCAGAAAGTGAGAAAGACAGAGACTCAACAGATAGGACAAGAATGTTTG<br>301 GTGCATTTGGATGTATCTGTAGAAACTCTGACCAACCTCAAAGTGACCAATTTGCTGAAT<br>361 AACCAGTCCTCTCCTTTTCTGTGAAACTAACTGCTGCGCCCCCACCTCCATAACCCCACA<br>421 GCTTGAGTACTTACTGCTTGGCGGGTCACCTTCACACTGCTGCTCCCACCCAGTTGAGTT<br>481 ATTTGCTTATTATTACTATAATAAAGCTTTTCTGTTGTCTC | SEQ ID NO:269 |
| BM735452 | No Homology | 1 TGAGAGAAGGGGGACAGGGGCGCCCTCCGCCAGCCTCCCGAGGCCTCAGTGCAGTGCCTT<br>61 CCCGGCCGTGCCTGCCTGGGCAGACCCCCAAGAAGCTGCCGGCCCTGCGGGGTGCCCCCG<br>121 GGCCCTGCCTTGGGGGCTGCCGCGTGCGCAGGTCCCACTCAGGATGACTCGGGTCCAGTG<br>181 GCCCAGGCGGGGCCTGCGAGTGGGGACCGCCCTCTCTGTGCCTCTCTCTCCTGCTGCCCT<br>241 CCAGGCCTGGGGGGGCACCAGGCCCGAACCCCGGAAGTCTGCAAAATTGGGTGCCCCAAG<br>301 AGCTGGAAACTCAGGAAACCCCAGGTGCTCAGGGCCCCGCCGTGTCTGGGGGGGCTCCGC<br>361 GGGGCAGACCCCCGTTAATATATGCAATTCCCCATCCCTGCTCTCTGCTCCCTAAATTAT | SEQ ID NO:270 |

| Gene Name | GenBank Homology | DNA SEQUENCE / DEDUCED AMINO ACID SEQUENCE | SEQUENCE IDENTIFIER: |
|---|---|---|---|
| | | 421 TGCCCGGCCGCCTCTCCCAGGCCCCAGAATCTGCCCACGGAGTCGGTGGGGCACGGCAGC<br>481 GCCCCCGGTTTCATATCTGTATTTATAATTATGTCTAAGTGTATATATGTAAAGAGATCT<br>541 TTCT | |
| BM735487 | No Homology | 1 CTGGCCATAGCATTAACCCCTCGCCCGGCCCGGACACAAGGAGGAAGCTCCTAGGGGCCA<br>61 GGGGCAGGACTGTGGAGGGACAGGCTTCCCCTCACNCCACCCACTCCCTCCTTCTGCCCC<br>121 CTCCCACATGCCCGCCTCGCCTCCCACCAGGACTCTAGCCCCAGAGTGGCTCCAGAAGGT<br>181 CCCCCGGGCGTCTGACCCCAGCGAAGGGCCAGCCTTGCTTGGTTCAGCTGGGGGTGCTTG<br>241 CTTGGGAGTTCGAAGTCACCCTAATCACGTAAATGGAACTCTCCTTTTCGAGCATCTCTC<br>301 CCTTCCCACACCTTCCTCCGTTAGCTTCCCCAAAAGAATTGTGTTATTAAACTTATTCTC<br>361 AATGAGTCCTCCTGC | SEQ ID NO:271 |
| BM735494 | Homo sapiens T-cell receptor beta (TCRB) mRNA. | 1 ATGGGCACCAGCCTCCTCTGCTGGATGGCCCTGTGTCTCCTGGGGGCAGATCACGCAGAT<br>61 ACTGGAGTCTCCCAGAACCCCAGACACAAGATCACAAAGAGGGGACAGAATGTAACTTTC<br>121 AGGTGTGATCCAATTTCTGAACACAACCGCCTTTATTGGTACCGACAGACCCTGGGGCAG<br>181 GGCCCAGAGTTTCTGACTTACTTCCAGAATGAAGCTCAACTAGAAAAATCAAGGCTGCTC<br>241 AGTGATCGGTTCTCTGCAGAGAGGCCTAAGGGATCTTTCTCCACCTTGGAGATCCAGCGC<br>301 ACAGAGCAGGGGGACTCGGCCATGTATCTCTGTGCCAGCAGCCCAGGGACTAGCTACGAG<br>361 CAGTACTTTGGGCCCGGCACCAGGCTCACTGTCACAGAGAACCTGAACCAGGTGACCCTA<br>421 CCCAAGGTGGCTGTGTTTGAACCTTCGGAAGCAGAGATCTCCCGCACCGAGAAGGCCACG<br>481 CTCGTGTGCCTGGCCACAGGCTTCTACCCCGACCACGTGGAGCTGAGCTGGTGGGTGAAT<br>541 GGGAAGGAAGTCCAGACTGGGGTCAGCACGGACCCTCAGCCCCTCAAGGAGCAGCCCGCC<br>601 CTCAATGACTCCAGCTACTGTCTGAGCAGCCGGCTGAGGGTCTCTGCTGCCTTCTGGCAC<br>661 AACCCCCGCAACCACTTCCGCTGCCAAGTCCAGTTCTACGGGCTCTCGGAGAATGACGAG<br>721 TGGACCCAGGATAGGGCCAAACCCGTCACCCAGATCGTCAGCGCCGAGGCCTGGGGTAGA<br>781 GCAGACTGTGGCTTCACCTCCGAGTCTTACCAGCAAGGGGTCCTGTCTGCCACCATCCTC<br>841 TATGAGATCTTGCTAGGGAAGGCCACCTTGTATGCCGTGCTGGTCAGTGCCCTCGTGCTG<br>901 ATGGCCATGGTCAAGAGAAAGGATTCCAGAGGCTAG | SEQ ID NO:272 |
| | | 1 M G T S L L C W M A L C L L G A D H A D<br>1 ATGGGCACCAGCCTCCTCTGCTGGATGGCCCTGTGTCTCCTGGGGGCAGATCACGCAGAT<br><br>21 T G V S Q N P R H K I T K R G Q N V T F<br>61 ACTGGAGTCTCCCAGAACCCCAGACACAAGATCACAAAGAGGGGACAGAATGTAACTTTC<br><br>41 R C D P I S E H N R L Y W Y R Q T L G Q<br>121 AGGTGTGATCCAATTTCTGAACACAACCGCCTTTATTGGTACCGACAGACCCTGGGGCAG<br><br>61 G P E F L T Y F Q N E A Q L E K S R L L<br>181 GGCCCAGAGTTTCTGACTTACTTCCAGAATGAAGCTCAACTAGAAAAATCAAGGCTGCTC | SEQ ID NO:273 |

| Gene Name | GenBank Homology | DNA SEQUENCE / DEDUCED AMINO ACID SEQUENCE | SEQUENCE IDENTIFIER: |
|---|---|---|---|
| | | 81 S D R F S A E R P K G S F S T L E I Q R<br>241 AGTGATCGGTTCTCTGCAGAGAGGCCTAAGGGATCTTTCTCCACCTTGGAGATCCAGCGC<br><br>101 T E Q G D S A M Y L C A S S P G T S Y E<br>301 ACAGAGCAGGGGGACTCGGCCATGTATCTCTGTGCCAGCAGCCCAGGGACTAGCTACGAG<br><br>121 Q Y F G P G T R L T V T E N L N Q V T L<br>361 CAGTACTTTGGGCCCGGCACCAGGCTCACTGTCACAGAGAACCTGAACCAGGTGACCCTA<br><br>141 P K V A V F E P S E A E I S R T E K A T<br>421 CCCAAGGTGGCTGTGTTTGAACCTTCGGAAGCAGAGATCTCCCGCACCGAGAAGGCCACG<br><br>161 L V C L A T G F Y P D H V E L S W W V N<br>481 CTCGTGTGCCTGGCCACAGGCTTCTACCCCGACCACGTGGAGCTGAGCTGGTGGGTGAAT<br><br>181 G K E V Q T G V S T D P Q P L K E Q P A<br>541 GGGAAGGAAGTCCAGACTGGGGTCAGCACGGACCCTCAGCCCCTCAAGGAGCAGCCCGCC<br><br>201 L N D S S Y C L S S R L R V S A A F W H<br>601 CTCAATGACTCCAGCTACTGTCTGAGCAGCCGGCTGAGGGTCTCTGCTGCCTTCTGGCAC<br><br>221 N P R N H F R C Q V Q F Y G L S E N D E<br>661 AACCCCCGCAACCACTTCCGCTGCCAAGTCCAGTTCTACGGGCTCTCGGAGAATGACGAG<br><br>241 W T Q D R A K P V T Q I V S A E A W G R<br>721 TGGACCCAGGATAGGGCCAAACCCGTCACCCAGATCGTCAGCGCCGAGGCCTGGGGTAGA<br><br>261 A D C G F T S E S Y Q Q G V L S A T I L<br>781 GCAGACTGTGGCTTCACCTCCGAGTCTTACCAGCAAGGGGTCCTGTCTGCCACCATCCTC<br><br>281 Y E I L L G K A T L Y A V L V S A L V L<br>841 TATGAGATCTTGCTAGGGAAGGCCACCTTGTATGCCGTGCTGGTCAGTGCCCTCGTGCTG<br><br>301 M A M V K R K D S R G -<br>901 ATGGCCATGGTCAAGAGAAAGGATTCCAGAGGCTAG | |
| BM735519 | Homo sapiens ring finger protein 10, mRNA (cDNA clone MGC:35311 IMAGE:5177665) | 1 cgactcgtcg ccattcccgg agcaggtcgg cctcggccca ggggcgagta tccgttgctg<br>61 tgtcggagac actagtcccc gacaccgaga cagccagccc tctcccctgc ctcgcggcgg<br>121 gagagcgtgt ccggccggcc ggccggcggg gctcgcgcaa cctccctcgc ctccccttcc<br>181 cccgcagcct ccgccccgcc aggcccggcc cggactcccg agccccggcc tcctcgtcct<br>241 cggtcgccgc tgccgccggg cttaacagcc ccgtccgccg cttctcttcc tagtttgaga<br>301 agccaaggaa ggaaacaggg aaaaatgtcg ccatgaaggc cgagaaccgc tgccgccgcc | SEQ ID NO:274 |

| Gene Name | GenBank Homology | DNA SEQUENCE / DEDUCED AMINO ACID SEQUENCE | SEQUENCE IDENTIFIER: |
|---|---|---|---|
| | | 361 gacccccgcc ggccctgaac gccatgagcc tgggtccccg ccgcgcccgc tccgctccga | |
| | | 421 ctgccgtcgc cgccgaggcc cccgttgatg ccgctgagct cccccaacgc cgccgccacc | |
| | | 481 gcctccgaca tggacaagaa cagcggctcc aacagctcct ccgcctcttc gggcagcagc | |
| | | 541 aaagggcaac agccgccccg ctccgcctcg gcggggccag ccggcgagtc taaacccaag | |
| | | 601 agcgatggaa agaactccag tggatccaag cgttataatc gcaaacgtga actttcctac | |
| | | 661 cccaaaaatg aaagttttaa caaccagtcc cgtcgctcca gttcacagaa aagcaagact | |
| | | 721 tttaacaaga tgcctcctca aaggggcggc ggcagcagca aactctttag ctcttctttt | |
| | | 781 aatggtggaa gacgagatga ggtagcagag gctcaacggg cagagtttag ccctgcccag | |
| | | 841 ttctctggtc ctaagaagat caacctgaac cacttgttga atttcacttt tgaacccogt | |
| | | 901 ggccagacgg gtcactttga aggcagtgga catggtagct ggggaaagag gaacaagtgg | |
| | | 961 ggacataagc cttttaacaa ggaactcttt ttacaggcca actgccaatt tgtggtgtct | |
| | | 1021 gaagaccaag actacacagc tcattttgct gatcctgata cattagttaa ctgggacttt | |
| | | 1081 gtggaacaag tgcgcatttg tagccatgaa gtgccatctt gcccaatatg cctctatcca | |
| | | 1141 cctactgcag ccaagataac ccgttgtgga cacatcttct gctgggcatg catcctgcac | |
| | | 1201 tatctttcac tgagtgagaa gacgtggagt aaatgtccca tctgttacag ttctgtgcat | |
| | | 1261 aagaaggatc tcaagagtgt tgttgccaca gagtcacatc agtatgttgt tggtgatacc | |
| | | 1321 attacgatgc agctgatgaa gagggagaaa ggggtgttgg tggctttgcc caaatccaaa | |
| | | 1381 tggatgaatg tagaccatcc cattcatcta ggagatgaac agcacagcca gtactccaag | |
| | | 1441 tttctgctgg cctctaagga gcaggtgctg caccgggtag ttctggagga gaaagtagca | |
| | | 1501 ctagagcagc agctggcaga ggagaagcac actcccgagt cctgctttat tgaggcagct | |
| | | 1561 atccaggagc tcaagactcg ggaagaggct ctgtcgggat tggccggaag cagaagggag | |
| | | 1621 gtcactggtg ttgtggctgc tctggaacaa ctggtgctga tggctccctt ggcgaaggag | |
| | | 1681 tctgtttttc aacccaggaa gggtgtgctg gagtatctgt ctgccttcga tgaagaaacc | |
| | | 1741 acggaagttt gttctctgga cactccttct agacctcttg ctctccctct ggtagaagag | |
| | | 1801 gaggaagcag tgtctgaacc agagcctgag ggggttgccag aggcctgtga tgacttggag | |
| | | 1861 ttagcagatg acaatcttaa agaggggacc atttgcactg agtccagcca gcaggaaccc | |
| | | 1921 atcaccaagt caggcttcac acgcctcagc agctctcctt gttactactt ttaccaagcg | |
| | | 1981 gaagatggac agcatatgtt cctgcaccct gtgaatgtgc gctgcctcgt gcgggagtac | |
| | | 2041 ggcagcctgg agaggagccc cgagaagatc tcagcaactg tggtggagat tgctggctac | |
| | | 2101 tccatgtctg aggatgttcg acagcgtcac agatatctct ctcacttgcc actcacctgt | |
| | | 2161 gagttcagca tctgtgaact ggctttgcaa cctcctgtgg tctctaagga aaccctagag | |
| | | 2221 atgttctcag atgacattga gaagaggaaa cgtcagcgcc aaaagaaggc tcgggaggaa | |
| | | 2281 cgccgccgag agcgcaggat tgagatagag gagaacaaga aacagggcaa gtacccagaa | |
| | | 2341 gtccacattc ccctcgagaa tctacagcag tttcctgcct tcaattctta tacctgctcc | |
| | | 2401 tctgattctg ctttgggtcc caccagcacc gagggccatg ggccctctc catttctcct | |
| | | 2461 ctcagcagaa gtccaggttc ccatgcagac tttctgctga cccctctgtc acccactgcc | |
| | | 2521 agtcagggca gtccctcatt ctgcgttggg agtctggaag aagactctcc cttcccttcc | |
| | | 2581 tttgcccaga tgctgagggt tggaaaagca aaagcagatg tgtggcccaa aactgctcca | |
| | | 2641 aagaaagatg agaacagctt agttcctcct gcccctgtgg acagcgacgg ggagagtgat | |
| | | 2701 aattcagacc gtgttcctgt gcccagtttt caaaattcct tcagccaagc tattgaagca | |
| | | 2761 gccttcatga aactggacac accagctact tcagatcccc tctctgaaga gaaaggagga | |
| | | 2821 aagaaaagaa aaaaacagaa acagaagctc ctgttcagca cctcagtcgt ccacaccaag | |
| | | 2881 tgacactact ggcccaggct accttctcca tctggttttt gtttttgttt ttttttcccc | |

| Gene Name | GenBank Homology | DNA SEQUENCE / DEDUCED AMINO ACID SEQUENCE | SEQUENCE IDENTIFIER: |
|---|---|---|---|
| | | 2941 catgcttttg tttggctgct gtaattttta agtatttgag tttgaacaga ttagctctgg<br>3001 ggggaggggg tttccacaat gtgaggggga accaagaaaa ttttaaatac agtgtatttt<br>3061 ccagcttcct gtctttacac caaaataaag tattgacaca agagaaaaaa aaaaaaaaaa<br>3121 aaaaaaaaa | |
| | | 1 M P L S S P N A A A T A S D M D K N S G<br>1 ATGCCGCTGAGCTCCCCCAACGCCGCCGCCACCGCCTCCGACATGGACAAGAACAGCGGC<br><br>21 S N S S S A S S G S S K G Q Q P P R S A<br>61 TCCAACAGCTCCTCCGCCTCTTCGGGCAGCAGCAAAGGGCAACAGCCGCCCCGCTCCGCC<br><br>41 S A G P A G E S K P K S D G K N S S G S<br>121 TCGGCGGGGCCAGCCGGCGAGTCTAAACCCAAGAGCGATGGAAAGAACTCCAGTGGATCC<br><br>61 K R Y N R K R E L S Y P K N E S F N N Q<br>181 AAGCGTTATAATCGCAAACGTGAACTTTCCTACCCCAAAAATGAAAGTTTTAACAACCAG<br><br>81 S R R S S S Q K S K T F N K M P P Q R G<br>241 TCCCGTCGCTCCAGTTCACAGAAAAGCAAGACTTTTAACAAGATGCCTCCTCAAAGGGGC<br><br>101 G G S S K L F S S S F N G G R R D E V A<br>301 GGCGGCAGCAGCAAACTCTTTAGCTCTTCTTTTAATGGTGGAAGACGAGATGAGGTAGCA<br><br>121 E A Q R A E F S P A Q F S G P K K I N L<br>361 GAGGCTCAACGGGCAGAGTTTAGCCCTGCCCAGTTCTCTGGTCCTAAGAAGATCAACCTG<br><br>141 N H L L N F T F E P R G Q T G H F E G S<br>421 AACCACTTGTTGAATTTCACTTTTGAACCCCGTGGCCAGACGGGTCACTTTGAAGGCAGT<br><br>161 G H G S W G K R N K W G H K P F N K E L<br>481 GGACATGGTAGCTGGGGAAAGAGGAACAAGTGGGGACATAAGCCTTTTAACAAGGAACTC<br><br>181 F L Q A N C Q F V V S E D Q D Y T A H F<br>541 TTTTTACAGGCCAACTGCCAATTTGTGGTGTCTGAAGACCAAGACTACACAGCTCATTTT<br><br>201 A D P D T L V N W D F V E Q V R I C S H<br>601 GCTGATCCTGATACATTAGTTAACTGGGACTTTGTGGAACAAGTGCGCATTTGTAGCCAT<br><br>221 E V P S C P I C L Y P P T A A K I T R C<br>661 GAAGTGCCATCTTGCCCAATATGCCTCTATCCACCTACTGCAGCCAAGATAACCCGTTGT<br><br>241 G H I F C W A C I L H Y L S L S E K T W<br>721 GGACACATCTTCTGCTGGGCATGCATCCTGCACTATCTTTCACTGAGTGAGAAGACGTGG | SEQ ID NO:275 |

| Gene Name | GenBank Homology | DNA SEQUENCE / DEDUCED AMINO ACID SEQUENCE | SEQUENCE IDENTIFIER: |
|---|---|---|---|
| | | 261 S K C P I C Y S S V H K K D L K S V V A<br>781 AGTAAATGTCCCATCTGTTACAGTTCTGTGCATAAGAAGGATCTCAAGAGTGTTGTTGCC<br><br>281 T E S H Q Y V V G D T I T M Q L M K R E<br>841 ACAGAGTCACATCAGTATGTTGTTGGTGATACCATTACGATGCAGCTGATGAAGAGGGAG<br><br>301 K G V L V A L P K S K W M N V D H P I H<br>901 AAAGGGGTGTTGGTGGCTTTGCCCAAATCCAAATGGATGAATGTAGACCATCCCATTCAT<br><br>321 L G D E Q H S Q Y S K L L L A S K E Q V<br>961 CTAGGAGATGAACAGCACAGCCAGTACTCCAAGTTGCTGCTGGCCTCTAAGGAGCAGGTG<br><br>341 L H R V V L E E K V A L E Q Q L A E E K<br>1021 CTGCACCGGGTAGTTCTGGAGGAGAAAGTAGCACTAGAGCAGCAGCTGGCAGAGGAGAAG<br><br>361 H T P E S C F I E A A I Q E L K T R E E<br>1081 CACACTCCCGAGTCCTGCTTTATTGAGGCAGCTATCCAGGAGCTCAAGACTCGGGAAGAG<br><br>381 A L S G L A G S R R E V T G V V A A L E<br>1141 GCTCTGTCGGGATTGGCCGGAAGCAGAAGGGAGGTCACTGGTGTTGTGGCTGCTCTGGAA<br><br>401 Q L V L M A P L A K E S V F Q P R K G V<br>1201 CAACTGGTGCTGATGGCTCCCTTGGCGAAGGAGTCTGTTTTTCAACCCAGGAAGGGTGTG<br><br>421 L E Y L S A F D E E T T E V C S L D T P<br>1261 CTGGAGTATCTGTCTGCCTTCGATGAAGAAACCACGGAAGTTTGTTCTCTGGACACTCCT<br><br>441. S R P L A L P L V E E E E A V S E P E P<br>1321 TCTAGACCTCTTGCTCTCCCTCTGGTAGAAGAGGAGGAAGCAGTGTCTGAACCAGAGCCT<br><br>461 E G L P E A C D D L E L A D D N L K E G<br>1381 GAGGGGTTGCCAGAGGCCTGTGATGACTTGGAGTTAGCAGATGACAATCTTAAAGAGGGG<br><br>481 T I C T E S S Q Q E P I T K S G F T R L<br>1441 ACCATTTGCACTGAGTCCAGCCAGCAGGAACCCATCACCAAGTCAGGCTTCACACGCCTC<br><br>501 S S S P C Y Y F Y Q A E D G Q H M F L H<br>1501 AGCAGCTCTCCTTGTTACTACTTTTACCAAGCGGAAGATGGACAGCATATGTTCCTGCAC<br><br>521 P V N V R C L V R E Y G S L E R S P E K<br>1561 CCTGTGAATGTGCGCTGCCTCGTGCGGGAGTACGGCAGCCTGGAGAGGAGCCCCGAGAAG | |

| Gene Name | GenBank Homology | DNA SEQUENCE / DEDUCED AMINO ACID SEQUENCE | SEQUENCE IDENTIFIER: |
|---|---|---|---|
| | | 541 I S A T V V E I A G Y S M S E D V R Q R<br>1621 ATCTCAGCAACTGTGGTGGAGATTGCTGGCTACTCCATGTCTGAGGATGTTCGACAGCGT<br><br>561 H R Y L S H L P L T C E F S I C E L A L<br>1681 CACAGATATCTCTCTCACTTGCCACTCACCTGTGAGTTCAGCATCTGTGAACTGGCTTTG<br><br>581 Q P P V V S K E T L E M F S D D I E K R<br>1741 CAACCTCCTGTGGTCTCTAAGGAAACCCTAGAGATGTTCTCAGATGACATTGAGAAGAGG<br><br>601 K R Q R Q K K A R E E R R R E R R I E I<br>1801 AAACGTCAGCGCCAAAAGAAGGCTCGGGAGGAACGCCGCCGAGAGCGCAGGATTGAGATA<br><br>621 E E N K K Q G K Y P E V H I P L E N L Q<br>1861 GAGGAGAACAAGAAACAGGGCAAGTACCCAGAAGTCCACATTCCCCTCGAGAATCTACAG<br><br>641 Q F P A F N S Y T C S S D S A L G P T S<br>1921 CAGTTTCCTGCCTTCAATTCTTATACCTGCTCCTCTGATTCTGCTTTGGGTCCCACCAGC<br><br>661 T E G H G A L S I S P L S R S P G S H A<br>1981 ACCGAGGGCCATGGGGCCCTCTCCATTTCTCCTCTCAGCAGAAGTCCAGGTTCCCATGCA<br><br>681 D F L L T P L S P T A S Q G S P S F C V<br>2041 GACTTTCTGCTGACCCCTCTGTCACCCACTGCCAGTCAGGGCAGTCCCTCATTCTGCGTT<br><br>701 G S L E E D S P F P S F A Q M L R V G K<br>2101 GGGAGTCTGGAAGAAGACTCTCCCTTCCCTTCCTTTGCCCAGATGCTGAGGGTTGGAAAA<br><br>721 A K A D V W P K T A P K K D E N S L V P<br>2161 GCAAAAGCAGATGTGTGGCCCAAAACTGCTCCAAAGAAAGATGAGAACAGCTTAGTTCCT<br><br>741 P A P V D S D G E S D N S D R V P V P S<br>2221 CCTGCCCCTGTGGACAGCGACGGGGAGAGTGATAATTCAGACCGTGTTCCTGTGCCCAGT<br><br>761 F Q N S F S Q A I E A A F M K L D T P A<br>2281 TTTCAAAATTCCTTCAGCCAAGCTATTGAAGCAGCCTTCATGAAACTGGACACACCAGCT<br><br>781 T S D P L S E E K G G K K R K K Q K Q K<br>2341 ACTTCAGATCCCCTCTCTGAAGAGAAAGGAGGAAAGAAAAGAAAAAAACAGAAACAGAAG<br><br>801 L L F S T S V V H T K -<br>2401 CTCCTGTTCAGCACCTCAGTCGTCCACACCAAGTGA | |
| BM735585 | Human Fc-epsilon- | | |

| Gene Name | GenBank Homology | DNA SEQUENCE / DEDUCED AMINO ACID SEQUENCE | SEQUENCE IDENTIFIER: |
|---|---|---|---|
| | receptor gamma-chain mRNA. | 1 CAGAACGGCCGATCTCCAGCCCAAGATGATTCCAGCAGTGGTCTTGCTCTTACTCCTTTT<br>61 GGTTGAACAAGCAGCGGCCCTGGGAGAGCCTCAGCTTTGCTATATTCTGGATGCCATCCT<br>121 GTTCTTGTATGGTATTGTCCTCACCCTGCTCTACTGTCGACTCAAGATCCAGGTGCGAAA<br>181 GGCAGCTATAGCCAGCTATGAGAAATCAGATGCCGTTTACACGGGCTTGAGTACCCGGAC<br>241 CCAGGAGACTTATGAGACTCTGAAGCATGAGAAACCGCCACAGTAACTTTAGAATAGATG<br>301 CGGTCATATTCTTCTTTGGCTTCTGGTTCTTCCAGCCCTCATGGTTGGCATCACACATGT<br>361 CTCCCTGCCATTAATGCTAGCTGACCCTACCCCTGTAATGATGCTATGCCCCCCATTAAC<br>421 GGACATCAGTGGTTCCTCTTCCCTGTTAAACACCTATGCTCAGATGCTATTCATTAATGT<br>481 TTATATTCTAGTCTCACTCTCTTGTCCCACCCTTCTTCTCTTCCCCATTCCCAACTCCAG<br>541 CTAAAATATGGGAAGGGAGAACCCCCAATAAAACTGCCATGGACTGGACTC | SEQ ID NO:276 |
| | | 1 M I P A V V L L L L L L V E Q A A A L G<br>1 ATGATTCCAGCAGTGGTCTTGCTCTTACTCCTTTTGGTTGAACAAGCAGCGGCCCTGGGA<br><br>21 E P Q L C Y I L D A I L F L Y G I V L T<br>61 GAGCCTCAGCTTTGCTATATTCTGGATGCCATCCTGTTCTTGTATGGTATTGTCCTCACC<br><br>41 L L Y C R L K I Q V R K A A I A S Y E K<br>121 CTGCTCTACTGTCGACTCAAGATCCAGGTGCGAAAGGCAGCTATAGCCAGCTATGAGAAA<br><br>61 S D A V Y T G L S T R T Q E T Y E T L K<br>181 TCAGATGCCGTTTACACGGGCTTGAGTACCCGGACCCAGGAGACTTATGAGACTCTGAAG<br><br>81 H E K P P Q -<br>241 CATGAGAAACCGCCACAGTAA | SEQ ID NO:277 |
| BM780906 | No homology | 1 GGGGTTTCTCCCCTCCTTTCCCTTTACCCGCAGTATCAAGCACAAGAATTGTTGGATTAA<br>61 AAAAAGAACTGATATCTTAGAACCCGAAAGAATCAATTGAATAGGATAAATCAGTACGTC<br>121 AGTGGTGGAGCTTTTGCCTGTAGCTTGAGAGGGGAAAGGTGGGAGGGAAAGGAGAAAACG<br>181 AAAGCTGAAACACATCTCTTGGGTCCTCCTGTTCCGTTTTCAAGGACCAGTCAGCTTCCC<br>241 ATCATAGTTTTGCCG | SEQ ID NO:278 |
| BM780914 | No homologies | 1 GGCACGAGAGGTACAGGACTTTGAAACGTTCTACGGGATGCCCAATCCCGGGACCTACAC<br>61 CGTAAGGATAAGAGCCCGTGGGTACTTCACCGGAATCGTGAGCGAGTGGAGCGCCCCCAA<br>121 GAACTTTGTGTGCGACCCCGAGGAGGAGGGCGCGCGCCTCCGCGTCCGGCTGACCGCCTC<br>181 GCTGACCGCGCTGGGCCCGCTGCTCGCGCTGGGGCTCCTCGTCCTGATCTGCAGAAAGTA<br>241 CTCGCTGATGCGGAA | SEQ ID NO:279 |
| BM780999 | Homo sapiens male-specific lethal 3- | 1 GGCTGCCCCGGAGCCTCGCCCTCCGCCACGATGAGCAAATGAGCGCGAGCGAGGGCATGA | SEQ ID NO:280 |

| Gene Name | GenBank Homology | DNA SEQUENCE / DEDUCED AMINO ACID SEQUENCE | SEQUENCE IDENTIFIER: |
|---|---|---|---|
| | like 1 (Drosophila), transcript variant 1, mRNA | 61 AATTTAAATTCCACTCAGGGGAGAAAGTGCTGTGCTTCGAGCCTGACCCCACCAAGGCGC<br>121 GAGTGCTGTACGATGCCAAGATTGTTGATGTTATTGTTGGGAAAGACGAAAAAGGCAGAA<br>181 AGATCCCAGAATATCTGATCCATTTTAATGGTTGGAACAGAAGCTGGGATAGATGGGCAG<br>241 CAGAAGATCATGTGCTTCGTGATACCGATGAAAATCGTAGATTACAGCGTAAATTGGCAA<br>301 GAAAAGCTGTAGCTCGCCTGAGGAGCACAGGAAGAAAGAAGAAGCGCTGCAGGTTGCCTG<br>361 GTGTGGACTCTGTCTTAAAAGGCCTCCCCACTGAAGAAAAAGATGAAAATGATGAAAACT<br>421 CATTAAGCAGTTCCTCTGACTGTAGTGAAAACAAGGATGAAGAAATAAGTGAAGAAAGTG<br>481 ATATTGAAGAAAAGACTGAAGTGAAAGAAGAACCAGAGCTTCAAACAAGAAGGGAAATGG<br>541 AAGAAAGAACAATAACTATAGAAATCCCTGAAGTTCTGAAGAAGCAGCTGGAGGATGATT<br>601 GTTACTACATTAACAGGAGGAAACGGTTAGTGAAACTTCCATGCCAGACCAACATCATAA<br>661 CGATTTTGGAATCCTATGTGAAGCATTTTGCTATCAATGCAGCCTTTTCAGCCAATGAGA<br>721 GGCCTCGTCACCATCACGTTATGCCACATGCCAACATGAACGTGCATTATATCCCAGCAG<br>781 AAAAGAATGTTGACCTTTGTAAGGAGATGGTGGATGGATTAAGAATAACCTTTGATTACA<br>841 CTCTCCCGTTGGTTTTACTCTATCCATATGAACAAGCTCAGTATAAAAAGGTGACTTCGT<br>901 CTAAATTTTTTCTTCCAATTAAGGAAAGTGCCACAAGCACTAACAGGAGCCAGGAGGAAC<br>961 TCTCTCCCAGTCCGCCTTTGTTGAATCCATCCACGCCACAGTCCACAGAGAGTCAGCCGA<br>1021 CCACCGGTGAACCAGCCACCCCCAAAAGGCGCAAAGCTGAGCCAGAAGCATTGCAGTCTC<br>1081 TGAGGCGGTCCACGCGCCACAGTGCCAACTGTGACAGGCTTTCTGAGAGCAGCGCTTCAC<br>1141 CTCAGCCCAAGCGCCGGCAGCAGGACACATCCGCCAGCATGCCCAAGCTCTTCCTGCACC<br>1201 TGGAAAAGAAGACACCTGTGCATAGCAGATCATCTTCACCTATTCCTCTGACTCCTAGCA<br>1261 AGGAAGGGAGTGCTGTGTTTGCTGGCTTTGAAGGGAGAAGAACTAATGAAATAAACGAGG<br>1321 TCCTCTCCTGGAAGCTTGTGCCTGACAATTACCCCCCAGGTGACCAGCCGCCTCCACCCT<br>1381 CTTACATTTATGGGGCACAACATTTGCTGCGATTGTTTGTGAAACTTCCAGAAATCCTTG<br>1441 GAAAGATGTCCTTTTCTGAGAAGAATCTGAAGGCTTTATTGAAGCACTTTGATCTCTTTT<br>1501 TGAGGTTTTTAGCAGAATACCACGATGACTTCTTCCCAGAGTCGGCTTATGTCGCTGCCT<br>1561 GTGAGGCACATTACAGCACCAAGAACCCCCGGGCAATTTATTAAAATGTTGTTGGTTCTG<br>1621 TAAGAGCAACTGCTCTGTCTAGTTTGGCGCTCTGGGTTCCAGGTGAATAACTAACAAGGT<br>1681 GGTGGGTCTTTACCCACAGCGCAAACACAATGCCCACCTTGGGGCTCTGTTGTTTGAGTT<br>1741 GCCCACATACTGCAGTTATTCTGTTAGGAATGATTCCCTGGGTGCCTGAAAGTGCTCTGA<br>1801 CACGACACTTGTTACTTTGCAGGCCATCTGTGATGGCAAGGAAAAAGCAACTATGTTCAC<br>1861 AGTGAAATATTCGTGGAATAGGTTAGGCCATTTCAGTAGACATTGCAGTTAGTTAGCAAG<br>1921 AACCACATTGTCTCTTTATTTGTTAGCATTAAACAAATTTTTTTTTGCAAATTGGTTTT<br>1981 ATTTTTTTGATGAGGCCGAGCAACTCTGTCCAACAGATTTTAGTTTTGCTTGGAAACCGC<br>2041 AAGGTAGTCCCAGAATATTTTAGAGGAATTGATATTGATGGCAAAAGAAAATTTGCAGCT<br>2101 ATACATTTGCTTGTTACAGTTCATTTTCTCTAAAACCTTATTTTTGGTGATCTAAATAAA<br>2161 GCATTTCCTGTCTTGTTTGAGATTTTACAGCTGTACTTTGTTGTGTAATGTTATGGTTCC<br>2221 CTTTCTGTAAACCTTATTTNTGGTGATCTAAATAAAGCCTGTCTTGTTTGAAAGAAAAAA<br>2281 AAAAAAAAAA<br><br>1 M S A S E G M K F K F H S G E K V L C F<br>1 ATGAGCGCGAGCGAGGGCATGAAATTTAAATTCCACTCAGGGGAGAAAGTGCTGTGCTTC<br><br>21 E P D P T K A R V L Y D A K I V D V I V | SEQ ID NO:281 |

| Gene Name | GenBank Homology | DNA SEQUENCE / DEDUCED AMINO ACID SEQUENCE | SEQUENCE IDENTIFIER: |
|---|---|---|---|
| | | 61 GAGCCTGACCCCACCAAGGCGCGAGTGCTGTACGATGCCAAGATTGTTGATGTTATTGTT<br><br>41 G K D E K G R K I P E Y L I H F N G W N<br>121 GGGAAAGACGAAAAAGGCAGAAAGATCCCAGAATATCTGATCCATTTTAATGGTTGGAAC<br><br>61 R S W D R W A A E D H V L R D T D E N R<br>181 AGAAGCTGGGATAGATGGGCAGCAGAAGATCATGTGCTTCGTGATACCGATGAAAATCGT<br><br>81 R L Q R K L A R K A V A R L R S T G R K<br>241 AGATTACAGCGTAAATTGGCAAGAAAAGCTGTAGCTCGCCTGAGGAGCACAGGAAGAAAG<br><br>101 K K R C R L P G V D S V L K G L P T E E<br>301 AAGAAGCGCTGCAGGTTGCCTGGTGTGGACTCTGTCTTAAAAGGCCTCCCCACTGAAGAA<br><br>121 K D E N D E N S L S S S S D C S E N K D<br>361 AAAGATGAAAATGATGAAAACTCATTAAGCAGTTCCTCTGACTGTAGTGAAAACAAGGAT<br><br>141 E E I S E E S D I E E K T E V K E E P E<br>421 GAAGAAATAAGTGAAGAAAGTGATATTGAAGAAAAGACTGAAGTGAAAGAAGAACCAGAG<br><br>161 L Q T R R E M E E R T I T I E I P E V L<br>481 CTTCAAACAAGAAGGGAAATGGAAGAAAGAACAATAACTATAGAAATCCCTGAAGTTCTG<br><br>181 K K Q L E D D C Y Y I N R R K R L V K L<br>541 AAGAAGCAGCTGGAGGATGATTGTTACTACATTAACAGGAGGAAACGGTTAGTGAAACTT<br><br>201 P C Q T N I I T I L E S Y V K H F A I N<br>601 CCATGCCAGACCAACATCATAACGATTTTGGAATCCTATGTGAAGCATTTTGCTATCAAT<br><br>221 A A F S A N E R P R H H H V M P H A N M<br>661 GCAGCCTTTTCAGCCAATGAGAGGCCTCGTCACCATCACGTTATGCCACATGCCAACATG<br><br>241 N V H Y I P A E K N V D L C K E M V D G<br>721 AACGTGCATTATATCCCAGCAGAAAAGAATGTTGACCTTTGTAAGGAGATGGTGGATGGA<br><br>261 L R I T F D Y T L P L V L L Y P Y E Q A<br>781 TTAAGAATAACCTTTGATTACACTCTCCCGTTGGTTTTACTCTATCCATATGAACAAGCT<br><br>281 Q Y K K V T S S K F F L P I K E S A T S<br>841 CAGTATAAAAAGGTGACTTCGTCTAAATTTTTTCTTCCAATTAAGGAAAGTGCCACAAGC<br><br>301 T N R S Q E E L S P S P P L L N P S T P<br>901 ACTAACAGGAGCCAGGAGGAACTCTCTCCCAGTCCGCCTTTGTTGAATCCATCCACGCCA | |

| Gene Name | GenBank Homology | DNA SEQUENCE / DEDUCED AMINO ACID SEQUENCE | SEQUENCE IDENTIFIER: |
|---|---|---|---|
| | | 321 Q S T E S Q P T T G E P A T P K R R K A<br>961 CAGTCCACAGAGAGTCAGCCGACCACCGGTGAACCAGCCACCCCCAAAAGGCGCAAAGCT<br><br>341 E P E A L Q S L R R S T R H S A N C D R<br>1021 GAGCCAGAAGCATTGCAGTCTCTGAGGCGGTCCACGCGCCACAGTGCCAACTGTGACAGG<br><br>361 L S E S S A S P Q P K R R Q Q D T S A S<br>1081 CTTTCTGAGAGCAGCGCTTCACCTCAGCCCAAGCGCCGGCAGCAGGACACATCCGCCAGC<br><br>381 M P K L F L H L E K K T P V H S R S S S<br>1141 ATGCCCAAGCTCTTCCTGCACCTGGAAAAGAAGACACCTGTGCATAGCAGATCATCTTCA<br><br>401 P I P L T P S K E G S A V F A G F E G R<br>1201 CCTATTCCTCTGACTCCTAGCAAGGAAGGGAGTGCTGTGTTTGCTGGCTTTGAAGGGAGA<br><br>421 R T N E I N E V L S W K L V P D N Y P P<br>1261 AGAACTAATGAAATAAACGAGGTCCTCTCCTGGAAGCTTGTGCCTGACAATTACCCCCCA<br><br>441 G D Q P P P P S Y I Y G A Q H L L R L F<br>1321 GGTGACCAGCCGCCTCCACCCTCTTACATTTATGGGGCACAACATTTGCTGCGATTGTTT<br><br>461 V K L P E I L G K M S F S E K N L K A L<br>1381 GTGAAACTTCCAGAAATCCTTGGAAAGATGTCCTTTTCTGAGAAGAATCTGAAGGCTTTA<br><br>481 L K H F D L F L R F L A E Y H D D F F P<br>1441 TTGAAGCACTTTGATCTCTTTTTGAGGTTTTTAGCAGAATACCACGATGACTTCTTCCCA<br><br>501 E S A Y V A A C E A H Y S T K N P R A I<br>1501 GAGTCGGCTTATGTCGCTGCCTGTGAGGCACATTACAGCACCAAGAACCCCCGGGCAATT<br><br>521 Y -<br>1561 TATTAA | |
| BM781033 | Homo sapiens replication protein A1, 70kDa, mRNA | 1 GGAGCTGTTGCGGGGTCCGCGGGGAAGTCTTGGCGGTGGAGCCATGGTCGGCCAACTGAG<br>61 CGAGGGGGCCATTGCGGCCATCATGCAGAAGGGGGATACAAACATAAAGCCCATCCTCCA<br>121 AGTCATCAACATCCGTCCCATTACTACGGGGAATAGTCCGCCGCGTTATCGACTGCTCAT<br>181 GAGTGATGGATTGAACACTCTATCCTCTTTCATGTTGGCGACACAGTTGAACCCTCTCGT<br>241 GGAGGAAGAACAATTGTCCAGCAACTGTGTATGCCAGATTCACAGATTTATTGTGAACAC<br>301 TCTGAAAGACGGAAGGAGAGTAGTTATCTTGATGGAATTAGAAGTTTTGAAGTCAGCTGA<br>361 AGCAGTTGGAGTGAAGATTGGCAATCCAGTGCCCTATAATGAAGGACTCGGGCAGCCGCA<br>421 AGTAGCTCCTCCAGCGCCAGCAGCCAGCCCAGCAGCAAGCAGCAGGCCCCAGCCGCAGAA | SEQ ID NO:282 |

| Gene Name | GenBank Homology | DNA SEQUENCE / DEDUCED AMINO ACID SEQUENCE | SEQUENCE IDENTIFIER: |
|---|---|---|---|
| | | 481 TGGAAGCTCGGGAATGGGTTCTACTGTTTCTAAGGCTTATGGTGCTTCAAAGACATTTGG | |
| | | 541 AAAAGCTGCAGGTCCCAGCCTGTCACACACTTCTGGGGGAACACAGTCCAAAGTGGTGCC | |
| | | 601 CATTGCCAGCCTCACTCCTTACCAGTCCAAGTGGACCATTTGTGCTCGTGTTACCAACAA | |
| | | 661 AAGTCAGATCCGTACCTGGAGCAACTCCCGAGGGGAAGGGAAGCTTTTCTCCCTAGAACT | |
| | | 721 GGTTGACGAAAGTGGTGAAATCCGAGCTACAGCTTTCAATGAGCAAGTGGACAAGTTCTT | |
| | | 781 TCCTCTTATTGAAGTGAACAAGGTGTATTATTTCTCGAAAGGCACCCTGAAGATTGCTAA | |
| | | 841 CAAGCAGTTCACAGCTGTTAAAAATGACTACGAGATGACCTTCAATAACGAGACTTCCGT | |
| | | 901 CATGCCCTGTGAGGACGACCATCATTTACCTACGGTTCAGTTTGATTTCACGGGGATTGA | |
| | | 961 TGACCTCGAGAACAAGTCGAAAGACTCACTTGTAGACATCATCGGGATCTGCAAGAGCTA | |
| | | 1021 TGAAGACGCCACTAAAATCACAGTGAGGTCTAACAACAGAGAAGTTGCCAAGAGGAATAT | |
| | | 1081 CTACTTGATGGACACATCTGGGAAGGTGGTGACTGCTACACTGTGGGGGGAAGATGCTGA | |
| | | 1141 TAAATTTGATGGTTCTAGACAGCCCGTGTTGGCTATCAAAGGAGCCCGAGTCTCTGATTT | |
| | | 1201 CGGTGGACGGAGCCTCTCCGTGCTGTCTTCAAGCACTATCATTGCGAATCCTGACATCCC | |
| | | 1261 AGAGGCCTATAAGCTTCGTGGATGGTTTGACGCAGAAGGACAAGCCTTAGATGGTGTTTC | |
| | | 1321 CATCTCTGATCTAAAGAGCGGCGGAGTCGGAGGGAGTAACACCAACTGGAAAACCTTGTA | |
| | | 1381 TGAGGTCAAATCCGAGAACCTGGGCCAAGGCGACAAGCCGGACTACTTTAGTTCTGTGGC | |
| | | 1441 CACAGTGGTGTATCTTCGCAAAGAGAACTGCATGTACCAAGCCTGCCCGACTCAGGACTG | |
| | | 1501 CAATAAGAAAGTGATTGATCAACAGAATGGATTGTACCGCTGTGAGAAGTGCGACACCGA | |
| | | 1561 ATTTCCCAATTTCAAGTACCGCATGATCCTGTCAGTAAATATTGCAGATTTTCAAGAGAA | |
| | | 1621 TCAGTGGGTGACTTGTTTCCAGGAGTCTGCTGAAGCTATCCTTGGACAAAATGCTGCTTA | |
| | | 1681 TCTTGGGGAATTAAAAGACAAGAATGAACAGGCATTTGAAGAAGTTTTCCAGAATGCCAA | |
| | | 1741 CTTCCGATCTTTCATATTCAGAGTCAGGGTCAAAGTGGAGACCTACAACGACGAGTCTCG | |
| | | 1801 AATTAAGGCCACTGTGATGGACGTGAAGCCCGTGGACTACAGAGAGTATGGCCGAAGGCT | |
| | | 1861 GGTCATGAGCATCAGGAGAAGTGCATTGATGTGAGAGGAGCAGTGCCAATCGGGCAGAAG | |
| | | 1921 TTTGCAAATAGGCAGAATGGAATCGATTTCCTCCCACCTCCGTGTGACGATCCCATGTTA | |
| | | 1981 GCTACACAGTGCAGAGGCTCTTGATGGTGGACTAAGCAATTTCCTCCCTTGTGCGCATCT | |
| | | 2041 CAGAACCCATCGGTAGGCAAAGGAAAATACGCTCAGGTGGTTGTGGTGTAGACTGTGTCA | |
| | | 2101 GGCCTACGGAGTCAGCCAGTGGCTAGCGCAAGACCAGTCACTCCCTCTGCCTTCAGGCTT | |
| | | 2161 CTGTCAATTTCATTATCTTCAAGCAGGAATTATGTTGCAAGTAAATTGACCCTAAATCTG | |
| | | 2221 CAGACAGTGAAATAAATTATGTAACTAGGTTTCTGCTTCTCCAGTGGTGACCACCCCCCC | |
| | | 2281 CCCATCCCCGCTCACAACTTGGGTTCTTCTCAGCGGGGCGAGCTGAGAAGCGGTCATGAG | |
| | | 2341 CACCTGGGTGTTTTAGTAAGCGCGTCTTCCTAGAATTCAAAGGCGCTCTCTTTCTAGAGG | |
| | | 2401 TGCCACATAGTTGTTAATGCTCAGAATGGCAATAGGGTAGAATTATTAATCAAAGACATA | |
| | | 2461 TCTTTTATATCTGAAGATAGCCTTCCTTCAGGGTTTAATAGACTGAGTCAGATGGGTCTG | |
| | | 2521 ATATTAATCAAAATTGTCTCTTCTGAGGAAGGCTGATAAGCATTGACTTGCTGTCCCCCA | |
| | | 2581 GGGACATCCACGCAACTACAAAGCCTTGCTTTAGTTTCACTTCAATTAATGTTGCATTTT | |
| | | 2641 GGTTGGTGAATGCACACTTTTTCTACCTGTACACATTCCTGCATTCATGTATTTTGTTTT | |
| | | 2701 TTTTGACTAAAGCTATGTTACATGGAAAGGATTTTGAAGCCCTTTGTTTCTCTTGCTTTG | |
| | | 2761 TTTTAATAAACAGTATATTCTTTGGTTGTGAATCCTAAAAAAAAAAAAAAAAAAAAAAAA | |
| | | 2821 AAAAA | |
| | | 1 M V G Q L S E G A I A A I M Q K G D T N | SEQ ID NO:283 |
| | | 1 ATGGTCGGCCAACTGAGCGAGGGGGCCATTGCGGCCATCATGCAGAAGGGGGATACAAAC | |

| Gene Name | GenBank Homology | DNA SEQUENCE / DEDUCED AMINO ACID SEQUENCE | SEQUENCE IDENTIFIER: |
|---|---|---|---|
| | | 21 I K P I L Q V I N I R P I T T G N S P P<br>61 ATAAAGCCCATCCTCCAAGTCATCAACATCCGTCCCATTACTACGGGGAATAGTCCGCCG<br><br>41 R Y R L L M S D G L N T L S S F M L A T<br>121 CGTTATCGACTGCTCATGAGTGATGGATTGAACACTCTATCCTCTTTCATGTTGGCGACA<br><br>61 Q L N P L V E E E Q L S S N C V C Q I H<br>181 CAGTTGAACCCTCTCGTGGAGGAAGAACAATTGTCCAGCAACTGTGTATGCCAGATTCAC<br><br>81 R F I V N T L K D G R R V V I L M E L E<br>241 AGATTTATTGTGAACACTCTGAAAGACGGAAGGAGAGTAGTTATCTTGATGGAATTAGAA<br><br>101 V L K S A E A V G V K I G N P V P Y N E<br>301 GTTTTGAAGTCAGCTGAAGCAGTTGGAGTGAAGATTGGCAATCCAGTGCCCTATAATGAA<br><br>121 G L G Q P Q V A P P A P A A S P A A S S<br>361 GGACTCGGGCAGCCGCAAGTAGCTCCTCCAGCGCCAGCAGCCAGCCCAGCAGCAAGCAGC<br><br>141 R P Q P Q N G S S G M G S T V S K A Y G<br>421 AGGCCCCAGCCGCAGAATGGAAGCTCGGGAATGGGTTCTACTGTTTCTAAGGCTTATGGT<br><br>161 A S K T F G K A A G P S L S H T S G G T<br>481 GCTTCAAAGACATTTGGAAAAGCTGCAGGTCCCAGCCTGTCACACACTTCTGGGGGAACA<br><br>181 Q S K V V P I A S L T P Y Q S K W T I C<br>541 CAGTCCAAAGTGGTGCCCATTGCCAGCCTCACTCCTTACCAGTCCAAGTGGACCATTTGT<br><br>201 A R V T N K S Q I R T W S N S R G E G K<br>601 GCTCGTGTTACCAACAAAAGTCAGATCCGTACCTGGAGCAACTCCCGAGGGGAAGGGAAG<br><br>221 L F S L E L V D E S G E I R A T A F N E<br>661 CTTTTCTCCCTAGAACTGGTTGACGAAAGTGGTGAAATCCGAGCTACAGCTTTCAATGAG<br><br>241 Q V D K F F P L I E V N K V Y Y F S K G<br>721 CAAGTGGACAAGTTCTTTCCTCTTATTGAAGTGAACAAGGTGTATTATTTCTCGAAAGGC<br><br>261 T L K I A N K Q F T A V K N D Y E M T F<br>781 ACCCTGAAGATTGCTAACAAGCAGTTCACAGCTGTTAAAAATGACTACGAGATGACCTTC<br><br>281 N N E T S V M P C E D D H H L P T V Q F<br>841 AATAACGAGACTTCCGTCATGCCCTGTGAGGACGACCATCATTTACCTACGGTTCAGTTT | |

| Gene Name | GenBank Homology | DNA SEQUENCE / DEDUCED AMINO ACID SEQUENCE | SEQUENCE IDENTIFIER: |
|---|---|---|---|
| | | 301 D F T G I D D L E N K S K D S L V D I I<br>901 GATTTCACGGGGATTGATGACCTCGAGAACAAGTCGAAAGACTCACTTGTAGACATCATC<br><br>321 G I C K S Y E D A T K I T V R S N N R E<br>961 GGGATCTGCAAGAGCTATGAAGACGCCACTAAAATCACAGTGAGGTCTAACAACAGAGAA<br><br>341 V A K R N I Y L M D T S G K V V T A T L<br>1021 GTTGCCAAGAGGAATATCTACTTGATGGACACATCTGGGAAGGTGGTGACTGCTACACTG<br><br>361 W G E D A D K F D G S R Q P V L A I K G<br>1081 TGGGGGGAAGATGCTGATAAATTTGATGGTTCTAGACAGCCCGTGTTGGCTATCAAAGGA<br><br>381 A R V S D F G G R S L S V L S S S T I I<br>1141 GCCCGAGTCTCTGATTTCGGTGGACGGAGCCTCTCCGTGCTGTCTTCAAGCACTATCATT<br><br>401 A N P D I P E A Y K L R G W F D A E G Q<br>1201 GCGAATCCTGACATCCCAGAGGCCTATAAGCTTCGTGGATGGTTTGACGCAGAAGGACAA<br><br>421 A L D G V S I S D L K S G G V G G S N T<br>1261 GCCTTAGATGGTGTTTCCATCTCTGATCTAAAGAGCGGCGGAGTCGGAGGGAGTAACACC<br><br>441 N W K T L Y E V K S E N L G Q G D K P D<br>1321 AACTGGAAAACCTTGTATGAGGTCAAATCCGAGAACCTGGGCCAAGGCGACAAGCCGGAC<br><br>461 Y F S S V A T V V Y L R K E N C M Y Q A<br>1381 TACTTTAGTTCTGTGGCCACAGTGGTGTATCTTCGCAAAGAGAACTGCATGTACCAAGCC<br><br>481 C P T Q D C N K K V I D Q Q N G L Y R C<br>1441 TGCCCGACTCAGGACTGCAATAAGAAAGTGATTGATCAACAGAATGGATTGTACCGCTGT<br><br>501 E K C D T E F P N F K Y R M I L S V N I<br>1501 GAGAAGTGCGACACCGAATTTCCCAATTTCAAGTACCGCATGATCCTGTCAGTAAATATT<br><br>521 A D F Q E N Q W V T C F Q E S A E A I L<br>1561 GCAGATTTTCAAGAGAATCAGTGGGTGACTTGTTTCCAGGAGTCTGCTGAAGCTATCCTT<br><br>541 G Q N A A Y L G E L K D K N E Q A F E E<br>1621 GGACAAAATGCTGCTTATCTTGGGGAATTAAAAGACAAGAATGAACAGGCATTTGAAGAA<br><br>561 V F Q N A N F R S F I F R V R V K V E T<br>1681 GTTTTCCAGAATGCCAACTTCCGATCTTTCATATTCAGAGTCAGGGTCAAAGTGGAGACC<br><br>581 Y N D E S R I K A T V M D V K P V D Y R | |

| Gene Name | GenBank Homology | DNA SEQUENCE / DEDUCED AMINO ACID SEQUENCE | SEQUENCE IDENTIFIER: |
|---|---|---|---|
| | | 1741 TACAACGACGAGTCTCGAATTAAGGCCACTGTGATGGACGTGAAGCCCGTGGACTACAGA<br><br>601 E Y G R R L V M S I R R S A L M -<br>1801 GAGTATGGCCGAAGGCTGGTCATGAGCATCAGGAGAAGTGCATTGATGTGA | |
| BM781067 | Human mRNA for T3 epsilon chain (20K) of T-cell receptor (from peripheral blood lymphocytes). | 1 GTAAGTCTGCTGGCCTCCGCCATCTTAGTAAAGTAACAGTCCCATGAAACAAAGATGCAG<br>61 TCGGGCACTCACTGGAGAGTTCTGGGCCTCTGCCTCTTATCAGTTGGCGTTTGGGGGCAA<br>121 GATGGTAATGAAGAAATGGGTGGTATTACACAGACACCATATAAAGTCTCCATCTCTGGA<br>181 ACCACAGTAATATTGACATGCCCTCAGTATCCTGGATCTGAAATACTATGGCAACACAAT<br>241 GATAAAAACATAGGCGGTGATGAGGATGATAAAAACATAGGCAGTGATGAGGATCACCTG<br>301 TCACTGAAGGAATTTTCAGAATTGGAGCAAAGTGGTTATTATGTCTGCTACCCCAGAGGA<br>361 AGCAAACCAGAAGATGCGAACTTTTATCTCTACCTGAAAGCCAGAGTGTGTGAGAACTGC<br>421 ATGGAGGTGGATCTGACGGCAGTGGCCACAATCATCGTAGTCGACCTCTGCATCACTCTG<br>481 GGCTTACTGCTGCTGGTGTATTACTGGAGCAAGTCTAAAAAGGCCAGGGCCAAGCCTGTG<br>541 ACGAGGGGAGCAGATGTTGGCGGCAGGCCCCGGGGACAAAACAAGGAGAGACCCCCGCCT<br>601 GTTCCCAATCCAGATTATGAGCCCATCCGGAAAGGCCAGCGGGACCTGTACGCTGGCCTG<br>661 AATCAGAGAGCCGTCTGACAGTTCCTGAGGACACTGCCTCCTGCTGGCCCAGCTCTCCTC<br>721 TCCAGTCCCCCTGCGACTCCCTGTTTCCTGGGCTAGTCTTGGACCCCACGAGAGAGAATC<br>781 GTTCCTCAGCCTCATGGTGAACTCGCGCCCTCCAGCCTGATCCCCCGCTCCCTCCTCCCT<br>841 GCCTTCTCTGCTGGTACCCAGTCCTAAAATATTGCTGCTTCCTCTTCCTTTGAAGCATCA<br>901 TCAGTAGTCACACCCTCACAGCTGGCCTGCCCTCTTGCCAGGATATTTATTTGTGCTATT<br>961 CACTCCCTTCCCTTTGGATGTAACTTCTCCGTTCAGTTCCCTCCTTTTCTTGCATGTAAG<br>1021 TTGTCCCCCATCCCAAAGTATTCCATCTACTTTTCTATCGCCGTCCCCTTTTGCAGCCCT<br>1081 CTCTGGGGATGGACTGGGTAAATGTTGACAGAGGCCCTGCCCCGTTCACAGATCCTGGCC<br>1141 CTGAGCCAGCCCTGTGCTCCTCCCTCCCCCAACACTCCCTACCAACCCCCTAATCCCCTA<br>1201 CTCCCTCCAACCCCCCCTCCCACTGTAGGCCCCTGGATGTTCGTTTGCATCTCCATGAAT<br>1261 GTGCTAGGCTCCTTGTCAGCTGAGAGAGAAAAGAAATAAAGTGTATTTGGCTGC | SEQ ID NO:284 |
| | | 1 M Q S G T H W R V L G L C L L S V G V W<br>1 ATGCAGTCGGGCACTCACTGGAGAGTTCTGGGCCTCTGCCTCTTATCAGTTGGCGTTTGG<br><br>21 G Q D G N E E M G G I T Q T P Y K V S I<br>61 GGGCAAGATGGTAATGAAGAAATGGGTGGTATTACACAGACACCATATAAAGTCTCCATC<br><br>41 S G T T V I L T C P Q Y P G S E I L W Q<br>121 TCTGGAACCACAGTAATATTGACATGCCCTCAGTATCCTGGATCTGAAATACTATGGCAA<br><br>61 H N D K N I G G D E D D K N I G S D E D<br>181 CACAATGATAAAAACATAGGCGGTGATGAGGATGATAAAAACATAGGCAGTGATGAGGAT<br><br>81 H L S L K E F S E L E Q S G Y Y V C Y P<br>241 CACCTGTCACTGAAGGAATTTTCAGAATTGGAGCAAAGTGGTTATTATGTCTGCTACCCC | SEQ ID NO:285 |

| Gene Name | GenBank Homology | DNA SEQUENCE / DEDUCED AMINO ACID SEQUENCE | SEQUENCE IDENTIFIER: |
|---|---|---|---|
| | | 101 R G S K P E D A N F Y L Y L K A R V C E<br>301 AGAGGAAGCAAACCAGAAGATGCGAACTTTTATCTCTACCTGAAAGCCAGAGTGTGTGAG<br><br>121 N C M E V D L T A V A T I I V V D L C I<br>361 AACTGCATGGAGGTGGATCTGACGGCAGTGGCCACAATCATCGTAGTCGACCTCTGCATC<br><br>141 T L G L L L L V Y Y W S K S K K A R A K<br>421 ACTCTGGGCTTACTGCTGCTGGTGTATTACTGGAGCAAGTCTAAAAAGGCCAGGGCCAAG<br><br>161 P V T R G A D V G G R P R G Q N K E R P<br>481 CCTGTGACGAGGGGAGCAGATGTTGGCGGCAGGCCCCGGGGACAAAACAAGGAGAGACCC<br><br>181 P P V P N P D Y E P I R K G Q R D L Y A<br>541 CCGCCTGTTCCCAATCCAGATTATGAGCCCATCCGGAAAGGCCAGCGGGACCTGTACGCT<br><br>201 G L N Q R A V -<br>601 GGCCTGAATCAGAGAGCCGTCTGA | |
| BM781103 | Human mRNA for cathepsin H | 1 TTGCCGGCGCAAGAGCCAAGCCGCCAGCGCTGCTATGTGGGCCACGCTGCCGCTGCTCTG<br>61 CGCCGGGGCCTGGCTCCTGGGAGTCCCCGTCTGCGGTGCCGCCGAACTGTCCGTGAACTC<br>121 CTTAGAGAAGTTTCACTTCAAGTCATGGATGTCTAAGCACCGTAAGACCTACAGTACGGA<br>181 GGAGTACCACCACAGGCTGCAGACGTTTGCCAGCAACTGGAGGAAGATAAACGCCCACAA<br>241 CAATGGGAACCACACATTTAAAATGGCACTGAACCAATTTTCAGACATGAGCTTTGCTGA<br>301 AATAAAACACAAGTATCTCTGGTCAGAGCCTCAGAATTGCTCAGCCACCAAAAGTAACTA<br>361 CCTTCGAGGTACTGGTCCCTACCCACCTTCCGTGGACTGGCGGAAAAAAGGAAATTTTGT<br>421 CTCACCTGTGAAAAATCAGGGTGCCTGCGGCAGTTGCTGGACTTTCTCCACCACTGGGGC<br>481 CCTGGAGTCTGCAATCGCCATCGCAACCGGAAAGATGCTGTCCTTGGCGGAACAGCAGCT<br>541 GGTGGACTGCGCCCAGGACTTCAATAATTACGGCTGCCAAGGGGGTCTCCCCAGCCAGGC<br>601 TTTCGAGTATATCCTGTACAACAAGGGGATCATGGGTGAAGACACCTACCCCTACCAGGG<br>661 CAAGGATGGTTATTGCAAGTTCCAACCTGGAAAGGCCATCGGCTTTGTCAAGGATGTAGC<br>721 CAACATCACAATCTATGACGAGGAAGCGATGGTGGAGGCTGTGGCCCTCTACAACCCTGT<br>781 GAGCTTTGCCTTTGAGGTGACTGAAGACTTTATGATGTACAGAAAGGGCATCTACTCCAG<br>841 TACTTCCTGTCATAAAACTCCAGATAAAGTAAACCACGCAGTACTGGCTGTTGGGTATGG<br>901 AGAAGAAAATGGGATACCCTACTGGATCGTGAAAAACTCTTGGGGTCCCCACTGGGGCAT<br>961 GAACGGGTACTTCCTCATTGAGCGTGGGAAGAACATGTGTGGTCTGGCCGCCTGTGCCTC<br>1021 CTACCCCATCCCTCTGGTGTGAGCCGTGGCAGCCGCAGCGCAGACTGGCGGAGAAGGAGA<br>1081 GGAACGGGCAGCCTGGCCATTGGTGGAAGTCCTGCCCTGGAGGAAGTTGTGGGGAGATCC<br>1141 ACTGGGACCCCCAACATTCTGCCCTCACCTCTGTGCCCAGCCTGGAAACCTACAGACAAG<br>1201 GAGGAGTTCCACCATGAGCTCACCCGTGTCTATGACGCAAAGATCACCAGCCATGTGCCT<br>1261 TAGTGTCCTTCTTAACTGACTCAAACCCACGTGGACCAAGAATATTCTTTCCTTCCTGAA<br>1321 GGGCTACTTTTCACATATAGAGCTCCAGGGACTGTCTTTTCTGTATTCGCTGTTCAATAA | SEQ ID NO:286 |

| Gene Name | GenBank Homology | DNA SEQUENCE / DEDUCED AMINO ACID SEQUENCE | SEQUENCE IDENTIFIER: |
|---|---|---|---|
| | | 1381 ACATTGAGTGAGCACCTCCA<br><br>1 M W A T L P L L C A G A W L L G V P V C<br>1 ATGTGGGCCACGCTGCCGCTGCTCTGCGCCGGGGCCTGGCTCCTGGGAGTCCCCGTCTGC<br><br>21 G A A E L S V N S L E K F H F K S W M S<br>61 GGTGCCGCCGAACTGTCCGTGAACTCCTTAGAGAAGTTTCACTTCAAGTCATGGATGTCT<br><br>41 K H R K T Y S T E E Y H H R L Q T F A S<br>121 AAGCACCGTAAGACCTACAGTACGGAGGAGTACCACCACAGGCTGCAGACGTTTGCCAGC<br><br>61 N W R K I N A H N N G N H T F K M A L N<br>181 AACTGGAGGAAGATAAACGCCCACAACAATGGGAACCACACATTTAAAATGGCACTGAAC<br><br>81 Q F S D M S F A E I K H K Y L W S E P Q<br>241 CAATTTTCAGACATGAGCTTTGCTGAAATAAAACACAAGTATCTCTGGTCAGAGCCTCAG<br><br>101 N C S A T K S N Y L R G T G P Y P P S V<br>301 AATTGCTCAGCCACCAAAAGTAACTACCTTCGAGGTACTGGTCCCTACCCACCTTCCGTG<br><br>121 D W R K K G N F V S P V K N Q G A C G S<br>361 GACTGGCGGAAAAAAGGAAATTTTGTCTCACCTGTGAAAAATCAGGGTGCCTGCGGCAGT<br><br>141 C W T F S T T G A L E S A I A I A T G K<br>421 TGCTGGACTTTCTCCACCACTGGGGCCCTGGAGTCTGCAATCGCCATCGCAACCGGAAAG<br><br>161 M L S L A E Q Q L V D C A Q D F N N Y G<br>481 ATGCTGTCCTTGGCGGAACAGCAGCTGGTGGACTGCGCCCAGGACTTCAATAATTACGGC<br><br>181 C Q G G L P S Q A F E Y I L Y N K G I M<br>541 TGCCAAGGGGGTCTCCCCAGCCAGGCTTTCGAGTATATCCTGTACAACAAGGGGATCATG<br><br>201 G E D T Y P Y Q G K D G Y C K F Q P G K<br>601 GGTGAAGACACCTACCCCTACCAGGGCAAGGATGGTTATTGCAAGTTCCAACCTGGAAAG<br><br>221 A I G F V K D V A N I T I Y D E E A M V<br>661 GCCATCGGCTTTGTCAAGGATGTAGCCAACATCACAATCTATGACGAGGAAGCGATGGTG<br><br>241 E A V A L Y N P V S F A F E V T E D F M<br>721 GAGGCTGTGGCCCTCTACAACCCTGTGAGCTTTGCCTTTGAGGTGACTGAAGACTTTATG<br><br>261 M Y R K G I Y S S T S C H K T P D K V N<br>781 ATGTACAGAAAGGGCATCTACTCCAGTACTTCCTGTCATAAAACTCCAGATAAAGTAAAC | SEQ ID NO:287 |

| Gene Name | GenBank Homology | DNA SEQUENCE / DEDUCED AMINO ACID SEQUENCE | SEQUENCE IDENTIFIER: |
|---|---|---|---|
| | | 281 H A V L A V G Y G E E N G I P Y W I V K<br>841 CACGCAGTACTGGCTGTTGGGTATGGAGAAGAAAATGGGATACCCTACTGGATCGTGAAA<br><br>301 N S W G P H W G M N G Y F L I E R G K N<br>901 AACTCTTGGGGTCCCCACTGGGGCATGAACGGGTACTTCCTCATTGAGCGTGGGAAGAAC<br><br>321 M C G L A A C A S Y P I P L V -<br>961 ATGTGTGGTCTGGCCGCCTGTGCCTCCTACCCCATCCCTCTGGTGTGA | |
| BM781186 | Homo sapiens membrane-spanning 4-domains, subfamily A, member 6A, transcript variant 1, mRNA (cDNA clone MGC:22650 IMAGE:3934920). | 1 GGGAGGTAAGTAGAAACCGTTGATGGGACTGAGAAACCAGAGTTAAAACCTCTTTGGAGC<br>61 TTCTGAGGGCTCAGCTGGAACCAACGGGCACAGTTGGCAGCACCATCATGATGTCACAAC<br>121 CCAAGACCAATGAGACCTTCATAGCTCTCACACCAAATGGCATCAGCTTCCTCCAAACAG<br>181 AGGATCCCAAACTTACCAACAAGAGGCAGGACAGCCTGAAGAAACATCTAAAGGCAGAGG<br>241 TCAAAGTTCTCGGGACTATCCAGATCCTGTGTGGGATGATGGTGTTGAGTTTGGGAATTA<br>301 TTTTGGCATCGGCTTCCTTCTCTCAGCATTTTACCCAAGCGTTTTCTATTCTGTTGAAGG<br>361 CTGCTTACCCATTCATAGGAGCCTTGTGTTTTGTCATCTCTGGATCTCTATCAATCATCA<br>421 CAGAGAAAAAGTCCACTAAGCCTTTGGTTCAGAGCAGCCTAGCTGCAAACATTCTGAGCT<br>481 CTCTATCAGCTCTGGTGGGTTTCATCCTCCTTTCTGTCAAACAGGCCACCTTAAATCCTG<br>541 CCTCACTGCAGTGTGAGTTGGACAAAAATAATATACCAACAAGAAGTTATGTTTCTTACT<br>601 TTTATCATGATTCACTTTATACCACGGACTGCTATACAGCCAAAGCCAGTCTGGCTGGAT<br>661 CCCTCTCTCTGATGCTGATTTGCACTCTGCTGGAATTCTGCCTAGCTGTGCTCACTGCTG<br>721 TGCTGCGGTGGAAACAGGCTTACTCTGACTTCCCTGGGAGTGTACTTTTCCTGCCTCACA<br>781 GTTACATTGGTAATTCTGGCATGTCCTCAAAAATGACTCATGACTGTGGATATGAAGAAC<br>841 TATTGACTTCTTAAGAAAAAAGGGAGAAATATTAATCAGAAAGTTGATTCTTATGATAAT<br>901 ATGGAAAAGTTAACCATTATAGAAAAGCAAAGCTTGAGTTTCCTAAATGTAAGCTTTTAA<br>961 AGTAATGAACATTAAAAAAAACCATTATTTCACTGTCATTTAAGATATGTGTTCATTGGG<br>1021 GATCTCTTGATTTGCCTGACATTGACTTCAGCAAAAGCACGGGGCTGTAAATTACCATTT<br>1081 ACTAGATTAGCCAAATAGTCTGAATTTCCAGAAAACAAGGCAGAATGATCATTCCCAGAA<br>1141 ACATTTCCCAGAAAATGTTTCCCAGAAAACTAGACAGAATGATCATTCAATGGATCACAG<br>1201 TGAAGCAAAGGACACAACTTTTTATTGTACCCCTTAATTGTCAACAGGAGTTAACTGATT<br>1261 TGTTGTGGTGCTCAGACTTTTTTATACAGGTGCTAGTGTTTTATCCTATGTATTTTAACT<br>1321 CATTAGTGCATAAAGGCAAGCCCCATATAATGAAGTCTCAGGGTATATGAAAGTAGCTGG<br>1381 CTTCAAAATAAAATTTTTGAGTGCAAAAAAAAAAAAAAAAAAAAAAAAAAAAA | SEQ ID NO:288 |
| | | 1 M M S Q P K T N E T F I A L T P N G I S<br>1 ATGATGTCACAACCCAAGACCAATGAGACCTTCATAGCTCTCACACCAAATGGCATCAGC<br><br>21 F L Q T E D P K L T N K R Q D S L K K H<br>61 TTCCTCCAAACAGAGGATCCCAAACTTACCAACAAGAGGCAGGACAGCCTGAAGAAACAT<br><br>41 L K A E V K V L G T I Q I L C G M M V L | SEQ ID NO:289 |

| Gene Name | GenBank Homology | DNA SEQUENCE / DEDUCED AMINO ACID SEQUENCE | SEQUENCE IDENTIFIER: |
|---|---|---|---|
| | | 121 CTAAAGGCAGAGGTCAAAGTTCTCGGGACTATCCAGATCCTGTGTGGGATGATGGTGTTG<br><br>61 S L G I I L A S A S F S Q H F T Q A F S<br>181 AGTTTGGGAATTATTTTGGCATCGGCTTCCTTCTCTCAGCATTTTACCCAAGCGTTTTCT<br><br>81 I L L K A A Y P F I G A L C F V I S G S<br>241 ATTCTGTTGAAGGCTGCTTACCCATTCATAGGAGCCTTGTGTTTTGTCATCTCTGGATCT<br><br>101 L S I I T E K K S T K P L V Q S S L A A<br>301 CTATCAATCATCACAGAGAAAAAGTCCACTAAGCCTTTGGTTCAGAGCAGCCTAGCTGCA<br><br>121 N I L S S L S A L V G F I L L S V K Q A<br>361 AACATTCTGAGCTCTCTATCAGCTCTGGTGGGTTTCATCCTCCTTTCTGTCAAACAGGCC<br><br>141 T L N P A S L Q C E L D K N N I P T R S<br>421 ACCTTAAATCCTGCCTCACTGCAGTGTGAGTTGGACAAAAATAATATACCAACAAGAAGT<br><br>161 Y V S Y F Y H D S L Y T T D C Y T A K A<br>481 TATGTTTCTTACTTTTATCATGATTCACTTTATACCACGGACTGCTATACAGCCAAAGCC<br><br>181 S L A G S L S L M L I C T L L E F C L A<br>541 AGTCTGGCTGGATCCCTCTCTCTGATGCTGATTTGCACTCTGCTGGAATTCTGCCTAGCT<br><br>201 V L T A V L R W K Q A Y S D F P G S V L<br>601 GTGCTCACTGCTGTGCTGCGGTGGAAACAGGCTTACTCTGACTTCCCTGGGAGTGTACTT<br><br>221 F L P H S Y I G N S G M S S K M T H D C<br>661 TTCCTGCCTCACAGTTACATTGGTAATTCTGGCATGTCCTCAAAAATGACTCATGACTGT<br><br>241 G Y E E L L T S -<br>721 GGATATGAAGAACTATTGACTTCTTAA | |
| BM781233 | H.sapiens mRNA for elongation factor 1-beta. | 1 GCCTCGAGGCGGGCGTCTTCGGTCATCTCCGGCGCTTCTAGGGCTGGTTCCCGTCATCTT<br>61 CGGGAGCCGTGGAGCTCTCGGATACAGCCGACACCATGGGTTTCGGAGACCTGAAAAGCC<br>121 CTGCCGGCCTCCAGGTGCTCAACGATTACCTGGCGGACAAGAGCTACATCGAGGGGTATG<br>181 TGCCATCACAAGCAGATGTGGCAGTATTTGAAGCCGTGTCCAGCCCACCGCCTGCCGACT<br>241 TGTGTCATGCCCTACGTTGGTATAATCACATCAAGTCTTACGAAAAGGAGAAGGCCAGTC<br>301 TGCCAGGAGTGAAGAAAGCTTTGGGCAAGTATGGTCCTGCGGATGTGGAAGACACCACAG<br>361 GAAGTGGAGCTACCGATAGTAAAGACGACGATGACATAGATCTCTTTGGGTCTGATGACG<br>421 AGGAGGAAAGTGAAGAAGCGAAGAGGCTAAGAGAAGAACGCCTTGCACAGTATGAGTCAA<br>481 AGAAAGCCAAGAAACCTGCACTTGTTGCCAAGTCTTCCATCTTACTAGATGTGAAACCTT<br>541 GGGATGACGAGACAGATATGGCAAAGCTGGAGGAGTGCGTCAGAAGCATTCAAGCAGACG | SEQ ID NO:290 |

| Gene Name | GenBank Homology | DNA SEQUENCE / DEDUCED AMINO ACID SEQUENCE | SEQUENCE IDENTIFIER: |
|---|---|---|---|
| | | 601 GCTTGGTCTGGGGCTCCTCTAAACTAGTTCCAGTGGGGTATGGAATTAAAAAACTTCAAA<br>661 TACAGTGTGTAGTTGAAGACGATAAAGTTGGAACTGATATGCTGGAGGAGCGGATCACTG<br>721 CTTTTGAGGACTATGTGCAGTCCATGGATGTGGCCGCTTTCAACAAGATCTAAAACCCGT<br>781 CGTGGATCATGGCATTTAAATAAAAGATTGAAAGATTA<br><br>1 M G F G D L K S P A G L Q V L N D Y L A<br>1 ATGGGTTTCGGAGACCTGAAAAGCCCTGCCGGCCTCCAGGTGCTCAACGATTACCTGGCG<br><br>21 D K S Y I E G Y V P S Q A D V A V F E A<br>61 GACAAGAGCTACATCGAGGGGTATGTGCCATCACAAGCAGATGTGGCAGTATTTGAAGCC<br><br>41 V S S P P P A D L C H A L R W Y N H I K<br>121 GTGTCCAGCCCACCGCCTGCCGACTTGTGTCATGCCCTACGTTGGTATAATCACATCAAG<br><br>61 S Y E K E K A S L P G V K K A L G K Y G<br>181 TCTTACGAAAAGGAGAAGGCCAGTCTGCCAGGAGTGAAGAAAGCTTTGGGCAAGTATGGT<br><br>81 P A D V E D T T G S G A T D S K D D D D<br>241 CCTGCGGATGTGGAAGACACCACAGGAAGTGGAGCTACCGATAGTAAAGACGACGATGAC<br><br>101 I D L F G S D D E E E S E E A K R L R E<br>301 ATAGATCTCTTTGGGTCTGATGACGAGGAGGAAAGTGAAGAAGCGAAGAGGCTAAGAGAA<br><br>121 E R L A Q Y E S K K A K K P A L V A K S<br>361 GAACGCCTTGCACAGTATGAGTCAAAGAAAGCCAAGAAACCTGCACTTGTTGCCAAGTCT<br><br>141 S I L L D V K P W D D E T D M A K L E E<br>421 TCCATCTTACTAGATGTGAAACCTTGGGATGACGAGACAGATATGGCAAAGCTGGAGGAG<br><br>161 C V R S I Q A D G L V W G S S K L V P V<br>481 TGCGTCAGAAGCATTCAAGCAGACGGCTTGGTCTGGGGCTCCTCTAAACTAGTTCCAGTG<br><br>181 G Y G I K K L Q I Q C V V E D D K V G T<br>541 GGGTATGGAATTAAAAAACTTCAAATACAGTGTGTAGTTGAAGACGATAAAGTTGGAACT<br><br>201 D M L E E R I T A F E D Y V Q S M D V A<br>601 GATATGCTGGAGGAGCGGATCACTGCTTTTGAGGACTATGTGCAGTCCATGGATGTGGCC<br><br>221 A F N K I -<br>661 GCTTTCAACAAGATCTAA | SEQ ID NO:291 |
| BM781237 | Homo sapiens transforming | 1 ggcggcggta gcagccaggc ttggccccg gcgtggagca gacgcggacc cctccttcct | SEQ ID NO:292 |

| Gene Name | GenBank Homology | DNA SEQUENCE / DEDUCED AMINO ACID SEQUENCE | SEQUENCE IDENTIFIER: |
|---|---|---|---|
| | acidic coiled-coil containing protein 3 (TACC3) mRNA | 61 ggcggcggcg gcgcgggctc agagcccggc aacgggcggg cgggcagaat gagtctgcag<br>121 gtcttaaacg acaaaaatgt cagcaatgaa aaaaatacag aaaattgcga cttcctgttt<br>181 tcgccaccag aagttaccgg aagatcgtct gttcttcgtg tgtcacagaa agaaaatgtg<br>241 ccacccaaga acctggccaa agctatgaag gtgacttttc agacacctct gcgggatcca<br>301 cagacgcaca ggattctaag tcctagcatg gccagcaaac ttgaggctcc tttcactcag<br>361 gatgacaccc ttggactgga aaactcacac ccggtctgga cacagaaaga gaaccaacag<br>421 ctcatcaagg aagtggatgc caaaactact catggaattc tacagaaacc agtggaggct<br>481 gacaccgacc tcctggggga tgcaagccca gcctttggga gtggcagctc cagcgagtct<br>541 ggcccaggtg ccctggctga cctggactgc tcaagctctt cccagagccc aggaagttct<br>601 gagaaccaaa tggtgtctcc aggaaaagtg tctggcagcc ctgagcaagc cgtggaggaa<br>661 aaccttagtt cctattcctt agacagaaga gtgacacccg cctctgagac cctagaagac<br>721 ccttgcagga cagagtccca gcacaaagcg gagactccgc acggagccga ggaagaatgc<br>781 aaagcggaga ctccgcacgg agccgaggag gaatgccggc acggtggggt ctgtgctccc<br>841 gcagcagtgg ccacttcgcc tcctggtgca atccctaagg aagcctgcgg aggagcaccc<br>901 ctgcagggtc tgcctggcga agccctgggc tgccctgcgg gtgtgggcac ccccgtgcca<br>961 gcagatggca ctcagaccct tacctgtgca cacacctctg ctcctgagag cacagcccca<br>1021 accaaccacc tggtggctgg cagggccatg accctgagtc ctcaggaaga agtggctgca<br>1081 ggccaaatgg ccagctcctc gaggagcgga cctgtaaaac tagaatttga tgtatctgat<br>1141 ggcgccacca gcaaaagggc acccccacca aggagactgg gagagaggtc cggcctcaag<br>1201 cctcccttga ggaaagcagc agtgaggcag caaaaggccc cgcaggaggt ggaggaggac<br>1261 gacggtagga gcggagcagg agaggacccc cccatgccag cttctcgggg ctcttaccac<br>1321 ctcgactggg acaaaatgga tgacccaaac ttcatcccgt tcggaggtga caccaagtct<br>1381 ggttgcagtg aggcccagcc cccagaaagc cctgagacca ggctgggcca gccagcggct<br>1441 gaacagttgc atgctgggcc tgccacggag gagccaggtc cctgtctgag ccagcagctg<br>1501 cattcagcct cagcggagga cacgcctgtg gtgcagttgg cagccgagac cccaacagca<br>1561 gagagcaagg agagagcctt gaactctgcc agcacctcgc ttcccacaag ctgtccaggc<br>1621 agtgagccag tgcccaccca tcagcagggg cagcctgcct tggagctgaa agaggagagc<br>1681 ttcagagacc ccgctgaggt tctaggcacg ggcgcggagg tggattacct ggagcagttt<br>1741 ggaacttcct cgtttaagga gtcggccttg aggaagcagt ccttatacct caagttcgac<br>1801 cccctcctga gggacagtcc tggtagacca gtgcccgtgg ccaccgagac cagcagcatg<br>1861 cacggtgcaa atgagactcc ctcaggacgt ccgcgggaag ccaagcttgt ggagttcgat<br>1921 ttcttgggag cactggacat tcctgtgcca ggcccacccc caggtgttcc cgcgcctggg<br>1981 ggcccacccc tgtccaccgg acctatagtg gacctgctcc agtacagcca gaaggacctg<br>2041 gatgcagtgg taaaggcgac acaggaggag aaccgggagc tgaggagcag gtgtgaggag<br>2101 ctccacggga agaacctgga actggggaag atcatggaca ggttcgaaga ggttgtgtac<br>2161 caggccatgg aggaagttca gaagcagaag gaactttcca aagctgaaat ccagaaagtt<br>2221 ctaaaagaaa aagaccaact taccacagat ctgaactcca tggagaagtc cttctccgac<br>2281 ctcttcaagc gttttgagaa acagaaagag gtgatcgagg gctaccgcaa gaacgaagag<br>2341 tcactgaaga agtgcgtgga ggattacctg gcaaggatca cccaggaggg ccagaggtac<br>2401 caagccctga aggcccacgc ggaggagaag ctgcagctgg caaacgagga gatcgcccag<br>2461 gtccggagca aggcccaggc ggaagcgttg gccctccagg ccagcctgag gaaggagcag<br>2521 atgcgcatcc agtcgctgga gaagacagtg gagcagaaga ctaaagagaa cgaggagctg<br>2581 accaggatct gcgacgacct catctccaag atggagaaga tctgacctcc acggagccgc | |

| Gene Name | GenBank Homology | DNA SEQUENCE / DEDUCED AMINO ACID SEQUENCE | SEQUENCE IDENTIFIER: |
|---|---|---|---|
| | | 2641 tgtccccgcc cccctgctcc cgtctgtctg tcctgtctga ttctcttagg tgtcatgttc<br>2701 tttttctgt cttgtcttca acttttttta aaactagatt gctttgaaaa catgactcaa<br>2761 taaaagtttc ctttcaattt aaaaaaaa<br><br>1 M S L Q V L N D K N V S N E K N T E N C<br>1 ATGAGTCTGCAGGTCTTAAACGACAAAAATGTCAGCAATGAAAAAAATACAGAAAATTGC<br><br>21 D F L F S P P E V T G R S S V L R V S Q<br>61 GACTTCCTGTTTTCGCCACCAGAAGTTACCGGAAGATCGTCTGTTCTTCGTGTGTCACAG<br><br>41 K E N V P P K N L A K A M K V T F Q T P<br>121 AAAGAAAATGTGCCACCCAAGAACCTGGCCAAAGCTATGAAGGTGACTTTTCAGACACCT<br><br>61 L R D P Q T H R I L S P S M A S K L E A<br>181 CTGCGGGATCCACAGACGCACAGGATTCTAAGTCCTAGCATGGCCAGCAAACTTGAGGCT<br><br>81 P F T Q D D T L G L E N S H P V W T Q K<br>241 CCTTTCACTCAGGATGACACCCTTGGACTGGAAAACTCACACCCGGTCTGGACACAGAAA<br><br>101 E N Q Q L I K E V D A K T T H G I L Q K<br>301 GAGAACCAACAGCTCATCAAGGAAGTGGATGCCAAAACTACTCATGGAATTCTACAGAAA<br><br>121 P V E A D T D L L G D A S P A F G S G S<br>361 CCAGTGGAGGCTGACACCGACCTCCTGGGGGATGCAAGCCCAGCCTTTGGGAGTGGCAGC<br><br>141 S S E S G P G A L A D L D C S S S S Q S<br>421 TCCAGCGAGTCTGGCCCAGGTGCCCTGGCTGACCTGGACTGCTCAAGCTCTTCCCAGAGC<br><br>161 P G S S E N Q M V S P G K V S G S P E Q<br>481 CCAGGAAGTTCTGAGAACCAAATGGTGTCTCCAGGAAAAGTGTCTGGCAGCCCTGAGCAA<br><br>181 A V E E N L S S Y S L D R R V T P A S E<br>541 GCCGTGGAGGAAAACCTTAGTTCCTATTCCTTAGACAGAAGAGTGACACCCGCCTCTGAG<br><br>201 T L E D P C R T E S Q H K A E T P H G A<br>601 ACCCTAGAAGACCCTTGCAGGACAGAGTCCCAGCACAAAGCGGAGACTCCGCACGGAGCC<br><br>221 E E E C K A E T P H G A E E E C R H G G<br>661 GAGGAAGAATGCAAAGCGGAGACTCCGCACGGAGCCGAGGAGGAATGCCGGCACGGTGGG<br><br>241 V C A P A A V A T S P P G A I P K E A C<br>721 GTCTGTGCTCCCGCAGCAGTGGCCACTTCGCCTCCTGGTGCAATCCCTAAGGAAGCCTGC | SEQ ID NO:293 |

| Gene Name | GenBank Homology | DNA SEQUENCE / DEDUCED AMINO ACID SEQUENCE | SEQUENCE IDENTIFIER: |
|---|---|---|---|
| | | 261 G G A P L Q G L P G E A L G C P A G V G<br>781 GGAGGAGCACCCCTGCAGGGTCTGCCTGGCGAAGCCCTGGGCTGCCCTGCGGGTGTGGGC<br><br>281 T P V P A D G T Q T L T C A H T S A P E<br>841 ACCCCCGTGCCAGCAGATGGCACTCAGACCCTTACCTGTGCACACACCTCTGCTCCTGAG<br><br>301 S T A P T N H L V A G R A M T L S P Q E<br>901 AGCACAGCCCCAACCAACCACCTGGTGGCTGGCAGGGCCATGACCCTGAGTCCTCAGGAA<br><br>321 E V A A G Q M A S S S R S G P V K L E F<br>961 GAAGTGGCTGCAGGCCAAATGGCCAGCTCCTCGAGGAGCGGACCTGTAAAACTAGAATTT<br><br>341 D V S D G A T S K R A P P P R R L G E R<br>1021 GATGTATCTGATGGCGCCACCAGCAAAAGGGCACCCCCACCAAGGAGACTGGGAGAGAGG<br><br>361 S G L K P P L R K A A V R Q Q K A P Q E<br>1081 TCCGGCCTCAAGCCTCCCTTGAGGAAAGCAGCAGTGAGGCAGCAAAAGGCCCCGCAGGAG<br><br>381 V E E D D G R S G A G E D P P M P A S R<br>1141 GTGGAGGAGGACGACGGTAGGAGCGGAGCAGGAGAGGACCCCCCCATGCCAGCTTCTCGG<br><br>401 G S Y H L D W D K M D D P N F I P F G G<br>1201 GGCTCTTACCACCTCGACTGGGACAAAATGGATGACCCAAACTTCATCCCGTTCGGAGGT<br><br>421 D T K S G C S E A Q P P E S P E T R L G<br>1261 GACACCAAGTCTGGTTGCAGTGAGGCCCAGCCCCCAGAAAGCCCTGAGACCAGGCTGGGC<br><br>441 Q P A A E Q L H A G P A T E E P G P C L<br>1321 CAGCCAGCGGCTGAACAGTTGCATGCTGGGCCTGCCACGGAGGAGCCAGGTCCCTGTCTG<br><br>461 S Q Q L H S A S A E D T P V V Q L A A E<br>1381 AGCCAGCAGCTGCATTCAGCCTCAGCGGAGGACACGCCTGTGGTGCAGTTGGCAGCCGAG<br><br>481 T P T A E S K E R A L N S A S T S L P T<br>1441 ACCCCAACAGCAGAGAGCAAGGAGAGAGCCTTGAACTCTGCCAGCACCTCGCTTCCCACA<br><br>501 S C P G S E P V P T H Q Q G Q P A L E L<br>1501 AGCTGTCCAGGCAGTGAGCCAGTGCCCACCCATCAGCAGGGGCAGCCTGCCTTGGAGCTG<br><br>521 K E E S F R D P A E V L G T G A E V D Y<br>1561 AAAGAGGAGAGCTTCAGAGACCCCGCTGAGGTTCTAGGCACGGGCGCGGAGGTGGATTAC<br><br>541 L E Q F G T S S F K E S A L R K Q S L Y | |

| Gene Name | GenBank Homology | DNA SEQUENCE / DEDUCED AMINO ACID SEQUENCE | SEQUENCE IDENTIFIER: |
|---|---|---|---|
| | | 1621 CTGGAGCAGTTTGGAACTTCCTCGTTTAAGGAGTCGGCCTTGAGGAAGCAGTCCTTATAC<br><br>561 L K F D P L L R D S P G R P V P V A T E<br>1681 CTCAAGTTCGACCCCCTCCTGAGGGACAGTCCTGGTAGACCAGTGCCCGTGGCCACCGAG<br><br>581 T S S M H G A N E T P S G R P R E A K L<br>1741 ACCAGCAGCATGCACGGTGCAAATGAGACTCCCTCAGGACGTCCGCGGGAAGCCAAGCTT<br><br>601 V E F D F L G A L D I P V P G P P P G V<br>1801 GTGGAGTTCGATTTCTTGGGAGCACTGGACATTCCTGTGCCAGGCCCACCCCCAGGTGTT<br><br>621 P A P G G P P L S T G P I V D L L Q Y S<br>1861 CCCGCGCCTGGGGGCCCACCCCTGTCCACCGGACCTATAGTGGACCTGCTCCAGTACAGC<br><br>641 Q K D L D A V V K A T Q E E N R E L R S<br>1921 CAGAAGGACCTGGATGCAGTGGTAAAGGCGACACAGGAGGAGAACCGGGAGCTGAGGAGC<br><br>661 R C E E L H G K N L E L G K I M D R F E<br>1981 AGGTGTGAGGAGCTCCACGGGAAGAACCTGGAACTGGGGAAGATCATGGACAGGTTCGAA<br><br>681 E V V Y Q A M E E V Q K Q K E L S K A E<br>2041 GAGGTTGTGTACCAGGCCATGGAGGAAGTTCAGAAGCAGAAGGAACTTTCCAAAGCTGAA<br><br>701 I Q K V L K E K D Q L T T D L N S M E K<br>2101 ATCCAGAAAGTTCTAAAAGAAAAAGACCAACTTACCACAGATCTGAACTCCATGGAGAAG<br><br>721 S F S D L F K R F E K Q K E V I E G Y R<br>2161 TCCTTCTCCGACCTCTTCAAGCGTTTTGAGAAACAGAAAGAGGTGATCGAGGGCTACCGC<br><br>741 K N E E S L K K C V E D Y L A R I T Q E<br>2221 AAGAACGAAGAGTCACTGAAGAAGTGCGTGGAGGATTACCTGGCAAGGATCACCCAGGAG<br><br>761 G Q R Y Q A L K A H A E E K L Q L A N E<br>2281 GGCCAGAGGTACCAAGCCCTGAAGGCCCACGCGGAGGAGAAGCTGCAGCTGGCAAACGAG<br><br>781 E I A Q V R S K A Q A E A L A L Q A S L<br>2341 GAGATCGCCCAGGTCCGGAGCAAGGCCCAGGCGGAAGCGTTGGCCCTCCAGGCCAGCCTG<br><br>801 R K E Q M R I Q S L E K T V E Q K T K E<br>2401 AGGAAGGAGCAGATGCGCATCCAGTCGCTGGAGAAGACAGTGGAGCAGAAGACTAAAGAG<br><br>821 N E E L T R I C D D L I S K M E K I -<br>2461 AACGAGGAGCTGACCAGGATCTGCGACGACCTCATCTCCAAGATGGAGAAGATCTGA | |

| Gene Name | GenBank Homology | DNA SEQUENCE / DEDUCED AMINO ACID SEQUENCE | SEQUENCE IDENTIFIER: |
|---|---|---|---|
| BM781269 | Equus caballus IL-1RII mRNA for interleukin-1 receptor type II. | 1 TGAAGGTCAGGCCTGGCAGCCGTCCCCAGGCGAGAAGCGACAAGGAGAAAGAATGCAGAG<br>61 CTACAGGCTACAGGGTGCTGGCCCTGAGATCTCCACTTTCTGCATCCTCCAGAAGTTGTC<br>121 GAGAACAATGCCCATTTTGTACATGTTGATATTGGGTGTTCCCGCCTTCACCATTCAGCC<br>181 TGAGGAGCGCACAGTGACTGCAGGAAACTGCCAATTTCTTGGCAAGCATTTCAAAACCGA<br>241 CCTCATGGTGGAAAGGGAGCCTGTGGTCCTGAGGTGCCCCCAGGCATGGTATAGGTGGTT<br>301 GGACTCTGCCAGCTCACATGTCAACGTGACGTGGCGTAAAAACGATTCTACTAGGATGGT<br>361 TCCAGAGGAAGAAGAGACGCGAATGTGGGTCCAGGACAATGCTCTCTGGATTTTGCCGGC<br>421 CTTGCAGGGGGACTCTGGCACCTACATCTGCACTGTCAGAAACGCCTCGTACTGCTATGA<br>481 AATGTCCACTGAGCTCAGGGTTTTTGAGAAGACAGAAGCTGCCCTGACTTACATCTCATA<br>541 CCCGCAGATCCTAACCTTGTCAACCTCTGGCTTACTAGTTTGCCCTGAGCTGAGTGAATT<br>601 CACCCGGAACAAAACTGACGTGGAGGTTCAATGGTACAAGGATTCTGTCCTTTTGGATCA<br>661 AGACAACGAGAAGTTTCTAAGTGTGGCAGGACCCCCTCGTTTACTTGTACAAAATGTGTC<br>721 TATGGAGGATGCGGGCTATTACAGCTGTGTCATGACCTTGATCCACAATGGCACACGAGT<br>781 CAACATCACTAGAAATATCGAACTCCGTGTCCAGAAAAGAAGAGAGGAGACTGTTCCTGT<br>841 GATTGTCTCCCCCCGCCAGACCATACTGGCTTCACTGGGGTCAAGACTGATAATCCCGTG<br>901 TAAGGTGTTTCTGGGAGCCGGCACACAGTGGACCACCGATCTGTGGTGGATGGCCAACAA<br>961 CACCAACATAGACATTGCCTACCGGGGAGGTCGCGTGACGGAGGGGCAGCGCCAGGAGTA<br>1021 CTCAGAAAATAATGAGAACTACATCGAAGTGCCATTGATTTTTGATCCAGTCATAAGAGA<br>1081 GGATTTGAACACGGATTTTAAATGTGTTACCTATAACACACGGAGTTTTCAGACGCTACA<br>1141 TACCACAGTCAAAGAAGCCGCCACGTTCCCCTGGGGACTGTACTGGCCCCTCTTTTGCT<br>1201 GGTCTTCTTGGTTTTGGGAGGAATATGGATGCGCAATCGGTGCAAACACAGACGTGGAAA<br>1261 AGCGTATGGTTTGACTGCATTAAAGATTGAGCGTCAAGTTTTTCAATCTTATCCAAGTAA<br>1321 AATAAAGGAAATAAAATAATTCAAATA | SEQ ID NO:294 |
| | | 1 M P I L Y M L I L G V P A F T I Q P E E<br>1 ATGCCCATTTTGTACATGTTGATATTGGGTGTTCCCGCCTTCACCATTCAGCCTGAGGAG<br><br>21 R T V T A G N C Q F L G K H F K T D L M<br>61 CGCACAGTGACTGCAGGAAACTGCCAATTTCTTGGCAAGCATTTCAAAACCGACCTCATG<br><br>41 V E R E P V V L R C P Q A W Y R W L D S<br>121 GTGGAAAGGGAGCCTGTGGTCCTGAGGTGCCCCCAGGCATGGTATAGGTGGTTGGACTCT<br><br>61 A S S H V N V T W R K N D S T R M V P E<br>181 GCCAGCTCACATGTCAACGTGACGTGGCGTAAAAACGATTCTACTAGGATGGTTCCAGAG<br><br>81 E E E T R M W V Q D N A L W I L P A L Q<br>241 GAAGAAGAGACGCGAATGTGGGTCCAGGACAATGCTCTCTGGATTTTGCCGGCCTTGCAG<br><br>101 G D S G T Y I C T V R N A S Y C Y E M S<br>301 GGGGACTCTGGCACCTACATCTGCACTGTCAGAAACGCCTCGTACTGCTATGAAATGTCC | SEQ ID NO:295 |

| Gene Name | GenBank Homology | DNA SEQUENCE / DEDUCED AMINO ACID SEQUENCE | SEQUENCE IDENTIFIER: |
|---|---|---|---|
| | | 121 T E L R V F E K T E A A L T Y I S Y P Q<br>361 ACTGAGCTCAGGGTTTTTGAGAAGACAGAAGCTGCCCTGACTTACATCTCATACCCGCAG<br><br>141 I L T L S T S G L L V C P E L S E F T R<br>421 ATCCTAACCTTGTCAACCTCTGGCTTACTAGTTTGCCCTGAGCTGAGTGAATTCACCCGG<br><br>161 N K T D V E V Q W Y K D S V L L D Q D N<br>481 AACAAAACTGACGTGGAGGTTCAATGGTACAAGGATTCTGTCCTTTTGGATCAAGACAAC<br><br>181 E K F L S V A G P P R L L V Q N V S M E<br>541 GAGAAGTTTCTAAGTGTGGCAGGACCCCCTCGTTTACTTGTACAAAATGTGTCTATGGAG<br><br>201 D A G Y Y S C V M T L I H N G T R V N I<br>601 GATGCGGGCTATTACAGCTGTGTCATGACCTTGATCCACAATGGCACACGAGTCAACATC<br><br>221 T R N I E L R V Q K R R E E T V P V I V<br>661 ACTAGAAATATCGAACTCCGTGTCCAGAAAAGAAGAGAGGAGACTGTTCCTGTGATTGTC<br><br>241 S P R Q T I L A S L G S R L I I P C K V<br>721 TCCCCCCGCCAGACCATACTGGCTTCACTGGGGTCAAGACTGATAATCCCGTGTAAGGTG<br><br>261 F L G A G T Q W T T D L W W M A N N T N<br>781 TTTCTGGGAGCCGGCACACAGTGGACCACCGATCTGTGGTGGATGGCCAACAACACCAAC<br><br>281 I D I A Y R G G R V T E G Q R Q E Y S E<br>841 ATAGACATTGCCTACCGGGGAGGTCGCGTGACGGAGGGGCAGCGCCAGGAGTACTCAGAA<br><br>301 N N E N Y I E V P L I F D P V I R E D L<br>901 AATAATGAGAACTACATCGAAGTGCCATTGATTTTTGATCCAGTCATAAGAGAGGATTTG<br><br>321 N T D F K C V T Y N T R S F Q T L H T T<br>961 AACACGGATTTTAAATGTGTTACCTATAACACACGGAGTTTTCAGACGCTACATACCACA<br><br>341 V K E A A T F P W G T V L A P L L L V F<br>1021 GTCAAAGAAGCCGCCACGTTCCCCTGGGGGACTGTACTGGCCCCTCTTTTGCTGGTCTTC<br><br>361 L V L G G I W M R N R C K H R R G K A Y<br>1081 TTGGTTTTGGGAGGAATATGGATGCGCAATCGGTGCAAACACAGACGTGGAAAAGCGTAT<br><br>381 G L T A L K I E R Q V F Q S Y P S K I K<br>1141 GGTTTGACTGCATTAAAGATTGAGCGTCAAGTTTTTCAATCTTATCCAAGTAAAATAAAG | |

| Gene Name | GenBank Homology | DNA SEQUENCE / DEDUCED AMINO ACID SEQUENCE | SEQUENCE IDENTIFIER: |
|---|---|---|---|
| | | 401 E I K -<br>1201 GAAATAAAATAA | |
| BM781278 | Homo sapiens chromosome 6 open reading frame 166, mRNA | 1 GTTGGACGTGTTCGCTTGGGTTCCTGCTGCGGCAGCCACCTCGCAATCTCTCTGCATCGA<br>61 TCGCCGCTCGCAAGCTACTGACCGTACTCGGGCGTATTAGGAGCCGCGTTCCAGCCTCAC<br>121 ACCCCACGGTGCTGTTTTCGACTTCAGAAAGGATCTAGCCTCAGCACAGAAGCGCCTCAG<br>181 GCGCGGCGCAAAGCTCGAGCGGACGGCGGGGGCGGCCGGAGCCTCTCTCGGGGGAGCCGC<br>241 GCCTGAGGAGGCGGAAGAACCCCCCTGACGCGACTGGCGTGTGCTTCTGCCCGCCACCGC<br>301 CCCTCCCGCTCTCACCCGGGCCGTCCCTGGCCACTGCCCCTGCCGCGGAGGCAGCGGCGG<br>361 CAGCGGCTCTCCTTTCCACAGCCGGCGCTCCGCGACCCGCTTGGCTCCTGAGCCCGTCGG<br>421 GTAGGCTCTCCTCGAGTTCCCGCTCTTCACCCCTTCCCTCACCCTCTTCTTTCGTCACCC<br>481 GTCCCCGACCCCACCCGAGCCCGGCGCCTCAGCTGCCCCCGGCCATGGCGTGCGGAGCCA<br>541 CTCTGAAAAGGACTCTGGATTTCGACCCGCTGTTGAGCCCGGCGTCCCCGAAGCGCAGGC<br>601 GATGTGCGCCATTGTCGGCGCCCACCTCGGCCGCTGCCTCCCCGTTGTCGGCGGCCGCGG<br>661 CCACCGCCGCCTCCTTCTCCGCTGCGGCCGCCTCGCCGCAGAAGTATCTCCGAATGGAGC<br>721 CATCCCCCTTCGGCGACGTCTCCTCCCGCCTCACCACAGAACAAATTCTGTACAACATAA<br>781 AACAAGAGTATAAACGAATGCAGAAGAGAAGACATTTAGAAACGAGTTTCCAACAGACAG<br>841 ATCCGTGTTGTACTTTTGATGCACAGCCACATGCATTCTTCCTCAGTGGACCAGCTTCGC<br>901 CAGGGGCTTCATCTGCAACATCCTCACCATTAAAAAAAGAACAGCCCTTATTCACTCTAC<br>961 GGCAGGTTGGGATGATCTGTGAACGTTTGTTGAAAGAACGTGAAGAGAAGGTTCGAGAAG<br>1021 AGTATGAAGAAATATTAAACACAAAACTTGCAGAACAATATGATGCATTTGTGAAGTTTA<br>1081 CGCATGATCAAATAATGCGACGATATGGAGAACAGCCTGCTAGTTATGTTTCATGAATCA<br>1141 CGTTTTCTGCATTTGTGGCTGCCTTGTTCTTTGTTGAATTGTTGCAAGAGGTCCCAATT<br>1201 ATGACATGCAGCAATGCCAATACCCCCCTATGAATACAGGTTATTTCAAGCTTTCGTCAG<br>1261 TGGCAACCACTTTAGGCAGCAACTGGTTTTGGAAATTTCCCTGATGTCAATACCACCCGG<br>1321 ATGTGGACCTTTGCTACCTGTATTAATACCAGTGGCCTCATTTGCTGTATCATTACAATT<br>1381 TGGCTTCTTACATTAATGTTTGAAAAGGATTAAATCTGGTATTCTAAGAACATGCCCTTC<br>1441 ACTGGTTGTGTAAATAAAACTGTAGAATGACAAAAAAAAAAAAAAAAAAAAA | SEQ ID NO:296 |
| | | 1 M A C G A T L K R T L D F D P L L S P A<br>1 ATGGCGTGCGGAGCCACTCTGAAAAGGACTCTGGATTTCGACCCGCTGTTGAGCCCGGCG<br><br>21 S P K R R R C A P L S A P T S A A A S P<br>61 TCCCCGAAGCGCAGGCGATGTGCGCCATTGTCGGCGCCCACCTCGGCCGCTGCCTCCCCG<br><br>41 L S A A A A T A A S F S A A A A S P Q K<br>121 TTGTCGGCGGCCGCGGCCACCGCCGCCTCCTTCTCCGCTGCGGCCGCCTCGCCGCAGAAG<br><br>61 Y L R M E P S P F G D V S S R L T T E Q<br>181 TATCTCCGAATGGAGCCATCCCCCTTCGGCGACGTCTCCTCCCGCCTCACCACAGAACAA<br><br>81 I L Y N I K Q E Y K R M Q K R R H L E T | SEQ ID NO:297 |

| Gene Name | GenBank Homology | DNA SEQUENCE / DEDUCED AMINO ACID SEQUENCE | SEQUENCE IDENTIFIER: |
|---|---|---|---|
| | | 241 ATTCTGTACAACATAAAACAAGAGTATAAACGAATGCAGAAGAGAAGACATTTAGAAACG<br><br>101 S F Q Q T D P C C T F D A Q P H A F F L<br>301 AGTTTCCAACAGACAGATCCGTGTTGTACTTTTGATGCACAGCCACATGCATTCTTCCTC<br><br>121 S G P A S P G A S S A T S S P L K K E Q<br>361 AGTGGACCAGCTTCGCCAGGGGCTTCATCTGCAACATCCTCACCATTAAAAAAAGAACAG<br><br>141 P L F T L R Q V G M I C E R L L K E R E<br>421 CCCTTATTCACTCTACGGCAGGTTGGGATGATCTGTGAACGTTTGTTGAAAGAACGTGAA<br><br>161 E K V R E E Y E E I L N T K L A E Q Y D<br>481 GAGAAGGTTCGAGAAGAGTATGAAGAAATATTAAACACAAAACTTGCAGAACAATATGAT<br><br>181 A F V K F T H D Q I M R R Y G E Q P A S<br>541 GCATTTGTGAAGTTTACGCATGATCAAATAATGCGACGATATGGAGAACAGCCTGCTAGT<br><br>201 Y V S -<br>601 TATGTTTCATGA | |
| BM781319 | Homo sapiens KIAK0002 mRNA. | 1 AGAGCGAGCAGGGGAGAGCGAGACCAGTTTTAAGGGGAGGACCGGTGCGAGTAAGGCAGC<br>61 CCCGAGGCTCTGCTCGCCCACCACCCAATCCTCGCCTCCCTTCTGCTCCACCTTCTCTCT<br>121 CTGCCCTCACCTCTCCCCCGAAAACCCCCTATTTAGCCAAAGGAAGGAGGTCAGGGGAAC<br>181 GCTCTCCCCTCCCCTTCCAAAAAACAAAAACAGAAAAACCCTTTTCCAGGCCGGGGAAAG<br>241 CAGGAGGGAGAGGGGCCGCCGGGCTGGCCATGGAGCTGCTGTGCCACGAGGTGGACCCGG<br>301 TCCGCAGGGCCGTGCGGGACCGCAACCTGCTCCGAGACGACCGCGTCCTGCAGAACCTGC<br>361 TCACCATCGAGGAGCGCTACCTTCCGCAGTGCTCCTACTTCAAGTGCGTGCAGAAGGACA<br>421 TCCAACCCTACATGCGCAGAATGGTGGCCACCTGGATGCTGGAGGTCTGTGAGGAACAGA<br>481 AGTGCGAAGAAGAGGTCTTCCCTCTGGCCATGAATTACCTGGACCGTTTCTTGGCTGGGG<br>541 TCCCGACTCCGAAGTCCCATCTGCAACTCCTGGGTGCTGTCTGCATGTTCCTGGCCTCCA<br>601 AACTCAAAGAGACCAGCCCGCTGACCGCGGAGAAGCTGTGCATTTACACCGACAACTCCA<br>661 TCAAGCCTCAGGAGCTGCTGGAGTGGGAACTGGTGGTGCTGGGGAAGTTGAAGTGGAACC<br>721 TGGCAGCTGTCACTCCTCATGACTTCATTGAGCACATCTTGCGCAAGCTGCCCCAGCAGC<br>781 GGGAGAAGCTGTCTCTGATCCGCAAGCATGCTCAGACCTTCATTGCTCTGTGTGCCACCG<br>841 ACTTTAAGTTTGCCATGTACCCACCGTCGATGATCGCAACTGGAAGTGTGGGAGCAGCCA<br>901 TCTGTGGGCTCCAGCAGGATGAGGAAGTGAGCTCGCTCACTTGTGATGCCCTGACTGAGC<br>961 TGCTGGCTAAGATCACCAACACAGACGTGGATTGTCTCAAAGCTTGCCAGGAGCAGATTG<br>1021 AGGCGGTGCTCCTCAATAGCCTGCAGCAGTACCGTCAGGACCAACGTGACGGATCCAAGT<br>1081 CGGAGGATGAACTGGACCAAGCCAGCACCCCTACAGACGTGCGGGATATCGACCTGTGAG<br>1141 GATGCCAGTTGGGCCGAAAGAGAGAGACGCGTCCATAATCTGGTCTCTTCTTCTTTCTGG<br>1201 TTGTTTTTGTTCTTTGTGTTTTAGGGTGAAACTTAAAAAAAAAATTCTGCCCCCACCTAG<br>1261 ATCATATTTAAAGATCTTTTAGAAGTGAGAGAAAAAGGTCCTACGAAAACGGAATAATAA | SEQ ID NO:298 |

| Gene Name | GenBank Homology | DNA SEQUENCE / DEDUCED AMINO ACID SEQUENCE | SEQUENCE IDENTIFIER: |
|---|---|---|---|
| | | 1321 AAAGCATTTGGTGCCTATTTGAAGTACAGCATAAGGGAATCCCTTGTATATGCGAACAGT<br>1381 TATTGTTTGATTATGTAAAAGTAATAGTAAAATGCTTACAGGAAAACCTGCAGAGTAGTT<br>1441 AGAGAATATGTATGCCTGCAATATGGAACAAATTAGAGGAGACTTTTTTTTTCATGTTA<br>1501 TGAGCTAGCACATACACCCCCTTGTAGTATAATTTCAAGGAACTGTGTACGCCATTTATG<br>1561 GCATGATTAGATTGCAAAGCAATGAACTCAAGAAGGAATTGAAATAAGGAGGGACATGAT<br>1621 GGGGAAGGAGTACAAAACAATCTCTCAACATGATTGAACCATTTGGGATGGAGAAGCACC<br>1681 TTTGCTCTCAGCCACCTGTTACTAAGTCAGGAGTGTAGTTGGATCTCTACATTAATGTCC<br>1741 TCTTGCTGTCTACAGTAGCTGCTACCTAAAAAAAGATGTTTTATTTTGCCAGTTGGACAC<br>1801 AGGTGATTGGCTCCTGGGTTTCATGTTCTGTGACATCCTGCTTCTTCTTCCAAATGCAGT<br>1861 TCATTGCAGACACCACCATATTGCTATCTAATGGGGAAATGTAGCTATGGGCCATAACCA<br>1921 AAACTCACATGAAACGGAGGCAGATGGAGACCAAGGGTGGGATCCAGAATGGAGTCTTTT<br>1981 CTGTTATTGTATTTAAAAGGGTAATGTGGCCTTGGCATTTCTTCTTAGAAAAAAACTAAT<br>2041 TTTTGGTGCTGATTGGCATGTCTGGTTCACAGTTTAGCATTGTTATAAACCATTCCATTC<br>2101 GAAAAGCACTTTGAAAAATTGTTCCCGAGCGATAGATGGGATGGTTTATGCAAGTCATGC<br>2161 TGAATACTCCTCCCCTCTTCTCTTTTGCCCCCTCCCTTCCTGCCCCCAGTCTGGGTTACT<br>2221 CTTCGCTTCTGGTATCTGGCGTTCTTTGGTACACAGTTCTGGTGTTCCTACCAGGACTCA<br>2281 AGAGACACCCCTTCCTGCTGACATTCCCATCACAACATTCCTCAGACAAGCCTGTAAACT<br>2341 AAAATCTGTTACCATCTGATGGCACAGAAGGATCTTAATTCCCATCTCTATACTTCTCCT<br>2401 TTGGACATGGAAAGAAAAGTTATTGCTGGTGCAAAGATAGATGGCTGAACATCAGGGTGT<br>2461 GGCATTTTGTTCCCTTTTCCGTTTTTTTTTTTTTTATTGTTGTTGTTAATTTTATTGCAA<br>2521 AGTTGTATTCAGCGTACTTGAATTTTTCTTCCTCTCCACTTCTTAGAGGCATTCAGTTAG<br>2581 CAAAGAGGTTGGAGCAACAACTTTTTTTTTTTTTTTTTGCACAATTGTAATTGACAGGTA<br>2641 ATGAAGCTATTTGTTAAAATATTTGCCTTTTTAAGTAAAAAAGAAAAATCAGAACAGGGC<br>2701 TATTTGAAGAATTATTTTATACACAGATTCTGCCTTGTTTCATAGTATGAGGGTTGAAGA<br>2761 CGGAAAACAATCTAAGGGTCTCTCATTTTTTTAATTTTGTTTTGTTCAGTTTGGTTTTTT<br>2821 TTTTTTTTTGCGCTGCTAAGAAGCTAAAGTCATCCATCCTTATTCACGTTGACAGTACCT<br>2881 AGCTGTAATGTTTCACAGAGTGTGCTGCTATTTTATAAACATTTTTATAATATATTATTT<br>2941 TACTGCTTAAATTCCAAGTCCTGAAGTAGATGGTTGAGATATGAGTTCTTCGTACTGGAA<br>3001 AAGCCCTTCCGTAGTTTGTTTTCTTCTGGTAGCATATTCATGGTTGTTTTTTTTTTTCTT<br>3061 TTTTGGTTTTTTGGTTTTTTTTTTTTTCCTCTGATCACATTCTTCAAAGACGGAGTATTCT<br>3121 TACCTCAGGTTTACTGGACAAAATCAATAACTACAAAAGGCAATGATTCACGCTTTTGTT<br>3181 TTCATAATACCTCACAACCGTACAGTTTCTGCTTGGGAGCCCATTCGCATGAGGAATACA<br>3241 GAAGCAGTGTGAGCAGGGCTGACTCCCTCTCAGGTGGAAGGCAGGGCGGTCTCACTCCCA<br>3301 GGGACCTTTTTGGTCATGGAGGCCATCGGGCTCCCAGTTAGACCCTGGTATCCTCATCAT<br>3361 GATGGAAAAAATACATTGAACCAAGGGATCCTCCCTCCCCTTCAAGGCAGACGTTCAGTA<br>3421 CAAACATTTATGCGGTAGGCTCAGATGTCGTAATTTGCACTTAGGTACCAGGTGTCAGGA<br>3481 AACAGACTAAAAAGAATTCCACCAGGCTGTTTGGAGATCCTCATCTTGGAGCTTTTTCAA<br>3541 AAGCGGGGCTTCATCTGCAAAGGGCCCTTTCATCTTGAAGTTTTTCCCCTCCGTCTTTCC<br>3601 CCTCCCCTGGCATGGACACCTTGTGTTTAGGATCATCTCTGCAGGTTTCCTAGGTCTGAA<br>3661 TCTGCGAGTAGATGAACCTGCAGCAAGCAGCGTTTATGGTGCTTCCTTCTCCCTCCTCTG<br>3721 TCTCAAACTGCGCAGGCAAGCACTATGCAAGCCCAGGCCCTCTGCTGAGCGGTACTAAAC<br>3781 GGTCGGGTTTTCAATCACACTGAATTGGCAGGATAAGAAAAATAGGTCAGATAAGTATGG<br>3841 GATGATAGTTGAAGGGAGGTGAAGAGGCTGCTTCTCTACAGAGGTGAAATTCCAGATGAG | |

| Gene Name | GenBank Homology | DNA SEQUENCE / DEDUCED AMINO ACID SEQUENCE | SEQUENCE IDENTIFIER: |
|---|---|---|---|
| | | 3901 TCAGTCTCTTGGGAAGTGTGTTTAGAAGGGTTCAGGACTTTGTGAGTTAGCATGACCCTA<br>3961 AAATTCTAGGGGATTTCTGGTGGGACAATGGGTGGTGAATTTTGAAGTTTTGGAGAGGGA<br>4021 AGTGGAGCAGCCAGCAAGTAAGCTAGCCAGAGTTTTCTCAAGAGCCAGCTTTGCTCAGCA<br>4081 CACTCTCCTGGGCCCCAAGGAGTCCCACGGAATGGGGAAAGTGGGAACCCTGGAGTTCTT<br>4141 GGGAATCTTGGAGCCTAAAGAGAAACCGAGGTGCAAATTCATTTCATGGTGACTGACCCT<br>4201 TGAGCTTAAACAGAAGCAGCAAATGAAAGAACCGGACAAATAAGGAAGGGCACAAGCCTA<br>4261 CCCGACTCTATTTACAGTCTGTAACTTTCCACTCTTCCTGTAGTCCCGAGGCCCCTGGGT<br>4321 CCTTCTAGCTTTTCTCTTTCCCATCCTTGGGGCCTTGTGTGATGATGGGTGTGGGGCTGC<br>4381 CGATGGGAAAGTCGGGGGTTGTTAGGCTTTTCTGCCTGCTCCTGCTTAAACACAAGAAGG<br>4441 AATCCTGGATTTTGCCCTCTCCTTAGCTCTTAGTCTCTTTGGTAGGAGTTTTGTTCCAGA<br>4501 GGAGCTCTCCCCCTTGGATTTGAACTTGCTCTTTTTGTTGTTGTTGTTCTTTCTCTTCTT<br>4561 TTTCTTACCTCCCACTAAAGGGGTTCCAAATTATCCTGGTCTTTTTCTACCTTGTTGTGT<br>4621 TTCTATCTCGTCTTTACTTCCATCTGTTTGTTTTTTTCTCCATCAGTGGGGGCCGAGTTG<br>4681 TTCCCCCAGCCTGCCAAATTTTGATCCTTCCCCTCTTTTGGCCAAATCCTAGGGGGAAGA<br>4741 AATCCTAGTATGCCAAAAATATATGCTAAGCATAATTAAACTCCATGCGGGTCCATAACA<br>4801 GCCAAGAAGCCTGCAGGAGAAAGCCAAGGGCAGTTCCCTCCGCAGAACACCCCATGCGTG<br>4861 CTGAGAGGCGAGCTCCTTGAAGAAGGGGCTGTTCTTCCAGGAGGCCTTATTTTGAACTGC<br>4921 CTCAGGACCCCACTGGAGAGCACAGCATGCCTTACTACTGGGTCATCCTTGGTCTATGTG<br>4981 CTCTGTACTGGAGGCTCTGTTCTGCCTCTTATCAGCCAGGTCAGGGGCACACATGGCTTA<br>5041 AGTGACAAAGCCAGAGGAGAAGACAACCCTGACAGCATCACGCTGCATCCCATTGCTAGC<br>5101 AGGATTGGCAACTCTTCAGACGGAGCTGCGCTTCCCTGCAGTCTAGCACCTCTAGGGCCT<br>5161 CTCCAGACTGTGCCCTGGGAGCTCTGGGACTGAAAGGTTAAGAACATAAGGCAGGATCAG<br>5221 ATGACTCTCTCCAAGAGGGCAGGGGAATTTTCTCTCCATGGGCCACAGGGGACAGGGCTG<br>5281 GGAGAAGAAATAGACTTGCACCTTATGTCATGTAAATAATTGATTTTCTAGTTCAAGAAG<br>5341 ATAATATTGGTAGTGTGGGAATTGGAGGTAGGAAGGGGAGGAAGTCTGAGTAAGCCAGTT<br>5401 GGCTTCTAAGCCAAAAGGATTCCTCTTTGTTTATCTCTGAGACAGTCCAACCTTGAGAAT<br>5461 AGCTTTAAAAGGGAAATTAATGCTGAGATGATAAAGTCCCCTTAAGCCAACAAACCCTCT<br>5521 GTAGCTATAGAATGAGTGCAGGTTTCTATTGGTGTGGACTCAGAGCAATTTACAAGAGCT<br>5581 GTTCATGCAGCCATCCATTTGTGCAAAATAGGGTAAGAAGATTCAAGAGGATATTTATTA<br>5641 CTTCCTCATACCACATGGCTTTTGATGATTCTGGATTCTAAACAACCCAGAATGGTCATT<br>5701 TCAGGCACAACGATACTACATTCGTGTGTGTCTGCTTTTAAACTTGGCTGGGCTATCAGA<br>5761 CCCTATTCTCGGCTCAGGTTTTGAGAAGCCATCAGCAAATGTGTACGTGCATGCTGTAGC<br>5821 TGCAGCCTGCATCCCTTCGCCTGCAGCCTACTTTGGGGAAATAAAGTGCCTTACTGACTG<br>5881 TAGCCATTACAGTATCCAATGTATTTTGACAGGTGCCTGTCCTTGAAAAACAAAGTTTCT<br>5941 ATTTTTATTTTTAATTGGTTTTAGTTCTTACTGGCCAACTCTTAAATCCCCAGCAAATCA<br>6001 CTGGGCCATTGGATTTTTTCCATTATGTTCATTACCCTTGTACCATGTACCTCAGATCTC<br>6061 TCTCTCTCTCCTCTCTCTCAGTTATATAGTTTCTTGTCTTGGACTTTTTTTTTCTTTTCT<br>6121 TTTTCTTTTTTTTTTTGCTTTAAAACAAGTGTGATGCCATATCGAGTCAATGTTATTCTC<br>6181 TCACAATGTACTCTATAAGAGGTGTGGGTGTTTATTTGGTCGGGATGCTAGCAAGTGCTA<br>6241 AAGTAGCATGATCAGTATAAACAAAAGGTTTTTAGGAAGTATGGAAAAAATGTTTTATTG<br>6301 GCTATGATGGTGACATGGTATAGTCAGCTACCTTTTAAGAGGTCCTATCTGTTCAGTGTT<br>6361 AAGTGATTTAAAAAAAATAATAACCTGTTATTCTGACTAGCTTAAAGATGGATTTGAAAA<br>6421 TGGTTTTGGATGCAATTAGGTTATGCTATTTGGACAATAAACTCACCTTGACCT | |

| Gene Name | GenBank Homology | DNA SEQUENCE / DEDUCED AMINO ACID SEQUENCE | SEQUENCE IDENTIFIER: |
|---|---|---|---|
| | | 1 M E L L C H E V D P V R R A V R D R N L<br>1 ATGGAGCTGCTGTGCCACGAGGTGGACCCGGTCCGCAGGGCCGTGCGGGACCGCAACCTG<br><br>21 L R D D R V L Q N L L T I E E R Y L P Q<br>61 CTCCGAGACGACCGCGTCCTGCAGAACCTGCTCACCATCGAGGAGCGCTACCTTCCGCAG<br><br>41 C S Y F K C V Q K D I Q P Y M R R M V A<br>121 TGCTCCTACTTCAAGTGCGTGCAGAAGGACATCCAACCCTACATGCGCAGAATGGTGGCC<br><br>61 T W M L E V C E E Q K C E E E V F P L A<br>181 ACCTGGATGCTGGAGGTCTGTGAGGAACAGAAGTGCGAAGAAGAGGTCTTCCCTCTGGCC<br><br>81 M N Y L D R F L A G V P T P K S H L Q L<br>241 ATGAATTACCTGGACCGTTTCTTGGCTGGGGTCCCGACTCCGAAGTCCCATCTGCAACTC<br><br>101 L G A V C M F L A S K L K E T S P L T A<br>301 CTGGGTGCTGTCTGCATGTTCCTGGCCTCCAAACTCAAAGAGACCAGCCCGCTGACCGCG<br><br>121 E K L C I Y T D N S I K P Q E L L E W E<br>361 GAGAAGCTGTGCATTTACACCGACAACTCCATCAAGCCTCAGGAGCTGCTGGAGTGGGAA<br><br>141 L V V L G K L K W N L A A V T P H D F I<br>421 CTGGTGGTGCTGGGGAAGTTGAAGTGGAACCTGGCAGCTGTCACTCCTCATGACTTCATT<br><br>161 E H I L R K L P Q Q R E K L S L I R K H<br>481 GAGCACATCTTGCGCAAGCTGCCCCAGCAGCGGGAGAAGCTGTCTCTGATCCGCAAGCAT<br><br>181 A Q T F I A L C A T D F K F A M Y P P S<br>541 GCTCAGACCTTCATTGCTCTGTGTGCCACCGACTTTAAGTTTGCCATGTACCCACCGTCG<br><br>201 M I A T G S V G A A I C G L Q Q D E E V<br>601 ATGATCGCAACTGGAAGTGTGGGAGCAGCCATCTGTGGGCTCCAGCAGGATGAGGAAGTG<br><br>221 S S L T C D A L T E L L A K I T N T D V<br>661 AGCTCGCTCACTTGTGATGCCCTGACTGAGCTGCTGGCTAAGATCACCAACACAGACGTG<br><br>241 D C L K A C Q E Q I E A V L L N S L Q Q<br>721 GATTGTCTCAAAGCTTGCCAGGAGCAGATTGAGGCGGTGCTCCTCAATAGCCTGCAGCAG<br><br>261 Y R Q D Q R D G S K S E D E L D Q A S T<br>781 TACCGTCAGGACCAACGTGACGGATCCAAGTCGGAGGATGAACTGGACCAAGCCAGCACC | SEQ ID NO:299 |

| Gene Name | GenBank Homology | DNA SEQUENCE / DEDUCED AMINO ACID SEQUENCE | SEQUENCE IDENTIFIER: |
|---|---|---|---|
| | | 281 P T D V R D I D L -<br>841 CCTACAGACGTGCGGGATATCGACCTGTGA | |
| BM781375 | Homo sapiens EPK2 mRNA for serine/threonine kinase. | 1 AAAGTTCATCCCCCCAGAATGAAAATGAGGACATTTGAGAAGGTGATTTAAGGTGTGGAC<br>61 ATTTGAGAAGGTGTCCTATCAAATTAGTAAACCAAAGGAAAAGTACTGAATAGATTAATC<br>121 CAAAACACTTACTGTTTTTTAAACGAAGAGGATTTCACCTTGACAGAAAAACAACTTTTA<br>181 TTCAATATGTATTTCCTGAAATTAAAGAGACAAGTACAGACTGAAAGGAAAATAGATTCG<br>241 TAAATAAGCTACGTCAACTCTATCCTGCTGAGGATAGCTCAGTGATGTTAAATCCTTTAC<br>301 AAATCCCTGGTTGTCTTCCTACAGACAAGACTGCTTTTTGATGGGACTGATATTAAGAGA<br>361 AATAGGACCTTTGGGGCATTCAACTCCTTGATAAAACTTAAAAGTATCGGCATGAGTGGC<br>421 TTAACAGAGGAAATAAAGAAGTTTTCAACTAAATCCAAAAGTGCGGTCATTTTCTTTACT<br>481 GCTGTTATTTTAAAAACCTCTTCATAACCATTGAAAAAGAATCGACAACTATTTTAAAAG<br>541 ATTAAAGAAAGGCAGATGTCTGCAAATAATTCCCCTCCATCAGCCCAGAAGTCTGTATTA<br>601 CCCACAGCTATTCCTGCTGTGCTTCCAGCTGCTTCTCCGTGTTCAAGTCCTAAGACGGGA<br>661 CTCTCTGCCCGACTCTCTAATGGAAGCTTCAGTGCACCATCACTCACCAACTCCAGAGGC<br>721 TCAGTGCATACAGTTTCATTTCTACTGCAAATTGGCCTCACACGGGAGAGTGTTACCATT<br>781 GAAGCCCAGGAACTGTCTTTATCTGCTGTCAAGGATCTTGTGTGCTCCATAGTTTATCAA<br>841 AAGTTTCCAGAGTGTGGATTCTTTGGCATGTATGACAAAATTCTTCTCTTTCGCCATGAC<br>901 ATGAACTCAGAAAACATTTTGCAGCTGATTACCTCAGCAGATGAAATACATGAAGGAGAC<br>961 CTAGTGGAAGTGGTTCTTTCAGCTTTAGCCACAGTAGAAGACTTCCAGATTCGTCCACAT<br>1021 ACTCTCTATGTACATTCTTACAAAGCTCCTACTTTCTGTGATTACTGTGGTGAGATGCTG<br>1081 TGGGGATTGGTACGTCAAGGACTGAAATGTGAAGGCTGTGGATTAAATTACCATAAACGA<br>1141 TGTGCCTTCAAGATTCCAAATAACTGTAGTGGAGTAAGAAAGAGACGTCTGTCAAATGTA<br>1201 TCTTTACCAGGACCCGGCCTCTCAGTTCCAAGACCCCTACAGCCTGAATATGTAGCCCTT<br>1261 CCCAGTGAAGAGTCACATGTCCACCAGGAACCAAGTAAGAGAATTCCTTCTTGGAGTGGT<br>1321 CGCCCAATCTGGATGGAAAAGATGGTAATGTGCAGAGTGAAAGTTCCACACACATTTGCT<br>1381 GTTCACTCTTACACCCGTCCCACGATATGTCAGTACTGCAAGCGGTTACTGAAAGGCCTC<br>1441 TTTCGCCAAGGAATGCAGTGTAAAGATTGCAAATTCAACTGCCATAAACGCTGTGCATCA<br>1501 AAAGTACCAAGAGACTGCCTTGGAGAGGTTACTTTCAATGGAGAACCTTCCAGTCTGGGA<br>1561 ACAGATACAGATATACCAATGGATATTGACAATAATGACATAAATAGTGATAGTAGTCGG<br>1621 GGTTTGGATGACACAGAAGAGCCATCACCCCCAGAAGATAAGATGTTCTTCTTGGATCCA<br>1681 TCTGATCTCGATGTGGAAAGAGATGAAGAAGCCGTTAAAACAATCAGTCCATCAACAAGC<br>1741 AATAATATTCCGCTAATGAGGGTTGTACAATCCATCAAGCACACAAAGAGGAAGAGCAGC<br>1801 ACAATGGTGAAGGAAGGGTGGATGGTCCATTACACCAGCAGGGATAACCTGAGAAAGAGG<br>1861 CATTATTGGAGACTTGACAGCAAATGTCTAACATTATTTCAGAATGAATCTGGATCAAAG<br>1921 TATTATAAGGAAATTCCACTTTCAGAAATTCTCCGCATATCTTCACCACGAGATTTCACA<br>1981 AACATTTCACAAGGCAGCAATCCACACTGTTTTGAAATCATTACTGATACTATGGTATAC<br>2041 TTCGTTGGTGAGAACAATGGGGACAGCTCTCATAATCCTGTTCTTGCTGCCACTGGAGTT<br>2101 GGACTTGATGTAGCACAGAGCTGGGAAAAAGCAATTCGCCAAGCCCTCATGCCTGTTACT<br>2161 CCTCAAGCAAGTGTTTGCACTTCTCCAGGGCAAGGGAAAGATCACAAAGATTTGTCTACA<br>2221 AGTATCTCTGTATCTAATTGTCAGATTCAGGAGAATGTGGATATCAGTACTGTTTACCAG<br>2281 ATCTTTGCAGATGAGGTGCTTGGTTCAGGCCAGTTTGGCATCGTTTATGGAGGAAAACAT | SEQ ID NO:300 |

| Gene Name | GenBank Homology | DNA SEQUENCE / DEDUCED AMINO ACID SEQUENCE | SEQUENCE IDENTIFIER: |
|---|---|---|---|
| | | 2341 AGAAAGACTGGGAGGGATGTGGCTATTAAAGTAATTGATAAGATGAGATTCCCCACAAAA<br>2401 CAAGAAAGTCAACTCCGTAATGAAGTGGCTATTTTACAGAATTTGCACCATCCTGGGATT<br>2461 GTAAACCTGGAATGTATGTTTGAAACCCCAGAACGAGTCTTTGTAGTAATGGAAAAGCTG<br>2521 CATGGAGATATGTTGGAAATGATTCTATCCAGTGAGAAAAGTCGGCTTCCAGAACGAATT<br>2581 ACTAAATTCATGGTCACACAGATACTTGTTGCTTTGAGGAATCTGCATTTTAAGAATATT<br>2641 GTGCACTGTGATTTAAAGCCAGAAAATGTGCTGCTTGCATCAGCAGAGCCATTTCCTCAG<br>2701 GTGAAGCTGTGTGACTTTGGATTTGCACGCATCATTGGTGAAAAGTCATTCAGGAGATCT<br>2761 GTGGTAGGAACTCCAGCATACTTAGCCCCTGAAGTTCTCCGGAGCAAAGGTTACAACCGT<br>2821 TCCCTAGATATGTGGTCAGTGGGAGTTATCATCTATGTGAGCCTCAGTGGCACATTTCCT<br>2881 TTTAATGAGGATGAAGATATAAATGACCAAATCCAAAATGCTGCATTTATGTACCCACCA<br>2941 AATCCATGGAGAGAAATTTCTGGTGAAGCAATTGATCTGATAAACAATCTGCTTCAAGTG<br>3001 AAGATGAGAAAACGTTACAGTGTTGACAAATCTCTTAGTCATCCCTGGCTACAGGACTAT<br>3061 CAGACTTGGCTTGACCTTAGAGAATTTGAAACTCGCATTGGAGAACGTTACATTACACAT<br>3121 GAAAGTGATGATGCTCGCTGGGAAATACATGCATACACACATAACCTTGTATACCCAAAG<br>3181 CACTTCATTATGGCTCCTAATCCAGATGATATGGAAGAAGATCCTTAATCACTGAGCTAA<br>3241 CCTAAATAAGGAAGGATTTCATTTTATGGACTGATATTTTGCTGTGTAACTTGTTCTTCG<br>3301 TAGATTGTCATCTGCAGTGCTGCAAAGATATGAAGAAATATGATAACGAATAAGTGACAC<br>3361 CAGTACTGTAGTTCATAATGAGTAGGTACAGGCGGGAAACTGAATAATAAGAAGTCATAA<br>3421 TGGAATCAAGGTGAAGCTTTTTATAAACTTTTTTAGCCTAAGCAATAACTGGTTTTGTAT<br>3481 TTTTTCTTAATCCTTCACTTTAATACAATAGGCTCACTTAATTTGTCTTCCCATTTCTCT<br>3541 TTATATATATATATATATATAAAAAAATATAAATATATGTTTGTTTGTTTGTTTTTTTAA<br>3601 GGAAAAACAAGTCAAGCTAGCATCCAGTTACTATATAGCTTGGCTAAATTATACAAGACT<br>3661 TACAAGATTGATTACTCGACAGGCTTGTATTTAAGAGATAACTGTGAGGTTACCATTATG<br>3721 TGATGTTACTATAAGGACTTTTAACATTGGTTTAACAAACCATAGAGGCATTGAAGGGTT<br>3781 TTTCTTAGATGCCTAGAAAAAGCACACTGGGCTGTTTTACCTTTCTTTTTTAGGTCAATC<br>3841 AAGACTCCAAAATAGTGATTCCTAACCTTTTTGGAGTTGCTCTGCTACTCTGAATATGTT<br>3901 CTATACAGCATAAGGATTGTCACCTTCTGTGTGTTGCAACAGCTTCTAAGATAATTAGGG<br>3961 ACAAATGATGTTACAAAAGGAAGAGTACTGCTGGTCTAAGTGCTGAGTTGTATGTCTTTG<br>4021 CATAGCTCCACTCTGCTGCTAAATATGCATGTTCTGACTGACACCATCTTGATGCCAGTA<br>4081 CTGGATTCCAGCATTCAGCAGGTGCAGATCTCGGCTTTACACAATTTATCTTTACCTAGG<br>4141 GTTCAGTCAGTAATTTCTGCTTTTTAGCCAGGGCCAGTGCAGGGTCAGTTAATGCTACAG<br>4201 TTACTGTATAGCAAACAGTATCCTTTTTTCTCCTTCCCCTAGCCTATTGGGCTTTGCAGA<br>4261 TATCTGGAGTGTTTTAAAGTCAATTATTTTAAGCAGTTTGAGGGGATGTGTAGGAGTGGA<br>4321 GCATGAAACAGTTTATAAGGCTGGGGCTGTATTATCAGCACAGCAAATTAAAGAATGAAA<br>4381 GAAGTACTTCTTTTTACATTTCAGCTCCAGCAGCCAGCTATTTAAAAAATATTTTTAAAT<br>4441 ATCTTCCCCAAAGTTTAAGATGTGGACCATCTACTTGTAAGAAACAGTGGCTTATTTCT<br>4501 TCATTTCTCAGTAATCATTGTAAACCTTTTTTTTTTTCTTCTCCACTCTAACAAAAGTAA<br>4561 AAGAACAAAACTTTTGCTGAGGTCTTGGACTCCACTACTTGTTAGTTATGTTGGCCTGGA<br>4621 CAGATGGCAGGTCTGTCGGCAGTTCCTCAGTCTGTAAAATGAAGATTATCATTCTTGCCT<br>4681 CTCCCTTTGTCACAGTGTTGTCGTGAAGATCAGATATGTATGAATGCAGTCAAATAAACT<br>4741 AAAAACTAGGAAAGTGTTAACTATCGTTGCCCACCGAATTTGAGGTAGCAAAAAAAAAAA<br>4801 AGAATGTTCTGGTACATGAACAGACTGAGAAGGCCTTTCGACATCCCTGAAGCTAGTCTC<br>4861 CTGTCTAGACTAAAAATATCCTTGAAAGAATAGTAGCAAACAGTATGCAGTACTCCTGTG | |

| Gene Name | GenBank Homology | DNA SEQUENCE / DEDUCED AMINO ACID SEQUENCE | SEQUENCE IDENTIFIER: |
|---|---|---|---|
| | | 4921 TTCTAAGTTCTTTTATGTTTTAGTTTACCCTCACAGCAGCCATAGTGTTAAGTCATTATG<br>4981 AGCTTCATCTTAAAGATAAGAAAACTGAGGCAAGGGGAGATTAACTTACTGCCAAATTTC<br>5041 AAACAGTTAGTAGTATTTGAGACCAGTAGTATGGCTCTAATCTTACCCTTAGCCCTCCCA<br>5101 ACCATTATGCTGTACTATCTACCCATAAATACCTCCAGGAAACATTCCCCTCTTGGTAAT<br>5161 TTGTCCTTCTATAGAAGCTTCAAAGATTAAATCAAATTAATTTTCAAAAAAAATTTTTTT<br>5221 TTAGAAATGTTATTTTCCCTGTGATAGAGGATGACTTCCCAGTTTCACCAAAGTCTGTTT<br>5281 ATATCAACGCGCAAAACAGAGTAATTCTGAGTCACTAGGCACCCTATTATGGTTTTACTG<br>5341 CATAAGGTGAAAATTAACTGGAATGATGTTTCTTTGCTATACTTAAATAATCAAACTTTA<br>5401 CAAGCCATGAAATTAATTGCACTCTTTTTGTTTTTATTGTTGAATGCCTAAGAAGTTTTC<br>5461 TAAAAAAATTTGCAGAGGCACTGTCAGATAATTTGGAGTTGAGTAACTACTCCAGATACT<br>5521 GTATAACTTTTTGTCTTTAAGTCGTGTTTGTATAAGAAATAATTGTTAGAAACATTTGAT<br>5581 AATGTACAAAGTAGTCTATAATGACACTGTTCAGTACATTTTTATATTTTTTGTTACATC<br>5641 CCACTTTTTGTAAATATCCTCAGAGAAGCTTGATAATAAAATGTATTTCCTATCACCATA<br>5701 CTTTTCCATGTGAAAACCTGAGCCTATTTCTAGTATAAGTATCCAAAGAAAAGTTTTACC<br>5761 TGGTTGTGTTATTTTACCAGAC | |
| | | 1 M S A N N S P P S A Q K S V L P T A I P<br>1 ATGTCTGCAAATAATTCCCCTCCATCAGCCCAGAAGTCTGTATTACCCACAGCTATTCCT<br><br>21 A V L P A A S P C S S P K T G L S A R L<br>61 GCTGTGCTTCCAGCTGCTTCTCCGTGTTCAAGTCCTAAGACGGGACTCTCTGCCCGACTC<br><br>41 S N G S F S A P S L T N S R G S V H T V<br>121 TCTAATGGAAGCTTCAGTGCACCATCACTCACCAACTCCAGAGGCTCAGTGCATACAGTT<br><br>61 S F L L Q I G L T R E S V T I E A Q E L<br>181 TCATTTCTACTGCAAATTGGCCTCACACGGGAGAGTGTTACCATTGAAGCCCAGGAACTG<br><br>81 S L S A V K D L V C S I V Y Q K F P E C<br>241 TCTTTATCTGCTGTCAAGGATCTTGTGTGCTCCATAGTTTATCAAAAGTTTCCAGAGTGT<br><br>101 G F F G M Y D K I L L F R H D M N S E N<br>301 GGATTCTTTGGCATGTATGACAAAATTCTTCTCTTTCGCCATGACATGAACTCAGAAAAC<br><br>121 I L Q L I T S A D E I H E G D L V E V V<br>361 ATTTTGCAGCTGATTACCTCAGCAGATGAAATACATGAAGGAGACCTAGTGGAAGTGGTT<br><br>141 L S A L A T V E D F Q I R P H T L Y V H<br>421 CTTTCAGCTTTAGCCACAGTAGAAGACTTCCAGATTCGTCCACATACTCTCTATGTACAT<br><br>161 S Y K A P T F C D Y C G E M L W G L V R<br>481 TCTTACAAAGCTCCTACTTTCTGTGATTACTGTGGTGAGATGCTGTGGGGATTGGTACGT | SEQ ID NO:301 |

| Gene Name | GenBank Homology | DNA SEQUENCE / DEDUCED AMINO ACID SEQUENCE | SEQUENCE IDENTIFIER: |
|---|---|---|---|
| | | 181 Q G L K C E G C G L N Y H K R C A F K I<br>541 CAAGGACTGAAATGTGAAGGCTGTGGATTAAATTACCATAAACGATGTGCCTTCAAGATT<br><br>201 P N N C S G V R K R R L S N V S L P G P<br>601 CCAAATAACTGTAGTGGAGTAAGAAAGAGACGTCTGTCAAATGTATCTTTACCAGGACCC<br><br>221 G L S V P R P L Q P E Y V A L P S E E S<br>661 GGCCTCTCAGTTCCAAGACCCCTACAGCCTGAATATGTAGCCCTTCCCAGTGAAGAGTCA<br><br>241 H V H Q E P S K R I P S W S G R P I W M<br>721 CATGTCCACCAGGAACCAAGTAAGAGAATTCCTTCTTGGAGTGGTCGCCCAATCTGGATG<br><br>261 E K M V M C R V K V P H T F A V H S Y T<br>781 GAAAAGATGGTAATGTGCAGAGTGAAAGTTCCACACACATTTGCTGTTCACTCTTACACC<br><br>281 R P T I C Q Y C K R L L K G L F R Q G M<br>841 CGTCCCACGATATGTCAGTACTGCAAGCGGTTACTGAAAGGCCTCTTTCGCCAAGGAATG<br><br>301 Q C K D C K F N C H K R C A S K V P R D<br>901 CAGTGTAAAGATTGCAAATTCAACTGCCATAAACGCTGTGCATCAAAAGTACCAAGAGAC<br><br>321 C L G E V T F N G E P S S L G T D T D I<br>961 TGCCTTGGAGAGGTTACTTTCAATGGAGAACCTTCCAGTCTGGGAACAGATACAGATATA<br><br>341 P M D I D N N D I N S D S S R G L D D T<br>1021 CCAATGGATATTGACAATAATGACATAAATAGTGATAGTAGTCGGGGTTTGGATGACACA<br><br>361 E E P S P P E D K M F F L D P S D L D V<br>1081 GAAGAGCCATCACCCCCAGAAGATAAGATGTTCTTCTTGGATCCATCTGATCTCGATGTG<br><br>381 E R D E E A V K T I S P S T S N N I P L<br>1141 GAAAGAGATGAAGAAGCCGTTAAAACAATCAGTCCATCAACAAGCAATAATATTCCGCTA<br><br>401 M R V V Q S I K H T K R K S S T M V K E<br>1201 ATGAGGGTTGTACAATCCATCAAGCACACAAAGAGGAAGAGCAGCACAATGGTGAAGGAA<br><br>421 G W M V H Y T S R D N L R K R H Y W R L<br>1261 GGGTGGATGGTCCATTACACCAGCAGGGATAACCTGAGAAAGAGGCATTATTGGAGACTT<br><br>441 D S K C L T L F Q N E S G S K Y Y K E I<br>1321 GACAGCAAATGTCTAACATTATTTCAGAATGAATCTGGATCAAAGTATTATAAGGAAATT<br><br>461 P L S E I L R I S S P R D F T N I S Q G | |

| Gene Name | GenBank Homology | DNA SEQUENCE / DEDUCED AMINO ACID SEQUENCE | SEQUENCE IDENTIFIER: |
|---|---|---|---|
| | | 1381 CCACTTTCAGAAATTCTCCGCATATCTTCACCACGAGATTTCACAAACATTTCACAAGGC<br><br>481 S N P H C F E I I T D T M V Y F V G E N<br>1441 AGCAATCCACACTGTTTTGAAATCATTACTGATACTATGGTATACTTCGTTGGTGAGAAC<br><br>501 N G D S S H N P V L A A T G V G L D V A<br>1501 AATGGGGACAGCTCTCATAATCCTGTTCTTGCTGCCACTGGAGTTGGACTTGATGTAGCA<br><br>521 Q S W E K A I R Q A L M P V T P Q A S V<br>1561 CAGAGCTGGGAAAAAGCAATTCGCCAAGCCCTCATGCCTGTTACTCCTCAAGCAAGTGTT<br><br>541 C T S P G Q G K D H K D L S T S I S V S<br>1621 TGCACTTCTCCAGGGCAAGGGAAAGATCACAAAGATTTGTCTACAAGTATCTCTGTATCT<br><br>561 N C Q I Q E N V D I S T V Y Q I F A D E<br>1681 AATTGTCAGATTCAGGAGAATGTGGATATCAGTACTGTTTACCAGATCTTTGCAGATGAG<br><br>581 V L G S G Q F G I V Y G G K H R K T G R<br>1741 GTGCTTGGTTCAGGCCAGTTTGGCATCGTTTATGGAGGAAAACATAGAAAGACTGGGAGG<br><br>601 D V A I K V I D K M R F P T K Q E S Q L<br>1801 GATGTGGCTATTAAAGTAATTGATAAGATGAGATTCCCCACAAAACAAGAAAGTCAACTC<br><br>621 R N E V A I L Q N L H H P G I V N L E C<br>1861 CGTAATGAAGTGGCTATTTTACAGAATTTGCACCATCCTGGGATTGTAAACCTGGAATGT<br><br>641 M F E T P E R V F V V M E K L H G D M L<br>1921 ATGTTTGAAACCCCAGAACGAGTCTTTGTAGTAATGGAAAAGCTGCATGGAGATATGTTG<br><br>661 E M I L S S E K S R L P E R I T K F M V<br>1981 GAAATGATTCTATCCAGTGAGAAAAGTCGGCTTCCAGAACGAATTACTAAATTCATGGTC<br><br>681 T Q I L V A L R N L H F K N I V H C D L<br>2041 ACACAGATACTTGTTGCTTTGAGGAATCTGCATTTTAAGAATATTGTGCACTGTGATTTA<br><br>701 K P E N V L L A S A E P F P Q V K L C D<br>2101 AAGCCAGAAAATGTGCTGCTTGCATCAGCAGAGCCATTTCCTCAGGTGAAGCTGTGTGAC<br><br>721 F G F A R I I G E K S F R R S V V G T P<br>2161 TTTGGATTTGCACGCATCATTGGTGAAAAGTCATTCAGGAGATCTGTGGTAGGAACTCCA<br><br>741 A Y L A P E V L R S K G Y N R S L D M W<br>2221 GCATACTTAGCCCCTGAAGTTCTCCGGAGCAAAGGTTACAACCGTTCCCTAGATATGTGG | |

| Gene Name | GenBank Homology | DNA SEQUENCE / DEDUCED AMINO ACID SEQUENCE | SEQUENCE IDENTIFIER: |
|---|---|---|---|
| | | 761 S V G V I I Y V S L S G T F P F N E D E<br>2281 TCAGTGGGAGTTATCATCTATGTGAGCCTCAGTGGCACATTTCCTTTTAATGAGGATGAA<br><br>781 D I N D Q I Q N A A F M Y P P N P W R E<br>2341 GATATAAATGACCAAATCCAAAATGCTGCATTTATGTACCCACCAAATCCATGGAGAGAA<br><br>801 I S G E A I D L I N N L L Q V K M R K R<br>2401 ATTTCTGGTGAAGCAATTGATCTGATAAACAATCTGCTTCAAGTGAAGATGAGAAAACGT<br><br>821 Y S V D K S L S H P W L Q D Y Q T W L D<br>2461 TACAGTGTTGACAAATCTCTTAGTCATCCCTGGCTACAGGACTATCAGACTTGGCTTGAC<br><br>841 L R E F E T R I G E R Y I T H E S D D A<br>2521 CTTAGAGAATTTGAAACTCGCATTGGAGAACGTTACATTACACATGAAAGTGATGATGCT<br><br>861 R W E I H A Y T H N L V Y P K H F I M A<br>2581 CGCTGGGAAATACATGCATACACACATAACCTTGTATACCCAAAGCACTTCATTATGGCT<br><br>881 P N P D D M E E D P -<br>2641 CCTAATCCAGATGATATGGAAGAAGATCCTTAA | |
| BM781379 | Homo sapiens inhibitor of Bruton agammaglobulinemia tyrosine kinase (IBTK), mRNA. | 1 attcctgtag tagcggctgt attgcagccg cctgccgaac tgacccgggt ctggggactg<br>61 gcccctctgg cgccgttcgg tttctcttat tgccttcact gaggatgagt ccctttgtgg<br>121 ctctatgtgg accctgcgga atccaccggc gcagtttcat ctagcgactg gtcacccttg<br>181 caattatgga tatttaaaag ggtcagacag tgtggagggg gagttcccct cctcactccc<br>241 ccttggtgct tgactccagg aataatttat aaactgtgga attttttaa atgaagaact<br>301 tgtatttgat atgaacttta tagagctatt tataattttt ttgatttaag tgccaaaaaa<br>361 ttgtataaag atatatagtt ttatactatt gtcaggagga tttaaattat cctaaaaagg<br>421 taatttattc tctgtaactt cctcaatagc acctttgtgt cctggctttt tcatttttta<br>481 aaattagttt tcacgattct gaagtaagtg gtataaaaac agttaggatg agttcaccca<br>541 tgcctgactg cacatcaaag tgtcgatccc tgaagcatgc tttggatgtc ctttctgtgg<br>601 taacaaaggg gagcgaaaac cagattaagg cctttctctc cagtcattgt tacaatgctg<br>661 caactatcaa ggatgttttt ggcaggaatg ccctccacct tgtttcctcc tgtggaaaga<br>721 aaggagtgtt agattggctt attcagaaag gagtggatct gttggtgaaa gacaaagagt<br>781 ctggatggac agcattgcac agaagcattt tttatggaca tattgattgt gtttggtctc<br>841 tattgaagca tggtgttagt ctgtatattc aagataaaga aggcttgtca gctttggatc<br>901 ttgtaatgaa ggatagacca actcatgtag tattcaagaa tactgatcct acagatgttt<br>961 atacttgggg cgataataca aattttaccc tgggtcatgg aagccagaat agcaaacatc<br>1021 atccagagtt ggtggatctg ttctccagga gtgggattta tatcaagcag gtggtgcttt<br>1081 gtaaatttca ctccgtgttt ctgtctcaga aagggcaggt ttatacctgt ggtcatggtc<br>1141 ctggagggcg attaggacat ggagatgaac agacatgctt ggtccctcgg cttgtggaag | SEQ ID NO:302 |

| Gene Name | GenBank Homology | DNA SEQUENCE / DEDUCED AMINO ACID SEQUENCE | SEQUENCE IDENTIFIER: |
|---|---|---|---|
| | | 1201 gactgaatgg tcataattgt tcccaagtgg cagctgctaa ggatcatact gttgtattaa<br>1261 ctgaagatgg atgtgtttat acatttggtc taaacatttt tcatcaatta ggaattattc<br>1321 caccgccttc cagttgtaat gtacccagac agatacaggc aaaatatctg aaaggaagga<br>1381 caatcattgg cgttgcagca ggcaggtttc atacagtcct atggactaga gaagctgttt<br>1441 acactatggg actaaatggt ggacaactgg gttgtttgct agatcccaat ggagaaaagt<br>1501 gtgtaactgc tcctcgtcag gtctctgccc ttcaccataa agacattgct ctgtctttgg<br>1561 ttgctgcaag tgatggagct acagtctgtg ttaccacaag gggagatatt tacttacttg<br>1621 cagactatca gtgcaagaag atggcttcta aacagttgaa cttgaaaaaa gttcttgtgt<br>1681 ctgggggtca tatggaatac aaggttgatc ctgaacattt gaaagaaaat gggggtcaaa<br>1741 aaatttgcat tcttgcaatg gatggagctg gaagggtgtt ttgctggaga tcagtcaaca<br>1801 gttctctgaa gcagtgtcga tgggcctatc cacgtcaggt cttcatttct gatattgctt<br>1861 taaatagaaa tgaaattcta tttgttacgc aagatggaga aggatttaga gggagatggt<br>1921 ttgaagagaa aagaaagagt tctgaaaaga aagagatttt atcaaacctt cacaattcct<br>1981 catcagatgt gtcttatgtc tctgatataa atagtgtgta tgaaagaatt cgacttgaga<br>2041 aacttacctt tgcacataga gctgttagtg tcagcacaga tccaagtgga tgcaactttg<br>2101 caatcctgca gtcagatcct aaaacaagcc tttatgaaat tccagctgtg tcctcatcat<br>2161 cctttttga agagtttggc aaactgttga gggaagcaga tgaaatggac agcattcatg<br>2221 atgtgacatt tcaagttggc aatagactct tccctgcaca taaatatatt ttggcagtgc<br>2281 attctgattt ttttcagaaa ttgtttcttt cagatggtaa tacttcagaa tttacagata<br>2341 tttaccagaa agatgaagat tctgcagggt gccatctctt tgtggtagag aaggttcatc<br>2401 ctgacatgtt tgaatacctt ttacaattta tatacacaga tacttgtgac tttttaactc<br>2461 atggcttcaa accaagaata cacttaaaca aaaacccaga agaatatcag ggaactctga<br>2521 attctcattt gaataaagtg aatttccatg aagatgataa ccagaagtct gcatttgaag<br>2581 tttacaaaag taatcaagct caaacagtta gtgagaggca gaagagcaaa cctaaatctt<br>2641 gtaaaaaagg aaaaaatatt agggaagatg atcctgtaag aatgttgcaa actgttgcaa<br>2701 agaaattcga cttcagtaat ttgagtagta ggttagatgg agtcagattt gaaaatgaaa<br>2761 aaattaatgt tattgccaag aacactggta ataaactgaa gctaagtcag aaaaaatgtt<br>2821 catttctgtg tgacgtgacc atgaaatcag tggatggaaa ggaatttcct tgtcataaat<br>2881 gtgttctttg tgctagactt gaatattttc atagtatgct gagtagctca tggattgagg<br>2941 cttccagttg tgcagctctg gaaatgccaa tacattctga tatattaaaa gttattttgg<br>3001 actacctcta tactgatgaa gctgtggtga taaaagaatc tcaaaatgta gattttattt<br>3061 gtagtgttct tgtggtggct gatcaacttc tcataacccg gttgaaagag atttgtgaag<br>3121 tagcattaac tgaaaaactt accctgaaga atgctgctat gctactggaa tttgcagcaa<br>3181 tgtatagtgc aaaacagttg aaactgtctt gtttacagtt tataggattg aatatggcag<br>3241 ctttacttga agcaaggtct cttgatgttt taagcgatgg tgttttgaag gatctttctg<br>3301 agttttaccg gaaaatgatt ccagcaatgg atagaagagt cattacacca tatcaagatg<br>3361 gaccagatat tagctatttg gaagtagaag atggagatat cttcttgaaa gaagaaataa<br>3421 atatggaaca aaatcattcg gaaactatgt tcaagaaagc aaaaacaaaa gctaaaaaga<br>3481 agccacgtaa acgttcagat agttctggag gttataacct ttcagatatt attcagagtc<br>3541 catcatctac aggattatta aagtctggta agaccaattc tgtggaatct cttccagaac<br>3601 tgttgacatc agactctgaa ggaagctatg caggagtggg tagtcctaga gatttacagt<br>3661 cccctgattt cacaacagga tttcattcag ataagattga ggcgaaagtc aaaccgtatg<br>3721 ttaatggtac atctcctgtg tattctaggg aagatttaaa accatgggaa aagtcaccaa | |

| Gene Name | GenBank Homology | DNA SEQUENCE / DEDUCED AMINO ACID SEQUENCE | SEQUENCE IDENTIFIER: |
|---|---|---|---|
| | | 3781 tacttaaaat atctgctcca cagcctattc ccagtaacag aattgatact accagctctg<br>3841 ccagttgggt tgctggttct ttcagtcctg tcagccctcc tgttgtggat ctcagaacta<br>3901 tcatggaaat agaagaaagt agacaaaaat gtggagctac accaaagtca catttaggca<br>3961 aaacagtttc tcatggagtt aaactttctc agaagcaacg aaaaatgatt gcattgacta<br>4021 ccaaggaaaa caattcagga atgaatagca tggaaacagt tttattcact ccttcaaaag<br>4081 cccccaaacc agtgaatgca tgggcatctt ctctgcattc agtttcatcc aagtcattcc<br>4141 gggatttctt actagaagaa aaaaagtctg ttactagcca tagttcaggc gatcatgtca<br>4201 aaaaagtttc ttttaaagga attgaaaatt ctcaggcacc aaaaattgtc agatgctcta<br>4261 cccatggtac cccaggacca gaaggcaacc atatttcaga tttaccactt ctagacagtc<br>4321 ccaatccctg gctatcttct tcagtgactg ctccatccat ggtagcccca gtcacttttg<br>4381 catctattgt agaagaagaa ctacaacaag aagcagctct tattagaagt cgagaaaaac<br>4441 cgttggctct gattcagatt gaggagcatg ccatacaaga tttattggtt ttctatgagg<br>4501 catttggcaa ccctgaagag tttgtcattg ttgaaaggac accgcaggga ccactggcag<br>4561 tacctatgtg gaataagcat ggatgctagt tcactgtgga gttgagatgc attttacata<br>4621 attatgagtt tgttcatata aagaaaagct gtggaaaaga gtcttagaga ttttgtaata<br>4681 tcattctaaa tagattaaga aaagatataa tttctttact gcagttaaat catataatgt<br>4741 ttgtatgatt aaaaataaat ttctcagaat tgtgatttta gtaactttat ataaaatgtg<br>4801 tgagacaaaa acttattaag gttaaataga attgtttctt ctgaataatc taacaaagga<br>4861 aaatataagt gattgaatca taagatataa ggggggtaaa gtattaaaaa taactttttt<br>4921 gtttgataac ttgagaattt agaagatttt gccaagtatg tgttgttgct tgacttctta<br>4981 aatatggcat tgatgaattt aaagtaggag catcagttat tacttctgat tcattaatgg<br>5041 ccagaatttt gtgtttggtg taatagttgt gtcaccattc ttgttgcttt ttaaaaatca<br>5101 ggctaatcat gtggtccatg tctcttcaaa gcttgacctg cacaaatgcc atatttctat<br>5161 ttggaccaca tattctccat tttgcattga gcagtagagt acagtggaaa gggaataaga<br>5221 atactgatta ttctgaacag tttagtccca agagaatagc gttttaaaaa agaaaaacaa<br>5281 gatttggagt cattgtgggt tatttttggt gggatggagg atcttaaaaa tgcctaattg<br>5341 tgagagaatc aattgctgaa agtgttaaaa tttctgaaaa taaatgctta attacatata<br>5401 caggaattaa atagtttgga agagggttgg attatcatta cctttacaat actgtataat<br>5461 cagaagttct ctgaacctca attgtatatc tagacataaa aattgttttc tgtataggat<br>5521 gttgtttggt ttgtttctga gtgtttaaat tttgcaaaaa caaatgttaa atttgtgctt<br>5581 cagtacctag ataaattgga aaggttaatg ttctagtttc tggaaggtaa gcctgggaga<br>5641 cacataagca attcactgct ataatttagt tgatgtaaaa tgacggaaac tgactcaata<br>5701 tgtcaggttt aactctgccc aaaagcagca gacatgtaag cagatgtgca ataaaaaatg<br>5761 atcttgatcc atttc | |
| | | 1 M S S P M P D C T S K C R S L K H A L D<br>1 ATGAGTTCACCCATGCCTGACTGCACATCAAAGTGTCGATCCCTGAAGCATGCTTTGGAT<br><br>21 V L S V V T K G S E N Q I K A F L S S H<br>61 GTCCTTTCTGTGGTAACAAAGGGGAGCGAAAACCAGATTAAGGCCTTTCTCTCCAGTCAT<br><br>41 C Y N A A T I K D V F G R N A L H L V S<br>121 TGTTACAATGCTGCAACTATCAAGGATGTTTTTGGCAGGAATGCCCTCCACCTTGTTTCC | SEQ ID NO:303 |

| Gene Name | GenBank Homology | DNA SEQUENCE / DEDUCED AMINO ACID SEQUENCE | SEQUENCE IDENTIFIER: |
|---|---|---|---|
| | | 61 S C G K K G V L D W L I Q K G V D L L V<br>181 TCCTGTGGAAAGAAAGGAGTGTTAGATTGGCTTATTCAGAAAGGAGTGGATCTGTTGGTG<br><br>81 K D K E S G W T A L H R S I F Y G H I D<br>241 AAAGACAAAGAGTCTGGATGGACAGCATTGCACAGAAGCATTTTTTATGGACATATTGAT<br><br>101 C V W S L L K H G V S L Y I Q D K E G L<br>301 TGTGTTTGGTCTCTATTGAAGCATGGTGTTAGTCTGTATATTCAAGATAAAGAAGGCTTG<br><br>121 S A L D L V M K D R P T H V V F K N T D<br>361 TCAGCTTTGGATCTTGTAATGAAGGATAGACCAACTCATGTAGTATTCAAGAATACTGAT<br><br>141 P T D V Y T W G D N T N F T L G H G S Q<br>421 CCTACAGATGTTTATACTTGGGGCGATAATACAAATTTTACCCTGGGTCATGGAAGCCAG<br><br>161 N S K H H P E L V D L F S R S G I Y I K<br>481 AATAGCAAACATCATCCAGAGTTGGTGGATCTGTTCTCCAGGAGTGGGATTTATATCAAG<br><br>181 Q V V L C K F H S V F L S Q K G Q V Y T<br>541 CAGGTGGTGCTTTGTAAATTTCACTCCGTGTTTCTGTCTCAGAAAGGGCAGGTTTATACC<br><br>201 C G H G P G G R L G H G D E Q T C L V P<br>601 TGTGGTCATGGTCCTGGAGGGCGATTAGGACATGGAGATGAACAGACATGCTTGGTCCCT<br><br>221 R L V E G L N G H N C S Q V A A A K D H<br>661 CGGCTTGTGGAAGGACTGAATGGTCATAATTGTTCCCAAGTGGCAGCTGCTAAGGATCAT<br><br>241 T V V L T E D G C V Y T F G L N I F H Q<br>721 ACTGTTGTATTAACTGAAGATGGATGTGTTTATACATTTGGTCTAAACATTTTTCATCAA<br><br>261 L G I I P P P S S C N V P R Q I Q A K Y<br>781 TTAGGAATTATTCCACCGCCTTCCAGTTGTAATGTACCCAGACAGATACAGGCAAAATAT<br><br>281 L K G R T I I G V A A G R F H T V L W T<br>841 CTGAAAGGAAGGACAATCATTGGCGTTGCAGCAGGCAGGTTTCATACAGTCCTATGGACT<br><br>301 R E A V Y T M G L N G G Q L G C L L D P<br>901 AGAGAAGCTGTTTACACTATGGGACTAAATGGTGGACAACTGGGTTGTTTGCTAGATCCC<br><br>321 N G E K C V T A P R Q V S A L H H K D I<br>961 AATGGAGAAAAGTGTGTAACTGCTCCTCGTCAGGTCTCTGCCCTTCACCATAAAGACATT | |

| Gene Name | GenBank Homology | DNA SEQUENCE / DEDUCED AMINO ACID SEQUENCE | SEQUENCE IDENTIFIER: |
|---|---|---|---|
| | | 341 A L S L V A A S D G A T V C V T T R G D<br>1021 GCTCTGTCTTTGGTTGCTGCAAGTGATGGAGCTACAGTCTGTGTTACCACAAGGGGAGAT<br><br>361 I Y L L A D Y Q C K K M A S K Q L N L K<br>1081 ATTTACTTACTTGCAGACTATCAGTGCAAGAAGATGGCTTCTAAACAGTTGAACTTGAAA<br><br>381 K V L V S G G H M E Y K V D P E H L K E<br>1141 AAAGTTCTTGTGTCTGGGGGTCATATGGAATACAAGGTTGATCCTGAACATTTGAAAGAA<br><br>401 N G G Q K I C I L A M D G A G R V F C W<br>1201 AATGGGGGTCAAAAAATTTGCATTCTTGCAATGGATGGAGCTGGAAGGGTGTTTTGCTGG<br><br>421 R S V N S S L K Q C R W A Y P R Q V F I<br>1261 AGATCAGTCAACAGTTCTCTGAAGCAGTGTCGATGGGCCTATCCACGTCAGGTCTTCATT<br><br>441 S D I A L N R N E I L F V T Q D G E G F<br>1321 TCTGATATTGCTTTAAATAGAAATGAAATTCTATTTGTTACGCAAGATGGAGAAGGATTT<br><br>461 R G R W F E E K R K S S E K K E I L S N<br>1381 AGAGGGAGATGGTTTGAAGAGAAAAGAAAGAGTTCTGAAAAGAAAGAGATTTTATCAAAC<br><br>481 L H N S S S D V S Y V S D I N S V Y E R<br>1441 CTTCACAATTCCTCATCAGATGTGTCTTATGTCTCTGATATAAATAGTGTGTATGAAAGA<br><br>501 I R L E K L T F A H R A V S V S T D P S<br>1501 ATTCGACTTGAGAAACTTACCTTTGCACATAGAGCTGTTAGTGTCAGCACAGATCCAAGT<br><br>521 G C N F A I L Q S D P K T S L Y E I P A<br>1561 GGATGCAACTTTGCAATCCTGCAGTCAGATCCTAAAACAAGCCTTTATGAAATTCCAGCT<br><br>541 V S S S S F F E E F G K L L R E A D E M<br>1621 GTGTCCTCATCATCCTTTTTTGAAGAGTTTGGCAAACTGTTGAGGGAAGCAGATGAAATG<br><br>561 D S I H D V T F Q V G N R L F P A H K Y<br>1681 GACAGCATTCATGATGTGACATTTCAAGTTGGCAATAGACTCTTCCCTGCACATAAATAT<br><br>581 I L A V H S D F F Q K L F L S D G N T S<br>1741 ATTTTGGCAGTGCATTCTGATTTTTTTCAGAAATTGTTTCTTTCAGATGGTAATACTTCA<br><br>601 E F T D I Y Q K D E D S A G C H L F V V<br>1801 GAATTTACAGATATTTACCAGAAAGATGAAGATTCTGCAGGGTGCCATCTCTTTGTGGTA<br><br>621 E K V H P D M F E Y L L Q F I Y T D T C | |

| Gene Name | GenBank Homology | DNA SEQUENCE / DEDUCED AMINO ACID SEQUENCE | SEQUENCE IDENTIFIER: |
|---|---|---|---|
| | | 1861 GAGAAGGTTCATCCTGACATGTTTGAATACCTTTTACAATTTATATACACAGATACTTGT<br><br>641 D F L T H G F K P R I H L N K N P E E Y<br>1921 GACTTTTTAACTCATGGCTTCAAACCAAGAATACACTTAAACAAAAACCCAGAAGAATAT<br><br>661 Q G T L N S H L N K V N F H E D D N Q K<br>1981 CAGGGAACTCTGAATTCTCATTTGAATAAAGTGAATTTCCATGAAGATGATAACCAGAAG<br><br>681 S A F E V Y K S N Q A Q T V S E R Q K S<br>2041 TCTGCATTTGAAGTTTACAAAAGTAATCAAGCTCAAACAGTTAGTGAGAGGCAGAAGAGC<br><br>701 K P K S C K K G K N I R E D D P V R M L<br>2101 AAACCTAAATCTTGTAAAAAAGGAAAAAATATTAGGGAAGATGATCCTGTAAGAATGTTG<br><br>721 Q T V A K K F D F S N L S S R L D G V R<br>2161 CAAACTGTTGCAAAGAAATTCGACTTCAGTAATTTGAGTAGTAGGTTAGATGGAGTCAGA<br><br>741 F E N E K I N V I A K N T G N K L K L S<br>2221 TTTGAAAATGAAAAAATTAATGTTATTGCCAAGAACACTGGTAATAAACTGAAGCTAAGT<br><br>761 Q K K C S F L C D V T M K S V D G K E F<br>2281 CAGAAAAAATGTTCATTTCTGTGTGACGTGACCATGAAATCAGTGGATGGAAAGGAATTT<br><br>781 P C H K C V L C A R L E Y F H S M L S S<br>2341 CCTTGTCATAAATGTGTTCTTTGTGCTAGACTTGAATATTTTCATAGTATGCTGAGTAGC<br><br>801 S W I E A S S C A A L E M P I H S D I L<br>2401 TCATGGATTGAGGCTTCCAGTTGTGCAGCTCTGGAAATGCCAATACATTCTGATATATTA<br><br>821 K V I L D Y L Y T D E A V V I K E S Q N<br>2461 AAAGTTATTTTGGACTACCTCTATACTGATGAAGCTGTGGTGATAAAAGAATCTCAAAAT<br><br>841 V D F I C S V L V V A D Q L L I T R L K<br>2521 GTAGATTTTATTTGTAGTGTTCTTGTGGTGGCTGATCAACTTCTCATAACCCGGTTGAAA<br><br>861 E I C E V A L T E K L T L K N A A M L L<br>2581 GAGATTTGTGAAGTAGCATTAACTGAAAAACTTACCCTGAAGAATGCTGCTATGCTACTG<br><br>881 E F A A M Y S A K Q L K L S C L Q F I G<br>2641 GAATTTGCAGCAATGTATAGTGCAAAACAGTTGAAACTGTCTTGTTTACAGTTTATAGGA<br><br>901 L N M A A L L E A R S L D V L S D G V L<br>2701 TTGAATATGGCAGCTTTACTTGAAGCAAGGTCTCTTGATGTTTTAAGCGATGGTGTTTTG | |

| Gene Name | GenBank Homology | DNA SEQUENCE / DEDUCED AMINO ACID SEQUENCE | SEQUENCE IDENTIFIER: |
|---|---|---|---|
| | | 921 K D L S E F Y R K M I P A M D R R V I T<br>2761 AAGGATCTTTCTGAGTTTTACCGGAAAATGATTCCAGCAATGGATAGAAGAGTCATTACA<br><br>941 P Y Q D G P D I S Y L E V E D G D I F L<br>2821 CCATATCAAGATGGACCAGATATTAGCTATTTGGAAGTAGAAGATGGAGATATCTTCTTG<br><br>961 K E E I N M E Q N H S E T M F K K A K T<br>2881 AAAGAAGAAATAAATATGGAACAAAATCATTCGGAAACTATGTTCAAGAAAGCAAAAACA<br><br>981 K A K K K P R K R S D S S G G Y N L S D<br>2941 AAAGCTAAAAAGAAGCCACGTAAACGTTCAGATAGTTCTGGAGGTTATAACCTTTCAGAT<br><br>1001 I I Q S P S S T G L L K S G K T N S V E<br>3001 ATTATTCAGAGTCCATCATCTACAGGATTATTAAAGTCTGGTAAGACCAATTCTGTGGAA<br><br>1021 S L P E L L T S D S E G S Y A G V G S P<br>3061 TCTCTTCCAGAACTGTTGACATCAGACTCTGAAGGAAGCTATGCAGGAGTGGGTAGTCCT<br><br>1041 R D L Q S P D F T T G F H S D K I E A K<br>3121 AGAGATTTACAGTCCCCTGATTTCACAACAGGATTTCATTCAGATAAGATTGAGGCGAAA<br><br>1061 V K P Y V N G T S P V Y S R E D L K P W<br>3181 GTCAAACCGTATGTTAATGGTACATCTCCTGTGTATTCTAGGGAAGATTTAAAACCATGG<br><br>1081 E K S P I L K I S A P Q P I P S N R I D<br>3241 GAAAAGTCACCAATACTTAAAATATCTGCTCCACAGCCTATTCCCAGTAACAGAATTGAT<br><br>1101 T T S S A S W V A G S F S P V S P P V V<br>3301 ACTACCAGCTCTGCCAGTTGGGTTGCTGGTTCTTTCAGTCCTGTCAGCCCTCCTGTTGTG<br><br>1121 D L R T I M E I E E S R Q K C G A T P K<br>3361 GATCTCAGAACTATCATGGAAATAGAAGAAAGTAGACAAAAATGTGGAGCTACACCAAAG<br><br>1141 S H L G K T V S H G V K L S Q K Q R K M<br>3421 TCACATTTAGGCAAAACAGTTTCTCATGGAGTTAAACTTTCTCAGAAGCAACGAAAAATG<br><br>1161 I A L T T K E N N S G M N S M E T V L F<br>3481 ATTGCATTGACTACCAAGGAAAACAATTCAGGAATGAATAGCATGGAAACAGTTTTATTC<br><br>1181 T P S K A P K P V N A W A S S L H S V S<br>3541 ACTCCTTCAAAAGCCCCCAAACCAGTGAATGCATGGGCATCTTCTCTGCATTCAGTTTCA | |

| Gene Name | GenBank Homology | DNA SEQUENCE / DEDUCED AMINO ACID SEQUENCE | SEQUENCE IDENTIFIER: |
|---|---|---|---|
| | | 1201 S K S F R D F L L E E K K S V T S H S S<br>3601 TCCAAGTCATTCCGGGATTTCTTACTAGAAGAAAAAAAGTCTGTTACTAGCCATAGTTCA<br><br>1221 G D H V K K V S F K G I E N S Q A P K I<br>3661 GGCGATCATGTCAAAAAAGTTTCTTTTAAAGGAATTGAAAATTCTCAGGCACCAAAAATT<br><br>1241 V R C S T H G T P G P E G N H I S D L P<br>3721 GTCAGATGCTCTACCCATGGTACCCCAGGACCAGAAGGCAACCATATTTCAGATTTACCA<br><br>1261 L L D S P N P W L S S S V T A P S M V A<br>3781 CTTCTAGACAGTCCCAATCCCTGGCTATCTTCTTCAGTGACTGCTCCATCCATGGTAGCC<br><br>1281 P V T F A S I V E E E L Q Q E A A L I R<br>3841 CCAGTCACTTTTGCATCTATTGTAGAAGAAGAACTACAACAAGAAGCAGCTCTTATTAGA<br><br>1301 S R E K P L A L I Q I E E H A I Q D L L<br>3901 AGTCGAGAAAAACCGTTGGCTCTGATTCAGATTGAGGAGCATGCCATACAAGATTTATTG<br><br>1321 V F Y E A F G N P E E F V I V E R T P Q<br>3961 GTTTTCTATGAGGCATTTGGCAACCCTGAAGAGTTTGTCATTGTTGAAAGGACACCGCAG<br><br>1341 G P L A V P M W N K H G C -<br>4021 GGACCACTGGCAGTACCTATGTGGAATAAGCATGGATGCTAG | |
| BM781394 | No Homology | 1 GGCACGAGGGCCCCTACAAATGCAAAACCCAGGTTTCTAACTGTTCAAGATATAGTCAAG<br>61 AGTTCAGCTTCACGTTTGCCAACCCAGTGACCACACCAGTGCTGAACATTAGTGCCTTTC<br>121 AAACAGAAACAGCCCGATACATAACACTCCGCTGCATCTCATACAATGGCTCTCTGCCCA<br>181 TCAATTACACTTTCTTTGAAAAAGACGTCGCCATATCACCTGCTATTTCCAAGAATGTAA<br>241 GGGAACCTGCTGAATTTAACTTAACCGAGAGGATCACCGTGACAAAGGGAAGAATATAGG<br>301 TGTAAAGCCAAAAACAGATTGCCCAACCATGCAAAATACAGTCAACCCTTCACCATGCCC<br>361 TCAACAGGCGGAGACAGCTGTCCTTTCTGCCTGCAGCTACTGCTTCCGGGGTTATTACTG<br>421 CTGCTGATAGTAATAATCCTAATGCTGGCATTTTGGATACTACCAAAGTACAAAGCAAGA<br>481 AAAGCTATGAGAGATAATGCACCCAGAAACTATGGAAATAAACCCATGGAAGTTGGAATA<br>541 TATGCAAATGTCTGTCAAAACCAAGCAGATGACAAATCTGTGCCAGGCTTGGAACCAAGG<br>601 CAGTGTGTTTCCACAGCCCAA | SEQ ID NO:304 |
| BM781409 | No homology | 1 GGCACGAGGAACTTTCATATATTGTGCCAGGTGCTAGGGATACAGTGATAAACAAAATTA<br>61 TCATGGTTCCTGCTCCTGGAGATCTTATGGTATAGTAGGATAAACAGAATTAAAAAAATA<br>121 AAAATAAATATTTAATGACAAATGACATGGAGGTTGTGAAAGAATAATAAGGGGATCCAT<br>181 TTTAGATGGAATGGTCTGAGAAGGTCTGTCTTCGAAGGTGACATATGTAAGCTGAGACCT | SEQ ID NO:305 |

| Gene Name | GenBank Homology | DNA SEQUENCE / DEDUCED AMINO ACID SEQUENCE | SEQUENCE IDENTIFIER: |
|---|---|---|---|
| | | 241 AAAGAAAAGGAGGAACCAGGCAGGCAGAGGAAAGGCCTTGAGGCAGATGTAAAAACTGTG<br>301 ACATAGAGAACTCTTTCCTCTAACAGAGGAGCTGAAAGAAGCTCAGGGAGGCTAAATAGA<br>361 GATAGTGCGGGGAGAGTGATGATAGCTGAGGCTTGAGATGGGTCAGACCATCCAGGGCCA<br>421 GGGAGACTCAGTTTCATTCTGAGTGCACTGGGAAGTCTTGCAGCGTTTTAAGCAGACAAG<br>481 CGAAGGATCTGATTTATATTAAAAATATTACTGTTGTAATATTGTCTTGTCTTGTGGAGA<br>541 ATGGATTGGAAGGAAAAACGAGCAACAGTTGCAGAGAGTTGTTCAGAATTTGTTGTAGTA<br>601 CTCCAGGTCAGCTCGTGC | |
| BM781416 | No homology | 1 TTTAATCGTTAATTGTTTTTTCCCTTGTCCGTTTTTATTTGGCATCCACCACAGACAAT<br>61 TTAAATTTCGTAGATGCGCTCAGAACGCAGCAGCAGGGTACGTAGCAGAAATGCAAAGGT<br>121 GGACAGGTAGTACAATTACTGGCTTTTCTGCTGTAAATAGAATGAAGGAAAATGACTCAA<br>181 ATCAAGTAAAACTAACACACATGATTTGATTGACAATAAAGTATTTGCCATCACATGCTG<br>241 CAGCTGTTTGTCAGGAACATGGTGTCATTCATTCATTCAGTAATCACGCTGCAGAAATTG<br>301 GCAGCCTGCACCTTATGCGTCACCATTACCTTTAGTCGTTTTTTTGTATTAATTGTAGCC<br>361 AACTCGTGC | SEQ ID NO:306 |
| BM781438 | Homo sapiens cysteine-rich motor neuron 1 (CRIM1) | 1 ggcccggctg cgaggaggag gcggcggcgg cgcaggagga tgtacttggt ggcgggggac<br>61 aggggggttgg ccggctgcgg gcacctcctg gtctcgctgc tggggctgct gctgctgctg<br>121 gcgcgctccg gcacccgggc gctggtctgc ctgccctgtg acgagtccaa gtgcgaggag<br>181 cccaggaact gcccggggag catcgtgcag ggcgtctgcg gctgctgcta cacgtgcgcc<br>241 agccagagga acgagagctg cggcggcacc ttcgggattt acggaacctg cgaccggggg<br>301 ctgcgttgtg tcatccgccc cccgctcaat ggcgactccc tcaccgagta cgaagcgggc<br>361 gtttgcgaag atgagaactg gactgatgac caactgcttg gttttaaacc atgcaatgaa<br>421 aaccttattg ctggctgcaa tataatcaat gggaaatgtg aatgtaacac cattcgaacc<br>481 tgcagcaatc cctttgagtt tccaagtcag gatatgtgcc tttcagcttt aaagagaatt<br>541 gaagaagaga agccagattg ctccaaggcc cgctgtgaag tccagttctc tccacgttgt<br>601 cctgaagatt ctgttctgat cgagggttat gctcctcctg gggagtgctg tcccttaccc<br>661 agccgctgcg tgtgcaaccc cgcaggctgt ctgcgcaaag tctgccagcc gggaaacctg<br>721 aacatactag tgtcaaaagc ctcagggaag ccgggagagt gctgtgacct ctatgagtgc<br>781 aaaccagttt tcggcgtgga ctgcaggact gtggaatgcc ctcctgttca gcagaccgcg<br>841 tgtcccccgg acagctatga aactcaagtc agactaactg cagatggttg ctgtactttg<br>901 ccaacaagat gcgagtgtct ctctggctta tgtggtttcc ccgtgtgtga ggtgggatcc<br>961 actccccgca tagtctctcg tggcgatggg acacctggaa agtgctgtga tgtctttgaa<br>1021 tgtgttaatg atacaaagcc agcctgcgta tttaacaatg tggaatatta tgatggagac<br>1081 atgtttcgaa tggacaactg tcggttctgt cgatgccaag gggcgttgc catctgcttc<br>1141 actgcccagt gtggtgagat aaactgcgag aggtactacg tgcccgaagg agagtgctgc<br>1201 ccagtgtgtg aagatccagt gtatcctttt aataatcccg ctggctgcta tgccaatggc<br>1261 ctgatccttg cccacggaga ccggtggcgg gaagacgact gcacattctg ccagtgcgtc<br>1321 aacggtgaac gccactgcgt tgcgaccgtc tgcggacaga cctgcacaaa ccctgtgaaa<br>1381 gtgcctgggg agtgttgccc tgtgtgcgaa gaaccaacca tcatcacagt tgatccacct<br>1441 gcatgtgggg agttatcaaa ctgcactctg acagggaagg actgcattaa tggtttcaaa | SEQ ID NO:307 |

| Gene Name | GenBank Homology | DNA SEQUENCE / DEDUCED AMINO ACID SEQUENCE | SEQUENCE IDENTIFIER: |
|---|---|---|---|
| | | 1501 cgcgatcaca atggttgtcg gacctgtcag tgcataaaca ccgaggaact atgttcagaa<br>1561 cgtaaacaag gctgcacctt gaactgtccc ttcggtttcc ttactgatgc ccaaaactgt<br>1621 gagatctgtg agtgccgccc aaggcccaag aagtgcagac ccataatctg tgacaagtat<br>1681 tgtccacttg gattgctgaa gaataagcac ggctgtgaca tctgtcgctg taagaaatgt<br>1741 ccagagctct catgcagtaa gatctgcccc ttgggtttcc agcaggacag tcacggctgt<br>1801 cttatctgca agtgcagaga ggcctctgct tcagctgggc cacccatcct gtcgggcact<br>1861 tgtctcaccg tggatggtca tcatcataaa aatgaggaga gctggcacga tgggtgccgg<br>1921 gaatgctact gtctcaatgg acgggaaatg tgtgccctga tcacctgccc ggtgcctgcc<br>1981 tgtggcaacc ccaccattca ccctggacag tgctgcccat catgtgcaga tgactttgtg<br>2041 gtgcagaagc cagagctcag tactccctcc atttgccacg cccctggagg agaatacttt<br>2101 gtggaaggag aaacgtggaa cattgactcc tgtactcagt gcacctgcca cagcggacgg<br>2161 gtgctgtgtg agacagaggt gtgcccaccg ctgctctgcc agaacccctc acgcacccag<br>2221 gattcctgct gcccacagtg tacagatcaa ccttttcggc cttccttgtc ccgcaataac<br>2281 agcgtaccta attactgcaa aaatgatgaa ggggatatat tcctggcagc tgagtcctgg<br>2341 aagcctgacg tttgtaccag ctgcatctgc attgatagcg taattagctg tttctctgag<br>2401 tcctgccctt ctgtatcctg tgaaagacct gtcttgagaa aaggccagtg ttgtccctac<br>2461 tgcatagaag acacaattcc aaagaaggtg gtgtgccact tcagtgggaa ggcctatgcc<br>2521 gacgaggagc ggtgggacct tgacagctgc acccactgct actgcctgca gggccagacc<br>2581 ctctgctcga ccgtcagctg ccccccctctg ccctgtgttg agcccatcaa cgtggaagga<br>2641 agttgctgcc caatgtgtcc agaaatgtat gtcccagaac caaccaatat acccattgag<br>2701 aagacaaacc atcgaggaga ggttgacctg gaggttcccc tgtggcccac gcctagtgaa<br>2761 aatgatatcg tccatctccc tagagatatg ggtcacctcc aggtagatta cagagataac<br>2821 aggctgcacc caagtgaaga ttcttcactg gactccattg cctcagttgt ggttcccata<br>2881 attatatgcc tctctattat aatagcattc ctattcatca atcagaagaa acagtggata<br>2941 ccactgcttt gctggtatcg aacaccaact aagccttctt ccttaaataa tcagctagta<br>3001 tctgtggact gcaagaaagg aaccagagtc caggtggaca gttcccagag aatgctaaga<br>3061 attgcagaac cagatgcaag attcagtggc ttctacagca tgcaaaaaca gaaccatcta<br>3121 caggcagaca atttctacca aacagtgtga agaaaggcaa ctaggatgag gtttcaaaag<br>3181 acggaagacg actaaatctg ctctaaaaag taaactagaa tttgtgcact tgcttagtgg<br>3241 attgtattgg attgtgactt gatgtacagc gctaagacct tactgggatg ggctctgtct<br>3301 acagcaatgt gcagaacaag cattcccact tttcctcaag ataactgacc aagtgttttc<br>3361 ttagaaccaa agtttttaaa gttgctaaga tatatttgcc tgtaagatag ctgtagagat<br>3421 atttggggtg gggacagtga gtttggatgg ggaaatgggt gggagggtgg tgttgggaag<br>3481 aaaaattggt cagcttggct cggggagaaa cctggtaaca taaaagcagt tcagtggccc<br>3541 agaggttatt tttttcctat tgctctgaag actgcactgg ttgctgcaaa gctcaggcct<br>3601 gaatgagcag gaaacaaaaa aggccttgcg acccagctgc cataaccacc ttagaactac<br>3661 cagacgagca catcagaacc ctttgacagc catcccaggt ctaaagccac aagtttcttt<br>3721 tctatacagt cacaactgca gtaggcagtg aggaagccag agaaatgcga tagcggcatt<br>3781 tctctaaagc gggttattaa ggatatatac agttacactt tttgctgctt ttattttctt<br>3841 ccaagccaat caatcagcca gttcctagca gagtcagcac atgaacaaga tctaagtcat<br>3901 ttcttgatgt gagcactgga gcttttttttt ttttacaacg tgacaggaag aggagggaga<br>3961 gggtgacgaa caccaggcat ttccaggggc tatatttcac tgtttgttgt tgctttgttc<br>4021 tgttatattg ttggttgttc atagtttttg ttgaagctct agcttaagaa gaaacttttt | |

| Gene Name | GenBank Homology | DNA SEQUENCE / DEDUCED AMINO ACID SEQUENCE | SEQUENCE IDENTIFIER: |
|---|---|---|---|
| | | 4081 ttaaaaagac tgtttgggga ttctttttcc ttattatata ctgattctac aaaatagaaa<br>4141 ctacttcatt ttaattgtat attattcaag cacctttgtt gaagctcaaa aaaaatgatg<br>4201 cctctttaaa ctttagcaat tataggagta tttatgtaac tatcttatgc ttcaaaaaac<br>4261 aaaagtattt gtgtgcatgt gtatataata tatatatata catatatatt tatacacata<br>4321 caatttatgt tttcctgttg aatgtatttt tatgagattt taaccagaac aaaggcagat<br>4381 aaacaggcat tccatagcag tgcttttgat cacttacaaa ttttttgaat aacacaaaat<br>4441 ctcattctac ctgcagttta attggaaaga tgtgtgtgtg agagtatgta tgtgtgtgtg<br>4501 tgtgtgtgtg tgtgtgcgcg cgcacgcacg ccttgagcag tcagcattgc acctgctatg<br>4561 gagaagggta ttcctttatt aaaatcttcc tcatttggat ttgctttcag ttggttttca<br>4621 atttgctcac tggccagaga cattgatggc agttcttatc tgcatcacta atcagctcct<br>4681 ggattttttt tttttttttt tcaaacaatg gtttgaaaca actactggaa tattgtccac<br>4741 aataagctgg aagtttgttg tagtatgcct caaatataac tgactgtata ctatagtggt<br>4801 aacttttcaa acagccctta gcacttttat actaattaac ccatttgtgc attgagtttt<br>4861 cttttaaaaa tgcttgttgt gaaagacaca gatacccagt atgcttaacg tgaaaagaaa<br>4921 atgtgttctg ttttgtaaag gaactttcaa gtattgttgt aaatacttgg acagaggttg<br>4981 ctgaacttta aaaaaaatta atttattatt ataatgacct aatttattaa tctgaagatt<br>5041 aaccattttt ttgtcttaga atatcaaaaa gaaaaagaaa aaggtgttct agctgtttgc<br>5101 atcaaaggaa aaaaagattt attatcaagg ggcaatattt ttatcttttc caaaataaat<br>5161 ttgttaatga tacattacaa aaatagattg acatcagcct gattagtata aattttgttg<br>5221 gtaattaatc cattcctggc ataaaaagtc tttatcaaaa aaaattgtag atgcttgctt<br>5281 tttgtttttt caatcatggc catattatga aaatactaac aggatatagg acaaggtgta<br>5341 aattttttta ttattatttt aaagatatga tttatcctga gtgctgtatc tattactctt<br>5401 ttactttggt tcctgttgtg ctcttgtaaa agaaaaatat aatttcctga agaataaaat<br>5461 agatatatgg cacttggagt gcatcatagt tctacagttt gtttttgttt tcttcaaaaa<br>5521 agctgtaaga gaattatctg caacttgatt cttggcagga aataaacatt ttgagttgaa<br>5581 atcaaaaaaa aaaaaaaaaa a | |
| | | 1 M Y L V A G D R G L A G C G H L L V S L<br>1 ATGTACTTGGTGGCGGGGACAGGGGGTTGGCCGGCTGCGGGCACCTCCTGGTCTCGCTG<br><br>21 L G L L L L L A R S G T R A L V C L P C<br>61 CTGGGGCTGCTGCTGCTGCTGGCGCGCTCCGGCACCCGGGCGCTGGTCTGCCTGCCCTGT<br><br>41 D E S K C E E P R N C P G S I V Q G V C<br>121 GACGAGTCCAAGTGCGAGGAGCCCAGGAACTGCCCGGGGAGCATCGTGCAGGGCGTCTGC<br><br>61 G C C Y T C A S Q R N E S C G G T F G I<br>181 GGCTGCTGCTACACGTGCGCCAGCCAGAGGAACGAGAGCTGCGGCGGCACCTTCGGGATT<br><br>81 Y G T C D R G L R. C V I R P P L N G D S<br>241 TACGGAACCTGCGACCGGGGGCTGCGTTGTGTCATCCGCCCCCCGCTCAATGGCGACTCC<br><br>101 L T E Y E A G V C E D E N W T D D Q L L | SEQ ID NO:308 |

| Gene Name | GenBank Homology | DNA SEQUENCE / DEDUCED AMINO ACID SEQUENCE | SEQUENCE IDENTIFIER: |
|---|---|---|---|
| | | 301 CTCACCGAGTACGAAGCGGGCGTTTGCGAAGATGAGAACTGGACTGATGACCAACTGCTT<br><br>121 G F K P C N E N L I A G C N I I N G K C<br>361 GGTTTTAAACCATGCAATGAAAACCTTATTGCTGGCTGCAATATAATCAATGGGAAATGT<br><br>141 E C N T I R T C S N P F E F P S Q D M C<br>421 GAATGTAACACCATTCGAACCTGCAGCAATCCCTTTGAGTTTCCAAGTCAGGATATGTGC<br><br>161 L S A L K R I E E E K P D C S K A R C E<br>481 CTTTCAGCTTTAAAGAGAATTGAAGAAGAGAAGCCAGATTGCTCCAAGGCCCGCTGTGAA<br><br>181 V Q F S P R C P E D S V L I E G Y A P P<br>541 GTCCAGTTCTCTCCACGTTGTCCTGAAGATTCTGTTCTGATCGAGGGTTATGCTCCTCCT<br><br>201 G E C C P L P S R C V C N P A G C L R K<br>601 GGGGAGTGCTGTCCCTTACCCAGCCGCTGCGTGTGCAACCCCGCAGGCTGTCTGCGCAAA<br><br>221 V C Q P G N L N I L V S K A S G K P G E<br>661 GTCTGCCAGCCGGGAAACCTGAACATACTAGTGTCAAAAGCCTCAGGGAAGCCGGGAGAG<br><br>241 C C D L Y E C K P V F G V D C R T V E C<br>721 TGCTGTGACCTCTATGAGTGCAAACCAGTTTTCGGCGTGGACTGCAGGACTGTGGAATGC<br><br>261 P P V Q Q T A C P P D S Y E T Q V R L T<br>781 CCTCCTGTTCAGCAGACCGCGTGTCCCCCGGACAGCTATGAAACTCAAGTCAGACTAACT<br><br>281 A D G C C T L P T R C E C L S G L C G F<br>841 GCAGATGGTTGCTGTACTTTGCCAACAAGATGCGAGTGTCTCTCTGGCTTATGTGGTTTC<br><br>301 P V C E V G S T P R I V S R G D G T P G<br>901 CCCGTGTGTGAGGTGGGATCCACTCCCCGCATAGTCTCTCGTGGCGATGGGACACCTGGA<br><br>321 K C C D V F E C V N D T K P A C V F N N<br>961 AAGTGCTGTGATGTCTTTGAATGTGTTAATGATACAAAGCCAGCCTGCGTATTTAACAAT<br><br>341 V E Y Y D G D M F R M D N C R F C R C Q<br>1021 GTGGAATATTATGATGGAGACATGTTTCGAATGGACAACTGTCGGTTCTGTCGATGCCAA<br><br>361 G G V A I C F T A Q C G E I N C E R Y Y<br>1081 GGGGGCGTTGCCATCTGCTTCACTGCCCAGTGTGGTGAGATAAACTGCGAGAGGTACTAC<br><br>381 V P E G E C C P V C E D P V Y P F N N P<br>1141 GTGCCCGAAGGAGAGTGCTGCCCAGTGTGTGAAGATCCAGTGTATCCTTTTAATAATCCC | |

| Gene Name | GenBank Homology | DNA SEQUENCE / DEDUCED AMINO ACID SEQUENCE | SEQUENCE IDENTIFIER: |
|---|---|---|---|
| | | 401 A G C Y A N G L I L A H G D R W R E D D<br>1201 GCTGGCTGCTATGCCAATGGCCTGATCCTTGCCCACGGAGACCGGTGGCGGGAAGACGAC<br><br>421 C T F C Q C V N G E R H C V A T V C G Q<br>1261 TGCACATTCTGCCAGTGCGTCAACGGTGAACGCCACTGCGTTGCGACCGTCTGCGGACAG<br><br>441 T C T N P V K V P G E C C P V C E E P T<br>1321 ACCTGCACAAACCCTGTGAAAGTGCCTGGGGAGTGTTGCCCTGTGTGCGAAGAACCAACC<br><br>461 I I T V D P P A C G E L S N C T L T G K<br>1381 ATCATCACAGTTGATCCACCTGCATGTGGGGAGTTATCAAACTGCACTCTGACAGGGAAG<br><br>481 D C I N G F K R D H N G C R T C Q C I N<br>1441 GACTGCATTAATGGTTTCAAACGCGATCACAATGGTTGTCGGACCTGTCAGTGCATAAAC<br><br>501 T E E L C S E R K Q G C T L N C P F G F<br>1501 ACCGAGGAACTATGTTCAGAACGTAAACAAGGCTGCACCTTGAACTGTCCCTTCGGTTTC<br><br>521 L T D A Q N C E I C E C R P R P K K C R<br>1561 CTTACTGATGCCCAAAACTGTGAGATCTGTGAGTGCCGCCCAAGGCCCAAGAAGTGCAGA<br><br>541 P I I C D K Y C P L G L L K N K H G C D<br>1621 CCCATAATCTGTGACAAGTATTGTCCACTTGGATTGCTGAAGAATAAGCACGGCTGTGAC<br><br>561 I C R C K K C P E L S C S K I C P L G F<br>1681 ATCTGTCGCTGTAAGAAATGTCCAGAGCTCTCATGCAGTAAGATCTGCCCCTTGGGTTTC<br><br>581 Q Q D S H G C L I C K C R E A S A S A G<br>1741 CAGCAGGACAGTCACGGCTGTCTTATCTGCAAGTGCAGAGAGGCCTCTGCTTCAGCTGGG<br><br>601 P P I L S G T C L T V D G H H H K N E E<br>1801 CCACCCATCCTGTCGGGCACTTGTCTCACCGTGGATGGTCATCATCATAAAAATGAGGAG<br><br>621 S W H D G C R E C Y C L N G R E M C A L<br>1861 AGCTGGCACGATGGGTGCCGGGAATGCTACTGTCTCAATGGACGGGAAATGTGTGCCCTG<br><br>641 I T C P V P A C G N P T I H P G Q C C P<br>1921 ATCACCTGCCCGGTGCCTGCCTGTGGCAACCCCACCATTCACCCTGGACAGTGCTGCCCA<br><br>661 S C A D D F V V Q K P E L S T P S I C H<br>1981 TCATGTGCAGATGACTTTGTGGTGCAGAAGCCAGAGCTCAGTACTCCCTCCATTTGCCAC | |

| Gene Name | GenBank Homology | DNA SEQUENCE / DEDUCED AMINO ACID SEQUENCE | SEQUENCE IDENTIFIER: |
|---|---|---|---|
| | | 681 A P G G E Y F V E G E T W N I D S C T Q<br>2041 GCCCCTGGAGGAGAATACTTTGTGGAAGGAGAAACGTGGAACATTGACTCCTGTACTCAG<br><br>701 C T C H S G R V L C E T E V C P P L L C<br>2101 TGCACCTGCCACAGCGGACGGGTGCTGTGTGAGACAGAGGTGTGCCCACCGCTGCTCTGC<br><br>721 Q N P S R T Q D S C C P Q C T D Q P F R<br>2161 CAGAACCCCTCACGCACCCAGGATTCCTGCTGCCCACAGTGTACAGATCAACCTTTTCGG<br><br>741 P S L S R N N S V P N Y C K N D E G D I<br>2221 CCTTCCTTGTCCCGCAATAACAGCGTACCTAATTACTGCAAAAATGATGAAGGGGATATA<br><br>761 F L A A E S W K P D V C T S C I C I D S<br>2281 TTCCTGGCAGCTGAGTCCTGGAAGCCTGACGTTTGTACCAGCTGCATCTGCATTGATAGC<br><br>781 V I S C F S E S C P S V S C E R P V L R<br>2341 GTAATTAGCTGTTTCTCTGAGTCCTGCCCTTCTGTATCCTGTGAAAGACCTGTCTTGAGA<br><br>801 K G Q C C P Y C I E D T I P K K V V C H<br>2401 AAAGGCCAGTGTTGTCCCTACTGCATAGAAGACACAATTCCAAAGAAGGTGGTGTGCCAC<br><br>821 F S G K A Y A D E E R W D L D S C T H C<br>2461 TTCAGTGGGAAGGCCTATGCCGACGAGGAGCGGTGGGACCTTGACAGCTGCACCCACTGC<br><br>841 Y C L Q G Q T L C S T V S C P P L P C V<br>2521 TACTGCCTGCAGGGCCAGACCCTCTGCTCGACCGTCAGCTGCCCCCCTCTGCCCTGTGTT<br><br>861 E P I N V E G S C C P M C P E M Y V P E<br>2581 GAGCCCATCAACGTGGAAGGAAGTTGCTGCCCAATGTGTCCAGAAATGTATGTCCCAGAA<br><br>881 P T N I P I E K T N H R G E V D L E V P<br>2641 CCAACCAATATACCCATTGAGAAGACAAACCATCGAGGAGAGGTTGACCTGGAGGTTCCC<br><br>901 L W P T P S E N D I V H L P R D M G H L<br>2701 CTGTGGCCCACGCCTAGTGAAAATGATATCGTCCATCTCCCTAGAGATATGGGTCACCTC<br><br>921 Q V D Y R D N R L H P S E D S S L D S I<br>2761 CAGGTAGATTACAGAGATAACAGGCTGCACCCAAGTGAAGATTCTTCACTGGACTCCATT<br><br>941 A S V V V P I I I C L S I I I A F L F I<br>2821 GCCTCAGTTGTGGTTCCCATAATTATATGCCTCTCTATTATAATAGCATTCCTATTCATC<br><br>961 N Q K K Q W I P L L C W Y R T P T K P S | |

| Gene Name | GenBank Homology | DNA SEQUENCE / DEDUCED AMINO ACID SEQUENCE | SEQUENCE IDENTIFIER: |
|---|---|---|---|
| | | 2881 AATCAGAAGAAACAGTGGATACCACTGCTTTGCTGGTATCGAACACCAACTAAGCCTTCT<br><br>981 S L N N Q L V S V D C K K G T R V Q V D<br>2941 TCCTTAAATAATCAGCTAGTATCTGTGGACTGCAAGAAAGGAACCAGAGTCCAGGTGGAC<br><br>1001 S S Q R M L R I A E P D A R F S G F Y S<br>3001 AGTTCCCAGAGAATGCTAAGAATTGCAGAACCAGATGCAAGATTCAGTGGCTTCTACAGC<br><br>1021 M Q K Q N H L Q A D N F Y Q T V -<br>3061 ATGCAAAAACAGAACCATCTACAGGCAGACAATTTCTACCAAACAGTGTGA | |
| Foe1075 | H.sapiens initiation factor 4B cDNA. | 1 TCTAGAGCCCTCTCCCAACATGGCGGCCTCAGCAAAAAAGAAGAATAAGAAGGGGAAGAC<br>61 TATCTCCCTAACAGACTTTCTGGCTGAGGATGGGGGTACTGGTGGAGGAAGCACCTATGT<br>121 TTCCAAACCAGTCAGCTGGGCTGATGAAACGGATGACCTGGAAGGAGATGTTTCGACCAC<br>181 TTGGCACAGTAACGATGACGATGTGTATAGGGCGCCTCCAATTGACCGTTCCATCCTTCC<br>241 CACTGCTCCACGGGCTGCTCGGGAACCCAATATCGACCGGAGCCGTCTTCCCAAATCGCC<br>301 ACCCTACACTGCTTTTCTAGGAAACCTACCCTATGATGTTACAGAAGAGTCAATTAAGGA<br>361 ATTCTTTCGAGGATTAAATATCAGTGCAGTGCGTTTACCACGTGAACCCAGCAATCCAGA<br>421 GAGGTTGAAAGGTTTTGGTTATGCTGAATTTGAGGACCTGGATTCCCTGCTCAGTGCCCT<br>481 GAGTCTCAATGAAGAGTCTCTAGGTAACAGGAGAATTCGAGTGGACGTTGCTGATCAAGC<br>541 ACAGGATAAAGACAGGGATGATCGTTCTTTTGGCCGTGATAGAAATCGGGATTCTGACAA<br>601 AACAGATACAGACTGGAGGGCTCGTCCTGCTACAGACAGCTTTGATGACTACCCACCTAG<br>661 AAGAGGTGATGATAGCTTTGGAGACAAGTATCGAGATCGTTATGATTCAGACCGGTATCG<br>721 GGATGGGTATCGGGATGGGTATCGGGATGGCCCACGCCGGGATATGGATCGATATGGTGG<br>781 CCGGGATCGCTATGATGACCGAGGCAGCAGAGACTATGATAGAGGCTATGATTCCCGGAT<br>841 AGGCAGTGGCAGAAGAGCATTTGGCAGTGGGTATCGCAGGGATGATGACTACAGAGGAGG<br>901 CGGGGACCGCTATGAAGACCGATATGACAGACGGGATGATCGGTCGTGGAGCTCCAGAGA<br>961 TGATTACTCTCGGGATGATTATAGGCGTGATGATAGAGGTCCCCCCCAAAGACCCAAACT<br>1021 GAATCTAAAGCCTCGGAGTACTCCTGAAGAAGATGATTCCTCTGCTAGTACCTCCCAGTC<br>1081 CACTCGAGCTGCTTCTATCTTTGGAGGGGCAAAGCCTGTTGACACAGCTGCTAGAGAAAG<br>1141 AGAAGTAGAAGAACGGCTACAGAAGGAACAAGAGAAGTTGCAGCGTCAGTGGAATGAGCC<br>1201 AAAACTAGAACGACGGCCTCGGGAGAGACACCCAAGCTGGCGAAGTGAAGAAACTCAGGA<br>1261 ACGGGAACGGTCGAGGACAGGAAGTGAGTCATCACAAACTGGGACCTCCACCACATCTAG<br>1321 CAGAAATGCACGAAGGAGAGAGAGTGAGAAGTCTCTAGAAAATGAAACACTCAATAAGGA<br>1381 GGAAGATTGCCACTCTCCAACTTCTAAACCTCCCAAACCTGATCAGCCCCTAAAGGTAAT<br>1441 GCCAGCCCCTCCACCAAAGGAGAATGCTTGGGTGAAGCGAAGTTCTAACCCTCCTGCTCG<br>1501 ATCTCAGAGCTCAGACACAGAGCAGCAGTCCCCTACAAGTGGTGGGGGAAAAGTAGCTCC<br>1561 AGCTCAACCATCTGAGGAAGGACCAGGAAGGAAAGATGAAAATAAAGTAGATGGGATGAA<br>1621 TGCCCCAAAAGGCCAAACTGGGAACTCTAGCCGTGGTCCAGGAGACGGAGGGAACAGAGA<br>1681 CCACTGGAAGGAGTCAGATAGGAAAGATGGCAAAAAGGATCAAGACTCCAGATCTGCACC<br>1741 TGAGCCAAAGAAACCTGAGGAAAATCCAGCTTCTAAGTTCAGTTCTGCAAGCAAGTATGC<br>1801 TGCTCTCTCTGTTGATGGTGAAGATGAAAATGAGGGAGAAGATTATGCCGAATAGACCTC | SEQ ID NO:309 |

| Gene Name | GenBank Homology | DNA SEQUENCE / DEDUCED AMINO ACID SEQUENCE | SEQUENCE IDENTIFIER: |
|---|---|---|---|
| | | 1861 TACATCCTGTGCTTTTCTCCTAGTTTCTCTCCACCCTGGAACATTCGAGAGCAAATCAAA<br>1921 ACCTCTATCCAGACAAGACAAAATAAAACTCAACATCTCCTGAAGACCTTTCTTACCTTT<br>1981 TTTTAAAAACAAAAANTGAAATTATTTTGCATGCTGCTGCAGCCTTTAAAGTATTGAAGT<br>2041 AACTGGAGAATTGCCAATACAGCCAGAGAGAAAGGACTACAGCTTTTTAGAGGAAAAGT<br>2101 TGTGGTGCGTTATGTCACCATGCAGTTGCCAGTGTGATTAGTGCCTAGGGGTCTCATTTA<br>2161 GCAGAAATGGTAATGACAGTGATATAATGCCTGGAACCTGGTTGGGCAGTAGGGGAGGGA<br>2221 GGTAGAAGGAAAAGTGTGAGATTTCTACCTTTTAGTTTTCATCCTATTGTGGCATATATG<br>2281 AATTCTCAAACATTATCTGAATAAATTTTCCACTCTTGGAAAGGTAGATTTAGCCTCAAG<br>2341 TTGTTCTAGTCTCCAGGAGGCTGCCAGCCCCTCCTCTTATTTAATTCTGAGTTTTGGGGG<br>2401 CCAGCCTAGAGGGAATTCCTTTTTTTTTTTTTTTAACCCCCCAGGGGGGTAGTTGGGAGT<br>2461 GAGACTATAGGCCATAAAGAATGGGACTGCATTGGACCAAAATAAATGGGAAAATCGTGG<br>2521 TTTGAAAAGAAGCTTTTGGGAAGTGATGAGTCATTTTGCACCAGGTAATAGGGGAAAATT<br>2581 GTGTGACCTCCAGCAAACACATGAATGGTTATTTCCTGGAGCCGGAAGCACTTGGGGGTC<br>2641 GTGGTAATTCCCAGTGTTTTCTGTGTCCTAGTTTTACCCTTTCTAAACACTGTCCTTTTT<br>2701 GAAAGTTTTGAATATATCCACATTCTATTGAAACCTTGAAACTAAAAATTTAGACTCTTA<br>2761 TCGTCATCTTAAGTTCTTCATGCTACTCTTAACCTCCCAAAAAGCAGTATCTAAGTCACA<br>2821 TACATGATGTCTTGGGCATTTTCTGAGCCATGGAGAACTCTGAAAGGAAGAATCGCTGCT<br>2881 TTTCTCAAGCAAATCGGTTTCTTGATGTCTTTTGGTTCTCCTTGCCTGCTCCTGATGCTT<br>2941 GGACCCCTTTTATTGATCAGAGTGCTCTAGAATAATGGATGGTCTTGGATGATGGATAAA<br>3001 TAGGGACAGGGACAGTTAAATTGGGAGCCTTTCTTACAACCTTGATGGGATTTTTCCCCC<br>3061 CAAGTTTCCTTCTCCACTGAAATGCCACACTAATGCTTGTTGGATTCATGAGGTGGCCAG<br>3121 ACCAATGTGTTGTTTTGTTGTTGTTTTTTTTTTAAGCTTCCCTTGAGAGAATAAGTGATA<br>3181 ATGGAGAGAGCTGTGTAACAAGATCCAGGTTTCTCTTGCAATACAGTAGCTAAACTTGCC<br>3241 TGCTTTTATATGCATTTTTGTAGGGATCAGCTTGGTAGACAGTATTAGCGGAGAAACACC<br>3301 TTGATCTTGGTTTGCAAGGCCTCCTCCGGTCAGTCCTAGGTTAGACCCTGTCCAGCCACG<br>3361 CGGGGCTGTTGGTGTTTGAGTGCTGCAGCAGTGGGCAGAATGAATCATTTACCCAGTGGA<br>3421 CAAAGGTGTGTACCAAGTGAATTTAAATAATTGGTGTGGATTGGCCAGTAGCTAAGAAGT<br>3481 GGGCTTTTAAAGAGTATTGAAGATTGAAAGGGTTTTTCTTTCTTTTTTAAAAAAGAAAAA<br>3541 CAAACTATTGATTGTAGATGATGAAAAGGTAGGGTTTGCCCTCTTCATGTCTACTCCTTC<br>3601 CAAATAGTTGTATCCAAAACTGTTTTTCCCTCTCCCCTACCTTGTCCCCCCTATTAAAAT<br>3661 AGAAACAGGGATTGATTTATGTCCCACTCCTCAATACGTGTAAAATTTGTACAAAAATAT<br>3721 CTTCTATGAAAATGATTTGTAATCTGTAGACAATACCTGGGAGATGTCTTGAGATGTAAA<br>3781 ATCCCATCCTTTCGGTTGTGGGATTTTTTGTTTTCTCCAAATAAATCTGATCTTTAAAGT<br>3841 TAAAAAAAAAAAAAAAACTCTAGAGTCGAGGAATTC | |
| | | 1 M A A S A K K K N K K G K T I S L T D F<br>1 ATGGCGGCCTCAGCAAAAAAGAAGAATAAGAAGGGGAAGACTATCTCCCTAACAGACTTT<br><br>21 L A E D G G T G G G S T Y V S K P V S W<br>61 CTGGCTGAGGATGGGGGTACTGGTGGAGGAAGCACCTATGTTTCCAAACCAGTCAGCTGG<br><br>41 A D E T D D L E G D V S T T W H S N D D<br>121 GCTGATGAAACGGATGACCTGGAAGGAGATGTTTCGACCACTTGGCACAGTAACGATGAC | SEQ ID NO:310 |

| Gene Name | GenBank Homology | DNA SEQUENCE / DEDUCED AMINO ACID SEQUENCE | SEQUENCE IDENTIFIER: |
|---|---|---|---|
| | | 61 D V Y R A P P I D R S I L P T A P R A A<br>181 GATGTGTATAGGGCGCCTCCAATTGACCGTTCCATCCTTCCCACTGCTCCACGGGCTGCT<br><br>81 R E P N I D R S R L P K S P P Y T A F L<br>241 CGGGAACCCAATATCGACCGGAGCCGTCTTCCCAAATCGCCACCCTACACTGCTTTTCTA<br><br>101 G N L P Y D V T E E S I K E F F R G L N<br>301 GGAAACCTACCCTATGATGTTACAGAAGAGTCAATTAAGGAATTCTTTCGAGGATTAAAT<br><br>121 I S A V R L P R E P S N P E R L K G F G<br>361 ATCAGTGCAGTGCGTTTACCACGTGAACCCAGCAATCCAGAGAGGTTGAAAGGTTTTGGT<br><br>141 Y A E F E D L D S L L S A L S L N E E S<br>421 TATGCTGAATTTGAGGACCTGGATTCCCTGCTCAGTGCCCTGAGTCTCAATGAAGAGTCT<br><br>161 L G N R R I R V D V A D Q A Q D K D R D<br>481 CTAGGTAACAGGAGAATTCGAGTGGACGTTGCTGATCAAGCACAGGATAAAGACAGGGAT<br><br>181 D R S F G R D R N R D S D K T D T D W R<br>541 GATCGTTCTTTTGGCCGTGATAGAAATCGGGATTCTGACAAAACAGATACAGACTGGAGG<br><br>201 A R P A T D S F D D Y P P R R G D D S F<br>601 GCTCGTCCTGCTACAGACAGCTTTGATGACTACCCACCTAGAAGAGGTGATGATAGCTTT<br><br>221 G D K Y R D R Y D S D R Y R D G Y R D G<br>661 GGAGACAAGTATCGAGATCGTTATGATTCAGACCGGTATCGGGATGGGTATCGGGATGGG<br><br>241 Y R D G P R R D M D R Y G G R D R Y D D<br>721 TATCGGGATGGCCCACGCCGGGATATGGATCGATATGGTGGCCGGGATCGCTATGATGAC<br><br>261 R G S R D Y D R G Y D S R I G S G R R A<br>781 CGAGGCAGCAGAGACTATGATAGAGGCTATGATTCCCGGATAGGCAGTGGCAGAAGAGCA<br><br>281 F G S G Y R R D D D Y R G G G D R Y E D<br>841 TTTGGCAGTGGGTATCGCAGGGATGATGACTACAGAGGAGGCGGGGACCGCTATGAAGAC<br><br>301 R Y D R R D D R S W S S R D D Y S R D D<br>901 CGATATGACAGACGGGATGATCGGTCGTGGAGCTCCAGAGATGATTACTCTCGGGATGAT<br><br>321 Y R R D D R G P P Q R P K L N L K P R S<br>961 TATAGGCGTGATGATAGAGGTCCCCCCCAAAGACCCAAACTGAATCTAAAGCCTCGGAGT | |

| Gene Name | GenBank Homology | DNA SEQUENCE / DEDUCED AMINO ACID SEQUENCE | SEQUENCE IDENTIFIER: |
|---|---|---|---|
| | | 341 T P E E D D S S A S T S Q S T R A A S I<br>1021 ACTCCTGAAGAAGATGATTCCTCTGCTAGTACCTCCCAGTCCACTCGAGCTGCTTCTATC<br><br>361 F G G A K P V D T A A R E R E V E E R L<br>1081 TTTGGAGGGGCAAAGCCTGTTGACACAGCTGCTAGAGAAAGAGAAGTAGAAGAACGGCTA<br><br>381 Q K E Q E K L Q R Q W N E P K L E R R P<br>1141 CAGAAGGAACAAGAGAAGTTGCAGCGTCAGTGGAATGAGCCAAAACTAGAACGACGGCCT<br><br>401 R E R H P S W R S E E T Q E R E R S R T<br>1201 CGGGAGAGACACCCAAGCTGGCGAAGTGAAGAAACTCAGGAACGGGAACGGTCGAGGACA<br><br>421 G S E S S Q T G T S T T S S R N A R R R<br>1261 GGAAGTGAGTCATCACAAACTGGGACCTCCACCACATCTAGCAGAAATGCACGAAGGAGA<br><br>441 E S E K S L E N E T L N K E E D C H S P<br>1321 GAGAGTGAGAAGTCTCTAGAAAATGAAACACTCAATAAGGAGGAAGATTGCCACTCTCCA<br><br>461 T S K P P K P D Q P L K V M P A P P P K<br>1381 ACTTCTAAACCTCCCAAACCTGATCAGCCCCTAAAGGTAATGCCAGCCCCTCCACCAAAG<br><br>481 E N A W V K R S S N P P A R S Q S S D T<br>1441 GAGAATGCTTGGGTGAAGCGAAGTTCTAACCCTCCTGCTCGATCTCAGAGCTCAGACACA<br><br>501 E Q Q S P T S G G G K V A P A Q P S E E<br>1501 GAGCAGCAGTCCCCTACAAGTGGTGGGGGAAAAGTAGCTCCAGCTCAACCATCTGAGGAA<br><br>521 G P G R K D E N K V D G M N A P K G Q T<br>1561 GGACCAGGAAGGAAAGATGAAAATAAAGTAGATGGGATGAATGCCCCAAAAGGCCAAACT<br><br>541 G N S S R G P G D G G N R D H W K E S D<br>1621 GGGAACTCTAGCCGTGGTCCAGGAGACGGAGGGAACAGAGACCACTGGAAGGAGTCAGAT<br><br>561 R K D G K K D Q D S R S A P E P K K P E<br>1681 AGGAAAGATGGCAAAAAGGATCAAGACTCCAGATCTGCACCTGAGCCAAAGAAACCTGAG<br><br>581 E N P A S K F S S A S K Y A A L S V D G<br>1741 GAAAATCCAGCTTCTAAGTTCAGTTCTGCAAGCAAGTATGCTGCTCTCTCTGTTGATGGT<br><br>601 E D E N E G E D Y A E -<br>1801 GAAGATGAAAATGAGGGAGAAGATTATGCCGAATAG | |
| Foe1092 | No homology | | |

| Gene Name | GenBank Homology | DNA SEQUENCE / DEDUCED AMINO ACID SEQUENCE | SEQUENCE IDENTIFIER: |
|---|---|---|---|
| | | 1 CCCCATTTTCACACCTGTCTTCCGATCTTCACACCTGCCTTCCCCATGTTCACACCTGCC<br>61 TCCCCATCTTCACGCCTGTCTTCCCTGTGTTCACACCTGTCTTCCCATATTCATACCTGC<br>121 ATTCCCCATGTTCACACTTGCCTCTCCATCTTCACAACTGCCTTCTCCATGTTTATACCT<br>181 GCCTTTCTCACCATTTTGGTTTGAAAGACAGTTTTCCATGGATGTTTTTTTCCAGAAAGT<br>241 CAGTATTATTTTTTAAATAAGAAAAATACTCCTTAAAAGATCACAATTGTGAAAACTACT<br>301 GTGGCCTTTCTGCTTTTCCAGATTTTAGTCTCCTTTCTCCCCATCCTCTGATCCAGCAAA<br>361 GATGGTGAGGACAAAGTGGTAAACTTCTCTGCACTCCATGATGGTCTACACATGGTCTGA<br>421 CCTGCACTGTTTCTGGCACCCACTGAAAAAGAAGTTGAGTAGACAGTGTAGAGTACAGAA<br>481 ATGTGGCTTTTGCCTAACACTTTTGCATCTCCAAAGTCTTAAGAGTTGGTTCATCCCATT<br>541 TGAGGACAATGTTCAGTTGTACATCTCAGAGTTGGAAATGGAAGAAAGCAGAGGGAGTCT<br>601 ATTTTCACTCATTCTTTCAATAAATATTTATTGAGCACCAAAAAAAAAAAAA | SEQ ID NO:311 |
| Foe1216 | Homo sapiens RNF4 mRNA. | 1 GAGGAGCTCTCCTGGGCGGCTGAAGAAGGAGCTTCTTCTCCGGAGTGCGAAGGCGGTGGC<br>61 GCCTGCGGACCTAACTAGCTCCAGGTTAGGCCGAGCTTTGCGGGAAAGCAGCGGACTTGA<br>121 AAATACTGGAAATCTGTCCGGATCCAAATTATTTTGCAAGCCAGATGAGTAACCAGAGGG<br>181 CATGAAAGGTTGAGAACATTTGACTTCCCTGCAAACCTTGGTATAGATCACTTCCTTTTC<br>241 TGTAGGAAAGGAAAGGCACCAAAGAGCACAATGAGTACAAGAAAGCGTCGTGGTGGAGCA<br>301 ATAAATTCTAGACAAGCTCAGAAGCGAACTCGGGAAGCAACCTCCACCCCCGAGATCTCC<br>361 TTGGAAGCAGAACCCATAGAACTCGTGGAAACTGCTGGAGATGAAATTGTGGACCTCACT<br>421 TGTGAATCTTTAGAGCCTGTGGTGGTTGATCTGACTCACAATGACTCTGTTGTGATTGTT<br>481 GACGAAAGAAGAAGACCAAGGAGGAATGCTAGGAGGCTGCCCCAGGACCATGCTGACAGC<br>541 TGTGTGGTGAGCAGTGACGATGAGGAGTTGTCCAGGGACAGAGACGTATATGTGACTACC<br>601 CATACTCCCAGAAACGCCAGGGATGAGGGCGCTACAGGCCTCAGGCCCTCAGGTACTGTC<br>661 AGTTGTCCCATCTGCATGGACGGATACTCAGAGATCGTGCAGAATGGACGTCTCATCGTT<br>721 TCCACAGAATGCGGCCATGTCTTCTGTAGCCAGTGCCTCCGTGATTCCCTGAAGAATGCT<br>781 AATACTTGCCCAACTTGTAGGAAAAAGATCAACCACAAACGGTACCACCCCATTTATATA<br>841 TGAAGTATTCAGAGCCCCCCAGGAGAGACGGATGGACAGACAGACAGCCAGGTTCTCCAG<br>901 TGGTATCTGCCTCCATTTTCCTGAGATCAAAAAGACTGTTTCGAAACCAACATCTGATAT<br>961 GTAAACTGCTCTTTTGTTTCCAACCCCTTCCTTTTGTTATCTCCAGTTTGATGCTATGGC<br>1021 GCTGGACCCAGGGCCCTCCCAGGCCATCTCTGTTCCTCTGGGGTGGTCCAGTTCTAGAGT<br>1081 GGGAGAAAGGGAGTCAGGCGCATTGGGAATCGTGGTTCCAGTCTGGTTGCAGAATCTGCA<br>1141 CATTTGCCAAGAAATTTTCCCTGTTTGGAAAGTTTGCCCCAGCTTTCCCGGGCACACCAC<br>1201 CTTTTGTCCCAAGTGTCTGCCGGTCGACCAATCTGCCTGCCACACATTGACCAAGCCAGA<br>1261 CCCGGTTCACCCAGCTCGAGGATCCCAGGTTGAAGAGTGGCCCCTTGAGGCCCTGGAAAG<br>1321 ACCAATCACTGGACTTCTTCCCTTGAGAGTCAGAGGTCACCCGTGATTCTGCCTGCACCT<br>1381 TATCATTGATCTGCAGTGATTTCTGCAAATCAAGAGAAACTCTGCAGGGCACTCCCCTGT<br>1441 TTCCTAAGAACGAAAAAGTGCAATAAAGGCCATTCGTTACCTACTTTTCAGCAGCCCACA<br>1501 AGATGTAGCACTATTAGTGTCCCCCTCAGAGGCTTAATGTTGCCTGTGGAGCAGTGCCCA<br>1561 TCCCAGCCCGTTTCTGCCCACCAGTTGTTCTCAGGAACCTTACCCATGCTCCAGCGTCCT<br>1621 TCACCTGGCACAGGACATGCAAGATAAATAGGGCAGGCACGTGTTTGGGTGTCCTCTCTT<br>1681 TTCTGATAAAATCCATCCCGTGTTTGCCACACGCCCTCCAGTCCTCAGTTCCCACTGCCT<br>1741 AACGTCTGCCCCCGTGTAGATACTGAGAGGTGGTGGCAGTAATTGTGGCCTTATCAGCCG | SEQ ID NO:312 |

| Gene Name | GenBank Homology | DNA SEQUENCE / DEDUCED AMINO ACID SEQUENCE | SEQUENCE IDENTIFIER: |
|---|---|---|---|
| | | 1801 CTCAGTTCCAGGCTTTTGCCCAGGTCACTGTTGCCCCATGTTCGGAGAACCTGGCCCACC<br>1861 TGTCTTGGCTTTCTCATCCTTCCCAACCCAGTGCCGTTTATTTCAGAAGCTTCCTGGCCA<br>1921 CTGGGCTTGGATGCTTCGGGCTTCTGACTGCTCCATAGGTTTTGACTGGTGAAACAGGGG<br>1981 CCCAGATGACAACCTCTCCTTCGCTCCACAGGTACGCGGGAGCCTCAGGTTCTCTCAGGG<br>2041 GCAGCAAAGTGGCCCAAGCTGCCCCTGACAGCACAGGGCCTGGGGGTGGCTAACGAGAGA<br>2101 GGCCTTACAGTGCCGGCATGCCTCCTCTTCCACTGTCGTCCTTCCTCAGAGGGCCTCACG<br>2161 CCAAACAAACGGCCTTTTCGTGTGAAACATCTTCAGGGCGGGAAAGGGGCCACTTCTGGC<br>2221 TTTGTTAGCAATAACTGACCTTCAGTTTACCCTTCTGAAGGAGCAGGGACTCAGCACAGA<br>2281 ATTCACTTTAAACGGGGCTGAAGGAGTGTTCCTCCTTTATGTGAAAAGAAAATCATTTTA<br>2341 CTCTTCATTTTGACTTTTTGACTGTTGGGCTCACTTCCAGTTAGTTTGAATGAAAGTACT<br>2401 AATTTTCTACTTGGAGTTGAGGAGGGCAGAATCCGCAGCTCTCATCATTGTGATGTGTAG<br>2461 CATGTCTGCCCTCTGACTGGACATCATTGCCATTAACTTTCTTCTGGGCATCACGGCAGT<br>2521 GTCACGATGCCCAGACTTGGAGCAAGGCAACCTTGGAGTCAGTCCACTCATAAAATATGG<br>2581 TAACACCCATTTTAAAATTTAAGTTTTGTCCTTAAAGACAACTTCAGTGGTTAATTATAA<br>2641 AAGTTGTGTTACTTCGTCCTAAATTAAATTGATAGAAAGATTTAAAAATGTGTTTTGTTT<br>2701 CTAGTATTCAGAAACTGCGAAGTAGGAAAAGGTTGATATGGAAACAATCCCTTTCCCTTC<br>2761 CTCAATGTACATAGTTCAAATCTTTCTTTGTTACATTTAAACTATATCCGTGTATGTCAG<br>2821 TCTGTTTTGGACTCCTCTGCTAGTGTTACAGATGGAAATAAAACCATTAATTTGAACCAA<br>2881 AAAAAAAAAAAAAAAAAAAAAAAAAAAAAAAAAAAAAAA | |
| | | 1 M S T R K R R G G A I N S R Q A Q K R T<br>1 ATGAGTACAAGAAAGCGTCGTGGTGGAGCAATAAATTCTAGACAAGCTCAGAAGCGAACT<br><br>21 R E A T S T P E I S L E A E P I E L V E<br>61 CGGGAAGCAACCTCCACCCCCGAGATCTCCTTGGAAGCAGAACCCATAGAACTCGTGGAA<br><br>41 T A G D E I V D L T C E S L E P V V V D<br>121 ACTGCTGGAGATGAAATTGTGGACCTCACTTGTGAATCTTTAGAGCCTGTGGTGGTTGAT<br><br>61 L T H N D S V V I V D E R R R P R R N A<br>181 CTGACTCACAATGACTCTGTTGTGATTGTTGACGAAAGAAGAAGACCAAGGAGGAATGCT<br><br>81 R R L P Q D H A D S C V V S S D D E E L<br>241 AGGAGGCTGCCCCAGGACCATGCTGACAGCTGTGTGGTGAGCAGTGACGATGAGGAGTTG<br><br>101 S R D R D V Y V T T H T P R N A R D E G<br>301 TCCAGGGACAGAGACGTATATGTGACTACCCATACTCCCAGAAACGCCAGGGATGAGGGC<br><br>121 A T G L R P S G T V S C P I C M D G Y S<br>361 GCTACAGGCCTCAGGCCCTCAGGTACTGTCAGTTGTCCCATCTGCATGGACGGATACTCA<br><br>141 E I V Q N G R L I V S T E C G H V F C S<br>421 GAGATCGTGCAGAATGGACGTCTCATCGTTTCCACAGAATGCGGCCATGTCTTCTGTAGC | SEQ ID NO:313 |

| Gene Name | GenBank Homology | DNA SEQUENCE / DEDUCED AMINO ACID SEQUENCE | SEQUENCE IDENTIFIER: |
|---|---|---|---|
| | | 161 Q C L R D S L K N A N T C P T C R K K I<br>481 CAGTGCCTCCGTGATTCCCTGAAGAATGCTAATACTTGCCCAACTTGTAGGAAAAAGATC<br><br>181 N H K R Y H P I Y I -<br>541 AACCACAAACGGTACCACCCCATTTATATATGA | |
| Foe1263 | No homology | 1 TTCTTTACAACATGGTTTTAGTGGAGTAGATTTCTTAAGACAGGAAACATTTTCTTTTTT<br>61 TTCTTTACAACATGGTTTTAGTGGAGTAGATTTCTTAAGACAGGAAACATTTTCTTTTTT<br>121 AAAGGCATTGTTCCTTCCAGTCCATCTCGTTTCTTTATAAAGAAATAAAGTGACTTTATT<br>181 TTAAACATAGTTCCACTGTCATTTCTGAAAAATGTAATGCTCTTTAAGTCATAGCACCGG<br>241 TAAACAAAGTAAGTTATGTAAGGGGGAAATGGTTGCAGCAAACTTTTTAACTGATTTTGT<br>301 CAAACCAACTAATAGGTTACTGCTTCCCACACTACATTGGTTCAAAAACAAAAAAAAGCT<br>361 CGACAGCACTCAGGTCCTAAAGAAGAAATTTAGCTCATAATAAAGAACATCTGTTATGGA<br>421 ACTAAACAACTGAAATAGGCTTGGTTTTAAGAGTTCCGGTATCTAAGTCACAATTTGAAA<br>481 CATTTCTAATAAACAGTATCTCTTGGAGTTTCAGTACTGTCACAAAGTAGTGAATAAGCA<br>541 TGTGCAGGGGTAAATGTAAGGATTGTTAACCTATATAAACAGAATTTTAAATTTCAGTGT<br>601 GTTTTATTCTGATTTTTAAGACAAGAGGTGGTCTGTAATGGATTCAAATGTTTCATTTGT<br>661 AGTATAATGAAATGTTTACAGAAAGAACTTTTTCATTAAAATATTTTTAGAAAAAAAAAA<br>721 AAAAAGGCATTGTTCCTTCCAGTCCATCTCGTTTCTTTATAAAGAAATAAAGTGACTTTA<br>781 TTTTAAACATAGTTCCACTGTCATTTCTGAAAAATGTAATGCTCTTTAAGTCATAGCACC<br>841 GGTAAACAAAGTAAGTTATGTAAGGGGGAAATGGTTGCAGCAAACTTTTTAACTGATTTT<br>901 GTCAAACCAACTAATAGGTTACTGCTTCCCACACTACATTGGTTCAAAAACAAAAAAAAG<br>961 CTCGACAGCACTCAGGTCCTAAAGAAGAAATTTAGCTCATAATAAAGAACATCTGTTATG<br>1021 GAACTAAACAACTGAAATAGGCTTGGTTTTAAGAGTTCCGGTATCTAAGTCACAATTTGA<br>1081 AACATTTCTAATAAACAGTATCTCTTGGAGTTTCAGTACTGTCACAAAGTAGTGAATAAG<br>1141 CATGTGCAGGGGTAAATGTAAGGATTGTTAACCTATATAAACAGAATTTTAAATTTCAGT<br>1201 GTGTTTTATTCTGATTTTTAAGACAAGAGGTGGTCTGTAATGGATTCAAATGTTTCATTT<br>1261 GTAGTATAATGAAATGTTTACAGAAAGAACTTTTTCATTAAAATATTTTTAGAAAAAAAA<br>1321 AAAA | SEQ ID NO:314 |
| Foe1288 | No Homology | 1 GCTTTCCTTCAGGGCTGTCAAGACTGCCGTGTGCCCGAGGTATGCAGGTTTACTTCAGAA<br>61 AACACGAGATGGTACCCAACGCATCACCTTTGGAAGCCAGCGGCAATGCCATTCTGCTAC<br>121 TATCCACTGACTTCCCACAGATTTGTTATCCTCAACAGAGGATGTAACACCACCTTGCCA<br>181 TGGTTGTCCAAATCTGGATTTTAGACATTTCAATTAATTGAGACTGTGATTTGGTTCTTC<br>241 CTTCCCATCATGCATTCTGGCATCAAGTCCCTTTCAGTGTTCTGAAGTTAGACTGAAGTT<br>301 TCTTTGACCCAAGACTACAAAGGTGAGACCCTGAAGTAAAGACAAGGTACACGTACATTA<br>361 TCTGAGTAACCATTTCCTTTGTGGCCAATCTGTGTATGCTTTTAGAAGTTTACAAGATGC<br>421 TTTATTTTTGTCTGTAACAGTCTCTGTCATTCATTTCTGTCGATAAAGTATTGGGACAGA<br>481 ATGAGGAAGTTTGCCTTGTATCTCCTAGTGCTAACAACACACTCCAGTCACGAGCCGGGC<br>541 TTTACAAAATAAAGCACTTTGACGACTCAAAAAAAAAAAA | SEQ ID NO:315 |

| Gene Name | GenBank Homology | DNA SEQUENCE / DEDUCED AMINO ACID SEQUENCE | SEQUENCE IDENTIFIER: |
|---|---|---|---|
| Foe1317 | Human 3-hydroxyacyl-CoA dehydrogenase, isoform 2 mRNA. | 1 CGTGTATACCCGCTCAACGCTGGGACGTTACAGCCAGGGCCAATGGGCAGAGCGGGACTC<br>61 GAGGCCCCGCCCCCGCCTTGTGGCGTCACGGGGACGCCGGGGGCGCGGGCTGCAGGGC<br>121 CGCGTAGGTCCCCGCCCCCAGAGTCTGGCTTTCCGCGGCTGCCTGCCTCGCGCGTCTTCC<br>181 CTGCCCGGGTCTCCTCGCTGTCGCCGCCGCTGCCACACCATGGCCTTCGTCACCAGGCAG<br>241 TTCATGCGTTCCGTGTCCTCCTCGTCCACCGCCTCGGCCTCGGCCAAGAAGATAATCGTC<br>301 AAGCACGTGACGGTCATCGGCGGCGGGCTGATGGGCGCCGGCATTGCCCAGGTTGCTGCA<br>361 GCAACTGGTCACACAGTAGTGTTGGTAGACCAGACAGAGGACATCCTGGCAAAATCCAAA<br>421 AAGGGAATTGAGGAAAGCCTTAGGAAAGTGGCAAAGAAGAAGTTTGCAGAAAACCCTAAG<br>481 GCCGGCGATGAATTTGTGGAGAAGACCCTGAGCACCATAGCGACCAGCACGGATGCAGCC<br>541 TCCGTTGTCCACAGCACAGACTTGGTGGTGGAAGCCATCGTGGAGAATCTGAAGGTGAAA<br>601 AACGAGCTCTTCAAAAGGCTGGACAAGTTTGCTGCTGAACATACAATCTTTGCCAGCAAC<br>661 ACTTCCTCCTTGCAGATTACAAGCATAGCTAATGCCACCACCAGACAAGACCGATTCGCT<br>721 GGCCTCCATTTCTTCAACCCAGTGCCTGTCATGAAACTTGTGGAGGTCATTAAAACACCA<br>781 ATGACCAGCCAGAAGACATTTGAATCTTTGGTAGACTTTAGCAAAGCCCTAGGAAAGCAT<br>841 CCTGTTTCTTGCAAGGACACTCCTGGGTTTATTGTGAACCGCCTCCTGGTTCCATACCTC<br>901 ATGGAAGCAATCAGGCTGTATGAACGAGACTTCCAAACGTGTGGTGATTCTAACTCGGGT<br>961 TTGGGCTTTTCTTTAAAAGGTGACGCATCCAAAGAAGACATTGACACTGCTATGAAATTA<br>1021 GGAGCCGGTTACCCCATGGGCCCATTTGAGCTTCTAGATTATGTCGGACTGGATACTACG<br>1081 AAGTTCATCGTGGATGGGTGGCATGAAATGGATGCAGAGAACCCATTACATCAGCCCAGC<br>1141 CCATCCTTAAATAAGCTGGTAGCAGAGAACAAGTTCGGCAAGAAGACTGGAGAAGGATTT<br>1201 TACAAATACAAGTGATGTGCAGCTTCTCCGGCTCTGAGAAGAACACCTGAGAGCGCTTTC<br>1261 CAGCCAGTGCCCCGAGTGCCTGTGGAATGCTCTTTGGTCAGACATTCCCTCACACAGTA<br>1321 CAGTTTAATAAATGTGCATTTTGATTGTAATCTATCGAAGTGATTATTACACCAGTTACA<br>1381 GCAGTAATAGATTCTCCATTGAGAACTAATTCCCTTTTTTAGTCTGTTTGTTTCTGTGTA<br>1441 TTTTCTAAAAAGCTTTACACCCTTGGTGCCTTGGAGCAAATATTTCTTCTGAACCTTGTC<br>1501 ATTTTCGTGAATAATTGCCTAGATTCCTTCTCTCATCAACGGGAAAGTACTTCCTCTGAG<br>1561 AGTGCGAGTGCACCATGCTCACTGTTGCTGCGTGGGAGAGTCACAAGCCACTGGCAAGCA<br>1621 AGTGGTATAGTCTGTGAAGCACTGCAGCGAGCAGCACCTGGATCTTGCCTTTATAAGAAC<br>1681 ATTTTACTACCTGCAGCTTTGAGTCTTGCCCTACATTTT | SEQ ID NO:316 |
| | | 1 M G R A G L E A P P P P C G V T G T P G<br>1 ATGGGCAGAGCGGGACTCGAGGCCCCGCCCCCGCCTTGTGGCGTCACGGGGACGCCGGGG<br><br>21 A R G L Q G R V G P R P Q S L A F R G C<br>61 GCGCGCGGGCTGCAGGGCCGCGTAGGTCCCCGCCCCCAGAGTCTGGCTTTCCGCGGCTGC<br><br>41 L P R A S S L P G S P R C R R R C H T M<br>121 CTGCCTCGCGCGTCTTCCCTGCCCGGGTCTCCTCGCTGTCGCCGCCGCTGCCACACCATG<br><br>61 A F V T R Q F M R S V S S S S T A S A S<br>181 GCCTTCGTCACCAGGCAGTTCATGCGTTCCGTGTCCTCCTCGTCCACCGCCTCGGCCTCG | SEQ ID NO:317 |

| Gene Name | GenBank Homology | DNA SEQUENCE / DEDUCED AMINO ACID SEQUENCE | SEQUENCE IDENTIFIER: |
|---|---|---|---|
| | | 81 A K K I I V K H V T V I G G G L M G A G<br>241 GCCAAGAAGATAATCGTCAAGCACGTGACGGTCATCGGCGGCGGGCTGATGGGCGCCGGC<br><br>101 I A Q V A A A T G H T V V L V D Q T E D<br>301 ATTGCCCAGGTTGCTGCAGCAACTGGTCACACAGTAGTGTTGGTAGACCAGACAGAGGAC<br><br>121 I L A K S K K G I E E S L R K V A K K K<br>361 ATCCTGGCAAAATCCAAAAAGGGAATTGAGGAAAGCCTTAGGAAAGTGGCAAAGAAGAAG<br><br>141 F A E N P K A G D E F V E K T L S T I A<br>421 TTTGCAGAAAACCCTAAGGCCGGCGATGAATTTGTGGAGAAGACCCTGAGCACCATAGCG<br><br>161 T S T D A A S V V H S T D L V V E A I V<br>481 ACCAGCACGGATGCAGCCTCCGTTGTCCACAGCACAGACTTGGTGGTGGAAGCCATCGTG<br><br>181 E N L K V K N E L F K R L D K F A A E H<br>541 GAGAATCTGAAGGTGAAAAACGAGCTCTTCAAAAGGCTGGACAAGTTTGCTGCTGAACAT<br><br>201 T I F A S N T S S L Q I T S I A N A T T<br>601 ACAATCTTTGCCAGCAACACTTCCTCCTTGCAGATTACAAGCATAGCTAATGCCACCACC<br><br>221 R Q D R F A G L H F F N P V P V M K L V<br>661 AGACAAGACCGATTCGCTGGCCTCCATTTCTTCAACCCAGTGCCTGTCATGAAACTTGTG<br><br>241 E V I K T P M T S Q K T F E S L V D F S<br>721 GAGGTCATTAAAACACCAATGACCAGCCAGAAGACATTTGAATCTTTGGTAGACTTTAGC<br><br>261 K A L G K H P V S C K D T P G F I V N R<br>781 AAAGCCCTAGGAAAGCATCCTGTTTCTTGCAAGGACACTCCTGGGTTTATTGTGAACCGC<br><br>281 L L V P Y L M E A I R L Y E R D F Q T C<br>841 CTCCTGGTTCCATACCTCATGGAAGCAATCAGGCTGTATGAACGAGACTTCCAAACGTGT<br><br>301 G D S N S G L G F S L K G D A S K E D I<br>901 GGTGATTCTAACTCGGGTTTGGGCTTTTCTTTAAAAGGTGACGCATCCAAAGAAGACATT<br><br>321 D T A M K L G A G Y P M G P F E L L D Y<br>961 GACACTGCTATGAAATTAGGAGCCGGTTACCCCATGGGCCCATTTGAGCTTCTAGATTAT<br><br>341 V G L D T T K F I V D G W H E M D A E N<br>1021 GTCGGACTGGATACTACGAAGTTCATCGTGGATGGGTGGCATGAAATGGATGCAGAGAAC | |

| Gene Name | GenBank Homology | DNA SEQUENCE / DEDUCED AMINO ACID SEQUENCE | SEQUENCE IDENTIFIER: |
|---|---|---|---|
| | | 361 P L H Q P S P S L N K L V A E N K F G K<br>1081 CCATTACATCAGCCCAGCCCATCCTTAAATAAGCTGGTAGCAGAGAACAAGTTCGGCAAG<br><br>381 K T G E G F Y K Y K -<br>1141 AAGACTGGAGAAGGATTTTACAAATACAAGTGA | |
| Foe1324 | Homo sapiens mRNA; cDNA DKFZp434I0612 (from clone DKFZp434I0612). | 1 GTAATTGTTAACTAATGTGCATTTTAAAATTCTCATTTGTCTTATGTACTGAGCCCTTAT<br>61 ACCAGTGCTAATTTATGTGACTCCTTTCTCCTGCAGCTAAGAGAAAAATACCTTTTTAAT<br>121 TCATTTATAGTACCCAGTTTTTAAAGAAGATTTATTTTGTAAAATTTTGCTTATGGTACA<br>181 TGTCATCTTAGCCTGTAAATAAATTAAAGCATTAATTTTTATCCCTCCCTGGTCTTTTCC<br>241 TCCTTCTGACTTTATACGTCTTTCTAGAGAGCTTATCTTCTATAATAACAATTCTTTGTT<br>301 TTAAAGTGAGAAAGATCAGTCTAAAGAAAAGGAGAAGAAAGTGAAAAAAACAATTCCTTC<br>361 CTGGGCTACCCTTTCTGCCAGCCAGCTAGCCAGGGCCCAGAAACAAACACCGATGGCTTC<br>421 TTCCCCACGTCCCAAGATGGATGCAATCTTAACTGAGGCCATTAAGGCATGCTTCCAGAA<br>481 GAGTGGTGCATCAGTGGTTGCTATTCGAAAATACATCATCCATAAGTATCCTTCTCTGGA<br>541 GCTGGAGAGAAGGGGTTATCTCCTTAAACAAGCACTGAAAAGAGAATTAAATAGAGGAGT<br>601 CATCAAACAGGTTAAAGGAAAAGGTGCTTCTGGAAGTTTTGTTGTGGTTCAGAAATCAAG<br>661 AAAAACACCTCAGAAATCCAGAAACAGAAAGAATAGGAGCTCTGCAGTGGATCCAGAACC<br>721 ACAAGTAAAATTGGAGGATGTCCTCCCACTGGCCTTTACTCGCCTTTGTGAACCTAAAGA<br>781 AGCTTCCTACAGTCTCATCAGGAAATATGTGTCTCAGTATTATCCTAAGCTTAGAGTGGA<br>841 CATCAGGCCTCAGCTGTTGAAGAACGCTCTGCAGAGAGCAGTAGAGAGGGGCCAGTTAGA<br>901 ACAGATAACTGGCAAAGGTGCTTCGGGGACATTCCAGCTGAAGAAATCAGGGGAGAAACC<br>961 CCTGCTTGGTGGAAGCCTGATGGAATATGCAATCTTGTCTGCCATTGCTGCCATGAATGA<br>1021 GCCGAAGACCTGCTCTACCACTGCTCTGAAGAAGTATGTCCTAGAGAATCACCCAGGAAC<br>1081 CAATTCTAACTATCAAATGCATTTGCTGAAAAAAACCCTGCAGAAATGCGAAAAGAATGG<br>1141 GTGGATGGAACAGATCTCTGGGAAAGGGTTCAGTGGCACCTTCCAGCTCTGTTTTCCCTA<br>1201 TTATCCCAGCCCAGGAGTTCTGTTTCCGAAGAAAGAGCCAGATGATTCTAGAGATGAGGA<br>1261 TGAAGATGAAGATGAGTCATCAGAAGAAGACTCTGAGGATGAAGAGCCGCCACCTAAGAG<br>1321 AAGGTTGCAGAAGAAAACCCCAGCCAAGTCCCCAGGGAAGGCCGCATCTGTGAAGCAGAG<br>1381 AGGGTCCAAACCTGCACCTAAAGTCTCAGCTGCCCAGCGGGGGAAAGCTAGGCCCTTGCC<br>1441 TAAGAAAGCACCTCCTAAGGCCAAAACGCCTGCCAAGAAGACCAGACCCTCATCCACAGT<br>1501 CATCAAGAAACCTAGTGGTGGCTCCTCAAAGAAGCCTGCAACCAGTGCAAGAAAGGAAGT<br>1561 AAAATTGCCGGGCAAGGGCAAATCCACCATGAAGAAGTCTTTCAGAGTGAAAAAGTAAAT<br>1621 TTTATAGGAAAAAAGGGTATCATGATGAAATTCAAAATCTTATTTTCTAAGGTCAGTGTG<br>1681 CATTTGTTTAGTTTTGATGCTTTTCAAATTACATTATTTTCCTCCCCTATGAACATTGTG<br>1741 GGGAGGGACTCTAAATAAACCAGTTTAGGCATTTGCTAGCTTTAGGTGCTTTTATTGGTG<br>1801 CCTGCCCTTTTCCTTGTTCATTTTAATTTCTGCAATAATCCTGGACTTTCCTAAACTATG<br>1861 TAATGTATACTTGTCCTTTTTCTCTGCCTCCCCCAACCCCCTGTTGTTTTTATGGTCAGC<br>1921 TTTGCCTTTTTTTTTTCTTCCAATTTTATCTAAACAGTTGCAGAGATTTTTATATTTGTA<br>1981 GAAAGCATCAAGAACGGTATGCCAGTCAGGTCCTGGAAGTAAAATGGAGGCACAATATAG<br>2041 CACTGACTGAGTTGTAAAGCCTCCTGCCTGGAGACTTCAGTTATAGCTGTAATAATTAAT<br>2101 CTTATTTATAAAAGCCACTCCACTAACCTTTTCTCTCCAACTGTAAACACAGAGACAGCT | SEQ ID NO:318 |

| Gene Name | GenBank Homology | DNA SEQUENCE / DEDUCED AMINO ACID SEQUENCE | SEQUENCE IDENTIFIER: |
|---|---|---|---|
| | | 2161 TTGGGAATAAGCCAAAAACAGGGTGATCTCATTAGATTTTGAAGATATATGACTCCTTTG<br>2221 GGCTACATTTCATATTGATCAATTTCTAGGTATTTTTCACTGGCCCAAAGTATTGCATTC<br>2281 CCTTAACAGCAAGCACAAGTTCTCTATATCACTTGTTTTTTGTTGTTGTTGTTGTTGTCG<br>2341 TCGTTGTTTTGAGACGGAGTCTTGCTCAGGTGCCCCGGAGTGCAGTGGTGCAATCTCAGC<br>2401 TCACTGCAACCTCCACCTCCTGGGTTCAAGCAATTCTCCTGCTTCAGCCTCCCGAGTAGC<br>2461 TGGGATTACAGGTGTGTACCACCACGCCTGGCAATTTTTTTGTATTTTTAGTAGAGATGG<br>2521 GGTTTCGCCGTGTTGGTCAGGCTGGTCTCGAACTCCTGACCTCAGGTGATCCGCCTGCCT<br>2581 CGGCCTCCCAAAGTGCTGGGATTACAGGAGTGAGCCACTGTGCCTGGCCTATCCCACTTG<br>2641 GTTTTTGACTGAAGGGGAAGTGTAGAAATATATTGATTTGTGATTTCTGGTGTCACCTGT<br>2701 GTTACCAAAAATCAAAACAAATCTTTTTTATTTTTTATTATTATTATTATTTTTGAGACA<br>2761 GAGTCTCGCTCTGTCGCCCAGTGTGGAGTGCAGTGGTGTGATCTTGGCTCACTGCAAACT<br>2821 CCGCCTCCCAGGTTCAAGCGATTCTCCCACCTCAGCCTCCTGAGTTGGGTCCTACAGGCG<br>2881 CACACGACCACGCCCAGCTAATTTTTTGTATTTTTAGTAGAGTTGGGGTTTCACCATGTT<br>2941 AGCCAGGATGGTCTCGATCTCCTGACCTCGTGATCCACTCACCTCAGCCTCCCAAAATCC<br>3001 TGGGGTTACAGATGTGAGCTACCACTCACGGCCCAAATCTTCTTGATCATATGTTTAAAT<br>3061 ATATTTTTTAATATTTGGAGCATGAGTTGTCACTTCTTGTTTGCCTTTTTTATAAGGAAA<br>3121 TGTTGGAGAGTTAAATCATTGCTAATGTAGAAATGTTAAGTGGAAAAACATACAATTTGC<br>3181 TAAAATAAACTAGATTCCAGATTCATCTCTGGGTTTTTTTCCCCTCCTTTCTTTCCACAG<br>3241 CACTTTTGATATCAAACAAGTGGCTTCCTTTTTGAGATATTAAAAAAAAAAAGAAAAGGA<br>3301 AAAAAGTAAATGAAGCCCAACTACCTAACCCTTTCTTATTTGTATTTGTTTTAGTATTGT<br>3361 GAGGTTGTGTTAAATAGTACTAGCTAGAAATACAAATTTCTGGTTATCATTTATCCTCCC<br>3421 TGTGGCACTTGACATTTTAATTGTCTTAAACCTTTTGGTGTACATCCTCTGGCCCCTTAA<br>3481 GTGCTGCCAGAAGCAGAAGATATCTGTCTCATTCATTCCACTTGCACTGTCTCCTGGGAT<br>3541 CTTGTCTGCAGCCCGACGTGTGGCTGGGAAATGGACTCGAGAAGATTGGCTTCAATTAGA<br>3601 TCCATAATCATGTGTGATCCCATCATGAGTTCATTGGAATTTGTGTTGCATGTAAGGCAA<br>3661 TCTTTCCTGTTGTAAATCTTCCTTTTTTTATGTACATATATTTTGAAAAATATGAATAAA<br>3721 CATGAAATTTTAAAAAAAAAAAAAAAAAAA<br><br>1 M A S S P R P K M D A I L T E A I K A C<br>1 ATGGCTTCTTCCCCACGTCCCAAGATGGATGCAATCTTAACTGAGGCCATTAAGGCATGC<br><br>21 F Q K S G A S V V A I R K Y I I H K Y P<br>61 TTCCAGAAGAGTGGTGCATCAGTGGTTGCTATTCGAAAATACATCATCCATAAGTATCCT<br><br>41 S L E L E R R G Y L L K Q A L K R E L N<br>121 TCTCTGGAGCTGGAGAGAAGGGGTTATCTCCTTAAACAAGCACTGAAAAGAGAATTAAAT<br><br>61 R G V I K Q V K G K G A S G S F V V V Q<br>181 AGAGGAGTCATCAAACAGGTTAAAGGAAAAGGTGCTTCTGGAAGTTTTGTTGTGGTTCAG<br><br>81 K S R K T P Q K S R N R K N R S S A V D<br>241 AAATCAAGAAAAACACCTCAGAAATCCAGAAACAGAAAGAATAGGAGCTCTGCAGTGGAT | SEQ ID NO:319 |

| Gene Name | GenBank Homology | DNA SEQUENCE / DEDUCED AMINO ACID SEQUENCE | SEQUENCE IDENTIFIER: |
|---|---|---|---|
| | | 101 P E P Q V K L E D V L P L A F T R L C E<br>301 CCAGAACCACAAGTAAAATTGGAGGATGTCCTCCCACTGGCCTTTACTCGCCTTTGTGAA<br><br>121 P K E A S Y S L I R K Y V S Q Y Y P K L<br>361 CCTAAAGAAGCTTCCTACAGTCTCATCAGGAAATATGTGTCTCAGTATTATCCTAAGCTT<br><br>141 R V D I R P Q L L K N A L Q R A V E R G<br>421 AGAGTGGACATCAGGCCTCAGCTGTTGAAGAACGCTCTGCAGAGAGCAGTAGAGAGGGGC<br><br>161 Q L E Q I T G K G A S G T F Q L K K S G<br>481 CAGTTAGAACAGATAACTGGCAAAGGTGCTTCGGGGACATTCCAGCTGAAGAAATCAGGG<br><br>181 E K P L L G G S L M E Y A I L S A I A A<br>541 GAGAAACCCCTGCTTGGTGGAAGCCTGATGGAATATGCAATCTTGTCTGCCATTGCTGCC<br><br>201 M N E P K T C S T T A L K K Y V L E N H<br>601 ATGAATGAGCCGAAGACCTGCTCTACCACTGCTCTGAAGAAGTATGTCCTAGAGAATCAC<br><br>221 P G T N S N Y Q M H L L K K T L Q K C E<br>661 CCAGGAACCAATTCTAACTATCAAATGCATTTGCTGAAAAAAACCCTGCAGAAATGCGAA<br><br>241 K N G W M E Q I S G K G F S G T F Q L C<br>721 AAGAATGGGTGGATGGAACAGATCTCTGGGAAAGGGTTCAGTGGCACCTTCCAGCTCTGT<br><br>261 F P Y Y P S P G V L F P K K E P D D S R<br>781 TTTCCCTATTATCCCAGCCCAGGAGTTCTGTTTCCGAAGAAAGAGCCAGATGATTCTAGA<br><br>281 D E D E D E D E S S E E D S E D E E P P<br>841 GATGAGGATGAAGATGAAGATGAGTCATCAGAAGAAGACTCTGAGGATGAAGAGCCGCCA<br><br>301 P K R R L Q K K T P A K S P G K A A S V<br>901 CCTAAGAGAAGGTTGCAGAAGAAAACCCCAGCCAAGTCCCCAGGGAAGGCCGCATCTGTG<br><br>321 K Q R G S K P A P K V S A A Q R G K A R<br>961 AAGCAGAGAGGGTCCAAACCTGCACCTAAAGTCTCAGCTGCCCAGCGGGGGAAAGCTAGG<br><br>341 P L P K K A P P K A K T P A K K T R P S<br>1021 CCCTTGCCTAAGAAAGCACCTCCTAAGGCCAAAACGCCTGCCAAGAAGACCAGACCCTCA<br><br>361 S T V I K K P S G G S S K K P A T S A R<br>1081 TCCACAGTCATCAAGAAACCTAGTGGTGGCTCCTCAAAGAAGCCTGCAACCAGTGCAAGA<br><br>381 K E V K L P G K G K S T M K K S F R V K | |

| Gene Name | GenBank Homology | DNA SEQUENCE / DEDUCED AMINO ACID SEQUENCE | SEQUENCE IDENTIFIER: |
|---|---|---|---|
| | | 1141 AAGGAAGTAAAATTGCCGGGCAAGGGCAAATCCACCATGAAGAAGTCTTTCAGAGTGAAA<br><br>401 K -<br>1201 AAGTAA | |
| Foe146 | No homology | 1 TTATTTATTTAGGATATAAATAGCAGAAAACCAATTACTATGTCAGAGTATGGACACTTG<br>61 ATACATAATGCCAAATTATTCTTCATAAGAGCTGTTGCCAAATCAGTGATAATATTCATT<br>121 TCACTGTACTCTTGCCAGCCATCACTCCAGTGATGGTTGTGGTGGTGGTTGTCGTCATTT<br>181 GGGGGCAGGTGCCTAATGTGCGGTCTTTAATGAATATCTAATGAATGTTTGAAAAAAATG<br>241 ATTATTCTCTGTTTATCAGGTACAAAGGTATTTATGGTACATGTATATGGTATGTGTATA<br>301 TGACTTGTCAAATTACTATTAACATTTTCTATAGCCTTAGATAATGCATGAAAAAGCATA<br>361 GTTCAGTGCCACTCACATAAGAAGTGCCCAATAAGTGTTAACTATTATTTTTGTCTACTT<br>421 GGTTTTACCACCTTCTAAAAGTATGTTGAAGTGTCTCTCTGTCATTGTGGATTTGTCAGT<br>481 TTCTCTCTGCATTTCTCAGAGTACTTGTTTTATATATTTTGAAGCTATTGTTAGATGATA<br>541 GATTCATGATTTGTAGTGGAATGTTCTTTTATCAATTTTTGAAAATTGCCCTTGTCTCAT<br>601 AATGCTTTTCATATTGAATTCTATTTTGTCTGCTATTAAATATTTCCATACCTGCAAAAA<br>661 AAAAA | SEQ ID NO:320 |
| Foe148 | Homo sapiens fucosyltransferase 8 (alpha (1,6) fucosyltransferase ) (FUT8), transcript variant 1 | 1 cgtttagtac agaaatctca tgggagagag catccatgca tttacaaatt gttattgaat<br>61 tattttattg aatgatgaca cccaaactga gctagaacat aattctggct ctgctagtac<br>121 atcttctgtg tgatcttgga caagtcactc tactttcctt tcaattttct tttctcacag<br>181 ggagataatc ataaaaacga ctgtaaagta cagcacttca tagagtgctt tttgtttaaa<br>241 gagctgacaa taaatacgag tctcaaggtc taggaaagcc tccctcacaa cctgagctgc<br>301 ttgaggacaa gggattttct tttgaatcag cagtacctta tttgtgtatc tgtgatagag<br>361 ttcctggtac ataagaaggt ctcaataaat atgtgaattt atgaatatta ggcagattgc<br>421 aaccttgaca ggccactgcc tcttaaatct cctttctgtg atcttttaat atttaacatc<br>481 taaaaggccg ccgctacttg ctttgggata agtatccccg gtatgtactt taaaatgccc<br>541 aagcctagag aaatgattct tgtcttaagg gcaccatttc gctctcccac cgtaaagcgc<br>601 cccaggcttg ggatctgggt cccaaggcta cagggaagag tttggaacgg gaagctcatc<br>661 ttccggccct ctgattggcc ggctcgcact ccactcacgc ggcgcgcagc tctgattggc<br>721 ctcggcggca cccctcgtcc cgcgactact ttgtgtgctg gggcggcgcg ctccggtcct<br>781 cccgctcagc tggcggtctg ggctgctctg gggcagccct tcggtccact gctctgcatc<br>841 gcgggcgccg ggaatttccc gagtccgagc gggttgctgc ttttgctcag aggacatcca<br>901 tgaccctaat ggtctttttg ttcaagataa agtgattttt tgcctttgtt gattaactgg<br>961 acaaattcag catgtagagc gcatgaagta caggacaata aagcttccta cacatatcac<br>1021 caggaggatc tctttgaaag attcactgca ggactaccag agagaataat ttgtctgaag<br>1081 catcatgtgt tgaaacaaca gaagtctatt cacctgtgca ctaactagaa acagagttac<br>1141 aatgttttca attctttgag ctccaggact ccagggaagt gagttgaaaa tctgaaaatg<br>1201 cggccatgga ctggttcctg gcgttggatt atgctcattc tttttgcctg ggggaccttg<br>1261 ctgttttata taggtggtca cttggtacga gataatgacc atcctgatca ctctagccga<br>1321 gaactgtcca agattctggc aaagcttgaa cgcttaaaac aacagaatga agacttgagg | SEQ ID NO:321 |

<table>
<tr><th>Gene Name</th><th>GenBank Homology</th><th>DNA SEQUENCE / DEDUCED AMINO ACID SEQUENCE</th><th>SEQUENCE IDENTIFIER:</th></tr>
<tr><td></td><td></td><td>1381 cgaatggccg aatctctccg gataccagaa ggccctattg atcaggggcc agctatagga<br>1441 agagtacgcg ttttagaaga gcagcttgtt aaggccaaag aacagattga aaattacaag<br>1501 aaacagacca gaaatggtct ggggaaggat catgaaatcc tgaggaggag gattgaaaat<br>1561 ggagctaaag agctctggtt tttcctacag agtgaattga agaaattaaa gaacttagaa<br>1621 ggaaatgaac tccaaagaca tgcagatgaa tttcttttgg atttaggaca tcatgaaagg<br>1681 tctataatga cggatctata ctacctcagt cagacagatg gagcaggtga ttggcgggaa<br>1741 aaagaggcca aagatctgac agaactggtt cagcggagaa taacatatct tcagaatccc<br>1801 aaggactgca gcaaagccaa aaagctggtg tgtaatatca acaaaggctg tggctatggc<br>1861 tgtcagctcc atcatgtggt ctactgcttc atgattgcat atggcaccca gcgaacactc<br>1921 atcttggaat ctcagaattg gcgctatgct actggtggat gggagactgt atttaggcct<br>1981 gtaagtgaga catgcacaga cagatctggc atctccactg gacactggtc aggtgaagtg<br>2041 aaggacaaaa atgttcaagt ggtcgagctt cccattgtag acagtcttca tccccgtcct<br>2101 ccatatttac ccttggctgt accagaagac ctcgcagatc gacttgtacg agtgcatggt<br>2161 gaccctgcag tgtggtgggt gtctcagttt gtcaaatact tgatccgccc acagccttgg<br>2221 ctagaaaaag aaatagaaga agccaccaag aagcttggct tcaaacatcc agttattgga<br>2281 gtccatgtca gacgcacaga caaagtggga acagaagctg ccttccatcc cattgaagag<br>2341 tacatggtgc atgttgaaga acattttcag cttcttgcac gcagaatgca agtggacaaa<br>2401 aaaagagtgt atttggccac agatgaccct tctttattaa aggaggcaaa aacaaagtac<br>2461 cccaattatg aatttattag tgataactct atttcctggt cagctggact gcacaatcga<br>2521 tacacagaaa attcacttcg tggagtgatc ctggatatac atttctctc tcaggcagac<br>2581 ttcctagtgt gtacttttc atcccaggtc tgtcgagttg cttatgaaat tatgcaaaca<br>2641 ctacatcctg atgcctctgc aaacttccat tctttagatg acatctacta ttttgggggc<br>2701 cagaatgccc acaatcaaat tgccatttat gctcaccaac cccgaactgc agatgaaatt<br>2761 cccatggaac ctggagatat cattggtgtg gctggaaatc attgggatgg ctattctaaa<br>2821 ggtgtcaaca ggaaattggg aaggacgggc ctatatccct cctacaaagt tcgagagaag<br>2881 atagaaacgg tcaagtaccc cacatatcct gaggctgaga aataaagctc agatggaaga<br>2941 gataaacgac caaactcagt tcgaccaaac tcagttcaaa ccatttcagc caaactgtag<br>3001 atgaagaggg ctctgatcta acaaaataag gttatatgag tagatactct cagcaccaag<br>3061 agcagctggg aactgacata ggcttcaatt ggtggaattc ctctttaaca agggctgcaa<br>3121 tgccctcata cccatgcaca gtacaataat gtactcacat ataacatgca aacaggttgt<br>3181 tttctacttt gcccctttca gtatgtcccc ataagacaaa cactgccata ttgtgtaatt<br>3241 taagtgacac agacattttg tgtgagactt aaaacatggt gcctatatct gagagacctg<br>3301 tgtgaactat tgagaagatc ggaacagctc cttactctga ggaagttgat tcttatttga<br>3361 tggtggtatt gtgaccactg aattcactcc agtcaacaga ttcagaatga gaatggacgt<br>3421 ttggtttttt tttgttttg tttttgtttt ttcctttata aggttgtctg ttttttttt<br>3481 tttaaataat tgcatcagtt cattgacctc atcattaata agtgaagaat acatcagaaa<br>3541 ataaaatatt cactctccat tagaaaattt tgtaaaacaa tgccatgaac aaattcttta<br>3601 gtactcaatg tttctggaca ttctctttga taacaaaaaa taaattttaa aaaggaattt<br>3661 tgtaaagttt ctagaatttt atatcattgg atgatatgtt gatcagcctt atgtggaaga<br>3721 actgtgataa aaagaggagc tttttagttt ttcagcttaa aaaaaaaaaa aaaaa</td><td></td></tr>
<tr><td></td><td></td><td>1 M R P W T G S W R W I M L I L F A W G T<br>1 ATGCGGCCATGGACTGGTTCCTGGCGTTGGATTATGCTCATTCTTTTTGCCTGGGGGACC</td><td>SEQ ID NO:322</td></tr>
</table>

| Gene Name | GenBank Homology | DNA SEQUENCE / DEDUCED AMINO ACID SEQUENCE | SEQUENCE IDENTIFIER: |
|---|---|---|---|
| | | 21 L L F Y I G G H L V R D N D H P D H S S<br>61 TTGCTGTTTTATATAGGTGGTCACTTGGTACGAGATAATGACCATCCTGATCACTCTAGC<br><br>41 R E L S K I L A K L E R L K Q Q N E D L<br>121 CGAGAACTGTCCAAGATTCTGGCAAAGCTTGAACGCTTAAAACAACAGAATGAAGACTTG<br><br>61 R R M A E S L R I P E G P I D Q G P A I<br>181 AGGCGAATGGCCGAATCTCTCCGGATACCAGAAGGCCCTATTGATCAGGGGCCAGCTATA<br><br>81 G R V R V L E E Q L V K A K E Q I E N Y<br>241 GGAAGAGTACGCGTTTTAGAAGAGCAGCTTGTTAAGGCCAAAGAACAGATTGAAAATTAC<br><br>101 K K Q T R N G L G K D H E I L R R R I E<br>301 AAGAAACAGACCAGAAATGGTCTGGGGAAGGATCATGAAATCCTGAGGAGGAGGATTGAA<br><br>121 N G A K E L W F F L Q S E L K K L K N L<br>361 AATGGAGCTAAAGAGCTCTGGTTTTTCCTACAGAGTGAATTGAAGAAATTAAAGAACTTA<br><br>141 E G N E L Q R H A D E F L L D L G H H E<br>421 GAAGGAAATGAACTCCAAAGACATGCAGATGAATTTCTTTTGGATTTAGGACATCATGAA<br><br>161 R S I M T D L Y Y L S Q T D G A G D W R<br>481 AGGTCTATAATGACGGATCTATACTACCTCAGTCAGACAGATGGAGCAGGTGATTGGCGG<br><br>181 E K E A K D L T E L V Q R R I T Y L Q N<br>541 GAAAAAGAGGCCAAAGATCTGACAGAACTGGTTCAGCGGAGAATAACATATCTTCAGAAT<br><br>201 P K D C S K A K K L V C N I N K G C G Y<br>601 CCCAAGGACTGCAGCAAAGCCAAAAAGCTGGTGTGTAATATCAACAAAGGCTGTGGCTAT<br><br>221 G C Q L H H V V Y C F M I A Y G T Q R T<br>661 GGCTGTCAGCTCCATCATGTGGTCTACTGCTTCATGATTGCATATGGCACCCAGCGAACA<br><br>241 L I L E S Q N W R Y A T G G W E T V F R<br>721 CTCATCTTGGAATCTCAGAATTGGCGCTATGCTACTGGTGGATGGGAGACTGTATTTAGG<br><br>261 P V S E T C T D R S G I S T G H W S G E<br>781 CCTGTAAGTGAGACATGCACAGACAGATCTGGCATCTCCACTGGACACTGGTCAGGTGAA<br><br>281 V K D K N V Q V V E L P I V D S L H P R<br>841 GTGAAGGACAAAAATGTTCAAGTGGTCGAGCTTCCCATTGTAGACAGTCTTCATCCCCGT | |

| Gene Name | GenBank Homology | DNA SEQUENCE / DEDUCED AMINO ACID SEQUENCE | SEQUENCE IDENTIFIER: |
|---|---|---|---|
| | | 301 P P Y L P L A V P E D L A D R L V R V H<br>901 CCTCCATATTTACCCTTGGCTGTACCAGAAGACCTCGCAGATCGACTTGTACGAGTGCAT<br><br>321 G D P A V W W V S Q F V K Y L I R P Q P<br>961 GGTGACCCTGCAGTGTGGTGGGTGTCTCAGTTTGTCAAATACTTGATCCGCCCACAGCCT<br><br>341 W L E K E I E E A T K K L G F K H P V I<br>1021 TGGCTAGAAAAAGAAATAGAAGAAGCCACCAAGAAGCTTGGCTTCAAACATCCAGTTATT<br><br>361 G V H V R R T D K V G T E A A F H P I E<br>1081 GGAGTCCATGTCAGACGCACAGACAAAGTGGGAACAGAAGCTGCCTTCCATCCCATTGAA<br><br>381 E Y M V H V E E H F Q L L A R R M Q V D<br>1141 GAGTACATGGTGCATGTTGAAGAACATTTTCAGCTTCTTGCACGCAGAATGCAAGTGGAC<br><br>401 K K R V Y L A T D D P S L L K E A K T K<br>1201 AAAAAAAGAGTGTATTTGGCCACAGATGACCCTTCTTTATTAAAGGAGGCAAAAACAAAG<br><br>421 Y P N Y E F I S D N S I S W S A G L H N<br>1261 TACCCCAATTATGAATTTATTAGTGATAACTCTATTTCCTGGTCAGCTGGACTGCACAAT<br><br>441 R Y T E N S L R G V I L D I H F L S Q A<br>1321 CGATACACAGAAAATTCACTTCGTGGAGTGATCCTGGATATACATTTTCTCTCTCAGGCA<br><br>461 D F L V C T F S S Q V C R V A Y E I M Q<br>1381 GACTTCCTAGTGTGTACTTTTTCATCCCAGGTCTGTCGAGTTGCTTATGAAATTATGCAA<br><br>481 T L H P D A S A N F H S L D D I Y Y F G<br>1441 ACACTACATCCTGATGCCTCTGCAAACTTCCATTCTTTAGATGACATCTACTATTTTGGG<br><br>501 G Q N A H N Q I A I Y A H Q P R T A D E<br>1501 GGCCAGAATGCCCACAATCAAATTGCCATTTATGCTCACCAACCCCGAACTGCAGATGAA<br><br>521 I P M E P G D I I G V A G N H W D G Y S<br>1561 ATTCCCATGGAACCTGGAGATATCATTGGTGTGGCTGGAAATCATTGGGATGGCTATTCT<br><br>541 K G V N R K L G R T G L Y P S Y K V R E<br>1621 AAAGGTGTCAACAGGAAATTGGGAAGGACGGGCCTATATCCCTCCTACAAAGTTCGAGAG<br><br>561 K I E T V K Y P T Y P E A E K -<br>1681 AAGATAGAAACGGTCAAGTACCCCACATATCCTGAGGCTGAGAAATAA | |
| Foe443 | Homo sapiens | | |

| Gene Name | GenBank Homology | DNA SEQUENCE / DEDUCED AMINO ACID SEQUENCE | SEQUENCE IDENTIFIER: |
|---|---|---|---|
| | tripeptidyl peptidase II mRNA | 1 gaattcccct ccatcctgcg tccatggcca ccgctgcgac tgaggagccc ttccctttc<br>61 acggtctcct gccgaagaag gagaccggag ccgcctcctt cctctgccgc tacccggagt<br>121 atgatgggcg gggggtgctc atcgcagtcc tggacacggg ggtcgacccg ggggctccgg<br>181 gcatgcaggt tacaactgat ggaaaaccaa aaatcgttga tatcattgat acaacaggaa<br>241 gtggcgatgt gaatactgct acagaagtag agccaaagga tggtgagatt gttggccttt<br>301 caggaagagt gcttaagatt cctgcaagct ggacaaatcc ctcaggcaaa tatcatattg<br>361 gcataaaaaa tggctatgac ttctatccta aggcactcaa ggaaaggata cagaaagaac<br>421 ggaaggaaaa aatctgggac cctgttcaca gagtggccct tgcagaagcc tgtagaaaac<br>481 aggaagaatt tgatgttgcc aacaacggct cttctcaagc aaataaacta atcaaggagg<br>541 aacttcaaag tcaagtggaa ttgctaaatt cttttgagaa gaaatacagc gatcctggcc<br>601 ctgtatatga ctgcttggta tggcatgatg gcgaagtctg gagagcctgc attgattcta<br>661 atgaagatgg ggacttgagt aaatctaccg tgttgagaaa ctacaaagaa gcccaagaat<br>721 atggctcttt tggcacagct gagatgttga attactccgt taatatatac gatgatagaa<br>781 acctgctctc cattgtgacc agtggaggag ctcatgggac acatgtagct agtatagctg<br>841 ctggacactt tccagaagaa cctgaacgga atggggtagc tcctggtgct caaattcttt<br>901 ccatcaagat tggtgataca agactaagca caatggaaac aggcacaggc ctcataagag<br>961 ctatgataga agttataaat cataagtgtg atcttgtcaa ctacagttac ggagaagcaa<br>1021 ctcactggcc aaattctggg agaatttgtg aagtaattaa tgaagcagta tggaagcata<br>1081 atataattta tgtttcaagt gctggaaata atggtccatg cctgtctaca gttggttgtc<br>1141 caggtggaac tacatcaagt gtgataggtg ttggtgctta tgtttctcct gatatgatgg<br>1201 ttgctgagta ttcactgaga gagaaattac ctgcaaatca atatacttgg tcttctagag<br>1261 gacctagtgc tgacggggcc cttggtgtga gtatcagtgc gccaggagga gccattgctt<br>1321 ctgttcctaa ctggacactg agagggacgc agctgatgaa tggaacatct atgtcttccc<br>1381 ccaatgcatg tggaggcatt gccctgatcc tttcaggtct gaaagctaat aacattgact<br>1441 acacagttca ttcagtcaga agagctctag aaaacactgc agtgaaggct gacaatatag<br>1501 aagtatttgc tcaaggacat ggtattattc aggttgataa agcctatgac tacctcgttc<br>1561 agaatacatc atttgctaat aaattaggtt ttactgttac tgttggaaat aaccgtggca<br>1621 tctacctccg agatcctgtt caggtggctg caccttcaga tcatggcgtt ggcattgaac<br>1681 ctgtatttcc ggagaacaca gaaaactctg aaaaaatatc ccttcagctt catttagctc<br>1741 tgacttcaaa ttcatcttgg gttcagtgtc ccagccattt ggaactcatg aatcaatgta<br>1801 gacacataaa catacgtgtg gatcccaggg gcttaagaga aggattgcat tatacagagg<br>1861 tatgtggcta tgatatagca tcccctaacg caggtccgct cttcagagtt ccgatcactg<br>1921 cagttatagc agcaaaagta aatgaatcat cacattatga tctagccttt acagatgtac<br>1981 actttaaacc tggtcaaatt cgaaggcatt ttattgaggt tcctgagggt gcaacatggg<br>2041 ctgaagtgac agtgtgttcg tgttcttctg aggtgtcagc aaagtttgtt ctacatgcag<br>2101 tccagcttgt gaagcaaaga gcatatcgaa gccatgaatt ctataagttt tgttctcttc<br>2161 cagagaaagg aacactgact gaagcttttc ctgtcctagg tggaaaagca attgaatttt<br>2221 gcattgctcg ttggtgggca agtctcagtg atgtcaacat tgattatacc atttctttcc<br>2281 atgggatagt gtgtactgct cctcagttaa acattcatgc atcggaagga atcaaccgct<br>2341 ttgatgttca gtcctccttg aaatacgaag atctggctcc ctgcataact ttgaagaact<br>2401 gggtccaaac actgcgccca gtgagtgcaa aaacaaaacc tttaggatca agagatgttt<br>2461 tgccaaataa ccgtcaactt tatgagatgg tcctgacata taactttcat caacccaaga<br>2521 gtggggaagt aactccaagc tgcccactac tttgtgaact attatatgaa tctgaatttg | SEQ ID NO:323 |

| Gene Name | GenBank Homology | DNA SEQUENCE / DEDUCED AMINO ACID SEQUENCE | SEQUENCE IDENTIFIER: |
|---|---|---|---|
| | | 2581 acagccaact gtggattatt tttgaccaga acaaaagaca gatgggttca ggcgatgcct<br>2641 atccacatca gtattctttg aaactggaga aaggagatta tacaattcga ctacagattc<br>2701 gccatgagca aatcagtgat ttggaacgcc ttaaagacct tccatttatt gtttctcata<br>2761 gattgtctaa taccttgagc ttagatattc atgaaaatca tagttttgca cttctaggga<br>2821 agaagaaatc aagcaatttg acattaccac ccaaatataa ccagccattc tttgttactt<br>2881 ccttacctga tgataaaata cctaaagggg caggacctgg atgctatctt gcaggatcct<br>2941 taacattgtc aaagactgaa ctaggaaaga aagctgatgt aatccctgtt cattactact<br>3001 taatacctcc accaacaaag actaagaatg gcagcaaaga taaggaaaaa gattcagaaa<br>3061 aagagaaaga tttaaaagaa gagtttactg aagcattacg agatcttaaa attcagtgga<br>3121 tgacaaagct ggattctagt gacatttata acgaattgaa agaaacatat cctaattatc<br>3181 ttcctctgta cgttgcacga cttcatcaat tggatgctga aaaggaacga atgaaaagac<br>3241 ttaatgaaat tgttgatgcg gcaaatgctg ttatttctca tatagatcaa acagccctag<br>3301 cagtttatat tgcaatgaag actgatccca ggcctgatgc agctactata aaaaatgaca<br>3361 tggacaaaca aaaatccacc ctcgtagatg ccctttgtag gaaaggttgt gccctggcag<br>3421 accatcttct tcacacccag gctcaagacg gagccatttc cactgatgca gaaggaaagg<br>3481 aggaggaagg agaaagtcct ttggattctc tggcagaaac attttgggaa actactaaat<br>3541 ggactgatct ctttgacaat aaggttttga catttgcata taaacatgca ttagtaaata<br>3601 aaatgtatgg gagaggcctt aaatttgcaa ctaaacttgt ggaagaaaaa ccaacaaaag<br>3661 aaaactggaa aaattgtatt caactgatga agttacttgg atggacccat tgtgcatctt<br>3721 ttactgaaaa ctggctcccc atcatgtatc ctcccgatta ttgcgtattc taaaatagga<br>3781 aacaagactt taaattttaa aaaaggaagt tttatagtga atgggtataa aaacaaattt<br>3841 gtggcatttt tagtctaatg catgttttca tccactatcc agtactgatt attaaaatga<br>3901 catgtattta tcagagaatt cactgacgtg tggcttaata catgtaaatc tagacctctg<br>3961 acatcatggt gtttcttaa tgcctcacat tgctggcacg gggatgtgcc ctgcctgcca<br>4021 gcacctagga cttcgagttg ggttgcagct tatgacatgc atgataggtt ttggaaggta<br>4081 acttttaact gcaaacctat aaagtactat tttttatttt ataaatgaac agggttttaa<br>4141 cgtgctcaac tttaattttt ttcaattgta tgaaggcctt aaaaaagcta cattaagcgt<br>4201 agctaaaatt atttattgga ctaaaaacta acagaacttc atttccagaa tttttttttt<br>4261 tttttttttt ttggcaaatg tttacattca attaagggga aaaagtagaa ccagcacaaa<br>4321 tgagtggcag ttgctggagc ataactgctt caataaatct tcatcttggg gtaattacag<br>4381 gcaagtcatt ttcacatcct cttgaggttc agagcatcag aatgaactct atgaatacat<br>4441 gtgtaagtgc cagacagctg aatctttatc aggtattgta aagatacaca tatgatatgt<br>4501 ttattaaaat tgaaataatg taaaacacat gaataaattt gcaaaaccaa gatcacagta<br>4561 caccatatgc actctggtac cttaattttt ttttataaat aataaaagtg aatattgaag<br>4621 cttctt | |
| | | 1 M A T A A T E E P F P F H G L L P K K E<br>1 ATGGCCACCGCTGCGACTGAGGAGCCCTTCCCTTTTCACGGTCTCCTGCCGAAGAAGGAG<br><br>21 T G A A S F L C R Y P E Y D G R G V L I<br>61 ACCGGAGCCGCCTCCTTCCTCTGCCGCTACCCGGAGTATGATGGGCGGGGGGTGCTCATC<br><br>41 A V L D T G V D P G A P G M Q V T T D G | SEQ ID NO:324 |

| Gene Name | GenBank Homology | DNA SEQUENCE / DEDUCED AMINO ACID SEQUENCE | SEQUENCE IDENTIFIER: |
|---|---|---|---|
| | | 121 GCAGTCCTGGACACGGGGGTCGACCCGGGGGCTCCGGGCATGCAGGTTACAACTGATGGA<br><br>61 K P K I V D I I D T T G S G D V N T A T<br>181 AAACCAAAAATCGTTGATATCATTGATACAACAGGAAGTGGCGATGTGAATACTGCTACA<br><br>81 E V E P K D G E I V G L S G R V L K I P<br>241 GAAGTAGAGCCAAAGGATGGTGAGATTGTTGGCCTTTCAGGAAGAGTGCTTAAGATTCCT<br><br>101 A S W T N P S G K Y H I G I K N G Y D F<br>301 GCAAGCTGGACAAATCCCTCAGGCAAATATCATATTGGCATAAAAAATGGCTATGACTTC<br><br>121 Y P K A L K E R I Q K E R K E K I W D P<br>361 TATCCTAAGGCACTCAAGGAAAGGATACAGAAAGAACGGAAGGAAAAAATCTGGGACCCT<br><br>141 V H R V A L A E A C R K Q E E F D V A N<br>421 GTTCACAGAGTGGCCCTTGCAGAAGCCTGTAGAAAACAGGAAGAATTTGATGTTGCCAAC<br><br>161 N G S S Q A N K L I K E E L Q S Q V E L<br>481 AACGGCTCTTCTCAAGCAAATAAACTAATCAAGGAGGAACTTCAAAGTCAAGTGGAATTG<br><br>181 L N S F E K K Y S D P G P V Y D C L V W<br>541 CTAAATTCTTTTGAGAAGAAATACAGCGATCCTGGCCCTGTATATGACTGCTTGGTATGG<br><br>201 H D G E V W R A C I D S N E D G D L S K<br>601 CATGATGGCGAAGTCTGGAGAGCCTGCATTGATTCTAATGAAGATGGGGACTTGAGTAAA<br><br>221 S T V L R N Y K E A Q E Y G S F G T A E<br>661 TCTACCGTGTTGAGAAACTACAAAGAAGCCCAAGAATATGGCTCTTTTGGCACAGCTGAG<br><br>241 M L N Y S V N I Y D D R N L L S I V T S<br>721 ATGTTGAATTACTCCGTTAATATATACGATGATAGAAACCTGCTCTCCATTGTGACCAGT<br><br>261 G G A H G T H V A S I A A G H F P E E P<br>781 GGAGGAGCTCATGGGACACATGTAGCTAGTATAGCTGCTGGACACTTTCCAGAAGAACCT<br><br>281 E R N G V A P G A Q I L S I K I G D T R<br>841 GAACGGAATGGGGTAGCTCCTGGTGCTCAAATTCTTTCCATCAAGATTGGTGATACAAGA<br><br>301 L S T M E T G T G L I R A M I E V I N H<br>901 CTAAGCACAATGGAAACAGGCACAGGCCTCATAAGAGCTATGATAGAAGTTATAAATCAT<br><br>321 K C D L V N Y S Y G E A T H W P N S G R<br>961 AAGTGTGATCTTGTCAACTACAGTTACGGAGAAGCAACTCACTGGCCAAATTCTGGGAGA | |

| Gene Name | GenBank Homology | DNA SEQUENCE / DEDUCED AMINO ACID SEQUENCE | SEQUENCE IDENTIFIER: |
|---|---|---|---|
| | | 341 I C E V I N E A V W K H N I I Y V S S A<br>1021 ATTTGTGAAGTAATTAATGAAGCAGTATGGAAGCATAATATAATTTATGTTTCAAGTGCT<br><br>361 G N N G P C L S T V G C P G G T T S S V<br>1081 GGAAATAATGGTCCATGCCTGTCTACAGTTGGTTGTCCAGGTGGAACTACATCAAGTGTG<br><br>381 I G V G A Y V S P D M M V A E Y S L R E<br>1141 ATAGGTGTTGGTGCTTATGTTTCTCCTGATATGATGGTTGCTGAGTATTCACTGAGAGAG<br><br>401 K L P A N Q Y T W S S R G P S A D G A L<br>1201 AAATTACCTGCAAATCAATATACTTGGTCTTCTAGAGGACCTAGTGCTGACGGGGCCCTT<br><br>421 G V S I S A P G G A I A S V P N W T L R<br>1261 GGTGTGAGTATCAGTGCGCCAGGAGGAGCCATTGCTTCTGTTCCTAACTGGACACTGAGA<br><br>441 G T Q L M N G T S M S S P N A C G G I A<br>1321 GGGACGCAGCTGATGAATGGAACATCTATGTCTTCCCCCAATGCATGTGGAGGCATTGCC<br><br>461 L I L S G L K A N N I D Y T V H S V R R<br>1381 CTGATCCTTTCAGGTCTGAAAGCTAATAACATTGACTACACAGTTCATTCAGTCAGAAGA<br><br>481 A L E N T A V K A D N I E V F A Q G H G<br>1441 GCTCTAGAAAACACTGCAGTGAAGGCTGACAATATAGAAGTATTTGCTCAAGGACATGGT<br><br>501 I I Q V D K A Y D Y L V Q N T S F A N K<br>1501 ATTATTCAGGTTGATAAAGCCTATGACTACCTCGTTCAGAATACATCATTTGCTAATAAA<br><br>521 L G F T V T V G N N R G I Y L R D P V Q<br>1561 TTAGGTTTTACTGTTACTGTTGGAAATAACCGTGGCATCTACCTCCGAGATCCTGTTCAG<br><br>541 V A A P S D H G V G I E P V F P E N T E<br>1621 GTGGCTGCACCTTCAGATCATGGCGTTGGCATTGAACCTGTATTTCCGGAGAACACAGAA<br><br>561 N S E K I S L Q L H L A L T S N S S W V<br>1681 AACTCTGAAAAAATATCCCTTCAGCTTCATTTAGCTCTGACTTCAAATTCATCTTGGGTT<br><br>581 Q C P S H L E L M N Q C R H I N I R V D<br>1741 CAGTGTCCCAGCCATTTGGAACTCATGAATCAATGTAGACACATAAACATACGTGTGGAT<br><br>601 P R G L R E G L H Y T E V C G Y D I A S<br>1801 CCCAGGGGCTTAAGAGAAGGATTGCATTATACAGAGGTATGTGGCTATGATATAGCATCC | |

| Gene Name | GenBank Homology | DNA SEQUENCE / DEDUCED AMINO ACID SEQUENCE | SEQUENCE IDENTIFIER: |
|---|---|---|---|
| | | 621 P N A G P L F R V P I T A V I A A K V N<br>1861 CCTAACGCAGGTCCGCTCTTCAGAGTTCCGATCACTGCAGTTATAGCAGCAAAAGTAAAT<br><br>641 E S S H Y D L A F T D V H F K P G Q I R<br>1921 GAATCATCACATTATGATCTAGCCTTTACAGATGTACACTTTAAACCTGGTCAAATTCGA<br><br>661 R H F I E V P E G A T W A E V T V C S C<br>1981 AGGCATTTTATTGAGGTTCCTGAGGGTGCAACATGGGCTGAAGTGACAGTGTGTTCGTGT<br><br>681 S S E V S A K F V L H A V Q L V K Q R A<br>2041 TCTTCTGAGGTGTCAGCAAAGTTTGTTCTACATGCAGTCCAGCTTGTGAAGCAAAGAGCA<br><br>701 Y R S H E F Y K F C S L P E K G T L T E<br>2101 TATCGAAGCCATGAATTCTATAAGTTTTGTTCTCTTCCAGAGAAAGGAACACTGACTGAA<br><br>721 A F P V L G G K A I E F C I A R W W A S<br>2161 GCTTTTCCTGTCCTAGGTGGAAAAGCAATTGAATTTTGCATTGCTCGTTGGTGGGCAAGT<br><br>741 L S D V N I D Y T I S F H G I V C T A P<br>2221 CTCAGTGATGTCAACATTGATTATACCATTTCTTTCCATGGGATAGTGTGTACTGCTCCT<br><br>761 Q L N I H A S E G I N R F D V Q S S L K<br>2281 CAGTTAAACATTCATGCATCGGAAGGAATCAACCGCTTTGATGTTCAGTCCTCCTTGAAA<br><br>781 Y E D L A P C I T L K N W V Q T L R P V<br>2341 TACGAAGATCTGGCTCCCTGCATAACTTTGAAGAACTGGGTCCAAACACTGCGCCCAGTG<br><br>801 S A K T K P L G S R D V L P N N R Q L Y<br>2401 AGTGCAAAAACAAAACCTTTAGGATCAAGAGATGTTTTGCCAAATAACCGTCAACTTTAT<br><br>821 E M V L T Y N F H Q P K S G E V T P S C<br>2461 GAGATGGTCCTGACATATAACTTTCATCAACCCAAGAGTGGGGAAGTAACTCCAAGCTGC<br><br>841 P L L C E L L Y E S E F D S Q L W I I F<br>2521 CCACTACTTTGTGAACTATTATATGAATCTGAATTTGACAGCCAACTGTGGATTATTTTT<br><br>861 D Q N K R Q M G S G D A Y P H Q Y S L K<br>2581 GACCAGAACAAAAGACAGATGGGTTCAGGCGATGCCTATCCACATCAGTATTCTTTGAAA<br><br>881 L E K G D Y T I R L Q I R H E Q I S D L<br>2641 CTGGAGAAAGGAGATTATACAATTCGACTACAGATTCGCCATGAGCAAATCAGTGATTTG<br><br>901 E R L K D L P F I V S H R L S N T L S L | |

| Gene Name | GenBank Homology | DNA SEQUENCE / DEDUCED AMINO ACID SEQUENCE | SEQUENCE IDENTIFIER: |
|---|---|---|---|
| | | 2701 GAACGCCTTAAAGACCTTCCATTTATTGTTTCTCATAGATTGTCTAATACCTTGAGCTTA<br><br>921 D I H E N H S F A L L G K K K S S N L T<br>2761 GATATTCATGAAAATCATAGTTTTGCACTTCTAGGGAAGAAGAAATCAAGCAATTTGACA<br><br>941 L P P K Y N Q P F F V T S L P D D K I P<br>2821 TTACCACCCAAATATAACCAGCCATTCTTTGTTACTTCCTTACCTGATGATAAAATACCT<br><br>961 K G A G P G C Y L A G S L T L S K T E L<br>2881 AAAGGGGCAGGACCTGGATGCTATCTTGCAGGATCCTTAACATTGTCAAAGACTGAACTA<br><br>981 G K K A D V I P V H Y Y L I P P P T K T<br>2941 GGAAAGAAAGCTGATGTAATCCCTGTTCATTACTACTTAATACCTCCACCAACAAAGACT<br><br>1001 K N G S K D K E K D S E K E K D L K E E<br>3001 AAGAATGGCAGCAAAGATAAGGAAAAAGATTCAGAAAAAGAGAAAGATTTAAAAGAAGAG<br><br>1021 F T E A L R D L K I Q W M T K L D S S D<br>3061 TTTACTGAAGCATTACGAGATCTTAAAATTCAGTGGATGACAAAGCTGGATTCTAGTGAC<br><br>1041 I Y N E L K E T Y P N Y L P L Y V A R L<br>3121 ATTTATAACGAATTGAAAGAAACATATCCTAATTATCTTCCTCTGTACGTTGCACGACTT<br><br>1061 H Q L D A E K E R M K R L N E I V D A A<br>3181 CATCAATTGGATGCTGAAAAGGAACGAATGAAAAGACTTAATGAAATTGTTGATGCGGCA<br><br>1081 N A V I S H I D Q T A L A V Y I A M K T<br>3241 AATGCTGTTATTTCTCATATAGATCAAACAGCCCTAGCAGTTTATATTGCAATGAAGACT<br><br>1101 D P R P D A A T I K N D M D K Q K S T L<br>3301 GATCCCAGGCCTGATGCAGCTACTATAAAAAATGACATGGACAAACAAAAATCCACCCTC<br><br>1121 V D A L C R K G C A L A D H L L H T Q A<br>3361 GTAGATGCCCTTTGTAGGAAAGGTTGTGCCCTGGCAGACCATCTTCTTCACACCCAGGCT<br><br>1141 Q D G A I S T D A E G K E E E G E S P L<br>3421 CAAGACGGAGCCATTTCCACTGATGCAGAAGGAAAGGAGGAGGAAGGAGAAAGTCCTTTG<br><br>1161 D S L A E T F W E T T K W T D L F D N K<br>3481 GATTCTCTGGCAGAAACATTTTGGGAAACTACTAAATGGACTGATCTCTTTGACAATAAG<br><br>1181 V L T F A Y K H A L V N K M Y G R G L K<br>3541 GTTTTGACATTTGCATATAAACATGCATTAGTAAATAAAATGTATGGGAGAGGCCTTAAA | |

| Gene Name | GenBank Homology | DNA SEQUENCE / DEDUCED AMINO ACID SEQUENCE | SEQUENCE IDENTIFIER: |
|---|---|---|---|
| | | 1201 F A T K L V E E K P T K E N W K N C I Q<br>3601 TTTGCAACTAAACTTGTGGAAGAAAAACCAACAAAAGAAAACTGGAAAAATTGTATTCAA<br><br>1221 L M K L L G W T H C A S F T E N W L P I<br>3661 CTGATGAAGTTACTTGGATGGACCCATTGTGCATCTTTTACTGAAAACTGGCTCCCCATC<br><br>1241 M Y P P D Y C V F -<br>3721 ATGTATCCTCCCGATTATTGCGTATTCTAA | |
| gi9717252 | Equus caballus Toll-like receptor 4 mRNA, complete cds | 1 GGCACGAGCGGCACGAGAGCAGCAGCTCCGAGGCTGCCCTGGCGGGCAGGCTGTTACGGT<br>61 GCGTCATGCTTTCACAGGGCCCCTTCTGGGCACAGAAAATGCCAGGATGATGCCGCCCAC<br>121 CCGCCTGGCTGGGACTCTGATCCCAGCCATGGCCTTCCTCTCCTGCCTGAGACCCGAGAG<br>181 CTGGGACCCTTGCGTGCAGGTGGTTCCTAACACTACTTACCAATGCATGGACCTGAATCT<br>241 CTACAAAATCCCTGAGAACATCCCCACATCAACCAAGGAACTGGACCTGAGCTTTAACCC<br>301 CTTGAAGGAGTTAGGCAGCCATAGCTTCTCCAACTTCCCAGAACTGCAGGTGCTGGATTT<br>361 GTCCAGGTGTGAAATTGAGATGATTGAAGATGATGCATATCAGGGCCTCAACCATCTCTC<br>421 CACCTTGATATTGACAGGAAATCCTATCAGGAGCTTAGCCCTGGGAGCCTTTTCTGGACT<br>481 CTCCAGTTTACAGACGCTGGTGGCCGTGGAGACAAAGCTTTCATCTCTAGAGAAGTTCCC<br>541 CATTGGACATCTCAAGACCTTGAAGGAGCTTAATGTGGCTCATAATCTTATCCATTCCTT<br>601 CAAGTTACCCGAGTATTTTTCTAAAATGCCCAACCTGGAGCACTTGGACCTTTCCAATAA<br>661 CAAGATCCAAAATATTTCTCATGAAGACTTGCGTGTGCTACATCAAATGCCCTTACTCAA<br>721 CCTTTCTTTAGACTTGTCCCTGAATCCTTTAGAGTTTATCCAACCAGATGCCTTTAAAGA<br>781 AATTAAGCTCCATAAACTGACTTTGAGAAGTAATTTTGATAGTATAGATGTAATGAAATC<br>841 TTGTATTCAAGGACTGGCTGGTTTAAAAGTCAATCGGTTGGTTCTGGGAGAATTTAAAAA<br>901 TGAAAGGAAATTGGAAAGATTTGACACATCTGCCCTGCGCGGACTGCACAATTTGACGAT<br>961 TGAAGAATTCCGGTTAGCATACATTGATAATTACAGCTCAAAGGATTCTATTGATTTACT<br>1021 TAATTGTTTGGCAGATATTTCTAAAATTTCTCTGGTGAGTCTGGATTTAGGCAATCTAAA<br>1081 AGATTTTCCTAAAGGTTTCGGATGGCAAGACTTCGAATTGGTTAACTGTAGAATTGAAGG<br>1141 ATTTCCCACATTGGAGCTCACCTCTCTCAAAAGGTTGGTTTTCACTTCCAACAAAGATAT<br>1201 GAAATCTTTTAATGAAGTTAAGCTACCAAGCCTTGAGTTTCTAGATCTCAGCAGAAATCG<br>1261 CTTGAGTTTCAAGTCCTGCTGTTCTGAGGCTGATTTGAAGACAACCAGACTGAAGCATTT<br>1321 AGATCTGAGCTTCAATGATGTTATTTCCATGAGTTCAAACTTCATGGGCTTAGAGCAACT<br>1381 AGAACATCTGGATTTCCAGCATTCCACTTTGAAACAGGCCAGTGATTTTCCAGTATTCTT<br>1441 ATCCCTCAAAAACCTCCGTTATCTTGATATTTCTTACACTAACACCCGAGTTGTCTTCCA<br>1501 TGGCATCTTTGATGGCTTGGTCAGCCTCCAAGTCTTGAAAATGGCTGGCAATTCTTTTAA<br>1561 GGACAACTTCCTTCCAAATATCTTCAGAGAGATGACTAACCTGACCACCCTGGACCTTTC<br>1621 TAAGTGTAACCTGGAACAGGTGTCCCAGGAGGCATTTTGCTTACTCCCTCGACTTCGGGT<br>1681 GTTAAATATGAGTCACAATAACCTCCTGTTCTTGGATATGCTTCCTTACAAACCTCTCCA<br>1741 TTCCCTCCAGATTCTGGATTGCAGTTTTAATCGTATTGTGGCCTTCAAGTGGCAAGAACT<br>1801 GCAGCATTTTCCAAGTAGTCTAGCTTCCTTAAATCTTACTCAAAATGACTTTGCTTGTGT<br>1861 TTGTGAATACCAGAGTTTTCTGCAGTGGGTCAAGGACCAGAGGCAGCTCTTGGTGGAAGT | SEQ ID NO:325 |

| Gene Name | GenBank Homology | DNA SEQUENCE / DEDUCED AMINO ACID SEQUENCE | SEQUENCE IDENTIFIER: |
|---|---|---|---|
| | | 1921 TGAACACTTGGTGTGTGCAATACCCTTACAGATGCGGGGCATGCCCGTGCTGGGTTTTAA<br>1981 CAATGCCACCTGTCAGATTAGCAAGACTATCGTTGGCGGGTCGGTTTTCAGTATACTCAT<br>2041 GGTTTCTGTCATAGCAGTTCTGGTCTATAAGTTCTATTTCCACCTGATGCTTCTTGCTGG<br>2101 CTGCAAAAAGTATGGGAGAGGTGAAAGCATCTATGATGCCTTTGTTATCTACTCAAGCCA<br>2161 GGACGAAGACTGGGTGAGGAATGAATTGGTAAAGAACTTGGAGGAGGGGGTGCCCCCCTT<br>2221 TCAACTCTGCCTTCACTACAGAGACTTCATTCCTGGTGTGGCCATCGCCGCCAACATCAT<br>2281 CCAGGAAGGTTTCCACAAAAGCCGGAAAGTTATTGTCGTGGTGTCCCAGCACTTCATTCA<br>2341 GAGCCGATGGTGTATCTTTGAGTATGAGATTGCCCAGACCTGGCAGTTTCTGAGCAGTCG<br>2401 TGCTGGCATCATCTTCATCGTCCTGCACAAGCTGGAGAAGTCCCTGCTCCGGCAGCAGGT<br>2461 GGAGCTGTATCGCCTTCTCAACAGGAACACTTACCTGGAGTGGGAGGACAGTGTCCTGGG<br>2521 GCGGCACATCTTCTGGAGACGACTCAGGAAAGCCTTGCTGGATGGTAAACCGTGGAGTCC<br>2581 AGCAGGAACAGCAGATGCAGCAGAAAGCAGACAACATGATGCAGAAACCTCTACCTGAGG<br>2641 AGGAAAAACTCCTGAGGTGCTTCTTGCCCAGCTGGACCCAATACTTGTTCAGTTAACAAC<br>2701 TATTAAATGCTGCAACATACCAGGCATTGTGCTAGGGGCAGGTGATTCTGTCGTGCACAA<br>2761 GATACACAGGACTGCTAATCCCTTTGAGTTTACAGTGCAGAGGGAATAAATGCCGTGCTA<br>2821 AAATACAGAACCTCCAGGGGGATGTTTTAACCAAGTCAGCTAAGGAGTCCGTGCCAGGGA<br>2881 AAGTCAACTCAACTCTTACCCCATCAAAATGAATCAGAACTGAGAGACTGGGCCCCAGTG<br>2941 AGTTCAGAAAAGGACATCGTCTCCCAAGTCTTTTGAATGGAAACCATCTCATCTTTGACA<br>3001 TCTTAGCCATCCTAAAACAAAACAAAGCAGTTTTGGTACTTTCAACTGAACAGCGTCTCT<br>3061 GTTCAATTTTCCCTTTTCTACTGAACACAATTTAAATTTTACTTGATGACTCAGAAGGCT<br>3121 CCTGATTCAGATCCTCCCTCCTCTTTAAGGCAGTTTCCTTATGAAGGTTAAACTCTTACG<br>3181 ACTAATTCCTAAGGAAATCTGATTGATACATATTCACAAACATCCTGGTCATTCTTTAGC<br>3241 GTGCTCTATTTATTAATAAATAGCAGGTTCTATTTAAAAAAAAAAAAAAAAAA | |
| | | 1 M M P P T R L A G T L I P A M A F L S C<br>1 ATGATGCCGCCCACCCGCCTGGCTGGGACTCTGATCCCAGCCATGGCCTTCCTCTCCTGC<br><br>21 L R P E S W D P C V Q V V P N T T Y Q C<br>61 CTGAGACCCGAGAGCTGGGACCCTTGCGTGCAGGTGGTTCCTAACACTACTTACCAATGC<br><br>41 M D L N L Y K I P E N I P T S T K E L D<br>121 ATGGACCTGAATCTCTACAAAATCCCTGAGAACATCCCCACATCAACCAAGGAACTGGAC<br><br>61 L S F N P L K E L G S H S F S N F P E L<br>181 CTGAGCTTTAACCCCTTGAAGGAGTTAGGCAGCCATAGCTTCTCCAACTTCCCAGAACTG<br><br>81 Q V L D L S R C E I E M I E D D A Y Q G<br>241 CAGGTGCTGGATTTGTCCAGGTGTGAAATTGAGATGATTGAAGATGATGCATATCAGGGC<br><br>101 L N H L S T L I L T G N P I R S L A L G<br>301 CTCAACCATCTCTCCACCTTGATATTGACAGGAAATCCTATCAGGAGCTTAGCCCTGGGA<br><br>121 A F S G L S S L Q T L V A V E T K L S S | SEQ ID NO:326 |

| Gene Name | GenBank Homology | DNA SEQUENCE / DEDUCED AMINO ACID SEQUENCE | SEQUENCE IDENTIFIER: |
|---|---|---|---|
| | | 361 GCCTTTTCTGGACTCTCCAGTTTACAGACGCTGGTGGCCGTGGAGACAAAGCTTTCATCT<br><br>141 L E K F P I G H L K T L K E L N V A H N<br>421 CTAGAGAAGTTCCCCATTGGACATCTCAAGACCTTGAAGGAGCTTAATGTGGCTCATAAT<br><br>161 L I H S F K L P E Y F S K M P N L E H L<br>481 CTTATCCATTCCTTCAAGTTACCCGAGTATTTTTCTAAAATGCCCAACCTGGAGCACTTG<br><br>181 D L S N N K I Q N I S H E D L R V L H Q<br>541 GACCTTTCCAATAACAAGATCCAAAATATTTCTCATGAAGACTTGCGTGTGCTACATCAA<br><br>201 M P L L N L S L D L S L N P L E F I Q P<br>601 ATGCCCTTACTCAACCTTTCTTTAGACTTGTCCCTGAATCCTTTAGAGTTTATCCAACCA<br><br>221 D A F K E I K L H K L T L R S N F D S I<br>661 GATGCCTTTAAAGAAATTAAGCTCCATAAACTGACTTTGAGAAGTAATTTTGATAGTATA<br><br>241 D V M K S C I Q G L A G L K V N R L V L<br>721 GATGTAATGAAATCTTGTATTCAAGGACTGGCTGGTTTAAAAGTCAATCGGTTGGTTCTG<br><br>261 G E F K N E R K L E R F D T S A L R G L<br>781 GGAGAATTTAAAAATGAAAGGAAATTGGAAAGATTTGACACATCTGCCCTGCGCGGACTG<br><br>281 H N L T I E E F R L A Y I D N Y S S K D<br>841 CACAATTTGACGATTGAAGAATTCCGGTTAGCATACATTGATAATTACAGCTCAAAGGAT<br><br>301 S I D L L N C L A D I S K I S L V S L D<br>901 TCTATTGATTTACTTAATTGTTTGGCAGATATTTCTAAAATTTCTCTGGTGAGTCTGGAT<br><br>321 L G N L K D F P K G F G W Q D F E L V N<br>961 TTAGGCAATCTAAAAGATTTTCCTAAAGGTTTCGGATGGCAAGACTTCGAATTGGTTAAC<br><br>341 C R I E G F P T L E L T S L K R L V F T<br>1021 TGTAGAATTGAAGGATTTCCCACATTGGAGCTCACCTCTCTCAAAAGGTTGGTTTTCACT<br><br>361 S N K D M K S F N E V K L P S L E F L D<br>1081 TCCAACAAAGATATGAAATCTTTTAATGAAGTTAAGCTACCAAGCCTTGAGTTTCTAGAT<br><br>381 L S R N R L S F K S C C S E A D L K T T<br>1141 CTCAGCAGAAATCGCTTGAGTTTCAAGTCCTGCTGTTCTGAGGCTGATTTGAAGACAACC<br><br>401 R L K H L D L S F N D V I S M S S N F M<br>1201 AGACTGAAGCATTTAGATCTGAGCTTCAATGATGTTATTTCCATGAGTTCAAACTTCATG | |

| Gene Name | GenBank Homology | DNA SEQUENCE / DEDUCED AMINO ACID SEQUENCE | SEQUENCE IDENTIFIER: |
|---|---|---|---|
| | | 421 G L E Q L E H L D F Q H S T L K Q A S D<br>1261 GGCTTAGAGCAACTAGAACATCTGGATTTCCAGCATTCCACTTTGAAACAGGCCAGTGAT<br><br>441 F P V F L S L K N L R Y L D I S Y T N T<br>1321 TTTCCAGTATTCTTATCCCTCAAAAACCTCCGTTATCTTGATATTTCTTACACTAACACC<br><br>461 R V V F H G I F D G L V S L Q V L K M A<br>1381 CGAGTTGTCTTCCATGGCATCTTTGATGGCTTGGTCAGCCTCCAAGTCTTGAAAATGGCT<br><br>481 G N S F K D N F L P N I F R E M T N L T<br>1441 GGCAATTCTTTTAAGGACAACTTCCTTCCAAATATCTTCAGAGAGATGACTAACCTGACC<br><br>501 T L D L S K C N L E Q V S Q E A F C L L<br>1501 ACCCTGGACCTTTCTAAGTGTAACCTGGAACAGGTGTCCCAGGAGGCATTTTGCTTACTC<br><br>521 P R L R V L N M S H N N L L F L D M L P<br>1561 CCTCGACTTCGGGTGTTAAATATGAGTCACAATAACCTCCTGTTCTTGGATATGCTTCCT<br><br>541 Y K P L H S L Q I L D C S F N R I V A F<br>1621 TACAAACCTCTCCATTCCCTCCAGATTCTGGATTGCAGTTTTAATCGTATTGTGGCCTTC<br><br>561 K W Q E L Q H F P S S L A S L N L T Q N<br>1681 AAGTGGCAAGAACTGCAGCATTTTCCAAGTAGTCTAGCTTCCTTAAATCTTACTCAAAAT<br><br>581 D F A C V C E Y Q S F L Q W V K D Q R Q<br>1741 GACTTTGCTTGTGTTTGTGAATACCAGAGTTTTCTGCAGTGGGTCAAGGACCAGAGGCAG<br><br>601 L L V E V E H L V C A I P L Q M R G M P<br>1801 CTCTTGGTGGAAGTTGAACACTTGGTGTGTGCAATACCCTTACAGATGCGGGGCATGCCC<br><br>621 V L G F N N A T C Q I S K T I V G G S V<br>1861 GTGCTGGGTTTTAACAATGCCACCTGTCAGATTAGCAAGACTATCGTTGGCGGGTCGGTT<br><br>641 F S I L M V S V I A V L V Y K F Y F H L<br>1921 TTCAGTATACTCATGGTTTCTGTCATAGCAGTTCTGGTCTATAAGTTCTATTTCCACCTG<br><br>661 M L L A G C K K Y G R G E S I Y D A F V<br>1981 ATGCTTCTTGCTGGCTGCAAAAAGTATGGGAGAGGTGAAAGCATCTATGATGCCTTTGTT<br><br>681 I Y S S Q D E D W V R N E L V K N L E E<br>2041 ATCTACTCAAGCCAGGACGAAGACTGGGTGAGGAATGAATTGGTAAAGAACTTGGAGGAG | |

| Gene Name | GenBank Homology | DNA SEQUENCE / DEDUCED AMINO ACID SEQUENCE | SEQUENCE IDENTIFIER: |
|---|---|---|---|
| | | 701 G V P P F Q L C L H Y R D F I P G V A I<br>2101 GGGGTGCCCCCCTTTCAACTCTGCCTTCACTACAGAGACTTCATTCCTGGTGTGGCCATC<br><br>721 A A N I I Q E G F H K S R K V I V V V S<br>2161 GCCGCCAACATCATCCAGGAAGGTTTCCACAAAAGCCGGAAAGTTATTGTCGTGGTGTCC<br><br>741 Q H F I Q S R W C I F E Y E I A Q T W Q<br>2221 CAGCACTTCATTCAGAGCCGATGGTGTATCTTTGAGTATGAGATTGCCCAGACCTGGCAG<br><br>761 F L S S R A G I I F I V L H K L E K S L<br>2281 TTTCTGAGCAGTCGTGCTGGCATCATCTTCATCGTCCTGCACAAGCTGGAGAAGTCCCTG<br><br>781 L R Q Q V E L Y R L L N R N T Y L E W E<br>2341 CTCCGGCAGCAGGTGGAGCTGTATCGCCTTCTCAACAGGAACACTTACCTGGAGTGGGAG<br><br>801 D S V L G R H I F W R R L R K A L L D G<br>2401 GACAGTGTCCTGGGGCGGCACATCTTCTGGAGACGACTCAGGAAAGCCTTGCTGGATGGT<br><br>821 K P W S P A G T A D A A E S R Q H D A E<br>2461 AAACCGTGGAGTCCAGCAGGAACAGCAGATGCAGCAGAAAGCAGACAACATGATGCAGAA<br><br>841 T S T -<br>2521 ACCTCTACCTGA | |

TABLE 2

| Probe Set Name | Sequence | Sequence Identifier |
|---|---|---|
| ILT11A mRNA for immunoglobulin-like transcript 11 protein | | |
| B1961620.V1.3_at | AGGACGTGACCTACGCCCAGGTGAA | SEQ ID NO:327 |
| B1961620.V1.3_at | AGGGACCCCAGAAGACATAGGAGCT | SEQ ID NO:328 |
| B1961620.V1.3_at | AGGTGAACCACTTGACCCTCAGAGG | SEQ ID NO:329 |
| B1961620.V1.3_at | AGTGGTACGCTGCTATGGCCATCCA | SEQ ID NO:330 |
| B1961620.V1.3_at | AGCAGAGCCCAGTGGTACGCTGCTA | SEQ ID NO:331 |
| B1961620.V1.3_at | CATAGGAGCTGCCTCCAGAGGACAC | SEQ ID NO:332 |
| B1961620.V1.3_at | GAGACTGCAGGGACCCCAGAAGACA | SEQ ID NO:333 |
| B1961620.V1.3_at | GATGCCACCCTCCATGGAGGGAGAC | SEQ ID NO:334 |
| B1961620.V1.3_at | GTATGCACAGGCTGCTATATCTGAA | SEQ ID NO:335 |
| B1961620.V1.3_at | TGGCCATCCACTAGCCCAGGAAGGA | SEQ ID NO:336 |
| B1961620.V1.3_at | TAGCCCAGGAAGGACCCGGATGCCA | SEQ ID NO:337 |
| No homology | | |
| B1961856.V1.3_at | ATGCTGGCAAAACCTCTAAGACTGT | SEQ ID NO:338 |
| B1961856.V1.3_at | AGTTTCCCTTCTGCAGCTTGTTTTT | SEQ ID NO:339 |
| B1961856.V1.3_at | ACCTGATTCTGCCTCTTTAATGAAT | SEQ ID NO:340 |
| B1961856.V1.3_at | ACAGTTCCTATGCATGGTTTTTAAC | SEQ ID NO:341 |
| B1961856.V1.3_at | CACGAGGGAAGAGGGTCTCCTTCCA | SEQ ID NO:342 |
| B1961856.V1.3_at | GCTTGTTTTTCTCACTATCTGTAT | SEQ ID NO:343 |
| B1961856.V1.3_at | GAACAATTTAATTTCTTGGCCGTGT | SEQ ID NO:344 |
| B1961856.V1.3_at | GGCCGTGTTTAGTAACAGTTCCTAT | SEQ ID NO:345 |
| B1961856.V1.3_at | GAAAGTTGATTTGTCCTAGTGCAAA | SEQ ID NO:346 |
| B1961856.V1.3_at | GAAACTCAATGCCTTATGTGCTCAC | SEQ ID NO:347 |
| B1961856.V1.3_at | TTATGTGCTCACTCAGTTTCCCTTC | SEQ ID NO:348 |
| Equus caballus tissue inhibitor of metalloproteinase-1 (TIMP-1) mRNA complete cds | | |
| B1961880.V1.3_at | AGACCACCTTACAGCGGCGTTATGA | SEQ ID NO:349 |
| B1961880.V1.3_at | AGCTGAAGCTTGCACAGTGTTCACC | SEQ ID NO:350 |
| B1961880.V1.3_at | ACAGGCTCTGACAAGGGCTTCCAGA | SEQ ID NO:351 |
| B1961880.V1.3_at | GAATCCTGTCTCCAGCAGAAGCTGA | SEQ ID NO:352 |
| B1961880.V1.3_at | GAATGCTCAGTGTTTCCCTGTTCAT | SEQ ID NO:353 |
| B1961880.V1.3_at | GAACCGCAGCGAGGAGTTTCTCATC | SEQ ID NO:354 |
| B1961880.V1.3_at | GGACGAGAAGCTGTACATCACCACC | SEQ ID NO:355 |
| B1961880.V1.3_at | TGAGTTCGTCATCAGGGCCAAGTTC | SEQ ID NO:356 |
| B1961880.V1.3_at | TGCGGATACTTCCACAGGTCGGAGA | SEQ ID NO:357 |
| B1961880.V1.3_at | TGATTGCTTGTGGACGGACCAGCTC | SEQ ID NO:358 |
| B1961880.V1.3_at | TTCTCATCGCCGGACAACTATTGGA | SEQ ID NO:359 |
| PREDICTED Homo sapiens zinc finger, SWIM domain containing 6 (ZSWIM6), mRNA | | |

(continued)

| Probe Set Name | Sequence | Sequence Identifier |
|---|---|---|
| ILT11A mRNA for immunoglobulin-like transcript 11 protein | | |
| B1961900.V1.3_at | GTTAAAAGGCTGACCCACGTGGCTT | SEQ ID NO:360 |
| B1961900.V1.3_at | AGAACACCTCCATCCTGTGATAAGT | SEQ ID NO:361 |
| B1961900.V1.3_at | ACGTGGCTTTGCAGTGCTGTTCGTC | SEQ ID NO:362 |
| B1961900.V1.3_at | AAAAGTCCACACAGCTCTCCTGTTT | SEQ ID NO:363 |
| B1961900.V1.3_at | GTCTACGTTCACAGCCTCAAAAAAG | SEQ ID NO:364 |
| B1961900.V1.3_at | GCTGTTCGTCCAGAAGCATGGCACA | SEQ ID NO:365 |
| B1961900.V1.3_at | GAACAGAGCCACACCGGTATTATAT | SEQ ID NO:366 |
| B1961900.V1.3_at | GTTCTATTGCGTTTGCTGACTAAAT | SEQ ID NO:367 |
| B1961900.V1.3_at | GGTTTGTTTTTACTTGAGCCTGCCT | SEQ ID NO:368 |
| B1961900.V1.3_at | GGATTCAGCTTTACTCCTTTGTAAC | SEQ ID NO:369 |
| B1961900.V1.3_at | TGAGCCTGCCTTTGTACCCTTTTTA | SEQ ID NO:370 |
| Equus caballus interleukin-4 receptor alpha-chain mRNA. | | |
| B1961932.V1.3_at | AGGATTTGCCTAGAGAGGCCCGTTC | SEQ ID NO:371 |
| B1961932.V1.3_at | TGTTACCCGAGTCTGACCCAGTCCT | SEQ ID NO:372 |
| B1961932.V1.3_at | GAATGACTTAGGAGGCCCCAGGAAA | SEQ ID NO:373 |
| B1961932.V1.3_at | ATCACTGGATTGGATGCTGAGCCTA | SEQ ID NO:374 |
| B1961932.V1.3_at | AGTCCTGGGTTAGCTGCCGCCATAT | SEQ ID NO:375 |
| B1961932.V1.3_at | AGAGCTTCATCTAGCTGGCACCAGA | SEQ ID NO:376 |
| B1961932.V1.3_at | GCTGCCGCCATATCACTGGATTGGA | SEQ ID NO:377 |
| B1961932.V1.3_at | GATGCTGAGCCTAGAAACTGATCAA | SEQ ID NO:378 |
| B1961932.V1.3_at | GTTCCTTCAACAGAGCTTCATCTAG | SEQ ID NO:379 |
| B1961932.V1.3_at | TGGCACCAGAGGCAGGATTGCACCT | SEQ ID NO:380 |
| B1961932.V1.3_at | TTGCACCTGTGGTGGGTGCTTAGCC | SEQ ID NO:381 |
| Equus caballus interleukin-4 receptor alpha-chain mRNA | | |
| B1961932.V1.3_s_at | AGGGAGTGGGTCACAGCCCATGACC | SEQ ID NO:382 |
| B1961932.V1.3_s_at | ATGGGATCCCCAGAGTGGACCTGCC | SEQ ID NO:383 |
| B1961932.V1.3_s_at | ATGGAGAGCGTTGCTGGCAGCCATA | SEQ ID NO:384 |
| B1961932.V1.3_s_at | AGTGACAGTTTGCTGTCAGGTCCCC | SEQ ID NO:385 |
| B1961932.V1.3_s_at | AGACCTTGGGTCGAGTAACGCTTGT | SEQ ID NO:386 |
| B1961932.V1.3_s_at | ACGTTTTGCCTGCACATGGCTCAGA | SEQ ID NO:387 |
| B1961932.V1.3_s_at | GCCAGTTTGTGCACCCATGGAGAGC | SEQ ID NO:388 |
| B1961932.V1.3_s_at | GAAATTGTGATTAGCCGGTAGTGAC | SEQ ID NO:389 |
| B1961932.V1.3_s_at | GAGTAACGCTTGTTTGTGTGTATCT | SEQ ID NO:390 |
| B1961932.V1.3_s_at | GGCATTCCAGACAGCTGACCCGGCA | SEQ ID NO:391 |
| B1961932.V1.3_s_at | TGGGCCTCCTCTTAGGCATGGGATC | SEQ ID NO:392 |
| Equus caballus adenosine A2A receptor mRNA | | |
| BM734452.V1.3_at | AGACTTTCCCAGGTTCGGGAGCTGC | SEQ ID NO:393 |

(continued)

| Probe Set Name | Sequence | Sequence Identifier |
|---|---|---|
| ILT11A mRNA for immunoglobulin-like transcript 11 protein | | |
| BM734452.V1.3_at | AAGTGGAGGCTCTAGCTGACTGCCT | SEQ ID NO:394 |
| BM734452.V1.3_at | CTCAGAGCTGCCCAAGTGGAGGCTC | SEQ ID NO:395 |
| BM734452.V1.3_at | CCGACTCCAGCAGCACGTAGAAGTG | SEQ ID NO:396 |
| BM734452.V1.3_at | GCCGTGAGCTGCCATGTGCTTCTCA | SEQ ID NO:397 |
| BM734452.V1.3_at | GCTGCTGTGTCCAGAGGTGGCATTT | SEQ ID NO:398 |
| BM734452.V1.3_at | GTAAGCGCGAGGAAATCCTTTTTAT | SEQ ID NO:399 |
| BM734452.V1.3_at | GGCATTTGACTATTTTTGACCAGGA | SEQ ID NO:400 |
| BM734452.V1.3_at | GGCTGCTGGGTCTGTTGTCAGTCCT | SEQ ID NO:401 |
| BM734452.V1.3_at | TGTCAGTCCTGCTGCTAACCTGGCA | SEQ ID NO:402 |
| BM734452.V1.3_at | TTTTATTGTATTATCTTCCCTCCCT | SEQ ID NO:403 |
| No Homology | | |
| BM734531.V1.3_at | ACCACTTCATGTTCTCTACAGAGCT | SEQ ID NO:404 |
| BM734531.V1.3_at | ACAGAGCTGTCCAGAGCCGAGGCTG | SEQ ID NO:405 |
| BM734531.V1.3_at | AAACGAGTCCGAGGGCACAGCCAGG | SEQ ID NO:406 |
| BM734531.V1.3_at | CGTTGCCCGCTGTTGGTCATGACAA | SEQ ID NO:407 |
| BM734531.V1.3_at | GAGTCTCTGTCAGGATCCTTTTGAA | SEQ ID NO:408 |
| BM734531.V1.3_at | GAGTCACCCAAGGAACTTATGCAGA | SEQ ID NO:409 |
| BM734531.V1.3_at | GTCCTGTGGCTTTTGTGTGTCTCTC | SEQ ID NO:410 |
| BM734531.V1.3_at | GGCCTTGCTTGAGAGAGGTCCATCC | SEQ ID NO:411 |
| BM734531.V1.3_at | GGTCACTTAGCAGCGACTTCTTGGA | SEQ ID NO:412 |
| BM734531.V1.3_at | GGAGCCAGGTGTCTGCATTTGAACA | SEQ ID NO:413 |
| BM734531.V1.3_at | TTATGCAGATGCCATGTCCTCACTC | SEQ ID NO:414 |
| Human UbA52 adrenal RNA for ubiquitin-52 amino acid fusion protein. | | |
| BM734661.V1.3_at | ATTTGCCGCAAGTGTTATGCTCGCC | SEQ ID NO:415 |
| BM734661.V1.3_at | ATCTTTGTGAAGACCCTGACGGGCA | SEQ ID NO:416 |
| BM734661.V1.3_at | AACCTGCGCCCCAAGAAGAAGGTCA | SEQ ID NO:417 |
| BM734661.V1.3_at | AGCAGCGTTTGATTTTTGCCGGCAA | SEQ ID NO:418 |
| BM734661.V1.3_at | GAAGGTCAAATAAGGCCCCTCCTCT | SEQ ID NO:419 |
| BM734661.V1.3_at | CACTCTCTCAGACTACAATATCCAG | SEQ ID NO:420 |
| BM734661.V1.3_at | GCAAGAAGAAGTGCGGCCACACCAA | SEQ ID NO:421 |
| BM734661.V1.3_at | GACGCAGACATGCAGATCTTTGTGA | SEQ ID NO:422 |
| BM734661.V1.3_at | GGTTGAGCCCAGTGACACCATTGAG | SEQ ID NO:423 |
| BM734661.V1.3_at | TCGTGCTGTCAACTGCCGCAAGAAG | SEQ ID NO:424 |
| BM734661.V1.3_at | TTTTGCCGGCAAACAGCTGGAGGAC | SEQ ID NO:425 |
| Homo sapiens STAT4 mRNA | | |
| BM734780.V1.3_at | AGTCATCTCTTATTCTTCATCTTTG | SEQ ID NO:426 |
| BM734780.V1.3_at | ACAGGATGACCTCTTGACACTCAAA | SEQ ID NO:427 |

(continued)

| Probe Set Name | Sequence | Sequence Identifier |
|---|---|---|
| ILT11A mRNA for immunoglobulin-like transcript 11 protein | | |
| BM734780.V1.3_at | AATAAAGGCCGCTTGTCTGCTTTGC | SEQ ID NO:428 |
| BM734780.V1.3_at | GAAAACCCTCTGAAGTACCTGTATC | SEQ ID NO:429 |
| BM734780.V1.3_at | GAGAGAAAACCTGAGTCCCACGACC | SEQ ID NO:430 |
| BM734780.V1.3_at | TGGCATGACACTCCCAATTGGGTGT | SEQ ID NO:431 |
| BM734780.V1.3_at | GATGAAGTCTCCATATTCTGCCGAA | SEQ ID NO:432 |
| BM734780.V1.3_at | GGTTGAAGTCTGAAGCTCTGCTCTC | SEQ ID NO:433 |
| BM734780.V1.3_at | TGATCTTTGCCAACAGTCATCTCTT | SEQ ID NO:434 |
| BM734780.V1.3_at | TATGTTCCTTCTGTTTTTATCCCCA | SEQ ID NO:435 |
| BM734780.V1.3_at | TTTGCTGACATCCTTCGAGACTACA | SEQ ID NO:436 |
| Homo sapiens phosphate cytidylyltransferase 1 choline alpha isoform, mRNA (cDNA clone MGC: 49915 | | |
| IMAGE: 5744747) | | |
| BM734844.V1.3_at | ATGTTTGCTCCAACACAGAGGACAG | SEQ ID NO:437 |
| BM734844.V1.3_at | ACATCAGACATCATCACCCGAATTG | SEQ ID NO:438 |
| BM734844.V1.3_at | AAGGCTGCAGCCTATGATATCAGTG | SEQ ID NO:439 |
| BM734844.V1.3_at | CATCACCTCCAGAAGCTCTTTGTTG | SEQ ID NO:440 |
| BM734844.V1.3_at | GAAATACCACTTGCAGGAGCGAGTT | SEQ ID NO:441 |
| BM734844.V1.3_at | GACAGAAGGTATCTCCACATCAGAC | SEQ ID NO:442 |
| BM734844.V1.3_at | GATGGCCTTTCTCTGGCAAGACTTC | SEQ ID NO:443 |
| BM734844.V1.3_at | GATGCTGCAGGCCATCAGTCCAAAG | SEQ ID NO:444 |
| BM734844.V1.3_at | GTCCGGGACTATGATGTGTACGCGA | SEQ ID NO:445 |
| BM734844.V1.3_at | GGGCTACACAGCCAAGGAGCTCAAT | SEQ ID NO:446 |
| BM734844.V1.3_at | TCAGTCCAAAGCAGAGTCCCAGCAG | SEQ ID NO:447 |
| No Homology | | |
| BM734891.V1.3_at | ACTGGTATTCTCAGCTTCGTATGCT | SEQ ID NO:448 |
| BM734891.V1.3_at | AAGGATCCTCCTTCTGGACTTGTTT | SEQ ID NO:449 |
| BM734891.V1.3_at | CAGCGAGATCAGCAAGGCCTTTTCT | SEQ ID NO:450 |
| BM734891.V1.3_at | CAATGTGCATGTTTCAGACCTTCTG | SEQ ID NO:451 |
| BM734891.V1.3_at | GCTTGCCACTGCCTTTGGGATATAG | SEQ ID NO:452 |
| BM734891.V1.3_at | TAATGGGTCCCTTCAGTCAGTGGCA | SEQ ID NO:453 |
| BM734891.V1.3_at | GTGACTTTTGGTTACCTGGGCACAC | SEQ ID NO:454 |
| BM734891.V1.3_at | GGTAGTGTTGGATCTCAGCCACCCA | SEQ ID NO:455 |
| BM734891.V1.3_at | TCGTATGCTGCAGTACCTGTGGACA | SEQ ID NO:456 |
| BM734891.V1.3_at | TATAGGGACTGCAGCCTTTGTTTCT | SEQ ID NO:457 |
| BM734891.V1.3_at | TTCTGCTCTCTCTGGAATGGGTAGT | SEQ ID NO:458 |
| Homo sapiens matrix metalloproteinase 9 (gelatinase B, 92kDa gelatinase, 92kDa type IV collagenase) mRNA (cDNA clone | | |

(continued)

| Probe Set Name | Sequence | Sequence Identifier |
|---|---|---|
| ILT11A mRNA for immunoglobulin-like transcript 11 protein | | |
| BM734900.V1.3_at | ACGACGTCTTCCAGTACCGAGAGAA | SEQ ID NO:459 |
| BM734900.V1.3_at | ACTGGCGCGTGAGTTCCCGGAATGA | SEQ ID NO:460 |
| BM734900.V1.3_at | AAGTGGGCTACGTGAGCTTCGACCT | SEQ ID NO:461 |
| BM734900.V1.3_at | GCTGGATTCCGCCTTTGAGGAGCCA | SEQ ID NO:462 |
| BM734900.V1.3_at | GAAGGAGCCAGTTTGCCGGTTTCAA | SEQ ID NO:463 |
| BM734900.V1.3_at | GAAGATTTTCTTCTTCTCTGGGCGC | SEQ ID NO:464 |
| BM734900.V1.3_at | GAGGAGCCACTCACCAAGAAGATTT | SEQ ID NO:465 |
| BM734900.V1.3_at | GTGTCAGCCCGGTGGACCAGATGTT | SEQ ID NO:466 |
| BM734900.V1.3_at | GTGTACACAGGCAAGTCGGCGCTAG | SEQ ID NO:467 |
| BM734900.V1.3_at | GGTTTCAAACTGGTGGGTCTGTTCT | SEQ ID NO:468 |
| BM734900.V1.3_at | TAGGCCCGAGGCGTCTGGACAAGCT | SEQ ID NO:469 |
| Homo sapiens S100 Calcium binding protein P, mRNA. | | |
| BM734933.V1.3_at | AGGGCAGCAAGCAGAGCCTGACCAA | SEQ ID NO:470 |
| BM734933.V1.3_at | AAAGCTCTTGTTGGCAGTCGTTTCC | SEQ ID NO:471 |
| BM734933.V1.3_at | GCCGAGGTGGACTTCAGCGAGTTCA | SEQ ID NO:472 |
| BM734933.V1.3_at | CCTGGCGTCCAGAAAGCTCTTGTTG | SEQ ID NO:473 |
| BM734933.V1.3_at | GAGCATGATCATCGACGTCTTTGCC | SEQ ID NO:474 |
| BM734933.V1.3_at | GAGTTCATCGTGTTTGTGGCCGCGC | SEQ ID NO:475 |
| BM734933.V1.3_at | GAGGTGGGACATTTCCTCGGCCACG | SEQ ID NO:476 |
| BM734933.V1.3_at | GTGGGTCCAAATCCAGCACCATGAC | SEQ ID NO:477 |
| BM734933.V1.3_at | GGTGCCGTGGACAAACTGCTCAAGG | SEQ ID NO:478 |
| BM734933.V1.3_at | TCATGGAGAAGGAGCTCCCGGGCTT | SEQ ID NO:479 |
| BM734933.V1.3_at | TCAAGGACCTGGACGCCAACGGAGA | SEQ ID NO:480 |
| Human mRNA for coupling protein G(s) alpha-subunit (alpha-S1) (stimulatory regulatory component Gs of adenylyl cyclase) | | |
| BM734975.V1.3_at | ATGATTAACAAAGCCACCTTTCCCT | SEQ ID NO:481 |
| BM734975.V1.3_at | ATTGTTCACGCAGTTAATCACCCAC | SEQ ID NO:482 |
| BM734975.V1.3_at | ATCTCTGTGATTCTGTTCCTCAACA | SEQ ID NO:483 |
| BM734975.V1.3_at | ATGCACCTCCGTCAGTATGAGCTGC | SEQ ID NO:484 |
| BM734975.V1.3_at | CTGCTCGCTGAGAAAGTCCTTGCTG | SEQ ID NO:485 |
| BM734975.V1.3_at | GCTCCCTTCAGCTTGGTTAAATATA | SEQ ID NO:486 |
| BM734975.V1.3_at | GAATTTCTGAGAATCAGCCCCGCTA | SEQ ID NO:487 |
| BM734975.V1.3_at | GACTACTTTCCAGAATTTGCTCGCT | SEQ ID NO:488 |
| BM734975.V1.3_at | GTTCCCTCTCTCTTTCAGTAAGTAA | SEQ ID NO:489 |
| BM734975.V1.3_at | GGCCAAGTACTTCATCCGTGACGAA | SEQ ID NO:490 |
| BM734975.V1.3_at | TCGCTACACTACTCCTGAGGATGCT | SEQ ID NO:491 |
| Human mRNA for macrophage inflammatory protein-2alpha (MIP2alpha) | | |

(continued)

| Probe Set Name | Sequence | Sequence Identifier |
|---|---|---|
| ILT11A mRNA for immunoglobulin-like transcript 11 protein | | |
| BM735057.V1.3_s_at | AAGGGCAGCTCCGACTGACCAGAGG | SEQ ID NO:492 |
| BM735057.V1.3_s_at | GCTGTAGCTGAAGTAGGCCCTGCCC | SEQ ID NO:493 |
| BM735057.V1.3_s_at | GCAGCTCCGACTGACCAGAGGAAGA | SEQ ID NO:494 |
| BM735057.V1.3_s_at | GAAGTAGGCCCTGCCCTTACAGGAA | SEQ ID NO:495 |
| BM735057.V1.3_s_at | GAAGCTGATCAGTGGCTGTAGCTGA | SEQ ID NO:496 |
| BM735057.V1.3_s_at | GACTGACCAGAGGAAGAAGCTGATC | SEQ ID NO:497 |
| BM735057.V1.3_s_at | GATGCTAAACAAGGGCAGCTCCGAC | SEQ ID NO:498 |
| BM735057.V1.3_s_at | GGAAGTTTGTCTCAACCCTGAAGCC | SEQ ID NO:499 |
| BM735057.V1.3_s_at | GGCCCTGCCCTTACAGGAATCAAAG | SEQ ID NO:500 |
| BM735057.V1.3_s_at | TGGACAGGAAGTTTGTCTCAACCCT | SEQ ID NO:501 |
| BM735057.V1.3_s_at | TAAACAAGGGCAGCTCCGACTGACC | SEQ ID NO:502 |
| Horse serpin mRNA. | | |
| BM735091.V1.3_at | ATGCTTGCCATGCTGATGCCAGAGG | SEQ ID NO:503 |
| BM735091.V1.3_at | ATCTAGCCCGCCTAGGTGTACAAGA | SEQ ID NO:504 |
| BM735091.V1.3_at | CATTCATTTTCTTCATTCGGCACAA | SEQ ID NO:505 |
| BM735091.V1.3_at | GCTAACATCCTGTTCTTAGGCAGAT | SEQ ID NO:506 |
| BM735091.V1.3_at | GCAAAGCTGATCTGTCTGGCATGTC | SEQ ID NO:507 |
| BM735091.V1.3_at | GAATCTGTATCTCGCTGAAGTCAAT | SEQ ID NO:508 |
| BM735091.V1.3_at | GAAAATTTCAATGCCGACCATCCAT | SEQ ID NO:509 |
| BM735091.V1.3_at | GGGCCAGAGATCTTTTCGTATCGAA | SEQ ID NO:510 |
| BM735091.V1.3_at | TGAAGTCAATGTCCACTTGCCCAGG | SEQ ID NO:511 |
| BM735091.V1.3_at | TTTGGCGTGGGTAGCTATTTTCCTT | SEQ ID NO:512 |
| BM735091.V1.3_at | TTAGGCAGATTTTCTTCCCCTTAGA | SEQ ID NO:513 |
| Equus caballus mRNA for ferritin light chain. | | |
| BM735286.V1.3_at | AGGAGCCTCCGGAGTCCAGCGGCCT | SEQ ID NO:514 |
| BM735286.V1.3_at | AGCTTTTTAACTAGCCTGGAGCCTT | SEQ ID NO:515 |
| BM735286.V1.3_at | CGTCAGAGCTTCTGCCTGAGCCTCT | SEQ ID NO:516 |
| BM735286.V1.3_at | CTGCAGCCACTAGAGATAGCTTTTT | SEQ ID NO:517 |
| BM735286.V1.3_at | CTCCGGAGTCCAGCGGCCTTTGAGG | SEQ ID NO:518 |
| BM735286.V1.3_at | CTGGCGTCAGAGCTTCTGCCTGAGC | SEQ ID NO:519 |
| BM735286.V1.3_at | GAGATAGCTTTTTAACTAGCCTGGA | SEQ ID NO:520 |
| BM735286.V1.3_at | TTTGGTATCCCCCTGGCGTCAGAGC | SEQ ID NO:521 |
| BM735286.V1.3_at | TCCCTGCAGCCACTAGAGATAGCTT | SEQ ID NO:522 |
| BM735286.V1.3_at | TTTTAACTAGCCTGGAGCCTTCTGC | SEQ ID NO:523 |
| BM735286.V1.3_at | TAACTAGCCTGGAGCCTTCTGCCCA | SEQ ID NO:524 |
| No Homology | | |
| BM735386.V1.3_at | ATTTGCTGACCTTGTGGTGCTCTCA | SEQ ID NO:525 |

(continued)

| Probe Set Name | Sequence | Sequence Identifier |
|---|---|---|
| ILT11A mRNA for immunoglobulin-like transcript 11 protein | | |
| BM735386.V1.3_at | AAGTTGTCACGTGCAGTGTCTTTCT | SEQ ID NO:526 |
| BM735386.V1.3_at | AAGGGCAATCCTGCTTCACTGGTGT | SEQ ID NO:527 |
| BM735386.V1.3_at | GCTGGTGCCCCTGTAAAGAGGCTTT | SEQ ID NO:528 |
| BM735386.V1.3_at | GAAGTTGTCACCATGAAGTTGTCAC | SEQ ID NO:529 |
| BM735386.V1.3_at | GAGCGTTTCCTTTACCTGGAAGTTG | SEQ ID NO:530 |
| BM735386.V1.3_at | GAGACCTGTTGTGCTGTGTTATTTT | SEQ ID NO:531 |
| BM735386.V1.3_at | GATGGCTGTTTTCCTGTCCCAGAGA | SEQ ID NO:532 |
| BM735386.V1.3_at | GATGTCTGACCTTAGTGTTCCAAAC | SEQ ID NO:533 |
| BM735386.V1.3_at | GTTAAGTATGTGGTTCCTTCTCAGA | SEQ ID NO:534 |
| BM735386.V1.3_at | TAAAGGATGTCCTGGTGCTGGCTCA | SEQ ID NO:535 |
| No Homology | | |
| BM735452.V1.3_at | CGAACCCCGGAAGTCTGCAAAATTG | SEQ ID NO:536 |
| BM735452.V1.3_at | AGGATGACTCGGGTCCAGTGGCCCA | SEQ ID NO:537 |
| BM735452.V1.3_at | AAGTCTGCAAAATTGGGTGCCCCAA | SEQ ID NO:538 |
| BM735452.V1.3_at | CTGCTCTCTGCTCCCTAAATTATTG | SEQ ID NO:539 |
| BM735452.V1.3_at | CCAGAATCTGCCCACGGAGTCGGTG | SEQ ID NO:540 |
| BM735452.V1.3_at | CCCCGGTTTCATATCTGTATTTATA | SEQ ID NO:541 |
| BM735452.V1.3_at | GGGTGCCCCAAGAGCTGGAAACTCA | SEQ ID NO:542 |
| BM735452.V1.3_at | GAGGCCTCAGTGCAGTGCCTTCCCG | SEQ ID NO:543 |
| BM735452.V1.3_at | GTGCGCAGGTCCCACTCAGGATGAC | SEQ ID NO:544 |
| BM735452.V1.3_at | GGAAACTCAGGAAACCCCAGGTGCT | SEQ ID NO:545 |
| BM735452.V1.3_at | TTAATATATGCAATTCCCCATCCCT | SEQ ID NO:546 |
| Homo sapiens male-specific lethal 3-like 1 (Drosophila); transcript variant 1, mRNA | | |
| BM780999.V1.3_at | TACTTCTTAGGTACCTGAACGGCCT | SEQ ID NO:547 |
| BM780999.V1.3_at | ATTTGAGTTGCTTACCTACTGTAGT | SEQ ID NO:548 |
| BM780999.V1.3_at | AGTGTGTCTAGGTTAGGCTGTTTCA | SEQ ID NO:549 |
| BM780999.V1.3_at | AAAGCATTTCCTGTCTTGTTTGAGA | SEQ ID NO:550 |
| BM780999.V1.3_at | GCTTGTTACAGTTCATTTTCTCTAA | SEQ ID NO:551 |
| BM780999.V1.3_at | GACAGGCAGGCCCATTAATTTGAGT | SEQ ID NO:552 |
| BM780999.V1.3_at | GATGGTGGGCCTTCAGACAGAGTAC | SEQ ID NO:553 |
| BM780999.V1.3_at | GATGAGGCCGAGCAACTCTGTCCAA | SEQ ID NO:554 |
| BM780999.V1.3_at | GGAAACCGCAAGGTAGTCCCAGAAT | SEQ ID NO:555 |
| BM780999.V1.3_at | TAATGTTATGGTTCCCTTTCTGTAA | SEQ ID NO:556 |
| BM780999.V1.3_at | TTGCCACTGTACACGTGATCTGTGA | SEQ ID NO:557 |
| Homo sapiens replication protein A1, 70kDa, mRNA | | |
| BM781033.V1.3_at | AGATAGCCTTCCTTCAGGGTTTAAT | SEQ ID NO:558 |
| BM781033.V1.3_at | AAAGGCGCTCTCTTTCTAGAGGTGC | SEQ ID NO:559 |

(continued)

| Probe Set Name | Sequence | Sequence Identifier |
|---|---|---|
| ILT11A mRNA for immunoglobulin-like transcript 11 protein | | |
| BM781033.V1.3_at | AAAGCCTTGCTTTAGTTTCACTTCA | SEQ ID NO:560 |
| BM781033.V1.3_at | AATTGACCCTAAATCTGCAGACAGT | SEQ ID NO:561 |
| BM781033.V1.3_at | CAGGGACATCCACGCAACTACAAAG | SEQ ID NO:562 |
| BM781033.V1.3_at | GAATGCACACTTTTTCTACCTGTAC | SEQ ID NO:563 |
| BM781033.V1.3_at | GAAGAAGCCCTTTGTTTCTCTTGCT | SEQ ID NO:564 |
| BM781033.V1.3_at | GACATTTCTGCAACTTGGGTTCCTT | SEQ ID NO:565 |
| BM781033.V1.3_at | GTAAGCGCGTCTTCCTAGAATTCAA | SEQ ID NO:566 |
| BM781033.V1.3_at | TAACTAGGTTTCTGCTCCATTGGTG | SEQ ID NO:567 |
| BM781033.V1.3_at | TACCTGTACATATTTCTTGCGTTTG | SEQ ID NO:568 |
| Human mRNA for cathepsin H | | |
| BM781103.V1.3_at | ATCCCTCTGGTGTGAGCCAGGATGG | SEQ ID NO:569 |
| BM781103.V1.3_at | AAGAATATTCTTTCCTTCCTGAAGG | SEQ ID NO:570 |
| BM781103.V1.3_at | CACCCACACCTTCTGATGCAAAGGA | SEQ ID NO:571 |
| BM781103.V1.3_at | CAGCCTTGTGCCTTTTAAGCATGAT | SEQ ID NO:572 |
| BM781103.V1.3_at | CATTGGTGGAAGTCCTGCCCTGGAG | SEQ ID NO:573 |
| BM781103.V1.3_at | CCCCGTGCTGTGTCCAGAAATCTAA | SEQ ID NO:574 |
| BM781103.V1.3_at | GCAGCCTGGCCATTGGTGGAAGTCC | SEQ ID NO:575 |
| BM781103.V1.3_at | GAAGAATTCCACCAAGCAACAAGCC | SEQ ID NO:576 |
| BM781103.V1.3_at | GATGCAAAGGATCACCAGCCTTGTG | SEQ ID NO:577 |
| BM781103.V1.3_at | TTCCTTCCTGAAGGGCTACTTTTCA | SEQ ID NO:578 |
| BM781103.V1.3_at | TTAACTGACTCAAACCCACGTGGAC | SEQ ID NO:579 |
| Homo sapiens transforming acidic coiled-coil containing protein 3 (TACC3) mRNA | | |
| BM781237.V1.3_s_at | GTACTACAGTACAGCCAGAAGGACC | SEQ ID NO:580 |
| BM781237.V1.3_s_at | AGCAGTCCTTATACCTCAAGTTCGA | SEQ ID NO:581 |
| BM781237.V1.3_s_at | AAGTTCGACCCGCTCCTGATGGACA | SEQ ID NO:582 |
| BM781237.V1.3_s_at | AAGAGAACCTGTCGCTCAGGAGCGA | SEQ ID NO:583 |
| BM781237.V1.3_s_at | GGACCCATCGTGGACGTACTACAGT | SEQ ID NO:584 |
| BM781237.V1.3_s_at | GTGGAACTTGACTTCCTAGGCCCTC | SEQ ID NO:585 |
| BM781237.V1.3_s_at | TGGACCCACCTTTGTGTGTCCTCGG | SEQ ID NO:586 |
| BM781237.V1.3_s_at | TGATGGACAGCCCTCAGAGACCTGC | SEQ ID NO:587 |
| BM781237.V1.3_s_at | TCCGGATGTGCCTGTGCTGGACCCA | SEQ ID NO:588 |
| BM781237.V1.3_s_at | TGCCGCCAATCTCGTGGAACTTGAC | SEQ ID NO:589 |
| BM781237.V1.3_s_at | TCCCCGCGACAGACAGTGTGCAGGA | SEQ ID NO:590 |
| Homo sapiens, chromosome 6 open reading frame 166, mRNA | | |
| BM781278.V1.3_at | ACATGCATTCTTCCTCAGTGGACCA | SEQ ID NO:591 |
| BM781278.V1.3_at | AAAGAACAGCCCTTATTCACTCTAC | SEQ ID NO:592 |
| BM781278.V1.3_at | CTGATGTCAATACCACCCGGATGTG | SEQ ID NO:593 |

(continued)

| Probe Set Name | Sequence | Sequence Identifier |
|---|---|---|
| ILT11A mRNA for immunoglobulin-like transcript 11 protein | | |
| BM781278.V1.3_at | CAGCCTGCTAGTTATGTTTCATGAA | SEQ ID NO:594 |
| BM781278.V1.3_at | CACGTTTTCTGCATTTGTGGGCTGC | SEQ ID NO:595 |
| BM781278.V1.3_at | TCTGCAACATCCTCACCATTAAAAA | SEQ ID NO:596 |
| BM781278.V1.3_at | GTGGCCTCATTTGCTGTATCATTAC | SEQ ID NO:597 |
| BM781278.V1.3_at | TGGCAACCACTTTAGGCAGCAACTG | SEQ ID NO:598 |
| BM781278.V1.3_at | TGTGGACCTTTGCTACCTGTATTAA | SEQ ID NO:599 |
| BM781278.V1.3_at | TTCAAGCTTTCGTCAGTGGCAACCA | SEQ ID NO:600 |
| BM781278.V1.3_at | TTATTCACTCTACGGCAGGTTGGGA | SEQ ID NO:601 |
| Homo sapiens KIAK0002 mRNA | | |
| BM781319.V1.3_at | ATAACCTGTTATTCTGACTAGCTTA | SEQ ID NO:602 |
| BM781319.V1.3_at | ATGTTCATTACCCTTGTACCATGTA | SEQ ID NO:603 |
| BM781319.V1.3_at | AGTGCCTTACTGACTGTAGCCATTA | SEQ ID NO:604 |
| BM781319.V1.3_at | GAGTCAATGTTATTCTCTCACAATG | SEQ ID NO:605 |
| BM781319.V1.3_at | GATCTCTCTCTCTCAGTTACAGTTT | SEQ ID NO:606 |
| BM781319.V1.3_at | GTTCTTACTGGCCAACTCTTAAATC | SEQ ID NO:607 |
| BM781319.V1.3_at | GTTTCTCATCTTGGACTTCTTTTAT | SEQ ID NO:608 |
| BM781319.V1.3_at | GTGTGATGCCATATCGAGTCAATGT | SEQ ID NO:609 |
| BM781319.V1.3_at | TGTACCATGTACCTCAGATCTCTCT | SEQ ID NO:610 |
| BM781319.V1.3_at | TCACTGGGCCATTGGATTTTTTCCA | SEQ ID NO:611 |
| BM781319.V1.3_at | TAAGAGGTCCTATCTGTTCAGTGTT | SEQ ID NO:612 |
| Homo sapiens inhibitor of Bruton a gammaglobulinemia tyrosine kinase (IBTK) mRNA | | |
| BM781379.V1.3_at | ATTCGGCAACCCTGATGAGTTTGTC | SEQ ID NO:613 |
| BM781379.V1.3_at | AGGGACCACTGGCAGTACCTATGTG | SEQ ID NO:614 |
| BM781379.V1.3_at | AGCATGGATGCTAGTTCGCCGTGGA | SEQ ID NO:615 |
| BM781379.V1.3_at | AAAACCCTTGGCTTTGATTCAGATT | SEQ ID NO:616 |
| BM781379.V1.3_at | GTTGAAAGGACACCGCAGGGACCAC | SEQ ID NO:617 |
| BM781379.V1.3_at | GAGGAGCACGCGATACAAGATTTAT | SEQ ID NO:618 |
| BM781379.V1.3_at | GATTCAGATTGAGGAGCACGCGATA | SEQ ID NO:619 |
| BM781379.V1.3_at | GTTCGCCGTGGACTTGAGATGTATT | SEQ ID NO:620 |
| BM781379.V1.3_at | GGTGCATTAGCTATTATTTCTGATC | SEQ ID NO:621 |
| BM781379.V1.3_at | GGTTTTCTATGAGGCATTCGGCAAC | SEQ ID NO:622 |
| BM781379.V1.3_at | GGATCTAATTTCTTTGCTGCAGTTT | SEQ ID NO:623 |
| No Homology | | |
| BM781394.V1.3_at | ACCAGTGCTGAACATTAGTGCCTTT | SEQ ID NO:624 |
| BM781394.V1.3_at | GAACCTGCTGAATTTAACTTAACCG | SEQ ID NO:625 |
| BM781394.V1.3_at | AAAATACAGTCAACCCTTCACCATG | SEQ ID NO:626 |
| BM781394.V1.3_at | AAGAGTTCAGCTTCACGTTTGCCAA | SEQ ID NO:627 |

(continued)

| Probe Set Name | Sequence | Sequence Identifier |
|---|---|---|
| ILT11A mRNA for immunoglobulin-like transcript 11 protein | | |
| BM781394.V1.3_at | CAACAGGCGGAGACAGCTGTCCTTT | SEQ ID NO:628 |
| BM781394.V1.3_at | CCATATCACCTGCTATTTCCAAGAA | SEQ ID NO:629 |
| BM781394.V1.3_at | GCTGCATCTCATACAATGGCTCTCT | SEQ ID NO:630 |
| BM781394.V1.3_at | GAAACAGCCCGATACATAACACTCC | SEQ ID NO:631 |
| BM781394.V1.3_at | TCTGCCCATCAATTACACTTTCTTT | SEQ ID NO:632 |
| BM781394.V1.3_at | TAACCGAGAGGATCACCGTGACAAA | SEQ ID NO:633 |
| BM781394.V1.3_at | TAATGCTGGCATTTTGGATACTACC | SEQ ID NO:634 |
| No Homology | | |
| BM781409.V1.3_at | ATGGATCCCCTTATTATTCTTTCAC | SEQ ID NO:635 |
| BM781409.V1.3_at | AGTTTTTACATCTGCCTCAAGGCCT | SEQ ID NO:636 |
| BM781409.V1.3_at | AGAATGAAACTGAGTCTCCCTGGCC | SEQ ID NO:637 |
| BM781409.V1.3_at | AGATCCTTCGCTTGTCTGCTTAAAA | SEQ ID NO:638 |
| BM781409.V1.3_at | AATTCTGAACAACTCTCTGCAACTG | SEQ ID NO:639 |
| BM781409.V1.3_at | GTTCCTCCTTTTCTTTAGGTCTCAG | SEQ ID NO:640 |
| BM781409.V1.3_at | GCACGAGCTGACCTGGAGTACTACA | SEQ ID NO:641 |
| BM781409.V1.3_at | GACTTCCCAGTGCACTCAGAATGAA | SEQ ID NO:642 |
| BM781409.V1.3_at | GAGTTCTCTATGTCACAGTTTTTAC | SEQ ID NO:643 |
| BM781409.V1.3_at | GTCTCAGCTTACATATGTCACCTTC | SEQ ID NO:644 |
| BM781409.V1.3_at | TTGTTTATCACTGTATCCCTAGCAC | SEQ ID NO:645 |
| No Homology | | |
| BM781416.V1.3_at | ATGCTGCAGCTGTTTGTCAGGAACA | SEQ ID NO:646 |
| BM781416.V1.3_at | AGTATTTGCCATCACATGCTGCAGC | SEQ ID NO:647 |
| BM781416.V1.3_at | ACAATTACTGGCTTTTCTGCTGTAA | SEQ ID NO:648 |
| BM781416.V1.3_at | CAGTAATCACGCTGCAGAAATTGGC | SEQ ID NO:649 |
| BM781416.V1.3_at | GAACGCAGCAGCAGGGTACGTAGCA | SEQ ID NO:650 |
| BM781416.V1.3_at | GAAATTGGCAGCCTGCACCTTATGC | SEQ ID NO:651 |
| BM781416.V1.3_at | TGTCCGTTTTTATTTGGGCATCCAC | SEQ ID NO:652 |
| BM781416.V1.3_at | GGGCATCCACCACAGACAATTTAAA | SEQ ID NO:653 |
| BM781416.V1.3_at | GGTGTCATTCATTCATTCAGTAATC | SEQ ID NO:654 |
| BM781416.V1.3_at | TAAATTTCGTAGATGCGCTCAGAAC | SEQ ID NO:655 |
| BM781416.V1.3_at | TTATGCGTCACCATTACCTTTAGTC | SEQ ID NO:656 |
| Homo sapiens cysteine-rich motor neuron 1 (CRIM1) | | |
| BM781438.V1.3_at | ATATGATTTATCCTGAGTGCTGTAT | SEQ ID NO:657 |
| BM781438.V1.3_at | ATCACTCTTTTACTTTGGTTCCTGT | SEQ ID NO:658 |
| BM781438.V1.3_at | AGATACCCAGTATGCTTAATGTGAA | SEQ ID NO:659 |
| BM781438.V1.3_at | ACTTGGACAGAGGTTGCTGAACTTT | SEQ ID NO:660 |
| BM781438.V1.3_at | AAAGGTGTTCTAGCTGTTTGCATCA | SEQ ID NO:661 |

(continued)

| Probe Set Name | Sequence | Sequence Identifier |
|---|---|---|
| ILT11A mRNA for immunoglobulin-like transcript 11 protein | | |
| BM781438.V1.3_at | AAAATAGAGTTACATCAGCCTGATT | SEQ ID NO:662 |
| BM781438.V1.3_at | GTTGACAATTAATCCATTCCTGGCA | SEQ ID NO:663 |
| BM781438.V1.3_at | GTGCTGTATCTATCACTCTTTTACT | SEQ ID NO:664 |
| BM781438.V1.3_at | TGGTTCCTGTTGTGCTCTTGTAAAA | SEQ ID NO:665 |
| BM781438.V1.3_at | TAAATGCTTGCTTTTTGTTTTTTCA | SEQ ID NO:666 |
| BM781438.V1.3_at | TTGTTTTTTCAATCATGGCCATATT | SEQ ID NO:667 |
| Homo sapiens RNF4 mRNA. | | |
| Foe1216.V1.3_at | ATTGCCATGAAGTTCCTGCTGGGCT | SEQ ID NO:668 |
| Foe1216.V1.3_at | CTTTCCCTTCCTCAATGTACATAGT | SEQ ID NO:669 |
| Foe1216.V1.3_at | TGACTTTTTGACTGTTGGGCTCACT | SEQ ID NO:670 |
| Foe1216.V1.3_at | GCTGGGCTTCGTGGAAACACTGTTT | SEQ ID NO:671 |
| Foe1216.V1.3_at | GATGATACCCAGACTTCAGGAGCCA | SEQ ID NO:672 |
| Foe1216.V1.3_at | GGGCAGGACATCAGTTTTCATCAGT | SEQ ID NO:673 |
| Foe1216.V1.3_at | GGAGTGTTCCTCCTTTATGTGAAAA | SEQ ID NO:674 |
| Foe1216.V1.3_at | TGGGCTCACTTCCAGTTAGTTTGAA | SEQ ID NO:675 |
| Foe1216.V1.3_at | TTCCGGTTAGACGTCATTGCCATGA | SEQ ID NO:676 |
| Foe1216.V1.3_at | TATCCGTGTATGTCAGTCTGTTTTG | SEQ ID NO:677 |
| Foe1216.V1.3_at | TTTGGACTCCTCTGCTAGTGTTACA | SEQ ID NO:678 |
| No Homology | | |
| Foe1263.V1.3_at | ATCTCTTGGAGTTTCAGTACTGTCA | SEQ ID NO:679 |
| Foe1263.V1.3_at | AGAGGTGGTCTGTAATGGATTCAAA | SEQ ID NO:680 |
| Foe1263.V1.3_at | ACATAGTTCCACTGTCATTTCTGAA | SEQ ID NO:681 |
| Foe1263.V1.3_at | AAAAAGCTCGACAGCACTCAGGTCC | SEQ ID NO:682 |
| Foe1263.V1.3_at | AACTAATAGGTTACTGCTTCCCACA | SEQ ID NO:683 |
| Foe1263.V1.3_at | CCAGTCCATCTCGTTTCTTTATAAA | SEQ ID NO:684 |
| Foe1263.V1.3_at | GCTTCCCACACTACATTGGTTCAAA | SEQ ID NO:685 |
| Foe1263.V1.3_at | GCTCTTTAAGTCATAGCACCGGTAA | SEQ ID NO:686 |
| Foe1263.V1.3_at | GAGTTCCGGTATCTAAGTCACAATT | SEQ ID NO:687 |
| Foe1263.V1.3_at | GGTTGCAGCAAACTTTTTAACTGAT | SEQ ID NO:688 |
| Foe1263.V1.3_at | TAAAGGCATTGTTCCTTCCAGTCCA | SEQ ID NO:689 |
| Human-3-hydroxyacyl - CoA dehydrogenase, isoform 2 mRNA | | |
| Foe1317.V1.3_at | ATAATTGCCTGAATTCGTTCCCTTG | SEQ ID NO:690 |
| Foe1317.V1.3_at | AGGGAAGCACTTCCTCCGAGGGCAT | SEQ ID NO:691 |
| Foe1317.V1.3_at | CTTCTGAACCTTGTCATTTTCGTGA | SEQ ID NO:692 |
| Foe1317.V1.3_at | AGCAGTGTCGAGAGCCATCGCGTGT | SEQ ID NO:693 |
| Foe1317.V1.3_at | GCAAGCTGCCTGTAAATCGACTCTA | SEQ ID NO:694 |
| Foe1317.V1.3_at | GAATGATGACTGTCTGTCTCTCTTT | SEQ ID NO:695 |

(continued)

| Probe Set Name | Sequence | Sequence Identifier |
|---|---|---|
| ILT11A mRNA for immunoglobulin-like transcript 11 protein | | |
| Foe1317.V1.3_at | GAAATGCAGTCTTTTAACCTATGAA | SEQ ID NO:696 |
| Foe1317.V1.3_at | GTAACGGCAGATTTCTCCATTGAGA | SEQ ID NO:697 |
| Foe1317.V1.3_at | GTCTCTCTTTTTTCCGTGACAAATC | SEQ ID NO:698 |
| Foe1317.V1.3_at | GGAAGAGGTTTCATTCACACCCGAA | SEQ ID NO:699 |
| Foe1317.V1.3_at | TACACCCTTGGTGCCTTGGAGCAAA | SEQ ID NO:700 |
| Homo sapiens mRNA; cDNA DKFZp434I0612 (from clone DKFZp434I0612). | | |
| Foe1324.V1.3_at | AAACCTTTTGGTGTACATCCTCTGG | SEQ ID NO:701 |
| Foe1324.V1.3_at | AGATATCTGTCTCATTCATTCCACT | SEQ ID NO:702 |
| Foe1324.V1.3_at | ACTTGCACTGTCTCCTGGGATCTTG | SEQ ID NO:703 |
| Foe1324.V1.3_at | AACAAGTGGCTTCCTTTTTGAGATA | SEQ ID NO:704 |
| Foe1324.V1.3_at | CTTTCTTTCCACAGCACTTTTGATA | SEQ ID NO:705 |
| Foe1324.V1.3_at | CTCCCTGTGGCACTTGACATTTTAA | SEQ ID NO:706 |
| Foe1324.V1.3_at | GTAAGGCAATCTTTCCTGTTGTAAA | SEQ ID NO:707 |
| Foe1324.V1.3_at | GTGTGATCCCATCATGAGTTCATTG | SEQ ID NO:708 |
| Foe1324.V1.3_at | TGGGATCTTGTCTGCAGCCCGACGT | SEQ ID NO:709 |
| Foe1324.V1.3_at | GGCCCCTTAAGTGCTGCCAGAAGCA | SEQ ID NO:710 |
| Foe1324.V1.3_at | TTCTGGTTATCATTTATCCTCCCTG | SEQ ID NO:711 |
| No Homology | | |
| Foe146.V1.3_at | ATTGTGGATTTGTCAGTTTCTCTCT | SEQ ID NO:712 |
| Foe146.V1.3_at | ATTCTTCATAAGAGCTGTTGCCAAA | SEQ ID NO:713 |
| Foe146.V1.3_at | ATTCATTTCACTGTACTCTTGCCAG | SEQ ID NO:714 |
| Foe146.V1.3_at | ATCACTCCAGTGATGGTTGTGGTGG | SEQ ID NO:715 |
| Foe146.V1.3_at: | AAAGCATAGTTCAGTGCCACTCACA | SEQ ID NO:716 |
| Foe146.V1.3_at | AAGTATGTTGAAGTGTCTCTCTGTC | SEQ ID NO:717 |
| Foe146.V1.3_at | AAGTGTCTCTCTGTCATTGTGGATT | SEQ ID NO:718 |
| Foe146.V1.3_at | AATTGCCCTTGTCTCATAATGCTTT | SEQ ID NO:719 |
| Foe146.V1.3_at | CAGGTGCCTAATGTGCGGTCTTTAA | SEQ ID NO:720 |
| Foe146.V1.3_at | TGTCTACTTGGTTTTACCACCTTCT | SEQ ID NO:721 |
| Foe146.V1.3_at | TCAGTTTCTCTCTGCATTTCTCAGA | SEQ ID NO:722 |
| Homo sapiens fucosyltransferase 8 (alpha (1,6) fucosyltransferase) (FUT8) transcript variant 1 | | |
| Foe148.V1.3_at | ATCAGTTCATCCACCTCATTATTAA | SEQ ID NO:723 |
| Foe148.V1.3_at | AGGTTGATTCTTAGCCAGTGGTGGT | SEQ ID NO:724 |
| Foe148.V1.3_at | AGTGTTTCTGGACATTTCTCTTTGA | SEQ ID NO:725 |
| Foe148.V1.3_at | CAGGTTCTTTAGTACTCAGTGTTTC | SEQ ID NO:726 |
| Foe148.V1.3_at | GTGACCTAACGAGAAGACCCGCATA | SEQ ID NO:727 |
| Foe148.V1.3_at | GATGCCTCATTGGTGGAATTCCTCT | SEQ ID NO:728 |

(continued)

| Probe Set Name | Sequence | Sequence Identifier |
|---|---|---|
| ILT11A mRNA for immunoglobulin-like transcript 11 protein | | |
| Foe148.V1.3_at | GTATTGTAACCGCTGAATTCACTCC | SEQ ID NO:729 |
| Foe148.V1.3_at | GTGCCTATATCCGAGAGACCTATGT | SEQ ID NO:730 |
| Foe148.V1.3 at | GTGAGACTTCAAACATGGTGCCTAT | SEQ ID NO:731 |
| Foe148.V1.3_at | TCAAACCCATGCACAGTCCAATAAT | SEQ ID NO:732 |
| Foe148.V1.3_at | TACTTTGCCCCTTCTTTCAAGATTA | SEQ ID NO:733 |
| Homo sapiens tripeptidyl peptidase II mRNA | | |
| Foe443.V1.3_at | AGGACTTTCAGTTGGGTTGCAGCTA | SEQ ID NO:734 |
| Foe443.V1.3_at | CCATAACTTTTAACTGCAAGCCAGT | SEQ ID NO:735 |
| Foe443.V1.3_at | AACATCCTCTTGACGTGCAGAGCAT | SEQ ID NO:736 |
| Foe443.V1.3_at | AATTGCTGGAGCATAACTGCTTCAA | SEQ ID NO:737 |
| Foe443.V1.3_at | CAGGGTTTTAACATGCTTGACTTTA | SEQ ID NO:738 |
| Foe443.V1.3_at | GTACACCATATCACTCTGATACCTT | SEQ ID NO:739 |
| Foe443.V1.3_at | GTGCCAGACAGCTAAATCTTTATCA | SEQ ID NO:740 |
| Foe443.V1.3_at | GGGTTGCAGCTATTACACGCATGAT | SEQ ID NO:741 |
| Foe443.V1.3_at | GGAGTTATTTCAGGCCAATCATTTT | SEQ ID NO:742 |
| Foe443.V1.3_at | TGTCTGCCAGCACCAAGGACTTTCA | SEQ ID NO:743 |
| Foe443.V1.3_at | GGCCAATCATTTTTAACATCCTCTT | SEQ ID NO:744 |
| Equus caballus Toll-like receptor 4 mRNA complete cds | | |
| GI9717252-3_at | ATCTTTGACATCTTAGCCATCCTAA | SEQ ID NO:745 |
| G19717252-3_at | AGAAGGCTCCTGATTCAGATCCTCC | SEQ ID NO:746 |
| GI9717252-3_at | ACATCGTCTCCCAAGTCTTTTGAAT | SEQ ID NO:747 |
| GI9717252-3_at | ACAGGACTGCTAATCCCTTTGAGTT | SEQ ID NO:748 |
| GI9717252-3_at | AAACATCCTGGTCATTCTTTAGCGT | SEQ ID NO:749 |
| G19717252-3_at | AAGTCAGCTAAGGAGTCCGTGCCAG | SEQ ID NO:750 |
| GI9717252-3_at | GCTGCAACATACCAGGCATTGTGCT | SEQ ID NO:751 |
| GI9717252-3_at | GAATGGAAACCATCTCATCTTTGAC | SEQ ID NO:752 |
| GI9717252-3_at | GACTGGGCCCCAGTGAGTTCAGAAA | SEQ ID NO:753 |
| GI9717252-3_at | GGCAGGTGATTCTGTCGTGCACAAG | SEQ ID NO:754 |
| GI9717252-3_at | TCTCTGTTCAATTTTCCCTTTTCTA | SEQ ID NO:755 |
| Equus caballus Toll-like receptor 4 mRNA, complete cds | | |
| GI9717252-3M_at | ATAAGTTCTATTTCCACCTGATGCT | SEQ ID NO:756 |
| GI9717252-3M_at | AGAGACTTCATTCCTGGTGTGGCCA | SEQ ID NO:757 |
| GI9717252-3M_at | AAAGTTATTGTCGTGGTGTCCCAGC | SEQ ID NO:758 |
| GI9717252-3M_at | CAGCACTTCATTCAGAGCCGATGGT | SEQ ID NO:759 |
| GI9717252-3M_at | GCGGGTCGGTTTTCAGTATACTCAT | SEQ ID NO:760 |
| GI9717252-3M_at | CTGATGCTTCTTGCTGGCTGCAAAA | SEQ ID NO:761 |
| GI9717252-3M_at | GCATGCCCGTGCTGGGTTTTAACAA | SEQ ID NO:762 |

(continued)

| Probe Set Name | Sequence | Sequence Identifier |
|---|---|---|
| ILT11A mRNA for immunoglobulin-like transcript 11 protein | | |
| GI9717252-3M_at | GATGCCTTTGTTATCTACTCAAGCC | SEQ ID NO:763 |
| GI9717252-3M_at | GGTGTGTGCAATACCCTTACAGATG | SEQ ID NO:764 |
| GI9717252-3M_at | TGAGTATGAGATTGCCCAGACCTGG | SEQ ID NO:765 |
| GI9717252-3M_at | TTAACAATGCCACCTGTCAGATTAG | SEQ ID NO:766 |
| Homo sapiens potassium channel tetrarisation domain containing 12 (KCTD12) | | |
| WBC001D02_V1.3_at | ATATAGAAGCTCCATGGGCTGCCCA | SEQ ID NO:767 |
| WBC001D02_V1.3_at | GAGGGAAACGACTCCTTGTCTTTCA | SEQ ID NO:768 |
| WBC001D02_V1.3_at | AGGAGACCTGGTTGCCTTATACCTT | SEQ ID NO:769 |
| WBC001D02_V1.3_at | AGATTCATGCGATCCAGTCTGTATA | SEQ ID NO:770 |
| WBC001D02_V1.3_at | CTGTGATGACCGAGAAGCCTTTCTT | SEQ ID NO:771 |
| WBC001D02_V1.3_at | GAACACGGAATAAACTCCCACTGCA | SEQ ID NO:772 |
| WBC001D02_V1.3_at | GGCTGCCCATCTGTAGATAGCTGTA | SEQ ID NO:773 |
| WBC001D02_V1.3_at | GGCTCCTTTGGATATTCTGTGATGA | SEQ ID NO:774 |
| WBC001D02_V1.3_at | TGCACATCCTGTCTACACCATAAGT | SEQ ID NO:775 |
| WBC001D02_V1.3_at | TTGCCTTATACCTTTACGCGAACAC | SEQ ID NO:776 |
| WBC001D02_V1.3_at | TTGTCTTTCATGCTGTGAGGCTCCT | SEQ ID NO:777 |
| Homo sapiens protein tyrosine phosphatase type IVA, member 2 | | |
| WBC001F10_V1.3_at | ATCAGTGCCTGCCTGCGTTAGGAGA | SEQ ID NO:778 |
| WBC001F10_V1.3_at | AGAACTGGATTTTCTTCCTCAAAAT | SEQ ID NO:779 |
| WBC001F10_V1.3_at | AAACTTTTCCCTTTGACATGCTCTG | SEQ ID NO:780 |
| WBC001F10_V1.3_at | CAGCATGTCCTGTAAGCTCAGATAG | SEQ ID NO:781 |
| WBC001F10_V1.3_at | CATGCTCTGATTTTTGTTGTTTGGG | SEQ ID NO:782 |
| WBC001F10_V1.3_at | GACTCCAAGGGTTTTGATCAGTATC | SEQ ID NO:783 |
| WBC001F10_V1.3_at | GATAGACTCTGTTGACTCCAAGGGT | SEQ ID NO:784 |
| WBC001F10_V1.3_at | GTGTGCAGCCTGATTTAAAACCAAC | SEQ ID NO:785 |
| WBC001F10_V1.3_at | GTGTGTGCGGTGTCCACCAGTATCA | SEQ ID NO:786 |
| WBC001F10_V1.3_at | GGAGAATTACATTCCTGGTTCTGTA | SEQ ID NO:787 |
| WBC001F10_V1.3_at | TTGCTAGCTGAAGCCAGTTTGACAT | SEQ ID NO:788 |
| Homo sapiens retinoblastoma-like 2 (p130) mRNA | | |
| WBC001F11_V1.3_at | ATTAAGAGGGATCAGCTGGCTAAGT | SEQ ID NO:789 |
| WBC001F11_V1.3_at | ATATCTTTGAGTGTGTTCCTGGCAG | SEQ ID NO:790 |
| WBC001F11_V1.3_at | ATCTTCTTTGATGCTTTTGTACTTT | SEQ ID NO:791 |
| WBC001F11_V1.3_at | TTGTTAAAGCCCCAGTAGCCACCTT | SEQ ID NO:792 |
| WBC001F11_V1.3_at | AAATTATGACCTCTTCCTTTAGGAG | SEQ ID NO:793 |
| WBC001F11_V1.3_at | AAACCTCTCAGATACTGCTACTGTA | SEQ ID NO:794 |
| WBC001F11_V1.3_at | GCTATTGTTCCAGCAGTTTTAACGT | SEQ ID NO:795 |
| WBC001F11_V1.3_at | GAAATTCTCCAGTTTTTGATTATTA | SEQ ID NO:796 |

(continued)

| Probe Set Name | Sequence | Sequence Identifier |
|---|---|---|
| ILT11A mRNA for immunoglobulin-like transcript 11 protein | | |
| WBC001F11_V1.3_at | GTAGCCACCTTTTGGGCATATTTGA | SEQ ID NO:797 |
| WBC001F11_V1.3_at | TACTAGGTAACTTCACATTGCTCTG | SEQ ID NO:798 |
| WBC001F11_V1.3_at | TATACACCTTTATTAATCGCTATTG | SEQ ID NO:799 |
| Homo sapiens TRAF-interacting protein with a forkhead associated domain mRNA (cDNA clone MGC: 20791 | | |
| WBC004E04_V1.3_at | ACGAAGTGGTGTGCCGGTACTGATC | SEQ ID NO:800 |
| WBC004E04_V1.3_at | CAACGGTCATTTGTTTTCAAGGTCA | SEQ ID NO:801 |
| WBC004E04_V1.3_at | ACTAGTGTTGTAGCCTGTTGGTACT | SEQ ID NO:802 |
| WBC004E04_V1.3_at | AAGGAACTCTCTCTTGGTTTGGTCA | SEQ ID NO:803 |
| WBC004E04_V1.3_at | GTTAGTCATCGATTTGGTCTCCTGT | SEQ ID NO:804 |
| WBC004E04_V1.3_at | GTACTTATAGTACCAGTGTCCTGGA | SEQ ID NO:805 |
| WBC004E04_V1.3_at | GGTCATATTATTTCACAACGGTCAT | SEQ ID NO:806 |
| WBC004E04_V1.3_at | TGTTATTGTTATCTGCTGTGCTGGC | SEQ ID NO:807 |
| WBC004E04_V1.3_at | TCTCCTGTTGCATTCCTGGATGTAT | SEQ ID NO:808 |
| WBC004E04_V1.3_at | TCCCCTTCGTTACTGTGGTATGTTA | SEQ ID NO:809 |
| WBC004E04_V1.3_at | TAGACGCTCCGCTTACGAAGTGGTG | SEQ ID NO:810 |
| Homo sapiens neural precursor cell expressed developmentally down-regulated 1 | | |
| WBC004G01_V1.3_at | ATTCTTTGTGTGCTGTGTGTATTAT | SEQ ID NO:811 |
| WBC004G01_V1.3_at | AACTATACCTTGTATACCGCACTCC | SEQ ID NO:812 |
| WBC004G01_V1.3_at | AAGTGTGTTTGCCTACATGCTGTAT | SEQ ID NO:813 |
| WBC004G01_V1.3_at | CTTCTTGTAGTCTCTTCTTCCAAAG | SEQ ID NO:814 |
| WBC004G01_V1.3_at | CTGCCCCATTTTGACTTCTGAGATG | SEQ ID NO:815 |
| WBC004G01_V1.3_at | GCCTTTTTCTATACATGCACTTAAC | SEQ ID NO:816 |
| WBC004G01_V1.3_at | GATCCATTATTATTTTCCTATTGCC | SEQ ID NO:817 |
| WBC004G01_V1.3_at | GTGATCTGCAGCTTCAGTGGTGTAA | SEQ ID NO:818 |
| WBC004G01_V1.3_at | GTGTACATCTGCTTGTGTGATCTGC | SEQ ID NO:819 |
| WBC004G01_V1.3_at | GTGCTAATGTGATCTTTCTCCTGAC | SEQ ID NO:820 |
| WBC004G01_V1.3_at | TACTGGCGTGTTTACCTTCTTGTAG | SEQ ID NO:821 |
| No homology | | |
| WBC004G09_V1.3_at | TCACCAGTCAGTTGCTCACAAAGTC | SEQ ID NO:822 |
| WBC004G09_V1.3_at | ATGCAGATCATACCACCCCAAAGTA | SEQ ID NO:823 |
| WBC004G09_V1.3_at | AGTATTTTAATTGCACCTTAGTGAA | SEQ ID NO:824 |
| WBC004G09_V1.3_at | AAGGTAACTTGTCCAAAGCCAGAAT | SEQ ID NO:825 |
| WBC004G09_V1.3_at | AAAACTAGTATGCTGGCTTGAAATA | SEQ ID NO:826 |
| WBC004G09_V1.3_at | AAGAATCTAGACTTCACCAGTCAGT | SEQ ID NO:827 |
| WBC004G09_V1.3_at | CTTTACACTGTTACCTGCTTAGGAA | SEQ ID NO:828 |

(continued)

| Probe Set Name | Sequence | Sequence Identifier |
|---|---|---|
| ILT11A mRNA for immunoglobulin-like transcript 11 protein | | |
| WBC004G09_V1.3_at | GAGGGAGATAATAGCCCTCCCACCA | SEQ ID NO:829 |
| WBC004G09_V1.3_at | GTTTGAACAGTTGTGTCCAGATTGA | SEQ ID NO:830 |
| WBC004G09_V1.3_at | TACCACCCCAAAGTAGCTCTTTAAT | SEQ ID NO:831 |
| WBC004G09_V1.3_at | TACTGGAATCCTTTACACTGTTACC | SEQ ID NO:832 |
| No homology | | |
| WBC005D09_V1.3_at | AAATCACTTTTCTTTCCCACTGACT | SEQ ID NO:833 |
| WBC005D09_V1.3_at | CACTGACTGTTTTTTCTGCTTGTTG | SEQ ID NO:834 |
| WBC005D09_V1.3_at | CTTGTGGGTATTGCTGTCTTGTTTT | SEQ ID NO:835 |
| WBC005D09_V1.3_at | CTGCCCATTGGCCTGAAAATACTCA | SEQ ID NO:836 |
| WBC005D09_V1.3_at | CTCCTTATCGCTTACAGTGTTCTTA | SEQ ID NO:837 |
| WBC005D09_V1.3_at | GAATGAAATTTCCTACGCACTACTG | SEQ ID NO:838 |
| WBC005D09_V1.3_at | GAGAACCTCGTGTCTGTTCTGATTC | SEQ ID NO:839 |
| WBC005D09_V1.3_at | GATATGTTCTTGTATTGACTGCCCA | SEQ ID NO:840 |
| WBC005D09_V1.3_at | TCCTTCCCTGATGCTAAATTTCCAA | SEQ ID NO:841 |
| WBC005D09_V1.3_at | TAAGGTCCCTTCTAACTTTCATGTC | SEQ ID NO:842 |
| WBC005D09_V1.3_at | TTAGGCAATCTTTCTTCTCCTTATC | SEQ ID NO:843 |
| No homology | | |
| WBC005E06_V1.3_at | ATTAGCTCTGTGGTGTCAGGCTCTA | SEQ ID NO:844 |
| WBC005E06_V1.3_at | AGTGCATCTGTGTATCCTGGTTTAA | SEQ ID NO:845 |
| WBC005E06_V1.3_at | AGCCATTTAAGGGTGGACCTCCACG | SEQ ID NO:846 |
| WBC005E06_V1.3_at | AGCCTTTTCAGCCTCCATTTTGAAG | SEQ ID NO:847 |
| WBC005E06_V1.3_at | AAGAGGACCTCTGACTTGCTTGAGA | SEQ ID NO:848 |
| WBC005E06_V1.3_at | CAGGCTCTAGAGTTACTTCTTGTCA | SEQ ID NO:849 |
| WBC005E06_V1.3_at | GCATAGTTTGCAGCCGTGTGGTAGT | SEQ ID NO:850 |
| WBC005E06_V1.3_at | GAAAATTAGCTCTCTGCCATGGTTT | SEQ ID NO:851 |
| WBC005E06_V1.3_at | GAGTTCTGTCAGACTTTTCCTTTTT | SEQ ID NO:852 |
| WBC005E06_V1.3_at | GATTGTTCCTTGCATTTCTGAGTAT | SEQ ID NO:853 |
| WBC005E06_V1.3_at | GGACCTCCACGACGATTCTGAAAAT | SEQ ID NO:854 |
| Homo sapiens vaccinia related kinase 2, mRNA (cDNA clone MGC:34431 IMAGE:5221315) | | |
| WBC005F01_V1.3_at | AGTCCAGGACCATACAGCTTCACAG | SEQ ID NO:855 |
| WBC005F01_V1.3_at | CAACCTACCAACACTGAATCATCAT | SEQ ID NO:856 |
| WBC005F01_V1.3_at | GAAACCCATCCAGATTTTACCAGTG | SEQ ID NO:857 |
| WBC005F01_V1.3_at | GTTCTACAGGATTTTGGCTTACTCA | SEQ ID NO:858 |
| WBC005F01_V1.3_at | GTGGCATACAATTCCCAAATTCATT | SEQ ID NO:859 |
| WBC005F01_V1.3_at | GGTATACATATGCTTCTACGCCTGG | SEQ ID NO:860 |
| WBC005F01_V1.3_at | GGCAGATGTGTATTATTATGGCTTC | SEQ ID NO:861 |
| WBC005F01_V1.3_at | GGCTTCATCATTCTTTTTCTTTTGG | SEQ ID NO:862 |

(continued)

| Probe Set Name | Sequence | Sequence Identifier |
|---|---|---|
| ILT11A mRNA for immunoglobulin-like transcript 11 protein | | |
| WBC005F01_V1.3_at | GGCTTACTCAGTTTGCTCTTAGTGA | SEQ ID NO:863 |
| WBC005F01_V1.3_at | TGTCTTGCTTTATATTTTCTCTGAA | SEQ ID NO:864 |
| WBC005F01_V1.3_at | TTCTACGCCTGGTATGGAGGTCACA | SEQ ID NO:865 |
| Polymeric immunoglobulin receptor 3 precursor (PIGR3) | | |
| WBC005F10_V1.3_at | TTTCGACTGGCGCAACGTTTGGTAA | SEQ ID NO:866 |
| WBC005F10_V1.3_at | ATAACTTTTGTACCGTGCCCTCTAT | SEQ ID NO:867 |
| WBC005F10_V1.3_at | AGTGGTTAAGGCTTCCTATCCAGAA | SEQ ID NO:868 |
| WBC005F10_V1.3_at | AGCCCAGCTTCCTGAGTACCAATAA | SEQ ID NO:869 |
| WBC005F10_V1.3_at | CAGTGACTGACCCATATGTAGCAAA | SEQ ID NO:870 |
| WBC005F10_V1.3_at | GCCTGCAGCGTGAGAAGACTTCCCA | SEQ ID NO:871 |
| WBC005F10_V1.3_at | GCTGCTCACTCACTGTGGGATGTTA | SEQ ID NO:872 |
| WBC005F10_V1.3_at | GATAATACCCACTTTCTAGGATTGT | SEQ ID NO:873 |
| WBC005F10_V1.3_at | GATGTTATGAAAGCGACCCTTCCAA | SEQ ID NO:874 |
| WBC005F10_V1.3_at | TAACGTTTGACCCTGAGGCCCAGAG | SEQ ID NO:875 |
| WBC005F10_V1.3_at | TTCCTTCTGGATCGTTTCTCCAAGA | SEQ ID NO:876 |
| No Homology | | |
| WBC007A08_V1.3_at | ATGTATGAGACAGCTTCCTCCATTG | SEQ ID NO:877 |
| WBC007A08_V1.3_at | AGTGCCCTTTCAGTATACCGTGTTG | SEQ ID NO:878 |
| WBC007A08_V1.3_at | AAATGCTTTCTCTGTGCTTTCGTTA | SEQ ID NO:879 |
| WBC007A08_V1.3_at | AAACAAATCTCTTCTACCAGCTTGC | SEQ ID NO:880 |
| WBC007A08_V1.3_at | AATCCAGGTCTTCTGATTCTCATTC | SEQ ID NO:881 |
| WBC007A08_V1.3_at | AATCAGTCGAGGTCAGCAGTTTGTA | SEQ ID NO:882 |
| WBC007A08_V1.3_at | GCTTGCACTCAGACCAACTTGGAAA | SEQ ID NO:883 |
| WBC007A08_V1.3_at | GAATTGTTGTTCTTCCTCTTTCCTA | SEQ ID NO:884 |
| WBC007A08_V1.3_at | GAGCCCTCTGTCTGTGTTTTCATCA | SEQ ID NO:885 |
| WBC007A08_V1.3_at | GATTGCAGCTCCTTATTTACTGGAA | SEQ ID NO:886 |
| WBC007A08_V1.3_at | GTATACCGTGTTGCCTCTAAAGATT | SEQ ID NO:887 |
| Homo sapiens mRNA for enolase 1 variant, clone adSE00169 | | |
| WBC007F03_V1.3_at | ATTGTCATCTCTGAGGTGTCCACAG | SEQ ID NO:888 |
| WBC007F03_V1.3_at | AGGTCCCCAGAGGTTTTGTGTGCAA | SEQ ID NO:889 |
| WBC007F03_V1.3_at | AAATCATTGTTTTTCTCACCTCGCT | SEQ ID NO:890 |
| WBC007F03_V1.3_at | GAAACCCTGGCTCTGTAATCATGTG | SEQ ID NO:891 |
| WBC007F03_V1.3_at | GATCAAGACTGGTGCACCTTGCCGA | SEQ ID NO:892 |
| WBC007F03_V1.3_at | GTCTGGAGCCGTGTGTTGTATCTAC | SEQ ID NO:893 |
| WBC007F03_V1.3_at | GGTAGTTGTTAACTGGCCTCTCCTA | SEQ ID NO:894 |
| WBC007F03_V1.3_at | GGCCAAGTACAACCAGATCCTCAGA | SEQ ID NO:895 |
| WBC007F03_V1.3_at | TGCAGTCACTGGTCAGCTCATTAGA | SEQ ID NO:896 |

(continued)

| Probe Set Name | Sequence | Sequence Identifier |
|---|---|---|
| ILT11A mRNA for immunoglobulin-like transcript 11 protein | | |
| WBC007F03_V1.3_at | TTTGCCGGCAGGAACTTCAGAAACC | SEQ ID NO:897 |
| WBC007F03_V1.3_at | TACTGTTCTCACTGCTTCCTTAGAA | SEQ ID NO:898 |
| Homo sapiens matrix metalloproteinase 8 (neutrophil collegenase) (MMP8) mRNA | | |
| WBC008A10_V1.3_at | AAAGCTATGTGCAAGTCCCAACCTC | SEQ ID NO:899 |
| WBC008A10_V1.3_at | AAATCTGGTGCTATTTTCCACTCTT | SEQ ID NO:900 |
| WBC008A10_V1.3_at | AAACACGTTTTAAGGCTATCTGCTA | SEQ ID NO:901 |
| WBC008A10_V1.3_at | AAGAACATGGCTCTTCTGACGCCTT | SEQ ID NO:902 |
| WBC008A10_V1.3_at | AAGGAAGGATCCTCCCTAGAGCCTT | SEQ ID NO:903 |
| WBC008A10_V1.3_at | CCAACCTCGGGTACCTATGAATGTA | SEQ ID NO:904 |
| WBC008A10_V1.3_at | GCTCCTATATTCCAAAGTCTCTTTA | SEQ ID NO:905 |
| WBC008A10_V1.3_at | GATAGTACTCCTTGCGTTTATTGAA | SEQ ID NO:906 |
| WBC008A10_V1.3_at | GTGAGTTGAATTGTGTTCCCCAAAA | SEQ ID NO:907 |
| WBC008A10_V1.3_at | TGACGCCTTGATTTTGCACTCACAG | SEQ ID NO:908 |
| WBC008A10_V1.3_at | TTCGTCACCTCTCTATTCATTATTT | SEQ ID NO:909 |
| Homo sapiens cDNA FLJ33312 fis, clone BNGH42005968; weakly similar to DIPZ PROTEIN | | |
| WBC008A12_V1.3_at | ATCTGTCAGCGTGTTTCTTTACTAC | SEQ ID NO:910 |
| WBC008A12_V1.3_at | AGTGGCTGCATCTCCAGGACAGACT | SEQ ID NO:911 |
| WBC008A12_V1.3_at | GAAGACATTACCCAGACTACCTAAA | SEQ ID NO:912 |
| WBC008A12_V1.3_at | AAGCTGAGATTAGACCTCCCATCGG | SEQ ID NO:913 |
| WBC008A12_V1.3_at | GTAGCGCAGACAGCAGTGCCTGTAT | SEQ ID NO:914 |
| WBC008A12_V1.3_at | GTAGATGGCCCATTCCCAGAAGAAA | SEQ ID NO:915 |
| WBC008A12_V1.3_at | GGAATTTTGTTCAGTCAGCCTTTAC | SEQ ID NO:916 |
| WBC008A12_V1.3_at | GGAGCACCCAGTTGCTGGTTTCTAA | SEQ ID NO:917 |
| WBC008A12_V1.3_at | TGCTGAGTTCTAGCCACTGATCTTA | SEQ ID NO:918 |
| WBC008A12_V1.3_at | TAGAGAGCATTTCCAATCAACCCAC | SEQ ID NO:919 |
| WBC008A12_V1.3_at | TTTATTTCTGCCTCTGGATACCAAG | SEQ ID NO:920 |
| Homo sapiens cDNA: FLJ21472 fis, clone, COL04936. | | |
| WBC008D09_V1.3_at | ATTTCTACAAGTCAACCTCCAATTC | SEQ ID NO:921 |
| WBC008D09_V1.3_at | AGTTTCACTGAGTTTGCTGGCTTTT | SEQ ID NO:922 |
| WBC008D09_V1.3_at | AGCCTCCTACAGTGCTTTAGTCATA | SEQ ID NO:923 |
| WBC008D09_V1.3_at | AAATGCTCATACATGTTCCCTTTTC | SEQ ID NO:924 |
| WBC008D09_V1.3_at | CACATAATGCTTTCTTTTTGGTCTT | SEQ ID NO:925 |
| WBC008D09_V1.3_at | CATCATCCATGCCTTATCAATTTCT | SEQ ID NO:926 |
| WBC008D09_V1.3_at | GCTATGTGTGAAAGAGCCTCCTACA | SEQ ID NO:927 |
| WBC008D09_V1.3_at | GAGCCTTAATTTCGTCCCTATTGAA | SEQ ID NO:928 |
| WBC008D09_V1.3_at | TGCCTGCATAGACTTACCCAGATGT | SEQ ID NO:929 |
| WBC008D09_V1.3_at | TATAAAACCGGTTAGTGTCCTACAG | SEQ ID NO:930 |

(continued)

| Probe Set Name | Sequence | Sequence Identifier |
|---|---|---|
| ILT11A mRNA for immunoglobulin-like transcript 11 protein | | |
| WBC008D09_V1.3_at | TATTTCCCAGGAGTCAGATGGCCCT | SEQ ID NO:931 |
| Homo sapiens integrin, beta 1 (fibronectin receptor, beta polypeptide, antigen CD29 includes MDF2, MSK12) transcript variant 1A, mRNA (cDNA clone MGC:17220 IMAGE 3924411) | | |
| WBC009F04_V1.3_at | ATCTTGGTAACTTTTATGCCTTCTT | SEQ ID NO:932 |
| WBC009F04_V1.3_at | AGGCCTTAATTTCCATAGTACTTGC | SEQ ID NO:933 |
| WBC009F04_V1.3_at | AGTGCCGTAATAGACCGCTGTATAG | SEQ ID NO:934 |
| WBC009F04_V1.3_at | AGTTACTTCTCACTGTCAGAGCTGC | SEQ ID NO:935 |
| WBC009F04_V1.3_at | ACCTGTGTGCCATTTTGAGAGTTAC | SEQ ID NO:936 |
| WBC009F04_V1.3_at | AATTCGCCTCTTAGTGAACGTGTGA | SEQ ID NO:937 |
| WBC009F04_V1.3_at | GCGACTCTTATTTTCAGGCAGTGCC | SEQ ID NO:938 |
| WBC009F04_V1.3_at | TGAAGCCTGGTGCTTTTTACCACCT | SEQ ID NO:939 |
| WBC009F04_V1.3_at | GGCTTACGCAACAGCTGTCACTTAA | SEQ ID NO:940 |
| WBC009F04_V1.3_at | TTTCATACCAGTCACATCCTTGTTT | SEQ ID NO:941 |
| WBC009F04_V1.3_at | TCAGAGCTGCCCATTTTGTTTCATA | SEQ ID NO:942 |
| No Homology | | |
| WBC009H07_V1.3_at | AGCATTTCATGTGTCTAGTGTGGCA | SEQ ID NO:943 |
| WBC009H07_V1.3_at | AGCCACCCGATGTTGCGTTTGGAAG | SEQ ID NO:944 |
| WBC009H07_V1.3_at | ACAGTATGGCTGTTTGCTTTGTCCT | SEQ ID NO:945 |
| WBC009H07_V1.3_at | AAACTCGTTTTGCTTCTTTGTATAT | SEQ ID NO:946 |
| WBC009H07_V1.3_at | CCAGGTGACATTCTTTCCAGGTACA | SEQ ID NO:947 |
| WBC009H07_V1.3_at | GCCTTGGGTTTATTGTGCACAGTTT | SEQ ID NO:948 |
| WBC009H07_V1.3_at | GCGAGATTTAGTACACCTGCACAGT | SEQ ID NO:949 |
| WBC009H07_V1.3_at | GCACATCCTGTTCACCTGTTGGTGA | SEQ ID NO:950 |
| WBC009H07_V1.3_at | GAAGTGTTTGTTGCTCCCGTTGGAG | SEQ ID NO:951 |
| WBC009H07_V1.3_at | GGCGTTCTTAGAAAGTACCCTGTCT | SEQ ID NO:952 |
| WBC009H07_V1.3_at | GGCATTTCTGGATGCGGCTGTCACA | SEQ ID NO:953 |
| Homo sapiens nuclear autoantigen GS2NA mRNA | | |
| WBC011D03_V1.3_at | AAGTCAGATTTTTGCCCATAGTGGA | SEQ ID NO:954 |
| WBC011D03_V1.3_at | AAGTGAGTCCATGACAGCTTCTCTG | SEQ ID NO:955 |
| WBC011D03_V1.3_at | CACCTGCCGGGATTTCATACTTAAA | SEQ ID NO:956 |
| WBC011D03_V1.3_at | AATTCACTCTCAGTTTGTAAGCCCC | SEQ ID NO:957 |
| WBC011D03_V1.3_at | GCAGACCCTTCATATCTTGGATACG | SEQ ID NO:958 |
| WBC011D03_V1.3_at | CTGCACACCTTACATTTCACAATTT | SEQ ID NO:959 |
| WBC011D03_V1.3_at | CCCACGTCAGGATCATGTTTCTTTA | SEQ ID NO:960 |
| WBC011D03_V1.3_at | GAAAAGCTACATGCCAACTACCAGA | SEQ ID NO:961 |
| WBC011D03_V1.3_at | GTATGTATTACCTGTCTGTCGAGGA | SEQ ID NO:962 |
| WBC011D03_V1.3_at | GTGACTGTTTGCTGTGTATGTGCCA | SEQ ID NO:963 |

(continued)

| Probe Set Name | Sequence | Sequence Identifier |
|---|---|---|
| ILT11A mRNA for immunoglobulin-like transcript 11 protein | | |
| WBC011D03_V1.3_at | GGAGATGCTGCATAAGGCCCCACGT | SEQ ID NO:964 |
| No Homology | | |
| WBC011F05_V1.3_at | ATAACCTGTTTCCTCTAAGATCTTG | SEQ ID NO:965 |
| WBC011F05_V1.3_at | ACAGGGTGTTTCCACTTGTGTAGCA | SEQ ID NO:966 |
| WBC011F05_V1.3_at | AAAACTCTACTAGGGCTCTTGCTGA | SEQ ID NO:967 |
| WBC011F05_V1.3_at | AAAAGGCATCTTTGTTCTCAACAGA | SEQ ID NO:968 |
| WBC011F05_V1.3_at | AAGCAGACGTCTCTGAGCGTTTCCA | SEQ ID NO:969 |
| WBC011F05_V1.3_at | GAGCGTTTCCAGTGTTTTATCCAAA | SEQ ID NO:970 |
| WBC011F05_V1.3_at | GCTGACTTCAGTGCCTTTTGTAAAT | SEQ ID NO:971 |
| WBC011F05_V1.3_at | GAGATTCTTTCTCCTCGTTAAGTTT | SEQ ID NO:972 |
| WBC011F05_V1.3_at | GAGGATGCCCAAGACCGAGCAACAG | SEQ ID NO:973 |
| WBC011F05_V1.3_at | GATTTTCCTCAACTTGCTTTTTGGG | SEQ ID NO:974 |
| WBC011F05_V1.3_at | TAAGATCTTGCTATACTGTCCTGTA | SEQ ID NO:975 |
| No Homology | | |
| WBC012A03_V1.3_at | ATCTATTTGGTTAGGCCTAGGCCAT | SEQ ID NO:976 |
| WBC012A03_V1.3_at | ATCGCCCCTTTTTATTCAACATCTG | SEQ ID NO:977 |
| WBC012A03_V1.3_at | AGTGCCCTGAGTGATGTCCAGTTCC | SEQ ID NO:978 |
| WBC012A03_V1.3_at | AGTCCCCATATGTGTTAATGTCTCA | SEQ ID NO:979 |
| WBC012A03_V1.3_at | AGCGTGGACTGTGCCTGAGACCGAC | SEQ ID NO:980 |
| WBC012A03_V1.3_at | CTAGGCCATGCCTTGGCTGAGTATA | SEQ ID NO:981 |
| WBC012A03_V1.3_at | GTCCAGTTCCATTAGACACTCTGTC | SEQ ID NO:982 |
| WBC012A03_V1.3_at | GGTGACTGTGAGAAATCGCCCCTTT | SEQ ID NO:983 |
| WBC012A03_V1.3_at | TGTACAGAGGCATTCAGCGTGGACT | SEQ ID NO:984 |
| WBC012A03_V1.3_at | TAGTCTGTACGTGAACTGCTCCAAG | SEQ ID NO:985 |
| WBC012A03_V1.3_at | TTTACTCTGTTAGGAATCTTCTCGA | SEQ ID NO:986 |
| H. sapiens mRNA for inwardly rectifing potassium channel Kir4.2. | | |
| WBC012C10_V1.3_at | AACGTCTGACGGCAGTGGTGTCACT | SEQ ID NO:987 |
| WBC012C10_V1.3_at | CTTTTCCTTTGATCTGGTGGCTAAA | SEQ ID NO:988 |
| WBC012C10_V1.3_at | GCATTTCCATGTTTGAGAGGCTTCC | SEQ ID NO:989 |
| WBC012C10_V1.3_at | GCACCTTTTATTGCGCAGATGCTGA | SEQ ID NO:990 |
| WBC012C10_V1.3_at | GAGGACCCTTTTGTTACAGCAGAGC | SEQ ID NO:991 |
| WBC012C10_V1.3_at | GAGGCTTCCTTTTAAATGCTTTGTC | SEQ ID NO:992 |
| WBC012C10_V1.3_at | GTGGATGCACTCTCAAAAAACCTCA | SEQ ID NO:993 |
| WBC012C10_V1.3_at | GGAAGAGCCCGGACTGCACCTTTTA | SEQ ID NO:994 |
| WBC012C10_V1.3_at | GGCTACAAACTTGATCTTTTCCTTT | SEQ ID NO:995 |
| WBC012C10_V1.3_at | TGGTGTCACTGCTGTTTAACTCTGC | SEQ ID NO:996 |
| WBC012C10_V1.3_at | TTAACTCTGCGAGCTGTTTCCACAT | SEQ ID NO:997 |

(continued)

| Probe Set Name | Sequence | Sequence Identifier |
|---|---|---|
| ILT11A mRNA for immunoglobulin-like transcript 11 protein | | |
| No homology. | | |
| WBC012E05_V1.3_at | ATGTACCTGCTGATCATCACTGTGT | SEQ ID NO:998 |
| WBC012E05_V1.3_at | AAATCTTTCAATAACCTGCCAGTGG | SEQ ID NO:999 |
| WBC012E05_V1.3_at | GACCGTCCTGACTGTATATTTTCAA | SEQ ID NO:1000 |
| WBC012E05_V1.3_at | GACGTTGTCTGTGATGGTGTCCACG | SEQ ID NO:1001 |
| WBC012E05_V1.3_at | GAGATCATGCTTGCGTTACTAGGAC | SEQ ID NO:1002 |
| WBC012E05_V1.3_at | GTCTGAGCCTCATGTACCTATTTGA | SEQ ID NO:1003 |
| WBC012E05_V1.3_at | GTATTCTGACTCTAACATGGACTTT | SEQ ID NO:1004 |
| WBC012E05_V1.3_at | GGATATGCTCACTGATCTGTGCTTA | SEQ ID NO:1005 |
| WBC012E05_V1.3_at | TCAGCCTTGCTGGATTTGGAGTCTT | SEQ ID NO:1006 |
| WBC012E05_V1.3_at | TTGTACATCTCACTGCTTAGCTTAG | SEQ ID NO:1007 |
| WBC012E05_V1.3_at | TTCTCTTCAGGTGCAGCCTTACTGG | SEQ ID NO:1008 |
| Homo sapiens sialyltransferase 1 (beta-galactoside alpha-2,6-sialyltransferase), transcript variant 2 mRNA | | |
| WBC013A09_V1.3_at | AGCTCAGGCCCGATCTGCTTGGGAA | SEQ ID NO:1009 |
| WBC013A09_V1.3_at | AGCCCATCTCTCTGAAGGGATTTAT | SEQ ID NO:1010 |
| WBC013A09_V1.3_at | AATTCCTGGCTCTCTTTATGTTCTG | SEQ ID NO:1011 |
| WBC013A09_V1.3_at | GCCGCTTACTGCTGCGTGAACTAAT | SEQ ID NO:1012 |
| WBC013A09_V1.3_at | GAATTGCAGGAAAGCCCATCTCTCT | SEQ ID NO:1013 |
| WBC013A09_V1.3_at | GAAGGTGGCATTCCATCAGCAGAAG | SEQ ID NO:1014 |
| WBC013A09_V1.3_at | GTAGCCATCTATTCAGCCTATATCA | SEQ ID NO:1015 |
| WBC013A09_V1.3_at | GTGAACTAATCTTTTCCACCTCTCT | SEQ ID NO:1016 |
| WBC013A09_V1.3_at | TGGTGTGGGACCCTGAGACTACACT | SEQ ID NO:1017 |
| WBC013A09_V1.3_at | TCATGATATCCTCTAATCCTTCCAA | SEQ ID NO:1018 |
| WBC013A09_V1.3_at | TTAATTCTGTGCTCTCTCTATATGG | SEQ ID NO:1019 |
| Homo sapiens nucleosome assembly protein 1 like 2 mRNA | | |
| WBC013A11_V1.3_at | ATCCTTGCTGCAGACTTTGAAATTG | SEQ ID NO:1020 |
| WBC013A11_V1.3_at | AACAGTTTCCAATGACTCTTTCTTT | SEQ ID NO:1021 |
| WBC013A11_V1.3_at | TACGTGAGCGTATAATCCCAAGATC | SEQ ID NO:1022 |
| WBC013A11_V1.3_at | GATGCTGAAGCTATCCTTGCTGCAG | SEQ ID NO:1023 |
| WBC013A11_V1.3_at | GATTCTGATCCCTTTTCTTTTGATG | SEQ ID NO:1024 |
| WBC013A11_V1.3_at | GTTGTTGTTTGTTTGTTTGCTTGCT | SEQ ID NO:1025 |
| WBC013A11_V1.3_at | GTGGGACAGTTCGTACTGTGACCAA | SEQ ID NO:1026 |
| WBC013A11_V1.3_at | GGTCTTCTGCTTCCCTGGAAAGGAT | SEQ ID NO:1027 |
| WBC013A11_V1.3_at | TGAGGATAACCTGCACTGGTCTTCT | SEQ ID NO:1028 |
| WBC013A11_V1.3_at | TGCTTGCTTTTGTTTTTGCAGCCTA | SEQ ID NO:1029 |
| WBC013A11_V1.3_at | TTGCCCCTCCTGAAGTTCCTGAGAG | SEQ ID NO:1030 |

(continued)

| Probe Set Name | Sequence | Sequence Identifier |
|---|---|---|
| ILT11A mRNA for immunoglobulin-like transcript 11 protein | | |
| Homo sapiens nucleosome assembly protein 1-like 2 mRNA | | |
| WBC013E05_V1.3_at | ATCCGAGCCCTACGCTCAGGTGGTG | SEQ ID NO:1031 |
| WBC013E05_V1.3_at | AGGTGGTGCCTTAAGACGCCAAGGA | SEQ ID NO:1032 |
| WBC013E05_V1.3_at | AGCTCTCATACTCTTCAGAATTCTC | SEQ ID NO:1033 |
| WBC013E05_V1.3_at | AAGACTGATGATTCCTTCCTGAAGA | SEQ ID NO:1034 |
| WBC013E05_V1.3_at | GATGATTCCTTCCTGAAGATCAGTT | SEQ ID NO:1035 |
| WBC013E05_V1.3_at | TGTCTCCATGGAAGCAATAATTCTC | SEQ ID NO:1036 |
| WBC013E05_V1.3_at | TGCTAGATGACCTCGCACTATTTAC | SEQ ID NO:1037 |
| WBC013E05_V1.3_at | TAGGGACCTGATCCATGTCTCCATG | SEQ ID NO:1038 |
| MBC013E05_V1.3_at | TAATCTCTAGGGACCTGATCCATGT | SEQ ID NO:1039 |
| WBC013E05_V1.3_at | TACGCTCAGGTGGTGCCTTAAGACG | SEQ ID NO:1040 |
| WBC013E05_V1.3_at | TTTTCCTCCCCAAGACTGATGATTC | SEQ ID NO:1041 |
| Homo sapiens cDNA FLJ45679 fis, clone ERLTF2001835 | | |
| WBC013E10_V1.3_at | AGAGGATCTCTCTTTTGAAAAGCTA | SEQ ID NO:1042 |
| WBC013E10_V1.3_at | AAGAACACTTACAGCTCATATACAG | SEQ ID NO:1043 |
| WBC013E10_V1.3_at | AAGGAGTTTCCTGTGGTTTCTGCCG | SEQ ID NO:1044 |
| WBC013E10_V1.3_at | CGACTATTACTTTCTCTCAAGTGCC | SEQ ID NO:1045 |
| WBC013E10_V1.3_at | GAATATGTTCTCCTTTGTAGCCTCT | SEQ ID NO:1046 |
| WBC013E10_V1.3_at | GAAAAACACAGCTTAGCCCCTTCCT | SEQ ID NO:1047 |
| WBC013E10_V1.3_at | GTAGAATGACACCAAAGCTGCAACT | SEQ ID NO:1048 |
| WBC013E10_V1.3_at | GGAAATCAGTCAGTACCATCGTTAA | SEQ ID NO:1049 |
| WBC013E10_V1.3_at | TTGTAGCCTCTTGACAAAGCAGGAA | SEQ ID NO.1050 |
| WBC013E10_V1.3_at | TTGTCCACTGAAACTTTGCTAACGT | SEQ ID NO:1051 |
| WBC013E10_V1.3_at | TTCCTCTAGCCTTCAGTCAATTTTT | SEQ ID NO:1052 |
| No Homology | | |
| WBC014E06_V1.3_at | ATGTATGCGTGAGAAGGCCCCGTGA | SEQ ID NO:1053 |
| WBC014E06_V1.3_at | ATTTATTTTTGGTTCTCTGCTGTAA | SEQ ID NO:1054 |
| WBC014E06_V1.3_at | AGGCCCCGTGATATAATAGTGCTTA | SEQ ID NO:1055 |
| WBC014E06_V1.3_at | ACCACACTTCTGTTGTCTTTTCAAA | SEQ ID NO:1056 |
| WBC014E06_V1.3_at | GTTACCTGCCTCCAGAGTTCTAAAA | SEQ ID NO:1057 |
| WBC014E06_V1.3_at | GTATGCTCAGCACCTAATGCACATA | SEQ ID NO:1058 |
| WBC014E06_V1.3_at | GTATTTTCAAACAGCTTTTCAGGAT | SEQ ID NO:1059 |
| WBC014E06_V1.3_at | TCTGTTGTAATTGCGTGTCTGTCCT | SEQ ID NO:1060 |
| WBC014E06_V1.3_at | TAAAATCACCTTTACTGACGGTTGT | SEQ ID NO:1061 |
| WBC014E06_V1.3_at | TAGTGCTTAGCATTCTCTGTTGTAA | SEQ ID NO:1062 |
| WBC014E06_V1.3_at | TTTCCTCTTGTCCTTAAACTCCATA | SEQ ID NO:1063 |
| Homo sapiens purinegic receptor P2Y, G-protein coupled, 13, transcript variant 2 | | |

(continued)

| Probe Set Name | Sequence | Sequence Identifier |
|---|---|---|
| ILT11A mRNA for immunoglobulin-like transcript 11 protein | | |
| NBC014H06_V1.3_at | CTAACATTTGTATGGACCCCTTCAT | SEQ ID NO:1064 |
| WBC014H06_V1.3_at | CAGCAGGACTGATTGCCGGGTGCAA | SEQ ID NO:1065 |
| WBC014H06_V1.3_at | ACCGTTTTTGTCCTAATGCTTCTGT | SEQ ID NO:1066 |
| WBC014H06_V1.3_at | ACCTCTCTGCCAAACGTGATCTTAA | SEQ ID NO:1067 |
| WBC014H06_V1.3_at | AAATCACACCATCCAGACTGACAAT | SEQ ID NO:1068 |
| WBC014H06_V1.3_at | AACAACTCTCTTTTTGGCAGCAACT | SEQ ID NO:1069 |
| WBC014H06_V1.3_at | AAGCCACACCGTCGTCTGTGAGAAA | SEQ ID NO:1070 |
| WBC014H06_V1.3_at | CTTTGCTCCATTTCATTTTGCCAGA | SEQ ID NO:1071 |
| WBC014H06_V1.3_at | GAGAAAGTGCGCCTCCTTAAAGGGT | SEQ ID NO:1072 |
| WBC014H06_V1.3_at | GCTTCTGTTTTATGTGGTGATCGCA | SEQ ID NO:1073 |
| WBC014H06_V1.3_at | TAAAGTGTTCGTTGTGGTGGCCGTC | SEQ ID NO:1074 |
| Homo sapiens copine III | | |
| WBC015B08_V1.3_at | ATAGTGTGCAGTTAGCGTGGTCCCC | SEQ ID NO:1075 |
| WBC015B08_V1.3_at | AGGCTCTTCTGTGCT'PCTATGATAT | SEQ ID NO:1076 |
| WBC015B08_V1.3_at | AGTGCCTTGTTCTGATTTTTGTTCT | SEQ ID NO:1077 |
| WBC015B08_V1.3_at | AGAGGCAGCCTTTCCAGAGCAAATC | SEQ ID NO:1078 |
| WBC015B08_V1.3_at | AAGTCGTCACGATCGCGAGAGTTGA | SEQ ID NO:1079 |
| WBC015B08_V1.3_at | CTGTCTCTTTATGTGTGCGGTTTAT | SEQ ID NO:1080 |
| WBC015B08_V1.3_at | CCCGCTGCCTGTACTGAATAGTGTG | SEQ ID NO:1081 |
| WBC015B08_V1.3_at | GTATCTTCCGTTTGAATTCCCTTTG | SEQ ID NO:1082 |
| WBC015B08_V1.3_at | GTGCCTGGGCAGATGTAGTTTCTCC | SEQ ID NO:1083 |
| WBC015B08_V1.3_at | TGCTTTGCCTTTTTCGTTCTGTAAA | SEQ ID NO:1084 |
| WBC015B08_V1.3_at | TTTAATAAACTCCTCCTGTCTCTTT | SEQ ID NO:1085 |
| No Homology | | |
| WBC016A12_V1.3_at | ATGCAGATCAGATGGCGTTTCTCCC | SEQ ID NO:1086 |
| WBC016A12_V1.3_at | ACGTGTTCAGACTTGTGTTTTCATA | SEQ ID NO:1087 |
| WBC016A12_V1.3_at | AAGTATGGGCTGTTTCACGTGTTCA | SEQ ID NO:1088 |
| WBC016A12_V1.3_at | GACTATGTACTGTCCACCTGCATTA | SEQ ID NO:1089 |
| WBC016A12_V1.3_at | GATTGATTTCCTACTAGTCTTTGAC | SEQ ID NO:1090 |
| WBC016A12_V1.3_at | GTTTCTCCCAGCTCAGTGGATTTTC | SEQ ID NO:1091 |
| WBC016A12_V1.3_at | GGAGCCTGGCTTATTTGATTGATTT | SEQ ID NO:1092 |
| WBC016A12_V1.3_at | GGAAGTCCCTGTTCTGACTATGTAC | SEQ ID NO:1093 |
| WBC016A12_V1.3_at | TCCCTTCATTCTGGTTTCCATGTGA | SEQ ID NO:1094 |
| WBC016A12_V1.3_at | TCACCAACTCCCTTTTGGATTTCAT | SEQ ID NO:1095 |
| WBC016A12_V1.3_at | TAAACAACCATGTCTGCATCTAGGG | SEQ ID NO:1096 |
| Homo sapiens mRNA for HsMcm6 | | |
| WBC016G11_V1.3_at | ATGATGTGTGTATCTTCCTGGGAAA | SEQ ID NO:1097 |

(continued)

| Probe Set Name | Sequence | Sequence Identifier |
|---|---|---|
| ILT11A mRNA for immunoglobulin-like transcript 11 protein | | |
| WBC016G11_V1.3_at | ACTGTGCTGCTTTTCATGTGTAGGA | SEQ ID NO:1098 |
| WBC016G11_V1.3_at | GAATGCTTCTGGGAGTGATGCTTCA | SEQ ID NO:1099 |
| WBC016G11_V1.3_at | GAAGAAGATCCCTACCTGGTGGTTA | SEQ ID NO:1100 |
| WBC016G11_V1.3_at | GAGTCTGCATGGAGCTCTCCTAGAG | SEQ ID NO:1101 |
| WBC016G11_V1.3_at | GTTAACCCTAACTACCTGCTTGAAG | SEQ ID NO:1102 |
| WBC016G11_V1.3_at | GTTTCTGTCATACGCTTTTATACCA | SEQ ID NO:1103 |
| WBC016G11_V1.3_at | GGATGGTTGTGTCATGTTTCTGTCA | SEQ ID NO:1104 |
| WBC016G11_V1.3_at | GGCTTTAGCCCTTTGTTTACTGTGC | SEQ ID NO:1105 |
| WBC016G11_V1.3_at | TCACCCACTACGATCATGTTCTAAT | SEQ ID NO:1106 |
| WBC016G11_V1.3_at | TAACCCGAGAAACTGCAGCCTGCTG | SEQ ID NO:1107 |
| Homo sapiens G protien-coupled receptor 155 | | |
| WBC017B09_V1.3_at | AGGAGACCTTCCGAGTTACAGCAAC | SEQ ID NO:1108 |
| WBC017B09_V1.3_at | ACACACGTCCTGTTATGTGCATGAA | SEQ ID NO:1109 |
| WBC017B09_V1.3_at | AAAGCCTCGTTGCTGTACCAGGAGA | SEQ ID NO:1110 |
| WBC017B09_V1.3_at | AAACCTGCATGGAAGCCTAGTGTAT | SEQ ID NO:1111 |
| WBC017B09_V1.3_at | GAATCGGAACATCTTTTCCCATGGG | SEQ ID NO:1112 |
| WBC017B09_V1.3_at | GATGCTTTCAGCAGAACTGTCTTGC | SEQ ID NO:1113 |
| WBC017B09_V1.3_at | GTAACTTTTACTCCTCTTGACAAGC | SEQ ID NO:1114 |
| WBC017B09_V1.3_at | TGTCCACACTGTAACTCTCATTATG | SEQ ID NO:1115 |
| WBC017B09_V1.3_at | TGCAGGCACCTCAGACTCAAGTGGA | SEQ ID NO:1116 |
| WBC017B09_V1.3_at | TTGATTCACATGCATGCAGGCACCT | SEQ ID NO:1117 |
| WBC017B09_V1.3_at | TTGCTGCGGCACTTTGTTTTAACAT | SEQ ID NO:1118 |
| No homology | | |
| WBC017C08_V1.3_at | ATGCTACCTTTTTAGGTTGTCCTGA | SEQ ID NO: 1119 |
| WBC017C08_V1.3_at | AAGTTGGTACAGTTATTTTCCTCAT | SEQ ID NO:1120 |
| WBC017C08_V1.3_at | AATACCCGTATTTTAGTTGTCATAG | SEQ ID NO:1121 |
| WBC017C08_V1.3_at | CGGTGTGACTTTGCTTAGGTTTCTC | SEQ ID NO:1122 |
| WBC017C08_V1.3_at | GCCATCTTTGGCCAAGTGTTAGGAG | SEQ ID NO:1123 |
| WBC017C08_V1.3_at | GAGTTTTGGCTTTGTAGTCAGGCAA | SEQ ID NO:1124 |
| WBC017C08_V1.3_at | GTCAGGCAAGCCTATGTTGGGTCCT | SEQ ID NO:1125 |
| WBC017C08_V1.3_at | GTGTATTCTCTTACTCTTGAGCTTT | SEQ ID NO:1126 |
| WBC017C08_V1.3_at | GGCTGTTGCCACTTAATAGCGGTGT | SEQ ID NO:1127 |
| WBC017C08_V1.3_at | TGAGCCTCATTATCCACATCTGTAA | SEQ ID NO:1128 |
| WBC017C08_V1.3_at | TTAGGTTTCTCAACTTCGCTGAGCC | SEQ ID NO:1129 |
| Human transcriptional repressor (CTCF) mRNA | | |
| WBC018B05_V1.3_at | ATTATGAGCGGGTCACAGCAGCCCT | SEQ ID NO:1130 |
| WBC018B05_V1.3_at | ATCATGTAGCAGAACGGCACCCAGA | SEQ ID NO:1131 |

(continued)

| Probe Set Name | Sequence | Sequence Identifier |
|---|---|---|
| ILT11A mRNA for immunoglobulin-like transcript 11 protein | | |
| WBC018B05_V1.3_at | AGAGCCTCAGTCTTGCTGATTTCAA | SEQ ID NO:1132 |
| WBC018B05_V1.3_at | GCCATCAGTTAGCTATCAGACTCTA | SEQ ID NO:1133 |
| WBC018B05_V1.3_at | GCGGAGCCAGCATTTGAACCTTGTA | SEQ ID NO:1134 |
| WBC018B05_V1.3_at | GACATTTTCTTTGCTCTGTTTGTAG | SEQ ID NO:1135 |
| WBC018B05_V1.3_at | GAGATCGTGAATTCCAGCCCGCGGA | SEQ ID NO:1136 |
| WBC018B05_V1.3_at | GATTCTTGCACATGAACTGTCACAC | SEQ ID NO:1137 |
| WBC018B05_V1.3_at | GTTTGTGTTCTGTAAATCCTCTGTT | SEQ ID NO:1138 |
| WBC018B05_V1.3_at | GGAGGATCTACTGCTGTACAGCTAA | SEQ ID NO:1139 |
| WBC018B05_V1.3_at | TGATTTCCCATCGACATTTTCTTTG | SEQ ID NO:1140 |
| No homology | | |
| WBC018D03_V1.3_at | ATGTCTTGGATTGCACTTTCGTGGA | SEQ ID NO:1141 |
| WBC018D03_V1.3_at | AGTTGGTGGCTCCAGTTTGCTTTTC | SEQ ID NO:1142 |
| WBC018D03_V1.3_at | AGCAGCCTTGGTCGTTTTCGTTGGA | SEQ ID NO:1143 |
| WBC018D03_V1.3_at | AGCTCATCTCAATGTCCTGTTGACT | SEQ ID NO:1144 |
| WBC018D03_V1.3_at | GGTTTTGATTCCTCTGCTAGGACGC | SEQ ID NO:1145 |
| WBC018D03_V1.3_at | GAAATCTTTGGTTTTGTCCTGTGAG | SEQ ID NO:1146 |
| WBC018D03_V1.3_at | GTAGATCACCGAAACTAACTTGCTG | SEQ ID NO:1147 |
| WBC018D03_V1.3_at | GTCCTGTGAGACCAGTGCTTGACAT | SEQ ID NO:1148 |
| WBC018D03_V1.3_at | TGCTTGACATCGTCTGTTCGCCTGA | SEQ ID NO:1149 |
| WBC018D03_V1.3_at | TAGGACGCTGCCTAAACCAGCTCAT | SEQ ID NO:1150 |
| WBC018D03_V1.3_at | TTTCTCTTCGGTCTCGTGGTAGCAG | SEQ ID NO:1151 |
| Human mRNA for integrin alpha-4 subunit | | |
| WBC018F03_V1.3_at | AATTTAACCTCACTGGATTGGCACC | SEQ ID NO:1152 |
| WBC018F03_V1.3_at | ATCAGGGCATTCTTATGTGAACTTG | SEQ ID NO:1153 |
| WBC018F03_V1.3_at | ATTAGGCCAGATTCTGCCCAGTTAG | SEQ ID NO:1154 |
| WBC018F03_V1.3_at | AGATTGCTGGATAACATGACTTTCA | SEQ ID NO:1155 |
| WBC018F03_V1.3_at | AAATGATGGCATTTCAGATTGCTGG | SEQ ID NO:1156 |
| WBC018F03_V1.3_at | GCCCAGTTAGATATTTTGCACAGTT | SEQ ID NO:1157 |
| WBC018F03_V1.3_at | GAACACAGCTCCCAATTAGGCCAGA | SEQ ID NO:1158 |
| WBC018F03_V1.3_at | GTACCTGCAATGTTTACTTTCAGTT | SEQ ID NO:1159 |
| WBC018F03_V1.3_at | GGAACATTAGTTCTTTCTTATTCAG | SEQ ID NO:1160 |
| WBC018F03_V1.3_at | TTGGCACCTCCCCAGGAAAATCAGG | SEQ ID NO:1161 |
| WBC018F03_V1.3_at | TTGTGGTTTGTCTGTACCAGGCCAT | SEQ ID NO:1162 |
| No Homology | | |
| WBC019B05_V1.3_at | ATATATGCTGTCTGTTTTGTGTACA | SEQ ID NO:1163 |
| WBC019B05_V1.3_at | AACTCACTCTTACAGTAGTCTGCTT | SEQ ID NO:1164 |
| WBC019B05_V1.3_at | AATGTTGTCTTTTAAATACTCCGAT | SEQ ID NO:1165 |

(continued)

| Probe Set Name | Sequence | Sequence Identifier |
|---|---|---|
| ILT11A mRNA for immunoglobulin-like transcript 11 protein | | |
| WBC019B05_V1.3_at | CAAGCCATTTGTGGGAATCCTAAAA | SEQ ID NO:1166 |
| WBC019B05_V1.3_at | CCAGCTGTTTATTTTATTGAGTCCT | SEQ ID NO:1167 |
| WBC019B05_V1.3_at | GAGTAACAAACTCAGCCTTCTGTAA | SEQ ID NO:1168 |
| WBC019B05_V1.3_at | GAGGATCATCTGACTTTTCACTTAC | SEQ ID NO:1169 |
| WBC019B05_V1.3_at | GTAGTCTGCTTATTTCCAGCTGTTT | SEQ ID NO:1170 |
| WBC019B05_V1.3_at | GTGCTTTATGTACTGTTATCCTATA | SEQ ID NO:1171 |
| WBC019B05_V1.3_at | TGTAAAAACTCCTTTTCTGCCACAC | SEQ ID NO:1172 |
| WBC019B05_V1.3_at | TTGGGTTTTCTTTTCAACTCACTCT | SEQ ID NO:1173 |
| Human proline-rich protein (PRP) mRNA | | |
| WBC019C11_V1.3_at | ATGTAAAGCTCTGTGTCCGAAACCA | SEQ ID NO:1174 |
| WBC019C11_V1.3_at | AGTTTATCCACTTTACCATCTCTTT | SEQ ID NO:1175 |
| WBC019C11_V1.3_at | AAAAATCATGCAGTGTCTCCCAAAG | SEQ ID NO:1176 |
| WBC019C11_V1.3_at | GCTGTATAAGCTGTCTCTGGAGATC | SEQ ID NO:1177 |
| WBC019C11_V1.3_at | GAAAAATGTACCTTGCTGCCTTCAA | SEQ ID NO:1178 |
| WBC019C11_V1.3_at | GACGGCTGTCTGTGGTTAAGGATCA | SEQ ID NO:1179 |
| WBC019C11_V1.3_at | GTGACATGTTTTTGCTTTCCTGGAC | SEQ ID NO:1180 |
| WBC019C11_V1.3_at | GGTTGGTCTCCAAAGTATCACTTGC | SEQ ID NO:1181 |
| WBC019C11_V1.3_at | TGAGAACGGCCCTGGAGCTGTATAA | SEQ ID NO:1182 |
| WBC019C11_V1.3_at | TGACCCTCACTATCGTTTGGTTGGT | SEQ ID NO:1183 |
| WBC019C11_V1.3_at | TTTTGTGCCATCACTCCTGGTAATA | SEQ ID NO:1184 |
| Human translation initiation factor 3 large subunit mRNA. | | |
| WBC020F05_V1.3_at | ATGACCGTCGTCGTGAGCGAGATGA | SEQ ID NO:1185 |
| WBC020F05_V1.3_at | ATGGATGGACCACGGTACGACGTTA | SEQ ID NO:1186 |
| WBC020F05_V1.3_at | AGATTGATCATGCACCGTATCCACA | SEQ ID NO:1187 |
| WBC020F05_V1.3_at | AGTTCTTGGAGACGTGCTGATGACA | SEQ ID NO:1188 |
| WBC020F05_V1.3_at | ACATAGGTTTGATCACATTCGCGGA | SEQ ID NO:1189 |
| WBC020F05_V1.3_at | AAGAGGACCTCCTTTGAGATCAGAA | SEQ ID NO:1190 |
| WBC020F05_V1.3_at | CGCGGATTATTAGACTTGTGCTTCA | SEQ ID NO:1191 |
| WBC020F05_V1.3_at | GAGAGATTCGAGTCGCCGTGACGAT | SEQ ID NO:1192 |
| WBC020F05_V1.3_at | GATAGGGAATCTCTTCGTCGTACTA | SEQ ID NO:1193 |
| WBC020F05_V1.3_at | GGATTCTTTAATGTTGTCTCACCAT | SEQ ID NO:1194 |
| WBC020F05_V1.3_at | TGGGTTCGAACTCATGTGTCACATA | SEQ ID NO:1195 |
| Human' CSaids binding protein (CSBP1) mRNA | | |
| WBC020H01_V1.3_at | ATGGCAATCACAGCTCGCCTGGGAT | SEQ ID NO:1196 |
| WBC020H01_V1.3_at | AGGTCAAATCCTCACGATGCACAGC | SEQ ID NO:1197 |
| WBC020H01_V1.3_at | ACAGTGCGGTGATGCATGCGCTCCA | SEQ ID NO:1198 |
| WBC020H01_V1.3_at | AAAGCTACTTCCAGTGTTGGCTATG | SEQ ID NO:1199 |

(continued)

| Probe Set Name | Sequence | Sequence Identifier |
|---|---|---|
| ILT11A mRNA for immunoglobulin-like transcript 11 protein | | |
| WBC020H01_V1.3_at | AAGGCCTCATCTCCTTTATATGCAG | SEQ ID NO:1200 |
| WBC020H01_V1.3_at | AAGGTGTTTCCATTTCTCCATCACA | SEQ ID NO:1201 |
| WBC020H01_V1.3_at | CATCTCCTGATTTCTCTGAACGGAA | SEQ ID NO:1202 |
| WBC020H01_V1.3_at | CTGAACTCAGCTGGAGCCTGTGGAA | SEQ ID NO:1203 |
| WBC020H01_V1.3_at | GAAGAGCTCCTTGGTTTCTGAGCAT | SEQ ID NO:1204 |
| WBC020H01_V1.3_at | GACCCTGGGTCAACTGGAGTGAGAA | SEQ ID NO:1205 |
| WBC020H01_V1.3_at | GAGGGCCCACATATTTAAATCTTCT | SEQ ID NO:1206 |
| No homology | | |
| WBC021E02_V1.3_at | TGATCGTTCACAAGCGTACTCACGC | SEQ ID NO:1207 |
| WBC021E02_V1.3_at | GCAGTTTTTTGTACTTGATCGTTCA | SEQ ID NO:1208 |
| WBC021E02_V1.3_at | GAATGTGGTAAAGCCCTCAGTTGTC | SEQ ID NO:1209 |
| WBC021E02_V1.3_at | GTACTCACGCTGGAGAGAAACCTTA | SEQ ID NO:1210 |
| WBC021E02_V1.3_at | GAACCACACTGGAGAGATGCTCTAG | SEQ ID NO:1211 |
| WBC021E02_V1.3_at | GAAACATGGGAAGCCCTTCACTGTT | SEQ ID NO:1212 |
| WBC021E02_V1.3_at | GAGAATCTCATCACAACCTGGCATG | SEQ ID NO:1213 |
| WBC021E02_V1.3_at | GTACCATGAAAGCACTTACCTGCAG | SEQ ID NO:1214 |
| WBC021E02_V1.3_at | TCAGTTGTCCCAATTCACTTTGAAG | SEQ ID NO:1215 |
| WBC021E02_V1.3_at | TTCCCATTTCCTTTTGGTACCATGA | SEQ ID NO:1216 |
| WBC021E02_V1.3_at | TTACCTGCAGAGAAATCCTGTCAGT | SEQ ID NO:1217 |
| No homology | | |
| WBC021E07_V1.3_at | AGTTCCCCTTCAAGGAGCCAGAGAG | SEQ ID NO:1218 |
| WBC021E07_V1.3_at | ACCAGCAGCTGGAAGCACCAAGTAT | SEQ ID NO:1219 |
| WBC021E07_V1.3_at | AACACACTGATTTCTTCTTGGGTCC | SEQ ID NO:1220 |
| WBC021E07_V1.3_at | CTGAGCTTCAGCACACTGGCAGAGA | SEQ ID NO:1221 |
| WBC021E07_V1.3_at | CGATCAGAGCTGTCCCTCAAGGAGG | SEQ ID NO:1222 |
| WBC021E07_V1.3_at | GAGTTTTATCAGCAGAATTCCCTAA | SEQ ID NO:1223 |
| WBC021E07_V1.3_at | GAGGAGAAACTCCAGGCTCTGAGCC | SEQ ID NO:1224 |
| WBC021E07_V1.3_at | GATTATTCTGAATACTGCTGAGCTT | SEQ ID NO:1225 |
| WBC021E07_V1.3_at | GATTGTTTCAAGTCGCTGGACACCA | SEQ ID NO:1226 |
| WBC021E07_V1.3_at | GGGTGATGCCTTGTCATGGTTAATT | SEQ ID NO:1227 |
| WBC021E07_V1.3_at | GGAGAGCCATTAGCAAACCAGCAGC | SEQ ID NO:1228 |
| Homo sapiens T-cell receptor interacting molecule, mRNA (cDNA clone MGC:3435.4 IMAGE:5227396). | | |
| WBC022B03_V1.3_at | AGAGTGTCCGTTTGTACACGCCAAA | SEQ ID NO:1229 |
| NBC022B03_V1.3_at | AGATGAGCAGTTACACGCGACGGAT | SEQ ID NO:1230 |
| WBC022B03_V1.3_at | CTGGACTCTGATCTAGCTCAATGAT | SEQ ID NO:1231 |
| WBC022B03_V1.3_at | GCCTTTCTATGACTGCTTTGGTTGA | SEQ ID NO:1232 |

(continued)

| Probe Set Name | Sequence | Sequence Identifier |
|---|---|---|
| ILT11A mRNA for immunoglobulin-like transcript 11 protein | | |
| WBC022B03_V1.3_at | GAACCTATGAACTAGCTGGACTCTG | SEQ ID NO:1233 |
| WBC022B03_V1.3_at | GAAACACGATCCTTATTTGCCAGAC | SEQ ID NO:1234 |
| WBC022B03_V1.3_at | GAAATTCCCATATGACACACTGTGA | SEQ ID NO:1235 |
| WBC022B03_V1.3_at | GACAGCCTGATCGTTTGTTGGTGAG | SEQ ID NO:1236 |
| WBC022B03_V1.3_at | GTTGATAGTTTTGCACCTGAGGACC | SEQ ID NO:1237 |
| WBC022B03_V1.3_at | TGAGATGGTGACTTTCCTCTTATCC | SEQ ID NO:1238 |
| WBC022B03_V1.3_at | TGATGATCCCATCAGGCTGTTCGGA | SEQ ID NO:1239 |
| PREDICTED Bos taurus similar to Caspase recruitment domain protein 8. (Apoptotic protein NDPP1) (DACAR) (CARD-inhibitor of NF-kappaB activating ligand) (CARDINAL) (Tumor-upregulated CARD-containing antagonist of CASP9) (TUCAN) (LOC511229), partial mRNA | | |
| WBC022C04_V1.3_at | AGCCCATTGGCTCTTATTAATCAGT | SEQ ID NO:1240 |
| WBC022C04_V1.3_at | AGAACGGTGATCTCTTCTTGAGGAA | SEQ ID NO:1241 |
| WBC022C04_V1.3_at | AACTATAGCTTTGCCTGTGAAGAAC | SEQ ID NO:1242 |
| WBC022C04_V1.3_at | TAGCCTTAGCCGCACCAAGGGAGAA | SEQ ID NO:1243 |
| WBC022C04_V1.3_at | CCATTAGTCTTCTGTAGGTGTTAGC | SEQ ID NO:1244 |
| WBC022C04_V1.3_at | CCAGGGAATATCGAGCCCATTGGCT | SEQ ID NO:1245 |
| WBC022C04_V1.3_at | TAAGCTGGTTTCCTAACATTTTCAA | SEQ ID NO:1246 |
| WBC022C04_V1.3_at | GTCAGGGATCCTGTGGAGTTCAAAT | SEQ ID NO:1247 |
| WBC022C04_V1.3_at | GATCATTTGCTCCACTCAGAGGTAG | SEQ ID NO:1248 |
| WBC022C04_V1.3_at | GTAGATGTTATCATTCTCCCAGAAT | SEQ ID NO:1249 |
| WBC022C04_V1.3_at | TTTTCAACCTTGCTCATACTCTGGA | SEQ ID NO:1250 |
| No-homology | | |
| WBC022D03_V1.3_at | AGGGACGATACTTCTTACTGTTGTT | SEQ ID NO:1251 |
| WBC022D03_V1.3_at | AGCAGTTTATGTGGTTGATTCTCAA | SEQ ID NO:1252 |
| WBC022D03_V1.3_at | ACGCTGGATTGTAGTCTGTTTACTT | SEQ ID NO:1253 |
| WBC022D03_V1.3_at | AACAGTGGTCTCTTTGACAAGGCAT | SEQ ID NO:1254 |
| WBC022D03_V1.3_at | CTTCTTACTGTTGTTGACGCTGGAT | SEQ ID NO:1255 |
| WBC022D03_V1.3_at | GCAGGGTGATGCTGTCTGGTCTTCA | SEQ ID NO:1256 |
| WBC022D03_V1.3_at | GACTGTATCTTATCCACCATTGTAT | SEQ ID NO:1257 |
| WBC022D03_V1.3_at | TTTATCCTAACAGTGGCCCTTTGCA | SEQ ID NO:1258 |
| WBC022D03_V1.3_at | TGTTTACTTGTCTTTCTCTTTCGTA | SEQ ID NO:1259 |
| WBC022D03_V1.3_at | TCTTCAGCTCTTCACACTTACTTAA | SEQ ID NO:1260 |
| WBC022D03_V1.3_at | TTAAGACCTGGCCTTGACTGAATTT | SEQ ID NO:1261 |
| No homology | | |
| WBC023F12_V1.3_at | GACCGTAAAACTATGTTCAGGCACT | SEQ ID NO:1262 |
| WBC023F12_V1.3_at | AATACCAGCAAGTCATGTCTTGTGT | SEQ ID NO:1263 |
| WBC023F12_V1.3_at | AATTGATTGCTGACTTGACCTGCCT | SEQ ID NO:1264 |

(continued)

| Probe Set Name | Sequence | Sequence Identifier |
|---|---|---|
| ILT11A mRNA for immunoglobulin-like transcript 11 protein | | |
| WBC023F12_V1.3_at | GACCTGCCTGGTCAAATATCTGAAT | SEQ ID NO:1265 |
| WBC023F12_V1.3_at | GATGGCATTACTGGGTAGATTCTAA | SEQ ID NO:1266 |
| WBC023F12_V1.3_at | GATTCTAACCCATGATTCTAACTGT | SEQ ID NO:1267 |
| WBC023F12_V1.3_at | GTTACAGTTCAAATTGTCCCACTTC | SEQ ID NO:1268 |
| WBC023F12_V1.3_at | GTATTACCCTCTATCATAACACACT | SEQ ID NO:1269 |
| WBC023F12_V1.3_at | GTAACTATTGTTGCTATTCATGTAA | SEQ ID NO:1270 |
| WBC023F12_V1.3_at | TACTGTATCAGCTGTAGTTGGGTGA | SEQ ID NO:1271 |
| WBC023F12_V1.3_at | TTCAGGCACTTACTCTTCGTTTATA | SEQ ID NO:1272 |
| Adducin 3 (gamma) (ADD3), transcript variant 2 | | |
| WBC024B05_V1.3_at | ATGAACCTCTGTGTCCTGTGGAAAA | SEQ ID NO:1273 |
| WBC024B05_V1.3_at | ATGAGCCAATGAACCTCTGTGTCCT | SEQ ID NO:1274 |
| WBC024B05_V1.3_at | AGTGAACTATTTGCACCTTTTGCTA | SEQ ID NO:1275 |
| WBC024B05_V1.3_at | CACCTTTTGCTAATGCCTCTATTTA | SEQ ID NO:1276 |
| WBC024B05_V1.3_at | CAGTGTTTTAATCTCTTAGTGGAAA | SEQ ID NO:1277 |
| WBC024B05_V1.3_at | CTGGTTCTGTTTGGCGTATGTGTAT | SEQ ID NO:1278 |
| WBC024B05_V1.3_at | GCCTCTATTTACTTGCTTTGGCATA | SEQ ID NO:1279 |
| WBC024B05_V1.3_at | GATCTCACTAACTACTGGAATCAGT | SEQ ID NO:1280 |
| WBC024B05_V1.3_at | GTAACCTGTGAACTATGCTTTTCCA | SEQ ID NO:1281 |
| WBC024B05_V1.3_at | GGAAACTCTCAGTTGCTTAATTCTG | SEQ ID NO:1282 |
| WBC024B05_V1.3_at | GGAAATTTCATTTTAGATCTCACTA | SEQ ID NO:1283 |
| No homology | | |
| WBC024F08_V1.3_at | ATGTGTGTCTCTATGTACCCAAGCC | SEQ ID NO:1284 |
| WBC024F08_V1.3_at | AGCTCTGCAAGTCACTTACCTGAAG | SEQ ID NO:1285 |
| WBC024F08_V1.3_at | AGACTCAGGGATGCTGTTTCCAGCT | SEQ ID NO:1286 |
| WBC024F08_V1.3_at | AGCGTGTGTCTACATGTGTGTCTCT | SEQ ID NO:1287 |
| WBC024F08_V1.3_at | AAGCACAGTGTCTCTCGAATTTCGG | SEQ ID NO:1288 |
| WBC024F08_V1.3_at | GAGAGGCGAGCATCTGGCTGTACTT | SEQ ID NO:1289 |
| WBC024F08_V1.3_at | GTACCCTCCTCACATTTTTGCATAT | SEQ ID NO:1290 |
| WBC024F08_V1.3_at | GGTGGCCACCTGCATGAGTGTATTA | SEQ ID NO:1291 |
| WBC024F08_V1.3_at | GGATGCATTCTCTTGTTTTGCTTGA | SEQ ID NO:1292 |
| WBC024F08_V1.3_at | TGAGTCCTGTGAGATGCCCTTGTTA | SEQ ID NO:1293 |
| WBC024F08_V1.3_at | TCTGGTTCTCTCTCTCAGGAATAAG | SEQ ID NO:1294 |
| Horse serpin mRNA | | |
| WBC024G03_V1.3_at | AGTAAAGCACCCCAATTAAGGCCTG | SEQ ID NO:1295 |
| WBC024G03_V1.3_at | AAAATGCTGCATTTTCTTGCTCCCA | SEQ ID NO:1296 |
| WBC024G03_V1.3_at | CCAGCTTCTTGCGTTTTCTGATGAA | SEQ ID NO:1297 |
| WBC024G03_V1.3_at | GACAAACTCGTCTTGACCGTTGTTT | SEQ ID NO:1298 |

(continued)

| Probe Set Name | Sequence | Sequence Identifier |
|---|---|---|
| ILT11A mRNA for immunoglobulin-like transcript 11 protein | | |
| WBC024G03_V1.3_at | GTTGTTACACCAGTGCGATGGCTTT | SEQ ID NO:1299 |
| WBC024G03_V1.3_at | GGCACAAAGATCTCACTGTTACCCA | SEQ ID NO:1300 |
| WBC024G03_V1.3_at | TAGGGCAGCACTCCAATTTCAAACT | SEQ ID NO:1301 |
| WBC024G03_V1.3_at | TTTTGTTCTCCTTTATCCTACCTGA | SEQ ID NO:1302 |
| WBC024G03_V1.3_at | TTCAAACTGTTAGCCACCTGGTGAT | SEQ ID NO:1303 |
| WBC024G03_V1.3_at | TTGTTTCTAACCCTTTGTTCCACAG | SEQ ID NO:1304 |
| WBC024G03_V1.3_at | TTGCTCCCACAAAGCTGGTATGCTG | SEQ ID NO:1305 |
| No homology | | |
| WBC024G08_V1.3_at | AGTGAACTTGTCCTTGCCAGATGGG | SEQ ID NO:1306 |
| WBC024G08_V1.3_at | ACTCAGCAGTGAGTTGCCTGTGGCT | SEQ ID NO:1307 |
| WBC024G08_V1.3_at | AAATAAGTGCATTCTCCTCAGAGGA | SEQ ID NO:1308 |
| WBC024G0B_V1.3_at | CTCCATGCCTTCAACTCAACAAATG | SEQ ID NO:1309 |
| WBC024G08_V1.3_at | GCTGGAAACAAGCTGTGCCTTCAAA | SEQ ID NO:1310 |
| WBC024G08_V1.3_at | GCCTATGTAGGCCTTTTCTGCAGAA | SEQ ID NO:1311 |
| WBC024G08_V1.3_at | GTCTTTAAGCATCTTCAGCTCTGAT | SEQ ID NO:1312 |
| WBC024G08_V1.3_at | GGGCTTTTTGTTCAGTCTGTATCAG | SEQ ID NO:1313 |
| WBC024G08_V1.3_at | GGAAACTATTTCTAACATCTGGTGA | SEQ ID NO:1314 |
| WBC024G08_V1.3_at | TGGCTGTCTTTTCTTGTATTCTGTA | SEQ ID NO:1315 |
| WBC024G08_V1.3_at | TCAGAACAGCGTTTGTGCTCCATGC | SEQ ID NO:1316 |
| Homo sapiens interferon gamma receptor 2 (interferon gamma transducer 1) mRNA | | |
| WBC024H07_V1.3_at | ATTTTGTTCATTGTCGGTTGGGCCA | SEQ ID NO:1317 |
| WBC024H07_V1.3_at | ATTTTCTCAATCATACACGTCGTTA | SEQ ID NO:1318 |
| WBC024H07_V1.3_at | AGGCAGGTCACCTAATCTGTCTCAG | SEQ ID NO:1319 |
| WBC024H07_V1.3_at | AGGACTGAGCTCCTGAAGCTGCTGA | SEQ ID NO:1320 |
| WBC024H07_V1.3_at | CCAGTCTCCCAGCTTAAAACGTGAT | SEQ ID NO:1321 |
| WBC024H07_V1.3_at | AAATGGCTCTGGCACACCTGTGATA | SEQ ID NO:1322 |
| WBC024H07_V1.3_at | GAAGCTGCTGATGTCCCTGTCAGGA | SEQ ID NO:1323 |
| WBC024H07_V1.3_at | GAAGATGTTCTCCAAAGCACTTTGA | SEQ ID NO:1324 |
| WBC024H07_V1.3_at | GAAGCAAAGCATTGGCCTGCTCCAC | SEQ ID NO:1325 |
| WBC024H07_V1.3_at | GTGTCTGTCGTTGTGTTTCCAGAGA | SEQ ID NO:1326 |
| WBC024H07_V1.3_at | TGTCAGGACTTTACGGAGCCCACTA | SEQ ID NO:1327 |
| No homology | | |
| WBC025A06_V1.3_at | ATCGTGTCAGCACAGAAACCTTCTC | SEQ ID NO:1328 |
| WBC025A06_V1.3_at | AGAGAATTAGTCACTGGGCCAGGCG | SEQ ID NO:1329 |
| WBC025A06_V1.3_at | ACAATTCAGAAGTGTCCAGCCCAGC | SEQ ID NO:1330 |
| WBC025A06_V1.3_at | AACCTTCTCAGGTACAGGCTTTGTT | SEQ ID NO:1331 |
| WBC025A06_V1.3_at | GCTTCCCACGGAACTGAGCAGAGAA | SEQ ID NO:1332 |

(continued)

| Probe Set Name | Sequence | Sequence Identifier |
|---|---|---|
| ILT11A mRNA for immunoglobulin-like transcript 11 protein | | |
| WBC025A06_V1.3_at | GAAGGGCAGCCCACTGGCATCGTGT | SEQ ID NO:1333 |
| WBC025A06_V1.3_at | GGTGTGAAAGACTCCTGCTATTATA | SEQ ID NO:1334 |
| WBC025A06_V1.3_at | TGTGTCGGTTACCACTCAAGACGCA | SEQ ID NO:1335 |
| WBC025A06_V1.3_at | TCCAGTGTGGTGGTTAGTCTGCCTT | SEQ ID NO:1336 |
| WBC025A06_V1.3_at | TATTATAAGCACTCAGTCCGCTTCC | SEQ ID NO:1337 |
| WBC025A06_V1.3_at | TTGCGGTTCTGGACTTTGTTTTTAT | SEQ ID NO:1338 |
| Homo sapiens TBP-interacting protein, mRNA | | |
| WBC025C03_V1.3_at | AGTTCCAGTCGCAGATCAGCTCCAA | SEQ ID NO:1339 |
| WBC025C03_V1.3_at | AGATGTTTGCTCCATACGGGACCAT | SEQ ID NO:1340 |
| WBC025C03_V1.3_at | AGAGCCCGCTGATGAGCGAGTTCCA | SEQ ID NO:1341 |
| WBC025C03_V1.3_at | AGACAGTTTGGCTTTCTTCCATTGT | SEQ ID NO:1342 |
| WBC02SC03_V1.3_at | AACTGAAGCGATCGGCCATGAGAGC | SEQ ID NO:1343 |
| WBC025C03_V1.3_at | GAAAGACTCATCGTCCACTAACTTG | SEQ ID NO:1344 |
| WBC025C03_V1.3_at | GGGACCATTACGTTGACCATACGAT | SEQ ID NO:1345 |
| WBC025C03_V1.3_at | GTGTATTTCCATAATCCAGAGGTTG | SEQ ID NO:1346 |
| WBC025C03_V1.3_at | GGTCCAGTATCTATTTGCCCTGTAA | SEQ ID NO:1347 |
| WBC025C03_V1.3_at | GGCAGCCATCTTTGAGAGCATCCAG | SEQ ID NO:1348 |
| WBC025C03_V1.3_at | TGACCATTCCCGAGGCCGAGAAGAG | SEQ ID NO:1349 |
| No Homology | | |
| WBC027B01_V1.3_at | ATCTTGGCACCTTGCTCTTTTAGAA | SEQ ID NO:1350 |
| WBC027B01_V1.3_at | AAAATACATTGCTCACCTTTCCTGT | SEQ ID NO:1351 |
| WBC027B01_V1.3_at | AACTCTAGAACTCTCAGACGCTGCG | SEQ ID NO:1352 |
| WBC027B01_V1.3_at | GATTCTCAGGGAAACTCCGACTGGA | SEQ ID NO:1353 |
| WBC027B01_V1.3_at | GCTAGATTTCAAAGGCTCGCTTCGC | SEQ ID NO:1354 |
| WBC027B01_V1.3_at | CTGGATTTATTTTTCTACCTGTGTA | SEQ ID NO:1355 |
| WBC027B01_V1.3_at | GCTGGTGCTCTATAGTTTTGTTCTC | SEQ ID NO:1356 |
| WBC027B01_V1.3_at | GTAAAATCCTGTCAGACCACCGATA | SEQ ID NO:1357 |
| WBC027B01_V1.3_at | GTACATCTAGTTCCTCACCATAGAA | SEQ ID NO:1358 |
| WBC027B01-V1.3_at | TCGCTTCGCAAAAATACTCCATCTT | SEQ ID NO:1359 |
| WBC027B01_V1.3_at | TTCAGCCCCTGAAGCTTGCTAATAT | SEQ ID NO:1360 |
| No homology | | |
| WBC027D09_V1.3_at | ATTTTTGCTGCCATACACTAACTCA | SEQ ID NO:1361 |
| WBC027D09_V1.3_at | ATTTTGCCTGCACTCTTCATTCTTA | SEQ ID NO:1362 |
| WBC027D09_V1.3_at | GAAACTTATGGAATGTGACCCTGAT | SEQ ID NO:1363 |
| WBC027D09_V1.3_at | GAGGATGCCACAGAGGTTTGCAAGA | SEQ ID NO:1364 |
| WBC027D09_V1.3_at | GATGTGTGGCAAATACTCAATGTTA | SEQ ID NO:1365 |
| WBC027D09_V1.3_at | GTGTTATTGCATTAGCTCCCTTCGA | SEQ ID NO:1366 |

(continued)

| Probe Set Name | Sequence | Sequence Identifier |
|---|---|---|
| ILT11A mRNA for immunoglobulin-like transcript 11 protein | | |
| WBC027D09_V1.3_at | GGAGCTACTTCACAATTTTGCCTGC | SEQ ID NO:1367 |
| WBC027D09_V1.3_at | TGGAAGCACCAAATACTGTCTTATT | SEQ ID NO:1368 |
| WBC027D09_V1.3_at | TTCGATGGGCATCTCTATGAATTAG | SEQ ID NO:1369 |
| WBC027D09_V1.3_at | TAACCCCTTGCACCATGACTGATGT | SEQ ID NO:1370 |
| WBC027D09_V1.3_at | TTGTGATCACTCTTTCTGACGCATA | SEQ ID NO:1371 |
| Homo sapiens collagenase mRNA. | | |
| WBC027F05_V1.3_at | ATACTGGGCTCTGAATGGCTATGAC | SEQ ID NO:1372 |
| WBC027F05_V1.3_at | ATGACATTCAGCAAGGTTATCCCAA | SEQ ID NO:1373 |
| WBC027F05_V1.3_at | AGATGACTTCTTTCTTTTCTTCAGC | SEQ ID NO:1374 |
| WBC027F05_V1.3_at | AGCATAGCAAGTACCTTTCCAGGAA | SEQ ID NO:1375 |
| WBC027F05_V1.3_at | AATTTCATTTCTCTGTTCTGGCCAT | SEQ ID NO:1376 |
| WBC027F05_V1.3_at | CTTCCCAAGCAGTGTCCAAGCAATT | SEQ ID NO:1377 |
| WBC027F05_V1.3_at | TCAGCGGACCAGTTTATTATGCATT | SEQ ID NO:1378 |
| WBC027F05_V1.3_at | GAAGCAGGTTATCCCCAAAGCATAG | SEQ ID NO:1379 |
| WBC027F05_V1.3_at | GACATATCAAACTACGGCTTCCCAA | SEQ ID NO:1380 |
| WBC027F05_V1.3_at | TGATGCAGCTGTTTCCTATAGGAGT | SEQ ID NO:1381 |
| WBC027F05_V1.3_at | TTTCTCTCCTGTGTTCCATTAGGTA | SEQ ID NO:1382 |
| Homo sapiens ribosomal protein S8, mRNA (cDNA clone MGC:90249 IMAGE:6527505) | | |
| WBC27H11_V1.3_at | ATGTATCGCTTCCAGACCAGGCCAG | SEQ ID NO:1383 |
| WBC027H11_V1.3_at | AGGCAAATAAGTCCCTCTGGTCCTA | SEQ ID NO:1384 |
| WBC027H11_V1.3_at | AGGGCAAGCTTCTCGCATGTATCGC | SEQ ID NO:1385 |
| WBC027H11_V1.3_at | AAAATCAGCAGTCTCCTGGAGGAGC | SEQ ID NO:1386 |
| WBC027H11_V1.3_at | AAGACCCTGGTGAAGAACTGCATCG | SEQ ID NO:1387 |
| WBC027H11_V1.3_at | CAGACGGCTATGTGCTCGAGGGCAA | SEQ ID NO:1388 |
| WBC027H11_V1.3_at | GAAAGCCCTACCACAAGAAGCGGAA | SEQ ID NO:1389 |
| WBC027H11_V1.3_at | GAACTGCATCGTGCTGGTTGACAGC | SEQ ID NO:1390 |
| WBC027H11_V1.3_at | GGCAAGGAGCTGGAGTTCTATCTGA | SEQ ID NO:1391 |
| WBC027H11_V1.3_at | GGAGCAGTTCCAGCAGGGCAAGCTT | SEQ ID NO:1392 |
| WBC027H11_V1.3_at | GGCTCCGAGTGCTGTACGCGCAAAA | SEQ ID NO:1393 |
| Homo sapiens hypothetical protein FLJ25421, mRNA (cDNA clone MGC: 33348 IMAGE 4831324) | | |
| WBC028D04_V1.3_at | GTGGTAAGACCTCAAACTGCAAGTG | SEQ ID NO:1394 |
| WBC028D04_V1.3_at | ACAAGCCTTTGCCTTGACTGAGGAG | SEQ ID NO:1395 |
| WBC028D04_V1.3_at | AAATCTTTCTTTTCTTGTGGATGAC | SEQ ID NO:1396 |
| WBC028D04_V1.3_at | AAGCCCTTTATTTTACAACTCTGTG | SEQ ID NO:1397 |
| WBC028D04_V1.3_at | AACTCTGTGGAGCTTCATTATTAAC | SEQ ID NO:1398 |
| WBC02BD04_V1.3_at | GATGGTTTGATTTCTCTTTGTCAGG | SEQ ID NO:1399 |

(continued)

| Probe Set Name | Sequence | Sequence Identifier |
|---|---|---|
| ILT11A mRNA for immunoglobulin-like transcript 11 protein | | |
| WBC028D04_V1.3_at | GTACGTGTCACATGTTCTCGTGTCG | SEQ ID NO:1400 |
| WBC02SD04_V1.3_at | GTGACTATTGCAGAGCCTTTGGATA | SEQ ID NO:1401 |
| WBC028D04_V1.3_at | TCGTGTCGGGTTTCAGGCTCTGCAA | SEQ ID NO:1402 |
| WBC028D04_V1.3_at | TCATGCTCTGTACTTGACCTCAAAA | SEQ ID NO:1403 |
| WBC028D04_V1.3_at | TATAGATTCTCATCACTGTGGCGAA | SEQ ID NO:1404 |
| Home sapiens regulator of G-protein signaling 13 mRNA | | |
| WBC028E11_V1.3_at | ATTCAGGAAGTATTCCGCCAGCCAT | SEQ ID NO:1405 |
| WBC028E11_V1.3_at | ATCACAGTCTTCTTCCGAATCTGAT | SEQ ID NO:1406 |
| WBC028E11_V1.3_at | AAAGATGCCATACATTTGCCTTTGG | SEQ ID NO:1407 |
| WBC028E11_V1.3_at | GAGGCATAGCTCCACGTTAGGACTG | SEQ ID NO:1408 |
| WEC028E11_V1.3_at | GAGTAATTAGCCCTCGTCTTTGGAC | SEQ ID NO:1409 |
| WBC028E11_V1.3_at | GAGATAGCGGCAGTCAGTCATTTGA | SEQ ID NO:1410 |
| WBC028E11_V1.3_at | GTCCACATTTGGGTTCATTCCTAAG | SEQ ID NO:1411 |
| WBC028E11_V1.3_at | GGACTTACCCAGTTATACGGAGCAC | SEQ ID NO:1412 |
| WBC028E11_V1.3_at | TGGTCACGGAAACATGCCCAGATGA | SEQ ID NO:1413 |
| WBC028E11_V1.3_at | TGCCGCATTGGCTCATATTCAGGAA | SEQ ID NO:1414 |
| WBC028E11_V1.3_at | TTTGAGGTCCTAGCGATCAGGCATC | SEQ ID NO:1415 |
| Homo sapiens mRNA for KIAA1689 protein, partial cds | | |
| WBC028G09_V1.3_at | CTCCTGAACACTTACGTCTTAGCAA | SEQ ID NO:1416 |
| WBC028G09_V1.3_at | ACACCTGTGTTTAATCTGATTCATG | SEQ ID NO:1417 |
| WBC028G09_V1.3_at | AACTTTGTTAACATCCGTCTCTTGC | SEQ ID NO:1418 |
| WBC028G09_V1.3_at | AATCATGTTATCTTTGGGCAGTCGT | SEQ ID NO:1419 |
| WBC028G09_V1.3_at | CGTCTCTTGCACATCTACAACAAAT | SEQ ID NO:1420 |
| WBC028G09_V1.3_at | GCATTCGAGAATCAAGTCAGCCAGA | SEQ ID NO:1421 |
| WBC028G09_V1.3_at | GACCATGTATACTCTCTTGCTTGAA | SEQ ID NO:1422 |
| WBC028G09_V1.3_at | TGGAGAGACCTGTTCACACCTGTGT | SEQ ID NO:1423 |
| WBC028G09_V1.3_at | TGGGCAGTCGTTGCATTCGAGAATC | SEQ ID NO:1424 |
| WBC028G09_V1.3_at | TGAATATCCACTGTTTACCACCATG | SEQ ID NO:1425 |
| WBC028G09_V1.3_at | TTTTCTTGGCTTTTCCTCCTGAACA | SEQ ID NO:1426 |
| Homo sapiens protein associated with Myc mRNA. | | |
| WBC030C11_V1.3_at | ATTACCGTCATTGGCCTGTGAAGCA | SEQ ID NO:1427 |
| WBC030C11_V1.3_at | AGTGCAATACTGAGTGTGCCTCATT | SEQ ID NO:1428 |
| WBC030C11_V1.3_at | AATCCTGTACAATTGCATCACGGGT | SEQ ID NO:1429 |
| WBC030C11_V1.3_at | CGGGACCATTTTGACATCCATGAAA | SEQ ID NO:1430 |
| WBC030C11_V1.3_at | GAATATTCTGGTTTATTTCCTAGAC | SEQ ID NO:1431 |
| WBC030C11_V1.3_at | GAAGGATGCCAAATACCATGTACAT | SEQ ID NO:1432 |
| WBC030C11_V1.3_at | GAATGATGCTGGCTTTCAAGCAAAA | SEQ ID NO:1433 |

(continued)

| Probe Set Name | Sequence | Sequence Identifier |
|---|---|---|
| ILT11A mRNA for immunoglobulin-like transcript 11 protein | | |
| WBC030C11_V1.3_at | GTATACTACCTTTTTGTAATCCTGT | SEQ ID NO:1434 |
| WBC030C11_V1.3_at | GTGCCTCATTATCTTGCAGCTGTAA | SEQ ID NO:1435 |
| WBC030C11_V1.3_at | GTGTTTGCATGTGGCCATTACCGTC | SEQ ID NO:1436 |
| WBC030C11_V1.3_at | TCCCCATTATTTCGGTTTATCTTCT | SEQ ID NO:1437 |
| Homo sapiens progestin and adipoO receptor family member XI (PAOR11) | | |
| WBC031B06_V1.3_at | ATAAATGAGCAGACCCTGCCTTTGG | SEQ ID NO:1438 |
| WBC031B06_V1.3_at | AGATGCCTGTTGTTTTATTGTGTAT | SEQ ID NO:1439 |
| WBC031B06_V1.3_at | AGAAACTCATGCATCTGTGGTCCAT | SEQ ID NO:1440 |
| WBC031B06_V1.3_at | CACCGCTATCTGCATGATTCTGAAC | SEQ ID NO:1441 |
| WBC031B06_V1.3_at | CTGCCTTTGGATTGGGTGCTGATAA | SEQ ID NO:1442 |
| WBC031B06_V1.3_at | GAAGTCTGCTCAAAGTAACCTGTCA | SEQ ID NO:1443 |
| WBC031B06_V1.3_at | GATTAATCTCCTCTTGGTGTTTTGA | SEQ ID NO:1444 |
| WBC031B06_V1.3_at | GTTTCCCCTCAGTGTCTTATATTAA | SEQ ID NO:1445 |
| WBC031B06_V1.3_at | GGGTGCTGATAACGACTATTTTCTA | SEQ ID NO:1446 |
| WBC031B06_V1.3_at | GGTCCATGGGTTATATCCTGGCTCA | SEQ ID NO:1447 |
| WBC031B06_V1.3_at | TATTATTTTTGGTTTCCCCTCAGTG | SEQ ID NO:1448 |
| Homo sapiens ribosomal protein L4, mRNA (cDNA clone MGC:87190 IMAGE:5259747) | | |
| WBC031C11_V1.3_at | CGTGGGACGTTTTTGCATTTGGACT | SEQ ID NO:1449 |
| WBC031C11_V1.3_at | GCTAAACCCGTATGCGAAGACCATG | SEQ ID NO:1450 |
| WBC031C11_V1.3_at | AGGCCAAGAATCACAAACTCCGGGT | SEQIDNO:1451 |
| WBC031C11_V1.3_at | AGATGATCTTTATGGCACTTGGCGT | SEQ ID NO:1452 |
| WBC031C11_V1.3_at | AGCCTGCTGCGTAAACTTGTTTGTT | SEQ ID NO:1453 |
| WBC031C11_V1.3_at | AAGAGTAACTACAACCTTCCCATGC | SEQ ID NO:1454 |
| WBC031C11_V1.3_at | AAGCGCTTTCCGCAAGTTAGATGAT | SEQ ID NO:1455 |
| WBC031C11_V1.3_at | GAAGACCATGCGCAGGAACACCATT | SEQ ID NO:1456 |
| WBC031C11_V1.3_at | GAAGATTCATCGCAGGGTCCTGAAG | SEQ ID NO:1457 |
| WBC031C11_V1.3_at | GTAAAGCTGCTTCCCTCAAGAGTAA | SEQ ID NO:1458 |
| WBC031C11_V1.3_at | GGTCACATCATTTTGGACAGCTCCT | SEQ ID NO:1459 |
| Homo sapiens syntenin (syc1) mRNA. | | |
| WBC031D02_V1.3_at | ATAGGACAAACTTCTGACTCCCTTC | SEQ ID NO:1460 |
| WBC031D02_V1.3_at | AACCGTGCTACTTTGGCATGGTCAT | SEQ ID NO:1461 |
| WBC031D02_V1.3_at | AAATGTAACTACTGCTGCCTTTCTG | SEQ ID NO:1462 |
| WBC031D02_V1.3_at | AAATGTTCAGCAGCCCAGAGCAGTT | SEQ ID NO:1463 |
| WBC031D02_V1.3_at | GAAACCCACCATGCAGTTCTGTATT | SEQ ID NO:1464 |
| WBC031D02_V1.3_at | GACTCCCTTCTTTCATTTTTAGATT | SEQ ID NO:1465 |
| WBC031D02_V1.3_at | GTTACTGGTATCACTTTCTGAGAAA | SEQ ID NO:1466 |
| WBC031D02_V1.3_at | GGTAATTTGTTCCATGTCCTGACTC | SEQ ID NO:1467 |

(continued)

| Probe Set Name | Sequence | Sequence Identifier |
|---|---|---|
| ILT11A mRNA for immunoglobulin-like transcript 11 protein | | |
| WBC031D02_V1.3_at | GGCAGTGACACTTAACCTGATTTTT | SEQ ID NO:1468 |
| WBC031D02_V1.3_at· | TAATTTGGGCATTCTACTCTTAGCA | SEQ ID NO:1469 |
| WBC031D02_V1.3_at | TAAACTGTCGCCTTAACTATGCTAA | SEQ ID NO:1470 |
| Homo sapiens solute carrier family 2 (facilitated glucose transporter), member 14, mRNA | | |
| WBC031G11_V1.3_at | AAATGGAGATTGGACCCCGATCTTC | SEQ ID NO:1471 |
| WBC031G11_V1.3_at | CCTCTGGTCATTGAGCCTTTGGATA | SEQ ID NO:1472 |
| WBC031G11_V1.3_at | GTATTTTAGTTCTCAGTTCTCACCA | SEQ ID NO:1473 |
| WBC031G11_V1.3_at | GAACAGGTGACTTTCCTCTAACTGT | SEQ ID NO:1474 |
| WBC031G11_V1.3_at | GATCTTCCCTTTCTTGTATGTTGCT | SEQ ID NO:1475 |
| WBC031G11_V1.3_at | GTGTGAGCTCAGGACATGTGGCCTC | SEQ ID NO:1476 |
| WBC031G11_V1.3_at | GGTCATCTACTTTCCCAGTATTTTT | SEQ ID NO:1477 |
| WBC031G11_V1.3_at | TTTTCCCGTTTCTCAGTGACCAGAT | SEQ ID NO:1478 |
| WBC031G11_V1.3_at | TGCTTACACATTTCAGGCCCTTTAG | SEQ ID NO:1479 |
| WBC031G11_V1.3_at | TTTACACTTGCTACAGGTCACGGAC | SEQ ID NO:1480 |
| WBC031G11_V1.3_at | TTCTGGCTCCTCAAAAGCAGTGGGT | SEQ ID NO:1481 |
| Homo sapiens hypothetical protein MGC12966, mRNA | | |
| WBC032D03_V1.3_at | AGATACCGTGTCTTCAGACTGTCTC | SEQ ID NO:1482 |
| WBC032D03_V1.3_at | AACCCTAGCAGCGTTGCCTGAGATA | SEQ ID NO:1483 |
| WBC032D03_V1.3_at | GAGGCCCTACGGTGAAAGTTGCAAT | SEQ ID NO:1484 |
| WBC032D03_V1.3_at | GCTGAATATTACTACACCGTGTGCT | SEQ ID NO:1485 |
| WBC032D03_V1.3_at | GTATTCGTAAAAGTCAGCCCCTCGG | SEQ ID NO:1486 |
| WBC032D03_V1.3_at | GTAGGCTGTTACCATTTCTGCGTTT | SEQ ID NO:1487 |
| WBC032D03_V1.3_at | TGAGATTTTTCTGAGGTGTCCCCTT | SEQ ID NO:1488 |
| WBC032D03_V1.3_at | TCCCCTTTTGTTGATACCTTCGATA | SEQ ID NO:1489 |
| WBC032D03_V1.3_at | TCTGACCATCTACCGGAAGTGCGTA | SEQ ID NO:1490 |
| WBC032D03_V1.3_at | TAAAAACGAGCTTGCGACTCTGCCG | SEQ ID NO:1491 |
| WBC032D03_V1.3_at | TACTTGCTCTGAACCTTGCTGAGTT | SEQ ID NO:1492 |
| Homo sapiens mRNA; cDNA DKFZp686H1588 | | |
| WBC032H02_V1.3_at | ATACGAGTCAGCTTCTGTAGCATAA | SEQ ID NO:1493 |
| WBC032H02_V1.3_at | AGTGTACAAGCGTCATTTATAGTTT | SEQ ID NO:1494 |
| WBC032H02_V1.3_at | AGTGGCCTCACTTCAAGCAGTTTGC | SEQ ID NO:1495 |
| WBC032H02_V1.3_at | AGCAGTTTGCCTTTGTTCTTTAAAT | SEQ ID NO:1496 |
| WBC032H02_V1.3_at | AACTTTTGCTTACTGTGCTCACATC | SEQ ID NO:1497 |
| WBC032H02_V1.3_at | AATTCTTCCACTTGTTTTACATCAC | SEQ ID NO:1498 |
| WBC032H02_V1.3_at | GTATTATGCTTTTCTGTTGAGGGAG | SEQ ID NO:1499 |
| WBC032H02_V1.3_at | GTGGTTTGTCACTGTAATACGAGTC | SEQ ID NO:1500 |
| WBC032H02_V1.3_at· | TTATTTGGGTTAAAGTGGCCTCACT | SEQ ID NO:1501 |

(continued)

| Probe Set Name | Sequence | Sequence Identifier |
|---|---|---|
| ILT11A mRNA for immunoglobulin-like transcript 11 protein | | |
| WBC032H02_V1.3_at | GGTTTGATTTCCTATAATTCTTCCA | SEQ ID NO:1502 |
| WBC032H02_V1.3_at | GGCTTTGAGGATTTTTCTGGCATTA | SEQ ID NO:1503 |
| No Homology | | |
| WBC033D12_V1.3_at | TTTTCCCATAACTGAGCCTACGTGA | SEQ ID NO:1504 |
| WBC033D12_V1.3_at | AAAATGTGTCACTCGCTTTCTTCTG | SEQ ID NO:1505 |
| WBC033D12_V1.3_at | CAGCGTGAACGTACCATAATTTTAA | SEQ ID NO:1506 |
| WBC033D12_V1.3_at | GCTCATGTCTTCATTTGCTTATCCA | SEQ ID NO:1507 |
| WBC033D12_V1.3_at | TTTTCTTTCAGGGAGTTTGCCCCAG | SEQ ID NO:1508 |
| WBC033D12_V1.3_at | GCAACTCTCTAGTGTCAGCGTGTAG | SEQ ID NO:1509 |
| WBC033D12_V1.3_at | GTTGTCCCTTCGGTGGTGTATGAAA | SEQ ID NO:1510 |
| WBC033D12_V1.3_at | TGTAGAGCCCAATACTTCCCAGTAT | SEQ ID NO:1511 |
| WBC033D12_V1.3_at | TATGCATTCCTTTGGTTACTTGTGA | SEQ ID NO:1512 |
| WBC033D12_V1.3_at | TAGTCTCTTGTTTCATTATGCATTC | SEQ ID NO:1513 |
| WBC033D12_V1.3_at | TTGGCAGATTGCTTCAGAGACATGT | SEQ ID NO:1514 |
| Homo sapiens splicing factor, arginine/serine-rich 2, mRNA (cDNA clone MGC: 5480 IMAGE 3452024) | | |
| WBC035E01_V1.3_at | ATAACCTAACTTATGCCCCATTTTG | SEQ ID NO:1515 |
| WBC035E01_V1.3_at· | ATGGCTATGTAACCCACGAGTGCTA | SEQ ID NO:1516 |
| WBC035E01_V1.3_at | ACGAGTGCTATCTAGGTGACCTTCC | SEQ ID NO:1517 |
| WBC035E01_V1.3_at | ACAATCTCAGTCCTTTTTCTTACAG | SEQ ID NO:1518 |
| WBC035E01_V1.3_at | AACAGTATGTCTTCCTGGGAGGGCA | SEQ ID NO:1519 |
| WBC035E01_V1.3_at | AAGTGAAGTGTCTAGTTGCTGCCCT | SEQ ID NO:1520 |
| WBC035E01_V1.3_at | CAGATGGTACTGCTTGTGCTCTCAA | SEQ ID NO:1521 |
| WBC035E01_V1.3_at | GCAGACTCTGCTATGTTTTCTTTGT | SEQ ID NO:1522 |
| WBC035E01_V1.3_at | GACACATTTCATATCCCACTTGTAG | SEQ ID NO:1523 |
| WBC035E01_V1.3_at | GTTGCTGCCCTTTTTACTGAGTTAA | SEQ ID NO:1524 |
| WBC035E01_V1.3_at | TGAGTCCTAGTTGCCATACCAGGTT | SEQ ID NO:1525 |
| Homo sapiens annexin A2, mRNA | | |
| WBC036E08_V1.3_at | GAAAATGCTTTCCTGAACCTGGTCC | SEQ ID NO:1526 |
| WBC036E08_V1.3_at | GAACAAGCCCCTGTATTTTGCTGAC | SEQ ID NO:1527 |
| WBC036E08_V1.3_at | GAGCTACAGCCCTTATGACATGTTG | SEQ ID NO:1528 |
| WBC036E08_V1.3_at | GAGGATGATGTTAGCCTTGTGCCCC | SEQ ID NO:1529 |
| WBC036E08_V1.3_at | GGCACGCGCGATAAGGTCCTGATCA | SEQ ID NO:1530 |
| WBC036E08_V1.3_at | TGTGCTTCCTGCTAACAGTTCTAGA | SEQ ID NO:1531 |
| WBC036E08_V1.3_at | TGATCAGAATCATGGTCTCCCGCAG | SEQ ID NO:1532 |
| WBC036E08_V1.3_at | TGACCGACTGTACGACTCCATGAAG | SEQ ID NO:1533 |
| WBC036E08_V1.3_at | TGCCTCTTGTGTACTGTGTCGTAAA | SEQ ID NO:1534 |

(continued)

| Probe Set Name | Sequence | Sequence Identifier |
|---|---|---|
| ILT11A mRNA for immunoglobulin-like transcript 11 protein | | |
| WBC036E08_V1.3_at | TCTAGTCTCTCCTTTTAGCCAAAGA | SEQ ID NO:1535 |
| WBC036E08_V1.3_at | TATTTTAGATGCCTACGCGTTACCC | SEQ ID NO:1536 |
| Homo sapiens programmed cell death 4 (neoplastic transformation inhibiutor), transcript variant 1, mRNA | | |
| WBC037C09_V1.3_at | AGGGAGCATACTGATTGGTCTTAAA | SEQ ID NO:1537 |
| WBC037C09_V1.3_at | AAGCTACCTTTTGTAAGTGCCATGT | SEQ ID NO:1538 |
| WBC037C09_V1.3_at | AAGTGCCATGTTTATGACCTAATCA | SEQ ID NO:1539 |
| WBC037C09_V1.3_at | CCATGTTGGCTGCTGCTGTTGAAAT | SEQ ID NO:1540 |
| WBC037C09_V1.3_at | GACCTAATCATTCCAAGTTTTGCAT | SEQ ID NO:1541 |
| WBC037C09_V1.3_at | GACTTATTCATCTTTCCAGGGAGCA | SEQ ID NO:1542 |
| WBC037C09_V1.3_at | GTGGAATATTCTCATAAGCTACCTT | SEQ ID NO:1543 |
| WBC037C09_V1.3_at | GGTGGCCGGAACATCTATTTTTCTA | SEQ ID NO:1544 |
| WBC037C09_V1.3_at | TAAGCTGCTAAAACCCCATGTTGGC | SEQ ID NO:1545 |
| WBC037C09_V1.3_at | TGCCACTCCTTTCTTTCAAGGACAG | SEQ ID NO:1546 |
| WBC037C09_V1.3_at | TTGCATTGATGTCTGACTGCCACTC | SEQ ID NO:1547 |
| Homo sapiens ribosomal protein- L3, mRNA (cDNA clone MGC: 70878 IMAGE; 3852576) | | |
| WBC038A02_V1.3_at | ATCTACAAGATTGGCCAGGGCTACC | SEQ ID NO:1548 |
| WBC038A02_V1.3_at | ATGGCCGCTTCCAGACTGTGGAGGA | SEQ ID NO:1549 |
| WBC038A02_V1.3_at | AGGGCTACCTCATCAAGGACGGCAA | SEQ ID NO:1550 |
| WBC038A02_V1.3_at | AGGTTGACCTCAAGTTCATCGACAC | SEQ ID NO:1551 |
| WBC038A02_V1.3_at | AGAAGCGAGTGCTGACCCTTCGCAA | SEQ ID NO:1552 |
| WBC038A02_V1.3_at | AGAACAATGCCTCCACTGATTACGA | SEQ ID NO:1553 |
| WBC038A02_V1.3_at | AAGTCCCTGCTGGTGCAGACGAAGC | SEQ ID NO:1554 |
| WBC038A02_V1.3_at | GACAAGAGCATCAACCCTCTGGGTG | SEQ ID NO:1555 |
| WBC038A02_V1.3_at | GACCAACGACTTTGTCATGCTGAAA | SEQ ID NO:1556 |
| WBC038A02_V1.3_at | GATGCCAGCATCAGATCGTGCAGCT | SEQ ID NO:1557 |
| WBC038A02_V1.3_at | GTGGCTTTGTCCACTACGGTGAAGT | SEQ ID NO:1558 |
| Homo sapiens ribosomal protein L15, mRNA.. | | |
| WBC038B01_V1.3_at | ATATATCGCATTCGTGTGCGCCGTG | SEQ ID NO:1559 |
| WBC038B01_V1.3_at | ATAAGGCGCGCAGACTGGGCTACAA | SEQ ID NO:1560 |
| WBC038B01_V1.3_at | AGAGGGCTGACATCTGCAGGCCGCA | SEQ ID NO:1561 |
| WBC038B01_V1.3_at | AGCTCAAGTTTGCTCGAAGCCTTCA | SEQ ID NO:1562 |
| WBC038B01_V1.3_at | ACTATTGGTGGTTCTCGCCGTGCAG | SEQ ID NO:1563 |
| WBC038B01_V1.3_at | AAGCCTTCAGTCTGTTGCCGAGGAA | SEQ ID NO:1564 |
| WBC038B01_V1.3_at | GCTTCACCGTTACCGCTAATATAAG | SEQ ID NO:1565 |
| WBC038B01_V1.3_at | GCAAGCCTGTGCATCATGGTGTTAA | SEQ ID NO:1566 |
| WBC038B01_V1.3_at | GACGTGATGCGATTTCTTCTCAGGG | SEQ ID NO:1567 |

(continued)

| Probe Set Name | Sequence | Sequence Identifier |
|---|---|---|
| ILT11A mRNA for immunoglobulin-like transcript 11 protein | | |
| WBC038B01_V1.3_at | GAGAAGGCGCAATACTCTGCAGCTT | SEQ ID NO:1568 |
| WBC038B01_V1.3_at | GAGGTGATCCTCATTGATCCATTCC | SEQ ID NO:1569 |
| Homo sapiens NPF/calponin-like protein, mRNA | | |
| WBC039D09_V1.3_at | ATTACTTCTAGCTTGGTTCCTACAA | SEQ ID NO:1570 |
| WBC039D09_V1.3_at | AGGATAATAGAGCTCCTCTCCTCTG | SEQ ID NO:1571 |
| WBC039D09_V1.3_at | AAAGTCTGTCTTGCCACTATGAGTA | SEQ ID NO:1572 |
| WBC039D09_V1.3_at | AATATTTTCTTCTCTAGCATCGCAC | SEQ ID NO:1573 |
| WBC039D09_V1.3_at | CTAGCATCGCACTGTCATTTTTTGT | SEQ ID NO:1574 |
| WBC039D09_V1.3_at | GTTGGGTCTCAACATTGGCTCAAGA | SEQ ID NO:1575 |
| WBC039D09_V1.3_at | GTAACCTAGCCTGAGACAAGTCCTG | SEQ ID NO:1576 |
| WBC039D09_V1.3_at | GGCTTATCATCTGAGAATGCGCCAT | SEQ ID NO:1577 |
| WBC039D09_V1.3_at | TGCGCCATCTTTTTCTCTGGATAAT | SEQ ID NO:1578 |
| WBC039D09_V1.3_at | TTTTTTCCAGTCCTGCCATATCTAA | SEQ ID NO:1579 |
| WBC039D09_V1.3_at | TTCTGCAGTTGTTTTGCGTTTCACT | SEQ ID NO:1580 |
| Leu-8 pan leukocyte antigen | | |
| WBC039F12_V1.3_at | ACATGCACCTTCAACTGCTCAGAAG | SEQ ID NO:1581 |
| WBC039F12_V1.3_at | AAACATTTGGCTGATTTTTGCTTTT | SEQ ID NO:1582 |
| WBC039F12_V1.3_at | GCAGGATACCAAGCTCTATGTTTTA | SEQ ID NO:1583 |
| WBC039F12_V1.3_at | GATCATCAGGAATCTGGTCCAGCAC | SEQ ID NO:1584 |
| WBC039F12_V1.3_at | GTTGGCATTTATCATTTGGCTGGCA | SEQ ID NO:1585 |
| WBC039F12_V1.3_at | GTTTTATAGACATCAGTCCCTGGAG | SEQ ID NO:1586 |
| WBC039F12_V1.3_at | GTGAAGCAGCCCAGTACATACTTTC | SEQ ID NO:1587 |
| WBC039F12_V1.3_at | GTCATGGTTACTGCATTATCTGGGT | SEQ ID NO:1588 |
| WBC039F12_V1.3_at | GGTCCAGCACTAGTCCAATGTGTCA | SEQ ID NO:1589 |
| WBC039F12_V1.3_at | TGGACAGGAGTTTCACGGCGATCAA | SEQ ID NO:1590 |
| WBC039F12_V1.3_at | TCATCCCTGTGGCAGTCATGGTTAC | SEQ ID NO:1591 |
| Human lymphocyte dihydropyrimidine dehydrogenase | | |
| WBC044E01_V1.3_at | ATATATTTTCTATTACTGCCAAACA | SEQ ID NO:1592 |
| WBC044E01_V1.3_at | ATCTCTTTTTTGTGGCATATTTTAA | SEQ ID NO:1593 |
| WBC044E01_V1.3_at | ATGCAACACCAGTTTGACTATTCTT | SEQ ID NO:1594 |
| WBC044E01_V1.3_at | AATGTGCACTTACCAAAAAATCTGT | SEQ ID NO:1595 |
| WBC044E01_V1.3_at | CCAGTTTGACTATTCTTTCTTTTAG | SEQ ID NO:1596 |
| WBC044E01_V1.3_at | GTTTGCATGCTCTGCTTCGGCGGCC | SEQ ID NO:1597 |
| WBC044E01_V1.3_at | GTAACAATCGGAAGCATATTTCTAT | SEQ ID NO:1598 |
| WBC044E01_V1.3_at | GTATGTCTCTATTTTCATAATCAGC | SEQ ID NO:1599 |
| WBC044E01_V1.3_at | GTACTTTTAGAAATGTGCACTTACC | SEQ ID NO:1600 |
| WBC044E01_V1.3_at | TGGGCACAGACCAGGCCACGCTGTG | SEQ ID NO:1601 |

(continued)

| Probe Set Name | Sequence | Sequence Identifier |
|---|---|---|
| ILT11A mRNA for immunoglobulin-like transcript 11 protein | | |
| WBC044E01_V1.3_at | TTCATTTATCTCTTTTTTGTGGCAT | SEQ ID NO:1602 |
| H.sapiens mRNA for BS69 protein. | | |
| WBC047A01_V1.3_at | CTCCTCGTTGCTGTGTGCTTAATTA | SEQ ID NO:1603 |
| WBC047A01_V1.3_at | GAACACTTACCTTCTCTGATTGTTT | SEQ ID NO:1604 |
| WBC047A01_V1.3_at | GTTGAGCTTAGCCACTATGCACATT | SEQ ID NO:1605 |
| WBC047A01_V1.3_at | GTTGACACGGTTGCACATTTCCAAT | SEQ ID NO:1606 |
| WBC047A01_V1.3_at | GTAATTATTCATTGTGGCTGTCCAG | SEQ ID NO:1607 |
| WBC047A01_V1.3_at | GGCTGTCCAGTTCTATGATGCATTC | SEQ ID NO:1608 |
| WBC047A01_V1.3_at | TGTTACTTTGGCTTTTTTGTCTCCG | SEQ ID NO:1609 |
| WBC047A01_V1.3_at | TGAGGCGTCGTAACCAAGAACACTT | SEQ ID NO:1610 |
| WBC047A01_V1.3_at | TCGATCCACCCCTATGAGAGCAAGT | SEQ ID NO:1611 |
| WBC047A01_V1.3_at | TGAACCGGGCAGTGACTTGGCGTCT | SEQ ID NO:1612 |
| WBC047A01_V1.3_at | TTAATTAGTGTTGCCACGGTGCTGT | SEQ ID NO:1613 |
| No homology | | |
| WBC107.V1.3_s_at | ATTCTTTTTCTTTTCCAGTATGAAG | SEQ ID NO:1614 |
| WBC107.V1.3_s_at | ATAGCTTTCATACCTACATTTCAAG | SEQ ID NO:1615 |
| WBC107.V1.3_s_at | ATACCTATCTCAGTTATTCTTTTTC | SEQ ID NO:1616 |
| WBC107.V1.3_s_at | CCAGTATGAAGTTTGCTGAATGTAC | SEQ ID NO:1617 |
| WBC107.V1.3_s_at | GCTAGACTTATTACTGTGCACGAGA | SEQ ID NO:1618 |
| WBC107.V1.3_s_at | GAAGCTGTTCTTGAACTAACACGGA | SEQ ID NO:1619 |
| WBC107.V1.3_s_at | GATTGTCTTGATTTGATCCGAAACA | SEQ ID NO:1620 |
| WBC107.V1.3_s_at | GGGCCGTGTCAGGAAATTACCTATA | SEQ ID NO:1621 |
| WBC107.V1.3_s_at | GGTGTAGAAGCTCAGCTAGACTTAT | SEQ ID NO:1622 |
| WBC107.V1.3_s_at | GGATAATATCTTCATGTTTCTGAAA | SEQ ID NO:1623 |
| WBC107.V1.3_s_at | TGTGCACGAGAGTAAATACCTATCT | SEQ ID NO:1624 |
| No Homology | | |
| WBC117.gRSP.V1.3_at | AAATAATCTTGCAGTACCGGAGTAT | SEQ ID NO:1625 |
| WBC117.gRSP.V1.3_at | GAAACTTTTCCTGTAGAGGGCCGCT | SEQ ID NO:1626 |
| WBC117.gRSP.V1.3_at | GCAAAAGCATTCATCAGCAGCGTGT | SEQ ID NO:1627 |
| WBC117.gRSP.V1.3_at | GAAAGTTTCCCACCTGCTAGGAAAG | SEQ ID NO:1628 |
| WBC117.gRSP.V1.3_at | GAAATGGCCTCAGGAAAGTTTCCCA | SEQ ID NO:1629 |
| WBC117.gRSP.V1.3_at: | GACCCTGCCGTCATCATGCAAAAGC | SEQ ID NO:1630 |
| WBC117.gRSP.V1.3_at | GAGGGCCGCTTTGTAAATATTTTAG | SEQ ID NO:1631 |
| WBC117.gRSP.V1.3_at | GAGGGCAACACAATTTCTGTCGCAA | SEQ ID NO:1632 |
| WBC117.gRSP.V1.3_at | TGGCCACTTTTGTCATTCAGCACTT | SEQ ID NO:1633 |
| WBC117.gRSP.V1.3_at | TGAATTTTACCTGTTTTCTACTCTA | SEQ ID NO:1634 |
| WBC117.gRSP.V1.3_at | TACCTATCTAATACAGCAGCCACTG | SEQ ID NO:1635 |

(continued)

| Probe Set Name | Sequence | Sequence Identifier |
|---|---|---|
| ILT11A mRNA for immunoglobulin-like transcript 11 protein | | |
| Homo sapiens CDC14 cell division cycle 14 homolog A (S cerevisiae) (CDC14A) transcript variant 1 mRNA | | |
| WBC215.gRSP.V1.3_at | AACACTAATTTGTGTGCAGCTATTA | SEQ ID NO:1636 |
| WBC215.gRSP.V1.3_at | AATTGACTACTGTAAATTTTCCACA | SEQ ID NO:1637 |
| WBC215.gRSP.V1.3_at | AATTGTGACCTTTTCTAAAGCTGAA | SEQ ID NO:1638 |
| WBC215.gRSP.V1.3_at | GAAAGGGAACCATATTGACAGTTTT | SEQ ID NO:1639 |
| WBC215.gRSP.V1.3_at | GACAGTTTTAGTTCTGTGAACACTA | SEQ ID NO:1640 |
| WBC215.gRSP.V1.3_at | GAGACTATTGTACGTGCTTTCAGCA | SEQ ID NO:1641 |
| WBC215.gRSP.V1.3_at | GTTGACTTCAATAACTACAGACCAA | SEQ ID NO:1642 |
| WBC215.gRSP.V1.3_at | GTTTGACTCAGGTTTGTTGACTTCA | SEQ ID NO:1643 |
| WBC215.gRSP.V1.3_at | GTATAGCATAGTAATTCTACTCCAA | SEQ ID NO:1644 |
| WBC215.gRSP.V1.3_at | GTGCTTTCAGCAAAACATCTTATTC | SEQ ID NO:1645 |
| WBC215.gRSP.V1.3_at | GGAGGGCTACCTTGAGACTATTGTA | SEQ ID NO:1646 |
| Homo sapiens hypothetical protein MGC5576, mRNA | | |
| WBC309.V1.3_at | ATTCAGATCTTTTCAGCTGTTCTTG | SEQ ID NO:1647 |
| WBC309.V1.3_at | ATCATACTGTGGTTCTAGGTTATCA | SEQ ID NO:1648 |
| WBC309.V1.3_at | AGGAAAACTCCTCTCTGTAGCACCA | SEQ ID NO:1649 |
| WBC309.V1.3_at | AGTGCCTGGCCATATGCCCAGCAAA | SEQ ID NO:1650 |
| WBC309.V1.3_at | AAAGCAAACTTTTTCCTGTAGAGAG | SEQ ID NO:1651 |
| WBC309.V1.3_at | AAGCAAGCACAGACCGTTCAGCGTA | SEQ ID NO:1652 |
| WBC309.V1.3_at | CTGTAGCACCATTTCTGTTTCTGAG | SEQ ID NO:1653 |
| WBC309.V1.3_at | GAAGTTTTTTCATGTGTTCCCAAAT | SEQ ID NO:1654 |
| WBC309.V1.3_at | GTTAGTGCAGCTCCAGAACTGTCAT | SEQ ID NO:1655 |
| WBC309.V1.3_at | TCAGCTGTTCTTGGGCCGTTATAAA | SEQ ID NO:1656 |
| WBC309.V1.3_at | TTTGATTTACCCTTCATCCATTGGC | SEQ ID NO:1657 |
| Homo sapiens lymphoid enhancer-binding factor 1 (LEF1) mRNA (cDNA clone MGC : 60112 IMAGE. 6054813) | | |
| WBC310.V1.3_at | AGCCGACCCGTTTGCAGTGAATTTT | SEQ ID NO:1658 |
| WBC310.V1.3_at | ATTTTCTGCCGATCTCTTCTTTTTT | SEQ ID NO:1659 |
| WBC310.V1.3_at | AAAGCCCAGCACTTGAATTGTTCTT | SEQ ID NO:1660 |
| WBC310.V1.3_at | AAACTTTCCCAGATATTCTCAGCCG | SEQ ID NO:1661 |
| WBC310.V1.3_at | GAGTCCTGTTTCGTTTTATTTTCTG | SEQ ID NO:1662 |
| WBC310.V1.3_at | GATGAGTGGCTTCTCTGTGATCACC | SEQ ID NO:1663 |
| WBC310.V1.3_at | GTCATCTAACGTTTGTCATTCCAGT | SEQ ID NO:1664 |
| WBC310.V1.3_at | TGTGATCACCTGTCACGATTCGTGG | SEQ ID NO:1665 |
| WBC310.V1.3_at | TGAATTTTCATTTCCTTCCTTCTTG | SEQ ID NO:1666 |
| WBC310.V1.3_at | TTGCCCCTTGTTGTAAAAAGCCCAG | SEQ ID NO:1667 |

(continued)

| Probe Set Name | Sequence | Sequence Identifier |
|---|---|---|
| ILT11A mRNA for immunoglobulin-like transcript 11 protein | | |
| WBC310.V1.3_at | TTGGTCGAGTGTTCGGCAGAGCCAC | SEQ ID NO:1668 |
| Homo sapiens lymphoid enhancer-binding factor 1, mRNA (cDNA clone MGC : 60112 IMAGE : 6054813) | | |
| WBC311.V1.3_at | GGTCTTGGAACATAGGAGGTGCTCA | SEQ ID NO:1669 |
| WBC311.V1.3_at | ATTTTTGGCCATATTTCACTTAGTT | SEQ ID NO:1670 |
| WBC311.V1.3_at | AGTATTCCCGTATTGCATTTTGCTA | SEQ ID NO:1671 |
| WBC311.V1.3_at | ACTCACTTTTTAACTGTACACCTTG | SEQ ID NO:1672 |
| WBC311.V1.3_at | AAATGTATTGTTTCAACTCACTTTT | SEQ ID NO:1673 |
| WBC311.V1.3_at | AAATACTAGTTGACTGCACAGCGAG | SEQ ID NO:1674 |
| WBC311.V1.3_at | AATGCATGATGATTATTTTTGGCCA | SEQ ID NO:1675 |
| WBC311.V1.3_at | GACTGCACAGCGAGTAGTGTACTAA | SEQ ID NO:1676 |
| WBC311.V1.3_at | GAGATGTCCTTATTGGGTAACCATG | SEQ ID NO:1677 |
| WBC311.V1.3_at | GTACACCTTGTAGTTATTTTCTATT | SEQ ID NO:1678 |
| WBC311.V1.3_at | TATTTCACTTAGTTTTTTCAGGCTA | SEQ ID NO:1679 |
| Homo sapiens maltase-glucoamylase mRNA. | | |
| WBC327.V1.3_at | ATTGCCAGGACAGTTCCAGGCTCTG | SEQ ID NO:1680 |
| WBC327.V1.3_at | ATAACTTCTTTTTGCAGTGGTGTGC | SEQ ID NO:1681 |
| WBC327.V1.3_at | AGCGTAATCAGGGATTGGGCCCCAC | SEQ ID NO:1682 |
| WBC327.V1.3_at | CAGTGGTGTGCTTTGAAATTTGTCA | SEQ ID NO:1683 |
| WBC327.V1.3_at | GCTCTGGAGCTAACTAAGCCATCAA | SEQ ID NO:1684 |
| WBC327.V1.3_at | GAAATGCTTAGCTATAGGACTGGGA | SEQ ID NO:1685 |
| WBC327.V1.3_at | GATGTATATAACCACAGTTCCCATA | SEQ ID NO:1686 |
| WBC327.V1.3_at | GTCTTATTTCTATACTCTGCCTGAT | SEQ ID NO:1687 |
| WBC327.V1.3_at | GTGTGCATGTCTATGTGAGCGTAAT | SEQ ID NO:1688 |
| WBC327.V1.3_at | GGAAGAGCCAACTCACCTGAAAGAA | SEQ ID NO:1689 |
| WBC327.V1.3_at | TTGGGCCCCACTCTTGGTAAATACA | SEQ ID NO:1690 |
| Homo sapiens radixin, mRNA (cDNA clone MGC : 48283 IMAGE : 52844-38), complete cds | | |
| WBC335.V1.3_at | AATTCCAACAGGCTTTTACCATTAG | SEQ ID NO:1691 |
| WBC335.V1.3_at | ATGCTTTCAAGCCTGTGTTCACTTT | SEQ ID NO:1692 |
| WBC335.V1.3_at | ACTAGAGGGTGAACGCTGCTGTTGA | SEQ ID NO:1693 |
| WBC335.V1.3_at | AACGCTGCTGTTGATGCTTTCAAGC | SEQ ID NO:1694 |
| WBC335.V1.3_at | AATCCATTTTTAAAGCTGTGTGAAT | SEQ ID NO:1695 |
| WBC335.V1.3_at | GAGCGTTAAAACAATTGCCATAAAG | SEQ ID NO:1696 |
| WBC335.V1.3_at | GAGAGCACATGTTCATATAGCAATG | SEQ ID NO:1697 |
| WBC335.V1.3_at | GTCACATCAGTTTGATATCAGTGTT | SEQ ID NO:1698 |
| WBC335.V1.3_at | GTGCACTACCATTGTCACATCAGTT | SEQ ID NO:1699 |
| WBC335.V1.3_at | GTGTTCACTTTTCCAGAGCGTTAAA | SEQ ID NO:1700 |

(continued)

| Probe Set Name | Sequence | Sequence Identifier |
|---|---|---|
| ILT11A mRNA for immunoglobulin-like transcript 11 protein | | |
| WBC335.V1.3_at | TTCATTTTGAATTGTGTGCACTACC | SEQ ID NO:1701 |
| No Homology | | |
| WBC374.V1.3_at | ATTTGGGCTTCTAACTGCAGCGTTT | SEQ ID NO:1702 |
| WBC374.V1.3_at | ATTTTGATTATGTTGCTTACCTTGA | SEQ ID NO:1703 |
| WBC374.V1.3_at | ATAATTTAGTGTCTCTGGCACACAC | SEQ ID NO:1704 |
| WBC374.V1.3_at | ATGTTTTCACATTCCTTTGTCCGGA | SEQ ID NO:1705 |
| WBC374.V1.3_at | ACTTCAGTTGTGATCTTGGTGACAT | SEQ ID NO:1706 |
| WBC374.V1.3_at | AACTCAGCATCCTTGTTTTCTGCAA | SEQ ID NO:1707 |
| WBC374.V1.3_at | CCATGTCTTTGTTGTACTTGTGCAG | SEQ ID NO:1708 |
| WBC374.V1.3_at | GACATATCTGTTGGCTTAGGTTTTA | SEQ ID NO:1709 |
| WBC374.V1.3_at | GATGCTTTTGTTTTGGGTTATGGTC | SEQ ID NO:1710 |
| WBC374.V1.3 at | TTATCGCAGATGTTCGGTTGACCAA | SEQ ID NO:1711 |
| WBC374.V1.3_at | TTGTAATTACCTTCCCATGTCTTTG | SEQ ID NO:1712 |
| No Homology | | |
| WBC964.gRSP.V1.3_at | AGTGATTCAGTTTTCTATAGCTTAC | SEQ ID NO:1713 |
| WBC964.gRSP.V1.3_at | ACCACTGCAAGAGGACGCTGAGATT | SEQ ID NO:1714 |
| WBC964.gRSP.V1.3_at | ACCCTATTTCTCACTGTCTTCATTA | SEQ ID NO:1715 |
| WBC964.gRSP.V1.3_at | ACTGTCTTCATTATTTCACGTAGGA | SEQ ID NO:1716 |
| WBC964.gRSP.V1.3_at | AAAGCCTTGTTATTATTTGATCCTA | SEQ ID NO:1717 |
| WBC964.gRSP.V1.3_at | CACTTCTAACCTGAAAGCTGCTTGG | SEQ ID NO:1718 |
| WBC964.gRSP.V1.3_at | CAAAATGGATCTGTGCGCTGTGGCA | SEQ ID NO:1719 |
| WBC964.gRSP.V1.3_at | GATTTTCTATGTCTTCCACTTCTAA | SEQ ID NO:1720 |
| WBC964.gRSP.V1.3_at | GTGCGCTGTGGCAGAACTGTTATTT | SEQ ID NO:1721 |
| WBC964.gRSP.V1.3_at | GGATTTTACAGCTCAACTAACAGTG | SEQ ID NO:1722 |
| WBC964.gRSP.V1.3_at | TACAGTTTATTTTTTGCACACCCTA | SEQ ID NO:1723 |
| Homo sapiens AT rich interactive domain 5A (MRF1-like) mRNA (cDNA clone MGC:75508 IMAGE : 30409017) | | |
| BM734632.V1.3_at | ACCGACTGACGCCTCAGGAGGAAAT | SEQ ID NO:1724 |
| BM734632.V1.3_at | GCTGAGAACTCCAGGCACCGACTGA | SEQ ID NO:1725 |
| BM734632.V1.3_at | AGGGCAGGCTGAGAACTCCAGGCAC | SEQ ID NO:1726 |
| BM734632.V1.3_at | AGTCTTAAAGATGGGCTGCTATTGG | SEQ ID NO:1727 |
| BM734632.V1.3_at | AGAAAGGGCAGCCTCTACCCTAAGC | SEQ ID NO:1728 |
| BM734632.V1.3_at | ACGCCTACCCCACTGAGGTACTGAA | SEQ ID NO:1729 |
| BM734632.V1.3_at | CTGGTCAAAGAACCCCAGCTGGTGT | SEQ ID NO:1730 |
| BM734632.V1.3_at | GGACTTCTTCCCCAGTCTTAAAGAT | SEQ ID NO:1731 |
| BM734632.V1.3_at | TGCATTCCACAAAGGCGGCTCCAGA | SEQ ID NO:1732 |
| BM734632.V1.3_at | TGAGGTACTGAAGCCCATCAGCCAG | SEQ ID NO:1733 |

(continued)

| Probe Set Name | Sequence | Sequence Identifier |
|---|---|---|
| ILT11A mRNA for immunoglobulin-like transcript 11 protein | | |
| BM734632.V1.3_at | TAAGCCTAAAGCCTGCTGGGTGTCC | SEQ ID NO:1734 |
| Homo sapiens sorting nexin 3 (SNX3), transcript variant 1 mRNA | | |
| WBC028C09_V1.3_at | AATGTGGTGCACACTATAGCCTCAC | SEQ ID NO:1735 |
| WBC02BC09_V1.3_at | GAAATAGCTCTCTGTGCTGTTTGAA | SEQ ID NO:1736 |
| WBC028C09_V1.3_at | GAGTAGCTCCCTTCATCTGGAAGAT | SEQ ID NO:1737 |
| WBC028C09_V1.3_at | GTATAACCTGCAGTGTCCTAACTAA | SEQ ID NO:1738 |
| WBC028C09_V1.3_at | GTAGGAGCACTACACCATTGGTTCC | SEQ ID NO:1739 |
| WBC028C09_V1.3_at | GGTTCCCTACCCTACATGTAAGAGA | SEQ ID NO:1740 |
| WBC028C09_V1.3_at | GTGTCATGTTTGCTTCTTGAATCTG | SEQ ID NO:1741 |
| WBC028C09_V1.3_at | TAGAATATTTCTCTTATCCCCTTTT | SEQ ID NO:1742 |
| WBC028C09_V1.3_at | TAGCCTCACAAACCTGTTATTCCAG | SEQ ID NO:1743 |
| WBC028C09_V1.3_at | TTTAGTGAAAATCCCTCATTCTGGC | SEQ ID NO:1744 |
| WBC028C09_V1.3_at | TTACTGGCTTGGTCTTATCTGCACA | SEQ ID NO:1745 |
| Human Lyn B protein mRNA. | | |
| WBC039G08_V1.3_at | ATGCCTATCATGACACTTCTTTCTA | SEQ ID NO:1746 |
| WBC039G08_V1.3_at | AAACTGCTCTCTCTTCCAAAGGGAT | SEQ ID NO:1747 |
| WBC039G08_V1.3_at | AAACATTTTCCTTCCTTGGAGGCTG | SEQ ID NO:1748 |
| WBC039G08_V1.3_at | AACGCGTCACTTGTGAGCCTGCAGA | SEQ ID NO:1749 |
| WBC039G08_V1.3_at | AATGGTCCAGCTTTTCAATTCCACC | SEQ ID NO:1750 |
| WBC039G08_V1.3_at | CTGCAGAACCTTCTAGGGCCTTATA | SEQ ID NO:1751 |
| WBC039G08_V1.3_at | GAATTTTCTTTGTTGCTTCAGATGA | SEQ ID NO:1752 |
| WBC039G08_V1.3_at | GGAAGCAGCAGCACTTTCCTGGTCT | SEQ ID NO:1753 |
| WBC039G08_V1.3_at | TCCTACCTACAGACTTTTTTGACAG | SEQ ID NO:1754 |
| WBC039G08_V1.3_at | TCCAAGATTTCAGTGATTTCCCCTC | SEQ ID NO:1755 |
| WBC039G08_V1.3_at | TTTCTATTTTCTCTGTGCTTTGGCT | SEQ ID NO:1756 |
| No homology | | |
| WBC21.V1.3_at | ATTCCTCTGTGTTTTATGTTACTGT | SEQ ID NO:1757 |
| WBC21.V1.3_at | ATCTGATTCCCCTAAATGCGTAGAA | SEQ ID NO:1758 |
| WBC21.V1.3_at | ATCACTATTTTCACTTTGACTGACA | SEQ ID NO:1759 |
| WBC21.V1.3_at | AGAGGCATTAGTGGCTTTGGCTAAA | SEQ ID NO:1760 |
| WBC21.V1.3_at | CAAACTACTTACCTGATTGTCAAGA | SEQ ID NO:1761 |
| WBC21.V1.3_at | AAGCATTGCTGTATCTATTTTTGAA | SEQ ID NO:1762 |
| WBC21.V1.3_at | CAAGTTATGCTGTTTCCTCTATTCT | SEQ ID NO:1763 |
| WBC21.V1.3_at | GACCTGTTAGGCAAAATCACTATTT | SEQ ID NO:1764 |
| WBC21.V1.3_at | TAAGACACTTGATGTATTCCTCTGT | SEQ ID NO:1765 |
| WBC21.V1.3_at | TAGAGACTCTTTCATCCATAAGCCC | SEQ ID NO:1766 |
| WBC21.V1.3_at | TTTAACAAAGCTAACCACATCCCCT | SEQ ID NO:1767 |

(continued)

| Probe Set Name | Sequence | Sequence Identifier |
|---|---|---|
| ILT11A mRNA for immunoglobulin-like transcript 11 protein | | |
| Homo sapiens arachidonate 5-lipoxygenase-activating protein (ALOX5AP) | | |
| WBC040E12_V1.3_at | AAATCATCATAACTCAACTCCTACG | SEQ ID NO:1768 |
| WBC040E12_V1.3_at | AACTTTGTGTCAGCGATACCCTTTA | SEQ ID NO:1769 |
| WBC040E12_V1.3_at | AACTATTATCTCATCCTCTTTTTCG | SEQ ID NO:1770 |
| WBC040E12_V1.3_at | AATACAGGGTTGGTGCTCTCATCTA | SEQ ID NO:1771 |
| WBC040E12_V1.3_at | GAAGTACTTCGTGGGCTATCTGGGA | SEQ ID NO:1772 |
| WBC040E1.2_V1.3_at | GAAACGCATCATACTGTTCCTGTTC | SEQ ID NO:1773 |
| WBC040E12_V1.3_at | GAGTTAACCTTGCTTTTCCTGGAAG | SEQ ID NO:1774 |
| WBC040E12_V1.3_at | GATCCATTCTGTGTGTTTCTTGACT | SEQ ID NO:1775 |
| WBC040E12_V1.3_at | TGGGCTTCGCGTCTTGAGTTAACCT | SEQ ID NO:1776 |
| WBC040E12_V1.3_at | TACGGTCCTCTTTAGATTGCTGTAA | SEQ ID NO:1777 |
| WBC040E12_V1.3_at | TTGACTTACCCTGCTTTCTGAAGAT | SEQ ID NO:1778 |
| No homology | | |
| BM780906.V1.3_at | AGCTGAAACACATCTCTTGGGTCCT | SEQ ID NO:1779 |
| BM780906.V1.3_at | GCTTCCCATCATAGTTTTGCCGTTA | SEQ ID NO:1780 |
| BM780906.V1.3_at | GATTCCCAGAATGCCATCGATGACC | SEQ ID NO:1781 |
| BM780906.V1.3_at | GTTCCGTTTTCAAGGACCAGTCAGC | SEQ ID NO:1782 |
| BM780906.V1.3_at | GTAACAGTGACTCCTGATTCCCAGA | SEQ ID NO:1783 |
| BM780906.V1.3_at | GTGTGAGCAGCTCCTCCTGTATAGT | SEQ ID NO:1784 |
| BM780906.V1.3_at | TGGAGCTTTTGCCTGTAGCTTGAGA | SEQ ID NO:1785 |
| BM780906.V1.3_at | TGTATAGTCCTCTTCTTCACTGAAT | SEQ ID NO:1786 |
| BM780906.V1.3_at | TCAGTACGTCAGTGGTGGAGCTTTT | SEQ ID NO:1787 |
| BM780906.V1.3_at | TTCACTGAATGCTGGAACCTCCAAC | SEQ ID NO:1788 |
| BM780906.V1.3_at | TTTACCCGCAGTATCAAGCACAAGA | SEQ ID NO:1789 |
| No homology | | |
| B1961872.V1.3_at | AGATTTGACCTGTTCATGCGTCTGT | SEQ ID NO:1790 |
| B1961872.V1.3_at | TCCGTCTCCCGGCTATATAGTAATC | SEQ ID NO:1791 |
| B1961872.V1.3_at | GAGTTGTCATTTTCTCTTCACTGGA | SEQ ID NO:1792 |
| B1961872.V1.3_at | GTTCATGCGTCTGTGGAGCAGCCCT | SEQ ID NO:1793 |
| B1961872.V1.3_at | GTAATCTTAGGTAGAGTGTTGCCTT | SEQ ID NO:1794 |
| B1961872.V1.3_at | GTCATTTTCTCTTCACTGGATGTTT | SEQ ID NO:1795 |
| B1961872.V1.3_at | TAGAGTGTTGCCTTGTGGGTTACCG | SEQ ID NO:1796 |
| B1961872.V1.3_at | TGGGTTACCGTTTGCTCTGAGACTT | SEQ ID NO:1797 |
| B1961872.V1.3_at | TCTGAGACTTCTCGGATGGACCCAC | SEQ ID NO:1798 |
| B1961872.V1.3_at | TCTCTTCACTGGATGTTTATTTATA | SEQ ID NO:1799 |
| B1961872.V1.3_at | TATGCTCTGTGTATAAGGATGAATT | SEQ ID NO:1800 |
| Equus caballus IL-1RII mRNA for interleukin-1 receptor type II | | |

(continued)

| Probe Set Name | Sequence | Sequence Identifier |
|---|---|---|
| ILT11A mRNA for immunoglobulin-like transcript 11 protein | | |
| BM781269.V1.3_at | ATGGCACACGAGTCAACATCACTAG | SEQ ID NO:1801 |
| BM781269.V1.3_at | ACCGATCTGTGGTGGATGGCCAACA | SEQ ID NO:1802 |
| BM781269.V1.3_at | ACTAGTTTGCCCTGAGCTGAGTGAA | SEQ ID NO:1803 |
| BM781269.V1.3_at | AAGACAGAAGCTGCCCTGACTTACA | SEQ ID NO:1804 |
| BM781269.V1.3_at | GAAACGCCTCGTACTGCTATGAAAT | SEQ ID NO:1805 |
| BM781269.V1.3_at | GAAATATCGAACTCCGTGTCCAGAA | SEQ ID NO:1806 |
| BM781269.V1.3_at | GATGCGGGCTATTACAGCTGTGTCA | SEQ ID NO:1807 |
| BM781269.V1.3_at | GTGTCATGACCTTGATCCACAATGG | SEQ ID NO:1808 |
| BM781269.V1.3_at | GGACAATGCTCTCTGGATTTTGCCG | SEQ ID NO:1809 |
| BM781269.V1.3_at | TAAGGTGTTTCTGGGAGCCGGCACA | SEQ ID NO:1810 |
| BM781269.V1.3_at | TTGTCAACCTCTGGCTTACTAGTTT | SEQ ID NO:1811 |
| No Homology | | |
| Foe1092.V1.3_at | ACTGTTTCTGGCACCCACTGAAAAA | SEQ ID NO:1812 |
| Foe1092.V1.3_at | ACATGGTCTGACCTGCACTGTTTCT | SEQ ID NO:1813 |
| Foe1092.V1.3_at | ACAACTGCCTTCTCCATGTTTATAC | SEQ ID NO:1814 |
| Foe1092.V1.3_at | GCTTTTCCAGATTTTAGTCTCCTTT | SEQ ID NO:1815 |
| Foe1092.V1.3_at | GAAAACTACTGTGGCCTTTCTGCTT | SEQ ID NO:1816 |
| Foe1092.V1.3_at | GAGTTGGTTCATCCCATTTGAGGAC | SEQ ID NO:1817 |
| Foe1092.V1.3_at | GAGGGAGTCTATTTTCACTCATTCT | SEQ ID NO:1818 |
| Foe1092.V1.3_at | GTGGCTTTTGCCTAACACTTTTGCA | SEQ ID NO:1819 |
| Foe1092.V1.3_at | TGTCTTCCCATATTCATACCTGCAT | SEQ ID NO:1820 |
| FoelO92.V1.3_at | TGCCTTTCTCACCATTTTGGTTTGA | SEQ ID NO:1821 |
| Foe1092.V1.3_at | TAAACTTCTCTGCACTCCATGATGG | SEQ ID NO:1822 |
| No Homology | | |
| WBC001E10_V1.3_at | AACATTAAGCACTGTTGGCCTCCTG | SEQ ID NO:1823 |
| WBC001E10_V1.3_at | AAGATGCTCATCCAGGTCATCCTGA | SEQ ID NO:1824 |
| WBC001E10_V1.3_at | CTGGTGCCCAGTGATCGGAGGTCCA | SEQ ID NO:1825 |
| WBC001E10_V1.3_at | GCCGTGATCATCTCTTGCCTAAGAT | SEQ ID NO:1826 |
| WBC001E10_V1.3_at | GACAAATTCACAGCTCTCACAGGAA | SEQ ID NO:1827 |
| WBC001E10_V1.3_at | GAAACAGTGTTCTCAACCTTGACAA | SEQ ID NO:1828 |
| WBC001E10_V1.3_at | GAGGGCCCCATTTGAGAAAGTCCGC | SEQ ID NO:1829 |
| WBC001E10_V1.3_at | GTCCAGGTGGAGATGCCGTGATCAT | SEQ ID NO:1830 |
| WBC001E10_V1.3_at | GGTCCTAAATTATTTTCTCTCTCCT | SEQ ID NO:1831 |
| WBC001E10_V1.3_at | GGATCCCTTACTGTGATGTTTGTTC | SEQ ID NO:1832 |
| WBC001E10_V1.3_at | TTCCTTCTGCAGTCTTTAGGGTGAA | SEQ ID NO:1833 |
| No Homology | | |
| WBC038D08_V1.3_at | ATCCGTACCTACCAAGAGCACAGAG | SEQ ID NO:1834 |

(continued)

| Probe Set Name | Sequence | Sequence Identifier |
|---|---|---|
| ILT11A mRNA for immunoglobulin-like transcript 11 protein | | |
| WBC03BD08_V1.3_at | ATGAGCAGGTAAAGCCAACGTTTTT | SEQ ID NO:1835 |
| WBC038D08_V1.3_at | AGGGACCTTGCTGCCTTTGATAAAT | SEQ ID NO:1836 |
| WBC038D08_V1.3_at | CTGTGACTGCCATGCTGGTGGGAAA | SEQ ID NO:1837 |
| WBC038D08_V1.3_at | CTGATTCCACACATTTCTCTTTTTT | SEQ ID NO:1838 |
| WBC038D08_V1.3_at | GACTATGGGTTTGGCAGGACTTCTG | SEQ ID NO:1839 |
| WBC038D08_V1.3_at | GAGCAAGTATGCACCCTGTGACTGC | SEQ ID NO:1840 |
| WBC038D08_V1.3_at | GTCGTTATCCATCTTTCTTTTTCTT | SEQ ID NO:1841 |
| WBC038D08_V1.3_at | GGTTAATCTGTTTGCTTTTCACTTA | SEQ ID NO:1842 |
| WBC038D08_V1.3_at | TAAATCCCATGATCAAGCCATCCGT | SEQ ID NO:1843 |
| WBC038D08_V1.3_at | TAAGGACCTGCCTATTGCTGATTCC | SEQ ID NO:1844 |
| Homo sapiens nuclear factor (erythroid-derived. 2) like 2, mRNA | | |
| WBC020H11_V1.3_at | AGCGAATACTCCCTGCAGCAGACGA | SEQ ID NO:1845 |
| WBC020H11_V1.3_at | AATTTCTTAGAACACCATTTGGGCT | SEQ ID NO:1846 |
| WBC020H11_V1.3_at | ACGAGAGACGGCAGCGTGTTCCTTG | SEQ ID NO:1847 |
| WBC020H11_V1.3_at | ACCATTTGGGCTAGTTTCTGTGTAA | SEQ ID NO:1848 |
| WBC020H11_V1.3_at | AAATTGTCTAGCTCTGGCAAAACAT | SEQ ID NO:1849 |
| WBC020H11_V1.3_at | CTAAAAGCTCCTACTGTGATGTGAA | SEQ ID NO:1850 |
| WBC020H11_V1.3_at | GAAGTCTTCAGCATGCTACGTGACG | SEQ ID NO:1851 |
| WBC020H11_V1.3_at | GACGAAGATGGAAGACCTTACTCCC | SEQ ID NO:1852 |
| WBC020H11_V1.3_at | GTTCTTATCTCATTTGTTACGTTCC | SEQ ID NO:1853 |
| WBC020H11_V1.3_at | GTGTTCCTTGTTCCCAAGAGCAGGA | SEQ ID NO:1854 |
| WBC020H11_V1.3_at | GGAAGATGACCTTTTGAGCTAGTGT | SEQ ID NO:1855 |
| Homo sapiens mRNA; cDNA DKFZp686D0662 (from clone DKFZp686D0662) | | |
| WBC033A01_V1.3_at | ATCTCTATCAGGAATACTGCCTCAG | SEQ ID NO:1856 |
| WBC033A01_V1.3_at | ATGCTGGCACATCATTTTGCTGGAG | SEQ ID NO:1857 |
| WBC033A01_V1.3_at | AGAGCATTAACCTGCCTAATCTTCA | SEQ ID NO:1858 |
| WBC033A01_V1.3_at | AACCTGCCTAATCTTCATGGTGAAA | SEQ ID NO:1859 |
| WBC033A01_V1.3_at | AATTACACCTTCTCTATTCTAGTCA | SEQ ID NO:1860 |
| WBC033A01_V1.3_at | GAGTTTTTTATATACTGTAGCCTGA | SEQ ID NO:1861 |
| WBC033A01_V1.3_at | GTGTTTGACTATTTTCCAACTTGTA | SEQ ID NO:1862 |
| WBC033A01_V1.3_at | TGTGCATGCTGCTGTCTCTGTTGGG | SEQ ID NO:1863 |
| WBC033A01_V1.3_at | TATTTTAGTAGCTGATGCTGGCACA | SEQ ID NO:1864 |
| WBC033A01_V1.3_at | TCTCTATTCTAGTCATGCTGTGCAT | SEQ ID NO:1865 |
| WBC033A01_V1.3_at | TACTGCCTCAGTTATTGAACTTTTT | SEQ ID NO:1866 |
| Homo sapiens membrane-spanning- 4-domains, subfamily A, member 6A, transcript variant 1, mRNA (cDNA clone MGC:22650 IMAGE:3934920) | | |
| BM781186.V1.3_at | ATAGGAGCCTTGTGTTTTGTCATCT | SEQ ID NO:1867 |

(continued)

| Probe Set Name | Sequence | Sequence Identifier |
|---|---|---|
| ILT11A mRNA for immunoglobulin-like transcript 11 protein | | |
| BM781186.V1.3_at | AAGCCTTTGGTTCAGAGCAGCCTAG | SEQ ID NO:1868 |
| BM781186.V1.3_at | AAGTTCTCGGGACTATCCAGATCCT | SEQ ID NO:1869 |
| BM781186.V1.3_at | CAGCATTTTACCCAAGCGTTTTCTA | SEQ ID NO:1870 |
| BM781186.V1.3_at | CTGCAAACATTCTGAGCTCTCTATC | SEQ ID NO:1871 |
| BM781186.V1.3_at | GAAGGCTGCTTACCCATTCATAGGA | SEQ ID NO:1872 |
| BM781186.V1.3_at | GAATTATTTTGGCATCGGCTTCCTT | SEQ ID NO:1873 |
| BM781186.V1.3_at | GTCATACTGGCTTCTTTGGGTCCTG | SEQ ID NO:1874 |
| BM781186.V1.3_at | GTCATCTCTGGATCTCTATCAATCA | SEQ ID NO:1875 |
| BM781186.V1.3_at | TAGCTGGAACCAACGGGCTGATCCT | SEQ ID NO:1876 |
| BM781186.V1.3_at | TATCAGCTCTGGTGGGTTTCATCCT | SEQ ID NO:1877 |
| No Homology | | |
| BM734511.V1.3_at | ATCTTCTCAATCTTTCTTTACCGGG | SEQ ID NO:1878 |
| BM734511.V1.3_at | ATGCCTCATCTTAGCAGTGATTTTG | SEQ ID NO:1879 |
| BM734511.V1.3_at | ATCTCTTTCCTCATTTTCCTGATAG | SEQ ID NO:1880 |
| BM734511.V1.3_at | AGGCTTATCTTTTTGTCTAGTGCAA | SEQ ID NO:1881 |
| BM734511.V1.3_at | AAACCTGACTGAGTTTTCCTGTCTA | SEQ ID NO:1882 |
| BM734511.V1.3_at | AAGCTCAGTACACTAATGCCTCATC | SEQ ID NO:1883 |
| BM734511.V1.3_at | AATACGCTTGGCACTGATGGGCACT | SEQ ID NO:1884 |
| BM734511.V1.3_at | CAGACCCCGGATGCTATTTATTCAA | SEQ ID NO:1885 |
| BM734511.V1.3_at | GATGAGGATGGCCTTCCTGTTTCAC | SEQ ID NO:1886 |
| BM734511.V1.3_at | GTTTGAATGAGTCCTTCGTGGGCCA | SEQ ID NO:1887 |
| BM734511.V1.3_at | TTTACCTAAATACTTCCAGCTCCTT | SEQ ID NO:1888 |
| No Homology | | |
| BM734719.V1.3_at | ACGCCAATGGGTCAAACTAACTCTG | SEQ ID NO:1889 |
| BM734719.V1.3_at | AGAAGTCCTCTCTGAGACTCAAGGG | SEQ ID NO:1890 |
| BM734719.V1.3_at | AAAGCCCATGAGCTGCTTCTTTGTT | SEQ ID NO:1891 |
| BM734719.V1.3_at | AAAAATCTCTCATCCTATTCTGCTT | SEQ ID NO:1892 |
| BM734719.V1.3_at | AAGCCTTCCTAAAAGCACACTTGCC | SEQ ID NO:1893 |
| BM734719.V1.3_at | AAGGGCTAAGGCAAGGTCTTCCAGA | SEQ ID NO:1894 |
| BM734719.V1.3_at | CAAGAAATGACAGCCTCCAAGCCTT | SEQ ID NO:1895 |
| BM734719.V1.3_at | GAAGCTTCTTCCCACCTAGAAAGAA | SEQ ID NO:1896 |
| BM734719.V1.3_at | GGTGACAACGCTGGCTGCTGAAAGC | SEQ ID NO:1897 |
| BM734719.V1.3_at | TTTCCACTGTCGTCAGAGCCAACAA | SEQ ID NO:1898 |
| BM734719.V1.3_at | TTCTTTGTTCTCTGTCACGGGACAA | SEQ ID NO:1899 |
| Human mRNA for T3 epsilon chain (20K) of T-cell receptor (from peripheral blood lymphocytes) | | |
| BM781067.V1.3_at | ATCCAGATTATGAGCCCATCCGGAA | SEQ ID NO:1900 |

(continued)

| Probe Set Name | Sequence | Sequence Identifier |
|---|---|---|
| ILT11A mRNA for immunoglobulin-like transcript 11 protein | | |
| BM781067.V1.3_at | AGGTAAATGAGACAGCCCTTCGCTC | SEQ ID NO:1901 |
| BM781067.V1.3_at | AGAGCCGTCTGACAGTTCCTGAGGA | SEQ ID NO:1902 |
| BM781067.V1.3_at | CAGTGGCCACAATCATCGTAGTCGA | SEQ ID NO:1903 |
| BM781067.V1.3_at | CCTTTGGCAACCGTCTGTGGGAATG | SEQ ID NO:1904 |
| BM781067.V1.3_at | GCTAGGCTCCTTGTCAGCTGAGAGA | SEQ ID NO:1905 |
| BM781067.V1.3_at | GAAGACCCATTATCTCTACCTGAAA | SEQ ID NO:1906 |
| BM781067.V1.3_at | GACCTGTACGCTGGCCTGAATCAGA | SEQ ID NO:1907 |
| BM781067.V1.3_at | GTAGGCCCCTGGATGTTCGTTTGCA | SEQ ID NO:1908 |
| BM781067.V1.3_at | GTTCGTTTGCATCTCCATGAATGTG | SEQ ID NO:1909 |
| BM781067.V1.3_at | GGGCTTACTGCTGCTGGTGTATTAC | SEQ ID NO:1910 |
| H. sapiens mRNA for elongation factor 1-beta. | | |
| BM781233.V1.3_at | ATAGATCTCTTTGGGTCTGATGACG | SEQ ID NO:1911 |
| BM781233.V1.3_at | ATGTGCAGTCCATGGATGTGGCCGC | SEQ ID NO:1912 |
| BM781233.V1.3_at | AGAAGGCCAGTCTGCCAGGAGTGAA | SEQ ID NO:1913 |
| BM781233.V1.3_at | AGAACGCCTTGCACAGTATGAGTCA | SEQ ID NO:1914 |
| BM781233.V1.3_at | AAAGCTTTGGGCAAGTATGGTCCTG | SEQ ID NO:1915 |
| BM781233.V1.3_at | AAGAAACCTGCACTTGTTGCCAAGT | SEQ ID NO:1916 |
| BM781233.V1.3_at | GCGGATCACTGCTTTTGAGGACTAT | SEQ ID NO:1917 |
| BM781233.V1.3_at | GGCTCCTCTAAACTAGTTCCAGTGG | SEQ ID NO:1918 |
| BM781233.V1.3_at | GGTCCTGCGGATGTGGAAGACACCA | SEQ ID NO:1919 |
| BM781233.V1.3_at | TGGATGTGGCCGCTTTCAACAAGAT | SEQ ID NO:1920 |
| BM781233.V1.3_at | TGCCAAGTCTTCCATCTTACTAGAT | SEQ ID NO:1921 |
| Homo sapiens EPK2 mRNA for serine/threonine kinase. | | |
| BM781375.V1.3_at | ATCACTGCTCCAAATATCTCCAAAG | SEQ ID NO:1922 |
| BM781375.V1.3_at | AGTCTGTTTATATCAACGCGCAAAA | SEQ ID NO:1923 |
| BM781375.V1.3_at | AGAAGACGTTTCCACAACATCTCTC | SEQ ID NO:1924 |
| BM781375.V1.3_at | AATGATGTTTCTTTGCTATACTTAA | SEQ ID NO:1925 |
| BM781375.V1.3_at | CCAGTGATTCTGCTGTACTGCCCAG | SEQ ID NO:1926 |
| BM781375.V1.3_at | GAAATGTATTTTCCCTGTGATTGAT | SEQ ID NO:1927 |
| BM781375.V1.3_at | GATACTGTATAACTCGCATAACCTT | SEQ ID NO:1928 |
| BM781375.V1.3_at | GTAATTCTGAGTCACTAGGCACCCT | SEQ ID NO:1929 |
| BM781375.V1.3_at | GGCACCCTATTATGGTTTTACTGCA | SEQ ID NO:1930 |
| BM781375.V1.3_at | TGCCCAGCCATAAAGATCTCCAGAA. | SEQ ID NO:1931 |
| BM781375.V1.3_at | TCTCTCTCTACTTCATTCTCATATA | SEQ ID NO:1932 |
| No Homology | | |
| WBC003D11_V1.3_at | ATCTTCATTTCCTCTTAGCTGTCAG | SEQ ID NO:1933 |
| WBC003D11_V1.3_at | AGACAGAGTGTGCATTCCTTCTTGC | SEQ ID NO:1934 |

(continued)

| Probe Set Name | Sequence | Sequence Identifier |
|---|---|---|
| ILT11A mRNA for immunoglobulin-like transcript 11 protein | | |
| WBC003D11_V1.3_at | CATCGGACCAACTTGTAGCTGACTA | SEQ ID NO:1935 |
| WBC003D11_V1.3_at | AATGTGCCCAGGCTGAACTGCTGGA | SEQ ID NO:1936 |
| WBC003D11_V1.3_at | CGATAAGTCTCGCTTGTTCTTGCAC | SEQ ID NO:1937 |
| WBC003D11_V1.3_at | GAGGTTGTCCTTAATCAGCCATCGG | SEQ ID NO:1938 |
| WBC003D11_V1.3_at | GAGGGTGATTCGCATCTTCATTTCC | SEQ ID NO:1939 |
| WBC003D11_V1.3_at | GTCACATCCATGAGCCCAGTCAAGA | SEQ ID NO:1940 |
| wBC003D11_V1.3_at | GTCATTCCATTATACCAGCTGAGGT | SEQ ID NO:1941 |
| WBC003D11_V1.3_at | TATGGCTTGGCCTTGAGACTTGCTT | SEQ ID NO:1942 |
| WBC003D11_V1.3_at | TTCTTGCCCGTTACACGATAAGTCT | SEQ ID NO:1943 |
| Human lymphoid restricted membrane protein (Jaw1) mRNA | | |
| WBC013C09_V1.3_at | AGACTTAGACACAATGGGCCACCAC | SEQ ID NO:1944 |
| WBC013C09_V1.3_at | ACAAGGCACTCTGTTTGTCTGTTGC | SEQ ID NO:1945 |
| WBC013C09_V1.3_at | AATGGGATGTCTCTTCAGCTTATGA | SEQ ID NO:1946 |
| WBC013C09_V1.3_at | CTCACAGGCCTGTTTTTCCAAAAGT | SEQ ID NO:1947 |
| WBC013C09_V1.3_at | GCAGCTTTGATGAGCTTCCTCACAG | SEQ ID NO:1948 |
| WBC013C09_V1.3_at | GAGGAAGCCTGCCTATGATCTTTTA | SEQ ID NO:1949 |
| WBC013C09_V1.3_at | TGACACAATAGCTTCCTGGACAACA | SEQ ID NO:1950 |
| WBC013C09_V1.3_at | GGGACTCCTGGATGTCTCTAGAACA | SEQ ID NO:1951 |
| WBC013C09_V1.3_at | TCTGTTGCCTTCATTGTACTGTTTG | SEQ ID NO:1952 |
| WBC013C09_V1.3_at | TATCTTGTGGCCATTTACCAGACTT | SEQ ID NO:1953 |
| WBC013C09_V1.3_at | TTGTGATTATCTCCCCAACTGAAAT | SEQ ID NO:1954 |
| Homo sapiens pro-oncosis receptor inducing membrane injury gene mRNA | | |
| WBC025B03_V1.3_at | AGTCAGTTTCTTTGGTTCTCTGCAA | SEQ ID NO:1955 |
| WBC025B03_V1.3_at | ACTTTCTTCTATACTTGCTTCTTTA | SEQ ID NO:1956 |
| WBC025B03_V1.3_at | AATTTGCCTTTCAAGTTTCCTGTGC | SEQ ID NO:1957 |
| WBC025B03_V1.3_at | AATTTTGTTTGCAGTCCTTGAGAAT | SEQ ID NO:1958 |
| WBC025B03_V1.3_at | CAAGTTTCCTGTGCTTTGGGTTTGA | SEQ ID NO:1959 |
| WBC025B03_V1.3_at | GACAGGCCTGAAACTTTCTTCTATA | SEQ ID NO:1960 |
| WBC025B03_V1.3_at | GGTGATGCAAACCAATATTCTGCTT | SEQ ID NO:1961 |
| WBC025B03_V1.3_at | GGATCAGCTTTTGGTATTTCATTGT | SEQ ID NO:1962 |
| WBC025B03_V1.3_at | TACCAGTGCTTCAATGCAGTCAGTT | SEQ ID NO:1963 |
| WBC025B03_V1.3_at | TAGTTATGTCTTTTCTCTGTATGTA | SEQ ID NO:1964 |
| WBC025B03_V1.3_at | TTCTGCTTTGGAGCACATTTCCTAA | SEQ ID NO:1965 |
| No Homology | | |
| WBC33.gRSP.V1.3_at | ATGGATATACCACAAGCTGCTTTAT | SEQ ID NO:1966 |
| WBC33.gRSP.V1.3_at | AGCAGTTTGGCTATTCTCAGTCCTT | SEQ ID NO:1967 |
| WBC33.gRSP.V1.3_at | AAAGGTCTCCTGGAATCATGATCAG | SEQ ID NO:1968 |

(continued)

| Probe Set Name | Sequence | Sequence Identifier |
|---|---|---|
| ILT11A mRNA for immunoglobulin-like transcript 11 protein | | |
| WBC33.gRSP.V1.3_at | AATCCATGAACACATCTTCTGTCAG | SEQ ID NO:1969 |
| WBC33.gRSP.V1.3_at | GAGCGTATGCTTTCATTTCTATTGG | SEQ ID NO:1970 |
| WBC33.gRSP.V1.3_at | GATCTTGTACCCTGAAAACTTCCTA | SEQ ID NO:1971 |
| WBC33.gRSP.V1.3_at | GTTGTCTCCTGTTTTGGTTATTACA | SEQ ID NO:1972 |
| WBC33.gRSP.V1.3_at | GTACATCCTGTTAGACATCCTACGT | SEQ ID NO:1973 |
| WBC33.gRSP.V1.3_at | TTTGCCCTCCCAAACGAATTGACTT | SEQ ID NO:1974 |
| WBC33.gRSP.V1.3_at | TGCTTTATCCATTCACCTGTTGACA | SEQ ID NO:1975 |
| WBC33.gRSP.V1.3_at | TTGACAGTCTTTTGGGTTGTCTCCT | SEQ ID NO:1976 |
| Homo sapiens neuronal apoptosis inhibitory protein mRNA | | |
| WBC014B05_V1.3_at | CAGCTAAATTTGTGTTTTCCGGAAT | SEQ ID NO:1977 |
| WBC014B05_V1.3_at | AACATGTGTTGTCTGCTGGTGACTC | SEQ ID NO:1978 |
| WBC014B05_V1.3_at | AACGGCCTTGGAATTCTCCTGTGAA | SEQ ID NO:1979 |
| WBC014B05_V1.3_at | AACCGCTCTGTACCCTAAATGTTAT | SEQ ID NO:1980 |
| WBC014B05_V1.3_at | AAGGCATACTCGCATGTTGCTAGAT | SEQ ID NO:1981 |
| WBC014B05_V1.3_at | GTTAGATAACCACGCGTCTGAGACT | SEQ ID NO:1982 |
| WBC014B05_V1.3_at | GGTGACTCCATGTTGCTGGATTCTA | SEQ ID NO:1983 |
| WBC014B05_V1.3_at | GGATTCTACATCTGCTCGTCCAGAA | SEQ ID NO;1984 |
| WBC014B05_V1.3_at | GGCTAACTCTAGGAAGGCATTCTTT | SEQ ID NO:1985 |
| WBC014B05_V1.3_at | TACTTTCTCCCTTTTTATACTGCTT | SEQ ID NO:1986 |
| WBC014B05_V1.3_at | TTCCAGAGCCTTTTGAGTCAACACT | SEQ ID NO:1987 |
| No Homology | | |
| BM734943.V1.3_at | ATTTCCTGGTAATCTCAATTCCTGC | SEQ ID NO:1988 |
| BM734943.V1.3_at | ATACGATGCCGTATGATCTGGGCCA | SEQ ID NO:1989 |
| BM734943.V1.3_at | AGACCTTTGCTGTGCGTCCTGGAGC | SEQ ID NO:1990 |
| BM734943.V1.3_at | AGCCATGGTCATTTGTGTTACGTAT | SEQ ID NO:1991 |
| BM734943.V1.3_at | AAACTTTCCTGAGCTTGTCTGAGTC | SEQ ID NO:1992 |
| BM734943.V1.3_at | CAAGGAACCCTTTCTGCAAGTCAGT | SEQ ID NO:1993 |
| BM734943.V1.3_at | CTGTGTGTACTATCTTTGCTGTTGT | SEQ ID NO:1994 |
| BM734943.V1.3_at | GAGGTCCCTGGGAGCAGTTGCACAT | SEQ ID NO:1995 |
| BM734943.V1.3_at | GTCTGAGTCTTGAATCGTTACCACG | SEQ ID NO:1996 |
| BM734943.V1.3_at | GGAGGGTGACATTGCTCAAGCCATG | SEQ ID NO:1997 |
| BM734943.V1.3_at | TCAGCATTCCTGTTTCCCTTATATC | SEQ ID NO:1998 |
| H. sapiens initiation factor 4B cDNA | | |
| Foe1075.V1.3_at | GTAAAATCCCATCCTTTCGGTTGTG | SEQ ID NO:1999 |
| Foe1075.V1.3_at | AAAGGTAGGGTTTGCCCTCTTCATG | SEQ ID NO:2000 |
| Foe1075.V1.3_at | ATGGCTTGGATTGGCCTAGTAGCTA | SEQ ID NO:2001 |
| Foe1075.V1.3_at | AATACAGTAGCTTGCCTGCTGGGAT | SEQ ID NO:2002 |

(continued)

| Probe Set Name | Sequence | Sequence Identifier |
|---|---|---|
| ILT11A mRNA for immunoglobulin-like transcript 11 protein | | |
| Foe1075.V1.3_at | GAATGAATCATTTACCCAGTGGACA | SEQ ID NO:2003 |
| Foe1075.V1.3_at | GTCCCACTCCTCAATACGTGTAAAA | SEQ ID NO:2004 |
| Foe1075.V1.3_at | GGAAGTGACTTTTAGAGATGCCCAA | SEQ ID NO:2005 |
| Foe1075.V1.3_at | TCAGTCCTAGGTTAGACCCTGTCCA | SEQ ID NO:2006 |
| Foe1075.V1.3_at | TGACACCTTGGTCTTTTGTTTGCAA | SEQ ID NO:2007 |
| Foe1075.V1.3_at | TCTGGATGTATCCTTGTGGGCACAT | SEQ ID NO:2008 |
| Foe1075.V1.3_at | TTCATGTCTACTCCTTCCAAATAGT | SEQ ID NO:2009 |
| No Homology | | |
| WBC038G11_V1.3_at | ATCTGTGGCGGATGTTTCTTCTCTG | SEQ ID NO:2010 |
| WBC038G11_V1.3_at | AGGAGTTCTCCTGAGCTCAGCCGAG | SEQ ID NO:2011 |
| WBC038G11_V1.3_at | AGCAGTTCTTCCTGAACGGCTTTGA | SEQ ID NO:2012 |
| WBC038G11_V1.3_at | AGACTGACAGCTGACTCCCAGGAGT | SEQ ID NO:2013 |
| WBC038G11_V1.3_at | ACGTGCATGTGTCTTCCAGGAGCAT | SEQ ID NO:2014 |
| WBC03BG11_V1.3_at | AAACAAGCGACATCAGCACCTGGGA | SEQ ID NO:2015 |
| WBC038G11_V1.3_at | AAGGAAGGGCTCTGCCTGAGCAGTT | SEQ ID NO:2016 |
| WBC038G11_V1.3_at | GAACTTGGTCGCATTTGGTCTGAAA | SEQ ID NO:2017 |
| WBC038G11_V1.3_at | GAAGGAGTTCCCTGATCGGCTACAG | SEQ ID NO:2018 |
| WBC038G11_V1.3_at | GTGAACTGCCTGTTGAACTTGGTCG | SEQ ID NO:2019 |
| WBC038G11_V1.3_at | GGAGCATTGCACGTTGCCTGTAGAA | SEQ ID NO:2020 |
| Homo sapiens mRNA; cDNA DKFZp686K06110 (from clone DKFZp686K06110) | | |
| WBC031F04_V1.3_at | ATGTGAAACACCTCTTTGCTGCATT | SEQ ID NO:2021 |
| WBC031F04_V1.3_at | ATGTGATTCTGAAGCCTGGCCAATG | SEQ ID NO:2022 |
| WBC031F04_V1.3_at | ACTGAGGACGGCTTTTCCTGGACAA | SEQ ID NO:2023 |
| WBC031F04_V1.3_at | AAAAGTCTTGTAGGTCACTGCGCCA | SEQ ID NO:2024 |
| WBC031F04_V1.3_at | CTGCATTCTTTTTCTTCGCTTACAA | SEQ ID NO:2025 |
| WBC031F04_V1.3_at | GTCTTTAATCATTGGTTCGGCTGCT | SEQ ID NO:2026 |
| WBC031F04_V1.3_at | GGTCTACTTTTTAATGGCTTTCATA | SEQ ID NO:2027 |
| WBC031F04_V1.3_at | TGGAAACGGTTTCACTTGCCCTTTG | SEQ ID NO:2028 |
| WBC031F04_V1.3_at | TGCGCCATCTCAGCCATAGTAGTTG | SEQ ID NO:2029 |
| WBC031F04_V1.3_at | TTCGGCTGCTTTTTGTACTTTGTTT | SEQ ID NO:2030 |
| WBC031F04-V1.3_at | TTGCAGTTTGCTTCCATGTGATTCT | SEQ ID NO:2031 |
| Homo sapiens triggering receptor expressed on myeloid cells 1, mRNA | | |
| BM734862.V1.3_at | ATCTACATCCATCTGGCAGTTGTGC | SEQ ID NO:2032 |
| BM734862.V1.3_at | ATGAGGATGACCTCTGATCTCCATC | SEQ ID NO:2033 |
| BM734862.V1.3_at | AGCATTGTCATTCCTGTGGCGTGCG | SEQ ID NO:2034 |
| BM734862.V1.3_at | ACAAAGGTTATTTCTGAGGCTCAGG | SEQ ID NO:2035 |
| BM734862.V1.3_at | CTCGTGACTAAGAGCCTGGTCCTTA | SEQ ID NO:2036 |

(continued)

| Probe Set Name | Sequence | Sequence Identifier |
|---|---|---|
| ILT11A mRNA for immunoglobulin-like transcript 11 protein | | |
| BM734862.V1.3_at | CCCTCATTTCACTGATGACCGTGGG | SEQ ID NO:2037 |
| BM734862.V1.3_at | GAAGTCATTTGGATCCTAGGCCCAT | SEQ ID NO:2038 |
| BM734862.V1.3_at | TGGCGTGCGCACTCGTGACTAAGAG | SEQ ID NO:2039 |
| BM734862.V1.3_at | GGCAGCGACATGAGTTGGATCCGTT | SEQ ID NO:2040 |
| BM734862.V1.3_at | TAAAAGAGCAGACACGGCCCCAAAC | SEQ ID NO:2041 |
| BM734862.V1.3_at | TTACTGTCCTGTTTGCTGTCACACA | SEQ ID NO:2042 |
| No Homology | | |
| BM735402.V1.3_at | ATAACCCCACAGCTTGAGTACTTAC | SEQ ID NO:2043 |
| BM735402.V1.3_at | ATTTGCTGAATAACCAGTCCTCTCC | SEQ ID NO:2044 |
| BM735402.V1.3_at | AATTCCGTTTTCACAGAAGGGCTCA | SEQ ID NO:2045 |
| BM735402.V1.3_at | AGAATCTTTTCTCCATGCATGTAAA | SEQ ID NO:2046 |
| BM735402.V1.3_at | GAGTACTTACTGCTTGGCGGGTCAC | SEQ ID NO:2047 |
| BM735402.V1.3_at | GTCCTCTCCTTTTCTGTGAAACTAA | SEQ ID NO:2048 |
| BM735402.V1.3_at | GGGCTCAATTTGTTCCTTATCGTCT | SEQ ID NO:2049 |
| BM735402.V1.3_at | TGGTACTCACCCATGGAGATGCTTA | SEQ ID NO:2050 |
| BM735402.V1.3_at | TCCCACCCAGTTGAGTTATTTGCTT | SEQ ID NO:2051 |
| BM735402.V1.3_at | TTCTCACTTACTTTCCCTTTAGGAG | SEQ ID NO:2052 |
| BM735402.V1.3_at | TTATCGTCTCCCCATTGAAGTGTCG | SEQ ID NO:2053 |
| Homo sapiens v-ets erythroblastosis virus E26 oncogene homolog 2 (avian) (ETS2), mRNA. | | |
| WBC025E03_V1.3_at | AGACGATTTCCAGTATCTCAGCACA | SEQ ID NO:2054 |
| WBC025E03_V1.3_at | CAGTGTATTCCTGGCCTTTATACGC | SEQ ID NO:2055 |
| WBC025E03_V1.3_at | AAGTCAGACACGCTTCACTAGCAGT | SEQ ID NO:2056 |
| WBC025E03_V1.3_at | CATGCGACTGGGAACATCTTGTTGC | SEQ ID NO:2057 |
| WBC025E03_V1.3_at | CGGTGGCTGCCATCTGTACAGAAGA | SEQ ID NO:2058 |
| WBC025E03_V1.3_at | GCTCGAGCCACATGAAATTGCCAAT | SEQ ID NO:2059 |
| WBC025E03_V1.3_at | GACAATTTGTCTCAATACCCACACG | SEQ ID NO:2060 |
| WBC025E03_V1.3_at | GTTTATCTCTCGGACATCTGTTTAT | SEQ ID NO:2061 |
| WBC025E03_V1.3_at | GGATGTTTTTCTCAAGCAGGCCCTG | SEQ ID NO:2062 |
| WBC025E03_V1.3_at | TGGCAGTTTTGTGTGGTCCAACCTA | SEQ ID NO:2063 |
| WBC025E03_V1.3_at | TTGGGCCTCTTCTTTCTCAAATAAT | SEQ ID NO:2064 |
| No homology | | |
| B1961438.V1.3_at | ATCCAGGGTGGAGTCTATGCAGAAA | SEQ ID NO:2065 |
| B1961438.V1.3_at | CAGAAAAGGGCCTCACCTGCCAGGG | SEQ ID NO:2066 |
| B1961438.V1.3_at | AAGGAGCCTTCTTGAGGTCACTGCA | SEQ ID NO:2067 |
| B1961438.V1.3_at | GAAATGACCCATAATCCAGCAACGC | SEQ ID NO:2068 |
| B1961438.V1.3_at | GACATGAGCGTTTGTGTTTGCTTTA | SEQ ID NO:2069 |
| B1961438.V1.3_at | GAGGTCACTGCATTTGATCTTCATG | SEQ ID NO:2070 |

(continued)

| Probe Set Name | Sequence | Sequence Identifier |
|---|---|---|
| ILT11A mRNA for immunoglobulin-like transcript 11 protein | | |
| B1961438.V1.3_at | GTGGGCAGAGACTTGGCATCATTGT | SEQ ID NO:2071 |
| B1961438.V1.3_at | GGCATCATTGTCATCCAGCAAATAA | SEQ ID NO:2072 |
| B1961438.V1.3_at | TCAGTCTCCCCTAGTCCAGAAAAGG | SEQ ID NO:2073 |
| B1961438.V1.3_at | TGCGCATTGCTTTCCAAGGAGCCTT | SEQ ID NO:2074 |
| B1961438.V1.3_at | TTTGATCTTCATGGCGGCACCTGGA | SEQ ID NO:2075 |
| No homology | | |
| BM780914.V1.3_at | GGGATGCCCAATCCCGGGACCTACA | SEQ ID NO:2076 |
| BM780914.V1.3_at | AAGAACTTTGTGTGCGACCCCGAGG | SEQ ID NO:2077 |
| BM780914.V1.3_at | GAGGTACAGGACTTTGAAACGTTCT | SEQ ID NO:2078 |
| BM780914.V1.3_at | ATAAGAGCCCGTGGGTACTTCACCG | SEQ ID NO:2079 |
| BM780914.V1.3_at | CTACACCGTAAGGATAAGAGCCCGT | SEQ ID NO:2080 |
| BM780914.V1.3_at | TTGAAACGTTCTACGGGATGCCCAA | SEQ ID NO:2081 |
| BM780914.V1.3_at | GGTACTTCACCGGAATCGTGAGCGA | SEQ ID NO:2082 |
| BM780914.V1.3_at | TGATGCGGAAGATCTTCCCGCCCAT | SEQ ID NO:2083 |
| BM780914.V1.3_at | TGCAGAAAGTACTCGCTGATGCGGA | SEQ ID NO:2084 |
| BM780914.V1.3_at | TGAGTGACAGCCTGCAGAACAAGCT | SEQ ID NO:2085 |
| BM780914.V1.3_at | TCCTCGTCCTGATCTGCAGAAAGTA | SEQ ID NO:2086 |
| Equus caballus matrix metalloproteinase 1 precursor (MMP1) | | |
| WBC030E04_V1.3_at | ATAATTTACTTCCACCAATGAGGGT | SEQ ID NO:2087 |
| WBC030E04_V1.3_at | ATTGATACTGGAGAGCAGCCTCAGA | SEQ ID NO:2088 |
| WBC030E04_V1.3_at | ATGACTTTCCTGGAATTGGCGACAA | SEQ ID NO:2089 |
| WBC030E04_V1.3_at | AAGCCAATAGCTGGTTCAACTGCAG | SEQ ID NO:2090 |
| WBC030E04_V1.3_at | GAATTTTGACTCTCCAGAAAGCCAA | SEQ ID NO:2091 |
| WBC030E04_V1.3_at | GAATGCAATGTTTCGTGTACTGCTA | SEQ ID NO:2092 |
| WBC030E04_V1.3_at | GAAGATGTTTTCCTGAAGAGCCATA | SEQ ID NO:2093 |
| WBC030E04_V1.3_at | GAGATACGAGTCTTCACCAGAGGAA | SEQ ID NO:2094 |
| WBC030E04_V1.3_at | GAGCTGCTACTAGTATTATCCTAAT | SEQ ID NO:2095 |
| WBC030E04_V1.3_at | GATTTACGGATTCATATGGGCCAAT | SEQ ID NO:2096 |
| WBC030E04_V1.3_at | TGGATTTTTCTATTTCTTTCACGGA | SEQ ID NO:2097 |
| No homology | | |
| BM734654.V1.3_at | ACCTAGACGACCTCTCGGGATTGAC | SEQ ID NO:2098 |
| BM734654.V1.3_at | CGATTCCGTTATGCGGTCCAAGCAA | SEQ ID NO:2099 |
| BM734654.V1.3_at | TGAACGGGACCAGCCAAACGACGGG | SEQ ID NO:2100 |
| BM734654.V1.3_at | AGAACTTCTCATGCTTCATCTACAT | SEQ ID NO:2101 |
| BM734654.V1.3_at | GACCGTGATGGTCAACACCAGCCAA | SEQ ID NO:2102 |
| BM734654.V1.3_at | GACGACGTCCAGTATTTCCTGTATA | SEQ ID NO:2103 |
| BM734654.V1.3_at | GGGATCCAGTTCTTCGATTCCGTTA | SEQ ID NO:2104 |

(continued)

| Probe Set Name | Sequence | Sequence Identifier |
|---|---|---|
| ILT11A mRNA for immunoglobulin-like transcript 11 protein | | |
| BM734654.V1.3_at | GGAATGTCCCCGTTACATGAGCGAC | SEQ ID NO:2105 |
| BM734654.V1.3_at | TCTACATCGTGCACTTCATGATCTG | SEQ ID NO:2106 |
| BM734654.V1.3_at | TTTCGGAGAAGCTCGTCTACACCAA | SEQ ID NO:2107 |
| BM734654.V1.3_at | TTGACCTCTTACAATTACTTCGTGG | SEQ ID NO:2108 |
| No Homology | | |
| BM734722.V1.3_at | CGGGCGACTCGCAGAATCAATACAT | SEQ ID NO:2109 |
| BM734722.V1.3_at | ACAGAGCCCCGGTCAGCGGGTGAAA | SEQ ID NO:2110 |
| BM734722.V1.3_at | AACTGAACGATAACCATCCGACCGA | SEQ ID NO:2111 |
| BM734722.V1.3_at | AATCAATACATTTTCCCGAGTCTGG | SEQ ID NO:2112 |
| BM734722.V1.3_at | TCGGCTGCCTGGTGAAGAGGTTCCT | SEQ ID NO:2113 |
| BM734722.V1.3_at | GCGTTTCTGCAGCTATTTTTCTACT | SEQ ID NO:2114 |
| BM734722.V1.3_at | GGTCCCGCGCATCAAAGACAAACTG | SEQ ID NO:2115 |
| BM734722.V1.3_at | GCGACTTCCAGTACGAGCTGGTCAT | SEQ ID NO:2116 |
| BM734722.V1.3_at | GAAGAGGTTCCTCCGGAGACACAGT | SEQ ID NO:2117 |
| BM734722.V1.3_at | GGAGACACAGTCTGTTCCAGCCGGT | SEQ ID NO:2118 |
| BM734722.V1.3_at | TGAAGTTTGGCCAAGAGGCTTCCCG | SEQ ID NO:2119 |
| Homo sapiens SUI1 isolog | | |
| B1961499.V1.3_at | AGGCCACCCTTCTAGCAGAGTTAAA | SEQ ID NO:2120 |
| B1961499.V1.3_at | AGAACTTGCGGCTTGACTAGCAGCA | SEQ ID NO:2121 |
| B1961499.V1.3_at | AAGAGTGATGCCCTGGTGTCTCCAG | SEQ ID NO:2122 |
| B1961499.V1.3_at | CAGCTGCCCAATGCCATGTGAAGTA | SEQ ID NO:2123 |
| B1961499.V1.3_at | GAGAGAATGTCACCACCTGAGCAGC | SEQ ID NO:2124 |
| B1961499.V1.3_at | GAGTTCCCTGCCCTGAGAGAATGTC | SEQ ID NO:2125 |
| B1961499.V1.3_at | GTCTAGTCTTGAAGTCCCTCATTTA | SEQ ID NO:2126 |
| B1961499.V1.3_at | GGTGATAGGACATCTGGCTTGCCAG | SEQ ID NO:2127 |
| B1961499.V1.3_at | GGCTTGCCAGCGAGGTCTTTTTGAC | SEQ ID NO:2128 |
| B1961499.V1.3_at | TAATGTGATGTTTCGGCCAAGCCCA | SEQ ID NO:2129 |
| B1961499.V1.3_at | TATGTGTCAGTCACGTAGCCAGCTG | SEQ ID NO:2130 |
| Homo sapiens guanine nucleotide binding protein (G protein), beta polypeptide 2-like 1 (GNB2L1), mRNA. | | |
| WBC048H02_V1.3_at | ATCGGCACCCGCTAGAAATACATGG | SEQ ID NO:2131 |
| WBC048H02_V1.3_at | ATCATTGTCTCCTGTGGCTGGGACA | SEQ ID NO:2132 |
| WBC048H02_V1.3_at | AGGCTACCTGAACACTGTCACTGTC | SEQ ID NO:2133 |
| WBC048H02_V1.3_at | AGACTCTGTTTGCTGGCTACACGGA | SEQ ID NO:2134 |
| WBC048H02_V1.3_at | AGAAGTTATCAGTACCAGCAGCAAG | SEQ ID NO:2135 |
| WBC048H02_V1.3_at | AGCTGAAGACCAATCACATCGGCCA | SEQ ID NO:2136 |
| WBC048H02_V1.3_at | GATCCCTCTGTGCTTCTGGAGGCAA | SEQ ID NO:2137 |

(continued)

| Probe Set Name | Sequence | Sequence Identifier |
|---|---|---|
| ILT11A mRNA for immunoglobulin-like transcript 11 protein | | |
| WBC04BH02_V1.3_at | GGCCAGGCCATGCTTTGGGATTTAA | SEQ ID NO:2138 |
| WBC048H02_V1.3_at | GGGACATCATCAACGCCTTGTGCTT | SEQ ID NO:2139 |
| WBC048H02_V1.3_at | GGCAAGCACCTTTACACACTAGATG | SEQ ID NO:2140 |
| WBC048H02_V1.3_at | TACACTGTCCAGGATGAGAGCCACT | SEQ ID NO:2141 |
| Pinin desmosome associated protein (PNN) | | |
| WBC012E07_V1.3_at | AATCAGATCTTTGCAGCTTTGAGGG | SEQ ID NO:2142 |
| WBC012E07_V1.3_at | AATTTGGTTGGCATTTTCTTCATGA | SEQ ID NO:2143 |
| WBC012E07_V1.3_at | CTTTTGTGTTGGCTCTAACTCTGAA | SEQ ID NO:2144 |
| WBC012E07_V1.3_at | CCTCCTCATCTTTAAACCACGTATT | SEQ ID NO:2145 |
| WBC012E07_V1.3_at | GAGCATCATTCTTTTGTCTCCATGG | SEQ ID NO:2146 |
| WBC012E07_V1.3_at | GTTTCCTCACTTTTATTTGCCTTAG | SEQ ID NO:2147 |
| WBC012E07_V1.3_at | GTCTCCATGGTTACTTGTGTGATAC | SEQ ID NO:2148 |
| WBC012E07_V1.3_at | GTGGTCTTGTCTTAACTTTTGTGTT | SEQ ID NO:2149 |
| WBC012E07_V1.3_at | GGAGTATCTGTTGCCCATTACTATA | SEQ ID NO:2150 |
| WBC012E07_V1.3_at | TACTGTTTTTTCTAATCTCCCTTGT | SEQ ID NO:2151 |
| WBC012E07_V1.3_at | TTTAATCTAAGCATTTTCCCCTCCT | SEQ ID NO:2152 |
| Homo sapiens T-cell receptor beta (TCRB) mRNA | | |
| BM735494.V1.3_at | GGGACACCCGATATAATTCGCCGCT | SEQ ID NO:2153 |
| BM735494.V1.3_at | AGATGATGAAAACCCCAGCTACTGT | SEQ ID NO:2154 |
| BM735494.V1.3_at | AGCTACTGTCTGAGCAGCCGGCTGA | SEQ ID NO:2155 |
| BM735494.V1.3_at | AACAGCTTCAAATCGTTTCTCACCT | SEQ ID NO:2156 |
| BM735494.V1.3_at | AGCCCTACAGGGAGCAGTCAGATGA | SEQ ID NO:2157 |
| BM735494.V1.3_at | ACAACGCTCATTTAAACCTTCACAT | SEQ ID NO:2158 |
| BM735494.V1.3_at | GCACCAGGCTCACTGTCACAGAGAA | SEQ ID NO:2159 |
| BM735494.V1.3_at | GACTCTGCTGTGTATTTCTGTGCCA | SEQ ID NO:2160 |
| BM735494.V1.3_at | GAGACCTCATCTCACGGGTGGGAAA | SEQ ID NO:2161 |
| BM735494.V1.3_at | GTGACCCTACCCAAGGTGGCTGTGT | SEQ ID NO:2162 |
| BM735494.V1.3_at | GTGGCTGTGTTTGAACCTTCGGAAG | SEQ ID NO:2163 |
| No Homology | | |
| Foe1288.V1.3_at | ATCAAGTCCCTTTCAGTGTTCTGAA | SEQ ID NO:2164 |
| Foe1288.V1.3_at | ATCACCTTTGGAAGCCAGCGGCAAT | SEQ ID NO:2165 |
| Foe1288.V1.3_at | AGAGGATGTAACACCACCTTGCCAT | SEQ ID NO:2166 |
| Foe1288.V1.3_at | AGTCTCTGTCATTCATTTCTGTCGA | SEQ ID NO:2167 |
| Foe1288.V1.3_at | AGCGGCAATGCCATTCTGCTACTAT | SEQ ID NO:2168 |
| Foe1288.V1.3_at | ACTTCCCACAGATTTGTTATCCTCA | SEQ ID NO:2169 |
| Foe1288.V1.3_at | AAGTTTGCCTTGTATCTCCTAGTGC | SEQ ID NO:2170 |
| Foe1288.V1.3_at | AAGACTGCCGTGTGCCCGAGGTATG | SEQ ID NO:2171 |

(continued)

| Probe Set Name | Sequence | Sequence Identifier |
|---|---|---|
| ILT11A mRNA for immunoglobulin-like transcript 11 protein | | |
| Foe1288.V1.3_at | GAGACTGTGATTTGGTTCTTCCTTC | SEQ ID NO:2172 |
| Foe1288.V1.3_at | GAGTAACCATTTCCTTTGTGGCCAA | SEQ ID NO:2173 |
| Foe1288.V1.3_at | TGTGGCCAATCTGTGTATGCTTTTA | SEQ ID NO:2174 |
| Homo sapiens non-POU domain containing octamer-binding, mRNA | | |
| WBC014G03_V1.3_at | ATATATTACCCCTTCATGTCCTAAG | SEQ ID NO:2175 |
| WBC014G03_V1.3_at | GATTTGTCCCTGATACCTTTGGAGT | SEQ ID NO:2176 |
| WBC014G03_V1.3_at | AATCTTGTGTTAACTTGCCTCCATC | SEQ ID NO:2177 |
| WBC014G03_V1.3_at | CATCCTGTTTTGTTTGCTTTCTCAT | SEQ ID NO:2178 |
| WBC014G03_V1.3_at | GCCTCCATCTTTTTCTTGGGTGAAG | SEQ ID NO:2179 |
| WBC014G03_V1.3_at | GCTGTTCACAGCTTTTCTTGGTAGG | SEQ ID NO:2180 |
| WBC014G03_V1.3_at | GAATGGCCCTTTTGTGTCTATGATG | SEQ ID NO:2181 |
| WBC014G03_V1.3_at | GTGCATACGGTTCTGTTATATCCCA | SEQ ID NO:2182 |
| WBC014G03_V1.3_at | GGTGTACCATGTTTGATTTGTCCCT | SEQ ID NO:2183 |
| WBC014G03_V1.3_at | GGACAGTAACTCTTAGCCTTGCCTA | SEQ ID NO:2184 |
| WBC014G03_V1.3_at | TAATCATTCCATTCTTTGTCCATCC | SEQ ID NO:2185 |
| H sapiens mRNA for P1 protein (P1. h) | | |
| WBC043B10_V1.3_at | GAGCCAAGAAGGTTTCTCCCGTGTT | SEQ ID NO:2186 |
| WBC043B10_V1.3_at | AAGAGACTGGATCACACTGCCTGCT | SEQ ID NO:2187 |
| WBC043B10_V1.3_at | AAGTCTTTGCCTTTATTCCCTGAAT | SEQ ID NO:2188 |
| WBC043B10_V1.3_at | CTTTGGATTGTTTTATGCTGCTGGA | SEQ ID NO:2189 |
| WBC043B10_V1.3_at | CTCCCGAGCACTTTGGTCCAGAATA | SEQ ID NO:2190 |
| WBC043B10_V1.3_at | GACTAATGCATACTTTTGACCTGAA | SEQ ID NO:2191 |
| WBC043B10_V1.3_at | GTGTTCGTTGGCTGTTTTCTTCCTC | SEQ ID NO:2192 |
| WBC043B10_V1.3_at | GGGCACCTTGGTTTCTGTTTATCCT | SEQ ID NO:2193 |
| WBC043B10_V1.3_at | GGACCAAGACTATTGCTTCTTCATT | SEQ ID NO:2194 |
| WBC043B10_V1.3_at | TGGGAGACATGTGATGACGCCGATC | SEQ ID NO:2195 |
| WBC043B10_V1.3_at | TTCTGTTTATCCTTTCTGTGTTCGT | SEQ ID NO:2196 |
| Human mRNA for calcium-binding protein in macrophages (MRP-14) macrophage migration inhibitory factor (MIF) -related protein. | | |
| B1961539.V1.3_at | ATATCATGGAGGACCTGGATACCAA | SEQ ID NO:2197 |
| B1961539.V1.3_at | ATCATCAATGTCTTCCACCAGTACT | SEQ ID NO:2198 |
| B1961539.V1.3_at | ACGGCCACAGCCACTAATCTGGAGG | SEQ ID NO:2199 |
| B1961539.V1.3_at | CAAGCAGCTGAGTTTCGAGGAGTTC | SEQ ID NO:2200 |
| B1961539.V1.3_at | GAGCTGGCGAACTTCCTCAAGAGTA | SEQ ID NO:2201 |
| B1961539.V1.3_at | GATGCACGAGCATGACCAAGGCCAC | SEQ ID NO:2202 |
| B1961539.V1.3_at | GAGGAGTTCATCATCCTGGTGGCCA | SEQ ID NO:2203 |
| B1961539.V1.3_at | GGAACTGTCCCAGATGGAGCGCGAC | SEQ ID NO:2204 |

(continued)

| Probe Set Name | Sequence | Sequence Identifier |
|---|---|---|
| ILT11A mRNA for immunoglobulin-like transcript 11 protein | | |
| B1961539.V1.3_at | GGAGAGTGGCCATGGTCACAGCCAT | SEQ ID NO:2205 |
| B1961539.V1.3_at | GGAGCGCGACATAGAGACCATCATC | SEQ ID NO:2206 |
| B1961539.V1.3_at | TGACGCATGCCTCCCATGAGAAGAT | SEQ ID NO:2207 |
| Homo sapiens actin related protein 2/3 complex subunit 1B (41 kD) (ARPC1B) mRNA. | | |
| B1961827.V1.3_at | AGAACAGCGTCAGCCAGATCTCGGT | SEQ ID NO:2208 |
| B1961827.V1.3_at | ACGGTCGCTTTGCTGAATGTTTCTA | SEQ ID NO:2209 |
| B1961827.V1.3_at | GCAGTTCTGCACTACGGGCATGGAC | SEQ ID NO:2210 |
| B1961827.V1.3_at | GAGCTTGGAATCAGCCTTGAAGGAC | SEQ ID NO:2211 |
| B1961827.V1.3_at | GAGCGCTTCCAGAACCTCGACAGGA | SEQ ID NO:2212 |
| B1961827.V1.3_at | GGGAAAGGCCAAGTGCTCGCAGTTC | SEQ ID NO:2213 |
| B1961827.V1.3_at | GTGGGAAGCTTTTCTTACCTGTTGA | SEQ ID NO:2214 |
| B1961827.V1.3_at | GGTCTGGACTCGCTGCACAAGAACA | SEQ ID NO:2215 |
| B1961827.V1.3_at | GGACGTGCCCAAGCAGAACTCGCAG | SEQ ID NO:2216 |
| B1961827.V1.3_at | TGAAGGAACACGTGCCTTTTTCTTA | SEQ ID NO:2217 |
| B1961827.V1.3_at | TGACCTGCCAGGATTATGTTGCCCT | SEQ ID NO:2218 |
| Fibroblast mRNA for aldolase A | | |
| B1961941.V1.3_at | AGGGCTTTAGGCTGTTCTTTCCCAT | SEQ ID NO:2219 |
| B1961941.V1.3_at | AGGAGGAGGCATCCATCAACCTCAA | SEQ ID NO:2220 |
| B1961941.V1.3_at | AGTGGAGGTATTCTAAGGCTGCCCC | SEQ ID NO:2221 |
| B1961941.V1.3_at | AAATACACCCCAAGTGGTCACGCTG | SEQ ID NO:2222 |
| B1961941.V1.3_at | TGAAGCCCAATATGGTAACCCCAGG | SEQ ID NO:2223 |
| B1961941.V1.3_at | GATTGCCATGGCAACTGTCACGGCA | SEQ ID NO:2224 |
| B1961941.V1.3_at | GTCTGTGGTATTGTCTGTGTATGCT | SEQ ID NO:2225 |
| B1961941.V1.3_at | GGAATATGTCAAGCGAGCCCTGGCC | SEQ ID NO:2226 |
| B1961941.V1.3_at | TGGGATCACCTTCCTATCTGGAGGC | SEQ ID NO:2227 |
| B1961941.V1.3_at | TTGCCTCCCTGGTGACATTGGTCTG | SEQ ID NO:2228 |
| B1961941.V1.3_at | TTCATCTCTAACCATGCCTACTAAG | SEQ ID NO:2229 |
| Human hemopoietic cell proteintyrosine kinase(HCK) gene | | |
| BM734694.V1.3_at | ATCATGACCCGCTGCTGGAAGAACA | SEQ ID NO:2230 |
| BM734694.V1.3_at | ATCAAGTCGGATGTCTGGTCCTTTG | SEQ ID NO:2231 |
| BM734694.V1.3_at | AGAGTTGGATTCCACTCACAGCTGT | SEQ ID NO:2232 |
| BM734694.V1.3_at | AAGTTGACATTTCTATGGCCTGGGA | SEQ ID NO:2233 |
| BM734694.V1.3_at | CAGAGTGTGCTGGAGGACTTCTACA | SEQ ID NO:2234 |
| BM734694.V1.3_at | GATACAGGATGCCTCGACCAGAGCA | SEQ ID NO:2235 |
| BM734694.V1.3_at | GAGCCAGTACCAACAGCAGCCGTGA | SEQ ID NO:2236 |
| BM734694.V1.3_at | GATTGTCACCTATGGCAGGATCCCT | SEQ ID NO:2237 |
| BM734694.V1.3_at | GGACAATCTCTTTTTGACTCTAGCA | SEQ ID NO:2238 |

(continued)

| Probe Set Name | Sequence | Sequence Identifier |
|---|---|---|
| ILT11A mRNA for immunoglobulin-like transcript 11 protein | | |
| BM734694.V1.3_at | TGCCCCGAGGAGCTTTACAACATCA | SEQ ID NO:2239 |
| BM734694.V1.3_at | TCCTTTGGTGTCCTGCTGATGGAGA | SEQ ID NO:2240 |
| No Homology | | |
| BM735487.V1.3_at | GAGTTCGAAGTCACCCTAATCACGT | SEQ ID NO:2241 |
| BM735487.V1.3_at | CACCAGGACTCTAGCCCCAGAGTGG | SEQ ID NO:2242 |
| BM735487.V1.3_at | AAATGGAACTCTCCTTTTCGAGCAT | SEQ ID NO:2243 |
| BM735487.V1.3_at | CGGCCCGGACACAAGGAGGAAGCTC | SEQ ID NO:2244 |
| BM735487.V1.3_at | GCGTCTGACCCCAGCGAAGGGCCAG | SEQ ID NO:2245 |
| BM735487.V1.3_at | GAAGGGCCAGCCTTGCTTGGTTCAG | SEQ ID NO:2246 |
| BM735487.V1.3_at | GACTGTGGAGGGACAGGCTTCCCCT | SEQ ID NO:2247 |
| BM735487.V1.3_at | GTTAGCTTCCCCAAAAGAATTGTGT | SEQ ID NO:2248 |
| BM735487.V1.3_at | GTCACCCTAATCACGTAAATGGAAC | SEQ ID NO:2249 |
| BM735487.V1.3_at | GTGCTTGCTTGGGAGTTCGAAGTCA | SEQ ID NO:2250 |
| BM735487.V1.3_at | TAGCCCCAGAGTGGCTCCAGAAGGT | SEQ ID NO:2251 |
| Homo sapiens ring finger protein 10, mRNA (cDNA clone MGC:35311 IMAGE: 5177665) | | |
| BM735519.V1.3_at | CAGGCTACCTTCTCCATTTGGTTTT | SEQ ID NO:2252 |
| BM735519.V1.3_at | AGATGTGTGGCCCAAAACTGCTCCA | SEQ ID NO:2253 |
| BM735519.V1.3_at | AGCCTTCATGAAGCTGGACACGCCA | SEQ ID NO:2254 |
| BM735519.V1.3_at | AGAAGCTCCTGTTCAGCACCTCAGT | SEQ ID NO:2255 |
| BM735519.V1.3_at | AATACAGTGTATTTTCCAGCTTCCT | SEQ ID NO:2256 |
| BM735519.V1.3_at | CAAGTGACACTACTGGCCCAGGCTA | SEQ ID NO:2257 |
| BM735519.V1.3_at | CCGTGCTTTTGTTTTGCTGCTGTAA | SEQ ID NO:2258 |
| BM735519.V1.3_at | TGAAAACAGCTTAATTCCTCCTGCC | SEQ ID NO:2259 |
| BM735519.V1.3_at | TGATAACTCGGACCGTGTTCCTGTG | SEQ ID NO:2260 |
| BM735519.V1.3_at | TTCCAGAATTCCTTCAGCCAAGCTA | SEQ ID NO:2261 |
| BM735519.V1.3_at | TTCGGATCCCCTCTCTGAAGAGAAA | SEQ ID NO:2262 |
| Human Fc-epsilon-receptor gamma-chain mRNA | | |
| BM735585.V1.3_at | ATGATTCCAGCAGTGGTCTTGCTCT | SEQ ID NO:2263 |
| BM735585.V1.3_at | ACCCTACCCCTGTAATGATGCTATG | SEQ ID NO:2264 |
| BM735585.V1.3_at | CTGCCATTAATGCTAGCTGACCCTA | SEQ ID NO:2265 |
| BM735585.V1.3_at | GTACCCGGACCCAGGAGACTTATGA | SEQ ID NO:2266 |
| BM735585.V1.3_at | GAGAGCCTCAGCTTTGCTATATTCT | SEQ ID NO:2267 |
| BM735585.V1.3_at | GATGCCATCCTGTTCTTGTATGGTA | SEQ ID NO:2268 |
| BM735585.V1.3_at | TGTTTATATTCTAGTCTCACCCCTA | SEQ ID NO:2269 |
| BM735585.V1.3_at | TTTCAAACAGATGCCCTTGGTCACA | SEQ ID NO:2270 |
| BM735585.V1.3_at | TAACGGACATCAGTGGTTCCTCTTC | SEQ ID NO:2271 |

(continued)

| Probe Set Name | Sequence | Sequence Identifier |
|---|---|---|
| ILT11A mRNA for immunoglobulin-like transcript 11 protein | | |
| BM735585.V1.3_at | TTGGTTGAACAAGCAGCGGCCCTGG | SEQ ID NO:2272 |
| BM735585.V1.3_at | TTGCTCTTACTCCTTTTGGTTGAAC | SEQ ID NO:2273 |
| Homo sapiens lysosomal-associated membrane protein 1 (LAMP1) mRNA | | |
| WBC038E02_V1.3_at | AGATTCTGGTGTTTGGTCTCTTCAC | SEQ ID NO:2274 |
| WBC038E02_V1.3_at | ACTGGCTCCATATCATTTGGCTTTC | SEQ ID NO:2275 |
| WBC038E02_V1.3_at | CTGCGTCTGCACTTGTTTGTGTACA | SEQ ID NO:2276 |
| WBC038E02_V1.3_at | CCATGCAAGAGCTGGCACTTTTTTA | SEQ ID NO:2277 |
| WBC038E02_V1.3_at | GACCCTCCTGTGTGCAATGCCGTAA | SEQ ID NO:2278 |
| WBC038E02_V1.3_at | GTCTCTTCACTCTTGATTCAGATCT | SEQ ID NO:2279 |
| WBC038E02_V1.3_at | GGGAGAGTCTCTGATCTTAACACTT | SEQ ID NO:2280 |
| WBC038E02_V1.3_at | TGTTTGTGTACAGTGATCGCTCTCA | SEQ ID NO:2281 |
| WBC038E02_V1.3_at | TGATTTACTGCTTGCAGCTCAGCGG | SEQ ID NO:2282 |
| WBC038E02_V1.3_at | TGATCGCTCTCACTCTGATTTACTG | SEQ ID NO:2283 |
| WBC038E02_V1.3_at | TTTTCGGTCTTCTGTTTGAGGTTCA | SEQ ID NO:2284 |
| Homo sapiens EGF-like-domain, multiple 5 (EGFL5), mRNA. | | |
| WBC012B04_V1.3_at | ATACGTTAAATTTCAGCCCAAGCAA | SEQ ID NO:2285 |
| WBC012B04_V1.3_at | ATCTTTCTTACTTGTTCCTTATGCG | SEQ ID NO:2286 |
| WBC012B04_V1.3_at | ATGCAAGTTTTCTCTTTAACCCTGA | SEQ ID NO:2287 |
| WBC012B04_V1.3_at | AGGCTTCCTGATGCATTTCTAAATT | SEQ ID NO:2288 |
| WBC012B04_V1.3_at | ACATATGCTGTACTAGGCTTCCTGA | SEQ ID NO:2289 |
| WBC012B04_V1.3_at | AAAACCGTGTCTCATACATATGCTG | SEQ ID NO:2290 |
| WBC012B04_V1.3_at | GAACCTTTTGGTCTGTTATGATCTT | SEQ ID NO:2291 |
| WBC012B04_V1.3_at | GTAACTGTGACAGCAGCCCTGGAAT | SEQ ID NO:2292 |
| WBC012B04_V1.3_at | GTCAGCCAGGGCTTCAGATCTCAGG | SEQ ID NO:2293 |
| WBC012B04_V1.3_at | GGCACAAGCACTCTGTAATACGTTA | SEQ ID NO:2294 |
| WBC012B04_V1.3_at | TTAACCCTGAGCCAGTGACTATGCC | SEQ ID NO:2295 |
| Human mRNA for T200 leukocyte common antigen (CD45, LC-A). | | |
| WBC027E07_V1.3_at | ATAGGGTGGTGGACCTTATGGCCCA | SEQ ID NO:2296 |
| WBC027E07_V1.3_at | ATATTGAGAGTCTCCTGACCTCCAC | SEQ ID NO:2297 |
| WBC027E07_V1.3_at | AGAAAGGCCCTCAGCTGCTGGGAAT | SEQ ID NO:2298 |
| WBC027E07_V1.3_at | ACTTTGTCAAGCTCATTTCCTGGTA | SEQ ID NO:2299 |
| WBC027E07_V1.3_at | GCTGGGAATGCTTGTCCCAACTTGA | SEQ ID NO:2300 |
| WBC027E07_V1.3_at | GAGACTAGTTCTCTCGTGACACCCA | SEQ ID NO:2301 |
| WBC027E07_V1.3_at | GGCCCACATGGCCTCCAAGGAGTAA | SEQ ID NO:2302 |
| WBC027E07_V1.3_at | GGAGCCCTACCTTGTCATGTACCAT | SEQ ID NO:2303 |
| WBC027E07_V1.3_at | GGACCACCAATCACCCAGCAAGAGA | SEQ ID NO:2304 |
| WBC027E07_V1.3_at | TGACCTCCACAGTTTCCATCTCAGA | SEQ ID NO:2305 |

(continued)

| Probe Set Name | Sequence | Sequence Identifier |
|---|---|---|
| ILT11A mRNA for immunoglobulin-like transcript 11 protein | | |
| WBC027E07_V1.3_at | TTGCCCTCAACGACCACTTTGTCAA | SEQ ID NO:2306 |
| No Homology | | |
| WBC030C04_V1.3_at | ATGCGCACATTTACTAGCACCTACT | SEQ ID NO:2307 |
| WBC030C04_V1.3_at | ATGGACTTCCCTGCATTGTATGAGA | SEQ ID NO:2308 |
| WBC030C04_V1.3_at | ATGTGCTGTGTGCTCTGTGTACCCT | SEQ ID NO:2309 |
| WBC030C04_V1.3_at | AGTTGTCTGGCCCAAAGCATCAGCA | SEQ ID NO:2310 |
| WBC030C04_V1.3_at | GAGTTCGATTCCCATCTGAAACGAC | SEQ ID NO:2311 |
| WBC030C04_V1.3_at | GAGACATTTTCCGTTGTCACAACTT | SEQ ID NO:2312 |
| WBC030C04_V1.3_at | GAGAGGTCACCGATGCTGCTAAACA | SEQ ID NO:2313 |
| WBC030C04_V1.3_at | GTGTGGCCTTAGTGACCAATCAGTC | SEQ ID NO:2314 |
| WBC030C04_V1.3_at | TGTGTACCCTCAGATCACGTGAACT | SEQ ID NO:2315 |
| WBC030C04_V1.3_at | TACTGAGGGCTTACCATGTGCTGTG | SEQ ID NO:2316 |
| WBC030C04_V1.3_at | TTCACCCCTCGGAACATCGATGATG | SEQ ID NO:2317 |

**TABLE 3**

| AMINO ACID SUB-CLASSIFICATION | |
|---|---|
| **Sub-classes** | **Amino acids** |
| Acidic | Aspartic acid, Glutamic acid |
| Basic | Noncyclic: Arginine, Lysine; Cyclic: Histidine |
| Charged | Aspartic acid, Glutamic acid, Arginine, Lysine, Histidine |
| Small | Glycine, Serine, Alanine, Threonine, Proline |
| Polar/neutral | Asparagine, Histidine, Glutamine, Cysteine, Serine, Threonine |
| Polar/large | Asparagine, Glutamine |
| Hydrophobic | Tyrosine, Valine, Isoleucine, Leucine, Methionine, Phenylalanine, Tryptophan |
| Aromatic | Tryptophan, Tyrosine, Phenylalanine |
| Residues that influence chain orientation | Glycine and Proline |

**TABLE 4**

| EXEMPLARY AND PREFERRED AMINO ACID SUBSTITUTIONS | | |
|---|---|---|
| **Original Residue** | **Exemplary Substitutions** | **Preferred Substitutions** |
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn, His, Lys, | Asn |
| Glu | Asp, Lys | Asp |
| Gly | Pro | Pro |
| His | Asn, Gln, Lys, Arg | Arg |
| Ile | Leu, Val, Met, Ala, Phe, Norleu | Leu |

(continued)

| EXEMPLARY AND PREFERRED AMINO ACID SUBSTITUTIONS | | |
|---|---|---|
| **Original Residue** | **Exemplary Substitutions** | **Preferred Substitutions** |
| Leu | Norleu, Ile, Val, Met, Ala, Phe | Ile |
| Lys | Arg, Gln, Asn | Arg |
| Met | Leu, Ile, Phe | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |
| Val | Ile, Leu, Met, Phe, Ala, Norleu | Leu |

**TABLE 5**
**GENE PRIORITY ORDER BASED ON T VALUE**

| Gene Name | M (24 hours) | T(24 hours) | Holm P (24hours) | M(72 hours) | T(72 hours) | FDR P(72 hours) |
|---|---|---|---|---|---|---|
| WBC033A01_V1.3_at | -2.662894 | -9.962659 | 0.000017 | -1.276358 | -4.955679 | 0.029004 |
| WBC009F04_V1.3_at | -2.022275 | -9.836701 | 0.000021 | -0.815793 | -4.117344 | 0.035681 |
| WBC024B05_V1.3_at | -1.664652 | -8.335464 | 0.000280 | -0.880469 | -4.570119 | 0.029004 |
| WBC039D09_V1.3_at | -1.872731 | -8.327496 | 0.000284 | -1.004384 | -4.632601 | 0.029004 |
| WBC018F03_V1.3_at | -2.361400 | -8.148680 | 0.000394 | | | |
| WBC004E04_V1.3_at | -2.162850 | -8.144995 | 0.000396 | -1.175666 | -4.591078 | 0.029004 |
| WBC012A03_V1.3_at | -1.338388 | -7.701847 | 0.000904 | -0.758502 | -4.523937 | 0.029004 |
| WBC001F11_V1.3_at | -1.823749 | -7.635242 | 0.001024 | -1.166865 | -5.061581 | 0.026896 |
| WBC33.gRSP.V1.3_at | -2.213668 | -7.568092 | 0.001163 | -1.291169 | -4.576337 | 0.029004 |
| BM734943.V1.3_at | -2.502148 | -7.546875 | 0.001211 | -1.453158 | -4.544783 | 0.029004 |
| WBC025B03_V1.3_at | -2.621996 | -7.517703 | 0.001281 | -1.320582 | -3.922683 | 0.047310 |
| WBC005D09_V1.3_at | -1.776450 | -7.461019 | 0.001428 | -0.961345 | -4.187062 | 0.033199 |
| WBC018D03_V1.3_at | -1.288566 | -7.385061 | 0.001652 | -0.811415 | -4.818958 | 0.029004 |
| WBC047A01_V1.3_at | -1.489422 | -7.333705 | 0.001822 | -0.869908 | -4.438289 | 0.029708 |
| BM781416.V1.3_at | -0.890777 | -7.202082 | 0.002353 | -0.636407 | -5.333266 | 0.024866 |
| WBC310.V1.3_at | -2.057025 | -7.191047 | 0.002403 | -1.148505 | -4.165566 | 0.033380 |
| WBC038A02_V1.3_at | -1.237604 | -7.149217 | 0.002608 | | | |
| Foe1324.V1.3_at | -0.967173 | -7.065375 | 0.003076 | -0.591431 | -4.481723 | 0.029391 |
| WBC014G03_V1.3_at | -0.822802 | -7.018150 | 0.003375 | | | |
| WBC031C11_V1.3_at | -1.229890 | -7.004847 | 0.003464 | | | |
| Foe1317.V1.3_at | -1.393445 | -7.004002 | 0.003469 | | | |
| WBC025C03_V1.3_at | -1.123663 | -7.000843 | 0.003490 | | | |
| WBC005F01_V1.3_at | -1.793647 | -6.973124 | 0.003685 | | | |
| BM781033.V1.3_at | -1.600021 | -6.959662 | 0.003784 | | | |
| WBC037C09_V1.3_at | -2.315585 | -6.944690 | 0.003897 | | | |
| WBC013A11_V1.3_at | -1.518652 | -6.906258 | 0.004204 | | | |
| BM781067.V1.3_at | -2.131608 | -6.868123 | 0.004537 | | | |
| BM734719.V1.3_at | -1.725505 | -6.864147 | 0.004572 | | | |
| WBC007A08_V1.3_at | -0.791783 | -6.841457 | 0.004782 | | | |
| WBC030C11_V1.3_at | -1.786902 | -6.811774 | 0.005075 | | | |
| BM781233.V1.3_at | -1.079172 | -6.802419 | 0.005170 | | | |
| BM780999.V1.3_at | -1.147959 | -6.682644 | 0.006574 | | | |

(continued)

**GENE PRIORITY ORDER BASED ON T VALUE**

| Gene Name | M (24 hours) | T(24 hours) | Holm P (24hours) | M(72 hours) | T(72 hours) | FDR P(72 hours) |
|---|---|---|---|---|---|---|
| WBC011F05_V1.3_at | -1.014702 | -6.644981 | 0.007086 | | | |
| Foe1263.V1.3_at | -1.368441 | -6.628304 | 0.007327 | -0.841507 | -4.224840 | 0.033199 |
| WBC001F10_V1.3_at | -1.108760 | -6.592549 | 0.007874 | | | |
| B1961856.V1.3_at | -2.437119 | -6.490502 | 0.009699 | | | |
| WBC014E06_V1.3_at | -1.576883 | -6.480146 | 0.009902 | | | |
| WBC022D03_V1.3_at | -2.226427 | -6.447584 | 0.010583 | -1.403789 | -4.212853 | 0.033199 |
| WBC022B03_V1.3_at | -2.495001 | -6.389631 | 0.011919 | | | |
| Foe148.V1.3_at | -1.210133 | -6.383976 | 0.012056 | | | |
| BM734975.V1.3_at | -0.956313 | -6.374726 | 0.012281 | | | |
| WBC309.V1.3_at | -1.320389 | -6.357556 | 0.012718 | | | |
| WBC964.gRSP.V1.3_at | -2.148632 | -6.308260 | 0.014086 | -1.418636 | -4.318625 | 0.033199 |
| Foe1216.V1.3_at | -0.610111 | -6.295728 | 0.014454 | | | |
| B1961438.V1.3_at | -1.874821 | -6.260291 | 0.015554 | | | |
| BM781103.V1.3_at | -1.590271 | -6.252160 | 0.015815 | | | |
| Foe1288.V1.3_at | -1.086282 | -6.246331 | 0.016004 | | | |
| BM734780.V1.3_at | -1.686861 | -6.126850 | 0.020531 | -1.177444 | -4.430605 | 0.029708 |
| BM781375.V1.3_at | -1.401878 | -6.104504 | 0.021514 | -0.885759 | -3.996806 | 0.043572 |
| WBC016G11_V1.3_at | -1.524048 | -6.096714 | 0.021863 | -0.964780 | -3.998437 | 0.043572 |
| WBC013E05_V1.3_at | -1.235319 | -6.073394 | 0.022944 | | | |
| WBC035E01_V1.3_at | -1.009758 | -6.067899 | 0.023204 | -0.657412 | -4.093653 | 0.036940 |
| Foe443.V1.3_at | -1.810221 | -6.050102 | 0.024086 | | | |
| WBC013A09_V1.3_at | -1.197534 | -6.028120 | 0.025226 | -0.798145 | -4.162797 | 0.033380 |
| WBC009H07_V1.3_at | -1.169165 | -6.027141 | 0.025270 | | | |
| WBC016A12_V1.3_at | -1.818692 | -5.989753 | 0.027347 | | | |
| WBC027H11_V1.3_at | -1.218607 | -5.989121 | 0.027374 | | | |
| WBC004G01_V1.3_at | -1.133747 | -5.983072 | 0.027719 | | | |
| BM735402.V1.3_at | -1.194590 | -5.964250 | 0.028841 | | | |
| BM735494.V1.3_at | -1.750520 | -5.958587 | 0.029181 | | | |
| WBC374.V1.3_at | -1.644790 | -5.957986 | 0.029208 | | | |
| WBC335.V1.3_at | -1.119690 | -5.956705 | 0.029278 | | | |
| WBC031 B06_V1.3_at | -1.371020 | -5.939056 | 0.030389 | -1.002910 | -4.502046 | 0.029145 |
| WBC019B05_V1.3_at | -2.812821 | -5.935107 | 0.030625 | | | |
| BM734661.V1.3_at | -1.009540 | -5.929543 | 0.030980 | | | |
| WBC028G09_V1.3_at | -1.536631 | -5.888696 | 0.033768 | | | |
| WBC032H02_V1.3_at | -1.665465 | -5.848697 | 0.036747 | -1.094870 | -3.983557 | 0.043957 |
| WBC038B01_V1.3_at | -1.238551 | -5.775724 | 0.042904 | | | |
| WBC004G09_V1.3_at | -2.481126 | -5.766468 | 0.043737 | | | |
| WBC020F05_V1.3_at | -1.228488 | -5.741651 | 0.046091 | | | |
| WBC022C04_V1.3_at | -1.051865 | -5.738198 | 0.046419 | | | |
| WBC107.V1.3_s_at | -1.968199 | -5.730559 | 0.047158 | | | |
| Foe1075.V1.3_at | -0.962785 | -5.729726 | 0.047226 | | | |
| WBC048H02_V1.3_at | -0.939329 | -5.728407 | 0.047345 | | | |
| WBC018B05_V1.3_at | -0.925714 | -5.703704 | 0.049903 | | | |
| BM734452.V1.3_at | 0.805204 | 5.725055 | 0.047672 | | | |
| WBC039F12_V1.3_at | 1.150275 | 5.734195 | 0.046805 | | | |
| WBC012C10_V1.3_at | 1.627067 | 5.750482 | 0.045237 | | | |
| WBC013C09_V1.3_at | 0.805300 | 5.766335 | 0.043737 | | | |
| B1961941.V1.3_at | 0.808670 | 5.772622 | 0.043176 | | | |

(continued)

**GENE PRIORITY ORDER BASED ON T VALUE**

| Gene Name | M (24 hours) | T(24 hours) | Holm P (24hours) | M(72 hours) | T(72 hours) | FDR P(72 hours) |
|---|---|---|---|---|---|---|
| BM735286.V1.3_at | 0.677464 | 5.796605 | 0.041035 | 0.447623 | 3.969701 | 0.043957 |
| BM734632.V1.3_at | 1.546635 | 5.799185 | 0.040822 | | | |
| BM734654.V1.3_at | 1.896641 | 5.824011 | 0.038717 | | | |
| WBC008A12_V1.3_at | 1.454425 | 5.843937 | 0.037111 | 0.953908 | 3.974301 | 0.043957 |
| WBC327.V1.3_at | 3.134368 | 5.849951 | 0.036661 | | | |
| B1961872.V1.3_at | 0.695445 | 5.879069 | 0.034458 | | | |
| WBC033012_V1.3_at | 1.564444 | 5.889206 | 0.033742 | | | |
| WBC031 D02_V1.3_at | 1.280462 | 5.903074 | 0.032769 | | | |
| BM735386.V1.3_at | 0.843738 | 5.936267 | 0.030560 | | | |
| WBC014H06_V1.3_at | 2.344460 | 6.078689 | 0.022696 | | | |
| WBC024G08_V1.3_at | 0.990922 | 6.081274 | 0.022580 | | | |
| BM781237.V1.3_s_at | 1.208673 | 6.145017 | 0.019767 | | | |
| WBC007F03_V1.3_at | 0.678768 | 6.158498 | 0.019221 | 0.555707 | 5.226559 | 0.024866 |
| WBC032D03_V1.3_at | 1.385989 | 6.179118 | 0.018412 | | | |
| WBC030E04_V1.3_at | 4.696429 | 6.196116 | 0.017773 | | | |
| WBC024F08_V1.3_at | 1.057724 | 6.288312 | 0.014675 | | | |
| WBC028C09_V1.3_at | 0.725926 | 6.362683 | 0.012587 | | | |
| WBC011 D03_V1.3_at | 1.853309 | 6.382535 | 0.012088 | | | |
| B1961620.V1.3_at | 1.747623 | 6.437617 | 0.010796 | | | |
| WBC012B04_V1.3_at | 2.475044 | 6.447033 | 0.010591 | | | |
| WBC020H01_V1.3_at | 1.314322 | 6.459585 | 0.010327 | | | |
| BM780906.V1.3_at | 1.288912 | 6.480826 | 0.009891 | | | |
| WBC012E05_V1.3_at | 2.297892 | 6.506542 | 0.009388 | | | |
| WBC028E11_V1.3_at | 2.406940 | 6.523695 | 0.009065 | | | |
| WBC031G11_V1.3_at | 1.225760 | 6.622921 | 0.007403 | | | |
| WBC024H07_V1.3_at | 0.976787 | 6.623779 | 0.007392 | | | |
| BM734844.V1.3_at | 0.933292 | 6.629950 | 0.007305 | | | |
| B1961539.V1.3_at | 4.852518 | 6.661050 | 0.006860 | | | |
| WBC025A06_V1.3_at | 1.823785 | 6.667810 | 0.006769 | | | |
| WBC020H11_V1.3_at | 0.986342 | 6.678247 | 0.006629 | 0.632519 | 4.440903 | 0.029708 |
| BM781394.V1.3_at | 1.762617 | 6.681449 | 0.006588 | | | |
| WBC001 E1 0_V1.3_at | 1.449566 | 6.698739 | 0.006365 | | | |
| WBC039G08_V1.3_at | 0.952917 | 6.714431 | 0.006167 | | | |
| BM734531.V1.3_at | 2.023074 | 6.721602 | 0.006080 | | | |
| WBC038D08_V1.3_at | 0.973751 | 6.723009 | 0.006065 | 0.739545 | 5.296198 | 0.024866 |
| WBC040E12_V1.3_at | 2.867191 | 6.757972 | | 0.005652 | | |
| B1961932.V1.3_s_at | 1.234657 | 6.798806 | 0.005206 | | | |
| WBC008D09_V1.3_at | 2.132599 | 6.847202 | 0.004729 | | | |
| WBC036E08_V1.3_at | 0.903370 | 6.917203 | 0.004114 | | | |
| BM735585.V1.3_at | 1.170140 | 6.927900 | 0.004028 | | | |
| WBC027E07_V1.3_at | 0.856915 | 6.949906 | 0.003858 | | | |
| B1961880.V1.3_at | 2.362559 | 6.989422 | 0.003569 | | | |
| WBC21.V1.3_at | 3.858556 | 7.040639 | 0.003228 | | | |
| BM734862.V1.3_at | 1.814033 | 7.048185 | 0.003181 | | | |
| B1961932.V1.3_at | 1.458519 | 7.135736 | 0.002677 | | | |
| WBC038G11_V1.3_at | 0.970456 | 7.150263 | 0.002603 | | | |
| BM735519.V1.3_at | 1.321178 | 7.203189 | 0.002348 | | | |
| WBC014B05_V1.3_at | 3.009994 | 7.237630 | 0.002196 | | | |

(continued)

**GENE PRIORITY ORDER BASED ON T VALUE**

| Gene Name | M (24 hours) | T(24 hours) | Holm P (24hours) | M(72 hours) | T(72 hours) | FDR P(72 hours) |
|---|---|---|---|---|---|---|
| WBC001D02_V1.3_at | 1.758208 | 7.274337 | 0.002044 | | | |
| WBC027F05_V1.3_at | 4.660849 | 7.282229 | 0.002013 | | | |
| BM734694.V1.3_at | 1.920448 | 7.314034 | 0.001893 | | | |
| BM734933.V1.3_at | 2.051080 | 7.334686 | 0.001820 | 1.052354 | 3.899225 | 0.048632 |
| GI9717252-3M_at | 2.344887 | 7.340340 | 0.001800 | 1.236716 | 4.010774 | 0.043572 |
| WBC044E01_V1.3_at | 1.317571 | 7.360577 | 0.001731 | 0.953823 | 5.524562 | 0.024866 |
| WBC027B01_V1.3_at | 0.925355 | 7.371553 | 0.001695 | | | |
| WBC003D11_V1.3_at | 4.056714 | 7.407465 | 0.001582 | | | |
| WBC021E07_V1.3_at | 0.996203 | 7.449580 | 0.001459 | | | |
| BM781186.V1.3_at | 2.960940 | 7.599579 | 0.001096 | | | |
| BM781269.V1.3_at | 5.802158 | 7.601829 | 0.001091 | | | |
| WBC019C11_V1.3_at | 1.276000 | 7.664242 | 0.000970 | | | |
| WBC025E03_V1.3_at | 2.105169 | 7.668296 | 0.000963 | | | |
| WBC008A10_V1.3_at | 5.312376 | 7.675685 | 0.000949 | | | |
| BM734900.V1.3_at | 2.168771 | 7.714757 | 0.000882 | | | |
| BM735452.V1.3_at | 1.455575 | 7.894775 | 0.000629 | | | |
| WBC005F10_V1.3_at | 1.513331 | 8.094985 | 0.000434 | | | |
| WBC028D04_V1.3_at | 2.350644 | 8.115434 | 0.000418 | | | |
| WBC031F04_V1.3_at | 2.689395 | 8.137599 | 0.000401 | 1.325124 | 4.155226 | 0.033380 |
| BM781278.V1.3_at | 0.846445 | 8.145422 | 0.000396 | | | |
| GI9717252-3_at | 2.268058 | 8.389991 | 0.000254 | 1.189076 | 4.557171 | 0.029004 |
| BM735091.V1.3_at | 2.927326 | 8.424147 | 0.000239 | | | |
| BM735057.V1.3_s_at | 0.903257 | 8.439540 | 0.000232 | | | |
| WBC015B08_V1.3_at | 1.759706 | 8.580595 | 0.000180 | | | |
| WBC030C04_V1.3_at | 2.989697 | 8.747470 | 0.000134 | 1.418361 | 4.302077 | 0.033199 |
| BM734722.V1.3_at | 1.873417 | 8.955619 | 0.000093 | 0.956808 | 4.742436 | 0.029004 |
| BM780914.V1.3_at | 2.022282 | 9.423418 | 0.000042 | | | |
| BM735487.V1.3_at | 0.940146 | 9.472548 | 0.000039 | | | |
| BM734891.V1.3_at | 1.763656 | 9.766923 | 0.000024 | 0.678177 | 3.896254 | 0.048632 |
| WBC024G03_V1.3_at | 2.694822 | 9.905797 | 0.000019 | | | |
| WBC117.gRSP.V1.3_at | 2.194570 | 10.473732 | 0.000008 | | | |
| WBC017B09_V1.3_at | | | | -0.819031 | -5.281446 | 0.024866 |
| WBC005E06_V1.3_at | | | | -0.554386 | -5.100535 | 0.026896 |
| B1961499.V1.3_at | | | | -0.407910 | -4.924593 | 0.029004 |
| WBC311.V1.3_at | | | | -0.607729 | -4.873480 | 0.029004 |
| BM734795.V1.3_at | | | | -1.446996 | -4.750582 | 0.029004 |
| BM781409.V1.3_at | | | | -0.780927 | -4.746039 | 0.029004 |
| BM781438.V1.3_at | | | | -1.044275 | -4.523519 | 0.029004 |
| BM781379.V1.3_at | | | | -0.556041 | -4.410744 | 0.030078 |
| BM781319.V1.3_at | | | | -1.102450 | -4.335677 | 0.033199 |
| WBC023F12_V1.3_at | | | | -1.739066 | -4.277504 | 0.033199 |
| WBC021 E02_V1.3_at | | | | -0.771972 | -4.269989 | 0.033199 |
| WBC012E07_V1.3_at | | | | -0.881844 | -4.248284 | 0.033199 |
| WBC043B10_V1.3_at | | | | -0.800840 | -4.233410 | 0.033199 |
| WBC215.gRSP.V1.3_at | | | | -1.002742 | -4.199851 | 0.033199 |
| WBC013E10_V1.3_at | | | | -1.090284 | -4.184730 | 0.033199 |
| Foe146.V1.3_at | | | | -0.733985 | -3.945641 | 0.045654 |
| WBC017C08_V1.3_at | | | | 0.889661 | 4.203909 | 0.033199 |

(continued)

**GENE PRIORITY ORDER BASED ON T VALUE**

| Gene Name | M (24 hours) | T(24 hours) | Holm P (24hours) | M(72 hours) | T(72 hours) | FDR P(72 hours) |
|---|---|---|---|---|---|---|
| BM734511.V1.3_at | | | | 1.107206 | 4.241555 | 0.033199 |
| Foe1092.V1.3_at | | | | 0.762973 | 4.291741 | 0.033199 |
| B1961827.V1.3_at | | | | 0.712699 | 4.382743 | 0.031054 |
| WBC038E02_V1.3_at | | | | 0.422022 | 4.521474 | 0.029004 |
| B1961900.V1.3_at | | | | 0.559448 | 4.667569 | 0.029004 |
| WBC027D09_V1.3_at | | | | 0.664437 | 5.274541 | 0.024866 |

**TABLE 6**

| TWO GENES SELECTED | | | | |
|---|---|---|---|---|
| Genes | | Sensitivity | specificity | Success |
| Foe1216 | BM734531 | 1 | 1 | 1 |
| BM734933 | WBC012A03 | 1 | 1 | 1 |
| Foe1263 | BM734531 | 1 | 1 | 1 |
| BM735452 | WBC003D11 | 1 | 1 | 1 |
| WBC008A10 | WBC020H11 | 1 | 1 | 1 |
| BM781409 | WBC019C11 | 1 | 1 | 1 |
| WBC013E10 | BM734844 | 1 | 1 | 1 |
| WBC038A02 | WBC032H02 | 1 | 1 | 1 |
| BM734943 | WBC005F10 | 1 | 1 | 1 |
| WBC030E04 | B1961438 | 1 | 1 | 1 |
| BM781375 | WBC031C11 | 1 | 1 | 1 |
| WBC310 | WBC309 | 1 | 1 | 1 |
| WBC036E08 | B1961932 | 1 | 1 | 1 |
| WBC001F10 | BM734722 | 1 | 1 | 1 |
| BM734900 | WBC21 | 1 | 1 | 1 |
| BM735452 | WBC038E02 | 1 | 1 | 1 |
| WBC024B05 | WBC047A01 | 1 | 1 | 1 |
| WBC024H07 | BM734780 | 1 | 1 | 1 |
| B1961856 | BM734862 | 1 | 1 | 1 |
| BM734661 | WBC030E04 | 1 | 1 | 1 |

**TABLE 7**

| THREE GENES SELECTED | | | | | |
|---|---|---|---|---|---|
| Genes | | | Sensitivity | Specificity | Success |
| BM781409 | BM734632 | BM781033 | 1 | 1 | 1 |
| WBC018B05 | WBC009F04 | WBC012E07 | 1 | 1 | 1 |
| WBC021E07 | WBC013A09 | Foe1324 | 1 | 1 | 1 |

(continued)

| THREE GENES SELECTED | | | | | |
|---|---|---|---|---|---|
| Genes | | | Sensitivity | Specificity | Success |
| WBC012E05 | BM735286 | WBC008A12 | 1 | 1 | 1 |
| WBC008A10 | WBC038G11 | WBC038B01 | 1 | 1 | 1 |
| Foe1263 | WBC028D04 | WBC012A03 | 1 | 1 | 1 |
| WBC018B05 | WBC009F04 | WBC024H07 | 1 | 1 | 1 |
| WBC008D09 | WBC028D04 | WBC039F12 | 1 | 1 | 1 |
| B1961499 | BM735091 | WBC015B08 | 1 | 1 | 1 |
| Foe1288 | WBC038B01 | WBC031C11 | 1 | 1 | 1 |
| WBC035E01 | BM781319 | G19717252 | 1 | 1 | 1 |
| WBC024F08 | WBC013A09 | WBC031F04 | 1 | 1 | 1 |
| BM781269 | WBC018D03 | B1961620 | 1 | 1 | 1 |
| BM781278 | B1961932 | WBC014B05 | 1 | 1 | 1 |
| WBC031F04 | Foe1324 | B1961941 | 1 | 1 | 1 |
| BM734654 | WBC335 | B1961880 | 1 | 1 | 1 |
| WBC009H07 | BM734933 | BM734661 | 1 | 1 | 1 |
| B1961941 | WBC025E03 | WBC327 | 1 | 1 | 1 |
| WBC018D03 | WBC014H06 | WBC011F05 | 1 | 1 | 1 |
| BM734694 | WBC005F01 | WBC011D03 | 1 | 1 | 1 |

**TABLE 8**

| FOUR GENES SELECTED | | | | | | |
|---|---|---|---|---|---|---|
| Genes | | | | Sensitivity | Specificity | Success |
| BM780914 | WBC030E04 | WBC031C11 | WBC005E06 | 1 | 1 | 1 |
| WBC021E02 | B1961438 | BM734531 | WBC310 | 1 | 1 | 1 |
| WBC374 | Foe1263 | BM734452 | BM781067 | 1 | 1 | 1 |
| WBC030C11 | B1961941 | WBC009H07 | WBC005F01 | 1 | 1 | 1 |
| WBC038G11 | WBC019B05 | WBC027F05 | WBC024B05 | 1 | 1 | 1 |
| BM781278 | WBC001D02 | WBC033D12 | WBC031D02 | 1 | 1 | 1 |
| WBC043B10 | WBC030C11 | WBC007F03 | WBC024H07 | 1 | 1 | 1 |
| BM781067 | WBC005F01 | WBC048H02 | BM781237 | 1 | 1 | 1 |
| BM734511 | WBC020H11 | WBC039F12 | WBC019B05 | 1 | 1 | 1 |
| WBC964 | B1961932 | WBC031D02 | WBC037C09 | 1 | 1 | 1 |
| WBC013E05 | WBC012C10 | WBC035E01 | BM781186 | 1 | 1 | 1 |
| WBC001E10 | BM781269 | BM734943 | BM735519 | 1 | 1 | 1 |
| B1961900 | WBC022C04 | BM735286 | WBC327 | 1 | 1 | 1 |
| WBC013C09 | WBC028E11 | GI9717252 | WBC019B05 | 1 | 1 | 1 |
| BM781409 | WBC024B05 | WBC014H06 | WBC036E08 | 1 | 1 | 1 |
| WBC21 | WBC018F03 | BM781379 | WBC037C09 | 1 | 1 | 1 |

(continued)

| FOUR GENES SELECTED | | | | | | |
|---|---|---|---|---|---|---|
| Genes | | | | Sensitivity | Specificity | Success |
| BM734531 | WBC374 | BM735386 | WBC030C11 | 1 | 1 | 1 |
| BM734632 | B1961827 | WBC013C09 | B1961900 | 1 | 1 | 1 |
| WBC031B06 | BM781103 | WBC039G08 | BM781186 | 1 | 1 | 1 |
| WBC014G03 | WBC018F03 | BM734654 | BM781033 | 1 | 1 | 1 |

**TABLE 9**

| FIVE GENES SELECTED | | | | | | | |
|---|---|---|---|---|---|---|---|
| Genes | | | | | Sensitivity | specificity | success |
| WBC007A08 | WBC012E05 | WBC018B05 | BM781394 | WBC040E12 | 1 | 1 | 1 |
| WBC038E02 | WBC025C03 | WBC013E05 | WBC004E04 | WBC005D09 | 1 | 1 | 1 |
| BM781379 | WBC038D08 | B1961499 | BM781033 | WBC022D03 | 1 | 1 | 1 |
| WBC005D09 | Foe1216 | WBC311 | B1961872 | BM781379 | 1 | 1 | 1 |
| WBC021E07 | WBC311 | WBC027H11 | B1961932 | BM734933 | 1 | 1 | 1 |
| B1961827 | WBC028C09 | BM735487 | WBC117 | BM735057 | 1 | 1 | 1 |
| B1961438 | WBC030C11 | WBC012E07 | WBC001D02 | BM735286 | 1 | 1 | 1 |
| WBC21 | WBC039D09 | WBC014E06 | WBC012E05 | W8C009H07 | 1 | 1 | 1 |
| WBC004G09 | WBC311 | WBC020F05 | WBC024G03 | WBC215 | 1 | 1 | 1 |
| B1961438 | WBC013A11 | BM781278 | BM734452 | Foe443 | 1 | 1 | 1 |
| WBC309 | WBC039F12 | WBC001E10 | WBC027H11 | BM780999 | 1 | 1 | 1 |
| B1961539 | BM734933 | BM781067 | BM781269 | WBC038E02 | 1 | 1 | 1 |
| BM734900 | B1961932 | WBC031G11 | BM734780 | BM734975 | 1 | 1 | 1 |
| Foe443 | WBC032D03 | WBC030E04 | WBC022D03 | WBC001D02 | 1 | 1 | 1 |
| Foe1263 | WBC022B03 | WBC040E12 | WBC013E05 | WBC009H07 | 1 | 1 | 1 |
| Foe1288 | BM734844 | BM781033 | WBC001F10 | B1961932 | 1 | 1 | 1 |
| WBC964 | WBC005E06 | WBC013C09 | BM781033 | WBC013E05 | 1 | 1 | 1 |
| WBC030C11 | WBC031G11 | WBC009F04 | WBC964 | BM734719 | 1 | 1 | 1 |
| WBC004E04 | BM734943 | WBC012B04 | WBC031B06 | WBC001F11 | 1 | 1 | 1 |
| Foe146 | Foe1317 | WBC008D09 | WBC020H11 | WBC003D11 | 1 | 1 | 1 |

## TABLE 10

| SIX GENES SELECTED | | | | | | | | |
|---|---|---|---|---|---|---|---|---|
| Genes | | | | | | Sensitivity | Specificity | Success |
| WBC035E01 | Foe1075 | WBC028C09 | WBC013A09 | WBC004E04 | WBC014E06 | 1 | 1 | 1 |
| WBC040E12 | WBC005F10 | BM735286 | BM735452 | WBC032H02 | BM781319 | 1 | 1 | 1 |
| Foe1317 | WBC024B05 | WBC027E07 | BM781375 | B1961539 | WBC028G09 | 1 | 1 | 1 |
| WBC001F10 | WBC040E12 | WBC022B03 | WBC031C11 | B1961499 | BM734780 | 1 | 1 | 1 |
| WBC017C08 | BM734719 | BM734891 | GI9717252.3M | Foe1317 | WBC031G11 | 1 | 1 | 1 |
| WBC044E01 | BM781409 | WBC043B10 | BM735452 | BM781379 | BM781033 | 1 | 1 | 1 |
| WBC048H02 | BM735091 | WBC016A12 | WBC012E05 | WBC031F04 | WBC012B04 | 1 | 1 | 1 |
| WBC019B05 | BM780999 | BM734722 | WBC023F12 | WBC008A12 | WBC012E05 | 1 | 1 | 1 |
| WBC031F04 | WBC038B01 | B1961539 | BM735386 | WBC024F08 | BM781278 | 1 | 1 | 1 |
| BM734632 | WBC020H01 | WBC012B04 | BM735452 | WBC009H07 | WBC018B05 | 1 | 1 | 1 |
| WBC038E02 | WBC044E01 | WBC027H11 | WBC038G11 | BM781409 | WBC031F04 | 1 | 1 | 1 |
| WBC005D09 | BM735091 | BM734722 | BM781394 | WBC011F05 | WBC374 | 1 | 1 | 1 |
| WBC018B05 | WBC018F03 | WBC032D03 | B1961499 | WBC027F05 | WBC117 | 1 | 1 | 1 |
| Foe1324 | B1961438 | BM734632 | WBC024F08 | WBC019C11 | WBC024G08 | 1 | 1 | 1 |
| WBC004G09 | WBC335 | Foe1317 | WBC038E02 | B1961932 | Foe1075 | 1 | 1 | 1 |
| WBC007A08 | WBC036E08 | Foe146 | BM781269 | B1961900 | WBC964 | 1 | 1 | 1 |
| WBC335 | WBC005F01 | WBC327 | BM734452 | WBC016G11 | BM735585 | 1 | 1 | 1 |
| BM734862 | GI9717252.3 | WBC022C04 | WBC018D03 | Foe1263 | WBC031D02 | 1 | 1 | 1 |
| WBC007F03 | BM734943 | B1961932 | WBC038A02 | WBC028C09 | WBC013E05 | 1 | 1 | 1 |
| Foe1092 | WBC030E04 | BM781269 | BM735386 | WBC009F04 | B1961856 | 1 | 1 | 1 |

**TABLE 11**

| SEVEN GENES SELECTED | | | | | | | | | |
|---|---|---|---|---|---|---|---|---|---|
| Genes | | | | | | | Sensitivity | Specificity | Success |
| B1961900 | WBC017C08 | BM734632 | WBC028C09 | BM735452 | WBC004E04 | WBC031F04 | 1 | 1 | 1 |
| WBC027E07 | WBC027H11 | BM781233 | WBC012E07 | B1961872 | WBC310 | WBC005E06 | 1 | 1 | 1 |
| WBC020H11 | WBC040E12 | WBC021E07 | BM735402 | WBC117 | WBC018B05 | B1961932 | 1 | 1 | 1 |
| WBC014G03 | BM781237_s | BM735487 | WBC033A01 | WBC019C11 | WBC012E05 | WBC215 | 1 | 1 | 1 |
| WBC215 | WBC014G03 | BM734975 | WBC031G11 | WBC017B09 | WBC013A09 | BM735386 | 1 | 1 | 1 |
| WBC018D03 | WBC021E02 | WBC027D09 | WBC038D08 | B1961620 | WBC964 | WBC027F05 | 1 | 1 | 1 |
| BM734531 | BM781379 | BM781067 | WBC015B08 | WBC004G09 | BM735091 | BM734844 | 1 | 1 | 1 |
| Foe1075 | WBC008A10 | WBC025C03 | WBC035E01 | BM735519 | WBC013E10 | BM734780 | 1 | 1 | 1 |
| BM781067 | BM780906 | WBC21 | WBC005E06 | WBC028E11 | BM734661 | WBC031G11 | 1 | 1 | 1 |
| WBC001F10 | B1961872 | WBC005E06 | BM734722 | BM735452 | BM734900 | WBC028C09 | 1 | 1 | 1 |
| WBC007A08 | WBC008D09 | WBC044E01 | BM781438 | BM735519 | WBC003D11 | WBC031 B06 | 1 | 1 | 1 |
| WBC003D11 | WBC038B01 | WBC048H02 | B1961620 | Foe443 | WBC310 | WBC017B09 | 1 | 1 | 1 |
| BM734661 | WBC038B01 | WBC036E08 | WBC001F11 | WBC309 | BM735585 | BM734975 | 1 | 1 | 1 |
| WBC004G01 | B1961827 | WBC335 | BM781416 | WBC038A02 | WBC022B03 | WBC036E08 | 1 | 1 | 1 |
| BM734891 | WBC028E11 | B1961941 | BM735057 | BM781438 | WBC024H07 | WBC015B08 | 1 | 1 | 1 |
| BM781269 | BM735494 | WBC027H11 | B1961941 | WBC028C09 | WBC032H02 | WBC374 | 1 | 1 | 1 |
| BM734975 | WBC037C09 | BM781103 | WBC21 | WBC030E04 | WBC043B10 | WBC311 | 1 | 1 | 1 |
| Foe1324 | WBC019B05 | BM781269 | WBC030C04 | BM735402 | BM734844 | WBC018D03 | 1 | 1 | 1 |
| WBC040E12 | WBC005F01 | B1961900 | Foe1075 | WBC027H11 | WBC039G08 | WBC023F12 | 1 | 1 | 1 |
| WBC017C08 | B1961539 | B1961856 | WBC004E04 | BM781033 | WBC001E10 | BM781278 | 1 | 1 | 1 |

TABLE 12

| EIGHT GENES SELECTED | | | | | | | | | | |
|---|---|---|---|---|---|---|---|---|---|---|
| Genes | | | | | | | | Sensitivity | Specificity | Success |
| WBC025A06 | WBC014B05 | Foe1216 | WBC039F12 | BM734654 | WBC020F05 | Foe1324 | WBC024F08 | 1 | 1 | 1 |
| BM781033 | WBC025A06 | BM780906 | BM735286 | WBC335 | WBC008A10 | BM781067 | WBC444E01 | 1 | 1 | 1 |
| Foe1263 | Foe146 | WBC020H11 | BM735487 | B1961856 | WBC033A01 | WBC107 | WBC025C03 | 1 | 1 | 1 |
| BM734900 | WBC025E03 | WBC031F04 | BM734452 | WBC013A11 | WBC048H02 | WBC020F05 | BM734844 | 1 | 1 | 1 |
| WBC011F05 | WBC033D12 | WBC012E05 | B1961438 | BM734511 | BM781033 | WBC011D03 | WBC022C04 | 1 | 1 | 1 |
| WBC020H01 | WBC044E01 | BM735487 | BM734654 | B1961438 | GI9717252.3M | WBC038D08 | BM781233 | 1 | 1 | 1 |
| WBC019B05 | WBC005F01 | Foe1288 | BM734891 | WBC025E03 | WBC038E02 | BM781409 | WBC027H11 | 1 | 1 | 1 |
| WBC011F05 | Foe1216 | WBC004E04 | BM781319 | WBC013A09 | B1961620 | WBC107 | WBC33 | 1 | 1 | 1 |
| B1961827 | WBC001F11 | BM781033 | BM781233 | WBC020H11 | WBC008D09 | BM735402 | BM735585 | 1 | 1 | 1 |
| WBC003D11 | WBC039G08 | WBC013C09 | WBC023F12 | WBC964 | WBC019C11 | WBC018B05 | WBC030C04 | 1 | 1 | 1 |
| WBC035E01 | WBC025A06 | WBC011F05 | BM734943 | WBC964 | BM781438 | BM734933 | WBC038B01 | 1 | 1 | 1 |
| BM781103 | WBC024F08 | BM735402 | WBC327 | BM781416 | B1961856 | WBC038A02 | WBC024G03 | 1 | 1 | 1 |
| BM781067 | WBC025A06 | WBC038A02 | WBC014H06 | BM781319 | B1961880 | WBC311 | WBC007F03 | 1 | 1 | 1 |
| WBC374 | WBC009F04 | WBC038A02 | B1961539 | WBC039D09 | WBC019B05 | BM734943 | WBC016A12 | 1 | 1 | 1 |
| WBC013E10 | B1961932 | Foe148 | WBC012E05 | WBC309 | BM734943 | BM734975 | B1961941 | 1 | 1 | 1 |
| WBC024G03 | BM780999 | WBC009F04 | BM781233 | BM735585 | WBC020F05 | B1961499 | WBC014E06 | 1 | 1 | 1 |
| WBC025B03 | BM735585 | WBC001F10 | WBC003D11 | WBC022B03 | WBC038A02 | Foe1324 | B1961539 | 1 | 1 | 1 |
| GI9717252.3M | WBC21 | WBC013E10 | WBC032D03 | WBC031D02 | WBC022D03 | BM734632 | BM735402 | 1 | 1 | 1 |
| WBC013E10 | BM781237 | WBC018D03 | BM780914 | WBC027E07 | WBC027B01 | BM734891 | Foe1216 | 1 | 1 | 1 |
| WBC028C09 | BM735519 | WBC013E10 | WBC038G11 | BM781233 | WBC027H11 | WBC011F05 | B1961620 | 1 | 1 | 1 |

**TABLE 13**

| NINE GENES SELECTED | | | | | | | | | | | |
|---|---|---|---|---|---|---|---|---|---|---|---|
| Genes | | | | | | | | | Sensitivity | Specificity | Success |
| BM734719 | WBC030C11 | BM734654 | WBC014G03 | WBC013C09 | WBC031F04 | WBC028C09 | BM735494 | WBC21 | 1 | 1 | 1 |
| BM735057 | BM781379 | B1961499 | WBC031G11 | WBC033D12 | WBC030E04 | WBC016G11 | BM734900 | BM734694 | 1 | 1 | 1 |
| BM781269 | WBC031D02 | WBC013C09 | WBC014H06 | Foe1324 | WBC008A10 | BM734661 | WBC215 | WBC005D09 | 1 | 1 | 1 |
| BM781233 | WBC014E06 | WBC327 | WBC007A08 | B1961932 | BM780906 | B1961900 | WBC004E04 | BM735286 | 1 | 1 | 1 |
| WBC025A06 | WBC21 | BM781233 | BM781278 | WBC310 | BM781394 | WBC019B05 | WBC107 | WBC012E07 | 1 | 1 | 1 |
| BM781416 | WBC020H01 | WBC374 | BM734943 | BM781394 | WBC027B01 | WBC024H07 | WBC027F05 | WBC027D09 | 1 | 1 | 1 |
| WBC016G11 | BM734780 | WBC024H07 | WBC030C11 | B1961941 | WBC022B03 | WBC019C11 | WBC024G08 | WBC309 | 1 | 1 | 1 |
| WBC038A02 | WBC017C08 | BM781416 | WBC030C11 | WBC027H11 | WBC021E02 | BM781237 | WBC033D12 | BM781233 | 1 | 1 | 1 |
| WBC008A12 | WBC037C09 | BM781375 | WBC009H07 | WBC012E05 | WBC030C11 | BM734891 | WBC016G11 | WBC311 | 1 | 1 | 1 |
| WBC335 | WBC025C03 | WBC032D03 | WBC001F11 | WBC374 | WBC020H11 | WBC007F03 | WBC017B09 | WBC005E06 | 1 | 1 | 1 |
| B1961932 | BM734975 | B1961856 | WBC004G01 | WBC013E10 | BM781409 | BM734900 | WBC016A12 | WBC107 | 1 | 1 | 1 |
| WBC038G11 | WBC048H02 | B1961856 | WBC008A10 | WBC024H07 | WBC014E06 | Foe1216 | B1961941 | BM781379 | 1 | 1 | 1 |
| WBC311 | B1961880 | WBC015B08 | BM735386 | WBC374 | WBC030E04 | WBC024F08 | WBC008D09 | WBC018D03 | 1 | 1 | 1 |
| WBC018F03 | BM735494 | WBC025B03 | BM734943 | BM734722 | Foe148 | WBC018B05 | WBC040E12 | WBC031F04 | 1 | 1 | 1 |
| BM781233 | BM734632 | WBC030C04 | BM781394 | BM734694 | BM734780 | BM781278 | B1961872 | WBC019C11 | 1 | 1 | 1 |
| WBC033D12 | Foe1317 | WBC009H07 | B1961856 | BM781186 | WBC327 | WBC005F01 | WBC037C09 | WBC012E05 | 1 | 1 | 1 |
| WBC023F12 | WBC025C03 | WBC044E01 | WBC001D02 | WBC009H07 | BM781409 | BM781103 | BM781067 | WBC008A10 | 1 | 1 | 1 |
| WBC030E04 | WBC335 | B1961438 | WBC028C09 | BM735487 | WBC018F03 | WBC030C04 | WBC038E02 | WBC031D02 | 1 | 1 | 1 |
| BM734780 | WBC013C09 | B1961438 | WBC023F12 | WBC014E06 | WBC011F05 | BM734943 | B1961872 | WBC028C09 | 1 | 1 | 1 |
| WBC028E11 | B1961880 | Foe148 | WBC028G09 | WBC031G11 | BM781379 | BM781067 | WBC327 | BM734694 | 1 | 1 | 1 |

TABLE 14

| TEN GENES SELECTED | | | |
|---|---|---|---|
| Genes | Sensitivity | Specificity | Success |
| WBC031B06; WBC031C11; WBC007F03; Foe1092; WBC33; WBC031F04; WBC005E06; B1961856; B1961499; WBC21 | 1 | 1 | 1 |
| WBC027B01; BM734891; BM781375; WBC030E04; WBC012A03; WBC011D03; WBC024B05; WBC014G03; WBC031B06; GI9717252.3M | 1 | 1 | 1 |
| WBC011D03; WBC009F04; BM781269; WBC027B01; WBC025B03; WBC022B03; B1961872; WBC014E06; WBC031D02; BM781379 | 1 | 1 | 1 |
| WBC025E03; WBC005F10: WBC024G08; BM734780; WBC014G03; WBC001D02; WBC008D09; WBC311; WBC107; BM781186 | 1 | 1 | 1 |
| BM781103; WBC048H02; WBC038D08; WBC011D03; WBC020H01; BM734722; WBC028G09; BM734632; WBC012A03; BM734452 | 1 | 1 | 1 |
| WBC008A10; WBC024H07; WBC044E01; WBC036E08; WBC030C11; BM781103; WBC009F04; BM780914; WBC025E03; WBC335 | 1 | 1 | 1 |
| WBC001E10; WBC036E08; BM781103; Foe1075; WBC019B05; WBC024G08; BM735494; WBC022B03; WBC009F04; B1961438 | 1 | 1 | 1 |
| WBC022C04; WBC012C10; WBC027B01; BM735519; BM781409; BM734632; BM781233; BM735286; WBC024G03; WBC043B10 | 1 | 1 | 1 |
| BM781233; BM734719; BM781237; WBC022B03; B1961856; BM735519; WBC013E10; B1961932; WBC008D09; BM734862 | 1 | 1 | 1 |
| WBC035E01; WBC032H02; WBC013E10; BM734943; BM781416; WBC001D02; WBC012E07; BM734661; WBC024F08; BM735057 | 1 | 1 | 1 |
| WBC017B09; WBC038B01; Foe148; WBC023F12; WBC013E05; WBC012C10; WBC018D03; BM734780; WBC001E10; WBC005F01 | 1 | 1 | 1 |
| WBC031D02; WBC33; WBC030C04; BM781416; WBC012E05; Foe1092; BM780906; WBC003D11; WBC036E08; WBC024G08 | 1 | 1 | 1 |
| WBC039G08; WBC013E10; WBC016A12; BM781067; WBC001F10; WBC007F03; WBC022B03; WBC017B09; WBC020H01; BM735494 | 1 | 1 | 1 |
| BM735452; WBC027E07; WBC037C09; B1961872; WBC016G11; WBC027F05; WBC024F08; WBC020H01; WBC014B05; WBC007F03 | 1 | 1 | 1 |
| WBC011D03; WBC019C11; WBC021E02; WBC001F11; WBC004E04; WBC964; WBC015B08; WBC033D12; WBC008D09; WBC007A08 | 1 | 1 | 1 |
| WBC028C09; BM734722; B1961900, BM734943; WBC024G08; WBC001E10; BM735286; WBC008A10; WBC019C11; WBC027E07 | 1 | 1 | 1 |
| BM734780; BM734719; BM734933; BM735286; WBC008D09; WBC012E05; WBC039F12; WBC027E07; Foe1288; Foe1263 | 1 | 1 | 1 |
| BM781233; WBC003D11; BM734780; B1961620; WBC020F05; WBC019B05; BM781103; B1961856; BM781394; WBC311 | 1 | 1 | 1 |
| B1961932; WBC003D11; WBC025C03; Foe1075; Foe1263; WBC014B05; WBC011D03; B1961932; BM781394; BM734943 | 1 | 1 | 1 |
| 81961499; WBC038A02; WBC014B05; WBC036E08; BM734900; WBC007A08; WBC014G03; B1961539; WBC007F03; WBC020H11 | 1 | 1 | 1 |

**TABLE 15**

| TWELVE GENES SELECTED | | | |
|---|---|---|---|
| Genes | Sensitivity | Specificity | Success |
| WBC014E06; WBC037C09; BM781278; WBC005F01; B1961856; BM735487; B1961880; WBC013A09; BM781103; WBC012E05; B1961499; WBC028C09 | 1 | 1 | 1 |
| WBC027E07; WBC038E02; BM781416; BM781394; BM735091; WBC020H01; BM734975; BM734694; WBC001F10; B1961880; BM735585; Foe1092 | 1 | 1 | 1 |
| B1961620; B1961932; BM781278; WBC028C09; BM735091; BM734654; WBC014E06; WBC21; BM781379; WBC012E07; BM781394; BM734862 | 1 | 1 | 1 |
| WBC013A09; WBC038A02; Foe1075; WBC032H02; BM734632; WBC040E12; WBC025E03; Foe1092; BM735519; B1961438; WBC374; WBC030C11 | 1 | 1 | 1 |
| BM734719; Foe1092; BM781438; Foe443; WBC039G08; BM735091; Foe1288; WBC016G11; BM734844; BM781233; BM735494; WBC047A01 | 1 | 1 | 1 |
| Foe1075; WBC001E10; Foe1216; WBC215; WBC028E11; WBC004E04; BM781379; WBC024G08; B1961932; B1961880; WBC008A10; WBC031D02 | 1 | 1 | 1 |
| WBC117; WBC031C11; WBC028C09; WBC048H02; WBC020H11; BM734531; WBC024F08; BM734694; BM781394; WBC016G11; BM735057; WBC005D09 | 1 | 1 | 1 |
| WBC008A12; B1961499; BM781237; WBC117; WBC030E04; B1961620; Foe443; BM781375; WBC005F01; BM734975; WBC028E11; Foe146 | 1 | 1 | 1 |
| WBC037C09; WBC017B09; BM734452; WBC005F01; BM781233; Foe148; BM735494; WBC027F05; BM735402; WBC033D12; BM734862; WBC019C11 | 1 | 1 | 1 |
| BM734844; Foe1324; Foe148; WBC027B01; WBC009F04; WBC004E04; WBC309; WBC038D08; WBC027E07; BM734452; Foe1092; B1961900 | 1 | 1 | 1 |
| B1961856; BM734862; BM735519; BM734900; WBC023F12; B1961620; BM781186; BM734933; WBC011F05; WBC014E06; BM781416; WBC007F03 | 1 | 1 | 1 |
| WBC001D02; BM781438; BM734511; BM734452; WBC036E08; BM734632; BM734694; B1961932; WBC022D03; BM734531; WBC011D03; WBC023F12 | 1 | 1 | 1 |
| WBC011F05; Foe1317; Foe1092; WBC024G03; WBC004G09; WBC012C10; B1961438; BM735585; WBC003D11; WBC009F04; BM734722; BM781237 | 1 | 1 | 1 |
| BM781379; BM734891; WBC015B08; Foe146; WBC001F11; WBC013A11; WBC016A12; WBC033D12; WBC374; WBC310; WBC013E10; WBC038A02 | 1 | 1 | 1 |
| WBC013C09; WBC014H06; BM781438; BM734661; WBC001F11; B1961827; WBC013E10; WBC036E08; WBC013A11; WBC005D09; BM735057; WBC031C11 | 1 | 1 | 1 |
| WBC005D09; BM734654; WBC044E01; BM781233; WBC215; WBC012B04; WBC024B05; BM735452; BM735091; B1961539; BM734661; WBC964 | 1 | 1 | 1 |

(continued)

| TWELVE GENES SELECTED | | | |
|---|---|---|---|
| Genes | Sensitivity | Specificity | Success |
| B1961856; BM781067; B1961941; WBC327; BM735386; BM734894; WBC038A02; WBC024G03; WBC018D03; GI9717252.3M; BM780914; WBC021E07 | 1 | 1 | 1 |
| BM734933; BM734661; BM734900; WBC001E10; WBC017B09; WBC004G01; WBC374; WBC039G08; WBC327; BM735585; WBC012B04; BM781186 | 1 | 1 | 1 |
| WBC021E07; B1961872; BM735091; WBC21; WBC032H02; WBC33; WBC011D03; BM781416; BM734933; Foe1324; WBC005D09; WBC030C11 | 1 | 1 | 1 |
| WBC021E02; B1961932; BM734452; BM734694; WBC009F04; WBC027E07; BM780914; BM734943; WBC024G03; WBC038G11; WBC004G09; WBC027H11 | 1 | 1 | 1 |

**TABLE 16**

| THIRTEEN GENES SELECTED | | | |
|---|---|---|---|
| Genes | Sensitivity | Specificity | Success |
| WBC335; WBC018F03; WBC014E06; WBC039F12; WBC311; Foe1288; BM735494; WBC035E01; WBC030C11; WBC020H11; WBC008D09 | 1 | 0.91 | 0.93 |
| WBC020H01; BM734891; WBC012E05; BM781394; BM734661; WBC21; BM735402; WBC008A12; Foe1092; BM734933; BM781103; WBC020F05; BM781416 | 1 | 0.73 | 0.8 |
| WBC038B01; GI9717252.3M; WBC011F05; BM735487; WBC036E08; WBC023F12; WBC038DO8; WBC033D12; WBC004G09; BM735519; WBC020H11; WBC009H07; BM781278 | 0.75 | 0.73 | 0.73 |
| BM734694; B1961620; WBC012B04; WBC013E05; WBC008A10; BM781409; WBC024B05; WBC020H01; B1961620; WBC028E11; WBC040E12; BM735585; WBC309 | 1 | 0.55 | 0.67 |
| WBC018F03; WBC037C09; WBC008A10; BM781186; WBC038E02; WBC030E04; WBC025E03; WBC013E05; WBC027E07; Foe1324; WBC013C09; WBC021E02; WBC018F03 | 0.25 | 0.73 | 0.6 |
| WBC028G09; WBC031B06; WBC027F05; WBC019B05; WBC020H01; WBC004E04; WBC028G09; BM734661; WBC030C04; BM781409; WBC027E07; WBC038E02; WBC001E10 | 1 | 0.09 | 0.33 |
| WBC964; WBC008D09; WBC33; WBC020H01; BM734862; B1961880; WBC013E05; WBC017B09; BM734891; WBC028C09; BM735057; BM734661 | 1 | 0.09 | 0.33 |
| Foe443; WBC374; WBC032D03; WBC031B06; WBC014E06; WBC036E08; WBC009F04; WBC025B03; B1961872; BM734891; BM735494; WBC014B05; WBC017B09; BM781438 | 1 | 0.09 | 0.33 |
| Foe1263; BM734780; BM734452; BM734632; BM734844; WBC024F08; WBC311; WBC038A02; WBC023F12; WBC027D09; WBC009H07; WBC021E07; WBC021E02 | 1 | 0.09 | 0.33 |
| WBC024G08; BM734511; WBC022D03; BM734943; WBC001E10; WBC011D03; WBC001F10; WBC024H07; BM735452; B1961856; BM781269; WBC004E04; WBC023F12; Foe1324 | 1 | 0.09 | 0.33 |

(continued)

| THIRTEEN GENES SELECTED | | | |
|---|---|---|---|
| **Genes** | **Sensitivity** | **Specificity** | **Success** |
| WBC107; BM735402; BM781375; WBC018F03; WBC033D12; WBC024H07; BM781379; GI9717252.3M; WBC032H02; WBC013A11; WBC005F10; WBC009H07 | 1 | 0.09 | 0.33 |
| B1961872; WBC215; WBC035E01; WBC012B04; WBC024H07; BM734862; WBC004G01; B1961620; BM734975; WBC019B05; BM734719; BM780914 | 1 | 0.09 | 0.33 |
| WBC024G03; B1961932; BM735494; WBC024H07; GI9717252.3; WBC020F05; WBC016G11; WBC011D03; WBC001F11; BM781269; WBC031B06; WBC027H11; BM735494; WBC011F05 | 1 | 0.09 | 0.33 |
| WBC038B01; BM780914; BM734661; WBC003D11; B1961941; WBC014G03; WBC017B09; BM781409; WBC021E07; WBC030C04; B1961620; BM781103 | 1 | 0.09 | 0.33 |
| BM735402; BM735494; B1961932; BM780906; GI9717252.3M; BM734719; WBC038B01; BM781409; BM734933; B1961827; B1961932; BM735494; WBC031C11; WBC013A11 | 1 | 0.09 | 0.33 |
| WBC024B05; WBC031F04; WBC018F03; WBC024G08; WBC018B05; WBC107; BM734531; Foe1216; BM781409; BM781186; BM735452; WBC038D08; WBC031B06 | 1 | 0.09 | 0.33 |
| WBC003D11; WBC038A02; B1961880; WBC016G11; BM781409; Foe146; WBC040E12; WBC019C11; BM735091; BM781186; WBC033A01; WBC008D09; BM780999 | 1 | 0.09 | 0.33 |
| WBC024G03; B1961827; WBC022D03; WBC038D08; WBC009H07; BM734452; WBC022C04; WBC038A02; WBC027E07; WBC374; Foe146; WBC117; Foe1317 | 1 | 0.09 | 0.33 |
| WBC012B04; WBC039D09; WBC311; BM781269; WBC310; WBC027B01; BM781186; WBC017B09; WBC038D08; WBC008A12; B1961880; B1961620 | 1 | 0.09 | 0.33 |
| Foe1075; WBC024G03; B1961900; WBC024F08; WBC020F05; WBC014H06; BM734844; BM735487; Foe1317; BM734654; WBC012E05; WBC012E07; WBC008D09; WBC037C09; BM734632; WBC032H02 | 1 | 0.09 | 0.33 |

**TABLE 17**
**ENDOTOXEMIA MARKER GENE ONTOLOGY**

| Gene Name | Cellular location | Pathway | Molecular function |
|---|---|---|---|
| B1961438.V1.3_at | NA | NA | NA |
| B1961499.V1.3_at | Cytoplasm | Translation initiation activity | regulation of translational initiation |
| B1961539.V1.3_at | NA | inflammatory response | signal transducer activity |
| B1961620.V1.3_at | NA | NA | NA |
| B1961827.V1.3_at | Arp2/3 protein complex | structural constituent of cytoskeleton | cell motility |
| B1961856.V1.3_at | NA | NA | NA |
| B1961872.V1.3_at | NA | NA | NA |

(continued)

**ENDOTOXEMIA MARKER GENE ONTOLOGY**

| Gene Name | Cellular location | Pathway | Molecular function |
|---|---|---|---|
| B1961880.V1.3_at | extracellular | negative regulation of membrane protein ectodomain proteolysis; positive regulation of cell proliferation | metalloendopeptidase inhibitor activity |
| B1961900.V1.3_at | NA | NA | NA |
| B1961932.V1.3_at | membrane | inflammatory response | interleukin-4 receptor activity |
| B1961932.V1.3_s_at | membrane | inflammatory response | interleukin-4 receptor activity |
| B1961941.V1.3_at | | glycolysis | fructose-bisphosphate aldolase activity |
| BM734452.V1.3_at | Integral to plasma membrane | NA | inflammatory response; phagocytosis |
| BM734511.V1.3_at | NA | NA | NA |
| BM734531.V1.3_at | NA | NA | NA |
| BM734632.V1.3_at | NA | NA | NA |
| BM734654.V1.3_at | NA | NA | NA |
| BM734661.V1.3_at | cytoplasm | protein modification | |
| BM734694.V1.3_at | membrane | protein amino acid phosphorylation | protein-tyrosine kinase activity |
| BM734719.V1.3_at | NA | NA | NA |
| BM734722.V1.3_at | NA | NA | NA |
| BM734780.V1.3_at | cytoplasm | JAK-STAT cascade | transcription factor activity |
| BM734795 | Nucleus, immune synapse | Granzyme A activity, protein binding | Immune response |
| BM734844.V1.3_at | membrane | transferase activity | biosynthesis |
| BM734862.V1.3_at | membrane | humoral immune response | receptor activity |
| BM734891.V1.3_at | NA | NA | NA |
| BM734900.V1.3_at | extracellular space | proteolysis and peptidolysis | collagenase activity |
| BM734933.V1.3_at | NA | NA | calcium ion binding |
| BM734943.V1.3_at | NA | NA | NA |
| BM734975.V1.3_at | Plasma membrane | signal transduction | GTP binding |
| BM735057.V1.3_s_at | extracellular space | inflammatory response | chemokine |
| BM735091.V1.3_at | cytoplasm | NA | serine-type endopeptidase inhibitor activity |
| BM735286.V1.3_at | cytoplasm | iron ion homeostasis | ferric iron binding |
| BM735386.V1.3_at | NA | NA | NA |
| BM735402.V1.3_at | NA | NA | NA |
| BM735452.V1.3_at | NA | NA | NA |
| BM735487.V1.3_at | NA | NA | NA |
| BM735494.V1.3_at | Plasma membrane | signal transduction | immune response |
| BM735519.V1.3_at | NA | NA | NA |
| BM735585.V1.3_at | membrane | immunoglobulin secretion | NA |
| BM780906.V1.3_at | NA | NA | NA |
| BM780914.V1.3_at | NA | NA | NA |
| BM780999.V1.3_at | nucleus | chromatin remodelling | NA |
| BM781033.V1.3_at | nucleus | DNA metabolism | single-stranded DNA binding |

(continued)

**ENDOTOXEMIA MARKER GENE ONTOLOGY**

| Gene Name | Cellular location | Pathway | Molecular function |
|---|---|---|---|
| BM781067.V1.3_at | membrane | positive regulation of T-cell proliferation | T-cell receptor binding |
| BM781103.V1.3_at | cytoplasm | proteolysis and peptidolysis | cathepsin H activity |
| BM781186.V1.3_at | membrane | NA | NA |
| BM781233.V1.3_at | cytoplasm | translational elongation | translation elongation factor activity |
| BM781237.V1.3_s_at | nucleus | cytokinesis | cell cycle |
| BM781269.V1.3_at | membrane | immune response | interleukin-1, Type II, blocking receptor activity |
| BM781278.V1.3_at | NA | NA | NA |
| BM781319.V1.3_at | | G1/S transition of mitotic cell cycle | NA |
| BM781375.V1.3_at | NA | protein kinase C activation | protein kinase C activity |
| BM781379.V1.3_at | cytoplasm | protein-tyrosine kinase activity | intracellular signalling |
| BM781394.V1.3_at | NA | NA | NA |
| BM781409.V1.3_at | NA | NA | NA |
| BM781416.V1.3_at | NA | NA | NA |
| BM781438.V1.3_at | integral to membrane | insulin-like growth factor receptor activity | neurogenesis |
| Foe1075.V1.3_at | cytoplasm | regulation of translational initiation | translation initiation factor activity |
| Foe1092.V1.3_at | NA | NA | NA |
| Foe1216.V1.3_at | nucleus | regulation of transcription from Pol II promoter | transcription coactivator activity |
| Foe1263.V1.3_at | NA | NA | NA |
| Foe1288.V1.3_at | NA | NA | NA |
| Foe1317.V1.3_at | | fatty acid metabolism | oxidoreductase activity |
| Foe1324.V1.3_at | nucleus | nucleosome assembly | DNA binding |
| Foe146.V1.3_at | NA | NA | NA |
| Foe148.V1.3_at | golgi apparatus | glycoprotein 6-alpha-L-fucosyltransferase activity | oligosaccharide biosynthesis |
| Foe443.V1.3_at | NA | subtilase activity | proteolysis |
| GI9717252-3_at | membrane | detection of pathogenic bacteria | lipopolysaccharide binding |
| GI9717252-3M_at | membrane | detection of pathogenic bacteria | lipopolysaccharide binding |
| WBC001 D02_V1.3_at | membrane | potassium ion transport | voltage-gated potassium channel activity |
| WBC001E10_V1.3_at | NA | NA | NA |
| WBC001F10_V1.3_at | NA | protein amino acid dephosphorylation | protein tyrosine phosphatase activity |
| WBC001 F11_V1.3_at | Nucleus | cell cycle | regulation of transcription |
| WBC003D11_V1.3_at | NA | NA | NA |
| WBC004E04_V1.3_at | Nucleus | NA | NA |
| WBC004G01_V1.3_at | NA | NA | NA |
| WBC004G09_V1.3_at | NA | NA | NA |
| WBC005D09_V1.3_at | NA | NA | NA |
| WBC005E06_V1.3_at | Nucleus | NA | DNA binding |

(continued)

**ENDOTOXEMIA MARKER GENE ONTOLOGY**

| Gene Name | Cellular location | Pathway | Molecular function |
|---|---|---|---|
| WBC005F01_V1.3_at | NA | protein amino acid phosphorylation | protein serine/threonine kinase activity |
| WBC005F10_V1.3_at | NA | NA | Receptor activity |
| WBC007A08_V1.3_at | NA | NA | NA |
| WBC007F03_V1.3_at | Nucleus | Transcription, glycolysis | phosphopyruvate hydratase activity |
| WBC008A10_V1.3_at | extracellular | proteolysis and peptidolysis | neutrophil collagenase activity |
| WBC008A12_V1.3_at | NA | electron transport | electron transporter activity |
| WBC008D09_V1.3_at | membrane | cation transport | ATPase activity, coupled to transmembrane movement of ions, phosphorylative mechanism |
| WBC009F04_V1.3_at | membrane | cell adhesion | protein binding |
| WBC009H07_V1.3_at | nucleus | NA | helicase activity |
| WBC011D03_V1.3_at | nucleus | cell cycle | calmodulin binding |
| WBC011F05_V1.3_at | NA | NA | NA |
| WBC012A03_V1.3_at | NA | NA | NA |
| WBC012B04_V1.3_at | Type I membrane protein | NA | calcium ion binding |
| WBC012C10_V1.3_at | membrane | potassium ion transport | inward rectifier potassium channel activity |
| WBC012E05_V1.3 at | NA | NA | NA |
| WBC012E07_V1.3_at | Plasma membrane | Structural molecule activity | cell adhesion |
| WBC013A09_V1.3_at | membrane | humoral immune response | beta-galactoside alpha-2,6-sialyltransferase activity |
| WBC013A11_V1.3_at | nucleus | nucleosome assembly | NA |
| WBC013C09_V1.3_at | membrane | hemocyte development | NA |
| WBC013E05_V1.3_at | membrane | immune response | NA |
| WBC013E10_V1.3_at | NA | NA | NA |
| WBC014B05_V1.3_at | NA | anti-apoptosis | NA |
| WBC014E06_V1.3_at | NA | NA | NA |
| WBC014G03_V1.3_at | Nucleus | nucleotide binding | mRNA processing |
| WBC014H06_V1.3_at | membrane | NA | G-protein coupled receptor activity |
| WBC015B08_V1.3_at | cytoplasm | kinase, transferase and transporter activity | lipid metabolism and molecular transport |
| WBC016A12_V1.3_at | NA | NA | NA |
| WBC016G11_V1.3_at | nucleus | DNA replication | DNA-dependent ATPase activity |
| WBC017B09_V1.3_at | Integral to membrane | intracellular signalling cascade | DNA binding |
| WBC017C08_V1.3_at | NA | NA | NA |
| WBC018B05_V1.3_at | nucleus | negative regulation of transcription from Pol II promoter | transcription corepressor activity |
| WBC018D03_V1.3_at | NA | NA | NA |
| WBC018F03_V1.3_at | membrane | cell adhesion | receptor activity |
| WBC019B05_V1.3_at | NA | NA | NA |
| WBC019C11_V1.3_at | extracellular | humoral immune response | |

(continued)

**ENDOTOXEMIA MARKER GENE ONTOLOGY**

| Gene Name | Cellular location | Pathway | Molecular function |
|---|---|---|---|
| WBC020F05_V1.3_at | cytoplasm | regulation of translational initiation | translation initiation factor activity |
| WBC020H01_V1.3_at | cytoplasm | antimicrobial humoral response (sensu Vertebrata) | MAP kinase kinase activity |
| WBC020H11_V1.3_at | nucleus | transcription from Pol II promoter | transcription factor activity |
| WBC021E02_V1.3_at | NA | NA | NA |
| WBC021E07_V1.3_at | Nucleus | intracellular signalling cascade | Transcription factor activity |
| WBC022B03_V1.3_at | NA | NA | T-cell receptor binding |
| WBC022C04_V1.3_at | Cytoplasmic and nuclear | Binds to caspase 1 | Negative regulation of caspase |
| WBC022D03_V1.3_at | NA | NA | NA |
| WBC023F12_V1.3_at | NA | NA | NA |
| WBC024B05_V1.3_at | membrane, cytoskeleton | NA | structural |
| WBC024F08_V1.3_at | NA | NA | NA |
| WBC024G03_V1.3_at | Extracellular | NA | inhibition of proteases |
| WBC024G08_V1.3_at | NA | NA | NA |
| WBC024H07_V1.3_at | membrane | cell surface receptor linked signal transduction | interferon-gamma receptor activity |
| WBC025A06_V1.3_at | NA | NA | NA |
| WBC025B03_V1.3_at | | | receptor activity |
| WBC025C03_V1.3_at | NA | Cell cycle | Protein binding |
| WBC025E03_V1.3_at | nucleus | skeletal development | DNA binding |
| WBC027B01_V1.3_at | NA | NA | NA |
| WBC027D09_V1.3_at | Na | NA | NA |
| WBC027E07_V1.3_at | membrane | cell surface receptor linked signal transduction | protein tyrosine phosphatase activity |
| WBC027F05_V1.3_at | extracellular | proteolysis and peptidolysis | neutrophil collagenase activity |
| WBC027H11_V1.3_at | cytoplasm | protein biosynthesis | structural constituent of ribosome |
| WBC028C09_V1.3_at | cytoplasm | NA | endocytosis |
| WBC028D04_V1.3_at | NA | NA | NA |
| WBC028E11_V1.2_at | cytoplasm | signal transduction | signal transducer activity |
| WBC028G09_V1.3_at | NA | NA | NA |
| WBC030C04_V1.3_at | NA | NA | NA |
| WBC030C11_V1.3_at | cytoplasm | protein ubiquitination | ubiquitin-protein ligase activity |
| WBC030E04_V1.3_at | extracellular | proteolysis and peptidolysis | interstitial collagenase activity |
| WBC031B06_V1.3_at | endosomes, lysosomes, plasma membrane | NA | receptor activity |
| WBC031C11_V1.3_at | cytoplasm | protein biosynthesis | structural constituent of ribosome |
| WBC031 D02_V1.3_at | membrane | actin cytoskeleton organization and biogenesis | cytoskeletal adaptor activity |
| WBC031 F04_V1.3_at | NA | NA | NA |

(continued)

**ENDOTOXEMIA MARKER GENE ONTOLOGY**

| Gene Name | Cellular location | Pathway | Molecular function |
|---|---|---|---|
| WBC031G11_V1.3_at | membrane | carbohydrate transport | glucose transporter activity |
| WBC032D03_V1.3_at | NA | NA | NA |
| WBC032H02_V1.3_at | NA | NA | NA |
| WBC033A01_V1.3_at | nucleus | regulation of transcription, DNA-dependent | transcription factor activity |
| WBC033D12_V1.3_at | NA | NA | NA |
| WBC035E01_V1.3_at | NA | NA | mRNA processing |
| WBC036E08_V1.3_at | Plasma membrane | phospholipase activity | skeletal development |
| WBC037C09_V1.3_at | NA | NA | NA |
| WBC038A02_V1.3_at | cytoplasm | protein biosynthesis | structural constituent of ribosome |
| WBC038B01_V1.3_at | cytoplasm | protein biosynthesis | structural constituent of ribosome |
| WBC038D08_V1.3_at | NA | NA | NA |
| WBC038E02_V1.3_at | Integral to plasma membrane | protein amino acid phosphorylation | protein-tyrosine kinase activity |
| WBC038G11_V1.3_at | NA | NA | NA |
| WBC039D09_V1.3_at | NA | ubiquitin cycle | ubiquitin conjugating enzyme activity |
| WBC039F12_V1.3_at | Plasma membrane | NA | cell adhesion |
| WBC039G08_V1.3_at | NA | NA | NA |
| WBC040E12_V1.3_at | integral to membrane | NA | NA |
| WBC043B10_V1.3_at | nucleus | DNA replication initiation | DNA binding |
| WBC044E01_V1.3_at | cytoplasm | purine catabolism | dihydropyrimidine dehydrogenase (NADP+) activity |
| WBC047A01_V1.3_at | nucleus | negative regulation of transcription from Pol II promoter | DNA binding |
| WBC048H02_V1.3_at | NA | protein kinase C activation | protein kinase C binding |
| WBC107.V1.3_s_at | NA | NA | NA |
| WBC117.gRSP.V1.3_at | NA | NA | NA |
| WBC21.V1.3_at | NA | NA | NA |
| WBC215.gRSP.V1.3_at | Nucleus | Cell proliferation | phosphoprotein phosphatase activity |
| WBC309.V1.3_at | NA | NA | NA |
| WBC310.V1.3_at | Nucleus, cytoplasm | transcription | regulation of transcription |
| WBC311.V1.3_at | nucleus | cell surface receptor linked signal transduction | DNA binding activity |
| WBC327.V1.3_at | membrane | starch catabolism | catalytic activity |
| WBC33.gRSP.V1.3_at | NA | NA | NA |
| WBC335.V1.3_at | cytoplasm (cytoskeleton) | NA | structural molecule activity |
| WBC374.V1.3_at | NA | NA | NA |
| WBC964.gRSP.V1.3_at | NA | NA | NA |

The invention also relates to the following aspects as defined in the following numbered paragraphs:

1. A method for diagnosing the presence of an endotoxemia-related condition in a test subject, comprising detecting in the test subject aberrant expression of at least one endotoxemia marker gene that is expressed in cells of the immune system and that is selected from the group consisting of: (a) a gene having a polynucleotide expression product comprising a nucleotide sequence that shares at least 50% sequence identity with the sequence set forth in any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof, (b) a gene having a polynucleotide expression product comprising a nucleotide sequence that encodes a polypeptide comprising the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (c) a gene having a polynucleotide expression product comprising a nucleotide sequence that encodes a polypeptide that shares at least 50% sequence similarity with at least a portion of the sequence set forth in SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 15 contiguous amino acid residues of that sequence; and (d) a gene having a polynucleotide expression product comprising a nucleotide sequence that hybridizes to the sequence of (a), (b), (c) or a complement thereof, under at least low stringency conditions.

2. A method according to paragraph 1,comprising detecting aberrant expression of an endotoxemia marker polynucleotide selected from the group consisting of (a) a polynucleotide comprising a nucleotide sequence that shares at least 50% sequence identity with the sequence set forth in any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof; (b) a polynucleotide comprising a nucleotide sequence that encodes a polypeptide comprising the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (c) a polynucleotide comprising a nucleotide sequence that encodes a polypeptide that shares at least 50% sequence similarity with at least a portion of the sequence set forth in SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

3. A method according to paragraph 1, comprising detecting aberrant expression of an endotoxemia marker polypeptide selected from the group consisting of: (i) a polypeptide comprising an amino acid sequence that shares at least 50% (sequence similarity with the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 17, 319, 322, 324 or 326; (ii) a polypeptide comprising a portion of the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 5 contiguous amino acid residues of that sequence; (iii) a polypeptide comprising an amino acid sequence that shares at least 30% similarity with at least 15 contiguous amino acid residues of the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; and (iv) a polypeptide comprising a portion of the sequence set forth in any one of SEQ ID: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 5 contiguous amino acid residues of that sequence and is immuno-interactive with an antigen-binding molecule that is immuno-interactive with a sequence of (i), (ii) or (iii).

4. A method according to paragraph 1, wherein the aberrant expression is detected by: (1) measuring in a biological sample obtained from the test subject the level or functional activity of an expression product of at least one endotoxemia marker gene and (2) comparing the measured level or functional activity of each expression product to the level or functional activity of a corresponding expression product in a reference sample obtained from one or more normal subjects or from one or more subjects lacking an endotoxemia-related condition, wherein a difference in the level or functional activity of the expression product in the biological sample as compared to the level or functional activity of the corresponding expression product in the reference sample is indicative of the presence of an endotoxemia-related condition in the test subject

5. A method according to paragraph 4, further comprising diagnosing the presence, stage or degree of an endotoxemia-related condition in the test subject when the measured level or functional activity of the or each expression product is 10% higher than the measured level or functional activity of the or each corresponding expression product.

6. A method according to paragrap 5, wherein the presence of endotoxemia-related condition is determined by detecting an increase in the level or functional activity of at least one endotoxemia marker polynucleotide selected from (a) a polynucleotide comprising a nucleotide sequence that shares at least 50% sequence identity with the sequence set forth in any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130,132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof, (b) a polynucleotide comprising a nucleotide sequence that encodes a polypeptide comprising the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (c) a polynucleotide comprising a nucleotide sequence that encodes a polypeptide that shares at least 50% sequence similarity with at least a portion of the sequence set forth in SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

7. A method according to paragraph 4, further comprising diagnosing the presence, stage or degree of an endotoxemia-related condition in the test subject when the measured level or functional activity of the or each expression product is 10% lower than the measured level or functional activity of the or each corresponding expression product.

8. A method according to paragraph 7, wherein the presence of an endotoxemia-related condition is determined by detecting a decrease in the level or functional activity of at least one endotoxemia marker polynucleotide selected from (a) a polynucleotide comprising a nucleotide sequence that shares at least 50% sequence identity with the sequence set forth in any one of SEQ ID NO: 5, 6, 7, 11, 13, 15, 16, 17, 18, 19, 21, 25, 26, 35, 37, 38, 41, 42, 43, 44, 50, 52, 54, 58, 60, 63, 64, 69, 70, 71, 73, 77, 79, 80, 81, 82, 83, 86, 92, 93, 96, 98, 100, 101, 102, 103, 104, 106, 115, 117, 128, 134, 137, 141, 143, 153, 155, 158, 162, 164, 166, 174, 182, 186, 188, 190, 195, 197, 199, 201, 205, 206, 207, 209, 210, 211, 214, 215, 216, 222, 223, 240, 244, 246, 248, 259, 260, 269, 272, 280, 282, 284, 286, 290, 298, 300, 302, 305, 306, 307, 309, 312, 314, 315, 316, 318, 320, 321, 323, or a complement thereof; (b) a polynucleotide comprising a nucleotide sequence that encodes a polypeptide comprising the amino acid sequence set forth in any one of SEQ ID NO: 8, 12, 14, 20, 22, 36, 51, 53, 55, 59, 65, 72, 74, 78, 87, 97, 99, 105, 116, 118, 129, 135, 138, 142, 144, 154, 156, 159, 163, 165, 167, 175, 183, 187, 189, 191, 196, 198, 200, 202, 208, 217, 241, 247, 249, 261, 273, 281, 283, 285, 287, 291, 299, 301, 303, 308, 310, 313, 317, 319, 322, 324; (c) a polynucleotide comprising a nucleotide sequence that encodes a polypeptide that shares at least 50% sequence similarity with at least a portion of the sequence set forth in SEQ ID NO: 8, 12, 14, 20, 22, 36, 51, 53, 55, 59, 65, 72, 74, 78, 87, 97, 99, 105, 116, 118, 129, 135, 138, 142, 144, 154, 156, 159, 163, 165, 167, 175, 183, 187, 189, 191, 196, 198, 200, 202, 208, 217, 241, 247, 249, 261, 273, 281, 283, 285, 287, 291, 299, 301, 303, 308, 310, 313, 317, 319, 322, 324, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

9. A method according to paragraph4,further comprising diagnosing the absence of an endotoxemia-related condition when the measured level or functional activity of the or each expression product is the same as or similar to the measured level or functional activity of the or each corresponding expression product.

10. A method according to paragraph4, wherein the measured level or functional activity of an individual expression product varies from the measured level or functional activity of an individual corresponding expression product by no more than about 5%.

11. A method according to paragraph4,comprising measuring the level or functional activity of individual expression products of at least about two endotoxemia marker genes.

12. A method according to paragraph4,comprising measuring the level or functional activity of individual expression products of at least one level one correlation endotoxemia marker gene selected from: (a) a polynucleotide comprising a nucleotide sequence that shares at least 50% sequence identity with the sequence set forth in any one of SEQ ID NO: 11, 23, 29, 35, 43, 44, 68, 81, 82, 84, 104, 105, 107, 119, 130, 136, 147, 155, 174, 192, 193, 245, 254, 255, 262, 264, 270, 271, 279, 296, or 325, or a complement thereof; (b) a polynucleotide comprising a nucleotide sequence that encodes a polypeptide comprising the amino acid sequence set forth in any one of SEQ ID NO: 12, 24, 30, 36, 85, 108, 120, 131, 148, 156, 175, 256, 263, 265, 297, or 326; (c) a polynucleotide comprising a nucleotide sequence that encodes a polypeptide that shares at least 50% sequence similarity with at least a portion of the sequence set forth in SEQ. ID NO: 12, 24, 30, 36, 85, 108, 120, 131, 148, 156, 175, 256, 263, 265, 297, or 326, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

13. A method according to paragraph4,comprising measuring the level or functional activity of individual expression products of at least one level two correlation endotoxemia marker gene selected from: (a) a polynucleotide comprising a nucleotide sequence that shares at least 50% ( sequence identity with the sequence set forth in any one of SEQ ID NO: 1, 7, 9, 10, 17, 18, 21, 25, 26, 33, 54, 61, 64, 79, 80, 90, 94, 115, 117, 121, 122, 125, 126, 143, 160, 162, 164, 172, 173, 178, 184, 186, 194, 199, 205, 206, 225, 229, 242, 244, 252, 257, 259, 274, 276, 282, 284, 288, 294, 306, 316, or 318, or a complement thereof; (b) a polynucleotide comprising a nucleotide sequence that encodes a polypeptide comprising the amino acid sequence set forth in any one of SEQ ID NO: 2, 8, 22, 34, 55, 62, 65, 91, 95, 116, 118, 127, 144, 161, 163, 165, 179, 185, 187, 200, 226, 230, 243, 253, 258, 275, 277, 283, 285, 289, 295, 317, or 319, (c) a polynucleotide comprising a nucleotide sequence that encodes a polypeptide that shares at least 50% sequence similarity with at least a portion of the sequence set forth in SEQ ID NO: 2, 8, 22, 34, 55, 62, 65, 91, 95, 116,118, 127, 144, 161, 163, 165, 179, 185, 187, 200, 226, 230, 243, 253, 258, 275, 277, 283, 285, 289, 295, 317, or 319, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

14. A method according to paragraph4,comprising measuring the level or functional activity of individual expression products of at least one level three correlation endotoxemia marker gene selected from: (a) a polynucleotide comprising a nucleotide sequence that shares at least 50% sequence identity with the sequence set forth in any one of SEQ ID NO: 3, 4, 5, 6, 13, 15, 16, 27, 31, 37, 38, 39, 41, 42, 45, 47, 49, 52, 56, 58, 63, 66, 67, 69, 70, 71, 77, 83, 86, 88, 96, 98, 100, 101, 106, 109, 110, 111, 113, 114, 128, 132, 134, 137, 139, 141, 145, 149, 151, 153, 157, 158, 166, 168, 169, 176, 180, 188, 190, 197, 203, 207, 209, 210, 211, 214, 215, 218, 220, 222, 223, 224, 231, 233, 236, 237, 239, 240, 241, 246, 250, 260, 266, 268, 269, 272, 278, 280, 286, 290, 292, 300, 304, 309, 312, 314, 315, 321, or 323, or a complement thereof; (b) a polynucleotide comprising a nucleotide sequence that encodes a polypeptide comprising the amino acid sequence set forth in any one of SEQ ID NO: 14, 28, 32, 40, 46, 48, 53, 57, 59, 72, 78, 87, 89, 97, 99, 112, 129, 133, 135, 138, 140, 142, 146, 150, 152, 154, 159, 167, 177, 181, 189, 191, 198, 204, 208, 219, 221, 232, 234, 238, 247, 251, 261, 267, 273, 281, 287, 291, 293, 301, 310, 313, 322, or 324; (c) a polynucleotide comprising a nucleotide sequence that encodes a polypeptide that shares at least 50% sequence similarity with at least a portion of the sequence set forth in SEQ ID NO: 14, 28, 32, 40, 46, 48, 53, 57, 59, 72, 78, 87, 89, 97, 99, 112, 129, 133, 135, 138, 140, 142, 146, 150, 152, 154, 159, 167, 177, 181, 189, 191, 198, 204, 208, 219, 221, 232, 234, 238, 247, 251, 261, 267, 273, 281, 287, 291, 293, 301, 310, 313, 322, or 324, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

15. A method according to paragraph4,comprising measuring the level or functional activity of individual expression products of at least one level four correlation endotoxemia marker gene selected from: (a) a polynucleotide comprising a nucleotide sequence that shares at least 50% sequence identity with the sequence set forth in any one of SEQ ID NO: 19, 50, 60, 73, 75, 92, 93, 102, 103, 123, 124, 170, 182, 195, 201, 212, 216, 227, 235, 248, 298, 302, 305, 307, 311, or 320, or a complement thereof; (b) a polynucleotide comprising a nucleotide sequence that encodes a polypeptide comprising the amino acid sequence set forth in any one of SEQ ID NO: 20, 51, 74, 76, 171, 183, 196, 202, 213, 217, 228, 249, 299, 303, or 308; (c) a polynucleotide comprising a nucleotide sequence that encodes a polypeptide that shares at least 50% sequence similarity with at least a portion of the sequence set forth in SEQ ID NO: 20, 51, 74, 76, 171, 183, 196, 202, 213, 217, 228, 249, 299, 303, or 308, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

16. A method according to paragraph4, wherein the biological sample comprises blood.

17. A method according to paragraph4,wherein the biological sample comprises peripheral blood.

18. A method according to paragraph4, wherein the biological sample comprises leukocytes.

19. A method according to paragraph4,wherein the expression product is a RNA molecule.

20. A method according to paragraph4, wherein the expression product is a polypeptide.

21. A method according to paragraph4, wherein the expression product is the same as the corresponding expression product

22. A method according to paragraph4, wherein the expression product is a variant of the corresponding expression product.

23. A method according to paragraph4, wherein the expression product or corresponding expression product is a target RNA or a DNA copy of the target RNA whose level is measured using at least one nucleic acid probe that hybridizes under at least low stringency conditions to the target RNA or to the DNA copy, wherein the nucleic acid probe comprises at least 15 contiguous nucleotides of an endotoxemia marker polynucleotide.

24. A method according to paragraph23, wherein the measured level or abundance of the target RNA or its DNA copy is normalized to the level or abundance of a reference RNA or a DNA copy of the reference RNA that is present in the same sample.

25. A method according to paragraph23, wherein the nucleic acid probe is immobilized on a solid or semi-solid support.

26. A method according to paragraph23, wherein the nucleic acid probe forms part of a spatial array of nucleic acid probes.

27. A method according to paragraph23, wherein the level of nucleic acid probe that is bound to the target RNA or to the DNA copy is measured by hybridization.

28. A method according to paragraph23, wherein the level of nucleic acid probe that is bound to the target RNA or to the DNA copy is measured by nucleic acid amplification.

29. A method according to paragraph23, wherein the level of nucleic acid probe that is bound to the target RNA or to the DNA copy is measured by nuclease protection assay.

30. A method according to paragraph23, wherein the probe for detecting the endotoxemia marker polynucleotide comprises a sequence as set forth in any one of SEQ ID NO: 327-2317.

31. A method according to paragraph23, wherein the expression product or corresponding expression product is a target polypeptide whose level is measured using at least one antigen-binding molecule that is immuno-interactive with the target polypeptide.

32. A method according to paragraph23, wherein the measured level of the target polypeptide is normalized to the level of a reference polypeptide that is present in the same sample.

33. A method according to paragraph23, wherein the antigen-binding molecule is immobilized on a solid or semi-solid support.

34. A method according to paragraph23, wherein the antigen-binding molecule forms part of a spatial array of antigen-binding molecule.

35. A method according to paragraph23, wherein the level of antigen-binding molecule that is bound to the target polypeptide is measured by immunoassay.

36. A method according to paragraph4, wherein the expression product or corresponding expression product is a target polypeptide whose level is measured using at least one substrate for the target polypeptide with which it reacts to produce a reaction product.

37. A method according to paragraph36, wherein the measured functional activity of the target polypeptide is normalized to the functional activity of a reference polypeptide that is present in the same sample.

38. A method according to paragraph4, wherein a system is used to perform the method, which comprises at least one end station coupled to a base station., wherein the base station is caused (a) to receive subject data from the end station via a communications network, wherein the subject data represents parameter values corresponding to the measured or normalized level or functional activity of at least one expression product in the biological sample, and (b) to compare the subject data with predetermined data representing the measured or normalized level or functional activity of at least one corresponding expression product in the reference sample to thereby determine any difference in the level or functional activity of the expression product in the biological sample as compared to the level or functional activity of the corresponding expression product in the reference sample.

39. A method according to paragraph38, wherein the base station is further caused to provide a diagnosis for the presence, absence or degree of an endotoxemia-related condition.

40. A method according to paragraph38, wherein the base station is further caused to transfer an indication of the diagnosis to the end station *via* the communications network.

41. A method according to paragraph 1, wherein detection of the aberrant expression is indicative of the presence or risk of an endotoxemia-related condition.

42. A method according to paragraph 1, wherein the test subject is a horse.

43. A method for treating, preventing or inhibiting the development of an endotoxemia-related condition in a subject, the method comprising detecting aberrant expression of at least one endotoxemia marker gene in the subject, and administering to the subject an effective amount of an agent that treats or ameliorates the symptoms or reverses or inhibits the development of the endotoxemia-related condition in the subject, wherein the endotoxemia marker gene is expressed in cells of the immune system and is selected from the group consisting of: (a) a gene having a polynucleotide expression product comprising a nucleotide sequence that shares at least 50% sequence identity with the sequence set forth in any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229,231,233, 235,236,237,239,240,242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325; or a complement thereof; (b) a gene having a polynucleotide expression product comprising a nucleotide sequence that encodes a polypeptide comprising the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281,283, 285, 287, 289, 291, 293, 295, 297, 299, 301, 303, 308, 310, 313, 317, 319, 322, 324 or 326; (c) a gene having a polynucleotide expression product comprising a nucleotide sequence that encodes a polypeptide that shares at least 50% sequence similarity with at least a portion of the sequence set forth in SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 15 contiguous amino acid residues of that sequence; and (d) a gene having a polynucleotide expression product comprising a nucleotide sequence that hybridizes to the sequence of (a), (b), (c) or a complement thereof, under at least low stringency conditions.

44. An isolated endotoxemia marker polynucleotide selected from: (a) a polynucleotide comprising a nucleotide sequence that shares at least 50% sequence identity with the sequence set forth in any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321; 323 or 325, or a complement thereof; (b) a polynucleotide comprising a portion of the sequence set forth in any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof, wherein the portion comprises at least 15 contiguous nucleotides of that sequence or complement; (c) a polynucleotide that hybridizes to the sequence of (a) or (b) or a complement thereof, under at least low stringency conditions; and (d) a polynucleotide comprising a portion of any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof, wherein the portion comprises at least 15 contiguous nucleotides of that sequence or complement and hybridizes to a sequence of (a), (b) or (c), or a complement thereof, under at least low stringency conditions.

45. A nucleic acid construct comprising an endotoxemia marker polynucleotide according to paragraph44, in operable connection with a regulatory element that is operable in a host cell.

46. An isolated host cell containing a nucleic acid construct according to paragraph45.

47. A probe comprising a nucleotide sequence that hybridizes under at least low stringency conditions to a polynucleotide according to paragraph44.

48. A probe according to paragraph47, consisting essentially of a nucleic acid sequence that corresponds or is complementary to at least a portion of a nucleotide sequence encoding the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion is at least 15 nucleotides in length.

49. A probe according to paragraph47, wherein the probe comprises a nucleotide sequence which is capable of hybridizing to at least a portion of a nucleotide sequence encoding the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; under at least low stringency conditions, wherein the portion is at least 15 nucleotides in length.

50. A probe according to paragraph47, wherein the probe comprise a nucleotide sequence that is capable of hybridizing to at least a portion of any one of SEQ ID NO: 1, 3, 4, 5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79r; 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270,271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, under at least low stringency conditions, wherein the portion is at least 15 nucleotides in length.

51. A probe according to paragraph47,| comprising a sequence as set forth in any one of SEQ ID NO: 145-2150.

52. A solid or semi-solid support comprising at least one probe according to paragraph47, | immobilized thereon.

53. Use of one or more endotoxemia marker polynucleotides according to paragraph44, or the use of one or more probes according to paragraph47, or the use of one or more endotoxemia marker polypeptides selected from the group consisting of: (i) a polypeptide comprising an amino acid sequence that shares at least 50% sequence similarity with the sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 23 8, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (ii) a polypeptide comprising an amino acid sequence that shares at least 50% sequence similarity with a polypeptide expression product of an endotoxemia marker gene that comprises a sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135, 138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (iii) a portion of the polypeptide according to (i) or (ii) wherein the portion comprises at least 5 contiguous amino acid residues of that polypeptide; (iv) a polypeptide comprising an amino acid sequence that shares at least 30% similarity with at least 15 contiguous amino acid residues of the polypeptide according to (i) or (ii); and (iv) a polypeptide comprising a portion of the polypeptide according to (i) or (ii), wherein the portion comprises at least 5 contiguous amino acid residues of the polypeptide according to (i) or (ii) and is immuno-interactive with an antigen-binding molecule that is immuno-interactive with a sequence of (i), (ii) or (iii), or the use of one or more antigen-binding molecules that are immuno-interactive with a said endotoxemia marker polypeptide, in the manufacture of a kit for diagnosing the presence of an endotoxemia-related condition in a subject.

54. A method for diagnosing the presence of an endotoxemia-related condition in a test subject, comprising detecting in the test subject aberrant expression of at least one endotoxemia marker polynucleotide that is expressed in cells of the immune system and that is selected from the group consisting of: (a) a polynucleotide comprising a nucleotide sequence that shares at least 50% sequence identity with the sequence set forth in any one of SEQ ID NO: 1, 3, 4,5, 6, 7, 9, 10, 11, 13, 15, 16, 17, 18, 19, 21, 23, 25, 26, 27, 29, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof, (b) a polynucleotide comprising a nucleotide sequence that encodes a polypeptide comprising the amino acid sequence set forth in any one of SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (c) a polynucleotide comprising a nucleotide sequence that encodes a polypeptide that shares at least 50% sequence similarity with at least a portion of the sequence set forth in SEQ ID NO: 2, 8, 12, 14, 20, 22, 24, 28, 30, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 15 contiguous amino acid residues of that sequence; and (d) a polynucleotide comprising a nucleotide sequence that hybridizes to the sequence of (a), (b), (c) or a complement thereof, under at least low stringency conditions.

## Claims

**1.** A method for detecting endotoxemia in a test subject, comprising detecting in the test subject aberrant expression of at least one endotoxemia marker gene that is expressed in cells of the immune system and that is selected from the group consisting of: (a) a gene having a polynucleotide expression product comprising a nucleotide sequence that shares at least 50% sequence identity with the sequence set forth in any one of SEQ ID NO: 29, 1, 3, 4, 5, 6, 7, 9, 10, 11, 13, 15, 16, 17, 18, 19, 21, 23, 25, 26, 27, 31, 33, 35, 37, 38, 39, 41, 42, 43, 44, 45, 47, 49, 50, 52, 54, 56, 58, 60, 61, 63, 64, 66, 67, 68, 69, 70, 71, 73, 75, 76, 77, 78, 79, 80, 81, 82, 83, 84, 86, 88, 90, 92, 93, 94, 96, 98, 100, 101, 102, 103, 104, 106, 107, 109, 110, 111, 113, 114, 115, 117, 119, 121, 122, 123, 124, 125, 126, 128, 130, 132, 134, 136, 137, 139, 141, 143, 145, 147, 149, 151, 153, 155, 157, 158, 160, 162, 164, 166, 168, 169, 170, 172, 173, 174, 176, 178, 180, 182, 184, 186, 188, 190, 192, 193, 194, 195, 197, 199, 201, 203, 205, 206, 207, 209, 210, 211, 212, 214, 215, 216, 218, 220, 222, 223, 224, 225, 227, 229, 231, 233, 235, 236, 237, 239, 240, 242, 244, 245, 246, 248, 250, 252, 254, 255, 257, 259 260, 262, 264, 266, 268, 269, 270, 271, 272, 274, 276, 278, 279, 280, 282, 284, 286, 288, 290, 292, 294, 296, 298, 300, 302, 304, 305, 306, 307, 309, 311, 312, 314, 315, 316, 318, 320 321, 323 or 325, or a complement thereof; (b) a gene having a polynucleotide expression product comprising a nucleotide sequence that encodes a polypeptide comprising the amino acid sequence set forth in any one of SEQ ID NO: 30, 2, 8, 12, 14, 20, 22, 24, 28, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326; (c) a gene having a polynucleotide expression product comprising a nucleotide sequence that encodes a polypeptide that shares at least 50% sequence similarity with at least a portion of the sequence set forth in any one of SEQ ID NO: 30, 2, 8, 12, 14, 20, 22, 24, 28, 32, 34, 36, 40, 46, 48, 51, 53, 55, 57, 59, 62, 65, 72, 74, 78, 85, 87, 89, 91, 95, 97, 99, 105, 108, 112, 116, 118, 120, 127, 129, 131, 133, 135,138, 140, 142, 144, 146, 148, 150, 152, 154, 156, 159, 161, 163, 165, 167, 171, 175, 177, 179, 181, 183, 185, 187, 189, 191, 196, 198, 200, 202, 204, 208, 213, 217, 219, 221, 226, 228, 230, 232, 234, 236, 238, 241, 243, 247, 249, 251, 253, 256, 258, 261, 263, 265, 267, 273, 275, 277, 281, 283, 285, 287, 289, 291, 293, 295, 297, 299, 301, 303, 308, 310, 313, 317, 319, 322, 324 or 326, wherein the portion comprises at least 15 contiguous amino acid residues of that sequence; and (d) a gene having a polynucleotide expression product comprising a nucleotide sequence that hybridizes to the sequence of (a), (b), (c) or a complement thereof, under at least low stringency conditions;
wherein the aberrant expression is detected by: (1) measuring in a biological sample obtained from the test subject the level or functional activity of an expression product of the at least one endotoxemia marker gene and (2) comparing the measured level or functional activity of each expression product to the level or functional activity of a corresponding expression product in a reference sample obtained from one or more normal subjects, wherein a difference in the level or functional activity of the expression product in the biological sample as compared to the level or functional activity of the corresponding expression product in the reference sample is indicative of the presence of endotoxemia; and
wherein the biological sample comprises blood.

**2.** A method according to claim 1, wherein the presence of endotoxemia is determined when the measured level or functional activity of the or each expression product is 10% higher or 10% lower than the measured level or functional activity of the or each corresponding expression product.

**3.** A method according to claim 1, wherein the absence of endotoxemia is determined when the measured level or functional activity of an individual expression product varies from the measured level or functional activity of an individual corresponding expression product by no more than about 5%.

**4.** A method according to any preceding claim, comprising measuring the level or functional activity of individual expression products of at least two endotoxemia marker genes.

**5.** A method according to any preceding claim, comprising measuring the level or functional activity of individual expression products of at least three endotoxemia marker genes.

**6.** A method according to any preceding claim, comprising measuring the level or functional activity of individual expression products of at least four endotoxemia marker genes.

**7.** A method according to any preceding claim, comprising measuring the level or functional activity of individual expression products of at least five endotoxemia marker genes.

**8.** A method according to any preceding claim, comprising measuring the level or functional activity of individual expression products of at least six endotoxemia marker genes.

**9.** A method according to any preceding claim, comprising measuring the level or functional activity of individual expression products of at least seven endotoxemia marker genes.

**10.** A method according to any preceding claim, wherein the biological sample comprises peripheral blood.

**11.** A method according to any preceding claim, wherein the biological sample comprises leukocytes.

**12.** A method according to any preceding claim, wherein the expression product is a RNA molecule.

**13.** A method according to any preceding claim, wherein the expression product is a target RNA or a DNA copy of the target RNA whose level is measured using at least one nucleic acid probe that hybridizes under at least low stringency conditions to the target RNA or to the DNA copy, wherein the nucleic acid probe comprises at least 15 contiguous nucleotides of an endotoxemia marker polynucleotide.

**14.** A system comprising at least one end station coupled to a base station,
wherein the base station is configured (a) to receive subject data from the end station via a communications network, wherein the subject data represents parameter values corresponding to a measured or normalized level or functional activity of at least one expression product in a biological sample, and (b) to compare the subject data with predetermined data representing a measured or normalized level or functional activity of at least one corresponding expression product in a reference sample to thereby determine any difference in the level or functional activity of the expression product in the biological sample as compared to the level or functional activity of the corresponding expression product in the reference sample.

**15.** Use of a therapeutic compound selected from vasoactive compounds, antibiotics, steroids, antibodies to endotoxin, and anti tumour necrosis factor agents, for the preparation of a medicament for treating an endotoxemia-related condition, wherein the therapeutic compound is administered in concert with an adjunctive therapy selected from non steroidal anti-inflammatory drugs, intravenous saline and oxygen.

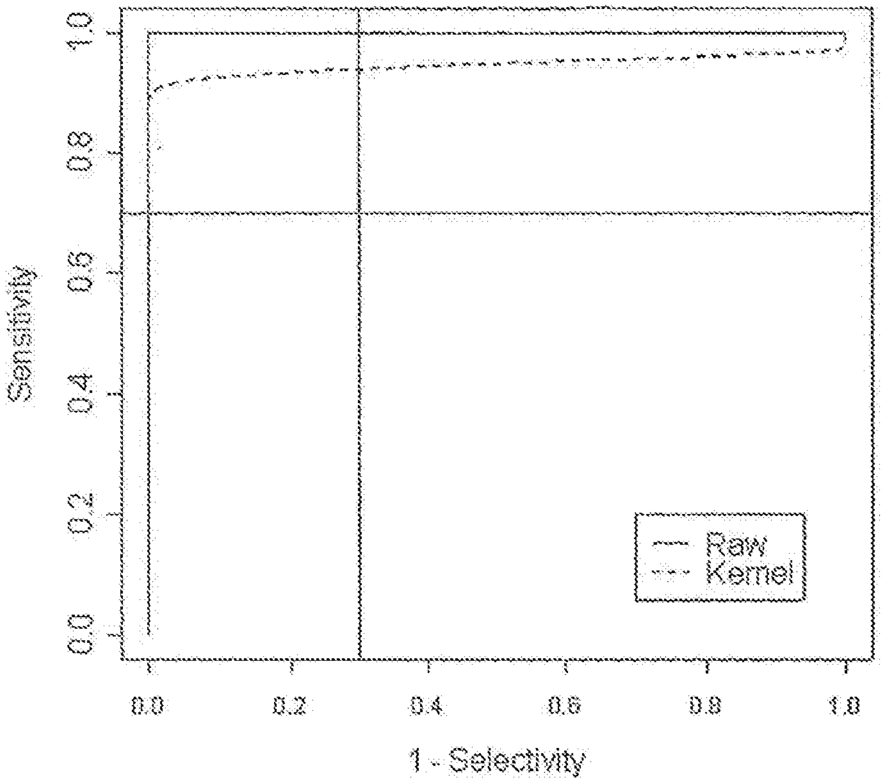


**0 Hours versus 24 Hours**

FIGURE 1

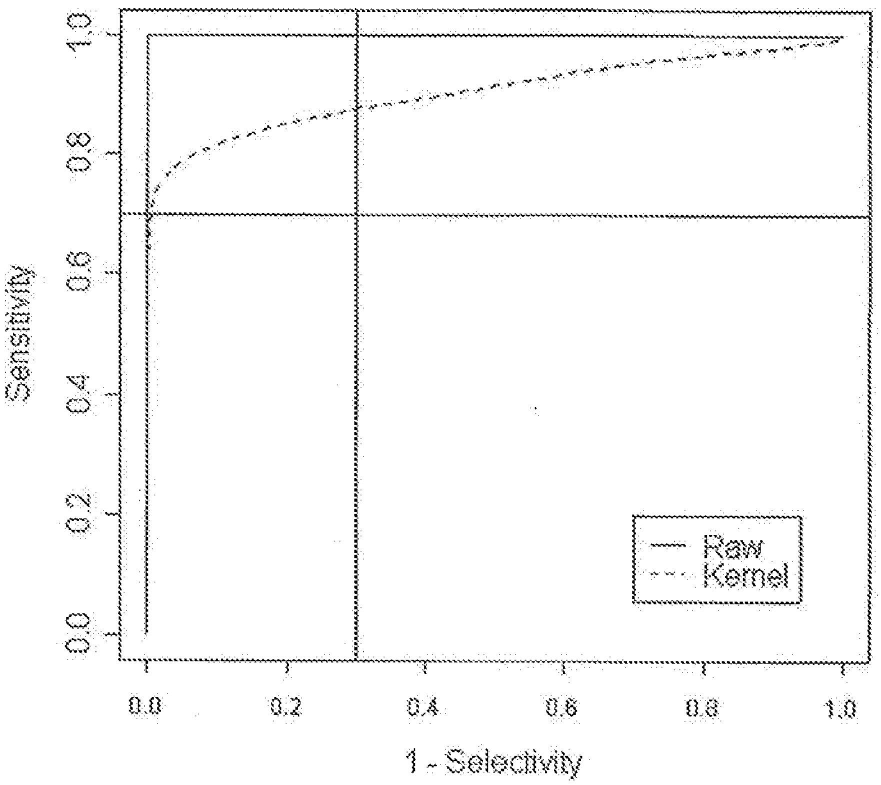


0 Hours versus 72 Hours

FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5424186 A
- WO 0039587 A
- US 4873192 A
- US 5091513 A
- EP 239400 A
- US 5223409 A, Kang
- WO 9218619 A, Dower
- WO 9117271 A, Winter
- WO 9220791 A, Markland
- WO 9215679 A, Breitling
- WO 9301288 A, McCafferty
- WO 9201047 A, Garrard
- WO 9209690 A, Ladner
- WO 9002809 A
- US 4366241 A
- US 4843000 A
- US 4849338 A
- US 4683195 A
- US 4683202 A
- US 4800159 A
- WO 9007641 A
- EP 320308 A
- US 4883750 A
- US 8700880 W
- GB 2202328 A
- US 8901025 W
- WO 8810315 A, Gingeras
- EP 329822 A, Davey
- WO 8906700 A
- WO 9525116 A
- WO 9535505 A
- US 5700637 A
- US 5445934 A
- WO 9306121 A
- US 5573909 A, Singer
- US 5326692 A, Brinkley
- US 5227487 A
- US 5274113 A
- US 5405975 A
- US 5433896 A
- US 5442045 A
- US 5451663 A
- US 5453517 A
- US 5459276 A
- US 5516864 A
- US 5648270 A
- US 5723218 A
- US 5143854 A, Pirrung
- US 5925525 A, Fodor
- US 20020055186 A
- US 20030003599 A
- WO 03062444 A
- WO 03077851 A
- WO 0259601 A
- WO 0239120 A
- WO 0179849 A
- WO 9939210 A
- WO 02090579 A
- AU 0301517 W


### Non-patent literature cited in the description


- **GLAUSER et al.** *Lancet,* September 1991, vol. 338, 732-736
- **COHEN J.** *Intensive Care Med.,* 2000, vol. 26, S51-S56
- **HURLEY JC.** *Clin Microbiol Reviews,* vol. 8 (2), 268-292
- **CASEY et al.** *Ann Intern Med.,* 1993, vol. 119, 771-778
- Gram negative septicemia and septic shock. WB Saunders Philadelphia, 1991
- **PARRILLO JE.** *Ann Intern Med.,* 1990, vol. 113, 227-242
- **PANACEK et al.** *Crit Care Med.,* 2004, vol. 32, 2173-2182
- **ULLOA ; TRACEY.** *Trends Mol Med.,* 2005, vol. 1, 56-63
- **BURAS et al.** *Nature Reviews Drug Discovery,* 2006, vol. 4, 854-865
- **ULLOA et al.** *Trends Mol Biol.,* 2005, vol. 11, 56-63
- **BONE RC.** *Crit Care Med.,* 1996, vol. 24, 1125-1128
- **SLOET VAN OLDRUITENBORGH-OOSTERBAAN MM.** *Vet Quarterly,* 1999, vol. 21 (4), 121-127
- **HOOD DM.** *Vet Clin Nth Amer Eq Pract,* 1999, vol. 15 (2), 287-294
- **HARLOW ; LANE.** Antibodies, A Laboratory Manual. Cold Spring Harbor Publications, 1988

- Peptide Synthesis. **ATHERTON ; SHEPHARD.** Synthetic Vaccines. Blackwell Scientific Publications
- **DEVERAUX et al.** *Nucleic Acids Research,* 1984, vol. 12, 387-395
- **ALTSCHUL et al.** *Nucl. Acids Res.,* 1997, vol. 25, 3389
- **AUSUBEL et al.** Current Protocols in Molecular Biology. John Wiley & Sons Inc, 1994
- **CHINAULT ; CARBON.** *Gene,* 1979, vol. 5, 111-126
- **SAMBROOK et al.** MOLECULAR CLONING. A LABORATORY MANUAL. Cold Spring Harbor Press, 1989
- **AUSUBEL et al.** CURRENT PROTOCOLS IN MOLECULAR BIOLOGY. John Wiley & Sons, Inc, 1994
- **KUNKEL.** *Proc. Natl. Acad. Sci. USA,* 1985, vol. 82, 488-492
- **KUNKEL et al.** *Methods in Enzymol.,* 1987, vol. 154, 367-382
- **WATSON, J. D. et al.** Molecular Biology of the Gene. Benjamin/Cummings, 1987
- **DAYHOFF et al.** Atlas of Protein Sequence and Structure. Natl. Biomed. Res. Found, 1978
- **ARKIN ; YOURVAN.** *Proc. Natl. Acad. Sci. USA,* 1992, vol. 89, 7811-7815
- **DELGRAVE et al.** *Protein Engineering,* 1993, vol. 6, 327-331
- A model of evolutionary change in proteins. Matrices for determining distance relationships. **DAYHOFF et al.** Atlas of protein sequence and structure. National Biomedical Research Foundation, 1978, vol. 5, 345-358
- **GONNET et al.** *Science,* 1992, vol. 256 (5062
- **ZUBAY, G.** Biochemistry. Wm.C. Brown Publishers, 1993
- **COLIGAN et al.** CURRENT PROTOCOLS IN PROTEIN SCIENCE. John Wiley & Sons, Inc, 1995
- **ROBERGE et al.** *Science,* 1995, vol. 269, 202
- **COLIGAN et al.** CURRENT PROTOCOLS IN IMMUNOLOGY. John Wiley & Sons, Inc, 1991
- **KÖHLER ; MILSTEIN.** *Nature,* 1975, vol. 256, 495-497
- **KREBER et al.** *J. Immunol. Methods,* 1997, vol. 201 (1), 35-55
- **WINTER ; MILSTEIN.** *Nature,* 1991, vol. 349, 293
- **PLÜCKTHUN et al.** *Antibody engineering: A practical approach.,* 1996, 203-252
- **GLOCKSCUTHER et al.** *Biochem.,* vol. 29, 1363-1367
- **REITER et al.** *J. Biol. Chem.,* 1994, vol. 269, 18327-18331
- **REITER et al.** *Biochem.,* 1994, vol. 33, 5451-5459
- **REITER et al.** *Cancer Res.,* 1994, vol. 54, 2714-2718
- **WEBBER et al.** *Mol. Immunol.,* 1995, vol. 32, 249-258
- **FUCHS et al.** *Bio/Technology,* 1991, vol. 9, 1370-1372
- **HAY et al.** *Hum Antibod Hybridomas,* 1992, vol. 3, 81-85
- **HUSE et al.** *Science,* 1989, vol. 246, 1275-1281
- **GRIFFTHS et al.** *EMBO J,* 1993, vol. 12, 725-734
- **HAWKINS et al.** *J Mol Biol,* 1992, vol. 226, 889-896
- **CLACKSON et al.** *Nature,* 1992, vol. 352, 624-628
- **GRAM et al.** *PNAS,* 1992, vol. 89, 3576-3580
- **GARRAD et al.** *Bio/Technology,* 1991, vol. 9, 1373-1377
- **HOOGENBOOM et al.** *Nuc Acid Res,* 1991, vol. 19, 4133-4137
- **BARBAS et al.** *PNAS,* 1991, vol. 88, 7978-7982
- **INNIS et al.** PCR Protocols. Academic Press, Inc, 1990
- **HIGUCHI et al.** *Biotechnology,* 1992, vol. 10, 413-417
- **WALKER et al.** *Proc. Natl. Acad. Sci. U.S.A,* 1992, vol. 89, 392-396
- **KWOH et al.** *Proc. Natl. Acad. Sci. U.S.A.,* 1989, vol. 86, 1173
- **VINCENT ; KONG.** *EMBO Reports,* 2004, vol. 5 (8), 795-800
- **FROHMAN, M. A.** PCR Protocols: A Guide to Methods and Applications. Academic Press, 1990
- **OHARA et al.** *Proc. Natl Acad. Sci. U.S.A.,* 1989, vol. 86, 5673-567
- **WU et al.** *Genomics,* 1989, vol. 4, 560
- **BELLUS.** *J Macromol. Sci. Pure, Appl. Chem.,* 1994, vol. A31 (1), 1355-1376
- **KRISTENSEN et al.** *Biotechniques,* vol. 30 (2), 318-322
- **HACIA et al.** *Nature Genetics,* 1996, vol. 14, 441-447
- **SHOEMAKER et al.** *Nature Genetics,* 1996, vol. 14, 450-456
- **PEASE et al.** *Proc. Natl. Acad. Sci. U.S.A.,* 1994, vol. 91, 5022-5026
- **FODOR et al.** *Science,* 1991, vol. 251, 767-773
- NUCLEIC ACID HYBRIDIZATION, A PRACTICAL APPROACH. IRL press, 1985
- **WALLACE et al.** *Nucl. Acids Res.,* 1979, vol. 6, 3543
- **WOOD et al.** *Proc. Natl. Acid. Sci. USA,* 1985, vol. 82, 1585
- **CHRISTENSEN et al.** *Biochem J,* 2001, vol. 354, 481-4
- **JALKANEN et al.** *J. Cell. Biol.,* 1985, vol. 101, 976-985

- **JALKANEN et al.** *J. Cell. Biol.,* 1987, vol. 105, 3087-3096
- **LACOBILLI et al.** *Breast Cancer Research and Treatment,* 1988, vol. 11, 19-30
- **GE.** *Nucleic Acids Res.,* 2000, vol. 28 (2), e3
- **POTYRAILO et al.** *Anal. Chem.,* 1998, vol. 70, 3419-3425
- **COHEN et al.** *Proc. Natl. Acad. Sci. USA,* 1998, vol. 95, 14272-14277
- **FUKUDA et al.** *Nucleic Acids Symp. Ser.,* 1997, vol. 37, 237-238
- **ROBERTS ; SZOSTAK.** *Proc. Natl. Acad. Sci. USA,* 1997, vol. 94, 12297-12302
- **LOPEZ et al.** *J. Chromatogr. B,* 2003, vol. 787, 19-27
- **CAHILL.** *Trends in Biotechnology,* 2000, vol. 7, 47-51
- **COHEN J ; GLAUSER MP.** *Lancet,* 1991, vol. 338, 736-739
- **VAN EPS A ; POLLITT CC.** *Equine Vet J.,* 2004, vol. 36 (3), 255-60
- **IRIZARRAY et al.** *Biostatistics,* 2002
- **RIZARRAY et al.** *Biostatistics,* 2002
- **KRISTENSEN et al.** *Biotechniques,* 2001, vol. 30 (2), 318-322
- **BUSTIN SA.** *J Mol Endocrinol,* 2000, vol. 25, 169-193
- **LONNSTEDT ; SPEED.** *Statistica Sinica,* 2002, vol. 12, 31-46
- **HOLM, S.** *Scandinavian Journal of Statistics,* 1979, vol. 6, 65-70
- **VENABLES ; RIPLEY.** Modem Applied Statistics. S, Springer, 2002
- **JOLLIFFE, I.T.** Principal components analysis. Springer-Verlag, 1986
- **LLOYD, C.J.** *Journal of the American Statistical Association,* 1998, vol. 93, 1356-1364
- **VENABLES W.N. ; RIPLEY B.D.** Modem Applied Statistics. Springer, 2002
- **ALTSCHUL, S.F. ; GISH, W. ; MILLER, W. ; MYERS, E.W. ; LIPMAN, D.J.** Basic local alignment search tool. *J. Mol. Biol.,* 1990, vol. 215, 403-410